(19) Europäisches Patentamt
European Patent Office
Office européen des brevets

(11) **EP 4 406 553 A1**

(12) **EUROPEAN PATENT APPLICATION**

(43) Date of publication:
**31.07.2024 Bulletin 2024/31**

(21) Application number: **23153806.7**

(22) Date of filing: **27.01.2023**

(51) International Patent Classification (IPC):
***A61K 39/395** (2006.01)* ***A61K 31/047** (2006.01)*
***C07K 16/24** (2006.01)*

(52) Cooperative Patent Classification (CPC):
**C07K 16/244; A61K 31/047; A61K 39/39591**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Fresenius Kabi Deutschland GmbH**
  **61352 Bad Homburg (DE)**
- **Coriolis Pharma Research GmbH**
  **82152 Martinsried (DE)**

(72) Inventors:
- **SYKES, Alison**
  **CH - 1262 Eysins (CH)**
- **HAUF, Waldemar**
  **82152 Planegg (DE)**
- **KRHAC LEVACIC, Ana**
  **82152 Planegg (DE)**
- **KEILHAUER, Eva**
  **82152 Planegg (DE)**

(74) Representative: **HGF**
**HGF Limited**
**1 City Walk**
**Leeds LS11 9DX (GB)**

# (54) STABLE SECUKINUMAB BIOPHARMACEUTICAL COMPOSITION

(57) The present invention relates to a biopharmaceutical composition comprising secukinumab. Such compositions are suitable for subcutaneous delivery.

EP 4 406 553 A1

## Description

### *INTRODUCTION*

The present invention relates to a biopharmaceutical composition, particularly a liquid (e.g. aqueous) biopharmaceutical composition, more particularly a liquid biopharmaceutical composition comprising a biopharmaceutical (e.g. an), most particularly a liquid biopharmaceutical composition comprising secukinumab. The present invention also relates *inter alia* to a package including the composition, drug delivery device including the composition, a kit including the composition, a method of manufacturing the aforesaid, and to methods of treatment using the aforesaid.

### *BACKGROUND*

Biopharmaceuticals, such as the anti-IL-17A (interleukin-17A antagonist) human IgG1/κ monoclonal antibody, secukinumab (marketed as Cosentyx®), are proving to be hugely successful in treating an ever-expanding range of autoimmune diseases, such as psoriasis, ankylosing spondylitis, and psoriatic arthritis.

Secukinumab is well known, as its structure and method of production. For instance, secukinumab is disclosed as AIN457 in WO2006/013107 (filed by Novartis Ag, Novartis Pharma Gmbh).

Cosentyx® is FDA approved pursuant to Biologic License Application (BLA) No. 125504, and relevant product characteristics, medical indications, and data related thereto is set forth in the Label published on 28 May 2021 (Reference ID 4803601 - hereinafter the "secukinumab label").

Cosentyx® products are for subcutaneous delivery, and are provided as either a Cosentyx® Injection (single-dose Sensoready pen or single-dose prefilled syringe, each fitted with a 27G fixed ½-inch needle), which is a ready-to-use liquid composition, or Cosentyx® for Injection, which are single-dose vials of lyophilised powder which must be reconstituted in water for injection (WFI) before use (in subcutaneous injection) - see Section 11, "DESCRIPTION", of the secukinumab label for further details.

Each vial (single-dose) of the lyophilised powder characterising the Cosentyx® for Injection product contains 150 mg secukinumab, 4.656 mg L-histidine/histidine hydrochloride monohydrate (in appropriate proportions to furnish the target pH), 0.6 mg polysorbate 80, and 92.43 mg sucrose. Following reconstitution with 1mL of sterile WFI yields the following liquid biopharmaceutical composition:

150 mg/mL secukinumab

30 mM histidine buffer system

0.6 mg/mL polysorbate 80

270 mM sucrose

Water for injection

Resulting in pH 5.8

Such lyophilised compositions are foreshadowed in WO2012/059598 (especially page 99, final paragraph thereof) filed by the originator of Cosentyx®, Novartis Ag.

The liquid biopharmaceutical composition characterising the Cosentyx® Injection product has the following aqueous formulation:

150 mg/mL secukinumab

20 mM histidine buffer system

5 mM L-methionine

0.2 mg/mL polysorbate 80

200 mM trehalose dihydrate

Water for injection

pH adjusted to pH 5.8

Such aqueous secukinumab formulations are foreshadowed in WO2016/103153, especially as the "preferred pharmaceutical product of secukinumab" in Section 1.3 on page 57 thereof.

In terms of providing viable secukinumab formulations, the earlier solution was evidently a lyophilised formulation. Unfortunately, lyophilised formulations suffer the disadvantage of requiring a qualified healthcare professional to reconstitute said lyophilised formulation before use. The need for a trained healthcare professional presents a logistical obstacle, whereas reconstitution involves an additional operational step which may be prone to human error, even by a trained healthcare professional.

The originator established a viable, storable liquid formulation of secukinumab, but according to WO2016/103153 certain features were introduced to mitigate undesirable protein oxidation - such mitigating features include, for example, a substantially oxygen-free (or low-oxygen) headspace alongside the inclusion, within the liquid formulation, of an extra excipient, namely methionine. Either or both such features may be considered undesirable.

An object of the present invention is to provide alternative secukinumab formulations to those of the prior art.

Another object of the present invention is to provide viable alternative secukinumab formulations to those of the prior art.

Another object of the present invention is to provide alternative secukinumab formulations which provide comparable or improved stability (especially antibody stability) compared to those of the prior art whilst using a different combination of features (possibly fewer features or different features).

Another object of the present invention is to provide formulations which alleviate or eliminate one or more are the aforesaid problems associated with prior art formulations, especially those of the originator formulations.

Another object of the present invention is to provide formulations which sufficiently withstand various stresses (agitation, heat, light) to which drug products may be exposed during manufacture, transport, and storage.

Desirably, any new formulations would solve at least one of the aforementioned problems and/or at least one problem inherent in the prior art, and may suitably solve two or more of said problems. Desirably, the problem(s) of the prior art may be solved whilst reducing the complexity of the formulation (be this in terms of product- or process-related features).

## *SUMMARY OF THE INVENTION*

### ***Composition Aspects***

According to an aspect of the present invention, there is provided a biopharmaceutical composition.

According to a further aspect of the present invention, there is provided a (preferably liquid, more preferably aqueous) biopharmaceutical composition comprising secukinumab and, optionally, one or more pharmaceutically-acceptable excipients, carriers, and/or diluents (especially water for injection).

According to a further aspect of the present invention, there is provided a (preferably liquid, more preferably aqueous) biopharmaceutical composition comprising secukinumab; wherein:

the composition further comprises one or more included ingredient(s)/component(s)/feature(s), suitably as defined herein (e.g. buffer, sugar, optional additional tonicifier, surfactant, water, or any combination thereof), said included ingredient(s)/component(s)/feature(s) optionally being present in any amounts, concentrations, relative concentrations (e.g. concentration ratios) as defined herein;

the composition is optionally further characterised by an (substantial) absence of one or more excluded ingredient(s)/component(s)/feature(s), suitably as defined herein (e.g. methionine, amino acids or certain amino acids such as histidine, buffer, disaccharide such as trehalose and/or sucrose, surfactants or certain surfactants);

the composition is optionally further characterised by one or more parameters (e.g. pH, osmolality) and/or features as defined herein; and

the composition optionally consists of said ingredient(s)/component(s)/feature(s), in which case said composition suitably includes a (optionally additional) diluent, preferably water.

According to a further aspect of the present invention, there is provided a (preferably liquid, more preferably aqueous) biopharmaceutical composition comprising secukinumab and, optionally, one or more pharmaceutically-acceptable excipients, carriers, and/or diluents (especially water for injection), wherein the composition is free of (or characterised by an absence of) methionine.

According to a further aspect of the present invention, there is provided a (preferably liquid, more preferably aqueous) biopharmaceutical composition comprising secukinumab and, optionally, one or more pharmaceutically-acceptable excipients, carriers, and/or diluents (especially water for injection), wherein the composition is free of (or characterised by an absence of) histidine.

According to a further aspect of the present invention, there is provided a (preferably liquid, more preferably aqueous) biopharmaceutical composition comprising secukinumab and, optionally, one or more pharmaceutically-acceptable excipients, carriers, and/or diluents (especially water for injection), wherein the composition is free of (or characterised by an absence of) trehalose (and suitably also free of sucrose).

According to a further aspect of the present invention, there is provided a (preferably liquid, more preferably aqueous) biopharmaceutical composition comprising secukinumab and, optionally, one or more pharmaceutically-acceptable excipients, carriers, and/or diluents (especially water for injection), wherein the composition is free of (or characterised by an absence of) methionine, histidine, and trehalose (and suitably also free of sucrose).

According to a further aspect of the present invention there is provided a method of manufacturing a biopharmaceutical composition (suitably as defined herein), the method comprising mixing together (or otherwise combining) secukinumab with one or more pharmaceutically-acceptable excipients, carriers, and/or diluents (wherein suitably the pharmaceutically-acceptable excipients, carriers, and/or diluents are those defined anywhere herein in relation to the biopharmaceutical composition), optionally in any amount, concentration, or form stipulated herein; and optionally adjusting any one or more parameters stipulated herein in relation to a biopharmaceutical composition (e.g. adjusting pH or osmolality).

According to a further aspect of the present invention there is provided a biopharmaceutical composition obtainable by, obtained by, or directly obtained by a method of manufacturing a biopharmaceutical composition as defined herein.

References herein to secukinumab include any biosimilar thereof. References herein to secukinumab may also include any biobetter or biosuperior thereof.

The aforementioned biopharmaceutical compositions preferably include (i.e. comprise or consist of) any, some, or all of the features/components/ingredients described herein in the context of features/components/ingredients to be included within such compositions (e.g. include any of a buffer, surfactant, sugar component, amino acid component, tonicifier, ionic-strength provider, antioxidant, and/or chelator), in any relevant amounts described herein; such biopharmaceutical compositions may suitably exclude (i.e. be free of, substantially free of, or be characterised by an absence or substantial absence of) any, some, or all of the features/components/ingredients described herein in the context of features/components/ingredients to be excluded from such compositions (e.g. exclude any of a buffer, surfactant, sugar component, amino acid component, tonicifier, ionic-strength provider, antioxidant, and/or chelator) or otherwise contain low-levels thereof (suitably with maximum amounts as defined herein); and/or such biopharmaceutical compositions may be preferably characterised by any, some, or all parameters (e.g. pH, osmolality) described herein.

The aforementioned biopharmaceutical composition is preferably a liquid biopharmaceutical composition, most preferably an aqueous biopharmaceutical composition. The present invention may, however, provide a solid biopharmaceutical composition, for instance, a lyophilised biopharmaceutical composition. Such a lyophilised biopharmaceutical composition is suitably capable of reconstitution into a liquid (e.g. aqueous) biopharmaceutical composition, suitably as defined herein. As such, a lyophilised composition may be defined by reference to the liquid composition (e.g. any liquid biopharmaceutical composition as defined herein) produced following its reconstitution or by a product-by-process comprising lyophilising a defined liquid composition (e.g. lyophilising any liquid biopharmaceutical composition as defined herein).

***Container Aspects***

According to a further aspect of the present invention there is provided a container comprising a liquid biopharmaceutical composition as defined herein. Preferably, the container comprises a headspace, wherein the oxygen content in the headspace is greater than 13%, most suitably greater than about 15%. Suitably the container is or is part of a drug-delivery device as defined herein.

According to a further aspect of the present invention there is provided a method of manufacturing a container (suitably as defined herein), the method comprising incorporating a (suitably liquid) biopharmaceutical composition (as defined herein) within a container, suitably so as to leave a headspace. Suitably said headspace is left with or otherwise conditioned to have an oxygen content greater than 13%, most suitably greater than about 15%.

According to a further aspect of the present invention there is provided a container comprising a liquid biopharmaceutical composition, as defined herein, and (substantially) no headspace.

According to a further aspect of the present invention there is provided a method of manufacturing a container (suitably as defined herein), the method comprising incorporating a (suitably liquid) biopharmaceutical composition (as defined herein) within a container so as to leave (substantially) no headspace.

According to a further aspect of the present invention there is provided a container obtainable, obtained by, or directly obtained by a method of manufacturing a container as defined herein.

Notwithstanding the above, the headspace may be characterised by an oxygen content less than 13%, suitably less than about 15%. The headspace may substantially comprise (or consist essentially of or consist of) an inert gas, such as nitrogen, suitably with an oxygen content less than 10%, suitably less than 5%, suitably less than 2%.

***Pharmaceutical Product* Aspects**

According to a further aspect of the present invention, there is provided a pharmaceutical product comprising a container, wherein said container comprises a headspace and a liquid biopharmaceutical composition as defined herein, wherein the oxygen content in the headspace is greater than 13%, most suitably greater than about 15%. Suitably the pharmaceutical product is or is part of a drug-delivery device as defined herein.

According to a further aspect of the present invention there is provided a method of manufacturing a pharmaceutical product (suitably as defined herein), the method comprising incorporating a (suitably liquid) biopharmaceutical composition (as defined herein) within a container so as to leave a headspace. Suitably said headspace is left with or otherwise conditioned to have an oxygen content greater than 13%, most suitably greater than about 15%.

According to a further aspect of the present invention there is provided a method of manufacturing a pharmaceutical product (suitably as defined herein), the method comprising incorporating a (suitably liquid) biopharmaceutical composition (as defined herein) within a container so as to leave (substantially) no headspace.

According to a further aspect of the present invention, there is provided a pharmaceutical product comprising a container, wherein said container comprises a headspace and a liquid biopharmaceutical composition as defined herein; wherein the oxygen content in the headspace is greater than 13%, most suitably greater than about 15%; and wherein the liquid biopharmaceutical composition is free of (or characterised by an absence of) methionine. Suitably the pharmaceutical product is or is part of a drug-delivery device as defined herein.

According to a further aspect of the present invention there is provided a method of manufacturing a pharmaceutical product (suitably as defined herein), the method comprising incorporating a (suitably liquid) biopharmaceutical composition (as defined herein) within a container so as to leave a headspace (suitably having an oxygen content greater than 13%, most suitably greater than about 15%), wherein the liquid biopharmaceutical composition is free of (or characterised by an absence of) methionine.

According to a further aspect of the present invention there is provided a method of manufacturing a pharmaceutical product (suitably as defined herein), the method comprising incorporating a (suitably liquid) biopharmaceutical composition (as defined herein) within a container so as to leave (substantially) no headspace, wherein the liquid biopharmaceutical composition is free of (or characterised by an absence of) methionine.

According to a further aspect of the present invention there is provided a pharmaceutical product obtainable, obtained by, or directly obtained by a method of manufacturing a pharmaceutical product as defined herein.

***Drug Delivery Device Aspects***

According to a further aspect of the present invention there is provided a drug delivery device (e.g. vial, ampoule, syringe, pre-filled syringe, injection pen (e.g. essentially incorporating a syringe), autoinjector, or intravenous bag) comprising a biopharmaceutical composition as defined herein. Most preferably, the drug delivery device is an injection pen (sometimes termed "injector pen") or a pre-filled syringe, most suitably fitted with a needle, suitably wherein the needle has a gauge between 21 and 35G (preferably 27G) and a length between 0.5 mm and 10 mM (preferably a ½-inch needle). Preferably, the drug delivery device comprises a headspace, wherein the oxygen content in the headspace is greater than 13%, most suitably greater than about 15%. Preferably the drug-delivery device comprises a container as defined herein. Preferably the drug-deliver device is or comprises a pharmaceutical product as defined herein.

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave a headspace (suitable having an oxygen content greater than 13%, most suitably greater than about 15%).

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave (substantially) no headspace.

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave a headspace (suitable having an oxygen content greater than 13%, most suitably greater than about 15%), wherein the liquid biopharmaceutical composition is free of (or characterised by an absence of) methionine.

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave (substantially) no headspace, wherein the liquid biopharmaceutical composition is free of (or characterised by an absence of) methionine.

### *Package Aspects*

According to a further aspect of the present invention there is provided a package comprising one or more containers as defined herein.

According to a further aspect of the present invention there is provided a package comprising one or more pharmaceutical products as defined herein.

According to a further aspect of the present invention there is provided a package comprising one or more drug-delivery devices as defined herein.

### *Kit Aspects*

According to a further aspect of the present invention there is provided a kit of parts comprising a drug delivery device as defined herein, a biopharmaceutical composition as defined herein (optionally contained in a container, suitably as defined herein, which container is suitably is, is associated with, or is a part of the drug delivery device), and optionally a set of instructions with directions regarding the administration (e.g. sub-cutaneous) of the biopharmaceutical composition.

### *Methods of Treatment Aspects*

According to a further aspect of the present invention there is provided a method of treating a disease or medical disorder in a patient in need of such treatment, said method comprising administering to said patient a therapeutically effective amount of a biopharmaceutical composition as defined herein. Administering most preferably comprises subcutaneously-administering a therapeutically effective amount of the biopharmaceutical composition, suitably *via* subcutaneous injection.

According to a further aspect of the present invention there is provided a biopharmaceutical composition, as defined herein, for use in treating a disease or medical disorder in a patient in need of such treatment.

According to a further aspect of the present invention there is provided a use of a biopharmaceutical composition, as defined herein, in the manufacture of a medicament for the treatment of a disease or disorder.

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is an IL-17A-related autoimmune disease.

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is selected from the group consisting of psoriasis, ankylosing spondylitis, psoriatic arthritis, and rheumatoid arthritis).

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is selected from the group consisting of psoriasis, ankylosing spondylitis, and psoriatic arthritis.

All of the aforesaid methods of treatment, composition(s) for use, and use of composition(s) in the manufacture of a medicament, are equally applicable to the relevant packages, containers, pharmaceutical products, drug-delivery devices, and kits incorporating said composition(s).

The aforementioned aspects and features may be applied to any of the embodiments of numbered paragraphs A1-A511, B1-B255, C1-C255, D1-D1152, E1-E1151, F1-F12, G1-G959, and H1-H1423 defined hereinafter. The aforementioned aspects and features may also be applied to any of the embodiments of numbered paragraphs 11-12400 and J1-J1158.

Any features, including optional, suitable, and preferred features, described in relation to any particular aspect of the invention may also be features, including optional, suitable and preferred features, of any other aspect of the present invention.

***BRIEF DESCRIPTION OF THE DRAWINGS***

For a better understanding of the invention, and to show how embodiments of the same are put into effect, reference is now made, by way of example, to the following figures, in which:

Figure 1 is an exemplary chromatogram of a water blank injection taken from Cysteinylation analysis of Secukinumab by HIC HPLC

Figure 2 is an exemplary chromatogram of a mobile phase A blank injection taken from Cysteinylation analysis of Secukinumab by HIC HPLC.

Figure 3 is an exemplary chromatogram of a FKS730 CYS CTL injection taken from Cysteinylation analysis of Secukinumab by HIC HPLC .

Figure 4 shows: a) an exemplary chromatographic profile of reference material (Cosentyx); and b) a zoomed in view of a) Cysteinylation analysis of Secukinumab by HIC HPLC.

Figure 5 shows a reference profile for Biorad gel filtration standard (Biorad GFS) taken from aggregate (HMW) analysis by SEC HPLC.

Figure 6 shows a reference profile for FKS730 (and also Cosentyx®) reference standard taken from aggregate analysis by SEC HPLC.

Figure 7 shows an enlarged view of reference profile for Cosentyx SNH96 reference standard taken from aggregate analysis by SEC HPLC.

Figure 8 shows a typical peak profile of the SST analyzed in fluorescence mode taken from charged variant analysis by icIEF.

Figure 9 shows a typical peak profile of the reference standard a) with CpB digestion; and b) without CpB digestion taken from charged variant analysis by icIEF.

Figure 10 shows nanoDSF results for F1-F9, namely: a) Mean Tm, (left bar) and Mean $Tm_2$ (right bar) for each formulation; and b) $Tm_{onset}$ (left bar) and $Tm_{agg}$ (right bar) for each formulation.

Figure 11 shows the results of micro-flow imaging (MFI), by reference to particles per container: a) for particles ≥ 2 μm; b) for particles ≥ 5 μm; c) for particles ≥ 10 μm; and d) for particles ≥ 25 μm.

Figure 12 shows SEC results as bar charts for each formulation F1-F9 (bars left to right are T0, T1m_25°C, and T1 m_40°C respectively) for: a) high molecular weights (HMW); b) low molecular weights (LMW); and c) monomer.

Figure 13 shows hydrophobic interaction chromatography (HIC) traces for: a) F1 at T1m_40°C, F2 at T1m_40°C, and Cosentyx; and b) F1 at T1m_40°C, F6 at T1 m_40°C, and Cosentyx.

Figure 14 shows hydrophobic interaction chromatography (HIC) results in the form of bar charts (left-to-right bars relate to T0, T1m_25C, and T1m_40C respectively with T0 results missing for "unknown" components) in relation to: a) uncysteinylated form; b) "unknown" components; c) oxidized form; d) pyroglutamate form.

Figure 15 shows the freeze thaw cycles (temperature against time) to which samples (in this case F2, F12, and F13) were subjected, alongside target and actual shelf temperatures.

Figure 16 shows nanoDSF results for F2, F10-F14, namely: a) Mean Tm, (left bar) and Mean $Tm_2$ (right bar) for

each formulation; and b) $Tm_{onset}$ (left bar) and $Tm_{agg}$ (right bar) for each formulation.

Figure 17 shows the results of micro-flow imaging (MFI) for each of F2, F10-F14, by reference to particles per container: a) for particles ≥ 2 μm; b) for particles ≥ 5 μm; c) for particles ≥ 10 μm; and d) for particles ≥ 25 μm.

Figure 18 shows SEC results as bar charts for each formulation F2, F10-F14 (bars left to right are T0, Tox, TFT, T1m_25°C respectively) for: a) high molecular weights (HMW); b) low molecular weights (LMW); and c) monomer.

Figure 19 shows hydrophobic interaction chromatography (HIC) results in the form of bar charts charts for each formulation F2, F10-F14 (left-to-right bars relate to T0, Tox, TFT, and T1m_25C respectively) in relation to: a) uncysteinylated form; b) "unknown" components; c) oxidized form; d) pyroglutamate form.

***DETAILED DESCRIPTION OF THE INVENTION***

## DEFINITIONS

Unless otherwise stated, the following terms used in the specification and claims have the following meanings set out below.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

For the avoidance of doubt, it is hereby stated that the information disclosed earlier in this specification under the heading "Background" is relevant to the invention and is to be read as part of the disclosure of the invention.

Unless otherwise stated, wherever a dependent claim/paragraph depends upon a stipulated claim/paragraph "...*or any other claims/paragraphs dependent thereon*" (or similar such language, e.g. "the composition as defined in paragraph A1 or any preceding paragraphs dependent thereon"), such "other claims/paragraphs" suitably include any claims/paragraphs that depend directly or indirectly (i.e. *via* one or more other dependencies) on the stipulated claim/paragraph. In such a manner, numbered paragraphs, much like patent claims, may be multiply dependent.

It will be readily understood that numbered paragraphs can include alphanumerically-"numbered" paragraphs (e.g. A1-A12, which are sequentially numbered 1-12 each with the prefix "A"). Wherever a range of numbered paragraphs is designated, for example *"the composition of A1-A12",* as with patent claims this discloses each numbered paragraph within that range - in the case of "the composition of B1-B6", this includes "the composition" as defined in any of paragraphs B1, B2, B3, B4, B5, and B6.

References herein to a biopharmaceutical or biopharmaceutical active, including secukinumab, include the originator drug substance, whether as commercially available, as described in a patent document, or as described elsewhere in the art, and also biosimilars thereof. Under appropriate circumstances, such references may also include biobetters (or biosuperiors) of an originator drug substance.

In general, a biopharmaceutical, biopharmaceutical active, biopharmaceutically-active substance, and such like, may pertain to any biopharmaceutical - that is a medical product (or biologic) or drug that is manufactured in, extracted from, or semi-synthesized from biological sources. As such, biopharmaceuticals may *inter alia* encompass blood or blood components, vaccines, gene therapies, recombinant therapeutic protein, allergenics, stem cells, somatic cells, tissues, as well as living cells used in cell therapy. Biopharmaceuticals may comprise sugars, proteins, nucleic acids and combinations thereof. Typically, biopharmaceuticals, or precursors or components thereof / therefor, are isolated from living sources, whether human, animal, plant, fungal, or microbial. Generally, however, biopharmaceuticals refer to drugs produced by recombinant DNA technology, such as proteins substantially identical to a body's native proteins (e.g. erythropoetin, growth hormone, insulin); monoclonal antibodies (e.g. adalimumab, nivolumab, pembrolizumab, secukinumab); and fusion proteins (e.g. etanercept).

"Antibodies", and pertinent nomenclature such as "monoclonal", "polyclonal", "IgG", "IgG1", are well known terms of art.

Secukinumab is an anti-IL-17A (interleukin-17A antagonist) human IgG1/κ monoclonal antibody, and is the key active ingredient in the biopharmaceutical product marketed as Cosentyx®. Secukinumab is well known, as its structure and method of production, and further details thereof are available in WO2006/013107 (filed by Novartis Ag, Novartis Pharma Gmbh, where secukinumab is referred to as AIN457. Details of secukinumab, including amino acid sequence of the heavy and light chains, are also available on the KEGG database under entry number D09967, and indeed other well-known databases, where secukinumab is CAS #1229022-83-6. Though Section 11 ("DESCRIPTION") of the FDA secukinumab Label published on 28 May 2021 (Reference ID 4803601 - herein also referred to as the "secukinumab label") states that "Secukinumab has a molecular mass of approximately 151 kDa; both heavy chains of secukinumab contain oligosaccharide chains", for the purposes of various molar calculations the molecular weight of secukinumab is suitably taken to be 148 kDa (reference molecular weight) based on details disclosed on the KEGG database, where the molecular formula is taken as $C_{6584}H_{10134}N_{1754}O_{2042}S_{44}$. As such, a liquid biopharmaceutical composition containing 150 mg/mL secukinumab may be considered a 1.01 mM solution of secukinumab. A liquid biopharmaceutical composition containing 200 mg/mL secukinumab may be considered a 1.35 mM solution of secukinumab. A liquid biopharmaceutical composition containing 190 mg/mL secukinumab may be considered a 1.28 mM solution of secukinumab. This is not intended to be in any way limiting regarding the nature of any biosimilars of secukinumab covered by the scope of the present invention, nor the level of glycosylation or oligosaccharides, either of which may affect the actual molecular weight. However, where the originator or a biosimilar does have a different molecular weight, the abovementioned reference molecular weight should be suitably used for the purposes of assessing whether or not such a biosimilar falls within the scope of any molar definitions stipulated within this specification. So, the number of moles in a known weight of said biosimilar should be calculated, just for the purposes of this invention, using the above reference molecular weight.

References herein to secukinumab may include biosimilars (or biobetters/biosuperiors) which, for instance, may share at least 75%, suitably at least 80%, suitably at least 85%, suitably at least 90%, suitably at least 95%, suitably at least 96%, suitably at least 97%, suitably at least 98%, suitably at least 99% or most suitably 100% amino acid sequence identity with amino acid sequences of secukinumab (e.g. as well known in the art, as defined in the aforementioned patent documents, and/or KEGG database). Alternatively or additionally, references herein to secukinumab may include biosimilars (or biobetters/biosuperiors) which exhibit at least 75%, suitably at least 80%, suitably at least 85%, suitably at least 90%, suitably at least 95%, suitably at least 96%, suitably at least 97%, suitably at least 98%, suitably at least 99% or most suitably 100% amino acid sequence homology with amino acid sequences of secukinumab. Alternatively or additionally, references herein to secukinumab may include biosimilars (or biobetters/biosuperiors) which exhibit at least 75%, suitably at least 80%, suitably at least 85%, suitably at least 90%, suitably at least 95%, suitably at least 96%, suitably at least 97%, suitably at least 98%, suitably at least 99% or most suitably 100% nucleic acid sequence identity with nucleic acid sequences encoding secukinumab. Alternatively or additionally, references herein to secukinumab may include biosimilars (or biobetters/biosuperiors) which exhibit at least 75%, suitably at least 80%, suitably at least 85%, suitably at least 90%, suitably at least 95%, suitably at least 96%, suitably at least 97%, suitably at least 98%, suitably at least 99% or most suitably 100% nucleic acid sequence homology with nucleic acid sequences encoding secukinumab. Alternatively or additionally, a biosimilar (or biobetters/biosuperiors) may have a different glycosylation profile, even if the protein (amino acid) sequence is the same or substantially the same or different to the extent specified above. Most preferably, references herein to secukinumab means biosimilars (or the originator) that has an identical amino acid primary sequence.

The term "sequence identity": the identity between two or more nucleic acid sequences, or two or more amino acid sequences, is expressed in terms of the identity or similarity between the sequences. Sequence identity can be measured in terms of percentage identity; the higher the percentage, the more identical the sequences are The percentage identity is calculated over the entire length of the sequence. Homologs or orthologs of nucleic acid or amino acid sequences possess a relatively high degree of sequence identity when aligned using standard methods. This homology is more significant when the orthologous proteins or cDNAs are derived from species which are more closely related (e.g., human and mouse sequences), compared to species more distantly related (e.g., human and C. elegans sequences).

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Nat. Acad Sci. USA 85:2444, 1988; Higgins & Sharp, Gene, 73:23744, 1988; Higgins & Sharp, CABIOS 5:151-3, 1989; Corpet et al., Nuc. Acids Res. 16:10881-90, 1988; Huang et al. Computer Appls. in the Biosciences 8, 155-65, 1992; and Pearson et al., Meth Mol. Bio. 24:307-31, 1994. Altschul et al., J. Mol. Biol. 215:403-10, 1990, presents a detailed consideration of sequence alignment methods and homology calculations.

The NCBI Basic Local Alignment Search Tool (BLAST) (Altschul et al., J. Mol. Biol. 215:403-10, 1990) is available from several sources, including the National Center for Biological Information (NCBI, National Library of Medicine, Building 38A, Room 8N805, Bethesda, Md. 20894,US) and on the Internet, for use in connection with the sequence analysis programs blastp, blastn, blastx, tblastn and tblastx. Additional information can be found at the NCBI web site.

BLASTN is used to compare nucleic acid sequences, while BLASTP is used to compare amino acid sequences. To compare two nucleic acid sequences, the options can be set as follows: -i is set to a file containing the first nucleic acid sequence to be compared (e.g., C:\seq1.txt); j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\B12seq-i c:\seq1.txt-j c:\seq2.txt-p blastn-o c:\output.bct-q-1-r2.

To compare two amino acid sequences, the options of B12seq can be set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\B12seq-i c:seq1.txt-j c:seq2.txt-p blastp-o c:\output.bct. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (e.g., 100 consecutive nucleotides or amino acid residues from a sequence set forth in an identified sequence), followed by multiplying the resulting value by 100. For example, a nucleic acid sequence that has 1166 matches when aligned with a test sequence having 1154 nucleotides is 75.0 percent identical to the test sequence (i.e., 1166/1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer.

For comparisons of amino acid sequences of greater than about 30 amino acids, the Blast 2 sequences function is employed using the default BLOSUM62 matrix set to default parameters, (gap existence cost of 11, and a per residue gap cost of 1). Homologs are typically characterized by possession of at least 70% sequence identity counted over the full-length alignment with an amino acid sequence using the NCBI Basic Blast 2.0, gapped blastp with databases such as the nr or swissprot database. Queries searched with the blastn program are filtered with DUST (Hancock and Armstrong, 1994, Comput. Appl. Biosci. 10:67-70). Other programs use SEG. In addition, a manual alignment can be performed. Proteins with even greater similarity will show increasing percentage identities when assessed by this method, such as at least 75%, 80%, 85%, 90%, 95%, or 99% sequence identity.

When aligning short peptides (fewer than around 30 amino acids), the alignment should be performed using the Blast 2 sequences function, employing the PAM30 matrix set to default parameters (open gap 9, extension gap I penalties). Proteins with even greater similarity to the reference sequence will show increasing percentage identities when assessed by this method, such as at least 60%, 70%, 75%, 80%, 85%, 90%, 95%, 98%, 99% sequence identity. When less than the entire sequence is being compared for sequence identity, homologs will typically possess at least 75% sequence identity over short windows of 10-20 amino acids, and can possess sequence identities of at least 85%, 90%, 95% or 98% depending on their identity to the reference sequence. Methods for determining sequence identity over such short windows are described at the NCBI web site.

One indication that two nucleic acid molecules are closely related is that the two molecules hybridize to each other under stringent conditions. Stringent conditions are sequence-dependent and are different under different environmental parameters.

Nucleic acid sequences that do not show a high degree of identity may nevertheless encode identical or similar (conserved) amino acid sequences, due to the degeneracy of the genetic code. Changes in a nucleic acid sequence can be made using this degeneracy to produce multiple nucleic acid molecules that all encode substantially the same protein. Such homologous nucleic acid sequences can, for example, possess at least 60%, 70%, 80%, 90%, 95%, 98%, or 99% sequence identity determined by this method.

The term "biosimilar" (also known as follow-on biologics) is well known in the art, and the skilled person would readily appreciate when a drug substance would be considered a biosimilar of any particular antibody drug, including secukinumab. Furthermore, such "biosimilars" would need to be officially approved as a "biosimilar" for marketing before said "biosimilar" is sold on the open market. The term "biosimilar" is generally used to describe subsequent versions (generally from a different source) of "innovator biopharmaceutical products" that have been previously officially granted marketing authorisation. Since biologics have a high degree of molecular complexity, and are generally sensitive to changes in manufacturing processes (e.g. if different cell lines are used in their production), and since subsequent follow-on manufacturers generally do not have access to the originator's molecular clone, cell bank, know-how regarding the fermentation and purification process, nor to the active drug substance itself (only the innovator's commercialized drug product), any "biosimilar" is unlikely to be exactly the same as the innovator drug product.

A "biosimilar" is suitably as defined by the US Food & Drug Administration (FDA), where biosimilar and interchangeable products are described thus (https://www.fda.gov/Drugs/DevelopmentApprovalProcess/HowDrugsareDevelopedandApproved/ApprovalApplications/Therapeutic BiologicApplications/Biosimilars/ucm580419.htm#biosimilar):

"A biosimilar is a biological product that is highly similar to and has no clinically meaningful differences from an existing FDA-approved reference product."

"A reference product is the single biological product, already approved by FDA, against which a proposed biosimilar product is compared. A reference product is approved based on, among other things, a full complement of safety and effectiveness data. A proposed biosimilar product is compared to and evaluated against a reference product to ensure that the product is highly similar and has no clinically meaningful differences."

"A manufacturer developing a proposed biosimilar demonstrates that its product is highly similar to the reference product by extensively analyzing (i.e., characterizing) the structure and function of both the reference product and the proposed biosimilar. State-of-the-art technology is used to compare characteristics of the products, such as purity, chemical identity, and bioactivity. The manufacturer uses results from these comparative tests, along with other information, to demonstrate that the biosimilar is highly similar to the reference product.

Minor differences between the reference product and the proposed biosimilar product in clinically inactive components are acceptable. For example, these could include minor differences in the stabilizer or buffer compared to what is used in the reference product. Any differences between the proposed biosimilar product and the reference product are carefully evaluated by FDA to ensure the biosimilar meets FDA's high approval standards.

As mentioned above, slight differences (i.e., acceptable within-product variations) are expected during the manufacturing process for biological products, regardless of whether the product is a biosimilar or a reference product. For both reference products and biosimilars, lot-to-lot differences (i.e., acceptable within-product differences) are carefully controlled and monitored."

"A manufacturer must also demonstrate that its proposed biosimilar product has no clinically meaningful differences from the reference product in terms of safety, purity, and potency (safety and effectiveness). This is generally demonstrated through human pharmacokinetic (exposure) and pharmacodynamic (response) studies, an assessment of clinical immunogenicity, and, if needed, additional clinical studies."

"An interchangeable product is a biosimilar product that meets additional requirements outlined by the Biologics Price Competition and Innovation Act. As part of fulfilling these additional requirements, information is needed to show that an interchangeable product is expected to produce the same clinical result as the reference product in any given patient. Also, for products administered to a patient more than once, the risk in terms of safety and reduced efficacy of switching back and forth between an interchangeable product and a reference product will have been evaluated. An interchangeable product may be substituted for the reference product without the involvement of the prescriber. FDA's high standards for approval should assure health care providers that they can be confident in the safety and effectiveness of an interchangeable product, just as they would be for an FDA-approved reference product."

"As mentioned above, an interchangeable product, in addition to being biosimilar, meets additional requirements based on further evaluation and testing of the product. A manufacturer of a proposed interchangeable product will need to provide additional information to show that an interchangeable product is expected to produce the same clinical result as the reference product in any given patient. Also, for a product that is administered to a patient more than once, a manufacturer will need to provide data and information to evaluate the risk, in terms of safety and decreased efficacy, of alternating or switching between the products.

As a result, a product approved as an interchangeable product means that FDA has concluded it may be substituted for the reference product without consulting the prescriber. For example, say a patient self-administers a biological product by injection to treat their rheumatoid arthritis. To receive the biosimilar instead of the reference product, the patient may need a prescription from a health care prescriber written specifically for that biosimilar. However, once a product is approved by FDA as interchangeable, the patient may be able to take a prescription for the reference product to the pharmacy and, depending on the state, the pharmacist could substitute the interchangeable product for the reference product without consulting the prescriber. Note that pharmacy laws and practices vary from state to state.

FDA undertakes a rigorous and thorough evaluation to ensure that all products, including biosimilar and interchangeable products, meet the Agency's high standards for approval."

The term "biobetter" (or "biosuperior") refers to improved versions of an existing biologics which, for instance, may have a slightly different structure (and generally a different primary amino acid sequence) that renders the drug safer and/or more effective. A biobetter has the same target as the original biologic but, in contrast to biosimilars, biobetters are different from the existing biologic drug and generally require regulatory evaluation as new drugs.

The term "buffer" (or "buffer system") is well known in the art. Herein, the terms "buffer" and "buffer system" may be used interchangeably. Herein, a "buffered solution" is a generally aqueous solution comprising a buffer (or buffer system) and optionally one or more further components (i.e. particularly applicable to compositions of the invention). Such a buffered solution comprises buffering species (i.e. the species in dynamic equilibrium with one another, e.g. acetate/acetic acid for an acetate buffer system) that contribute to the buffer/buffer system, and thus comprises a mixture of an acid (usually a weak acid, e.g. acetic acid, citric acid, imidazolium form of histidine, succinic acid) and its conjugate base (e.g. an acetate or citrate salt, for example, sodium acetate, sodium citrate, histidine, succinate salt) or alternatively a mixture of a base (usually a weak base, e.g. histidine, succinate salt) and its conjugate acid (e.g. protonated histidine salt, succinic acid). The pH of a "buffer solution" will change very only slightly upon addition of a small quantity of strong acid or base due to the "buffering effect" imparted by the buffer/buffer system.

Herein, a "buffer system" comprises one or more buffering agent(s) and/or an acid/base conjugate(s) thereof, and most suitably comprises one buffering agent only and an acid/base conjugate thereof. As such, though a "buffer system" may in fact comprise one or more buffer systems (e.g. a mixed buffer system, such as a dual-buffer system, for instance, comprising or consisting of an acetate buffer system and a histidine buffer system), suitably the buffer system is a single buffer system (e.g. a mono-buffer system, for instance, comprising or consisting of a histidine buffer system).

Unless stated otherwise, any concentrations stipulated herein in relation to a "buffer system" in general suitably refers to the combined concentration of all of the buffering agent(s) and/or acid/base conjugate(s) that contribute to the overall buffer system (whether a mono-buffer system or mixed buffer system). As such, a given concentration of a general buffer system that consists only of an acetate buffer system generally relates to the combined concentration of acetate (or acetate salt(s), e.g. sodium acetate) and acetic acid. However, a given concentration of a general buffer system that consists of both an acetate buffer system and a histidine buffer system (i.e. a dual-buffer system) generally relates to the combined concentration of acetate, acetic acid, histidine, and protonated histidine.

Unless stated otherwise, any concentrations stipulated herein in relation to a specific "buffer system" (e.g. an acetate buffer system as opposed to a buffer system in general) suitably refers to the combined concentration of the buffering agent(s) and/or acid/base conjugate(s) that constitute that specific buffer system, thus suitably excluding concentrations of any other buffering species that do not contribute to that specific buffer system. As such, a given concentration of an acetate buffer system generally relates to the combined concentration of acetate (or acetate salt(s), e.g. sodium acetate) and acetic acid, whereas a given concentration of a histidine buffer system generally relates to the combined concentration of histidine and protonated histidine (imidazolium form of histidine).

Herein, the term "buffering agent" refers to an acid or base component (usually a weak acid or weak base) of a buffer or buffer solution. A buffering agent helps maintain the pH of a given solution at or near to a pre-determined value, and the buffering agents are generally chosen to complement the pre-determined value. A buffering agent is suitably a single compound which gives rise to a desired buffering effect, especially when said buffering agent is mixed with (and suitably capable of proton exchange with) an appropriate amount (depending on the pre-determined pH desired) of its corresponding "acid/base conjugate", or if the required amount of its corresponding "acid/base conjugate" is formed *in situ* - this may be achieved by adding strong acid or base until the required pH is reached. By way of example:

- An acetate "buffering agent" is suitably an acetate salt, for example, sodium acetate, suitably mixed with its acid/base conjugate, acetic acid. Such a buffer system may be formed by simply mixing a given amount of sodium acetate with a given amount of acetic acid. Alternatively, however, such a buffer may be formed by adding a given amount of a base, suitably a strong base (e.g. sodium hydroxide) to the acetic acid until the desired pH (and thus the desired balance of sodium acetate/acetic acid) is reached. Herein, except where the contrary is stated, any concentrations given in relation to an acetate buffer or acetate buffering agent suitably refer to the combined concentration of the buffering agent(s) (e.g. sodium acetate) and/or acid/base conjugate(s) thereof (e.g. acetic acid).. The skilled person is readily able to calculate such concentrations. Such concentrations may be calculated by reference to the combined concentrations of buffering agent(s) and acid/base conjugate(s), where a buffer system is formed by simply mixing together buffering agent(s) and acid/base conjugate(s). Alternatively, where a buffer system is formed by mixing either the buffering agent(s) or acid/base conjugate(s) with a pH adjuster (e.g. strong acid or strong base) to produce a mixture of each, suitably such concentrations may be calculated by reference to the starting amounts/concentrations of the buffering agent(s) or acid/base conjugate(s) respectively. For example, where a buffer system is formed using a known amount/concentration of acetic acid which is mixed with a pH adjuster (e.g. sodium hydroxide) until the desired pH is reached, the concentration of the buffer system may be calculated by reference to the initial amount of acetic acid.

Herein, an "acid/base conjugate" refers to the conjugate acid or conjugate base (whichever is relevant at a particular pH - typically the conjugate acid in the context of the present invention) of a particular "buffering agent". The acid/base conjugate of an acetate buffering agent (e.g. sodium acetate) is suitably acetic acid.

Herein, the term "buffering species" refers to the particular species (excluding any associated counteranions or countercations - i.e. ignore sodium ions for sodium acetate/acetic acid systems) of a given buffer system which are in dynamic equilibrium with (and proton-exchange with) one another. For example, acetate anions and acetic acid together constitute the "acetate buffering species" of a "acetate buffer system".

Since it is somewhat difficult to define quantities (whether absolute or relative) of a buffer system by reference to weight (since the total weight will depend on the desired pH, which will affect the amount of counterions present), herein weight-based quantities may instead be determined by reference to a theoretical weight of the relevant "buffering species". At least two species are present in any given set of "buffering species" (in relative amounts that can only be determined by reference to the pH), each with a different molecular weight (which usually differs by just 1). Therefore, to enable viable weight calculations and references, for the purposes of this specification the weight of any given set of "buffering species" is given as a theoretical weight based on just one of the buffering species, namely the most acidic of the buffering species (i.e. the most protonated form at any given pH). So the weight of a given set of "buffering species" is quoted as the weight of acid-species equivalents. By way of example, in an acetate buffer system the acetate buffering species may consist of acetate anions (ignore countercations) and acetic acid. The weight of the "buffering species" is therefore calculated as if acetic acid was the only species present in the buffer system (even though acetate is clearly present alongside acetic acid). Thus, any reference to a weight or weight ratio involving a "acetate buffering species" suitably refers to the theoretical weight of acetic acid equivalents within the buffer system. As such, where a composition is formed by adding a pH adjuster (e.g. sodium hydroxide) to a fixed amount of acetic acid, the original weight of acetic acid may be considered to be the weight of the "buffering species" regardless of the ultimate pH. Alternatively, if the concentration (i.e. molarity) of a buffer system is known, this can be converted into a weight of "buffering species" by reference to the molecular weight of the most acidic form of the relevant buffering species (e.g. acetic acid), and ignoring the fact that acetate anions are also present.

Compositions of the invention, particularly liquid (e.g. aqueous) compositions, may be considered "buffered solutions", thereby comprising a buffer system (optionally comprising one or more specific buffer systems) and one or more additional components/ingredients of the composition.

The overall pH of the composition comprising the relevant buffer system(s) is generally a reflection of the equilibrium concentration of each of the relevant buffering species (i.e. the balance of buffering agent(s) to acid/base conjugate(s)), and *vice versa* - modifying the pH may affect the equilibrium concentration of each of the relevant buffering species. This phenomenon is mathematically encapsulated in the well-known "Henderson-Hasselbalch equation" which, in the context of buffer systems and compositions comprising buffer systems, links pH (e.g. pH of an overall composition) with the relative conjugate acid 1 conjugate base concentrations for the or each buffer system, and the $pK_a$ of said conjugate acid. The equation in question is:

$$pH = pK_a + \log_{10}\frac{[Base]}{[Acid]}$$

The $pK_a$s of various conjugate acids are well documented. For instance, the conjugate acid (protonated form) of histidine (i.e. the imidazolium form of histidine) has a $pK_a$ of about 6. From this it is possible either to determine a pH from known input concentrations of free histidine and imidazolium histidine (conjugate base and conjugate acid respectively) or to determine the prevailing relative concentrations of free histidine and imidazolium histidine in a histidine-buffered solution exhibiting a known pH. Suitably, this calculation may be applied to biopharmaceutical compositions of the invention. Suitably, this calculation may be applied to biopharmaceutical compositions of the invention minus the bioactive agent (e.g. the composition without the protein).

Herein, in the context of the present specification, a "strong acid" is suitably one having a $pK_a$ of -1.0 or less, whereas a "weak acid" is suitably one having a $pK_a$ of 2.0 or more. Herein, in the context of the present specification, a "strong base" is suitably one whose conjugate acid has a $pK_a$ of 12 or higher (suitably 14 or higher), whereas a "weak base" is suitably one whose conjugate acid has a $pK_a$ of 10 or less.

Unless stated otherwise, references herein to a "pKa" should be construed as a pKa value in water at standard ambient temperature and pressure (SATP), suitably of the conjugate acid of the relevant species.

Herein, an "osmolality" of a formulation may be measured by a variety of methods (and with a variety of equipment, such as an osmometer) well known in the art. The osmolality of a formulation may be measured using methods (and equipment) as mentioned herein, such as in the Example section. Alternatively, the osmolality of a formulation may be calculated as well-known in the art, noting the osmolality is generally the same as osmolarity in aqueous formulations with a density of approximately 1 g/mL (which, in general, may be presumed for compositions of the invention). For instance, the osmolarity of a solution, in osmoles per liter (osmol/L) may be calculated as follows:

$$Osmolarity = \sum_i \varphi_i n_i C_i$$

where each index i represents the identity of a given solute, φ is the osmotic coefficient relevant to each said solute (and is preferably approximated to 1 for all solutes for the purposes of the invention), *n* is the number of particles (e.g. ions) into which each said solute molecule dissociates (for sorbitol *n* is 1, for NaCl *n* is 2, for $MgCl_2$ *n* is 3), and C is the molar concentration of each said solute. Since osmotic coefficients may be ignored (i.e. presumed to be 1 for each solute), the osmolarity (and thus osmolality, since formulations of the invention may be presumed to have a density of approximately 1 g/mL) calculated may be simplified thus:

$$Osmolarity = \sum_i n_i C_i$$

Suitably, this calculation may be applied to biopharmaceutical compositions of the invention. Suitably, this calculation may be applied to biopharmaceutical compositions of the invention minus the bioactive agent (e.g. the composition without the protein).

Herein, a "stabiliser" refers to a component which facilitates maintainance of the structural integrity of the biopharmaceutical drug, particularly during freezing and/or lyophilization and/or storage (especially when exposed to stress). This stabilising effect may arise for a variety of reasons, though typically such stabilisers may act as osmolytes which mitigate against protein denaturation. Typical stabilisers include amino acids (i.e. free amino acids not part of a peptide or protein - e.g. glycine, arginine, histidine, aspartic acid, lysine) and sugar stabilisers, such as a sugar polyol (e.g. mannitol, sorbitol), and/or a disaccharide (e.g. trehalose, sucrose, maltose, lactose).

Herein, a "surfactant" or "surfactant component" is suitably an ingredient or ingredients comprising one or more surfactants (suitably non-ionic surfactants), though suitably the surfactant is a single surfactant. The term "surfactant" is well known in the art.

An "amino acid component" is suitably an ingredient or ingredients comprising one or more (free) amino acids, though an amino acid component preferably consist of a single amino acid. Such references have no bearing on the presence of amino acid residue(s) within a protein structure. Compositions of the invention may include or exclude such an amino acid component, or alternatively may include or exclude a specific amino acid or a specific subset of amino acids.

Unless stated otherwise, references herein to an "amino acid" or "amino acids", whether specific (e.g. arginine, histidine) or general (e.g. any amino acid), in the context of their presence or otherwise within compositions (especially pharmaceutical liquid compositions of the invention) relate to the corresponding free amino acid(s) (regardless of its/their protonation state and/or salt form, though for consistency amounts are suitably calculated by reference to the free amino acid *per se*). This may suitably include natural and/or artificial amino acids. Unless stated to the contrary, such references are not intended to relate to amino acid residue(s) covalently incorporated as part of a larger compound (as opposed to a composition comprising multiple compounds), such as a peptide or protein (where such amino acid residues are linked *via* peptide bonds). As such, though an antibody, as a protein, contains amino acid residues, it is not considered to comprise any "free amino acid(s)". By way of example, a composition defined as being "*free of arginine*" does not contain any free arginine but it may still include one or more proteins (e.g. secukinumab) which do themselves comprise arginine residues.

Unless stated otherwise, references herein to any one or more "amino acids", whether specific or general, suitably relate to the L- stereoisomers or a racemate thereof, most suitably L-amino acids.

Herein, a "sugar component" is suitably an ingredient or ingredients comprising one or more sugar(s) and/or sugar alcohol(s), though a sugar component may consist of a single sugar (e.g. trehalose, sucrose) or sugar alcohol (e.g. sorbitol, mannitol), and excludes sugars appended (e.g. *via* glycosylation) to an antibody or active biopharmaceutical ingredient.

Herein, a "non-reducing sugar" is generally a sugar without any aldehyde moieties or without the capability of forming an aldehyde moiety (e.g. through isomerism).

Herein, a "tonicity modifier" or "tonicifier" refers to a reagent whose inclusion within a composition suitably contributes to (or increases) the overall osmolality and osmolarity of the composition. Suitably, a tonicifier, as used herein includes an agent which functions to render a solution similar in osmotic characteristics to physiologic fluids.

Herein, an "ionic strength provider" is a compound, typically a salt, that imparts (or increases) ionic strength in a solution, generally an aqueous solution. Unless otherwise stated, herein ionic strength is measured as a molar ionic strength of a solution (I), which is a function of the concentration of all ions present in said solution. Molar ionic strength may be expressed thus:

$$I = \frac{1}{2} \cdot \sum_{i=1}^{n} c_i z_i^2$$

where I is the molar ionic strength, $c_i$ is the molar concentration of ion i (preferably as a molarity, such as mM), $z_i$ is the charge number of that ion, and the sum is taken over all ions in solution. A salt such as sodium chloride, which has a single 1 + charged cation and single 1- charged cation, the ionic strength is equal to the concentration. For a salt such as $MgSO_4$, each ion is doubly-charged (i.e. with a 2+ cation and a 2- anion), which leads to an ionic strength that is four times higher than its actual molar concentration. Meanwhile, $Na_2SO_4$ and $MgCl_2$ have an ionic strength that is three times higher than the actual molar concentrations of the compounds themselves. The concept of ionic strength is well understood in the art of chemistry.

Herein, an "antioxidant" or "antioxidant component" is an ingredient or ingredients comprising one or more antioxidant compounds, though an antioxidant component may consist of a single antioxidant compound. An antioxidant in the context of the compositions of the invention preferably mitigate oxidation of groups within the biopharmaceutical active (i.e. secukinumab) that might otherwise be vulnerable to oxidation.

"Chelator" is a term of art referring to a compound capable of complexing, preferably in a multidentate manner, with various groups, molecules, atoms, or ions, and may exert an antioxidant effect in its own right.

Herein, unless otherwise stated, references to specific amounts of a given component of a composition, especially a buffer system (or buffering agent thereof), stabiliser, sugar, amino acid, surfactant, or tonicifier, suitably relate to the amounts of the pure anhydrous form of the relevant component (or compositions formed by using said amounts of the pure anhydrous form), even though such a component may be used in a non-anhydrous form when forming the composition. Amounts of any corresponding non-anhydrous forms (e.g. monohydrates, dihydrates, etc.) may be readily calculated by simply using the appropriate multiplier. For instance, unless stated otherwise (as per the Examples, where quantities relate to trehalose dihydrate), amounts stipulated in relation to trehalose refer to the anhydrous form of trehalose (or compositions formed by using the stipulated amounts/concentrations of anhydrous trehalose), which has a molecular weight of 342.296 g/mol, so to calculate the corresponding amount of trehalose dihydrate needed to form the same composition (less water would have to be added) it is necessary to multiply the stipulated amount by 378.33/342.296, since 378.33 is the molecular weight of trehalose dihydrate. The skilled person would readily understand how to judiciously adjust the quantity of diluent/water depending on the form of the components used, in order to derive the target concentrations.

Herein, the term "pharmaceutical composition" or "biopharmaceutical composition" refers to a formulation of a (bio)pharmaceutical active which renders the biological activity of the active ingredient therapeutically effective, but which does not include other ingredients which are obviously toxic to a subject to which the formulation are intended to be administered. Herein, references to a "composition" generally refer to a biopharmaceutical composition as defined herein.

Herein, the term "stable" generally refers to the physical stability and/or chemical stability and/or biological stability of a component, typically an active or composition thereof, during preservation/storage. The term "stable" may, however, refer to chemical stability, especially with respect to a biopharmaceutical active. For aqueous compositions of a biologic, storage stability may preferably mean that the biologic is sufficiently stable (i.e. with prescribed limits for patient safety) when stored at 2-8°C for at least 6 months, preferably at least 12 months, preferably up to 24 months. However, accelerated stability studies may be used to provide relevant stability information.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

In the context of the present invention, a "therapeutically effective amount" or "effective amount" of the antibody means an amount that is effective, when administered to a mammal for treating a disease or disorder, in prophylactic and therapeutic aspect and the antibody is effective in treatment of the diseases concerned.

The "therapeutically effective amount" will vary depending on the compound, the disease and its severity and the age, weight, etc., of the mammal to be treated.

The term "recombinant human antibody" is intended to include a human antibody prepared, expressed, produced or isolated using a recombinant method.

Herein, unless stated otherwise, where a composition is defined by reference to a plurality of broadly-defined ingredients, suitably said ingredients are additive in the sense of them being separate (preferably non-overlapping) ingredients/components - as such, a given specific ingredient can suitably only fall within the scope of one of the broadly-defined ingredients. As such, compositions comprising both a buffer and a tonicifier suitably include two separate components, a particular buffer (e.g. an acetate buffer system) and a particular tonicifier (e.g. an amino acid, sodium chloride, etc.) despite the fact that a buffer can in theory also serve as a tonicifier and, in certain circumstances (where a tonicifier exhibits a buffering effect), *vice versa.* Which specific ingredient constitutes which broadly-defined ingredient will be readily appreciated by a skilled person, especially in circumstances where amounts, concentrations, or ratios are pertinent.

Herein, the term "component" is often used interchangeably with the term "ingredient", especially in the context of compositions. A component/ingredient may be a specific component/ingredient, such as sorbitol, or a general component/ingredient (e.g. ingredient class) such as a sugar component, where a specific example of the sugar component is sorbitol.

An ingredient (or component) may in itself be composed of (or comprise) a plurality of ingredients (e.g. sub-ingredients). For example, a buffer system suitably includes relevant conjugate acid(s) and conjugate base(s). As such, any general principles defined herein in respect of a composition (e.g. amounts; the notion of a composition consisting essentially of one or more components - this may equally apply to a component with sub-components/sub-ingredients) may also be applicable to a component capable itself of comprising a plurality of sub-components.

A person skilled in the art will be readily familiar with language such as "[*an item or items*]...selected from the group consisting of [*plurality of items*]" (and similar such language) and related clauses such as "...and/or any combination thereof" (or similar such language), especially in the context of patent documents - for example "a composition comprising at least one ingredient selected from the group consisting of [*list of ingredients*], and/or any combination thereof". A skilled person will suitably accord such language its standard appropriate meaning. Herein, where a composition or product is said to include a plurality (or defined number or number range) of "items selected from the group consisting of [*distinct list of plurality of items*]..." (or similar such language), each of the plurality (or defined number or number range) of selections is most suitably a distinct selection from the items listed, especially where the listed items are generalised items, such as generalised ingredients (e.g. classes/types/categories of ingredients), especially where said items (e.g. ingredients) are listed in singular form - as such "selected" may be substituted for "distinctly selected". For example, a composition consisting of three different specific sugars (e.g. mannitol, sorbitol, and trehalose) suitably would not constitute "a composition comprising a plurality of ingredients selected form the group consisting of a sugar, a surfactant, and a salt", whereas a composition consisting of a specific sugar (e.g. sorbitol), a specific surfactant (e.g. polysorbate 80), and a specific salt (e.g. sodium chloride) suitably would constitute "a composition comprising a plurality of ingredients selected form the group consisting of a sugar, a surfactant, and a salt". Alternatively, however, especially where listed items are pluralised (especially where said items are generalised items, such as generalised ingredients), where a composition or product is said to include a plurality (or defined number or number range) of "items selected from the group consisting of [*list of items, especially where listed items are listed in pluralised language*]..." (or similar such language), several of the plurality (or defined number or number range) of selections may be indistinct selections from the items listed (e.g. several may be selected from a single listed item) - as such "selected" may be substituted for "distinctly or indistinctly selected". For example, a composition consisting of three different specific sugars (e.g. mannitol, sorbitol, and trehalose) may, in such circumstances, constitute "a composition comprising a plurality of ingredients selected from the group consisting of sugars, surfactants, and salts". Where the term "any combination thereof" (or similar such language) is used herein in connection with a group/list of items from which selections are made, this suitably permits combinations of items from the group/list, though suitably such combinations are in any case permitted, unless context dictates to the contrary, even without qualifications such as "any combination thereof". Such combinations may be, and preferably are (especially where generalised items are listed in singular form), combinations of distinct selections. Alternatively, such combinations may be, especially where generalised items are listed in pluralised form, combinations of indistinct selections.

Herein, amounts stipulated for components and ingredients, whether specified in terms of "parts", ppm (parts per million), percentages (%, e.g. wt%), or ratios, are intended to be by weight, unless stated otherwise.

Where the quantity or concentration of a particular component of a given composition is specified as a weight percentage (wt% or %w/w), said weight percentage refers to the percentage of said component by weight relative to the total weight of the composition as a whole. It will be understood by those skilled in the art that the sum of weight percentages of all components of a composition (whether or not specified) will total 100 wt%. However, where not all components are listed (e.g. where compositions are said to "comprise" one or more particular components), the weight percentage balance may optionally be made up to 100 wt% by unspecified ingredients (e.g. a diluent, such as water, or other non-essentially but suitable additives).

Where a composition is said to comprise a plurality of stipulated ingredients (optionally in stipulated amounts, concentrations, relative molar ratios, relative weight ratios, etc.), said composition may optionally include additional ingredients other than those stipulated. However, in certain embodiments (especially embodiments and aspects defined herein that contain a plurality of ingredients/components, especially where amounts are stipulated, especially where a diluent, e.g. water, is also present), a composition said to comprise a plurality of stipulated ingredients may in fact consist essentially of or consist of all the stipulated ingredients.

Herein, molar ratios (or parts by moles) between respective components may be expressed in a number of ways, as well known in the art. For example, molar ratios between Component X, Component Y, and Component Z may be expressed in terms of absolute values (e.g. $x_1 : y_1 : z_1$) or ranges (e.g. $x_1$-$x_2 : y_1$-$y_2 : z_1$-$z_2$) by:

- Component X, Component Y, and Component Z in a molar ratio of $x_1 : y_1 : z_1$.
- Component X, Component Y, and Component Z in a molar ratio of $x_1$-$x_2 : y_1$-$y_2 : z_1$-$z_2$.
- Component X, Component Y, and Component Z in a molar ratio of 1 : $y_1$-$y_2 : z_1$-$z_2$.
- $x_1$ Component X : $y_1$ Component Y : z, Component Z.
- $x_1$-$x_2$ Component X : $y_1$-$y_2$ Component Y : $z_1$-$z_2$ Component Z.
- 1 Component X : $y_1$-$y_2$ Component Y : $z_1$-$z_2$ Component Z.

Herein, where a composition is said to "consists essentially of" particular components/ingredients, said composition suitably comprises at least 70 wt% of said components/ingredients, suitably at least 90 wt% thereof, suitably at least 95 wt% thereof, most suitably at least 99 wt% thereof. Suitably, a composition said to "consist essentially of' particular components/ingredients consists of said components/ingredients save for one or more trace (suitably *de minimis*) impurities.

Herein, wherever a composition is said to be "free of [a *particular component*]" or "characterised by an absence of [a *particular component*]", this suitably means that the composition in question is either substantially free or entirely free of said component.

The term "substantially free" when used in relation to a given component of a composition (e.g. "a liquid biopharmaceutical composition substantially free of methionine"), refers to a composition to which essentially none of said component has been added. As explained above, such references have no bearing on the presence of amino acid residue(s) within a protein structure. When a composition is "substantially free" of a given component, said composition suitably comprises no more than 0.1 wt% of said component, suitably no more than 0.01 wt% of said component, suitably no more than 0.001 wt% of said component, suitably no more than 0.0001 wt% of said component, suitably no more than 0.00001 wt%, suitably no more than 0.000001 wt%, suitably no more than 0.0000001 wt% thereof, most suitably no more than 0.0001 parts per billion (by weight).

The term "entirely free", when used in relation to a given component of a composition (e.g. "a liquid biopharmaceutical composition entirely free of methionine"), refers to a composition containing none of said component. As explained above, such references have no bearing on the presence of amino acid residue(s) within a protein structure.

Suitably, unless stated otherwise, where reference is made to a parameter (e.g. pH, pKa, etc.) or state of a material (e.g. liquid, gas, etc.) which may depend on pressure and/or temperature, suitably in the absence of further clarification such a reference refers to said parameter at standard ambient temperature and pressure (SATP). SATP is a temperature of 298.15 K (25 °C, 77 °F) and an absolute pressure of 100 kPa (14.504 psi, 0.987 atm).

Herein, unless incompatible in a given context, wherever a component is stipulated which is capable of ionization (e.g. protonation or deprotonation), the definition of said component suitably includes any suitable salts thereof, suitably pharmaceutically acceptable salts thereof. For example, references herein to the succinic acid suitably includes succinate salts, unless the context dictates otherwise. Likewise, unless incompatible in a given context, wherever a component is stipulated which is capable of neutralisation, the definition of said component suitably includes neutralised forms thereof. For instance, references herein to succinates may suitably include succinic acid.

## GENERAL POINTS AND ADVANTAGES REGARDING THE INVENTION

Many of the advantages of the present invention, and indeed the challenges involved in its conception and development, will be self-evident. The inventive endeavours elucidated in this disclosure represent a significant contribution to the art, a contribution to which the present invention is commensurate in scope.

The present invention is provides alternative secukinumab formulations to those of the prior art. In general, the present invention provides viable alternative secukinumab formulations to those of the prior art. Suitably, such alternative secukinumab formulations provide comparable or improved stability (especially antibody stability) compared to those of the prior art, typically whilst using a different combination of features - in some cases fewer features. Many of the formulations of the invention alleviate or eliminate one or more problems associated with prior art formulations, especially those of the originator Cosentyx® formulations and other such formulations described in WO2012/059598 and WO2016/103153. Secukinumab formulations of the invention can also generally withstand various stresses (agitation, heat, light) to which drug products may be exposed during manufacture, transport, and storage.

The present invention can suitably address problems of the prior art whilst reducing formulation complexity (be this in terms of product- or process-related features). In some cases, secukinumab formulations of the invention solve more than one problem inherent in the prior art, sometimes addressing two or more of said problems.

## BIOPHARMACEUTICAL COMPOSITION

The present invention provides a biopharmaceutical composition (generally comprising secukinumab), suitably as defined herein, most suitably a liquid biopharmaceutical composition. The biopharmaceutical composition is most suitably a liquid biopharmaceutical composition, and thus suitably comprises a diluent. The biopharmaceutical composition is most suitably an aqueous biopharmaceutical composition, and thus suitably comprises a water as a diluent.

The present invention may, however, provide a solid biopharmaceutical composition, for instance, a lyophilised biopharmaceutical composition. Such a lyophilised biopharmaceutical composition is suitably capable of reconstitution into a liquid biopharmaceutical composition, suitably as defined herein. Generally, amounts and concentrations herein relate to liquid biopharmaceutical compositions. However, such amounts may be readily converted into amounts within a corresponding solid (lyophilised) composition (which in any case will be typically intended to be reconstituted before administration so as to yield the concentrations of said liquid biopharmaceutical composition), and may be expressed as (weight or molar) ratios between the respective components or as absolute concentrations (by taking account of the removal of the diluent).

Embodiments of the invention which define an open-ended "biopharmaceutical composition comprising...[a *plurality of ingredients*]" (or similar such wording) may suitably be defined as close-ended (e.g. as a "biopharmaceutical composition consisting of...[the *plurality of ingredients*]", or similar such wording), especially where the biopharmaceutical composition (i.e. liquid biopharmaceutical composition) is defined as containing a diluent (which is preferably water). Alternatively, such embodiments may be defined as substantially or essentially close-ended (e.g. as a "biopharmaceutical composition consisting essentially of...[the *plurality of ingredients*]", or similar such wording), especially where the biopharmaceutical composition is defined as containing a diluent (which is preferably water).

The composition comprises a biopharmaceutical active. Herein, aspects and embodiments of the invention which refer to "a biopharmaceutical active" or something similar, suitably mean a biopharmaceutical active which is an antibody, preferably an anti-IL-17A antibody suitably of the IgG1 subclass, more preferably an anti-IL-17A (interleukin-1 7A antagonist) human IgG1/κ monoclonal antibody, most preferably secukinumab. In general, different antibodies (especially those of a different IgG subclass and/or with a different antigen target) will tend to behave differently under otherwise like conditions.

The biopharmaceutical composition preferably comprises secukinumab and at least one ingredient selected (preferably distinctly selected) from the group consisting of a buffer system, a sugar component, an amino acid component, a surfactant, a tonicifier, an ionic-strength provider, an antioxidant, a chelator, and/or a diluent. The biopharmaceutical composition may also be further characterised by one or more of a pH, osmolality, and/or ionic strength.

The biopharmaceutical composition suitably comprises secukinumab and between one and eight ingredients selected (preferably distinctly selected) from the group consisting of a buffer system, a sugar component, an amino acid component, a surfactant, a tonicifier, an ionic-strength provider, an antioxidant, a chelator, and a diluent.

The biopharmaceutical composition suitably comprises secukinumab, a diluent, and between one and seven ingredients selected (preferably distinctly selected) from the group consisting of a buffer system, a sugar component, an amino acid component, a surfactant, a tonicifier, an ionic-strength provider, an antioxidant, and a chelator. As such, the biopharmaceutical composition is preferably a liquid biopharmaceutical composition, most preferably an aqueous biopharmaceutical composition.

The biopharmaceutical composition preferably comprises secukinumab and one or more, preferably two or more, more preferably three or more, ingredients selected (preferably distinctly selected) from the group consisting of a buffer system, a sugar component, an amino acid component, a surfactant, a tonicifier, an ionic-strength provider, an antioxidant, a chelator, and a diluent. Such a biopharmaceutical preferably comprises at most seven, more preferably at most six, more preferably at most five ingredients selected (preferably distinctly selected) from the group consisting of a buffer system, a sugar component, an amino acid component, a surfactant, a tonicifier, an ionic-strength provider, an antioxidant, a chelator, and a diluent. Where the aforesaid biopharmaceutical composition is a liquid biopharmaceutical composition (which is preferable), the biopharmaceutical composition suitably further comprises a diluent, which preferably comprises and most preferably is water.

The biopharmaceutical composition suitably comprises a buffer system.

The biopharmaceutical composition suitably comprises a sugar component.

The biopharmaceutical composition suitably comprises an amino acid component.

The biopharmaceutical composition suitably comprises a surfactant.

The biopharmaceutical composition suitably comprises a tonicifier. The tonicifier may suitably be the same ingredient as the ionic-strength provider.

The biopharmaceutical composition suitably comprises an ionic-strength provider. The ionic-strength provider may suitably be the same ingredient as the tonicifier.

The biopharmaceutical composition suitably comprises an antioxidant.

The biopharmaceutical composition suitably comprises a chelator.

The biopharmaceutical composition suitably comprises a diluent, preferably water.

## *Embodiments of Combinations of Ingredients*

### A) General Aspects and Embodiments Relating to Combinations of Ingredients

The biopharmaceutical composition suitably comprises, or consists of (suitably along with a diluent, which is preferably water), a biopharmaceutical active (which is preferably secukinumab) and one or more of any of a buffer system, a sugar component, an amino acid component, a surfactant, a tonicifier, an ionic-strength provider, an antioxidant, a chelator, and/or a diluent. The following numbered paragraphs A1-A511 disclose specific aspects and embodiments of the invention, wherein the biopharmaceutical composition comprises, consists of (suitably along with a diluent, which is preferably water), and/or is otherwise characterised by:

A1. secukinumab; and a buffer system.

A2. secukinumab; and a sugar component.

A3. secukinumab; and an amino acid component.

A4. secukinumab; and a surfactant.

A5. secukinumab; and a tonicifier.

A6. secukinumab; and an ionic strength provider.

A7. secukinumab; and an antioxidant.

A8. secukinumab; and a chelator.

A9. secukinumab; and a diluent.

A10. secukinumab; a buffer system; and a sugar component.

A11. secukinumab; a buffer system; and an amino acid component.

A12. secukinumab; a buffer system; and a surfactant.

A13. secukinumab; a buffer system; and a tonicifier.

A14. secukinumab; a buffer system; and an ionic strength provider.

A15. secukinumab; a buffer system; and an antioxidant.

A16. secukinumab; a buffer system; and a chelator.

A17. secukinumab; a buffer system; and a diluent.

A18. secukinumab; a sugar component; and an amino acid component.

A19. secukinumab; a sugar component; and a surfactant.

A20. secukinumab; a sugar component; and a tonicifier.

A21. secukinumab; a sugar component; and an ionic strength provider.

A22. secukinumab; a sugar component; and an antioxidant.

A23. secukinumab; a sugar component; and a chelator.

A24. secukinumab; a sugar component; and a diluent.

A25. secukinumab; an amino acid component; and a surfactant.

(continued)

A26. secukinumab; an amino acid component; and a tonicifier.
A27. secukinumab; an amino acid component; and an ionic strength provider.
A28. secukinumab; an amino acid component; and an antioxidant.
A29. secukinumab; an amino acid component; and a chelator.
A30. secukinumab; an amino acid component; and a diluent.
A31. secukinumab; a surfactant; and a tonicifier.
A32. secukinumab; a surfactant; and an ionic strength provider.
A33. secukinumab; a surfactant; and an antioxidant.
A34. secukinumab; a surfactant; and a chelator.
A35. secukinumab; a surfactant; and a diluent.
A36. secukinumab; a tonicifier; and an ionic strength provider.
A37. secukinumab; a tonicifier; and an antioxidant.
A38. secukinumab; a tonicifier; and a chelator.
A39. secukinumab; a tonicifier; and a diluent.
A40. secukinumab; an ionic strength provider; and an antioxidant.
A41. secukinumab; an ionic strength provider; and a chelator.
A42. secukinumab; an ionic strength provider; and a diluent.
A43. secukinumab; an antioxidant; and a chelator.
A44. secukinumab; an antioxidant; and a diluent.
A45. secukinumab; a chelator; and a diluent.
A46. secukinumab; a buffer system; a sugar component; and an amino acid component.
A47. secukinumab; a buffer system; a sugar component; and a surfactant.
A48. secukinumab; a buffer system; a sugar component; and a tonicifier.
A49. secukinumab; a buffer system; a sugar component; and an ionic strength provider.
A50. secukinumab; a buffer system; a sugar component; and an antioxidant.
A51. secukinumab; a buffer system; a sugar component; and a chelator.
A52. secukinumab; a buffer system; a sugar component; and a diluent.
A53. secukinumab; a buffer system; an amino acid component; and a surfactant.
A54. secukinumab; a buffer system; an amino acid component; and a tonicifier.
A55. secukinumab; a buffer system; an amino acid component; and an ionic strength provider.
A56. secukinumab; a buffer system; an amino acid component; and an antioxidant.
A57. secukinumab; a buffer system; an amino acid component; and a chelator.
A58. secukinumab; a buffer system; an amino acid component; and a diluent.
A59. secukinumab; a buffer system; a surfactant; and a tonicifier.
A60. secukinumab; a buffer system; a surfactant; and an ionic strength provider.
A61. secukinumab; a buffer system; a surfactant; and an antioxidant.
A62. secukinumab; a buffer system; a surfactant; and a chelator.
A63. secukinumab; a buffer system; a surfactant; and a diluent.
A64. secukinumab; a buffer system; a tonicifier; and an ionic strength provider.
A65. secukinumab; a buffer system; a tonicifier; and an antioxidant.
A66. secukinumab; a buffer system; a tonicifier; and a chelator.
A67. secukinumab; a buffer system; a tonicifier; and a diluent.
A68. secukinumab; a buffer system; an ionic strength provider; and an antioxidant.
A69. secukinumab; a buffer system; an ionic strength provider; and a chelator.
A70. secukinumab; a buffer system; an ionic strength provider; and a diluent.
A71. secukinumab; a buffer system; an antioxidant; and a chelator.
A72. secukinumab; a buffer system; an antioxidant; and a diluent.
A73. secukinumab; a buffer system; a chelator; and a diluent.
A74. secukinumab; a sugar component; an amino acid component; and a surfactant.
A75. secukinumab; a sugar component; an amino acid component; and a tonicifier.
A76. secukinumab; a sugar component; an amino acid component; and an ionic strength provider.

(continued)

A77. secukinumab; a sugar component; an amino acid component; and an antioxidant.
A78. secukinumab; a sugar component; an amino acid component; and a chelator.
A79. secukinumab; a sugar component; an amino acid component; and a diluent.
A80. secukinumab; a sugar component; a surfactant; and a tonicifier.
A81. secukinumab; a sugar component; a surfactant; and an ionic strength provider.
A82. secukinumab; a sugar component; a surfactant; and an antioxidant.
A83. secukinumab; a sugar component; a surfactant; and a chelator.
A84. secukinumab; a sugar component; a surfactant; and a diluent.
A85. secukinumab; a sugar component; a tonicifier; and an ionic strength provider.
A86. secukinumab; a sugar component; a tonicifier; and an antioxidant.
A87. secukinumab; a sugar component; a tonicifier; and a chelator.
A88. secukinumab; a sugar component; a tonicifier; and a diluent.
A89. secukinumab; a sugar component; an ionic strength provider; and an antioxidant.
A90. secukinumab; a sugar component; an ionic strength provider; and a chelator.
A91. secukinumab; a sugar component; an ionic strength provider; and a diluent.
A92. secukinumab; a sugar component; an antioxidant; and a chelator.
A93. secukinumab; a sugar component; an antioxidant; and a diluent.
A94. secukinumab; a sugar component; a chelator; and a diluent.
A95. secukinumab; an amino acid component; a surfactant; and a tonicifier.
A96. secukinumab; an amino acid component; a surfactant; and an ionic strength provider.
A97. secukinumab; an amino acid component; a surfactant; and an antioxidant.
A98. secukinumab; an amino acid component; a surfactant; and a chelator.
A99. secukinumab; an amino acid component; a surfactant; and a diluent.
A100. secukinumab; an amino acid component; a tonicifier; and an ionic strength provider.
A101. secukinumab; an amino acid component; a tonicifier; and an antioxidant.
A102. secukinumab; an amino acid component; a tonicifier; and a chelator.
A103. secukinumab; an amino acid component; a tonicifier; and a diluent.
A104. secukinumab; an amino acid component; an ionic strength provider; and an antioxidant.
A105. secukinumab; an amino acid component; an ionic strength provider; and a chelator.
A106. secukinumab; an amino acid component; an ionic strength provider; and a diluent.
A107. secukinumab; an amino acid component; an antioxidant; and a chelator.
A108. secukinumab; an amino acid component; an antioxidant; and a diluent.
A109. secukinumab; an amino acid component; a chelator; and a diluent.
A110. secukinumab; a surfactant; a tonicifier; and an ionic strength provider.
A111. secukinumab; a surfactant; a tonicifier; and an antioxidant.
A112. secukinumab; a surfactant; a tonicifier; and a chelator.
A113. secukinumab; a surfactant; a tonicifier; and a diluent.
A114. secukinumab; a surfactant; an ionic strength provider; and an antioxidant.
A115. secukinumab; a surfactant; an ionic strength provider; and a chelator.
A116. secukinumab; a surfactant; an ionic strength provider; and a diluent.
A1 17. secukinumab; a surfactant; an antioxidant; and a chelator.
A118. secukinumab; a surfactant; an antioxidant; and a diluent.
A119. secukinumab; a surfactant; a chelator; and a diluent.
A120. secukinumab; a tonicifier; an ionic strength provider; and an antioxidant.
A121. secukinumab; a tonicifier; an ionic strength provider; and a chelator.
A122. secukinumab; a tonicifier; an ionic strength provider; and a diluent.
A123. secukinumab; a tonicifier; an antioxidant; and a chelator.
A124. secukinumab; a tonicifier; an antioxidant; and a diluent.
A125. secukinumab; a tonicifier; a chelator; and a diluent.
A126. secukinumab; an ionic strength provider; an antioxidant; and a chelator.
A127. secukinumab; an ionic strength provider; an antioxidant; and a diluent.

(continued)

A128. secukinumab; an ionic strength provider; a chelator; and a diluent.
A129. secukinumab; an antioxidant; a chelator; and a diluent.
A130. secukinumab; a buffer system; a sugar component; an amino acid component; and a surfactant.
A131. secukinumab; a buffer system; a sugar component; an amino acid component; and a tonicifier.
A132. secukinumab; a buffer system; a sugar component; an amino acid component; and an ionic strength provider.
A133. secukinumab; a buffer system; a sugar component; an amino acid component; and an antioxidant.
A134. secukinumab; a buffer system; a sugar component; an amino acid component; and a chelator.
A135. secukinumab; a buffer system; a sugar component; an amino acid component; and a diluent.
A136. secukinumab; a buffer system; a sugar component; a surfactant; and a tonicifier.
A137. secukinumab; a buffer system; a sugar component; a surfactant; and an ionic strength provider.
A138. secukinumab; a buffer system; a sugar component; a surfactant; and an antioxidant.
A139. secukinumab; a buffer system; a sugar component; a surfactant; and a chelator.
A140. secukinumab; a buffer system; a sugar component; a surfactant; and a diluent.
A141. secukinumab; a buffer system; a sugar component; a tonicifier; and an ionic strength provider.
A142. secukinumab; a buffer system; a sugar component; a tonicifier; and an antioxidant.
A143. secukinumab; a buffer system; a sugar component; a tonicifier; and a chelator.
A144. secukinumab; a buffer system; a sugar component; a tonicifier; and a diluent.
A145. secukinumab; a buffer system; a sugar component; an ionic strength provider; and an antioxidant.
A146. secukinumab; a buffer system; a sugar component; an ionic strength provider; and a chelator.
A147. secukinumab; a buffer system; a sugar component; an ionic strength provider; and a diluent.
A148. secukinumab; a buffer system; a sugar component; an antioxidant; and a chelator.
A149. secukinumab; a buffer system; a sugar component; an antioxidant; and a diluent.
A150. secukinumab; a buffer system; a sugar component; a chelator; and a diluent.
A151. secukinumab; a buffer system; an amino acid component; a surfactant; and a tonicifier.
A152. secukinumab; a buffer system; an amino acid component; a surfactant; and an ionic strength provider.
A153. secukinumab; a buffer system; an amino acid component; a surfactant; and an antioxidant.
A154. secukinumab; a buffer system; an amino acid component; a surfactant; and a chelator.
A155. secukinumab; a buffer system; an amino acid component; a surfactant; and a diluent.
A156. secukinumab; a buffer system; an amino acid component; a tonicifier; and an ionic strength provider.
A157. secukinumab; a buffer system; an amino acid component; a tonicifier; and an antioxidant.
A158. secukinumab; a buffer system; an amino acid component; a tonicifier; and a chelator.
A159. secukinumab; a buffer system; an amino acid component; a tonicifier; and a diluent.
A160. secukinumab; a buffer system; an amino acid component; an ionic strength provider; and an antioxidant.
A161. secukinumab; a buffer system; an amino acid component; an ionic strength provider; and a chelator.
A162. secukinumab; a buffer system; an amino acid component; an ionic strength provider; and a diluent.
A163. secukinumab; a buffer system; an amino acid component; an antioxidant; and a chelator.
A164. secukinumab; a buffer system; an amino acid component; an antioxidant; and a diluent.
A165. secukinumab; a buffer system; an amino acid component; a chelator; and a diluent.
A166. secukinumab; a buffer system; a surfactant; a tonicifier; and an ionic strength provider.
A167. secukinumab; a buffer system; a surfactant; a tonicifier; and an antioxidant.
A168. secukinumab; a buffer system; a surfactant; a tonicifier; and a chelator.
A169. secukinumab; a buffer system; a surfactant; a tonicifier; and a diluent.
A170. secukinumab; a buffer system; a surfactant; an ionic strength provider; and an antioxidant.
A171. secukinumab; a buffer system; a surfactant; an ionic strength provider; and a chelator.
A172. secukinumab; a buffer system; a surfactant; an ionic strength provider; and a diluent.
A173. secukinumab; a buffer system; a surfactant; an antioxidant; and a chelator.
A174. secukinumab; a buffer system; a surfactant; an antioxidant; and a diluent.
A175. secukinumab; a buffer system; a surfactant; a chelator; and a diluent.
A176. secukinumab; a buffer system; a tonicifier; an ionic strength provider; and an antioxidant.
A177. secukinumab; a buffer system; a tonicifier; an ionic strength provider; and a chelator.
A178. secukinumab; a buffer system; a tonicifier; an ionic strength provider; and a diluent.

(continued)

A179. secukinumab; a buffer system; a tonicifier; an antioxidant; and a chelator.
A180. secukinumab; a buffer system; a tonicifier; an antioxidant; and a diluent.
A181. secukinumab; a buffer system; a tonicifier; a chelator; and a diluent.
A182. secukinumab; a buffer system; an ionic strength provider; an antioxidant; and a chelator.
A183. secukinumab; a buffer system; an ionic strength provider; an antioxidant; and a diluent.
A184. secukinumab; a buffer system; an ionic strength provider; a chelator; and a diluent.
A185. secukinumab; a buffer system; an antioxidant; a chelator; and a diluent.
A186. secukinumab; a sugar component; an amino acid component; a surfactant; and a tonicifier.
A187. secukinumab; a sugar component; an amino acid component; a surfactant; and an ionic strength provider.
A188. secukinumab; a sugar component; an amino acid component; a surfactant; and an antioxidant.
A189. secukinumab; a sugar component; an amino acid component; a surfactant; and a chelator.
A190. secukinumab; a sugar component; an amino acid component; a surfactant; and a diluent.
A191. secukinumab; a sugar component; an amino acid component; a tonicifier; and an ionic strength provider.
A192. secukinumab; a sugar component; an amino acid component; a tonicifier; and an antioxidant.
A193. secukinumab; a sugar component; an amino acid component; a tonicifier; and a chelator.
A194. secukinumab; a sugar component; an amino acid component; a tonicifier; and a diluent.
A195. secukinumab; a sugar component; an amino acid component; an ionic strength provider; and an antioxidant.
A196. secukinumab; a sugar component; an amino acid component; an ionic strength provider; and a chelator.
A197. secukinumab; a sugar component; an amino acid component; an ionic strength provider; and a diluent.
A198. secukinumab; a sugar component; an amino acid component; an antioxidant; and a chelator.
A199. secukinumab; a sugar component; an amino acid component; an antioxidant; and a diluent.
A200. secukinumab; a sugar component; an amino acid component; a chelator; and a diluent.
A201. secukinumab; a sugar component; a surfactant; a tonicifier; and an ionic strength provider.
A202. secukinumab; a sugar component; a surfactant; a tonicifier; and an antioxidant.
A203. secukinumab; a sugar component; a surfactant; a tonicifier; and a chelator.
A204. secukinumab; a sugar component; a surfactant; a tonicifier; and a diluent.
A205. secukinumab; a sugar component; a surfactant; an ionic strength provider; and an antioxidant.
A206. secukinumab; a sugar component; a surfactant; an ionic strength provider; and a chelator.
A207. secukinumab; a sugar component; a surfactant; an ionic strength provider; and a diluent.
A208. secukinumab; a sugar component; a surfactant; an antioxidant; and a chelator.
A209. secukinumab; a sugar component; a surfactant; an antioxidant; and a diluent.
A210. secukinumab; a sugar component; a surfactant; a chelator; and a diluent.
A211. secukinumab; a sugar component; a tonicifier; an ionic strength provider; and an antioxidant.
A212. secukinumab; a sugar component; a tonicifier; an ionic strength provider; and a chelator.
A213. secukinumab; a sugar component; a tonicifier; an ionic strength provider; and a diluent.
A214. secukinumab; a sugar component; a tonicifier; an antioxidant; and a chelator.
A215. secukinumab; a sugar component; a tonicifier; an antioxidant; and a diluent.
A216. secukinumab; a sugar component; a tonicifier; a chelator; and a diluent.
A217. secukinumab; a sugar component; an ionic strength provider; an antioxidant; and a chelator.
A218. secukinumab; a sugar component; an ionic strength provider; an antioxidant; and a diluent.
A219. secukinumab; a sugar component; an ionic strength provider; a chelator; and a diluent.
A220. secukinumab; a sugar component; an antioxidant; a chelator; and a diluent.
A221. secukinumab; an amino acid component; a surfactant; a tonicifier; and an ionic strength provider.
A222. secukinumab; an amino acid component; a surfactant; a tonicifier; and an antioxidant.
A223. secukinumab; an amino acid component; a surfactant; a tonicifier; and a chelator.
A224. secukinumab; an amino acid component; a surfactant; a tonicifier; and a diluent.
A225. secukinumab; an amino acid component; a surfactant; an ionic strength provider; and an antioxidant.
A226. secukinumab; an amino acid component; a surfactant; an ionic strength provider; and a chelator.
A227. secukinumab; an amino acid component; a surfactant; an ionic strength provider; and a diluent.
A228. secukinumab; an amino acid component; a surfactant; an antioxidant; and a chelator.
A229. secukinumab; an amino acid component; a surfactant; an antioxidant; and a diluent.

(continued)

A230. secukinumab; an amino acid component; a surfactant; a chelator; and a diluent.
A231. secukinumab; an amino acid component; a tonicifier; an ionic strength provider; and an antioxidant.
A232. secukinumab; an amino acid component; a tonicifier; an ionic strength provider; and a chelator.
A233. secukinumab; an amino acid component; a tonicifier; an ionic strength provider; and a diluent.
A234. secukinumab; an amino acid component; a tonicifier; an antioxidant; and a chelator.
A235. secukinumab; an amino acid component; a tonicifier; an antioxidant; and a diluent.
A236. secukinumab; an amino acid component; a tonicifier; a chelator; and a diluent.
A237. secukinumab; an amino acid component; an ionic strength provider; an antioxidant; and a chelator.
A238. secukinumab; an amino acid component; an ionic strength provider; an antioxidant; and a diluent.
A239. secukinumab; an amino acid component; an ionic strength provider; a chelator; and a diluent.
A240. secukinumab; an amino acid component; an antioxidant; a chelator; and a diluent.
A241. secukinumab; a surfactant; a tonicifier; an ionic strength provider; and an antioxidant.
A242. secukinumab; a surfactant; a tonicifier; an ionic strength provider; and a chelator.
A243. secukinumab; a surfactant; a tonicifier; an ionic strength provider; and a diluent.
A244. secukinumab; a surfactant; a tonicifier; an antioxidant; and a chelator.
A245. secukinumab; a surfactant; a tonicifier; an antioxidant; and a diluent.
A246. secukinumab; a surfactant; a tonicifier; a chelator; and a diluent.
A247. secukinumab; a surfactant; an ionic strength provider; an antioxidant; and a chelator.
A248. secukinumab; a surfactant; an ionic strength provider; an antioxidant; and a diluent.
A249. secukinumab; a surfactant; an ionic strength provider; a chelator; and a diluent.
A250. secukinumab; a surfactant; an antioxidant; a chelator; and a diluent.
A251. secukinumab; a tonicifier; an ionic strength provider; an antioxidant; and a chelator.
A252. secukinumab; a tonicifier; an ionic strength provider; an antioxidant; and a diluent.
A253. secukinumab; a tonicifier; an ionic strength provider; a chelator; and a diluent.
A254. secukinumab; a tonicifier; an antioxidant; a chelator; and a diluent.
A255. secukinumab; an ionic strength provider; an antioxidant; a chelator; and a diluent.
A256. secukinumab; a buffer system; a sugar component; an amino acid component; a surfactant; and a tonicifier.
A257. secukinumab; a buffer system; a sugar component; an amino acid component; a surfactant; and an ionic strength provider.
A258. secukinumab; a buffer system; a sugar component; an amino acid component; a surfactant; and an antioxidant.
A259. secukinumab; a buffer system; a sugar component; an amino acid component; a surfactant; and a chelator.
A260. secukinumab; a buffer system; a sugar component; an amino acid component; a surfactant; and a diluent.
A261. secukinumab; a buffer system; a sugar component; an amino acid component; a tonicifier; and an ionic strength provider.
A262. secukinumab; a buffer system; a sugar component; an amino acid component; a tonicifier; and an antioxidant.
A263. secukinumab; a buffer system; a sugar component; an amino acid component; a tonicifier; and a chelator.
A264. secukinumab; a buffer system; a sugar component; an amino acid component; a tonicifier; and a diluent.
A265. secukinumab; a buffer system; a sugar component; an amino acid component; an ionic strength provider; and an antioxidant.
A266. secukinumab; a buffer system; a sugar component; an amino acid component; an ionic strength provider; and a chelator.
A267. secukinumab; a buffer system; a sugar component; an amino acid component; an ionic strength provider; and a diluent.
A268. secukinumab; a buffer system; a sugar component; an amino acid component; an antioxidant; and a chelator.
A269. secukinumab; a buffer system; a sugar component; an amino acid component; an antioxidant; and a diluent.
A270. secukinumab; a buffer system; a sugar component; an amino acid component; a chelator; and a diluent.
A271. secukinumab; a buffer system; a sugar component; a surfactant; a tonicifier; and an ionic strength provider.
A272. secukinumab; a buffer system; a sugar component; a surfactant; a tonicifier; and an antioxidant.
A273. secukinumab; a buffer system; a sugar component; a surfactant; a tonicifier; and a chelator.

(continued)

A274. secukinumab; a buffer system; a sugar component; a surfactant; a tonicifier; and a diluent.
A275. secukinumab; a buffer system; a sugar component; a surfactant; an ionic strength provider; and an antioxidant.
A276. secukinumab; a buffer system; a sugar component; a surfactant; an ionic strength provider; and a chelator.
A277. secukinumab; a buffer system; a sugar component; a surfactant; an ionic strength provider; and a diluent.
A278. secukinumab; a buffer system; a sugar component; a surfactant; an antioxidant; and a chelator.
A279. secukinumab; a buffer system; a sugar component; a surfactant; an antioxidant; and a diluent.
A280. secukinumab; a buffer system; a sugar component; a surfactant; a chelator; and a diluent.
A281. secukinumab; a buffer system; a sugar component; a tonicifier; an ionic strength provider; and an antioxidant.
A282. secukinumab; a buffer system; a sugar component; a tonicifier; an ionic strength provider; and a chelator.
A283. secukinumab; a buffer system; a sugar component; a tonicifier; an ionic strength provider; and a diluent.
A284. secukinumab; a buffer system; a sugar component; a tonicifier; an antioxidant; and a chelator.
A285. secukinumab; a buffer system; a sugar component; a tonicifier; an antioxidant; and a diluent.
A286. secukinumab; a buffer system; a sugar component; a tonicifier; a chelator; and a diluent.
A287. secukinumab; a buffer system; a sugar component; an ionic strength provider; an antioxidant; and a chelator.
A288. secukinumab; a buffer system; a sugar component; an ionic strength provider; an antioxidant; and a diluent.
A289. secukinumab; a buffer system; a sugar component; an ionic strength provider; a chelator; and a diluent.
A290. secukinumab; a buffer system; a sugar component; an antioxidant; a chelator; and a diluent.
A291. secukinumab; a buffer system; an amino acid component; a surfactant; a tonicifier; and an ionic strength provider.
A292. secukinumab; a buffer system; an amino acid component; a surfactant; a tonicifier; and an antioxidant.
A293. secukinumab; a buffer system; an amino acid component; a surfactant; a tonicifier; and a chelator.
A294. secukinumab; a buffer system; an amino acid component; a surfactant; a tonicifier; and a diluent.
A295. secukinumab; a buffer system; an amino acid component; a surfactant; an ionic strength provider; and an antioxidant.
A296. secukinumab; a buffer system; an amino acid component; a surfactant; an ionic strength provider; and a chelator.
A297. secukinumab; a buffer system; an amino acid component; a surfactant; an ionic strength provider; and a diluent.
A298. secukinumab; a buffer system; an amino acid component; a surfactant; an antioxidant; and a chelator.
A299. secukinumab; a buffer system; an amino acid component; a surfactant; an antioxidant; and a diluent.
A300. secukinumab; a buffer system; an amino acid component; a surfactant; a chelator; and a diluent.
A301. secukinumab; a buffer system; an amino acid component; a tonicifier; an ionic strength provider; and an antioxidant.
A302. secukinumab; a buffer system; an amino acid component; a tonicifier; an ionic strength provider; and a chelator.
A303. secukinumab; a buffer system; an amino acid component; a tonicifier; an ionic strength provider; and a diluent.
A304. secukinumab; a buffer system; an amino acid component; a tonicifier; an antioxidant; and a chelator.
A305. secukinumab; a buffer system; an amino acid component; a tonicifier; an antioxidant; and a diluent.
A306. secukinumab; a buffer system; an amino acid component; a tonicifier; a chelator; and a diluent.
A307. secukinumab; a buffer system; an amino acid component; an ionic strength provider; an antioxidant; and a chelator.
A308. secukinumab; a buffer system; an amino acid component; an ionic strength provider; an antioxidant; and a diluent.
A309. secukinumab; a buffer system; an amino acid component; an ionic strength provider; a chelator; and a diluent.
A310. secukinumab; a buffer system; an amino acid component; an antioxidant; a chelator; and a diluent.
A311. secukinumab; a buffer system; a surfactant; a tonicifier; an ionic strength provider; and an antioxidant.
A312. secukinumab; a buffer system; a surfactant; a tonicifier; an ionic strength provider; and a chelator.
A313. secukinumab; a buffer system; a surfactant; a tonicifier; an ionic strength provider; and a diluent.
A314. secukinumab; a buffer system; a surfactant; a tonicifier; an antioxidant; and a chelator.
A315. secukinumab; a buffer system; a surfactant; a tonicifier; an antioxidant; and a diluent.

(continued)

A316. secukinumab; a buffer system; a surfactant; a tonicifier; a chelator; and a diluent.
A317. secukinumab; a buffer system; a surfactant; an ionic strength provider; an antioxidant; and a chelator.
A318. secukinumab; a buffer system; a surfactant; an ionic strength provider; an antioxidant; and a diluent.
A319. secukinumab; a buffer system; a surfactant; an ionic strength provider; a chelator; and a diluent.
A320. secukinumab; a buffer system; a surfactant; an antioxidant; a chelator; and a diluent.
A321. secukinumab; a buffer system; a tonicifier; an ionic strength provider; an antioxidant; and a chelator.
A322. secukinumab; a buffer system; a tonicifier; an ionic strength provider; an antioxidant; and a diluent.
A323. secukinumab; a buffer system; a tonicifier; an ionic strength provider; a chelator; and a diluent.
A324. secukinumab; a buffer system; a tonicifier; an antioxidant; a chelator; and a diluent.
A325. secukinumab; a buffer system; an ionic strength provider; an antioxidant; a chelator; and a diluent.
A326. secukinumab; a sugar component; an amino acid component; a surfactant; a tonicifier; and an ionic strength provider.
A327. secukinumab; a sugar component; an amino acid component; a surfactant; a tonicifier; and an antioxidant.
A328. secukinumab; a sugar component; an amino acid component; a surfactant; a tonicifier; and a chelator.
A329. secukinumab; a sugar component; an amino acid component; a surfactant; a tonicifier; and a diluent.
A330. secukinumab; a sugar component; an amino acid component; a surfactant; an ionic strength provider; and an antioxidant.
A331. secukinumab; a sugar component; an amino acid component; a surfactant; an ionic strength provider; and a chelator.
A332. secukinumab; a sugar component; an amino acid component; a surfactant; an ionic strength provider; and a diluent.
A333. secukinumab; a sugar component; an amino acid component; a surfactant; an antioxidant; and a chelator.
A334. secukinumab; a sugar component; an amino acid component; a surfactant; an antioxidant; and a diluent.
A335. secukinumab; a sugar component; an amino acid component; a surfactant; a chelator; and a diluent.
A336. secukinumab; a sugar component; an amino acid component; a tonicifier; an ionic strength provider; and an antioxidant.
A337. secukinumab; a sugar component; an amino acid component; a tonicifier; an ionic strength provider; and a chelator.
A338. secukinumab; a sugar component; an amino acid component; a tonicifier; an ionic strength provider; and a diluent.
A339. secukinumab; a sugar component; an amino acid component; a tonicifier; an antioxidant; and a chelator.
A340. secukinumab; a sugar component; an amino acid component; a tonicifier; an antioxidant; and a diluent.
A341. secukinumab; a sugar component; an amino acid component; a tonicifier; a chelator; and a diluent.
A342. secukinumab; a sugar component; an amino acid component; an ionic strength provider; an antioxidant; and a chelator.
A343. secukinumab; a sugar component; an amino acid component; an ionic strength provider; an antioxidant; and a diluent.
A344. secukinumab; a sugar component; an amino acid component; an ionic strength provider; a chelator; and a diluent.
A345. secukinumab; a sugar component; an amino acid component; an antioxidant; a chelator; and a diluent.
A346. secukinumab; a sugar component; a surfactant; a tonicifier; an ionic strength provider; and an antioxidant.
A347. secukinumab; a sugar component; a surfactant; a tonicifier; an ionic strength provider; and a chelator.
A348. secukinumab; a sugar component; a surfactant; a tonicifier; an ionic strength provider; and a diluent.
A349. secukinumab; a sugar component; a surfactant; a tonicifier; an antioxidant; and a chelator.
A350. secukinumab; a sugar component; a surfactant; a tonicifier; an antioxidant; and a diluent.
A351. secukinumab; a sugar component; a surfactant; a tonicifier; a chelator; and a diluent.
A352. secukinumab; a sugar component; a surfactant; an ionic strength provider; an antioxidant; and a chelator.
A353. secukinumab; a sugar component; a surfactant; an ionic strength provider; an antioxidant; and a diluent.
A354. secukinumab; a sugar component; a surfactant; an ionic strength provider; a chelator; and a diluent.
A355. secukinumab; a sugar component; a surfactant; an antioxidant; a chelator; and a diluent.
A356. secukinumab; a sugar component; a tonicifier; an ionic strength provider; an antioxidant; and a chelator.
A357. secukinumab; a sugar component; a tonicifier; an ionic strength provider; an antioxidant; and a diluent.

(continued)

A358. secukinumab; a sugar component; a tonicifier; an ionic strength provider; a chelator; and a diluent.

A359. secukinumab; a sugar component; a tonicifier; an antioxidant; a chelator; and a diluent.

A360. secukinumab; a sugar component; an ionic strength provider; an antioxidant; a chelator; and a diluent.

A361. secukinumab; an amino acid component; a surfactant; a tonicifier; an ionic strength provider; and an antioxidant.

A362. secukinumab; an amino acid component; a surfactant; a tonicifier; an ionic strength provider; and a chelator.

A363. secukinumab; an amino acid component; a surfactant; a tonicifier; an ionic strength provider; and a diluent.

A364. secukinumab; an amino acid component; a surfactant; a tonicifier; an antioxidant; and a chelator.

A365. secukinumab; an amino acid component; a surfactant; a tonicifier; an antioxidant; and a diluent.

A366. secukinumab; an amino acid component; a surfactant; a tonicifier; a chelator; and a diluent.

A367. secukinumab; an amino acid component; a surfactant; an ionic strength provider; an antioxidant; and a chelator.

A368. secukinumab; an amino acid component; a surfactant; an ionic strength provider; an antioxidant; and a diluent.

A369. secukinumab; an amino acid component; a surfactant; an ionic strength provider; a chelator; and a diluent.

A370. secukinumab; an amino acid component; a surfactant; an antioxidant; a chelator; and a diluent.

A371. secukinumab; an amino acid component; a tonicifier; an ionic strength provider; an antioxidant; and a chelator.

A372. secukinumab; an amino acid component; a tonicifier; an ionic strength provider; an antioxidant; and a diluent.

A373. secukinumab; an amino acid component; a tonicifier; an ionic strength provider; a chelator; and a diluent.

A374. secukinumab; an amino acid component; a tonicifier; an antioxidant; a chelator; and a diluent.

A375. secukinumab; an amino acid component; an ionic strength provider; an antioxidant; a chelator; and a diluent.

A376. secukinumab; a surfactant; a tonicifier; an ionic strength provider; an antioxidant; and a chelator.

A377. secukinumab; a surfactant; a tonicifier; an ionic strength provider; an antioxidant; and a diluent.

A378. secukinumab; a surfactant; a tonicifier; an ionic strength provider; a chelator; and a diluent.

A379. secukinumab; a surfactant; a tonicifier; an antioxidant; a chelator; and a diluent.

A380. secukinumab; a surfactant; an ionic strength provider; an antioxidant; a chelator; and a diluent.

A381. secukinumab; a tonicifier; an ionic strength provider; an antioxidant; a chelator; and a diluent.

A382. secukinumab; a buffer system; a sugar component; an amino acid component; a surfactant; a tonicifier; and an ionic strength provider.

A383. secukinumab; a buffer system; a sugar component; an amino acid component; a surfactant; a tonicifier; and an antioxidant.

A384. secukinumab; a buffer system; a sugar component; an amino acid component; a surfactant; a tonicifier; and a chelator.

A385. secukinumab; a buffer system; a sugar component; an amino acid component; a surfactant; a tonicifier; and a diluent.

A386. secukinumab; a buffer system; a sugar component; an amino acid component; a surfactant; an ionic strength provider; and an antioxidant.

A387. secukinumab; a buffer system; a sugar component; an amino acid component; a surfactant; an ionic strength provider; and a chelator.

A388. secukinumab; a buffer system; a sugar component; an amino acid component; a surfactant; an ionic strength provider; and a diluent.

A389. secukinumab; a buffer system; a sugar component; an amino acid component; a surfactant; an antioxidant; and a chelator.

A390. secukinumab; a buffer system; a sugar component; an amino acid component; a surfactant; an antioxidant; and a diluent.

A391. secukinumab; a buffer system; a sugar component; an amino acid component; a surfactant; a chelator; and a diluent.

A392. secukinumab; a buffer system; a sugar component; an amino acid component; a tonicifier; an ionic strength provider; and an antioxidant.

A393. secukinumab; a buffer system; a sugar component; an amino acid component; a tonicifier; an ionic strength provider; and a chelator.

(continued)

A394. secukinumab; a buffer system; a sugar component; an amino acid component; a tonicifier; an ionic strength provider; and a diluent.
A395. secukinumab; a buffer system; a sugar component; an amino acid component; a tonicifier; an antioxidant; and a chelator.
A396. secukinumab; a buffer system; a sugar component; an amino acid component; a tonicifier; an antioxidant; and a diluent.
A397. secukinumab; a buffer system; a sugar component; an amino acid component; a tonicifier; a chelator; and a diluent.
A398. secukinumab; a buffer system; a sugar component; an amino acid component; an ionic strength provider; an antioxidant; and a chelator.
A399. secukinumab; a buffer system; a sugar component; an amino acid component; an ionic strength provider; an antioxidant; and a diluent.
A400. secukinumab; a buffer system; a sugar component; an amino acid component; an ionic strength provider; a chelator; and a diluent.
A401. secukinumab; a buffer system; a sugar component; an amino acid component; an antioxidant; a chelator; and a diluent.
A402. secukinumab; a buffer system; a sugar component; a surfactant; a tonicifier; an ionic strength provider; and an antioxidant.
A403. secukinumab; a buffer system; a sugar component; a surfactant; a tonicifier; an ionic strength provider; and a chelator.
A404. secukinumab; a buffer system; a sugar component; a surfactant; a tonicifier; an ionic strength provider; and a diluent.
A405. secukinumab; a buffer system; a sugar component; a surfactant; a tonicifier; an antioxidant; and a chelator.
A406. secukinumab; a buffer system; a sugar component; a surfactant; a tonicifier; an antioxidant; and a diluent.
A407. secukinumab; a buffer system; a sugar component; a surfactant; a tonicifier; a chelator; and a diluent.
A408. secukinumab; a buffer system; a sugar component; a surfactant; an ionic strength provider; an antioxidant; and a chelator.
A409. secukinumab; a buffer system; a sugar component; a surfactant; an ionic strength provider; an antioxidant; and a diluent.
A410. secukinumab; a buffer system; a sugar component; a surfactant; an ionic strength provider; a chelator; and a diluent.
A411. secukinumab; a buffer system; a sugar component; a surfactant; an antioxidant; a chelator; and a diluent.
A412. secukinumab; a buffer system; a sugar component; a tonicifier; an ionic strength provider; an antioxidant; and a chelator.
A413. secukinumab; a buffer system; a sugar component; a tonicifier; an ionic strength provider; an antioxidant; and a diluent.
A414. secukinumab; a buffer system; a sugar component; a tonicifier; an ionic strength provider; a chelator; and a diluent.
A415. secukinumab; a buffer system; a sugar component; a tonicifier; an antioxidant; a chelator; and a diluent.
A416. secukinumab; a buffer system; a sugar component; an ionic strength provider; an antioxidant; a chelator; and a diluent.
A417. secukinumab; a buffer system; an amino acid component; a surfactant; a tonicifier; an ionic strength provider; and an antioxidant.
A418. secukinumab; a buffer system; an amino acid component; a surfactant; a tonicifier; an ionic strength provider; and a chelator.
A419. secukinumab; a buffer system; an amino acid component; a surfactant; a tonicifier; an ionic strength provider; and a diluent.
A420. secukinumab; a buffer system; an amino acid component; a surfactant; a tonicifier; an antioxidant; and a chelator.
A421. secukinumab; a buffer system; an amino acid component; a surfactant; a tonicifier; an antioxidant; and a diluent.
A422. secukinumab; a buffer system; an amino acid component; a surfactant; a tonicifier; a chelator; and a diluent.

(continued)

A423. secukinumab; a buffer system; an amino acid component; a surfactant; an ionic strength provider; an antioxidant; and a chelator.

A424. secukinumab; a buffer system; an amino acid component; a surfactant; an ionic strength provider; an antioxidant; and a diluent.

A425. secukinumab; a buffer system; an amino acid component; a surfactant; an ionic strength provider; a chelator; and a diluent.

A426. secukinumab; a buffer system; an amino acid component; a surfactant; an antioxidant; a chelator; and a diluent.

A427. secukinumab; a buffer system; an amino acid component; a tonicifier; an ionic strength provider; an antioxidant; and a chelator.

A428. secukinumab; a buffer system; an amino acid component; a tonicifier; an ionic strength provider; an antioxidant; and a diluent.

A429. secukinumab; a buffer system; an amino acid component; a tonicifier; an ionic strength provider; a chelator; and a diluent.

A430. secukinumab; a buffer system; an amino acid component; a tonicifier; an antioxidant; a chelator; and a diluent.

A431. secukinumab; a buffer system; an amino acid component; an ionic strength provider; an antioxidant; a chelator; and a diluent.

A432. secukinumab; a buffer system; a surfactant; a tonicifier; an ionic strength provider; an antioxidant; and a chelator.

A433. secukinumab; a buffer system; a surfactant; a tonicifier; an ionic strength provider; an antioxidant; and a diluent.

A434. secukinumab; a buffer system; a surfactant; a tonicifier; an ionic strength provider; a chelator; and a diluent.

A435. secukinumab; a buffer system; a surfactant; a tonicifier; an antioxidant; a chelator; and a diluent.

A436. secukinumab; a buffer system; a surfactant; an ionic strength provider; an antioxidant; a chelator; and a diluent.

A437. secukinumab; a buffer system; a tonicifier; an ionic strength provider; an antioxidant; a chelator; and a diluent.

A438. secukinumab; a sugar component; an amino acid component; a surfactant; a tonicifier; an ionic strength provider; and an antioxidant.

A439. secukinumab; a sugar component; an amino acid component; a surfactant; a tonicifier; an ionic strength provider; and a chelator.

A440. secukinumab; a sugar component; an amino acid component; a surfactant; a tonicifier; an ionic strength provider; and a diluent.

A441. secukinumab; a sugar component; an amino acid component; a surfactant; a tonicifier; an antioxidant; and a chelator.

A442. secukinumab; a sugar component; an amino acid component; a surfactant; a tonicifier; an antioxidant; and a diluent.

A443. secukinumab; a sugar component; an amino acid component; a surfactant; a tonicifier; a chelator; and a diluent.

A444. secukinumab; a sugar component; an amino acid component; a surfactant; an ionic strength provider; an antioxidant; and a chelator.

A445. secukinumab; a sugar component; an amino acid component; a surfactant; an ionic strength provider; an antioxidant; and a diluent.

A446. secukinumab; a sugar component; an amino acid component; a surfactant; an ionic strength provider; a chelator; and a diluent.

A447. secukinumab; a sugar component; an amino acid component; a surfactant; an antioxidant; a chelator; and a diluent.

A448. secukinumab; a sugar component; an amino acid component; a tonicifier; an ionic strength provider; an antioxidant; and a chelator.

A449. secukinumab; a sugar component; an amino acid component; a tonicifier; an ionic strength provider; an antioxidant; and a diluent.

A450. secukinumab; a sugar component; an amino acid component; a tonicifier; an ionic strength provider; a chelator; and a diluent.

(continued)

A451. secukinumab; a sugar component; an amino acid component; a tonicifier; an antioxidant; a chelator; and a diluent.

A452. secukinumab; a sugar component; an amino acid component; an ionic strength provider; an antioxidant; a chelator; and a diluent.

A453. secukinumab; a sugar component; a surfactant; a tonicifier; an ionic strength provider; an antioxidant; and a chelator.

A454. secukinumab; a sugar component; a surfactant; a tonicifier; an ionic strength provider; an antioxidant; and a diluent.

A455. secukinumab; a sugar component; a surfactant; a tonicifier; an ionic strength provider; a chelator; and a diluent.

A456. secukinumab; a sugar component; a surfactant; a tonicifier; an antioxidant; a chelator; and a diluent.

A457. secukinumab; a sugar component; a surfactant; an ionic strength provider; an antioxidant; a chelator; and a diluent.

A458. secukinumab; a sugar component; a tonicifier; an ionic strength provider; an antioxidant; a chelator; and a diluent.

A459. secukinumab; an amino acid component; a surfactant; a tonicifier; an ionic strength provider; an antioxidant; and a chelator.

A460. secukinumab; an amino acid component; a surfactant; a tonicifier; an ionic strength provider; an antioxidant; and a diluent.

A461. secukinumab; an amino acid component; a surfactant; a tonicifier; an ionic strength provider; a chelator; and a diluent.

A462. secukinumab; an amino acid component; a surfactant; a tonicifier; an antioxidant; a chelator; and a diluent.

A463. secukinumab; an amino acid component; a surfactant; an ionic strength provider; an antioxidant; a chelator; and a diluent.

A464. secukinumab; an amino acid component; a tonicifier; an ionic strength provider; an antioxidant; a chelator; and a diluent.

A465. secukinumab; a surfactant; a tonicifier; an ionic strength provider; an antioxidant; a chelator; and a diluent.

A466. secukinumab; a buffer system; a sugar component; an amino acid component; a surfactant; a tonicifier; an ionic strength provider; and an antioxidant.

A467. secukinumab; a buffer system; a sugar component; an amino acid component; a surfactant; a tonicifier; an ionic strength provider; and a chelator.

A468. secukinumab; a buffer system; a sugar component; an amino acid component; a surfactant; a tonicifier; an ionic strength provider; and a diluent.

A469. secukinumab; a buffer system; a sugar component; an amino acid component; a surfactant; a tonicifier; an antioxidant; and a chelator.

A470. secukinumab; a buffer system; a sugar component; an amino acid component; a surfactant; a tonicifier; an antioxidant; and a diluent.

A471. secukinumab; a buffer system; a sugar component; an amino acid component; a surfactant; a tonicifier; a chelator; and a diluent.

A472. secukinumab; a buffer system; a sugar component; an amino acid component; a surfactant; an ionic strength provider; an antioxidant; and a chelator.

A473. secukinumab; a buffer system; a sugar component; an amino acid component; a surfactant; an ionic strength provider; an antioxidant; and a diluent.

A474. secukinumab; a buffer system; a sugar component; an amino acid component; a surfactant; an ionic strength provider; a chelator; and a diluent.

A475. secukinumab; a buffer system; a sugar component; an amino acid component; a surfactant; an antioxidant; a chelator; and a diluent.

A476. secukinumab; a buffer system; a sugar component; an amino acid component; a tonicifier; an ionic strength provider; an antioxidant; and a chelator.

A477. secukinumab; a buffer system; a sugar component; an amino acid component; a tonicifier; an ionic strength provider; an antioxidant; and a diluent.

A478. secukinumab; a buffer system; a sugar component; an amino acid component; a tonicifier; an ionic strength provider; a chelator; and a diluent.

(continued)

A479. secukinumab; a buffer system; a sugar component; an amino acid component; a tonicifier; an antioxidant; a chelator; and a diluent.

A480. secukinumab; a buffer system; a sugar component; an amino acid component; an ionic strength provider; an antioxidant; a chelator; and a diluent.

A481. secukinumab; a buffer system; a sugar component; a surfactant; a tonicifier; an ionic strength provider; an antioxidant; and a chelator.

A482. secukinumab; a buffer system; a sugar component; a surfactant; a tonicifier; an ionic strength provider; an antioxidant; and a diluent.

A483. secukinumab; a buffer system; a sugar component; a surfactant; a tonicifier; an ionic strength provider; a chelator; and a diluent.

A484. secukinumab; a buffer system; a sugar component; a surfactant; a tonicifier; an antioxidant; a chelator; and a diluent.

A485. secukinumab; a buffer system; a sugar component; a surfactant; an ionic strength provider; an antioxidant; a chelator; and a diluent.

A486. secukinumab; a buffer system; a sugar component; a tonicifier; an ionic strength provider; an antioxidant; a chelator; and a diluent.

A487. secukinumab; a buffer system; an amino acid component; a surfactant; a tonicifier; an ionic strength provider; an antioxidant; and a chelator.

A488. secukinumab; a buffer system; an amino acid component; a surfactant; a tonicifier; an ionic strength provider; an antioxidant; and a diluent.

A489. secukinumab; a buffer system; an amino acid component; a surfactant; a tonicifier; an ionic strength provider; a chelator; and a diluent.

A490. secukinumab; a buffer system; an amino acid component; a surfactant; a tonicifier; an antioxidant; a chelator; and a diluent.

A491. secukinumab; a buffer system; an amino acid component; a surfactant; an ionic strength provider; an antioxidant; a chelator; and a diluent.

A492. secukinumab; a buffer system; an amino acid component; a tonicifier; an ionic strength provider; an antioxidant; a chelator; and a diluent.

A493. secukinumab; a buffer system; a surfactant; a tonicifier; an ionic strength provider; an antioxidant; a chelator; and a diluent.

A494. secukinumab; a sugar component; an amino acid component; a surfactant; a tonicifier; an ionic strength provider; an antioxidant; and a chelator.

A495. secukinumab; a sugar component; an amino acid component; a surfactant; a tonicifier; an ionic strength provider; an antioxidant; and a diluent.

A496. secukinumab; a sugar component; an amino acid component; a surfactant; a tonicifier; an ionic strength provider; a chelator; and a diluent.

A497. secukinumab; a sugar component; an amino acid component; a surfactant; a tonicifier; an antioxidant; a chelator; and a diluent.

A498. secukinumab; a sugar component; an amino acid component; a surfactant; an ionic strength provider; an antioxidant; a chelator; and a diluent.

A499. secukinumab; a sugar component; an amino acid component; a tonicifier; an ionic strength provider; an antioxidant; a chelator; and a diluent.

A500. secukinumab; a sugar component; a surfactant; a tonicifier; an ionic strength provider; an antioxidant; a chelator; and a diluent.

A501. secukinumab; an amino acid component; a surfactant; a tonicifier; an ionic strength provider; an antioxidant; a chelator; and a diluent.

A502. secukinumab; a buffer system; a sugar component; an amino acid component; a surfactant; a tonicifier; an ionic strength provider; an antioxidant; and a chelator.

A503. secukinumab; a buffer system; a sugar component; an amino acid component; a surfactant; a tonicifier; an ionic strength provider; an antioxidant; and a diluent.

A504. secukinumab; a buffer system; a sugar component; an amino acid component; a surfactant; a tonicifier; an ionic strength provider; a chelator; and a diluent.

(continued)

A505. secukinumab; a buffer system; a sugar component; an amino acid component; a surfactant; a tonicifier; an antioxidant; a chelator; and a diluent.
A506. secukinumab; a buffer system; a sugar component; an amino acid component; a surfactant; an ionic strength provider; an antioxidant; a chelator; and a diluent.
A507. secukinumab; a buffer system; a sugar component; an amino acid component; a tonicifier; an ionic strength provider; an antioxidant; a chelator; and a diluent.
A508. secukinumab; a buffer system; a sugar component; a surfactant; a tonicifier; an ionic strength provider; an antioxidant; a chelator; and a diluent.
A509. secukinumab; a buffer system; an amino acid component; a surfactant; a tonicifier; an ionic strength provider; an antioxidant; a chelator; and a diluent.
A510. secukinumab; a sugar component; an amino acid component; a surfactant; a tonicifier; an ionic strength provider; an antioxidant; a chelator; and a diluent.
A511. secukinumab; a buffer system; a sugar component; an amino acid component; a surfactant; a tonicifier; an ionic strength provider; an antioxidant; a chelator; and a diluent.

The embodiments of A1-A511 may be further defined or characterised by features of numbered paragraphs B1-B255, C1-C255, D1-D1152, E1-E1151, F1-F12, G1-G959, H1-H1423, 11-12400, and J1-J1158.

*Embodiments of Combinations of Ingredients Quantified by Broad Absolute Concentrations*

B) General Aspects and Embodiments Relating to Combinations of Ingredients Quantified by Broad Concentrations

The biopharmaceutical composition suitably comprises, or consists of (suitably along with a diluent, which is preferably water), 100-350 mg/mL biopharmaceutical active (which is preferably secukinumab), a diluent, and one or more of any of 1-70 mM buffer system; 30-400 mM sugar component; 2-350 mM amino acid component; 0.001-5 mg/mL surfactant; 5-300 mM tonicifier; 5-300 mM ionic strength provider; 0.001-300 mM antioxidant; 0.0001-5 mM chelator. The following numbered paragraphs B1-B255 disclose specific aspects and embodiments of the invention, wherein the biopharmaceutical composition comprises, consists of (suitably along with a diluent, which is preferably water), and/or is otherwise characterised by:

B1. 100-350 mg/mL secukinumab; 1-70 mM buffer system; and a diluent.
B2. 100-350 mg/mL secukinumab; 30-400 mM sugar component; and a diluent.
B3. 100-350 mg/mL secukinumab; 2-350 mM amino acid component; and a diluent.
B4. 100-350 mg/mL secukinumab; 0.001-5 mg/mL surfactant; and a diluent.
B5. 100-350 mg/mL secukinumab; 5-300 mM tonicifier; and a diluent.
B6. 100-350 mg/mL secukinumab; 5-300 mM ionic strength provider; and a diluent.
B7. 100-350 mg/mL secukinumab; 0.001-300 mM antioxidant; and a diluent.
B8. 100-350 mg/mL secukinumab; 0.0001-5 mM chelator; and a diluent.
B9. 100-350 mg/mL secukinumab; 1-70 mM buffer system; 30-400 mM sugar component; and a diluent.
B10. 100-350 mg/mL secukinumab; 1-70 mM buffer system; 2-350 mM amino acid component; and a diluent.
B11. 100-350 mg/mL secukinumab; 1-70 mM buffer system; 0.001-5 mg/mL surfactant; and a diluent.
B12. 100-350 mg/mL secukinumab; 1-70 mM buffer system; 5-300 mM tonicifier; and a diluent.
B13. 100-350 mg/mL secukinumab; 1-70 mM buffer system; 5-300 mM ionic strength provider; and a diluent.
B14. 100-350 mg/mL secukinumab; 1-70 mM buffer system; 0.001-300 mM antioxidant; and a diluent.
B15. 100-350 mg/mL secukinumab; 1-70 mM buffer system; 0.0001-5 mM chelator; and a diluent.
B16. 100-350 mg/mL secukinumab; 30-400 mM sugar component; 2-350 mM amino acid component; and a diluent.
B17. 100-350 mg/mL secukinumab; 30-400 mM sugar component; 0.001-5 mg/mL surfactant; and a diluent.
B18. 100-350 mg/mL secukinumab; 30-400 mM sugar component; 5-300 mM tonicifier; and a diluent.
B19. 100-350 mg/mL secukinumab; 30-400 mM sugar component; 5-300 mM ionic strength provider; and a diluent.
B20. 100-350 mg/mL secukinumab; 30-400 mM sugar component; 0.001-300 mM antioxidant; and a diluent.
B21. 100-350 mg/mL secukinumab; 30-400 mM sugar component; 0.0001-5 mM chelator; and a diluent.

(continued)

B22. 100-350 mg/mL secukinumab; 2-350 mM amino acid component; 0.001-5 mg/mL surfactant; and a diluent.
B23. 100-350 mg/mL secukinumab; 2-350 mM amino acid component; 5-300 mM tonicifier; and a diluent.
B24. 100-350 mg/mL secukinumab; 2-350 mM amino acid component; 5-300 mM ionic strength provider; and a diluent.
B25. 100-350 mg/mL secukinumab; 2-350 mM amino acid component; 0.001-300 mM antioxidant; and a diluent.
B26. 100-350 mg/mL secukinumab; 2-350 mM amino acid component; 0.0001-5 mM chelator; and a diluent.
B27. 100-350 mg/mL secukinumab; 0.001-5 mg/mL surfactant; 5-300 mM tonicifier; and a diluent.
B28. 100-350 mg/mL secukinumab; 0.001-5 mg/mL surfactant; 5-300 mM ionic strength provider; and a diluent.
B29. 100-350 mg/mL secukinumab; 0.001-5 mg/mL surfactant; 0.001-300 mM antioxidant; and a diluent.
B30. 100-350 mg/mL secukinumab; 0.001-5 mg/mL surfactant; 0.0001-5 mM chelator; and a diluent.
B31. 100-350 mg/mL secukinumab; 5-300 mM tonicifier; 5-300 mM ionic strength provider; and a diluent.
B32. 100-350 mg/mL secukinumab; 5-300 mM tonicifier; 0.001-300 mM antioxidant; and a diluent.
B33. 100-350 mg/mL secukinumab; 5-300 mM tonicifier; 0.0001-5 mM chelator; and a diluent.
B34. 100-350 mg/mL secukinumab; 5-300 mM ionic strength provider; 0.001-300 mM antioxidant; and a diluent.
B35. 100-350 mg/mL secukinumab; 5-300 mM ionic strength provider; 0.0001-5 mM chelator; and a diluent.
B36. 100-350 mg/mL secukinumab; 0.001-300 mM antioxidant; 0.0001-5 mM chelator; and a diluent.
B37. 100-350 mg/mL secukinumab; 1-70 mM buffer system; 30-400 mM sugar component; 2-350 mM amino acid component; and a diluent.
B38. 100-350 mg/mL secukinumab; 1-70 mM buffer system; 30-400 mM sugar component; 0.001-5 mg/mL surfactant; and a diluent.
B39. 100-350 mg/mL secukinumab; 1-70 mM buffer system; 30-400 mM sugar component; 5-300 mM tonicifier; and a diluent.
B40. 100-350 mg/mL secukinumab; 1-70 mM buffer system; 30-400 mM sugar component; 5-300 mM ionic strength provider; and a diluent.
B41. 100-350 mg/mL secukinumab; 1-70 mM buffer system; 30-400 mM sugar component; 0.001-300 mM antioxidant; and a diluent.
B42. 100-350 mg/mL secukinumab; 1-70 mM buffer system; 30-400 mM sugar component; 0.0001-5 mM chelator; and a diluent.
B43. 100-350 mg/mL secukinumab; 1-70 mM buffer system; 2-350 mM amino acid component; 0.001-5 mg/mL surfactant; and a diluent.
B44. 100-350 mg/mL secukinumab; 1-70 mM buffer system; 2-350 mM amino acid component; 5-300 mM tonicifier; and a diluent.
B45. 100-350 mg/mL secukinumab; 1-70 mM buffer system; 2-350 mM amino acid component; 5-300 mM ionic strength provider; and a diluent.
B46. 100-350 mg/mL secukinumab; 1-70 mM buffer system; 2-350 mM amino acid component; 0.001-300 mM antioxidant; and a diluent.
B47. 100-350 mg/mL secukinumab; 1-70 mM buffer system; 2-350 mM amino acid component; 0.0001-5 mM chelator; and a diluent.
B48. 100-350 mg/mL secukinumab; 1-70 mM buffer system; 0.001-5 mg/mL surfactant; 5-300 mM tonicifier; and a diluent.
B49. 100-350 mg/mL secukinumab; 1-70 mM buffer system; 0.001 -5 mg/mL surfactant; 5-300 mM ionic strength provider; and a diluent.
B50. 100-350 mg/mL secukinumab; 1-70 mM buffer system; 0.001-5 mg/mL surfactant; 0.001-300 mM antioxidant; and a diluent.
B51. 100-350 mg/mL secukinumab; 1-70 mM buffer system; 0.001-5 mg/mL surfactant; 0.0001-5 mM chelator; and a diluent.
B52. 100-350 mg/mL secukinumab; 1-70 mM buffer system; 5-300 mM tonicifier; 5-300 mM ionic strength provider; and a diluent.
B53. 100-350 mg/mL secukinumab; 1-70 mM buffer system; 5-300 mM tonicifier; 0.001-300 mM antioxidant; and a diluent.
B54. 100-350 mg/mL secukinumab; 1-70 mM buffer system; 5-300 mM tonicifier; 0.0001-5 mM chelator; and a diluent.

(continued)

B55. 100-350 mg/mL secukinumab; 1-70 mM buffer system; 5-300 mM ionic strength provider; 0.001-300 mM antioxidant; and a diluent.

B56. 100-350 mg/mL secukinumab; 1-70 mM buffer system; 5-300 mM ionic strength provider; 0.0001-5 mM chelator; and a diluent.

B57. 100-350 mg/mL secukinumab; 1-70 mM buffer system; 0.001-300 mM antioxidant; 0.0001-5 mM chelator; and a diluent.

B58. 100-350 mg/mL secukinumab; 30-400 mM sugar component; 2-350 mM amino acid component; 0.001-5 mg/mL surfactant; and a diluent.

B59. 100-350 mg/mL secukinumab; 30-400 mM sugar component; 2-350 mM amino acid component; 5-300 mM tonicifier; and a diluent.

B60. 100-350 mg/mL secukinumab; 30-400 mM sugar component; 2-350 mM amino acid component; 5-300 mM ionic strength provider; and a diluent.

B61. 100-350 mg/mL secukinumab; 30-400 mM sugar component; 2-350 mM amino acid component; 0.001-300 mM antioxidant; and a diluent.

B62. 100-350 mg/mL secukinumab; 30-400 mM sugar component; 2-350 mM amino acid component; 0.0001-5 mM chelator; and a diluent.

B63. 100-350 mg/mL secukinumab; 30-400 mM sugar component; 0.001-5 mg/mL surfactant; 5-300 mM tonicifier; and a diluent.

B64. 100-350 mg/mL secukinumab; 30-400 mM sugar component; 0.001-5 mg/mL surfactant; 5-300 mM ionic strength provider; and a diluent.

B65. 100-350 mg/mL secukinumab; 30-400 mM sugar component; 0.001-5 mg/mL surfactant; 0.001-300 mM antioxidant; and a diluent.

B66. 100-350 mg/mL secukinumab; 30-400 mM sugar component; 0.001-5 mg/mL surfactant; 0.0001-5 mM chelator; and a diluent.

B67. 100-350 mg/mL secukinumab; 30-400 mM sugar component; 5-300 mM tonicifier; 5-300 mM ionic strength provider; and a diluent.

B68. 100-350 mg/mL secukinumab; 30-400 mM sugar component; 5-300 mM tonicifier; 0.001-300 mM antioxidant; and a diluent.

B69. 100-350 mg/mL secukinumab; 30-400 mM sugar component; 5-300 mM tonicifier; 0.0001-5 mM chelator; and a diluent.

B70. 100-350 mg/mL secukinumab; 30-400 mM sugar component; 5-300 mM ionic strength provider; 0.001-300 mM antioxidant; and a diluent.

B71. 100-350 mg/mL secukinumab; 30-400 mM sugar component; 5-300 mM ionic strength provider; 0.0001-5 mM chelator; and a diluent.

B72. 100-350 mg/mL secukinumab; 30-400 mM sugar component; 0.001-300 mM antioxidant; 0.0001-5 mM chelator; and a diluent.

B73. 100-350 mg/mL secukinumab; 2-350 mM amino acid component; 0.001-5 mg/mL surfactant; 5-300 mM tonicifier; and a diluent.

B74. 100-350 mg/mL secukinumab; 2-350 mM amino acid component; 0.001-5 mg/mL surfactant; 5-300 mM ionic strength provider; and a diluent.

B75. 100-350 mg/mL secukinumab; 2-350 mM amino acid component; 0.001-5 mg/mL surfactant; 0.001-300 mM antioxidant; and a diluent.

B76. 100-350 mg/mL secukinumab; 2-350 mM amino acid component; 0.001-5 mg/mL surfactant; 0.0001-5 mM chelator; and a diluent.

B77. 100-350 mg/mL secukinumab; 2-350 mM amino acid component; 5-300 mM tonicifier; 5-300 mM ionic strength provider; and a diluent.

B78. 100-350 mg/mL secukinumab; 2-350 mM amino acid component; 5-300 mM tonicifier; 0.001-300 mM antioxidant; and a diluent.

B79. 100-350 mg/mL secukinumab; 2-350 mM amino acid component; 5-300 mM tonicifier; 0.0001-5 mM chelator; and a diluent.

B80. 100-350 mg/mL secukinumab; 2-350 mM amino acid component; 5-300 mM ionic strength provider; 0.001-300 mM antioxidant; and a diluent.

(continued)

B81. 100-350 mg/mL secukinumab; 2-350 mM amino acid component; 5-300 mM ionic strength provider; 0.0001-5 mM chelator; and a diluent.

B82. 100-350 mg/mL secukinumab; 2-350 mM amino acid component; 0.001-300 mM antioxidant; 0.0001-5 mM chelator; and a diluent.

B83. 100-350 mg/mL secukinumab; 0.001-5 mg/mL surfactant; 5-300 mM tonicifier; 5-300 mM ionic strength provider; and a diluent.

B84. 100-350 mg/mL secukinumab; 0.001-5 mg/mL surfactant; 5-300 mM tonicifier; 0.001-300 mM antioxidant; and a diluent.

B85. 100-350 mg/mL secukinumab; 0.001-5 mg/mL surfactant; 5-300 mM tonicifier; 0.0001-5 mM chelator; and a diluent.

B86. 100-350 mg/mL secukinumab; 0.001-5 mg/mL surfactant; 5-300 mM ionic strength provider; 0.001-300 mM antioxidant; and a diluent.

B87. 100-350 mg/mL secukinumab; 0.001-5 mg/mL surfactant; 5-300 mM ionic strength provider; 0.0001-5 mM chelator; and a diluent.

B88. 100-350 mg/mL secukinumab; 0.001-5 mg/mL surfactant; 0.001-300 mM antioxidant; 0.0001-5 mM chelator; and a diluent.

B89. 100-350 mg/mL secukinumab; 5-300 mM tonicifier; 5-300 mM ionic strength provider; 0.001-300 mM antioxidant; and a diluent.

B90. 100-350 mg/mL secukinumab; 5-300 mM tonicifier; 5-300 mM ionic strength provider; 0.0001-5 mM chelator; and a diluent.

B91. 100-350 mg/mL secukinumab; 5-300 mM tonicifier; 0.001-300 mM antioxidant; 0.0001-5 mM chelator; and a diluent.

B92. 100-350 mg/mL secukinumab; 5-300 mM ionic strength provider; 0.001-300 mM antioxidant; 0.0001-5 mM chelator; and a diluent.

B93. 100-350 mg/mL secukinumab; 1-70 mM buffer system; 30-400 mM sugar component; 2-350 mM amino acid component; 0.001-5 mg/mL surfactant; and a diluent.

B94. 100-350 mg/mL secukinumab; 1-70 mM buffer system; 30-400 mM sugar component; 2-350 mM amino acid component; 5-300 mM tonicifier; and a diluent.

B95. 100-350 mg/mL secukinumab; 1-70 mM buffer system; 30-400 mM sugar component; 2-350 mM amino acid component; 5-300 mM ionic strength provider; and a diluent.

B96. 100-350 mg/mL secukinumab; 1-70 mM buffer system; 30-400 mM sugar component; 2-350 mM amino acid component; 0.001-300 mM antioxidant; and a diluent.

B97. 100-350 mg/mL secukinumab; 1-70 mM buffer system; 30-400 mM sugar component; 2-350 mM amino acid component; 0.0001-5 mM chelator; and a diluent.

B98. 100-350 mg/mL secukinumab; 1-70 mM buffer system; 30-400 mM sugar component; 0.001-5 mg/mL surfactant; 5-300 mM tonicifier; and a diluent.

B99. 100-350 mg/mL secukinumab; 1-70 mM buffer system; 30-400 mM sugar component; 0.001-5 mg/mL surfactant; 5-300 mM ionic strength provider; and a diluent.

B100. 100-350 mg/mL secukinumab; 1-70 mM buffer system; 30-400 mM sugar component; 0.001-5 mg/mL surfactant; 0.001-300 mM antioxidant; and a diluent.

B101. 100-350 mg/mL secukinumab; 1-70 mM buffer system; 30-400 mM sugar component; 0.001-5 mg/mL surfactant; 0.0001-5 mM chelator; and a diluent.

B102. 100-350 mg/mL secukinumab; 1-70 mM buffer system; 30-400 mM sugar component; 5-300 mM tonicifier; 5-300 mM ionic strength provider; and a diluent.

B103. 100-350 mg/mL secukinumab; 1-70 mM buffer system; 30-400 mM sugar component; 5-300 mM tonicifier; 0.001-300 mM antioxidant; and a diluent.

B104. 100-350 mg/mL secukinumab; 1-70 mM buffer system; 30-400 mM sugar component; 5-300 mM tonicifier; 0.0001-5 mM chelator; and a diluent.

B105. 100-350 mg/mL secukinumab; 1-70 mM buffer system; 30-400 mM sugar component; 5-300 mM ionic strength provider; 0.001-300 mM antioxidant; and a diluent.

B106. 100-350 mg/mL secukinumab; 1-70 mM buffer system; 30-400 mM sugar component; 5-300 mM ionic strength provider; 0.0001-5 mM chelator; and a diluent.

(continued)

B107. 100-350 mg/mL secukinumab; 1-70 mM buffer system; 30-400 mM sugar component; 0.001-300 mM antioxidant; 0.0001-5 mM chelator; and a diluent.

B108. 100-350 mg/mL secukinumab; 1-70 mM buffer system; 2-350 mM amino acid component; 0.001-5 mg/mL surfactant; 5-300 mM tonicifier; and a diluent.

B109. 100-350 mg/mL secukinumab; 1-70 mM buffer system; 2-350 mM amino acid component; 0.001-5 mg/mL surfactant; 5-300 mM ionic strength provider; and a diluent.

B110. 100-350 mg/mL secukinumab; 1-70 mM buffer system; 2-350 mM amino acid component; 0.001 -5 mg/mL surfactant; 0.001 - 300 mM antioxidant; and a diluent.

B111. 100-350 mg/mL secukinumab; 1-70 mM buffer system; 2-350 mM amino acid component; 0.001-5 mg/mL surfactant; 0.0001-5 mM chelator; and a diluent.

B112. 100-350 mg/mL secukinumab; 1-70 mM buffer system; 2-350 mM amino acid component; 5-300 mM tonicifier; 5-300 mM ionic strength provider; and a diluent.

B113. 100-350 mg/mL secukinumab; 1-70 mM buffer system; 2-350 mM amino acid component; 5-300 mM tonicifier; 0.001-300 mM antioxidant; and a diluent.

B114. 100-350 mg/mL secukinumab; 1-70 mM buffer system; 2-350 mM amino acid component; 5-300 mM tonicifier; 0.0001-5 mM chelator; and a diluent.

B115. 100-350 mg/mL secukinumab; 1-70 mM buffer system; 2-350 mM amino acid component; 5-300 mM ionic strength provider; 0.001-300 mM antioxidant; and a diluent.

B116. 100-350 mg/mL secukinumab; 1-70 mM buffer system; 2-350 mM amino acid component; 5-300 mM ionic strength provider; 0.0001-5 mM chelator; and a diluent.

B117. 100-350 mg/mL secukinumab; 1-70 mM buffer system; 2-350 mM amino acid component; 0.001-300 mM antioxidant; 0.0001-5 mM chelator; and a diluent.

B118. 100-350 mg/mL secukinumab; 1-70 mM buffer system; 0.001-5 mg/mL surfactant; 5-300 mM tonicifier; 5-300 mM ionic strength provider; and a diluent.

B119. 100-350 mg/mL secukinumab; 1-70 mM buffer system; 0.001-5 mg/mL surfactant; 5-300 mM tonicifier; 0.001-300 mM antioxidant; and a diluent.

B120. 100-350 mg/mL secukinumab; 1-70 mM buffer system; 0.001-5 mg/mL surfactant; 5-300 mM tonicifier; 0.0001-5 mM chelator; and a diluent.

B121. 100-350 mg/mL secukinumab; 1-70 mM buffer system; 0.001-5 mg/mL surfactant; 5-300 mM ionic strength provider; 0.001-300 mM antioxidant; and a diluent.

B122. 100-350 mg/mL secukinumab; 1-70 mM buffer system; 0.001-5 mg/mL surfactant; 5-300 mM ionic strength provider; 0.0001-5 mM chelator; and a diluent.

B123. 100-350 mg/mL secukinumab; 1-70 mM buffer system; 0.001-5 mg/mL surfactant; 0.001-300 mM antioxidant; 0.0001-5 mM chelator; and a diluent.

B124. 100-350 mg/mL secukinumab; 1-70 mM buffer system; 5-300 mM tonicifier; 5-300 mM ionic strength provider; 0.001-300 mM antioxidant; and a diluent.

B125. 100-350 mg/mL secukinumab; 1-70 mM buffer system; 5-300 mM tonicifier; 5-300 mM ionic strength provider; 0.0001-5 mM chelator; and a diluent.

B126. 100-350 mg/mL secukinumab; 1-70 mM buffer system; 5-300 mM tonicifier; 0.001-300 mM antioxidant; 0.0001-5 mM chelator; and a diluent.

B127. 100-350 mg/mL secukinumab; 1-70 mM buffer system; 5-300 mM ionic strength provider; 0.001-300 mM antioxidant; 0.0001-5 mM chelator; and a diluent.

B128. 100-350 mg/mL secukinumab; 30-400 mM sugar component; 2-350 mM amino acid component; 0.001-5 mg/mL surfactant; 5-300 mM tonicifier; and a diluent.

B129. 100-350 mg/mL secukinumab; 30-400 mM sugar component; 2-350 mM amino acid component; 0.001-5 mg/mL surfactant; 5-300 mM ionic strength provider; and a diluent.

B130. 100-350 mg/mL secukinumab; 30-400 mM sugar component; 2-350 mM amino acid component; 0.001-5 mg/mL surfactant; 0.001-300 mM antioxidant; and a diluent.

B131. 100-350 mg/mL secukinumab; 30-400 mM sugar component; 2-350 mM amino acid component; 0.001-5 mg/mL surfactant; 0.0001-5 mM chelator; and a diluent.

B132. 100-350 mg/mL secukinumab; 30-400 mM sugar component; 2-350 mM amino acid component; 5-300 mM tonicifier; 5-300 mM ionic strength provider; and a diluent.

(continued)

B133. 100-350 mg/mL secukinumab; 30-400 mM sugar component; 2-350 mM amino acid component; 5-300 mM tonicifier; 0.001-300 mM antioxidant; and a diluent.

B134. 100-350 mg/mL secukinumab; 30-400 mM sugar component; 2-350 mM amino acid component; 5-300 mM tonicifier; 0.0001-5 mM chelator; and a diluent.

B135. 100-350 mg/mL secukinumab; 30-400 mM sugar component; 2-350 mM amino acid component; 5-300 mM ionic strength provider; 0.001-300 mM antioxidant; and a diluent.

B136. 100-350 mg/mL secukinumab; 30-400 mM sugar component; 2-350 mM amino acid component; 5-300 mM ionic strength provider; 0.0001-5 mM chelator; and a diluent.

B137. 100-350 mg/mL secukinumab; 30-400 mM sugar component; 2-350 mM amino acid component; 0.001-300 mM antioxidant; 0.0001-5 mM chelator; and a diluent.

B138. 100-350 mg/mL secukinumab; 30-400 mM sugar component; 0.001-5 mg/mL surfactant; 5-300 mM tonicifier; 5-300 mM ionic strength provider; and a diluent.

B139. 100-350 mg/mL secukinumab; 30-400 mM sugar component; 0.001-5 mg/mL surfactant; 5-300 mM tonicifier; 0.001-300 mM antioxidant; and a diluent.

B140. 100-350 mg/mL secukinumab; 30-400 mM sugar component; 0.001-5 mg/mL surfactant; 5-300 mM tonicifier; 0.0001-5 mM chelator; and a diluent.

B141. 100-350 mg/mL secukinumab; 30-400 mM sugar component; 0.001-5 mg/mL surfactant; 5-300 mM ionic strength provider; 0.001-300 mM antioxidant; and a diluent.

B142. 100-350 mg/mL secukinumab; 30-400 mM sugar component; 0.001-5 mg/mL surfactant; 5-300 mM ionic strength provider; 0.0001-5 mM chelator; and a diluent.

B143. 100-350 mg/mL secukinumab; 30-400 mM sugar component; 0.001-5 mg/mL surfactant; 0.001-300 mM antioxidant; 0.0001-5 mM chelator; and a diluent.

B144. 100-350 mg/mL secukinumab; 30-400 mM sugar component; 5-300 mM tonicifier; 5-300 mM ionic strength provider; 0.001-300 mM antioxidant; and a diluent.

B145. 100-350 mg/mL secukinumab; 30-400 mM sugar component; 5-300 mM tonicifier; 5-300 mM ionic strength provider; 0.0001-5 mM chelator; and a diluent.

B146. 100-350 mg/mL secukinumab; 30-400 mM sugar component; 5-300 mM tonicifier; 0.001-300 mM antioxidant; 0.0001-5 mM chelator; and a diluent.

B147. 100-350 mg/mL secukinumab; 30-400 mM sugar component; 5-300 mM ionic strength provider; 0.001-300 mM antioxidant; 0.0001-5 mM chelator; and a diluent.

B148. 100-350 mg/mL secukinumab; 2-350 mM amino acid component; 0.001-5 mg/mL surfactant; 5-300 mM tonicifier; 5-300 mM ionic strength provider; and a diluent.

B149. 100-350 mg/mL secukinumab; 2-350 mM amino acid component; 0.001-5 mg/mL surfactant; 5-300 mM tonicifier; 0.001-300 mM antioxidant; and a diluent.

B150. 100-350 mg/mL secukinumab; 2-350 mM amino acid component; 0.001-5 mg/mL surfactant; 5-300 mM tonicifier; 0.0001-5 mM chelator; and a diluent.

B151. 100-350 mg/mL secukinumab; 2-350 mM amino acid component; 0.001-5 mg/mL surfactant; 5-300 mM ionic strength provider; 0.001-300 mM antioxidant; and a diluent.

B152. 100-350 mg/mL secukinumab; 2-350 mM amino acid component; 0.001-5 mg/mL surfactant; 5-300 mM ionic strength provider; 0.0001-5 mM chelator; and a diluent.

B153. 100-350 mg/mL secukinumab; 2-350 mM amino acid component; 0.001-5 mg/mL surfactant; 0.001-300 mM antioxidant; 0.0001-5 mM chelator; and a diluent.

B154. 100-350 mg/mL secukinumab; 2-350 mM amino acid component; 5-300 mM tonicifier; 5-300 mM ionic strength provider; 0.001-300 mM antioxidant; and a diluent.

B155. 100-350 mg/mL secukinumab; 2-350 mM amino acid component; 5-300 mM tonicifier; 5-300 mM ionic strength provider; 0.0001-5 mM chelator; and a diluent.

B156. 100-350 mg/mL secukinumab; 2-350 mM amino acid component; 5-300 mM tonicifier; 0.001-300 mM antioxidant; 0.0001-5 mM chelator; and a diluent.

B157. 100-350 mg/mL secukinumab; 2-350 mM amino acid component; 5-300 mM ionic strength provider; 0.001-300 mM antioxidant; 0.0001-5 mM chelator; and a diluent.

B158. 100-350 mg/mL secukinumab; 0.001-5 mg/mL surfactant; 5-300 mM tonicifier; 5-300 mM ionic strength provider; 0.001-300 mM antioxidant; and a diluent.

(continued)

B159. 100-350 mg/mL secukinumab; 0.001-5 mg/mL surfactant; 5-300 mM tonicifier; 5-300 mM ionic strength provider; 0.0001-5 mM chelator; and a diluent.

B160. 100-350 mg/mL secukinumab; 0.001-5 mg/mL surfactant; 5-300 mM tonicifier; 0.001-300 mM antioxidant; 0.0001-5 mM chelator; and a diluent.

B161. 100-350 mg/mL secukinumab; 0.001-5 mg/mL surfactant; 5-300 mM ionic strength provider; 0.001-300 mM antioxidant; 0.0001-5 mM chelator; and a diluent.

B162. 100-350 mg/mL secukinumab; 5-300 mM tonicifier; 5-300 mM ionic strength provider; 0.001-300 mM antioxidant; 0.0001-5 mM chelator; and a diluent.

B163. 100-350 mg/mL secukinumab; 1-70 mM buffer system; 30-400 mM sugar component; 2-350 mM amino acid component; 0.001-5 mg/mL surfactant; 5-300 mM tonicifier; and a diluent.

B164. 100-350 mg/mL secukinumab; 1-70 mM buffer system; 30-400 mM sugar component; 2-350 mM amino acid component; 0.001-5 mg/mL surfactant; 5-300 mM ionic strength provider; and a diluent.

B165. 100-350 mg/mL secukinumab; 1-70 mM buffer system; 30-400 mM sugar component; 2-350 mM amino acid component; 0.001-5 mg/mL surfactant; 0.001-300 mM antioxidant; and a diluent.

B166. 100-350 mg/mL secukinumab; 1-70 mM buffer system; 30-400 mM sugar component; 2-350 mM amino acid component; 0.001-5 mg/mL surfactant; 0.0001-5 mM chelator; and a diluent.

B167. 100-350 mg/mL secukinumab; 1-70 mM buffer system; 30-400 mM sugar component; 2-350 mM amino acid component; 5-300 mM tonicifier; 5-300 mM ionic strength provider; and a diluent.

B168. 100-350 mg/mL secukinumab; 1-70 mM buffer system; 30-400 mM sugar component; 2-350 mM amino acid component; 5-300 mM tonicifier; 0.001-300 mM antioxidant; and a diluent.

B169. 100-350 mg/mL secukinumab; 1-70 mM buffer system; 30-400 mM sugar component; 2-350 mM amino acid component; 5-300 mM tonicifier; 0.0001-5 mM chelator; and a diluent.

B170. 100-350 mg/mL secukinumab; 1-70 mM buffer system; 30-400 mM sugar component; 2-350 mM amino acid component; 5-300 mM ionic strength provider; 0.001-300 mM antioxidant; and a diluent.

B171. 100-350 mg/mL secukinumab; 1-70 mM buffer system; 30-400 mM sugar component; 2-350 mM amino acid component; 5-300 mM ionic strength provider; 0.0001-5 mM chelator; and a diluent.

B172. 100-350 mg/mL secukinumab; 1-70 mM buffer system; 30-400 mM sugar component; 2-350 mM amino acid component; 0.001-300 mM antioxidant; 0.0001-5 mM chelator; and a diluent.

B173. 100-350 mg/mL secukinumab; 1-70 mM buffer system; 30-400 mM sugar component; 0.001-5 mg/mL surfactant; 5-300 mM tonicifier; 5-300 mM ionic strength provider; and a diluent.

B174. 100-350 mg/mL secukinumab; 1-70 mM buffer system; 30-400 mM sugar component; 0.001-5 mg/mL surfactant; 5-300 mM tonicifier; 0.001-300 mM antioxidant; and a diluent.

B175. 100-350 mg/mL secukinumab; 1-70 mM buffer system; 30-400 mM sugar component; 0.001-5 mg/mL surfactant; 5-300 mM tonicifier; 0.0001-5 mM chelator; and a diluent.

B176. 100-350 mg/mL secukinumab; 1-70 mM buffer system; 30-400 mM sugar component; 0.001-5 mg/mL surfactant; 5-300 mM ionic strength provider; 0.001-300 mM antioxidant; and a diluent.

B177. 100-350 mg/mL secukinumab; 1-70 mM buffer system; 30-400 mM sugar component; 0.001-5 mg/mL surfactant; 5-300 mM ionic strength provider; 0.0001-5 mM chelator; and a diluent.

B178. 100-350 mg/mL secukinumab; 1-70 mM buffer system; 30-400 mM sugar component; 0.001-5 mg/mL surfactant; 0.001-300 mM antioxidant; 0.0001-5 mM chelator; and a diluent.

B179. 100-350 mg/mL secukinumab; 1-70 mM buffer system; 30-400 mM sugar component; 5-300 mM tonicifier; 5-300 mM ionic strength provider; 0.001-300 mM antioxidant; and a diluent.

B180. 100-350 mg/mL secukinumab; 1-70 mM buffer system; 30-400 mM sugar component; 5-300 mM tonicifier; 5-300 mM ionic strength provider; 0.0001-5 mM chelator; and a diluent.

B181. 100-350 mg/mL secukinumab; 1-70 mM buffer system; 30-400 mM sugar component; 5-300 mM tonicifier; 0.001-300 mM antioxidant; 0.0001-5 mM chelator; and a diluent.

B182. 100-350 mg/mL secukinumab; 1-70 mM buffer system; 30-400 mM sugar component; 5-300 mM ionic strength provider; 0.001-300 mM antioxidant; 0.0001-5 mM chelator; and a diluent.

B183. 100-350 mg/mL secukinumab; 1-70 mM buffer system; 2-350 mM amino acid component; 0.001-5 mg/mL surfactant; 5-300 mM tonicifier; 5-300 mM ionic strength provider; and a diluent.

B184. 100-350 mg/mL secukinumab; 1-70 mM buffer system; 2-350 mM amino acid component; 0.001-5 mg/mL surfactant; 5-300 mM tonicifier; 0.001-300 mM antioxidant; and a diluent.

(continued)

B185. 100-350 mg/mL secukinumab; 1-70 mM buffer system; 2-350 mM amino acid component; 0.001-5 mg/mL surfactant; 5-300 mM tonicifier; 0.0001-5 mM chelator; and a diluent.

B186. 100-350 mg/mL secukinumab; 1-70 mM buffer system; 2-350 mM amino acid component; 0.001-5 mg/mL surfactant; 5-300 mM ionic strength provider; 0.001-300 mM antioxidant; and a diluent.

B187. 100-350 mg/mL secukinumab; 1-70 mM buffer system; 2-350 mM amino acid component; 0.001-5 mg/mL surfactant; 5-300 mM ionic strength provider; 0.0001-5 mM chelator; and a diluent.

B188. 100-350 mg/mL secukinumab; 1-70 mM buffer system; 2-350 mM amino acid component; 0.001 -5 mg/mL surfactant; 0.001-300 mM antioxidant; 0.0001-5 mM chelator; and a diluent.

B189. 100-350 mg/mL secukinumab; 1-70 mM buffer system; 2-350 mM amino acid component; 5-300 mM tonicifier; 5-300 mM ionic strength provider; 0.001-300 mM antioxidant; and a diluent.

B190. 100-350 mg/mL secukinumab; 1-70 mM buffer system; 2-350 mM amino acid component; 5-300 mM tonicifier; 5-300 mM ionic strength provider; 0.0001-5 mM chelator; and a diluent.

B191. 100-350 mg/mL secukinumab; 1-70 mM buffer system; 2-350 mM amino acid component; 5-300 mM tonicifier; 0.001-300 mM antioxidant; 0.0001-5 mM chelator; and a diluent.

B192. 100-350 mg/mL secukinumab; 1-70 mM buffer system; 2-350 mM amino acid component; 5-300 mM ionic strength provider; 0.001-300 mM antioxidant; 0.0001-5 mM chelator; and a diluent.

B193. 100-350 mg/mL secukinumab; 1-70 mM buffer system; 0.001-5 mg/mL surfactant; 5-300 mM tonicifier; 5-300 mM ionic strength provider; 0.001-300 mM antioxidant; and a diluent.

B194. 100-350 mg/mL secukinumab; 1-70 mM buffer system; 0.001-5 mg/mL surfactant; 5-300 mM tonicifier; 5-300 mM ionic strength provider; 0.0001-5 mM chelator; and a diluent.

B195. 100-350 mg/mL secukinumab; 1-70 mM buffer system; 0.001-5 mg/mL surfactant; 5-300 mM tonicifier; 0.001-300 mM antioxidant; 0.0001-5 mM chelator; and a diluent.

B196. 100-350 mg/mL secukinumab; 1-70 mM buffer system; 0.001-5 mg/mL surfactant; 5-300 mM ionic strength provider; 0.001-300 mM antioxidant; 0.0001-5 mM chelator; and a diluent.

B197. 100-350 mg/mL secukinumab; 1-70 mM buffer system; 5-300 mM tonicifier; 5-300 mM ionic strength provider; 0.001-300 mM antioxidant; 0.0001-5 mM chelator; and a diluent.

B198. 100-350 mg/mL secukinumab; 30-400 mM sugar component; 2-350 mM amino acid component; 0.001-5 mg/mL surfactant; 5-300 mM tonicifier; 5-300 mM ionic strength provider; and a diluent.

B199. 100-350 mg/mL secukinumab; 30-400 mM sugar component; 2-350 mM amino acid component; 0.001-5 mg/mL surfactant; 5-300 mM tonicifier; 0.001-300 mM antioxidant; and a diluent.

B200. 100-350 mg/mL secukinumab; 30-400 mM sugar component; 2-350 mM amino acid component; 0.001-5 mg/mL surfactant; 5-300 mM tonicifier; 0.0001-5 mM chelator; and a diluent.

B201. 100-350 mg/mL secukinumab; 30-400 mM sugar component; 2-350 mM amino acid component; 0.001-5 mg/mL surfactant; 5-300 mM ionic strength provider; 0.001-300 mM antioxidant; and a diluent.

B202. 100-350 mg/mL secukinumab; 30-400 mM sugar component; 2-350 mM amino acid component; 0.001-5 mg/mL surfactant; 5-300 mM ionic strength provider; 0.0001-5 mM chelator; and a diluent.

B203. 100-350 mg/mL secukinumab; 30-400 mM sugar component; 2-350 mM amino acid component; 0.001-5 mg/mL surfactant; 0.001-300 mM antioxidant; 0.0001-5 mM chelator; and a diluent.

B204. 100-350 mg/mL secukinumab; 30-400 mM sugar component; 2-350 mM amino acid component; 5-300 mM tonicifier; 5-300 mM ionic strength provider; 0.001-300 mM antioxidant; and a diluent.

B205. 100-350 mg/mL secukinumab; 30-400 mM sugar component; 2-350 mM amino acid component; 5-300 mM tonicifier; 5-300 mM ionic strength provider; 0.0001-5 mM chelator; and a diluent.

B206. 100-350 mg/mL secukinumab; 30-400 mM sugar component; 2-350 mM amino acid component; 5-300 mM tonicifier; 0.001-300 mM antioxidant; 0.0001-5 mM chelator; and a diluent.

B207. 100-350 mg/mL secukinumab; 30-400 mM sugar component; 2-350 mM amino acid component; 5-300 mM ionic strength provider; 0.001-300 mM antioxidant; 0.0001-5 mM chelator; and a diluent.

B208. 100-350 mg/mL secukinumab; 30-400 mM sugar component; 0.001-5 mg/mL surfactant; 5-300 mM tonicifier; 5-300 mM ionic strength provider; 0.001-300 mM antioxidant; and a diluent.

B209. 100-350 mg/mL secukinumab; 30-400 mM sugar component; 0.001-5 mg/mL surfactant; 5-300 mM tonicifier; 5-300 mM ionic strength provider; 0.0001-5 mM chelator; and a diluent.

B210. 100-350 mg/mL secukinumab; 30-400 mM sugar component; 0.001-5 mg/mL surfactant; 5-300 mM tonicifier; 0.001-300 mM antioxidant; 0.0001-5 mM chelator; and a diluent.

(continued)

B211. 100-350 mg/mL secukinumab; 30-400 mM sugar component; 0.001-5 mg/mL surfactant; 5-300 mM ionic strength provider; 0.001-300 mM antioxidant; 0.0001-5 mM chelator; and a diluent.

B212. 100-350 mg/mL secukinumab; 30-400 mM sugar component; 5-300 mM tonicifier; 5-300 mM ionic strength provider; 0.001-300 mM antioxidant; 0.0001-5 mM chelator; and a diluent.

B213. 100-350 mg/mL secukinumab; 2-350 mM amino acid component; 0.001-5 mg/mL surfactant; 5-300 mM tonicifier; 5-300 mM ionic strength provider; 0.001-300 mM antioxidant; and a diluent.

B214. 100-350 mg/mL secukinumab; 2-350 mM amino acid component; 0.001-5 mg/mL surfactant; 5-300 mM tonicifier; 5-300 mM ionic strength provider; 0.0001-5 mM chelator; and a diluent.

B215. 100-350 mg/mL secukinumab; 2-350 mM amino acid component; 0.001-5 mg/mL surfactant; 5-300 mM tonicifier; 0.001-300 mM antioxidant; 0.0001-5 mM chelator; and a diluent.

B216. 100-350 mg/mL secukinumab; 2-350 mM amino acid component; 0.001-5 mg/mL surfactant; 5-300 mM ionic strength provider; 0.001-300 mM antioxidant; 0.0001-5 mM chelator; and a diluent.

B217. 100-350 mg/mL secukinumab; 2-350 mM amino acid component; 5-300 mM tonicifier; 5-300 mM ionic strength provider; 0.001-300 mM antioxidant; 0.0001-5 mM chelator; and a diluent.

B218. 100-350 mg/mL secukinumab; 0.001-5 mg/mL surfactant; 5-300 mM tonicifier; 5-300 mM ionic strength provider; 0.001-300 mM antioxidant; 0.0001-5 mM chelator; and a diluent.

B219. 100-350 mg/mL secukinumab; 1-70 mM buffer system; 30-400 mM sugar component; 2-350 mM amino acid component; 0.001-5 mg/mL surfactant; 5-300 mM tonicifier; 5-300 mM ionic strength provider; and a diluent.

B220. 100-350 mg/mL secukinumab; 1-70 mM buffer system; 30-400 mM sugar component; 2-350 mM amino acid component; 0.001-5 mg/mL surfactant; 5-300 mM tonicifier; 0.001-300 mM antioxidant; and a diluent.

B221. 100-350 mg/mL secukinumab; 1-70 mM buffer system; 30-400 mM sugar component; 2-350 mM amino acid component; 0.001-5 mg/mL surfactant; 5-300 mM tonicifier; 0.0001-5 mM chelator; and a diluent.

B222. 100-350 mg/mL secukinumab; 1-70 mM buffer system; 30-400 mM sugar component; 2-350 mM amino acid component; 0.001-5 mg/mL surfactant; 5-300 mM ionic strength provider; 0.001-300 mM antioxidant; and a diluent.

B223. 100-350 mg/mL secukinumab; 1-70 mM buffer system; 30-400 mM sugar component; 2-350 mM amino acid component; 0.001-5 mg/mL surfactant; 5-300 mM ionic strength provider; 0.0001-5 mM chelator; and a diluent.

B224. 100-350 mg/mL secukinumab; 1-70 mM buffer system; 30-400 mM sugar component; 2-350 mM amino acid component; 0.001-5 mg/mL surfactant; 0.001-300 mM antioxidant; 0.0001-5 mM chelator; and a diluent.

B225. 100-350 mg/mL secukinumab; 1-70 mM buffer system; 30-400 mM sugar component; 2-350 mM amino acid component; 5-300 mM tonicifier; 5-300 mM ionic strength provider; 0.001-300 mM antioxidant; and a diluent.

B226. 100-350 mg/mL secukinumab; 1-70 mM buffer system; 30-400 mM sugar component; 2-350 mM amino acid component; 5-300 mM tonicifier; 5-300 mM ionic strength provider; 0.0001-5 mM chelator; and a diluent.

B227. 100-350 mg/mL secukinumab; 1-70 mM buffer system; 30-400 mM sugar component; 2-350 mM amino acid component; 5-300 mM tonicifier; 0.001-300 mM antioxidant; 0.0001-5 mM chelator; and a diluent.

B228. 100-350 mg/mL secukinumab; 1-70 mM buffer system; 30-400 mM sugar component; 2-350 mM amino acid component; 5-300 mM ionic strength provider; 0.001-300 mM antioxidant; 0.0001-5 mM chelator; and a diluent.

B229. 100-350 mg/mL secukinumab; 1-70 mM buffer system; 30-400 mM sugar component; 0.001-5 mg/mL surfactant; 5-300 mM tonicifier; 5-300 mM ionic strength provider; 0.001-300 mM antioxidant; and a diluent.

B230. 100-350 mg/mL secukinumab; 1-70 mM buffer system; 30-400 mM sugar component; 0.001-5 mg/mL surfactant; 5-300 mM tonicifier; 5-300 mM ionic strength provider; 0.0001-5 mM chelator; and a diluent.

B231. 100-350 mg/mL secukinumab; 1-70 mM buffer system; 30-400 mM sugar component; 0.001-5 mg/mL surfactant; 5-300 mM tonicifier; 0.001-300 mM antioxidant; 0.0001-5 mM chelator; and a diluent.

B232. 100-350 mg/mL secukinumab; 1-70 mM buffer system; 30-400 mM sugar component; 0.001-5 mg/mL surfactant; 5-300 mM ionic strength provider; 0.001-300 mM antioxidant; 0.0001-5 mM chelator; and a diluent.

B233. 100-350 mg/mL secukinumab; 1-70 mM buffer system; 30-400 mM sugar component; 5-300 mM tonicifier; 5-300 mM ionic strength provider; 0.001-300 mM antioxidant; 0.0001-5 mM chelator; and a diluent.

(continued)

B234. 100-350 mg/mL secukinumab; 1-70 mM buffer system; 2-350 mM amino acid component; 0.001-5 mg/mL surfactant; 5-300 mM tonicifier; 5-300 mM ionic strength provider; 0.001-300 mM antioxidant; and a diluent.

B235. 100-350 mg/mL secukinumab; 1-70 mM buffer system; 2-350 mM amino acid component; 0.001-5 mg/mL surfactant; 5-300 mM tonicifier; 5-300 mM ionic strength provider; 0.0001-5 mM chelator; and a diluent.

B236. 100-350 mg/mL secukinumab; 1-70 mM buffer system; 2-350 mM amino acid component; 0.001-5 mg/mL surfactant; 5-300 mM tonicifier; 0.001-300 mM antioxidant; 0.0001-5 mM chelator; and a diluent.

B237. 100-350 mg/mL secukinumab; 1-70 mM buffer system; 2-350 mM amino acid component; 0.001-5 mg/mL surfactant; 5-300 mM ionic strength provider; 0.001-300 mM antioxidant; 0.0001-5 mM chelator; and a diluent.

B238. 100-350 mg/mL secukinumab; 1-70 mM buffer system; 2-350 mM amino acid component; 5-300 mM tonicifier; 5-300 mM ionic strength provider; 0.001-300 mM antioxidant; 0.0001-5 mM chelator; and a diluent.

B239. 100-350 mg/mL secukinumab; 1-70 mM buffer system; 0.001-5 mg/mL surfactant; 5-300 mM tonicifier; 5-300 mM ionic strength provider; 0.001-300 mM antioxidant; 0.0001-5 mM chelator; and a diluent.

B240. 100-350 mg/mL secukinumab; 30-400 mM sugar component; 2-350 mM amino acid component; 0.001-5 mg/mL surfactant; 5-300 mM tonicifier; 5-300 mM ionic strength provider; 0.001-300 mM antioxidant; and a diluent.

B241. 100-350 mg/mL secukinumab; 30-400 mM sugar component; 2-350 mM amino acid component; 0.001-5 mg/mL surfactant; 5-300 mM tonicifier; 5-300 mM ionic strength provider; 0.0001-5 mM chelator; and a diluent.

B242. 100-350 mg/mL secukinumab; 30-400 mM sugar component; 2-350 mM amino acid component; 0.001-5 mg/mL surfactant; 5-300 mM tonicifier; 0.001-300 mM antioxidant; 0.0001-5 mM chelator; and a diluent.

B243. 100-350 mg/mL secukinumab; 30-400 mM sugar component; 2-350 mM amino acid component; 0.001-5 mg/mL surfactant; 5-300 mM ionic strength provider; 0.001-300 mM antioxidant; 0.0001-5 mM chelator; and a diluent.

B244. 100-350 mg/mL secukinumab; 30-400 mM sugar component; 2-350 mM amino acid component; 5-300 mM tonicifier; 5-300 mM ionic strength provider; 0.001-300 mM antioxidant; 0.0001-5 mM chelator; and a diluent.

B245. 100-350 mg/mL secukinumab; 30-400 mM sugar component; 0.001-5 mg/mL surfactant; 5-300 mM tonicifier; 5-300 mM ionic strength provider; 0.001-300 mM antioxidant; 0.0001-5 mM chelator; and a diluent.

B246. 100-350 mg/mL secukinumab; 2-350 mM amino acid component; 0.001-5 mg/mL surfactant; 5-300 mM tonicifier; 5-300 mM ionic strength provider; 0.001-300 mM antioxidant; 0.0001-5 mM chelator; and a diluent.

B247. 100-350 mg/mL secukinumab; 1-70 mM buffer system; 30-400 mM sugar component; 2-350 mM amino acid component; 0.001-5 mg/mL surfactant; 5-300 mM tonicifier; 5-300 mM ionic strength provider; 0.001-300 mM antioxidant; and a diluent.

B248. 100-350 mg/mL secukinumab; 1-70 mM buffer system; 30-400 mM sugar component; 2-350 mM amino acid component; 0.001-5 mg/mL surfactant; 5-300 mM tonicifier; 5-300 mM ionic strength provider; 0.0001-5 mM chelator; and a diluent.

B249. 100-350 mg/mL secukinumab; 1-70 mM buffer system; 30-400 mM sugar component; 2-350 mM amino acid component; 0.001-5 mg/mL surfactant; 5-300 mM tonicifier; 0.001-300 mM antioxidant; 0.0001-5 mM chelator; and a diluent.

B250. 100-350 mg/mL secukinumab; 1-70 mM buffer system; 30-400 mM sugar component; 2-350 mM amino acid component; 0.001-5 mg/mL surfactant; 5-300 mM ionic strength provider; 0.001-300 mM antioxidant; 0.0001-5 mM chelator; and a diluent.

B251. 100-350 mg/mL secukinumab; 1-70 mM buffer system; 30-400 mM sugar component; 2-350 mM amino acid component; 5-300 mM tonicifier; 5-300 mM ionic strength provider; 0.001-300 mM antioxidant; 0.0001-5 mM chelator; and a diluent.

B252. 100-350 mg/mL secukinumab; 1-70 mM buffer system; 30-400 mM sugar component; 0.001-5 mg/mL surfactant; 5-300 mM tonicifier; 5-300 mM ionic strength provider; 0.001-300 mM antioxidant; 0.0001-5 mM chelator; and a diluent.

B253. 100-350 mg/mL secukinumab; 1-70 mM buffer system; 2-350 mM amino acid component; 0.001-5 mg/mL surfactant; 5-300 mM tonicifier; 5-300 mM ionic strength provider; 0.001-300 mM antioxidant; 0.0001-5 mM chelator; and a diluent.

B254. 100-350 mg/mL secukinumab; 30-400 mM sugar component; 2-350 mM amino acid component; 0.001-5 mg/mL surfactant; 5-300 mM tonicifier; 5-300 mM ionic strength provider; 0.001-300 mM antioxidant; 0.0001-5 mM chelator; and a diluent.

(continued)

B255. 100-350 mg/mL secukinumab; 1-70 mM buffer system; 30-400 mM sugar component; 2-350 mM amino acid component; 0.001-5 mg/mL surfactant; 5-300 mM tonicifier; 5-300 mM ionic strength provider; 0.001-300 mM antioxidant; 0.0001-5 mM chelator; and a diluent.

The embodiments of B1-B255 may be further defined or characterised by features of numbered paragraphs C1-C255, D1-D1152, E1-E1151, F1-F12, G1-G959, H1-H1423, 11-12400, and J1-J1158.

*Embodiments of Combinations of Ingredients Quantified by Broad Molar Ratios*

C) General Aspects and Embodiments Relating to Combinations of Ingredients Quantified by Broad Molar Ratios

The biopharmaceutical composition suitably comprises, or consists of (suitably along with a diluent, which is preferably water), secukinumab alongside one or more (preferably two or more) of a buffer system, a sugar component, an amino acid component, a surfactant, an antioxidant, a tonicifier, an ionic strength provider, and/or a chelator, in a respective molar ratio of 0.135-2.70 : 1-70 : 30-400 : 2-350 : 0.0008-3.82 : 0.001-300 : 5-300 : 5-300 : 0.0001-5. The following numbered paragraphs C1-C255 disclose specific aspects and embodiments of the invention, wherein the biopharmaceutical composition comprises, consists of (suitably along with a diluent, which is preferably water), the following components in molar ratios as numerically specified:

C1. 0.135-2.70 secukinumab : 1-70 buffer system.
C2. 0.135-2.70 secukinumab : 30-400 sugar component.
C3. 0.135-2.70 secukinumab : 2-350 amino acid component.
C4. 0.135-2.70 secukinumab : 0.0008-3.82 surfactant.
C5. 0.135-2.70 secukinumab : 0.001-300 antioxidant.
C6. 0.135-2.70 secukinumab : 5-300 tonicifier.
C7. 0.135-2.70 secukinumab : 5-300 ionic strength provider.
C8. 0.135-2.70 secukinumab : 0.0001-5 chelator.
C9. 0.135-2.70 secukinumab : 1-70 buffer system : 30-400 sugar component.
C10. 0.135-2.70 secukinumab : 1-70 buffer system : 2-350 amino acid component.
C11. 0.135-2.70 secukinumab : 1-70 buffer system : 0.0008-3.82 surfactant.
C12. 0.135-2.70 secukinumab : 1-70 buffer system : 0.001-300 antioxidant.
C13. 0.135-2.70 secukinumab : 1-70 buffer system : 5-300 tonicifier.
C14. 0.135-2.70 secukinumab : 1-70 buffer system : 5-300 ionic strength provider.
C15. 0.135-2.70 secukinumab : 1-70 buffer system : 0.0001-5 chelator.
C16. 0.135-2.70 secukinumab : 30-400 sugar component : 2-350 amino acid component.
C17. 0.135-2.70 secukinumab : 30-400 sugar component : 0.0008-3.82 surfactant.
C18. 0.135-2.70 secukinumab : 30-400 sugar component : 0.001-300 antioxidant.
C19. 0.135-2.70 secukinumab : 30-400 sugar component : 5-300 tonicifier.
C20. 0.135-2.70 secukinumab : 30-400 sugar component : 5-300 ionic strength provider.
C21. 0.135-2.70 secukinumab : 30-400 sugar component : 0.0001-5 chelator.
C22. 0.135-2.70 secukinumab : 2-350 amino acid component : 0.0008-3.82 surfactant.
C23. 0.135-2.70 secukinumab : 2-350 amino acid component : 0.001-300 antioxidant.
C24. 0.135-2.70 secukinumab : 2-350 amino acid component : 5-300 tonicifier.
C25. 0.135-2.70 secukinumab : 2-350 amino acid component : 5-300 ionic strength provider.
C26. 0.135-2.70 secukinumab : 2-350 amino acid component : 0.0001-5 chelator.
C27. 0.135-2.70 secukinumab : 0.0008-3.82 surfactant : 0.001-300 antioxidant.
C28. 0.135-2.70 secukinumab : 0.0008-3.82 surfactant : 5-300 tonicifier.
C29. 0.135-2.70 secukinumab : 0.0008-3.82 surfactant : 5-300 ionic strength provider.
C30. 0.135-2.70 secukinumab : 0.0008-3.82 surfactant : 0.0001-5 chelator.
C31. 0.135-2.70 secukinumab : 0.001-300 antioxidant : 5-300 tonicifier.
C32. 0.135-2.70 secukinumab : 0.001-300 antioxidant : 5-300 ionic strength provider.
C33. 0.135-2.70 secukinumab : 0.001-300 antioxidant : 0.0001-5 chelator.

(continued)

C34. 0.135-2.70 secukinumab : 5-300 tonicifier : 5-300 ionic strength provider.
C35. 0.135-2.70 secukinumab : 5-300 tonicifier : 0.0001-5 chelator.
C36. 0.135-2.70 secukinumab : 5-300 ionic strength provider : 0.0001-5 chelator.
C37. 0.135-2.70 secukinumab : 1-70 buffer system : 30-400 sugar component : 2-350 amino acid component.
C38. 0.135-2.70 secukinumab : 1-70 buffer system : 30-400 sugar component : 0.0008-3.82 surfactant.
C39. 0.135-2.70 secukinumab : 1-70 buffer system : 30-400 sugar component : 0.001-300 antioxidant.
C40. 0.135-2.70 secukinumab : 1-70 buffer system : 30-400 sugar component : 5-300 tonicifier.
C41. 0.135-2.70 secukinumab : 1-70 buffer system : 30-400 sugar component : 5-300 ionic strength provider.
C42. 0.135-2.70 secukinumab : 1-70 buffer system : 30-400 sugar component : 0.0001-5 chelator.
C43. 0.135-2.70 secukinumab : 1-70 buffer system : 2-350 amino acid component : 0.0008-3.82 surfactant.
C44. 0.135-2.70 secukinumab : 1-70 buffer system : 2-350 amino acid component : 0.001-300 antioxidant.
C45. 0.135-2.70 secukinumab : 1-70 buffer system : 2-350 amino acid component : 5-300 tonicifier.
C46. 0.135-2.70 secukinumab : 1-70 buffer system : 2-350 amino acid component : 5-300 ionic strength provider.
C47. 0.135-2.70 secukinumab : 1-70 buffer system : 2-350 amino acid component : 0.0001-5 chelator.
C48. 0.135-2.70 secukinumab : 1-70 buffer system : 0.0008-3.82 surfactant : 0.001-300 antioxidant.
C49. 0.135-2.70 secukinumab : 1-70 buffer system : 0.0008-3.82 surfactant : 5-300 tonicifier.
C50. 0.135-2.70 secukinumab : 1-70 buffer system : 0.0008-3.82 surfactant : 5-300 ionic strength provider.
C51. 0.135-2.70 secukinumab : 1-70 buffer system : 0.0008-3.82 surfactant : 0.0001-5 chelator.
C52. 0.135-2.70 secukinumab : 1-70 buffer system : 0.001-300 antioxidant : 5-300 tonicifier.
C53. 0.135-2.70 secukinumab : 1-70 buffer system : 0.001-300 antioxidant : 5-300 ionic strength provider.
C54. 0.135-2.70 secukinumab : 1-70 buffer system : 0.001-300 antioxidant : 0.0001-5 chelator.
C55. 0.135-2.70 secukinumab : 1-70 buffer system : 5-300 tonicifier : 5-300 ionic strength provider.
C56. 0.135-2.70 secukinumab : 1-70 buffer system : 5-300 tonicifier : 0.0001-5 chelator.
C57. 0.135-2.70 secukinumab : 1-70 buffer system : 5-300 ionic strength provider : 0.0001-5 chelator.
C58. 0.135-2.70 secukinumab : 30-400 sugar component : 2-350 amino acid component : 0.0008-3.82 surfactant.
C59. 0.135-2.70 secukinumab : 30-400 sugar component : 2-350 amino acid component : 0.001-300 antioxidant.
C60. 0.135-2.70 secukinumab : 30-400 sugar component : 2-350 amino acid component : 5-300 tonicifier.
C61. 0.135-2.70 secukinumab : 30-400 sugar component : 2-350 amino acid component : 5-300 ionic strength provider.
C62. 0.135-2.70 secukinumab : 30-400 sugar component : 2-350 amino acid component : 0.0001-5 chelator.
C63. 0.135-2.70 secukinumab : 30-400 sugar component : 0.0008-3.82 surfactant : 0.001-300 antioxidant.
C64. 0.135-2.70 secukinumab : 30-400 sugar component : 0.0008-3.82 surfactant : 5-300 tonicifier.
C65. 0.135-2.70 secukinumab : 30-400 sugar component: 0.0008-3.82 surfactant: 5-300 ionic strength provider.
C66. 0.135-2.70 secukinumab : 30-400 sugar component: 0.0008-3.82 surfactant: 0.0001-5 chelator.
C67. 0.135-2.70 secukinumab : 30-400 sugar component: 0.001-300 antioxidant: 5-300 tonicifier.
C68. 0.135-2.70 secukinumab : 30-400 sugar component: 0.001-300 antioxidant: 5-300 ionic strength provider.
C69. 0.135-2.70 secukinumab : 30-400 sugar component: 0.001-300 antioxidant: 0.0001-5 chelator.
C70. 0.135-2.70 secukinumab : 30-400 sugar component: 5-300 tonicifier : 5-300 ionic strength provider.
C71. 0.135-2.70 secukinumab : 30-400 sugar component: 5-300 tonicifier : 0.0001-5 chelator.
C72. 0.135-2.70 secukinumab : 30-400 sugar component: 5-300 ionic strength provider: 0.0001-5 chelator.
C73. 0.135-2.70 secukinumab : 2-350 amino acid component: 0.0008-3.82 surfactant: 0.001-300 antioxidant.
C74. 0.135-2.70 secukinumab : 2-350 amino acid component: 0.0008-3.82 surfactant: 5-300 tonicifier.
C75. 0.135-2.70 secukinumab : 2-350 amino acid component: 0.0008-3.82 surfactant: 5-300 ionic strength provider.
C76. 0.135-2.70 secukinumab : 2-350 amino acid component: 0.0008-3.82 surfactant: 0.0001-5 chelator.
C77. 0.135-2.70 secukinumab : 2-350 amino acid component: 0.001-300 antioxidant: 5-300 tonicifier.
C78. 0.135-2.70 secukinumab : 2-350 amino acid component: 0.001-300 antioxidant: 5-300 ionic strength provider.
C79. 0.135-2.70 secukinumab : 2-350 amino acid component: 0.001-300 antioxidant: 0.0001-5 chelator.
C80. 0.135-2.70 secukinumab : 2-350 amino acid component: 5-300 tonicifier : 5-300 ionic strength provider.
C81. 0.135-2.70 secukinumab : 2-350 amino acid component: 5-300 tonicifier : 0.0001-5 chelator.

(continued)

C82. 0.135-2.70 secukinumab : 2-350 amino acid component: 5-300 ionic strength provider: 0.0001-5 chelator.
C83. 0.135-2.70 secukinumab : 0.0008-3.82 surfactant: 0.001-300 antioxidant: 5-300 tonicifier.
C84. 0.135-2.70 secukinumab : 0.0008-3.82 surfactant: 0.001-300 antioxidant: 5-300 ionic strength provider.
C85. 0.135-2.70 secukinumab : 0.0008-3.82 surfactant: 0.001-300 antioxidant: 0.0001-5 chelator.
C86. 0.135-2.70 secukinumab : 0.0008-3.82 surfactant: 5-300 tonicifier : 5-300 ionic strength provider.
C87. 0.135-2.70 secukinumab : 0.0008-3.82 surfactant: 5-300 tonicifier : 0.0001-5 chelator.
C88. 0.135-2.70 secukinumab : 0.0008-3.82 surfactant: 5-300 ionic strength provider: 0.0001-5 chelator.
C89. 0.135-2.70 secukinumab : 0.001-300 antioxidant: 5-300 tonicifier : 5-300 ionic strength provider.
C90. 0.135-2.70 secukinumab : 0.001-300 antioxidant: 5-300 tonicifier : 0.0001-5 chelator.
C91. 0.135-2.70 secukinumab : 0.001-300 antioxidant: 5-300 ionic strength provider: 0.0001-5 chelator.
C92. 0.135-2.70 secukinumab : 5-300 tonicifier : 5-300 ionic strength provider: 0.0001-5 chelator.
C93. 0.135-2.70 secukinumab : 1-70 buffer system : 30-400 sugar component : 2-350 amino acid component : 0.0008-3.82 surfactant.
C94. 0.135-2.70 secukinumab : 1-70 buffer system : 30-400 sugar component : 2-350 amino acid component : 0.001-300 antioxidant.
C95. 0.135-2.70 secukinumab : 1-70 buffer system : 30-400 sugar component: 2-350 amino acid component: 5-300 tonicifier.
C96. 0.135-2.70 secukinumab : 1-70 buffer system : 30-400 sugar component : 2-350 amino acid component : 5-300 ionic strength provider.
C97. 0.135-2.70 secukinumab : 1-70 buffer system : 30-400 sugar component: 2-350 amino acid component: 0.0001-5 chelator.
C98. 0.135-2.70 secukinumab : 1-70 buffer system : 30-400 sugar component: 0.0008-3.82 surfactant: 0.001-300 antioxidant.
C99. 0.135-2.70 secukinumab : 1-70 buffer system : 30-400 sugar component: 0.0008-3.82 surfactant: 5-300 tonicifier.
C100. 0.135-2.70 secukinumab : 1-70 buffer system : 30-400 sugar component : 0.0008-3.82 surfactant : 5-300 ionic strength provider.
C101. 0.135-2.70 secukinumab : 1-70 buffer system : 30-400 sugar component: 0.0008-3.82 surfactant: 0.0001-5 chelator.
C102. 0.135-2.70 secukinumab : 1-70 buffer system : 30-400 sugar component: 0.001-300 antioxidant: 5-300 tonicifier.
C103. 0.135-2.70 secukinumab : 1-70 buffer system : 30-400 sugar component : 0.001-300 antioxidant : 5-300 ionic strength provider.
C104. 0.135-2.70 secukinumab : 1-70 buffer system : 30-400 sugar component: 0.001-300 antioxidant: 0.0001-5 chelator.
C105. 0.135-2.70 secukinumab : 1-70 buffer system : 30-400 sugar component: 5-300 tonicifier : 5-300 ionic strength provider.
C106. 0.135-2.70 secukinumab : 1-70 buffer system : 30-400 sugar component: 5-300 tonicifier : 0.0001-5 chelator.
C107. 0.135-2.70 secukinumab : 1-70 buffer system : 30-400 sugar component : 5-300 ionic strength provider : 0.0001-5 chelator.
C108. 0.135-2.70 secukinumab : 1-70 buffer system : 2-350 amino acid component : 0.0008-3.82 surfactant : 0.001-300 antioxidant.
C109. 0.135-2.70 secukinumab : 1-70 buffer system : 2-350 amino acid component : 0.0008-3.82 surfactant : 5-300 tonicifier.
C110. 0.135-2.70 secukinumab : 1-70 buffer system : 2-350 amino acid component : 0.0008-3.82 surfactant : 5-300 ionic strength provider.
C111. 0.135-2.70 secukinumab : 1-70 buffer system : 2-350 amino acid component : 0.0008-3.82 surfactant : 0.0001-5 chelator.
C112. 0.135-2.70 secukinumab : 1-70 buffer system : 2-350 amino acid component : 0.001-300 antioxidant : 5-300 tonicifier.
C113. 0.135-2.70 secukinumab : 1-70 buffer system : 2-350 amino acid component : 0.001-300 antioxidant : 5-300 ionic strength provider.

(continued)

C114. 0.135-2.70 secukinumab : 1-70 buffer system : 2-350 amino acid component : 0.001-300 antioxidant : 0.0001-5 chelator.

C115. 0.135-2.70 secukinumab : 1-70 buffer system : 2-350 amino acid component : 5-300 tonicifier : 5-300 ionic strength provider.

C116. 0.135-2.70 secukinumab : 1-70 buffer system : 2-350 amino acid component : 5-300 tonicifier : 0.0001-5 chelator.

C117. 0.135-2.70 secukinumab : 1-70 buffer system : 2-350 amino acid component : 5-300 ionic strength provider : 0.0001-5 chelator.

C118. 0.135-2.70 secukinumab : 1-70 buffer system : 0.0008-3.82 surfactant : 0.001-300 antioxidant : 5-300 tonicifier.

C119. 0.135-2.70 secukinumab : 1-70 buffer system : 0.0008-3.82 surfactant : 0.001-300 antioxidant : 5-300 ionic strength provider.

C120. 0.135-2.70 secukinumab : 1-70 buffer system : 0.0008-3.82 surfactant : 0.001-300 antioxidant : 0.0001-5 chelator.

C121. 0.135-2.70 secukinumab : 1-70 buffer system : 0.0008-3.82 surfactant : 5-300 tonicifier : 5-300 ionic strength provider.

C122. 0.135-2.70 secukinumab : 1-70 buffer system : 0.0008-3.82 surfactant : 5-300 tonicifier : 0.0001-5 chelator.

C123. 0.135-2.70 secukinumab : 1-70 buffer system : 0.0008-3.82 surfactant : 5-300 ionic strength provider : 0.0001-5 chelator.

C124. 0.135-2.70 secukinumab : 1-70 buffer system : 0.001-300 antioxidant : 5-300 tonicifier : 5-300 ionic strength provider.

C125. 0.135-2.70 secukinumab : 1-70 buffer system : 0.001-300 antioxidant : 5-300 tonicifier : 0.0001-5 chelator.

C126. 0.135-2.70 secukinumab : 1-70 buffer system : 0.001-300 antioxidant : 5-300 ionic strength provider : 0.0001-5 chelator.

C127. 0.135-2.70 secukinumab : 1-70 buffer system : 5-300 tonicifier : 5-300 ionic strength provider : 0.0001-5 chelator.

C128. 0.135-2.70 secukinumab : 30-400 sugar component : 2-350 amino acid component : 0.0008-3.82 surfactant : 0.001-300 antioxidant.

C129. 0.135-2.70 secukinumab : 30-400 sugar component : 2-350 amino acid component : 0.0008-3.82 surfactant : 5-300 tonicifier.

C130. 0.135-2.70 secukinumab : 30-400 sugar component: 2-350 amino acid component : 0.0008-3.82 surfactant: 5-300 ionic strength provider.

C131. 0.135-2.70 secukinumab : 30-400 sugar component : 2-350 amino acid component : 0.0008-3.82 surfactant : 0.0001-5 chelator.

C132. 0.135-2.70 secukinumab : 30-400 sugar component : 2-350 amino acid component : 0.001-300 antioxidant : 5-300 tonicifier.

C133. 0.135-2.70 secukinumab : 30-400 sugar component : 2-350 amino acid component : 0.001-300 antioxidant : 5-300 ionic strength provider.

C134. 0.135-2.70 secukinumab : 30-400 sugar component : 2-350 amino acid component : 0.001-300 antioxidant : 0.0001-5 chelator.

C135. 0.135-2.70 secukinumab : 30-400 sugar component : 2-350 amino acid component : 5-300 tonicifier : 5-300 ionic strength provider.

C136. 0.135-2.70 secukinumab : 30-400 sugar component : 2-350 amino acid component : 5-300 tonicifier : 0.0001-5 chelator.

C137. 0.135-2.70 secukinumab : 30-400 sugar component : 2-350 amino acid component : 5-300 ionic strength provider : 0.0001-5 chelator.

C138. 0.135-2.70 secukinumab : 30-400 sugar component : 0.0008-3.82 surfactant : 0.001-300 antioxidant : 5-300 tonicifier.

C139. 0.135-2.70 secukinumab : 30-400 sugar component : 0.0008-3.82 surfactant : 0.001-300 antioxidant : 5-300 ionic strength provider.

C140. 0.135-2.70 secukinumab : 30-400 sugar component : 0.0008-3.82 surfactant : 0.001-300 antioxidant : 0.0001-5 chelator.

(continued)

C141. 0.135-2.70 secukinumab : 30-400 sugar component : 0.0008-3.82 surfactant : 5-300 tonicifier : 5-300 ionic strength provider.

C142. 0.135-2.70 secukinumab : 30-400 sugar component: 0.0008-3.82 surfactant : 5-300 tonicifier : 0.0001-5 chelator.

C143. 0.135-2.70 secukinumab : 30-400 sugar component : 0.0008-3.82 surfactant : 5-300 ionic strength provider : 0.0001-5 chelator.

C144. 0.135-2.70 secukinumab : 30-400 sugar component: 0.001-300 antioxidant : 5-300 tonicifier : 5-300 ionic strength provider.

C145. 0.135-2.70 secukinumab : 30-400 sugar component: 0.001-300 antioxidant : 5-300 tonicifier: 0.0001-5 chelator.

C146. 0.135-2.70 secukinumab : 30-400 sugar component : 0.001-300 antioxidant : 5-300 ionic strength provider : 0.0001-5 chelator.

C147. 0.135-2.70 secukinumab : 30-400 sugar component: 5-300 tonicifier : 5-300 ionic strength provider : 0.0001-5 chelator.

C148. 0.135-2.70 secukinumab : 2-350 amino acid component: 0.0008-3.82 surfactant : 0.001-300 antioxidant : 5-300 tonicifier.

C149. 0.135-2.70 secukinumab : 2-350 amino acid component : 0.0008-3.82 surfactant : 0.001-300 antioxidant : 5-300 ionic strength provider.

C150. 0.135-2.70 secukinumab : 2-350 amino acid component: 0.0008-3.82 surfactant : 0.001-300 antioxidant : 0.0001-5 chelator.

C151. 0.135-2.70 secukinumab : 2-350 amino acid component : 0.0008-3.82 surfactant : 5-300 tonicifier : 5-300 ionic strength provider.

C152. 0.135-2.70 secukinumab : 2-350 amino acid component: 0.0008-3.82 surfactant : 5-300 tonicifier : 0.0001-5 chelator.

C153. 0.135-2.70 secukinumab : 2-350 amino acid component: 0.0008-3.82 surfactant : 5-300 ionic strength provider : 0.0001-5 chelator.

C154. 0.135-2.70 secukinumab : 2-350 amino acid component : 0.001-300 antioxidant : 5-300 tonicifier : 5-300 ionic strength provider.

C155. 0.135-2.70 secukinumab : 2-350 amino acid component: 0.001-300 antioxidant : 5-300 tonicifier : 0.0001-5 chelator.

C156. 0.135-2.70 secukinumab : 2-350 amino acid component: 0.001-300 antioxidant : 5-300 ionic strength provider : 0.0001-5 chelator.

C157. 0.135-2.70 secukinumab : 2-350 amino acid component: 5-300 tonicifier : 5-300 ionic strength provider : 0.0001-5 chelator.

C158. 0.135-2.70 secukinumab : 0.0008-3.82 surfactant: 0.001-300 antioxidant : 5-300 tonicifier : 5-300 ionic strength provider.

C159. 0.135-2.70 secukinumab : 0.0008-3.82 surfactant: 0.001-300 antioxidant : 5-300 tonicifier : 0.0001-5 chelator.

C160. 0.135-2.70 secukinumab : 0.0008-3.82 surfactant: 0.001-300 antioxidant : 5-300 ionic strength provider : 0.0001-5 chelator.

C161. 0.135-2.70 secukinumab : 0.0008-3.82 surfactant: 5-300 tonicifier : 5-300 ionic strength provider : 0.0001-5 chelator.

C162. 0.135-2.70 secukinumab : 0.001-300 antioxidant: 5-300 tonicifier : 5-300 ionic strength provider : 0.0001-5 chelator.

C163. 0.135-2.70 secukinumab : 1-70 buffer system : 30-400 sugar component : 2-350 amino acid component : 0.0008-3.82 surfactant: 0.001-300 antioxidant.

C164. 0.135-2.70 secukinumab : 1-70 buffer system : 30-400 sugar component : 2-350 amino acid component : 0.0008-3.82 surfactant: 5-300 tonicifier.

C165. 0.135-2.70 secukinumab : 1-70 buffer system : 30-400 sugar component : 2-350 amino acid component : 0.0008-3.82 surfactant: 5-300 ionic strength provider.

C166. 0.135-2.70 secukinumab : 1-70 buffer system : 30-400 sugar component : 2-350 amino acid component : 0.0008-3.82 surfactant: 0.0001-5 chelator.

(continued)

C167. 0.135-2.70 secukinumab : 1-70 buffer system : 30-400 sugar component : 2-350 amino acid component : 0.001-300 antioxidant: 5-300 tonicifier.

C168. 0.135-2.70 secukinumab : 1-70 buffer system : 30-400 sugar component : 2-350 amino acid component : 0.001-300 antioxidant: 5-300 ionic strength provider.

C169. 0.135-2.70 secukinumab : 1-70 buffer system : 30-400 sugar component : 2-350 amino acid component : 0.001-300 antioxidant: 0.0001-5 chelator.

C170. 0.135-2.70 secukinumab : 1-70 buffer system : 30-400 sugar component : 2-350 amino acid component: 5-300 tonicifier : 5-300 ionic strength provider.

C171. 0.135-2.70 secukinumab : 1-70 buffer system : 30-400 sugar component : 2-350 amino acid component: 5-300 tonicifier : 0.0001-5 chelator.

C172. 0.135-2.70 secukinumab : 1-70 buffer system : 30-400 sugar component : 2-350 amino acid component : 5-300 ionic strength provider: 0.0001-5 chelator.

C173. 0.135-2.70 secukinumab : 1-70 buffer system : 30-400 sugar component: 0.0008-3.82 surfactant : 0.001-300 antioxidant : 5-300 tonicifier.

C174. 0.135-2.70 secukinumab : 1-70 buffer system : 30-400 sugar component: 0.0008-3.82 surfactant : 0.001-300 antioxidant : 5-300 ionic strength provider.

C175. 0.135-2.70 secukinumab : 1-70 buffer system : 30-400 sugar component: 0.0008-3.82 surfactant : 0.001-300 antioxidant : 0.0001-5 chelator.

C176. 0.135-2.70 secukinumab : 1-70 buffer system : 30-400 sugar component: 0.0008-3.82 surfactant : 5-300 tonicifier : 5-300 ionic strength provider.

C177. 0.135-2.70 secukinumab : 1-70 buffer system : 30-400 sugar component: 0.0008-3.82 surfactant : 5-300 tonicifier : 0.0001-5 chelator.

C178. 0.135-2.70 secukinumab : 1-70 buffer system : 30-400 sugar component : 0.0008-3.82 surfactant : 5-300 ionic strength provider : 0.0001-5 chelator.

C179. 0.135-2.70 secukinumab : 1-70 buffer system : 30-400 sugar component: 0.001-300 antioxidant : 5-300 tonicifier : 5-300 ionic strength provider.

C180. 0.135-2.70 secukinumab : 1-70 buffer system : 30-400 sugar component: 0.001-300 antioxidant : 5-300 tonicifier : 0.0001-5 chelator.

C181. 0.135-2.70 secukinumab : 1-70 buffer system : 30-400 sugar component : 0.001-300 antioxidant : 5-300 ionic strength provider : 0.0001-5 chelator.

C182. 0.135-2.70 secukinumab : 1-70 buffer system : 30-400 sugar component: 5-300 tonicifier : 5-300 ionic strength provider: 0.0001-5 chelator.

C183. 0.135-2.70 secukinumab : 1-70 buffer system : 2-350 amino acid component : 0.0008-3.82 surfactant : 0.001-300 antioxidant: 5-300 tonicifier.

C184. 0.135-2.70 secukinumab : 1-70 buffer system : 2-350 amino acid component : 0.0008-3.82 surfactant : 0.001-300 antioxidant: 5-300 ionic strength provider.

C185. 0.135-2.70 secukinumab : 1-70 buffer system : 2-350 amino acid component : 0.0008-3.82 surfactant : 0.001-300 antioxidant: 0.0001-5 chelator.

C186. 0.135-2.70 secukinumab : 1-70 buffer system : 2-350 amino acid component: 0.0008-3.82 surfactant : 5-300 tonicifier : 5-300 ionic strength provider.

C187. 0.135-2.70 secukinumab : 1-70 buffer system : 2-350 amino acid component : 0.0008-3.82 surfactant : 5-300 tonicifier: 0.0001-5 chelator.

C188. 0.135-2.70 secukinumab : 1-70 buffer system : 2-350 amino acid component: 0.0008-3.82 surfactant : 5-300 ionic strength provider: 0.0001-5 chelator.

C189. 0.135-2.70 secukinumab : 1-70 buffer system : 2-350 amino acid component: 0.001-300 antioxidant : 5-300 tonicifier : 5-300 ionic strength provider.

C190. 0.135-2.70 secukinumab : 1-70 buffer system : 2-350 amino acid component : 0.001-300 antioxidant : 5-300 tonicifier : 0.0001-5 chelator.

C191. 0.135-2.70 secukinumab : 1-70 buffer system : 2-350 amino acid component : 0.001-300 antioxidant : 5-300 ionic strength provider: 0.0001-5 chelator.

C192. 0.135-2.70 secukinumab : 1-70 buffer system : 2-350 amino acid component: 5-300 tonicifier : 5-300 ionic strength provider : 0.0001-5 chelator.

(continued)

C193. 0.135-2.70 secukinumab : 1-70 buffer system : 0.0008-3.82 surfactant: 0.001-300 antioxidant: 5-300 tonicifier : 5-300 ionic strength provider.

C194. 0.135-2.70 secukinumab : 1-70 buffer system : 0.0008-3.82 surfactant: 0.001-300 antioxidant: 5-300 tonicifier: 0.0001-5 chelator.

C195. 0.135-2.70 secukinumab : 1-70 buffer system : 0.0008-3.82 surfactant: 0.001-300 antioxidant: 5-300 ionic strength provider : 0.0001-5 chelator.

C196. 0.135-2.70 secukinumab : 1-70 buffer system : 0.0008-3.82 surfactant : 5-300 tonicifier : 5-300 ionic strength provider : 0.0001-5 chelator.

C197. 0.135-2.70 secukinumab : 1-70 buffer system : 0.001-300 antioxidant : 5-300 tonicifier : 5-300 ionic strength provider : 0.0001-5 chelator.

C198. 0.135-2.70 secukinumab : 30-400 sugar component : 2-350 amino acid component : 0.0008-3.82 surfactant : 0.001-300 antioxidant: 5-300 tonicifier.

C199. 0.135-2.70 secukinumab : 30-400 sugar component : 2-350 amino acid component : 0.0008-3.82 surfactant : 0.001-300 antioxidant: 5-300 ionic strength provider.

C200. 0.135-2.70 secukinumab : 30-400 sugar component : 2-350 amino acid component : 0.0008-3.82 surfactant : 0.001-300 antioxidant: 0.0001-5 chelator.

C201. 0.135-2.70 secukinumab : 30-400 sugar component : 2-350 amino acid component : 0.0008-3.82 surfactant : 5-300 tonicifier : 5-300 ionic strength provider.

C202. 0.135-2.70 secukinumab : 30-400 sugar component : 2-350 amino acid component : 0.0008-3.82 surfactant : 5-300 tonicifier : 0.0001-5 chelator.

C203. 0.135-2.70 secukinumab : 30-400 sugar component : 2-350 amino acid component : 0.0008-3.82 surfactant : 5-300 ionic strength provider : 0.0001-5 chelator.

C204. 0.135-2.70 secukinumab : 30-400 sugar component : 2-350 amino acid component : 0.001-300 antioxidant : 5-300 tonicifier : 5-300 ionic strength provider.

C205. 0.135-2.70 secukinumab : 30-400 sugar component : 2-350 amino acid component : 0.001-300 antioxidant : 5-300 tonicifier : 0.0001-5 chelator.

C206. 0.135-2.70 secukinumab : 30-400 sugar component : 2-350 amino acid component : 0.001-300 antioxidant : 5-300 ionic strength provider : 0.0001-5 chelator.

C207. 0.135-2.70 secukinumab : 30-400 sugar component : 2-350 amino acid component : 5-300 tonicifier : 5-300 ionic strength provider: 0.0001-5 chelator.

C208. 0.135-2.70 secukinumab : 30-400 sugar component : 0.0008-3.82 surfactant : 0.001-300 antioxidant : 5-300 tonicifier : 5-300 ionic strength provider.

C209. 0.135-2.70 secukinumab : 30-400 sugar component : 0.0008-3.82 surfactant : 0.001-300 antioxidant : 5-300 tonicifier : 0.0001-5 chelator.

C210. 0.135-2.70 secukinumab : 30-400 sugar component : 0.0008-3.82 surfactant : 0.001-300 antioxidant : 5-300 ionic strength provider: 0.0001-5 chelator.

C211. 0.135-2.70 secukinumab : 30-400 sugar component : 0.0008-3.82 surfactant : 5-300 tonicifier: 5-300 ionic strength provider : 0.0001-5 chelator.

C212. 0.135-2.70 secukinumab : 30-400 sugar component : 0.001-300 antioxidant : 5-300 tonicifier : 5-300 ionic strength provider : 0.0001-5 chelator.

C213. 0.135-2.70 secukinumab : 2-350 amino acid component : 0.0008-3.82 surfactant : 0.001-300 antioxidant : 5-300 tonicifier : 5-300 ionic strength provider.

C214. 0.135-2.70 secukinumab : 2-350 amino acid component : 0.0008-3.82 surfactant : 0.001-300 antioxidant : 5-300 tonicifier : 0.0001-5 chelator.

C215. 0.135-2.70 secukinumab : 2-350 amino acid component : 0.0008-3.82 surfactant : 0.001-300 antioxidant : 5-300 ionic strength provider : 0.0001-5 chelator.

C216. 0.135-2.70 secukinumab : 2-350 amino acid component : 0.0008-3.82 surfactant : 5-300 tonicifier : 5-300 ionic strength provider: 0.0001-5 chelator.

C217. 0.135-2.70 secukinumab : 2-350 amino acid component : 0.001-300 antioxidant : 5-300 tonicifier : 5-300 ionic strength provider: 0.0001-5 chelator.

C218. 0.135-2.70 secukinumab : 0.0008-3.82 surfactant: 0.001-300 antioxidant : 5-300 tonicifier: 5-300 ionic strength provider: 0.0001-5 chelator.

(continued)

C219. 0.135-2.70 secukinumab : 1-70 buffer system : 30-400 sugar component : 2-350 amino acid component : 0.0008-3.82 surfactant: 0.001-300 antioxidant: 5-300 tonicifier.

C220. 0.135-2.70 secukinumab : 1-70 buffer system : 30-400 sugar component : 2-350 amino acid component : 0.0008-3.82 surfactant: 0.001-300 antioxidant: 5-300 ionic strength provider.

C221. 0.135-2.70 secukinumab : 1-70 buffer system : 30-400 sugar component : 2-350 amino acid component : 0.0008-3.82 surfactant: 0.001-300 antioxidant: 0.0001-5 chelator.

C222. 0.135-2.70 secukinumab : 1-70 buffer system : 30-400 sugar component : 2-350 amino acid component : 0.0008-3.82 surfactant: 5-300 tonicifier : 5-300 ionic strength provider.

C223. 0.135-2.70 secukinumab : 1-70 buffer system : 30-400 sugar component : 2-350 amino acid component : 0.0008-3.82 surfactant: 5-300 tonicifier : 0.0001-5 chelator.

C224. 0.135-2.70 secukinumab : 1-70 buffer system : 30-400 sugar component : 2-350 amino acid component : 0.0008-3.82 surfactant: 5-300 ionic strength provider: 0.0001-5 chelator.

C225. 0.135-2.70 secukinumab : 1-70 buffer system : 30-400 sugar component : 2-350 amino acid component : 0.001-300 antioxidant: 5-300 tonicifier : 5-300 ionic strength provider.

C226. 0.135-2.70 secukinumab : 1-70 buffer system : 30-400 sugar component : 2-350 amino acid component : 0.001-300 antioxidant: 5-300 tonicifier : 0.0001-5 chelator.

C227. 0.135-2.70 secukinumab : 1-70 buffer system : 30-400 sugar component : 2-350 amino acid component : 0.001-300 antioxidant: 5-300 ionic strength provider: 0.0001-5 chelator.

C228. 0.135-2.70 secukinumab : 1-70 buffer system : 30-400 sugar component: 2-350 amino acid component : 5-300 tonicifier : 5-300 ionic strength provider: 0.0001-5 chelator.

C229. 0.135-2.70 secukinumab : 1-70 buffer system : 30-400 sugar component: 0.0008-3.82 surfactant : 0.001-300 antioxidant : 5-300 tonicifier : 5-300 ionic strength provider.

C230. 0.135-2.70 secukinumab : 1-70 buffer system : 30-400 sugar component: 0.0008-3.82 surfactant : 0.001-300 antioxidant : 5-300 tonicifier : 0.0001-5 chelator.

C231. 0.135-2.70 secukinumab : 1-70 buffer system : 30-400 sugar component: 0.0008-3.82 surfactant : 0.001-300 antioxidant : 5-300 ionic strength provider: 0.0001-5 chelator.

C232. 0.135-2.70 secukinumab : 1-70 buffer system : 30-400 sugar component: 0.0008-3.82 surfactant : 5-300 tonicifier : 5-300 ionic strength provider: 0.0001-5 chelator.

C233. 0.135-2.70 secukinumab : 1-70 buffer system : 30-400 sugar component: 0.001-300 antioxidant : 5-300 tonicifier : 5-300 ionic strength provider: 0.0001-5 chelator.

C234. 0.135-2.70 secukinumab : 1-70 buffer system : 2-350 amino acid component : 0.0008-3.82 surfactant : 0.001-300 antioxidant: 5-300 tonicifier : 5-300 ionic strength provider.

C235. 0.135-2.70 secukinumab : 1-70 buffer system : 2-350 amino acid component : 0.0008-3.82 surfactant : 0.001-300 antioxidant: 5-300 tonicifier : 0.0001-5 chelator.

C236. 0.135-2.70 secukinumab : 1-70 buffer system : 2-350 amino acid component : 0.0008-3.82 surfactant : 0.001-300 antioxidant: 5-300 ionic strength provider: 0.0001-5 chelator.

C237. 0.135-2.70 secukinumab : 1-70 buffer system : 2-350 amino acid component: 0.0008-3.82 surfactant : 5-300 tonicifier : 5-300 ionic strength provider: 0.0001-5 chelator.

C238. 0.135-2.70 secukinumab : 1-70 buffer system : 2-350 amino acid component: 0.001-300 antioxidant : 5-300 tonicifier : 5-300 ionic strength provider: 0.0001-5 chelator.

C239. 0.135-2.70 secukinumab : 1-70 buffer system : 0.0008-3.82 surfactant: 0.001-300 antioxidant : 5-300 tonicifier : 5-300 ionic strength provider: 0.0001-5 chelator.

C240. 0.135-2.70 secukinumab : 30-400 sugar component : 2-350 amino acid component : 0.0008-3.82 surfactant : 0.001-300 antioxidant: 5-300 tonicifier : 5-300 ionic strength provider.

C241. 0.135-2.70 secukinumab : 30-400 sugar component : 2-350 amino acid component : 0.0008-3.82 surfactant : 0.001-300 antioxidant: 5-300 tonicifier : 0.0001-5 chelator.

C242. 0.135-2.70 secukinumab : 30-400 sugar component : 2-350 amino acid component : 0.0008-3.82 surfactant : 0.001-300 antioxidant: 5-300 ionic strength provider: 0.0001-5 chelator.

C243. 0.135-2.70 secukinumab : 30-400 sugar component: 2-350 amino acid component : 0.0008-3.82 surfactant : 5-300 tonicifier : 5-300 ionic strength provider: 0.0001-5 chelator.

C244. 0.135-2.70 secukinumab : 30-400 sugar component : 2-350 amino acid component: 0.001-300 antioxidant : 5-300 tonicifier : 5-300 ionic strength provider: 0.0001-5 chelator.

(continued)

C245. 0.135-2.70 secukinumab : 30-400 sugar component : 0.0008-3.82 surfactant : 0.001-300 antioxidant: 5-300 tonicifier: 5-300 ionic strength provider: 0.0001-5 chelator.

C246. 0.135-2.70 secukinumab : 2-350 amino acid component : 0.0008-3.82 surfactant : 0.001-300 antioxidant : 5-300 tonicifier : 5-300 ionic strength provider: 0.0001-5 chelator.

C247. 0.135-2.70 secukinumab : 1-70 buffer system : 30-400 sugar component : 2-350 amino acid component : 0.0008-3.82 surfactant: 0.001-300 antioxidant : 5-300 tonicifier : 5-300 ionic strength provider.

C248. 0.135-2.70 secukinumab : 1-70 buffer system : 30-400 sugar component : 2-350 amino acid component : 0.0008-3.82 surfactant: 0.001-300 antioxidant : 5-300 tonicifier : 0.0001-5 chelator.

C249. 0.135-2.70 secukinumab : 1-70 buffer system : 30-400 sugar component : 2-350 amino acid component : 0.0008-3.82 surfactant: 0.001-300 antioxidant : 5-300 ionic strength provider : 0.0001-5 chelator.

C250. 0.135-2.70 secukinumab : 1-70 buffer system : 30-400 sugar component : 2-350 amino acid component : 0.0008-3.82 surfactant: 5-300 tonicifier : 5-300 ionic strength provider: 0.0001-5 chelator.

C251. 0.135-2.70 secukinumab : 1-70 buffer system : 30-400 sugar component : 2-350 amino acid component : 0.001-300 antioxidant: 5-300 tonicifier : 5-300 ionic strength provider : 0.0001-5 chelator.

C252. 0.135-2.70 secukinumab : 1-70 buffer system : 30-400 sugar component: 0.0008-3.82 surfactant : 0.001-300 antioxidant : 5-300 tonicifier : 5-300 ionic strength provider : 0.0001-5 chelator.

C253. 0.135-2.70 secukinumab : 1-70 buffer system : 2-350 amino acid component : 0.0008-3.82 surfactant : 0.001-300 antioxidant: 5-300 tonicifier : 5-300 ionic strength provider : 0.0001-5 chelator.

C254. 0.135-2.70 secukinumab : 30-400 sugar component : 2-350 amino acid component : 0.0008-3.82 surfactant : 0.001-300 antioxidant: 5-300 tonicifier : 5-300 ionic strength provider : 0.0001-5 chelator.

C255. 0.135-2.70 secukinumab : 1-70 buffer system : 30-400 sugar component : 2-350 amino acid component : 0.0008-3.82 surfactant : 0.001-300 antioxidant : 5-300 tonicifier : 5-300 ionic strength provider : 0.0001-5 chelator.

The embodiments of C1-C255 may be further defined or characterised by features of numbered paragraphs D1-D1152, E1-E1151, F1-F12, G1-G959, H1-H1423, 11-12400, and J1-J1158.

*Embodiments of Combinations of Ingredients Quantified by Narrower Absolute Concentrations*

D) General Aspects and Embodiments Relating to Combinations of Ingredients Quantified by Narrower Concentrations

The biopharmaceutical composition suitably comprises, or consists of (suitably along with a diluent, which is preferably water), 120-220 mg/mL biopharmaceutical active (which is preferably secukinumab), a diluent, and one or more of any of: 1-9 mM or 51-60 mM buffer system; 50-129 mM or 130-174 mM sugar component or 200-250 mM sugar component; 20-99 mM or 100-149 mM amino acid component; 0.01-1.2 mg/mL surfactant; 10-129 mM tonicifier; 10-129 mM ionic strength provider; 0.1-20 mM antioxidant; and 0.001-2 mM chelator. The following numbered paragraphs D1-D1152 disclose specific aspects and embodiments of the invention, wherein the biopharmaceutical composition comprises, consists of (suitably along with a diluent, which is preferably water), and/or is otherwise characterised by:

D1. 120-220 mg/mL secukinumab; and a diluent.

D2. 120-220 mg/mL secukinumab; 1-9 mM buffer system; and a diluent.

D3. 120-220 mg/mL secukinumab; 51-60 mM buffer system; and a diluent.

D4. 120-220 mg/mL secukinumab; 50-129 mM sugar component; and a diluent.

D5. 120-220 mg/mL secukinumab; 130-174 mM sugar component; and a diluent.

D6. 120-220 mg/mL secukinumab; 20-99 mM amino acid component; and a diluent.

D7. 120-220 mg/mL secukinumab; 100-149 mM amino acid component; and a diluent.

D8. 120-220 mg/mL secukinumab; 0.01-1.2 mg/mL surfactant; and a diluent.

D9. 120-220 mg/mL secukinumab; 10-129 mM tonicifier; and a diluent.

D10. 120-220 mg/mL secukinumab; 10-129 mM ionic strength provider; and a diluent.

D11. 120-220 mg/mL secukinumab; 0.1-20 mM antioxidant; and a diluent.

D12. 120-220 mg/mL secukinumab; 0.001-2 mM chelator; and a diluent.

D13. 120-220 mg/mL secukinumab; 1-9 mM buffer system; 50-129 mM sugar component; and a diluent.

D14. 120-220 mg/mL secukinumab; 51-60 mM buffer system; 50-129 mM sugar component; and a diluent.

(continued)

D15. 120-220 mg/mL secukinumab; 1-9 mM buffer system; 130-174 mM sugar component; and a diluent.
D16. 120-220 mg/mL secukinumab; 51-60 mM buffer system; 130-174 mM sugar component; and a diluent.
D17. 120-220 mg/mL secukinumab; 1-9 mM buffer system; 20-99 mM amino acid component; and a diluent.
D18. 120-220 mg/mL secukinumab; 51-60 mM buffer system; 20-99 mM amino acid component; and a diluent.
D19. 120-220 mg/mL secukinumab; 1-9 mM buffer system; 100-149 mM amino acid component; and a diluent.
D20. 120-220 mg/mL secukinumab; 51-60 mM buffer system; 100-149 mM amino acid component; and a diluent.
D21. 120-220 mg/mL secukinumab; 1-9 mM buffer system; 0.01-1.2 mg/mL surfactant; and a diluent.
D22. 120-220 mg/mL secukinumab; 51-60 mM buffer system; 0.01-1.2 mg/mL surfactant; and a diluent.
D23. 120-220 mg/mL secukinumab; 1-9 mM buffer system; 10-129 mM tonicifier; and a diluent.
D24. 120-220 mg/mL secukinumab; 51-60 mM buffer system; 10-129 mM tonicifier; and a diluent.
D25. 120-220 mg/mL secukinumab; 1-9 mM buffer system; 10-129 mM ionic strength provider; and a diluent.
D26. 120-220 mg/mL secukinumab; 51-60 mM buffer system; 10-129 mM ionic strength provider; and a diluent.
D27. 120-220 mg/mL secukinumab; 1-9 mM buffer system; 0.1-20 mM antioxidant; and a diluent.
D28. 120-220 mg/mL secukinumab; 51-60 mM buffer system; 0.1-20 mM antioxidant; and a diluent.
D29. 120-220 mg/mL secukinumab; 1-9 mM buffer system; 0.001-2 mM chelator; and a diluent.
D30. 120-220 mg/mL secukinumab; 51-60 mM buffer system; 0.001-2 mM chelator; and a diluent.
D31. 120-220 mg/mL secukinumab; 50-129 mM sugar component; 20-99 mM amino acid component; and a diluent.
D32. 120-220 mg/mL secukinumab; 130-174 mM sugar component; 20-99 mM amino acid component; and a diluent.
D33. 120-220 mg/mL secukinumab; 50-129 mM sugar component; 100-149 mM amino acid component; and a diluent.
D34. 120-220 mg/mL secukinumab; 130-174 mM sugar component; 100-149 mM amino acid component; and a diluent.
D35. 120-220 mg/mL secukinumab; 50-129 mM sugar component; 0.01-1.2 mg/mL surfactant; and a diluent.
D36. 120-220 mg/mL secukinumab; 130-174 mM sugar component; 0.01-1.2 mg/mL surfactant; and a diluent.
D37. 120-220 mg/mL secukinumab; 50-129 mM sugar component; 10-129 mM tonicifier; and a diluent.
D38. 120-220 mg/mL secukinumab; 130-174 mM sugar component; 10-129 mM tonicifier; and a diluent.
D39. 120-220 mg/mL secukinumab; 50-129 mM sugar component; 10-129 mM ionic strength provider; and a diluent.
D40. 120-220 mg/mL secukinumab; 130-174 mM sugar component; 10-129 mM ionic strength provider; and a diluent.
D41. 120-220 mg/mL secukinumab; 50-129 mM sugar component; 0.1-20 mM antioxidant; and a diluent.
D42. 120-220 mg/mL secukinumab; 130-174 mM sugar component; 0.1-20 mM antioxidant; and a diluent.
D43. 120-220 mg/mL secukinumab; 50-129 mM sugar component; 0.001-2 mM chelator; and a diluent.
D44. 120-220 mg/mL secukinumab; 130-174 mM sugar component; 0.001-2 mM chelator; and a diluent.
D45. 120-220 mg/mL secukinumab; 20-99 mM amino acid component; 0.01-1.2 mg/mL surfactant; and a diluent.
D46. 120-220 mg/mL secukinumab; 100-149 mM amino acid component; 0.01-1.2 mg/mL surfactant; and a diluent.
D47. 120-220 mg/mL secukinumab; 20-99 mM amino acid component; 10-129 mM tonicifier; and a diluent.
D48. 120-220 mg/mL secukinumab; 100-149 mM amino acid component; 10-129 mM tonicifier; and a diluent.
D49. 120-220 mg/mL secukinumab; 20-99 mM amino acid component; 10-129 mM ionic strength provider; and a diluent.
D50. 120-220 mg/mL secukinumab; 100-149 mM amino acid component; 10-129 mM ionic strength provider; and a diluent.
D51. 120-220 mg/mL secukinumab; 20-99 mM amino acid component; 0.1-20 mM antioxidant; and a diluent.
D52. 120-220 mg/mL secukinumab; 100-149 mM amino acid component; 0.1-20 mM antioxidant; and a diluent.
D53. 120-220 mg/mL secukinumab; 20-99 mM amino acid component; 0.001-2 mM chelator; and a diluent.
D54. 120-220 mg/mL secukinumab; 100-149 mM amino acid component; 0.001-2 mM chelator; and a diluent.
D55. 120-220 mg/mL secukinumab; 0.01-1.2 mg/mL surfactant; 10-129 mM tonicifier; and a diluent.

(continued)

| | |
|---|---|
| D56. | 120-220 mg/mL secukinumab; 0.01-1.2 mg/mL surfactant; 10-129 mM ionic strength provider; and a diluent. |
| D57. | 120-220 mg/mL secukinumab; 0.01-1.2 mg/mL surfactant; 0.1-20 mM antioxidant; and a diluent. |
| D58. | 120-220 mg/mL secukinumab; 0.01-1.2 mg/mL surfactant; 0.001-2 mM chelator; and a diluent. |
| D59. | 120-220 mg/mL secukinumab; 10-129 mM tonicifier; 10-129 mM ionic strength provider; and a diluent. |
| D60. | 120-220 mg/mL secukinumab; 10-129 mM tonicifier; 0.1-20 mM antioxidant; and a diluent. |
| D61. | 120-220 mg/mL secukinumab; 10-129 mM tonicifier; 0.001-2 mM chelator; and a diluent. |
| D62. | 120-220 mg/mL secukinumab; 10-129 mM ionic strength provider; 0.1-20 mM antioxidant; and a diluent. |
| D63. | 120-220 mg/mL secukinumab; 10-129 mM ionic strength provider; 0.001-2 mM chelator; and a diluent. |
| D64. | 120-220 mg/mL secukinumab; 0.1-20 mM antioxidant; 0.001-2 mM chelator; and a diluent. |
| D65. | 120-220 mg/mL secukinumab; 1-9 mM buffer system; 50-129 mM sugar component; 20-99 mM amino acid component; and a diluent. |
| D66. | 120-220 mg/mL secukinumab; 51-60 mM buffer system; 50-129 mM sugar component; 20-99 mM amino acid component; and a diluent. |
| D67. | 120-220 mg/mL secukinumab; 1-9 mM buffer system; 130-174 mM sugar component; 20-99 mM amino acid component; and a diluent. |
| D68. | 120-220 mg/mL secukinumab; 51-60 mM buffer system; 130-174 mM sugar component; 20-99 mM amino acid component; and a diluent. |
| D69. | 120-220 mg/mL secukinumab; 1-9 mM buffer system; 50-129 mM sugar component; 100-149 mM amino acid component; and a diluent. |
| D70. | 120-220 mg/mL secukinumab; 51-60 mM buffer system; 50-129 mM sugar component; 100-149 mM amino acid component; and a diluent. |
| D71. | 120-220 mg/mL secukinumab; 1-9 mM buffer system; 130-174 mM sugar component; 100-149 mM amino acid component; and a diluent. |
| D72. | 120-220 mg/mL secukinumab; 51-60 mM buffer system; 130-174 mM sugar component; 100-149 mM amino acid component; and a diluent. |
| D73. | 120-220 mg/mL secukinumab; 1-9 mM buffer system; 50-129 mM sugar component; 0.01-1.2 mg/mL surfactant; and a diluent. |
| D74. | 120-220 mg/mL secukinumab; 51-60 mM buffer system; 50-129 mM sugar component; 0.01-1.2 mg/mL surfactant; and a diluent. |
| D75. | 120-220 mg/mL secukinumab; 1-9 mM buffer system; 130-174 mM sugar component; 0.01-1.2 mg/mL surfactant; and a diluent. |
| D76. | 120-220 mg/mL secukinumab; 51-60 mM buffer system; 130-174 mM sugar component; 0.01-1.2 mg/mL surfactant; and a diluent. |
| D77. | 120-220 mg/mL secukinumab; 1-9 mM buffer system; 50-129 mM sugar component; 10-129 mM tonicifier; and a diluent. |
| D78. | 120-220 mg/mL secukinumab; 51-60 mM buffer system; 50-129 mM sugar component; 10-129 mM tonicifier; and a diluent. |
| D79. | 120-220 mg/mL secukinumab; 1-9 mM buffer system; 130-174 mM sugar component; 10-129 mM tonicifier; and a diluent. |
| D80. | 120-220 mg/mL secukinumab; 51-60 mM buffer system; 130-174 mM sugar component; 10-129 mM tonicifier; and a diluent. |
| D81. | 120-220 mg/mL secukinumab; 1-9 mM buffer system; 50-129 mM sugar component; 10-129 mM ionic strength provider; and a diluent. |
| D82. | 120-220 mg/mL secukinumab; 51-60 mM buffer system; 50-129 mM sugar component; 10-129 mM ionic strength provider; and a diluent. |
| D83. | 120-220 mg/mL secukinumab; 1-9 mM buffer system; 130-174 mM sugar component; 10-129 mM ionic strength provider; and a diluent. |
| D84. | 120-220 mg/mL secukinumab; 51-60 mM buffer system; 130-174 mM sugar component; 10-129 mM ionic strength provider; and a diluent. |
| D85. | 120-220 mg/mL secukinumab; 1-9 mM buffer system; 50-129 mM sugar component; 0.1-20 mM antioxidant; and a diluent. |

(continued)

D86. 120-220 mg/mL secukinumab; 51-60 mM buffer system; 50-129 mM sugar component; 0.1-20 mM antioxidant; and a diluent.

D87. 120-220 mg/mL secukinumab; 1-9 mM buffer system; 130-174 mM sugar component; 0.1-20 mM antioxidant; and a diluent.

D88. 120-220 mg/mL secukinumab; 51-60 mM buffer system; 130-174 mM sugar component; 0.1-20 mM antioxidant; and a diluent.

D89. 120-220 mg/mL secukinumab; 1-9 mM buffer system; 50-129 mM sugar component; 0.001-2 mM chelator; and a diluent.

D90. 120-220 mg/mL secukinumab; 51-60 mM buffer system; 50-129 mM sugar component; 0.001-2 mM chelator; and a diluent.

D91. 120-220 mg/mL secukinumab; 1-9 mM buffer system; 130-174 mM sugar component; 0.001-2 mM chelator; and a diluent.

D92. 120-220 mg/mL secukinumab; 51-60 mM buffer system; 130-174 mM sugar component; 0.001-2 mM chelator; and a diluent.

D93. 120-220 mg/mL secukinumab; 1-9 mM buffer system; 20-99 mM amino acid component; 0.01-1.2 mg/mL surfactant; and a diluent.

D94. 120-220 mg/mL secukinumab; 51-60 mM buffer system; 20-99 mM amino acid component; 0.01-1.2 mg/mL surfactant; and a diluent.

D95. 120-220 mg/mL secukinumab; 1-9 mM buffer system; 100-149 mM amino acid component; 0.01-1.2 mg/mL surfactant; and a diluent.

D96. 120-220 mg/mL secukinumab; 51-60 mM buffer system; 100-149 mM amino acid component; 0.01-1.2 mg/mL surfactant; and a diluent.

D97. 120-220 mg/mL secukinumab; 1-9 mM buffer system; 20-99 mM amino acid component; 10-129 mM tonicifier; and a diluent.

D98. 120-220 mg/mL secukinumab; 51-60 mM buffer system; 20-99 mM amino acid component; 10-129 mM tonicifier; and a diluent.

D99. 120-220 mg/mL secukinumab; 1-9 mM buffer system; 100-149 mM amino acid component; 10-129 mM tonicifier; and a diluent.

D100. 120-220 mg/mL secukinumab; 51-60 mM buffer system; 100-149 mM amino acid component; 10-129 mM tonicifier; and a diluent.

D101. 120-220 mg/mL secukinumab; 1-9 mM buffer system; 20-99 mM amino acid component; 10-129 mM ionic strength provider; and a diluent.

D102. 120-220 mg/mL secukinumab; 51-60 mM buffer system; 20-99 mM amino acid component; 10-129 mM ionic strength provider; and a diluent.

D103. 120-220 mg/mL secukinumab; 1-9 mM buffer system; 100-149 mM amino acid component; 10-129 mM ionic strength provider; and a diluent.

D104. 120-220 mg/mL secukinumab; 51-60 mM buffer system; 100-149 mM amino acid component; 10-129 mM ionic strength provider; and a diluent.

D105. 120-220 mg/mL secukinumab; 1-9 mM buffer system; 20-99 mM amino acid component; 0.1-20 mM antioxidant; and a diluent.

D106. 120-220 mg/mL secukinumab; 51-60 mM buffer system; 20-99 mM amino acid component; 0.1-20 mM antioxidant; and a diluent.

D107. 120-220 mg/mL secukinumab; 1-9 mM buffer system; 100-149 mM amino acid component; 0.1-20 mM antioxidant; and a diluent.

D108. 120-220 mg/mL secukinumab; 51-60 mM buffer system; 100-149 mM amino acid component; 0.1-20 mM antioxidant; and a diluent.

D109. 120-220 mg/mL secukinumab; 1-9 mM buffer system; 20-99 mM amino acid component; 0.001-2 mM chelator; and a diluent.

D110. 120-220 mg/mL secukinumab; 51-60 mM buffer system; 20-99 mM amino acid component; 0.001-2 mM chelator; and a diluent.

D111. 120-220 mg/mL secukinumab; 1-9 mM buffer system; 100-149 mM amino acid component; 0.001-2 mM chelator; and a diluent.

(continued)

D112. 120-220 mg/mL secukinumab; 51-60 mM buffer system; 100-149 mM amino acid component; 0.001-2 mM chelator; and a diluent.

D113. 120-220 mg/mL secukinumab; 1-9 mM buffer system; 0.01-1.2 mg/mL surfactant; 10-129 mM tonicifier; and a diluent.

D114. 120-220 mg/mL secukinumab; 51-60 mM buffer system; 0.01-1.2 mg/mL surfactant; 10-129 mM tonicifier; and a diluent.

D115. 120-220 mg/mL secukinumab; 1-9 mM buffer system; 0.01-1.2 mg/mL surfactant; 10-129 mM ionic strength provider; and a diluent.

D116. 120-220 mg/mL secukinumab; 51-60 mM buffer system; 0.01-1.2 mg/mL surfactant; 10-129 mM ionic strength provider; and a diluent.

D117. 120-220 mg/mL secukinumab; 1-9 mM buffer system; 0.01-1.2 mg/mL surfactant; 0.1-20 mM antioxidant; and a diluent.

D118. 120-220 mg/mL secukinumab; 51-60 mM buffer system; 0.01-1.2 mg/mL surfactant; 0.1-20 mM antioxidant; and a diluent.

D119. 120-220 mg/mL secukinumab; 1-9 mM buffer system; 0.01-1.2 mg/mL surfactant; 0.001-2 mM chelator; and a diluent.

D120. 120-220 mg/mL secukinumab; 51-60 mM buffer system; 0.01-1.2 mg/mL surfactant; 0.001-2 mM chelator; and a diluent.

D121. 120-220 mg/mL secukinumab; 1-9 mM buffer system; 10-129 mM tonicifier; 10-129 mM ionic strength provider; and a diluent.

D122. 120-220 mg/mL secukinumab; 51-60 mM buffer system; 10-129 mM tonicifier; 10-129 mM ionic strength provider; and a diluent.

D123. 120-220 mg/mL secukinumab; 1-9 mM buffer system; 10-129 mM tonicifier; 0.1-20 mM antioxidant; and a diluent.

D124. 120-220 mg/mL secukinumab; 51-60 mM buffer system; 10-129 mM tonicifier; 0.1-20 mM antioxidant; and a diluent.

D125. 120-220 mg/mL secukinumab; 1-9 mM buffer system; 10-129 mM tonicifier; 0.001-2 mM chelator; and a diluent.

D126. 120-220 mg/mL secukinumab; 51-60 mM buffer system; 10-129 mM tonicifier; 0.001-2 mM chelator; and a diluent.

D127. 120-220 mg/mL secukinumab; 1-9 mM buffer system; 10-129 mM ionic strength provider; 0.1-20 mM antioxidant; and a diluent.

D128. 120-220 mg/mL secukinumab; 51-60 mM buffer system; 10-129 mM ionic strength provider; 0.1-20 mM antioxidant; and a diluent.

D129. 120-220 mg/mL secukinumab; 1-9 mM buffer system; 10-129 mM ionic strength provider; 0.001-2 mM chelator; and a diluent.

D130. 120-220 mg/mL secukinumab; 51-60 mM buffer system; 10-129 mM ionic strength provider; 0.001-2 mM chelator; and a diluent.

D131. 120-220 mg/mL secukinumab; 1-9 mM buffer system; 0.1-20 mM antioxidant; 0.001-2 mM chelator; and a diluent.

D132. 120-220 mg/mL secukinumab; 51-60 mM buffer system; 0.1-20 mM antioxidant; 0.001-2 mM chelator; and a diluent.

D133. 120-220 mg/mL secukinumab; 50-129 mM sugar component; 20-99 mM amino acid component; 0.01-1.2 mg/mL surfactant; and a diluent.

D134. 120-220 mg/mL secukinumab; 130-174 mM sugar component; 20-99 mM amino acid component; 0.01-1.2 mg/mL surfactant; and a diluent.

D135. 120-220 mg/mL secukinumab; 50-129 mM sugar component; 100-149 mM amino acid component; 0.01-1.2 mg/mL surfactant; and a diluent.

D136. 120-220 mg/mL secukinumab; 130-174 mM sugar component; 100-149 mM amino acid component; 0.01-1.2 mg/mL surfactant; and a diluent.

D137. 120-220 mg/mL secukinumab; 50-129 mM sugar component; 20-99 mM amino acid component; 10-129 mM tonicifier; and a diluent.

(continued)

D138. 120-220 mg/mL secukinumab; 130-174 mM sugar component; 20-99 mM amino acid component; 10-129 mM tonicifier; and a diluent.

D139. 120-220 mg/mL secukinumab; 50-129 mM sugar component; 100-149 mM amino acid component; 10-129 mM tonicifier; and a diluent.

D140. 120-220 mg/mL secukinumab; 130-174 mM sugar component; 100-149 mM amino acid component; 10-129 mM tonicifier; and a diluent.

D141. 120-220 mg/mL secukinumab; 50-129 mM sugar component; 20-99 mM amino acid component; 10-129 mM ionic strength provider; and a diluent.

D142. 120-220 mg/mL secukinumab; 130-174 mM sugar component; 20-99 mM amino acid component; 10-129 mM ionic strength provider; and a diluent.

D143. 120-220 mg/mL secukinumab; 50-129 mM sugar component; 100-149 mM amino acid component; 10-129 mM ionic strength provider; and a diluent.

D144. 120-220 mg/mL secukinumab; 130-174 mM sugar component; 100-149 mM amino acid component; 10-129 mM ionic strength provider; and a diluent.

D145. 120-220 mg/mL secukinumab; 50-129 mM sugar component; 20-99 mM amino acid component; 0.1-20 mM antioxidant; and a diluent.

D146. 120-220 mg/mL secukinumab; 130-174 mM sugar component; 20-99 mM amino acid component; 0.1-20 mM antioxidant; and a diluent.

D147. 120-220 mg/mL secukinumab; 50-129 mM sugar component; 100-149 mM amino acid component; 0.1-20 mM antioxidant; and a diluent.

D148. 120-220 mg/mL secukinumab; 130-174 mM sugar component; 100-149 mM amino acid component; 0.1-20 mM antioxidant; and a diluent.

D149. 120-220 mg/mL secukinumab; 50-129 mM sugar component; 20-99 mM amino acid component; 0.001-2 mM chelator; and a diluent.

D150. 120-220 mg/mL secukinumab; 130-174 mM sugar component; 20-99 mM amino acid component; 0.001-2 mM chelator; and a diluent.

D151. 120-220 mg/mL secukinumab; 50-129 mM sugar component; 100-149 mM amino acid component; 0.001-2 mM chelator; and a diluent.

D152. 120-220 mg/mL secukinumab; 130-174 mM sugar component; 100-149 mM amino acid component; 0.001-2 mM chelator; and a diluent.

D153. 120-220 mg/mL secukinumab; 50-129 mM sugar component; 0.01-1.2 mg/mL surfactant; 10-129 mM tonicifier; and a diluent.

D154. 120-220 mg/mL secukinumab; 130-174 mM sugar component; 0.01-1.2 mg/mL surfactant; 10-129 mM tonicifier; and a diluent.

D155. 120-220 mg/mL secukinumab; 50-129 mM sugar component; 0.01-1.2 mg/mL surfactant; 10-129 mM ionic strength provider; and a diluent.

D156. 120-220 mg/mL secukinumab; 130-174 mM sugar component; 0.01-1.2 mg/mL surfactant; 10-129 mM ionic strength provider; and a diluent.

D157. 120-220 mg/mL secukinumab; 50-129 mM sugar component; 0.01-1.2 mg/mL surfactant; 0.1-20 mM antioxidant; and a diluent.

D158. 120-220 mg/mL secukinumab; 130-174 mM sugar component; 0.01-1.2 mg/mL surfactant; 0.1-20 mM antioxidant; and a diluent.

D159. 120-220 mg/mL secukinumab; 50-129 mM sugar component; 0.01-1.2 mg/mL surfactant; 0.001-2 mM chelator; and a diluent.

D160. 120-220 mg/mL secukinumab; 130-174 mM sugar component; 0.01-1.2 mg/mL surfactant; 0.001-2 mM chelator; and a diluent.

D161. 120-220 mg/mL secukinumab; 50-129 mM sugar component; 10-129 mM tonicifier; 10-129 mM ionic strength provider; and a diluent.

D162. 120-220 mg/mL secukinumab; 130-174 mM sugar component; 10-129 mM tonicifier; 10-129 mM ionic strength provider; and a diluent.

D163. 120-220 mg/mL secukinumab; 50-129 mM sugar component; 10-129 mM tonicifier; 0.1-20 mM antioxidant; and a diluent.

(continued)

D164. 120-220 mg/mL secukinumab; 130-174 mM sugar component; 10-129 mM tonicifier; 0.1-20 mM antioxidant; and a diluent.

D165. 120-220 mg/mL secukinumab; 50-129 mM sugar component; 10-129 mM tonicifier; 0.001-2 mM chelator; and a diluent.

D166. 120-220 mg/mL secukinumab; 130-174 mM sugar component; 10-129 mM tonicifier; 0.001-2 mM chelator; and a diluent.

D167. 120-220 mg/mL secukinumab; 50-129 mM sugar component; 10-129 mM ionic strength provider; 0.1-20 mM antioxidant; and a diluent.

D168. 120-220 mg/mL secukinumab; 130-174 mM sugar component; 10-129 mM ionic strength provider; 0.1-20 mM antioxidant; and a diluent.

D169. 120-220 mg/mL secukinumab; 50-129 mM sugar component; 10-129 mM ionic strength provider; 0.001-2 mM chelator; and a diluent.

D170. 120-220 mg/mL secukinumab; 130-174 mM sugar component; 10-129 mM ionic strength provider; 0.001-2 mM chelator; and a diluent.

D171. 120-220 mg/mL secukinumab; 50-129 mM sugar component; 0.1-20 mM antioxidant; 0.001-2 mM chelator; and a diluent.

D172. 120-220 mg/mL secukinumab; 130-174 mM sugar component; 0.1-20 mM antioxidant; 0.001-2 mM chelator; and a diluent.

D173. 120-220 mg/mL secukinumab; 20-99 mM amino acid component; 0.01-1.2 mg/mL surfactant; 10-129 mM tonicifier; and a diluent.

D174. 120-220 mg/mL secukinumab; 100-149 mM amino acid component; 0.01-1.2 mg/mL surfactant; 10-129 mM tonicifier; and a diluent.

D175. 120-220 mg/mL secukinumab; 20-99 mM amino acid component; 0.01-1.2 mg/mL surfactant; 10-129 mM ionic strength provider; and a diluent.

D176. 120-220 mg/mL secukinumab; 100-149 mM amino acid component; 0.01-1.2 mg/mL surfactant; 10-129 mM ionic strength provider; and a diluent.

D177. 120-220 mg/mL secukinumab; 20-99 mM amino acid component; 0.01-1.2 mg/mL surfactant; 0.1-20 mM antioxidant; and a diluent.

D178. 120-220 mg/mL secukinumab; 100-149 mM amino acid component; 0.01-1.2 mg/mL surfactant; 0.1-20 mM antioxidant; and a diluent.

D179. 120-220 mg/mL secukinumab; 20-99 mM amino acid component; 0.01-1.2 mg/mL surfactant; 0.001-2 mM chelator; and a diluent.

D180. 120-220 mg/mL secukinumab; 100-149 mM amino acid component; 0.01-1.2 mg/mL surfactant; 0.001-2 mM chelator; and a diluent.

D181. 120-220 mg/mL secukinumab; 20-99 mM amino acid component; 10-129 mM tonicifier; 10-129 mM ionic strength provider; and a diluent.

D182. 120-220 mg/mL secukinumab; 100-149 mM amino acid component; 10-129 mM tonicifier; 10-129 mM ionic strength provider; and a diluent.

D183. 120-220 mg/mL secukinumab; 20-99 mM amino acid component; 10-129 mM tonicifier; 0.1-20 mM antioxidant; and a diluent.

D184. 120-220 mg/mL secukinumab; 100-149 mM amino acid component; 10-129 mM tonicifier; 0.1-20 mM antioxidant; and a diluent.

D185. 120-220 mg/mL secukinumab; 20-99 mM amino acid component; 10-129 mM tonicifier; 0.001-2 mM chelator; and a diluent.

D186. 120-220 mg/mL secukinumab; 100-149 mM amino acid component; 10-129 mM tonicifier; 0.001-2 mM chelator; and a diluent.

D187. 120-220 mg/mL secukinumab; 20-99 mM amino acid component; 10-129 mM ionic strength provider; 0.1-20 mM antioxidant; and a diluent.

D188. 120-220 mg/mL secukinumab; 100-149 mM amino acid component; 10-129 mM ionic strength provider; 0.1-20 mM antioxidant; and a diluent.

D189. 120-220 mg/mL secukinumab; 20-99 mM amino acid component; 10-129 mM ionic strength provider; 0.001-2 mM chelator; and a diluent.

(continued)

D190. 120-220 mg/mL secukinumab; 100-149 mM amino acid component; 10-129 mM ionic strength provider; 0.001-2 mM chelator; and a diluent.

D191. 120-220 mg/mL secukinumab; 20-99 mM amino acid component; 0.1-20 mM antioxidant; 0.001-2 mM chelator; and a diluent.

D192. 120-220 mg/mL secukinumab; 100-149 mM amino acid component; 0.1-20 mM antioxidant; 0.001-2 mM chelator; and a diluent.

D193. 120-220 mg/mL secukinumab; 0.01-1.2 mg/mL surfactant; 10-129 mM tonicifier; 10-129 mM ionic strength provider; and a diluent.

D194. 120-220 mg/mL secukinumab; 0.01-1.2 mg/mL surfactant; 10-129 mM tonicifier; 0.1-20 mM antioxidant; and a diluent.

D195. 120-220 mg/mL secukinumab; 0.01-1.2 mg/mL surfactant; 10-129 mM tonicifier; 0.001 -2 mM chelator; and a diluent.

D196. 120-220 mg/mL secukinumab; 0.01-1.2 mg/mL surfactant; 10-129 mM ionic strength provider; 0.1-20 mM antioxidant; and a diluent.

D197. 120-220 mg/mL secukinumab; 0.01-1.2 mg/mL surfactant; 10-129 mM ionic strength provider; 0.001-2 mM chelator; and a diluent.

D198. 120-220 mg/mL secukinumab; 0.01-1.2 mg/mL surfactant; 0.1-20 mM antioxidant; 0.001-2 mM chelator; and a diluent.

D199. 120-220 mg/mL secukinumab; 10-129 mM tonicifier; 10-129 mM ionic strength provider; 0.1-20 mM antioxidant; and a diluent.

D200. 120-220 mg/mL secukinumab; 10-129 mM tonicifier; 10-129 mM ionic strength provider; 0.001-2 mM chelator; and a diluent.

D201. 120-220 mg/mL secukinumab; 10-129 mM tonicifier; 0.1-20 mM antioxidant; 0.001-2 mM chelator; and a diluent.

D202. 120-220 mg/mL secukinumab; 10-129 mM ionic strength provider; 0.1-20 mM antioxidant; 0.001-2 mM chelator; and a diluent.

D203. 120-220 mg/mL secukinumab; 1-9 mM buffer system; 50-129 mM sugar component; 20-99 mM amino acid component; 0.01-1.2 mg/mL surfactant; and a diluent.

D204. 120-220 mg/mL secukinumab; 51-60 mM buffer system; 50-129 mM sugar component; 20-99 mM amino acid component; 0.01-1.2 mg/mL surfactant; and a diluent.

D205. 120-220 mg/mL secukinumab; 1-9 mM buffer system; 130-174 mM sugar component; 20-99 mM amino acid component; 0.01-1.2 mg/mL surfactant; and a diluent.

D206. 120-220 mg/mL secukinumab; 51-60 mM buffer system; 130-174 mM sugar component; 20-99 mM amino acid component; 0.01-1.2 mg/mL surfactant; and a diluent.

D207. 120-220 mg/mL secukinumab; 1-9 mM buffer system; 50-129 mM sugar component; 100-149 mM amino acid component; 0.01-1.2 mg/mL surfactant; and a diluent.

D208. 120-220 mg/mL secukinumab; 51-60 mM buffer system; 50-129 mM sugar component; 100-149 mM amino acid component; 0.01-1.2 mg/mL surfactant; and a diluent.

D209. 120-220 mg/mL secukinumab; 1-9 mM buffer system; 130-174 mM sugar component; 100-149 mM amino acid component; 0.01-1.2 mg/mL surfactant; and a diluent.

D210. 120-220 mg/mL secukinumab; 51-60 mM buffer system; 130-174 mM sugar component; 100-149 mM amino acid component; 0.01-1.2 mg/mL surfactant; and a diluent.

D211. 120-220 mg/mL secukinumab; 1-9 mM buffer system; 50-129 mM sugar component; 20-99 mM amino acid component; 10-129 mM tonicifier; and a diluent.

D212. 120-220 mg/mL secukinumab; 51-60 mM buffer system; 50-129 mM sugar component; 20-99 mM amino acid component; 10-129 mM tonicifier; and a diluent.

D213. 120-220 mg/mL secukinumab; 1-9 mM buffer system; 130-174 mM sugar component; 20-99 mM amino acid component; 10-129 mM tonicifier; and a diluent.

D214. 120-220 mg/mL secukinumab; 51-60 mM buffer system; 130-174 mM sugar component; 20-99 mM amino acid component; 10-129 mM tonicifier; and a diluent.

D215. 120-220 mg/mL secukinumab; 1-9 mM buffer system; 50-129 mM sugar component; 100-149 mM amino acid component; 10-129 mM tonicifier; and a diluent.

(continued)

D216. 120-220 mg/mL secukinumab; 51-60 mM buffer system; 50-129 mM sugar component; 100-149 mM amino acid component; 10-129 mM tonicifier; and a diluent.

D217. 120-220 mg/mL secukinumab; 1-9 mM buffer system; 130-174 mM sugar component; 100-149 mM amino acid component; 10-129 mM tonicifier; and a diluent.

D218. 120-220 mg/mL secukinumab; 51-60 mM buffer system; 130-174 mM sugar component; 100-149 mM amino acid component; 10-129 mM tonicifier; and a diluent.

D219. 120-220 mg/mL secukinumab; 1-9 mM buffer system; 50-129 mM sugar component; 20-99 mM amino acid component; 10-129 mM ionic strength provider; and a diluent.

D220. 120-220 mg/mL secukinumab; 51-60 mM buffer system; 50-129 mM sugar component; 20-99 mM amino acid component; 10-129 mM ionic strength provider; and a diluent.

D221. 120-220 mg/mL secukinumab; 1-9 mM buffer system; 130-174 mM sugar component; 20-99 mM amino acid component; 10-129 mM ionic strength provider; and a diluent.

D222. 120-220 mg/mL secukinumab; 51-60 mM buffer system; 130-174 mM sugar component; 20-99 mM amino acid component; 10-129 mM ionic strength provider; and a diluent.

D223. 120-220 mg/mL secukinumab; 1-9 mM buffer system; 50-129 mM sugar component; 100-149 mM amino acid component; 10-129 mM ionic strength provider; and a diluent.

D224. 120-220 mg/mL secukinumab; 51-60 mM buffer system; 50-129 mM sugar component; 100-149 mM amino acid component; 10-129 mM ionic strength provider; and a diluent.

D225. 120-220 mg/mL secukinumab; 1-9 mM buffer system; 130-174 mM sugar component; 100-149 mM amino acid component; 10-129 mM ionic strength provider; and a diluent.

D226. 120-220 mg/mL secukinumab; 51-60 mM buffer system; 130-174 mM sugar component; 100-149 mM amino acid component; 10-129 mM ionic strength provider; and a diluent.

D227. 120-220 mg/mL secukinumab; 1-9 mM buffer system; 50-129 mM sugar component; 20-99 mM amino acid component; 0.1-20 mM antioxidant; and a diluent.

D228. 120-220 mg/mL secukinumab; 51-60 mM buffer system; 50-129 mM sugar component; 20-99 mM amino acid component; 0.1-20 mM antioxidant; and a diluent.

D229. 120-220 mg/mL secukinumab; 1-9 mM buffer system; 130-174 mM sugar component; 20-99 mM amino acid component; 0.1-20 mM antioxidant; and a diluent.

D230. 120-220 mg/mL secukinumab; 51-60 mM buffer system; 130-174 mM sugar component; 20-99 mM amino acid component; 0.1-20 mM antioxidant; and a diluent.

D231. 120-220 mg/mL secukinumab; 1-9 mM buffer system; 50-129 mM sugar component; 100-149 mM amino acid component; 0.1-20 mM antioxidant; and a diluent.

D232. 120-220 mg/mL secukinumab; 51-60 mM buffer system; 50-129 mM sugar component; 100-149 mM amino acid component; 0.1-20 mM antioxidant; and a diluent.

D233. 120-220 mg/mL secukinumab; 1-9 mM buffer system; 130-174 mM sugar component; 100-149 mM amino acid component; 0.1-20 mM antioxidant; and a diluent.

D234. 120-220 mg/mL secukinumab; 51-60 mM buffer system; 130-174 mM sugar component; 100-149 mM amino acid component; 0.1-20 mM antioxidant; and a diluent.

D235. 120-220 mg/mL secukinumab; 1-9 mM buffer system; 50-129 mM sugar component; 20-99 mM amino acid component; 0.001-2 mM chelator; and a diluent.

D236. 120-220 mg/mL secukinumab; 51-60 mM buffer system; 50-129 mM sugar component; 20-99 mM amino acid component; 0.001-2 mM chelator; and a diluent.

D237. 120-220 mg/mL secukinumab; 1-9 mM buffer system; 130-174 mM sugar component; 20-99 mM amino acid component; 0.001-2 mM chelator; and a diluent.

D238. 120-220 mg/mL secukinumab; 51-60 mM buffer system; 130-174 mM sugar component; 20-99 mM amino acid component; 0.001-2 mM chelator; and a diluent.

D239. 120-220 mg/mL secukinumab; 1-9 mM buffer system; 50-129 mM sugar component; 100-149 mM amino acid component; 0.001-2 mM chelator; and a diluent.

D240. 120-220 mg/mL secukinumab; 51-60 mM buffer system; 50-129 mM sugar component; 100-149 mM amino acid component; 0.001-2 mM chelator; and a diluent.

D241. 120-220 mg/mL secukinumab; 1-9 mM buffer system; 130-174 mM sugar component; 100-149 mM amino acid component; 0.001-2 mM chelator; and a diluent.

(continued)

D242. 120-220 mg/mL secukinumab; 51-60 mM buffer system; 130-174 mM sugar component; 100-149 mM amino acid component; 0.001-2 mM chelator; and a diluent.

D243. 120-220 mg/mL secukinumab; 1-9 mM buffer system; 50-129 mM sugar component; 0.01-1.2 mg/mL surfactant; 10-129 mM tonicifier; and a diluent.

D244. 120-220 mg/mL secukinumab; 51-60 mM buffer system; 50-129 mM sugar component; 0.01-1.2 mg/mL surfactant; 10-129 mM tonicifier; and a diluent.

D245. 120-220 mg/mL secukinumab; 1-9 mM buffer system; 130-174 mM sugar component; 0.01-1.2 mg/mL surfactant; 10-129 mM tonicifier; and a diluent.

D246. 120-220 mg/mL secukinumab; 51-60 mM buffer system; 130-174 mM sugar component; 0.01-1.2 mg/mL surfactant; 10-129 mM tonicifier; and a diluent.

D247. 120-220 mg/mL secukinumab; 1-9 mM buffer system; 50-129 mM sugar component; 0.01-1.2 mg/mL surfactant; 10-129 mM ionic strength provider; and a diluent.

D248. 120-220 mg/mL secukinumab; 51-60 mM buffer system; 50-129 mM sugar component; 0.01-1.2 mg/mL surfactant; 10-129 mM ionic strength provider; and a diluent.

D249. 120-220 mg/mL secukinumab; 1-9 mM buffer system; 130-174 mM sugar component; 0.01-1.2 mg/mL surfactant; 10-129 mM ionic strength provider; and a diluent.

D250. 120-220 mg/mL secukinumab; 51-60 mM buffer system; 130-174 mM sugar component; 0.01-1.2 mg/mL surfactant; 10-129 mM ionic strength provider; and a diluent.

D251. 120-220 mg/mL secukinumab; 1-9 mM buffer system; 50-129 mM sugar component; 0.01-1.2 mg/mL surfactant; 0.1-20 mM antioxidant; and a diluent.

D252. 120-220 mg/mL secukinumab; 51-60 mM buffer system; 50-129 mM sugar component; 0.01-1.2 mg/mL surfactant; 0.1-20 mM antioxidant; and a diluent.

D253. 120-220 mg/mL secukinumab; 1-9 mM buffer system; 130-174 mM sugar component; 0.01-1.2 mg/mL surfactant; 0.1-20 mM antioxidant; and a diluent.

D254. 120-220 mg/mL secukinumab; 51-60 mM buffer system; 130-174 mM sugar component; 0.01-1.2 mg/mL surfactant; 0.1-20 mM antioxidant; and a diluent.

D255. 120-220 mg/mL secukinumab; 1-9 mM buffer system; 50-129 mM sugar component; 0.01-1.2 mg/mL surfactant; 0.001-2 mM chelator; and a diluent.

D256. 120-220 mg/mL secukinumab; 51-60 mM buffer system; 50-129 mM sugar component; 0.01-1.2 mg/mL surfactant; 0.001-2 mM chelator; and a diluent.

D257. 120-220 mg/mL secukinumab; 1-9 mM buffer system; 130-174 mM sugar component; 0.01-1.2 mg/mL surfactant; 0.001-2 mM chelator; and a diluent.

D258. 120-220 mg/mL secukinumab; 51-60 mM buffer system; 130-174 mM sugar component; 0.01-1.2 mg/mL surfactant; 0.001-2 mM chelator; and a diluent.

D259. 120-220 mg/mL secukinumab; 1-9 mM buffer system; 50-129 mM sugar component; 10-129 mM tonicifier; 10-129 mM ionic strength provider; and a diluent.

D260. 120-220 mg/mL secukinumab; 51-60 mM buffer system; 50-129 mM sugar component; 10-129 mM tonicifier; 10-129 mM ionic strength provider; and a diluent.

D261. 120-220 mg/mL secukinumab; 1-9 mM buffer system; 130-174 mM sugar component; 10-129 mM tonicifier; 10-129 mM ionic strength provider; and a diluent.

D262. 120-220 mg/mL secukinumab; 51-60 mM buffer system; 130-174 mM sugar component; 10-129 mM tonicifier; 10-129 mM ionic strength provider; and a diluent.

D263. 120-220 mg/mL secukinumab; 1-9 mM buffer system; 50-129 mM sugar component; 10-129 mM tonicifier; 0.1-20 mM antioxidant; and a diluent.

D264. 120-220 mg/mL secukinumab; 51-60 mM buffer system; 50-129 mM sugar component; 10-129 mM tonicifier; 0.1-20 mM antioxidant; and a diluent.

D265. 120-220 mg/mL secukinumab; 1-9 mM buffer system; 130-174 mM sugar component; 10-129 mM tonicifier; 0.1-20 mM antioxidant; and a diluent.

D266. 120-220 mg/mL secukinumab; 51-60 mM buffer system; 130-174 mM sugar component; 10-129 mM tonicifier; 0.1-20 mM antioxidant; and a diluent.

D267. 120-220 mg/mL secukinumab; 1-9 mM buffer system; 50-129 mM sugar component; 10-129 mM tonicifier; 0.001-2 mM chelator; and a diluent.

(continued)

D268. 120-220 mg/mL secukinumab; 51-60 mM buffer system; 50-129 mM sugar component; 10-129 mM tonicifier; 0.001-2 mM chelator; and a diluent.

D269. 120-220 mg/mL secukinumab; 1-9 mM buffer system; 130-174 mM sugar component; 10-129 mM tonicifier; 0.001-2 mM chelator; and a diluent.

D270. 120-220 mg/mL secukinumab; 51-60 mM buffer system; 130-174 mM sugar component; 10-129 mM tonicifier; 0.001 -2 mM chelator; and a diluent.

D271. 120-220 mg/mL secukinumab; 1-9 mM buffer system; 50-129 mM sugar component; 10-129 mM ionic strength provider; 0.1-20 mM antioxidant; and a diluent.

D272. 120-220 mg/mL secukinumab; 51-60 mM buffer system; 50-129 mM sugar component; 10-129 mM ionic strength provider; 0.1-20 mM antioxidant; and a diluent.

D273. 120-220 mg/mL secukinumab; 1-9 mM buffer system; 130-174 mM sugar component; 10-129 mM ionic strength provider; 0.1-20 mM antioxidant; and a diluent.

D274. 120-220 mg/mL secukinumab; 51-60 mM buffer system; 130-174 mM sugar component; 10-129 mM ionic strength provider; 0.1-20 mM antioxidant; and a diluent.

D275. 120-220 mg/mL secukinumab; 1-9 mM buffer system; 50-129 mM sugar component; 10-129 mM ionic strength provider; 0.001-2 mM chelator; and a diluent.

D276. 120-220 mg/mL secukinumab; 51-60 mM buffer system; 50-129 mM sugar component; 10-129 mM ionic strength provider; 0.001-2 mM chelator; and a diluent.

D277. 120-220 mg/mL secukinumab; 1-9 mM buffer system; 130-174 mM sugar component; 10-129 mM ionic strength provider; 0.001-2 mM chelator; and a diluent.

D278. 120-220 mg/mL secukinumab; 51-60 mM buffer system; 130-174 mM sugar component; 10-129 mM ionic strength provider; 0.001-2 mM chelator; and a diluent.

D279. 120-220 mg/mL secukinumab; 1-9 mM buffer system; 50-129 mM sugar component; 0.1-20 mM antioxidant; 0.001-2 mM chelator; and a diluent.

D280. 120-220 mg/mL secukinumab; 51-60 mM buffer system; 50-129 mM sugar component; 0.1-20 mM antioxidant; 0.001-2 mM chelator; and a diluent.

D281. 120-220 mg/mL secukinumab; 1-9 mM buffer system; 130-174 mM sugar component; 0.1-20 mM antioxidant; 0.001-2 mM chelator; and a diluent.

D282. 120-220 mg/mL secukinumab; 51-60 mM buffer system; 130-174 mM sugar component; 0.1-20 mM antioxidant; 0.001-2 mM chelator; and a diluent.

D283. 120-220 mg/mL secukinumab; 1-9 mM buffer system; 20-99 mM amino acid component; 0.01-1.2 mg/mL surfactant; 10-129 mM tonicifier; and a diluent.

D284. 120-220 mg/mL secukinumab; 51-60 mM buffer system; 20-99 mM amino acid component; 0.01-1.2 mg/mL surfactant; 10-129 mM tonicifier; and a diluent.

D285. 120-220 mg/mL secukinumab; 1-9 mM buffer system; 100-149 mM amino acid component; 0.01-1.2 mg/mL surfactant; 10-129 mM tonicifier; and a diluent.

D286. 120-220 mg/mL secukinumab; 51-60 mM buffer system; 100-149 mM amino acid component; 0.01-1.2 mg/mL surfactant; 10-129 mM tonicifier; and a diluent.

D287. 120-220 mg/mL secukinumab; 1-9 mM buffer system; 20-99 mM amino acid component; 0.01-1.2 mg/mL surfactant; 10-129 mM ionic strength provider; and a diluent.

D288. 120-220 mg/mL secukinumab; 51-60 mM buffer system; 20-99 mM amino acid component; 0.01-1.2 mg/mL surfactant; 10-129 mM ionic strength provider; and a diluent.

D289. 120-220 mg/mL secukinumab; 1-9 mM buffer system; 100-149 mM amino acid component; 0.01-1.2 mg/mL surfactant; 10-129 mM ionic strength provider; and a diluent.

D290. 120-220 mg/mL secukinumab; 51-60 mM buffer system; 100-149 mM amino acid component; 0.01-1.2 mg/mL surfactant; 10-129 mM ionic strength provider; and a diluent.

D291. 120-220 mg/mL secukinumab; 1-9 mM buffer system; 20-99 mM amino acid component; 0.01-1.2 mg/mL surfactant; 0.1-20 mM antioxidant; and a diluent.

D292. 120-220 mg/mL secukinumab; 51-60 mM buffer system; 20-99 mM amino acid component; 0.01-1.2 mg/mL surfactant; 0.1-20 mM antioxidant; and a diluent.

D293. 120-220 mg/mL secukinumab; 1-9 mM buffer system; 100-149 mM amino acid component; 0.01-1.2 mg/mL surfactant; 0.1-20 mM antioxidant; and a diluent.

(continued)

D294. 120-220 mg/mL secukinumab; 51-60 mM buffer system; 100-149 mM amino acid component; 0.01-1.2 mg/mL surfactant; 0.1-20 mM antioxidant; and a diluent.

D295. 120-220 mg/mL secukinumab; 1-9 mM buffer system; 20-99 mM amino acid component; 0.01-1.2 mg/mL surfactant; 0.001-2 mM chelator; and a diluent.

D296. 120-220 mg/mL secukinumab; 51-60 mM buffer system; 20-99 mM amino acid component; 0.01-1.2 mg/mL surfactant; 0.001-2 mM chelator; and a diluent.

D297. 120-220 mg/mL secukinumab; 1-9 mM buffer system; 100-149 mM amino acid component; 0.01-1.2 mg/mL surfactant; 0.001-2 mM chelator; and a diluent.

D298. 120-220 mg/mL secukinumab; 51-60 mM buffer system; 100-149 mM amino acid component; 0.01-1.2 mg/mL surfactant; 0.001-2 mM chelator; and a diluent.

D299. 120-220 mg/mL secukinumab; 1-9 mM buffer system; 20-99 mM amino acid component; 10-129 mM tonicifier; 10-129 mM ionic strength provider; and a diluent.

D300. 120-220 mg/mL secukinumab; 51-60 mM buffer system; 20-99 mM amino acid component; 10-129 mM tonicifier; 10-129 mM ionic strength provider; and a diluent.

D301. 120-220 mg/mL secukinumab; 1-9 mM buffer system; 100-149 mM amino acid component; 10-129 mM tonicifier; 10-129 mM ionic strength provider; and a diluent.

D302. 120-220 mg/mL secukinumab; 51-60 mM buffer system; 100-149 mM amino acid component; 10-129 mM tonicifier; 10-129 mM ionic strength provider; and a diluent.

D303. 120-220 mg/mL secukinumab; 1-9 mM buffer system; 20-99 mM amino acid component; 10-129 mM tonicifier; 0.1-20 mM antioxidant; and a diluent.

D304. 120-220 mg/mL secukinumab; 51-60 mM buffer system; 20-99 mM amino acid component; 10-129 mM tonicifier; 0.1-20 mM antioxidant; and a diluent.

D305. 120-220 mg/mL secukinumab; 1-9 mM buffer system; 100-149 mM amino acid component; 10-129 mM tonicifier; 0.1-20 mM antioxidant; and a diluent.

D306. 120-220 mg/mL secukinumab; 51-60 mM buffer system; 100-149 mM amino acid component; 10-129 mM tonicifier; 0.1-20 mM antioxidant; and a diluent.

D307. 120-220 mg/mL secukinumab; 1-9 mM buffer system; 20-99 mM amino acid component; 10-129 mM tonicifier; 0.001-2 mM chelator; and a diluent.

D308. 120-220 mg/mL secukinumab; 51-60 mM buffer system; 20-99 mM amino acid component; 10-129 mM tonicifier; 0.001-2 mM chelator; and a diluent.

D309. 120-220 mg/mL secukinumab; 1-9 mM buffer system; 100-149 mM amino acid component; 10-129 mM tonicifier; 0.001-2 mM chelator; and a diluent.

D310. 120-220 mg/mL secukinumab; 51-60 mM buffer system; 100-149 mM amino acid component; 10-129 mM tonicifier; 0.001-2 mM chelator; and a diluent.

D311. 120-220 mg/mL secukinumab; 1-9 mM buffer system; 20-99 mM amino acid component; 10-129 mM ionic strength provider; 0.1-20 mM antioxidant; and a diluent.

D312. 120-220 mg/mL secukinumab; 51-60 mM buffer system; 20-99 mM amino acid component; 10-129 mM ionic strength provider; 0.1-20 mM antioxidant; and a diluent.

D313. 120-220 mg/mL secukinumab; 1-9 mM buffer system; 100-149 mM amino acid component; 10-129 mM ionic strength provider; 0.1-20 mM antioxidant; and a diluent.

D314. 120-220 mg/mL secukinumab; 51-60 mM buffer system; 100-149 mM amino acid component; 10-129 mM ionic strength provider; 0.1-20 mM antioxidant; and a diluent.

D315. 120-220 mg/mL secukinumab; 1-9 mM buffer system; 20-99 mM amino acid component; 10-129 mM ionic strength provider; 0.001-2 mM chelator; and a diluent.

D316. 120-220 mg/mL secukinumab; 51-60 mM buffer system; 20-99 mM amino acid component; 10-129 mM ionic strength provider; 0.001-2 mM chelator; and a diluent.

D317. 120-220 mg/mL secukinumab; 1-9 mM buffer system; 100-149 mM amino acid component; 10-129 mM ionic strength provider; 0.001-2 mM chelator; and a diluent.

D318. 120-220 mg/mL secukinumab; 51-60 mM buffer system; 100-149 mM amino acid component; 10-129 mM ionic strength provider; 0.001-2 mM chelator; and a diluent.

D319. 120-220 mg/mL secukinumab; 1-9 mM buffer system; 20-99 mM amino acid component; 0.1-20 mM antioxidant; 0.001-2 mM chelator; and a diluent.

(continued)

| | |
|---|---|
| D320. | 120-220 mg/mL secukinumab; 51-60 mM buffer system; 20-99 mM amino acid component; 0.1-20 mM antioxidant; 0.001-2 mM chelator; and a diluent. |
| D321. | 120-220 mg/mL secukinumab; 1-9 mM buffer system; 100-149 mM amino acid component; 0.1-20 mM antioxidant; 0.001-2 mM chelator; and a diluent. |
| D322. | 120-220 mg/mL secukinumab; 51-60 mM buffer system; 100-149 mM amino acid component; 0.1-20 mM antioxidant; 0.001-2 mM chelator; and a diluent. |
| D323. | 120-220 mg/mL secukinumab; 1-9 mM buffer system; 0.01-1.2 mg/mL surfactant; 10-129 mM tonicifier; 10-129 mM ionic strength provider; and a diluent. |
| D324. | 120-220 mg/mL secukinumab; 51-60 mM buffer system; 0.01-1.2 mg/mL surfactant; 10-129 mM tonicifier; 10-129 mM ionic strength provider; and a diluent. |
| D325. | 120-220 mg/mL secukinumab; 1-9 mM buffer system; 0.01-1.2 mg/mL surfactant; 10-129 mM tonicifier; 0.1-20 mM antioxidant; and a diluent. |
| D326. | 120-220 mg/mL secukinumab; 51-60 mM buffer system; 0.01-1.2 mg/mL surfactant; 10-129 mM tonicifier; 0.1-20 mM antioxidant; and a diluent. |
| D327. | 120-220 mg/mL secukinumab; 1-9 mM buffer system; 0.01-1.2 mg/mL surfactant; 10-129 mM tonicifier; 0.001-2 mM chelator; and a diluent. |
| D328. | 120-220 mg/mL secukinumab; 51-60 mM buffer system; 0.01-1.2 mg/mL surfactant; 10-129 mM tonicifier; 0.001-2 mM chelator; and a diluent. |
| D329. | 120-220 mg/mL secukinumab; 1-9 mM buffer system; 0.01-1.2 mg/mL surfactant; 10-129 mM ionic strength provider; 0.1-20 mM antioxidant; and a diluent. |
| D330. | 120-220 mg/mL secukinumab; 51-60 mM buffer system; 0.01-1.2 mg/mL surfactant; 10-129 mM ionic strength provider; 0.1-20 mM antioxidant; and a diluent. |
| D331. | 120-220 mg/mL secukinumab; 1-9 mM buffer system; 0.01-1.2 mg/mL surfactant; 10-129 mM ionic strength provider; 0.001-2 mM chelator; and a diluent. |
| D332. | 120-220 mg/mL secukinumab; 51-60 mM buffer system; 0.01-1.2 mg/mL surfactant; 10-129 mM ionic strength provider; 0.001-2 mM chelator; and a diluent. |
| D333. | 120-220 mg/mL secukinumab; 1-9 mM buffer system; 0.01-1.2 mg/mL surfactant; 0.1-20 mM antioxidant; 0.001-2 mM chelator; and a diluent. |
| D334. | 120-220 mg/mL secukinumab; 51-60 mM buffer system; 0.01-1.2 mg/mL surfactant; 0.1-20 mM antioxidant; 0.001-2 mM chelator; and a diluent. |
| D335. | 120-220 mg/mL secukinumab; 1-9 mM buffer system; 10-129 mM tonicifier; 10-129 mM ionic strength provider; 0.1-20 mM antioxidant; and a diluent. |
| D336. | 120-220 mg/mL secukinumab; 51-60 mM buffer system; 10-129 mM tonicifier; 10-129 mM ionic strength provider; 0.1-20 mM antioxidant; and a diluent. |
| D337. | 120-220 mg/mL secukinumab; 1-9 mM buffer system; 10-129 mM tonicifier; 10-129 mM ionic strength provider; 0.001-2 mM chelator; and a diluent. |
| D338. | 120-220 mg/mL secukinumab; 51-60 mM buffer system; 10-129 mM tonicifier; 10-129 mM ionic strength provider; 0.001-2 mM chelator; and a diluent. |
| D339. | 120-220 mg/mL secukinumab; 1-9 mM buffer system; 10-129 mM tonicifier; 0.1-20 mM antioxidant; 0.001-2 mM chelator; and a diluent. |
| D340. | 120-220 mg/mL secukinumab; 51-60 mM buffer system; 10-129 mM tonicifier; 0.1-20 mM antioxidant; 0.001-2 mM chelator; and a diluent. |
| D341. | 120-220 mg/mL secukinumab; 1-9 mM buffer system; 10-129 mM ionic strength provider; 0.1-20 mM antioxidant; 0.001-2 mM chelator; and a diluent. |
| D342. | 120-220 mg/mL secukinumab; 51-60 mM buffer system; 10-129 mM ionic strength provider; 0.1-20 mM antioxidant; 0.001-2 mM chelator; and a diluent. |
| D343. | 120-220 mg/mL secukinumab; 50-129 mM sugar component; 20-99 mM amino acid component; 0.01-1.2 mg/mL surfactant; 10-129 mM tonicifier; and a diluent. |
| D344. | 120-220 mg/mL secukinumab; 130-174 mM sugar component; 20-99 mM amino acid component; 0.01-1.2 mg/mL surfactant; 10-129 mM tonicifier; and a diluent. |
| D345. | 120-220 mg/mL secukinumab; 50-129 mM sugar component; 100-149 mM amino acid component; 0.01-1.2 mg/mL surfactant; 10-129 mM tonicifier; and a diluent. |

(continued)

D346. 120-220 mg/mL secukinumab; 130-174 mM sugar component; 100-149 mM amino acid component; 0.01-1.2 mg/mL surfactant; 10-129 mM tonicifier; and a diluent.

D347. 120-220 mg/mL secukinumab; 50-129 mM sugar component; 20-99 mM amino acid component; 0.01-1.2 mg/mL surfactant; 10-129 mM ionic strength provider; and a diluent.

D348. 120-220 mg/mL secukinumab; 130-174 mM sugar component; 20-99 mM amino acid component; 0.01-1.2 mg/mL surfactant; 10-129 mM ionic strength provider; and a diluent.

D349. 120-220 mg/mL secukinumab; 50-129 mM sugar component; 100-149 mM amino acid component; 0.01-1.2 mg/mL surfactant; 10-129 mM ionic strength provider; and a diluent.

D350. 120-220 mg/mL secukinumab; 130-174 mM sugar component; 100-149 mM amino acid component; 0.01-1.2 mg/mL surfactant; 10-129 mM ionic strength provider; and a diluent.

D351. 120-220 mg/mL secukinumab; 50-129 mM sugar component; 20-99 mM amino acid component; 0.01-1.2 mg/mL surfactant; 0.1-20 mM antioxidant; and a diluent.

D352. 120-220 mg/mL secukinumab; 130-174 mM sugar component; 20-99 mM amino acid component; 0.01-1.2 mg/mL surfactant; 0.1-20 mM antioxidant; and a diluent.

D353. 120-220 mg/mL secukinumab; 50-129 mM sugar component; 100-149 mM amino acid component; 0.01-1.2 mg/mL surfactant; 0.1-20 mM antioxidant; and a diluent.

D354. 120-220 mg/mL secukinumab; 130-174 mM sugar component; 100-149 mM amino acid component; 0.01-1.2 mg/mL surfactant; 0.1-20 mM antioxidant; and a diluent.

D355. 120-220 mg/mL secukinumab; 50-129 mM sugar component; 20-99 mM amino acid component; 0.01-1.2 mg/mL surfactant; 0.001-2 mM chelator; and a diluent.

D356. 120-220 mg/mL secukinumab; 130-174 mM sugar component; 20-99 mM amino acid component; 0.01-1.2 mg/mL surfactant; 0.001-2 mM chelator; and a diluent.

D357. 120-220 mg/mL secukinumab; 50-129 mM sugar component; 100-149 mM amino acid component; 0.01-1.2 mg/mL surfactant; 0.001-2 mM chelator; and a diluent.

D358. 120-220 mg/mL secukinumab; 130-174 mM sugar component; 100-149 mM amino acid component; 0.01-1.2 mg/mL surfactant; 0.001-2 mM chelator; and a diluent.

D359. 120-220 mg/mL secukinumab; 50-129 mM sugar component; 20-99 mM amino acid component; 10-129 mM tonicifier; 10-129 mM ionic strength provider; and a diluent.

D360. 120-220 mg/mL secukinumab; 130-174 mM sugar component; 20-99 mM amino acid component; 10-129 mM tonicifier; 10-129 mM ionic strength provider; and a diluent.

D361. 120-220 mg/mL secukinumab; 50-129 mM sugar component; 100-149 mM amino acid component; 10-129 mM tonicifier; 10-129 mM ionic strength provider; and a diluent.

D362. 120-220 mg/mL secukinumab; 130-174 mM sugar component; 100-149 mM amino acid component; 10-129 mM tonicifier; 10-129 mM ionic strength provider; and a diluent.

D363. 120-220 mg/mL secukinumab; 50-129 mM sugar component; 20-99 mM amino acid component; 10-129 mM tonicifier; 0.1-20 mM antioxidant; and a diluent.

D364. 120-220 mg/mL secukinumab; 130-174 mM sugar component; 20-99 mM amino acid component; 10-129 mM tonicifier; 0.1-20 mM antioxidant; and a diluent.

D365. 120-220 mg/mL secukinumab; 50-129 mM sugar component; 100-149 mM amino acid component; 10-129 mM tonicifier; 0.1-20 mM antioxidant; and a diluent.

D366. 120-220 mg/mL secukinumab; 130-174 mM sugar component; 100-149 mM amino acid component; 10-129 mM tonicifier; 0.1-20 mM antioxidant; and a diluent.

D367. 120-220 mg/mL secukinumab; 50-129 mM sugar component; 20-99 mM amino acid component; 10-129 mM tonicifier; 0.001-2 mM chelator; and a diluent.

D368. 120-220 mg/mL secukinumab; 130-174 mM sugar component; 20-99 mM amino acid component; 10-129 mM tonicifier; 0.001-2 mM chelator; and a diluent.

D369. 120-220 mg/mL secukinumab; 50-129 mM sugar component; 100-149 mM amino acid component; 10-129 mM tonicifier; 0.001-2 mM chelator; and a diluent.

D370. 120-220 mg/mL secukinumab; 130-174 mM sugar component; 100-149 mM amino acid component; 10-129 mM tonicifier; 0.001-2 mM chelator; and a diluent.

D371. 120-220 mg/mL secukinumab; 50-129 mM sugar component; 20-99 mM amino acid component; 10-129 mM ionic strength provider; 0.1-20 mM antioxidant; and a diluent.

(continued)

D372. 120-220 mg/mL secukinumab; 130-174 mM sugar component; 20-99 mM amino acid component; 10-129 mM ionic strength provider; 0.1-20 mM antioxidant; and a diluent.

D373. 120-220 mg/mL secukinumab; 50-129 mM sugar component; 100-149 mM amino acid component; 10-129 mM ionic strength provider; 0.1-20 mM antioxidant; and a diluent.

D374. 120-220 mg/mL secukinumab; 130-174 mM sugar component; 100-149 mM amino acid component; 10-129 mM ionic strength provider; 0.1-20 mM antioxidant; and a diluent.

D375. 120-220 mg/mL secukinumab; 50-129 mM sugar component; 20-99 mM amino acid component; 10-129 mM ionic strength provider; 0.001-2 mM chelator; and a diluent.

D376. 120-220 mg/mL secukinumab; 130-174 mM sugar component; 20-99 mM amino acid component; 10-129 mM ionic strength provider; 0.001-2 mM chelator; and a diluent.

D377. 120-220 mg/mL secukinumab; 50-129 mM sugar component; 100-149 mM amino acid component; 10-129 mM ionic strength provider; 0.001-2 mM chelator; and a diluent.

D378. 120-220 mg/mL secukinumab; 130-174 mM sugar component; 100-149 mM amino acid component; 10-129 mM ionic strength provider; 0.001-2 mM chelator; and a diluent.

D379. 120-220 mg/mL secukinumab; 50-129 mM sugar component; 20-99 mM amino acid component; 0.1-20 mM antioxidant; 0.001-2 mM chelator; and a diluent.

D380. 120-220 mg/mL secukinumab; 130-174 mM sugar component; 20-99 mM amino acid component; 0.1-20 mM antioxidant; 0.001-2 mM chelator; and a diluent.

D381. 120-220 mg/mL secukinumab; 50-129 mM sugar component; 100-149 mM amino acid component; 0.1-20 mM antioxidant; 0.001-2 mM chelator; and a diluent.

D382. 120-220 mg/mL secukinumab; 130-174 mM sugar component; 100-149 mM amino acid component; 0.1-20 mM antioxidant; 0.001-2 mM chelator; and a diluent.

D383. 120-220 mg/mL secukinumab; 50-129 mM sugar component; 0.01-1.2 mg/mL surfactant; 10-129 mM tonicifier; 10-129 mM ionic strength provider; and a diluent.

D384. 120-220 mg/mL secukinumab; 130-174 mM sugar component; 0.01-1.2 mg/mL surfactant; 10-129 mM tonicifier; 10-129 mM ionic strength provider; and a diluent.

D385. 120-220 mg/mL secukinumab; 50-129 mM sugar component; 0.01-1.2 mg/mL surfactant; 10-129 mM tonicifier; 0.1-20 mM antioxidant; and a diluent.

D386. 120-220 mg/mL secukinumab; 130-174 mM sugar component; 0.01-1.2 mg/mL surfactant; 10-129 mM tonicifier; 0.1-20 mM antioxidant; and a diluent.

D387. 120-220 mg/mL secukinumab; 50-129 mM sugar component; 0.01-1.2 mg/mL surfactant; 10-129 mM tonicifier; 0.001-2 mM chelator; and a diluent.

D388. 120-220 mg/mL secukinumab; 130-174 mM sugar component; 0.01-1.2 mg/mL surfactant; 10-129 mM tonicifier; 0.001-2 mM chelator; and a diluent.

D389. 120-220 mg/mL secukinumab; 50-129 mM sugar component; 0.01-1.2 mg/mL surfactant; 10-129 mM ionic strength provider; 0.1-20 mM antioxidant; and a diluent.

D390. 120-220 mg/mL secukinumab; 130-174 mM sugar component; 0.01-1.2 mg/mL surfactant; 10-129 mM ionic strength provider; 0.1-20 mM antioxidant; and a diluent.

D391. 120-220 mg/mL secukinumab; 50-129 mM sugar component; 0.01-1.2 mg/mL surfactant; 10-129 mM ionic strength provider; 0.001-2 mM chelator; and a diluent.

D392. 120-220 mg/mL secukinumab; 130-174 mM sugar component; 0.01-1.2 mg/mL surfactant; 10-129 mM ionic strength provider; 0.001-2 mM chelator; and a diluent.

D393. 120-220 mg/mL secukinumab; 50-129 mM sugar component; 0.01-1.2 mg/mL surfactant; 0.1-20 mM antioxidant; 0.001-2 mM chelator; and a diluent.

D394. 120-220 mg/mL secukinumab; 130-174 mM sugar component; 0.01-1.2 mg/mL surfactant; 0.1-20 mM antioxidant; 0.001-2 mM chelator; and a diluent.

D395. 120-220 mg/mL secukinumab; 50-129 mM sugar component; 10-129 mM tonicifier; 10-129 mM ionic strength provider; 0.1-20 mM antioxidant; and a diluent.

D396. 120-220 mg/mL secukinumab; 130-174 mM sugar component; 10-129 mM tonicifier; 10-129 mM ionic strength provider; 0.1-20 mM antioxidant; and a diluent.

D397. 120-220 mg/mL secukinumab; 50-129 mM sugar component; 10-129 mM tonicifier; 10-129 mM ionic strength provider; 0.001-2 mM chelator; and a diluent.

(continued)

D398. 120-220 mg/mL secukinumab; 130-174 mM sugar component; 10-129 mM tonicifier; 10-129 mM ionic strength provider; 0.001-2 mM chelator; and a diluent.

D399. 120-220 mg/mL secukinumab; 50-129 mM sugar component; 10-129 mM tonicifier; 0.1-20 mM antioxidant; 0.001-2 mM chelator; and a diluent.

D400. 120-220 mg/mL secukinumab; 130-174 mM sugar component; 10-129 mM tonicifier; 0.1-20 mM antioxidant; 0.001-2 mM chelator; and a diluent.

D401. 120-220 mg/mL secukinumab; 50-129 mM sugar component; 10-129 mM ionic strength provider; 0.1-20 mM antioxidant; 0.001-2 mM chelator; and a diluent.

D402. 120-220 mg/mL secukinumab; 130-174 mM sugar component; 10-129 mM ionic strength provider; 0.1-20 mM antioxidant; 0.001-2 mM chelator; and a diluent.

D403. 120-220 mg/mL secukinumab; 20-99 mM amino acid component; 0.01-1.2 mg/mL surfactant; 10-129 mM tonicifier; 10-129 mM ionic strength provider; and a diluent.

D404. 120-220 mg/mL secukinumab; 100-149 mM amino acid component; 0.01-1.2 mg/mL surfactant; 10-129 mM tonicifier; 10-129 mM ionic strength provider; and a diluent.

D405. 120-220 mg/mL secukinumab; 20-99 mM amino acid component; 0.01-1.2 mg/mL surfactant; 10-129 mM tonicifier; 0.1-20 mM antioxidant; and a diluent.

D406. 120-220 mg/mL secukinumab; 100-149 mM amino acid component; 0.01-1.2 mg/mL surfactant; 10-129 mM tonicifier; 0.1-20 mM antioxidant; and a diluent.

D407. 120-220 mg/mL secukinumab; 20-99 mM amino acid component; 0.01-1.2 mg/mL surfactant; 10-129 mM tonicifier; 0.001-2 mM chelator; and a diluent.

D408. 120-220 mg/mL secukinumab; 100-149 mM amino acid component; 0.01-1.2 mg/mL surfactant; 10-129 mM tonicifier; 0.001-2 mM chelator; and a diluent.

D409. 120-220 mg/mL secukinumab; 20-99 mM amino acid component; 0.01-1.2 mg/mL surfactant; 10-129 mM ionic strength provider; 0.1-20 mM antioxidant; and a diluent.

D410. 120-220 mg/mL secukinumab; 100-149 mM amino acid component; 0.01-1.2 mg/mL surfactant; 10-129 mM ionic strength provider; 0.1-20 mM antioxidant; and a diluent.

D411. 120-220 mg/mL secukinumab; 20-99 mM amino acid component; 0.01-1.2 mg/mL surfactant; 10-129 mM ionic strength provider; 0.001-2 mM chelator; and a diluent.

D412. 120-220 mg/mL secukinumab; 100-149 mM amino acid component; 0.01-1.2 mg/mL surfactant; 10-129 mM ionic strength provider; 0.001-2 mM chelator; and a diluent.

D413. 120-220 mg/mL secukinumab; 20-99 mM amino acid component; 0.01-1.2 mg/mL surfactant; 0.1-20 mM antioxidant; 0.001-2 mM chelator; and a diluent.

D414. 120-220 mg/mL secukinumab; 100-149 mM amino acid component; 0.01-1.2 mg/mL surfactant; 0.1-20 mM antioxidant; 0.001-2 mM chelator; and a diluent.

D415. 120-220 mg/mL secukinumab; 20-99 mM amino acid component; 10-129 mM tonicifier; 10-129 mM ionic strength provider; 0.1-20 mM antioxidant; and a diluent.

D416. 120-220 mg/mL secukinumab; 100-149 mM amino acid component; 10-129 mM tonicifier; 10-129 mM ionic strength provider; 0.1-20 mM antioxidant; and a diluent.

D417. 120-220 mg/mL secukinumab; 20-99 mM amino acid component; 10-129 mM tonicifier; 10-129 mM ionic strength provider; 0.001-2 mM chelator; and a diluent.

D418. 120-220 mg/mL secukinumab; 100-149 mM amino acid component; 10-129 mM tonicifier; 10-129 mM ionic strength provider; 0.001-2 mM chelator; and a diluent.

D419. 120-220 mg/mL secukinumab; 20-99 mM amino acid component; 10-129 mM tonicifier; 0.1-20 mM antioxidant; 0.001-2 mM chelator; and a diluent.

D420. 120-220 mg/mL secukinumab; 100-149 mM amino acid component; 10-129 mM tonicifier; 0.1-20 mM antioxidant; 0.001-2 mM chelator; and a diluent.

D421. 120-220 mg/mL secukinumab; 20-99 mM amino acid component; 10-129 mM ionic strength provider; 0.1-20 mM antioxidant; 0.001-2 mM chelator; and a diluent.

D422. 120-220 mg/mL secukinumab; 100-149 mM amino acid component; 10-129 mM ionic strength provider; 0.1-20 mM antioxidant; 0.001-2 mM chelator; and a diluent.

D423. 120-220 mg/mL secukinumab; 0.01-1.2 mg/mL surfactant; 10-129 mM tonicifier; 10-129 mM ionic strength provider; 0.1-20 mM antioxidant; and a diluent.

(continued)

D424. 120-220 mg/mL secukinumab; 0.01-1.2 mg/mL surfactant; 10-129 mM tonicifier; 10-129 mM ionic strength provider; 0.001-2 mM chelator; and a diluent.

D425. 120-220 mg/mL secukinumab; 0.01-1.2 mg/mL surfactant; 10-129 mM tonicifier; 0.1-20 mM antioxidant; 0.001-2 mM chelator; and a diluent.

D426. 120-220 mg/mL secukinumab; 0.01-1.2 mg/mL surfactant; 10-129 mM ionic strength provider; 0.1-20 mM antioxidant; 0.001-2 mM chelator; and a diluent.

D427. 120-220 mg/mL secukinumab; 10-129 mM tonicifier; 10-129 mM ionic strength provider; 0.1-20 mM antioxidant; 0.001-2 mM chelator; and a diluent.

D428. 120-220 mg/mL secukinumab; 1-9 mM buffer system; 50-129 mM sugar component; 20-99 mM amino acid component; 0.01-1.2 mg/mL surfactant; 10-129 mM tonicifier; and a diluent.

D429. 120-220 mg/mL secukinumab; 51-60 mM buffer system; 50-129 mM sugar component; 20-99 mM amino acid component; 0.01-1.2 mg/mL surfactant; 10-129 mM tonicifier; and a diluent.

D430. 120-220 mg/mL secukinumab; 1-9 mM buffer system; 130-174 mM sugar component; 20-99 mM amino acid component; 0.01-1.2 mg/mL surfactant; 10-129 mM tonicifier; and a diluent.

D431. 120-220 mg/mL secukinumab; 51-60 mM buffer system; 130-174 mM sugar component; 20-99 mM amino acid component; 0.01-1.2 mg/mL surfactant; 10-129 mM tonicifier; and a diluent.

D432. 120-220 mg/mL secukinumab; 1-9 mM buffer system; 50-129 mM sugar component; 100-149 mM amino acid component; 0.01-1.2 mg/mL surfactant; 10-129 mM tonicifier; and a diluent.

D433. 120-220 mg/mL secukinumab; 51-60 mM buffer system; 50-129 mM sugar component; 100-149 mM amino acid component; 0.01-1.2 mg/mL surfactant; 10-129 mM tonicifier; and a diluent.

D434. 120-220 mg/mL secukinumab; 1-9 mM buffer system; 130-174 mM sugar component; 100-149 mM amino acid component; 0.01-1.2 mg/mL surfactant; 10-129 mM tonicifier; and a diluent.

D435. 120-220 mg/mL secukinumab; 51-60 mM buffer system; 130-174 mM sugar component; 100-149 mM amino acid component; 0.01-1.2 mg/mL surfactant; 10-129 mM tonicifier; and a diluent.

D436. 120-220 mg/mL secukinumab; 1-9 mM buffer system; 50-129 mM sugar component; 20-99 mM amino acid component; 0.01-1.2 mg/mL surfactant; 10-129 mM ionic strength provider; and a diluent.

D437. 120-220 mg/mL secukinumab; 51-60 mM buffer system; 50-129 mM sugar component; 20-99 mM amino acid component; 0.01-1.2 mg/mL surfactant; 10-129 mM ionic strength provider; and a diluent.

D438. 120-220 mg/mL secukinumab; 1-9 mM buffer system; 130-174 mM sugar component; 20-99 mM amino acid component; 0.01-1.2 mg/mL surfactant; 10-129 mM ionic strength provider; and a diluent.

D439. 120-220 mg/mL secukinumab; 51-60 mM buffer system; 130-174 mM sugar component; 20-99 mM amino acid component; 0.01-1.2 mg/mL surfactant; 10-129 mM ionic strength provider; and a diluent.

D440. 120-220 mg/mL secukinumab; 1-9 mM buffer system; 50-129 mM sugar component; 100-149 mM amino acid component; 0.01-1.2 mg/mL surfactant; 10-129 mM ionic strength provider; and a diluent.

D441. 120-220 mg/mL secukinumab; 51-60 mM buffer system; 50-129 mM sugar component; 100-149 mM amino acid component; 0.01-1.2 mg/mL surfactant; 10-129 mM ionic strength provider; and a diluent.

D442. 120-220 mg/mL secukinumab; 1-9 mM buffer system; 130-174 mM sugar component; 100-149 mM amino acid component; 0.01-1.2 mg/mL surfactant; 10-129 mM ionic strength provider; and a diluent.

D443. 120-220 mg/mL secukinumab; 51-60 mM buffer system; 130-174 mM sugar component; 100-149 mM amino acid component; 0.01-1.2 mg/mL surfactant; 10-129 mM ionic strength provider; and a diluent.

D444. 120-220 mg/mL secukinumab; 1-9 mM buffer system; 50-129 mM sugar component; 20-99 mM amino acid component; 0.01-1.2 mg/mL surfactant; 0.1-20 mM antioxidant; and a diluent.

D445. 120-220 mg/mL secukinumab; 51-60 mM buffer system; 50-129 mM sugar component; 20-99 mM amino acid component; 0.01-1.2 mg/mL surfactant; 0.1-20 mM antioxidant; and a diluent.

D446. 120-220 mg/mL secukinumab; 1-9 mM buffer system; 130-174 mM sugar component; 20-99 mM amino acid component; 0.01-1.2 mg/mL surfactant; 0.1-20 mM antioxidant; and a diluent.

D447. 120-220 mg/mL secukinumab; 51-60 mM buffer system; 130-174 mM sugar component; 20-99 mM amino acid component; 0.01-1.2 mg/mL surfactant; 0.1-20 mM antioxidant; and a diluent.

D448. 120-220 mg/mL secukinumab; 1-9 mM buffer system; 50-129 mM sugar component; 100-149 mM amino acid component; 0.01-1.2 mg/mL surfactant; 0.1-20 mM antioxidant; and a diluent.

D449. 120-220 mg/mL secukinumab; 51-60 mM buffer system; 50-129 mM sugar component; 100-149 mM amino acid component; 0.01-1.2 mg/mL surfactant; 0.1-20 mM antioxidant; and a diluent.

(continued)

D450. 120-220 mg/mL secukinumab; 1-9 mM buffer system; 130-174 mM sugar component; 100-149 mM amino acid component; 0.01-1.2 mg/mL surfactant; 0.1-20 mM antioxidant; and a diluent.

D451. 120-220 mg/mL secukinumab; 51-60 mM buffer system; 130-174 mM sugar component; 100-149 mM amino acid component; 0.01-1.2 mg/mL surfactant; 0.1-20 mM antioxidant; and a diluent.

D452. 120-220 mg/mL secukinumab; 1-9 mM buffer system; 50-129 mM sugar component; 20-99 mM amino acid component; 0.01-1.2 mg/mL surfactant; 0.001-2 mM chelator; and a diluent.

D453. 120-220 mg/mL secukinumab; 51-60 mM buffer system; 50-129 mM sugar component; 20-99 mM amino acid component; 0.01-1.2 mg/mL surfactant; 0.001-2 mM chelator; and a diluent.

D454. 120-220 mg/mL secukinumab; 1-9 mM buffer system; 130-174 mM sugar component; 20-99 mM amino acid component; 0.01-1.2 mg/mL surfactant; 0.001-2 mM chelator; and a diluent.

D455. 120-220 mg/mL secukinumab; 51-60 mM buffer system; 130-174 mM sugar component; 20-99 mM amino acid component; 0.01-1.2 mg/mL surfactant; 0.001-2 mM chelator; and a diluent.

D456. 120-220 mg/mL secukinumab; 1-9 mM buffer system; 50-129 mM sugar component; 100-149 mM amino acid component; 0.01-1.2 mg/mL surfactant; 0.001-2 mM chelator; and a diluent.

D457. 120-220 mg/mL secukinumab; 51-60 mM buffer system; 50-129 mM sugar component; 100-149 mM amino acid component; 0.01-1.2 mg/mL surfactant; 0.001-2 mM chelator; and a diluent.

D458. 120-220 mg/mL secukinumab; 1-9 mM buffer system; 130-174 mM sugar component; 100-149 mM amino acid component; 0.01-1.2 mg/mL surfactant; 0.001-2 mM chelator; and a diluent.

D459. 120-220 mg/mL secukinumab; 51-60 mM buffer system; 130-174 mM sugar component; 100-149 mM amino acid component; 0.01-1.2 mg/mL surfactant; 0.001-2 mM chelator; and a diluent.

D460. 120-220 mg/mL secukinumab; 1-9 mM buffer system; 50-129 mM sugar component; 20-99 mM amino acid component; 10-129 mM tonicifier; 10-129 mM ionic strength provider; and a diluent.

D461. 120-220 mg/mL secukinumab; 51-60 mM buffer system; 50-129 mM sugar component; 20-99 mM amino acid component; 10-129 mM tonicifier; 10-129 mM ionic strength provider; and a diluent.

D462. 120-220 mg/mL secukinumab; 1-9 mM buffer system; 130-174 mM sugar component; 20-99 mM amino acid component; 10-129 mM tonicifier; 10-129 mM ionic strength provider; and a diluent.

D463. 120-220 mg/mL secukinumab; 51-60 mM buffer system; 130-174 mM sugar component; 20-99 mM amino acid component; 10-129 mM tonicifier; 10-129 mM ionic strength provider; and a diluent.

D464. 120-220 mg/mL secukinumab; 1-9 mM buffer system; 50-129 mM sugar component; 100-149 mM amino acid component; 10-129 mM tonicifier; 10-129 mM ionic strength provider; and a diluent.

D465. 120-220 mg/mL secukinumab; 51-60 mM buffer system; 50-129 mM sugar component; 100-149 mM amino acid component; 10-129 mM tonicifier; 10-129 mM ionic strength provider; and a diluent.

D466. 120-220 mg/mL secukinumab; 1-9 mM buffer system; 130-174 mM sugar component; 100-149 mM amino acid component; 10-129 mM tonicifier; 10-129 mM ionic strength provider; and a diluent.

D467. 120-220 mg/mL secukinumab; 51-60 mM buffer system; 130-174 mM sugar component; 100-149 mM amino acid component; 10-129 mM tonicifier; 10-129 mM ionic strength provider; and a diluent.

D468. 120-220 mg/mL secukinumab; 1-9 mM buffer system; 50-129 mM sugar component; 20-99 mM amino acid component; 10-129 mM tonicifier; 0.1-20 mM antioxidant; and a diluent.

D469. 120-220 mg/mL secukinumab; 51-60 mM buffer system; 50-129 mM sugar component; 20-99 mM amino acid component; 10-129 mM tonicifier; 0.1-20 mM antioxidant; and a diluent.

D470. 120-220 mg/mL secukinumab; 1-9 mM buffer system; 130-174 mM sugar component; 20-99 mM amino acid component; 10-129 mM tonicifier; 0.1-20 mM antioxidant; and a diluent.

D471. 120-220 mg/mL secukinumab; 51-60 mM buffer system; 130-174 mM sugar component; 20-99 mM amino acid component; 10-129 mM tonicifier; 0.1-20 mM antioxidant; and a diluent.

D472. 120-220 mg/mL secukinumab; 1-9 mM buffer system; 50-129 mM sugar component; 100-149 mM amino acid component; 10-129 mM tonicifier; 0.1-20 mM antioxidant; and a diluent.

D473. 120-220 mg/mL secukinumab; 51-60 mM buffer system; 50-129 mM sugar component; 100-149 mM amino acid component; 10-129 mM tonicifier; 0.1-20 mM antioxidant; and a diluent.

D474. 120-220 mg/mL secukinumab; 1-9 mM buffer system; 130-174 mM sugar component; 100-149 mM amino acid component; 10-129 mM tonicifier; 0.1-20 mM antioxidant; and a diluent.

D475. 120-220 mg/mL secukinumab; 51-60 mM buffer system; 130-174 mM sugar component; 100-149 mM amino acid component; 10-129 mM tonicifier; 0.1-20 mM antioxidant; and a diluent.

(continued)

D476. 120-220 mg/mL secukinumab; 1-9 mM buffer system; 50-129 mM sugar component; 20-99 mM amino acid component; 10-129 mM tonicifier; 0.001-2 mM chelator; and a diluent.

D477. 120-220 mg/mL secukinumab; 51-60 mM buffer system; 50-129 mM sugar component; 20-99 mM amino acid component; 10-129 mM tonicifier; 0.001-2 mM chelator; and a diluent.

D478. 120-220 mg/mL secukinumab; 1-9 mM buffer system; 130-174 mM sugar component; 20-99 mM amino acid component; 10-129 mM tonicifier; 0.001-2 mM chelator; and a diluent.

D479. 120-220 mg/mL secukinumab; 51-60 mM buffer system; 130-174 mM sugar component; 20-99 mM amino acid component; 10-129 mM tonicifier; 0.001-2 mM chelator; and a diluent.

D480. 120-220 mg/mL secukinumab; 1-9 mM buffer system; 50-129 mM sugar component; 100-149 mM amino acid component; 10-129 mM tonicifier; 0.001-2 mM chelator; and a diluent.

D481. 120-220 mg/mL secukinumab; 51-60 mM buffer system; 50-129 mM sugar component; 100-149 mM amino acid component; 10-129 mM tonicifier; 0.001-2 mM chelator; and a diluent.

D482. 120-220 mg/mL secukinumab; 1-9 mM buffer system; 130-174 mM sugar component; 100-149 mM amino acid component; 10-129 mM tonicifier; 0.001-2 mM chelator; and a diluent.

D483. 120-220 mg/mL secukinumab; 51-60 mM buffer system; 130-174 mM sugar component; 100-149 mM amino acid component; 10-129 mM tonicifier; 0.001-2 mM chelator; and a diluent.

D484. 120-220 mg/mL secukinumab; 1-9 mM buffer system; 50-129 mM sugar component; 20-99 mM amino acid component; 10-129 mM ionic strength provider; 0.1-20 mM antioxidant; and a diluent.

D485. 120-220 mg/mL secukinumab; 51-60 mM buffer system; 50-129 mM sugar component; 20-99 mM amino acid component; 10-129 mM ionic strength provider; 0.1-20 mM antioxidant; and a diluent.

D486. 120-220 mg/mL secukinumab; 1-9 mM buffer system; 130-174 mM sugar component; 20-99 mM amino acid component; 10-129 mM ionic strength provider; 0.1-20 mM antioxidant; and a diluent.

D487. 120-220 mg/mL secukinumab; 51-60 mM buffer system; 130-174 mM sugar component; 20-99 mM amino acid component; 10-129 mM ionic strength provider; 0.1-20 mM antioxidant; and a diluent.

D488. 120-220 mg/mL secukinumab; 1-9 mM buffer system; 50-129 mM sugar component; 100-149 mM amino acid component; 10-129 mM ionic strength provider; 0.1-20 mM antioxidant; and a diluent.

D489. 120-220 mg/mL secukinumab; 51-60 mM buffer system; 50-129 mM sugar component; 100-149 mM amino acid component; 10-129 mM ionic strength provider; 0.1-20 mM antioxidant; and a diluent.

D490. 120-220 mg/mL secukinumab; 1-9 mM buffer system; 130-174 mM sugar component; 100-149 mM amino acid component; 10-129 mM ionic strength provider; 0.1-20 mM antioxidant; and a diluent.

D491. 120-220 mg/mL secukinumab; 51-60 mM buffer system; 130-174 mM sugar component; 100-149 mM amino acid component; 10-129 mM ionic strength provider; 0.1-20 mM antioxidant; and a diluent.

D492. 120-220 mg/mL secukinumab; 1-9 mM buffer system; 50-129 mM sugar component; 20-99 mM amino acid component; 10-129 mM ionic strength provider; 0.001-2 mM chelator; and a diluent.

D493. 120-220 mg/mL secukinumab; 51-60 mM buffer system; 50-129 mM sugar component; 20-99 mM amino acid component; 10-129 mM ionic strength provider; 0.001-2 mM chelator; and a diluent.

D494. 120-220 mg/mL secukinumab; 1-9 mM buffer system; 130-174 mM sugar component; 20-99 mM amino acid component; 10-129 mM ionic strength provider; 0.001-2 mM chelator; and a diluent.

D495. 120-220 mg/mL secukinumab; 51-60 mM buffer system; 130-174 mM sugar component; 20-99 mM amino acid component; 10-129 mM ionic strength provider; 0.001-2 mM chelator; and a diluent.

D496. 120-220 mg/mL secukinumab; 1-9 mM buffer system; 50-129 mM sugar component; 100-149 mM amino acid component; 10-129 mM ionic strength provider; 0.001-2 mM chelator; and a diluent.

D497. 120-220 mg/mL secukinumab; 51-60 mM buffer system; 50-129 mM sugar component; 100-149 mM amino acid component; 10-129 mM ionic strength provider; 0.001-2 mM chelator; and a diluent.

D498. 120-220 mg/mL secukinumab; 1-9 mM buffer system; 130-174 mM sugar component; 100-149 mM amino acid component; 10-129 mM ionic strength provider; 0.001-2 mM chelator; and a diluent.

D499. 120-220 mg/mL secukinumab; 51-60 mM buffer system; 130-174 mM sugar component; 100-149 mM amino acid component; 10-129 mM ionic strength provider; 0.001-2 mM chelator; and a diluent.

D500. 120-220 mg/mL secukinumab; 1-9 mM buffer system; 50-129 mM sugar component; 20-99 mM amino acid component; 0.1-20 mM antioxidant; 0.001-2 mM chelator; and a diluent.

D501. 120-220 mg/mL secukinumab; 51-60 mM buffer system; 50-129 mM sugar component; 20-99 mM amino acid component; 0.1-20 mM antioxidant; 0.001-2 mM chelator; and a diluent.

(continued)

| | |
|---|---|
| D502. | 120-220 mg/mL secukinumab; 1-9 mM buffer system; 130-174 mM sugar component; 20-99 mM amino acid component; 0.1-20 mM antioxidant; 0.001-2 mM chelator; and a diluent. |
| D503. | 120-220 mg/mL secukinumab; 51-60 mM buffer system; 130-174 mM sugar component; 20-99 mM amino acid component; 0.1-20 mM antioxidant; 0.001-2 mM chelator; and a diluent. |
| D504. | 120-220 mg/mL secukinumab; 1-9 mM buffer system; 50-129 mM sugar component; 100-149 mM amino acid component; 0.1-20 mM antioxidant; 0.001-2 mM chelator; and a diluent. |
| D505. | 120-220 mg/mL secukinumab; 51-60 mM buffer system; 50-129 mM sugar component; 100-149 mM amino acid component; 0.1-20 mM antioxidant; 0.001-2 mM chelator; and a diluent. |
| D506. | 120-220 mg/mL secukinumab; 1-9 mM buffer system; 130-174 mM sugar component; 100-149 mM amino acid component; 0.1-20 mM antioxidant; 0.001-2 mM chelator; and a diluent. |
| D507. | 120-220 mg/mL secukinumab; 51-60 mM buffer system; 130-174 mM sugar component; 100-149 mM amino acid component; 0.1-20 mM antioxidant; 0.001-2 mM chelator; and a diluent. |
| D508. | 120-220 mg/mL secukinumab; 1-9 mM buffer system; 50-129 mM sugar component; 0.01-1.2 mg/mL surfactant; 10-129 mM tonicifier; 10-129 mM ionic strength provider; and a diluent. |
| D509. | 120-220 mg/mL secukinumab; 51-60 mM buffer system; 50-129 mM sugar component; 0.01-1.2 mg/mL surfactant; 10-129 mM tonicifier; 10-129 mM ionic strength provider; and a diluent. |
| D510. | 120-220 mg/mL secukinumab; 1-9 mM buffer system; 130-174 mM sugar component; 0.01-1.2 mg/mL surfactant; 10-129 mM tonicifier; 10-129 mM ionic strength provider; and a diluent. |
| D511. | 120-220 mg/mL secukinumab; 51-60 mM buffer system; 130-174 mM sugar component; 0.01-1.2 mg/mL surfactant; 10-129 mM tonicifier; 10-129 mM ionic strength provider; and a diluent. |
| D512. | 120-220 mg/mL secukinumab; 1-9 mM buffer system; 50-129 mM sugar component; 0.01-1.2 mg/mL surfactant; 10-129 mM tonicifier; 0.1-20 mM antioxidant; and a diluent. |
| D513. | 120-220 mg/mL secukinumab; 51-60 mM buffer system; 50-129 mM sugar component; 0.01-1.2 mg/mL surfactant; 10-129 mM tonicifier; 0.1-20 mM antioxidant; and a diluent. |
| D514. | 120-220 mg/mL secukinumab; 1-9 mM buffer system; 130-174 mM sugar component; 0.01-1.2 mg/mL surfactant; 10-129 mM tonicifier; 0.1-20 mM antioxidant; and a diluent. |
| D515. | 120-220 mg/mL secukinumab; 51-60 mM buffer system; 130-174 mM sugar component; 0.01-1.2 mg/mL surfactant; 10-129 mM tonicifier; 0.1-20 mM antioxidant; and a diluent. |
| D516. | 120-220 mg/mL secukinumab; 1-9 mM buffer system; 50-129 mM sugar component; 0.01-1.2 mg/mL surfactant; 10-129 mM tonicifier; 0.001-2 mM chelator; and a diluent. |
| D517. | 120-220 mg/mL secukinumab; 51-60 mM buffer system; 50-129 mM sugar component; 0.01-1.2 mg/mL surfactant; 10-129 mM tonicifier; 0.001-2 mM chelator; and a diluent. |
| D518. | 120-220 mg/mL secukinumab; 1-9 mM buffer system; 130-174 mM sugar component; 0.01-1.2 mg/mL surfactant; 10-129 mM tonicifier; 0.001-2 mM chelator; and a diluent. |
| D519. | 120-220 mg/mL secukinumab; 51-60 mM buffer system; 130-174 mM sugar component; 0.01-1.2 mg/mL surfactant; 10-129 mM tonicifier; 0.001-2 mM chelator; and a diluent. |
| D520. | 120-220 mg/mL secukinumab; 1-9 mM buffer system; 50-129 mM sugar component; 0.01-1.2 mg/mL surfactant; 10-129 mM ionic strength provider; 0.1-20 mM antioxidant; and a diluent. |
| D521. | 120-220 mg/mL secukinumab; 51-60 mM buffer system; 50-129 mM sugar component; 0.01-1.2 mg/mL surfactant; 10-129 mM ionic strength provider; 0.1-20 mM antioxidant; and a diluent. |
| D522. | 120-220 mg/mL secukinumab; 1-9 mM buffer system; 130-174 mM sugar component; 0.01-1.2 mg/mL surfactant; 10-129 mM ionic strength provider; 0.1-20 mM antioxidant; and a diluent. |
| D523. | 120-220 mg/mL secukinumab; 51-60 mM buffer system; 130-174 mM sugar component; 0.01-1.2 mg/mL surfactant; 10-129 mM ionic strength provider; 0.1-20 mM antioxidant; and a diluent. |
| D524. | 120-220 mg/mL secukinumab; 1-9 mM buffer system; 50-129 mM sugar component; 0.01-1.2 mg/mL surfactant; 10-129 mM ionic strength provider; 0.001-2 mM chelator; and a diluent. |
| D525. | 120-220 mg/mL secukinumab; 51-60 mM buffer system; 50-129 mM sugar component; 0.01-1.2 mg/mL surfactant; 10-129 mM ionic strength provider; 0.001-2 mM chelator; and a diluent. |
| D526. | 120-220 mg/mL secukinumab; 1-9 mM buffer system; 130-174 mM sugar component; 0.01-1.2 mg/mL surfactant; 10-129 mM ionic strength provider; 0.001-2 mM chelator; and a diluent. |
| D527. | 120-220 mg/mL secukinumab; 51-60 mM buffer system; 130-174 mM sugar component; 0.01-1.2 mg/mL surfactant; 10-129 mM ionic strength provider; 0.001-2 mM chelator; and a diluent. |

(continued)

D528. 120-220 mg/mL secukinumab; 1-9 mM buffer system; 50-129 mM sugar component; 0.01-1.2 mg/mL surfactant; 0.1-20 mM antioxidant; 0.001-2 mM chelator; and a diluent.

D529. 120-220 mg/mL secukinumab; 51-60 mM buffer system; 50-129 mM sugar component; 0.01-1.2 mg/mL surfactant; 0.1-20 mM antioxidant; 0.001-2 mM chelator; and a diluent.

D530. 120-220 mg/mL secukinumab; 1-9 mM buffer system; 130-174 mM sugar component; 0.01-1.2 mg/mL surfactant; 0.1-20 mM antioxidant; 0.001-2 mM chelator; and a diluent.

D531. 120-220 mg/mL secukinumab; 51-60 mM buffer system; 130-174 mM sugar component; 0.01-1.2 mg/mL surfactant; 0.1-20 mM antioxidant; 0.001-2 mM chelator; and a diluent.

D532. 120-220 mg/mL secukinumab; 1-9 mM buffer system; 50-129 mM sugar component; 10-129 mM tonicifier; 10-129 mM ionic strength provider; 0.1-20 mM antioxidant; and a diluent.

D533. 120-220 mg/mL secukinumab; 51-60 mM buffer system; 50-129 mM sugar component; 10-129 mM tonicifier; 10-129 mM ionic strength provider; 0.1-20 mM antioxidant; and a diluent.

D534. 120-220 mg/mL secukinumab; 1-9 mM buffer system; 130-174 mM sugar component; 10-129 mM tonicifier; 10-129 mM ionic strength provider; 0.1-20 mM antioxidant; and a diluent.

D535. 120-220 mg/mL secukinumab; 51-60 mM buffer system; 130-174 mM sugar component; 10-129 mM tonicifier; 10-129 mM ionic strength provider; 0.1-20 mM antioxidant; and a diluent.

D536. 120-220 mg/mL secukinumab; 1-9 mM buffer system; 50-129 mM sugar component; 10-129 mM tonicifier; 10-129 mM ionic strength provider; 0.001-2 mM chelator; and a diluent.

D537. 120-220 mg/mL secukinumab; 51-60 mM buffer system; 50-129 mM sugar component; 10-129 mM tonicifier; 10-129 mM ionic strength provider; 0.001-2 mM chelator; and a diluent.

D538. 120-220 mg/mL secukinumab; 1-9 mM buffer system; 130-174 mM sugar component; 10-129 mM tonicifier; 10-129 mM ionic strength provider; 0.001-2 mM chelator; and a diluent.

D539. 120-220 mg/mL secukinumab; 51-60 mM buffer system; 130-174 mM sugar component; 10-129 mM tonicifier; 10-129 mM ionic strength provider; 0.001-2 mM chelator; and a diluent.

D540. 120-220 mg/mL secukinumab; 1-9 mM buffer system; 50-129 mM sugar component; 10-129 mM tonicifier; 0.1-20 mM antioxidant; 0.001-2 mM chelator; and a diluent.

D541. 120-220 mg/mL secukinumab; 51-60 mM buffer system; 50-129 mM sugar component; 10-129 mM tonicifier; 0.1-20 mM antioxidant; 0.001-2 mM chelator; and a diluent.

D542. 120-220 mg/mL secukinumab; 1-9 mM buffer system; 130-174 mM sugar component; 10-129 mM tonicifier; 0.1-20 mM antioxidant; 0.001-2 mM chelator; and a diluent.

D543. 120-220 mg/mL secukinumab; 51-60 mM buffer system; 130-174 mM sugar component; 10-129 mM tonicifier; 0.1-20 mM antioxidant; 0.001-2 mM chelator; and a diluent.

D544. 120-220 mg/mL secukinumab; 1-9 mM buffer system; 50-129 mM sugar component; 10-129 mM ionic strength provider; 0.1-20 mM antioxidant; 0.001-2 mM chelator; and a diluent.

D545. 120-220 mg/mL secukinumab; 51-60 mM buffer system; 50-129 mM sugar component; 10-129 mM ionic strength provider; 0.1-20 mM antioxidant; 0.001-2 mM chelator; and a diluent.

D546. 120-220 mg/mL secukinumab; 1-9 mM buffer system; 130-174 mM sugar component; 10-129 mM ionic strength provider; 0.1-20 mM antioxidant; 0.001-2 mM chelator; and a diluent.

D547. 120-220 mg/mL secukinumab; 51-60 mM buffer system; 130-174 mM sugar component; 10-129 mM ionic strength provider; 0.1-20 mM antioxidant; 0.001-2 mM chelator; and a diluent.

D548. 120-220 mg/mL secukinumab; 1-9 mM buffer system; 20-99 mM amino acid component; 0.01-1.2 mg/mL surfactant; 10-129 mM tonicifier; 10-129 mM ionic strength provider; and a diluent.

D549. 120-220 mg/mL secukinumab; 51-60 mM buffer system; 20-99 mM amino acid component; 0.01-1.2 mg/mL surfactant; 10-129 mM tonicifier; 10-129 mM ionic strength provider; and a diluent.

D550. 120-220 mg/mL secukinumab; 1-9 mM buffer system; 100-149 mM amino acid component; 0.01-1.2 mg/mL surfactant; 10-129 mM tonicifier; 10-129 mM ionic strength provider; and a diluent.

D551. 120-220 mg/mL secukinumab; 51-60 mM buffer system; 100-149 mM amino acid component; 0.01-1.2 mg/mL surfactant; 10-129 mM tonicifier; 10-129 mM ionic strength provider; and a diluent.

D552. 120-220 mg/mL secukinumab; 1-9 mM buffer system; 20-99 mM amino acid component; 0.01-1.2 mg/mL surfactant; 10-129 mM tonicifier; 0.1-20 mM antioxidant; and a diluent.

D553. 120-220 mg/mL secukinumab; 51-60 mM buffer system; 20-99 mM amino acid component; 0.01-1.2 mg/mL surfactant; 10-129 mM tonicifier; 0.1-20 mM antioxidant; and a diluent.

(continued)

D554. 120-220 mg/mL secukinumab; 1-9 mM buffer system; 100-149 mM amino acid component; 0.01-1.2 mg/mL surfactant; 10-129 mM tonicifier; 0.1-20 mM antioxidant; and a diluent.

D555. 120-220 mg/mL secukinumab; 51-60 mM buffer system; 100-149 mM amino acid component; 0.01-1.2 mg/mL surfactant; 10-129 mM tonicifier; 0.1-20 mM antioxidant; and a diluent.

D556. 120-220 mg/mL secukinumab; 1-9 mM buffer system; 20-99 mM amino acid component; 0.01-1.2 mg/mL surfactant; 10-129 mM tonicifier; 0.001-2 mM chelator; and a diluent.

D557. 120-220 mg/mL secukinumab; 51-60 mM buffer system; 20-99 mM amino acid component; 0.01-1.2 mg/mL surfactant; 10-129 mM tonicifier; 0.001-2 mM chelator; and a diluent.

D558. 120-220 mg/mL secukinumab; 1-9 mM buffer system; 100-149 mM amino acid component; 0.01-1.2 mg/mL surfactant; 10-129 mM tonicifier; 0.001-2 mM chelator; and a diluent.

D559. 120-220 mg/mL secukinumab; 51-60 mM buffer system; 100-149 mM amino acid component; 0.01-1.2 mg/mL surfactant; 10-129 mM tonicifier; 0.001-2 mM chelator; and a diluent.

D560. 120-220 mg/mL secukinumab; 1-9 mM buffer system; 20-99 mM amino acid component; 0.01-1.2 mg/mL surfactant; 10-129 mM ionic strength provider; 0.1-20 mM antioxidant; and a diluent.

D561. 120-220 mg/mL secukinumab; 51-60 mM buffer system; 20-99 mM amino acid component; 0.01-1.2 mg/mL surfactant; 10-129 mM ionic strength provider; 0.1-20 mM antioxidant; and a diluent.

D562. 120-220 mg/mL secukinumab; 1-9 mM buffer system; 100-149 mM amino acid component; 0.01-1.2 mg/mL surfactant; 10-129 mM ionic strength provider; 0.1-20 mM antioxidant; and a diluent.

D563. 120-220 mg/mL secukinumab; 51-60 mM buffer system; 100-149 mM amino acid component; 0.01-1.2 mg/mL surfactant; 10-129 mM ionic strength provider; 0.1-20 mM antioxidant; and a diluent.

D564. 120-220 mg/mL secukinumab; 1-9 mM buffer system; 20-99 mM amino acid component; 0.01-1.2 mg/mL surfactant; 10-129 mM ionic strength provider; 0.001-2 mM chelator; and a diluent.

D565. 120-220 mg/mL secukinumab; 51-60 mM buffer system; 20-99 mM amino acid component; 0.01-1.2 mg/mL surfactant; 10-129 mM ionic strength provider; 0.001-2 mM chelator; and a diluent.

D566. 120-220 mg/mL secukinumab; 1-9 mM buffer system; 100-149 mM amino acid component; 0.01-1.2 mg/mL surfactant; 10-129 mM ionic strength provider; 0.001-2 mM chelator; and a diluent.

D567. 120-220 mg/mL secukinumab; 51-60 mM buffer system; 100-149 mM amino acid component; 0.01-1.2 mg/mL surfactant; 10-129 mM ionic strength provider; 0.001-2 mM chelator; and a diluent.

D568. 120-220 mg/mL secukinumab; 1-9 mM buffer system; 20-99 mM amino acid component; 0.01-1.2 mg/mL surfactant; 0.1-20 mM antioxidant; 0.001-2 mM chelator; and a diluent.

D569. 120-220 mg/mL secukinumab; 51-60 mM buffer system; 20-99 mM amino acid component; 0.01-1.2 mg/mL surfactant; 0.1-20 mM antioxidant; 0.001-2 mM chelator; and a diluent.

D570. 120-220 mg/mL secukinumab; 1-9 mM buffer system; 100-149 mM amino acid component; 0.01-1.2 mg/mL surfactant; 0.1-20 mM antioxidant; 0.001-2 mM chelator; and a diluent.

D571. 120-220 mg/mL secukinumab; 51-60 mM buffer system; 100-149 mM amino acid component; 0.01-1.2 mg/mL surfactant; 0.1-20 mM antioxidant; 0.001-2 mM chelator; and a diluent.

D572. 120-220 mg/mL secukinumab; 1-9 mM buffer system; 20-99 mM amino acid component; 10-129 mM tonicifier; 10-129 mM ionic strength provider; 0.1-20 mM antioxidant; and a diluent.

D573. 120-220 mg/mL secukinumab; 51-60 mM buffer system; 20-99 mM amino acid component; 10-129 mM tonicifier; 10-129 mM ionic strength provider; 0.1-20 mM antioxidant; and a diluent.

D574. 120-220 mg/mL secukinumab; 1-9 mM buffer system; 100-149 mM amino acid component; 10-129 mM tonicifier; 10-129 mM ionic strength provider; 0.1-20 mM antioxidant; and a diluent.

D575. 120-220 mg/mL secukinumab; 51-60 mM buffer system; 100-149 mM amino acid component; 10-129 mM tonicifier; 10-129 mM ionic strength provider; 0.1-20 mM antioxidant; and a diluent.

D576. 120-220 mg/mL secukinumab; 1-9 mM buffer system; 20-99 mM amino acid component; 10-129 mM tonicifier; 10-129 mM ionic strength provider; 0.001-2 mM chelator; and a diluent.

D577. 120-220 mg/mL secukinumab; 51-60 mM buffer system; 20-99 mM amino acid component; 10-129 mM tonicifier; 10-129 mM ionic strength provider; 0.001-2 mM chelator; and a diluent.

D578. 120-220 mg/mL secukinumab; 1-9 mM buffer system; 100-149 mM amino acid component; 10-129 mM tonicifier; 10-129 mM ionic strength provider; 0.001-2 mM chelator; and a diluent.

D579. 120-220 mg/mL secukinumab; 51-60 mM buffer system; 100-149 mM amino acid component; 10-129 mM tonicifier; 10-129 mM ionic strength provider; 0.001-2 mM chelator; and a diluent.

(continued)

D580. 120-220 mg/mL secukinumab; 1-9 mM buffer system; 20-99 mM amino acid component; 10-129 mM tonicifier; 0.1-20 mM antioxidant; 0.001-2 mM chelator; and a diluent.

D581. 120-220 mg/mL secukinumab; 51-60 mM buffer system; 20-99 mM amino acid component; 10-129 mM tonicifier; 0.1-20 mM antioxidant; 0.001-2 mM chelator; and a diluent.

D582. 120-220 mg/mL secukinumab; 1-9 mM buffer system; 100-149 mM amino acid component; 10-129 mM tonicifier; 0.1-20 mM antioxidant; 0.001-2 mM chelator; and a diluent.

D583. 120-220 mg/mL secukinumab; 51-60 mM buffer system; 100-149 mM amino acid component; 10-129 mM tonicifier; 0.1-20 mM antioxidant; 0.001-2 mM chelator; and a diluent.

D584. 120-220 mg/mL secukinumab; 1-9 mM buffer system; 20-99 mM amino acid component; 10-129 mM ionic strength provider; 0.1-20 mM antioxidant; 0.001-2 mM chelator; and a diluent.

D585. 120-220 mg/mL secukinumab; 51-60 mM buffer system; 20-99 mM amino acid component; 10-129 mM ionic strength provider; 0.1-20 mM antioxidant; 0.001-2 mM chelator; and a diluent.

D586. 120-220 mg/mL secukinumab; 1-9 mM buffer system; 100-149 mM amino acid component; 10-129 mM ionic strength provider; 0.1-20 mM antioxidant; 0.001-2 mM chelator; and a diluent.

D587. 120-220 mg/mL secukinumab; 51-60 mM buffer system; 100-149 mM amino acid component; 10-129 mM ionic strength provider; 0.1-20 mM antioxidant; 0.001-2 mM chelator; and a diluent.

D588. 120-220 mg/mL secukinumab; 1-9 mM buffer system; 0.01-1.2 mg/mL surfactant; 10-129 mM tonicifier; 10-129 mM ionic strength provider; 0.1-20 mM antioxidant; and a diluent.

D589. 120-220 mg/mL secukinumab; 51-60 mM buffer system; 0.01-1.2 mg/mL surfactant; 10-129 mM tonicifier; 10-129 mM ionic strength provider; 0.1-20 mM antioxidant; and a diluent.

D590. 120-220 mg/mL secukinumab; 1-9 mM buffer system; 0.01-1.2 mg/mL surfactant; 10-129 mM tonicifier; 10-129 mM ionic strength provider; 0.001-2 mM chelator; and a diluent.

D591. 120-220 mg/mL secukinumab; 51-60 mM buffer system; 0.01-1.2 mg/mL surfactant; 10-129 mM tonicifier; 10-129 mM ionic strength provider; 0.001-2 mM chelator; and a diluent.

D592. 120-220 mg/mL secukinumab; 1-9 mM buffer system; 0.01-1.2 mg/mL surfactant; 10-129 mM tonicifier; 0.1-20 mM antioxidant; 0.001-2 mM chelator; and a diluent.

D593. 120-220 mg/mL secukinumab; 51-60 mM buffer system; 0.01-1.2 mg/mL surfactant; 10-129 mM tonicifier; 0.1-20 mM antioxidant; 0.001-2 mM chelator; and a diluent.

D594. 120-220 mg/mL secukinumab; 1-9 mM buffer system; 0.01-1.2 mg/mL surfactant; 10-129 mM ionic strength provider; 0.1-20 mM antioxidant; 0.001-2 mM chelator; and a diluent.

D595. 120-220 mg/mL secukinumab; 51-60 mM buffer system; 0.01-1.2 mg/mL surfactant; 10-129 mM ionic strength provider; 0.1-20 mM antioxidant; 0.001-2 mM chelator; and a diluent.

D596. 120-220 mg/mL secukinumab; 1-9 mM buffer system; 10-129 mM tonicifier; 10-129 mM ionic strength provider; 0.1-20 mM antioxidant; 0.001-2 mM chelator; and a diluent.

D597. 120-220 mg/mL secukinumab; 51-60 mM buffer system; 10-129 mM tonicifier; 10-129 mM ionic strength provider; 0.1-20 mM antioxidant; 0.001-2 mM chelator; and a diluent.

D598. 120-220 mg/mL secukinumab; 50-129 mM sugar component; 20-99 mM amino acid component; 0.01-1.2 mg/mL surfactant; 10-129 mM tonicifier; 10-129 mM ionic strength provider; and a diluent.

D599. 120-220 mg/mL secukinumab; 130-174 mM sugar component; 20-99 mM amino acid component; 0.01-1.2 mg/mL surfactant; 10-129 mM tonicifier; 10-129 mM ionic strength provider; and a diluent.

D600. 120-220 mg/mL secukinumab; 50-129 mM sugar component; 100-149 mM amino acid component; 0.01-1.2 mg/mL surfactant; 10-129 mM tonicifier; 10-129 mM ionic strength provider; and a diluent.

D601. 120-220 mg/mL secukinumab; 130-174 mM sugar component; 100-149 mM amino acid component; 0.01-1.2 mg/mL surfactant; 10-129 mM tonicifier; 10-129 mM ionic strength provider; and a diluent.

D602. 120-220 mg/mL secukinumab; 50-129 mM sugar component; 20-99 mM amino acid component; 0.01-1.2 mg/mL surfactant; 10-129 mM tonicifier; 0.1-20 mM antioxidant; and a diluent.

D603. 120-220 mg/mL secukinumab; 130-174 mM sugar component; 20-99 mM amino acid component; 0.01-1.2 mg/mL surfactant; 10-129 mM tonicifier; 0.1-20 mM antioxidant; and a diluent.

D604. 120-220 mg/mL secukinumab; 50-129 mM sugar component; 100-149 mM amino acid component; 0.01-1.2 mg/mL surfactant; 10-129 mM tonicifier; 0.1-20 mM antioxidant; and a diluent.

D605. 120-220 mg/mL secukinumab; 130-174 mM sugar component; 100-149 mM amino acid component; 0.01-1.2 mg/mL surfactant; 10-129 mM tonicifier; 0.1-20 mM antioxidant; and a diluent.

(continued)

D606. 120-220 mg/mL secukinumab; 50-129 mM sugar component; 20-99 mM amino acid component; 0.01-1.2 mg/mL surfactant; 10-129 mM tonicifier; 0.001-2 mM chelator; and a diluent.

D607. 120-220 mg/mL secukinumab; 130-174 mM sugar component; 20-99 mM amino acid component; 0.01-1.2 mg/mL surfactant; 10-129 mM tonicifier; 0.001-2 mM chelator; and a diluent.

D608. 120-220 mg/mL secukinumab; 50-129 mM sugar component; 100-149 mM amino acid component; 0.01-1.2 mg/mL surfactant; 10-129 mM tonicifier; 0.001-2 mM chelator; and a diluent.

D609. 120-220 mg/mL secukinumab; 130-174 mM sugar component; 100-149 mM amino acid component; 0.01-1.2 mg/mL surfactant; 10-129 mM tonicifier; 0.001-2 mM chelator; and a diluent.

D610. 120-220 mg/mL secukinumab; 50-129 mM sugar component; 20-99 mM amino acid component; 0.01-1.2 mg/mL surfactant; 10-129 mM ionic strength provider; 0.1-20 mM antioxidant; and a diluent.

D611. 120-220 mg/mL secukinumab; 130-174 mM sugar component; 20-99 mM amino acid component; 0.01-1.2 mg/mL surfactant; 10-129 mM ionic strength provider; 0.1-20 mM antioxidant; and a diluent.

D612. 120-220 mg/mL secukinumab; 50-129 mM sugar component; 100-149 mM amino acid component; 0.01-1.2 mg/mL surfactant; 10-129 mM ionic strength provider; 0.1-20 mM antioxidant; and a diluent.

D613. 120-220 mg/mL secukinumab; 130-174 mM sugar component; 100-149 mM amino acid component; 0.01-1.2 mg/mL surfactant; 10-129 mM ionic strength provider; 0.1-20 mM antioxidant; and a diluent.

D614. 120-220 mg/mL secukinumab; 50-129 mM sugar component; 20-99 mM amino acid component; 0.01-1.2 mg/mL surfactant; 10-129 mM ionic strength provider; 0.001-2 mM chelator; and a diluent.

D615. 120-220 mg/mL secukinumab; 130-174 mM sugar component; 20-99 mM amino acid component; 0.01-1.2 mg/mL surfactant; 10-129 mM ionic strength provider; 0.001-2 mM chelator; and a diluent.

D616. 120-220 mg/mL secukinumab; 50-129 mM sugar component; 100-149 mM amino acid component; 0.01-1.2 mg/mL surfactant; 10-129 mM ionic strength provider; 0.001-2 mM chelator; and a diluent.

D617. 120-220 mg/mL secukinumab; 130-174 mM sugar component; 100-149 mM amino acid component; 0.01-1.2 mg/mL surfactant; 10-129 mM ionic strength provider; 0.001-2 mM chelator; and a diluent.

D618. 120-220 mg/mL secukinumab; 50-129 mM sugar component; 20-99 mM amino acid component; 0.01-1.2 mg/mL surfactant; 0.1-20 mM antioxidant; 0.001-2 mM chelator; and a diluent.

D619. 120-220 mg/mL secukinumab; 130-174 mM sugar component; 20-99 mM amino acid component; 0.01-1.2 mg/mL surfactant; 0.1-20 mM antioxidant; 0.001-2 mM chelator; and a diluent.

D620. 120-220 mg/mL secukinumab; 50-129 mM sugar component; 100-149 mM amino acid component; 0.01-1.2 mg/mL surfactant; 0.1-20 mM antioxidant; 0.001-2 mM chelator; and a diluent.

D621. 120-220 mg/mL secukinumab; 130-174 mM sugar component; 100-149 mM amino acid component; 0.01-1.2 mg/mL surfactant; 0.1-20 mM antioxidant; 0.001-2 mM chelator; and a diluent.

D622. 120-220 mg/mL secukinumab; 50-129 mM sugar component; 20-99 mM amino acid component; 10-129 mM tonicifier; 10-129 mM ionic strength provider; 0.1-20 mM antioxidant; and a diluent.

D623. 120-220 mg/mL secukinumab; 130-174 mM sugar component; 20-99 mM amino acid component; 10-129 mM tonicifier; 10-129 mM ionic strength provider; 0.1-20 mM antioxidant; and a diluent.

D624. 120-220 mg/mL secukinumab; 50-129 mM sugar component; 100-149 mM amino acid component; 10-129 mM tonicifier; 10-129 mM ionic strength provider; 0.1-20 mM antioxidant; and a diluent.

D625. 120-220 mg/mL secukinumab; 130-174 mM sugar component; 100-149 mM amino acid component; 10-129 mM tonicifier; 10-129 mM ionic strength provider; 0.1-20 mM antioxidant; and a diluent.

D626. 120-220 mg/mL secukinumab; 50-129 mM sugar component; 20-99 mM amino acid component; 10-129 mM tonicifier; 10-129 mM ionic strength provider; 0.001-2 mM chelator; and a diluent.

D627. 120-220 mg/mL secukinumab; 130-174 mM sugar component; 20-99 mM amino acid component; 10-129 mM tonicifier; 10-129 mM ionic strength provider; 0.001-2 mM chelator; and a diluent.

D628. 120-220 mg/mL secukinumab; 50-129 mM sugar component; 100-149 mM amino acid component; 10-129 mM tonicifier; 10-129 mM ionic strength provider; 0.001-2 mM chelator; and a diluent.

D629. 120-220 mg/mL secukinumab; 130-174 mM sugar component; 100-149 mM amino acid component; 10-129 mM tonicifier; 10-129 mM ionic strength provider; 0.001-2 mM chelator; and a diluent.

D630. 120-220 mg/mL secukinumab; 50-129 mM sugar component; 20-99 mM amino acid component; 10-129 mM tonicifier; 0.1-20 mM antioxidant; 0.001-2 mM chelator; and a diluent.

D631. 120-220 mg/mL secukinumab; 130-174 mM sugar component; 20-99 mM amino acid component; 10-129 mM tonicifier; 0.1-20 mM antioxidant; 0.001-2 mM chelator; and a diluent.

(continued)

D632. 120-220 mg/mL secukinumab; 50-129 mM sugar component; 100-149 mM amino acid component; 10-129 mM tonicifier; 0.1-20 mM antioxidant; 0.001-2 mM chelator; and a diluent.

D633. 120-220 mg/mL secukinumab; 130-174 mM sugar component; 100-149 mM amino acid component; 10-129 mM tonicifier; 0.1-20 mM antioxidant; 0.001-2 mM chelator; and a diluent.

D634. 120-220 mg/mL secukinumab; 50-129 mM sugar component; 20-99 mM amino acid component; 10-129 mM ionic strength provider; 0.1-20 mM antioxidant; 0.001-2 mM chelator; and a diluent.

D635. 120-220 mg/mL secukinumab; 130-174 mM sugar component; 20-99 mM amino acid component; 10-129 mM ionic strength provider; 0.1-20 mM antioxidant; 0.001-2 mM chelator; and a diluent.

D636. 120-220 mg/mL secukinumab; 50-129 mM sugar component; 100-149 mM amino acid component; 10-129 mM ionic strength provider; 0.1-20 mM antioxidant; 0.001-2 mM chelator; and a diluent.

D637. 120-220 mg/mL secukinumab; 130-174 mM sugar component; 100-149 mM amino acid component; 10-129 mM ionic strength provider; 0.1-20 mM antioxidant; 0.001-2 mM chelator; and a diluent.

D638. 120-220 mg/mL secukinumab; 50-129 mM sugar component; 0.01-1.2 mg/mL surfactant; 10-129 mM tonicifier; 10-129 mM ionic strength provider; 0.1-20 mM antioxidant; and a diluent.

D639. 120-220 mg/mL secukinumab; 130-174 mM sugar component; 0.01-1.2 mg/mL surfactant; 10-129 mM tonicifier; 10-129 mM ionic strength provider; 0.1-20 mM antioxidant; and a diluent.

D640. 120-220 mg/mL secukinumab; 50-129 mM sugar component; 0.01-1.2 mg/mL surfactant; 10-129 mM tonicifier; 10-129 mM ionic strength provider; 0.001-2 mM chelator; and a diluent.

D641. 120-220 mg/mL secukinumab; 130-174 mM sugar component; 0.01-1.2 mg/mL surfactant; 10-129 mM tonicifier; 10-129 mM ionic strength provider; 0.001-2 mM chelator; and a diluent.

D642. 120-220 mg/mL secukinumab; 50-129 mM sugar component; 0.01-1.2 mg/mL surfactant; 10-129 mM tonicifier; 0.1-20 mM antioxidant; 0.001-2 mM chelator; and a diluent.

D643. 120-220 mg/mL secukinumab; 130-174 mM sugar component; 0.01-1.2 mg/mL surfactant; 10-129 mM tonicifier; 0.1-20 mM antioxidant; 0.001-2 mM chelator; and a diluent.

D644. 120-220 mg/mL secukinumab; 50-129 mM sugar component; 0.01-1.2 mg/mL surfactant; 10-129 mM ionic strength provider; 0.1-20 mM antioxidant; 0.001-2 mM chelator; and a diluent.

D645. 120-220 mg/mL secukinumab; 130-174 mM sugar component; 0.01-1.2 mg/mL surfactant; 10-129 mM ionic strength provider; 0.1-20 mM antioxidant; 0.001-2 mM chelator; and a diluent.

D646. 120-220 mg/mL secukinumab; 50-129 mM sugar component; 10-129 mM tonicifier; 10-129 mM ionic strength provider; 0.1-20 mM antioxidant; 0.001-2 mM chelator; and a diluent.

D647. 120-220 mg/mL secukinumab; 130-174 mM sugar component; 10-129 mM tonicifier; 10-129 mM ionic strength provider; 0.1-20 mM antioxidant; 0.001-2 mM chelator; and a diluent.

D648. 120-220 mg/mL secukinumab; 20-99 mM amino acid component; 0.01-1.2 mg/mL surfactant; 10-129 mM tonicifier; 10-129 mM ionic strength provider; 0.1-20 mM antioxidant; and a diluent.

D649. 120-220 mg/mL secukinumab; 100-149 mM amino acid component; 0.01-1.2 mg/mL surfactant; 10-129 mM tonicifier; 10-129 mM ionic strength provider; 0.1-20 mM antioxidant; and a diluent.

D650. 120-220 mg/mL secukinumab; 20-99 mM amino acid component; 0.01-1.2 mg/mL surfactant; 10-129 mM tonicifier; 10-129 mM ionic strength provider; 0.001-2 mM chelator; and a diluent.

D651. 120-220 mg/mL secukinumab; 100-149 mM amino acid component; 0.01-1.2 mg/mL surfactant; 10-129 mM tonicifier; 10-129 mM ionic strength provider; 0.001-2 mM chelator; and a diluent.

D652. 120-220 mg/mL secukinumab; 20-99 mM amino acid component; 0.01-1.2 mg/mL surfactant; 10-129 mM tonicifier; 0.1-20 mM antioxidant; 0.001-2 mM chelator; and a diluent.

D653. 120-220 mg/mL secukinumab; 100-149 mM amino acid component; 0.01-1.2 mg/mL surfactant; 10-129 mM tonicifier; 0.1-20 mM antioxidant; 0.001-2 mM chelator; and a diluent.

D654. 120-220 mg/mL secukinumab; 20-99 mM amino acid component; 0.01-1.2 mg/mL surfactant; 10-129 mM ionic strength provider; 0.1-20 mM antioxidant; 0.001-2 mM chelator; and a diluent.

D655. 120-220 mg/mL secukinumab; 100-149 mM amino acid component; 0.01-1.2 mg/mL surfactant; 10-129 mM ionic strength provider; 0.1-20 mM antioxidant; 0.001-2 mM chelator; and a diluent.

D656. 120-220 mg/mL secukinumab; 20-99 mM amino acid component; 10-129 mM tonicifier; 10-129 mM ionic strength provider; 0.1-20 mM antioxidant; 0.001-2 mM chelator; and a diluent.

D657. 120-220 mg/mL secukinumab; 100-149 mM amino acid component; 10-129 mM tonicifier; 10-129 mM ionic strength provider; 0.1-20 mM antioxidant; 0.001-2 mM chelator; and a diluent.

(continued)

D658. 120-220 mg/mL secukinumab; 0.01-1.2 mg/mL surfactant; 10-129 mM tonicifier; 10-129 mM ionic strength provider; 0.1-20 mM antioxidant; 0.001-2 mM chelator; and a diluent.

D659. 120-220 mg/mL secukinumab; 1-9 mM buffer system; 50-129 mM sugar component; 20-99 mM amino acid component; 0.01-1.2 mg/mL surfactant; 10-129 mM tonicifier; 10-129 mM ionic strength provider; and a diluent.

D660. 120-220 mg/mL secukinumab; 51-60 mM buffer system; 50-129 mM sugar component; 20-99 mM amino acid component; 0.01-1.2 mg/mL surfactant; 10-129 mM tonicifier; 10-129 mM ionic strength provider; and a diluent.

D661. 120-220 mg/mL secukinumab; 1-9 mM buffer system; 130-174 mM sugar component; 20-99 mM amino acid component; 0.01-1.2 mg/mL surfactant; 10-129 mM tonicifier; 10-129 mM ionic strength provider; and a diluent.

D662. 120-220 mg/mL secukinumab; 51-60 mM buffer system; 130-174 mM sugar component; 20-99 mM amino acid component; 0.01-1.2 mg/mL surfactant; 10-129 mM tonicifier; 10-129 mM ionic strength provider; and a diluent.

D663. 120-220 mg/mL secukinumab; 1-9 mM buffer system; 50-129 mM sugar component; 100-149 mM amino acid component; 0.01-1.2 mg/mL surfactant; 10-129 mM tonicifier; 10-129 mM ionic strength provider; and a diluent.

D664. 120-220 mg/mL secukinumab; 51-60 mM buffer system; 50-129 mM sugar component; 100-149 mM amino acid component; 0.01-1.2 mg/mL surfactant; 10-129 mM tonicifier; 10-129 mM ionic strength provider; and a diluent.

D665. 120-220 mg/mL secukinumab; 1-9 mM buffer system; 130-174 mM sugar component; 100-149 mM amino acid component; 0.01-1.2 mg/mL surfactant; 10-129 mM tonicifier; 10-129 mM ionic strength provider; and a diluent.

D666. 120-220 mg/mL secukinumab; 51-60 mM buffer system; 130-174 mM sugar component; 100-149 mM amino acid component; 0.01-1.2 mg/mL surfactant; 10-129 mM tonicifier; 10-129 mM ionic strength provider; and a diluent.

D667. 120-220 mg/mL secukinumab; 1-9 mM buffer system; 50-129 mM sugar component; 20-99 mM amino acid component; 0.01-1.2 mg/mL surfactant; 10-129 mM tonicifier; 0.1-20 mM antioxidant; and a diluent.

D668. 120-220 mg/mL secukinumab; 51-60 mM buffer system; 50-129 mM sugar component; 20-99 mM amino acid component; 0.01-1.2 mg/mL surfactant; 10-129 mM tonicifier; 0.1-20 mM antioxidant; and a diluent.

D669. 120-220 mg/mL secukinumab; 1-9 mM buffer system; 130-174 mM sugar component; 20-99 mM amino acid component; 0.01-1.2 mg/mL surfactant; 10-129 mM tonicifier; 0.1-20 mM antioxidant; and a diluent.

D670. 120-220 mg/mL secukinumab; 51-60 mM buffer system; 130-174 mM sugar component; 20-99 mM amino acid component; 0.01-1.2 mg/mL surfactant; 10-129 mM tonicifier; 0.1-20 mM antioxidant; and a diluent.

D671. 120-220 mg/mL secukinumab; 1-9 mM buffer system; 50-129 mM sugar component; 100-149 mM amino acid component; 0.01-1.2 mg/mL surfactant; 10-129 mM tonicifier; 0.1-20 mM antioxidant; and a diluent.

D672. 120-220 mg/mL secukinumab; 51-60 mM buffer system; 50-129 mM sugar component; 100-149 mM amino acid component; 0.01-1.2 mg/mL surfactant; 10-129 mM tonicifier; 0.1-20 mM antioxidant; and a diluent.

D673. 120-220 mg/mL secukinumab; 1-9 mM buffer system; 130-174 mM sugar component; 100-149 mM amino acid component; 0.01-1.2 mg/mL surfactant; 10-129 mM tonicifier; 0.1-20 mM antioxidant; and a diluent.

D674. 120-220 mg/mL secukinumab; 51-60 mM buffer system; 130-174 mM sugar component; 100-149 mM amino acid component; 0.01-1.2 mg/mL surfactant; 10-129 mM tonicifier; 0.1-20 mM antioxidant; and a diluent.

D675. 120-220 mg/mL secukinumab; 1-9 mM buffer system; 50-129 mM sugar component; 20-99 mM amino acid component; 0.01-1.2 mg/mL surfactant; 10-129 mM tonicifier; 0.001-2 mM chelator; and a diluent.

D676. 120-220 mg/mL secukinumab; 51-60 mM buffer system; 50-129 mM sugar component; 20-99 mM amino acid component; 0.01-1.2 mg/mL surfactant; 10-129 mM tonicifier; 0.001-2 mM chelator; and a diluent.

D677. 120-220 mg/mL secukinumab; 1-9 mM buffer system; 130-174 mM sugar component; 20-99 mM amino acid component; 0.01-1.2 mg/mL surfactant; 10-129 mM tonicifier; 0.001-2 mM chelator; and a diluent.

D678. 120-220 mg/mL secukinumab; 51-60 mM buffer system; 130-174 mM sugar component; 20-99 mM amino acid component; 0.01-1.2 mg/mL surfactant; 10-129 mM tonicifier; 0.001-2 mM chelator; and a diluent.

D679. 120-220 mg/mL secukinumab; 1-9 mM buffer system; 50-129 mM sugar component; 100-149 mM amino acid component; 0.01-1.2 mg/mL surfactant; 10-129 mM tonicifier; 0.001-2 mM chelator; and a diluent.

(continued)

D680. 120-220 mg/mL secukinumab; 51-60 mM buffer system; 50-129 mM sugar component; 100-149 mM amino acid component; 0.01-1.2 mg/mL surfactant; 10-129 mM tonicifier; 0.001-2 mM chelator; and a diluent.

D681. 120-220 mg/mL secukinumab; 1-9 mM buffer system; 130-174 mM sugar component; 100-149 mM amino acid component; 0.01-1.2 mg/mL surfactant; 10-129 mM tonicifier; 0.001-2 mM chelator; and a diluent.

D682. 120-220 mg/mL secukinumab; 51-60 mM buffer system; 130-174 mM sugar component; 100-149 mM amino acid component; 0.01-1.2 mg/mL surfactant; 10-129 mM tonicifier; 0.001-2 mM chelator; and a diluent.

D683. 120-220 mg/mL secukinumab; 1-9 mM buffer system; 50-129 mM sugar component; 20-99 mM amino acid component; 0.01-1.2 mg/mL surfactant; 10-129 mM ionic strength provider; 0.1-20 mM antioxidant; and a diluent.

D684. 120-220 mg/mL secukinumab; 51-60 mM buffer system; 50-129 mM sugar component; 20-99 mM amino acid component; 0.01-1.2 mg/mL surfactant; 10-129 mM ionic strength provider; 0.1-20 mM antioxidant; and a diluent.

D685. 120-220 mg/mL secukinumab; 1-9 mM buffer system; 130-174 mM sugar component; 20-99 mM amino acid component; 0.01-1.2 mg/mL surfactant; 10-129 mM ionic strength provider; 0.1-20 mM antioxidant; and a diluent.

D686. 120-220 mg/mL secukinumab; 51-60 mM buffer system; 130-174 mM sugar component; 20-99 mM amino acid component; 0.01-1.2 mg/mL surfactant; 10-129 mM ionic strength provider; 0.1-20 mM antioxidant; and a diluent.

D687. 120-220 mg/mL secukinumab; 1-9 mM buffer system; 50-129 mM sugar component; 100-149 mM amino acid component; 0.01-1.2 mg/mL surfactant; 10-129 mM ionic strength provider; 0.1-20 mM antioxidant; and a diluent.

D688. 120-220 mg/mL secukinumab; 51-60 mM buffer system; 50-129 mM sugar component; 100-149 mM amino acid component; 0.01-1.2 mg/mL surfactant; 10-129 mM ionic strength provider; 0.1-20 mM antioxidant; and a diluent.

D689. 120-220 mg/mL secukinumab; 1-9 mM buffer system; 130-174 mM sugar component; 100-149 mM amino acid component; 0.01-1.2 mg/mL surfactant; 10-129 mM ionic strength provider; 0.1-20 mM antioxidant; and a diluent.

D690. 120-220 mg/mL secukinumab; 51-60 mM buffer system; 130-174 mM sugar component; 100-149 mM amino acid component; 0.01-1.2 mg/mL surfactant; 10-129 mM ionic strength provider; 0.1-20 mM antioxidant; and a diluent.

D691. 120-220 mg/mL secukinumab; 1-9 mM buffer system; 50-129 mM sugar component; 20-99 mM amino acid component; 0.01-1.2 mg/mL surfactant; 10-129 mM ionic strength provider; 0.001-2 mM chelator; and a diluent.

D692. 120-220 mg/mL secukinumab; 51-60 mM buffer system; 50-129 mM sugar component; 20-99 mM amino acid component; 0.01-1.2 mg/mL surfactant; 10-129 mM ionic strength provider; 0.001-2 mM chelator; and a diluent.

D693. 120-220 mg/mL secukinumab; 1-9 mM buffer system; 130-174 mM sugar component; 20-99 mM amino acid component; 0.01-1.2 mg/mL surfactant; 10-129 mM ionic strength provider; 0.001-2 mM chelator; and a diluent.

D694. 120-220 mg/mL secukinumab; 51-60 mM buffer system; 130-174 mM sugar component; 20-99 mM amino acid component; 0.01-1.2 mg/mL surfactant; 10-129 mM ionic strength provider; 0.001-2 mM chelator; and a diluent.

D695. 120-220 mg/mL secukinumab; 1-9 mM buffer system; 50-129 mM sugar component; 100-149 mM amino acid component; 0.01-1.2 mg/mL surfactant; 10-129 mM ionic strength provider; 0.001-2 mM chelator; and a diluent.

D696. 120-220 mg/mL secukinumab; 51-60 mM buffer system; 50-129 mM sugar component; 100-149 mM amino acid component; 0.01-1.2 mg/mL surfactant; 10-129 mM ionic strength provider; 0.001-2 mM chelator; and a diluent.

D697. 120-220 mg/mL secukinumab; 1-9 mM buffer system; 130-174 mM sugar component; 100-149 mM amino acid component; 0.01-1.2 mg/mL surfactant; 10-129 mM ionic strength provider; 0.001-2 mM chelator; and a diluent.

(continued)

D698. 120-220 mg/mL secukinumab; 51-60 mM buffer system; 130-174 mM sugar component; 100-149 mM amino acid component; 0.01-1.2 mg/mL surfactant; 10-129 mM ionic strength provider; 0.001-2 mM chelator; and a diluent.

D699. 120-220 mg/mL secukinumab; 1-9 mM buffer system; 50-129 mM sugar component; 20-99 mM amino acid component; 0.01-1.2 mg/mL surfactant; 0.1-20 mM antioxidant; 0.001-2 mM chelator; and a diluent.

D700. 120-220 mg/mL secukinumab; 51-60 mM buffer system; 50-129 mM sugar component; 20-99 mM amino acid component; 0.01-1.2 mg/mL surfactant; 0.1-20 mM antioxidant; 0.001-2 mM chelator; and a diluent.

D701. 120-220 mg/mL secukinumab; 1-9 mM buffer system; 130-174 mM sugar component; 20-99 mM amino acid component; 0.01-1.2 mg/mL surfactant; 0.1-20 mM antioxidant; 0.001-2 mM chelator; and a diluent.

D702. 120-220 mg/mL secukinumab; 51-60 mM buffer system; 130-174 mM sugar component; 20-99 mM amino acid component; 0.01-1.2 mg/mL surfactant; 0.1-20 mM antioxidant; 0.001-2 mM chelator; and a diluent.

D703. 120-220 mg/mL secukinumab; 1-9 mM buffer system; 50-129 mM sugar component; 100-149 mM amino acid component; 0.01-1.2 mg/mL surfactant; 0.1-20 mM antioxidant; 0.001-2 mM chelator; and a diluent.

D704. 120-220 mg/mL secukinumab; 51-60 mM buffer system; 50-129 mM sugar component; 100-149 mM amino acid component; 0.01-1.2 mg/mL surfactant; 0.1-20 mM antioxidant; 0.001-2 mM chelator; and a diluent.

D705. 120-220 mg/mL secukinumab; 1-9 mM buffer system; 130-174 mM sugar component; 100-149 mM amino acid component; 0.01-1.2 mg/mL surfactant; 0.1-20 mM antioxidant; 0.001-2 mM chelator; and a diluent.

D706. 120-220 mg/mL secukinumab; 51-60 mM buffer system; 130-174 mM sugar component; 100-149 mM amino acid component; 0.01-1.2 mg/mL surfactant; 0.1-20 mM antioxidant; 0.001-2 mM chelator; and a diluent.

D707. 120-220 mg/mL secukinumab; 1-9 mM buffer system; 50-129 mM sugar component; 20-99 mM amino acid component; 10-129 mM tonicifier; 10-129 mM ionic strength provider; 0.1-20 mM antioxidant; and a diluent.

D708. 120-220 mg/mL secukinumab; 51-60 mM buffer system; 50-129 mM sugar component; 20-99 mM amino acid component; 10-129 mM tonicifier; 10-129 mM ionic strength provider; 0.1-20 mM antioxidant; and a diluent.

D709. 120-220 mg/mL secukinumab; 1-9 mM buffer system; 130-174 mM sugar component; 20-99 mM amino acid component; 10-129 mM tonicifier; 10-129 mM ionic strength provider; 0.1-20 mM antioxidant; and a diluent.

D710. 120-220 mg/mL secukinumab; 51-60 mM buffer system; 130-174 mM sugar component; 20-99 mM amino acid component; 10-129 mM tonicifier; 10-129 mM ionic strength provider; 0.1-20 mM antioxidant; and a diluent.

D711. 120-220 mg/mL secukinumab; 1-9 mM buffer system; 50-129 mM sugar component; 100-149 mM amino acid component; 10-129 mM tonicifier; 10-129 mM ionic strength provider; 0.1-20 mM antioxidant; and a diluent.

D712. 120-220 mg/mL secukinumab; 51-60 mM buffer system; 50-129 mM sugar component; 100-149 mM amino acid component; 10-129 mM tonicifier; 10-129 mM ionic strength provider; 0.1-20 mM antioxidant; and a diluent.

D713. 120-220 mg/mL secukinumab; 1-9 mM buffer system; 130-174 mM sugar component; 100-149 mM amino acid component; 10-129 mM tonicifier; 10-129 mM ionic strength provider; 0.1-20 mM antioxidant; and a diluent.

D714. 120-220 mg/mL secukinumab; 51-60 mM buffer system; 130-174 mM sugar component; 100-149 mM amino acid component; 10-129 mM tonicifier; 10-129 mM ionic strength provider; 0.1-20 mM antioxidant; and a diluent.

D715. 120-220 mg/mL secukinumab; 1-9 mM buffer system; 50-129 mM sugar component; 20-99 mM amino acid component; 10-129 mM tonicifier; 10-129 mM ionic strength provider; 0.001-2 mM chelator; and a diluent.

D716. 120-220 mg/mL secukinumab; 51-60 mM buffer system; 50-129 mM sugar component; 20-99 mM amino acid component; 10-129 mM tonicifier; 10-129 mM ionic strength provider; 0.001-2 mM chelator; and a diluent.

D717. 120-220 mg/mL secukinumab; 1-9 mM buffer system; 130-174 mM sugar component; 20-99 mM amino acid component; 10-129 mM tonicifier; 10-129 mM ionic strength provider; 0.001-2 mM chelator; and a diluent.

(continued)

D718. 120-220 mg/mL secukinumab; 51-60 mM buffer system; 130-174 mM sugar component; 20-99 mM amino acid component; 10-129 mM tonicifier; 10-129 mM ionic strength provider; 0.001-2 mM chelator; and a diluent.

D719. 120-220 mg/mL secukinumab; 1-9 mM buffer system; 50-129 mM sugar component; 100-149 mM amino acid component; 10-129 mM tonicifier; 10-129 mM ionic strength provider; 0.001-2 mM chelator; and a diluent.

D720. 120-220 mg/mL secukinumab; 51-60 mM buffer system; 50-129 mM sugar component; 100-149 mM amino acid component; 10-129 mM tonicifier; 10-129 mM ionic strength provider; 0.001-2 mM chelator; and a diluent.

D721. 120-220 mg/mL secukinumab; 1-9 mM buffer system; 130-174 mM sugar component; 100-149 mM amino acid component; 10-129 mM tonicifier; 10-129 mM ionic strength provider; 0.001-2 mM chelator; and a diluent.

D722. 120-220 mg/mL secukinumab; 51-60 mM buffer system; 130-174 mM sugar component; 100-149 mM amino acid component; 10-129 mM tonicifier; 10-129 mM ionic strength provider; 0.001-2 mM chelator; and a diluent.

D723. 120-220 mg/mL secukinumab; 1-9 mM buffer system; 50-129 mM sugar component; 20-99 mM amino acid component; 10-129 mM tonicifier; 0.1-20 mM antioxidant; 0.001-2 mM chelator; and a diluent.

D724. 120-220 mg/mL secukinumab; 51-60 mM buffer system; 50-129 mM sugar component; 20-99 mM amino acid component; 10-129 mM tonicifier; 0.1-20 mM antioxidant; 0.001-2 mM chelator; and a diluent.

D725. 120-220 mg/mL secukinumab; 1-9 mM buffer system; 130-174 mM sugar component; 20-99 mM amino acid component; 10-129 mM tonicifier; 0.1-20 mM antioxidant; 0.001-2 mM chelator; and a diluent.

D726. 120-220 mg/mL secukinumab; 51-60 mM buffer system; 130-174 mM sugar component; 20-99 mM amino acid component; 10-129 mM tonicifier; 0.1-20 mM antioxidant; 0.001-2 mM chelator; and a diluent.

D727. 120-220 mg/mL secukinumab; 1-9 mM buffer system; 50-129 mM sugar component; 100-149 mM amino acid component; 10-129 mM tonicifier; 0.1-20 mM antioxidant; 0.001-2 mM chelator; and a diluent.

D728. 120-220 mg/mL secukinumab; 51-60 mM buffer system; 50-129 mM sugar component; 100-149 mM amino acid component; 10-129 mM tonicifier; 0.1-20 mM antioxidant; 0.001-2 mM chelator; and a diluent.

D729. 120-220 mg/mL secukinumab; 1-9 mM buffer system; 130-174 mM sugar component; 100-149 mM amino acid component; 10-129 mM tonicifier; 0.1-20 mM antioxidant; 0.001-2 mM chelator; and a diluent.

D730. 120-220 mg/mL secukinumab; 51-60 mM buffer system; 130-174 mM sugar component; 100-149 mM amino acid component; 10-129 mM tonicifier; 0.1-20 mM antioxidant; 0.001-2 mM chelator; and a diluent.

D731. 120-220 mg/mL secukinumab; 1-9 mM buffer system; 50-129 mM sugar component; 20-99 mM amino acid component; 10-129 mM ionic strength provider; 0.1-20 mM antioxidant; 0.001-2 mM chelator; and a diluent.

D732. 120-220 mg/mL secukinumab; 51-60 mM buffer system; 50-129 mM sugar component; 20-99 mM amino acid component; 10-129 mM ionic strength provider; 0.1-20 mM antioxidant; 0.001-2 mM chelator; and a diluent.

D733. 120-220 mg/mL secukinumab; 1-9 mM buffer system; 130-174 mM sugar component; 20-99 mM amino acid component; 10-129 mM ionic strength provider; 0.1-20 mM antioxidant; 0.001-2 mM chelator; and a diluent.

D734. 120-220 mg/mL secukinumab; 51-60 mM buffer system; 130-174 mM sugar component; 20-99 mM amino acid component; 10-129 mM ionic strength provider; 0.1-20 mM antioxidant; 0.001-2 mM chelator; and a diluent.

D735. 120-220 mg/mL secukinumab; 1-9 mM buffer system; 50-129 mM sugar component; 100-149 mM amino acid component; 10-129 mM ionic strength provider; 0.1-20 mM antioxidant; 0.001-2 mM chelator; and a diluent.

D736. 120-220 mg/mL secukinumab; 51-60 mM buffer system; 50-129 mM sugar component; 100-149 mM amino acid component; 10-129 mM ionic strength provider; 0.1-20 mM antioxidant; 0.001-2 mM chelator; and a diluent.

D737. 120-220 mg/mL secukinumab; 1-9 mM buffer system; 130-174 mM sugar component; 100-149 mM amino acid component; 10-129 mM ionic strength provider; 0.1-20 mM antioxidant; 0.001-2 mM chelator; and a diluent.

(continued)

D738. 120-220 mg/mL secukinumab; 51-60 mM buffer system; 130-174 mM sugar component; 100-149 mM amino acid component; 10-129 mM ionic strength provider; 0.1-20 mM antioxidant; 0.001-2 mM chelator; and a diluent.

D739. 120-220 mg/mL secukinumab; 1-9 mM buffer system; 50-129 mM sugar component; 0.01-1.2 mg/mL surfactant; 10-129 mM tonicifier; 10-129 mM ionic strength provider; 0.1-20 mM antioxidant; and a diluent.

D740. 120-220 mg/mL secukinumab; 51-60 mM buffer system; 50-129 mM sugar component; 0.01-1.2 mg/mL surfactant; 10-129 mM tonicifier; 10-129 mM ionic strength provider; 0.1-20 mM antioxidant; and a diluent.

D741. 120-220 mg/mL secukinumab; 1-9 mM buffer system; 130-174 mM sugar component; 0.01-1.2 mg/mL surfactant; 10-129 mM tonicifier; 10-129 mM ionic strength provider; 0.1-20 mM antioxidant; and a diluent.

D742. 120-220 mg/mL secukinumab; 51-60 mM buffer system; 130-174 mM sugar component; 0.01-1.2 mg/mL surfactant; 10-129 mM tonicifier; 10-129 mM ionic strength provider; 0.1-20 mM antioxidant; and a diluent.

D743. 120-220 mg/mL secukinumab; 1-9 mM buffer system; 50-129 mM sugar component; 0.01-1.2 mg/mL surfactant; 10-129 mM tonicifier; 10-129 mM ionic strength provider; 0.001-2 mM chelator; and a diluent.

D744. 120-220 mg/mL secukinumab; 51-60 mM buffer system; 50-129 mM sugar component; 0.01-1.2 mg/mL surfactant; 10-129 mM tonicifier; 10-129 mM ionic strength provider; 0.001-2 mM chelator; and a diluent.

D745. 120-220 mg/mL secukinumab; 1-9 mM buffer system; 130-174 mM sugar component; 0.01-1.2 mg/mL surfactant; 10-129 mM tonicifier; 10-129 mM ionic strength provider; 0.001-2 mM chelator; and a diluent.

D746. 120-220 mg/mL secukinumab; 51-60 mM buffer system; 130-174 mM sugar component; 0.01-1.2 mg/mL surfactant; 10-129 mM tonicifier; 10-129 mM ionic strength provider; 0.001-2 mM chelator; and a diluent.

D747. 120-220 mg/mL secukinumab; 1-9 mM buffer system; 50-129 mM sugar component; 0.01-1.2 mg/mL surfactant; 10-129 mM tonicifier; 0.1-20 mM antioxidant; 0.001-2 mM chelator; and a diluent.

D748. 120-220 mg/mL secukinumab; 51-60 mM buffer system; 50-129 mM sugar component; 0.01-1.2 mg/mL surfactant; 10-129 mM tonicifier; 0.1-20 mM antioxidant; 0.001-2 mM chelator; and a diluent.

D749. 120-220 mg/mL secukinumab; 1-9 mM buffer system; 130-174 mM sugar component; 0.01-1.2 mg/mL surfactant; 10-129 mM tonicifier; 0.1-20 mM antioxidant; 0.001-2 mM chelator; and a diluent.

D750. 120-220 mg/mL secukinumab; 51-60 mM buffer system; 130-174 mM sugar component; 0.01-1.2 mg/mL surfactant; 10-129 mM tonicifier; 0.1-20 mM antioxidant; 0.001-2 mM chelator; and a diluent.

D751. 120-220 mg/mL secukinumab; 1-9 mM buffer system; 50-129 mM sugar component; 0.01-1.2 mg/mL surfactant; 10-129 mM ionic strength provider; 0.1-20 mM antioxidant; 0.001-2 mM chelator; and a diluent.

D752. 120-220 mg/mL secukinumab; 51-60 mM buffer system; 50-129 mM sugar component; 0.01-1.2 mg/mL surfactant; 10-129 mM ionic strength provider; 0.1-20 mM antioxidant; 0.001-2 mM chelator; and a diluent.

D753. 120-220 mg/mL secukinumab; 1-9 mM buffer system; 130-174 mM sugar component; 0.01-1.2 mg/mL surfactant; 10-129 mM ionic strength provider; 0.1-20 mM antioxidant; 0.001-2 mM chelator; and a diluent.

D754. 120-220 mg/mL secukinumab; 51-60 mM buffer system; 130-174 mM sugar component; 0.01-1.2 mg/mL surfactant; 10-129 mM ionic strength provider; 0.1-20 mM antioxidant; 0.001-2 mM chelator; and a diluent.

D755. 120-220 mg/mL secukinumab; 1-9 mM buffer system; 50-129 mM sugar component; 10-129 mM tonicifier; 10-129 mM ionic strength provider; 0.1-20 mM antioxidant; 0.001-2 mM chelator; and a diluent.

D756. 120-220 mg/mL secukinumab; 51-60 mM buffer system; 50-129 mM sugar component; 10-129 mM tonicifier; 10-129 mM ionic strength provider; 0.1-20 mM antioxidant; 0.001-2 mM chelator; and a diluent.

D757. 120-220 mg/mL secukinumab; 1-9 mM buffer system; 130-174 mM sugar component; 10-129 mM tonicifier; 10-129 mM ionic strength provider; 0.1-20 mM antioxidant; 0.001-2 mM chelator; and a diluent.

D758. 120-220 mg/mL secukinumab; 51-60 mM buffer system; 130-174 mM sugar component; 10-129 mM tonicifier; 10-129 mM ionic strength provider; 0.1-20 mM antioxidant; 0.001-2 mM chelator; and a diluent.

D759. 120-220 mg/mL secukinumab; 1-9 mM buffer system; 20-99 mM amino acid component; 0.01-1.2 mg/mL surfactant; 10-129 mM tonicifier; 10-129 mM ionic strength provider; 0.1-20 mM antioxidant; and a diluent.

D760. 120-220 mg/mL secukinumab; 51-60 mM buffer system; 20-99 mM amino acid component; 0.01-1.2 mg/mL surfactant; 10-129 mM tonicifier; 10-129 mM ionic strength provider; 0.1-20 mM antioxidant; and a diluent.

D761. 120-220 mg/mL secukinumab; 1-9 mM buffer system; 100-149 mM amino acid component; 0.01-1.2 mg/mL surfactant; 10-129 mM tonicifier; 10-129 mM ionic strength provider; 0.1-20 mM antioxidant; and a diluent.

(continued)

D762. 120-220 mg/mL secukinumab; 51-60 mM buffer system; 100-149 mM amino acid component; 0.01-1.2 mg/mL surfactant; 10-129 mM tonicifier; 10-129 mM ionic strength provider; 0.1-20 mM antioxidant; and a diluent.

D763. 120-220 mg/mL secukinumab; 1-9 mM buffer system; 20-99 mM amino acid component; 0.01-1.2 mg/mL surfactant; 10-129 mM tonicifier; 10-129 mM ionic strength provider; 0.001-2 mM chelator; and a diluent.

D764. 120-220 mg/mL secukinumab; 51-60 mM buffer system; 20-99 mM amino acid component; 0.01-1.2 mg/mL surfactant; 10-129 mM tonicifier; 10-129 mM ionic strength provider; 0.001-2 mM chelator; and a diluent.

D765. 120-220 mg/mL secukinumab; 1-9 mM buffer system; 100-149 mM amino acid component; 0.01-1.2 mg/mL surfactant; 10-129 mM tonicifier; 10-129 mM ionic strength provider; 0.001-2 mM chelator; and a diluent.

D766. 120-220 mg/mL secukinumab; 51-60 mM buffer system; 100-149 mM amino acid component; 0.01-1.2 mg/mL surfactant; 10-129 mM tonicifier; 10-129 mM ionic strength provider; 0.001-2 mM chelator; and a diluent.

D767. 120-220 mg/mL secukinumab; 1-9 mM buffer system; 20-99 mM amino acid component; 0.01-1.2 mg/mL surfactant; 10-129 mM tonicifier; 0.1-20 mM antioxidant; 0.001-2 mM chelator; and a diluent.

D768. 120-220 mg/mL secukinumab; 51-60 mM buffer system; 20-99 mM amino acid component; 0.01-1.2 mg/mL surfactant; 10-129 mM tonicifier; 0.1-20 mM antioxidant; 0.001-2 mM chelator; and a diluent.

D769. 120-220 mg/mL secukinumab; 1-9 mM buffer system; 100-149 mM amino acid component; 0.01-1.2 mg/mL surfactant; 10-129 mM tonicifier; 0.1-20 mM antioxidant; 0.001-2 mM chelator; and a diluent.

D770. 120-220 mg/mL secukinumab; 51-60 mM buffer system; 100-149 mM amino acid component; 0.01-1.2 mg/mL surfactant; 10-129 mM tonicifier; 0.1-20 mM antioxidant; 0.001-2 mM chelator; and a diluent.

D771. 120-220 mg/mL secukinumab; 1-9 mM buffer system; 20-99 mM amino acid component; 0.01-1.2 mg/mL surfactant; 10-129 mM ionic strength provider; 0.1-20 mM antioxidant; 0.001-2 mM chelator; and a diluent.

D772. 120-220 mg/mL secukinumab; 51-60 mM buffer system; 20-99 mM amino acid component; 0.01-1.2 mg/mL surfactant; 10-129 mM ionic strength provider; 0.1-20 mM antioxidant; 0.001-2 mM chelator; and a diluent.

D773. 120-220 mg/mL secukinumab; 1-9 mM buffer system; 100-149 mM amino acid component; 0.01-1.2 mg/mL surfactant; 10-129 mM ionic strength provider; 0.1-20 mM antioxidant; 0.001-2 mM chelator; and a diluent.

D774. 120-220 mg/mL secukinumab; 51-60 mM buffer system; 100-149 mM amino acid component; 0.01-1.2 mg/mL surfactant; 10-129 mM ionic strength provider; 0.1-20 mM antioxidant; 0.001-2 mM chelator; and a diluent.

D775. 120-220 mg/mL secukinumab; 1-9 mM buffer system; 20-99 mM amino acid component; 10-129 mM tonicifier; 10-129 mM ionic strength provider; 0.1-20 mM antioxidant; 0.001-2 mM chelator; and a diluent.

D776. 120-220 mg/mL secukinumab; 51-60 mM buffer system; 20-99 mM amino acid component; 10-129 mM tonicifier; 10-129 mM ionic strength provider; 0.1-20 mM antioxidant; 0.001-2 mM chelator; and a diluent.

D777. 120-220 mg/mL secukinumab; 1-9 mM buffer system; 100-149 mM amino acid component; 10-129 mM tonicifier; 10-129 mM ionic strength provider; 0.1-20 mM antioxidant; 0.001-2 mM chelator; and a diluent.

D778. 120-220 mg/mL secukinumab; 51-60 mM buffer system; 100-149 mM amino acid component; 10-129 mM tonicifier; 10-129 mM ionic strength provider; 0.1-20 mM antioxidant; 0.001-2 mM chelator; and a diluent.

D779. 120-220 mg/mL secukinumab; 1-9 mM buffer system; 0.01-1.2 mg/mL surfactant; 10-129 mM tonicifier; 10-129 mM ionic strength provider; 0.1-20 mM antioxidant; 0.001-2 mM chelator; and a diluent.

D780. 120-220 mg/mL secukinumab; 51-60 mM buffer system; 0.01-1.2 mg/mL surfactant; 10-129 mM tonicifier; 10-129 mM ionic strength provider; 0.1-20 mM antioxidant; 0.001-2 mM chelator; and a diluent.

D781. 120-220 mg/mL secukinumab; 50-129 mM sugar component; 20-99 mM amino acid component; 0.01-1.2 mg/mL surfactant; 10-129 mM tonicifier; 10-129 mM ionic strength provider; 0.1-20 mM antioxidant; and a diluent.

D782. 120-220 mg/mL secukinumab; 130-174 mM sugar component; 20-99 mM amino acid component; 0.01-1.2 mg/mL surfactant; 10-129 mM tonicifier; 10-129 mM ionic strength provider; 0.1-20 mM antioxidant; and a diluent.

(continued)

D783. 120-220 mg/mL secukinumab; 50-129 mM sugar component; 100-149 mM amino acid component; 0.01-1.2 mg/mL surfactant; 10-129 mM tonicifier; 10-129 mM ionic strength provider; 0.1-20 mM antioxidant; and a diluent.

D784. 120-220 mg/mL secukinumab; 130-174 mM sugar component; 100-149 mM amino acid component; 0.01-1.2 mg/mL surfactant; 10-129 mM tonicifier; 10-129 mM ionic strength provider; 0.1-20 mM antioxidant; and a diluent.

D785. 120-220 mg/mL secukinumab; 50-129 mM sugar component; 20-99 mM amino acid component; 0.01-1.2 mg/mL surfactant; 10-129 mM tonicifier; 10-129 mM ionic strength provider; 0.001-2 mM chelator; and a diluent.

D786. 120-220 mg/mL secukinumab; 130-174 mM sugar component; 20-99 mM amino acid component; 0.01-1.2 mg/mL surfactant; 10-129 mM tonicifier; 10-129 mM ionic strength provider; 0.001-2 mM chelator; and a diluent.

D787. 120-220 mg/mL secukinumab; 50-129 mM sugar component; 100-149 mM amino acid component; 0.01-1.2 mg/mL surfactant; 10-129 mM tonicifier; 10-129 mM ionic strength provider; 0.001-2 mM chelator; and a diluent.

D788. 120-220 mg/mL secukinumab; 130-174 mM sugar component; 100-149 mM amino acid component; 0.01-1.2 mg/mL surfactant; 10-129 mM tonicifier; 10-129 mM ionic strength provider; 0.001-2 mM chelator; and a diluent.

D789. 120-220 mg/mL secukinumab; 50-129 mM sugar component; 20-99 mM amino acid component; 0.01-1.2 mg/mL surfactant; 10-129 mM tonicifier; 0.1-20 mM antioxidant; 0.001-2 mM chelator; and a diluent.

D790. 120-220 mg/mL secukinumab; 130-174 mM sugar component; 20-99 mM amino acid component; 0.01-1.2 mg/mL surfactant; 10-129 mM tonicifier; 0.1-20 mM antioxidant; 0.001-2 mM chelator; and a diluent.

D791. 120-220 mg/mL secukinumab; 50-129 mM sugar component; 100-149 mM amino acid component; 0.01-1.2 mg/mL surfactant; 10-129 mM tonicifier; 0.1-20 mM antioxidant; 0.001-2 mM chelator; and a diluent.

D792. 120-220 mg/mL secukinumab; 130-174 mM sugar component; 100-149 mM amino acid component; 0.01-1.2 mg/mL surfactant; 10-129 mM tonicifier; 0.1-20 mM antioxidant; 0.001-2 mM chelator; and a diluent.

D793. 120-220 mg/mL secukinumab; 50-129 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D794. 120-220 mg/mL secukinumab; 130-174 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D795. 120-220 mg/mL secukinumab; 50-129 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D796. 120-220 mg/mL secukinumab; 130-174 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D797. 120-220 mg/mL secukinumab; 50-129 mM sugar component; 20-99 mM amino acid component; 10-129 mM tonicifier; 10-129 mM ionic strength provider; 0.1-20 mM antioxidant; 0.001-2 mM chelator; and a diluent.

D798. 120-220 mg/mL secukinumab; 130-174 mM sugar component; 20-99 mM amino acid component; 10-129 mM tonicifier; 10-129 mM ionic strength provider; 0.1-20 mM antioxidant; 0.001-2 mM chelator; and a diluent.

D799. 120-220 mg/mL secukinumab; 50-129 mM sugar component; 100-149 mM amino acid component; 10-129 mM tonicifier; 10-129 mM ionic strength provider; 0.1-20 mM antioxidant; 0.001-2 mM chelator; and a diluent.

D800. 120-220 mg/mL secukinumab; 130-174 mM sugar component; 100-149 mM amino acid component; 10-129 mM tonicifier; 10-129 mM ionic strength provider; 0.1-20 mM antioxidant; 0.001-2 mM chelator; and a diluent.

D801. 120-220 mg/mL secukinumab; 50-129 mM sugar component; 0.01-1.2 mg/mL surfactant; 10-129 mM tonicifier; 10-129 mM ionic strength provider; 0.1-20 mM antioxidant; 0.001-2 mM chelator; and a diluent.

(continued)

| | |
|---|---|
| D802. | 120-220 mg/mL secukinumab; 130-174 mM sugar component; 0.01-1.2 mg/mL surfactant; 10-129 mM tonicifier; 10-129 mM ionic strength provider; 0.1-20 mM antioxidant; 0.001-2 mM chelator; and a diluent. |
| D803. | 120-220 mg/mL secukinumab; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent. |
| D804. | 120-220 mg/mL secukinumab; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent. |
| D805. | 120-220 mg/mL secukinumab; 1-9 mM buffer system; 50-129 mM sugar component; 20-99 mM amino acid component; 0.01-1.2 mg/mL surfactant; 10-129 mM tonicifier; 10-129 mM ionic strength provider; 0.1-20 mM antioxidant; and a diluent. |
| D806. | 120-220 mg/mL secukinumab; 51-60 mM buffer system; 50-129 mM sugar component; 20-99 mM amino acid component; 0.01-1.2 mg/mL surfactant; 10-129 mM tonicifier; 10-129 mM ionic strength provider; 0.1-20 mM antioxidant; and a diluent. |
| D807. | 120-220 mg/mL secukinumab; 1-9 mM buffer system; 130-174 mM sugar component; 20-99 mM amino acid component; 0.01-1.2 mg/mL surfactant; 10-129 mM tonicifier; 10-129 mM ionic strength provider; 0.1-20 mM antioxidant; and a diluent. |
| D808. | 120-220 mg/mL secukinumab; 51-60 mM buffer system; 130-174 mM sugar component; 20-99 mM amino acid component; 0.01-1.2 mg/mL surfactant; 10-129 mM tonicifier; 10-129 mM ionic strength provider; 0.1-20 mM antioxidant; and a diluent. |
| D809. | 120-220 mg/mL secukinumab; 1-9 mM buffer system; 50-129 mM sugar component; 100-149 mM amino acid component; 0.01-1.2 mg/mL surfactant; 10-129 mM tonicifier; 10-129 mM ionic strength provider; 0.1-20 mM antioxidant; and a diluent. |
| D810. | 120-220 mg/mL secukinumab; 51-60 mM buffer system; 50-129 mM sugar component; 100-149 mM amino acid component; 0.01-1.2 mg/mL surfactant; 10-129 mM tonicifier; 10-129 mM ionic strength provider; 0.1-20 mM antioxidant; and a diluent. |
| D811. | 120-220 mg/mL secukinumab; 1-9 mM buffer system; 130-174 mM sugar component; 100-149 mM amino acid component; 0.01-1.2 mg/mL surfactant; 10-129 mM tonicifier; 10-129 mM ionic strength provider; 0.1-20 mM antioxidant; and a diluent. |
| D812. | 120-220 mg/mL secukinumab; 51-60 mM buffer system; 130-174 mM sugar component; 100-149 mM amino acid component; 0.01-1.2 mg/mL surfactant; 10-129 mM tonicifier; 10-129 mM ionic strength provider; 0.1-20 mM antioxidant; and a diluent. |
| D813. | 120-220 mg/mL secukinumab; 1-9 mM buffer system; 50-129 mM sugar component; 20-99 mM amino acid component; 0.01-1.2 mg/mL surfactant; 10-129 mM tonicifier; 10-129 mM ionic strength provider; 0.001-2 mM chelator; and a diluent. |
| D814. | 120-220 mg/mL secukinumab; 51-60 mM buffer system; 50-129 mM sugar component; 20-99 mM amino acid component; 0.01-1.2 mg/mL surfactant; 10-129 mM tonicifier; 10-129 mM ionic strength provider; 0.001-2 mM chelator; and a diluent. |
| D815. | 120-220 mg/mL secukinumab; 1-9 mM buffer system; 130-174 mM sugar component; 20-99 mM amino acid component; 0.01-1.2 mg/mL surfactant; 10-129 mM tonicifier; 10-129 mM ionic strength provider; 0.001-2 mM chelator; and a diluent. |
| D816. | 120-220 mg/mL secukinumab; 51-60 mM buffer system; 130-174 mM sugar component; 20-99 mM amino acid component; 0.01-1.2 mg/mL surfactant; 10-129 mM tonicifier; 10-129 mM ionic strength provider; 0.001-2 mM chelator; and a diluent. |
| D817. | 120-220 mg/mL secukinumab; 1-9 mM buffer system; 50-129 mM sugar component; 100-149 mM amino acid component; 0.01-1.2 mg/mL surfactant; 10-129 mM tonicifier; 10-129 mM ionic strength provider; 0.001-2 mM chelator; and a diluent. |
| D818. | 120-220 mg/mL secukinumab; 51-60 mM buffer system; 50-129 mM sugar component; 100-149 mM amino acid component; 0.01-1.2 mg/mL surfactant; 10-129 mM tonicifier; 10-129 mM ionic strength provider; 0.001-2 mM chelator; and a diluent. |
| D819. | 120-220 mg/mL secukinumab; 1-9 mM buffer system; 130-174 mM sugar component; 100-149 mM amino acid component; 0.01-1.2 mg/mL surfactant; 10-129 mM tonicifier; 10-129 mM ionic strength provider; 0.001-2 mM chelator; and a diluent. |

(continued)

D820. 120-220 mg/mL secukinumab; 51-60 mM buffer system; 130-174 mM sugar component; 100-149 mM amino acid component; 0.01-1.2 mg/mL surfactant; 10-129 mM tonicifier; 10-129 mM ionic strength provider; 0.001-2 mM chelator; and a diluent.

D821. 120-220 mg/mL secukinumab; 1-9 mM buffer system; 50-129 mM sugar component; 20-99 mM amino acid component; 0.01-1.2 mg/mL surfactant; 10-129 mM tonicifier; 0.1-20 mM antioxidant; 0.001-2 mM chelator; and a diluent.

D822. 120-220 mg/mL secukinumab; 51-60 mM buffer system; 50-129 mM sugar component; 20-99 mM amino acid component; 0.01-1.2 mg/mL surfactant; 10-129 mM tonicifier; 0.1-20 mM antioxidant; 0.001-2 mM chelator; and a diluent.

D823. 120-220 mg/mL secukinumab; 1-9 mM buffer system; 130-174 mM sugar component; 20-99 mM amino acid component; 0.01-1.2 mg/mL surfactant; 10-129 mM tonicifier; 0.1-20 mM antioxidant; 0.001-2 mM chelator; and a diluent.

D824. 120-220 mg/mL secukinumab; 51-60 mM buffer system; 130-174 mM sugar component; 20-99 mM amino acid component; 0.01-1.2 mg/mL surfactant; 10-129 mM tonicifier; 0.1-20 mM antioxidant; 0.001-2 mM chelator; and a diluent.

D825. 120-220 mg/mL secukinumab; 1-9 mM buffer system; 50-129 mM sugar component; 100-149 mM amino acid component; 0.01-1.2 mg/mL surfactant; 10-129 mM tonicifier; 0.1-20 mM antioxidant; 0.001-2 mM chelator; and a diluent.

D826. 120-220 mg/mL secukinumab; 51-60 mM buffer system; 50-129 mM sugar component; 100-149 mM amino acid component; 0.01-1.2 mg/mL surfactant; 10-129 mM tonicifier; 0.1-20 mM antioxidant; 0.001-2 mM chelator; and a diluent.

D827. 120-220 mg/mL secukinumab; 1-9 mM buffer system; 130-174 mM sugar component; 100-149 mM amino acid component; 0.01-1.2 mg/mL surfactant; 10-129 mM tonicifier; 0.1-20 mM antioxidant; 0.001-2 mM chelator; and a diluent.

D828. 120-220 mg/mL secukinumab; 51-60 mM buffer system; 130-174 mM sugar component; 100-149 mM amino acid component; 0.01-1.2 mg/mL surfactant; 10-129 mM tonicifier; 0.1-20 mM antioxidant; 0.001-2 mM chelator; and a diluent.

D829. 120-220 mg/mL secukinumab; 1-9 mM buffer system; 50-129 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D830. 120-220 mg/mL secukinumab; 51-60 mM buffer system; 50-129 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D831. 120-220 mg/mL secukinumab; 1-9 mM buffer system; 130-174 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D832. 120-220 mg/mL secukinumab; 51-60 mM buffer system; 130-174 mM sugar component; 20-99 mM amino acid component; 0.01-1.2 mg/mL surfactant; 10-129 mM ionic strength provider; 0.1-20 mM antioxidant; 0.001-2 mM chelator; and a diluent.

D833. 120-220 mg/mL secukinumab; 1-9 mM buffer system; 50-129 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D834. 120-220 mg/mL secukinumab; 51-60 mM buffer system; 50-129 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D835. 120-220 mg/mL secukinumab; 1-9 mM buffer system; 130-174 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D836. 120-220 mg/mL secukinumab; 51-60 mM buffer system; 130-174 mM sugar component; 100-149 mM amino acid component; 0.01-1.2 mg/mL surfactant; 10-129 mM ionic strength provider; 0.1-20 mM antioxidant; 0.001-2 mM chelator; and a diluent.

D837. 120-220 mg/mL secukinumab; 1-9 mM buffer system; 50-129 mM sugar component; 20-99 mM amino acid component; 10-129 mM tonicifier; 10-129 mM ionic strength provider; 0.1-20 mM antioxidant; 0.001-2 mM chelator; and a diluent.

(continued)

D838. 120-220 mg/mL secukinumab; 51-60 mM buffer system; 50-129 mM sugar component; 20-99 mM amino acid component; 10-129 mM tonicifier; 10-129 mM ionic strength provider; 0.1-20 mM antioxidant; 0.001-2 mM chelator; and a diluent.

D839. 120-220 mg/mL secukinumab; 1-9 mM buffer system; 130-174 mM sugar component; 20-99 mM amino acid component; 10-129 mM tonicifier; 10-129 mM ionic strength provider; 0.1-20 mM antioxidant; 0.001-2 mM chelator; and a diluent.

D840. 120-220 mg/mL secukinumab; 51-60 mM buffer system; 130-174 mM sugar component; 20-99 mM amino acid component; 10-129 mM tonicifier; 10-129 mM ionic strength provider; 0.1-20 mM antioxidant; 0.001-2 mM chelator; and a diluent.

D841. 120-220 mg/mL secukinumab; 1-9 mM buffer system; 50-129 mM sugar component; 100-149 mM amino acid component; 10-129 mM tonicifier; 10-129 mM ionic strength provider; 0.1-20 mM antioxidant; 0.001-2 mM chelator; and a diluent.

D842. 120-220 mg/mL secukinumab; 51-60 mM buffer system; 50-129 mM sugar component; 100-149 mM amino acid component; 10-129 mM tonicifier; 10-129 mM ionic strength provider; 0.1-20 mM antioxidant; 0.001-2 mM chelator; and a diluent.

D843. 120-220 mg/mL secukinumab; 1-9 mM buffer system; 130-174 mM sugar component; 100-149 mM amino acid component; 10-129 mM tonicifier; 10-129 mM ionic strength provider; 0.1-20 mM antioxidant; 0.001-2 mM chelator; and a diluent.

D844. 120-220 mg/mL secukinumab; 51-60 mM buffer system; 130-174 mM sugar component; 100-149 mM amino acid component; 10-129 mM tonicifier; 10-129 mM ionic strength provider; 0.1-20 mM antioxidant; 0.001-2 mM chelator; and a diluent.

D845. 120-220 mg/mL secukinumab; 1-9 mM buffer system; 50-129 mM sugar component; 0.01-1.2 mg/mL surfactant; 10-129 mM tonicifier; 10-129 mM ionic strength provider; 0.1-20 mM antioxidant; 0.001-2 mM chelator; and a diluent.

D846. 120-220 mg/mL secukinumab; 51-60 mM buffer system; 50-129 mM sugar component; 0.01-1.2 mg/mL surfactant; 10-129 mM tonicifier; 10-129 mM ionic strength provider; 0.1-20 mM antioxidant; 0.001-2 mM chelator; and a diluent.

D847. 120-220 mg/mL secukinumab; 1-9 mM buffer system; 130-174 mM sugar component; 0.01-1.2 mg/mL surfactant; 10-129 mM tonicifier; 10-129 mM ionic strength provider; 0.1-20 mM antioxidant; 0.001-2 mM chelator; and a diluent.

D848. 120-220 mg/mL secukinumab; 51-60 mM buffer system; 130-174 mM sugar component; 0.01-1.2 mg/mL surfactant; 10-129 mM tonicifier; 10-129 mM ionic strength provider; 0.1-20 mM antioxidant; 0.001-2 mM chelator; and a diluent.

D849. 120-220 mg/mL secukinumab; 1-9 mM buffer system; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D850. 120-220 mg/mL secukinumab; 51-60 mM buffer system; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D851. 120-220 mg/mL secukinumab; 1-9 mM buffer system; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D852. 120-220 mg/mL secukinumab; 51-60 mM buffer system; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D853. 120-220 mg/mL secukinumab; 50-129 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D854. 120-220 mg/mL secukinumab; 130-174 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D855. 120-220 mg/mL secukinumab; 50-129 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

(continued)

D856. 120-220 mg/mL secukinumab; 130-174 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D857. 120-220 mg/mL secukinumab; 1-9 mM buffer system; 50-129 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D858. 120-220 mg/mL secukinumab; 51-60 mM buffer system; 50-129 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D859. 120-220 mg/mL secukinumab; 1-9 mM buffer system; 130-174 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D860. 120-220 mg/mL secukinumab; 51-60 mM buffer system; 130-174 mM sugar component; 20-99 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D861. 120-220 mg/mL secukinumab; 1-9 mM buffer system; 50-129 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D862. 120-220 mg/mL secukinumab; 51-60 mM buffer system; 50-129 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D863. 120-220 mg/mL secukinumab; 1-9 mM buffer system; 130-174 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D864. 120-220 mg/mL secukinumab; 51-60 mM buffer system; 130-174 mM sugar component; 100-149 mM amino acid component; 0.01-1.2 mg/mL surfactant; 10-129 mM tonicifier; 10-129 mM ionic strength provider; 0.1-20 mM antioxidant; 0.001-2 mM chelator; and a diluent.

D865. 120-220 mg/mL secukinumab; and 200-250 mM sugar component.

D866. 120-220 mg/mL secukinumab; 1-9 mM buffer system; and 200-250 mM sugar component.

D867. 120-220 mg/mL secukinumab; 51-60 mM buffer system; and 200-250 mM sugar component.

D868. 120-220 mg/mL secukinumab; 200-250 mM sugar component; and 20-99 mM amino acid component.

D869. 120-220 mg/mL secukinumab; 200-250 mM sugar component; and 100-149 mM amino acid component.

D870. 120-220 mg/mL secukinumab; 200-250 mM sugar component; and 0.01-1.2 mg/mL surfactant.

D871. 120-220 mg/mL secukinumab; 200-250 mM sugar component; and 0.1-20 mM antioxidant.

D872. 120-220 mg/mL secukinumab; 200-250 mM sugar component; and 10-129 mM tonicifier.

D873. 120-220 mg/mL secukinumab; 200-250 mM sugar component; and 10-129 mM ionic strength provider.

D874. 120-220 mg/mL secukinumab; 200-250 mM sugar component; and 0.001-2 mM chelator.

D875. 120-220 mg/mL secukinumab; 1-9 mM buffer system; 200-250 mM sugar component; and 20-99 mM amino acid component.

D876. 120-220 mg/mL secukinumab; 51-60 mM buffer system; 200-250 mM sugar component; and 20-99 mM amino acid component.

D877. 120-220 mg/mL secukinumab; 1-9 mM buffer system; 200-250 mM sugar component; and 100-149 mM amino acid component.

D878. 120-220 mg/mL secukinumab; 51-60 mM buffer system; 200-250 mM sugar component; and 100-149 mM amino acid component.

D879. 120-220 mg/mL secukinumab; 1-9 mM buffer system; 200-250 mM sugar component; and 0.01-1.2 mg/mL surfactant.

D880. 120-220 mg/mL secukinumab; 51-60 mM buffer system; 200-250 mM sugar component; and 0.01-1.2 mg/mL surfactant.

D881. 120-220 mg/mL secukinumab; 1-9 mM buffer system; 200-250 mM sugar component; and 0.1-20 mM antioxidant.

D882. 120-220 mg/mL secukinumab; 51-60 mM buffer system; 200-250 mM sugar component; and 0.1-20 mM antioxidant.

(continued)

D883. 120-220 mg/mL secukinumab; 1-9 mM buffer system; 200-250 mM sugar component; and 10-129 mM tonicifier.

D884. 120-220 mg/mL secukinumab; 51-60 mM buffer system; 200-250 mM sugar component; and 10-129 mM tonicifier.

D885. 120-220 mg/mL secukinumab; 1-9 mM buffer system; 200-250 mM sugar component; and 10-129 mM ionic strength provider.

D886. 120-220 mg/mL secukinumab; 51-60 mM buffer system; 200-250 mM sugar component; and 10-129 mM ionic strength provider.

D887. 120-220 mg/mL secukinumab; 1-9 mM buffer system; 200-250 mM sugar component; and 0.001-2 mM chelator.

D888. 120-220 mg/mL secukinumab; 51-60 mM buffer system; 200-250 mM sugar component; and 0.001-2 mM chelator.

D889. 120-220 mg/mL secukinumab; 200-250 mM sugar component; 20-99 mM amino acid component; and 0.01-1.2 mg/mL surfactant.

D890. 120-220 mg/mL secukinumab; 200-250 mM sugar component; 100-149 mM amino acid component; and 0.01-1.2 mg/mL surfactant.

D891. 120-220 mg/mL secukinumab; 200-250 mM sugar component; 20-99 mM amino acid component; and 0.1-20 mM antioxidant.

D892. 120-220 mg/mL secukinumab; 200-250 mM sugar component; 100-149 mM amino acid component; and 0.1-20 mM antioxidant.

D893. 120-220 mg/mL secukinumab; 200-250 mM sugar component; 20-99 mM amino acid component; and 10-129 mM tonicifier.

D894. 120-220 mg/mL secukinumab; 200-250 mM sugar component; 100-149 mM amino acid component; and 10-129 mM tonicifier.

D895. 120-220 mg/mL secukinumab; 200-250 mM sugar component; 20-99 mM amino acid component; and 10-129 mM ionic strength provider.

D896. 120-220 mg/mL secukinumab; 200-250 mM sugar component; 100-149 mM amino acid component; and 10-129 mM ionic strength provider.

D897. 120-220 mg/mL secukinumab; 200-250 mM sugar component; 20-99 mM amino acid component; and 0.001-2 mM chelator.

D898. 120-220 mg/mL secukinumab; 200-250 mM sugar component; 100-149 mM amino acid component; and 0.001-2 mM chelator.

D899. 120-220 mg/mL secukinumab; 200-250 mM sugar component; 0.01-1.2 mg/mL surfactant; and 0.1-20 mM antioxidant.

D900. 120-220 mg/mL secukinumab; 200-250 mM sugar component; 0.01-1.2 mg/mL surfactant; and 10-129 mM tonicifier.

D901. 120-220 mg/mL secukinumab; 200-250 mM sugar component; 0.01-1.2 mg/mL surfactant; and 10-129 mM ionic strength provider.

D902. 120-220 mg/mL secukinumab; 200-250 mM sugar component; 0.01-1.2 mg/mL surfactant; and 0.001-2 mM chelator.

D903. 120-220 mg/mL secukinumab; 200-250 mM sugar component; 0.1-20 mM antioxidant; and 10-129 mM tonicifier.

D904. 120-220 mg/mL secukinumab; 200-250 mM sugar component; 0.1-20 mM antioxidant; and 10-129 mM ionic strength provider.

D905. 120-220 mg/mL secukinumab; 200-250 mM sugar component; 0.1-20 mM antioxidant; and 0.001-2 mM chelator.

D906. 120-220 mg/mL secukinumab; 200-250 mM sugar component; 10-129 mM tonicifier; and 10-129 mM ionic strength provider.

D907. 120-220 mg/mL secukinumab; 200-250 mM sugar component; 10-129 mM tonicifier; and 0.001-2 mM chelator.

D908. 120-220 mg/mL secukinumab; 200-250 mM sugar component; 10-129 mM ionic strength provider; and 0.001-2 mM chelator.

(continued)

D909. 120-220 mg/mL secukinumab; 1-9 mM buffer system; 200-250 mM sugar component; 20-99 mM amino acid component; and 0.01-1.2 mg/mL surfactant.

D910. 120-220 mg/mL secukinumab; 51-60 mM buffer system; 200-250 mM sugar component; 20-99 mM amino acid component; and 0.01-1.2 mg/mL surfactant.

D911. 120-220 mg/mL secukinumab; 1-9 mM buffer system; 200-250 mM sugar component; 100-149 mM amino acid component; and 0.01-1.2 mg/mL surfactant.

D912. 120-220 mg/mL secukinumab; 51-60 mM buffer system; 200-250 mM sugar component; 100-149 mM amino acid component; and 0.01-1.2 mg/mL surfactant.

D913. 120-220 mg/mL secukinumab; 1-9 mM buffer system; 200-250 mM sugar component; 20-99 mM amino acid component; and 0.1-20 mM antioxidant.

D914. 120-220 mg/mL secukinumab; 51-60 mM buffer system; 200-250 mM sugar component; 20-99 mM amino acid component; and 0.1-20 mM antioxidant.

D915. 120-220 mg/mL secukinumab; 1-9 mM buffer system; 200-250 mM sugar component; 100-149 mM amino acid component; and 0.1-20 mM antioxidant.

D916. 120-220 mg/mL secukinumab; 51-60 mM buffer system; 200-250 mM sugar component; 100-149 mM amino acid component; and 0.1-20 mM antioxidant.

D917. 120-220 mg/mL secukinumab; 1-9 mM buffer system; 200-250 mM sugar component; 20-99 mM amino acid component; and 10-129 mM tonicifier.

D918. 120-220 mg/mL secukinumab; 51-60 mM buffer system; 200-250 mM sugar component; 20-99 mM amino acid component; and 10-129 mM tonicifier.

D919. 120-220 mg/mL secukinumab; 1-9 mM buffer system; 200-250 mM sugar component; 100-149 mM amino acid component; and 10-129 mM tonicifier.

D920. 120-220 mg/mL secukinumab; 51-60 mM buffer system; 200-250 mM sugar component; 100-149 mM amino acid component; and 10-129 mM tonicifier.

D921. 120-220 mg/mL secukinumab; 1-9 mM buffer system; 200-250 mM sugar component; 20-99 mM amino acid component; and 10-129 mM ionic strength provider.

D922. 120-220 mg/mL secukinumab; 51-60 mM buffer system; 200-250 mM sugar component; 20-99 mM amino acid component; and 10-129 mM ionic strength provider.

D923. 120-220 mg/mL secukinumab; 1-9 mM buffer system; 200-250 mM sugar component; 100-149 mM amino acid component; and 10-129 mM ionic strength provider.

D924. 120-220 mg/mL secukinumab; 51-60 mM buffer system; 200-250 mM sugar component; 100-149 mM amino acid component; and 10-129 mM ionic strength provider.

D925. 120-220 mg/mL secukinumab; 1-9 mM buffer system; 200-250 mM sugar component; 20-99 mM amino acid component; and 0.001-2 mM chelator.

D926. 120-220 mg/mL secukinumab; 51-60 mM buffer system; 200-250 mM sugar component; 20-99 mM amino acid component; and 0.001-2 mM chelator.

D927. 120-220 mg/mL secukinumab; 1-9 mM buffer system; 200-250 mM sugar component; 100-149 mM amino acid component; and 0.001-2 mM chelator.

D928. 120-220 mg/mL secukinumab; 51-60 mM buffer system; 200-250 mM sugar component; 100-149 mM amino acid component; and 0.001-2 mM chelator.

D929. 120-220 mg/mL secukinumab; 1-9 mM buffer system; 200-250 mM sugar component; 0.01-1.2 mg/mL surfactant; and 0.1-20 mM antioxidant.

D930. 120-220 mg/mL secukinumab; 51-60 mM buffer system; 200-250 mM sugar component; 0.01-1.2 mg/mL surfactant; and 0.1-20 mM antioxidant.

D931. 120-220 mg/mL secukinumab; 1-9 mM buffer system; 200-250 mM sugar component; 0.01-1.2 mg/mL surfactant; and 10-129 mM tonicifier.

D932. 120-220 mg/mL secukinumab; 51-60 mM buffer system; 200-250 mM sugar component; 0.01-1.2 mg/mL surfactant; and 10-129 mM tonicifier.

D933. 120-220 mg/mL secukinumab; 1-9 mM buffer system; 200-250 mM sugar component; 0.01-1.2 mg/mL surfactant; and 10-129 mM ionic strength provider.

D934. 120-220 mg/mL secukinumab; 51-60 mM buffer system; 200-250 mM sugar component; 0.01-1.2 mg/mL surfactant; and 10-129 mM ionic strength provider.

(continued)

D935. 120-220 mg/mL secukinumab; 1-9 mM buffer system; 200-250 mM sugar component; 0.01-1.2 mg/mL surfactant; and 0.001-2 mM chelator.

D936. 120-220 mg/mL secukinumab; 51-60 mM buffer system; 200-250 mM sugar component; 0.01-1.2 mg/mL surfactant; and 0.001-2 mM chelator.

D937. 120-220 mg/mL secukinumab; 1-9 mM buffer system; 200-250 mM sugar component; 0.1-20 mM antioxidant; and 10-129 mM tonicifier.

D938. 120-220 mg/mL secukinumab; 51-60 mM buffer system; 200-250 mM sugar component; 0.1-20 mM antioxidant; and 10-129 mM tonicifier.

D939. 120-220 mg/mL secukinumab; 1-9 mM buffer system; 200-250 mM sugar component; 0.1-20 mM antioxidant; and 10-129 mM ionic strength provider.

D940. 120-220 mg/mL secukinumab; 51-60 mM buffer system; 200-250 mM sugar component; 0.1-20 mM antioxidant; and 10-129 mM ionic strength provider.

D941. 120-220 mg/mL secukinumab; 1-9 mM buffer system; 200-250 mM sugar component; 0.1-20 mM antioxidant; and 0.001-2 mM chelator.

D942. 120-220 mg/mL secukinumab; 51-60 mM buffer system; 200-250 mM sugar component; 0.1-20 mM antioxidant; and 0.001-2 mM chelator.

D943. 120-220 mg/mL secukinumab; 1-9 mM buffer system; 200-250 mM sugar component; 10-129 mM tonicifier; and 10-129 mM ionic strength provider.

D944. 120-220 mg/mL secukinumab; 51-60 mM buffer system; 200-250 mM sugar component; 10-129 mM tonicifier; and 10-129 mM ionic strength provider.

D945. 120-220 mg/mL secukinumab; 1-9 mM buffer system; 200-250 mM sugar component; 10-129 mM tonicifier; and 0.001-2 mM chelator.

D946. 120-220 mg/mL secukinumab; 51-60 mM buffer system; 200-250 mM sugar component; 10-129 mM tonicifier; and 0.001 - 2 mM chelator.

D947. 120-220 mg/mL secukinumab; 1-9 mM buffer system; 200-250 mM sugar component; 10-129 mM ionic strength provider; and 0.001-2 mM chelator.

D948. 120-220 mg/mL secukinumab; 51-60 mM buffer system; 200-250 mM sugar component; 10-129 mM ionic strength provider; and 0.001-2 mM chelator.

D949. 120-220 mg/mL secukinumab; 200-250 mM sugar component; 20-99 mM amino acid component; 0.01-1.2 mg/mL surfactant; and 0.1-20 mM antioxidant.

D950. 120-220 mg/mL secukinumab; 200-250 mM sugar component; 100-149 mM amino acid component; 0.01-1.2 mg/mL surfactant; and 0.1-20 mM antioxidant.

D951. 120-220 mg/mL secukinumab; 200-250 mM sugar component; 20-99 mM amino acid component; 0.01-1.2 mg/mL surfactant; and 10-129 mM tonicifier.

D952. 120-220 mg/mL secukinumab; 200-250 mM sugar component; 100-149 mM amino acid component; 0.01-1.2 mg/mL surfactant; and 10-129 mM tonicifier.

D953. 120-220 mg/mL secukinumab; 200-250 mM sugar component; 20-99 mM amino acid component; 0.01-1.2 mg/mL surfactant; and 10-129 mM ionic strength provider.

D954. 120-220 mg/mL secukinumab; 200-250 mM sugar component; 100-149 mM amino acid component; 0.01-1.2 mg/mL surfactant; and 10-129 mM ionic strength provider.

D955. 120-220 mg/mL secukinumab; 200-250 mM sugar component; 20-99 mM amino acid component; 0.01-1.2 mg/mL surfactant; and 0.001-2 mM chelator.

D956. 120-220 mg/mL secukinumab; 200-250 mM sugar component; 100-149 mM amino acid component; 0.01-1.2 mg/mL surfactant; and 0.001-2 mM chelator.

D957. 120-220 mg/mL secukinumab; 200-250 mM sugar component; 20-99 mM amino acid component; 0.1-20 mM antioxidant; and 10-129 mM tonicifier.

D958. 120-220 mg/mL secukinumab; 200-250 mM sugar component; 100-149 mM amino acid component; 0.1-20 mM antioxidant; and 10-129 mM tonicifier.

D959. 120-220 mg/mL secukinumab; 200-250 mM sugar component; 20-99 mM amino acid component; 0.1-20 mM antioxidant; and 10-129 mM ionic strength provider.

D960. 120-220 mg/mL secukinumab; 200-250 mM sugar component; 100-149 mM amino acid component; 0.1-20 mM antioxidant; and 10-129 mM ionic strength provider.

(continued)

D961. 120-220 mg/mL secukinumab; 200-250 mM sugar component; 20-99 mM amino acid component; 0.1-20 mM antioxidant; and 0.001-2 mM chelator.

D962. 120-220 mg/mL secukinumab; 200-250 mM sugar component; 100-149 mM amino acid component; 0.1-20 mM antioxidant; and 0.001-2 mM chelator.

D963. 120-220 mg/mL secukinumab; 200-250 mM sugar component; 20-99 mM amino acid component; 10-129 mM tonicifier; and 10-129 mM ionic strength provider.

D964. 120-220 mg/mL secukinumab; 200-250 mM sugar component; 100-149 mM amino acid component; 10-129 mM tonicifier; and 10-129 mM ionic strength provider.

D965. 120-220 mg/mL secukinumab; 200-250 mM sugar component; 20-99 mM amino acid component; 10-129 mM tonicifier; and 0.001-2 mM chelator.

D966. 120-220 mg/mL secukinumab; 200-250 mM sugar component; 100-149 mM amino acid component; 10-129 mM tonicifier; and 0.001-2 mM chelator.

D967. 120-220 mg/mL secukinumab; 200-250 mM sugar component; 20-99 mM amino acid component; 10-129 mM ionic strength provider; and 0.001-2 mM chelator.

D968. 120-220 mg/mL secukinumab; 200-250 mM sugar component; 100-149 mM amino acid component; 10-129 mM ionic strength provider; and 0.001-2 mM chelator.

D969. 120-220 mg/mL secukinumab; 200-250 mM sugar component; 0.01-1.2 mg/mL surfactant; 0.1-20 mM antioxidant; and 10-129 mM tonicifier.

D970. 120-220 mg/mL secukinumab; 200-250 mM sugar component; 0.01-1.2 mg/mL surfactant; 0.1-20 mM antioxidant; and 10-129 mM ionic strength provider.

D971. 120-220 mg/mL secukinumab; 200-250 mM sugar component; 0.01-1.2 mg/mL surfactant; 0.1-20 mM antioxidant; and 0.001-2 mM chelator.

D972. 120-220 mg/mL secukinumab; 200-250 mM sugar component; 0.01-1.2 mg/mL surfactant; 10-129 mM tonicifier; and 10-129 mM ionic strength provider.

D973. 120-220 mg/mL secukinumab; 200-250 mM sugar component; 0.01-1.2 mg/mL surfactant; 10-129 mM tonicifier; and 0.001-2 mM chelator.

D974. 120-220 mg/mL secukinumab; 200-250 mM sugar component; 0.01-1.2 mg/mL surfactant; 10-129 mM ionic strength provider; and 0.001-2 mM chelator.

D975. 120-220 mg/mL secukinumab; 200-250 mM sugar component; 0.1-20 mM antioxidant; 10-129 mM tonicifier; and 10-129 mM ionic strength provider.

D976. 120-220 mg/mL secukinumab; 200-250 mM sugar component; 0.1-20 mM antioxidant; 10-129 mM tonicifier; and 0.001-2 mM chelator.

D977. 120-220 mg/mL secukinumab; 200-250 mM sugar component; 0.1-20 mM antioxidant; 10-129 mM ionic strength provider; and 0.001-2 mM chelator.

D978. 120-220 mg/mL secukinumab; 200-250 mM sugar component; 10-129 mM tonicifier; 10-129 mM ionic strength provider; and 0.001-2 mM chelator.

D979. 120-220 mg/mL secukinumab; 1-9 mM buffer system; 200-250 mM sugar component; 20-99 mM amino acid component; 0.01-1.2 mg/mL surfactant; and 0.1-20 mM antioxidant.

D980. 120-220 mg/mL secukinumab; 51-60 mM buffer system; 200-250 mM sugar component; 20-99 mM amino acid component; 0.01-1.2 mg/mL surfactant; and 0.1-20 mM antioxidant.

D981. 120-220 mg/mL secukinumab; 1-9 mM buffer system; 200-250 mM sugar component; 100-149 mM amino acid component; 0.01-1.2 mg/mL surfactant; and 0.1-20 mM antioxidant.

D982. 120-220 mg/mL secukinumab; 51-60 mM buffer system; 200-250 mM sugar component; 100-149 mM amino acid component; 0.01-1.2 mg/mL surfactant; and 0.1-20 mM antioxidant.

D983. 120-220 mg/mL secukinumab; 1-9 mM buffer system; 200-250 mM sugar component; 20-99 mM amino acid component; 0.01-1.2 mg/mL surfactant; and 10-129 mM tonicifier.

D984. 120-220 mg/mL secukinumab; 51-60 mM buffer system; 200-250 mM sugar component; 20-99 mM amino acid component; 0.01-1.2 mg/mL surfactant; and 10-129 mM tonicifier.

D985. 120-220 mg/mL secukinumab; 1-9 mM buffer system; 200-250 mM sugar component; 100-149 mM amino acid component; 0.01-1.2 mg/mL surfactant; and 10-129 mM tonicifier.

D986. 120-220 mg/mL secukinumab; 51-60 mM buffer system; 200-250 mM sugar component; 100-149 mM amino acid component; 0.01-1.2 mg/mL surfactant; and 10-129 mM tonicifier.

(continued)

D987. 120-220 mg/mL secukinumab; 1-9 mM buffer system; 200-250 mM sugar component; 20-99 mM amino acid component; 0.01-1.2 mg/mL surfactant; and 10-129 mM ionic strength provider.

D988. 120-220 mg/mL secukinumab; 51-60 mM buffer system; 200-250 mM sugar component; 20-99 mM amino acid component; 0.01-1.2 mg/mL surfactant; and 10-129 mM ionic strength provider.

D989. 120-220 mg/mL secukinumab; 1-9 mM buffer system; 200-250 mM sugar component; 100-149 mM amino acid component; 0.01-1.2 mg/mL surfactant; and 10-129 mM ionic strength provider.

D990. 120-220 mg/mL secukinumab; 51-60 mM buffer system; 200-250 mM sugar component; 100-149 mM amino acid component; 0.01-1.2 mg/mL surfactant; and 10-129 mM ionic strength provider.

D991. 120-220 mg/mL secukinumab; 1-9 mM buffer system; 200-250 mM sugar component; 20-99 mM amino acid component; 0.01-1.2 mg/mL surfactant; and 0.001-2 mM chelator.

D992. 120-220 mg/mL secukinumab; 51-60 mM buffer system; 200-250 mM sugar component; 20-99 mM amino acid component; 0.01-1.2 mg/mL surfactant; and 0.001-2 mM chelator.

D993. 120-220 mg/mL secukinumab; 1-9 mM buffer system; 200-250 mM sugar component; 100-149 mM amino acid component; 0.01-1.2 mg/mL surfactant; and 0.001-2 mM chelator.

D994. 120-220 mg/mL secukinumab; 51-60 mM buffer system; 200-250 mM sugar component; 100-149 mM amino acid component; 0.01-1.2 mg/mL surfactant; and 0.001-2 mM chelator.

D995. 120-220 mg/mL secukinumab; 1-9 mM buffer system; 200-250 mM sugar component; 20-99 mM amino acid component; 0.1-20 mM antioxidant; and 10-129 mM tonicifier.

D996. 120-220 mg/mL secukinumab; 51-60 mM buffer system; 200-250 mM sugar component; 20-99 mM amino acid component; 0.1-20 mM antioxidant; and 10-129 mM tonicifier.

D997. 120-220 mg/mL secukinumab; 1-9 mM buffer system; 200-250 mM sugar component; 100-149 mM amino acid component; 0.1-20 mM antioxidant; and 10-129 mM tonicifier.

D998. 120-220 mg/mL secukinumab; 51-60 mM buffer system; 200-250 mM sugar component; 100-149 mM amino acid component; 0.1-20 mM antioxidant; and 10-129 mM tonicifier.

D999. 120-220 mg/mL secukinumab; 1-9 mM buffer system; 200-250 mM sugar component; 20-99 mM amino acid component; 0.1-20 mM antioxidant; and 10-129 mM ionic strength provider.

D1000 . 120-220 mg/mL secukinumab; 51-60 mM buffer system; 200-250 mM sugar component; 20-99 mM amino acid component; 0.1-20 mM antioxidant; and 10-129 mM ionic strength provider.

D1001 . 120-220 mg/mL secukinumab; 1-9 mM buffer system; 200-250 mM sugar component; 100-149 mM amino acid component; 0.1-20 mM antioxidant; and 10-129 mM ionic strength provider.

D1002 . 120-220 mg/mL secukinumab; 51-60 mM buffer system; 200-250 mM sugar component; 100-149 mM amino acid component; 0.1-20 mM antioxidant; and 10-129 mM ionic strength provider.

D1003 . 120-220 mg/mL secukinumab; 1-9 mM buffer system; 200-250 mM sugar component; 20-99 mM amino acid component; 0.1-20 mM antioxidant; and 0.001-2 mM chelator.

D1004 . 120-220 mg/mL secukinumab; 51-60 mM buffer system; 200-250 mM sugar component; 20-99 mM amino acid component; 0.1-20 mM antioxidant; and 0.001-2 mM chelator.

D1005 . 120-220 mg/mL secukinumab; 1-9 mM buffer system; 200-250 mM sugar component; 100-149 mM amino acid component; 0.1-20 mM antioxidant; and 0.001-2 mM chelator.

D1006 . 120-220 mg/mL secukinumab; 51-60 mM buffer system; 200-250 mM sugar component; 100-149 mM amino acid component; 0.1-20 mM antioxidant; and 0.001-2 mM chelator.

D1007 . 120-220 mg/mL secukinumab; 1-9 mM buffer system; 200-250 mM sugar component; 20-99 mM amino acid component; 10-129 mM tonicifier; and 10-129 mM ionic strength provider.

D1008 . 120-220 mg/mL secukinumab; 51-60 mM buffer system; 200-250 mM sugar component; 20-99 mM amino acid component; 10-129 mM tonicifier; and 10-129 mM ionic strength provider.

D1009 . 120-220 mg/mL secukinumab; 1-9 mM buffer system; 200-250 mM sugar component; 100-149 mM amino acid component; 10-129 mM tonicifier; and 10-129 mM ionic strength provider.

D1010 . 120-220 mg/mL secukinumab; 51-60 mM buffer system; 200-250 mM sugar component; 100-149 mM amino acid component; 10-129 mM tonicifier; and 10-129 mM ionic strength provider.

D1011 . 120-220 mg/mL secukinumab; 1-9 mM buffer system; 200-250 mM sugar component; 20-99 mM amino acid component; 10-129 mM tonicifier; and 0.001-2 mM chelator.

D1012 . 120-220 mg/mL secukinumab; 51-60 mM buffer system; 200-250 mM sugar component; 20-99 mM amino acid component; 10-129 mM tonicifier; and 0.001-2 mM chelator.

(continued)

D1013 . 120-220 mg/mL secukinumab; 1-9 mM buffer system; 200-250 mM sugar component; 100-149 mM amino acid component; 10-129 mM tonicifier; and 0.001-2 mM chelator.

D1014 . 120-220 mg/mL secukinumab; 51-60 mM buffer system; 200-250 mM sugar component; 100-149 mM amino acid component; 10-129 mM tonicifier; and 0.001-2 mM chelator.

D1015 . 120-220 mg/mL secukinumab; 1-9 mM buffer system; 200-250 mM sugar component; 20-99 mM amino acid component; 10-129 mM ionic strength provider; and 0.001-2 mM chelator.

D1016 . 120-220 mg/mL secukinumab; 51-60 mM buffer system; 200-250 mM sugar component; 20-99 mM amino acid component; 10-129 mM ionic strength provider; and 0.001-2 mM chelator.

D1017 . 120-220 mg/mL secukinumab; 1-9 mM buffer system; 200-250 mM sugar component; 100-149 mM amino acid component; 10-129 mM ionic strength provider; and 0.001-2 mM chelator.

D1018 . 120-220 mg/mL secukinumab; 51-60 mM buffer system; 200-250 mM sugar component; 100-149 mM amino acid component; 10-129 mM ionic strength provider; and 0.001-2 mM chelator.

D1019 . 120-220 mg/mL secukinumab; 1-9 mM buffer system; 200-250 mM sugar component; 0.01-1.2 mg/mL surfactant; 0.1-20 mM antioxidant; and 10-129 mM tonicifier.

D1020 . 120-220 mg/mL secukinumab; 51-60 mM buffer system; 200-250 mM sugar component; 0.01-1.2 mg/mL surfactant; 0.1-20 mM antioxidant; and 10-129 mM tonicifier.

D1021 . 120-220 mg/mL secukinumab; 1-9 mM buffer system; 200-250 mM sugar component; 0.01-1.2 mg/mL surfactant; 0.1-20 mM antioxidant; and 10-129 mM ionic strength provider.

D1022 . 120-220 mg/mL secukinumab; 51-60 mM buffer system; 200-250 mM sugar component; 0.01-1.2 mg/mL surfactant; 0.1-20 mM antioxidant; and 10-129 mM ionic strength provider.

D1023 . 120-220 mg/mL secukinumab; 1-9 mM buffer system; 200-250 mM sugar component; 0.01-1.2 mg/mL surfactant; 0.1-20 mM antioxidant; and 0.001-2 mM chelator.

D1024 . 120-220 mg/mL secukinumab; 51-60 mM buffer system; 200-250 mM sugar component; 0.01-1.2 mg/mL surfactant; 0.1-20 mM antioxidant; and 0.001-2 mM chelator.

D1025 . 120-220 mg/mL secukinumab; 1-9 mM buffer system; 200-250 mM sugar component; 0.01-1.2 mg/mL surfactant; 10-129 mM tonicifier; and 10-129 mM ionic strength provider.

D1026 . 120-220 mg/mL secukinumab; 51-60 mM buffer system; 200-250 mM sugar component; 0.01-1.2 mg/mL surfactant; 10-129 mM tonicifier; and 10-129 mM ionic strength provider.

D1027 . 120-220 mg/mL secukinumab; 1-9 mM buffer system; 200-250 mM sugar component; 0.01-1.2 mg/mL surfactant; 10-129 mM tonicifier; and 0.001-2 mM chelator.

D1028 . 120-220 mg/mL secukinumab; 51-60 mM buffer system; 200-250 mM sugar component; 0.01-1.2 mg/mL surfactant; 10-129 mM tonicifier; and 0.001-2 mM chelator.

D1029 . 120-220 mg/mL secukinumab; 1-9 mM buffer system; 200-250 mM sugar component; 0.01-1.2 mg/mL surfactant; 10-129 mM ionic strength provider; and 0.001-2 mM chelator.

D1030 . 120-220 mg/mL secukinumab; 51-60 mM buffer system; 200-250 mM sugar component; 0.01-1.2 mg/mL surfactant; 10-129 mM ionic strength provider; and 0.001-2 mM chelator.

D1031 . 120-220 mg/mL secukinumab; 1-9 mM buffer system; 200-250 mM sugar component; 0.1-20 mM antioxidant; 10-129 mM tonicifier; and 10-129 mM ionic strength provider.

D1032 . 120-220 mg/mL secukinumab; 51-60 mM buffer system; 200-250 mM sugar component; 0.1-20 mM antioxidant; 10-129 mM tonicifier; and 10-129 mM ionic strength provider.

D1033 . 120-220 mg/mL secukinumab; 1-9 mM buffer system; 200-250 mM sugar component; 0.1-20 mM antioxidant; 10-129 mM tonicifier; and 0.001-2 mM chelator.

D1034 . 120-220 mg/mL secukinumab; 51-60 mM buffer system; 200-250 mM sugar component; 0.1-20 mM antioxidant; 10-129 mM tonicifier; and 0.001-2 mM chelator.

D1035 . 120-220 mg/mL secukinumab; 1-9 mM buffer system; 200-250 mM sugar component; 0.1-20 mM antioxidant; 10-129 mM ionic strength provider; and 0.001-2 mM chelator.

D1036 . 120-220 mg/mL secukinumab; 51-60 mM buffer system; 200-250 mM sugar component; 0.1-20 mM antioxidant; 10-129 mM ionic strength provider; and 0.001-2 mM chelator.

D1037 . 120-220 mg/mL secukinumab; 1-9 mM buffer system; 200-250 mM sugar component; 10-129 mM tonicifier; 10-129 mM ionic strength provider; and 0.001-2 mM chelator.

D1038 . 120-220 mg/mL secukinumab; 51-60 mM buffer system; 200-250 mM sugar component; 10-129 mM tonicifier; 10-129 mM ionic strength provider; and 0.001-2 mM chelator.

(continued)

D1039. 120-220 mg/mL secukinumab; 200-250 mM sugar component; 20-99 mM amino acid component; 0.01-1.2 mg/mL surfactant; 0.1-20 mM antioxidant; and 10-129 mM tonicifier.

D1040. 120-220 mg/mL secukinumab; 200-250 mM sugar component; 100-149 mM amino acid component; 0.01-1.2 mg/mL surfactant; 0.1-20 mM antioxidant; and 10-129 mM tonicifier.

D1041. 120-220 mg/mL secukinumab; 200-250 mM sugar component; 20-99 mM amino acid component; 0.01-1.2 mg/mL surfactant; 0.1-20 mM antioxidant; and 10-129 mM ionic strength provider.

D1042. 120-220 mg/mL secukinumab; 200-250 mM sugar component; 100-149 mM amino acid component; 0.01-1.2 mg/mL surfactant; 0.1-20 mM antioxidant; and 10-129 mM ionic strength provider.

D1043. 120-220 mg/mL secukinumab; 200-250 mM sugar component; 20-99 mM amino acid component; 0.01-1.2 mg/mL surfactant; 0.1-20 mM antioxidant; and 0.001-2 mM chelator.

D1044. 120-220 mg/mL secukinumab; 200-250 mM sugar component; 100-149 mM amino acid component; 0.01-1.2 mg/mL surfactant; 0.1-20 mM antioxidant; and 0.001-2 mM chelator.

D1045. 120-220 mg/mL secukinumab; 200-250 mM sugar component; 20-99 mM amino acid component; 0.01-1.2 mg/mL surfactant; 10-129 mM tonicifier; and 10-129 mM ionic strength provider.

D1046. 120-220 mg/mL secukinumab; 200-250 mM sugar component; 100-149 mM amino acid component; 0.01-1.2 mg/mL surfactant; 10-129 mM tonicifier; and 10-129 mM ionic strength provider.

D1047. 120-220 mg/mL secukinumab; 200-250 mM sugar component; 20-99 mM amino acid component; 0.01-1.2 mg/mL surfactant; 10-129 mM tonicifier; and 0.001-2 mM chelator.

D1048. 120-220 mg/mL secukinumab; 200-250 mM sugar component; 100-149 mM amino acid component; 0.01-1.2 mg/mL surfactant; 10-129 mM tonicifier; and 0.001-2 mM chelator.

D1049. 120-220 mg/mL secukinumab; 200-250 mM sugar component; 20-99 mM amino acid component; 0.01-1.2 mg/mL surfactant; 10-129 mM ionic strength provider; and 0.001-2 mM chelator.

D1050. 120-220 mg/mL secukinumab; 200-250 mM sugar component; 100-149 mM amino acid component; 0.01-1.2 mg/mL surfactant; 10-129 mM ionic strength provider; and 0.001-2 mM chelator.

D1051 . 120-220 mg/mL secukinumab; 200-250 mM sugar component; 20-99 mM amino acid component; 0.1-20 mM antioxidant; 10-129 mM tonicifier; and 10-129 mM ionic strength provider.

D1052 . 120-220 mg/mL secukinumab; 200-250 mM sugar component; 100-149 mM amino acid component; 0.1-20 mM antioxidant; 10-129 mM tonicifier; and 10-129 mM ionic strength provider.

D1053 . 120-220 mg/mL secukinumab; 200-250 mM sugar component; 20-99 mM amino acid component; 0.1-20 mM antioxidant; 10-129 mM tonicifier; and 0.001-2 mM chelator.

D1054 . 120-220 mg/mL secukinumab; 200-250 mM sugar component; 100-149 mM amino acid component; 0.1-20 mM antioxidant; 10-129 mM tonicifier; and 0.001-2 mM chelator.

D1055 . 120-220 mg/mL secukinumab; 200-250 mM sugar component; 20-99 mM amino acid component; 0.1-20 mM antioxidant; 10-129 mM ionic strength provider; and 0.001-2 mM chelator.

D1056 . 120-220 mg/mL secukinumab; 200-250 mM sugar component; 100-149 mM amino acid component; 0.1-20 mM antioxidant; 10-129 mM ionic strength provider; and 0.001-2 mM chelator.

D1057 . 120-220 mg/mL secukinumab; 200-250 mM sugar component; 20-99 mM amino acid component; 10-129 mM tonicifier; 10-129 mM ionic strength provider; and 0.001-2 mM chelator.

D1058 . 120-220 mg/mL secukinumab; 200-250 mM sugar component; 100-149 mM amino acid component; 10-129 mM tonicifier; 10-129 mM ionic strength provider; and 0.001-2 mM chelator.

D1059 . 120-220 mg/mL secukinumab; 200-250 mM sugar component; 0.01-1.2 mg/mL surfactant; 0.1-20 mM antioxidant; 10-129 mM tonicifier; and 10-129 mM ionic strength provider.

D1060 . 120-220 mg/mL secukinumab; 200-250 mM sugar component; 0.01-1.2 mg/mL surfactant; 0.1-20 mM antioxidant; 10-129 mM tonicifier; and 0.001-2 mM chelator.

D1061 . 120-220 mg/mL secukinumab; 200-250 mM sugar component; 0.01-1.2 mg/mL surfactant; 0.1-20 mM antioxidant; 10-129 mM ionic strength provider; and 0.001-2 mM chelator.

D1062 . 120-220 mg/mL secukinumab; 200-250 mM sugar component; 0.01-1.2 mg/mL surfactant; 10-129 mM tonicifier; 10-129 mM ionic strength provider; and 0.001-2 mM chelator.

D1063 . 120-220 mg/mL secukinumab; 200-250 mM sugar component; 0.1-20 mM antioxidant; 10-129 mM tonicifier; 10-129 mM ionic strength provider; and 0.001-2 mM chelator.

D1064 . 120-220 mg/mL secukinumab; 1-9 mM buffer system; 200-250 mM sugar component; 20-99 mM amino acid component; 0.01-1.2 mg/mL surfactant; 0.1-20 mM antioxidant; and 10-129 mM tonicifier.

(continued)

D1065 . 120-220 mg/mL secukinumab; 51-60 mM buffer system; 200-250 mM sugar component; 20-99 mM amino acid component; 0.01-1.2 mg/mL surfactant; 0.1-20 mM antioxidant; and 10-129 mM tonicifier.

D1066 . 120-220 mg/mL secukinumab; 1-9 mM buffer system; 200-250 mM sugar component; 100-149 mM amino acid component; 0.01-1.2 mg/mL surfactant; 0.1-20 mM antioxidant; and 10-129 mM tonicifier.

D1067 . 120-220 mg/mL secukinumab; 51-60 mM buffer system; 200-250 mM sugar component; 100-149 mM amino acid component; 0.01-1.2 mg/mL surfactant; 0.1-20 mM antioxidant; and 10-129 mM tonicifier.

D1068 . 120-220 mg/mL secukinumab; 1-9 mM buffer system; 200-250 mM sugar component; 20-99 mM amino acid component; 0.01-1.2 mg/mL surfactant; 0.1-20 mM antioxidant; and 10-129 mM ionic strength provider.

D1069 . 120-220 mg/mL secukinumab; 51-60 mM buffer system; 200-250 mM sugar component; 20-99 mM amino acid component; 0.01-1.2 mg/mL surfactant; 0.1-20 mM antioxidant; and 10-129 mM ionic strength provider.

D1070 . 120-220 mg/mL secukinumab; 1-9 mM buffer system; 200-250 mM sugar component; 100-149 mM amino acid component; 0.01-1.2 mg/mL surfactant; 0.1-20 mM antioxidant; and 10-129 mM ionic strength provider.

D1071 . 120-220 mg/mL secukinumab; 51-60 mM buffer system; 200-250 mM sugar component; 100-149 mM amino acid component; 0.01-1.2 mg/mL surfactant; 0.1-20 mM antioxidant; and 10-129 mM ionic strength provider.

D1072 . 120-220 mg/mL secukinumab; 1-9 mM buffer system; 200-250 mM sugar component; 20-99 mM amino acid component; 0.01-1.2 mg/mL surfactant; 0.1-20 mM antioxidant; and 0.001-2 mM chelator.

D1073 . 120-220 mg/mL secukinumab; 51-60 mM buffer system; 200-250 mM sugar component; 20-99 mM amino acid component; 0.01-1.2 mg/mL surfactant; 0.1-20 mM antioxidant; and 0.001-2 mM chelator.

D1074 . 120-220 mg/mL secukinumab; 1-9 mM buffer system; 200-250 mM sugar component; 100-149 mM amino acid component; 0.01-1.2 mg/mL surfactant; 0.1-20 mM antioxidant; and 0.001-2 mM chelator.

D1075 . 120-220 mg/mL secukinumab; 51-60 mM buffer system; 200-250 mM sugar component; 100-149 mM amino acid component; 0.01-1.2 mg/mL surfactant; 0.1-20 mM antioxidant; and 0.001-2 mM chelator.

D1076 . 120-220 mg/mL secukinumab; 1-9 mM buffer system; 200-250 mM sugar component; 20-99 mM amino acid component; 0.01-1.2 mg/mL surfactant; 10-129 mM tonicifier; and 10-129 mM ionic strength provider.

D1077 . 120-220 mg/mL secukinumab; 51-60 mM buffer system; 200-250 mM sugar component; 20-99 mM amino acid component; 0.01-1.2 mg/mL surfactant; 10-129 mM tonicifier; and 10-129 mM ionic strength provider.

D1078 . 120-220 mg/mL secukinumab; 1-9 mM buffer system; 200-250 mM sugar component; 100-149 mM amino acid component; 0.01-1.2 mg/mL surfactant; 10-129 mM tonicifier; and 10-129 mM ionic strength provider.

D1079 . 120-220 mg/mL secukinumab; 51-60 mM buffer system; 200-250 mM sugar component; 100-149 mM amino acid component; 0.01-1.2 mg/mL surfactant; 10-129 mM tonicifier; and 10-129 mM ionic strength provider.

D1080 . 120-220 mg/mL secukinumab; 1-9 mM buffer system; 200-250 mM sugar component; 20-99 mM amino acid component; 0.01-1.2 mg/mL surfactant; 10-129 mM tonicifier; and 0.001-2 mM chelator.

D1081 . 120-220 mg/mL secukinumab; 51-60 mM buffer system; 200-250 mM sugar component; 20-99 mM amino acid component; 0.01-1.2 mg/mL surfactant; 10-129 mM tonicifier; and 0.001-2 mM chelator.

D1082 . 120-220 mg/mL secukinumab; 1-9 mM buffer system; 200-250 mM sugar component; 100-149 mM amino acid component; 0.01-1.2 mg/mL surfactant; 10-129 mM tonicifier; and 0.001-2 mM chelator.

D1083 . 120-220 mg/mL secukinumab; 51-60 mM buffer system; 200-250 mM sugar component; 100-149 mM amino acid component; 0.01-1.2 mg/mL surfactant; 10-129 mM tonicifier; and 0.001-2 mM chelator.

D1084 . 120-220 mg/mL secukinumab; 1-9 mM buffer system; 200-250 mM sugar component; 20-99 mM amino acid component; 0.01-1.2 mg/mL surfactant; 10-129 mM ionic strength provider; and 0.001-2 mM chelator.

D1085 . 120-220 mg/mL secukinumab; 51-60 mM buffer system; 200-250 mM sugar component; 20-99 mM amino acid component; 0.01-1.2 mg/mL surfactant; 10-129 mM ionic strength provider; and 0.001-2 mM chelator.

D1086 . 120-220 mg/mL secukinumab; 1-9 mM buffer system; 200-250 mM sugar component; 100-149 mM amino acid component; 0.01-1.2 mg/mL surfactant; 10-129 mM ionic strength provider; and 0.001-2 mM chelator.

D1087 . 120-220 mg/mL secukinumab; 51-60 mM buffer system; 200-250 mM sugar component; 100-149 mM amino acid component; 0.01-1.2 mg/mL surfactant; 10-129 mM ionic strength provider; and 0.001-2 mM chelator.

(continued)

D1088 . 120-220 mg/mL secukinumab; 1-9 mM buffer system; 200-250 mM sugar component; 20-99 mM amino acid component; 0.1-20 mM antioxidant; 10-129 mM tonicifier; and 10-129 mM ionic strength provider.

D1089 . 120-220 mg/mL secukinumab; 51-60 mM buffer system; 200-250 mM sugar component; 20-99 mM amino acid component; 0.1-20 mM antioxidant; 10-129 mM tonicifier; and 10-129 mM ionic strength provider.

D1090 . 120-220 mg/mL secukinumab; 1-9 mM buffer system; 200-250 mM sugar component; 100-149 mM amino acid component; 0.1-20 mM antioxidant; 10-129 mM tonicifier; and 10-129 mM ionic strength provider.

D1091 . 120-220 mg/mL secukinumab; 51-60 mM buffer system; 200-250 mM sugar component; 100-149 mM amino acid component; 0.1-20 mM antioxidant; 10-129 mM tonicifier; and 10-129 mM ionic strength provider.

D1092 . 120-220 mg/mL secukinumab; 1-9 mM buffer system; 200-250 mM sugar component; 20-99 mM amino acid component; 0.1-20 mM antioxidant; 10-129 mM tonicifier; and 0.001-2 mM chelator.

D1093 . 120-220 mg/mL secukinumab; 51-60 mM buffer system; 200-250 mM sugar component; 20-99 mM amino acid component; 0.1-20 mM antioxidant; 10-129 mM tonicifier; and 0.001-2 mM chelator.

D1094 . 120-220 mg/mL secukinumab; 1-9 mM buffer system; 200-250 mM sugar component; 100-149 mM amino acid component; 0.1-20 mM antioxidant; 10-129 mM tonicifier; and 0.001-2 mM chelator.

D1095 . 120-220 mg/mL secukinumab; 51-60 mM buffer system; 200-250 mM sugar component; 100-149 mM amino acid component; 0.1-20 mM antioxidant; 10-129 mM tonicifier; and 0.001-2 mM chelator.

D1096 . 120-220 mg/mL secukinumab; 1-9 mM buffer system; 200-250 mM sugar component; 20-99 mM amino acid component; 0.1-20 mM antioxidant; 10-129 mM ionic strength provider; and 0.001-2 mM chelator.

D1097 . 120-220 mg/mL secukinumab; 51-60 mM buffer system; 200-250 mM sugar component; 20-99 mM amino acid component; 0.1-20 mM antioxidant; 10-129 mM ionic strength provider; and 0.001-2 mM chelator.

D1098 . 120-220 mg/mL secukinumab; 1-9 mM buffer system; 200-250 mM sugar component; 100-149 mM amino acid component; 0.1-20 mM antioxidant; 10-129 mM ionic strength provider; and 0.001-2 mM chelator.

D1099 . 120-220 mg/mL secukinumab; 51-60 mM buffer system; 200-250 mM sugar component; 100-149 mM amino acid component; 0.1-20 mM antioxidant; 10-129 mM ionic strength provider; and 0.001-2 mM chelator.

D1100 . 120-220 mg/mL secukinumab; 1-9 mM buffer system; 200-250 mM sugar component; 20-99 mM amino acid component; 10-129 mM tonicifier; 10-129 mM ionic strength provider; and 0.001-2 mM chelator.

D1101 . 120-220 mg/mL secukinumab; 51-60 mM buffer system; 200-250 mM sugar component; 20-99 mM amino acid component; 10-129 mM tonicifier; 10-129 mM ionic strength provider; and 0.001-2 mM chelator.

D1102 . 120-220 mg/mL secukinumab; 1-9 mM buffer system; 200-250 mM sugar component; 100-149 mM amino acid component; 10-129 mM tonicifier; 10-129 mM ionic strength provider; and 0.001-2 mM chelator.

D1103 . 120-220 mg/mL secukinumab; 51-60 mM buffer system; 200-250 mM sugar component; 100-149 mM amino acid component; 10-129 mM tonicifier; 10-129 mM ionic strength provider; and 0.001-2 mM chelator.

D1104 . 120-220 mg/mL secukinumab; 1-9 mM buffer system; 200-250 mM sugar component; 0.01-1.2 mg/mL surfactant; 0.1-20 mM antioxidant; 10-129 mM tonicifier; and 10-129 mM ionic strength provider.

D1105 . 120-220 mg/mL secukinumab; 51-60 mM buffer system; 200-250 mM sugar component; 0.01-1.2 mg/mL surfactant; 0.1-20 mM antioxidant; 10-129 mM tonicifier; and 10-129 mM ionic strength provider.

D1106 . 120-220 mg/mL secukinumab; 1-9 mM buffer system; 200-250 mM sugar component; 0.01-1.2 mg/mL surfactant; 0.1-20 mM antioxidant; 10-129 mM tonicifier; and 0.001-2 mM chelator.

D1107 . 120-220 mg/mL secukinumab; 51-60 mM buffer system; 200-250 mM sugar component; 0.01-1.2 mg/mL surfactant; 0.1-20 mM antioxidant; 10-129 mM tonicifier; and 0.001-2 mM chelator.

D1108 . 120-220 mg/mL secukinumab; 1-9 mM buffer system; 200-250 mM sugar component; 0.01-1.2 mg/mL surfactant; 0.1-20 mM antioxidant; 10-129 mM ionic strength provider; and 0.001-2 mM chelator.

D1109 . 120-220 mg/mL secukinumab; 51-60 mM buffer system; 200-250 mM sugar component; 0.01-1.2 mg/mL surfactant; 0.1-20 mM antioxidant; 10-129 mM ionic strength provider; and 0.001-2 mM chelator.

D1110 . 120-220 mg/mL secukinumab; 1-9 mM buffer system; 200-250 mM sugar component; 0.01-1.2 mg/mL surfactant; 10-129 mM tonicifier; 10-129 mM ionic strength provider; and 0.001-2 mM chelator.

D1111 . 120-220 mg/mL secukinumab; 51-60 mM buffer system; 200-250 mM sugar component; 0.01-1.2 mg/mL surfactant; 10-129 mM tonicifier; 10-129 mM ionic strength provider; and 0.001-2 mM chelator.

D1112 . 120-220 mg/mL secukinumab; 1-9 mM buffer system; 200-250 mM sugar component; 0.1-20 mM antioxidant; 10-129 mM tonicifier; 10-129 mM ionic strength provider; and 0.001-2 mM chelator.

(continued)

D1113 . 120-220 mg/mL secukinumab; 51-60 mM buffer system; 200-250 mM sugar component; 0.1-20 mM antioxidant; 10-129 mM tonicifier; 10-129 mM ionic strength provider; and 0.001 -2 mM chelator.

D1114. 120-220 mg/mL secukinumab; 200-250 mM sugar component; 20-99 mM amino acid component; 0.01-1.2 mg/mL surfactant; 0.1-20 mM antioxidant; 10-129 mM tonicifier; and 10-129 mM ionic strength provider.

D1115. 120-220 mg/mL secukinumab; 200-250 mM sugar component; 100-149 mM amino acid component; 0.01-1.2 mg/mL surfactant; 0.1-20 mM antioxidant; 10-129 mM tonicifier; and 10-129 mM ionic strength provider.

D1116 . 120-220 mg/mL secukinumab; 200-250 mM sugar component; 20-99 mM amino acid component; 0.01-1.2 mg/mL surfactant; 0.1-20 mM antioxidant; 10-129 mM tonicifier; and 0.001-2 mM chelator.

D1117 . 120-220 mg/mL secukinumab; 200-250 mM sugar component; 100-149 mM amino acid component; 0.01-1.2 mg/mL surfactant; 0.1-20 mM antioxidant; 10-129 mM tonicifier; and 0.001-2 mM chelator.

D1118 . 120-220 mg/mL secukinumab; 200-250 mM sugar component; 20-99 mM amino acid component; 0.01-1.2 mg/mL surfactant; 0.1-20 mM antioxidant; 10-129 mM ionic strength provider; and 0.001-2 mM chelator.

D1119 . 120-220 mg/mL secukinumab; 200-250 mM sugar component; 100-149 mM amino acid component; 0.01-1.2 mg/mL surfactant; 0.1-20 mM antioxidant; 10-129 mM ionic strength provider; and 0.001-2 mM chelator.

D1120. 120-220 mg/mL secukinumab; 200-250 mM sugar component; 20-99 mM amino acid component; 0.01-1.2 mg/mL surfactant; 10-129 mM tonicifier; 10-129 mM ionic strength provider; and 0.001-2 mM chelator.

D1121 . 120-220 mg/mL secukinumab; 200-250 mM sugar component; 100-149 mM amino acid component; 0.01-1.2 mg/mL surfactant; 10-129 mM tonicifier; 10-129 mM ionic strength provider; and 0.001-2 mM chelator.

D1122 . 120-220 mg/mL secukinumab; 200-250 mM sugar component; 20-99 mM amino acid component; 0.1-20 mM antioxidant; 10-129 mM tonicifier; 10-129 mM ionic strength provider; and 0.001-2 mM chelator.

D1123 . 120-220 mg/mL secukinumab; 200-250 mM sugar component; 100-149 mM amino acid component; 0.1-20 mM antioxidant; 10-129 mM tonicifier; 10-129 mM ionic strength provider; and 0.001-2 mM chelator.

D1124 . 120-220 mg/mL secukinumab; 200-250 mM sugar component; 0.01-1.2 mg/mL surfactant; 0.1-20 mM antioxidant; 10-129 mM tonicifier; 10-129 mM ionic strength provider; and 0.001-2 mM chelator.

D1125 . 120-220 mg/mL secukinumab; 1-9 mM buffer system; 200-250 mM sugar component; 20-99 mM amino acid component; 0.01-1.2 mg/mL surfactant; 0.1-20 mM antioxidant; 10-129 mM tonicifier; and 10-129 mM ionic strength provider.

D1126 . 120-220 mg/mL secukinumab; 51-60 mM buffer system; 200-250 mM sugar component; 20-99 mM amino acid component; 0.01-1.2 mg/mL surfactant; 0.1-20 mM antioxidant; 10-129 mM tonicifier; and 10-129 mM ionic strength provider.

D1127 . 120-220 mg/mL secukinumab; 1-9 mM buffer system; 200-250 mM sugar component; 100-149 mM amino acid component; 0.01-1.2 mg/mL surfactant; 0.1-20 mM antioxidant; 10-129 mM tonicifier; and 10-129 mM ionic strength provider.

D1128 . 120-220 mg/mL secukinumab; 51-60 mM buffer system; 200-250 mM sugar component; 100-149 mM amino acid component; 0.01-1.2 mg/mL surfactant; 0.1-20 mM antioxidant; 10-129 mM tonicifier; and 10-129 mM ionic strength provider.

D1129 . 120-220 mg/mL secukinumab; 1-9 mM buffer system; 200-250 mM sugar component; 20-99 mM amino acid component; 0.01-1.2 mg/mL surfactant; 0.1-20 mM antioxidant; 10-129 mM tonicifier; and 0.001-2 mM chelator.

D1130 . 120-220 mg/mL secukinumab; 51-60 mM buffer system; 200-250 mM sugar component; 20-99 mM amino acid component; 0.01-1.2 mg/mL surfactant; 0.1-20 mM antioxidant; 10-129 mM tonicifier; and 0.001-2 mM chelator.

D1131 . 120-220 mg/mL secukinumab; 1-9 mM buffer system; 200-250 mM sugar component; 100-149 mM amino acid component; 0.01-1.2 mg/mL surfactant; 0.1-20 mM antioxidant; 10-129 mM tonicifier; and 0.001-2 mM chelator.

D1132 . 120-220 mg/mL secukinumab; 51-60 mM buffer system; 200-250 mM sugar component; 100-149 mM amino acid component; 0.01-1.2 mg/mL surfactant; 0.1-20 mM antioxidant; 10-129 mM tonicifier; and 0.001-2 mM chelator.

D1133 . 120-220 mg/mL secukinumab; 1-9 mM buffer system; 200-250 mM sugar component; 20-99 mM amino acid component; 0.01-1.2 mg/mL surfactant; 0.1-20 mM antioxidant; 10-129 mM ionic strength provider; and 0.001-2 mM chelator.

(continued)

D1134 . 120-220 mg/mL secukinumab; 51-60 mM buffer system; 200-250 mM sugar component; 20-99 mM amino acid component; 0.01-1.2 mg/mL surfactant; 0.1-20 mM antioxidant; 10-129 mM ionic strength provider; and 0.001-2 mM chelator.

D1135 . 120-220 mg/mL secukinumab; 1-9 mM buffer system; 200-250 mM sugar component; 100-149 mM amino acid component; 0.01-1.2 mg/mL surfactant; 0.1-20 mM antioxidant; 10-129 mM ionic strength provider; and 0.001-2 mM chelator.

D1136 . 120-220 mg/mL secukinumab; 51-60 mM buffer system; 200-250 mM sugar component; 100-149 mM amino acid component; 0.01-1.2 mg/mL surfactant; 0.1-20 mM antioxidant; 10-129 mM ionic strength provider; and 0.001-2 mM chelator.

D1137 . 120-220 mg/mL secukinumab; 1-9 mM buffer system; 200-250 mM sugar component; 20-99 mM amino acid component; 0.01-1.2 mg/mL surfactant; 10-129 mM tonicifier; 10-129 mM ionic strength provider; and 0.001-2 mM chelator.

D1138 . 120-220 mg/mL secukinumab; 51-60 mM buffer system; 200-250 mM sugar component; 20-99 mM amino acid component; 0.01-1.2 mg/mL surfactant; 10-129 mM tonicifier; 10-129 mM ionic strength provider; and 0.001-2 mM chelator.

D1139 . 120-220 mg/mL secukinumab; 1-9 mM buffer system; 200-250 mM sugar component; 100-149 mM amino acid component; 0.01-1.2 mg/mL surfactant; 10-129 mM tonicifier; 10-129 mM ionic strength provider; and 0.001-2 mM chelator.

D1140 . 120-220 mg/mL secukinumab; 51-60 mM buffer system; 200-250 mM sugar component; 100-149 mM amino acid component; 0.01-1.2 mg/mL surfactant; 10-129 mM tonicifier; 10-129 mM ionic strength provider; and 0.001-2 mM chelator.

D1141 . 120-220 mg/mL secukinumab; 1-9 mM buffer system; 200-250 mM sugar component; 20-99 mM amino acid component; 0.1-20 mM antioxidant; 10-129 mM tonicifier; 10-129 mM ionic strength provider; and 0.001-2 mM chelator.

D1142 . 120-220 mg/mL secukinumab; 51-60 mM buffer system; 200-250 mM sugar component; 20-99 mM amino acid component; 0.1-20 mM antioxidant; 10-129 mM tonicifier; 10-129 mM ionic strength provider; and 0.001-2 mM chelator.

D1143 . 120-220 mg/mL secukinumab; 1-9 mM buffer system; 200-250 mM sugar component; 100-149 mM amino acid component; 0.1-20 mM antioxidant; 10-129 mM tonicifier; 10-129 mM ionic strength provider; and 0.001-2 mM chelator.

D1144 . 120-220 mg/mL secukinumab; 51-60 mM buffer system; 200-250 mM sugar component; 100-149 mM amino acid component; 0.1-20 mM antioxidant; 10-129 mM tonicifier; 10-129 mM ionic strength provider; and 0.001-2 mM chelator.

D1145 . 120-220 mg/mL secukinumab; 1-9 mM buffer system; 200-250 mM sugar component; 0.01-1.2 mg/mL surfactant; 0.1-20 mM antioxidant; 10-129 mM tonicifier; 10-129 mM ionic strength provider; and 0.001-2 mM chelator.

D1146 . 120-220 mg/mL secukinumab; 51-60 mM buffer system; 200-250 mM sugar component; 0.01-1.2 mg/mL surfactant; 0.1-20 mM antioxidant; 10-129 mM tonicifier; 10-129 mM ionic strength provider; and 0.001-2 mM chelator.

D1147. 120-220 mg/mL secukinumab; 200-250 mM sugar component; 20-99 mM amino acid component; 0.01-1.2 mg/mL surfactant; 0.1-20 mM antioxidant; 10-129 mM tonicifier; 10-129 mM ionic strength provider; and 0.001-2 mM chelator.

D1148. 120-220 mg/mL secukinumab; 200-250 mM sugar component; 100-149 mM amino acid component; 0.01-1.2 mg/mL surfactant; 0.1-20 mM antioxidant; 10-129 mM tonicifier; 10-129 mM ionic strength provider; and 0.001-2 mM chelator.

D1149 . 120-220 mg/mL secukinumab; 1-9 mM buffer system; 200-250 mM sugar component; 20-99 mM amino acid component; 0.01-1.2 mg/mL surfactant; 0.1-20 mM antioxidant; 10-129 mM tonicifier; 10-129 mM ionic strength provider; and 0.001-2 mM chelator.

D1150 . 120-220 mg/mL secukinumab; 51-60 mM buffer system; 200-250 mM sugar component; 20-99 mM amino acid component; 0.01-1.2 mg/mL surfactant; 0.1-20 mM antioxidant; 10-129 mM tonicifier; 10-129 mM ionic strength provider; and 0.001-2 mM chelator.

D1151 . 120-220 mg/mL secukinumab; 1-9 mM buffer system; 200-250 mM sugar component; 100-149 mM amino acid component; 0.01-1.2 mg/mL surfactant; 0.1-20 mM antioxidant; 10-129 mM tonicifier; 10-129 mM ionic strength provider; and 0.001-2 mM chelator.

(continued)

D1152 . 120-220 mg/mL secukinumab; 51-60 mM buffer system; 200-250 mM sugar component; 100-149 mM amino acid component; 0.01-1.2 mg/mL surfactant; 0.1-20 mM antioxidant; 10-129 mM tonicifier; 10-129 mM ionic strength provider; and 0.001-2 mM chelator.

The embodiments of D1-D1152 may be further defined or characterised by features of numbered paragraphs E1-E1151, F1-F12, G1-G959, H1-H1423, 11-12400, and J1-J1158.

*Embodiments of Combinations of Ingredients Quantified by Narrower Molar Ratios*

E) General Aspects and Embodiments Relating to Combinations of Ingredients Quantified by Narrower Molar Ratios

The biopharmaceutical composition suitably comprises, or consists of (suitably along with a diluent, which is preferably water), secukinumab alongside one or more (preferably two or more) of a buffer system, a sugar component, an amino acid component, a surfactant, an antioxidant, a tonicifier, an ionic strength provider, and/or a chelator, in a respective molar ratio of 0.81-1.49 : 1-9 (or 51-60) : 50-129 (or 130-174 or 200-250) : 20-99 (or 100-149) : 0.008-0.92 : 0.1-20 : 10-129 : 10-129 : 0.001-2. The following numbered paragraphs E1-E1151 disclose specific aspects and embodiments of the invention, wherein the biopharmaceutical composition comprises, consists of (suitably along with a diluent, which is preferably water), the following components in molar ratios as numerically specified:

E1. 0.81-1.49 secukinumab : 1-9 buffer system.
E2. 0.81-1.49 secukinumab : 51-60 buffer system.
E3. 0.81-1.49 secukinumab : 50-129 sugar component.
E4. 0.81-1.49 secukinumab : 130-174 sugar component.
E5. 0.81-1.49 secukinumab : 20-99 amino acid component.
E6. 0.81-1.49 secukinumab : 100-149 amino acid component.
E7. 0.81-1.49 secukinumab : 0.008-0.92 surfactant.
E8. 0.81-1.49 secukinumab : 0.1-20 antioxidant.
E9. 0.81-1.49 secukinumab : 10-129 tonicifier.
E10. 0.81-1.49 secukinumab : 10-129 ionic strength provider.
E11. 0.81-1.49 secukinumab : 0.001-2 chelator.
E12. 0.81-1.49 secukinumab : 1-9 buffer system : 50-129 sugar component.
E13. 0.81-1.49 secukinumab : 51-60 buffer system : 50-129 sugar component.
E14. 0.81-1.49 secukinumab : 1-9 buffer system : 130-174 sugar component.
E15. 0.81-1.49 secukinumab : 51-60 buffer system : 130-174 sugar component.
E16. 0.81-1.49 secukinumab : 1-9 buffer system : 20-99 amino acid component.
E17. 0.81-1.49 secukinumab : 51-60 buffer system : 20-99 amino acid component.
E18. 0.81-1.49 secukinumab : 1-9 buffer system : 100-149 amino acid component.
E19. 0.81-1.49 secukinumab : 51-60 buffer system : 100-149 amino acid component.
E20. 0.81-1.49 secukinumab : 1-9 buffer system : 0.008-0.92 surfactant.
E21. 0.81-1.49 secukinumab : 51-60 buffer system : 0.008-0.92 surfactant.
E22. 0.81-1.49 secukinumab : 1-9 buffer system : 0.1-20 antioxidant.
E23. 0.81-1.49 secukinumab : 51-60 buffer system : 0.1-20 antioxidant.
E24. 0.81-1.49 secukinumab : 1-9 buffer system : 10-129 tonicifier.
E25. 0.81-1.49 secukinumab : 51-60 buffer system : 10-129 tonicifier.
E26. 0.81-1.49 secukinumab : 1-9 buffer system : 10-129 ionic strength provider.
E27. 0.81-1.49 secukinumab : 51-60 buffer system : 10-129 ionic strength provider.
E28. 0.81-1.49 secukinumab : 1-9 buffer system : 0.001-2 chelator.
E29. 0.81-1.49 secukinumab : 51-60 buffer system : 0.001-2 chelator.
E30. 0.81-1.49 secukinumab : 50-129 sugar component: 20-99 amino acid component.
E31. 0.81-1.49 secukinumab : 130-174 sugar component: 20-99 amino acid component.
E32. 0.81-1.49 secukinumab : 50-129 sugar component: 100-149 amino acid component.
E33. 0.81-1.49 secukinumab : 130-174 sugar component: 100-149 amino acid component.

(continued)

E34. 0.81-1.49 secukinumab : 50-129 sugar component : 0.008-0.92 surfactant.
E35. 0.81-1.49 secukinumab : 130-174 sugar component: 0.008-0.92 surfactant.
E36. 0.81-1.49 secukinumab : 50-129 sugar component: 0.1-20 antioxidant.
E37. 0.81-1.49 secukinumab : 130-174 sugar component: 0.1-20 antioxidant.
E38. 0.81-1.49 secukinumab : 50-129 sugar component: 10-129 tonicifier.
E39. 0.81-1.49 secukinumab : 130-174 sugar component: 10-129 tonicifier.
E40. 0.81-1.49 secukinumab : 50-129 sugar component: 10-129 ionic strength provider.
E41. 0.81-1.49 secukinumab : 130-174 sugar component: 10-129 ionic strength provider.
E42. 0.81-1.49 secukinumab : 50-129 sugar component: 0.001-2 chelator.
E43. 0.81-1.49 secukinumab : 130-174 sugar component: 0.001-2 chelator.
E44. 0.81-1.49 secukinumab : 20-99 amino acid component: 0.008-0.92 surfactant.
E45. 0.81-1.49 secukinumab : 100-149 amino acid component: 0.008-0.92 surfactant.
E46. 0.81-1.49 secukinumab : 20-99 amino acid component: 0.1-20 antioxidant.
E47. 0.81-1.49 secukinumab : 100-149 amino acid component: 0.1-20 antioxidant.
E48. 0.81-1.49 secukinumab : 20-99 amino acid component: 10-129 tonicifier.
E49. 0.81-1.49 secukinumab : 100-149 amino acid component: 10-129 tonicifier.
E50. 0.81-1.49 secukinumab : 20-99 amino acid component: 10-129 ionic strength provider.
E51. 0.81-1.49 secukinumab : 100-149 amino acid component: 10-129 ionic strength provider.
E52. 0.81-1.49 secukinumab : 20-99 amino acid component: 0.001-2 chelator.
E53. 0.81-1.49 secukinumab : 100-149 amino acid component: 0.001-2 chelator.
E54. 0.81-1.49 secukinumab : 0.008-0.92 surfactant: 0.1-20 antioxidant.
E55. 0.81-1.49 secukinumab : 0.008-0.92 surfactant: 10-129 tonicifier.
E56. 0.81-1.49 secukinumab : 0.008-0.92 surfactant: 10-129 ionic strength provider.
E57. 0.81-1.49 secukinumab : 0.008-0.92 surfactant: 0.001-2 chelator.
E58. 0.81-1.49 secukinumab : 0.1-20 antioxidant: 10-129 tonicifier.
E59. 0.81-1.49 secukinumab : 0.1-20 antioxidant: 10-129 ionic strength provider.
E60. 0.81-1.49 secukinumab : 0.1-20 antioxidant: 0.001-2 chelator.
E61. 0.81-1.49 secukinumab : 10-129 tonicifier : 10-129 ionic strength provider.
E62. 0.81-1.49 secukinumab : 10-129 tonicifier : 0.001-2 chelator.
E63. 0.81-1.49 secukinumab : 10-129 ionic strength provider: 0.001-2 chelator.
E64. 0.81-1.49 secukinumab : 1-9 buffer system : 50-129 sugar component: 20-99 amino acid component.
E65. 0.81-1.49 secukinumab : 51-60 buffer system : 50-129 sugar component: 20-99 amino acid component.
E66. 0.81-1.49 secukinumab : 1-9 buffer system : 130-174 sugar component: 20-99 amino acid component.
E67. 0.81-1.49 secukinumab : 51-60 buffer system : 130-174 sugar component: 20-99 amino acid component.
E68. 0.81-1.49 secukinumab : 1-9 buffer system : 50-129 sugar component: 100-149 amino acid component.
E69. 0.81-1.49 secukinumab : 51-60 buffer system : 50-129 sugar component: 100-149 amino acid component.
E70. 0.81-1.49 secukinumab : 1-9 buffer system : 130-174 sugar component: 100-149 amino acid component.
E71. 0.81-1.49 secukinumab : 51-60 buffer system : 130-174 sugar component: 100-149 amino acid component.
E72. 0.81-1.49 secukinumab : 1-9 buffer system : 50-129 sugar component : 0.008-0.92 surfactant.
E73. 0.81-1.49 secukinumab : 51-60 buffer system : 50-129 sugar component : 0.008-0.92 surfactant.
E74. 0.81-1.49 secukinumab : 1-9 buffer system : 130-174 sugar component : 0.008-0.92 surfactant.
E75. 0.81-1.49 secukinumab : 51-60 buffer system : 130-174 sugar component : 0.008-0.92 surfactant.
E76. 0.81-1.49 secukinumab : 1-9 buffer system : 50-129 sugar component: 0.1-20 antioxidant.
E77. 0.81-1.49 secukinumab : 51-60 buffer system : 50-129 sugar component: 0.1-20 antioxidant.
E78. 0.81-1.49 secukinumab : 1-9 buffer system : 130-174 sugar component: 0.1-20 antioxidant.
E79. 0.81-1.49 secukinumab : 51-60 buffer system : 130-174 sugar component: 0.1-20 antioxidant.
E80. 0.81-1.49 secukinumab : 1-9 buffer system : 50-129 sugar component: 10-129 tonicifier.
E81. 0.81-1.49 secukinumab : 51-60 buffer system : 50-129 sugar component: 10-129 tonicifier.
E82. 0.81-1.49 secukinumab : 1-9 buffer system : 130-174 sugar component: 10-129 tonicifier.
E83. 0.81-1.49 secukinumab : 51-60 buffer system : 130-174 sugar component: 10-129 tonicifier.

(continued)

E84. 0.81-1.49 secukinumab : 1-9 buffer system : 50-129 sugar component: 10-129 ionic strength provider.
E85. 0.81-1.49 secukinumab : 51-60 buffer system : 50-129 sugar component: 10-129 ionic strength provider.
E86. 0.81-1.49 secukinumab : 1-9 buffer system : 130-174 sugar component: 10-129 ionic strength provider.
E87. 0.81-1.49 secukinumab : 51-60 buffer system : 130-174 sugar component: 10-129 ionic strength provider.
E88. 0.81-1.49 secukinumab : 1-9 buffer system : 50-129 sugar component: 0.001-2 chelator.
E89. 0.81-1.49 secukinumab : 51-60 buffer system : 50-129 sugar component: 0.001-2 chelator.
E90. 0.81-1.49 secukinumab : 1-9 buffer system : 130-174 sugar component: 0.001-2 chelator.
E91. 0.81-1.49 secukinumab : 51-60 buffer system : 130-174 sugar component: 0.001-2 chelator.
E92. 0.81-1.49 secukinumab : 1-9 buffer system : 20-99 amino acid component: 0.008-0.92 surfactant.
E93. 0.81-1.49 secukinumab : 51-60 buffer system : 20-99 amino acid component: 0.008-0.92 surfactant.
E94. 0.81-1.49 secukinumab : 1-9 buffer system : 100-149 amino acid component: 0.008-0.92 surfactant.
E95. 0.81-1.49 secukinumab : 51-60 buffer system : 100-149 amino acid component : 0.008-0.92 surfactant.
E96. 0.81-1.49 secukinumab : 1-9 buffer system : 20-99 amino acid component: 0.1-20 antioxidant.
E97. 0.81-1.49 secukinumab : 51-60 buffer system : 20-99 amino acid component: 0.1-20 antioxidant.
E98. 0.81-1.49 secukinumab : 1-9 buffer system : 100-149 amino acid component: 0.1-20 antioxidant.
E99. 0.81-1.49 secukinumab : 51-60 buffer system : 100-149 amino acid component: 0.1-20 antioxidant.
E100. 0.81-1.49 secukinumab : 1-9 buffer system : 20-99 amino acid component: 10-129 tonicifier.
E101. 0.81-1.49 secukinumab : 51-60 buffer system : 20-99 amino acid component: 10-129 tonicifier.
E102. 0.81-1.49 secukinumab : 1-9 buffer system : 100-149 amino acid component: 10-129 tonicifier.
E103. 0.81-1.49 secukinumab : 51-60 buffer system : 100-149 amino acid component: 10-129 tonicifier.
E104. 0.81-1.49 secukinumab : 1-9 buffer system : 20-99 amino acid component: 10-129 ionic strength provider.
E105. 0.81-1.49 secukinumab : 51-60 buffer system : 20-99 amino acid component: 10-129 ionic strength provider.
E106. 0.81-1.49 secukinumab : 1-9 buffer system : 100-149 amino acid component: 10-129 ionic strength provider.
E107. 0.81-1.49 secukinumab : 51-60 buffer system : 100-149 amino acid component: 10-129 ionic strength provider.
E108. 0.81-1.49 secukinumab : 1-9 buffer system : 20-99 amino acid component: 0.001-2 chelator.
E109. 0.81-1.49 secukinumab : 51-60 buffer system : 20-99 amino acid component: 0.001-2 chelator.
E110. 0.81-1.49 secukinumab : 1-9 buffer system : 100-149 amino acid component: 0.001-2 chelator.
E111. 0.81-1.49 secukinumab : 51-60 buffer system : 100-149 amino acid component: 0.001-2 chelator.
E112. 0.81-1.49 secukinumab : 1-9 buffer system : 0.008-0.92 surfactant: 0.1-20 antioxidant.
E113. 0.81-1.49 secukinumab : 51-60 buffer system : 0.008-0.92 surfactant: 0.1-20 antioxidant.
E114. 0.81-1.49 secukinumab : 1-9 buffer system : 0.008-0.92 surfactant: 10-129 tonicifier.
E115. 0.81-1.49 secukinumab : 51-60 buffer system : 0.008-0.92 surfactant: 10-129 tonicifier.
E116. 0.81-1.49 secukinumab : 1-9 buffer system : 0.008-0.92 surfactant: 10-129 ionic strength provider.
E117. 0.81-1.49 secukinumab : 51-60 buffer system : 0.008-0.92 surfactant: 10-129 ionic strength provider.
E118. 0.81-1.49 secukinumab : 1-9 buffer system : 0.008-0.92 surfactant: 0.001-2 chelator.
E119. 0.81-1.49 secukinumab : 51-60 buffer system : 0.008-0.92 surfactant: 0.001-2 chelator.
E120. 0.81-1.49 secukinumab : 1-9 buffer system : 0.1-20 antioxidant: 10-129 tonicifier.
E121. 0.81-1.49 secukinumab : 51-60 buffer system : 0.1-20 antioxidant: 10-129 tonicifier.
E122. 0.81-1.49 secukinumab : 1-9 buffer system : 0.1-20 antioxidant: 10-129 ionic strength provider.
E123. 0.81-1.49 secukinumab : 51-60 buffer system : 0.1-20 antioxidant: 10-129 ionic strength provider.
E124. 0.81-1.49 secukinumab : 1-9 buffer system : 0.1-20 antioxidant: 0.001-2 chelator.
E125. 0.81-1.49 secukinumab : 51-60 buffer system : 0.1-20 antioxidant: 0.001-2 chelator.
E126. 0.81-1.49 secukinumab : 1-9 buffer system : 10-129 tonicifier : 10-129 ionic strength provider.
E127. 0.81-1.49 secukinumab : 51-60 buffer system : 10-129 tonicifier : 10-129 ionic strength provider.
E128. 0.81-1.49 secukinumab : 1-9 buffer system : 10-129 tonicifier : 0.001-2 chelator.
E129. 0.81-1.49 secukinumab : 51-60 buffer system : 10-129 tonicifier : 0.001-2 chelator.
E130. 0.81-1.49 secukinumab : 1-9 buffer system : 10-129 ionic strength provider: 0.001-2 chelator.
E131. 0.81-1.49 secukinumab : 51-60 buffer system : 10-129 ionic strength provider: 0.001-2 chelator.

(continued)

E132. 0.81-1.49 secukinumab : 50-129 sugar component: 20-99 amino acid component : 0.008-0.92 surfactant.

E133. 0.81-1.49 secukinumab : 130-174 sugar component: 20-99 amino acid component: 0.008-0.92 surfactant.

E134. 0.81-1.49 secukinumab : 50-129 sugar component: 100-149 amino acid component : 0.008-0.92 surfactant.

E135. 0.81-1.49 secukinumab : 130-174 sugar component: 100-149 amino acid component: 0.008-0.92 surfactant.

E136. 0.81-1.49 secukinumab : 50-129 sugar component: 20-99 amino acid component: 0.1-20 antioxidant.

E137. 0.81-1.49 secukinumab : 130-174 sugar component: 20-99 amino acid component: 0.1-20 antioxidant.

E138. 0.81-1.49 secukinumab : 50-129 sugar component: 100-149 amino acid component: 0.1-20 antioxidant.

E139. 0.81-1.49 secukinumab : 130-174 sugar component: 100-149 amino acid component: 0.1-20 antioxidant.

E140. 0.81-1.49 secukinumab : 50-129 sugar component: 20-99 amino acid component: 10-129 tonicifier.

E141. 0.81-1.49 secukinumab : 130-174 sugar component: 20-99 amino acid component: 10-129 tonicifier.

E142. 0.81-1.49 secukinumab : 50-129 sugar component: 100-149 amino acid component: 10-129 tonicifier.

E143. 0.81-1.49 secukinumab : 130-174 sugar component: 100-149 amino acid component: 10-129 tonicifier.

E144. 0.81-1.49 secukinumab : 50-129 sugar component: 20-99 amino acid component: 10-129 ionic strength provider.

E145. 0.81-1.49 secukinumab : 130-174 sugar component: 20-99 amino acid component: 10-129 ionic strength provider.

E146. 0.81-1.49 secukinumab : 50-129 sugar component: 100-149 amino acid component: 10-129 ionic strength provider.

E147. 0.81-1.49 secukinumab : 130-174 sugar component: 100-149 amino acid component: 10-129 ionic strength provider.

E148. 0.81-1.49 secukinumab : 50-129 sugar component: 20-99 amino acid component: 0.001-2 chelator.

E149. 0.81-1.49 secukinumab : 130-174 sugar component: 20-99 amino acid component: 0.001-2 chelator.

E150. 0.81-1.49 secukinumab : 50-129 sugar component: 100-149 amino acid component: 0.001-2 chelator.

E151. 0.81-1.49 secukinumab : 130-174 sugar component: 100-149 amino acid component: 0.001-2 chelator.

E152. 0.81-1.49 secukinumab : 50-129 sugar component: 0.008-0.92 surfactant: 0.1-20 antioxidant.

E153. 0.81-1.49 secukinumab : 130-174 sugar component: 0.008-0.92 surfactant: 0.1-20 antioxidant.

E154. 0.81-1.49 secukinumab : 50-129 sugar component: 0.008-0.92 surfactant: 10-129 tonicifier.

E155. 0.81-1.49 secukinumab : 130-174 sugar component: 0.008-0.92 surfactant: 10-129 tonicifier.

E156. 0.81-1.49 secukinumab : 50-129 sugar component: 0.008-0.92 surfactant: 10-129 ionic strength provider.

E157. 0.81-1.49 secukinumab : 130-174 sugar component: 0.008-0.92 surfactant: 10-129 ionic strength provider.

E158. 0.81-1.49 secukinumab : 50-129 sugar component: 0.008-0.92 surfactant: 0.001-2 chelator.

E159. 0.81-1.49 secukinumab : 130-174 sugar component: 0.008-0.92 surfactant: 0.001-2 chelator.

E160. 0.81-1.49 secukinumab : 50-129 sugar component: 0.1-20 antioxidant: 10-129 tonicifier.

E161. 0.81-1.49 secukinumab : 130-174 sugar component: 0.1-20 antioxidant: 10-129 tonicifier.

E162. 0.81-1.49 secukinumab : 50-129 sugar component: 0.1-20 antioxidant: 10-129 ionic strength provider.

E163. 0.81-1.49 secukinumab : 130-174 sugar component: 0.1-20 antioxidant: 10-129 ionic strength provider.

E164. 0.81-1.49 secukinumab : 50-129 sugar component: 0.1-20 antioxidant: 0.001-2 chelator.

E165. 0.81-1.49 secukinumab : 130-174 sugar component: 0.1-20 antioxidant: 0.001-2 chelator.

E166. 0.81-1.49 secukinumab : 50-129 sugar component: 10-129 tonicifier : 10-129 ionic strength provider.

E167. 0.81-1.49 secukinumab : 130-174 sugar component: 10-129 tonicifier: 10-129 ionic strength provider.

E168. 0.81-1.49 secukinumab : 50-129 sugar component: 10-129 tonicifier : 0.001-2 chelator.

E169. 0.81-1.49 secukinumab : 130-174 sugar component: 10-129 tonicifier : 0.001-2 chelator.

E170. 0.81-1.49 secukinumab : 50-129 sugar component: 10-129 ionic strength provider: 0.001-2 chelator.

E171. 0.81-1.49 secukinumab : 130-174 sugar component: 10-129 ionic strength provider: 0.001-2 chelator.

E172. 0.81-1.49 secukinumab : 20-99 amino acid component: 0.008-0.92 surfactant: 0.1-20 antioxidant.

E173. 0.81-1.49 secukinumab : 100-149 amino acid component: 0.008-0.92 surfactant: 0.1-20 antioxidant.

E174. 0.81-1.49 secukinumab : 20-99 amino acid component: 0.008-0.92 surfactant: 10-129 tonicifier.

E175. 0.81-1.49 secukinumab : 100-149 amino acid component: 0.008-0.92 surfactant: 10-129 tonicifier.

(continued)

E176. 0.81-1.49 secukinumab : 20-99 amino acid component: 0.008-0.92 surfactant: 10-129 ionic strength provider.

E177. 0.81-1.49 secukinumab : 100-149 amino acid component: 0.008-0.92 surfactant: 10-129 ionic strength provider.

E178. 0.81-1.49 secukinumab : 20-99 amino acid component: 0.008-0.92 surfactant: 0.001-2 chelator.

E179. 0.81-1.49 secukinumab : 100-149 amino acid component: 0.008-0.92 surfactant: 0.001-2 chelator.

E180. 0.81-1.49 secukinumab : 20-99 amino acid component: 0.1-20 antioxidant: 10-129 tonicifier.

E181. 0.81-1.49 secukinumab : 100-149 amino acid component: 0.1-20 antioxidant: 10-129 tonicifier.

E182. 0.81-1.49 secukinumab : 20-99 amino acid component: 0.1-20 antioxidant: 10-129 ionic strength provider.

E183. 0.81-1.49 secukinumab : 100-149 amino acid component: 0.1-20 antioxidant: 10-129 ionic strength provider.

E184. 0.81-1.49 secukinumab : 20-99 amino acid component: 0.1-20 antioxidant: 0.001-2 chelator.

E185. 0.81-1.49 secukinumab : 100-149 amino acid component: 0.1-20 antioxidant: 0.001-2 chelator.

E186. 0.81-1.49 secukinumab : 20-99 amino acid component: 10-129 tonicifier: 10-129 ionic strength provider.

E187. 0.81-1.49 secukinumab : 100-149 amino acid component: 10-129 tonicifier : 10-129 ionic strength provider.

E188. 0.81-1.49 secukinumab : 20-99 amino acid component: 10-129 tonicifier: 0.001-2 chelator.

E189. 0.81-1.49 secukinumab : 100-149 amino acid component: 10-129 tonicifier : 0.001-2 chelator.

E190. 0.81-1.49 secukinumab : 20-99 amino acid component: 10-129 ionic strength provider: 0.001-2 chelator.

E191. 0.81-1.49 secukinumab : 100-149 amino acid component: 10-129 ionic strength provider: 0.001-2 chelator.

E192. 0.81-1.49 secukinumab : 0.008-0.92 surfactant: 0.1-20 antioxidant: 10-129 tonicifier.

E193. 0.81-1.49 secukinumab : 0.008-0.92 surfactant: 0.1-20 antioxidant: 10-129 ionic strength provider.

E194. 0.81-1.49 secukinumab : 0.008-0.92 surfactant: 0.1-20 antioxidant: 0.001-2 chelator.

E195. 0.81-1.49 secukinumab : 0.008-0.92 surfactant: 10-129 tonicifier: 10-129 ionic strength provider.

E196. 0.81-1.49 secukinumab : 0.008-0.92 surfactant: 10-129 tonicifier: 0.001-2 chelator.

E197. 0.81-1.49 secukinumab : 0.008-0.92 surfactant: 10-129 ionic strength provider: 0.001-2 chelator.

E198. 0.81-1.49 secukinumab : 0.1-20 antioxidant: 10-129 tonicifier : 10-129 ionic strength provider.

E199. 0.81-1.49 secukinumab : 0.1-20 antioxidant: 10-129 tonicifier: 0.001-2 chelator.

E200. 0.81-1.49 secukinumab : 0.1-20 antioxidant: 10-129 ionic strength provider: 0.001-2 chelator.

E201. 0.81-1.49 secukinumab : 10-129 tonicifier : 10-129 ionic strength provider: 0.001-2 chelator.

E202. 0.81-1.49 secukinumab : 1-9 buffer system : 50-129 sugar component : 20-99 amino acid component : 0.008-0.92 surfactant.

E203. 0.81-1.49 secukinumab : 51-60 buffer system : 50-129 sugar component : 20-99 amino acid component : 0.008-0.92 surfactant.

E204. 0.81-1.49 secukinumab : 1-9 buffer system : 130-174 sugar component : 20-99 amino acid component : 0.008-0.92 surfactant.

E205. 0.81-1.49 secukinumab : 51-60 buffer system : 130-174 sugar component : 20-99 amino acid component : 0.008-0.92 surfactant.

E206. 0.81-1.49 secukinumab : 1-9 buffer system : 50-129 sugar component : 100-149 amino acid component : 0.008-0.92 surfactant.

E207. 0.81-1.49 secukinumab : 51-60 buffer system : 50-129 sugar component : 100-149 amino acid component : 0.008-0.92 surfactant.

E208. 0.81-1.49 secukinumab : 1-9 buffer system : 130-174 sugar component : 100-149 amino acid component : 0.008-0.92 surfactant.

E209. 0.81-1.49 secukinumab : 51-60 buffer system : 130-174 sugar component : 100-149 amino acid component : 0.008-0.92 surfactant.

E210. 0.81-1.49 secukinumab : 1-9 buffer system : 50-129 sugar component: 20-99 amino acid component: 0.1-20 antioxidant.

E211. 0.81-1.49 secukinumab : 51-60 buffer system : 50-129 sugar component: 20-99 amino acid component: 0.1-20 antioxidant.

E212. 0.81-1.49 secukinumab : 1-9 buffer system : 130-174 sugar component: 20-99 amino acid component: 0.1-20 antioxidant.

(continued)

E213. 0.81-1.49 secukinumab : 51-60 buffer system : 130-174 sugar component : 20-99 amino acid component : 0.1-20 antioxidant.

E214. 0.81-1.49 secukinumab : 1-9 buffer system : 50-129 sugar component: 100-149 amino acid component: 0.1-20 antioxidant.

E215. 0.81-1.49 secukinumab : 51-60 buffer system : 50-129 sugar component : 100-149 amino acid component : 0.1-20 antioxidant.

E216. 0.81-1.49 secukinumab : 1-9 buffer system : 130-174 sugar component : 100-149 amino acid component : 0.1-20 antioxidant.

E217. 0.81-1.49 secukinumab : 51-60 buffer system : 130-174 sugar component : 100-149 amino acid component : 0.1-20 antioxidant.

E218. 0.81-1.49 secukinumab : 1-9 buffer system : 50-129 sugar component: 20-99 amino acid component: 10-129 tonicifier.

E219. 0.81-1.49 secukinumab : 51-60 buffer system : 50-129 sugar component: 20-99 amino acid component: 10-129 tonicifier.

E220. 0.81-1.49 secukinumab : 1-9 buffer system : 130-174 sugar component: 20-99 amino acid component: 10-129 tonicifier.

E221. 0.81-1.49 secukinumab : 51-60 buffer system : 130-174 sugar component: 20-99 amino acid component: 10-129 tonicifier.

E222. 0.81-1.49 secukinumab : 1-9 buffer system : 50-129 sugar component: 100-149 amino acid component: 10-129 tonicifier.

E223. 0.81-1.49 secukinumab: 51-60 buffer system : 50-129 sugar component : 100-149 amino acid component: 10-129 tonicifier.

E224. 0.81-1.49 secukinumab : 1-9 buffer system : 130-174 sugar component: 100-149 amino acid component: 10-129 tonicifier.

E225. 0.81-1.49 secukinumab : 51-60 buffer system : 130-174 sugar component : 100-149 amino acid component : 10-129 tonicifier.

E226. 0.81-1.49 secukinumab : 1-9 buffer system : 50-129 sugar component: 20-99 amino acid component: 10-129 ionic strength provider.

E227. 0.81-1.49 secukinumab : 51-60 buffer system : 50-129 sugar component : 20-99 amino acid component : 10-129 ionic strength provider.

E228. 0.81-1.49 secukinumab : 1-9 buffer system : 130-174 sugar component : 20-99 amino acid component : 10-129 ionic strength provider.

E229. 0.81-1.49 secukinumab : 51-60 buffer system : 130-174 sugar component : 20-99 amino acid component : 10-129 ionic strength provider.

E230. 0.81-1.49 secukinumab : 1-9 buffer system : 50-129 sugar component : 100-149 amino acid component : 10-129 ionic strength provider.

E231. 0.81-1.49 secukinumab : 51-60 buffer system : 50-129 sugar component: 100-149 amino acid component: 10-129 ionic strength provider.

E232. 0.81-1.49 secukinumab : 1-9 buffer system : 130-174 sugar component : 100-149 amino acid component : 10-129 ionic strength provider.

E233. 0.81-1.49 secukinumab : 51-60 buffer system : 130-174 sugar component: 100-149 amino acid component: 10-129 ionic strength provider.

E234. 0.81-1.49 secukinumab : 1-9 buffer system : 50-129 sugar component: 20-99 amino acid component: 0.001-2 chelator.

E235. 0.81-1.49 secukinumab : 51-60 buffer system : 50-129 sugar component: 20-99 amino acid component: 0.001-2 chelator.

E236. 0.81-1.49 secukinumab : 1-9 buffer system : 130-174 sugar component: 20-99 amino acid component: 0.001-2 chelator.

E237. 0.81-1.49 secukinumab : 51-60 buffer system : 130-174 sugar component: 20-99 amino acid component: 0.001-2 chelator.

E238. 0.81-1.49 secukinumab : 1-9 buffer system : 50-129 sugar component: 100-149 amino acid component: 0.001-2 chelator.

(continued)

E239. 0.81-1.49 secukinumab : 51-60 buffer system : 50-129 sugar component : 100-149 amino acid component : 0.001-2 chelator.

E240. 0.81-1.49 secukinumab : 1-9 buffer system : 130-174 sugar component: 100-149 amino acid component: 0.001-2 chelator.

E241. 0.81-1.49 secukinumab : 51-60 buffer system : 130-174 sugar component : 100-149 amino acid component : 0.001-2 chelator.

E242. 0.81-1.49 secukinumab : 1-9 buffer system : 50-129 sugar component: 0.008-0.92 surfactant: 0.1-20 antioxidant.

E243. 0.81-1.49 secukinumab : 51-60 buffer system : 50-129 sugar component: 0.008-0.92 surfactant: 0.1-20 antioxidant.

E244. 0.81-1.49 secukinumab : 1-9 buffer system : 130-174 sugar component: 0.008-0.92 surfactant: 0.1-20 antioxidant.

E245. 0.81-1.49 secukinumab : 51-60 buffer system : 130-174 sugar component: 0.008-0.92 surfactant: 0.1-20 antioxidant.

E246. 0.81-1.49 secukinumab : 1-9 buffer system : 50-129 sugar component: 0.008-0.92 surfactant: 10-129 tonicifier.

E247. 0.81-1.49 secukinumab : 51-60 buffer system : 50-129 sugar component: 0.008-0.92 surfactant: 10-129 tonicifier.

E248. 0.81-1.49 secukinumab : 1-9 buffer system : 130-174 sugar component: 0.008-0.92 surfactant: 10-129 tonicifier.

E249. 0.81-1.49 secukinumab : 51-60 buffer system : 130-174 sugar component: 0.008-0.92 surfactant: 10-129 tonicifier.

E250. 0.81-1.49 secukinumab : 1-9 buffer system : 50-129 sugar component : 0.008-0.92 surfactant : 10-129 ionic strength provider.

E251. 0.81-1.49 secukinumab : 51-60 buffer system : 50-129 sugar component : 0.008-0.92 surfactant : 10-129 ionic strength provider.

E252. 0.81-1.49 secukinumab : 1-9 buffer system : 130-174 sugar component : 0.008-0.92 surfactant : 10-129 ionic strength provider.

E253. 0.81-1.49 secukinumab : 51-60 buffer system : 130-174 sugar component : 0.008-0.92 surfactant : 10-129 ionic strength provider.

E254. 0.81-1.49 secukinumab : 1-9 buffer system : 50-129 sugar component: 0.008-0.92 surfactant: 0.001-2 chelator.

E255. 0.81-1.49 secukinumab : 51-60 buffer system : 50-129 sugar component: 0.008-0.92 surfactant: 0.001-2 chelator.

E256. 0.81-1.49 secukinumab : 1-9 buffer system : 130-174 sugar component: 0.008-0.92 surfactant: 0.001-2 chelator.

E257. 0.81-1.49 secukinumab : 51-60 buffer system : 130-174 sugar component : 0.008-0.92 surfactant: 0.001-2 chelator.

E258. 0.81-1.49 secukinumab : 1-9 buffer system : 50-129 sugar component: 0.1-20 antioxidant: 10-129 tonicifier.

E259. 0.81-1.49 secukinumab : 51-60 buffer system : 50-129 sugar component: 0.1-20 antioxidant: 10-129 tonicifier.

E260. 0.81-1.49 secukinumab : 1-9 buffer system : 130-174 sugar component: 0.1-20 antioxidant: 10-129 tonicifier.

E261. 0.81-1.49 secukinumab : 51-60 buffer system : 130-174 sugar component: 0.1-20 antioxidant: 10-129 tonicifier.

E262. 0.81-1.49 secukinumab : 1-9 buffer system : 50-129 sugar component: 0.1-20 antioxidant: 10-129 ionic strength provider.

E263. 0.81-1.49 secukinumab : 51-60 buffer system : 50-129 sugar component : 0.1-20 antioxidant: 10-129 ionic strength provider.

E264. 0.81-1.49 secukinumab : 1-9 buffer system : 130-174 sugar component: 0.1-20 antioxidant: 10-129 ionic strength provider.

(continued)

E265. 0.81-1.49 secukinumab : 51-60 buffer system : 130-174 sugar component : 0.1-20 antioxidant : 10-129 ionic strength provider.

E266. 0.81-1.49 secukinumab : 1-9 buffer system : 50-129 sugar component: 0.1-20 antioxidant: 0.001-2 chelator.

E267. 0.81-1.49 secukinumab : 51-60 buffer system : 50-129 sugar component: 0.1-20 antioxidant: 0.001-2 chelator.

E268. 0.81-1.49 secukinumab : 1-9 buffer system : 130-174 sugar component: 0.1-20 antioxidant: 0.001-2 chelator.

E269. 0.81-1.49 secukinumab : 51-60 buffer system : 130-174 sugar component: 0.1-20 antioxidant: 0.001-2 chelator.

E270. 0.81-1.49 secukinumab : 1-9 buffer system : 50-129 sugar component: 10-129 tonicifier : 10-129 ionic strength provider.

E271. 0.81-1.49 secukinumab : 51-60 buffer system : 50-129 sugar component: 10-129 tonicifier: 10-129 ionic strength provider.

E272. 0.81-1.49 secukinumab : 1-9 buffer system : 130-174 sugar component: 10-129 tonicifier : 10-129 ionic strength provider.

E273. 0.81-1.49 secukinumab : 51-60 buffer system : 130-174 sugar component : 10-129 tonicifier : 10-129 ionic strength provider.

E274. 0.81-1.49 secukinumab : 1-9 buffer system : 50-129 sugar component: 10-129 tonicifier: 0.001-2 chelator.

E275. 0.81-1.49 secukinumab : 51-60 buffer system : 50-129 sugar component: 10-129 tonicifier: 0.001-2 chelator.

E276. 0.81-1.49 secukinumab : 1-9 buffer system : 130-174 sugar component: 10-129 tonicifier : 0.001-2 chelator.

E277. 0.81-1.49 secukinumab : 51-60 buffer system : 130-174 sugar component: 10-129 tonicifier : 0.001-2 chelator.

E278. 0.81-1.49 secukinumab : 1-9 buffer system : 50-129 sugar component: 10-129 ionic strength provider: 0.001-2 chelator.

E279. 0.81-1.49 secukinumab : 51-60 buffer system : 50-129 sugar component: 10-129 ionic strength provider: 0.001-2 chelator.

E280. 0.81-1.49 secukinumab : 1-9 buffer system : 130-174 sugar component: 10-129 ionic strength provider: 0.001-2 chelator.

E281. 0.81-1.49 secukinumab: 51-60 buffer system : 130-174 sugar component : 10-129 ionic strength provider: 0.001-2 chelator.

E282. 0.81-1.49 secukinumab : 1-9 buffer system : 20-99 amino acid component: 0.008-0.92 surfactant: 0.1-20 antioxidant.

E283. 0.81-1.49 secukinumab : 51-60 buffer system : 20-99 amino acid component: 0.008-0.92 surfactant: 0.1-20 antioxidant.

E284. 0.81-1.49 secukinumab : 1-9 buffer system : 100-149 amino acid component: 0.008-0.92 surfactant: 0.1-20 antioxidant.

E285. 0.81-1.49 secukinumab : 51-60 buffer system : 100-149 amino acid component: 0.008-0.92 surfactant: 0.1-20 antioxidant.

E286. 0.81-1.49 secukinumab : 1-9 buffer system : 20-99 amino acid component: 0.008-0.92 surfactant: 10-129 tonicifier.

E287. 0.81-1.49 secukinumab : 51-60 buffer system : 20-99 amino acid component: 0.008-0.92 surfactant: 10-129 tonicifier.

E288. 0.81-1.49 secukinumab : 1-9 buffer system : 100-149 amino acid component: 0.008-0.92 surfactant: 10-129 tonicifier.

E289. 0.81-1.49 secukinumab : 51-60 buffer system : 100-149 amino acid component: 0.008-0.92 surfactant: 10-129 tonicifier.

E290. 0.81-1.49 secukinumab : 1-9 buffer system : 20-99 amino acid component: 0.008-0.92 surfactant: 10-129 ionic strength provider.

E291. 0.81-1.49 secukinumab : 51-60 buffer system : 20-99 amino acid component: 0.008-0.92 surfactant: 10-129 ionic strength provider.

(continued)

E292. 0.81-1.49 secukinumab : 1-9 buffer system : 100-149 amino acid component: 0.008-0.92 surfactant: 10-129 ionic strength provider.

E293. 0.81-1.49 secukinumab : 51-60 buffer system : 100-149 amino acid component : 0.008-0.92 surfactant : 10-129 ionic strength provider.

E294. 0.81-1.49 secukinumab : 1-9 buffer system : 20-99 amino acid component: 0.008-0.92 surfactant: 0.001-2 chelator.

E295. 0.81-1.49 secukinumab : 51-60 buffer system : 20-99 amino acid component: 0.008-0.92 surfactant: 0.001-2 chelator.

E296. 0.81-1.49 secukinumab : 1-9 buffer system : 100-149 amino acid component: 0.008-0.92 surfactant: 0.001-2 chelator.

E297. 0.81-1.49 secukinumab : 51-60 buffer system : 100-149 amino acid component: 0.008-0.92 surfactant: 0.001-2 chelator.

E298. 0.81-1.49 secukinumab : 1-9 buffer system : 20-99 amino acid component: 0.1-20 antioxidant: 10-129 tonicifier.

E299. 0.81-1.49 secukinumab : 51-60 buffer system : 20-99 amino acid component: 0.1-20 antioxidant: 10-129 tonicifier.

E300. 0.81-1.49 secukinumab : 1-9 buffer system : 100-149 amino acid component: 0.1-20 antioxidant: 10-129 tonicifier.

E301. 0.81-1.49 secukinumab : 51-60 buffer system : 100-149 amino acid component: 0.1-20 antioxidant: 10-129 tonicifier.

E302. 0.81-1.49 secukinumab : 1-9 buffer system : 20-99 amino acid component : 0.1-20 antioxidant : 10-129 ionic strength provider.

E303. 0.81-1.49 secukinumab : 51-60 buffer system : 20-99 amino acid component : 0.1-20 antioxidant : 10-129 ionic strength provider.

E304. 0.81-1.49 secukinumab : 1-9 buffer system : 100-149 amino acid component : 0.1-20 antioxidant : 10-129 ionic strength provider.

E305. 0.81-1.49 secukinumab : 51-60 buffer system : 100-149 amino acid component: 0.1-20 antioxidant: 10-129 ionic strength provider.

E306. 0.81-1.49 secukinumab : 1-9 buffer system : 20-99 amino acid component: 0.1-20 antioxidant: 0.001-2 chelator.

E307. 0.81-1.49 secukinumab : 51-60 buffer system : 20-99 amino acid component: 0.1-20 antioxidant: 0.001-2 chelator.

E308. 0.81-1.49 secukinumab : 1-9 buffer system : 100-149 amino acid component: 0.1-20 antioxidant: 0.001-2 chelator.

E309. 0.81-1.49 secukinumab : 51-60 buffer system : 100-149 amino acid component: 0.1-20 antioxidant: 0.001-2 chelator.

E310. 0.81-1.49 secukinumab : 1-9 buffer system : 20-99 amino acid component: 10-129 tonicifier: 10-129 ionic strength provider.

E311. 0.81-1.49 secukinumab : 51-60 buffer system : 20-99 amino acid component : 10-129 tonicifier : 10-129 ionic strength provider.

E312. 0.81-1.49 secukinumab : 1-9 buffer system : 100-149 amino acid component : 10-129 tonicifier : 10-129 ionic strength provider.

E313. 0.81-1.49 secukinumab : 51-60 buffer system : 100-149 amino acid component : 10-129 tonicifier: 10-129 ionic strength provider.

E314. 0.81-1.49 secukinumab : 1-9 buffer system : 20-99 amino acid component: 10-129 tonicifier: 0.001-2 chelator.

E315. 0.81-1.49 secukinumab : 51-60 buffer system : 20-99 amino acid component: 10-129 tonicifier : 0.001-2 chelator.

E316. 0.81-1.49 secukinumab : 1-9 buffer system : 100-149 amino acid component: 10-129 tonicifier : 0.001-2 chelator.

E317. 0.81-1.49 secukinumab : 51-60 buffer system : 100-149 amino acid component: 10-129 tonicifier : 0.001-2 chelator.

(continued)

E318. 0.81-1.49 secukinumab: 1-9 buffer system : 20-99 amino acid component: 10-129 ionic strength provider: 0.001-2 chelator.

E319. 0.81-1.49 secukinumab : 51-60 buffer system : 20-99 amino acid component : 10-129 ionic strength provider: 0.001-2 chelator.

E320. 0.81-1.49 secukinumab : 1-9 buffer system : 100-149 amino acid component : 10-129 ionic strength provider: 0.001-2 chelator.

E321. 0.81-1.49 secukinumab : 51-60 buffer system : 100-149 amino acid component: 10-129 ionic strength provider: 0.001-2 chelator.

E322. 0.81-1.49 secukinumab : 1-9 buffer system : 0.008-0.92 surfactant: 0.1-20 antioxidant: 10-129 tonicifier.

E323. 0.81-1.49 secukinumab : 51-60 buffer system : 0.008-0.92 surfactant: 0.1-20 antioxidant: 10-129 tonicifier.

E324. 0.81-1.49 secukinumab : 1-9 buffer system : 0.008-0.92 surfactant: 0.1-20 antioxidant: 10-129 ionic strength provider.

E325. 0.81-1.49 secukinumab : 51-60 buffer system : 0.008-0.92 surfactant: 0.1-20 antioxidant: 10-129 ionic strength provider.

E326. 0.81-1.49 secukinumab : 1-9 buffer system : 0.008-0.92 surfactant: 0.1-20 antioxidant: 0.001-2 chelator.

E327. 0.81-1.49 secukinumab : 51-60 buffer system : 0.008-0.92 surfactant: 0.1-20 antioxidant: 0.001-2 chelator.

E328. 0.81-1.49 secukinumab : 1-9 buffer system : 0.008-0.92 surfactant: 10-129 tonicifier : 10-129 ionic strength provider.

E329. 0.81-1.49 secukinumab : 51-60 buffer system : 0.008-0.92 surfactant: 10-129 tonicifier : 10-129 ionic strength provider.

E330. 0.81-1.49 secukinumab : 1-9 buffer system : 0.008-0.92 surfactant: 10-129 tonicifier : 0.001-2 chelator.

E331. 0.81-1.49 secukinumab : 51-60 buffer system : 0.008-0.92 surfactant: 10-129 tonicifier : 0.001-2 chelator.

E332. 0.81-1.49 secukinumab : 1-9 buffer system : 0.008-0.92 surfactant: 10-129 ionic strength provider: 0.001-2 chelator.

E333. 0.81-1.49 secukinumab : 51-60 buffer system : 0.008-0.92 surfactant: 10-129 ionic strength provider: 0.001-2 chelator.

E334. 0.81-1.49 secukinumab : 1-9 buffer system : 0.1-20 antioxidant: 10-129 tonicifier : 10-129 ionic strength provider.

E335. 0.81-1.49 secukinumab : 51-60 buffer system : 0.1-20 antioxidant: 10-129 tonicifier : 10-129 ionic strength provider.

E336. 0.81-1.49 secukinumab : 1-9 buffer system : 0.1-20 antioxidant: 10-129 tonicifier : 0.001-2 chelator.

E337. 0.81-1.49 secukinumab : 51-60 buffer system : 0.1-20 antioxidant: 10-129 tonicifier : 0.001-2 chelator.

E338. 0.81-1.49 secukinumab : 1-9 buffer system : 0.1-20 antioxidant: 10-129 ionic strength provider: 0.001-2 chelator.

E339. 0.81-1.49 secukinumab : 51-60 buffer system : 0.1-20 antioxidant: 10-129 ionic strength provider: 0.001-2 chelator.

E340. 0.81-1.49 secukinumab : 1-9 buffer system : 10-129 tonicifier : 10-129 ionic strength provider: 0.001-2 chelator.

E341. 0.81-1.49 secukinumab : 51-60 buffer system : 10-129 tonicifier : 10-129 ionic strength provider: 0.001-2 chelator.

E342. 0.81-1.49 secukinumab : 50-129 sugar component : 20-99 amino acid component : 0.008-0.92 surfactant : 0.1-20 antioxidant.

E343. 0.81-1.49 secukinumab : 130-174 sugar component : 20-99 amino acid component : 0.008-0.92 surfactant : 0.1-20 antioxidant.

E344. 0.81-1.49 secukinumab : 50-129 sugar component : 100-149 amino acid component : 0.008-0.92 surfactant : 0.1-20 antioxidant.

E345. 0.81-1.49 secukinumab : 130-174 sugar component : 100-149 amino acid component : 0.008-0.92 surfactant : 0.1-20 antioxidant.

E346. 0.81-1.49 secukinumab : 50-129 sugar component: 20-99 amino acid component: 0.008-0.92 surfactant: 10-129 tonicifier.

E347. 0.81-1.49 secukinumab : 130-174 sugar component : 20-99 amino acid component : 0.008-0.92 surfactant : 10-129 tonicifier.

(continued)

E348. 0.81-1.49 secukinumab : 50-129 sugar component : 100-149 amino acid component : 0.008-0.92 surfactant : 10-129 tonicifier.

E349. 0.81-1.49 secukinumab : 130-174 sugar component : 100-149 amino acid component : 0.008-0.92 surfactant : 10-129 tonicifier.

E350. 0.81-1.49 secukinumab : 50-129 sugar component : 20-99 amino acid component : 0.008-0.92 surfactant : 10-129 ionic strength provider.

E351. 0.81-1.49 secukinumab : 130-174 sugar component: 20-99 amino acid component: 0.008-0.92 surfactant: 10-129 ionic strength provider.

E352. 0.81-1.49 secukinumab : 50-129 sugar component: 100-149 amino acid component: 0.008-0.92 surfactant: 10-129 ionic strength provider.

E353. 0.81-1.49 secukinumab : 130-174 sugar component: 100-149 amino acid component: 0.008-0.92 surfactant: 10-129 ionic strength provider.

E354. 0.81-1.49 secukinumab : 50-129 sugar component: 20-99 amino acid component: 0.008-0.92 surfactant: 0.001-2 chelator.

E355. 0.81-1.49 secukinumab : 130-174 sugar component : 20-99 amino acid component : 0.008-0.92 surfactant : 0.001-2 chelator.

E356. 0.81-1.49 secukinumab : 50-129 sugar component : 100-149 amino acid component : 0.008-0.92 surfactant : 0.001-2 chelator.

E357. 0.81-1.49 secukinumab : 130-174 sugar component : 100-149 amino acid component : 0.008-0.92 surfactant : 0.001-2 chelator.

E358. 0.81-1.49 secukinumab : 50-129 sugar component: 20-99 amino acid component: 0.1-20 antioxidant: 10-129 tonicifier.

E359. 0.81-1.49 secukinumab : 130-174 sugar component: 20-99 amino acid component: 0.1-20 antioxidant: 10-129 tonicifier.

E360. 0.81-1.49 secukinumab : 50-129 sugar component: 100-149 amino acid component: 0.1-20 antioxidant: 10-129 tonicifier.

E361. 0.81-1.49 secukinumab : 130-174 sugar component: 100-149 amino acid component: 0.1-20 antioxidant: 10-129 tonicifier.

E362. 0.81-1.49 secukinumab: 50-129 sugar component: 20-99 amino acid component: 0.1-20 antioxidant: 10-129 ionic strength provider.

E363. 0.81-1.49 secukinumab : 130-174 sugar component : 20-99 amino acid component : 0.1-20 antioxidant : 10-129 ionic strength provider.

E364. 0.81-1.49 secukinumab : 50-129 sugar component : 100-149 amino acid component : 0.1-20 antioxidant : 10-129 ionic strength provider.

E365. 0.81-1.49 secukinumab : 130-174 sugar component : 100-149 amino acid component : 0.1-20 antioxidant : 10-129 ionic strength provider.

E366. 0.81-1.49 secukinumab : 50-129 sugar component: 20-99 amino acid component: 0.1-20 antioxidant: 0.001-2 chelator.

E367. 0.81-1.49 secukinumab : 130-174 sugar component: 20-99 amino acid component: 0.1-20 antioxidant: 0.001-2 chelator.

E368. 0.81-1.49 secukinumab : 50-129 sugar component: 100-149 amino acid component: 0.1-20 antioxidant: 0.001-2 chelator.

E369. 0.81-1.49 secukinumab : 130-174 sugar component: 100-149 amino acid component: 0.1-20 antioxidant: 0.001-2 chelator.

E370. 0.81-1.49 secukinumab : 50-129 sugar component: 20-99 amino acid component: 10-129 tonicifier: 10-129 ionic strength provider.

E371. 0.81-1.49 secukinumab : 130-174 sugar component: 20-99 amino acid component: 10-129 tonicifier: 10-129 ionic strength provider.

E372. 0.81-1.49 secukinumab : 50-129 sugar component : 100-149 amino acid component : 10-129 tonicifier : 10-129 ionic strength provider.

E373. 0.81-1.49 secukinumab : 130-174 sugar component : 100-149 amino acid component : 10-129 tonicifier : 10-129 ionic strength provider.

(continued)

E374. 0.81-1.49 secukinumab : 50-129 sugar component: 20-99 amino acid component: 10-129 tonicifier : 0.001-2 chelator.

E375. 0.81-1.49 secukinumab : 130-174 sugar component: 20-99 amino acid component: 10-129 tonicifier : 0.001-2 chelator.

E376. 0.81-1.49 secukinumab : 50-129 sugar component: 100-149 amino acid component: 10-129 tonicifier: 0.001-2 chelator.

E377. 0.81-1.49 secukinumab : 130-174 sugar component: 100-149 amino acid component: 10-129 tonicifier: 0.001-2 chelator.

E378. 0.81-1.49 secukinumab : 50-129 sugar component: 20-99 amino acid component: 10-129 ionic strength provider: 0.001-2 chelator.

E379. 0.81-1.49 secukinumab : 130-174 sugar component: 20-99 amino acid component: 10-129 ionic strength provider: 0.001-2 chelator.

E380. 0.81-1.49 secukinumab : 50-129 sugar component: 100-149 amino acid component: 10-129 ionic strength provider: 0.001-2 chelator.

E381. 0.81-1.49 secukinumab : 130-174 sugar component : 100-149 amino acid component : 10-129 ionic strength provider: 0.001-2 chelator.

E382. 0.81-1.49 secukinumab : 50-129 sugar component: 0.008-0.92 surfactant: 0.1-20 antioxidant: 10-129 tonicifier.

E383. 0.81-1.49 secukinumab : 130-174 sugar component: 0.008-0.92 surfactant: 0.1-20 antioxidant: 10-129 tonicifier.

E384. 0.81-1.49 secukinumab : 50-129 sugar component : 0.008-0.92 surfactant : 0.1-20 antioxidant : 10-129 ionic strength provider.

E385. 0.81-1.49 secukinumab : 130-174 sugar component : 0.008-0.92 surfactant : 0.1-20 antioxidant : 10-129 ionic strength provider.

E386. 0.81-1.49 secukinumab : 50-129 sugar component: 0.008-0.92 surfactant: 0.1-20 antioxidant: 0.001-2 chelator.

E387. 0.81-1.49 secukinumab : 130-174 sugar component: 0.008-0.92 surfactant: 0.1-20 antioxidant: 0.001-2 chelator.

E388. 0.81-1.49 secukinumab: 50-129 sugar component : 0.008-0.92 surfactant: 10-129 tonicifier: 10-129 ionic strength provider.

E389. 0.81-1.49 secukinumab : 130-174 sugar component : 0.008-0.92 surfactant : 10-129 tonicifier : 10-129 ionic strength provider.

E390. 0.81-1.49 secukinumab : 50-129 sugar component: 0.008-0.92 surfactant: 10-129 tonicifier : 0.001-2 chelator.

E391. 0.81-1.49 secukinumab : 130-174 sugar component: 0.008-0.92 surfactant: 10-129 tonicifier: 0.001-2 chelator.

E392. 0.81-1.49 secukinumab : 50-129 sugar component : 0.008-0.92 surfactant: 10-129 ionic strength provider: 0.001-2 chelator.

E393. 0.81-1.49 secukinumab : 130-174 sugar component : 0.008-0.92 surfactant : 10-129 ionic strength provider : 0.001-2 chelator.

E394. 0.81-1.49 secukinumab : 50-129 sugar component: 0.1-20 antioxidant: 10-129 tonicifier : 10-129 ionic strength provider.

E395. 0.81-1.49 secukinumab : 130-174 sugar component: 0.1-20 antioxidant: 10-129 tonicifier : 10-129 ionic strength provider.

E396. 0.81-1.49 secukinumab : 50-129 sugar component: 0.1-20 antioxidant: 10-129 tonicifier : 0.001-2 chelator.

E397. 0.81-1.49 secukinumab : 130-174 sugar component: 0.1-20 antioxidant: 10-129 tonicifier: 0.001-2 chelator.

E398. 0.81-1.49 secukinumab : 50-129 sugar component: 0.1-20 antioxidant: 10-129 ionic strength provider: 0.001-2 chelator.

E399. 0.81-1.49 secukinumab : 130-174 sugar component: 0.1-20 antioxidant: 10-129 ionic strength provider: 0.001-2 chelator.

E400. 0.81-1.49 secukinumab : 50-129 sugar component: 10-129 tonicifier : 10-129 ionic strength provider: 0.001-2 chelator.

(continued)

E401. 0.81-1.49 secukinumab : 130-174 sugar component: 10-129 tonicifier : 10-129 ionic strength provider: 0.001-2 chelator.

E402. 0.81-1.49 secukinumab : 20-99 amino acid component: 0.008-0.92 surfactant: 0.1-20 antioxidant: 10-129 tonicifier.

E403. 0.81-1.49 secukinumab : 100-149 amino acid component: 0.008-0.92 surfactant: 0.1-20 antioxidant: 10-129 tonicifier.

E404. 0.81-1.49 secukinumab : 20-99 amino acid component: 0.008-0.92 surfactant: 0.1-20 antioxidant : 10-129 ionic strength provider.

E405. 0.81-1.49 secukinumab : 100-149 amino acid component: 0.008-0.92 surfactant: 0.1-20 antioxidant: 10-129 ionic strength provider.

E406. 0.81-1.49 secukinumab : 20-99 amino acid component: 0.008-0.92 surfactant: 0.1-20 antioxidant: 0.001-2 chelator.

E407. 0.81-1.49 secukinumab : 100-149 amino acid component: 0.008-0.92 surfactant: 0.1-20 antioxidant: 0.001-2 chelator.

E408. 0.81-1.49 secukinumab : 20-99 amino acid component : 0.008-0.92 surfactant : 10-129 tonicifier : 10-129 ionic strength provider.

E409. 0.81-1.49 secukinumab : 100-149 amino acid component: 0.008-0.92 surfactant: 10-129 tonicifier : 10-129 ionic strength provider.

E410. 0.81-1.49 secukinumab : 20-99 amino acid component: 0.008-0.92 surfactant: 10-129 tonicifier: 0.001-2 chelator.

E411. 0.81-1.49 secukinumab : 100-149 amino acid component: 0.008-0.92 surfactant: 10-129 tonicifier : 0.001-2 chelator.

E412. 0.81-1.49 secukinumab : 20-99 amino acid component : 0.008-0.92 surfactant : 10-129 ionic strength provider: 0.001-2 chelator.

E413. 0.81-1.49 secukinumab : 100-149 amino acid component: 0.008-0.92 surfactant: 10-129 ionic strength provider: 0.001-2 chelator.

E414. 0.81-1.49 secukinumab : 20-99 amino acid component : 0.1-20 antioxidant : 10-129 tonicifier : 10-129 ionic strength provider.

E415. 0.81-1.49 secukinumab : 100-149 amino acid component : 0.1-20 antioxidant : 10-129 tonicifier : 10-129 ionic strength provider.

E416. 0.81-1.49 secukinumab : 20-99 amino acid component: 0.1-20 antioxidant: 10-129 tonicifier : 0.001-2 chelator.

E417. 0.81-1.49 secukinumab : 100-149 amino acid component: 0.1-20 antioxidant: 10-129 tonicifier : 0.001-2 chelator.

E418. 0.81-1.49 secukinumab : 20-99 amino acid component : 0.1-20 antioxidant : 10-129 ionic strength provider : 0.001-2 chelator.

E419. 0.81-1.49 secukinumab : 100-149 amino acid component : 0.1-20 antioxidant : 10-129 ionic strength provider: 0.001-2 chelator.

E420. 0.81-1.49 secukinumab : 20-99 amino acid component: 10-129 tonicifier: 10-129 ionic strength provider: 0.001-2 chelator.

E421. 0.81-1.49 secukinumab : 100-149 amino acid component : 10-129 tonicifier : 10-129 ionic strength provider : 0.001-2 chelator.

E422. 0.81-1.49 secukinumab : 0.008-0.92 surfactant: 0.1-20 antioxidant: 10-129 tonicifier: 10-129 ionic strength provider.

E423. 0.81-1.49 secukinumab : 0.008-0.92 surfactant: 0.1-20 antioxidant: 10-129 tonicifier: 0.001-2 chelator.

E424. 0.81-1.49 secukinumab : 0.008-0.92 surfactant: 0.1-20 antioxidant: 10-129 ionic strength provider: 0.001-2 chelator.

E425. 0.81-1.49 secukinumab : 0.008-0.92 surfactant: 10-129 tonicifier : 10-129 ionic strength provider: 0.001-2 chelator.

E426. 0.81-1.49 secukinumab : 0.1-20 antioxidant: 10-129 tonicifier : 10-129 ionic strength provider: 0.001-2 chelator.

(continued)

E427. 0.81-1.49 secukinumab : 1-9 buffer system : 50-129 sugar component : 20-99 amino acid component: 0.008-0.92 surfactant : 0.1-20 antioxidant.

E428. 0.81-1.49 secukinumab : 51-60 buffer system : 50-129 sugar component : 20-99 amino acid component : 0.008-0.92 surfactant: 0.1-20 antioxidant.

E429. 0.81-1.49 secukinumab : 1-9 buffer system : 130-174 sugar component : 20-99 amino acid component : 0.008-0.92 surfactant: 0.1-20 antioxidant.

E430. 0.81-1.49 secukinumab : 51-60 buffer system : 130-174 sugar component : 20-99 amino acid component : 0.008-0.92 surfactant: 0.1-20 antioxidant.

E431. 0.81-1.49 secukinumab : 1-9 buffer system : 50-129 sugar component : 100-149 amino acid component : 0.008-0.92 surfactant: 0.1-20 antioxidant.

E432. 0.81-1.49 secukinumab : 51-60 buffer system : 50-129 sugar component : 100-149 amino acid component : 0.008-0.92 surfactant: 0.1-20 antioxidant.

E433. 0.81-1.49 secukinumab : 1-9 buffer system : 130-174 sugar component : 100-149 amino acid component : 0.008-0.92 surfactant: 0.1-20 antioxidant.

E434. 0.81-1.49 secukinumab : 51-60 buffer system : 130-174 sugar component : 100-149 amino acid component : 0.008-0.92 surfactant: 0.1-20 antioxidant.

E435. 0.81-1.49 secukinumab : 1-9 buffer system : 50-129 sugar component : 20-99 amino acid component: 0.008-0.92 surfactant : 10-129 tonicifier.

E436. 0.81-1.49 secukinumab : 51-60 buffer system : 50-129 sugar component : 20-99 amino acid component : 0.008-0.92 surfactant: 10-129 tonicifier.

E437. 0.81-1.49 secukinumab : 1-9 buffer system : 130-174 sugar component : 20-99 amino acid component : 0.008-0.92 surfactant: 10-129 tonicifier.

E438. 0.81-1.49 secukinumab : 51-60 buffer system : 130-174 sugar component : 20-99 amino acid component : 0.008-0.92 surfactant: 10-129 tonicifier.

E439. 0.81-1.49 secukinumab : 1-9 buffer system : 50-129 sugar component : 100-149 amino acid component : 0.008-0.92 surfactant: 10-129 tonicifier.

E440. 0.81-1.49 secukinumab : 51-60 buffer system : 50-129 sugar component : 100-149 amino acid component : 0.008-0.92 surfactant: 10-129 tonicifier.

E441. 0.81-1.49 secukinumab : 1-9 buffer system : 130-174 sugar component : 100-149 amino acid component : 0.008-0.92 surfactant: 10-129 tonicifier.

E442. 0.81-1.49 secukinumab : 51-60 buffer system : 130-174 sugar component : 100-149 amino acid component : 0.008-0.92 surfactant: 10-129 tonicifier.

E443. 0.81-1.49 secukinumab : 1-9 buffer system : 50-129 sugar component : 20-99 amino acid component: 0.008-0.92 surfactant : 10-129 ionic strength provider.

E444. 0.81-1.49 secukinumab : 51-60 buffer system : 50-129 sugar component : 20-99 amino acid component : 0.008-0.92 surfactant: 10-129 ionic strength provider.

E445. 0.81-1.49 secukinumab : 1-9 buffer system : 130-174 sugar component : 20-99 amino acid component : 0.008-0.92 surfactant: 10-129 ionic strength provider.

E446. 0.81-1.49 secukinumab : 51-60 buffer system : 130-174 sugar component : 20-99 amino acid component : 0.008-0.92 surfactant: 10-129 ionic strength provider.

E447. 0.81-1.49 secukinumab : 1-9 buffer system : 50-129 sugar component : 100-149 amino acid component : 0.008-0.92 surfactant: 10-129 ionic strength provider.

E448. 0.81-1.49 secukinumab : 51-60 buffer system : 50-129 sugar component : 100-149 amino acid component : 0.008-0.92 surfactant: 10-129 ionic strength provider.

E449. 0.81-1.49 secukinumab : 1-9 buffer system : 130-174 sugar component : 100-149 amino acid component : 0.008-0.92 surfactant: 10-129 ionic strength provider.

E450. 0.81-1.49 secukinumab : 51-60 buffer system : 130-174 sugar component : 100-149 amino acid component : 0.008-0.92 surfactant: 10-129 ionic strength provider.

E451. 0.81-1.49 secukinumab : 1-9 buffer system : 50-129 sugar component : 20-99 amino acid component: 0.008-0.92 surfactant : 0.001-2 chelator.

E452. 0.81-1.49 secukinumab : 51-60 buffer system : 50-129 sugar component : 20-99 amino acid component : 0.008-0.92 surfactant: 0.001-2 chelator.

(continued)

E453. 0.81-1.49 secukinumab : 1-9 buffer system : 130-174 sugar component : 20-99 amino acid component : 0.008-0.92 surfactant: 0.001-2 chelator.

E454. 0.81-1.49 secukinumab : 51-60 buffer system : 130-174 sugar component : 20-99 amino acid component : 0.008-0.92 surfactant: 0.001-2 chelator.

E455. 0.81-1.49 secukinumab : 1-9 buffer system : 50-129 sugar component : 100-149 amino acid component : 0.008-0.92 surfactant: 0.001-2 chelator.

E456. 0.81-1.49 secukinumab : 51-60 buffer system : 50-129 sugar component : 100-149 amino acid component : 0.008-0.92 surfactant: 0.001-2 chelator.

E457. 0.81-1.49 secukinumab : 1-9 buffer system : 130-174 sugar component : 100-149 amino acid component : 0.008-0.92 surfactant: 0.001-2 chelator.

E458. 0.81-1.49 secukinumab : 51-60 buffer system : 130-174 sugar component : 100-149 amino acid component : 0.008-0.92 surfactant: 0.001-2 chelator.

E459. 0.81-1.49 secukinumab : 1-9 buffer system : 50-129 sugar component: 20-99 amino acid component: 0.1-20 antioxidant : 10-129 tonicifier.

E460. 0.81-1.49 secukinumab : 51-60 buffer system : 50-129 sugar component: 20-99 amino acid component: 0.1-20 antioxidant : 10-129 tonicifier.

E461. 0.81-1.49 secukinumab : 1-9 buffer system : 130-174 sugar component: 20-99 amino acid component: 0.1-20 antioxidant : 10-129 tonicifier.

E462. 0.81-1.49 secukinumab: 51-60 buffer system : 130-174 sugar component : 20-99 amino acid component: 0.1-20 antioxidant : 10-129 tonicifier.

E463. 0.81-1.49 secukinumab : 1-9 buffer system : 50-129 sugar component: 100-149 amino acid component: 0.1-20 antioxidant : 10-129 tonicifier.

E464. 0.81-1.49 secukinumab : 51-60 buffer system : 50-129 sugar component : 100-149 amino acid component : 0.1-20 antioxidant: 10-129 tonicifier.

E465. 0.81-1.49 secukinumab: 1-9 buffer system : 130-174 sugar component : 100-149 amino acid component: 0.1-20 antioxidant : 10-129 tonicifier.

E466. 0.81-1.49 secukinumab : 51-60 buffer system : 130-174 sugar component : 100-149 amino acid component : 0.1-20 antioxidant: 10-129 tonicifier.

E467. 0.81-1.49 secukinumab : 1-9 buffer system : 50-129 sugar component: 20-99 amino acid component: 0.1-20 antioxidant : 10-129 ionic strength provider.

E468. 0.81-1.49 secukinumab : 51-60 buffer system : 50-129 sugar component: 20-99 amino acid component: 0.1-20 antioxidant : 10-129 ionic strength provider.

E469. 0.81-1.49 secukinumab : 1-9 buffer system : 130-174 sugar component: 20-99 amino acid component: 0.1-20 antioxidant : 10-129 ionic strength provider.

E470. 0.81-1.49 secukinumab: 51-60 buffer system : 130-174 sugar component : 20-99 amino acid component: 0.1-20 antioxidant : 10-129 ionic strength provider.

E471. 0.81-1.49 secukinumab : 1-9 buffer system : 50-129 sugar component: 100-149 amino acid component: 0.1-20 antioxidant : 10-129 ionic strength provider.

E472. 0.81-1.49 secukinumab : 51-60 buffer system : 50-129 sugar component : 100-149 amino acid component : 0.1-20 antioxidant: 10-129 ionic strength provider.

E473. 0.81-1.49 secukinumab: 1-9 buffer system : 130-174 sugar component : 100-149 amino acid component: 0.1-20 antioxidant : 10-129 ionic strength provider.

E474. 0.81-1.49 secukinumab : 51-60 buffer system : 130-174 sugar component : 100-149 amino acid component : 0.1-20 antioxidant: 10-129 ionic strength provider.

E475. 0.81-1.49 secukinumab : 1-9 buffer system : 50-129 sugar component: 20-99 amino acid component: 0.1-20 antioxidant: 0.001-2 chelator.

E476. 0.81-1.49 secukinumab : 51-60 buffer system : 50-129 sugar component: 20-99 amino acid component: 0.1-20 antioxidant : 0.001-2 chelator.

E477. 0.81-1.49 secukinumab : 1-9 buffer system : 130-174 sugar component: 20-99 amino acid component: 0.1-20 antioxidant : 0.001-2 chelator.

E478. 0.81-1.49 secukinumab: 51-60 buffer system : 130-174 sugar component : 20-99 amino acid component: 0.1-20 antioxidant : 0.001-2 chelator.

(continued)

E479. 0.81-1.49 secukinumab : 1-9 buffer system : 50-129 sugar component: 100-149 amino acid component: 0.1-20 antioxidant : 0.001-2 chelator.

E480. 0.81-1.49 secukinumab : 51-60 buffer system : 50-129 sugar component : 100-149 amino acid component : 0.1-20 antioxidant: 0.001-2 chelator.

E481. 0.81-1.49 secukinumab: 1-9 buffer system : 130-174 sugar component : 100-149 amino acid component: 0.1-20 antioxidant : 0.001-2 chelator.

E482. 0.81-1.49 secukinumab : 51-60 buffer system : 130-174 sugar component : 100-149 amino acid component : 0.1-20 antioxidant: 0.001-2 chelator.

E483. 0.81-1.49 secukinumab : 1-9 buffer system : 50-129 sugar component: 20-99 amino acid component: 10-129 tonicifier : 10-129 ionic strength provider.

E484. 0.81-1.49 secukinumab : 51-60 buffer system : 50-129 sugar component: 20-99 amino acid component: 10-129 tonicifier : 10-129 ionic strength provider.

E485. 0.81-1.49 secukinumab : 1-9 buffer system : 130-174 sugar component: 20-99 amino acid component: 10-129 tonicifier: 10-129 ionic strength provider.

E486. 0.81-1.49 secukinumab : 51-60 buffer system : 130-174 sugar component: 20-99 amino acid component: 10-129 tonicifier : 10-129 ionic strength provider.

E487. 0.81-1.49 secukinumab : 1-9 buffer system : 50-129 sugar component: 100-149 amino acid component: 10-129 tonicifier : 10-129 ionic strength provider.

E488. 0.81-1.49 secukinumab : 51-60 buffer system : 50-129 sugar component: 100-149 amino acid component: 10-129 tonicifier : 10-129 ionic strength provider.

E489. 0.81-1.49 secukinumab : 1-9 buffer system : 130-174 sugar component: 100-149 amino acid component: 10-129 tonicifier : 10-129 ionic strength provider.

E490. 0.81-1.49 secukinumab : 51-60 buffer system : 130-174 sugar component : 100-149 amino acid component : 10-129 tonicifier : 10-129 ionic strength provider.

E491. 0.81-1.49 secukinumab : 1-9 buffer system : 50-129 sugar component: 20-99 amino acid component: 10-129 tonicifier : 0.001-2 chelator.

E492. 0.81-1.49 secukinumab : 51-60 buffer system : 50-129 sugar component: 20-99 amino acid component: 10-129 tonicifier : 0.001-2 chelator.

E493. 0.81-1.49 secukinumab : 1-9 buffer system : 130-174 sugar component: 20-99 amino acid component: 10-129 tonicifier: 0.001-2 chelator.

E494. 0.81-1.49 secukinumab : 51-60 buffer system : 130-174 sugar component: 20-99 amino acid component: 10-129 tonicifier : 0.001-2 chelator.

E495. 0.81-1.49 secukinumab : 1-9 buffer system : 50-129 sugar component: 100-149 amino acid component: 10-129 tonicifier : 0.001-2 chelator.

E496. 0.81-1.49 secukinumab : 51-60 buffer system : 50-129 sugar component: 100-149 amino acid component: 10-129 tonicifier : 0.001-2 chelator.

E497. 0.81-1.49 secukinumab : 1-9 buffer system : 130-174 sugar component: 100-149 amino acid component: 10-129 tonicifier : 0.001-2 chelator.

E498. 0.81-1.49 secukinumab : 51-60 buffer system : 130-174 sugar component : 100-149 amino acid component : 10-129 tonicifier: 0.001-2 chelator.

E499. 0.81-1.49 secukinumab : 1-9 buffer system : 50-129 sugar component: 20-99 amino acid component: 10-129 ionic strength provider: 0.001-2 chelator.

E500. 0.81-1.49 secukinumab : 51-60 buffer system : 50-129 sugar component : 20-99 amino acid component : 10-129 ionic strength provider: 0.001-2 chelator.

E501. 0.81-1.49 secukinumab : 1-9 buffer system : 130-174 sugar component : 20-99 amino acid component : 10-129 ionic strength provider: 0.001-2 chelator.

E502. 0.81-1.49 secukinumab : 51-60 buffer system : 130-174 sugar component : 20-99 amino acid component : 10-129 ionic strength provider: 0.001-2 chelator.

E503. 0.81-1.49 secukinumab : 1-9 buffer system : 50-129 sugar component : 100-149 amino acid component : 10-129 ionic strength provider: 0.001-2 chelator.

E504. 0.81-1.49 secukinumab : 51-60 buffer system : 50-129 sugar component: 100-149 amino acid component: 10-129 ionic strength provider: 0.001-2 chelator.

(continued)

E505. 0.81-1.49 secukinumab : 1-9 buffer system : 130-174 sugar component : 100-149 amino acid component : 10-129 ionic strength provider: 0.001-2 chelator.

E506. 0.81-1.49 secukinumab : 51-60 buffer system : 130-174 sugar component: 100-149 amino acid component: 10-129 ionic strength provider: 0.001-2 chelator.

E507. 0.81-1.49 secukinumab : 1-9 buffer system : 50-129 sugar component: 0.008-0.92 surfactant: 0.1-20 antioxidant: 10-129 tonicifier.

E508. 0.81-1.49 secukinumab : 51-60 buffer system : 50-129 sugar component: 0.008-0.92 surfactant: 0.1-20 antioxidant: 10-129 tonicifier.

E509. 0.81-1.49 secukinumab : 1-9 buffer system : 130-174 sugar component: 0.008-0.92 surfactant: 0.1-20 antioxidant: 10-129 tonicifier.

E510. 0.81-1.49 secukinumab : 51-60 buffer system : 130-174 sugar component: 0.008-0.92 surfactant: 0.1-20 antioxidant: 10-129 tonicifier.

E511. 0.81-1.49 secukinumab : 1-9 buffer system : 50-129 sugar component: 0.008-0.92 surfactant: 0.1-20 antioxidant: 10-129 ionic strength provider.

E512. 0.81-1.49 secukinumab : 51-60 buffer system : 50-129 sugar component: 0.008-0.92 surfactant: 0.1-20 antioxidant: 10-129 ionic strength provider.

E513. 0.81-1.49 secukinumab : 1-9 buffer system : 130-174 sugar component: 0.008-0.92 surfactant: 0.1-20 antioxidant: 10-129 ionic strength provider.

E514. 0.81-1.49 secukinumab : 51-60 buffer system : 130-174 sugar component: 0.008-0.92 surfactant: 0.1-20 antioxidant: 10-129 ionic strength provider.

E515. 0.81-1.49 secukinumab : 1-9 buffer system : 50-129 sugar component: 0.008-0.92 surfactant: 0.1-20 antioxidant: 0.001-2 chelator.

E516. 0.81-1.49 secukinumab : 51-60 buffer system : 50-129 sugar component: 0.008-0.92 surfactant: 0.1-20 antioxidant: 0.001-2 chelator.

E517. 0.81-1.49 secukinumab : 1-9 buffer system : 130-174 sugar component: 0.008-0.92 surfactant: 0.1-20 antioxidant: 0.001-2 chelator.

E518. 0.81-1.49 secukinumab : 51-60 buffer system : 130-174 sugar component : 0.008-0.92 surfactant : 0.1-20 antioxidant : 0.001-2 chelator.

E519. 0.81-1.49 secukinumab : 1-9 buffer system : 50-129 sugar component: 0.008-0.92 surfactant: 10-129 tonicifier : 10-129 ionic strength provider.

E520. 0.81-1.49 secukinumab : 51-60 buffer system : 50-129 sugar component: 0.008-0.92 surfactant: 10-129 tonicifier : 10-129 ionic strength provider.

E521. 0.81-1.49 secukinumab : 1-9 buffer system : 130-174 sugar component: 0.008-0.92 surfactant: 10-129 tonicifier : 10-129 ionic strength provider.

E522. 0.81-1.49 secukinumab : 51-60 buffer system : 130-174 sugar component : 0.008-0.92 surfactant: 10-129 tonicifier : 10-129 ionic strength provider.

E523. 0.81-1.49 secukinumab : 1-9 buffer system : 50-129 sugar component: 0.008-0.92 surfactant: 10-129 tonicifier : 0.001-2 chelator.

E524. 0.81-1.49 secukinumab : 51-60 buffer system : 50-129 sugar component: 0.008-0.92 surfactant: 10-129 tonicifier: 0.001-2 chelator.

E525. 0.81-1.49 secukinumab : 1-9 buffer system : 130-174 sugar component: 0.008-0.92 surfactant: 10-129 tonicifier : 0.001-2 chelator.

E526. 0.81-1.49 secukinumab : 51-60 buffer system : 130-174 sugar component: 0.008-0.92 surfactant: 10-129 tonicifier: 0.001-2 chelator.

E527. 0.81-1.49 secukinumab : 1-9 buffer system : 50-129 sugar component : 0.008-0.92 surfactant : 10-129 ionic strength provider: 0.001-2 chelator.

E528. 0.81-1.49 secukinumab : 51-60 buffer system : 50-129 sugar component : 0.008-0.92 surfactant : 10-129 ionic strength provider: 0.001-2 chelator.

E529. 0.81-1.49 secukinumab : 1-9 buffer system : 130-174 sugar component : 0.008-0.92 surfactant : 10-129 ionic strength provider: 0.001-2 chelator.

E530. 0.81-1.49 secukinumab : 51-60 buffer system : 130-174 sugar component : 0.008-0.92 surfactant : 10-129 ionic strength provider: 0.001-2 chelator.

(continued)

E531. 0.81-1.49 secukinumab : 1-9 buffer system : 50-129 sugar component: 0.1-20 antioxidant: 10-129 tonicifier : 10-129 ionic strength provider.

E532. 0.81-1.49 secukinumab : 51-60 buffer system : 50-129 sugar component : 0.1-20 antioxidant : 10-129 tonicifier : 10-129 ionic strength provider.

E533. 0.81-1.49 secukinumab : 1-9 buffer system : 130-174 sugar component: 0.1-20 antioxidant: 10-129 tonicifier: 10-129 ionic strength provider.

E534. 0.81-1.49 secukinumab : 51-60 buffer system : 130-174 sugar component : 0.1-20 antioxidant: 10-129 tonicifier : 10-129 ionic strength provider.

E535. 0.81-1.49 secukinumab : 1-9 buffer system : 50-129 sugar component : 0.1-20 antioxidant : 10-129 tonicifier : 0.001-2 chelator.

E536. 0.81-1.49 secukinumab : 51-60 buffer system : 50-129 sugar component : 0.1-20 antioxidant: 10-129 tonicifier : 0.001-2 chelator.

E537. 0.81-1.49 secukinumab : 1-9 buffer system : 130-174 sugar component : 0.1-20 antioxidant : 10-129 tonicifier: 0.001-2 chelator.

E538. 0.81-1.49 secukinumab : 51-60 buffer system : 130-174 sugar component: 0.1-20 antioxidant: 10-129 tonicifier : 0.001-2 chelator.

E539. 0.81-1.49 secukinumab : 1-9 buffer system : 50-129 sugar component: 0.1-20 antioxidant: 10-129 ionic strength provider : 0.001-2 chelator.

E540. 0.81-1.49 secukinumab : 51-60 buffer system : 50-129 sugar component: 0.1-20 antioxidant: 10-129 ionic strength provider : 0.001-2 chelator.

E541. 0.81-1.49 secukinumab : 1-9 buffer system : 130-174 sugar component: 0.1-20 antioxidant: 10-129 ionic strength provider : 0.001-2 chelator.

E542. 0.81-1.49 secukinumab : 51-60 buffer system : 130-174 sugar component : 0.1-20 antioxidant : 10-129 ionic strength provider: 0.001-2 chelator.

E543. 0.81-1.49 secukinumab : 1-9 buffer system : 50-129 sugar component: 10-129 tonicifier : 10-129 ionic strength provider: 0.001-2 chelator.

E544. 0.81-1.49 secukinumab : 51-60 buffer system : 50-129 sugar component: 10-129 tonicifier: 10-129 ionic strength provider : 0.001-2 chelator.

E545. 0.81-1.49 secukinumab : 1-9 buffer system : 130-174 sugar component: 10-129 tonicifier : 10-129 ionic strength provider: 0.001-2 chelator.

E546. 0.81-1.49 secukinumab : 51-60 buffer system : 130-174 sugar component: 10-129 tonicifier: 10-129 ionic strength provider : 0.001-2 chelator.

E547. 0.81-1.49 secukinumab : 1-9 buffer system : 20-99 amino acid component: 0.008-0.92 surfactant: 0.1-20 antioxidant: 10-129 tonicifier.

E548. 0.81-1.49 secukinumab : 51-60 buffer system : 20-99 amino acid component: 0.008-0.92 surfactant: 0.1-20 antioxidant : 10-129 tonicifier.

E549. 0.81-1.49 secukinumab : 1-9 buffer system : 100-149 amino acid component: 0.008-0.92 surfactant: 0.1-20 antioxidant : 10-129 tonicifier.

E550. 0.81-1.49 secukinumab : 51-60 buffer system : 100-149 amino acid component: 0.008-0.92 surfactant: 0.1-20 antioxidant : 10-129 tonicifier.

E551. 0.81-1.49 secukinumab : 1-9 buffer system : 20-99 amino acid component: 0.008-0.92 surfactant: 0.1-20 antioxidant: 10-129 ionic strength provider.

E552. 0.81-1.49 secukinumab : 51-60 buffer system : 20-99 amino acid component: 0.008-0.92 surfactant: 0.1-20 antioxidant : 10-129 ionic strength provider.

E553. 0.81-1.49 secukinumab : 1-9 buffer system : 100-149 amino acid component: 0.008-0.92 surfactant: 0.1-20 antioxidant : 10-129 ionic strength provider.

E554. 0.81-1.49 secukinumab : 51-60 buffer system : 100-149 amino acid component: 0.008-0.92 surfactant: 0.1-20 antioxidant : 10-129 ionic strength provider.

E555. 0.81-1.49 secukinumab : 1-9 buffer system : 20-99 amino acid component : 0.008-0.92 surfactant : 0.1-20 antioxidant : 0.001-2 chelator.

E556. 0.81-1.49 secukinumab : 51-60 buffer system : 20-99 amino acid component: 0.008-0.92 surfactant: 0.1-20 antioxidant: 0.001-2 chelator.

(continued)

E557. 0.81-1.49 secukinumab : 1-9 buffer system : 100-149 amino acid component: 0.008-0.92 surfactant: 0.1-20 antioxidant: 0.001-2 chelator.

E558. 0.81-1.49 secukinumab : 51-60 buffer system : 100-149 amino acid component: 0.008-0.92 surfactant: 0.1-20 antioxidant : 0.001-2 chelator.

E559. 0.81-1.49 secukinumab : 1-9 buffer system : 20-99 amino acid component: 0.008-0.92 surfactant: 10-129 tonicifier : 10-129 ionic strength provider.

E560. 0.81-1.49 secukinumab : 51-60 buffer system : 20-99 amino acid component: 0.008-0.92 surfactant: 10-129 tonicifier: 10-129 ionic strength provider.

E561. 0.81-1.49 secukinumab : 1-9 buffer system : 100-149 amino acid component: 0.008-0.92 surfactant: 10-129 tonicifier: 10-129 ionic strength provider.

E562. 0.81-1.49 secukinumab : 51-60 buffer system : 100-149 amino acid component: 0.008-0.92 surfactant: 10-129 tonicifier : 10-129 ionic strength provider.

E563. 0.81-1.49 secukinumab : 1-9 buffer system : 20-99 amino acid component: 0.008-0.92 surfactant: 10-129 tonicifier : 0.001-2 chelator.

E564. 0.81-1.49 secukinumab : 51-60 buffer system : 20-99 amino acid component : 0.008-0.92 surfactant : 10-129 tonicifier : 0.001-2 chelator.

E565. 0.81-1.49 secukinumab : 1-9 buffer system : 100-149 amino acid component : 0.008-0.92 surfactant : 10-129 tonicifier : 0.001-2 chelator.

E566. 0.81-1.49 secukinumab : 51-60 buffer system : 100-149 amino acid component: 0.008-0.92 surfactant: 10-129 tonicifier: 0.001-2 chelator.

E567. 0.81-1.49 secukinumab : 1-9 buffer system : 20-99 amino acid component: 0.008-0.92 surfactant: 10-129 ionic strength provider: 0.001-2 chelator.

E568. 0.81-1.49 secukinumab : 51-60 buffer system : 20-99 amino acid component: 0.008-0.92 surfactant: 10-129 ionic strength provider: 0.001-2 chelator.

E569. 0.81-1.49 secukinumab : 1-9 buffer system : 100-149 amino acid component: 0.008-0.92 surfactant: 10-129 ionic strength provider: 0.001-2 chelator.

E570. 0.81-1.49 secukinumab : 51-60 buffer system : 100-149 amino acid component : 0.008-0.92 surfactant : 10-129 ionic strength provider: 0.001-2 chelator.

E571. 0.81-1.49 secukinumab : 1-9 buffer system : 20-99 amino acid component: 0.1-20 antioxidant: 10-129 tonicifier : 10-129 ionic strength provider.

E572. 0.81-1.49 secukinumab : 51-60 buffer system : 20-99 amino acid component: 0.1-20 antioxidant: 10-129 tonicifier: 10-129 ionic strength provider.

E573. 0.81-1.49 secukinumab : 1-9 buffer system : 100-149 amino acid component: 0.1-20 antioxidant: 10-129 tonicifier: 10-129 ionic strength provider.

E574. 0.81-1.49 secukinumab : 51-60 buffer system : 100-149 amino acid component: 0.1-20 antioxidant: 10-129 tonicifier : 10-129 ionic strength provider.

E575. 0.81-1.49 secukinumab : 1-9 buffer system : 20-99 amino acid component: 0.1-20 antioxidant: 10-129 tonicifier : 0.001-2 chelator.

E576. 0.81-1.49 secukinumab : 51-60 buffer system : 20-99 amino acid component: 0.1-20 antioxidant: 10-129 tonicifier : 0.001-2 chelator.

E577. 0.81-1.49 secukinumab : 1-9 buffer system : 100-149 amino acid component: 0.1-20 antioxidant: 10-129 tonicifier : 0.001-2 chelator.

E578. 0.81-1.49 secukinumab : 51-60 buffer system : 100-149 amino acid component : 0.1-20 antioxidant : 10-129 tonicifier : 0.001-2 chelator.

E579. 0.81-1.49 secukinumab : 1-9 buffer system : 20-99 amino acid component : 0.1-20 antioxidant : 10-129 ionic strength provider: 0.001-2 chelator.

E580. 0.81-1.49 secukinumab : 51-60 buffer system : 20-99 amino acid component : 0.1-20 antioxidant : 10-129 ionic strength provider: 0.001-2 chelator.

E581. 0.81-1.49 secukinumab : 1-9 buffer system : 100-149 amino acid component : 0.1-20 antioxidant : 10-129 ionic strength provider: 0.001-2 chelator.

E582. 0.81-1.49 secukinumab : 51-60 buffer system : 100-149 amino acid component: 0.1-20 antioxidant: 10-129 ionic strength provider: 0.001-2 chelator.

(continued)

E583. 0.81-1.49 secukinumab : 1-9 buffer system : 20-99 amino acid component: 10-129 tonicifier: 10-129 ionic strength provider : 0.001-2 chelator.

E584. 0.81-1.49 secukinumab : 51-60 buffer system : 20-99 amino acid component : 10-129 tonicifier : 10-129 ionic strength provider: 0.001-2 chelator.

E585. 0.81-1.49 secukinumab : 1-9 buffer system : 100-149 amino acid component : 10-129 tonicifier : 10-129 ionic strength provider: 0.001-2 chelator.

E586. 0.81-1.49 secukinumab : 51-60 buffer system : 100-149 amino acid component : 10-129 tonicifier: 10-129 ionic strength provider: 0.001-2 chelator.

E587. 0.81-1.49 secukinumab : 1-9 buffer system : 0.008-0.92 surfactant : 0.1-20 antioxidant : 10-129 tonicifier : 10-129 ionic strength provider.

E588. 0.81-1.49 secukinumab : 51-60 buffer system : 0.008-0.92 surfactant: 0.1-20 antioxidant: 10-129 tonicifier : 10-129 ionic strength provider.

E589. 0.81-1.49 secukinumab : 1-9 buffer system : 0.008-0.92 surfactant: 0.1-20 antioxidant: 10-129 tonicifier : 0.001-2 chelator.

E590. 0.81-1.49 secukinumab : 51-60 buffer system : 0.008-0.92 surfactant : 0.1-20 antioxidant : 10-129 tonicifier : 0.001-2 chelator.

E591. 0.81-1.49 secukinumab : 1-9 buffer system : 0.008-0.92 surfactant : 0.1-20 antioxidant : 10-129 ionic strength provider: 0.001-2 chelator.

E592. 0.81-1.49 secukinumab : 51-60 buffer system : 0.008-0.92 surfactant: 0.1-20 antioxidant : 10-129 ionic strength provider: 0.001-2 chelator.

E593. 0.81-1.49 secukinumab : 1-9 buffer system : 0.008-0.92 surfactant: 10-129 tonicifier: 10-129 ionic strength provider: 0.001-2 chelator.

E594. 0.81-1.49 secukinumab : 51-60 buffer system : 0.008-0.92 surfactant : 10-129 tonicifier : 10-129 ionic strength provider: 0.001-2 chelator.

E595. 0.81-1.49 secukinumab : 1-9 buffer system : 0.1-20 antioxidant: 10-129 tonicifier : 10-129 ionic strength provider: 0.001-2 chelator.

E596. 0.81-1.49 secukinumab : 51-60 buffer system : 0.1-20 antioxidant: 10-129 tonicifier: 10-129 ionic strength provider: 0.001-2 chelator.

E597. 0.81-1.49 secukinumab : 50-129 sugar component : 20-99 amino acid component : 0.008-0.92 surfactant : 0.1-20 antioxidant: 10-129 tonicifier.

E598. 0.81-1.49 secukinumab : 130-174 sugar component : 20-99 amino acid component : 0.008-0.92 surfactant : 0.1-20 antioxidant: 10-129 tonicifier.

E599. 0.81-1.49 secukinumab : 50-129 sugar component : 100-149 amino acid component : 0.008-0.92 surfactant : 0.1-20 antioxidant: 10-129 tonicifier.

E600. 0.81-1.49 secukinumab : 130-174 sugar component : 100-149 amino acid component : 0.008-0.92 surfactant : 0.1-20 antioxidant: 10-129 tonicifier.

E601. 0.81-1.49 secukinumab : 50-129 sugar component : 20-99 amino acid component : 0.008-0.92 surfactant : 0.1-20 antioxidant: 10-129 ionic strength provider.

E602. 0.81-1.49 secukinumab : 130-174 sugar component : 20-99 amino acid component : 0.008-0.92 surfactant : 0.1-20 antioxidant: 10-129 ionic strength provider.

E603. 0.81-1.49 secukinumab : 50-129 sugar component : 100-149 amino acid component : 0.008-0.92 surfactant : 0.1-20 antioxidant: 10-129 ionic strength provider.

E604. 0.81-1.49 secukinumab : 130-174 sugar component : 100-149 amino acid component : 0.008-0.92 surfactant : 0.1-20 antioxidant: 10-129 ionic strength provider.

E605. 0.81-1.49 secukinumab : 50-129 sugar component : 20-99 amino acid component : 0.008-0.92 surfactant : 0.1-20 antioxidant: 0.001-2 chelator.

E606. 0.81-1.49 secukinumab : 130-174 sugar component : 20-99 amino acid component : 0.008-0.92 surfactant : 0.1-20 antioxidant: 0.001-2 chelator.

E607. 0.81-1.49 secukinumab : 50-129 sugar component : 100-149 amino acid component : 0.008-0.92 surfactant : 0.1-20 antioxidant: 0.001-2 chelator.

E608. 0.81-1.49 secukinumab : 130-174 sugar component : 100-149 amino acid component : 0.008-0.92 surfactant : 0.1-20 antioxidant: 0.001-2 chelator.

(continued)

E609. 0.81-1.49 secukinumab : 50-129 sugar component: 20-99 amino acid component: 0.008-0.92 surfactant: 10-129 tonicifier : 10-129 ionic strength provider.

E610. 0.81-1.49 secukinumab : 130-174 sugar component: 20-99 amino acid component: 0.008-0.92 surfactant: 10-129 tonicifier : 10-129 ionic strength provider.

E611. 0.81-1.49 secukinumab : 50-129 sugar component : 100-149 amino acid component : 0.008-0.92 surfactant : 10-129 tonicifier : 10-129 ionic strength provider.

E612. 0.81-1.49 secukinumab : 130-174 sugar component : 100-149 amino acid component : 0.008-0.92 surfactant : 10-129 tonicifier : 10-129 ionic strength provider.

E613. 0.81-1.49 secukinumab : 50-129 sugar component: 20-99 amino acid component: 0.008-0.92 surfactant: 10-129 tonicifier : 0.001-2 chelator.

E614. 0.81-1.49 secukinumab : 130-174 sugar component: 20-99 amino acid component: 0.008-0.92 surfactant: 10-129 tonicifier : 0.001-2 chelator.

E615. 0.81-1.49 secukinumab : 50-129 sugar component : 100-149 amino acid component : 0.008-0.92 surfactant : 10-129 tonicifier: 0.001-2 chelator.

E616. 0.81-1.49 secukinumab : 130-174 sugar component : 100-149 amino acid component : 0.008-0.92 surfactant : 10-129 tonicifier: 0.001-2 chelator.

E617. 0.81-1.49 secukinumab : 50-129 sugar component : 20-99 amino acid component : 0.008-0.92 surfactant : 10-129 ionic strength provider: 0.001-2 chelator.

E618. 0.81-1.49 secukinumab : 130-174 sugar component: 20-99 amino acid component: 0.008-0.92 surfactant: 10-129 ionic strength provider: 0.001-2 chelator.

E619. 0.81-1.49 secukinumab : 50-129 sugar component: 100-149 amino acid component: 0.008-0.92 surfactant: 10-129 ionic strength provider: 0.001-2 chelator.

E620. 0.81-1.49 secukinumab : 130-174 sugar component: 100-149 amino acid component: 0.008-0.92 surfactant: 10-129 ionic strength provider: 0.001-2 chelator.

E621. 0.81-1.49 secukinumab : 50-129 sugar component: 20-99 amino acid component: 0.1-20 antioxidant: 10-129 tonicifier : 10-129 ionic strength provider.

E622. 0.81-1.49 secukinumab : 130-174 sugar component: 20-99 amino acid component: 0.1-20 antioxidant: 10-129 tonicifier : 10-129 ionic strength provider.

E623. 0.81-1.49 secukinumab : 50-129 sugar component: 100-149 amino acid component: 0.1-20 antioxidant: 10-129 tonicifier : 10-129 ionic strength provider.

E624. 0.81-1.49 secukinumab : 130-174 sugar component: 100-149 amino acid component: 0.1-20 antioxidant: 10-129 tonicifier : 10-129 ionic strength provider.

E625. 0.81-1.49 secukinumab : 50-129 sugar component: 20-99 amino acid component: 0.1-20 antioxidant: 10-129 tonicifier : 0.001-2 chelator.

E626. 0.81-1.49 secukinumab : 130-174 sugar component: 20-99 amino acid component: 0.1-20 antioxidant: 10-129 tonicifier : 0.001-2 chelator.

E627. 0.81-1.49 secukinumab : 50-129 sugar component: 100-149 amino acid component: 0.1-20 antioxidant: 10-129 tonicifier : 0.001-2 chelator.

E628. 0.81-1.49 secukinumab : 130-174 sugar component: 100-149 amino acid component: 0.1-20 antioxidant: 10-129 tonicifier : 0.001-2 chelator.

E629. 0.81-1.49 secukinumab: 50-129 sugar component: 20-99 amino acid component: 0.1-20 antioxidant: 10-129 ionic strength provider: 0.001-2 chelator.

E630. 0.81-1.49 secukinumab : 130-174 sugar component : 20-99 amino acid component : 0.1-20 antioxidant : 10-129 ionic strength provider: 0.001-2 chelator.

E631. 0.81-1.49 secukinumab : 50-129 sugar component : 100-149 amino acid component : 0.1-20 antioxidant : 10-129 ionic strength provider: 0.001-2 chelator.

E632. 0.81-1.49 secukinumab : 130-174 sugar component : 100-149 amino acid component : 0.1-20 antioxidant : 10-129 ionic strength provider: 0.001-2 chelator.

E633. 0.81-1.49 secukinumab : 50-129 sugar component: 20-99 amino acid component: 10-129 tonicifier: 10-129 ionic strength provider: 0.001-2 chelator.

E634. 0.81-1.49 secukinumab : 130-174 sugar component: 20-99 amino acid component: 10-129 tonicifier : 10-129 ionic strength provider: 0.001-2 chelator.

(continued)

E635. 0.81-1.49 secukinumab : 50-129 sugar component : 100-149 amino acid component : 10-129 tonicifier : 10-129 ionic strength provider: 0.001-2 chelator.

E636. 0.81-1.49 secukinumab : 130-174 sugar component : 100-149 amino acid component : 10-129 tonicifier : 10-129 ionic strength provider: 0.001-2 chelator.

E637. 0.81-1.49 secukinumab : 50-129 sugar component: 0.008-0.92 surfactant: 0.1-20 antioxidant: 10-129 tonicifier : 10-129 ionic strength provider.

E638. 0.81-1.49 secukinumab : 130-174 sugar component: 0.008-0.92 surfactant: 0.1-20 antioxidant: 10-129 tonicifier : 10-129 ionic strength provider.

E639. 0.81-1.49 secukinumab : 50-129 sugar component: 0.008-0.92 surfactant: 0.1-20 antioxidant: 10-129 tonicifier : 0.001-2 chelator.

E640. 0.81-1.49 secukinumab : 130-174 sugar component: 0.008-0.92 surfactant: 0.1-20 antioxidant: 10-129 tonicifier: 0.001-2 chelator.

E641. 0.81-1.49 secukinumab : 50-129 sugar component : 0.008-0.92 surfactant : 0.1-20 antioxidant : 10-129 ionic strength provider: 0.001-2 chelator.

E642. 0.81-1.49 secukinumab : 130-174 sugar component : 0.008-0.92 surfactant : 0.1-20 antioxidant : 10-129 ionic strength provider: 0.001-2 chelator.

E643. 0.81-1.49 secukinumab : 50-129 sugar component: 0.008-0.92 surfactant: 10-129 tonicifier : 10-129 ionic strength provider : 0.001-2 chelator.

E644. 0.81-1.49 secukinumab : 130-174 sugar component : 0.008-0.92 surfactant : 10-129 tonicifier : 10-129 ionic strength provider: 0.001-2 chelator.

E645. 0.81-1.49 secukinumab : 50-129 sugar component: 0.1-20 antioxidant: 10-129 tonicifier : 10-129 ionic strength provider: 0.001-2 chelator.

E646. 0.81-1.49 secukinumab : 130-174 sugar component: 0.1-20 antioxidant: 10-129 tonicifier : 10-129 ionic strength provider : 0.001-2 chelator.

E647. 0.81-1.49 secukinumab : 20-99 amino acid component: 0.008-0.92 surfactant: 0.1-20 antioxidant: 10-129 tonicifier : 10-129 ionic strength provider.

E648. 0.81-1.49 secukinumab : 100-149 amino acid component: 0.008-0.92 surfactant: 0.1-20 antioxidant: 10-129 tonicifier: 10-129 ionic strength provider.

E649. 0.81-1.49 secukinumab : 20-99 amino acid component: 0.008-0.92 surfactant: 0.1-20 antioxidant: 10-129 tonicifier: 0.001-2 chelator.

E650. 0.81-1.49 secukinumab : 100-149 amino acid component : 0.008-0.92 surfactant : 0.1-20 antioxidant: 10-129 tonicifier: 0.001-2 chelator.

E651. 0.81-1.49 secukinumab : 20-99 amino acid component: 0.008-0.92 surfactant: 0.1-20 antioxidant : 10-129 ionic strength provider: 0.001-2 chelator.

E652. 0.81-1.49 secukinumab : 100-149 amino acid component: 0.008-0.92 surfactant: 0.1-20 antioxidant: 10-129 ionic strength provider: 0.001-2 chelator.

E653. 0.81-1.49 secukinumab : 20-99 amino acid component : 0.008-0.92 surfactant : 10-129 tonicifier : 10-129 ionic strength provider: 0.001-2 chelator.

E654. 0.81-1.49 secukinumab : 100-149 amino acid component: 0.008-0.92 surfactant: 10-129 tonicifier : 10-129 ionic strength provider: 0.001-2 chelator.

E655. 0.81-1.49 secukinumab : 20-99 amino acid component: 0.1-20 antioxidant: 10-129 tonicifier: 10-129 ionic strength provider : 0.001-2 chelator.

E656. 0.81-1.49 secukinumab : 100-149 amino acid component : 0.1-20 antioxidant : 10-129 tonicifier : 10-129 ionic strength provider: 0.001-2 chelator.

E657. 0.81-1.49 secukinumab : 0.008-0.92 surfactant : 0.1-20 antioxidant : 10-129 tonicifier : 10-129 ionic strength provider : 0.001-2 chelator.

E658. 0.81-1.49 secukinumab : 1-9 buffer system : 50-129 sugar component : 20-99 amino acid component: 0.008-0.92 surfactant : 0.1-20 antioxidant: 10-129 tonicifier.

E659. 0.81-1.49 secukinumab : 51-60 buffer system : 50-129 sugar component : 20-99 amino acid component : 0.008-0.92 surfactant: 0.1-20 antioxidant: 10-129 tonicifier.

E660. 0.81-1.49 secukinumab : 1-9 buffer system : 130-174 sugar component : 20-99 amino acid component : 0.008-0.92 surfactant: 0.1-20 antioxidant: 10-129 tonicifier.

(continued)

E661. 0.81-1.49 secukinumab : 51-60 buffer system : 130-174 sugar component : 20-99 amino acid component : 0.008-0.92 surfactant: 0.1-20 antioxidant: 10-129 tonicifier.

E662. 0.81-1.49 secukinumab : 1-9 buffer system : 50-129 sugar component : 100-149 amino acid component : 0.008-0.92 surfactant: 0.1-20 antioxidant: 10-129 tonicifier.

E663. 0.81-1.49 secukinumab : 51-60 buffer system : 50-129 sugar component : 100-149 amino acid component : 0.008-0.92 surfactant: 0.1-20 antioxidant: 10-129 tonicifier.

E664. 0.81-1.49 secukinumab : 1-9 buffer system : 130-174 sugar component : 100-149 amino acid component : 0.008-0.92 surfactant: 0.1-20 antioxidant: 10-129 tonicifier.

E665. 0.81-1.49 secukinumab : 51-60 buffer system : 130-174 sugar component : 100-149 amino acid component : 0.008-0.92 surfactant: 0.1-20 antioxidant: 10-129 tonicifier.

E666. 0.81-1.49 secukinumab : 1-9 buffer system : 50-129 sugar component : 20-99 amino acid component: 0.008-0.92 surfactant : 0.1-20 antioxidant: 10-129 ionic strength provider.

E667. 0.81-1.49 secukinumab : 51-60 buffer system : 50-129 sugar component : 20-99 amino acid component : 0.008-0.92 surfactant: 0.1-20 antioxidant: 10-129 ionic strength provider.

E668. 0.81-1.49 secukinumab : 1-9 buffer system : 130-174 sugar component : 20-99 amino acid component : 0.008-0.92 surfactant: 0.1-20 antioxidant: 10-129 ionic strength provider.

E669. 0.81-1.49 secukinumab : 51-60 buffer system : 130-174 sugar component : 20-99 amino acid component : 0.008-0.92 surfactant: 0.1-20 antioxidant: 10-129 ionic strength provider.

E670. 0.81-1.49 secukinumab : 1-9 buffer system : 50-129 sugar component : 100-149 amino acid component : 0.008-0.92 surfactant: 0.1-20 antioxidant: 10-129 ionic strength provider.

E671. 0.81-1.49 secukinumab : 51-60 buffer system : 50-129 sugar component : 100-149 amino acid component : 0.008-0.92 surfactant: 0.1-20 antioxidant: 10-129 ionic strength provider.

E672. 0.81-1.49 secukinumab : 1-9 buffer system : 130-174 sugar component : 100-149 amino acid component : 0.008-0.92 surfactant: 0.1-20 antioxidant: 10-129 ionic strength provider.

E673. 0.81-1.49 secukinumab : 51-60 buffer system : 130-174 sugar component : 100-149 amino acid component : 0.008-0.92 surfactant: 0.1-20 antioxidant: 10-129 ionic strength provider.

E674. 0.81-1.49 secukinumab : 1-9 buffer system : 50-129 sugar component : 20-99 amino acid component: 0.008-0.92 surfactant : 0.1-20 antioxidant: 0.001-2 chelator.

E675. 0.81-1.49 secukinumab : 51-60 buffer system : 50-129 sugar component : 20-99 amino acid component : 0.008-0.92 surfactant: 0.1-20 antioxidant: 0.001-2 chelator.

E676. 0.81-1.49 secukinumab : 1-9 buffer system : 130-174 sugar component : 20-99 amino acid component : 0.008-0.92 surfactant: 0.1-20 antioxidant: 0.001-2 chelator.

E677. 0.81-1.49 secukinumab : 51-60 buffer system : 130-174 sugar component : 20-99 amino acid component : 0.008-0.92 surfactant: 0.1-20 antioxidant: 0.001-2 chelator.

E678. 0.81-1.49 secukinumab : 1-9 buffer system : 50-129 sugar component : 100-149 amino acid component : 0.008-0.92 surfactant: 0.1-20 antioxidant: 0.001-2 chelator.

E679. 0.81-1.49 secukinumab : 51-60 buffer system : 50-129 sugar component : 100-149 amino acid component : 0.008-0.92 surfactant: 0.1-20 antioxidant: 0.001-2 chelator.

E680. 0.81-1.49 secukinumab : 1-9 buffer system : 130-174 sugar component : 100-149 amino acid component : 0.008-0.92 surfactant: 0.1-20 antioxidant: 0.001-2 chelator.

E681. 0.81-1.49 secukinumab : 51-60 buffer system : 130-174 sugar component : 100-149 amino acid component : 0.008-0.92 surfactant: 0.1-20 antioxidant: 0.001-2 chelator.

E682. 0.81-1.49 secukinumab : 1-9 buffer system : 50-129 sugar component : 20-99 amino acid component: 0.008-0.92 surfactant : 10-129 tonicifier : 10-129 ionic strength provider.

E683. 0.81-1.49 secukinumab : 51-60 buffer system : 50-129 sugar component : 20-99 amino acid component : 0.008-0.92 surfactant: 10-129 tonicifier : 10-129 ionic strength provider.

E684. 0.81-1.49 secukinumab : 1-9 buffer system : 130-174 sugar component : 20-99 amino acid component : 0.008-0.92 surfactant: 10-129 tonicifier : 10-129 ionic strength provider.

E685. 0.81-1.49 secukinumab : 51-60 buffer system : 130-174 sugar component : 20-99 amino acid component : 0.008-0.92 surfactant: 10-129 tonicifier : 10-129 ionic strength provider.

E686. 0.81-1.49 secukinumab : 1-9 buffer system : 50-129 sugar component : 100-149 amino acid component : 0.008-0.92 surfactant: 10-129 tonicifier : 10-129 ionic strength provider.

(continued)

E687. 0.81-1.49 secukinumab : 51-60 buffer system : 50-129 sugar component : 100-149 amino acid component : 0.008-0.92 surfactant: 10-129 tonicifier : 10-129 ionic strength provider.

E688. 0.81-1.49 secukinumab : 1-9 buffer system : 130-174 sugar component : 100-149 amino acid component : 0.008-0.92 surfactant: 10-129 tonicifier : 10-129 ionic strength provider.

E689. 0.81-1.49 secukinumab : 51-60 buffer system : 130-174 sugar component : 100-149 amino acid component : 0.008-0.92 surfactant: 10-129 tonicifier : 10-129 ionic strength provider.

E690. 0.81-1.49 secukinumab : 1-9 buffer system : 50-129 sugar component : 20-99 amino acid component : 0.008-0.92 surfactant : 10-129 tonicifier: 0.001-2 chelator.

E691. 0.81-1.49 secukinumab : 51-60 buffer system : 50-129 sugar component : 20-99 amino acid component : 0.008-0.92 surfactant: 10-129 tonicifier : 0.001-2 chelator.

E692. 0.81-1.49 secukinumab : 1-9 buffer system : 130-174 sugar component : 20-99 amino acid component : 0.008-0.92 surfactant: 10-129 tonicifier : 0.001-2 chelator.

E693. 0.81-1.49 secukinumab : 51-60 buffer system : 130-174 sugar component : 20-99 amino acid component : 0.008-0.92 surfactant: 10-129 tonicifier : 0.001-2 chelator.

E694. 0.81-1.49 secukinumab : 1-9 buffer system : 50-129 sugar component : 100-149 amino acid component : 0.008-0.92 surfactant: 10-129 tonicifier : 0.001-2 chelator.

E695. 0.81-1.49 secukinumab : 51-60 buffer system : 50-129 sugar component : 100-149 amino acid component : 0.008-0.92 surfactant: 10-129 tonicifier : 0.001-2 chelator.

E696. 0.81-1.49 secukinumab : 1-9 buffer system : 130-174 sugar component : 100-149 amino acid component : 0.008-0.92 surfactant: 10-129 tonicifier : 0.001-2 chelator.

E697. 0.81-1.49 secukinumab : 51-60 buffer system : 130-174 sugar component : 100-149 amino acid component : 0.008-0.92 surfactant: 10-129 tonicifier : 0.001-2 chelator.

E698. 0.81-1.49 secukinumab : 1-9 buffer system : 50-129 sugar component : 20-99 amino acid component: 0.008-0.92 surfactant : 10-129 ionic strength provider: 0.001-2 chelator.

E699. 0.81-1.49 secukinumab : 51-60 buffer system : 50-129 sugar component : 20-99 amino acid component : 0.008-0.92 surfactant: 10-129 ionic strength provider: 0.001-2 chelator.

E700. 0.81-1.49 secukinumab : 1-9 buffer system : 130-174 sugar component : 20-99 amino acid component : 0.008-0.92 surfactant: 10-129 ionic strength provider: 0.001-2 chelator.

E701. 0.81-1.49 secukinumab : 51-60 buffer system : 130-174 sugar component : 20-99 amino acid component : 0.008-0.92 surfactant: 10-129 ionic strength provider: 0.001-2 chelator.

E702. 0.81-1.49 secukinumab : 1-9 buffer system : 50-129 sugar component : 100-149 amino acid component : 0.008-0.92 surfactant: 10-129 ionic strength provider: 0.001-2 chelator.

E703. 0.81-1.49 secukinumab : 51-60 buffer system : 50-129 sugar component : 100-149 amino acid component : 0.008-0.92 surfactant: 10-129 ionic strength provider: 0.001-2 chelator.

E704. 0.81-1.49 secukinumab : 1-9 buffer system : 130-174 sugar component : 100-149 amino acid component : 0.008-0.92 surfactant: 10-129 ionic strength provider: 0.001-2 chelator.

E705. 0.81-1.49 secukinumab : 51-60 buffer system : 130-174 sugar component : 100-149 amino acid component : 0.008-0.92 surfactant: 10-129 ionic strength provider: 0.001-2 chelator.

E706. 0.81-1.49 secukinumab : 1-9 buffer system : 50-129 sugar component: 20-99 amino acid component: 0.1-20 antioxidant : 10-129 tonicifier : 10-129 ionic strength provider.

E707. 0.81-1.49 secukinumab : 51-60 buffer system : 50-129 sugar component: 20-99 amino acid component: 0.1-20 antioxidant : 10-129 tonicifier : 10-129 ionic strength provider.

E708. 0.81-1.49 secukinumab : 1-9 buffer system : 130-174 sugar component: 20-99 amino acid component: 0.1-20 antioxidant : 10-129 tonicifier : 10-129 ionic strength provider.

E709. 0.81-1.49 secukinumab: 51-60 buffer system : 130-174 sugar component : 20-99 amino acid component: 0.1-20 antioxidant : 10-129 tonicifier : 10-129 ionic strength provider.

E710. 0.81-1.49 secukinumab : 1-9 buffer system : 50-129 sugar component: 100-149 amino acid component: 0.1-20 antioxidant : 10-129 tonicifier : 10-129 ionic strength provider.

E711. 0.81-1.49 secukinumab : 51-60 buffer system : 50-129 sugar component : 100-149 amino acid component : 0.1-20 antioxidant: 10-129 tonicifier: 10-129 ionic strength provider.

E712. 0.81-1.49 secukinumab : 1-9 buffer system : 130-174 sugar component : 100-149 amino acid component: 0.1-20 antioxidant : 10-129 tonicifier : 10-129 ionic strength provider.

(continued)

E713. 0.81-1.49 secukinumab : 51-60 buffer system : 130-174 sugar component : 100-149 amino acid component : 0.1-20 antioxidant: 10-129 tonicifier: 10-129 ionic strength provider.

E714. 0.81-1.49 secukinumab : 1-9 buffer system : 50-129 sugar component: 20-99 amino acid component: 0.1-20 antioxidant : 10-129 tonicifier : 0.001-2 chelator.

E715. 0.81-1.49 secukinumab : 51-60 buffer system : 50-129 sugar component: 20-99 amino acid component: 0.1-20 antioxidant : 10-129 tonicifier : 0.001-2 chelator.

E716. 0.81-1.49 secukinumab : 1-9 buffer system : 130-174 sugar component: 20-99 amino acid component: 0.1-20 antioxidant : 10-129 tonicifier : 0.001-2 chelator.

E717. 0.81-1.49 secukinumab: 51-60 buffer system : 130-174 sugar component : 20-99 amino acid component: 0.1-20 antioxidant : 10-129 tonicifier : 0.001-2 chelator.

E718. 0.81-1.49 secukinumab : 1-9 buffer system : 50-129 sugar component: 100-149 amino acid component: 0.1-20 antioxidant : 10-129 tonicifier : 0.001-2 chelator.

E719. 0.81-1.49 secukinumab : 51-60 buffer system : 50-129 sugar component : 100-149 amino acid component : 0.1-20 antioxidant: 10-129 tonicifier: 0.001-2 chelator.

E720. 0.81-1.49 secukinumab: 1-9 buffer system : 130-174 sugar component : 100-149 amino acid component: 0.1-20 antioxidant : 10-129 tonicifier : 0.001 -2 chelator.

E721. 0.81-1.49 secukinumab : 51-60 buffer system : 130-174 sugar component : 100-149 amino acid component : 0.1-20 antioxidant: 10-129 tonicifier: 0.001-2 chelator.

E722. 0.81-1.49 secukinumab : 1-9 buffer system : 50-129 sugar component: 20-99 amino acid component: 0.1-20 antioxidant : 10-129 ionic strength provider: 0.001-2 chelator.

E723. 0.81-1.49 secukinumab : 51-60 buffer system : 50-129 sugar component: 20-99 amino acid component: 0.1-20 antioxidant : 10-129 ionic strength provider: 0.001-2 chelator.

E724. 0.81-1.49 secukinumab : 1-9 buffer system : 130-174 sugar component: 20-99 amino acid component: 0.1-20 antioxidant : 10-129 ionic strength provider: 0.001-2 chelator.

E725. 0.81-1.49 secukinumab: 51-60 buffer system : 130-174 sugar component : 20-99 amino acid component: 0.1-20 antioxidant : 10-129 ionic strength provider: 0.001-2 chelator.

E726. 0.81-1.49 secukinumab : 1-9 buffer system : 50-129 sugar component: 100-149 amino acid component: 0.1-20 antioxidant : 10-129 ionic strength provider: 0.001-2 chelator.

E727. 0.81-1.49 secukinumab : 51-60 buffer system : 50-129 sugar component : 100-149 amino acid component : 0.1-20 antioxidant: 10-129 ionic strength provider: 0.001-2 chelator.

E728. 0.81-1.49 secukinumab: 1-9 buffer system : 130-174 sugar component : 100-149 amino acid component: 0.1-20 antioxidant : 10-129 ionic strength provider: 0.001-2 chelator.

E729. 0.81-1.49 secukinumab : 51-60 buffer system : 130-174 sugar component : 100-149 amino acid component : 0.1-20 antioxidant: 10-129 ionic strength provider: 0.001-2 chelator.

E730. 0.81-1.49 secukinumab : 1-9 buffer system : 50-129 sugar component: 20-99 amino acid component : 10-129 tonicifier : 10-129 ionic strength provider: 0.001-2 chelator.

E731. 0.81-1.49 secukinumab : 51-60 buffer system : 50-129 sugar component: 20-99 amino acid component: 10-129 tonicifier : 10-129 ionic strength provider: 0.001-2 chelator.

E732. 0.81-1.49 secukinumab : 1-9 buffer system : 130-174 sugar component: 20-99 amino acid component: 10-129 tonicifier: 10-129 ionic strength provider: 0.001-2 chelator.

E733. 0.81-1.49 secukinumab : 51-60 buffer system : 130-174 sugar component: 20-99 amino acid component: 10-129 tonicifier : 10-129 ionic strength provider: 0.001-2 chelator.

E734. 0.81-1.49 secukinumab : 1-9 buffer system : 50-129 sugar component: 100-149 amino acid component: 10-129 tonicifier : 10-129 ionic strength provider: 0.001-2 chelator.

E735. 0.81-1.49 secukinumab : 51-60 buffer system : 50-129 sugar component: 100-149 amino acid component: 10-129 tonicifier : 10-129 ionic strength provider: 0.001-2 chelator.

E736. 0.81-1.49 secukinumab : 1-9 buffer system : 130-174 sugar component: 100-149 amino acid component: 10-129 tonicifier : 10-129 ionic strength provider: 0.001-2 chelator.

E737. 0.81-1.49 secukinumab : 51-60 buffer system : 130-174 sugar component : 100-149 amino acid component : 10-129 tonicifier : 10-129 ionic strength provider: 0.001-2 chelator.

E738. 0.81-1.49 secukinumab : 1-9 buffer system : 50-129 sugar component: 0.008-0.92 surfactant: 0.1-20 antioxidant : 10-129 tonicifier : 10-129 ionic strength provider.

(continued)

E739. 0.81-1.49 secukinumab : 51-60 buffer system : 50-129 sugar component: 0.008-0.92 surfactant: 0.1-20 antioxidant: 10-129 tonicifier : 10-129 ionic strength provider.

E740. 0.81-1.49 secukinumab : 1-9 buffer system : 130-174 sugar component: 0.008-0.92 surfactant: 0.1-20 antioxidant: 10-129 tonicifier : 10-129 ionic strength provider.

E741. 0.81-1.49 secukinumab : 51-60 buffer system : 130-174 sugar component : 0.008-0.92 surfactant: 0.1-20 antioxidant: 10-129 tonicifier : 10-129 ionic strength provider.

E742. 0.81-1.49 secukinumab : 1-9 buffer system : 50-129 sugar component: 0.008-0.92 surfactant: 0.1-20 antioxidant: 10-129 tonicifier: 0.001-2 chelator.

E743. 0.81-1.49 secukinumab : 51-60 buffer system : 50-129 sugar component: 0.008-0.92 surfactant: 0.1-20 antioxidant: 10-129 tonicifier : 0.001-2 chelator.

E744. 0.81-1.49 secukinumab : 1-9 buffer system : 130-174 sugar component: 0.008-0.92 surfactant: 0.1-20 antioxidant: 10-129 tonicifier: 0.001-2 chelator.

E745. 0.81-1.49 secukinumab : 51-60 buffer system : 130-174 sugar component: 0.008-0.92 surfactant: 0.1-20 antioxidant: 10-129 tonicifier : 0.001-2 chelator.

E746. 0.81-1.49 secukinumab : 1-9 buffer system : 50-129 sugar component: 0.008-0.92 surfactant: 0.1-20 antioxidant: 10-129 ionic strength provider: 0.001-2 chelator.

E747. 0.81-1.49 secukinumab : 51-60 buffer system : 50-129 sugar component: 0.008-0.92 surfactant: 0.1-20 antioxidant: 10-129 ionic strength provider: 0.001-2 chelator.

E748. 0.81-1.49 secukinumab : 1-9 buffer system : 130-174 sugar component: 0.008-0.92 surfactant: 0.1-20 antioxidant: 10-129 ionic strength provider: 0.001-2 chelator.

E749. 0.81-1.49 secukinumab : 51-60 buffer system : 130-174 sugar component: 0.008-0.92 surfactant: 0.1-20 antioxidant: 10-129 ionic strength provider: 0.001-2 chelator.

E750. 0.81-1.49 secukinumab : 1-9 buffer system : 50-129 sugar component: 0.008-0.92 surfactant: 10-129 tonicifier : 10-129 ionic strength provider: 0.001-2 chelator.

E751. 0.81-1.49 secukinumab : 51-60 buffer system : 50-129 sugar component: 0.008-0.92 surfactant: 10-129 tonicifier : 10-129 ionic strength provider: 0.001-2 chelator.

E752. 0.81-1.49 secukinumab : 1-9 buffer system : 130-174 sugar component: 0.008-0.92 surfactant: 10-129 tonicifier : 10-129 ionic strength provider: 0.001-2 chelator.

E753. 0.81-1.49 secukinumab : 51-60 buffer system : 130-174 sugar component : 0.008-0.92 surfactant: 10-129 tonicifier : 10-129 ionic strength provider: 0.001-2 chelator.

E754. 0.81-1.49 secukinumab : 1-9 buffer system : 50-129 sugar component: 0.1-20 antioxidant: 10-129 tonicifier : 10-129 ionic strength provider: 0.001-2 chelator.

E755. 0.81-1.49 secukinumab : 51-60 buffer system : 50-129 sugar component : 0.1-20 antioxidant : 10-129 tonicifier : 10-129 ionic strength provider: 0.001-2 chelator.

E756. 0.81-1.49 secukinumab : 1-9 buffer system : 130-174 sugar component: 0.1-20 antioxidant: 10-129 tonicifier: 10-129 ionic strength provider: 0.001-2 chelator.

E757. 0.81-1.49 secukinumab : 51-60 buffer system : 130-174 sugar component : 0.1-20 antioxidant: 10-129 tonicifier : 10-129 ionic strength provider: 0.001-2 chelator.

E758. 0.81-1.49 secukinumab : 1-9 buffer system : 20-99 amino acid component: 0.008-0.92 surfactant: 0.1-20 antioxidant: 10-129 tonicifier : 10-129 ionic strength provider.

E759. 0.81-1.49 secukinumab : 51-60 buffer system : 20-99 amino acid component: 0.008-0.92 surfactant: 0.1-20 antioxidant : 10-129 tonicifier : 10-129 ionic strength provider.

E760. 0.81-1.49 secukinumab : 1-9 buffer system : 100-149 amino acid component: 0.008-0.92 surfactant: 0.1-20 antioxidant : 10-129 tonicifier : 10-129 ionic strength provider.

E761. 0.81-1.49 secukinumab : 51-60 buffer system : 100-149 amino acid component: 0.008-0.92 surfactant: 0.1-20 antioxidant : 10-129 tonicifier : 10-129 ionic strength provider.

E762. 0.81-1.49 secukinumab : 1-9 buffer system : 20-99 amino acid component: 0.008-0.92 surfactant: 0.1-20 antioxidant: 10-129 tonicifier : 0.001-2 chelator.

E763. 0.81-1.49 secukinumab : 51-60 buffer system : 20-99 amino acid component: 0.008-0.92 surfactant: 0.1-20 antioxidant : 10-129 tonicifier : 0.001-2 chelator.

E764. 0.81-1.49 secukinumab : 1-9 buffer system : 100-149 amino acid component: 0.008-0.92 surfactant: 0.1-20 antioxidant : 10-129 tonicifier : 0.001-2 chelator.

(continued)

E765. 0.81-1.49 secukinumab : 51-60 buffer system : 100-149 amino acid component: 0.008-0.92 surfactant: 0.1-20 antioxidant : 10-129 tonicifier : 0.001-2 chelator.

E766. 0.81-1.49 secukinumab : 1-9 buffer system : 20-99 amino acid component: 0.008-0.92 surfactant: 0.1-20 antioxidant: 10-129 ionic strength provider: 0.001-2 chelator.

E767. 0.81-1.49 secukinumab : 51-60 buffer system : 20-99 amino acid component: 0.008-0.92 surfactant: 0.1-20 antioxidant : 10-129 ionic strength provider: 0.001-2 chelator.

E768. 0.81-1.49 secukinumab : 1-9 buffer system : 100-149 amino acid component: 0.008-0.92 surfactant: 0.1-20 antioxidant : 10-129 ionic strength provider: 0.001-2 chelator.

E769. 0.81-1.49 secukinumab : 51-60 buffer system : 100-149 amino acid component: 0.008-0.92 surfactant: 0.1-20 antioxidant : 10-129 ionic strength provider: 0.001-2 chelator.

E770. 0.81-1.49 secukinumab : 1-9 buffer system : 20-99 amino acid component: 0.008-0.92 surfactant: 10-129 tonicifier : 10-129 ionic strength provider: 0.001-2 chelator.

E771. 0.81-1.49 secukinumab : 51-60 buffer system : 20-99 amino acid component: 0.008-0.92 surfactant: 10-129 tonicifier: 10-129 ionic strength provider: 0.001-2 chelator.

E772. 0.81-1.49 secukinumab : 1-9 buffer system : 100-149 amino acid component: 0.008-0.92 surfactant: 10-129 tonicifier: 10-129 ionic strength provider: 0.001-2 chelator.

E773. 0.81-1.49 secukinumab : 51-60 buffer system : 100-149 amino acid component: 0.008-0.92 surfactant: 10-129 tonicifier: 10-129 ionic strength provider: 0.001-2 chelator.

E774. 0.81-1.49 secukinumab : 1-9 buffer system : 20-99 amino acid component: 0.1-20 antioxidant: 10-129 tonicifier : 10-129 ionic strength provider: 0.001-2 chelator.

E775. 0.81-1.49 secukinumab : 51-60 buffer system : 20-99 amino acid component: 0.1-20 antioxidant: 10-129 tonicifier: 10-129 ionic strength provider: 0.001-2 chelator.

E776. 0.81-1.49 secukinumab : 1-9 buffer system : 100-149 amino acid component: 0.1-20 antioxidant: 10-129 tonicifier: 10-129 ionic strength provider: 0.001-2 chelator.

E777. 0.81-1.49 secukinumab : 51-60 buffer system : 100-149 amino acid component: 0.1-20 antioxidant: 10-129 tonicifier: 10-129 ionic strength provider: 0.001-2 chelator.

E778. 0.81-1.49 secukinumab : 1-9 buffer system : 0.008-0.92 surfactant : 0.1-20 antioxidant : 10-129 tonicifier : 10-129 ionic strength provider: 0.001-2 chelator.

E779. 0.81-1.49 secukinumab : 51-60 buffer system : 0.008-0.92 surfactant: 0.1-20 antioxidant: 10-129 tonicifier : 10-129 ionic strength provider: 0.001-2 chelator.

E780. 0.81-1.49 secukinumab : 50-129 sugar component : 20-99 amino acid component : 0.008-0.92 surfactant : 0.1-20 antioxidant: 10-129 tonicifier : 10-129 ionic strength provider.

E781. 0.81-1.49 secukinumab : 130-174 sugar component : 20-99 amino acid component : 0.008-0.92 surfactant : 0.1-20 antioxidant: 10-129 tonicifier : 10-129 ionic strength provider.

E782. 0.81-1.49 secukinumab : 50-129 sugar component : 100-149 amino acid component : 0.008-0.92 surfactant : 0.1-20 antioxidant: 10-129 tonicifier : 10-129 ionic strength provider.

E783. 0.81-1.49 secukinumab : 130-174 sugar component : 100-149 amino acid component : 0.008-0.92 surfactant : 0.1-20 antioxidant: 10-129 tonicifier : 10-129 ionic strength provider.

E784. 0.81-1.49 secukinumab : 50-129 sugar component : 20-99 amino acid component : 0.008-0.92 surfactant : 0.1-20 antioxidant: 10-129 tonicifier : 0.001-2 chelator.

E785. 0.81-1.49 secukinumab : 130-174 sugar component : 20-99 amino acid component : 0.008-0.92 surfactant : 0.1-20 antioxidant: 10-129 tonicifier : 0.001-2 chelator.

E786. 0.81-1.49 secukinumab : 50-129 sugar component : 100-149 amino acid component : 0.008-0.92 surfactant : 0.1-20 antioxidant: 10-129 tonicifier : 0.001-2 chelator.

E787. 0.81-1.49 secukinumab : 130-174 sugar component : 100-149 amino acid component : 0.008-0.92 surfactant : 0.1-20 antioxidant: 10-129 tonicifier : 0.001-2 chelator.

E788. 0.81-1.49 secukinumab : 50-129 sugar component : 20-99 amino acid component : 0.008-0.92 surfactant : 0.1-20 antioxidant: 10-129 ionic strength provider: 0.001-2 chelator.

E789. 0.81-1.49 secukinumab : 130-174 sugar component : 20-99 amino acid component : 0.008-0.92 surfactant : 0.1-20 antioxidant: 10-129 ionic strength provider: 0.001-2 chelator.

E790. 0.81-1.49 secukinumab : 50-129 sugar component : 100-149 amino acid component : 0.008-0.92 surfactant : 0.1-20 antioxidant: 10-129 ionic strength provider: 0.001-2 chelator.

(continued)

E791. 0.81-1.49 secukinumab : 130-174 sugar component : 100-149 amino acid component : 0.008-0.92 surfactant : 0.1-20 antioxidant: 10-129 ionic strength provider: 0.001-2 chelator.

E792. 0.81-1.49 secukinumab : 50-129 sugar component: 20-99 amino acid component: 0.008-0.92 surfactant: 10-129 tonicifier : 10-129 ionic strength provider: 0.001-2 chelator.

E793. 0.81-1.49 secukinumab : 130-174 sugar component: 20-99 amino acid component: 0.008-0.92 surfactant: 10-129 tonicifier : 10-129 ionic strength provider: 0.001-2 chelator.

E794. 0.81-1.49 secukinumab : 50-129 sugar component : 100-149 amino acid component : 0.008-0.92 surfactant : 10-129 tonicifier: 10-129 ionic strength provider: 0.001-2 chelator.

E795. 0.81-1.49 secukinumab : 130-174 sugar component : 100-149 amino acid component : 0.008-0.92 surfactant : 10-129 tonicifier: 10-129 ionic strength provider: 0.001-2 chelator.

E796. 0.81-1.49 secukinumab : 50-129 sugar component: 20-99 amino acid component: 0.1-20 antioxidant: 10-129 tonicifier : 10-129 ionic strength provider: 0.001-2 chelator.

E797. 0.81-1.49 secukinumab : 130-174 sugar component: 20-99 amino acid component: 0.1-20 antioxidant: 10-129 tonicifier : 10-129 ionic strength provider: 0.001-2 chelator.

E798. 0.81-1.49 secukinumab : 50-129 sugar component: 100-149 amino acid component: 0.1-20 antioxidant: 10-129 tonicifier : 10-129 ionic strength provider: 0.001-2 chelator.

E799. 0.81-1.49 secukinumab : 130-174 sugar component: 100-149 amino acid component: 0.1-20 antioxidant: 10-129 tonicifier : 10-129 ionic strength provider: 0.001-2 chelator.

E800. 0.81-1.49 secukinumab : 50-129 sugar component: 0.008-0.92 surfactant: 0.1-20 antioxidant: 10-129 tonicifier : 10-129 ionic strength provider: 0.001-2 chelator.

E801. 0.81-1 .49 secukinumab : 130-174 sugar component: 0.008-0.92 surfactant: 0.1-20 antioxidant: 10-129 tonicifier : 10-129 ionic strength provider: 0.001-2 chelator.

E802. 0.81-1.49 secukinumab : 20-99 amino acid component: 0.008-0.92 surfactant: 0.1-20 antioxidant: 10-129 tonicifier : 10-129 ionic strength provider: 0.001-2 chelator.

E803. 0.81-1.49 secukinumab : 100-149 amino acid component: 0.008-0.92 surfactant: 0.1-20 antioxidant: 10-129 tonicifier: 10-129 ionic strength provider: 0.001-2 chelator.

E804. 0.81-1.49 secukinumab : 1-9 buffer system : 50-129 sugar component : 20-99 amino acid component: 0.008-0.92 surfactant : 0.1-20 antioxidant: 10-129 tonicifier : 10-129 ionic strength provider.

E805. 0.81-1.49 secukinumab : 51-60 buffer system : 50-129 sugar component : 20-99 amino acid component : 0.008-0.92 surfactant: 0.1-20 antioxidant: 10-129 tonicifier : 10-129 ionic strength provider.

E806. 0.81-1.49 secukinumab : 1-9 buffer system : 130-174 sugar component : 20-99 amino acid component : 0.008-0.92 surfactant: 0.1-20 antioxidant: 10-129 tonicifier : 10-129 ionic strength provider.

E807. 0.81-1.49 secukinumab : 51-60 buffer system : 130-174 sugar component : 20-99 amino acid component : 0.008-0.92 surfactant: 0.1-20 antioxidant: 10-129 tonicifier : 10-129 ionic strength provider.

E808. 0.81-1.49 secukinumab : 1-9 buffer system : 50-129 sugar component : 100-149 amino acid component : 0.008-0.92 surfactant: 0.1-20 antioxidant: 10-129 tonicifier : 10-129 ionic strength provider.

E809. 0.81-1.49 secukinumab : 51-60 buffer system : 50-129 sugar component : 100-149 amino acid component : 0.008-0.92 surfactant: 0.1-20 antioxidant: 10-129 tonicifier : 10-129 ionic strength provider.

E810. 0.81-1.49 secukinumab : 1-9 buffer system : 130-174 sugar component : 100-149 amino acid component : 0.008-0.92 surfactant: 0.1-20 antioxidant: 10-129 tonicifier : 10-129 ionic strength provider.

E811. 0.81-1.49 secukinumab : 51-60 buffer system : 130-174 sugar component : 100-149 amino acid component : 0.008-0.92 surfactant: 0.1-20 antioxidant: 10-129 tonicifier : 10-129 ionic strength provider.

E812. 0.81-1.49 secukinumab : 1-9 buffer system : 50-129 sugar component : 20-99 amino acid component: 0.008-0.92 surfactant : 0.1-20 antioxidant: 10-129 tonicifier : 0.001-2 chelator.

E813. 0.81-1.49 secukinumab : 51-60 buffer system : 50-129 sugar component : 20-99 amino acid component : 0.008-0.92 surfactant: 0.1-20 antioxidant: 10-129 tonicifier : 0.001-2 chelator.

E814. 0.81-1.49 secukinumab : 1-9 buffer system : 130-174 sugar component : 20-99 amino acid component : 0.008-0.92 surfactant: 0.1-20 antioxidant: 10-129 tonicifier : 0.001-2 chelator.

E815. 0.81-1.49 secukinumab : 51-60 buffer system : 130-174 sugar component : 20-99 amino acid component : 0.008-0.92 surfactant: 0.1-20 antioxidant: 10-129 tonicifier : 0.001-2 chelator.

E816. 0.81-1.49 secukinumab : 1-9 buffer system : 50-129 sugar component : 100-149 amino acid component : 0.008-0.92 surfactant: 0.1-20 antioxidant: 10-129 tonicifier : 0.001-2 chelator.

(continued)

E817. 0.81-1.49 secukinumab : 51-60 buffer system : 50-129 sugar component : 100-149 amino acid component : 0.008-0.92 surfactant: 0.1-20 antioxidant: 10-129 tonicifier : 0.001-2 chelator.

E818. 0.81-1.49 secukinumab : 1-9 buffer system : 130-174 sugar component : 100-149 amino acid component : 0.008-0.92 surfactant: 0.1-20 antioxidant: 10-129 tonicifier : 0.001-2 chelator.

E819. 0.81-1.49 secukinumab : 51-60 buffer system : 130-174 sugar component : 100-149 amino acid component : 0.008-0.92 surfactant: 0.1-20 antioxidant: 10-129 tonicifier : 0.001-2 chelator.

E820. 0.81-1.49 secukinumab : 1-9 buffer system : 50-129 sugar component : 20-99 amino acid component: 0.008-0.92 surfactant : 0.1-20 antioxidant: 10-129 ionic strength provider: 0.001-2 chelator.

E821. 0.81-1.49 secukinumab : 51-60 buffer system : 50-129 sugar component : 20-99 amino acid component : 0.008-0.92 surfactant: 0.1-20 antioxidant: 10-129 ionic strength provider: 0.001-2 chelator.

E822. 0.81-1.49 secukinumab : 1-9 buffer system : 130-174 sugar component : 20-99 amino acid component : 0.008-0.92 surfactant: 0.1-20 antioxidant: 10-129 ionic strength provider: 0.001-2 chelator.

E823. 0.81-1.49 secukinumab : 51-60 buffer system : 130-174 sugar component : 20-99 amino acid component : 0.008-0.92 surfactant: 0.1-20 antioxidant: 10-129 ionic strength provider: 0.001-2 chelator.

E824. 0.81-1.49 secukinumab : 1-9 buffer system : 50-129 sugar component : 100-149 amino acid component : 0.008-0.92 surfactant: 0.1-20 antioxidant: 10-129 ionic strength provider: 0.001-2 chelator.

E825. 0.81-1.49 secukinumab : 51-60 buffer system : 50-129 sugar component : 100-149 amino acid component : 0.008-0.92 surfactant: 0.1-20 antioxidant: 10-129 ionic strength provider: 0.001-2 chelator.

E826. 0.81-1.49 secukinumab : 1-9 buffer system : 130-174 sugar component : 100-149 amino acid component : 0.008-0.92 surfactant: 0.1-20 antioxidant: 10-129 ionic strength provider: 0.001-2 chelator.

E827. 0.81-1.49 secukinumab : 51-60 buffer system : 130-174 sugar component : 100-149 amino acid component : 0.008-0.92 surfactant: 0.1-20 antioxidant: 10-129 ionic strength provider: 0.001-2 chelator.

E828. 0.81-1.49 secukinumab : 1-9 buffer system : 50-129 sugar component : 20-99 amino acid component: 0.008-0.92 surfactant : 10-129 tonicifier : 10-129 ionic strength provider: 0.001-2 chelator.

E829. 0.81-1.49 secukinumab : 51-60 buffer system : 50-129 sugar component : 20-99 amino acid component : 0.008-0.92 surfactant: 10-129 tonicifier : 10-129 ionic strength provider: 0.001-2 chelator.

E830. 0.81-1.49 secukinumab : 1-9 buffer system : 130-174 sugar component : 20-99 amino acid component : 0.008-0.92 surfactant: 10-129 tonicifier : 10-129 ionic strength provider: 0.001-2 chelator.

E831. 0.81-1.49 secukinumab : 51-60 buffer system : 130-174 sugar component : 20-99 amino acid component : 0.008-0.92 surfactant: 10-129 tonicifier : 10-129 ionic strength provider: 0.001-2 chelator.

E832. 0.81-1.49 secukinumab : 1-9 buffer system : 50-129 sugar component : 100-149 amino acid component : 0.008-0.92 surfactant: 10-129 tonicifier : 10-129 ionic strength provider: 0.001-2 chelator.

E833. 0.81-1.49 secukinumab : 51-60 buffer system : 50-129 sugar component : 100-149 amino acid component : 0.008-0.92 surfactant: 10-129 tonicifier : 10-129 ionic strength provider: 0.001-2 chelator.

E834. 0.81-1.49 secukinumab : 1-9 buffer system : 130-174 sugar component : 100-149 amino acid component : 0.008-0.92 surfactant: 10-129 tonicifier : 10-129 ionic strength provider: 0.001-2 chelator.

E835. 0.81-1.49 secukinumab : 51-60 buffer system : 130-174 sugar component : 100-149 amino acid component : 0.008-0.92 surfactant: 10-129 tonicifier : 10-129 ionic strength provider: 0.001-2 chelator.

E836. 0.81-1.49 secukinumab : 1-9 buffer system : 50-129 sugar component: 20-99 amino acid component: 0.1-20 antioxidant : 10-129 tonicifier : 10-129 ionic strength provider: 0.001-2 chelator.

E837. 0.81-1.49 secukinumab : 51-60 buffer system : 50-129 sugar component: 20-99 amino acid component: 0.1-20 antioxidant : 10-129 tonicifier : 10-129 ionic strength provider: 0.001-2 chelator.

E838. 0.81-1.49 secukinumab : 1-9 buffer system : 130-174 sugar component: 20-99 amino acid component: 0.1-20 antioxidant : 10-129 tonicifier : 10-129 ionic strength provider: 0.001-2 chelator.

E839. 0.81-1.49 secukinumab: 51-60 buffer system : 130-174 sugar component : 20-99 amino acid component: 0.1-20 antioxidant : 10-129 tonicifier : 10-129 ionic strength provider: 0.001-2 chelator.

E840. 0.81-1.49 secukinumab : 1-9 buffer system : 50-129 sugar component: 100-149 amino acid component: 0.1-20 antioxidant : 10-129 tonicifier : 10-129 ionic strength provider: 0.001-2 chelator.

E841. 0.81-1.49 secukinumab : 51-60 buffer system : 50-129 sugar component : 100-149 amino acid component : 0.1-20 antioxidant: 10-129 tonicifier: 10-129 ionic strength provider: 0.001-2 chelator.

E842. 0.81-1.49 secukinumab: 1-9 buffer system : 130-174 sugar component : 100-149 amino acid component: 0.1-20 antioxidant : 10-129 tonicifier: 10-129 ionic strength provider: 0.001-2 chelator.

(continued)

E843. 0.81-1.49 secukinumab : 51-60 buffer system : 130-174 sugar component : 100-149 amino acid component : 0.1-20 antioxidant: 10-129 tonicifier: 10-129 ionic strength provider: 0.001-2 chelator.

E844. 0.81-1.49 secukinumab : 1-9 buffer system : 50-129 sugar component: 0.008-0.92 surfactant: 0.1-20 antioxidant: 10-129 tonicifier: 10-129 ionic strength provider: 0.001-2 chelator.

E845. 0.81-1.49 secukinumab : 51-60 buffer system : 50-129 sugar component: 0.008-0.92 surfactant: 0.1-20 antioxidant: 10-129 tonicifier : 10-129 ionic strength provider: 0.001-2 chelator.

E846. 0.81-1.49 secukinumab : 1-9 buffer system : 130-174 sugar component: 0.008-0.92 surfactant: 0.1-20 antioxidant: 10-129 tonicifier: 10-129 ionic strength provider: 0.001-2 chelator.

E847. 0.81-1.49 secukinumab : 51-60 buffer system : 130-174 sugar component: 0.008-0.92 surfactant: 0.1-20 antioxidant: 10-129 tonicifier : 10-129 ionic strength provider: 0.001-2 chelator.

E848. 0.81-1.49 secukinumab : 1-9 buffer system : 20-99 amino acid component: 0.008-0.92 surfactant: 0.1-20 antioxidant: 10-129 tonicifier : 10-129 ionic strength provider: 0.001-2 chelator.

E849. 0.81-1.49 secukinumab : 51-60 buffer system : 20-99 amino acid component: 0.008-0.92 surfactant: 0.1-20 antioxidant: 10-129 tonicifier : 10-129 ionic strength provider: 0.001-2 chelator.

E850. 0.81-1.49 secukinumab : 1-9 buffer system : 100-149 amino acid component: 0.008-0.92 surfactant: 0.1-20 antioxidant: 10-129 tonicifier : 10-129 ionic strength provider: 0.001-2 chelator.

E851. 0.81-1.49 secukinumab : 51-60 buffer system : 100-149 amino acid component: 0.008-0.92 surfactant: 0.1-20 antioxidant : 10-129 tonicifier: 10-129 ionic strength provider: 0.001-2 chelator.

E852. 0.81-1.49 secukinumab : 50-129 sugar component : 20-99 amino acid component : 0.008-0.92 surfactant : 0.1-20 antioxidant: 10-129 tonicifier: 10-129 ionic strength provider: 0.001-2 chelator.

E853. 0.81-1.49 secukinumab : 130-174 sugar component : 20-99 amino acid component : 0.008-0.92 surfactant : 0.1-20 antioxidant: 10-129 tonicifier: 10-129 ionic strength provider: 0.001-2 chelator.

E854. 0.81-1.49 secukinumab : 50-129 sugar component : 100-149 amino acid component : 0.008-0.92 surfactant : 0.1-20 antioxidant: 10-129 tonicifier: 10-129 ionic strength provider: 0.001-2 chelator.

E855. 0.81-1.49 secukinumab : 130-174 sugar component : 100-149 amino acid component : 0.008-0.92 surfactant : 0.1-20 antioxidant: 10-129 tonicifier: 10-129 ionic strength provider: 0.001-2 chelator.

E856. 0.81-1.49 secukinumab : 1-9 buffer system : 50-129 sugar component : 20-99 amino acid component: 0.008-0.92 surfactant : 0.1-20 antioxidant: 10-129 tonicifier : 10-129 ionic strength provider: 0.001-2 chelator.

E857. 0.81-1.49 secukinumab : 51-60 buffer system : 50-129 sugar component : 20-99 amino acid component : 0.008-0.92 surfactant: 0.1-20 antioxidant: 10-129 tonicifier : 10-129 ionic strength provider: 0.001-2 chelator.

E858. 0.81-1.49 secukinumab : 1-9 buffer system : 130-174 sugar component : 20-99 amino acid component : 0.008-0.92 surfactant: 0.1-20 antioxidant: 10-129 tonicifier : 10-129 ionic strength provider: 0.001-2 chelator.

E859. 0.81-1.49 secukinumab : 51-60 buffer system : 130-174 sugar component : 20-99 amino acid component : 0.008-0.92 surfactant: 0.1-20 antioxidant: 10-129 tonicifier : 10-129 ionic strength provider: 0.001-2 chelator.

E860. 0.81-1.49 secukinumab : 1-9 buffer system : 50-129 sugar component : 100-149 amino acid component : 0.008-0.92 surfactant: 0.1-20 antioxidant: 10-129 tonicifier : 10-129 ionic strength provider: 0.001-2 chelator.

E861. 0.81-1.49 secukinumab : 51-60 buffer system : 50-129 sugar component : 100-149 amino acid component : 0.008-0.92 surfactant: 0.1-20 antioxidant: 10-129 tonicifier : 10-129 ionic strength provider: 0.001-2 chelator.

E862. 0.81-1.49 secukinumab : 1-9 buffer system : 130-174 sugar component : 100-149 amino acid component : 0.008-0.92 surfactant: 0.1-20 antioxidant: 10-129 tonicifier : 10-129 ionic strength provider: 0.001-2 chelator.

E863. 0.81-1.49 secukinumab : 51-60 buffer system : 130-174 sugar component : 100-149 amino acid component : 0.008-0.92 surfactant: 0.1-20 antioxidant: 10-129 tonicifier : 10-129 ionic strength provider: 0.001-2 chelator.

E864. 0.81-1.49 secukinumab : 200-250 sugar component.

E865. 0.81-1.49 secukinumab : 1-9 buffer system : 200-250 sugar component.

E866. 0.81-1.49 secukinumab : 51-60 buffer system : 200-250 sugar component.

(continued)

E867. 0.81-1.49 secukinumab : 200-250 sugar component: 20-99 amino acid component.
E868. 0.81-1.49 secukinumab : 200-250 sugar component: 100-149 amino acid component.
E869. 0.81-1.49 secukinumab : 200-250 sugar component: 0.008-0.92 surfactant.
E870. 0.81-1.49 secukinumab : 200-250 sugar component: 0.1-20 antioxidant.
E871. 0.81-1.49 secukinumab : 200-250 sugar component: 10-129 tonicifier.
E872. 0.81-1.49 secukinumab : 200-250 sugar component: 10-129 ionic strength provider.
E873. 0.81-1.49 secukinumab : 200-250 sugar component: 0.001-2 chelator.
E874. 0.81-1.49 secukinumab : 1-9 buffer system : 200-250 sugar component: 20-99 amino acid component.
E875. 0.81-1.49 secukinumab : 51-60 buffer system : 200-250 sugar component: 20-99 amino acid component.
E876. 0.81-1.49 secukinumab : 1-9 buffer system : 200-250 sugar component: 100-149 amino acid component.
E877. 0.81-1.49 secukinumab : 51-60 buffer system : 200-250 sugar component: 100-149 amino acid component.
E878. 0.81-1.49 secukinumab : 1-9 buffer system : 200-250 sugar component : 0.008-0.92 surfactant.
E879. 0.81-1.49 secukinumab : 51-60 buffer system : 200-250 sugar component : 0.008-0.92 surfactant.
E880. 0.81-1.49 secukinumab : 1-9 buffer system : 200-250 sugar component: 0.1-20 antioxidant.
E881. 0.81-1.49 secukinumab : 51-60 buffer system : 200-250 sugar component: 0.1-20 antioxidant.
E882. 0.81-1.49 secukinumab : 1-9 buffer system : 200-250 sugar component: 10-129 tonicifier.
E883. 0.81-1.49 secukinumab : 51-60 buffer system : 200-250 sugar component: 10-129 tonicifier.
E884. 0.81-1.49 secukinumab : 1-9 buffer system : 200-250 sugar component: 10-129 ionic strength provider.
E885. 0.81-1.49 secukinumab : 51-60 buffer system : 200-250 sugar component: 10-129 ionic strength provider.
E886. 0.81-1.49 secukinumab : 1-9 buffer system : 200-250 sugar component: 0.001-2 chelator.
E887. 0.81-1.49 secukinumab : 51-60 buffer system : 200-250 sugar component: 0.001-2 chelator.
E888. 0.81-1.49 secukinumab : 200-250 sugar component: 20-99 amino acid component: 0.008-0.92 surfactant.
E889. 0.81-1.49 secukinumab : 200-250 sugar component: 100-149 amino acid component: 0.008-0.92 surfactant.
E890. 0.81-1.49 secukinumab : 200-250 sugar component: 20-99 amino acid component: 0.1-20 antioxidant.
E891. 0.81-1.49 secukinumab : 200-250 sugar component: 100-149 amino acid component: 0.1-20 antioxidant.
E892. 0.81-1.49 secukinumab : 200-250 sugar component: 20-99 amino acid component: 10-129 tonicifier.
E893. 0.81-1.49 secukinumab : 200-250 sugar component: 100-149 amino acid component: 10-129 tonicifier.
E894. 0.81-1.49 secukinumab : 200-250 sugar component: 20-99 amino acid component: 10-129 ionic strength provider.
E895. 0.81-1.49 secukinumab : 200-250 sugar component: 100-149 amino acid component: 10-129 ionic strength provider.
E896. 0.81-1.49 secukinumab : 200-250 sugar component: 20-99 amino acid component: 0.001-2 chelator.
E897. 0.81-1.49 secukinumab : 200-250 sugar component: 100-149 amino acid component: 0.001-2 chelator.
E898. 0.81-1.49 secukinumab : 200-250 sugar component: 0.008-0.92 surfactant: 0.1-20 antioxidant.
E899. 0.81-1.49 secukinumab : 200-250 sugar component: 0.008-0.92 surfactant: 10-129 tonicifier.
E900. 0.81-1.49 secukinumab : 200-250 sugar component: 0.008-0.92 surfactant: 10-129 ionic strength provider.
E901. 0.81-1.49 secukinumab : 200-250 sugar component: 0.008-0.92 surfactant: 0.001-2 chelator.
E902. 0.81-1.49 secukinumab : 200-250 sugar component: 0.1-20 antioxidant: 10-129 tonicifier.
E903. 0.81-1.49 secukinumab : 200-250 sugar component: 0.1-20 antioxidant: 10-129 ionic strength provider.
E904. 0.81-1.49 secukinumab : 200-250 sugar component: 0.1-20 antioxidant: 0.001-2 chelator.
E905. 0.81-1.49 secukinumab : 200-250 sugar component: 10-129 tonicifier: 10-129 ionic strength provider.
E906. 0.81-1.49 secukinumab : 200-250 sugar component: 10-129 tonicifier: 0.001-2 chelator.
E907. 0.81-1.49 secukinumab : 200-250 sugar component: 10-129 ionic strength provider: 0.001-2 chelator.
E908. 0.81-1.49 secukinumab : 1-9 buffer system : 200-250 sugar component : 20-99 amino acid component : 0.008-0.92 surfactant.
E909. 0.81-1.49 secukinumab : 51-60 buffer system : 200-250 sugar component : 20-99 amino acid component : 0.008-0.92 surfactant.
E910. 0.81-1.49 secukinumab : 1-9 buffer system : 200-250 sugar component : 100-149 amino acid component : 0.008-0.92 surfactant.

(continued)

E911. 0.81-1.49 secukinumab : 51-60 buffer system : 200-250 sugar component : 100-149 amino acid component : 0.008-0.92 surfactant.

E912. 0.81-1.49 secukinumab : 1-9 buffer system : 200-250 sugar component: 20-99 amino acid component: 0.1-20 antioxidant.

E913. 0.81-1.49 secukinumab : 51-60 buffer system : 200-250 sugar component : 20-99 amino acid component : 0.1-20 antioxidant.

E914. 0.81-1.49 secukinumab : 1-9 buffer system : 200-250 sugar component : 100-149 amino acid component : 0.1-20 antioxidant.

E915. 0.81-1.49 secukinumab : 51-60 buffer system : 200-250 sugar component : 100-149 amino acid component : 0.1-20 antioxidant.

E916. 0.81-1.49 secukinumab : 1-9 buffer system : 200-250 sugar component: 20-99 amino acid component: 10-129 tonicifier.

E917. 0.81-1.49 secukinumab : 51-60 buffer system : 200-250 sugar component: 20-99 amino acid component: 10-129 tonicifier.

E918. 0.81-1.49 secukinumab : 1-9 buffer system : 200-250 sugar component: 100-149 amino acid component: 10-129 tonicifier.

E919. 0.81-1.49 secukinumab : 51-60 buffer system : 200-250 sugar component : 100-149 amino acid component : 10-129 tonicifier.

E920. 0.81-1.49 secukinumab : 1-9 buffer system : 200-250 sugar component : 20-99 amino acid component : 10-129 ionic strength provider.

E921. 0.81-1.49 secukinumab : 51-60 buffer system : 200-250 sugar component : 20-99 amino acid component : 10-129 ionic strength provider.

E922. 0.81-1.49 secukinumab : 1-9 buffer system : 200-250 sugar component : 100-149 amino acid component : 10-129 ionic strength provider.

E923. 0.81-1.49 secukinumab : 51-60 buffer system : 200-250 sugar component: 100-149 amino acid component: 10-129 ionic strength provider.

E924. 0.81-1.49 secukinumab : 1-9 buffer system : 200-250 sugar component: 20-99 amino acid component: 0.001-2 chelator.

E925. 0.81-1.49 secukinumab : 51-60 buffer system : 200-250 sugar component: 20-99 amino acid component: 0.001-2 chelator.

E926. 0.81-1.49 secukinumab : 1-9 buffer system : 200-250 sugar component: 100-149 amino acid component: 0.001-2 chelator.

E927. 0.81-1.49 secukinumab : 51-60 buffer system : 200-250 sugar component : 100-149 amino acid component : 0.001-2 chelator.

E928. 0.81-1.49 secukinumab : 1-9 buffer system : 200-250 sugar component: 0.008-0.92 surfactant: 0.1-20 antioxidant.

E929. 0.81-1.49 secukinumab : 51-60 buffer system : 200-250 sugar component: 0.008-0.92 surfactant: 0.1-20 antioxidant.

E930. 0.81-1.49 secukinumab : 1-9 buffer system : 200-250 sugar component: 0.008-0.92 surfactant: 10-129 tonicifier.

E931. 0.81-1.49 secukinumab : 51-60 buffer system : 200-250 sugar component: 0.008-0.92 surfactant: 10-129 tonicifier.

E932. 0.81-1.49 secukinumab : 1-9 buffer system : 200-250 sugar component : 0.008-0.92 surfactant : 10-129 ionic strength provider.

E933. 0.81-1.49 secukinumab : 51-60 buffer system : 200-250 sugar component : 0.008-0.92 surfactant : 10-129 ionic strength provider.

E934. 0.81-1.49 secukinumab : 1-9 buffer system : 200-250 sugar component: 0.008-0.92 surfactant: 0.001-2 chelator.

E935. 0.81-1.49 secukinumab : 51-60 buffer system : 200-250 sugar component: 0.008-0.92 surfactant: 0.001-2 chelator.

E936. 0.81-1.49 secukinumab : 1-9 buffer system : 200-250 sugar component: 0.1-20 antioxidant: 10-129 tonicifier.

(continued)

E937. 0.81-1.49 secukinumab : 51-60 buffer system : 200-250 sugar component: 0.1-20 antioxidant: 10-129 tonicifier.

E938. 0.81-1.49 secukinumab : 1-9 buffer system : 200-250 sugar component: 0.1-20 antioxidant: 10-129 ionic strength provider.

E939. 0.81-1.49 secukinumab : 51-60 buffer system : 200-250 sugar component : 0.1-20 antioxidant : 10-129 ionic strength provider.

E940. 0.81-1.49 secukinumab : 1-9 buffer system : 200-250 sugar component: 0.1-20 antioxidant: 0.001-2 chelator.

E941. 0.81-1.49 secukinumab : 51-60 buffer system : 200-250 sugar component: 0.1-20 antioxidant: 0.001-2 chelator.

E942. 0.81-1.49 secukinumab : 1-9 buffer system : 200-250 sugar component: 10-129 tonicifier : 10-129 ionic strength provider.

E943. 0.81-1.49 secukinumab : 51-60 buffer system : 200-250 sugar component : 10-129 tonicifier: 10-129 ionic strength provider.

E944. 0.81-1.49 secukinumab : 1-9 buffer system : 200-250 sugar component: 10-129 tonicifier : 0.001-2 chelator.

E945. 0.81-1.49 secukinumab : 51-60 buffer system : 200-250 sugar component: 10-129 tonicifier : 0.001-2 chelator.

E946. 0.81-1.49 secukinumab : 1-9 buffer system : 200-250 sugar component: 10-129 ionic strength provider: 0.001-2 chelator.

E947. 0.81-1.49 secukinumab: 51-60 buffer system : 200-250 sugar component : 10-129 ionic strength provider: 0.001-2 chelator.

E948. 0.81-1.49 secukinumab : 200-250 sugar component : 20-99 amino acid component : 0.008-0.92 surfactant : 0.1-20 antioxidant.

E949. 0.81-1.49 secukinumab : 200-250 sugar component : 100-149 amino acid component : 0.008-0.92 surfactant : 0.1-20 antioxidant.

E950. 0.81-1.49 secukinumab : 200-250 sugar component : 20-99 amino acid component : 0.008-0.92 surfactant : 10-129 tonicifier.

E951. 0.81-1.49 secukinumab : 200-250 sugar component : 100-149 amino acid component : 0.008-0.92 surfactant : 10-129 tonicifier.

E952. 0.81-1.49 secukinumab : 200-250 sugar component: 20-99 amino acid component: 0.008-0.92 surfactant: 10-129 ionic strength provider.

E953. 0.81-1.49 secukinumab : 200-250 sugar component: 100-149 amino acid component: 0.008-0.92 surfactant: 10-129 ionic strength provider.

E954. 0.81-1.49 secukinumab : 200-250 sugar component : 20-99 amino acid component : 0.008-0.92 surfactant : 0.001-2 chelator.

E955. 0.81-1.49 secukinumab : 200-250 sugar component : 100-149 amino acid component : 0.008-0.92 surfactant : 0.001-2 chelator.

E956. 0.81-1.49 secukinumab : 200-250 sugar component: 20-99 amino acid component: 0.1-20 antioxidant: 10-129 tonicifier.

E957. 0.81-1.49 secukinumab : 200-250 sugar component: 100-149 amino acid component: 0.1-20 antioxidant: 10-129 tonicifier.

E958. 0.81-1.49 secukinumab : 200-250 sugar component : 20-99 amino acid component : 0.1-20 antioxidant : 10-129 ionic strength provider.

E959. 0.81-1.49 secukinumab : 200-250 sugar component : 100-149 amino acid component : 0.1-20 antioxidant : 10-129 ionic strength provider.

E960. 0.81-1.49 secukinumab : 200-250 sugar component: 20-99 amino acid component: 0.1-20 antioxidant: 0.001-2 chelator.

E961. 0.81-1.49 secukinumab : 200-250 sugar component: 100-149 amino acid component: 0.1-20 antioxidant: 0.001-2 chelator.

E962. 0.81-1.49 secukinumab : 200-250 sugar component: 20-99 amino acid component: 10-129 tonicifier: 10-129 ionic strength provider.

(continued)

E963. 0.81-1.49 secukinumab : 200-250 sugar component : 100-149 amino acid component : 10-129 tonicifier : 10-129 ionic strength provider.

E964. 0.81-1.49 secukinumab : 200-250 sugar component: 20-99 amino acid component: 10-129 tonicifier : 0.001-2 chelator.

E965. 0.81-1.49 secukinumab : 200-250 sugar component: 100-149 amino acid component: 10-129 tonicifier: 0.001-2 chelator.

E966. 0.81-1.49 secukinumab : 200-250 sugar component: 20-99 amino acid component: 10-129 ionic strength provider: 0.001-2 chelator.

E967. 0.81-1.49 secukinumab : 200-250 sugar component : 100-149 amino acid component : 10-129 ionic strength provider: 0.001-2 chelator.

E968. 0.81-1.49 secukinumab : 200-250 sugar component: 0.008-0.92 surfactant: 0.1-20 antioxidant: 10-129 tonicifier.

E969. 0.81-1.49 secukinumab : 200-250 sugar component : 0.008-0.92 surfactant : 0.1-20 antioxidant : 10-129 ionic strength provider.

E970. 0.81-1.49 secukinumab : 200-250 sugar component: 0.008-0.92 surfactant: 0.1-20 antioxidant: 0.001-2 chelator.

E971. 0.81-1.49 secukinumab : 200-250 sugar component : 0.008-0.92 surfactant : 10-129 tonicifier : 10-129 ionic strength provider.

E972. 0.81-1.49 secukinumab : 200-250 sugar component: 0.008-0.92 surfactant: 10-129 tonicifier: 0.001-2 chelator.

E973. 0.81-1.49 secukinumab : 200-250 sugar component : 0.008-0.92 surfactant : 10-129 ionic strength provider : 0.001-2 chelator.

E974. 0.81-1.49 secukinumab : 200-250 sugar component: 0.1-20 antioxidant: 10-129 tonicifier: 10-129 ionic strength provider.

E975. 0.81-1.49 secukinumab : 200-250 sugar component: 0.1-20 antioxidant: 10-129 tonicifier: 0.001-2 chelator.

E976. 0.81-1.49 secukinumab : 200-250 sugar component: 0.1-20 antioxidant: 10-129 ionic strength provider: 0.001-2 chelator.

E977. 0.81-1.49 secukinumab : 200-250 sugar component: 10-129 tonicifier: 10-129 ionic strength provider: 0.001-2 chelator.

E978. 0.81-1.49 secukinumab : 1-9 buffer system : 200-250 sugar component : 20-99 amino acid component : 0.008-0.92 surfactant: 0.1-20 antioxidant.

E979. 0.81-1.49 secukinumab : 51-60 buffer system : 200-250 sugar component : 20-99 amino acid component : 0.008-0.92 surfactant: 0.1-20 antioxidant.

E980. 0.81-1.49 secukinumab : 1-9 buffer system : 200-250 sugar component : 100-149 amino acid component : 0.008-0.92 surfactant: 0.1-20 antioxidant.

E981. 0.81-1.49 secukinumab : 51-60 buffer system : 200-250 sugar component : 100-149 amino acid component : 0.008-0.92 surfactant: 0.1-20 antioxidant.

E982. 0.81-1.49 secukinumab : 1-9 buffer system : 200-250 sugar component : 20-99 amino acid component : 0.008-0.92 surfactant: 10-129 tonicifier.

E983. 0.81-1.49 secukinumab : 51-60 buffer system : 200-250 sugar component : 20-99 amino acid component : 0.008-0.92 surfactant: 10-129 tonicifier.

E984. 0.81-1.49 secukinumab : 1-9 buffer system : 200-250 sugar component : 100-149 amino acid component : 0.008-0.92 surfactant: 10-129 tonicifier.

E985. 0.81-1.49 secukinumab : 51-60 buffer system : 200-250 sugar component : 100-149 amino acid component : 0.008-0.92 surfactant: 10-129 tonicifier.

E986. 0.81-1.49 secukinumab : 1-9 buffer system : 200-250 sugar component : 20-99 amino acid component : 0.008-0.92 surfactant: 10-129 ionic strength provider.

E987. 0.81-1.49 secukinumab : 51-60 buffer system : 200-250 sugar component : 20-99 amino acid component : 0.008-0.92 surfactant: 10-129 ionic strength provider.

E988. 0.81-1.49 secukinumab : 1-9 buffer system : 200-250 sugar component : 100-149 amino acid component : 0.008-0.92 surfactant: 10-129 ionic strength provider.

(continued)

E989. 0.81-1.49 secukinumab : 51-60 buffer system : 200-250 sugar component : 100-149 amino acid component : 0.008-0.92 surfactant: 10-129 ionic strength provider.

E990. 0.81-1.49 secukinumab : 1-9 buffer system : 200-250 sugar component : 20-99 amino acid component : 0.008-0.92 surfactant: 0.001-2 chelator.

E991. 0.81-1.49 secukinumab : 51-60 buffer system : 200-250 sugar component : 20-99 amino acid component : 0.008-0.92 surfactant: 0.001-2 chelator.

E992. 0.81-1.49 secukinumab : 1-9 buffer system : 200-250 sugar component : 100-149 amino acid component : 0.008-0.92 surfactant: 0.001-2 chelator.

E993. 0.81-1.49 secukinumab : 51-60 buffer system : 200-250 sugar component : 100-149 amino acid component : 0.008-0.92 surfactant: 0.001-2 chelator.

E994. 0.81-1.49 secukinumab : 1-9 buffer system : 200-250 sugar component: 20-99 amino acid component: 0.1-20 antioxidant : 10-129 tonicifier.

E995. 0.81-1.49 secukinumab: 51-60 buffer system : 200-250 sugar component : 20-99 amino acid component: 0.1-20 antioxidant : 10-129 tonicifier.

E996. 0.81-1.49 secukinumab: 1-9 buffer system : 200-250 sugar component : 100-149 amino acid component: 0.1-20 antioxidant : 10-129 tonicifier.

E997. 0.81-1.49 secukinumab : 51-60 buffer system : 200-250 sugar component : 100-149 amino acid component : 0.1-20 antioxidant: 10-129 tonicifier.

E998. 0.81-1.49 secukinumab : 1-9 buffer system : 200-250 sugar component: 20-99 amino acid component: 0.1-20 antioxidant : 10-129 ionic strength provider.

E999. 0.81-1.49 secukinumab : 51-60 buffer system : 200-250 sugar component : 20-99 amino acid component: 0.1-20 antioxidant : 10-129 ionic strength provider.

E1000 . 0.81-1.49 secukinumab: 1-9 buffer system : 200-250 sugar component : 100-149 amino acid component: 0.1-20 antioxidant : 10-129 ionic strength provider.

E1001. 0.81-1.49 secukinumab : 51-60 buffer system : 200-250 sugar component : 100-149 amino acid component : 0.1-20 antioxidant: 10-129 ionic strength provider.

E1002. 0.81-1.49 secukinumab : 1-9 buffer system : 200-250 sugar component: 20-99 amino acid component: 0.1-20 antioxidant : 0.001-2 chelator.

E1003. 0.81-1.49 secukinumab: 51-60 buffer system : 200-250 sugar component : 20-99 amino acid component: 0.1-20 antioxidant : 0.001-2 chelator.

E1004. 0.81-1.49 secukinumab: 1-9 buffer system : 200-250 sugar component : 100-149 amino acid component: 0.1-20 antioxidant : 0.001-2 chelator.

E1005. 0.81-1.49 secukinumab : 51-60 buffer system : 200-250 sugar component : 100-149 amino acid component : 0.1-20 antioxidant: 0.001-2 chelator.

E1006. 0.81-1.49 secukinumab : 1-9 buffer system : 200-250 sugar component: 20-99 amino acid component: 10-129 tonicifier: 10-129 ionic strength provider.

E1007. 0.81-1.49 secukinumab : 51-60 buffer system : 200-250 sugar component: 20-99 amino acid component: 10-129 tonicifier : 10-129 ionic strength provider.

E1008. 0.81-1.49 secukinumab : 1-9 buffer system : 200-250 sugar component: 100-149 amino acid component: 10-129 tonicifier : 10-129 ionic strength provider.

E1009. 0.81-1.49 secukinumab : 51-60 buffer system : 200-250 sugar component : 100-149 amino acid component : 10-129 tonicifier : 10-129 ionic strength provider.

E1010. 0.81-1.49 secukinumab : 1-9 buffer system : 200-250 sugar component: 20-99 amino acid component: 10-129 tonicifier: 0.001-2 chelator.

E1011. 0.81-1.49 secukinumab : 51-60 buffer system : 200-250 sugar component: 20-99 amino acid component: 10-129 tonicifier : 0.001-2 chelator.

E1012. 0.81-1.49 secukinumab : 1-9 buffer system : 200-250 sugar component: 100-149 amino acid component: 10-129 tonicifier : 0.001-2 chelator.

E1013. 0.81-1.49 secukinumab : 51-60 buffer system : 200-250 sugar component : 100-149 amino acid component : 10-129 tonicifier: 0.001-2 chelator.

E1014. 0.81-1.49 secukinumab : 1-9 buffer system : 200-250 sugar component : 20-99 amino acid component : 10-129 ionic strength provider: 0.001-2 chelator.

(continued)

E1015. 0.81-1.49 secukinumab : 51-60 buffer system : 200-250 sugar component : 20-99 amino acid component : 10-129 ionic strength provider: 0.001-2 chelator.

E1016. 0.81-1.49 secukinumab : 1-9 buffer system : 200-250 sugar component : 100-149 amino acid component : 10-129 ionic strength provider: 0.001-2 chelator.

E1017. 0.81-1.49 secukinumab : 51-60 buffer system : 200-250 sugar component: 100-149 amino acid component: 10-129 ionic strength provider: 0.001-2 chelator.

E1018. 0.81-1.49 secukinumab : 1-9 buffer system : 200-250 sugar component: 0.008-0.92 surfactant: 0.1-20 antioxidant: 10-129 tonicifier.

E1019. 0.81-1.49 secukinumab : 51-60 buffer system : 200-250 sugar component: 0.008-0.92 surfactant: 0.1-20 antioxidant: 10-129 tonicifier.

E1020. 0.81-1.49 secukinumab : 1-9 buffer system : 200-250 sugar component: 0.008-0.92 surfactant: 0.1-20 antioxidant: 10-129 ionic strength provider.

E1021. 0.81-1.49 secukinumab : 51-60 buffer system : 200-250 sugar component: 0.008-0.92 surfactant: 0.1-20 antioxidant: 10-129 ionic strength provider.

E1022. 0.81-1.49 secukinumab : 1-9 buffer system : 200-250 sugar component: 0.008-0.92 surfactant: 0.1-20 antioxidant: 0.001-2 chelator.

E1023. 0.81-1.49 secukinumab : 51-60 buffer system : 200-250 sugar component : 0.008-0.92 surfactant : 0.1-20 antioxidant : 0.001-2 chelator.

E1024. 0.81-1.49 secukinumab : 1-9 buffer system : 200-250 sugar component: 0.008-0.92 surfactant: 10-129 tonicifier : 10-129 ionic strength provider.

E1025. 0.81-1.49 secukinumab : 51-60 buffer system : 200-250 sugar component : 0.008-0.92 surfactant: 10-129 tonicifier : 10-129 ionic strength provider.

E1026. 0.81-1.49 secukinumab : 1-9 buffer system : 200-250 sugar component: 0.008-0.92 surfactant: 10-129 tonicifier: 0.001-2 chelator.

E1027. 0.81-1.49 secukinumab : 51-60 buffer system : 200-250 sugar component: 0.008-0.92 surfactant: 10-129 tonicifier: 0.001-2 chelator.

E1028. 0.81-1.49 secukinumab : 1-9 buffer system : 200-250 sugar component : 0.008-0.92 surfactant : 10-129 ionic strength provider: 0.001-2 chelator.

E1029. 0.81-1.49 secukinumab : 51-60 buffer system : 200-250 sugar component : 0.008-0.92 surfactant : 10-129 ionic strength provider: 0.001-2 chelator.

E1030. 0.81-1.49 secukinumab : 1-9 buffer system : 200-250 sugar component: 0.1-20 antioxidant: 10-129 tonicifier: 10-129 ionic strength provider.

E1031. 0.81-1.49 secukinumab : 51-60 buffer system : 200-250 sugar component : 0.1-20 antioxidant: 10-129 tonicifier: 10-129 ionic strength provider.

E1032. 0.81-1.49 secukinumab : 1-9 buffer system : 200-250 sugar component : 0.1-20 antioxidant : 10-129 tonicifier: 0.001-2 chelator.

E1033. 0.81-1.49 secukinumab : 51-60 buffer system : 200-250 sugar component: 0.1-20 antioxidant: 10-129 tonicifier : 0.001-2 chelator.

E1034. 0.81-1.49 secukinumab : 1-9 buffer system : 200-250 sugar component: 0.1-20 antioxidant: 10-129 ionic strength provider : 0.001-2 chelator.

E1035. 0.81-1.49 secukinumab : 51-60 buffer system : 200-250 sugar component : 0.1-20 antioxidant : 10-129 ionic strength provider: 0.001-2 chelator.

E1036. 0.81-1.49 secukinumab : 1-9 buffer system : 200-250 sugar component: 10-129 tonicifier : 10-129 ionic strength provider: 0.001-2 chelator.

E1037. 0.81-1.49 secukinumab : 51-60 buffer system : 200-250 sugar component: 10-129 tonicifier: 10-129 ionic strength provider : 0.001-2 chelator.

E1038. 0.81-1.49 secukinumab : 200-250 sugar component : 20-99 amino acid component : 0.008-0.92 surfactant : 0.1-20 antioxidant: 10-129 tonicifier.

E1039. 0.81-1.49 secukinumab : 200-250 sugar component : 100-149 amino acid component : 0.008-0.92 surfactant : 0.1-20 antioxidant: 10-129 tonicifier.

E1040. 0.81-1.49 secukinumab : 200-250 sugar component : 20-99 amino acid component : 0.008-0.92 surfactant : 0.1-20 antioxidant: 10-129 ionic strength provider.

(continued)

E1041. 0.81-1.49 secukinumab : 200-250 sugar component : 100-149 amino acid component : 0.008-0.92 surfactant : 0.1-20 antioxidant: 10-129 ionic strength provider.

E1042. 0.81-1.49 secukinumab : 200-250 sugar component : 20-99 amino acid component : 0.008-0.92 surfactant : 0.1-20 antioxidant: 0.001-2 chelator.

E1043. 0.81-1.49 secukinumab : 200-250 sugar component : 100-149 amino acid component : 0.008-0.92 surfactant : 0.1-20 antioxidant: 0.001-2 chelator.

E1044. 0.81-1.49 secukinumab : 200-250 sugar component: 20-99 amino acid component: 0.008-0.92 surfactant: 10-129 tonicifier : 10-129 ionic strength provider.

E1045. 0.81-1.49 secukinumab : 200-250 sugar component : 100-149 amino acid component : 0.008-0.92 surfactant : 10-129 tonicifier : 10-129 ionic strength provider.

E1046. 0.81-1.49 secukinumab : 200-250 sugar component: 20-99 amino acid component: 0.008-0.92 surfactant: 10-129 tonicifier : 0.001-2 chelator.

E1047. 0.81-1.49 secukinumab : 200-250 sugar component : 100-149 amino acid component : 0.008-0.92 surfactant : 10-129 tonicifier: 0.001-2 chelator.

E1048. 0.81-1.49 secukinumab : 200-250 sugar component: 20-99 amino acid component: 0.008-0.92 surfactant: 10-129 ionic strength provider: 0.001-2 chelator.

E1049. 0.81-1.49 secukinumab : 200-250 sugar component: 100-149 amino acid component: 0.008-0.92 surfactant: 10-129 ionic strength provider: 0.001-2 chelator.

E1050. 0.81-1.49 secukinumab : 200-250 sugar component: 20-99 amino acid component: 0.1-20 antioxidant: 10-129 tonicifier : 10-129 ionic strength provider.

E1051. 0.81-1.49 secukinumab : 200-250 sugar component: 100-149 amino acid component: 0.1-20 antioxidant: 10-129 tonicifier : 10-129 ionic strength provider.

E1052. 0.81-1.49 secukinumab : 200-250 sugar component: 20-99 amino acid component: 0.1-20 antioxidant: 10-129 tonicifier : 0.001-2 chelator.

E1053. 0.81-1.49 secukinumab : 200-250 sugar component: 100-149 amino acid component: 0.1-20 antioxidant: 10-129 tonicifier : 0.001-2 chelator.

E1054. 0.81-1.49 secukinumab : 200-250 sugar component : 20-99 amino acid component : 0.1-20 antioxidant : 10-129 ionic strength provider: 0.001-2 chelator.

E1055. 0.81-1.49 secukinumab : 200-250 sugar component : 100-149 amino acid component : 0.1-20 antioxidant : 10-129 ionic strength provider: 0.001-2 chelator.

E1056. 0.81-1.49 secukinumab : 200-250 sugar component: 20-99 amino acid component: 10-129 tonicifier: 10-129 ionic strength provider: 0.001-2 chelator.

E1057. 0.81-1.49 secukinumab : 200-250 sugar component : 100-149 amino acid component : 10-129 tonicifier : 10-129 ionic strength provider: 0.001-2 chelator.

E1058. 0.81-1.49 secukinumab : 200-250 sugar component: 0.008-0.92 surfactant: 0.1-20 antioxidant: 10-129 tonicifier: 10-129 ionic strength provider.

E1059. 0.81-1.49 secukinumab : 200-250 sugar component: 0.008-0.92 surfactant: 0.1-20 antioxidant: 10-129 tonicifier: 0.001-2 chelator.

E1060. 0.81-1.49 secukinumab : 200-250 sugar component : 0.008-0.92 surfactant : 0.1-20 antioxidant : 10-129 ionic strength provider: 0.001-2 chelator.

E1061. 0.81-1.49 secukinumab : 200-250 sugar component : 0.008-0.92 surfactant : 10-129 tonicifier : 10-129 ionic strength provider: 0.001-2 chelator.

E1062. 0.81-1.49 secukinumab : 200-250 sugar component: 0.1-20 antioxidant: 10-129 tonicifier : 10-129 ionic strength provider : 0.001-2 chelator.

E1063. 0.81-1.49 secukinumab : 1-9 buffer system : 200-250 sugar component : 20-99 amino acid component : 0.008-0.92 surfactant: 0.1-20 antioxidant: 10-129 tonicifier.

E1064. 0.81-1.49 secukinumab : 51-60 buffer system : 200-250 sugar component : 20-99 amino acid component : 0.008-0.92 surfactant: 0.1-20 antioxidant: 10-129 tonicifier.

E1065. 0.81-1.49 secukinumab : 1-9 buffer system : 200-250 sugar component : 100-149 amino acid component : 0.008-0.92 surfactant: 0.1-20 antioxidant: 10-129 tonicifier.

E1066. 0.81-1.49 secukinumab : 51-60 buffer system : 200-250 sugar component : 100-149 amino acid component : 0.008-0.92 surfactant: 0.1-20 antioxidant: 10-129 tonicifier.

(continued)

E1067. 0.81-1.49 secukinumab : 1-9 buffer system : 200-250 sugar component : 20-99 amino acid component : 0.008-0.92 surfactant: 0.1-20 antioxidant: 10-129 ionic strength provider.

E1068. 0.81-1.49 secukinumab : 51-60 buffer system : 200-250 sugar component : 20-99 amino acid component : 0.008-0.92 surfactant: 0.1-20 antioxidant: 10-129 ionic strength provider.

E1069. 0.81-1.49 secukinumab : 1-9 buffer system : 200-250 sugar component : 100-149 amino acid component : 0.008-0.92 surfactant: 0.1-20 antioxidant: 10-129 ionic strength provider.

E1070. 0.81-1.49 secukinumab : 51-60 buffer system : 200-250 sugar component : 100-149 amino acid component : 0.008-0.92 surfactant: 0.1-20 antioxidant: 10-129 ionic strength provider.

E1071. 0.81-1.49 secukinumab : 1-9 buffer system : 200-250 sugar component : 20-99 amino acid component : 0.008-0.92 surfactant: 0.1-20 antioxidant: 0.001-2 chelator.

E1072. 0.81-1.49 secukinumab : 51-60 buffer system : 200-250 sugar component : 20-99 amino acid component : 0.008-0.92 surfactant: 0.1-20 antioxidant: 0.001-2 chelator.

E1073. 0.81-1.49 secukinumab : 1-9 buffer system : 200-250 sugar component : 100-149 amino acid component : 0.008-0.92 surfactant: 0.1-20 antioxidant: 0.001-2 chelator.

E1074. 0.81-1.49 secukinumab : 51-60 buffer system : 200-250 sugar component : 100-149 amino acid component : 0.008-0.92 surfactant: 0.1-20 antioxidant: 0.001-2 chelator.

E1075. 0.81-1.49 secukinumab : 1-9 buffer system : 200-250 sugar component : 20-99 amino acid component : 0.008-0.92 surfactant: 10-129 tonicifier : 10-129 ionic strength provider.

E1076. 0.81-1.49 secukinumab : 51-60 buffer system : 200-250 sugar component : 20-99 amino acid component : 0.008-0.92 surfactant: 10-129 tonicifier : 10-129 ionic strength provider.

E1077. 0.81-1.49 secukinumab : 1-9 buffer system : 200-250 sugar component : 100-149 amino acid component : 0.008-0.92 surfactant: 10-129 tonicifier : 10-129 ionic strength provider.

E1078. 0.81-1.49 secukinumab : 51-60 buffer system : 200-250 sugar component : 100-149 amino acid component : 0.008-0.92 surfactant: 10-129 tonicifier : 10-129 ionic strength provider.

E1079. 0.81-1.49 secukinumab : 1-9 buffer system : 200-250 sugar component : 20-99 amino acid component : 0.008-0.92 surfactant: 10-129 tonicifier : 0.001-2 chelator.

E1080. 0.81-1.49 secukinumab : 51-60 buffer system : 200-250 sugar component : 20-99 amino acid component : 0.008-0.92 surfactant: 10-129 tonicifier : 0.001-2 chelator.

E1081. 0.81-1.49 secukinumab : 1-9 buffer system : 200-250 sugar component : 100-149 amino acid component : 0.008-0.92 surfactant: 10-129 tonicifier : 0.001-2 chelator.

E1082. 0.81-1.49 secukinumab : 51-60 buffer system : 200-250 sugar component : 100-149 amino acid component : 0.008-0.92 surfactant: 10-129 tonicifier : 0.001-2 chelator.

E1083. 0.81-1.49 secukinumab : 1-9 buffer system : 200-250 sugar component : 20-99 amino acid component : 0.008-0.92 surfactant: 10-129 ionic strength provider: 0.001-2 chelator.

E1084. 0.81-1.49 secukinumab : 51-60 buffer system : 200-250 sugar component : 20-99 amino acid component : 0.008-0.92 surfactant: 10-129 ionic strength provider: 0.001-2 chelator.

E1085. 0.81-1.49 secukinumab : 1-9 buffer system : 200-250 sugar component : 100-149 amino acid component : 0.008-0.92 surfactant: 10-129 ionic strength provider: 0.001-2 chelator.

E1086. 0.81-1.49 secukinumab : 51-60 buffer system : 200-250 sugar component : 100-149 amino acid component : 0.008-0.92 surfactant: 10-129 ionic strength provider: 0.001-2 chelator.

E1087. 0.81-1.49 secukinumab : 1-9 buffer system : 200-250 sugar component: 20-99 amino acid component: 0.1-20 antioxidant : 10-129 tonicifier : 10-129 ionic strength provider.

E1088. 0.81-1.49 secukinumab: 51-60 buffer system : 200-250 sugar component : 20-99 amino acid component: 0.1-20 antioxidant : 10-129 tonicifier : 10-129 ionic strength provider.

E1089. 0.81-1.49 secukinumab: 1-9 buffer system : 200-250 sugar component : 100-149 amino acid component: 0.1-20 antioxidant : 10-129 tonicifier : 10-129 ionic strength provider.

E1090. 0.81-1.49 secukinumab : 51-60 buffer system : 200-250 sugar component : 100-149 amino acid component : 0.1-20 antioxidant: 10-129 tonicifier: 10-129 ionic strength provider.

E1091. 0.81-1.49 secukinumab : 1-9 buffer system : 200-250 sugar component: 20-99 amino acid component: 0.1-20 antioxidant : 10-129 tonicifier : 0.001-2 chelator.

E1092. 0.81-1.49 secukinumab: 51-60 buffer system : 200-250 sugar component : 20-99 amino acid component: 0.1-20 antioxidant : 10-129 tonicifier : 0.001-2 chelator.

(continued)

E1093. 0.81-1.49 secukinumab: 1-9 buffer system : 200-250 sugar component : 100-149 amino acid component: 0.1-20 antioxidant : 10-129 tonicifier : 0.001-2 chelator.

E1094. 0.81-1.49 secukinumab : 51-60 buffer system : 200-250 sugar component : 100-149 amino acid component : 0.1-20 antioxidant: 10-129 tonicifier: 0.001-2 chelator.

E1095. 0.81-1.49 secukinumab : 1-9 buffer system : 200-250 sugar component: 20-99 amino acid component: 0.1-20 antioxidant : 10-129 ionic strength provider: 0.001-2 chelator.

E1096. 0.81-1.49 secukinumab: 51-60 buffer system : 200-250 sugar component : 20-99 amino acid component: 0.1-20 antioxidant : 10-129 ionic strength provider: 0.001-2 chelator.

E1097. 0.81-1.49 secukinumab: 1-9 buffer system : 200-250 sugar component : 100-149 amino acid component: 0.1-20 antioxidant : 10-129 ionic strength provider: 0.001-2 chelator.

E1098. 0.81-1.49 secukinumab : 51-60 buffer system : 200-250 sugar component : 100-149 amino acid component : 0.1-20 antioxidant: 10-129 ionic strength provider: 0.001-2 chelator.

E1099. 0.81-1.49 secukinumab : 1-9 buffer system : 200-250 sugar component: 20-99 amino acid component: 10-129 tonicifier: 10-129 ionic strength provider: 0.001-2 chelator.

E1100. 0.81-1.49 secukinumab : 51-60 buffer system : 200-250 sugar component: 20-99 amino acid component: 10-129 tonicifier : 10-129 ionic strength provider: 0.001-2 chelator.

E1101. 0.81-1.49 secukinumab : 1-9 buffer system : 200-250 sugar component: 100-149 amino acid component: 10-129 tonicifier : 10-129 ionic strength provider: 0.001-2 chelator.

E1102. 0.81-1.49 secukinumab : 51-60 buffer system : 200-250 sugar component : 100-149 amino acid component : 10-129 tonicifier: 10-129 ionic strength provider: 0.001-2 chelator.

E1103. 0.81-1.49 secukinumab : 1-9 buffer system : 200-250 sugar component: 0.008-0.92 surfactant: 0.1-20 antioxidant: 10-129 tonicifier : 10-129 ionic strength provider.

E1104. 0.81-1.49 secukinumab : 51-60 buffer system : 200-250 sugar component: 0.008-0.92 surfactant: 0.1-20 antioxidant: 10-129 tonicifier : 10-129 ionic strength provider.

E1105. 0.81-1.49 secukinumab : 1-9 buffer system : 200-250 sugar component: 0.008-0.92 surfactant: 0.1-20 antioxidant: 10-129 tonicifier: 0.001-2 chelator.

E1106. 0.81-1.49 secukinumab : 51-60 buffer system : 200-250 sugar component: 0.008-0.92 surfactant: 0.1-20 antioxidant: 10-129 tonicifier : 0.001-2 chelator.

E1107. 0.81-1.49 secukinumab : 1-9 buffer system : 200-250 sugar component: 0.008-0.92 surfactant: 0.1-20 antioxidant: 10-129 ionic strength provider: 0.001-2 chelator.

E1108. 0.81-1.49 secukinumab : 51-60 buffer system : 200-250 sugar component: 0.008-0.92 surfactant: 0.1-20 antioxidant: 10-129 ionic strength provider: 0.001-2 chelator.

E1109. 0.81-1.49 secukinumab : 1-9 buffer system : 200-250 sugar component: 0.008-0.92 surfactant: 10-129 tonicifier : 10-129 ionic strength provider: 0.001-2 chelator.

E1110. 0.81-1.49 secukinumab : 51-60 buffer system : 200-250 sugar component : 0.008-0.92 surfactant: 10-129 tonicifier : 10-129 ionic strength provider: 0.001-2 chelator.

E1111. 0.81-1.49 secukinumab : 1-9 buffer system : 200-250 sugar component: 0.1-20 antioxidant: 10-129 tonicifier: 10-129 ionic strength provider: 0.001-2 chelator.

E1112. 0.81-1.49 secukinumab : 51-60 buffer system : 200-250 sugar component : 0.1-20 antioxidant: 10-129 tonicifier: 10-129 ionic strength provider: 0.001-2 chelator.

E1113. 0.81-1.49 secukinumab : 200-250 sugar component : 20-99 amino acid component : 0.008-0.92 surfactant : 0.1-20 antioxidant: 10-129 tonicifier: 10-129 ionic strength provider.

E1114. 0.81-1.49 secukinumab : 200-250 sugar component : 100-149 amino acid component : 0.008-0.92 surfactant : 0.1-20 antioxidant: 10-129 tonicifier: 10-129 ionic strength provider.

E1115. 0.81-1.49 secukinumab : 200-250 sugar component : 20-99 amino acid component : 0.008-0.92 surfactant : 0.1-20 antioxidant: 10-129 tonicifier: 0.001-2 chelator.

E1116. 0.81-1.49 secukinumab : 200-250 sugar component : 100-149 amino acid component : 0.008-0.92 surfactant : 0.1-20 antioxidant: 10-129 tonicifier: 0.001-2 chelator.

E1117. 0.81-1.49 secukinumab : 200-250 sugar component : 20-99 amino acid component : 0.008-0.92 surfactant : 0.1-20 antioxidant: 10-129 ionic strength provider: 0.001-2 chelator.

E1118. 0.81-1.49 secukinumab : 200-250 sugar component : 100-149 amino acid component : 0.008-0.92 surfactant : 0.1-20 antioxidant: 10-129 ionic strength provider: 0.001-2 chelator.

(continued)

E1119. 0.81-1.49 secukinumab : 200-250 sugar component: 20-99 amino acid component: 0.008-0.92 surfactant: 10-129 tonicifier : 10-129 ionic strength provider: 0.001-2 chelator.

E1120. 0.81-1.49 secukinumab : 200-250 sugar component : 100-149 amino acid component : 0.008-0.92 surfactant : 10-129 tonicifier : 10-129 ionic strength provider: 0.001-2 chelator.

E1121. 0.81-1.49 secukinumab : 200-250 sugar component: 20-99 amino acid component: 0.1-20 antioxidant: 10-129 tonicifier : 10-129 ionic strength provider: 0.001-2 chelator.

E1122. 0.81-1.49 secukinumab : 200-250 sugar component: 100-149 amino acid component: 0.1-20 antioxidant: 10-129 tonicifier : 10-129 ionic strength provider: 0.001-2 chelator.

E1123. 0.81-1.49 secukinumab : 200-250 sugar component: 0.008-0.92 surfactant: 0.1-20 antioxidant: 10-129 tonicifier: 10-129 ionic strength provider: 0.001-2 chelator.

E1124. 0.81-1.49 secukinumab : 1-9 buffer system : 200-250 sugar component : 20-99 amino acid component : 0.008-0.92 surfactant: 0.1-20 antioxidant: 10-129 tonicifier : 10-129 ionic strength provider.

E1125. 0.81-1.49 secukinumab : 51-60 buffer system : 200-250 sugar component : 20-99 amino acid component : 0.008-0.92 surfactant: 0.1-20 antioxidant: 10-129 tonicifier : 10-129 ionic strength provider.

E1126. 0.81-1.49 secukinumab : 1-9 buffer system : 200-250 sugar component : 100-149 amino acid component : 0.008-0.92 surfactant: 0.1-20 antioxidant: 10-129 tonicifier : 10-129 ionic strength provider.

E1127. 0.81-1.49 secukinumab : 51-60 buffer system : 200-250 sugar component : 100-149 amino acid component : 0.008-0.92 surfactant: 0.1-20 antioxidant: 10-129 tonicifier : 10-129 ionic strength provider.

E1128. 0.81-1.49 secukinumab : 1-9 buffer system : 200-250 sugar component : 20-99 amino acid component : 0.008-0.92 surfactant: 0.1-20 antioxidant: 10-129 tonicifier : 0.001-2 chelator.

E1129. 0.81-1.49 secukinumab : 51-60 buffer system : 200-250 sugar component : 20-99 amino acid component : 0.008-0.92 surfactant: 0.1-20 antioxidant: 10-129 tonicifier : 0.001-2 chelator.

E1130. 0.81-1.49 secukinumab : 1-9 buffer system : 200-250 sugar component : 100-149 amino acid component : 0.008-0.92 surfactant: 0.1-20 antioxidant: 10-129 tonicifier : 0.001-2 chelator.

E1131. 0.81-1.49 secukinumab : 51-60 buffer system : 200-250 sugar component : 100-149 amino acid component : 0.008-0.92 surfactant: 0.1-20 antioxidant: 10-129 tonicifier : 0.001-2 chelator.

E1132. 0.81-1.49 secukinumab : 1-9 buffer system : 200-250 sugar component : 20-99 amino acid component : 0.008-0.92 surfactant: 0.1-20 antioxidant: 10-129 ionic strength provider: 0.001-2 chelator.

E1133. 0.81-1.49 secukinumab : 51-60 buffer system : 200-250 sugar component : 20-99 amino acid component : 0.008-0.92 surfactant: 0.1-20 antioxidant: 10-129 ionic strength provider: 0.001-2 chelator.

E1134. 0.81-1.49 secukinumab : 1-9 buffer system : 200-250 sugar component : 100-149 amino acid component : 0.008-0.92 surfactant: 0.1-20 antioxidant: 10-129 ionic strength provider: 0.001-2 chelator.

E1135. 0.81-1.49 secukinumab : 51-60 buffer system : 200-250 sugar component : 100-149 amino acid component : 0.008-0.92 surfactant: 0.1-20 antioxidant: 10-129 ionic strength provider: 0.001-2 chelator.

E1136. 0.81-1.49 secukinumab : 1-9 buffer system : 200-250 sugar component : 20-99 amino acid component : 0.008-0.92 surfactant: 10-129 tonicifier : 10-129 ionic strength provider: 0.001-2 chelator.

E1137. 0.81-1.49 secukinumab : 51-60 buffer system : 200-250 sugar component : 20-99 amino acid component : 0.008-0.92 surfactant: 10-129 tonicifier : 10-129 ionic strength provider: 0.001-2 chelator.

E1138. 0.81-1.49 secukinumab : 1-9 buffer system : 200-250 sugar component : 100-149 amino acid component : 0.008-0.92 surfactant: 10-129 tonicifier : 10-129 ionic strength provider: 0.001-2 chelator.

E1139. 0.81-1.49 secukinumab : 51-60 buffer system : 200-250 sugar component : 100-149 amino acid component : 0.008-0.92 surfactant: 10-129 tonicifier : 10-129 ionic strength provider: 0.001-2 chelator.

E1140. 0.81-1.49 secukinumab : 1-9 buffer system : 200-250 sugar component: 20-99 amino acid component: 0.1-20 antioxidant : 10-129 tonicifier : 10-129 ionic strength provider: 0.001-2 chelator.

E1141. 0.81-1.49 secukinumab: 51-60 buffer system : 200-250 sugar component : 20-99 amino acid component: 0.1-20 antioxidant : 10-129 tonicifier : 10-129 ionic strength provider: 0.001-2 chelator.

E1142. 0.81-1.49 secukinumab: 1-9 buffer system : 200-250 sugar component : 100-149 amino acid component: 0.1-20 antioxidant : 10-129 tonicifier : 10-129 ionic strength provider: 0.001-2 chelator.

E1143. 0.81-1.49 secukinumab : 51-60 buffer system : 200-250 sugar component : 100-149 amino acid component : 0.1-20 antioxidant: 10-129 tonicifier: 10-129 ionic strength provider: 0.001-2 chelator.

E1144. 0.81-1.49 secukinumab : 1-9 buffer system : 200-250 sugar component: 0.008-0.92 surfactant: 0.1-20 antioxidant: 10-129 tonicifier : 10-129 ionic strength provider: 0.001-2 chelator.

(continued)

E1145. 0.81-1.49 secukinumab : 51-60 buffer system : 200-250 sugar component: 0.008-0.92 surfactant: 0.1-20 antioxidant: 10-129 tonicifier : 10-129 ionic strength provider: 0.001-2 chelator.

E1146. 0.81-1.49 secukinumab : 200-250 sugar component : 20-99 amino acid component : 0.008-0.92 surfactant : 0.1-20 antioxidant: 10-129 tonicifier: 10-129 ionic strength provider: 0.001-2 chelator.

E1147. 0.81-1.49 secukinumab : 200-250 sugar component : 100-149 amino acid component : 0.008-0.92 surfactant : 0.1-20 antioxidant: 10-129 tonicifier: 10-129 ionic strength provider: 0.001-2 chelator.

E1148. 0.81-1.49 secukinumab : 1-9 buffer system : 200-250 sugar component : 20-99 amino acid component : 0.008-0.92 surfactant: 0.1-20 antioxidant: 10-129 tonicifier : 10-129 ionic strength provider: 0.001-2 chelator.

E1149. 0.81-1.49 secukinumab : 51-60 buffer system : 200-250 sugar component : 20-99 amino acid component : 0.008-0.92 surfactant: 0.1-20 antioxidant: 10-129 tonicifier : 10-129 ionic strength provider: 0.001-2 chelator.

E1150. 0.81-1.49 secukinumab : 1-9 buffer system : 200-250 sugar component : 100-149 amino acid component : 0.008-0.92 surfactant: 0.1-20 antioxidant: 10-129 tonicifier : 10-129 ionic strength provider: 0.001-2 chelator.

E1151. 0.81-1.49 secukinumab : 51-60 buffer system : 200-250 sugar component : 100-149 amino acid component : 0.008-0.92 surfactant: 0.1-20 antioxidant: 10-129 tonicifier : 10-129 ionic strength provider: 0.001-2 chelator.

The embodiments of E1-E1151 may be further defined or characterised by features of numbered paragraphs F1-F12, G1-G959, H1-H1423, 11-12400, and J1-J1158.

*Potentially Absent Ingredients*

F) General Aspects and Embodiments Relating to Absent Ingredients

The biopharmaceutical composition suitably comprises, or consists of (suitably along with a diluent, which is preferably water), a biopharmaceutical active (which is preferably secukinumab) and one or more other ingredients/components (e.g. any, some, or all of one or more buffer systems, one or more sugar components, one or more amino acid components, one or more surfactants, one or more tonicifiers, one or more ionic-strength providers, one or more antioxidants, one or more chelators, and/or one or more diluents), but is further characterised by an absence of one or more particular ingredients / components (e.g. an absence of a histidine buffer system; an absence of a buffer system, suitably an absence of any, some, or all buffer systems as defined herein; an absence of trehalose and sucrose; an absence of a sugar component, suitably an absence of any, some, or all sugar components defined herein; an absence of histidine and methionine; an absence of an amino acid component, suitably an absence of any, some, or all amino acid components defined herein; an absence of polysorbate 80; an absence of surfactant, suitably an absence of any, some, or all surfactants defined herein; an absence of a (further) tonicifier, suitably an absence of any, some, or all tonicifiers defined herein; an absence of a (further) ionic strength provider, suitably an absence of any, some, or all ionic strength providers defined herein; an absence of methionine; and/or an absence of an antioxidant, suitably an absence of any, some, or all antioxidants defined herein). The following numbered paragraphs F1-F12 disclose specific aspects and embodiments of the invention, wherein the biopharmaceutical composition comprises, consists of (suitably along with a diluent, which is preferably water), and/or is otherwise characterised by:

F1. secukinumab; and an absence of a histidine buffer system.

F2. secukinumab; and an absence of a buffer system (suitably an absence of any, some, or all buffer systems defined herein).

F3. secukinumab; and an absence of trehalose and sucrose.

F4. secukinumab; and an absence of a sugar component (suitably an absence of any, some, or all sugar components defined herein) .

F5. secukinumab; and an absence of histidine and methionine.

F6. secukinumab; and an absence of an amino acid component (suitably an absence of any, some, or all amino acid components defined herein) or, for compositions that do (or optionally) contain an amino acid, they are preferably free of ALL of the following amino acids: methionine, histidine, arginine, glycine, proline, and lysine.

F7. secukinumab; and an absence of polysorbate 80.

(continued)

F8. secukinumab; and an absence of surfactant (suitably an absence of any, some, or all surfactants defined herein).

F9. secukinumab; and an absence of a (further) tonicifier (suitably an absence of any, some, or all tonicifiers defined herein).

F10. secukinumab; and an absence of a (further) ionic strength provider (suitably an absence of any, some, or all ionic strength providers defined herein).

F11. secukinumab; and an absence of methionine.

F12. secukinumab; and an absence of an antioxidant (suitably an absence of any, some, or all antioxidants defined herein).

The aspects and embodiments of F1-F12 may be suitably interdependent such that one or more absences/exclusions apply.

The embodiments of F1-F12 may be further defined or characterised by features of numbered paragraphs A1-A511, B1-B255, C1-C255, D1-D1152, E1-E1151, G1-G959, and H1-H1423.

It is particularly preferable that compositions of the invention exclude (i.e. be free of) histidine (or histidine buffer systems). It is also preferred for compositions of the invention to exclude trehalose and sucrose, though especially trehalose. It is especially preferred that compositions of the invention exclude methionine - indeed the present invention may provide alternative solutions to using methionine (or indeed using any antioxidants), or otherwise compensates for a lack thereof. Most preferably, compositions of the invention exclude all of the aforesaid.

For compositions that do (or optionally) contain an amino acid, they are preferably free of ALL of the following amino acids: methionine, histidine, arginine, glycine, proline, and lysine.

G) General Aspects and Embodiments Relating to Combinations of Absent Ingredients

As such, the following further numbered paragraphs G1-G959 (which are suitably based on any of F1-F12) disclose specific aspects and embodiments of the invention, wherein the biopharmaceutical composition comprises, consists of (suitably along with a diluent, which is preferably water), and/or is otherwise characterised by:

G1. secukinumab; an absence of a histidine buffer system; and an absence of trehalose and sucrose.

G2. secukinumab; an absence of a buffer system; and an absence of trehalose and sucrose.

G3. secukinumab; an absence of a histidine buffer system; and an absence of a sugar component.

G4. secukinumab; an absence of a buffer system; and an absence of a sugar component.

G5. secukinumab; an absence of a histidine buffer system; and an absence of histidine and methionine.

G6. secukinumab; an absence of a buffer system; and an absence of histidine and methionine.

G7. secukinumab; an absence of a histidine buffer system; and an absence of an amino acid component.

G8. secukinumab; an absence of a buffer system; and an absence of an amino acid component.

G9. secukinumab; an absence of a histidine buffer system; and an absence of polysorbate 80.

G10. secukinumab; an absence of a buffer system; and an absence of polysorbate 80.

G11. secukinumab; an absence of a histidine buffer system; and an absence of a surfactant.

G12. secukinumab; an absence of a buffer system; and an absence of a surfactant.

G13. secukinumab; an absence of a histidine buffer system; and an absence of a (further) tonicifier.

G14. secukinumab; an absence of a buffer system; and an absence of a (further) tonicifier.

G15. secukinumab; an absence of a histidine buffer system; and an absence of a (further) ionic strength provider.

G16. secukinumab; an absence of a buffer system; and an absence of a (further) ionic strength provider.

G17. secukinumab; an absence of a histidine buffer system; and an absence of methionine.

G18. secukinumab; an absence of a buffer system; and an absence of methionine.

G19. secukinumab; an absence of a histidine buffer system; and an absence of an antioxidant.

G20. secukinumab; an absence of a buffer system; and an absence of an antioxidant.

G21. secukinumab; an absence of trehalose and sucrose; and an absence of histidine and methionine.

G22. secukinumab; an absence of a sugar component; and an absence of histidine and methionine.

G23. secukinumab; an absence of trehalose and sucrose; and an absence of an amino acid component.

G24. secukinumab; an absence of a sugar component; and an absence of an amino acid component.

G25. secukinumab; an absence of trehalose and sucrose; and an absence of polysorbate 80.

(continued)

G26. secukinumab; an absence of a sugar component; and an absence of polysorbate 80.
G27. secukinumab; an absence of trehalose and sucrose; and an absence of a surfactant.
G28. secukinumab; an absence of a sugar component; and an absence of a surfactant.
G29. secukinumab; an absence of trehalose and sucrose; and an absence of a (further) tonicifier.
G30. secukinumab; an absence of a sugar component; and an absence of a (further) tonicifier.
G31. secukinumab; an absence of trehalose and sucrose; and an absence of a (further) ionic strength provider.
G32. secukinumab; an absence of a sugar component; and an absence of a (further) ionic strength provider.
G33. secukinumab; an absence of trehalose and sucrose; and an absence of methionine.
G34. secukinumab; an absence of a sugar component; and an absence of methionine.
G35. secukinumab; an absence of trehalose and sucrose; and an absence of an antioxidant.
G36. secukinumab; an absence of a sugar component; and an absence of an antioxidant.
G37. secukinumab; an absence of histidine and methionine; and an absence of polysorbate 80.
G38. secukinumab; an absence of an amino acid component; and an absence of polysorbate 80.
G39. secukinumab; an absence of histidine and methionine; and an absence of a surfactant.
G40. secukinumab; an absence of an amino acid component; and an absence of a surfactant.
G41. secukinumab; an absence of histidine and methionine; and an absence of a (further) tonicifier.
G42. secukinumab; an absence of an amino acid component; and an absence of a (further) tonicifier.
G43. secukinumab; an absence of histidine and methionine; and an absence of a (further) ionic strength provider.
G44. secukinumab; an absence of an amino acid component; and an absence of a (further) ionic strength provider.
G45. secukinumab; an absence of histidine and methionine; and an absence of methionine.
G46. secukinumab; an absence of an amino acid component; and an absence of methionine.
G47. secukinumab; an absence of histidine and methionine; and an absence of an antioxidant.
G48. secukinumab; an absence of an amino acid component; and an absence of an antioxidant.
G49. secukinumab; an absence of polysorbate 80; and an absence of a (further) tonicifier.
G50. secukinumab; an absence of a surfactant; and an absence of a (further) tonicifier.
G51. secukinumab; an absence of polysorbate 80; and an absence of a (further) ionic strength provider.
G52. secukinumab; an absence of a surfactant; and an absence of a (further) ionic strength provider.
G53. secukinumab; an absence of polysorbate 80; and an absence of methionine.
G54. secukinumab; an absence of a surfactant; and an absence of methionine.
G55. secukinumab; an absence of polysorbate 80; and an absence of an antioxidant.
G56. secukinumab; an absence of a surfactant; and an absence of an antioxidant.
G57. secukinumab; an absence of a (further) tonicifier; and an absence of a (further) ionic strength provider.
G58. secukinumab; an absence of a (further) tonicifier; and an absence of methionine.
G59. secukinumab; an absence of a (further) tonicifier; and an absence of an antioxidant.
G60. secukinumab; an absence of a (further) ionic strength provider; and an absence of methionine.
G61. secukinumab; an absence of a (further) ionic strength provider; and an absence of an antioxidant.
G62. secukinumab; an absence of a histidine buffer system; an absence of trehalose and sucrose; and an absence of histidine and methionine.
G63. secukinumab; an absence of a buffer system; an absence of trehalose and sucrose; and an absence of histidine and methionine.
G64. secukinumab; an absence of a histidine buffer system; an absence of a sugar component; and an absence of histidine and methionine.
G65. secukinumab; an absence of a buffer system; an absence of a sugar component; and an absence of histidine and methionine.
G66. secukinumab; an absence of a histidine buffer system; an absence of trehalose and sucrose; and an absence of an amino acid component.
G67. secukinumab; an absence of a buffer system; an absence of trehalose and sucrose; and an absence of an amino acid component.
G68. secukinumab; an absence of a histidine buffer system; an absence of a sugar component; and an absence of an amino acid component.

(continued)

G69. secukinumab; an absence of a buffer system; an absence of a sugar component; and an absence of an amino acid component.

G70. secukinumab; an absence of a histidine buffer system; an absence of trehalose and sucrose; and an absence of polysorbate 80.

G71. secukinumab; an absence of a buffer system; an absence of trehalose and sucrose; and an absence of polysorbate 80.

G72. secukinumab; an absence of a histidine buffer system; an absence of a sugar component; and an absence of polysorbate 80.

G73. secukinumab; an absence of a buffer system; an absence of a sugar component; and an absence of polysorbate 80.

G74. secukinumab; an absence of a histidine buffer system; an absence of trehalose and sucrose; and an absence of a surfactant.

G75. secukinumab; an absence of a buffer system; an absence of trehalose and sucrose; and an absence of a surfactant.

G76. secukinumab; an absence of a histidine buffer system; an absence of a sugar component; and an absence of a surfactant.

G77. secukinumab; an absence of a buffer system; an absence of a sugar component; and an absence of a surfactant.

G78. secukinumab; an absence of a histidine buffer system; an absence of trehalose and sucrose; and an absence of a (further) tonicifier.

G79. secukinumab; an absence of a buffer system; an absence of trehalose and sucrose; and an absence of a (further) tonicifier.

G80. secukinumab; an absence of a histidine buffer system; an absence of a sugar component; and an absence of a (further) tonicifier.

G81. secukinumab; an absence of a buffer system; an absence of a sugar component; and an absence of a (further) tonicifier.

G82. secukinumab; an absence of a histidine buffer system; an absence of trehalose and sucrose; and an absence of a (further) ionic strength provider.

G83. secukinumab; an absence of a buffer system; an absence of trehalose and sucrose; and an absence of a (further) ionic strength provider.

G84. secukinumab; an absence of a histidine buffer system; an absence of a sugar component; and an absence of a (further) ionic strength provider.

G85. secukinumab; an absence of a buffer system; an absence of a sugar component; and an absence of a (further) ionic strength provider.

G86. secukinumab; an absence of a histidine buffer system; an absence of trehalose and sucrose; and an absence of methionine.

G87. secukinumab; an absence of a buffer system; an absence of trehalose and sucrose; and an absence of methionine.

G88. secukinumab; an absence of a histidine buffer system; an absence of a sugar component; and an absence of methionine.

G89. secukinumab; an absence of a buffer system; an absence of a sugar component; and an absence of methionine.

G90. secukinumab; an absence of a histidine buffer system; an absence of trehalose and sucrose; and an absence of an antioxidant.

(continued)

G91. secukinumab; an absence of a buffer system; an absence of trehalose and sucrose; and an absence of an antioxidant.

G92. secukinumab; an absence of a histidine buffer system; an absence of a sugar component; and an absence of an antioxidant.

G93. secukinumab; an absence of a buffer system; an absence of a sugar component; and an absence of an antioxidant.

G94. secukinumab; an absence of a histidine buffer system; an absence of histidine and methionine; and an absence of polysorbate 80.

G95. secukinumab; an absence of a buffer system; an absence of histidine and methionine; and an absence of polysorbate 80.

G96. secukinumab; an absence of a histidine buffer system; an absence of an amino acid component; and an absence of polysorbate 80.

G97. secukinumab; an absence of a buffer system; an absence of an amino acid component; and an absence of polysorbate 80.

G98. secukinumab; an absence of a histidine buffer system; an absence of histidine and methionine; and an absence of a surfactant.

G99. secukinumab; an absence of a buffer system; an absence of histidine and methionine; and an absence of a surfactant.

G100. secukinumab; an absence of a histidine buffer system; an absence of an amino acid component; and an absence of a surfactant.

G101. secukinumab; an absence of a buffer system; an absence of an amino acid component; and an absence of a surfactant.

G102. secukinumab; an absence of a histidine buffer system; an absence of histidine and methionine; and an absence of a (further) tonicifier.

G103. secukinumab; an absence of a buffer system; an absence of histidine and methionine; and an absence of a (further) tonicifier.

G104. secukinumab; an absence of a histidine buffer system; an absence of an amino acid component; and an absence of a (further) tonicifier.

G105. secukinumab; an absence of a buffer system; an absence of an amino acid component; and an absence of a (further) tonicifier.

G106. secukinumab; an absence of a histidine buffer system; an absence of histidine and methionine; and an absence of a (further) ionic strength provider.

G107. secukinumab; an absence of a buffer system; an absence of histidine and methionine; and an absence of a (further) ionic strength provider.

G108. secukinumab; an absence of a histidine buffer system; an absence of an amino acid component; and an absence of a (further) ionic strength provider.

G109. secukinumab; an absence of a buffer system; an absence of an amino acid component; and an absence of a (further) ionic strength provider.

G110. secukinumab; an absence of a histidine buffer system; an absence of histidine and methionine; and an absence of methionine .

(continued)

G111. secukinumab; an absence of a buffer system; an absence of histidine and methionine; and an absence of methionine.

G112. secukinumab; an absence of a histidine buffer system; an absence of an amino acid component; and an absence of methionine .

G113. secukinumab; an absence of a buffer system; an absence of an amino acid component; and an absence of methionine.

G114. secukinumab; an absence of a histidine buffer system; an absence of histidine and methionine; and an absence of an antioxidant.

G115. secukinumab; an absence of a buffer system; an absence of histidine and methionine; and an absence of an antioxidant.

G116. secukinumab; an absence of a histidine buffer system; an absence of an amino acid component; and an absence of an antioxidant.

G117. secukinumab; an absence of a buffer system; an absence of an amino acid component; and an absence of an antioxidant.

G118. secukinumab; an absence of a histidine buffer system; an absence of polysorbate 80; and an absence of a (further) tonicifier.

G119. secukinumab; an absence of a buffer system; an absence of polysorbate 80; and an absence of a (further) tonicifier.

G120. secukinumab; an absence of a histidine buffer system; an absence of a surfactant; and an absence of a (further) tonicifier.

G121. secukinumab; an absence of a buffer system; an absence of a surfactant; and an absence of a (further) tonicifier.

G122. secukinumab; an absence of a histidine buffer system; an absence of polysorbate 80; and an absence of a (further) ionic strength provider.

G123. secukinumab; an absence of a buffer system; an absence of polysorbate 80; and an absence of a (further) ionic strength provider.

G124. secukinumab; an absence of a histidine buffer system; an absence of a surfactant; and an absence of a (further) ionic strength provider.

G125. secukinumab; an absence of a buffer system; an absence of a surfactant; and an absence of a (further) ionic strength provider.

G126. secukinumab; an absence of a histidine buffer system; an absence of polysorbate 80; and an absence of methionine.

G127. secukinumab; an absence of a buffer system; an absence of polysorbate 80; and an absence of methionine.

G128. secukinumab; an absence of a histidine buffer system; an absence of a surfactant; and an absence of methionine.

G129. secukinumab; an absence of a buffer system; an absence of a surfactant; and an absence of methionine.

G130. secukinumab; an absence of a histidine buffer system; an absence of polysorbate 80; and an absence of an antioxidant.

G131. secukinumab; an absence of a buffer system; an absence of polysorbate 80; and an absence of an antioxidant.

G132. secukinumab; an absence of a histidine buffer system; an absence of a surfactant; and an absence of an antioxidant.

G133. secukinumab; an absence of a buffer system; an absence of a surfactant; and an absence of an antioxidant.

G134. secukinumab; an absence of a histidine buffer system; an absence of a (further) tonicifier; and an absence of a (further) ionic strength provider.

(continued)

G135. secukinumab; an absence of a buffer system; an absence of a (further) tonicifier; and an absence of a (further) ionic strength provider.

G136. secukinumab; an absence of a histidine buffer system; an absence of a (further) tonicifier; and an absence of methionine.

G137. secukinumab; an absence of a buffer system; an absence of a (further) tonicifier; and an absence of methionine.

G138. secukinumab; an absence of a histidine buffer system; an absence of a (further) tonicifier; and an absence of an antioxidant.

G139. secukinumab; an absence of a buffer system; an absence of a (further) tonicifier; and an absence of an antioxidant.

G140. secukinumab; an absence of a histidine buffer system; an absence of a (further) ionic strength provider; and an absence of methionine .

G141. secukinumab; an absence of a buffer system; an absence of a (further) ionic strength provider; and an absence of methionine .

G142. secukinumab; an absence of a histidine buffer system; an absence of a (further) ionic strength provider; and an absence of an antioxidant.

G143. secukinumab; an absence of a buffer system; an absence of a (further) ionic strength provider; and an absence of an antioxidant.

G144. secukinumab; an absence of trehalose and sucrose; an absence of histidine and methionine; and an absence of polysorbate 80.

G145. secukinumab; an absence of a sugar component; an absence of histidine and methionine; and an absence of polysorbate 80.

G146. secukinumab; an absence of trehalose and sucrose; an absence of an amino acid component; and an absence of polysorbate 80.

G147. secukinumab; an absence of a sugar component; an absence of an amino acid component; and an absence of polysorbate 80.

G148. secukinumab; an absence of trehalose and sucrose; an absence of histidine and methionine; and an absence of a surfactant.

G149. secukinumab; an absence of a sugar component; an absence of histidine and methionine; and an absence of a surfactant.

G150. secukinumab; an absence of trehalose and sucrose; an absence of an amino acid component; and an absence of a surfactant.

G151. secukinumab; an absence of a sugar component; an absence of an amino acid component; and an absence of a surfactant.

G152. secukinumab; an absence of trehalose and sucrose; an absence of histidine and methionine; and an absence of a (further) tonicifier.

G153. secukinumab; an absence of a sugar component; an absence of histidine and methionine; and an absence of a (further) tonicifier.

G154. secukinumab; an absence of trehalose and sucrose; an absence of an amino acid component; and an absence of a (further)

(continued)

tonicifier.

G155. secukinumab; an absence of a sugar component; an absence of an amino acid component; and an absence of a (further) tonicifier.

G156. secukinumab; an absence of trehalose and sucrose; an absence of histidine and methionine; and an absence of a (further) ionic strength provider.

G157. secukinumab; an absence of a sugar component; an absence of histidine and methionine; and an absence of a (further) ionic strength provider.

G158. secukinumab; an absence of trehalose and sucrose; an absence of an amino acid component; and an absence of a (further) ionic strength provider.

G159. secukinumab; an absence of a sugar component; an absence of an amino acid component; and an absence of a (further) ionic strength provider.

G160. secukinumab; an absence of trehalose and sucrose; an absence of histidine and methionine; and an absence of methionine.

G161. secukinumab; an absence of a sugar component; an absence of histidine and methionine; and an absence of methionine.

G162. secukinumab; an absence of trehalose and sucrose; an absence of an amino acid component; and an absence of methionine .

G163. secukinumab; an absence of a sugar component; an absence of an amino acid component; and an absence of methionine.

G164. secukinumab; an absence of trehalose and sucrose; an absence of histidine and methionine; and an absence of an antioxidant.

G165. secukinumab; an absence of a sugar component; an absence of histidine and methionine; and an absence of an antioxidant.

G166. secukinumab; an absence of trehalose and sucrose; an absence of an amino acid component; and an absence of an antioxidant.

G167. secukinumab; an absence of a sugar component; an absence of an amino acid component; and an absence of an antioxidant.

G168. secukinumab; an absence of trehalose and sucrose; an absence of polysorbate 80; and an absence of a (further) tonicifier.

G169. secukinumab; an absence of a sugar component; an absence of polysorbate 80; and an absence of a (further) tonicifier.

G170. secukinumab; an absence of trehalose and sucrose; an absence of a surfactant; and an absence of a (further) tonicifier.

G171. secukinumab; an absence of a sugar component; an absence of a surfactant; and an absence of a (further) tonicifier.

G172. secukinumab; an absence of trehalose and sucrose; an absence of polysorbate 80; and an absence of a (further) ionic strength provider.

G173. secukinumab; an absence of a sugar component; an absence of polysorbate 80; and an absence of a (further) ionic strength provider.

G174. secukinumab; an absence of trehalose and sucrose; an absence of a surfactant; and an absence of a (further) ionic strength provider.

(continued)

G175. secukinumab; an absence of a sugar component; an absence of a surfactant; and an absence of a (further) ionic strength provider.

G176. secukinumab; an absence of trehalose and sucrose; an absence of polysorbate 80; and an absence of methionine.

G177. secukinumab; an absence of a sugar component; an absence of polysorbate 80; and an absence of methionine.

G178. secukinumab; an absence of trehalose and sucrose; an absence of a surfactant; and an absence of methionine.

G179. secukinumab; an absence of a sugar component; an absence of a surfactant; and an absence of methionine.

G180. secukinumab; an absence of trehalose and sucrose; an absence of polysorbate 80; and an absence of an antioxidant.

G181. secukinumab; an absence of a sugar component; an absence of polysorbate 80; and an absence of an antioxidant.

G182. secukinumab; an absence of trehalose and sucrose; an absence of a surfactant; and an absence of an antioxidant.

G183. secukinumab; an absence of a sugar component; an absence of a surfactant; and an absence of an antioxidant.

G184. secukinumab; an absence of trehalose and sucrose; an absence of a (further) tonicifier; and an absence of a (further) ionic strength provider.

G185. secukinumab; an absence of a sugar component; an absence of a (further) tonicifier; and an absence of a (further) ionic strength provider.

G186. secukinumab; an absence of trehalose and sucrose; an absence of a (further) tonicifier; and an absence of methionine.

G187. secukinumab; an absence of a sugar component; an absence of a (further) tonicifier; and an absence of methionine.

G188. secukinumab; an absence of trehalose and sucrose; an absence of a (further) tonicifier; and an absence of an antioxidant.

G189. secukinumab; an absence of a sugar component; an absence of a (further) tonicifier; and an absence of an antioxidant.

G190. secukinumab; an absence of trehalose and sucrose; an absence of a (further) ionic strength provider; and an absence of methionine .

G191. secukinumab; an absence of a sugar component; an absence of a (further) ionic strength provider; and an absence of methionine .

G192. secukinumab; an absence of trehalose and sucrose; an absence of a (further) ionic strength provider; and an absence of an antioxidant.

G193. secukinumab; an absence of a sugar component; an absence of a (further) ionic strength provider; and an absence of an antioxidant.

G194. secukinumab; an absence of histidine and methionine; an absence of polysorbate 80; and an absence of a (further) tonicifier.

G195. secukinumab; an absence of an amino acid component; an absence of polysorbate 80; and an absence of a (further) tonicifier.

G196. secukinumab; an absence of histidine and methionine; an absence of a surfactant; and an absence of a (further) tonicifier.

G197. secukinumab; an absence of an amino acid component; an absence of a surfactant; and an absence of a (further) tonicifier.

(continued)

G198. secukinumab; an absence of histidine and methionine; an absence of polysorbate 80; and an absence of a (further) ionic strength provider.

G199. secukinumab; an absence of an amino acid component; an absence of polysorbate 80; and an absence of a (further) ionic strength provider.

G200. secukinumab; an absence of histidine and methionine; an absence of a surfactant; and an absence of a (further) ionic strength provider.

G201. secukinumab; an absence of an amino acid component; an absence of a surfactant; and an absence of a (further) ionic strength provider.

G202. secukinumab; an absence of histidine and methionine; an absence of polysorbate 80; and an absence of methionine.

G203. secukinumab; an absence of an amino acid component; an absence of polysorbate 80; and an absence of methionine.

G204. secukinumab; an absence of histidine and methionine; an absence of a surfactant; and an absence of methionine.

G205. secukinumab; an absence of an amino acid component; an absence of a surfactant; and an absence of methionine.

G206. secukinumab; an absence of histidine and methionine; an absence of polysorbate 80; and an absence of an antioxidant.

G207. secukinumab; an absence of an amino acid component; an absence of polysorbate 80; and an absence of an antioxidant.

G208. secukinumab; an absence of histidine and methionine; an absence of a surfactant; and an absence of an antioxidant.

G209. secukinumab; an absence of an amino acid component; an absence of a surfactant; and an absence of an antioxidant.

G210. secukinumab; an absence of histidine and methionine; an absence of a (further) tonicifier; and an absence of a (further) ionic strength provider.

G211. secukinumab; an absence of an amino acid component; an absence of a (further) tonicifier; and an absence of a (further) ionic strength provider.

G212. secukinumab; an absence of histidine and methionine; an absence of a (further) tonicifier; and an absence of methionine.

G213. secukinumab; an absence of an amino acid component; an absence of a (further) tonicifier; and an absence of methionine.

G214. secukinumab; an absence of histidine and methionine; an absence of a (further) tonicifier; and an absence of an antioxidant.

G215. secukinumab; an absence of an amino acid component; an absence of a (further) tonicifier; and an absence of an antioxidant.

G216. secukinumab; an absence of histidine and methionine; an absence of a (further) ionic strength provider; and an absence of methionine.

G217. secukinumab; an absence of an amino acid component; an absence of a (further) ionic strength provider; and an absence of methionine.

G218. secukinumab; an absence of histidine and methionine; an absence of a (further) ionic strength provider; and an absence of an antioxidant.

(continued)

G219. secukinumab; an absence of an amino acid component; an absence of a (further) ionic strength provider; and an absence of an antioxidant.

G220. secukinumab; an absence of polysorbate 80; an absence of a (further) tonicifier; and an absence of a (further) ionic strength provider.

G221. secukinumab; an absence of a surfactant; an absence of a (further) tonicifier; and an absence of a (further) ionic strength provider.

G222. secukinumab; an absence of polysorbate 80; an absence of a (further) tonicifier; and an absence of methionine.

G223. secukinumab; an absence of a surfactant; an absence of a (further) tonicifier; and an absence of methionine.

G224. secukinumab; an absence of polysorbate 80; an absence of a (further) tonicifier; and an absence of an antioxidant.

G225. secukinumab; an absence of a surfactant; an absence of a (further) tonicifier; and an absence of an antioxidant.

G226. secukinumab; an absence of polysorbate 80; an absence of a (further) ionic strength provider; and an absence of methionine .

G227. secukinumab; an absence of a surfactant; an absence of a (further) ionic strength provider; and an absence of methionine.

G228. secukinumab; an absence of polysorbate 80; an absence of a (further) ionic strength provider; and an absence of an antioxidant.

G229. secukinumab; an absence of a surfactant; an absence of a (further) ionic strength provider; and an absence of an antioxidant.

G230. secukinumab; an absence of a (further) tonicifier; an absence of a (further) ionic strength provider; and an absence of methionine .

G231. secukinumab; an absence of a (further) tonicifier; an absence of a (further) ionic strength provider; and an absence of an antioxidant.

G232. secukinumab; an absence of a histidine buffer system; an absence of trehalose and sucrose; an absence of histidine and methionine; and an absence of polysorbate 80.

G233. secukinumab; an absence of a buffer system; an absence of trehalose and sucrose; an absence of histidine and methionine; and an absence of polysorbate 80.

G234. secukinumab; an absence of a histidine buffer system; an absence of a sugar component; an absence of histidine and methionine; and an absence of polysorbate 80.

G235. secukinumab; an absence of a buffer system; an absence of a sugar component; an absence of histidine and methionine; and an absence of polysorbate 80.

G236. secukinumab; an absence of a histidine buffer system; an absence of trehalose and sucrose; an absence of an amino acid component; and an absence of polysorbate 80.

G237. secukinumab; an absence of a buffer system; an absence of trehalose and sucrose; an absence of an amino acid component; and an absence of polysorbate 80.

(continued)

G238. secukinumab; an absence of a histidine buffer system; an absence of a sugar component; an absence of an amino acid component; and an absence of polysorbate 80.

G239. secukinumab; an absence of a buffer system; an absence of a sugar component; an absence of an amino acid component; and an absence of polysorbate 80.

G240. secukinumab; an absence of a histidine buffer system; an absence of trehalose and sucrose; an absence of histidine and methionine; and an absence of a surfactant.

G241. secukinumab; an absence of a buffer system; an absence of trehalose and sucrose; an absence of histidine and methionine; and an absence of a surfactant.

G242. secukinumab; an absence of a histidine buffer system; an absence of a sugar component; an absence of histidine and methionine; and an absence of a surfactant.

G243. secukinumab; an absence of a buffer system; an absence of a sugar component; an absence of histidine and methionine; and an absence of a surfactant.

G244. secukinumab; an absence of a histidine buffer system; an absence of trehalose and sucrose; an absence of an amino acid component; and an absence of a surfactant.

G245. secukinumab; an absence of a buffer system; an absence of trehalose and sucrose; an absence of an amino acid component; and an absence of a surfactant.

G246. secukinumab; an absence of a histidine buffer system; an absence of a sugar component; an absence of an amino acid component; and an absence of a surfactant.

G247. secukinumab; an absence of a buffer system; an absence of a sugar component; an absence of an amino acid component; and an absence of a surfactant.

G248. secukinumab; an absence of a histidine buffer system; an absence of trehalose and sucrose; an absence of histidine and methionine; and an absence of a (further) tonicifier.

G249. secukinumab; an absence of a buffer system; an absence of trehalose and sucrose; an absence of histidine and methionine; and an absence of a (further) tonicifier.

G250. secukinumab; an absence of a histidine buffer system; an absence of a sugar component; an absence of histidine and methionine; and an absence of a (further) tonicifier.

G251. secukinumab; an absence of a buffer system; an absence of a sugar component; an absence of histidine and methionine; and an absence of a (further) tonicifier.

G252. secukinumab; an absence of a histidine buffer system; an absence of trehalose and sucrose; an absence of an amino acid component; and an absence of a (further) tonicifier.

G253. secukinumab; an absence of a buffer system; an absence of trehalose and sucrose; an absence of an amino acid component; and an absence of a (further) tonicifier.

G254. secukinumab; an absence of a histidine buffer system; an absence of a sugar component; an absence of an amino acid component; and an absence of a (further) tonicifier.

(continued)

G255. secukinumab; an absence of a buffer system; an absence of a sugar component; an absence of an amino acid component;
and an absence of a (further) tonicifier.

G256. secukinumab; an absence of a histidine buffer system; an absence of trehalose and sucrose; an absence of histidine and
methionine; and an absence of a (further) ionic strength provider.

G257. secukinumab; an absence of a buffer system; an absence of trehalose and sucrose; an absence of histidine and methionine;
and an absence of a (further) ionic strength provider.

G258. secukinumab; an absence of a histidine buffer system; an absence of a sugar component; an absence of histidine and
methionine; and an absence of a (further) ionic strength provider.

G259. secukinumab; an absence of a buffer system; an absence of a sugar component; an absence of histidine and methionine;
and an absence of a (further) ionic strength provider.

G260. secukinumab; an absence of a histidine buffer system; an absence of trehalose and sucrose; an absence of an amino acid
component; and an absence of a (further) ionic strength provider.

G261. secukinumab; an absence of a buffer system; an absence of trehalose and sucrose; an absence of an amino acid
component; and an absence of a (further) ionic strength provider.

G262. secukinumab; an absence of a histidine buffer system; an absence of a sugar component; an absence of an amino acid
component; and an absence of a (further) ionic strength provider.

G263. secukinumab; an absence of a buffer system; an absence of a sugar component; an absence of an amino acid component;
and an absence of a (further) ionic strength provider.

G264. secukinumab; an absence of a histidine buffer system; an absence of trehalose and sucrose; an absence of histidine and
methionine; and an absence of methionine.

G265. secukinumab; an absence of a buffer system; an absence of trehalose and sucrose; an absence of histidine and methionine;
and an absence of methionine.

G266. secukinumab; an absence of a histidine buffer system; an absence of a sugar component; an absence of histidine and
methionine; and an absence of methionine.

G267. secukinumab; an absence of a buffer system; an absence of a sugar component; an absence of histidine and methionine;
and an absence of methionine.

G268. secukinumab; an absence of a histidine buffer system; an absence of trehalose and sucrose; an absence of an amino acid
component; and an absence of methionine.

G269. secukinumab; an absence of a buffer system; an absence of trehalose and sucrose; an absence of an amino acid
component; and an absence of methionine.

G270. secukinumab; an absence of a histidine buffer system; an absence of a sugar component; an absence of an amino acid
component; and an absence of methionine.

G271. secukinumab; an absence of a buffer system; an absence of a sugar component; an absence of an amino acid component;
and an absence of methionine.

(continued)

G272. secukinumab; an absence of a histidine buffer system; an absence of trehalose and sucrose; an absence of histidine and methionine; and an absence of an antioxidant.

G273. secukinumab; an absence of a buffer system; an absence of trehalose and sucrose; an absence of histidine and methionine; and an absence of an antioxidant.

G274. secukinumab; an absence of a histidine buffer system; an absence of a sugar component; an absence of histidine and methionine; and an absence of an antioxidant.

G275. secukinumab; an absence of a buffer system; an absence of a sugar component; an absence of histidine and methionine; and an absence of an antioxidant.

G276. secukinumab; an absence of a histidine buffer system; an absence of trehalose and sucrose; an absence of an amino acid component; and an absence of an antioxidant.

G277. secukinumab; an absence of a buffer system; an absence of trehalose and sucrose; an absence of an amino acid component; and an absence of an antioxidant.

G278. secukinumab; an absence of a histidine buffer system; an absence of a sugar component; an absence of an amino acid component; and an absence of an antioxidant.

G279. secukinumab; an absence of a buffer system; an absence of a sugar component; an absence of an amino acid component; and an absence of an antioxidant.

G280. secukinumab; an absence of a histidine buffer system; an absence of trehalose and sucrose; an absence of polysorbate 80; and an absence of a (further) tonicifier.

G281. secukinumab; an absence of a buffer system; an absence of trehalose and sucrose; an absence of polysorbate 80; and an absence of a (further) tonicifier.

G282. secukinumab; an absence of a histidine buffer system; an absence of a sugar component; an absence of polysorbate 80; and an absence of a (further) tonicifier.

G283. secukinumab; an absence of a buffer system; an absence of a sugar component; an absence of polysorbate 80; and an absence of a (further) tonicifier.

G284. secukinumab; an absence of a histidine buffer system; an absence of trehalose and sucrose; an absence of a surfactant; and an absence of a (further) tonicifier.

G285. secukinumab; an absence of a buffer system; an absence of trehalose and sucrose; an absence of a surfactant; and an absence of a (further) tonicifier.

G286. secukinumab; an absence of a histidine buffer system; an absence of a sugar component; an absence of a surfactant; and an absence of a (further) tonicifier.

G287. secukinumab; an absence of a buffer system; an absence of a sugar component; an absence of a surfactant; and an absence of a (further) tonicifier.

G288. secukinumab; an absence of a histidine buffer system; an absence of trehalose and sucrose; an absence of polysorbate 80; and an absence of a (further) ionic strength provider.

(continued)

G289. secukinumab; an absence of a buffer system; an absence of trehalose and sucrose; an absence of polysorbate 80; and an absence of a (further) ionic strength provider.

G290. secukinumab; an absence of a histidine buffer system; an absence of a sugar component; an absence of polysorbate 80; and an absence of a (further) ionic strength provider.

G291. secukinumab; an absence of a buffer system; an absence of a sugar component; an absence of polysorbate 80; and an absence of a (further) ionic strength provider.

G292. secukinumab; an absence of a histidine buffer system; an absence of trehalose and sucrose; an absence of a surfactant; and an absence of a (further) ionic strength provider.

G293. secukinumab; an absence of a buffer system; an absence of trehalose and sucrose; an absence of a surfactant; and an absence of a (further) ionic strength provider.

G294. secukinumab; an absence of a histidine buffer system; an absence of a sugar component; an absence of a surfactant; and an absence of a (further) ionic strength provider.

G295. secukinumab; an absence of a buffer system; an absence of a sugar component; an absence of a surfactant; and an absence of a (further) ionic strength provider.

G296. secukinumab; an absence of a histidine buffer system; an absence of trehalose and sucrose; an absence of polysorbate 80; and an absence of methionine.

G297. secukinumab; an absence of a buffer system; an absence of trehalose and sucrose; an absence of polysorbate 80; and an absence of methionine.

G298. secukinumab; an absence of a histidine buffer system; an absence of a sugar component; an absence of polysorbate 80; and an absence of methionine.

G299. secukinumab; an absence of a buffer system; an absence of a sugar component; an absence of polysorbate 80; and an absence of methionine.

G300. secukinumab; an absence of a histidine buffer system; an absence of trehalose and sucrose; an absence of a surfactant; and an absence of methionine.

G301. secukinumab; an absence of a buffer system; an absence of trehalose and sucrose; an absence of a surfactant; and an absence of methionine.

G302. secukinumab; an absence of a histidine buffer system; an absence of a sugar component; an absence of a surfactant; and an absence of methionine.

G303. secukinumab; an absence of a buffer system; an absence of a sugar component; an absence of a surfactant; and an absence of methionine.

G304. secukinumab; an absence of a histidine buffer system; an absence of trehalose and sucrose; an absence of polysorbate 80; and an absence of an antioxidant.

G305. secukinumab; an absence of a buffer system; an absence of trehalose and sucrose; an absence of polysorbate 80; and an absence of an antioxidant.

(continued)

G306. secukinumab; an absence of a histidine buffer system; an absence of a sugar component; an absence of polysorbate 80; and an absence of an antioxidant.

G307. secukinumab; an absence of a buffer system; an absence of a sugar component; an absence of polysorbate 80; and an absence of an antioxidant.

G308. secukinumab; an absence of a histidine buffer system; an absence of trehalose and sucrose; an absence of a surfactant; and an absence of an antioxidant.

G309. secukinumab; an absence of a buffer system; an absence of trehalose and sucrose; an absence of a surfactant; and an absence of an antioxidant.

G310. secukinumab; an absence of a histidine buffer system; an absence of a sugar component; an absence of a surfactant; and an absence of an antioxidant.

G311. secukinumab; an absence of a buffer system; an absence of a sugar component; an absence of a surfactant; and an absence of an antioxidant.

G312. secukinumab; an absence of a histidine buffer system; an absence of trehalose and sucrose; an absence of a (further) tonicifier; and an absence of a (further) ionic strength provider.

G313. secukinumab; an absence of a buffer system; an absence of trehalose and sucrose; an absence of a (further) tonicifier; and an absence of a (further) ionic strength provider.

G314. secukinumab; an absence of a histidine buffer system; an absence of a sugar component; an absence of a (further) tonicifier; and an absence of a (further) ionic strength provider.

G315. secukinumab; an absence of a buffer system; an absence of a sugar component; an absence of a (further) tonicifier; and an absence of a (further) ionic strength provider.

G316. secukinumab; an absence of a histidine buffer system; an absence of trehalose and sucrose; an absence of a (further) tonicifier; and an absence of methionine.

G317. secukinumab; an absence of a buffer system; an absence of trehalose and sucrose; an absence of a (further) tonicifier; and an absence of methionine.

G318. secukinumab; an absence of a histidine buffer system; an absence of a sugar component; an absence of a (further) tonicifier; and an absence of methionine.

G319. secukinumab; an absence of a buffer system; an absence of a sugar component; an absence of a (further) tonicifier; and an absence of methionine.

G320. secukinumab; an absence of a histidine buffer system; an absence of trehalose and sucrose; an absence of a (further) tonicifier; and an absence of an antioxidant.

G321. secukinumab; an absence of a buffer system; an absence of trehalose and sucrose; an absence of a (further) tonicifier; and an absence of an antioxidant.

G322. secukinumab; an absence of a histidine buffer system; an absence of a sugar component; an absence of a (further) tonicifier; and an absence of an antioxidant.

(continued)

G323. secukinumab; an absence of a buffer system; an absence of a sugar component; an absence of a (further) tonicifier; and an absence of an antioxidant.

G324. secukinumab; an absence of a histidine buffer system; an absence of trehalose and sucrose; an absence of a (further) ionic strength provider; and an absence of methionine.

G325. secukinumab; an absence of a buffer system; an absence of trehalose and sucrose; an absence of a (further) ionic strength provider; and an absence of methionine.

G326. secukinumab; an absence of a histidine buffer system; an absence of a sugar component; an absence of a (further) ionic strength provider; and an absence of methionine.

G327. secukinumab; an absence of a buffer system; an absence of a sugar component; an absence of a (further) ionic strength provider; and an absence of methionine.

G328. secukinumab; an absence of a histidine buffer system; an absence of trehalose and sucrose; an absence of a (further) ionic strength provider; and an absence of an antioxidant.

G329. secukinumab; an absence of a buffer system; an absence of trehalose and sucrose; an absence of a (further) ionic strength provider; and an absence of an antioxidant.

G330. secukinumab; an absence of a histidine buffer system; an absence of a sugar component; an absence of a (further) ionic strength provider; and an absence of an antioxidant.

G331. secukinumab; an absence of a buffer system; an absence of a sugar component; an absence of a (further) ionic strength provider; and an absence of an antioxidant.

G332. secukinumab; an absence of a histidine buffer system; an absence of histidine and methionine; an absence of polysorbate 80; and an absence of a (further) tonicifier.

G333. secukinumab; an absence of a buffer system; an absence of histidine and methionine; an absence of polysorbate 80; and an absence of a (further) tonicifier.

G334. secukinumab; an absence of a histidine buffer system; an absence of an amino acid component; an absence of polysorbate 80; and an absence of a (further) tonicifier.

G335. secukinumab; an absence of a buffer system; an absence of an amino acid component; an absence of polysorbate 80; and an absence of a (further) tonicifier.

G336. secukinumab; an absence of a histidine buffer system; an absence of histidine and methionine; an absence of a surfactant; and an absence of a (further) tonicifier.

G337. secukinumab; an absence of a buffer system; an absence of histidine and methionine; an absence of a surfactant; and an absence of a (further) tonicifier.

G338. secukinumab; an absence of a histidine buffer system; an absence of an amino acid component; an absence of a surfactant; and an absence of a (further) tonicifier.

G339. secukinumab; an absence of a buffer system; an absence of an amino acid component; an absence of a surfactant; and an absence of a (further) tonicifier.

(continued)

G340. secukinumab; an absence of a histidine buffer system; an absence of histidine and methionine; an absence of polysorbate 80; and an absence of a (further) ionic strength provider.

G341. secukinumab; an absence of a buffer system; an absence of histidine and methionine; an absence of polysorbate 80; and an absence of a (further) ionic strength provider.

G342. secukinumab; an absence of a histidine buffer system; an absence of an amino acid component; an absence of polysorbate 80; and an absence of a (further) ionic strength provider.

G343. secukinumab; an absence of a buffer system; an absence of an amino acid component; an absence of polysorbate 80; and an absence of a (further) ionic strength provider.

G344. secukinumab; an absence of a histidine buffer system; an absence of histidine and methionine; an absence of a surfactant; and an absence of a (further) ionic strength provider.

G345. secukinumab; an absence of a buffer system; an absence of histidine and methionine; an absence of a surfactant; and an absence of a (further) ionic strength provider.

G346. secukinumab; an absence of a histidine buffer system; an absence of an amino acid component; an absence of a surfactant; and an absence of a (further) ionic strength provider.

G347. secukinumab; an absence of a buffer system; an absence of an amino acid component; an absence of a surfactant; and an absence of a (further) ionic strength provider.

G348. secukinumab; an absence of a histidine buffer system; an absence of histidine and methionine; an absence of polysorbate 80; and an absence of methionine.

G349. secukinumab; an absence of a buffer system; an absence of histidine and methionine; an absence of polysorbate 80; and an absence of methionine.

G350. secukinumab; an absence of a histidine buffer system; an absence of an amino acid component; an absence of polysorbate 80; and an absence of methionine.

G351. secukinumab; an absence of a buffer system; an absence of an amino acid component; an absence of polysorbate 80; and an absence of methionine.

G352. secukinumab; an absence of a histidine buffer system; an absence of histidine and methionine; an absence of a surfactant; and an absence of methionine.

G353. secukinumab; an absence of a buffer system; an absence of histidine and methionine; an absence of a surfactant; and an absence of methionine.

G354. secukinumab; an absence of a histidine buffer system; an absence of an amino acid component; an absence of a surfactant; and an absence of methionine.

G355. secukinumab; an absence of a buffer system; an absence of an amino acid component; an absence of a surfactant; and an absence of methionine.

G356. secukinumab; an absence of a histidine buffer system; an absence of histidine and methionine; an absence of polysorbate 80; and an absence of an antioxidant.

(continued)

G357. secukinumab; an absence of a buffer system; an absence of histidine and methionine; an absence of polysorbate 80; and an absence of an antioxidant.

G358. secukinumab; an absence of a histidine buffer system; an absence of an amino acid component; an absence of polysorbate 80; and an absence of an antioxidant.

G359. secukinumab; an absence of a buffer system; an absence of an amino acid component; an absence of polysorbate 80; and an absence of an antioxidant.

G360. secukinumab; an absence of a histidine buffer system; an absence of histidine and methionine; an absence of a surfactant; and an absence of an antioxidant.

G361. secukinumab; an absence of a buffer system; an absence of histidine and methionine; an absence of a surfactant; and an absence of an antioxidant.

G362. secukinumab; an absence of a histidine buffer system; an absence of an amino acid component; an absence of a surfactant; and an absence of an antioxidant.

G363. secukinumab; an absence of a buffer system; an absence of an amino acid component; an absence of a surfactant; and an absence of an antioxidant.

G364. secukinumab; an absence of a histidine buffer system; an absence of histidine and methionine; an absence of a (further) tonicifier; and an absence of a (further) ionic strength provider.

G365. secukinumab; an absence of a buffer system; an absence of histidine and methionine; an absence of a (further) tonicifier; and an absence of a (further) ionic strength provider.

G366. secukinumab; an absence of a histidine buffer system; an absence of an amino acid component; an absence of a (further) tonicifier; and an absence of a (further) ionic strength provider.

G367. secukinumab; an absence of a buffer system; an absence of an amino acid component; an absence of a (further) tonicifier; and an absence of a (further) ionic strength provider.

G368. secukinumab; an absence of a histidine buffer system; an absence of histidine and methionine; an absence of a (further) tonicifier; and an absence of methionine.

G369. secukinumab; an absence of a buffer system; an absence of histidine and methionine; an absence of a (further) tonicifier; and an absence of methionine.

G370. secukinumab; an absence of a histidine buffer system; an absence of an amino acid component; an absence of a (further) tonicifier; and an absence of methionine.

G371. secukinumab; an absence of a buffer system; an absence of an amino acid component; an absence of a (further) tonicifier; and an absence of methionine.

G372. secukinumab; an absence of a histidine buffer system; an absence of histidine and methionine; an absence of a (further) tonicifier; and an absence of an antioxidant.

G373. secukinumab; an absence of a buffer system; an absence of histidine and methionine; an absence of a (further) tonicifier; and an absence of an antioxidant.

(continued)

G374. secukinumab; an absence of a histidine buffer system; an absence of an amino acid component; an absence of a (further) tonicifier; and an absence of an antioxidant.

G375. secukinumab; an absence of a buffer system; an absence of an amino acid component; an absence of a (further) tonicifier; and an absence of an antioxidant.

G376. secukinumab; an absence of a histidine buffer system; an absence of histidine and methionine; an absence of a (further) ionic strength provider; and an absence of methionine.

G377. secukinumab; an absence of a buffer system; an absence of histidine and methionine; an absence of a (further) ionic strength provider; and an absence of methionine.

G378. secukinumab; an absence of a histidine buffer system; an absence of an amino acid component; an absence of a (further) ionic strength provider; and an absence of methionine.

G379. secukinumab; an absence of a buffer system; an absence of an amino acid component; an absence of a (further) ionic strength provider; and an absence of methionine.

G380. secukinumab; an absence of a histidine buffer system; an absence of histidine and methionine; an absence of a (further) ionic strength provider; and an absence of an antioxidant.

G381. secukinumab; an absence of a buffer system; an absence of histidine and methionine; an absence of a (further) ionic strength provider; and an absence of an antioxidant.

G382. secukinumab; an absence of a histidine buffer system; an absence of an amino acid component; an absence of a (further) ionic strength provider; and an absence of an antioxidant.

G383. secukinumab; an absence of a buffer system; an absence of an amino acid component; an absence of a (further) ionic strength provider; and an absence of an antioxidant.

G384. secukinumab; an absence of a histidine buffer system; an absence of polysorbate 80; an absence of a (further) tonicifier; and an absence of a (further) ionic strength provider.

G385. secukinumab; an absence of a buffer system; an absence of polysorbate 80; an absence of a (further) tonicifier; and an absence of a (further) ionic strength provider.

G386. secukinumab; an absence of a histidine buffer system; an absence of a surfactant; an absence of a (further) tonicifier; and an absence of a (further) ionic strength provider.

G387. secukinumab; an absence of a buffer system; an absence of a surfactant; an absence of a (further) tonicifier; and an absence of a (further) ionic strength provider.

G388. secukinumab; an absence of a histidine buffer system; an absence of polysorbate 80; an absence of a (further) tonicifier; and an absence of methionine.

G389. secukinumab; an absence of a buffer system; an absence of polysorbate 80; an absence of a (further) tonicifier; and an absence of methionine.

G390. secukinumab; an absence of a histidine buffer system; an absence of a surfactant; an absence of a (further) tonicifier; and an absence of methionine.

(continued)

G391. secukinumab; an absence of a buffer system; an absence of a surfactant; an absence of a (further) tonicifier; and an absence of methionine.

G392. secukinumab; an absence of a histidine buffer system; an absence of polysorbate 80; an absence of a (further) tonicifier; and an absence of an antioxidant.

G393. secukinumab; an absence of a buffer system; an absence of polysorbate 80; an absence of a (further) tonicifier; and an absence of an antioxidant.

G394. secukinumab; an absence of a histidine buffer system; an absence of a surfactant; an absence of a (further) tonicifier; and an absence of an antioxidant.

G395. secukinumab; an absence of a buffer system; an absence of a surfactant; an absence of a (further) tonicifier; and an absence of an antioxidant.

G396. secukinumab; an absence of a histidine buffer system; an absence of polysorbate 80; an absence of a (further) ionic strength provider; and an absence of methionine.

G397. secukinumab; an absence of a buffer system; an absence of polysorbate 80; an absence of a (further) ionic strength provider; and an absence of methionine.

G398. secukinumab; an absence of a histidine buffer system; an absence of a surfactant; an absence of a (further) ionic strength provider; and an absence of methionine.

G399. secukinumab; an absence of a buffer system; an absence of a surfactant; an absence of a (further) ionic strength provider; and an absence of methionine.

G400. secukinumab; an absence of a histidine buffer system; an absence of polysorbate 80; an absence of a (further) ionic strength provider; and an absence of an antioxidant.

G401. secukinumab; an absence of a buffer system; an absence of polysorbate 80; an absence of a (further) ionic strength provider; and an absence of an antioxidant.

G402. secukinumab; an absence of a histidine buffer system; an absence of a surfactant; an absence of a (further) ionic strength provider; and an absence of an antioxidant.

G403. secukinumab; an absence of a buffer system; an absence of a surfactant; an absence of a (further) ionic strength provider; and an absence of an antioxidant.

G404. secukinumab; an absence of a histidine buffer system; an absence of a (further) tonicifier; an absence of a (further) ionic strength provider; and an absence of methionine.

G405. secukinumab; an absence of a buffer system; an absence of a (further) tonicifier; an absence of a (further) ionic strength provider; and an absence of methionine.

G406. secukinumab; an absence of a histidine buffer system; an absence of a (further) tonicifier; an absence of a (further) ionic strength provider; and an absence of an antioxidant.

G407. secukinumab; an absence of a buffer system; an absence of a (further) tonicifier; an absence of a (further) ionic strength provider; and an absence of an antioxidant.

(continued)

G408. secukinumab; an absence of trehalose and sucrose; an absence of histidine and methionine; an absence of polysorbate 80; and an absence of a (further) tonicifier.

G409. secukinumab; an absence of a sugar component; an absence of histidine and methionine; an absence of polysorbate 80; and an absence of a (further) tonicifier.

G410. secukinumab; an absence of trehalose and sucrose; an absence of an amino acid component; an absence of polysorbate 80; and an absence of a (further) tonicifier.

G411. secukinumab; an absence of a sugar component; an absence of an amino acid component; an absence of polysorbate 80; and an absence of a (further) tonicifier.

G412. secukinumab; an absence of trehalose and sucrose; an absence of histidine and methionine; an absence of a surfactant; and an absence of a (further) tonicifier.

G413. secukinumab; an absence of a sugar component; an absence of histidine and methionine; an absence of a surfactant; and an absence of a (further) tonicifier.

G414. secukinumab; an absence of trehalose and sucrose; an absence of an amino acid component; an absence of a surfactant; and an absence of a (further) tonicifier.

G415. secukinumab; an absence of a sugar component; an absence of an amino acid component; an absence of a surfactant; and an absence of a (further) tonicifier.

G416. secukinumab; an absence of trehalose and sucrose; an absence of histidine and methionine; an absence of polysorbate 80; and an absence of a (further) ionic strength provider.

G417. secukinumab; an absence of a sugar component; an absence of histidine and methionine; an absence of polysorbate 80; and an absence of a (further) ionic strength provider.

G418. secukinumab; an absence of trehalose and sucrose; an absence of an amino acid component; an absence of polysorbate 80; and an absence of a (further) ionic strength provider.

G419. secukinumab; an absence of a sugar component; an absence of an amino acid component; an absence of polysorbate 80; and an absence of a (further) ionic strength provider.

G420. secukinumab; an absence of trehalose and sucrose; an absence of histidine and methionine; an absence of a surfactant; and an absence of a (further) ionic strength provider.

G421. secukinumab; an absence of a sugar component; an absence of histidine and methionine; an absence of a surfactant; and an absence of a (further) ionic strength provider.

G422. secukinumab; an absence of trehalose and sucrose; an absence of an amino acid component; an absence of a surfactant; and an absence of a (further) ionic strength provider.

G423. secukinumab; an absence of a sugar component; an absence of an amino acid component; an absence of a surfactant; and an absence of a (further) ionic strength provider.

G424. secukinumab; an absence of trehalose and sucrose; an absence of histidine and methionine; an absence of polysorbate 80; and an absence of methionine.

(continued)

G425. secukinumab; an absence of a sugar component; an absence of histidine and methionine; an absence of polysorbate 80; and an absence of methionine.

G426. secukinumab; an absence of trehalose and sucrose; an absence of an amino acid component; an absence of polysorbate 80; and an absence of methionine.

G427. secukinumab; an absence of a sugar component; an absence of an amino acid component; an absence of polysorbate 80; and an absence of methionine.

G428. secukinumab; an absence of trehalose and sucrose; an absence of histidine and methionine; an absence of a surfactant; and an absence of methionine.

G429. secukinumab; an absence of a sugar component; an absence of histidine and methionine; an absence of a surfactant; and an absence of methionine.

G430. secukinumab; an absence of trehalose and sucrose; an absence of an amino acid component; an absence of a surfactant; and an absence of methionine.

G431. secukinumab; an absence of a sugar component; an absence of an amino acid component; an absence of a surfactant; and an absence of methionine.

G432. secukinumab; an absence of trehalose and sucrose; an absence of histidine and methionine; an absence of polysorbate 80; and an absence of an antioxidant.

G433. secukinumab; an absence of a sugar component; an absence of histidine and methionine; an absence of polysorbate 80; and an absence of an antioxidant.

G434. secukinumab; an absence of trehalose and sucrose; an absence of an amino acid component; an absence of polysorbate 80; and an absence of an antioxidant.

G435. secukinumab; an absence of a sugar component; an absence of an amino acid component; an absence of polysorbate 80; and an absence of an antioxidant.

G436. secukinumab; an absence of trehalose and sucrose; an absence of histidine and methionine; an absence of a surfactant; and an absence of an antioxidant.

G437. secukinumab; an absence of a sugar component; an absence of histidine and methionine; an absence of a surfactant; and an absence of an antioxidant.

G438. secukinumab; an absence of trehalose and sucrose; an absence of an amino acid component; an absence of a surfactant; and an absence of an antioxidant.

G439. secukinumab; an absence of a sugar component; an absence of an amino acid component; an absence of a surfactant; and an absence of an antioxidant.

G440. secukinumab; an absence of trehalose and sucrose; an absence of histidine and methionine; an absence of a (further) tonicifier; and an absence of a (further) ionic strength provider.

G441. secukinumab; an absence of a sugar component; an absence of histidine and methionine; an absence of a (further) tonicifier; and an absence of a (further) ionic strength provider.

(continued)

G442. secukinumab; an absence of trehalose and sucrose; an absence of an amino acid component; an absence of a (further) tonicifier; and an absence of a (further) ionic strength provider.

G443. secukinumab; an absence of a sugar component; an absence of an amino acid component; an absence of a (further) tonicifier; and an absence of a (further) ionic strength provider.

G444. secukinumab; an absence of trehalose and sucrose; an absence of histidine and methionine; an absence of a (further) tonicifier; and an absence of methionine.

G445. secukinumab; an absence of a sugar component; an absence of histidine and methionine; an absence of a (further) tonicifier; and an absence of methionine.

G446. secukinumab; an absence of trehalose and sucrose; an absence of an amino acid component; an absence of a (further) tonicifier; and an absence of methionine.

G447. secukinumab; an absence of a sugar component; an absence of an amino acid component; an absence of a (further) tonicifier; and an absence of methionine.

G448. secukinumab; an absence of trehalose and sucrose; an absence of histidine and methionine; an absence of a (further) tonicifier; and an absence of an antioxidant.

G449. secukinumab; an absence of a sugar component; an absence of histidine and methionine; an absence of a (further) tonicifier; and an absence of an antioxidant.

G450. secukinumab; an absence of trehalose and sucrose; an absence of an amino acid component; an absence of a (further) tonicifier; and an absence of an antioxidant.

G451. secukinumab; an absence of a sugar component; an absence of an amino acid component; an absence of a (further) tonicifier; and an absence of an antioxidant.

G452. secukinumab; an absence of trehalose and sucrose; an absence of histidine and methionine; an absence of a (further) ionic strength provider; and an absence of methionine.

G453. secukinumab; an absence of a sugar component; an absence of histidine and methionine; an absence of a (further) ionic strength provider; and an absence of methionine.

G454. secukinumab; an absence of trehalose and sucrose; an absence of an amino acid component; an absence of a (further) ionic strength provider; and an absence of methionine.

G455. secukinumab; an absence of a sugar component; an absence of an amino acid component; an absence of a (further) ionic strength provider; and an absence of methionine.

G456. secukinumab; an absence of trehalose and sucrose; an absence of histidine and methionine; an absence of a (further) ionic strength provider; and an absence of an antioxidant.

G457. secukinumab; an absence of a sugar component; an absence of histidine and methionine; an absence of a (further) ionic strength provider; and an absence of an antioxidant.

G458. secukinumab; an absence of trehalose and sucrose; an absence of an amino acid component; an absence of a (further) ionic strength provider; and an absence of an antioxidant.

(continued)

G459. secukinumab; an absence of a sugar component; an absence of an amino acid component; an absence of a (further) ionic strength provider; and an absence of an antioxidant.

G460. secukinumab; an absence of trehalose and sucrose; an absence of polysorbate 80; an absence of a (further) tonicifier; and an absence of a (further) ionic strength provider.

G461. secukinumab; an absence of a sugar component; an absence of polysorbate 80; an absence of a (further) tonicifier; and an absence of a (further) ionic strength provider.

G462. secukinumab; an absence of trehalose and sucrose; an absence of a surfactant; an absence of a (further) tonicifier; and an absence of a (further) ionic strength provider.

G463. secukinumab; an absence of a sugar component; an absence of a surfactant; an absence of a (further) tonicifier; and an absence of a (further) ionic strength provider.

G464. secukinumab; an absence of trehalose and sucrose; an absence of polysorbate 80; an absence of a (further) tonicifier; and an absence of methionine.

G465. secukinumab; an absence of a sugar component; an absence of polysorbate 80; an absence of a (further) tonicifier; and an absence of methionine.

G466. secukinumab; an absence of trehalose and sucrose; an absence of a surfactant; an absence of a (further) tonicifier; and an absence of methionine.

G467. secukinumab; an absence of a sugar component; an absence of a surfactant; an absence of a (further) tonicifier; and an absence of methionine.

G468. secukinumab; an absence of trehalose and sucrose; an absence of polysorbate 80; an absence of a (further) tonicifier; and an absence of an antioxidant.

G469. secukinumab; an absence of a sugar component; an absence of polysorbate 80; an absence of a (further) tonicifier; and an absence of an antioxidant.

G470. secukinumab; an absence of trehalose and sucrose; an absence of a surfactant; an absence of a (further) tonicifier; and an absence of an antioxidant.

G471. secukinumab; an absence of a sugar component; an absence of a surfactant; an absence of a (further) tonicifier; and an absence of an antioxidant.

G472. secukinumab; an absence of trehalose and sucrose; an absence of polysorbate 80; an absence of a (further) ionic strength provider; and an absence of methionine.

G473. secukinumab; an absence of a sugar component; an absence of polysorbate 80; an absence of a (further) ionic strength provider; and an absence of methionine.

G474. secukinumab; an absence of trehalose and sucrose; an absence of a surfactant; an absence of a (further) ionic strength provider; and an absence of methionine.

G475. secukinumab; an absence of a sugar component; an absence of a surfactant; an absence of a (further) ionic strength provider; and an absence of methionine.

(continued)

G476. secukinumab; an absence of trehalose and sucrose; an absence of polysorbate 80; an absence of a (further) ionic strength provider; and an absence of an antioxidant.

G477. secukinumab; an absence of a sugar component; an absence of polysorbate 80; an absence of a (further) ionic strength provider; and an absence of an antioxidant.

G478. secukinumab; an absence of trehalose and sucrose; an absence of a surfactant; an absence of a (further) ionic strength provider; and an absence of an antioxidant.

G479. secukinumab; an absence of a sugar component; an absence of a surfactant; an absence of a (further) ionic strength provider; and an absence of an antioxidant.

G480. secukinumab; an absence of trehalose and sucrose; an absence of a (further) tonicifier; an absence of a (further) ionic strength provider; and an absence of methionine.

G481. secukinumab; an absence of a sugar component; an absence of a (further) tonicifier; an absence of a (further) ionic strength provider; and an absence of methionine.

G482. secukinumab; an absence of trehalose and sucrose; an absence of a (further) tonicifier; an absence of a (further) ionic strength provider; and an absence of an antioxidant.

G483. secukinumab; an absence of a sugar component; an absence of a (further) tonicifier; an absence of a (further) ionic strength provider; and an absence of an antioxidant.

G484. secukinumab; an absence of histidine and methionine; an absence of polysorbate 80; an absence of a (further) tonicifier; and an absence of a (further) ionic strength provider.

G485. secukinumab; an absence of an amino acid component; an absence of polysorbate 80; an absence of a (further) tonicifier; and an absence of a (further) ionic strength provider.

G486. secukinumab; an absence of histidine and methionine; an absence of a surfactant; an absence of a (further) tonicifier; and an absence of a (further) ionic strength provider.

G487. secukinumab; an absence of an amino acid component; an absence of a surfactant; an absence of a (further) tonicifier; and an absence of a (further) ionic strength provider.

G488. secukinumab; an absence of histidine and methionine; an absence of polysorbate 80; an absence of a (further) tonicifier; and an absence of methionine.

G489. secukinumab; an absence of an amino acid component; an absence of polysorbate 80; an absence of a (further) tonicifier; and an absence of methionine.

G490. secukinumab; an absence of histidine and methionine; an absence of a surfactant; an absence of a (further) tonicifier; and an absence of methionine.

G491. secukinumab; an absence of an amino acid component; an absence of a surfactant; an absence of a (further) tonicifier; and an absence of methionine.

G492. secukinumab; an absence of histidine and methionine; an absence of polysorbate 80; an absence of a (further) tonicifier; and an absence of an antioxidant.

(continued)

G493. secukinumab; an absence of an amino acid component; an absence of polysorbate 80; an absence of a (further) tonicifier; and an absence of an antioxidant.

G494. secukinumab; an absence of histidine and methionine; an absence of a surfactant; an absence of a (further) tonicifier; and an absence of an antioxidant.

G495. secukinumab; an absence of an amino acid component; an absence of a surfactant; an absence of a (further) tonicifier; and an absence of an antioxidant.

G496. secukinumab; an absence of histidine and methionine; an absence of polysorbate 80; an absence of a (further) ionic strength provider; and an absence of methionine.

G497. secukinumab; an absence of an amino acid component; an absence of polysorbate 80; an absence of a (further) ionic strength provider; and an absence of methionine.

G498. secukinumab; an absence of histidine and methionine; an absence of a surfactant; an absence of a (further) ionic strength provider; and an absence of methionine.

G499. secukinumab; an absence of an amino acid component; an absence of a surfactant; an absence of a (further) ionic strength provider; and an absence of methionine.

G500. secukinumab; an absence of histidine and methionine; an absence of polysorbate 80; an absence of a (further) ionic strength provider; and an absence of an antioxidant.

G501. secukinumab; an absence of an amino acid component; an absence of polysorbate 80; an absence of a (further) ionic strength provider; and an absence of an antioxidant.

G502. secukinumab; an absence of histidine and methionine; an absence of a surfactant; an absence of a (further) ionic strength provider; and an absence of an antioxidant.

G503. secukinumab; an absence of an amino acid component; an absence of a surfactant; an absence of a (further) ionic strength provider; and an absence of an antioxidant.

G504. secukinumab; an absence of histidine and methionine; an absence of a (further) tonicifier; an absence of a (further) ionic strength provider; and an absence of methionine.

G505. secukinumab; an absence of an amino acid component; an absence of a (further) tonicifier; an absence of a (further) ionic strength provider; and an absence of methionine.

G506. secukinumab; an absence of histidine and methionine; an absence of a (further) tonicifier; an absence of a (further) ionic strength provider; and an absence of an antioxidant.

G507. secukinumab; an absence of an amino acid component; an absence of a (further) tonicifier; an absence of a (further) ionic strength provider; and an absence of an antioxidant.

G508. secukinumab; an absence of polysorbate 80; an absence of a (further) tonicifier; an absence of a (further) ionic strength provider; and an absence of methionine.

G509. secukinumab; an absence of a surfactant; an absence of a (further) tonicifier; an absence of a (further) ionic strength provider; and an absence of methionine.

(continued)

G510. secukinumab; an absence of polysorbate 80; an absence of a (further) tonicifier; an absence of a (further) ionic strength provider; and an absence of an antioxidant.

G511. secukinumab; an absence of a surfactant; an absence of a (further) tonicifier; an absence of a (further) ionic strength provider; and an absence of an antioxidant.

G512. secukinumab; an absence of a histidine buffer system; an absence of trehalose and sucrose; an absence of histidine and methionine; an absence of polysorbate 80; and an absence of a (further) tonicifier.

G513. secukinumab; an absence of a buffer system; an absence of trehalose and sucrose; an absence of histidine and methionine; an absence of polysorbate 80; and an absence of a (further) tonicifier.

G514. secukinumab; an absence of a histidine buffer system; an absence of a sugar component; an absence of histidine and methionine; an absence of polysorbate 80; and an absence of a (further) tonicifier.

G515. secukinumab; an absence of a buffer system; an absence of a sugar component; an absence of histidine and methionine; an absence of polysorbate 80; and an absence of a (further) tonicifier.

G516. secukinumab; an absence of a histidine buffer system; an absence of trehalose and sucrose; an absence of an amino acid component; an absence of polysorbate 80; and an absence of a (further) tonicifier.

G517. secukinumab; an absence of a buffer system; an absence of trehalose and sucrose; an absence of an amino acid component; an absence of polysorbate 80; and an absence of a (further) tonicifier.

G518. secukinumab; an absence of a histidine buffer system; an absence of a sugar component; an absence of an amino acid component; an absence of polysorbate 80; and an absence of a (further) tonicifier.

G519. secukinumab; an absence of a buffer system; an absence of a sugar component; an absence of an amino acid component; an absence of polysorbate 80; and an absence of a (further) tonicifier.

G520. secukinumab; an absence of a histidine buffer system; an absence of trehalose and sucrose; an absence of histidine and methionine; an absence of a surfactant; and an absence of a (further) tonicifier.

G521. secukinumab; an absence of a buffer system; an absence of trehalose and sucrose; an absence of histidine and methionine; an absence of a surfactant; and an absence of a (further) tonicifier.

G522. secukinumab; an absence of a histidine buffer system; an absence of a sugar component; an absence of histidine and methionine; an absence of a surfactant; and an absence of a (further) tonicifier.

G523. secukinumab; an absence of a buffer system; an absence of a sugar component; an absence of histidine and methionine; an absence of a surfactant; and an absence of a (further) tonicifier.

G524. secukinumab; an absence of a histidine buffer system; an absence of trehalose and sucrose; an absence of an amino acid component; an absence of a surfactant; and an absence of a (further) tonicifier.

G525. secukinumab; an absence of a buffer system; an absence of trehalose and sucrose; an absence of an amino acid component; an absence of a surfactant; and an absence of a (further) tonicifier.

G526. secukinumab; an absence of a histidine buffer system; an absence of a sugar component; an absence of an amino acid component; an absence of a surfactant; and an absence of a (further) tonicifier.

(continued)

G527. secukinumab; an absence of a buffer system; an absence of a sugar component; an absence of an amino acid component; an absence of a surfactant; and an absence of a (further) tonicifier.

G528. secukinumab; an absence of a histidine buffer system; an absence of trehalose and sucrose; an absence of histidine and methionine; an absence of polysorbate 80; and an absence of a (further) ionic strength provider.

G529. secukinumab; an absence of a buffer system; an absence of trehalose and sucrose; an absence of histidine and methionine; an absence of polysorbate 80; and an absence of a (further) ionic strength provider.

G530. secukinumab; an absence of a histidine buffer system; an absence of a sugar component; an absence of histidine and methionine; an absence of polysorbate 80; and an absence of a (further) ionic strength provider.

G531. secukinumab; an absence of a buffer system; an absence of a sugar component; an absence of histidine and methionine; an absence of polysorbate 80; and an absence of a (further) ionic strength provider.

G532. secukinumab; an absence of a histidine buffer system; an absence of trehalose and sucrose; an absence of an amino acid component; an absence of polysorbate 80; and an absence of a (further) ionic strength provider.

G533. secukinumab; an absence of a buffer system; an absence of trehalose and sucrose; an absence of an amino acid component; an absence of polysorbate 80; and an absence of a (further) ionic strength provider.

G534. secukinumab; an absence of a histidine buffer system; an absence of a sugar component; an absence of an amino acid component; an absence of polysorbate 80; and an absence of a (further) ionic strength provider.

G535. secukinumab; an absence of a buffer system; an absence of a sugar component; an absence of an amino acid component; an absence of polysorbate 80; and an absence of a (further) ionic strength provider.

G536. secukinumab; an absence of a histidine buffer system; an absence of trehalose and sucrose; an absence of histidine and methionine; an absence of a surfactant; and an absence of a (further) ionic strength provider.

G537. secukinumab; an absence of a buffer system; an absence of trehalose and sucrose; an absence of histidine and methionine; an absence of a surfactant; and an absence of a (further) ionic strength provider.

G538. secukinumab; an absence of a histidine buffer system; an absence of a sugar component; an absence of histidine and methionine; an absence of a surfactant; and an absence of a (further) ionic strength provider.

G539. secukinumab; an absence of a buffer system; an absence of a sugar component; an absence of histidine and methionine; an absence of a surfactant; and an absence of a (further) ionic strength provider.

G540. secukinumab; an absence of a histidine buffer system; an absence of trehalose and sucrose; an absence of an amino acid component; an absence of a surfactant; and an absence of a (further) ionic strength provider.

G541. secukinumab; an absence of a buffer system; an absence of trehalose and sucrose; an absence of an amino acid component; an absence of a surfactant; and an absence of a (further) ionic strength provider.

G542. secukinumab; an absence of a histidine buffer system; an absence of a sugar component; an absence of an amino acid component; an absence of a surfactant; and an absence of a (further) ionic strength provider.

G543. secukinumab; an absence of a buffer system; an absence of a sugar component; an absence of an amino acid component; an absence of a surfactant; and an absence of a (further) ionic strength provider.

(continued)

G544. secukinumab; an absence of a histidine buffer system; an absence of trehalose and sucrose; an absence of histidine and methionine; an absence of polysorbate 80; and an absence of methionine.

G545. secukinumab; an absence of a buffer system; an absence of trehalose and sucrose; an absence of histidine and methionine; an absence of polysorbate 80; and an absence of methionine.

G546. secukinumab; an absence of a histidine buffer system; an absence of a sugar component; an absence of histidine and methionine; an absence of polysorbate 80; and an absence of methionine.

G547. secukinumab; an absence of a buffer system; an absence of a sugar component; an absence of histidine and methionine; an absence of polysorbate 80; and an absence of methionine.

G548. secukinumab; an absence of a histidine buffer system; an absence of trehalose and sucrose; an absence of an amino acid component; an absence of polysorbate 80; and an absence of methionine.

G549. secukinumab; an absence of a buffer system; an absence of trehalose and sucrose; an absence of an amino acid component; an absence of polysorbate 80; and an absence of methionine.

G550. secukinumab; an absence of a histidine buffer system; an absence of a sugar component; an absence of an amino acid component; an absence of polysorbate 80; and an absence of methionine.

G551. secukinumab; an absence of a buffer system; an absence of a sugar component; an absence of an amino acid component; an absence of polysorbate 80; and an absence of methionine.

G552. secukinumab; an absence of a histidine buffer system; an absence of trehalose and sucrose; an absence of histidine and methionine; an absence of a surfactant; and an absence of methionine.

G553. secukinumab; an absence of a buffer system; an absence of trehalose and sucrose; an absence of histidine and methionine; an absence of a surfactant; and an absence of methionine.

G554. secukinumab; an absence of a histidine buffer system; an absence of a sugar component; an absence of histidine and methionine; an absence of a surfactant; and an absence of methionine.

G555. secukinumab; an absence of a buffer system; an absence of a sugar component; an absence of histidine and methionine; an absence of a surfactant; and an absence of methionine.

G556. secukinumab; an absence of a histidine buffer system; an absence of trehalose and sucrose; an absence of an amino acid component; an absence of a surfactant; and an absence of methionine.

G557. secukinumab; an absence of a buffer system; an absence of trehalose and sucrose; an absence of an amino acid component; an absence of a surfactant; and an absence of methionine.

G558. secukinumab; an absence of a histidine buffer system; an absence of a sugar component; an absence of an amino acid component; an absence of a surfactant; and an absence of methionine.

G559. secukinumab; an absence of a buffer system; an absence of a sugar component; an absence of an amino acid component; an absence of a surfactant; and an absence of methionine.

G560. secukinumab; an absence of a histidine buffer system; an absence of trehalose and sucrose; an absence of histidine and methionine; an absence of polysorbate 80; and an absence of an antioxidant.

(continued)

G561. secukinumab; an absence of a buffer system; an absence of trehalose and sucrose; an absence of histidine and methionine; an absence of polysorbate 80; and an absence of an antioxidant.

G562. secukinumab; an absence of a histidine buffer system; an absence of a sugar component; an absence of histidine and methionine; an absence of polysorbate 80; and an absence of an antioxidant.

G563. secukinumab; an absence of a buffer system; an absence of a sugar component; an absence of histidine and methionine; an absence of polysorbate 80; and an absence of an antioxidant.

G564. secukinumab; an absence of a histidine buffer system; an absence of trehalose and sucrose; an absence of an amino acid component; an absence of polysorbate 80; and an absence of an antioxidant.

G565. secukinumab; an absence of a buffer system; an absence of trehalose and sucrose; an absence of an amino acid component; an absence of polysorbate 80; and an absence of an antioxidant.

G566. secukinumab; an absence of a histidine buffer system; an absence of a sugar component; an absence of an amino acid component; an absence of polysorbate 80; and an absence of an antioxidant.

G567. secukinumab; an absence of a buffer system; an absence of a sugar component; an absence of an amino acid component; an absence of polysorbate 80; and an absence of an antioxidant.

G568. secukinumab; an absence of a histidine buffer system; an absence of trehalose and sucrose; an absence of histidine and methionine; an absence of a surfactant; and an absence of an antioxidant.

G569. secukinumab; an absence of a buffer system; an absence of trehalose and sucrose; an absence of histidine and methionine; an absence of a surfactant; and an absence of an antioxidant.

G570. secukinumab; an absence of a histidine buffer system; an absence of a sugar component; an absence of histidine and methionine; an absence of a surfactant; and an absence of an antioxidant.

G571. secukinumab; an absence of a buffer system; an absence of a sugar component; an absence of histidine and methionine; an absence of a surfactant; and an absence of an antioxidant.

G572. secukinumab; an absence of a histidine buffer system; an absence of trehalose and sucrose; an absence of an amino acid component; an absence of a surfactant; and an absence of an antioxidant.

G573. secukinumab; an absence of a buffer system; an absence of trehalose and sucrose; an absence of an amino acid component; an absence of a surfactant; and an absence of an antioxidant.

G574. secukinumab; an absence of a histidine buffer system; an absence of a sugar component; an absence of an amino acid component; an absence of a surfactant; and an absence of an antioxidant.

G575. secukinumab; an absence of a buffer system; an absence of a sugar component; an absence of an amino acid component; an absence of a surfactant; and an absence of an antioxidant.

G576. secukinumab; an absence of a histidine buffer system; an absence of trehalose and sucrose; an absence of histidine and methionine; an absence of a (further) tonicifier; and an absence of a (further) ionic strength provider.

G577. secukinumab; an absence of a buffer system; an absence of trehalose and sucrose; an absence of histidine and methionine; an absence of a (further) tonicifier; and an absence of a (further) ionic strength provider.

(continued)

G578. secukinumab; an absence of a histidine buffer system; an absence of a sugar component; an absence of histidine and methionine; an absence of a (further) tonicifier; and an absence of a (further) ionic strength provider.

G579. secukinumab; an absence of a buffer system; an absence of a sugar component; an absence of histidine and methionine; an absence of a (further) tonicifier; and an absence of a (further) ionic strength provider.

G580. secukinumab; an absence of a histidine buffer system; an absence of trehalose and sucrose; an absence of an amino acid component; an absence of a (further) tonicifier; and an absence of a (further) ionic strength provider.

G581. secukinumab; an absence of a buffer system; an absence of trehalose and sucrose; an absence of an amino acid component; an absence of a (further) tonicifier; and an absence of a (further) ionic strength provider.

G582. secukinumab; an absence of a histidine buffer system; an absence of a sugar component; an absence of an amino acid component; an absence of a (further) tonicifier; and an absence of a (further) ionic strength provider.

G583. secukinumab; an absence of a buffer system; an absence of a sugar component; an absence of an amino acid component; an absence of a (further) tonicifier; and an absence of a (further) ionic strength provider.

G584. secukinumab; an absence of a histidine buffer system; an absence of trehalose and sucrose; an absence of histidine and methionine; an absence of a (further) tonicifier; and an absence of methionine.

G585. secukinumab; an absence of a buffer system; an absence of trehalose and sucrose; an absence of histidine and methionine; an absence of a (further) tonicifier; and an absence of methionine.

G586. secukinumab; an absence of a histidine buffer system; an absence of a sugar component; an absence of histidine and methionine; an absence of a (further) tonicifier; and an absence of methionine.

G587. secukinumab; an absence of a buffer system; an absence of a sugar component; an absence of histidine and methionine; an absence of a (further) tonicifier; and an absence of methionine.

G588. secukinumab; an absence of a histidine buffer system; an absence of trehalose and sucrose; an absence of an amino acid component; an absence of a (further) tonicifier; and an absence of methionine.

G589. secukinumab; an absence of a buffer system; an absence of trehalose and sucrose; an absence of an amino acid component; an absence of a (further) tonicifier; and an absence of methionine.

G590. secukinumab; an absence of a histidine buffer system; an absence of a sugar component; an absence of an amino acid component; an absence of a (further) tonicifier; and an absence of methionine.

G591. secukinumab; an absence of a buffer system; an absence of a sugar component; an absence of an amino acid component; an absence of a (further) tonicifier; and an absence of methionine.

G592. secukinumab; an absence of a histidine buffer system; an absence of trehalose and sucrose; an absence of histidine and methionine; an absence of a (further) tonicifier; and an absence of an antioxidant.

G593. secukinumab; an absence of a buffer system; an absence of trehalose and sucrose; an absence of histidine and methionine; an absence of a (further) tonicifier; and an absence of an antioxidant.

G594. secukinumab; an absence of a histidine buffer system; an absence of a sugar component; an absence of histidine and methionine; an absence of a (further) tonicifier; and an absence of an antioxidant.

(continued)

G595. secukinumab; an absence of a buffer system; an absence of a sugar component; an absence of histidine and methionine; an absence of a (further) tonicifier; and an absence of an antioxidant.

G596. secukinumab; an absence of a histidine buffer system; an absence of trehalose and sucrose; an absence of an amino acid component; an absence of a (further) tonicifier; and an absence of an antioxidant.

G597. secukinumab; an absence of a buffer system; an absence of trehalose and sucrose; an absence of an amino acid component; an absence of a (further) tonicifier; and an absence of an antioxidant.

G598. secukinumab; an absence of a histidine buffer system; an absence of a sugar component; an absence of an amino acid component; an absence of a (further) tonicifier; and an absence of an antioxidant.

G599. secukinumab; an absence of a buffer system; an absence of a sugar component; an absence of an amino acid component; an absence of a (further) tonicifier; and an absence of an antioxidant.

G600. secukinumab; an absence of a histidine buffer system; an absence of trehalose and sucrose; an absence of histidine and methionine; an absence of a (further) ionic strength provider; and an absence of methionine.

G601. secukinumab; an absence of a buffer system; an absence of trehalose and sucrose; an absence of histidine and methionine; an absence of a (further) ionic strength provider; and an absence of methionine.

G602. secukinumab; an absence of a histidine buffer system; an absence of a sugar component; an absence of histidine and methionine; an absence of a (further) ionic strength provider; and an absence of methionine.

G603. secukinumab; an absence of a buffer system; an absence of a sugar component; an absence of histidine and methionine; an absence of a (further) ionic strength provider; and an absence of methionine.

G604. secukinumab; an absence of a histidine buffer system; an absence of trehalose and sucrose; an absence of an amino acid component; an absence of a (further) ionic strength provider; and an absence of methionine.

G605. secukinumab; an absence of a buffer system; an absence of trehalose and sucrose; an absence of an amino acid component; an absence of a (further) ionic strength provider; and an absence of methionine.

G606. secukinumab; an absence of a histidine buffer system; an absence of a sugar component; an absence of an amino acid component; an absence of a (further) ionic strength provider; and an absence of methionine.

G607. secukinumab; an absence of a buffer system; an absence of a sugar component; an absence of an amino acid component; an absence of a (further) ionic strength provider; and an absence of methionine.

G608. secukinumab; an absence of a histidine buffer system; an absence of trehalose and sucrose; an absence of histidine and methionine; an absence of a (further) ionic strength provider; and an absence of an antioxidant.

G609. secukinumab; an absence of a buffer system; an absence of trehalose and sucrose; an absence of histidine and methionine; an absence of a (further) ionic strength provider; and an absence of an antioxidant.

G610. secukinumab; an absence of a histidine buffer system; an absence of a sugar component; an absence of histidine and methionine; an absence of a (further) ionic strength provider; and an absence of an antioxidant.

G611. secukinumab; an absence of a buffer system; an absence of a sugar component; an absence of histidine and methionine; an absence of a (further) ionic strength provider; and an absence of an antioxidant.

(continued)

G612. secukinumab; an absence of a histidine buffer system; an absence of trehalose and sucrose; an absence of an amino acid component; an absence of a (further) ionic strength provider; and an absence of an antioxidant.

G613. secukinumab; an absence of a buffer system; an absence of trehalose and sucrose; an absence of an amino acid component; an absence of a (further) ionic strength provider; and an absence of an antioxidant.

G614. secukinumab; an absence of a histidine buffer system; an absence of a sugar component; an absence of an amino acid component; an absence of a (further) ionic strength provider; and an absence of an antioxidant.

G615. secukinumab; an absence of a buffer system; an absence of a sugar component; an absence of an amino acid component; an absence of a (further) ionic strength provider; and an absence of an antioxidant.

G616. secukinumab; an absence of a histidine buffer system; an absence of trehalose and sucrose; an absence of polysorbate 80; an absence of a (further) tonicifier; and an absence of a (further) ionic strength provider.

G617. secukinumab; an absence of a buffer system; an absence of trehalose and sucrose; an absence of polysorbate 80; an absence of a (further) tonicifier; and an absence of a (further) ionic strength provider.

G618. secukinumab; an absence of a histidine buffer system; an absence of a sugar component; an absence of polysorbate 80; an absence of a (further) tonicifier; and an absence of a (further) ionic strength provider.

G619. secukinumab; an absence of a buffer system; an absence of a sugar component; an absence of polysorbate 80; an absence of a (further) tonicifier; and an absence of a (further) ionic strength provider.

G620. secukinumab; an absence of a histidine buffer system; an absence of trehalose and sucrose; an absence of a surfactant; an absence of a (further) tonicifier; and an absence of a (further) ionic strength provider.

G621. secukinumab; an absence of a buffer system; an absence of trehalose and sucrose; an absence of a surfactant; an absence of a (further) tonicifier; and an absence of a (further) ionic strength provider.

G622. secukinumab; an absence of a histidine buffer system; an absence of a sugar component; an absence of a surfactant; an absence of a (further) tonicifier; and an absence of a (further) ionic strength provider.

G623. secukinumab; an absence of a buffer system; an absence of a sugar component; an absence of a surfactant; an absence of a (further) tonicifier; and an absence of a (further) ionic strength provider.

G624. secukinumab; an absence of a histidine buffer system; an absence of trehalose and sucrose; an absence of polysorbate 80; an absence of a (further) tonicifier; and an absence of methionine.

G625. secukinumab; an absence of a buffer system; an absence of trehalose and sucrose; an absence of polysorbate 80; an absence of a (further) tonicifier; and an absence of methionine.

G626. secukinumab; an absence of a histidine buffer system; an absence of a sugar component; an absence of polysorbate 80; an absence of a (further) tonicifier; and an absence of methionine.

G627. secukinumab; an absence of a buffer system; an absence of a sugar component; an absence of polysorbate 80; an absence of a (further) tonicifier; and an absence of methionine.

G628. secukinumab; an absence of a histidine buffer system; an absence of trehalose and sucrose; an absence of a surfactant; an absence of a (further) tonicifier; and an absence of methionine.

(continued)

G629. secukinumab; an absence of a buffer system; an absence of trehalose and sucrose; an absence of a surfactant; an absence of a (further) tonicifier; and an absence of methionine.

G630. secukinumab; an absence of a histidine buffer system; an absence of a sugar component; an absence of a surfactant; an absence of a (further) tonicifier; and an absence of methionine.

G631. secukinumab; an absence of a buffer system; an absence of a sugar component; an absence of a surfactant; an absence of a (further) tonicifier; and an absence of methionine.

G632. secukinumab; an absence of a histidine buffer system; an absence of trehalose and sucrose; an absence of polysorbate 80; an absence of a (further) tonicifier; and an absence of an antioxidant.

G633. secukinumab; an absence of a buffer system; an absence of trehalose and sucrose; an absence of polysorbate 80; an absence of a (further) tonicifier; and an absence of an antioxidant.

G634. secukinumab; an absence of a histidine buffer system; an absence of a sugar component; an absence of polysorbate 80; an absence of a (further) tonicifier; and an absence of an antioxidant.

G635. secukinumab; an absence of a buffer system; an absence of a sugar component; an absence of polysorbate 80; an absence of a (further) tonicifier; and an absence of an antioxidant.

G636. secukinumab; an absence of a histidine buffer system; an absence of trehalose and sucrose; an absence of a surfactant; an absence of a (further) tonicifier; and an absence of an antioxidant.

G637. secukinumab; an absence of a buffer system; an absence of trehalose and sucrose; an absence of a surfactant; an absence of a (further) tonicifier; and an absence of an antioxidant.

G638. secukinumab; an absence of a histidine buffer system; an absence of a sugar component; an absence of a surfactant; an absence of a (further) tonicifier; and an absence of an antioxidant.

G639. secukinumab; an absence of a buffer system; an absence of a sugar component; an absence of a surfactant; an absence of a (further) tonicifier; and an absence of an antioxidant.

G640. secukinumab; an absence of a histidine buffer system; an absence of trehalose and sucrose; an absence of polysorbate 80; an absence of a (further) ionic strength provider; and an absence of methionine.

G641. secukinumab; an absence of a buffer system; an absence of trehalose and sucrose; an absence of polysorbate 80; an absence of a (further) ionic strength provider; and an absence of methionine.

G642. secukinumab; an absence of a histidine buffer system; an absence of a sugar component; an absence of polysorbate 80; an absence of a (further) ionic strength provider; and an absence of methionine.

G643. secukinumab; an absence of a buffer system; an absence of a sugar component; an absence of polysorbate 80; an absence of a (further) ionic strength provider; and an absence of methionine.

G644. secukinumab; an absence of a histidine buffer system; an absence of trehalose and sucrose; an absence of a surfactant; an absence of a (further) ionic strength provider; and an absence of methionine.

G645. secukinumab; an absence of a buffer system; an absence of trehalose and sucrose; an absence of a surfactant; an absence of a (further) ionic strength provider; and an absence of methionine.

(continued)

G646. secukinumab; an absence of a histidine buffer system; an absence of a sugar component; an absence of a surfactant; an absence of a (further) ionic strength provider; and an absence of methionine.

G647. secukinumab; an absence of a buffer system; an absence of a sugar component; an absence of a surfactant; an absence of a (further) ionic strength provider; and an absence of methionine.

G648. secukinumab; an absence of a histidine buffer system; an absence of trehalose and sucrose; an absence of polysorbate 80; an absence of a (further) ionic strength provider; and an absence of an antioxidant.

G649. secukinumab; an absence of a buffer system; an absence of trehalose and sucrose; an absence of polysorbate 80; an absence of a (further) ionic strength provider; and an absence of an antioxidant.

G650. secukinumab; an absence of a histidine buffer system; an absence of a sugar component; an absence of polysorbate 80; an absence of a (further) ionic strength provider; and an absence of an antioxidant.

G651. secukinumab; an absence of a buffer system; an absence of a sugar component; an absence of polysorbate 80; an absence of a (further) ionic strength provider; and an absence of an antioxidant.

G652. secukinumab; an absence of a histidine buffer system; an absence of trehalose and sucrose; an absence of a surfactant; an absence of a (further) ionic strength provider; and an absence of an antioxidant.

G653. secukinumab; an absence of a buffer system; an absence of trehalose and sucrose; an absence of a surfactant; an absence of a (further) ionic strength provider; and an absence of an antioxidant.

G654. secukinumab; an absence of a histidine buffer system; an absence of a sugar component; an absence of a surfactant; an absence of a (further) ionic strength provider; and an absence of an antioxidant.

G655. secukinumab; an absence of a buffer system; an absence of a sugar component; an absence of a surfactant; an absence of a (further) ionic strength provider; and an absence of an antioxidant.

G656. secukinumab; an absence of a histidine buffer system; an absence of trehalose and sucrose; an absence of a (further) tonicifier; an absence of a (further) ionic strength provider; and an absence of methionine.

G657. secukinumab; an absence of a buffer system; an absence of trehalose and sucrose; an absence of a (further) tonicifier; an absence of a (further) ionic strength provider; and an absence of methionine.

G658. secukinumab; an absence of a histidine buffer system; an absence of a sugar component; an absence of a (further) tonicifier; an absence of a (further) ionic strength provider; and an absence of methionine.

G659. secukinumab; an absence of a buffer system; an absence of a sugar component; an absence of a (further) tonicifier; an absence of a (further) ionic strength provider; and an absence of methionine.

G660. secukinumab; an absence of a histidine buffer system; an absence of trehalose and sucrose; an absence of a (further) tonicifier; an absence of a (further) ionic strength provider; and an absence of an antioxidant.

G661. secukinumab; an absence of a buffer system; an absence of trehalose and sucrose; an absence of a (further) tonicifier; an absence of a (further) ionic strength provider; and an absence of an antioxidant.

G662. secukinumab; an absence of a histidine buffer system; an absence of a sugar component; an absence of a (further) tonicifier; an absence of a (further) ionic strength provider; and an absence of an antioxidant.

(continued)

G663. secukinumab; an absence of a buffer system; an absence of a sugar component; an absence of a (further) tonicifier; an absence of a (further) ionic strength provider; and an absence of an antioxidant.

G664. secukinumab; an absence of a histidine buffer system; an absence of histidine and methionine; an absence of polysorbate 80; an absence of a (further) tonicifier; and an absence of a (further) ionic strength provider.

G665. secukinumab; an absence of a buffer system; an absence of histidine and methionine; an absence of polysorbate 80; an absence of a (further) tonicifier; and an absence of a (further) ionic strength provider.

G666. secukinumab; an absence of a histidine buffer system; an absence of an amino acid component; an absence of polysorbate 80; an absence of a (further) tonicifier; and an absence of a (further) ionic strength provider.

G667. secukinumab; an absence of a buffer system; an absence of an amino acid component; an absence of polysorbate 80; an absence of a (further) tonicifier; and an absence of a (further) ionic strength provider.

G668. secukinumab; an absence of a histidine buffer system; an absence of histidine and methionine; an absence of a surfactant; an absence of a (further) tonicifier; and an absence of a (further) ionic strength provider.

G669. secukinumab; an absence of a buffer system; an absence of histidine and methionine; an absence of a surfactant; an absence of a (further) tonicifier; and an absence of a (further) ionic strength provider.

G670. secukinumab; an absence of a histidine buffer system; an absence of an amino acid component; an absence of a surfactant; an absence of a (further) tonicifier; and an absence of a (further) ionic strength provider.

G671. secukinumab; an absence of a buffer system; an absence of an amino acid component; an absence of a surfactant; an absence of a (further) tonicifier; and an absence of a (further) ionic strength provider.

G672. secukinumab; an absence of a histidine buffer system; an absence of histidine and methionine; an absence of polysorbate 80; an absence of a (further) tonicifier; and an absence of methionine.

G673. secukinumab; an absence of a buffer system; an absence of histidine and methionine; an absence of polysorbate 80; an absence of a (further) tonicifier; and an absence of methionine.

G674. secukinumab; an absence of a histidine buffer system; an absence of an amino acid component; an absence of polysorbate 80; an absence of a (further) tonicifier; and an absence of methionine.

G675. secukinumab; an absence of a buffer system; an absence of an amino acid component; an absence of polysorbate 80; an absence of a (further) tonicifier; and an absence of methionine.

G676. secukinumab; an absence of a histidine buffer system; an absence of histidine and methionine; an absence of a surfactant; an absence of a (further) tonicifier; and an absence of methionine.

G677. secukinumab; an absence of a buffer system; an absence of histidine and methionine; an absence of a surfactant; an absence of a (further) tonicifier; and an absence of methionine.

G678. secukinumab; an absence of a histidine buffer system; an absence of an amino acid component; an absence of a surfactant; an absence of a (further) tonicifier; and an absence of methionine.

G679. secukinumab; an absence of a buffer system; an absence of an amino acid component; an absence of a surfactant; an absence of a (further) tonicifier; and an absence of methionine.

(continued)

G680. secukinumab; an absence of a histidine buffer system; an absence of histidine and methionine; an absence of polysorbate 80; an absence of a (further) tonicifier; and an absence of an antioxidant.

G681. secukinumab; an absence of a buffer system; an absence of histidine and methionine; an absence of polysorbate 80; an absence of a (further) tonicifier; and an absence of an antioxidant.

G682. secukinumab; an absence of a histidine buffer system; an absence of an amino acid component; an absence of polysorbate 80; an absence of a (further) tonicifier; and an absence of an antioxidant.

G683. secukinumab; an absence of a buffer system; an absence of an amino acid component; an absence of polysorbate 80; an absence of a (further) tonicifier; and an absence of an antioxidant.

G684. secukinumab; an absence of a histidine buffer system; an absence of histidine and methionine; an absence of a surfactant; an absence of a (further) tonicifier; and an absence of an antioxidant.

G685. secukinumab; an absence of a buffer system; an absence of histidine and methionine; an absence of a surfactant; an absence of a (further) tonicifier; and an absence of an antioxidant.

G686. secukinumab; an absence of a histidine buffer system; an absence of an amino acid component; an absence of a surfactant; an absence of a (further) tonicifier; and an absence of an antioxidant.

G687. secukinumab; an absence of a buffer system; an absence of an amino acid component; an absence of a surfactant; an absence of a (further) tonicifier; and an absence of an antioxidant.

G688. secukinumab; an absence of a histidine buffer system; an absence of histidine and methionine; an absence of polysorbate 80; an absence of a (further) ionic strength provider; and an absence of methionine.

G689. secukinumab; an absence of a buffer system; an absence of histidine and methionine; an absence of polysorbate 80; an absence of a (further) ionic strength provider; and an absence of methionine.

G690. secukinumab; an absence of a histidine buffer system; an absence of an amino acid component; an absence of polysorbate 80; an absence of a (further) ionic strength provider; and an absence of methionine.

G691. secukinumab; an absence of a buffer system; an absence of an amino acid component; an absence of polysorbate 80; an absence of a (further) ionic strength provider; and an absence of methionine.

G692. secukinumab; an absence of a histidine buffer system; an absence of histidine and methionine; an absence of a surfactant; an absence of a (further) ionic strength provider; and an absence of methionine.

G693. secukinumab; an absence of a buffer system; an absence of histidine and methionine; an absence of a surfactant; an absence of a (further) ionic strength provider; and an absence of methionine.

G694. secukinumab; an absence of a histidine buffer system; an absence of an amino acid component; an absence of a surfactant; an absence of a (further) ionic strength provider; and an absence of methionine.

G695. secukinumab; an absence of a buffer system; an absence of an amino acid component; an absence of a surfactant; an absence of a (further) ionic strength provider; and an absence of methionine.

G696. secukinumab; an absence of a histidine buffer system; an absence of histidine and methionine; an absence of polysorbate 80; an absence of a (further) ionic strength provider; and an absence of an antioxidant.

(continued)

G697. secukinumab; an absence of a buffer system; an absence of histidine and methionine; an absence of polysorbate 80; an absence of a (further) ionic strength provider; and an absence of an antioxidant.

G698. secukinumab; an absence of a histidine buffer system; an absence of an amino acid component; an absence of polysorbate 80; an absence of a (further) ionic strength provider; and an absence of an antioxidant.

G699. secukinumab; an absence of a buffer system; an absence of an amino acid component; an absence of polysorbate 80; an absence of a (further) ionic strength provider; and an absence of an antioxidant.

G700. secukinumab; an absence of a histidine buffer system; an absence of histidine and methionine; an absence of a surfactant; an absence of a (further) ionic strength provider; and an absence of an antioxidant.

G701. secukinumab; an absence of a buffer system; an absence of histidine and methionine; an absence of a surfactant; an absence of a (further) ionic strength provider; and an absence of an antioxidant.

G702. secukinumab; an absence of a histidine buffer system; an absence of an amino acid component; an absence of a surfactant; an absence of a (further) ionic strength provider; and an absence of an antioxidant.

G703. secukinumab; an absence of a buffer system; an absence of an amino acid component; an absence of a surfactant; an absence of a (further) ionic strength provider; and an absence of an antioxidant.

G704. secukinumab; an absence of a histidine buffer system; an absence of histidine and methionine; an absence of a (further) tonicifier; an absence of a (further) ionic strength provider; and an absence of methionine.

G705. secukinumab; an absence of a buffer system; an absence of histidine and methionine; an absence of a (further) tonicifier; an absence of a (further) ionic strength provider; and an absence of methionine.

G706. secukinumab; an absence of a histidine buffer system; an absence of an amino acid component; an absence of a (further) tonicifier; an absence of a (further) ionic strength provider; and an absence of methionine.

G707. secukinumab; an absence of a buffer system; an absence of an amino acid component; an absence of a (further) tonicifier; an absence of a (further) ionic strength provider; and an absence of methionine.

G708. secukinumab; an absence of a histidine buffer system; an absence of histidine and methionine; an absence of a (further) tonicifier; an absence of a (further) ionic strength provider; and an absence of an antioxidant.

G709. secukinumab; an absence of a buffer system; an absence of histidine and methionine; an absence of a (further) tonicifier; an absence of a (further) ionic strength provider; and an absence of an antioxidant.

G710. secukinumab; an absence of a histidine buffer system; an absence of an amino acid component; an absence of a (further) tonicifier; an absence of a (further) ionic strength provider; and an absence of an antioxidant.

G711. secukinumab; an absence of a buffer system; an absence of an amino acid component; an absence of a (further) tonicifier; an absence of a (further) ionic strength provider; and an absence of an antioxidant.

G712. secukinumab; an absence of a histidine buffer system; an absence of polysorbate 80; an absence of a (further) tonicifier; an absence of a (further) ionic strength provider; and an absence of methionine.

G713. secukinumab; an absence of a buffer system; an absence of polysorbate 80; an absence of a (further) tonicifier; an absence of a (further) ionic strength provider; and an absence of methionine.

(continued)

G714. secukinumab; an absence of a histidine buffer system; an absence of a surfactant; an absence of a (further) tonicifier; an absence of a (further) ionic strength provider; and an absence of methionine.
G715. secukinumab; an absence of a buffer system; an absence of a surfactant; an absence of a (further) tonicifier; an absence of a (further) ionic strength provider; and an absence of methionine.
G716. secukinumab; an absence of a histidine buffer system; an absence of polysorbate 80; an absence of a (further) tonicifier; an absence of a (further) ionic strength provider; and an absence of an antioxidant.
G717. secukinumab; an absence of a buffer system; an absence of polysorbate 80; an absence of a (further) tonicifier; an absence of a (further) ionic strength provider; and an absence of an antioxidant.
G718. secukinumab; an absence of a histidine buffer system; an absence of a surfactant; an absence of a (further) tonicifier; an absence of a (further) ionic strength provider; and an absence of an antioxidant.
G719. secukinumab; an absence of a buffer system; an absence of a surfactant; an absence of a (further) tonicifier; an absence of a (further) ionic strength provider; and an absence of an antioxidant.
G720. secukinumab; an absence of trehalose and sucrose; an absence of histidine and methionine; an absence of polysorbate 80; an absence of a (further) tonicifier; and an absence of a (further) ionic strength provider.
G721. secukinumab; an absence of a sugar component; an absence of histidine and methionine; an absence of polysorbate 80; an absence of a (further) tonicifier; and an absence of a (further) ionic strength provider.
G722. secukinumab; an absence of trehalose and sucrose; an absence of an amino acid component; an absence of polysorbate 80; an absence of a (further) tonicifier; and an absence of a (further) ionic strength provider.
G723. secukinumab; an absence of a sugar component; an absence of an amino acid component; an absence of polysorbate 80; an absence of a (further) tonicifier; and an absence of a (further) ionic strength provider.
G724. secukinumab; an absence of trehalose and sucrose; an absence of histidine and methionine; an absence of a surfactant; an absence of a (further) tonicifier; and an absence of a (further) ionic strength provider.
G725. secukinumab; an absence of a sugar component; an absence of histidine and methionine; an absence of a surfactant; an absence of a (further) tonicifier; and an absence of a (further) ionic strength provider.
G726. secukinumab; an absence of trehalose and sucrose; an absence of an amino acid component; an absence of a surfactant; an absence of a (further) tonicifier; and an absence of a (further) ionic strength provider.
G727. secukinumab; an absence of a sugar component; an absence of an amino acid component; an absence of a surfactant; an absence of a (further) tonicifier; and an absence of a (further) ionic strength provider.
G728. secukinumab; an absence of trehalose and sucrose; an absence of histidine and methionine; an absence of polysorbate 80; an absence of a (further) tonicifier; and an absence of methionine.
G729. secukinumab; an absence of a sugar component; an absence of histidine and methionine; an absence of polysorbate 80; an absence of a (further) tonicifier; and an absence of methionine.
G730. secukinumab; an absence of trehalose and sucrose; an absence of an amino acid component; an absence of polysorbate 80; an absence of a (further) tonicifier; and an absence of methionine.

(continued)

G731. secukinumab; an absence of a sugar component; an absence of an amino acid component; an absence of polysorbate 80; an absence of a (further) tonicifier; and an absence of methionine.

G732. secukinumab; an absence of trehalose and sucrose; an absence of histidine and methionine; an absence of a surfactant; an absence of a (further) tonicifier; and an absence of methionine.

G733. secukinumab; an absence of a sugar component; an absence of histidine and methionine; an absence of a surfactant; an absence of a (further) tonicifier; and an absence of methionine.

G734. secukinumab; an absence of trehalose and sucrose; an absence of an amino acid component; an absence of a surfactant; an absence of a (further) tonicifier; and an absence of methionine.

G735. secukinumab; an absence of a sugar component; an absence of an amino acid component; an absence of a surfactant; an absence of a (further) tonicifier; and an absence of methionine.

G736. secukinumab; an absence of trehalose and sucrose; an absence of histidine and methionine; an absence of polysorbate 80; an absence of a (further) tonicifier; and an absence of an antioxidant.

G737. secukinumab; an absence of a sugar component; an absence of histidine and methionine; an absence of polysorbate 80; an absence of a (further) tonicifier; and an absence of an antioxidant.

G738. secukinumab; an absence of trehalose and sucrose; an absence of an amino acid component; an absence of polysorbate 80; an absence of a (further) tonicifier; and an absence of an antioxidant.

G739. secukinumab; an absence of a sugar component; an absence of an amino acid component; an absence of polysorbate 80; an absence of a (further) tonicifier; and an absence of an antioxidant.

G740. secukinumab; an absence of trehalose and sucrose; an absence of histidine and methionine; an absence of a surfactant; an absence of a (further) tonicifier; and an absence of an antioxidant.

G741. secukinumab; an absence of a sugar component; an absence of histidine and methionine; an absence of a surfactant; an absence of a (further) tonicifier; and an absence of an antioxidant.

G742. secukinumab; an absence of trehalose and sucrose; an absence of an amino acid component; an absence of a surfactant; an absence of a (further) tonicifier; and an absence of an antioxidant.

G743. secukinumab; an absence of a sugar component; an absence of an amino acid component; an absence of a surfactant; an absence of a (further) tonicifier; and an absence of an antioxidant.

G744. secukinumab; an absence of trehalose and sucrose; an absence of histidine and methionine; an absence of polysorbate 80; an absence of a (further) ionic strength provider; and an absence of methionine.

G745. secukinumab; an absence of a sugar component; an absence of histidine and methionine; an absence of polysorbate 80; an absence of a (further) ionic strength provider; and an absence of methionine.

G746. secukinumab; an absence of trehalose and sucrose; an absence of an amino acid component; an absence of polysorbate 80; an absence of a (further) ionic strength provider; and an absence of methionine.

G747. secukinumab; an absence of a sugar component; an absence of an amino acid component; an absence of polysorbate 80; an absence of a (further) ionic strength provider; and an absence of methionine.

(continued)

G748. secukinumab; an absence of trehalose and sucrose; an absence of histidine and methionine; an absence of a surfactant; an absence of a (further) ionic strength provider; and an absence of methionine.

G749. secukinumab; an absence of a sugar component; an absence of histidine and methionine; an absence of a surfactant; an absence of a (further) ionic strength provider; and an absence of methionine.

G750. secukinumab; an absence of trehalose and sucrose; an absence of an amino acid component; an absence of a surfactant; an absence of a (further) ionic strength provider; and an absence of methionine.

G751. secukinumab; an absence of a sugar component; an absence of an amino acid component; an absence of a surfactant; an absence of a (further) ionic strength provider; and an absence of methionine.

G752. secukinumab; an absence of trehalose and sucrose; an absence of histidine and methionine; an absence of polysorbate 80; an absence of a (further) ionic strength provider; and an absence of an antioxidant.

G753. secukinumab; an absence of a sugar component; an absence of histidine and methionine; an absence of polysorbate 80; an absence of a (further) ionic strength provider; and an absence of an antioxidant.

G754. secukinumab; an absence of trehalose and sucrose; an absence of an amino acid component; an absence of polysorbate 80; an absence of a (further) ionic strength provider; and an absence of an antioxidant.

G755. secukinumab; an absence of a sugar component; an absence of an amino acid component; an absence of polysorbate 80; an absence of a (further) ionic strength provider; and an absence of an antioxidant.

G756. secukinumab; an absence of trehalose and sucrose; an absence of histidine and methionine; an absence of a surfactant; an absence of a (further) ionic strength provider; and an absence of an antioxidant.

G757. secukinumab; an absence of a sugar component; an absence of histidine and methionine; an absence of a surfactant; an absence of a (further) ionic strength provider; and an absence of an antioxidant.

G758. secukinumab; an absence of trehalose and sucrose; an absence of an amino acid component; an absence of a surfactant; an absence of a (further) ionic strength provider; and an absence of an antioxidant.

G759. secukinumab; an absence of a sugar component; an absence of an amino acid component; an absence of a surfactant; an absence of a (further) ionic strength provider; and an absence of an antioxidant.

G760. secukinumab; an absence of trehalose and sucrose; an absence of histidine and methionine; an absence of a (further) tonicifier; an absence of a (further) ionic strength provider; and an absence of methionine.

G761. secukinumab; an absence of a sugar component; an absence of histidine and methionine; an absence of a (further) tonicifier; an absence of a (further) ionic strength provider; and an absence of methionine.

G762. secukinumab; an absence of trehalose and sucrose; an absence of an amino acid component; an absence of a (further) tonicifier; an absence of a (further) ionic strength provider; and an absence of methionine.

G763. secukinumab; an absence of a sugar component; an absence of an amino acid component; an absence of a (further) tonicifier; an absence of a (further) ionic strength provider; and an absence of methionine.

G764. secukinumab; an absence of trehalose and sucrose; an absence of histidine and methionine; an absence of a (further) tonicifier; an absence of a (further) ionic strength provider; and an absence of an antioxidant.

(continued)

G765. secukinumab; an absence of a sugar component; an absence of histidine and methionine; an absence of a (further) tonicifier; an absence of a (further) ionic strength provider; and an absence of an antioxidant.

G766. secukinumab; an absence of trehalose and sucrose; an absence of an amino acid component; an absence of a (further) tonicifier; an absence of a (further) ionic strength provider; and an absence of an antioxidant.

G767. secukinumab; an absence of a sugar component; an absence of an amino acid component; an absence of a (further) tonicifier; an absence of a (further) ionic strength provider; and an absence of an antioxidant.

G768. secukinumab; an absence of trehalose and sucrose; an absence of polysorbate 80; an absence of a (further) tonicifier; an absence of a (further) ionic strength provider; and an absence of methionine.

G769. secukinumab; an absence of a sugar component; an absence of polysorbate 80; an absence of a (further) tonicifier; an absence of a (further) ionic strength provider; and an absence of methionine.

G770. secukinumab; an absence of trehalose and sucrose; an absence of a surfactant; an absence of a (further) tonicifier; an absence of a (further) ionic strength provider; and an absence of methionine.

G771. secukinumab; an absence of a sugar component; an absence of a surfactant; an absence of a (further) tonicifier; an absence of a (further) ionic strength provider; and an absence of methionine.

G772. secukinumab; an absence of trehalose and sucrose; an absence of polysorbate 80; an absence of a (further) tonicifier; an absence of a (further) ionic strength provider; and an absence of an antioxidant.

G773. secukinumab; an absence of a sugar component; an absence of polysorbate 80; an absence of a (further) tonicifier; an absence of a (further) ionic strength provider; and an absence of an antioxidant.

G774. secukinumab; an absence of trehalose and sucrose; an absence of a surfactant; an absence of a (further) tonicifier; an absence of a (further) ionic strength provider; and an absence of an antioxidant.

G775. secukinumab; an absence of a sugar component; an absence of a surfactant; an absence of a (further) tonicifier; an absence of a (further) ionic strength provider; and an absence of an antioxidant.

G776. secukinumab; an absence of histidine and methionine; an absence of polysorbate 80; an absence of a (further) tonicifier; an absence of a (further) ionic strength provider; and an absence of methionine.

G777. secukinumab; an absence of an amino acid component; an absence of polysorbate 80; an absence of a (further) tonicifier; an absence of a (further) ionic strength provider; and an absence of methionine.

G778. secukinumab; an absence of histidine and methionine; an absence of a surfactant; an absence of a (further) tonicifier; an absence of a (further) ionic strength provider; and an absence of methionine.

G779. secukinumab; an absence of an amino acid component; an absence of a surfactant; an absence of a (further) tonicifier; an absence of a (further) ionic strength provider; and an absence of methionine.

G780. secukinumab; an absence of histidine and methionine; an absence of polysorbate 80; an absence of a (further) tonicifier; an absence of a (further) ionic strength provider; and an absence of an antioxidant.

G781. secukinumab; an absence of an amino acid component; an absence of polysorbate 80; an absence of a (further) tonicifier; an absence of a (further) ionic strength provider; and an absence of an antioxidant.

(continued)

G782. secukinumab; an absence of histidine and methionine; an absence of a surfactant; an absence of a (further) tonicifier; an absence of a (further) ionic strength provider; and an absence of an antioxidant.

G783. secukinumab; an absence of an amino acid component; an absence of a surfactant; an absence of a (further) tonicifier; an absence of a (further) ionic strength provider; and an absence of an antioxidant.

G784. secukinumab; an absence of a histidine buffer system; an absence of trehalose and sucrose; an absence of histidine and methionine; an absence of polysorbate 80; an absence of a (further) tonicifier; and an absence of a (further) ionic strength provider.

G785. secukinumab; an absence of a buffer system; an absence of trehalose and sucrose; an absence of histidine and methionine; an absence of polysorbate 80; an absence of a (further) tonicifier; and an absence of a (further) ionic strength provider.

G786. secukinumab; an absence of a histidine buffer system; an absence of a sugar component; an absence of histidine and methionine; an absence of polysorbate 80; an absence of a (further) tonicifier; and an absence of a (further) ionic strength provider.

G787. secukinumab; an absence of a buffer system; an absence of a sugar component; an absence of histidine and methionine; an absence of polysorbate 80; an absence of a (further) tonicifier; and an absence of a (further) ionic strength provider.

G788. secukinumab; an absence of a histidine buffer system; an absence of trehalose and sucrose; an absence of an amino acid component; an absence of polysorbate 80; an absence of a (further) tonicifier; and an absence of a (further) ionic strength provider.

G789. secukinumab; an absence of a buffer system; an absence of trehalose and sucrose; an absence of an amino acid component; an absence of polysorbate 80; an absence of a (further) tonicifier; and an absence of a (further) ionic strength provider.

G790. secukinumab; an absence of a histidine buffer system; an absence of a sugar component; an absence of an amino acid component; an absence of polysorbate 80; an absence of a (further) tonicifier; and an absence of a (further) ionic strength provider.

G791. secukinumab; an absence of a buffer system; an absence of a sugar component; an absence of an amino acid component; an absence of polysorbate 80; an absence of a (further) tonicifier; and an absence of a (further) ionic strength provider.

G792. secukinumab; an absence of a histidine buffer system; an absence of trehalose and sucrose; an absence of histidine and methionine; an absence of a surfactant; an absence of a (further) tonicifier; and an absence of a (further) ionic strength provider.

G793. secukinumab; an absence of a buffer system; an absence of trehalose and sucrose; an absence of histidine and methionine; an absence of a surfactant; an absence of a (further) tonicifier; and an absence of a (further) ionic strength provider.

G794. secukinumab; an absence of a histidine buffer system; an absence of a sugar component; an absence of histidine and methionine; an absence of a surfactant; an absence of a (further) tonicifier; and an absence of a (further) ionic strength provider.

(continued)

G795. secukinumab; an absence of a buffer system; an absence of a sugar component; an absence of histidine and methionine; an absence of a surfactant; an absence of a (further) tonicifier; and an absence of a (further) ionic strength provider.

G796. secukinumab; an absence of a histidine buffer system; an absence of trehalose and sucrose; an absence of an amino acid component; an absence of a surfactant; an absence of a (further) tonicifier; and an absence of a (further) ionic strength provider.

G797. secukinumab; an absence of a buffer system; an absence of trehalose and sucrose; an absence of an amino acid component; an absence of a surfactant; an absence of a (further) tonicifier; and an absence of a (further) ionic strength provider.

G798. secukinumab; an absence of a histidine buffer system; an absence of a sugar component; an absence of an amino acid component; an absence of a surfactant; an absence of a (further) tonicifier; and an absence of a (further) ionic strength provider.

G799. secukinumab; an absence of a buffer system; an absence of a sugar component; an absence of an amino acid component; an absence of a surfactant; an absence of a (further) tonicifier; and an absence of a (further) ionic strength provider.

G800. secukinumab; an absence of a histidine buffer system; an absence of trehalose and sucrose; an absence of histidine and methionine; an absence of polysorbate 80; an absence of a (further) tonicifier; and an absence of methionine.

G801. secukinumab; an absence of a buffer system; an absence of trehalose and sucrose; an absence of histidine and methionine; an absence of polysorbate 80; an absence of a (further) tonicifier; and an absence of methionine.

G802. secukinumab; an absence of a histidine buffer system; an absence of a sugar component; an absence of histidine and methionine; an absence of polysorbate 80; an absence of a (further) tonicifier; and an absence of methionine.

G803. secukinumab; an absence of a buffer system; an absence of a sugar component; an absence of histidine and methionine; an absence of polysorbate 80; an absence of a (further) tonicifier; and an absence of methionine.

G804. secukinumab; an absence of a histidine buffer system; an absence of trehalose and sucrose; an absence of an amino acid component; an absence of polysorbate 80; an absence of a (further) tonicifier; and an absence of methionine.

G805. secukinumab; an absence of a buffer system; an absence of trehalose and sucrose; an absence of an amino acid component; an absence of polysorbate 80; an absence of a (further) tonicifier; and an absence of methionine.

G806. secukinumab; an absence of a histidine buffer system; an absence of a sugar component; an absence of an amino acid component; an absence of polysorbate 80; an absence of a (further) tonicifier; and an absence of methionine.

G807. secukinumab; an absence of a buffer system; an absence of a sugar component; an absence of an amino acid component; an absence of polysorbate 80; an absence of a (further) tonicifier; and an absence of methionine.

G808. secukinumab; an absence of a histidine buffer system; an absence of trehalose and sucrose; an absence of histidine and methionine; an absence of a surfactant; an absence of a (further) tonicifier; and an absence of methionine.

G809. secukinumab; an absence of a buffer system; an absence of trehalose and sucrose; an absence of histidine and methionine; an absence of a surfactant; an absence of a (further) tonicifier; and an absence of methionine.

(continued)

G810. secukinumab; an absence of a histidine buffer system; an absence of a sugar component; an absence of histidine and methionine; an absence of a surfactant; an absence of a (further) tonicifier; and an absence of methionine.

G811. secukinumab; an absence of a buffer system; an absence of a sugar component; an absence of histidine and methionine; an absence of a surfactant; an absence of a (further) tonicifier; and an absence of methionine.

G812. secukinumab; an absence of a histidine buffer system; an absence of trehalose and sucrose; an absence of an amino acid component; an absence of a surfactant; an absence of a (further) tonicifier; and an absence of methionine.

G813. secukinumab; an absence of a buffer system; an absence of trehalose and sucrose; an absence of an amino acid component; an absence of a surfactant; an absence of a (further) tonicifier; and an absence of methionine.

G814. secukinumab; an absence of a histidine buffer system; an absence of a sugar component; an absence of an amino acid component; an absence of a surfactant; an absence of a (further) tonicifier; and an absence of methionine.

G815. secukinumab; an absence of a buffer system; an absence of a sugar component; an absence of an amino acid component; an absence of a surfactant; an absence of a (further) tonicifier; and an absence of methionine.

G816. secukinumab; an absence of a histidine buffer system; an absence of trehalose and sucrose; an absence of histidine and methionine; an absence of polysorbate 80; an absence of a (further) tonicifier; and an absence of an antioxidant.

G817. secukinumab; an absence of a buffer system; an absence of trehalose and sucrose; an absence of histidine and methionine; an absence of polysorbate 80; an absence of a (further) tonicifier; and an absence of an antioxidant.

G818. secukinumab; an absence of a histidine buffer system; an absence of a sugar component; an absence of histidine and methionine; an absence of polysorbate 80; an absence of a (further) tonicifier; and an absence of an antioxidant.

G819. secukinumab; an absence of a buffer system; an absence of a sugar component; an absence of histidine and methionine; an absence of polysorbate 80; an absence of a (further) tonicifier; and an absence of an antioxidant.

G820. secukinumab; an absence of a histidine buffer system; an absence of trehalose and sucrose; an absence of an amino acid component; an absence of polysorbate 80; an absence of a (further) tonicifier; and an absence of an antioxidant.

G821. secukinumab; an absence of a buffer system; an absence of trehalose and sucrose; an absence of an amino acid component; an absence of polysorbate 80; an absence of a (further) tonicifier; and an absence of an antioxidant.

G822. secukinumab; an absence of a histidine buffer system; an absence of a sugar component; an absence of an amino acid component; an absence of polysorbate 80; an absence of a (further) tonicifier; and an absence of an antioxidant.

G823. secukinumab; an absence of a buffer system; an absence of a sugar component; an absence of an amino acid component; an absence of polysorbate 80; an absence of a (further) tonicifier; and an absence of an antioxidant.

G824. secukinumab; an absence of a histidine buffer system; an absence of trehalose and sucrose; an absence of histidine and methionine; an absence of a surfactant; an absence of a (further) tonicifier; and an absence of an antioxidant.

G825. secukinumab; an absence of a buffer system; an absence of trehalose and sucrose; an absence of histidine and methionine; an absence of a surfactant; an absence of a (further) tonicifier; and an absence of an antioxidant.

(continued)

G826. secukinumab; an absence of a histidine buffer system; an absence of a sugar component; an absence of histidine and methionine; an absence of a surfactant; an absence of a (further) tonicifier; and an absence of an antioxidant.

G827. secukinumab; an absence of a buffer system; an absence of a sugar component; an absence of histidine and methionine; an absence of a surfactant; an absence of a (further) tonicifier; and an absence of an antioxidant.

G828. secukinumab; an absence of a histidine buffer system; an absence of trehalose and sucrose; an absence of an amino acid component; an absence of a surfactant; an absence of a (further) tonicifier; and an absence of an antioxidant.

G829. secukinumab; an absence of a buffer system; an absence of trehalose and sucrose; an absence of an amino acid component; an absence of a surfactant; an absence of a (further) tonicifier; and an absence of an antioxidant.

G830. secukinumab; an absence of a histidine buffer system; an absence of a sugar component; an absence of an amino acid component; an absence of a surfactant; an absence of a (further) tonicifier; and an absence of an antioxidant.

G831. secukinumab; an absence of a buffer system; an absence of a sugar component; an absence of an amino acid component; an absence of a surfactant; an absence of a (further) tonicifier; and an absence of an antioxidant.

G832. secukinumab; an absence of a histidine buffer system; an absence of trehalose and sucrose; an absence of histidine and methionine; an absence of polysorbate 80; an absence of a (further) ionic strength provider; and an absence of methionine.

G833. secukinumab; an absence of a buffer system; an absence of trehalose and sucrose; an absence of histidine and methionine; an absence of polysorbate 80; an absence of a (further) ionic strength provider; and an absence of methionine.

G834. secukinumab; an absence of a histidine buffer system; an absence of a sugar component; an absence of histidine and methionine; an absence of polysorbate 80; an absence of a (further) ionic strength provider; and an absence of methionine.

G835. secukinumab; an absence of a buffer system; an absence of a sugar component; an absence of histidine and methionine; an absence of polysorbate 80; an absence of a (further) ionic strength provider; and an absence of methionine.

G836. secukinumab; an absence of a histidine buffer system; an absence of trehalose and sucrose; an absence of an amino acid component; an absence of polysorbate 80; an absence of a (further) ionic strength provider; and an absence of methionine.

G837. secukinumab; an absence of a buffer system; an absence of trehalose and sucrose; an absence of an amino acid component; an absence of polysorbate 80; an absence of a (further) ionic strength provider; and an absence of methionine.

G838. secukinumab; an absence of a histidine buffer system; an absence of a sugar component; an absence of an amino acid component; an absence of polysorbate 80; an absence of a (further) ionic strength provider; and an absence of methionine.

G839. secukinumab; an absence of a buffer system; an absence of a sugar component; an absence of an amino acid component; an absence of polysorbate 80; an absence of a (further) ionic strength provider; and an absence of methionine.

G840. secukinumab; an absence of a histidine buffer system; an absence of trehalose and sucrose; an absence of histidine and

(continued)

methionine; an absence of a surfactant; an absence of a (further) ionic strength provider; and an absence of methionine.

G841. secukinumab; an absence of a buffer system; an absence of trehalose and sucrose; an absence of histidine and methionine;
an absence of a surfactant; an absence of a (further) ionic strength provider; and an absence of methionine.

G842. secukinumab; an absence of a histidine buffer system; an absence of a sugar component; an absence of histidine and
methionine; an absence of a surfactant; an absence of a (further) ionic strength provider; and an absence of methionine.

G843. secukinumab; an absence of a buffer system; an absence of a sugar component; an absence of histidine and methionine;
an absence of a surfactant; an absence of a (further) ionic strength provider; and an absence of methionine.

G844. secukinumab; an absence of a histidine buffer system; an absence of trehalose and sucrose; an absence of an amino acid
component; an absence of a surfactant; an absence of a (further) ionic strength provider; and an absence of methionine.

G845. secukinumab; an absence of a buffer system; an absence of trehalose and sucrose; an absence of an amino acid
component; an absence of a surfactant; an absence of a (further) ionic strength provider; and an absence of methionine.

G846. secukinumab; an absence of a histidine buffer system; an absence of a sugar component; an absence of an amino acid
component; an absence of a surfactant; an absence of a (further) ionic strength provider; and an absence of methionine.

G847. secukinumab; an absence of a buffer system; an absence of a sugar component; an absence of an amino acid component;
an absence of a surfactant; an absence of a (further) ionic strength provider; and an absence of methionine.

G848. secukinumab; an absence of a histidine buffer system; an absence of trehalose and sucrose; an absence of histidine and
methionine; an absence of polysorbate 80; an absence of a (further) ionic strength provider; and an absence of an
antioxidant.

G849. secukinumab; an absence of a buffer system; an absence of trehalose and sucrose; an absence of histidine and methionine;
an absence of polysorbate 80; an absence of a (further) ionic strength provider; and an absence of an antioxidant.

G850. secukinumab; an absence of a histidine buffer system; an absence of a sugar component; an absence of histidine and
methionine; an absence of polysorbate 80; an absence of a (further) ionic strength provider; and an absence of an
antioxidant.

G851. secukinumab; an absence of a buffer system; an absence of a sugar component; an absence of histidine and methionine;
an absence of polysorbate 80; an absence of a (further) ionic strength provider; and an absence of an antioxidant.

G852. secukinumab; an absence of a histidine buffer system; an absence of trehalose and sucrose; an absence of an amino acid
component; an absence of polysorbate 80; an absence of a (further) ionic strength provider; and an absence of an
antioxidant.

G853. secukinumab; an absence of a buffer system; an absence of trehalose and sucrose; an absence of an amino acid

(continued)

component; an absence of polysorbate 80; an absence of a (further) ionic strength provider; and an absence of an antioxidant.

G854. secukinumab; an absence of a histidine buffer system; an absence of a sugar component; an absence of an amino acid component; an absence of polysorbate 80; an absence of a (further) ionic strength provider; and an absence of an antioxidant.

G855. secukinumab; an absence of a buffer system; an absence of a sugar component; an absence of an amino acid component; an absence of polysorbate 80; an absence of a (further) ionic strength provider; and an absence of an antioxidant.

G856. secukinumab; an absence of a histidine buffer system; an absence of trehalose and sucrose; an absence of histidine and methionine; an absence of a surfactant; an absence of a (further) ionic strength provider; and an absence of an antioxidant.

G857. secukinumab; an absence of a buffer system; an absence of trehalose and sucrose; an absence of histidine and methionine; an absence of a surfactant; an absence of a (further) ionic strength provider; and an absence of an antioxidant.

G858. secukinumab; an absence of a histidine buffer system; an absence of a sugar component; an absence of histidine and methionine; an absence of a surfactant; an absence of a (further) ionic strength provider; and an absence of an antioxidant.

G859. secukinumab; an absence of a buffer system; an absence of a sugar component; an absence of histidine and methionine; an absence of a surfactant; an absence of a (further) ionic strength provider; and an absence of an antioxidant.

G860. secukinumab; an absence of a histidine buffer system; an absence of trehalose and sucrose; an absence of an amino acid component; an absence of a surfactant; an absence of a (further) ionic strength provider; and an absence of an antioxidant.

G861. secukinumab; an absence of a buffer system; an absence of trehalose and sucrose; an absence of an amino acid component; an absence of a surfactant; an absence of a (further) ionic strength provider; and an absence of an antioxidant.

G862. secukinumab; an absence of a histidine buffer system; an absence of a sugar component; an absence of an amino acid component; an absence of a surfactant; an absence of a (further) ionic strength provider; and an absence of an antioxidant.

G863. secukinumab; an absence of a buffer system; an absence of a sugar component; an absence of an amino acid component; an absence of a surfactant; an absence of a (further) ionic strength provider; and an absence of an antioxidant.

G864. secukinumab; an absence of a histidine buffer system; an absence of trehalose and sucrose; an absence of histidine and methionine; an absence of a (further) tonicifier; an absence of a (further) ionic strength provider; and an absence of methionine .

G865. secukinumab; an absence of a buffer system; an absence of trehalose and sucrose; an absence of histidine and methionine; an absence of a (further) tonicifier; an absence of a (further) ionic strength provider; and an absence of methionine.

(continued)

G866. secukinumab; an absence of a histidine buffer system; an absence of a sugar component; an absence of histidine and methionine; an absence of a (further) tonicifier; an absence of a (further) ionic strength provider; and an absence of methionine .

G867. secukinumab; an absence of a buffer system; an absence of a sugar component; an absence of histidine and methionine; an absence of a (further) tonicifier; an absence of a (further) ionic strength provider; and an absence of methionine.

G868. secukinumab; an absence of a histidine buffer system; an absence of trehalose and sucrose; an absence of an amino acid component; an absence of a (further) tonicifier; an absence of a (further) ionic strength provider; and an absence of methionine .

G869. secukinumab; an absence of a buffer system; an absence of trehalose and sucrose; an absence of an amino acid component; an absence of a (further) tonicifier; an absence of a (further) ionic strength provider; and an absence of methionine .

G870. secukinumab; an absence of a histidine buffer system; an absence of a sugar component; an absence of an amino acid component; an absence of a (further) tonicifier; an absence of a (further) ionic strength provider; and an absence of methionine .

G871. secukinumab; an absence of a buffer system; an absence of a sugar component; an absence of an amino acid component; an absence of a (further) tonicifier; an absence of a (further) ionic strength provider; and an absence of methionine.

G872. secukinumab; an absence of a histidine buffer system; an absence of trehalose and sucrose; an absence of histidine and methionine; an absence of a (further) tonicifier; an absence of a (further) ionic strength provider; and an absence of an antioxidant.

G873. secukinumab; an absence of a buffer system; an absence of trehalose and sucrose; an absence of histidine and methionine; an absence of a (further) tonicifier; an absence of a (further) ionic strength provider; and an absence of an antioxidant.

G874. secukinumab; an absence of a histidine buffer system; an absence of a sugar component; an absence of histidine and methionine; an absence of a (further) tonicifier; an absence of a (further) ionic strength provider; and an absence of an antioxidant.

G875. secukinumab; an absence of a buffer system; an absence of a sugar component; an absence of histidine and methionine; an absence of a (further) tonicifier; an absence of a (further) ionic strength provider; and an absence of an antioxidant.

G876. secukinumab; an absence of a histidine buffer system; an absence of trehalose and sucrose; an absence of an amino acid component; an absence of a (further) tonicifier; an absence of a (further) ionic strength provider; and an absence of an antioxidant.

G877. secukinumab; an absence of a buffer system; an absence of trehalose and sucrose; an absence of an amino acid

(continued)

component; an absence of a (further) tonicifier; an absence of a (further) ionic strength provider; and an absence of an antioxidant.

G878. secukinumab; an absence of a histidine buffer system; an absence of a sugar component; an absence of an amino acid component; an absence of a (further) tonicifier; an absence of a (further) ionic strength provider; and an absence of an antioxidant.

G879. secukinumab; an absence of a buffer system; an absence of a sugar component; an absence of an amino acid component; an absence of a (further) tonicifier; an absence of a (further) ionic strength provider; and an absence of an antioxidant.

G880. secukinumab; an absence of a histidine buffer system; an absence of trehalose and sucrose; an absence of polysorbate 80; an absence of a (further) tonicifier; an absence of a (further) ionic strength provider; and an absence of methionine.

G881. secukinumab; an absence of a buffer system; an absence of trehalose and sucrose; an absence of polysorbate 80; an absence of a (further) tonicifier; an absence of a (further) ionic strength provider; and an absence of methionine.

G882. secukinumab; an absence of a histidine buffer system; an absence of a sugar component; an absence of polysorbate 80; an absence of a (further) tonicifier; an absence of a (further) ionic strength provider; and an absence of methionine.

G883. secukinumab; an absence of a buffer system; an absence of a sugar component; an absence of polysorbate 80; an absence of a (further) tonicifier; an absence of a (further) ionic strength provider; and an absence of methionine.

G884. secukinumab; an absence of a histidine buffer system; an absence of trehalose and sucrose; an absence of a surfactant; an absence of a (further) tonicifier; an absence of a (further) ionic strength provider; and an absence of methionine.

G885. secukinumab; an absence of a buffer system; an absence of trehalose and sucrose; an absence of a surfactant; an absence of a (further) tonicifier; an absence of a (further) ionic strength provider; and an absence of methionine.

G886. secukinumab; an absence of a histidine buffer system; an absence of a sugar component; an absence of a surfactant; an absence of a (further) tonicifier; an absence of a (further) ionic strength provider; and an absence of methionine.

G887. secukinumab; an absence of a buffer system; an absence of a sugar component; an absence of a surfactant; an absence of a (further) tonicifier; an absence of a (further) ionic strength provider; and an absence of methionine.

G888. secukinumab; an absence of a histidine buffer system; an absence of trehalose and sucrose; an absence of polysorbate 80; an absence of a (further) tonicifier; an absence of a (further) ionic strength provider; and an absence of an antioxidant.

G889. secukinumab; an absence of a buffer system; an absence of trehalose and sucrose; an absence of polysorbate 80; an absence of a (further) tonicifier; an absence of a (further) ionic strength provider; and an absence of an antioxidant.

G890. secukinumab; an absence of a histidine buffer system; an absence of a sugar component; an absence of polysorbate 80; an absence of a (further) tonicifier; an absence of a (further) ionic strength provider; and an absence of an antioxidant.

(continued)

G891. secukinumab; an absence of a buffer system; an absence of a sugar component; an absence of polysorbate 80; an absence of a (further) tonicifier; an absence of a (further) ionic strength provider; and an absence of an antioxidant.

G892. secukinumab; an absence of a histidine buffer system; an absence of trehalose and sucrose; an absence of a surfactant; an absence of a (further) tonicifier; an absence of a (further) ionic strength provider; and an absence of an antioxidant.

G893. secukinumab; an absence of a buffer system; an absence of trehalose and sucrose; an absence of a surfactant; an absence of a (further) tonicifier; an absence of a (further) ionic strength provider; and an absence of an antioxidant.

G894. secukinumab; an absence of a histidine buffer system; an absence of a sugar component; an absence of a surfactant; an absence of a (further) tonicifier; an absence of a (further) ionic strength provider; and an absence of an antioxidant.

G895. secukinumab; an absence of a buffer system; an absence of a sugar component; an absence of a surfactant; an absence of a (further) tonicifier; an absence of a (further) ionic strength provider; and an absence of an antioxidant.

G896. secukinumab; an absence of a histidine buffer system; an absence of histidine and methionine; an absence of polysorbate 80; an absence of a (further) tonicifier; an absence of a (further) ionic strength provider; and an absence of methionine.

G897. secukinumab; an absence of a buffer system; an absence of histidine and methionine; an absence of polysorbate 80; an absence of a (further) tonicifier; an absence of a (further) ionic strength provider; and an absence of methionine.

G898. secukinumab; an absence of a histidine buffer system; an absence of an amino acid component; an absence of polysorbate 80; an absence of a (further) tonicifier; an absence of a (further) ionic strength provider; and an absence of methionine.

G899. secukinumab; an absence of a buffer system; an absence of an amino acid component; an absence of polysorbate 80; an absence of a (further) tonicifier; an absence of a (further) ionic strength provider; and an absence of methionine.

G900. secukinumab; an absence of a histidine buffer system; an absence of histidine and methionine; an absence of a surfactant; an absence of a (further) tonicifier; an absence of a (further) ionic strength provider; and an absence of methionine.

G901. secukinumab; an absence of a buffer system; an absence of histidine and methionine; an absence of a surfactant; an absence of a (further) tonicifier; an absence of a (further) ionic strength provider; and an absence of methionine.

G902. secukinumab; an absence of a histidine buffer system; an absence of an amino acid component; an absence of a surfactant; an absence of a (further) tonicifier; an absence of a (further) ionic strength provider; and an absence of methionine.

G903. secukinumab; an absence of a buffer system; an absence of an amino acid component; an absence of a surfactant; an absence of a (further) tonicifier; an absence of a (further) ionic strength provider; and an absence of methionine.

G904. secukinumab; an absence of a histidine buffer system; an absence of histidine and methionine; an absence of polysorbate 80; an absence of a (further) tonicifier; an absence of a (further) ionic strength provider; and an absence of an antioxidant.

(continued)

G905. secukinumab; an absence of a buffer system; an absence of histidine and methionine; an absence of polysorbate 80; an absence of a (further) tonicifier; an absence of a (further) ionic strength provider; and an absence of an antioxidant.

G906. secukinumab; an absence of a histidine buffer system; an absence of an amino acid component; an absence of polysorbate 80; an absence of a (further) tonicifier; an absence of a (further) ionic strength provider; and an absence of an antioxidant.

G907. secukinumab; an absence of a buffer system; an absence of an amino acid component; an absence of polysorbate 80; an absence of a (further) tonicifier; an absence of a (further) ionic strength provider; and an absence of an antioxidant.

G908. secukinumab; an absence of a histidine buffer system; an absence of histidine and methionine; an absence of a surfactant; an absence of a (further) tonicifier; an absence of a (further) ionic strength provider; and an absence of an antioxidant.

G909. secukinumab; an absence of a buffer system; an absence of histidine and methionine; an absence of a surfactant; an absence of a (further) tonicifier; an absence of a (further) ionic strength provider; and an absence of an antioxidant.

G910. secukinumab; an absence of a histidine buffer system; an absence of an amino acid component; an absence of a surfactant; an absence of a (further) tonicifier; an absence of a (further) ionic strength provider; and an absence of an antioxidant.

G911. secukinumab; an absence of a buffer system; an absence of an amino acid component; an absence of a surfactant; an absence of a (further) tonicifier; an absence of a (further) ionic strength provider; and an absence of an antioxidant.

G912. secukinumab; an absence of trehalose and sucrose; an absence of histidine and methionine; an absence of polysorbate 80; an absence of a (further) tonicifier; an absence of a (further) ionic strength provider; and an absence of methionine.

G913. secukinumab; an absence of a sugar component; an absence of histidine and methionine; an absence of polysorbate 80; an absence of a (further) tonicifier; an absence of a (further) ionic strength provider; and an absence of methionine.

G914. secukinumab; an absence of trehalose and sucrose; an absence of an amino acid component; an absence of polysorbate 80; an absence of a (further) tonicifier; an absence of a (further) ionic strength provider; and an absence of methionine.

G915. secukinumab; an absence of a sugar component; an absence of an amino acid component; an absence of polysorbate 80; an absence of a (further) tonicifier; an absence of a (further) ionic strength provider; and an absence of methionine.

G916. secukinumab; an absence of trehalose and sucrose; an absence of histidine and methionine; an absence of a surfactant; an absence of a (further) tonicifier; an absence of a (further) ionic strength provider; and an absence of methionine.

G917. secukinumab; an absence of a sugar component; an absence of histidine and methionine; an absence of a surfactant; an absence of a (further) tonicifier; an absence of a (further) ionic strength provider; and an absence of methionine.

(continued)

G918. secukinumab; an absence of trehalose and sucrose; an absence of an amino acid component; an absence of a surfactant; an absence of a (further) tonicifier; an absence of a (further) ionic strength provider; and an absence of methionine.

G919. secukinumab; an absence of a sugar component; an absence of an amino acid component; an absence of a surfactant; an absence of a (further) tonicifier; an absence of a (further) ionic strength provider; and an absence of methionine.

G920. secukinumab; an absence of trehalose and sucrose; an absence of histidine and methionine; an absence of polysorbate 80; an absence of a (further) tonicifier; an absence of a (further) ionic strength provider; and an absence of an antioxidant.

G921. secukinumab; an absence of a sugar component; an absence of histidine and methionine; an absence of polysorbate 80; an absence of a (further) tonicifier; an absence of a (further) ionic strength provider; and an absence of an antioxidant.

G922. secukinumab; an absence of trehalose and sucrose; an absence of an amino acid component; an absence of polysorbate 80; an absence of a (further) tonicifier; an absence of a (further) ionic strength provider; and an absence of an antioxidant.

G923. secukinumab; an absence of a sugar component; an absence of an amino acid component; an absence of polysorbate 80; an absence of a (further) tonicifier; an absence of a (further) ionic strength provider; and an absence of an antioxidant.

G924. secukinumab; an absence of trehalose and sucrose; an absence of histidine and methionine; an absence of a surfactant; an absence of a (further) tonicifier; an absence of a (further) ionic strength provider; and an absence of an antioxidant.

G925. secukinumab; an absence of a sugar component; an absence of histidine and methionine; an absence of a surfactant; an absence of a (further) tonicifier; an absence of a (further) ionic strength provider; and an absence of an antioxidant.

G926. secukinumab; an absence of trehalose and sucrose; an absence of an amino acid component; an absence of a surfactant; an absence of a (further) tonicifier; an absence of a (further) ionic strength provider; and an absence of an antioxidant.

G927. secukinumab; an absence of a sugar component; an absence of an amino acid component; an absence of a surfactant; an absence of a (further) tonicifier; an absence of a (further) ionic strength provider; and an absence of an antioxidant.

G928. secukinumab; an absence of a histidine buffer system; an absence of trehalose and sucrose; an absence of histidine and methionine; an absence of polysorbate 80; an absence of a (further) tonicifier; an absence of a (further) ionic strength provider; and an absence of methionine.

G929. secukinumab; an absence of a buffer system; an absence of trehalose and sucrose; an absence of histidine and methionine; an absence of polysorbate 80; an absence of a (further) tonicifier; an absence of a (further) ionic strength provider; and an absence of methionine.

G930. secukinumab; an absence of a histidine buffer system; an absence of a sugar component; an absence of histidine and

(continued)

methionine; an absence of polysorbate 80; an absence of a (further) tonicifier; an absence of a (further) ionic strength provider; and an absence of methionine.

G931. secukinumab; an absence of a buffer system; an absence of a sugar component; an absence of histidine and methionine; an absence of polysorbate 80; an absence of a (further) tonicifier; an absence of a (further) ionic strength provider; and an absence of methionine.

G932. secukinumab; an absence of a histidine buffer system; an absence of trehalose and sucrose; an absence of an amino acid component; an absence of polysorbate 80; an absence of a (further) tonicifier; an absence of a (further) ionic strength provider; and an absence of methionine.

G933. secukinumab; an absence of a buffer system; an absence of trehalose and sucrose; an absence of an amino acid component; an absence of polysorbate 80; an absence of a (further) tonicifier; an absence of a (further) ionic strength provider; and an absence of methionine.

G934. secukinumab; an absence of a histidine buffer system; an absence of a sugar component; an absence of an amino acid component; an absence of polysorbate 80; an absence of a (further) tonicifier; an absence of a (further) ionic strength provider; and an absence of methionine.

G935. secukinumab; an absence of a buffer system; an absence of a sugar component; an absence of an amino acid component; an absence of polysorbate 80; an absence of a (further) tonicifier; an absence of a (further) ionic strength provider; and an absence of methionine.

G936. secukinumab; an absence of a histidine buffer system; an absence of trehalose and sucrose; an absence of histidine and methionine; an absence of a surfactant; an absence of a (further) tonicifier; an absence of a (further) ionic strength provider; and an absence of methionine.

G937. secukinumab; an absence of a buffer system; an absence of trehalose and sucrose; an absence of histidine and methionine; an absence of a surfactant; an absence of a (further) tonicifier; an absence of a (further) ionic strength provider; and an absence of methionine.

G938. secukinumab; an absence of a histidine buffer system; an absence of a sugar component; an absence of histidine and methionine; an absence of a surfactant; an absence of a (further) tonicifier; an absence of a (further) ionic strength provider; and an absence of methionine.

G939. secukinumab; an absence of a buffer system; an absence of a sugar component; an absence of histidine and methionine; an absence of a surfactant; an absence of a (further) tonicifier; an absence of a (further) ionic strength provider; and an absence of methionine.

G940. secukinumab; an absence of a histidine buffer system; an absence of trehalose and sucrose; an absence of an amino acid component; an absence of a surfactant; an absence of a (further) tonicifier; an absence of a (further) ionic strength provider; and an absence of methionine.

(continued)

G941. secukinumab; an absence of a buffer system; an absence of trehalose and sucrose; an absence of an amino acid component; an absence of a surfactant; an absence of a (further) tonicifier; an absence of a (further) ionic strength provider; and an absence of methionine.

G942. secukinumab; an absence of a histidine buffer system; an absence of a sugar component; an absence of an amino acid component; an absence of a surfactant; an absence of a (further) tonicifier; an absence of a (further) ionic strength provider; and an absence of methionine.

G943. secukinumab; an absence of a buffer system; an absence of a sugar component; an absence of an amino acid component; an absence of a surfactant; an absence of a (further) tonicifier; an absence of a (further) ionic strength provider; and an absence of methionine.

G944. secukinumab; an absence of a histidine buffer system; an absence of trehalose and sucrose; an absence of histidine and methionine; an absence of polysorbate 80; an absence of a (further) tonicifier; an absence of a (further) ionic strength provider; and an absence of an antioxidant.

G945. secukinumab; an absence of a buffer system; an absence of trehalose and sucrose; an absence of histidine and methionine; an absence of polysorbate 80; an absence of a (further) tonicifier; an absence of a (further) ionic strength provider; and an absence of an antioxidant.

G946. secukinumab; an absence of a histidine buffer system; an absence of a sugar component; an absence of histidine and methionine; an absence of polysorbate 80; an absence of a (further) tonicifier; an absence of a (further) ionic strength provider; and an absence of an antioxidant.

G947. secukinumab; an absence of a buffer system; an absence of a sugar component; an absence of histidine and methionine; an absence of polysorbate 80; an absence of a (further) tonicifier; an absence of a (further) ionic strength provider; and an absence of an antioxidant.

G948. secukinumab; an absence of a histidine buffer system; an absence of trehalose and sucrose; an absence of an amino acid component; an absence of polysorbate 80; an absence of a (further) tonicifier; an absence of a (further) ionic strength provider; and an absence of an antioxidant.

G949. secukinumab; an absence of a buffer system; an absence of trehalose and sucrose; an absence of an amino acid component; an absence of polysorbate 80; an absence of a (further) tonicifier; an absence of a (further) ionic strength provider; and an absence of an antioxidant.

G950. secukinumab; an absence of a histidine buffer system; an absence of a sugar component; an absence of an amino acid component; an absence of polysorbate 80; an absence of a (further) tonicifier; an absence of a (further) ionic strength provider; and an absence of an antioxidant.

G951. secukinumab; an absence of a buffer system; an absence of a sugar component; an absence of an amino acid component; an absence of polysorbate 80; an absence of a (further) tonicifier; an absence of a (further) ionic strength provider; and an absence of an antioxidant.

G952. secukinumab; an absence of a histidine buffer system; an absence of trehalose and sucrose; an absence of histidine and methionine; an absence of a surfactant; an absence of a (further) tonicifier; an absence of a (further) ionic strength provider; and an absence of an antioxidant.

G953. secukinumab; an absence of a buffer system; an absence of trehalose and sucrose; an absence of histidine and methionine; an absence of a surfactant; an absence of a (further) tonicifier; an absence of a (further) ionic strength provider; and an absence of an antioxidant.

(continued)

G954. secukinumab; an absence of a histidine buffer system; an absence of a sugar component; an absence of histidine and methionine; an absence of a surfactant; an absence of a (further) tonicifier; an absence of a (further) ionic strength provider; and an absence of an antioxidant.

G955. secukinumab; an absence of a buffer system; an absence of a sugar component; an absence of histidine and methionine; an absence of a surfactant; an absence of a (further) tonicifier; an absence of a (further) ionic strength provider; and an absence of an antioxidant.

G956. secukinumab; an absence of a histidine buffer system; an absence of trehalose and sucrose; an absence of an amino acid component; an absence of a surfactant; an absence of a (further) tonicifier; an absence of a (further) ionic strength provider; and an absence of an antioxidant.

G957. secukinumab; an absence of a buffer system; an absence of trehalose and sucrose; an absence of an amino acid component; an absence of a surfactant; an absence of a (further) tonicifier; an absence of a (further) ionic strength provider; and an absence of an antioxidant.

G958. secukinumab; an absence of a histidine buffer system; an absence of a sugar component; an absence of an amino acid component; an absence of a surfactant; an absence of a (further) tonicifier; an absence of a (further) ionic strength provider; and an absence of an antioxidant.

G959. secukinumab; an absence of a buffer system; an absence of a sugar component; an absence of an amino acid component; an absence of a surfactant; an absence of a (further) tonicifier; an absence of a (further) ionic strength provider; and an absence of an antioxidant.

The (substantial) exclusion of certain ingredient(s)/component(s)/feature(s) can be as significant as included ingredient(s)/component(s)/feature(s).

The embodiments of G1-G959 may be further defined or characterised by features of numbered paragraphs A1-A51 1, B1-B255, C1-C255, D1-D1152, E1-E1151, F1-F12, H1-H1423, 11-12400, and J1-J1158.

***Biopharmaceutical Active***

The biopharmaceutical composition preferably comprises a biopharmaceutical active. Aspects and embodiments of the invention which refer to "a biopharmaceutical active" or something similar, suitably mean a biopharmaceutical active which is an antibody, preferably an anti-IL-17A antibody suitably of the IgG1 subclass, more preferably an anti-IL-17A (interleukin-17A antagonist) human IgG1/κ monoclonal antibody, most preferably secukinumab. In general, different antibodies (especially those of a different IgG subclass and/or with a different antigen target) will tend to behave differently under otherwise like conditions.

Most preferably, the biopharmaceutical active is secukinumab. As such, the biopharmaceutical composition preferably comprises secukinumab.

In an embodiment, the biopharmaceutical composition comprises 20-400 mg/mL secukinumab. In an embodiment, the biopharmaceutical composition comprises 20-175 mg/mL secukinumab. In an embodiment, the biopharmaceutical composition comprises 176-400 mg/mL secukinumab. In an embodiment, the biopharmaceutical composition comprises 100-350 mg/mL secukinumab. In an embodiment, the biopharmaceutical composition comprises 150-300 mg/mL secukinumab. In an embodiment, the biopharmaceutical composition comprises 120-220 mg/mL secukinumab.

In an embodiment, the biopharmaceutical composition comprises 145-190 mg/mL secukinumab.

In an embodiment, the biopharmaceutical composition comprises 145-170 mg/mL secukinumab. In an embodiment, the biopharmaceutical composition comprises 145-155 mg/mL secukinumab. In a particularly preferred embodiment, the biopharmaceutical composition comprises 150 mg/mL secukinumab.

In an embodiment, the biopharmaceutical composition comprises 155-170 mg/mL secukinumab. In an embodiment, the biopharmaceutical composition comprises 165 mg/mL secukinumab. In an embodiment, the biopharmaceutical composition comprises 160-185 mg/mL secukinumab. In an embodiment, the biopharmaceutical composition comprises 175-185 mg/mL secukinumab. In a particularly preferred embodiment, the biopharmaceutical composition comprises 180 mg/mL secukinumab.

In a particularly preferred embodiment, the biopharmaceutical composition comprises 176-300 mg/mL secukinumab. In a particularly preferred embodiment, the biopharmaceutical composition comprises 176-220 mg/mL secukinumab. In an embodiment, the biopharmaceutical composition comprises 180-300 mg/mL secukinumab. In an embodiment, the biopharmaceutical composition comprises 180-220 mg/mL secukinumab. In an embodiment, the biopharmaceutical composition comprises 185-205 mg/mL secukinumab. In an embodiment, the biopharmaceutical composition comprises 185-195 mg/mL secukinumab. In an embodiment, the biopharmaceutical composition comprises 190 mg/mL secukinumab. In an embodiment, the biopharmaceutical composition comprises 195-205 mg/mL secukinumab. In an embodiment, the biopharmaceutical composition comprises 200 mg/mL secukinumab.

Suitably, the biopharmaceutical composition comprises 0.135-2.70 mM secukinumab. Suitably, the biopharmaceutical composition comprises 0.135-1.18 mM secukinumab. Suitably, the biopharmaceutical composition comprises 1.19-2.70 mM secukinumab. Suitably, the biopharmaceutical composition comprises 0.68-2.36 mM secukinumab. Suitably, the biopharmaceutical composition comprises 1.01-2.03 mM secukinumab. Suitably, the biopharmaceutical composition comprises 0.81-1.49 mM secukinumab.

In an embodiment, the biopharmaceutical composition comprises 0.98-1.28 mM secukinumab. In an embodiment, the biopharmaceutical composition comprises 0.98-1.15 mM secukinumab. In an embodiment, the biopharmaceutical composition comprises 0.98-1.05 mM secukinumab. In an embodiment, the biopharmaceutical composition comprises 1.01 mM secukinumab.

In an embodiment, the biopharmaceutical composition comprises 1.05-1.15 mM secukinumab. In an embodiment, the biopharmaceutical composition comprises 1.11 mM secukinumab. In an embodiment, the biopharmaceutical composition comprises 1.08-1.25 mM secukinumab. In an embodiment, the biopharmaceutical composition comprises 1.18-1.25 mM secukinumab. In an embodiment, the biopharmaceutical composition comprises 1.22 mM secukinumab.

Suitably, the biopharmaceutical composition comprises 1.19-2.03 mM secukinumab. Suitably, the biopharmaceutical composition comprises 1.19-1.49 mM secukinumab. Suitably, the biopharmaceutical composition comprises 1.22-2.03 mM secukinumab. Suitably, the biopharmaceutical composition comprises 1.22-1.49 mM secukinumab. Suitably, the biopharmaceutical composition comprises 1.25-1.39 mM secukinumab. Suitably, the biopharmaceutical composition comprises 1.25-1.32 mM secukinumab. Suitably, the biopharmaceutical composition comprises 1.28 mM secukinumab. Suitably, the biopharmaceutical composition comprises 1.32-1.39 mM secukinumab. Suitably, the biopharmaceutical composition comprises 1.35 mM secukinumab.

The aforementioned characteristics of a biopharmaceutical composition, in particular in relation to secukinumab and/or amounts thereof, may be applied to any of the embodiments defined in numbered paragraphs A1-A511, B1-B255, C1-C255, D1-D1152, E1-E1151, F1-F12, G1-G959, H1-H1423, 11-12400, and J1-J1158.

***Buffers***

The biopharmaceutical composition suitably further comprises a buffer system.

The buffer system suitably is or comprises a monoprotic buffer system.

The buffer system suitably is or comprises a polyprotic buffer system.

The buffer system suitably is or comprises an zwitterionic buffer system.

The buffer system suitably is or comprises a single-buffer system. The buffer system suitably is or comprises a multi-buffer system.

The buffer system suitably is or comprises a dual-buffer system. The buffer system suitably is or comprises a dual-buffer system consisting of a first buffer system and a second buffer system in a respective molar ratio of between 20:1 and 1:20. The buffer system suitably is or comprises a dual-buffer system consisting of a first buffer system and a second buffer system in a respective molar ratio of between 5:1 and 1:5. The buffer system suitably is or comprises a dual-buffer system consisting of a first buffer system and a second buffer system in a respective molar ratio of between 3:1 and 1:3. The buffer system suitably is or comprises a dual-buffer system consisting of a first buffer system and a second buffer system in a respective molar ratio of between 2:1 and 1:2. The buffer system suitably is or comprises a dual-buffer system consisting of a first buffer system and a second buffer system in a molar ratio of 1:1. Unless stated otherwise, where the buffer system is a dual-buffer system (e.g. a phosphate-citrate buffer system), amounts or concentrations of said dual-buffer system are total (i.e. combined) amounts/concentrations of the two buffer systems constituting said dual-buffer system. When molar ratios are stipulated in relation to the two buffer systems, such molar ratios divide any such total (i.e. combined) amounts/concentrations between the two buffer systems.

The buffer system suitably is or comprises a buffer system selected from the group consisting of a histidine buffer system, a succinate buffer system, an adipate buffer system, an acetate buffer system, a phosphate buffer system, a citrate buffer system, a phosphate-citrate buffer system, a phosphate-succinate buffer system, a phosphate acetate buffer system, a formate buffer system, a lactate buffer system, a salicylate buffer system, a benzoate buffer system, a maleate buffer system, a malate buffer system, a fumarate buffer system, a tartrate buffer system, a glycine buffer system, a lysine buffer system, a glycylglycine buffer system, a glutamate buffer system, an asparate buffer system, a histidine-acetate buffer system, a gluconate buffer system, a MES buffer system, and any combination thereof (suitably up to a maximum of two specific buffer systems).

The buffer system suitably is or comprises a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a formate buffer system, a lactate buffer system, a salicylate buffer system, a benzoate buffer system, a maleate buffer system, a malate buffer system, a fumarate buffer system, a tartrate buffer system, a glycine buffer system, a lysine buffer system, a glycylglycine buffer system, a glutamate buffer system, an asparate buffer system, a gluconate buffer system, a MES buffer system, and any combination thereof (suitably up to a maximum of two specific buffer systems).

The buffer system suitably is or comprises a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a glycine buffer system, a glutamate buffer system, a gluconate buffer system, a MES buffer system, and any combination thereof (suitably up to a maximum of two specific buffer systems).

The buffer system suitably is or comprises a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a MES buffer system, and any combination thereof (suitably up to a maximum of two specific buffer systems).

The buffer system suitably is or comprises a buffer system selected from the group consisting of a succinate buffer system, an adipate buffer system, a phosphate buffer system, a citrate buffer system, a phosphate-citrate buffer system, and any combination thereof (suitably up to a maximum of two specific buffer systems).

The buffer system suitably is or comprises a histidine buffer system.

The buffer system suitably is or comprises a buffer system excluding histidine.

The buffer system suitably is or comprises a succinate buffer system. The buffer system suitably is or comprises an adipate buffer system. The buffer system suitably is or comprises an acetate buffer system. The buffer system suitably is or comprises a phosphate buffer system. The buffer system suitably is or comprises a citrate buffer system. The buffer system suitably is or comprises a formate buffer system. The buffer system suitably is or comprises a lactate buffer system. The buffer system suitably is or comprises a salicylate buffer system. The buffer system suitably is or comprises a benzoate buffer system. The buffer system suitably is or comprises a maleate buffer system. The buffer system suitably is or comprises a malate buffer system. The buffer system suitably is or comprises a fumarate buffer system. The buffer system suitably is or comprises a tartrate buffer system. The buffer system suitably is or comprises a glycine buffer system. The buffer system suitably is or comprises a lysine buffer system. The buffer system suitably is or comprises a glycylglycine buffer system. The buffer system suitably is or comprises a glutamate buffer system. The buffer system suitably is or comprises an asparate buffer system. The buffer system suitably is or comprises a histidine-acetate buffer system. The buffer system suitably is or comprises a gluconate buffer system. The buffer system suitably is or comprises a MES buffer system.

The buffer system suitably is or comprises a phosphate-citrate buffer system. The buffer system suitably is or comprises a phosphate-citrate buffer system consisting of a phosphate buffer system and a citrate buffer system in a respective molar ratio of between 20:1 and 1:20. The buffer system suitably is or comprises a phosphate-citrate buffer system consisting of a phosphate buffer system and a citrate buffer system in a respective molar ratio of between 5:1 and 1:5. The buffer system suitably is or comprises a phosphate-citrate buffer system consisting of a phosphate buffer system and a citrate buffer system in a respective molar ratio of between 3:1 and 1:3. The buffer system suitably is or comprises a phosphate-citrate buffer system consisting of a phosphate buffer system and a citrate buffer system in a respective molar ratio of between 2:1 and 1:2. The buffer system suitably is or comprises a phosphate-citrate buffer system consisting of a phosphate buffer system and a citrate buffer system in a molar ratio of 1:1. Unless stated otherwise, amounts or concentrations of a phosphate-citrate buffer system are total (i.e. combined) amounts/concentrations of the phosphate buffer system and citrate buffer system constituting said dual-buffer system. When molar ratios are stipulated in relation to the phosphate and citrate buffer systems, such molar ratios divide any such total (i.e. combined) amounts/concentrations between the two buffer systems.

The buffer system suitably is or comprises a phosphate-succinate buffer system. The buffer system suitably is or comprises a phosphate-succinate buffer system consisting of a phosphate buffer system and a succinate buffer system in a respective molar ratio of between 20:1 and 1:20. The buffer system suitably is or comprises a phosphate-succinate buffer system consisting of a phosphate buffer system and a succinate buffer system in a respective molar ratio of between 5:1 and 1:5. The buffer system suitably is or comprises a phosphate-succinate buffer system consisting of a phosphate buffer system and a succinate buffer system in a respective molar ratio of between 3:1 and 1:3. The buffer system suitably is or comprises a phosphate-succinate buffer system consisting of a phosphate buffer system and a succinate buffer system in a respective molar ratio of between 2:1 and 1:2. The buffer system suitably is or comprises a phosphate-succinate buffer system consisting of a phosphate buffer system and a succinate buffer system in a molar ratio of 1:1. Unless stated otherwise, amounts or concentrations of a phosphate-succinate buffer system are total (i.e. combined) amounts/concentrations of the phosphate buffer system and succinate buffer system constituting said dual-buffer system. When molar ratios are stipulated in relation to the phosphate and succinate buffer systems, such molar ratios divide any such total (i.e. combined) amounts/concentrations between the two buffer systems.

The buffer system suitably is or comprises a phosphate-acetate buffer system. The buffer system suitably is or comprises a phosphate-acetate buffer system consisting of a phosphate buffer system and a acetate buffer system in a respective molar ratio of between 20:1 and 1:20. The buffer system suitably is or comprises a phosphate-acetate buffer system consisting of a phosphate buffer system and a acetate buffer system in a respective molar ratio of between 5:1 and 1:5. The buffer system suitably is or comprises a phosphate-acetate buffer system consisting of a phosphate buffer system and a acetate buffer system in a respective molar ratio of between 3:1 and 1:3. The buffer system suitably is or comprises a phosphate-acetate buffer system consisting of a phosphate buffer system and a acetate buffer system in a respective molar ratio of between 2:1 and 1:2. The buffer system suitably is or comprises a phosphate-acetate buffer system consisting of a phosphate buffer system and a acetate buffer system in a molar ratio of 1:1. Unless stated otherwise, amounts or concentrations of a phosphate-acetate buffer system are total (i.e. combined) amounts/concentrations of the phosphate buffer system and acetate buffer system constituting said dual-buffer system. When molar ratios are stipulated in relation to the phosphate and acetate buffer systems, such molar ratios divide any such total (i.e. combined) amounts/concentrations between the two buffer systems.

The biopharmaceutical composition suitably comprises 1-70 mM buffer system. The biopharmaceutical composition suitably comprises 2-60 mM buffer system. The biopharmaceutical composition suitably comprises 3-56 mM buffer system. The biopharmaceutical composition suitably comprises 5-50 mM buffer system. The biopharmaceutical composition suitably comprises 10-30 mM buffer system. The biopharmaceutical composition suitably comprises 20 mM buffer system. The biopharmaceutical composition suitably comprises less than 10 mM buffer system. The biopharmaceutical composition suitably comprises 1-9 mM buffer system. The biopharmaceutical composition suitably comprises 6-10 mM buffer system. The biopharmaceutical composition suitably comprises 8 mM buffer system. The biopharmaceutical composition suitably comprises less than 5 mM buffer system. The biopharmaceutical composition suitably comprises 0.5-4.5 mM buffer system. The biopharmaceutical composition suitably comprises 1-4 mM buffer system. The biopharmaceutical composition suitably comprises 2-6 mM buffer system. The biopharmaceutical composition suitably comprises 4 mM buffer system. The biopharmaceutical composition suitably comprises greater than 50 mM buffer system. The biopharmaceutical composition suitably comprises 51-70 mM buffer system. The biopharmaceutical composition suitably comprises 51-60 mM buffer system. The biopharmaceutical composition suitably comprises 55 mM buffer system.

The biopharmaceutical composition suitably comprises 1-70 mM buffer system excluding histidine. The biopharmaceutical composition suitably comprises 10-30 mM buffer system excluding histidine. The biopharmaceutical composition suitably comprises 1-9 mM buffer system excluding histidine. The biopharmaceutical composition suitably comprises 8 mM buffer system excluding histidine. The biopharmaceutical composition suitably comprises less than 5 mM buffer system excluding histidine. The biopharmaceutical composition suitably comprises 0.5-4.5 mM buffer system excluding histidine. The biopharmaceutical composition suitably comprises 4 mM buffer system excluding histidine. The biopharmaceutical composition suitably comprises 51-70 mM buffer system excluding histidine. The biopharmaceutical composition suitably comprises 51-60 mM buffer system excluding histidine. The biopharmaceutical composition suitably comprises 55 mM buffer system excluding histidine.

The biopharmaceutical composition suitably comprises 1-70 mM histidine buffer system. The biopharmaceutical composition suitably comprises 10-30 mM histidine buffer system. The biopharmaceutical composition suitably comprises 20 mM histidine buffer system. The biopharmaceutical composition suitably comprises 1-9 mM histidine buffer system. The biopharmaceutical composition suitably comprises 4 mM histidine buffer system.

The biopharmaceutical composition suitably comprises 1-70 mM succinate buffer system. The biopharmaceutical composition suitably comprises 10-30 mM succinate buffer system. The biopharmaceutical composition suitably comprises 1-9 mM succinate buffer system. The biopharmaceutical composition suitably comprises 8 mM succinate buffer system. The biopharmaceutical composition suitably comprises less than 5 mM succinate buffer system. The biopharmaceutical composition suitably comprises 0.5-4.5 mM succinate buffer system. The biopharmaceutical composition suitably comprises 4 mM succinate buffer system. The biopharmaceutical composition suitably comprises 51-70 mM succinate buffer system. The biopharmaceutical composition suitably comprises 51-60 mM succinate buffer system. The biopharmaceutical composition suitably comprises 55 mM succinate buffer system.

The biopharmaceutical composition suitably comprises 1-70 mM acetate buffer system. The biopharmaceutical composition suitably comprises 10-30 mM acetate buffer system. The biopharmaceutical composition suitably comprises 1-9 mM acetate buffer system. The biopharmaceutical composition suitably comprises 8 mM acetate buffer system. The biopharmaceutical composition suitably comprises less than 5 mM acetate buffer system. The biopharmaceutical composition suitably comprises 0.5-4.5 mM acetate buffer system. The biopharmaceutical composition suitably comprises 4 mM acetate buffer system. The biopharmaceutical composition suitably comprises 51-70 mM acetate buffer system. The biopharmaceutical composition suitably comprises 51-60 mM acetate buffer system. The biopharmaceutical composition suitably comprises 55 mM acetate buffer system.

The biopharmaceutical composition suitably comprises 1-70 mM adipate buffer system. The biopharmaceutical composition suitably comprises 10-30 mM adipate buffer system. The biopharmaceutical composition suitably comprises 25 mM adipate buffer system. The biopharmaceutical composition suitably comprises 1-9 mM adipate buffer system. The biopharmaceutical composition suitably comprises 8 mM adipate buffer system. The biopharmaceutical composition suitably comprises less than 5 mM adipate buffer system. The biopharmaceutical composition suitably comprises 0.5-4.5 mM adipate buffer system. The biopharmaceutical composition suitably comprises 4 mM adipate buffer system. The biopharmaceutical composition suitably comprises 51-70 mM adipate buffer system. The biopharmaceutical composition suitably comprises 51-60 mM adipate buffer system. The biopharmaceutical composition suitably comprises 55 mM adipate buffer system.

The biopharmaceutical composition suitably comprises 1-70 mM MES buffer system. The biopharmaceutical composition suitably comprises 10-30 mM MES buffer system. The biopharmaceutical composition suitably comprises 1-9 mM MES buffer system. The biopharmaceutical composition suitably comprises 8 mM MES buffer system. The biopharmaceutical composition suitably comprises less than 5 mM MES buffer system. The biopharmaceutical composition suitably comprises 0.5-4.5 mM MES buffer system. The biopharmaceutical composition suitably comprises 4 mM MES buffer system. The biopharmaceutical composition suitably comprises 51-70 mM MES buffer system. The biopharmaceutical composition suitably comprises 51-60 mM MES buffer system. The biopharmaceutical composition suitably comprises 55 mM MES buffer system.

The biopharmaceutical composition suitably comprises 1-70 mM phosphate buffer system. The biopharmaceutical composition suitably comprises 10-30 mM phosphate buffer system. The biopharmaceutical composition suitably comprises 1-9 mM phosphate buffer system. The biopharmaceutical composition suitably comprises 8 mM phosphate buffer system. The biopharmaceutical composition suitably comprises less than 5 mM phosphate buffer system. The biopharmaceutical composition suitably comprises 0.5-4.5 mM phosphate buffer system. The biopharmaceutical composition suitably comprises 4 mM phosphate buffer system. The biopharmaceutical composition suitably comprises 51-70 mM phosphate buffer system. The biopharmaceutical composition suitably comprises 51-60 mM phosphate buffer system. The biopharmaceutical composition suitably comprises 55 mM phosphate buffer system.

The biopharmaceutical composition suitably comprises 1-70 mM citrate buffer system. The biopharmaceutical composition suitably comprises 10-30 mM citrate buffer system. The biopharmaceutical composition suitably comprises 1-9 mM citrate buffer system. The biopharmaceutical composition suitably comprises 8 mM citrate buffer system. The biopharmaceutical composition suitably comprises less than 5 mM citrate buffer system. The biopharmaceutical composition suitably comprises 0.5-4.5 mM citrate buffer system. The biopharmaceutical composition suitably comprises 4 mM citrate buffer system. The biopharmaceutical composition suitably comprises 51-70 mM citrate buffer system. The biopharmaceutical composition suitably comprises 51-60 mM citrate buffer system. The biopharmaceutical composition suitably comprises 55 mM citrate buffer system.

The biopharmaceutical composition suitably comprises 1-9 mM dual-buffer system. The biopharmaceutical composition suitably comprises 0.5-4.5 mM dual-buffer system. The biopharmaceutical composition suitably comprises 4 mM dual-buffer system. The biopharmaceutical composition suitably comprises 1-9 mM dual-buffer system (with a first and second buffer system in molar ratio between 3:1 to 1:3). The biopharmaceutical composition suitably comprises 0.5-4.5 mM dual-buffer system (with a first and second buffer system in molar ratio between 3:1 to 1:3). The biopharmaceutical composition suitably comprises 4 mM dual-buffer system (with a first and second buffer system in molar ratio between 3:1 to 1:3).

The biopharmaceutical composition suitably comprises 1-70 mM phosphate-citrate buffer system. The biopharmaceutical composition suitably comprises 10-30 mM phosphate-citrate buffer system. The biopharmaceutical composition suitably comprises 1-9 mM phosphate-citrate buffer system. The biopharmaceutical composition suitably comprises 8 mM phosphate-citrate buffer system. The biopharmaceutical composition suitably comprises less than 5 mM phosphate-citrate buffer system. The biopharmaceutical composition suitably comprises 0.5-4.5 mM phosphate-citrate buffer system. The biopharmaceutical composition suitably comprises 4 mM phosphate-citrate buffer system. The biopharmaceutical composition suitably comprises 51-70 mM phosphate-citrate buffer system. The biopharmaceutical composition suitably comprises 51-60 mM phosphate-citrate buffer system. The biopharmaceutical composition suitably comprises 55 mM phosphate-citrate buffer system.

The biopharmaceutical composition suitably comprises 1-70 mM phosphate-citrate buffer system consisting of a phosphate buffer system and a citrate buffer system in a respective molar ratio of between 20:1 and 1:20. The biopharmaceutical composition suitably comprises 10-30 mM phosphate-citrate buffer system consisting of a phosphate buffer system and a citrate buffer system in a respective molar ratio of between 20:1 and 1:20. The biopharmaceutical composition suitably comprises 1-9 mM phosphate-citrate buffer system consisting of a phosphate buffer system and a citrate buffer system in a respective molar ratio of between 20:1 and 1:20. The biopharmaceutical composition suitably comprises 8 mM phosphate-citrate buffer system consisting of a phosphate buffer system and a citrate buffer system in a respective molar ratio of between 20:1 and 1:20. The biopharmaceutical composition suitably comprises less than 5 mM phosphate-citrate buffer system consisting of a phosphate buffer system and a citrate buffer system in a respective molar ratio of between 20:1 and 1:20. The biopharmaceutical composition suitably comprises 0.5-4.5 mM phosphate-citrate buffer system consisting of a phosphate buffer system and a citrate buffer system in a respective molar ratio of between 20:1 and 1:20. The biopharmaceutical composition suitably comprises 4 mM phosphate-citrate buffer system consisting of a phosphate buffer system and a citrate buffer system in a respective molar ratio of between 20:1 and 1:20. The biopharmaceutical composition suitably comprises 51-70 mM phosphate-citrate buffer system consisting of a phosphate buffer system and a citrate buffer system in a respective molar ratio of between 20:1 and 1:20. The biopharmaceutical composition suitably comprises 51-60 mM phosphate-citrate buffer system consisting of a phosphate buffer system and a citrate buffer system in a respective molar ratio of between 20:1 and 1:20. The biopharmaceutical composition suitably comprises 55 mM phosphate-citrate buffer system consisting of a phosphate buffer system and a citrate buffer system in a respective molar ratio of between 20:1 and 1:20.

The biopharmaceutical composition suitably comprises 1-70 mM phosphate-citrate buffer system consisting of a phosphate buffer system and a citrate buffer system in a respective molar ratio of between 5:1 and 1:5. The biopharmaceutical composition suitably comprises 10-30 mM phosphate-citrate buffer system consisting of a phosphate buffer system and a citrate buffer system in a respective molar ratio of between 5:1 and 1:5. The biopharmaceutical composition suitably comprises 1-9 mM phosphate-citrate buffer system consisting of a phosphate buffer system and a citrate buffer system in a respective molar ratio of between 5:1 and 1:5. The biopharmaceutical composition suitably comprises 8 mM phosphate-citrate buffer system consisting of a phosphate buffer system and a citrate buffer system in a respective molar ratio of between 5:1 and 1:5. The biopharmaceutical composition suitably comprises less than 5 mM phosphate-citrate buffer system consisting of a phosphate buffer system and a citrate buffer system in a respective molar ratio of between 5:1 and 1:5. The biopharmaceutical composition suitably comprises 0.5-4.5 mM phosphate-citrate buffer system consisting of a phosphate buffer system and a citrate buffer system in a respective molar ratio of between 5:1 and 1:5. The biopharmaceutical composition suitably comprises 4 mM phosphate-citrate buffer system consisting of a phosphate buffer system and a citrate buffer system in a respective molar ratio of between 5:1 and 1:5. The biopharmaceutical composition suitably comprises 51-70 mM phosphate-citrate buffer system consisting of a phosphate buffer system and a citrate buffer system in a respective molar ratio of between 5:1 and 1:5. The biopharmaceutical composition suitably comprises 51-60 mM phosphate-citrate buffer system consisting of a phosphate buffer system and a citrate buffer system in a respective molar ratio of between 5:1 and 1:5. The biopharmaceutical composition suitably comprises 55 mM phosphate-citrate buffer system consisting of a phosphate buffer system and a citrate buffer system in a respective molar ratio of between 5:1 and 1:5.

The biopharmaceutical composition suitably comprises 1-70 mM phosphate-citrate buffer system consisting of a phosphate buffer system and a citrate buffer system in a respective molar ratio of between 3:1 and 1:3. The biopharmaceutical composition suitably comprises 10-30 mM phosphate-citrate buffer system consisting of a phosphate buffer system and a citrate buffer system in a respective molar ratio of between 3:1 and 1:3. The biopharmaceutical composition suitably comprises 1-9 mM phosphate-citrate buffer system consisting of a phosphate buffer system and a citrate buffer system in a respective molar ratio of between 3:1 and 1:3. The biopharmaceutical composition suitably comprises 8 mM phosphate-citrate buffer system consisting of a phosphate buffer system and a citrate buffer system in a respective molar ratio of between 3:1 and 1:3. The biopharmaceutical composition suitably comprises less than 5 mM phosphate-citrate buffer system consisting of a phosphate buffer system and a citrate buffer system in a respective molar ratio of between 3:1 and 1:3. The biopharmaceutical composition suitably comprises 0.5-4.5 mM phosphate-citrate buffer system consisting of a phosphate buffer system and a citrate buffer system in a respective molar ratio of between 3:1 and 1:3. The biopharmaceutical composition suitably comprises 4 mM phosphate-citrate buffer system consisting of a phosphate buffer system and a citrate buffer system in a respective molar ratio of between 3:1 and 1:3. The biopharmaceutical composition suitably comprises 51-70 mM phosphate-citrate buffer system consisting of a phosphate buffer system and a citrate buffer system in a respective molar ratio of between 3:1 and 1:3. The biopharmaceutical composition suitably comprises 51-60 mM phosphate-citrate buffer system consisting of a phosphate buffer system and a citrate buffer system in a respective molar ratio of between 3:1 and 1:3. The biopharmaceutical composition suitably comprises 55 mM phosphate-citrate buffer system consisting of a phosphate buffer system and a citrate buffer system in a respective molar ratio of between 3:1 and 1:3.

The biopharmaceutical composition suitably comprises 1-70 mM phosphate-citrate buffer system consisting of a phosphate buffer system and a citrate buffer system in a respective molar ratio of between 2:1 and 1:2. The biopharmaceutical composition suitably comprises 10-30 mM phosphate-citrate buffer system consisting of a phosphate buffer system and a citrate buffer system in a respective molar ratio of between 2:1 and 1:2. The biopharmaceutical composition suitably comprises 1-9 mM phosphate-citrate buffer system consisting of a phosphate buffer system and a citrate buffer system in a respective molar ratio of between 2:1 and 1:2. The biopharmaceutical composition suitably comprises 8 mM phosphate-citrate buffer system consisting of a phosphate buffer system and a citrate buffer system in a respective molar ratio of between 2:1 and 1:2. The biopharmaceutical composition suitably comprises less than 5 mM phosphate-citrate buffer system consisting of a phosphate buffer system and a citrate buffer system in a respective molar ratio of between 2:1 and 1:2. The biopharmaceutical composition suitably comprises 0.5-4.5 mM phosphate-citrate buffer system consisting of a phosphate buffer system and a citrate buffer system in a respective molar ratio of between 2:1 and 1:2. The biopharmaceutical composition suitably comprises 4 mM phosphate-citrate buffer system consisting of a phosphate buffer system and a citrate buffer system in a respective molar ratio of between 2:1 and 1:2. The biopharmaceutical composition suitably comprises 51-70 mM phosphate-citrate buffer system consisting of a phosphate buffer system and a citrate buffer system in a respective molar ratio of between 2:1 and 1:2. The biopharmaceutical composition suitably comprises 51-60 mM phosphate-citrate buffer system consisting of a phosphate buffer system and a citrate buffer system in a respective molar ratio of between 2:1 and 1:2. The biopharmaceutical composition suitably comprises 55 mM phosphate-citrate buffer system consisting of a phosphate buffer system and a citrate buffer system in a respective molar ratio of between 2:1 and 1:2.

The biopharmaceutical composition suitably comprises 1-70 mM phosphate-citrate buffer system consisting of a phosphate buffer system and a citrate buffer system in a respective molar ratio of 1:1. The biopharmaceutical composition suitably comprises 10-30 mM phosphate-citrate buffer system consisting of a phosphate buffer system and a citrate buffer system in a respective molar ratio of 1:1. The biopharmaceutical composition suitably comprises 1-9 mM phosphate-citrate buffer system consisting of a phosphate buffer system and a citrate buffer system in a respective molar ratio of 1:1. The biopharmaceutical composition suitably comprises 8 mM phosphate-citrate buffer system consisting of a phosphate buffer system and a citrate buffer system in a respective molar ratio of 1:1. The biopharmaceutical composition suitably comprises less than 5 mM phosphate-citrate buffer system consisting of a phosphate buffer system and a citrate buffer system in a respective molar ratio of 1:1. The biopharmaceutical composition suitably comprises 0.5-4.5 mM phosphate-citrate buffer system consisting of a phosphate buffer system and a citrate buffer system in a respective molar ratio of 1:1. The biopharmaceutical composition suitably comprises 4 mM phosphate-citrate buffer system consisting of a phosphate buffer system and a citrate buffer system in a respective molar ratio of 1:1. The biopharmaceutical composition suitably comprises 51-70 mM phosphate-citrate buffer system consisting of a phosphate buffer system and a citrate buffer system in a respective molar ratio of 1:1. The biopharmaceutical composition suitably comprises 51-60 mM phosphate-citrate buffer system consisting of a phosphate buffer system and a citrate buffer system in a respective molar ratio of 1:1. The biopharmaceutical composition suitably comprises 55 mM phosphate-citrate buffer system consisting of a phosphate buffer system and a citrate buffer system in a respective molar ratio of 1:1. By way of example, 4 mM phosphate-citrate buffer system consisting of a phosphate buffer system and a citrate buffer system in a respective molar ratio of 1:1 means there is 2 mM phosphate buffer system and 2 mM citrate buffer system in the overall 4 mM phosphate-citrate buffer system.

The biopharmaceutical composition suitably comprises 1-70 mM phosphate-succinate buffer system. The biopharmaceutical composition suitably comprises 10-30 mM phosphate-succinate buffer system. The biopharmaceutical composition suitably comprises 1-9 mM phosphate-succinate buffer system. The biopharmaceutical composition suitably comprises 8 mM phosphate-succinate buffer system. The biopharmaceutical composition suitably comprises less than 5 mM phosphate-succinate buffer system. The biopharmaceutical composition suitably comprises 0.5-4.5 mM phosphate-succinate buffer system. The biopharmaceutical composition suitably comprises 4 mM phosphate-succinate buffer system. The biopharmaceutical composition suitably comprises 51-70 mM phosphate-succinate buffer system. The biopharmaceutical composition suitably comprises 51-60 mM phosphate-succinate buffer system. The biopharmaceutical composition suitably comprises 55 mM phosphate-succinate buffer system.

The biopharmaceutical composition suitably comprises 1-70 mM phosphate-succinate buffer system consisting of a phosphate buffer system and a succinate buffer system in a respective molar ratio of between 20:1 and 1:20. The biopharmaceutical composition suitably comprises 10-30 mM phosphate-succinate buffer system consisting of a phosphate buffer system and a succinate buffer system in a respective molar ratio of between 20:1 and 1:20. The biopharmaceutical composition suitably comprises 1-9 mM phosphate-succinate buffer system consisting of a phosphate buffer system and a succinate buffer system in a respective molar ratio of between 20:1 and 1:20. The biopharmaceutical composition suitably comprises 8 mM phosphate-succinate buffer system consisting of a phosphate buffer system and a succinate buffer system in a respective molar ratio of between 20:1 and 1:20. The biopharmaceutical composition suitably comprises less than 5 mM phosphate-succinate buffer system consisting of a phosphate buffer system and a succinate buffer system in a respective molar ratio of between 20:1 and 1:20. The biopharmaceutical composition suitably comprises 0.5-4.5 mM phosphate-succinate buffer system consisting of a phosphate buffer system and a succinate buffer system in a respective molar ratio of between 20:1 and 1:20. The biopharmaceutical composition suitably comprises 4 mM phosphate-succinate buffer system consisting of a phosphate buffer system and a succinate buffer system in a respective molar ratio of between 20:1 and 1:20. The biopharmaceutical composition suitably comprises 51-70 mM phosphate-succinate buffer system consisting of a phosphate buffer system and a succinate buffer system in a respective molar ratio of between 20:1 and 1:20. The biopharmaceutical composition suitably comprises 51-60 mM phosphate-succinate buffer system consisting of a phosphate buffer system and a succinate buffer system in a respective molar ratio of between 20:1 and 1:20. The biopharmaceutical composition suitably comprises 55 mM phosphate-succinate buffer system consisting of a phosphate buffer system and a succinate buffer system in a respective molar ratio of between 20:1 and 1:20.

The biopharmaceutical composition suitably comprises 1-70 mM phosphate-succinate buffer system consisting of a phosphate buffer system and a succinate buffer system in a respective molar ratio of between 5:1 and 1:5. The biopharmaceutical composition suitably comprises 10-30 mM phosphate-succinate buffer system consisting of a phosphate buffer system and a succinate buffer system in a respective molar ratio of between 5:1 and 1:5. The biopharmaceutical composition suitably comprises 1-9 mM phosphate-succinate buffer system consisting of a phosphate buffer system and a succinate buffer system in a respective molar ratio of between 5:1 and 1:5. The biopharmaceutical composition suitably comprises 8 mM phosphate-succinate buffer system consisting of a phosphate buffer system and a succinate buffer system in a respective molar ratio of between 5:1 and 1:5. The biopharmaceutical composition suitably comprises less than 5 mM phosphate-succinate buffer system consisting of a phosphate buffer system and a succinate buffer system in a respective molar ratio of between 5:1 and 1:5. The biopharmaceutical composition suitably comprises 0.5-4.5 mM phosphate-succinate buffer system consisting of a phosphate buffer system and a succinate buffer system in a respective molar ratio of between 5:1 and 1:5. The biopharmaceutical composition suitably comprises 4 mM phosphate-succinate buffer system consisting of a phosphate buffer system and a succinate buffer system in a respective molar ratio of between 5:1 and 1:5. The biopharmaceutical composition suitably comprises 51-70 mM phosphate-succinate buffer system consisting of a phosphate buffer system and a succinate buffer system in a respective molar ratio of between 5:1 and 1:5. The biopharmaceutical composition suitably comprises 51-60 mM phosphate-succinate buffer system consisting of a phosphate buffer system and a succinate buffer system in a respective molar ratio of between 5:1 and 1:5. The biopharmaceutical composition suitably comprises 55 mM phosphate-succinate buffer system consisting of a phosphate buffer system and a succinate buffer system in a respective molar ratio of between 5:1 and 1:5.

The biopharmaceutical composition suitably comprises 1-70 mM phosphate-succinate buffer system consisting of a phosphate buffer system and a succinate buffer system in a respective molar ratio of between 3:1 and 1:3. The biopharmaceutical composition suitably comprises 10-30 mM phosphate-succinate buffer system consisting of a phosphate buffer system and a succinate buffer system in a respective molar ratio of between 3:1 and 1:3. The biopharmaceutical composition suitably comprises 1-9 mM phosphate-succinate buffer system consisting of a phosphate buffer system and a succinate buffer system in a respective molar ratio of between 3:1 and 1:3. The biopharmaceutical composition suitably comprises 8 mM phosphate-succinate buffer system consisting of a phosphate buffer system and a succinate buffer system in a respective molar ratio of between 3:1 and 1:3. The biopharmaceutical composition suitably comprises less than 5 mM phosphate-succinate buffer system consisting of a phosphate buffer system and a succinate buffer system in a respective molar ratio of between 3:1 and 1:3. The biopharmaceutical composition suitably comprises 0.5-4.5 mM phosphate-succinate buffer system consisting of a phosphate buffer system and a succinate buffer system in a respective molar ratio of between 3:1 and 1:3. The biopharmaceutical composition suitably comprises 4 mM phosphate-succinate buffer system consisting of a phosphate buffer system and a succinate buffer system in a respective molar ratio of between 3:1 and 1:3. The biopharmaceutical composition suitably comprises 51-70 mM phosphate-succinate buffer system consisting of a phosphate buffer system and a succinate buffer system in a respective molar ratio of between 3:1 and 1:3. The biopharmaceutical composition suitably comprises 51-60 mM phosphate-succinate buffer system consisting of a phosphate buffer system and a succinate buffer system in a respective molar ratio of between 3:1 and 1:3. The biopharmaceutical composition suitably comprises 55 mM phosphate-succinate buffer system consisting of a phosphate buffer system and a succinate buffer system in a respective molar ratio of between 3:1 and 1:3.

The biopharmaceutical composition suitably comprises 1-70 mM phosphate-succinate buffer system consisting of a phosphate buffer system and a succinate buffer system in a respective molar ratio of between 2:1 and 1:2. The biopharmaceutical composition suitably comprises 10-30 mM phosphate-succinate buffer system consisting of a phosphate buffer system and a succinate buffer system in a respective molar ratio of between 2:1 and 1:2. The biopharmaceutical composition suitably comprises 1-9 mM phosphate-succinate buffer system consisting of a phosphate buffer system and a succinate buffer system in a respective molar ratio of between 2:1 and 1:2. The biopharmaceutical composition suitably comprises 8 mM phosphate-succinate buffer system consisting of a phosphate buffer system and a succinate buffer system in a respective molar ratio of between 2:1 and 1:2. The biopharmaceutical composition suitably comprises less than 5 mM phosphate-succinate buffer system consisting of a phosphate buffer system and a succinate buffer system in a respective molar ratio of between 2:1 and 1:2. The biopharmaceutical composition suitably comprises 0.5-4.5 mM phosphate-succinate buffer system consisting of a phosphate buffer system and a succinate buffer system in a respective molar ratio of between 2:1 and 1:2. The biopharmaceutical composition suitably comprises 4 mM phosphate-succinate buffer system consisting of a phosphate buffer system and a succinate buffer system in a respective molar ratio of between 2:1 and 1:2. The biopharmaceutical composition suitably comprises 51-70 mM phosphate-succinate buffer system consisting of a phosphate buffer system and a succinate buffer system in a respective molar ratio of between 2:1 and 1:2. The biopharmaceutical composition suitably comprises 51-60 mM phosphate-succinate buffer system consisting of a phosphate buffer system and a succinate buffer system in a respective molar ratio of between 2:1 and 1:2. The biopharmaceutical composition suitably comprises 55 mM phosphate-succinate buffer system consisting of a phosphate buffer system and a succinate buffer system in a respective molar ratio of between 2:1 and 1:2.

The biopharmaceutical composition suitably comprises 1-70 mM phosphate-succinate buffer system consisting of a phosphate buffer system and a succinate buffer system in a respective molar ratio of 1:1. The biopharmaceutical composition suitably comprises 10-30 mM phosphate-succinate buffer system consisting of a phosphate buffer system and a succinate buffer system in a respective molar ratio of 1:1. The biopharmaceutical composition suitably comprises 1-9 mM phosphate-succinate buffer system consisting of a phosphate buffer system and a succinate buffer system in a respective molar ratio of 1:1. The biopharmaceutical composition suitably comprises 8 mM phosphate-succinate buffer system consisting of a phosphate buffer system and a succinate buffer system in a respective molar ratio of 1:1. The biopharmaceutical composition suitably comprises less than 5 mM phosphate-succinate buffer system consisting of a phosphate buffer system and a succinate buffer system in a respective molar ratio of 1:1. The biopharmaceutical composition suitably comprises 0.5-4.5 mM phosphate-succinate buffer system consisting of a phosphate buffer system and a succinate buffer system in a respective molar ratio of 1:1. The biopharmaceutical composition suitably comprises 4 mM phosphate-succinate buffer system consisting of a phosphate buffer system and a succinate buffer system in a respective molar ratio of 1:1. The biopharmaceutical composition suitably comprises 51-70 mM phosphate-succinate buffer system consisting of a phosphate buffer system and a succinate buffer system in a respective molar ratio of 1:1. The biopharmaceutical composition suitably comprises 51-60 mM phosphate-succinate buffer system consisting of a phosphate buffer system and a succinate buffer system in a respective molar ratio of 1:1. The biopharmaceutical composition suitably comprises 55 mM phosphate-succinate buffer system consisting of a phosphate buffer system and a succinate buffer system in a respective molar ratio of 1:1. By way of example, 4 mM phosphate-succinate buffer system consisting of a phosphate buffer system and a succinate buffer system in a respective molar ratio of 1:1 means there is 2 mM phosphate buffer system and 2 mM succinate buffer system in the overall 4 mM phosphate-succinate buffer system.

The biopharmaceutical composition suitably comprises 1-70 mM phosphate-acetate buffer system. The biopharmaceutical composition suitably comprises 10-30 mM phosphate-acetate buffer system. The biopharmaceutical composition suitably comprises 1-9 mM phosphate-acetate buffer system. The biopharmaceutical composition suitably comprises 8 mM phosphate-acetate buffer system. The biopharmaceutical composition suitably comprises less than 5 mM phosphate-acetate buffer system. The biopharmaceutical composition suitably comprises 0.5-4.5 mM phosphate-acetate buffer system. The biopharmaceutical composition suitably comprises 4 mM phosphate-acetate buffer system. The biopharmaceutical composition suitably comprises 51-70 mM phosphate-acetate buffer system. The biopharmaceutical composition suitably comprises 51-60 mM phosphate-acetate buffer system. The biopharmaceutical composition suitably comprises 55 mM phosphate-acetate buffer system.

The biopharmaceutical composition suitably comprises 1-70 mM phosphate-acetate buffer system consisting of a phosphate buffer system and a acetate buffer system in a respective molar ratio of between 20:1 and 1:20. The biopharmaceutical composition suitably comprises 10-30 mM phosphate-acetate buffer system consisting of a phosphate buffer system and a acetate buffer system in a respective molar ratio of between 20:1 and 1:20. The biopharmaceutical composition suitably comprises 1-9 mM phosphate-acetate buffer system consisting of a phosphate buffer system and a acetate buffer system in a respective molar ratio of between 20:1 and 1:20. The biopharmaceutical composition suitably comprises 8 mM phosphate-acetate buffer system consisting of a phosphate buffer system and a acetate buffer system in a respective molar ratio of between 20:1 and 1:20. The biopharmaceutical composition suitably comprises less than 5 mM phosphate-acetate buffer system consisting of a phosphate buffer system and a acetate buffer system in a respective molar ratio of between 20:1 and 1:20. The biopharmaceutical composition suitably comprises 0.5-4.5 mM phosphate-acetate buffer system consisting of a phosphate buffer system and a acetate buffer system in a respective molar ratio of between 20:1 and 1:20. The biopharmaceutical composition suitably comprises 4 mM phosphate-acetate buffer system consisting of a phosphate buffer system and a acetate buffer system in a respective molar ratio of between 20:1 and 1:20. The biopharmaceutical composition suitably comprises 51-70 mM phosphate-acetate buffer system consisting of a phosphate buffer system and a acetate buffer system in a respective molar ratio of between 20:1 and 1:20. The biopharmaceutical composition suitably comprises 51-60 mM phosphate-acetate buffer system consisting of a phosphate buffer system and a acetate buffer system in a respective molar ratio of between 20:1 and 1:20. The biopharmaceutical composition suitably comprises 55 mM phosphate-acetate buffer system consisting of a phosphate buffer system and a acetate buffer system in a respective molar ratio of between 20:1 and 1:20.

The biopharmaceutical composition suitably comprises 1-70 mM phosphate-acetate buffer system consisting of a phosphate buffer system and a acetate buffer system in a respective molar ratio of between 5:1 and 1:5. The biopharmaceutical composition suitably comprises 10-30 mM phosphate-acetate buffer system consisting of a phosphate buffer system and a acetate buffer system in a respective molar ratio of between 5:1 and 1:5. The biopharmaceutical composition suitably comprises 1-9 mM phosphate-acetate buffer system consisting of a phosphate buffer system and a acetate buffer system in a respective molar ratio of between 5:1 and 1:5. The biopharmaceutical composition suitably comprises 8 mM phosphate-acetate buffer system consisting of a phosphate buffer system and a acetate buffer system in a respective molar ratio of between 5:1 and 1:5. The biopharmaceutical composition suitably comprises less than 5 mM phosphate-acetate buffer system consisting of a phosphate buffer system and a acetate buffer system in a respective molar ratio of between 5:1 and 1:5. The biopharmaceutical composition suitably comprises 0.5-4.5 mM phosphate-acetate buffer system consisting of a phosphate buffer system and a acetate buffer system in a respective molar ratio of between 5:1 and 1:5. The biopharmaceutical composition suitably comprises 4 mM phosphate-acetate buffer system consisting of a phosphate buffer system and a acetate buffer system in a respective molar ratio of between 5:1 and 1:5. The biopharmaceutical composition suitably comprises 51-70 mM phosphate-acetate buffer system consisting of a phosphate buffer system and a acetate buffer system in a respective molar ratio of between 5:1 and 1:5. The biopharmaceutical composition suitably comprises 51-60 mM phosphate-acetate buffer system consisting of a phosphate buffer system and a acetate buffer system in a respective molar ratio of between 5:1 and 1:5. The biopharmaceutical composition suitably comprises 55 mM phosphate-acetate buffer system consisting of a phosphate buffer system and a acetate buffer system in a respective molar ratio of between 5:1 and 1:5.

The biopharmaceutical composition suitably comprises 1-70 mM phosphate-acetate buffer system consisting of a phosphate buffer system and a acetate buffer system in a respective molar ratio of between 3:1 and 1:3. The biopharmaceutical composition suitably comprises 10-30 mM phosphate-acetate buffer system consisting of a phosphate buffer system and a acetate buffer system in a respective molar ratio of between 3:1 and 1:3. The biopharmaceutical composition suitably comprises 1-9 mM phosphate-acetate buffer system consisting of a phosphate buffer system and a acetate buffer system in a respective molar ratio of between 3:1 and 1:3. The biopharmaceutical composition suitably comprises 8 mM phosphate-acetate buffer system consisting of a phosphate buffer system and a acetate buffer system in a respective molar ratio of between 3:1 and 1:3. The biopharmaceutical composition suitably comprises less than 5 mM phosphate-acetate buffer system consisting of a phosphate buffer system and a acetate buffer system in a respective molar ratio of between 3:1 and 1:3. The biopharmaceutical composition suitably comprises 0.5-4.5 mM phosphate-acetate buffer system consisting of a phosphate buffer system and a acetate buffer system in a respective molar ratio of between 3:1 and 1:3. The biopharmaceutical composition suitably comprises 4 mM phosphate-acetate buffer system consisting of a phosphate buffer system and a acetate buffer system in a respective molar ratio of between 3:1 and 1:3. The biopharmaceutical composition suitably comprises 51-70 mM phosphate-acetate buffer system consisting of a phosphate buffer system and a acetate buffer system in a respective molar ratio of between 3:1 and 1:3. The biopharmaceutical composition suitably comprises 51-60 mM phosphate-acetate buffer system consisting of a phosphate buffer system and a acetate buffer system in a respective molar ratio of between 3:1 and 1:3. The biopharmaceutical composition suitably comprises 55 mM phosphate-acetate buffer system consisting of a phosphate buffer system and a acetate buffer system in a respective molar ratio of between 3:1 and 1:3.

The biopharmaceutical composition suitably comprises 1-70 mM phosphate-acetate buffer system consisting of a phosphate buffer system and a acetate buffer system in a respective molar ratio of between 2:1 and 1:2. The biopharmaceutical composition suitably comprises 10-30 mM phosphate-acetate buffer system consisting of a phosphate buffer system and a acetate buffer system in a respective molar ratio of between 2:1 and 1:2. The biopharmaceutical composition suitably comprises 1-9 mM phosphate-acetate buffer system consisting of a phosphate buffer system and a acetate buffer system in a respective molar ratio of between 2:1 and 1:2. The biopharmaceutical composition suitably comprises 8 mM phosphate-acetate buffer system consisting of a phosphate buffer system and a acetate buffer system in a respective molar ratio of between 2:1 and 1:2. The biopharmaceutical composition suitably comprises less than 5 mM phosphate-acetate buffer system consisting of a phosphate buffer system and a acetate buffer system in a respective molar ratio of between 2:1 and 1:2. The biopharmaceutical composition suitably comprises 0.5-4.5 mM phosphate-acetate buffer system consisting of a phosphate buffer system and a acetate buffer system in a respective molar ratio of between 2:1 and 1:2. The biopharmaceutical composition suitably comprises 4 mM phosphate-acetate buffer system consisting of a phosphate buffer system and a acetate buffer system in a respective molar ratio of between 2:1 and 1:2. The biopharmaceutical composition suitably comprises 51-70 mM phosphate-acetate buffer system consisting of a phosphate buffer system and a acetate buffer system in a respective molar ratio of between 2:1 and 1:2. The biopharmaceutical composition suitably comprises 51-60 mM phosphate-acetate buffer system consisting of a phosphate buffer system and a acetate buffer system in a respective molar ratio of between 2:1 and 1:2. The biopharmaceutical composition suitably comprises 55 mM phosphate-acetate buffer system consisting of a phosphate buffer system and a acetate buffer system in a respective molar ratio of between 2:1 and 1:2.

The biopharmaceutical composition suitably comprises 1-70 mM phosphate-acetate buffer system consisting of a phosphate buffer system and a acetate buffer system in a respective molar ratio of 1:1. The biopharmaceutical composition suitably comprises 10-30 mM phosphate-acetate buffer system consisting of a phosphate buffer system and a acetate buffer system in a respective molar ratio of 1:1. The biopharmaceutical composition suitably comprises 1-9 mM phosphate-acetate buffer system consisting of a phosphate buffer system and a acetate buffer system in a respective molar ratio of 1:1. The biopharmaceutical composition suitably comprises 8 mM phosphate-acetate buffer system consisting of a phosphate buffer system and a acetate buffer system in a respective molar ratio of 1:1. The biopharmaceutical composition suitably comprises less than 5 mM phosphate-acetate buffer system consisting of a phosphate buffer system and a acetate buffer system in a respective molar ratio of 1:1. The biopharmaceutical composition suitably comprises 0.5-4.5 mM phosphate-acetate buffer system consisting of a phosphate buffer system and a acetate buffer system in a respective molar ratio of 1:1. The biopharmaceutical composition suitably comprises 4 mM phosphate-acetate buffer system consisting of a phosphate buffer system and a acetate buffer system in a respective molar ratio of 1:1. The biopharmaceutical composition suitably comprises 51-70 mM phosphate-acetate buffer system consisting of a phosphate buffer system and a acetate buffer system in a respective molar ratio of 1:1. The biopharmaceutical composition suitably comprises 51-60 mM phosphate-acetate buffer system consisting of a phosphate buffer system and a acetate buffer system in a respective molar ratio of 1:1. The biopharmaceutical composition suitably comprises 55 mM phosphate-acetate buffer system consisting of a phosphate buffer system and a acetate buffer system in a respective molar ratio of 1:1. By way of example, 4 mM phosphate-acetate buffer system consisting of a phosphate buffer system and a acetate buffer system in a respective molar ratio of 1:1 means there is 2 mM phosphate buffer system and 2 mM acetate buffer system in the overall 4 mM phosphate-acetate buffer system.

Preferably, the biopharmaceutical composition is characterised by an absence of a histidine buffer system.

In an embodiment, the biopharmaceutical composition is characterised by an absence of a buffer system. The biopharmaceutical composition may be characterised by an absence of any, some, or all buffer systems defined herein.

The aforementioned characteristics of a biopharmaceutical composition, in particular in relation to buffers, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, 11-12400, and J1-J1158.

## *Composition pH*

The biopharmaceutical composition is preferably characterised by a pH, especially where said biopharmaceutical composition is an aqueous biopharmaceutical composition (i.e. comprising a diluent, which is water). The pH is suitably defined as the pH at SATP. The pH is preferably the pH of the composition as a whole. However, the pH may be defined by reference to the composition without its biopharmaceutical active. Where a buffer system is present, the pH may be dependent on the balance of buffering species in any particular buffer system - e.g. the relative concentrations of conjugate acid(s) and conjugate base(s) within any given buffer system. Likewise, the relative concentrations of conjugate acid(s) and conjugate base(s) within any given buffer system may be dependent on pH, as indicated by the aforementioned Henderson-Hasselbalch equation which illustrates an interdependency between pH, relative concentrations of buffering species, and $pK_a$ (of relevant conjugate acid(s)). The pH may be judiciously adjusted, as well known in the art (e.g. by the addition of strong acid or strong base).

The biopharmaceutical composition suitably has a pH of pH 4-8. The biopharmaceutical composition suitably has a pH of pH 4.5-7. The biopharmaceutical composition suitably has a pH of pH 4.5-6.5. The biopharmaceutical composition suitably has a pH of pH 5-6.5.

The biopharmaceutical composition suitably has a pH of pH 4-5.1.

The biopharmaceutical composition suitably has a pH of pH 6.3-7.

The biopharmaceutical composition suitably has a pH of pH 5.2-6.2.

The biopharmaceutical composition suitably has a pH of pH 4.4-4.8. The biopharmaceutical composition suitably has a pH of pH 4.5-4.7. The biopharmaceutical composition suitably has a pH of pH 4.6.

The biopharmaceutical composition suitably has a pH of pH 4.5-5.5. The biopharmaceutical composition suitably has a pH of pH 4.8-5.2. The biopharmaceutical composition suitably has a pH of pH 4.9-5.1. The biopharmaceutical composition suitably has a pH of pH 5.0. The biopharmaceutical composition suitably has a pH of pH 5.1.

The biopharmaceutical composition suitably has a pH of pH 5.6-6.2. The biopharmaceutical composition suitably has a pH of pH 5.6-6.0. The biopharmaceutical composition suitably has a pH of pH 5.6. The biopharmaceutical composition suitably has a pH of pH 5.7. The biopharmaceutical composition suitably has a pH of pH 5.7-5.9. The biopharmaceutical composition suitably has a pH of pH 5.8.

The biopharmaceutical composition suitably has a pH of pH 5.6-6.4. The biopharmaceutical composition suitably has a pH of pH 5.8-6.2. The biopharmaceutical composition suitably has a pH of pH 5.9-6.1. The biopharmaceutical composition suitably has a pH of pH 6.0.

The biopharmaceutical composition suitably has a pH of pH 5.9-6.6. The biopharmaceutical composition suitably has a pH of pH 6.0-6.5. The biopharmaceutical composition suitably has a pH of pH 6.1. The biopharmaceutical composition suitably has a pH of pH 6.2-6.4. The biopharmaceutical composition suitably has a pH of pH 6.3. The biopharmaceutical composition suitably has a pH of pH 6.2.

The biopharmaceutical composition suitably has a pH of pH 5.0-6.0. The biopharmaceutical composition suitably has a pH of pH 5.6-6.0. The biopharmaceutical composition suitably has a pH of pH 5.7-5.9. The biopharmaceutical composition suitably has a pH of pH 5.8.

The biopharmaceutical composition suitably has a pH of pH 6.3-6.7. The biopharmaceutical composition suitably has a pH of pH 6.5.

The aforementioned characteristics of a biopharmaceutical composition, in particular in relation to buffer systems, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

## *Sugar Component*

The biopharmaceutical composition suitably comprises a sugar component. The biopharmaceutical composition suitably comprises a sugar component that stabilizes the biopharmaceutical active, especially in an aqueous solution. The biopharmaceutical composition suitably comprises a sugar component that facilitates maintainance of the structural integrity of the biopharmaceutical active, particularly during freezing and/or lyophilization and/or storage (especially when exposed to stress). The biopharmaceutical composition suitably comprises a sugar component that acts as a lyoprotectant.

Suitably, the sugar component is a single sugar component. Suitably, the sugar component is a single sugar component consisting of a single compound.

Suitably, the sugar component is a multi-sugar component. Suitably, the sugar component is a multi-sugar component consisting of a plurality of compounds.

Suitably, the sugar component is a non-reducing sugar component. Suitably, the sugar component is a non-reducing sugar component consisting of only non-reducing compound(s).

Suitably, the sugar component is a sugar component consisting of one or more sugar(s) and/or one or more sugar alcohol(s). Suitably, the sugar component is a sugar component consisting of one or more sugar(s). Suitably, the sugar component is a sugar component consisting of one or more sugar alcohol(s). Suitably, the sugar component is a sugar component that is a single sugar or a single sugar alcohol. Suitably, the sugar component is a sugar component that is a single sugar. Suitably, the sugar component is a sugar component that is a single sugar alcohol.

Suitably, the sugar component is a sugar component consisting of one or more short (e.g. 1-3C) polyols or glycols, one or more sugar alcohols, one or more monosaccharides, one or more disaccharidess, one or more polysaccharides, one or more complex carbohydrates, or any combination thereof.

Suitably, the sugar component is a sugar component comprising or consisting of one or more short (e.g. 1-3C) polyols or glycols.

Suitably, the sugar component is a sugar component comprising or consisting of one or more sugar alcohols.

Suitably, the sugar component is a sugar component comprising or consisting of one or more monosaccharides.

Suitably, the sugar component is a sugar component comprising or consisting of one or more disaccharides.

Suitably, the sugar component is a sugar component comprising or consisting of one or more polysaccharides.

Suitably, the sugar component is a sugar component comprising or consisting of one or more complex carbohydrates.

Suitably, the sugar component is a sugar component excluding trehalose.

Suitably, the sugar component is a sugar component excluding sucrose.

Suitably, the sugar component is a sugar component excluding trehalose and sucrose.

Suitably, the sugar component is a sugar component excluding disaccharides.

Suitably, the sugar component is a sugar component selected from one or more sugar alcohols, one or more monosaccharides, one or more trisaccharides, or any combination thereof.

Suitably, the sugar component is one or more sugar alcohols. Suitably, the sugar component is a sugar alcohol.

Suitably, the sugar component is a sugar component comprising or consisting of one or more short (e.g. 1-3C) polyols or glycols selected from the group consisting of glycerol, propylene glycol, 2-methyl-2,4-pentanediol, and any combination thereof. Suitably, the sugar component is glycerol and/or propylene glycol. Suitably, the sugar component is glycerol. Suitably, the sugar component is propylene glycol.

Suitably, the sugar component is a sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. Suitably, the sugar component is a sugar component comprising or consisting of one or more sugar alcohols selected from mannitol, sorbitol, or a combination thereof. Suitably, the sugar component is a sugar component comprising or consisting of mannitol and/or sorbitol. Suitably, the sugar component is mannitol and/or sorbitol. Suitably, the sugar component is mannitol. Suitably, the sugar component is sorbitol.

Suitably, the sugar component is a sugar component comprising or consisting of one or more monosaccharides selected from the group consisting of glucose, mannose, galactose, ribose, xylose, and any combination thereof. Suitably, the sugar component is glucose and/or mannose. Suitably, the sugar component is glucose. Suitably, the sugar component is mannose.

Suitably, the sugar component is a sugar component comprising or consisting of one or more disaccharides selected from the group consisting of trehalose, sucrose, maltose, lactose, and any combination thereof. Suitably, the sugar component is trehalose. Suitably, the sugar component is sucrose. Suitably, the sugar component is maltose and/or lactose. Suitably, the sugar component is maltose. Suitably, the sugar component is lactose.

Suitably, the sugar component is a sugar component comprising or consisting of one or more polysaccharides and/or one or more complex carbohydrates selected from the group consisting of cyclodextrins, maltodextrins, raffinose, dextran, and any combination thereof.

Suitably, the sugar component is a cyclodextrin or a derivative thereof.

Suitably, the sugar component is raffinose.

Suitably, the sugar component is a sugar component comprising one or more reducing sugars selected from the group consisting of fructose, mannose, maltose, lactose, arabinose, xylose, ribose, rhamnose, galactose, glucose, and any combination thereof.

The biopharmaceutical composition suitably comprises 30-400 mM sugar component. The biopharmaceutical composition suitably comprises 50-350 mM sugar component. The biopharmaceutical composition suitably comprises 100-300 mM sugar component. The biopharmaceutical composition suitably comprises 175-300 mM sugar component. The biopharmaceutical composition suitably comprises 200-300 mM sugar component.

The biopharmaceutical composition suitably comprises 150-250 mM sugar component. The biopharmaceutical composition suitably comprises 200-250 mM sugar component. The biopharmaceutical composition suitably comprises 200 mM sugar component. The biopharmaceutical composition suitably comprises 230 mM sugar component.

The biopharmaceutical composition suitably comprises 250-300 mM sugar component. The biopharmaceutical composition suitably comprises 270 mM sugar component.

The biopharmaceutical composition suitably comprises 50-200 mM sugar component.

The biopharmaceutical composition suitably comprises less than 130 mM sugar component. The biopharmaceutical composition suitably comprises 50-129 mM sugar component. The biopharmaceutical composition suitably comprises 70-129 mM sugar component. The biopharmaceutical composition suitably comprises 100-150 mM sugar component. The biopharmaceutical composition suitably comprises 120-130 mM sugar component. The biopharmaceutical composition suitably comprises 50-350 mM sugar component wherein no single compound thereof exceeds 129 mM. The biopharmaceutical composition suitably comprises 175-300 mM sugar component wherein no single compound thereof exceeds 129 mM. The biopharmaceutical composition suitably comprises 200-300 mM sugar component wherein no single compound thereof exceeds 129 mM. The biopharmaceutical composition suitably comprises 80 mM sugar component. The biopharmaceutical composition suitably comprises 125 mM sugar component. The biopharmaceutical composition suitably comprises less than 130 mM single sugar component. The biopharmaceutical composition suitably comprises 50-129 mM single sugar component. The biopharmaceutical composition suitably comprises 70-129 mM single sugar component. The biopharmaceutical composition suitably comprises 100-150 mM single sugar component. The biopharmaceutical composition suitably comprises 120-130 mM single sugar component. The biopharmaceutical composition suitably comprises 80 mM single sugar component. The biopharmaceutical composition suitably comprises 125 mM single sugar component.

The biopharmaceutical composition suitably comprises less than 175 mM sugar component. The biopharmaceutical composition suitably comprises 50-174 mM sugar component. The biopharmaceutical composition suitably comprises 70-174 mM sugar component. The biopharmaceutical composition suitably comprises 130-174 mM sugar component. The biopharmaceutical composition suitably comprises 50-350 mM sugar component wherein no single compound thereof exceeds 174 mM. The biopharmaceutical composition suitably comprises 175-300 mM sugar component wherein no single compound thereof exceeds 174 mM. The biopharmaceutical composition suitably comprises 200-300 mM sugar component wherein no single compound thereof exceeds 174 mM. The biopharmaceutical composition suitably comprises 150 mM sugar component. The biopharmaceutical composition suitably comprises less than 175 mM single sugar component. The biopharmaceutical composition suitably comprises 50-174 mM single sugar component. The biopharmaceutical composition suitably comprises 70-174 mM single sugar component. The biopharmaceutical composition suitably comprises 130-174 mM single sugar component. The biopharmaceutical composition suitably comprises 150 mM single sugar component.

The biopharmaceutical composition suitably comprises less than 200 mM sugar component. The biopharmaceutical composition suitably comprises 50-199 mM sugar component. The biopharmaceutical composition suitably comprises 70-199 mM sugar component. The biopharmaceutical composition suitably comprises 175-199 mM sugar component. The biopharmaceutical composition suitably comprises 50-350 mM sugar component wherein no single compound thereof exceeds 199 mM. The biopharmaceutical composition suitably comprises 175-300 mM sugar component wherein no single compound thereof exceeds 199 mM. The biopharmaceutical composition suitably comprises 200-300 mM sugar component wherein no single compound thereof exceeds 199 mM. The biopharmaceutical composition suitably comprises 180 mM sugar component. The biopharmaceutical composition suitably comprises less than 200 mM single sugar component. The biopharmaceutical composition suitably comprises 50-199 mM single sugar component. The biopharmaceutical composition suitably comprises 70-199 mM single sugar component. The biopharmaceutical composition suitably comprises 175-199 mM single sugar component. The biopharmaceutical composition suitably comprises 180 mM single sugar component.

The biopharmaceutical composition suitably comprises 50-350 mM sugar component excluding trehalose and sucrose. The biopharmaceutical composition suitably comprises 50-129 mM sugar component excluding trehalose and sucrose. The biopharmaceutical composition suitably comprises 80 mM sugar component excluding trehalose and sucrose. The biopharmaceutical composition suitably comprises 125 mM sugar component excluding trehalose and sucrose. The biopharmaceutical composition suitably comprises 130-174 mM sugar component excluding trehalose and sucrose. The biopharmaceutical composition suitably comprises 150 mM sugar component excluding trehalose and sucrose. The biopharmaceutical composition suitably comprises 175-199 mM sugar component excluding trehalose and sucrose. The biopharmaceutical composition suitably comprises 180 mM sugar component excluding trehalose and sucrose. The biopharmaceutical composition suitably comprises 200-250 mM sugar component excluding trehalose and sucrose. The biopharmaceutical composition suitably comprises 200 mM sugar component excluding trehalose and sucrose. The biopharmaceutical composition suitably comprises 230 mM sugar component excluding trehalose and sucrose.

The biopharmaceutical composition suitably comprises 50-350 mM one or more sugar alcohols. The biopharmaceutical composition suitably comprises 50-129 mM one or more sugar alcohols. The biopharmaceutical composition suitably comprises 100-150 mM one or more sugar alcohols. The biopharmaceutical composition suitably comprises 120-130 mM one or more sugar alcohols. The biopharmaceutical composition suitably comprises 80 mM one or more sugar alcohols. The biopharmaceutical composition suitably comprises 125 mM one or more sugar alcohols. The biopharmaceutical composition suitably comprises 130-174 mM one or more sugar alcohols. The biopharmaceutical composition suitably comprises 150 mM one or more sugar alcohols. The biopharmaceutical composition suitably comprises 175-199 mM one or more sugar alcohols. The biopharmaceutical composition suitably comprises 180 mM one or more sugar alcohols. The biopharmaceutical composition suitably comprises 200-250 mM one or more sugar alcohols. The biopharmaceutical composition suitably comprises 200 mM one or more sugar alcohols. The biopharmaceutical composition suitably comprises 230 mM one or more sugar alcohols.

The biopharmaceutical composition suitably comprises 50-350 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 50-129 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 80 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 125 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 130-174 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 150 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 175-199 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 180 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 200-250 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 200 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof. The biopharmaceutical composition suitably comprises 230 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof.

The biopharmaceutical composition suitably comprises 50-350 mM mannitol or sorbitol. The biopharmaceutical composition suitably comprises 50-129 mM mannitol or sorbitol. The biopharmaceutical composition suitably comprises 80 mM mannitol or sorbitol. The biopharmaceutical composition suitably comprises 125 mM mannitol or sorbitol. The biopharmaceutical composition suitably comprises 130-174 mM mannitol or sorbitol. The biopharmaceutical composition suitably comprises 150 mM mannitol or sorbitol. The biopharmaceutical composition suitably comprises 175-199 mM mannitol or sorbitol. The biopharmaceutical composition suitably comprises 180 mM mannitol or sorbitol. The biopharmaceutical composition suitably comprises 200 mM mannitol or sorbitol. The biopharmaceutical composition suitably comprises 200-250 mM mannitol or sorbitol. The biopharmaceutical composition suitably comprises 200 mM mannitol or sorbitol. The biopharmaceutical composition suitably comprises 230 mM mannitol or sorbitol.

The biopharmaceutical composition suitably comprises 50-350 mM mannitol and sorbitol (combined concentration), wherein the molar ratio of mannitol to sorbitol is between 1:20 and 20:1. The biopharmaceutical composition suitably comprises 50-129 mM mannitol and sorbitol (combined concentration), wherein the molar ratio of mannitol to sorbitol is between 1:20 and 20:1. The biopharmaceutical composition suitably comprises 80 mM mannitol and sorbitol (combined concentration), wherein the molar ratio of mannitol to sorbitol is between 1:20 and 20:1. The biopharmaceutical composition suitably comprises 125 mM mannitol and sorbitol (combined concentration), wherein the molar ratio of mannitol to sorbitol is between 1:20 and 20:1. The biopharmaceutical composition suitably comprises 130-174 mM mannitol and sorbitol (combined concentration), wherein the molar ratio of mannitol to sorbitol is between 1:20 and 20:1. The biopharmaceutical composition suitably comprises 150 mM mannitol and sorbitol (combined concentration), wherein the molar ratio of mannitol to sorbitol is between 1:20 and 20:1. The biopharmaceutical composition suitably comprises 175-199 mM mannitol and sorbitol (combined concentration), wherein the molar ratio of mannitol to sorbitol is between 1:20 and 20:1. The biopharmaceutical composition suitably comprises 180 mM mannitol and sorbitol (combined concentration), wherein the molar ratio of mannitol to sorbitol is between 1:20 and 20:1. 200-250 mM mannitol and sorbitol (combined concentration), wherein the molar ratio of mannitol to sorbitol is between 1:20 and 20:1. The biopharmaceutical composition suitably comprises 200 mM mannitol and sorbitol (combined concentration), wherein the molar ratio of mannitol to sorbitol is between 1:20 and 20:1. The biopharmaceutical composition suitably comprises 230 mM mannitol and sorbitol (combined concentration), wherein the molar ratio of mannitol to sorbitol is between 1:20 and 20:1.

The biopharmaceutical composition suitably comprises 50-350 mM mannitol and sorbitol (combined concentration), wherein the molar ratio of mannitol to sorbitol is between 1:5 and 5:1. The biopharmaceutical composition suitably comprises 50-129 mM mannitol and sorbitol (combined concentration), wherein the molar ratio of mannitol to sorbitol is between 1:5 and 5:1. The biopharmaceutical composition suitably comprises 80 mM mannitol and sorbitol (combined concentration), wherein the molar ratio of mannitol to sorbitol is between 1:5 and 5:1. The biopharmaceutical composition suitably comprises 125 mM mannitol and sorbitol (combined concentration), wherein the molar ratio of mannitol to sorbitol is between 1:5 and 5:1. The biopharmaceutical composition suitably comprises 130-174 mM mannitol and sorbitol (combined concentration), wherein the molar ratio of mannitol to sorbitol is between 1:5 and 5:1. The biopharmaceutical composition suitably comprises 150 mM mannitol and sorbitol (combined concentration), wherein the molar ratio of mannitol to sorbitol is between 1:5 and 5:1. The biopharmaceutical composition suitably comprises 175-199 mM mannitol and sorbitol (combined concentration), wherein the molar ratio of mannitol to sorbitol is between 1:5 and 5:1. The biopharmaceutical composition suitably comprises 180 mM mannitol and sorbitol (combined concentration), wherein the molar ratio of mannitol to sorbitol is between 1:5 and 5:1. The biopharmaceutical composition suitably comprises 200 mM mannitol and sorbitol (combined concentration), wherein the molar ratio of mannitol to sorbitol is between 1:5 and 5:1. The biopharmaceutical composition suitably comprises 230 mM mannitol and sorbitol (combined concentration), wherein the molar ratio of mannitol to sorbitol is between 1:5 and 5:1.

The biopharmaceutical composition suitably comprises 50-350 mM mannitol. The biopharmaceutical composition suitably comprises 50-129 mM mannitol. The biopharmaceutical composition suitably comprises 80 mM mannitol. The biopharmaceutical composition suitably comprises 125 mM mannitol. The biopharmaceutical composition suitably comprises 130-174 mM mannitol. The biopharmaceutical composition suitably comprises 150 mM mannitol. The biopharmaceutical composition suitably comprises 175-199 mM mannitol. The biopharmaceutical composition suitably comprises 180 mM mannitol. The biopharmaceutical composition suitably comprises 200-250 mM mannitol. The biopharmaceutical composition suitably comprises 200 mM mannitol. The biopharmaceutical composition suitably comprises 230 mM mannitol.

The biopharmaceutical composition suitably comprises 50-350 mM sorbitol. The biopharmaceutical composition suitably comprises 40-80 mM sorbitol. The biopharmaceutical composition suitably comprises 50 mM sorbitol. The biopharmaceutical composition suitably comprises 70 mM sorbitol. The biopharmaceutical composition suitably comprises 50-129 mM sorbitol. The biopharmaceutical composition suitably comprises 100-150 mM sorbitol. The biopharmaceutical composition suitably comprises 120-130 mM sorbitol. The biopharmaceutical composition suitably comprises 80 mM sorbitol. The biopharmaceutical composition suitably comprises 125 mM sorbitol. The biopharmaceutical composition suitably comprises 130-174 mM sorbitol. The biopharmaceutical composition suitably comprises 140 mM sorbitol. The biopharmaceutical composition suitably comprises 150 mM sorbitol. The biopharmaceutical composition suitably comprises 175-199 mM sorbitol. The biopharmaceutical composition suitably comprises 180 mM sorbitol. The biopharmaceutical composition suitably comprises 200-250 mM sorbitol. The biopharmaceutical composition suitably comprises 200 mM sorbitol. The biopharmaceutical composition suitably comprises 230 mM sorbitol.

The biopharmaceutical composition suitably comprises a combination of sorbitol and sodium chloride. The biopharmaceutical composition suitably comprises a combination of 50-129 mM sorbitol and 10-100 mM sodium chloride. The biopharmaceutical composition suitably comprises a combination of 120-130 mM sorbitol and 50-70 mM sodium chloride. The biopharmaceutical composition suitably comprises a combination of 125 mM sorbitol and 60 mM sodium chloride.

The biopharmaceutical composition may suitably be characterised by an absence of sugars or sugar alcohols.

The biopharmaceutical composition may suitably be characterised by an absence of trehalose. The biopharmaceutical composition may suitably be characterised by an absence of sucrose. The biopharmaceutical composition may suitably be characterised by an absence of trehalose and sucrose. The biopharmaceutical composition may suitably be characterised by an absence of disaccharides.

The biopharmaceutical composition suitably comprises 50-350 mM trehalose. The biopharmaceutical composition suitably comprises 150-250 mM trehalose. The biopharmaceutical composition suitably comprises 200 mM trehalose. The biopharmaceutical composition suitably comprises 50-350 mM sucrose. The biopharmaceutical composition suitably comprises 150-250 mM sucrose. The biopharmaceutical composition suitably comprises 200 mM sucrose.

The biopharmaceutical composition may suitably be characterised by an absence of a sugar component. The biopharmaceutical composition may suitably be characterised by an absence of any, some, or all sugar components defined herein.

The aforementioned characteristics of a biopharmaceutical composition, in particular in relation to sugar components, amounts thereof, and/or absences thereof, may be applied to any of the embodiments defined in numbered paragraphs A1-A51 1, B1-B255, C1-C255, D1-D1152, E1-E1151, F1-F12, G1-G959, H1-H1423, I1-I2400, and J1-J1158.

***Amino Acid Component***

The biopharmaceutical composition suitably comprises an amino acid component.

Suitably, the amino acid component is an amino acid component that stabilises the biopharmaceutical active, especially in aqueous solution.

Suitably, the amino acid component is a single amino acid component. Suitably, the amino acid component is a single amino acid component consisting of a single amino acid compound.

Suitably, the amino acid component is a multi-amino acid component. Suitably, the amino acid component is a multi-amino acid component consisting of a plurality of amino acid compounds.

Suitably, the amino acid component is an amino acid component consisting of one or more a natural amino acids, one or more a peptides (short peptide, suitably at most 3 amino acids in length, preferably at most 2 amino acids in length), one or more synthetic amino acids, or any combination thereof.

Suitably, the amino acid component is an amino acid component consisting of one or more a natural amino acids. Suitably, the amino acid component is an amino acid component consisting of one or more amino acids selected from alanine, arginine, asparagine, aspartic acid, cysteine, glutamine, glutamic acid, glycine, glycylglycine, histidine, isoleucine, leucine, lysine, lysyllysine, methionine, phenylalanine, proline, serine, threonine, tryptophan, tyrosine, valine, or any combination thereof. Suitably, the amino acid component is an amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. Suitably, the amino acid component is an amino acid component consisting of one or more amino acids selected arginine, cysteine, glycine, lysine, phenylalanine, proline, or any combination thereof. Suitably, the amino acid component is an amino acid component consisting of one or more amino acids selected from arginine, glycine, glycylglycine, lysine, lysyllysine, or any combination thereof. Suitably, the amino acid component is arginine and/or glycine. Suitably, the amino acid component is arginine. Suitably, the amino acid component is glycine. Suitably, the amino acid component is lysine. Suitably, the amino acid component is phenylalanine. Suitably, the amino acid component is proline.

Suitably, the amino acid component is an amino acid component excluding methionine. Suitably, the amino acid component is an amino acid component excluding histidine. Suitably, the amino acid component is an amino acid component excluding histidine and methionine. Suitably, the amino acid component is an amino acid component excluding arginine. Suitably, the amino acid component is an amino acid component excluding glycine. Suitably, the amino acid component is an amino acid component excluding arginine and glycine. Suitably, the amino acid component is an amino acid component excluding arginine, glycine, methionine, and histidine.

The biopharmaceutical composition suitably comprises 2-350 mM amino acid component. The biopharmaceutical composition suitably comprises 20-300 mM amino acid component. The biopharmaceutical composition suitably comprises 30-250 mM amino acid component. The biopharmaceutical composition suitably comprises 100-300 mM amino acid component.

The biopharmaceutical composition suitably comprises less than 100 mM amino acid component. The biopharmaceutical composition suitably comprises 20-99 mM amino acid component. The biopharmaceutical composition suitably comprises 50-99 mM amino acid component. The biopharmaceutical composition suitably comprises 70-99 mM amino acid component. The biopharmaceutical composition suitably comprises 40 mM amino acid component. The biopharmaceutical composition suitably comprises 60 mM amino acid component. The biopharmaceutical composition suitably comprises 80 mM amino acid component. The biopharmaceutical composition suitably comprises 2-350 mM amino acid component wherein no single amino acid compound exceeds 99 mM. The biopharmaceutical composition suitably comprises less than 100 mM single amino acid component. The biopharmaceutical composition suitably comprises 20-99 mM single amino acid component. The biopharmaceutical composition suitably comprises 50-99 mM single amino acid component. The biopharmaceutical composition suitably comprises 70-99 mM single amino acid component. The biopharmaceutical composition suitably comprises 40 mM single amino acid component. The biopharmaceutical composition suitably comprises 80 mM single amino acid component.

The biopharmaceutical composition suitably comprises less than 150 mM amino acid component. The biopharmaceutical composition suitably comprises 20-149 mM amino acid component. The biopharmaceutical composition suitably comprises 50-149 mM amino acid component. The biopharmaceutical composition suitably comprises 100-149 mM amino acid component. The biopharmaceutical composition suitably comprises 110-140 mM amino acid component. The biopharmaceutical composition suitably comprises 120-130 mM amino acid component. The biopharmaceutical composition suitably comprises 120 mM amino acid component. The biopharmaceutical composition suitably comprises 125 mM amino acid component. The biopharmaceutical composition suitably comprises 2-350 mM amino acid component wherein no single amino acid compound exceeds 149 mM. The biopharmaceutical composition suitably comprises less than 150 mM single amino acid component. The biopharmaceutical composition suitably comprises 20-149 mM single amino acid component. The biopharmaceutical composition suitably comprises 50-149 mM single amino acid component. The biopharmaceutical composition suitably comprises 100-149 mM single amino acid component. The biopharmaceutical composition suitably comprises 110-140 mM single amino acid component. The biopharmaceutical composition suitably comprises 120-130 mM single amino acid component. The biopharmaceutical composition suitably comprises 120 mM single amino acid component.The biopharmaceutical composition suitably comprises 125 mM single amino acid component.

The biopharmaceutical composition suitably comprises less than 200 mM amino acid component. The biopharmaceutical composition suitably comprises 20-199 mM amino acid component. The biopharmaceutical composition suitably comprises 50-199 mM amino acid component. The biopharmaceutical composition suitably comprises 100-199 mM amino acid component. The biopharmaceutical composition suitably comprises 150-199 mM amino acid component. The biopharmaceutical composition suitably comprises 170 mM amino acid component. The biopharmaceutical composition suitably comprises 175 mM amino acid component. The biopharmaceutical composition suitably comprises 2-350 mM amino acid component wherein no single amino acid compound exceeds 199 mM. The biopharmaceutical composition suitably comprises less than 200 mM single amino acid component. The biopharmaceutical composition suitably comprises 20-199 mM single amino acid component. The biopharmaceutical composition suitably comprises 50-199 mM single amino acid component. The biopharmaceutical composition suitably comprises 100-199 mM single amino acid component. The biopharmaceutical composition suitably comprises 150-199 mM single amino acid component. The biopharmaceutical composition suitably comprises 175 mM single amino acid component.

The biopharmaceutical composition suitably comprises 2-350 mM amino acid component excluding histidine and methionine. The biopharmaceutical composition suitably comprises 20-99 mM amino acid component excluding histidine and methionine. The biopharmaceutical composition suitably comprises 40 mM amino acid component excluding histidine and methionine. The biopharmaceutical composition suitably comprises 80 mM amino acid component excluding histidine and methionine. The biopharmaceutical composition suitably comprises 100-149 mM amino acid component excluding histidine and methionine. The biopharmaceutical composition suitably comprises 125 mM amino acid component excluding histidine and methionine. The biopharmaceutical composition suitably comprises 150-199 mM amino acid component excluding histidine and methionine. The biopharmaceutical composition suitably comprises 175 mM amino acid component excluding histidine and methionine.

The biopharmaceutical composition suitably comprises 2-350 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 20-99 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 40 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 80 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 100-149 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 125 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 150-199 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof. The biopharmaceutical composition suitably comprises 175 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

The biopharmaceutical composition suitably comprises 2-350 mM arginine or glycine. The biopharmaceutical composition suitably comprises 20-99 mM arginine or glycine. The biopharmaceutical composition suitably comprises 40 mM arginine or glycine. The biopharmaceutical composition suitably comprises 80 mM arginine or glycine. The biopharmaceutical composition suitably comprises 100-149 mM arginine or glycine. The biopharmaceutical composition suitably comprises 125 mM arginine or glycine. The biopharmaceutical composition suitably comprises 150-199 mM arginine or glycine. The biopharmaceutical composition suitably comprises 175 mM arginine or glycine.

The biopharmaceutical composition suitably comprises 2-350 mM arginine and glycine (combined concentration), wherein the molar ratio of arginine to glycine is between 20:1 and 1:20. The biopharmaceutical composition suitably comprises 20-99 mM arginine and glycine (combined concentration), wherein the molar ratio of arginine to glycine is between 20:1 and 1:20. The biopharmaceutical composition suitably comprises 40 mM arginine and glycine (combined concentration), wherein the molar ratio of arginine to glycine is between 20:1 and 1:20. The biopharmaceutical composition suitably comprises 80 mM arginine and glycine (combined concentration), wherein the molar ratio of arginine to glycine is between 20:1 and 1:20. The biopharmaceutical composition suitably comprises 100-149 mM arginine and glycine (combined concentration), wherein the molar ratio of arginine to glycine is between 20:1 and 1:20. The biopharmaceutical composition suitably comprises 125 mM arginine and glycine (combined concentration), wherein the molar ratio of arginine to glycine is between 20:1 and 1:20. The biopharmaceutical composition suitably comprises 150-199 mM arginine and glycine (combined concentration), wherein the molar ratio of arginine to glycine is between 20:1 and 1:20. The biopharmaceutical composition suitably comprises 175 mM arginine and glycine (combined concentration), wherein the molar ratio of arginine to glycine is between 20:1 and 1:20.

The biopharmaceutical composition suitably comprises 2-350 mM arginine and glycine (combined concentration), wherein the molar ratio of arginine to glycine is between 5:1 and 1:5. The biopharmaceutical composition suitably comprises 20-99 mM arginine and glycine (combined concentration), wherein the molar ratio of arginine to glycine is between 5:1 and 1:5. The biopharmaceutical composition suitably comprises 40 mM arginine and glycine (combined concentration), wherein the molar ratio of arginine to glycine is between 5:1 and 1:5. The biopharmaceutical composition suitably comprises 80 mM arginine and glycine (combined concentration), wherein the molar ratio of arginine to glycine is between 5:1 and 1:5. The biopharmaceutical composition suitably comprises 100-149 mM arginine and glycine (combined concentration), wherein the molar ratio of arginine to glycine is between 5:1 and 1:5. The biopharmaceutical composition suitably comprises 125 mM arginine and glycine (combined concentration), wherein the molar ratio of arginine to glycine is between 5:1 and 1:5. The biopharmaceutical composition suitably comprises 150-199 mM arginine and glycine (combined concentration), wherein the molar ratio of arginine to glycine is between 5:1 and 1:5. The biopharmaceutical composition suitably comprises 175 mM arginine and glycine (combined concentration), wherein the molar ratio of arginine to glycine is between 5:1 and 1:5.

The biopharmaceutical composition suitably comprises 2-350 mM arginine. The biopharmaceutical composition suitably comprises 20-99 mM arginine. The biopharmaceutical composition suitably comprises 40 mM arginine. The biopharmaceutical composition suitably comprises 60 mM arginine. The biopharmaceutical composition suitably comprises 80 mM arginine. The biopharmaceutical composition suitably comprises 100-149 mM arginine. The biopharmaceutical composition suitably comprises 125 mM arginine. The biopharmaceutical composition suitably comprises 120 mM arginine. The biopharmaceutical composition suitably comprises 150-199 mM arginine. The biopharmaceutical composition suitably comprises 175 mM arginine.

The biopharmaceutical composition suitably comprises 2-350 mM glycine. The biopharmaceutical composition suitably comprises 20-99 mM glycine. The biopharmaceutical composition suitably comprises 40 mM glycine. The biopharmaceutical composition suitably comprises 80 mM glycine. The biopharmaceutical composition suitably comprises 100-149 mM glycine. The biopharmaceutical composition suitably comprises 125 mM glycine. The biopharmaceutical composition suitably comprises 150-199 mM glycine. The biopharmaceutical composition suitably comprises 170 mM glycine. The biopharmaceutical composition suitably comprises 175 mM glycine.

The biopharmaceutical composition suitably comprises 2-350 mM lysine. The biopharmaceutical composition suitably comprises 20-99 mM lysine. The biopharmaceutical composition suitably comprises 40 mM lysine. The biopharmaceutical composition suitably comprises 80 mM lysine. The biopharmaceutical composition suitably comprises 100-149 mM lysine. The biopharmaceutical composition suitably comprises 120 mM lysine. The biopharmaceutical composition suitably comprises 125 mM lysine. The biopharmaceutical composition suitably comprises 150-199 mM lysine. The biopharmaceutical composition suitably comprises 170 mM lysine. The biopharmaceutical composition suitably comprises 175 mM lysine.

The biopharmaceutical composition suitably comprises 2-350 mM proline. The biopharmaceutical composition suitably comprises 20-99 mM proline. The biopharmaceutical composition suitably comprises 40 mM proline. The biopharmaceutical composition suitably comprises 80 mM proline. The biopharmaceutical composition suitably comprises 100-149 mM proline. The biopharmaceutical composition suitably comprises 125 mM proline. The biopharmaceutical composition suitably comprises 120 mM proline. The biopharmaceutical composition suitably comprises 150-199 mM proline. The biopharmaceutical composition suitably comprises 170 mM proline. The biopharmaceutical composition suitably comprises 175 mM proline.

The biopharmaceutical composition suitably comprises 2-350 mM phenylalanine. The biopharmaceutical composition suitably comprises 5-99 mM phenylalanine. The biopharmaceutical composition suitably comprises 5-40 mM phenylalanine. The biopharmaceutical composition suitably comprises 20 mM phenylalanine.

The aforementioned characteristics of a biopharmaceutical composition, in particular in relation to amino acid components, amounts thereof, and/or absences thereof, may be applied to any of the embodiments defined in numbered paragraphs A1-A51 1, B1-B255, C1-C255, D1-D1152, E1-E1151, F1-F12, G1-G959, H1-H1423, I1-I2400, and J1-J1158.

***Surfactants***

The biopharmaceutical composition suitably comprises a surfactant. Suitably, the surfactant is a surfactant that mitigates against aggregation of the biopharmaceutical active.

Suitably, the surfactant is a single surfactant.

Suitably, the surfactant is a non-ionic surfactant.

Suitably, the surfactant is a surfactant selected from the group consisting of a fatty alcohol, a fatty alcohol ether, a fatty acid ester, a fatty acid amide, a polyoxyalkylene alkyl ether, a polyoxyethylene alkyl ether, a non-ionic block copolymer, alpha-tocopherol, and any combination thereof. Suitably, the surfactant is a surfactant selected from the group consisting of polysorbates, spans, poloxamers, kolliphors, and any combination thereof. Suitably, the surfactant is a surfactant selected from the group consisting of a sorbitan ester (e.g. Span), an ethoxylated sorbitan ester (e.g. polysorbate), and any combination thereof. Suitably, the surfactant is a surfactant selected from the group consisting of sorbitan monolaurate, sorbitan monostearate, sorbitan tristearate, and any combination thereof.

Suitably, the surfactant is a polysorbate surfactant. Suitably, the surfactant is a surfactant selected from the group consisting of polysorbate 20, polysorbate 40, polysorbate 60, polysorbate 80, and any combination thereof.

Suitably, the surfactant is a surfactant selected from the group consisting of glycerol monostearate, glycerol monolaurate, polyoxylglycerides (e.g. lauroyl polyoxylglycerides), and any combination thereof.

Suitably, the surfactant is a surfactant selected from Macrogol 15 hydroxystearate, macrogol cetostearyl ether, macrogol stearyl ether, polyoxyethylene castor oil derivatives, polyoxyethylene stearates, and any combination thereof.

Suitably, the surfactant is a surfactant that is or comprises a block alkoxylate. Suitably, the surfactant is a surfactant selected from the group consisting of poloxamers, poloxamer 182, poloxamer 188 (Pluronic F68), poloxamer 407 (Pluronic F127), Synperonics, Kolliphors, and any combination thereof. Suitably, the surfactant is a poloxamer. Suitably, the surfactant is Poloxamer 188 (e.g. Pluronic F68). Suitably, the surfactant is Poloxamer 407 (e.g. Pluronic F127).

Suitably, the surfactant is kolliphor hs-15.

Suitably, the surfactant is a surfactant selected from the group consisting of polysorbate 20, polysorbate 80, poloxamer 182, poloxamer 188, poloxamer 407, kolliphor hs-15, and any combination thereof.

Suitably, the surfactant is polysorbate 20 or polysorbate 80. Suitably, the surfactant is polysorbate 20. Suitably, the surfactant is polysorbate 80.

Suitably, the surfactant is a surfactant excluding polysorbate 80. Suitably, the surfactant is a surfactant excluding polysorbates. Suitably, the surfactant is a surfactant excluding poloxamers. Suitably, the surfactant is a surfactant excluding polysorbates and poloxamers.

Suitably, the biopharmaceutical composition comprises 0.001-5 mg/mL surfactant. Suitably, the biopharmaceutical composition comprises 0.005-3 mg/mL surfactant.

Suitably, the biopharmaceutical composition comprises 0.01-2 mg/mL surfactant. Suitably, the biopharmaceutical composition comprises 0.05-1.5 mg/mL surfactant. Suitably, the biopharmaceutical composition comprises 0.01-1.2 mg/mL surfactant. Suitably, the biopharmaceutical composition comprises 0.1-1.1 mg/mL surfactant. Suitably, the biopharmaceutical composition comprises 0.1-0.7 mg/mL surfactant.

Suitably, the biopharmaceutical composition comprises less than 0.1 mg/mL surfactant. Suitably, the biopharmaceutical composition comprises 0.001-0.05 mg/mL surfactant. Suitably, the biopharmaceutical composition comprises 0.01-0.09 mg/mL surfactant. Suitably, the biopharmaceutical composition comprises 0.01 mg/mL surfactant.

Suitably, the biopharmaceutical composition comprises less than 0.2 mg/mL surfactant. Suitably, the biopharmaceutical composition comprises 0.001-0.15 mg/mL surfactant. Suitably, the biopharmaceutical composition comprises 0.01-0.15 mg/mL surfactant. Suitably, the biopharmaceutical composition comprises 0.1 mg/mL surfactant.

Suitably, the biopharmaceutical composition comprises 0.05-0.4 mg/mL surfactant. Suitably, the biopharmaceutical composition comprises 0.1-0.3 mg/mL surfactant. Suitably, the biopharmaceutical composition comprises 0.2 mg/mL surfactant. Suitably, the biopharmaceutical composition comprises 0.3 mg/mL surfactant.

Suitably, the biopharmaceutical composition comprises 0.25-0.75 mg/mL surfactant. Suitably, the biopharmaceutical composition comprises 0.4-0.6 mg/mL. Suitably, the biopharmaceutical composition comprises 0.5 mg/mL surfactant.

Suitably, the biopharmaceutical composition comprises 0.8-1.2 mg/mL surfactant. Suitably, the biopharmaceutical composition comprises 0.9-1.1 mg/mL surfactant. Suitably, the biopharmaceutical composition comprises 1 mg/mL surfactant.

Suitably, the biopharmaceutical composition comprises greater than 1 mg/mL surfactant. Suitably, the biopharmaceutical composition comprises 1.01-2 mg/mL surfactant. Suitably, the biopharmaceutical composition comprises 1.05-1.5 mg/mL surfactant. Suitably, the biopharmaceutical composition comprises 1.2 mg/mL surfactant.

Suitably, the biopharmaceutical composition comprises 0.1-0.5 mg/mL surfactant. Suitably, the biopharmaceutical composition comprises 0.1-0.3 mg/mL surfactant. Suitably, the biopharmaceutical composition comprises 0.2 mg/mL surfactant.

Suitably, the biopharmaceutical composition comprises 0.001-0.09 mg/mL surfactant. Suitably, the biopharmaceutical composition comprises 0.001-0.05 mg/mL surfactant. Suitably, the biopharmaceutical composition comprises 0.05 mg/mL surfactant.

Suitably, the biopharmaceutical composition comprises 0.0008-3.82 mM surfactant. Suitably, the biopharmaceutical composition comprises 0.004-2.29 mM surfactant. Suitably, the biopharmaceutical composition comprises 0.008-1.53 mM surfactant. Suitably, the biopharmaceutical composition comprises 0.04-1.15 mM surfactant. Suitably, the biopharmaceutical composition comprises 0.008-0.92 mM surfactant. Suitably, the biopharmaceutical composition comprises 0.08-0.84 mM surfactant. Suitably, the biopharmaceutical composition comprises 0.08-0.53 mM surfactant. Suitably, the biopharmaceutical composition comprises 0.08 mM surfactant. Suitably, the biopharmaceutical composition comprises 0.0008-0.04 mM surfactant. Suitably, the biopharmaceutical composition comprises 0.0008-0.07 mM surfactant. Suitably, the biopharmaceutical composition comprises 0.008 mM surfactant. Suitably, the biopharmaceutical composition comprises 0.0008-0.11 mM surfactant. Suitably, the biopharmaceutical composition comprises 0.008-0.11 mM surfactant. Suitably, the biopharmaceutical composition comprises 0.08 mM surfactant. Suitably, the biopharmaceutical composition comprises 0.04-0.31 mM surfactant. Suitably, the biopharmaceutical composition comprises 0.08-0.23 mM surfactant. Suitably, the biopharmaceutical composition comprises 0.15 mM surfactant. Suitably, the biopharmaceutical composition comprises 0.19-0.57 mM surfactant. Suitably, the biopharmaceutical composition comprises 0.31-0.46 mM surfactant. Suitably, the biopharmaceutical composition comprises 0.38 mM surfactant. Suitably, the biopharmaceutical composition comprises 0.61-0.92 mM surfactant. Suitably, the biopharmaceutical composition comprises 0.68-0.84 mM surfactant. Suitably, the biopharmaceutical composition comprises 0.76 mM surfactant. Suitably, the biopharmaceutical composition comprises 0.77-1.53 mM surfactant. Suitably, the biopharmaceutical composition comprises 0.80-1.15 mM surfactant. Suitably, the biopharmaceutical composition comprises 0.92 mM surfactant. Suitably, the biopharmaceutical composition comprises 0.08-0.38 mM surfactant. Suitably, the biopharmaceutical composition comprises 0.08-0.23 mM surfactant. Suitably, the biopharmaceutical composition comprises 0.15 mM surfactant. Suitably, the biopharmaceutical composition comprises 0.0008-0.07 mM surfactant. Suitably, the biopharmaceutical composition comprises 0.0008-0.04 mM surfactant. Suitably, the biopharmaceutical composition comprises 0.04 mM surfactant.

Suitably, the biopharmaceutical composition comprises 0.001-5 mg/mL surfactant selected from the group consisting of polysorbates, spans, poloxamers, and kolliphors. Suitably, the biopharmaceutical composition comprises 0.01-2 mg/mL surfactant selected from the group consisting of polysorbates, spans, poloxamers, and kolliphors. Suitably, the biopharmaceutical composition comprises 0.1-1.1 mg/mL surfactant selected from the group consisting of polysorbates, spans, poloxamers, and kolliphors. Suitably, the biopharmaceutical composition comprises 0.001-0.05 mg/mL surfactant selected from the group consisting of polysorbates, spans, poloxamers, and kolliphors. Suitably, the biopharmaceutical composition comprises 0.01 mg/mL surfactant selected from the group consisting of polysorbates, spans, poloxamers, and kolliphors. Suitably, the biopharmaceutical composition comprises 0.001-0.15 mg/mL surfactant selected from the group consisting of polysorbates, spans, poloxamers, and kolliphors. Suitably, the biopharmaceutical composition comprises 0.1 mg/mL surfactant selected from the group consisting of polysorbates, spans, poloxamers, and kolliphors. Suitably, the biopharmaceutical composition comprises 0.1-0.3 mg/mL surfactant selected from the group consisting of polysorbates, spans, poloxamers, and kolliphors. Suitably, the biopharmaceutical composition comprises 0.2 mg/mL surfactant selected from the group consisting of polysorbates, spans, poloxamers, and kolliphors. Suitably, the biopharmaceutical composition comprises 0.25-0.75 mg/mL surfactant selected from the group consisting of polysorbates, spans, poloxamers, and kolliphors. Suitably, the biopharmaceutical composition comprises 0.5 mg/mL surfactant selected from the group consisting of polysorbates, spans, poloxamers, and kolliphors. Suitably, the biopharmaceutical composition comprises 0.8-1.2 mg/mL surfactant selected from the group consisting of polysorbates, spans, poloxamers, and kolliphors. Suitably, the biopharmaceutical composition comprises 1 mg/mL surfactant selected from the group consisting of polysorbates, spans, poloxamers, and kolliphors. Suitably, the biopharmaceutical composition comprises 1.2 mg/mL surfactant selected from the group consisting of polysorbates, spans, poloxamers, and kolliphors.

Suitably, the biopharmaceutical composition comprises 0.001-5 mg/mL surfactant selected from the group consisting of polysorbates and poloxamers. Suitably, the biopharmaceutical composition comprises 0.01-2 mg/mL surfactant selected from the group consisting of polysorbates and poloxamers. Suitably, the biopharmaceutical composition comprises 0.1-1.1 mg/mL surfactant selected from the group consisting of polysorbates and poloxamers. Suitably, the biopharmaceutical composition comprises 0.001-0.05 mg/mL surfactant selected from the group consisting of polysorbates and poloxamers. Suitably, the biopharmaceutical composition comprises 0.01 mg/mL surfactant selected from the group consisting of polysorbates and poloxamers. Suitably, the biopharmaceutical composition comprises 0.001-0.15 mg/mL surfactant selected from the group consisting of polysorbates and poloxamers. Suitably, the biopharmaceutical composition comprises 0.1 mg/mL surfactant selected from the group consisting of polysorbates and poloxamers. Suitably, the biopharmaceutical composition comprises 0.1-0.3 mg/mL surfactant selected from the group consisting of polysorbates and poloxamers. Suitably, the biopharmaceutical composition comprises 0.2 mg/mL surfactant selected from the group consisting of polysorbates and poloxamers. Suitably, the biopharmaceutical composition comprises 0.25-0.75 mg/mL surfactant selected from the group consisting of polysorbates and poloxamers. Suitably, the biopharmaceutical composition comprises 0.5 mg/mL surfactant selected from the group consisting of polysorbates and poloxamers. Suitably, the biopharmaceutical composition comprises 0.8-1.2 mg/mL surfactant selected from the group consisting of polysorbates and poloxamers. Suitably, the biopharmaceutical composition comprises 1 mg/mL surfactant selected from the group consisting of polysorbates and poloxamers. Suitably, the biopharmaceutical composition comprises 1.2 mg/mL surfactant selected from the group consisting of polysorbates and poloxamers.

Suitably, the biopharmaceutical composition comprises 0.001-5 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.01-2 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.1-1.1 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.001-0.05 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.01 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.001-0.15 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.1 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.1-0.3 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.2 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.3 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.25-0.75 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.5 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 0.8-1.2 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 1 mg/mL surfactant selected from the group consisting of polysorbate 20, polysorbate 40, polysorbate 60, polysorbate 80, poloxamer 182, poloxamer 188, and poloxamer 407. Suitably, the biopharmaceutical composition comprises 1.2 mg/mL surfactant selected from the group consisting of polysorbate 20, polysorbate 40, polysorbate 60, polysorbate 80, poloxamer 182, poloxamer 188, and poloxamer 407.

Suitably, the biopharmaceutical composition comprises 0.001-5 mg/mL polysorbate 20 or polysorbate 80. Suitably, the biopharmaceutical composition comprises 0.01-2 mg/mL polysorbate 20 or polysorbate 80. Suitably, the biopharmaceutical composition comprises 0.1-1.1 mg/mL polysorbate 20 or polysorbate 80. Suitably, the biopharmaceutical composition comprises 0.001-0.05 mg/mL polysorbate 20 or polysorbate 80. Suitably, the biopharmaceutical composition comprises 0.01 mg/mL polysorbate 20 or polysorbate 80. Suitably, the biopharmaceutical composition comprises 0.001-0.15 mg/mL polysorbate 20 or polysorbate 80. Suitably, the biopharmaceutical composition comprises 0.1 mg/mL polysorbate 20 or polysorbate 80. Suitably, the biopharmaceutical composition comprises 0.1-0.3 mg/mL polysorbate 20 or polysorbate 80. Suitably, the biopharmaceutical composition comprises 0.2 mg/mL polysorbate 20 or polysorbate 80. Suitably, the biopharmaceutical composition comprises 0.25-0.75 mg/mL polysorbate 20 or polysorbate 80. Suitably, the biopharmaceutical composition comprises 0.5 mg/mL polysorbate 20 or polysorbate 80. Suitably, the biopharmaceutical composition comprises 0.8-1.2 mg/mL polysorbate 20 or polysorbate 80. Suitably, the biopharmaceutical composition comprises 1 mg/mL polysorbate 20 or polysorbate 80. Suitably, the biopharmaceutical composition comprises 1.2 mg/mL polysorbate 20 or polysorbate 80.

Suitably, the biopharmaceutical composition comprises 0.001-5 mg/mL polysorbate 20. Suitably, the biopharmaceutical composition comprises 0.01-2 mg/mL polysorbate 20. Suitably, the biopharmaceutical composition comprises 0.1-1.1 mg/mL polysorbate 20. Suitably, the biopharmaceutical composition comprises 0.001-0.05 mg/mL polysorbate 20. Suitably, the biopharmaceutical composition comprises 0.01 mg/mL polysorbate 20. Suitably, the biopharmaceutical composition comprises 0.001-0.15 mg/mL polysorbate 20. Suitably, the biopharmaceutical composition comprises 0.1 mg/mL polysorbate 20. Suitably, the biopharmaceutical composition comprises 0.1-0.3 mg/mL polysorbate 20. Suitably, the biopharmaceutical composition comprises 0.2 mg/mL polysorbate 20. Suitably, the biopharmaceutical composition comprises 0.3 mg/mL polysorbate 20. Suitably, the biopharmaceutical composition comprises 0.25-0.75 mg/mL polysorbate 20. Suitably, the biopharmaceutical composition comprises 0.5 mg/mL polysorbate 20. Suitably, the biopharmaceutical composition comprises 0.8-1.2 mg/mL polysorbate 20. Suitably, the biopharmaceutical composition comprises 1 mg/mL polysorbate 20. Suitably, the biopharmaceutical composition comprises 1.2 mg/mL polysorbate 20.

Suitably, the biopharmaceutical composition comprises 0.001-5 mg/mL polysorbate 80. Suitably, the biopharmaceutical composition comprises 0.01-2 mg/mL polysorbate 80. Suitably, the biopharmaceutical composition comprises 0.1-1.1 mg/mL polysorbate 80. Suitably, the biopharmaceutical composition comprises 0.001-0.05 mg/mL polysorbate 80. Suitably, the biopharmaceutical composition comprises 0.01 mg/mL polysorbate 80. Suitably, the biopharmaceutical composition comprises 0.001-0.15 mg/mL polysorbate 80. Suitably, the biopharmaceutical composition comprises 0.1 mg/mL polysorbate 80. Suitably, the biopharmaceutical composition comprises 0.1-0.3 mg/mL polysorbate 80. Suitably, the biopharmaceutical composition comprises 0.2 mg/mL polysorbate 80.. Suitably, the biopharmaceutical composition comprises 0.3 mg/mL polysorbate 80. Suitably, the biopharmaceutical composition comprises 0.25-0.75 mg/mL polysorbate 80. Suitably, the biopharmaceutical composition comprises 0.5 mg/mL polysorbate 80. Suitably, the biopharmaceutical composition comprises 0.8-1.2 mg/mL polysorbate 80. Suitably, the biopharmaceutical composition comprises 1 mg/mL polysorbate 80. Suitably, the biopharmaceutical composition comprises 1.2 mg/mL polysorbate 80.

The biopharmaceutical composition suitably comprises 0.001-5 mg/mL poloxamer 188. The biopharmaceutical composition suitably comprises 0.01-2 mg/mL poloxamer 188. The biopharmaceutical composition suitably comprises 0.1-1.1 mg/mL poloxamer 188. The biopharmaceutical composition suitably comprises 1.0 mg/mL poloxamer 188. The biopharmaceutical composition suitably comprises 0.001-0.05 mg/mL poloxamer 188. The biopharmaceutical composition suitably comprises 0.01 mg/mL poloxamer 188. The biopharmaceutical composition suitably comprises 0.001-0.15 mg/mL poloxamer 188. The biopharmaceutical composition suitably comprises 0.1 mg/mL poloxamer 188. The biopharmaceutical composition suitably comprises 0.1-0.3 mg/mL poloxamer 188. The biopharmaceutical composition suitably comprises 0.2 mg/mL poloxamer 188. The biopharmaceutical composition suitably comprises 0.3 mg/mL poloxamer 188. The biopharmaceutical composition suitably comprises 0.25-0.75 mg/mL poloxamer 188. The biopharmaceutical composition suitably comprises 0.5 mg/mL poloxamer 188. The biopharmaceutical composition suitably comprises 0.8-1.2 mg/mL poloxamer 188. The biopharmaceutical composition suitably comprises 1 mg/mL poloxamer 188. The biopharmaceutical composition suitably comprises 1.2 mg/mL poloxamer 188.

The biopharmaceutical composition may be characterised by an absence of polysorbate 80. The biopharmaceutical composition may be characterised by an absence of surfactants polysorbates. The biopharmaceutical composition may be characterised by an absence of surfactants poloxamers. The biopharmaceutical composition may be characterised by an absence of surfactants polysorbates and poloxamers. The biopharmaceutical composition may be characterised by an absence of a surfactant. The biopharmaceutical composition may be characterised by an absence of any, some, or all surfactants defined herein.

The aforementioned characteristics of a biopharmaceutical composition, in particular in relation to surfactants, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

***Antioxidants***

The biopharmaceutical composition suitably comprises an antioxidant. Suitably, the antioxidant may mitigate against oxidation of the biopharmaceutical active, especially portions thereof that are potentially vulnerable to oxidation.

Suitably the antioxidant is a single antioxidant.

Suitably the antioxidant is an antioxidant selected from the group consisting of an amino acid antioxidant, a peptide antioxidant, a mineral (or inorganic) antioxidant, a vitamin antioxidant, a carotenoid antioxidant, a polyphenol antioxidant, an aromatic antioxidant, a phenolic antioxidant, a chelating agent antioxidant, a thiol antioxidant, and any combination thereof.

Suitably the antioxidant is an amino acid antioxidant. Suitably the antioxidant is an amino acid antioxidant selected from the group consisting of methionine, N-acetyl-l-cysteine, cysteine, glutathione, and any combination thereof. Suitably the antioxidant is an amino acid antioxidant selected from the group consisting of N-acetyl-l-cysteine, cysteine, glutathione, and any combination thereof. Suitably the antioxidant is methionine. Suitably the antioxidant is N-acetyl-l-cysteine. Suitably the antioxidant is cysteine. Suitably the antioxidant is glutathione.

Suitably the antioxidant is a mineral (or inorganic) antioxidant selected from the group consisting of a reducing metal compound (such as a compound of magnesium, iron, zinc, copper, and/or manganese), a thiosulfate compound (e.g. metal thiosulfate, ammonium thiosulfate), a bisulfite compound (e.g. metal bisulfite, ammonium bisulfite), a metabisulfite compound (e.g. metal metabisulfite, ammonium metabisulfite), a formaldehyde sulfoxylate (e.g. metal formaldehyde sulfoxylate, ammonium formaldehyde sulfoxylate), a glutamate compound (e.g. metal glutamate, ammonium glutamate, glutamic acid), a thioglycolate compound (e.g. metal thioglycolate, ammonium thioglycolate), a compound of sulfur, a compound of selenium, thiourea, and any combination thereof. Suitably the antioxidant is a mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the antioxidant is a mineral (or inorganic) antioxidant selected from the group consisting of a reducing metal compound (such as a compound of magnesium, iron, zinc, copper, and/or manganese), sodium thiosulfate, sodium bisulfite, sodium metabisulfite, sodium formaldehyde sulfoxylate, monosodium glutamate, sodium thioglycolate, a compound of sulfur, a compound of selenium, thiourea, and any combination thereof. Suitably the antioxidant is a mineral (or inorganic) antioxidant selected from the group consisting of sodium thiosulfate, sodium bisulfite, sodium metabisulfite, sodium formaldehyde sulfoxylate, and any combination thereof. Suitably the antioxidant is a thiosulfate. Suitably the antioxidant is sodium thiosulfate.

Suitably the antioxidant is a vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the antioxidant is ascorbic acid (and/or any salt or ester thereof).

Suitably the antioxidant is a carotenoid antioxidant selected from the group consisting of beta-carotene, lycopene, lutein, and zeaxanthin, and any combination thereof.

Suitably the antioxidant is a polyphenol antioxidant selected from the group consisting of phenolic acids, flavonoids, gingerol, curcumin, resveratrol, quercetin, and any combination thereof.

Suitably the antioxidant is an aromatic and/or phenolic antioxidant selected from the group consisting of butylated hydroxytoluene, butylated hydroxy anisole, gentisic acid, propyl gallate, and any combination thereof.

Suitably the antioxidant is a chelating agent antioxidant selected from EDTA (and/or any salt thereof) and/or pentetic acid (and/or any salt thereof). Suitably the antioxidant is EDTA (and/or any salt thereof). Suitably the antioxidant is pentetic acid (and/or any salt thereof).

Suitably the antioxidant is monothioglycerol. Suitably the antioxidant is an antioxidant selected from the group consisting of a thiosulfate, pentetic acid (and/or any salt thereof), cysteine, glutathione, and ascorbic acid. Suitably the antioxidant is an antioxidant selected from the group consisting of a thiosulfate, pentetic acid (and/or any salt thereof), cysteine, and ascorbic acid. Suitably the antioxidant is an antioxidant selected from the group consisting of a thiosulfate, pentetic acid (and/or any salt thereof), and cysteine.

Suitably the antioxidant is an antioxidant selected from thiosulfate and/or pentetic acid (and/or any salt thereof).

Suitably the biopharmaceutical composition comprises 0.001-300 mM antioxidant. Suitably the biopharmaceutical composition comprises 0.01-50 mM antioxidant. Suitably the biopharmaceutical composition comprises 0.1-20 mM antioxidant.

Suitably the biopharmaceutical composition comprises less than 2.5 mM antioxidant. Suitably the biopharmaceutical composition comprises 0.001-2.4 mM antioxidant. Suitably the biopharmaceutical composition comprises 0.01-2 mM antioxidant. Suitably the biopharmaceutical composition comprises 0.1-1 mM antioxidant. Suitably the biopharmaceutical composition comprises 0.5 mM antioxidant.

Suitably the biopharmaceutical composition comprises 1-4 mM antioxidant. Suitably the biopharmaceutical composition comprises 2 mM antioxidant.

Suitably the biopharmaceutical composition comprises 6-10 mM antioxidant. Suitably the biopharmaceutical composition comprises 7 mM antioxidant.

Suitably the biopharmaceutical composition comprises 1-10 mM antioxidant. Suitably the biopharmaceutical composition comprises 5 mM antioxidant.

Suitably the biopharmaceutical composition comprises more than 20 mM antioxidant. Suitably the biopharmaceutical composition comprises 21-300 mM antioxidant. Suitably the biopharmaceutical composition comprises 200 mM antioxidant.

Suitably the biopharmaceutical composition comprises 0.001-300 mM amino acid antioxidant selected from the group consisting of N-acetyl-l-cysteine, cysteine, glutathione, and any combination thereof. Suitably the biopharmaceutical composition comprises 0.1-20 mM amino acid antioxidant selected from the group consisting of N-acetyl-l-cysteine, cysteine, glutathione, and any combination thereof. Suitably the biopharmaceutical composition comprises 0.001-2.4 mM amino acid antioxidant selected from the group consisting of N-acetyl-l-cysteine, cysteine, glutathione, and any combination thereof. Suitably the biopharmaceutical composition comprises 0.5 mM amino acid antioxidant selected from the group consisting of N-acetyl-l-cysteine, cysteine, glutathione, and any combination thereof. Suitably the biopharmaceutical composition comprises 7 mM amino acid antioxidant selected from the group consisting of N-acetyl-l-cysteine, cysteine, glutathione, and any combination thereof. Suitably the biopharmaceutical composition comprises 1-10 mM amino acid antioxidant selected from the group consisting of N-acetyl-l-cysteine, cysteine, glutathione, and any combination thereof. Suitably the biopharmaceutical composition comprises 2 mM amino acid antioxidant selected from the group consisting of N-acetyl-l-cysteine, cysteine, glutathione, and any combination thereof. Suitably the biopharmaceutical composition comprises 5 mM amino acid antioxidant selected from the group consisting of N-acetyl-l-cysteine, cysteine, glutathione, and any combination thereof. Suitably the biopharmaceutical composition comprises 21-300 mM amino acid antioxidant selected from the group consisting of N-acetyl-l-cysteine, cysteine, glutathione, and any combination thereof. Suitably the biopharmaceutical composition comprises 200 mM amino acid antioxidant selected from the group consisting of N-acetyl-l-cysteine, cysteine, glutathione, and any combination thereof. Suitably the biopharmaceutical composition comprises 0.001-300 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 0.1-20 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 0.001-2.4 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 0.5 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 7 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 1-10 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 5 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 21-300 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 200 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof. Suitably the biopharmaceutical composition comprises 0.001-300 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 0.1-20 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 0.001-2.4 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 0.5 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 7 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 1-10 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 5 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 21-300 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof. Suitably the biopharmaceutical composition comprises 200 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

Suitably the biopharmaceutical composition comprises 0.001-300 mM thiosulfate (especially sodium thiosulfate). Suitably the biopharmaceutical composition comprises 0.1-20 mM thiosulfate (especially sodium thiosulfate). Suitably the biopharmaceutical composition comprises 0.001-2.4 mM thiosulfate (especially sodium thiosulfate). Suitably the biopharmaceutical composition comprises 0.5 mM thiosulfate (especially sodium thiosulfate). Suitably the biopharmaceutical composition comprises 7 mM thiosulfate (especially sodium thiosulfate). Suitably the biopharmaceutical composition comprises 1-10 mM thiosulfate (especially sodium thiosulfate). Suitably the biopharmaceutical composition comprises 5 mM thiosulfate (especially sodium thiosulfate). Suitably the biopharmaceutical composition comprises 21-300 mM thiosulfate (especially sodium thiosulfate). Suitably the biopharmaceutical composition comprises 200 mM thiosulfate (especially sodium thiosulfate).

The biopharmaceutical composition suitably comprises 0.001-300 mM methionine. The biopharmaceutical composition suitably comprises 0.1-20 mM methionine. The biopharmaceutical composition suitably comprises 0.001-2.4 mM methionine. The biopharmaceutical composition suitably comprises 0.5 mM methionine. The biopharmaceutical composition suitably comprises 7 mM methionine. The biopharmaceutical composition suitably comprises 1-10 mM methionine. The biopharmaceutical composition suitably comprises 5 mM methionine.

The biopharmaceutical composition suitably comprises 0.001-300 mM cysteine. The biopharmaceutical composition suitably comprises 0.1-20 mM cysteine. The biopharmaceutical composition suitably comprises 0.001-2.4 mM cysteine. The biopharmaceutical composition suitably comprises 0.5 mM cysteine. The biopharmaceutical composition suitably comprises 2 mM cysteine. The biopharmaceutical composition suitably comprises 1-10 mM cysteine. The biopharmaceutical composition suitably comprises 5 mM cysteine.

Suitably the biopharmaceutical composition comprises 0.001-300 mM EDTA (and/or any salt thereof). Suitably the biopharmaceutical composition comprises 0.1-20 mM EDTA (and/or any salt thereof). Suitably the biopharmaceutical composition comprises 0.001-2.4 mM EDTA (and/or any salt thereof). Suitably the biopharmaceutical composition comprises 0.5 mM EDTA (and/or any salt thereof). Suitably the biopharmaceutical composition comprises 7 mM EDTA (and/or any salt thereof). Suitably the biopharmaceutical composition comprises 1-10 mM EDTA (and/or any salt thereof). Suitably the biopharmaceutical composition comprises 2 mM EDTA (and/or any salt thereof). Suitably the biopharmaceutical composition comprises 5 mM EDTA (and/or any salt thereof). Suitably the biopharmaceutical composition comprises 21-300 mM EDTA (and/or any salt thereof). Suitably the biopharmaceutical composition comprises 200 mM EDTA (and/or any salt thereof).

Suitably the biopharmaceutical composition comprises 0.001-300 mM pentetic acid (and/or any salt thereof). Suitably the biopharmaceutical composition comprises 0.1-20 mM pentetic acid (and/or any salt thereof). Suitably the biopharmaceutical composition comprises 0.001-2.4 mM pentetic acid (and/or any salt thereof). Suitably the biopharmaceutical composition comprises 0.5 mM pentetic acid (and/or any salt thereof). Suitably the biopharmaceutical composition comprises 7 mM pentetic acid (and/or any salt thereof). Suitably the biopharmaceutical composition comprises 1-10 mM pentetic acid (and/or any salt thereof). Suitably the biopharmaceutical composition comprises 5 mM pentetic acid (and/or any salt thereof). Suitably the biopharmaceutical composition comprises 21-300 mM pentetic acid (and/or any salt thereof). Suitably the biopharmaceutical composition comprises 200 mM pentetic acid (and/or any salt thereof).

The biopharmaceutical composition may be suitably characterised by an absence of methionine. The biopharmaceutical composition may be suitably characterised by an absence of an antioxidant. The biopharmaceutical composition may be suitably characterised by an absence of any, some, or all antioxidants defined herein.

The aforementioned characteristics of a biopharmaceutical composition, in particular in relation to antioxidants, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

***Tonicifiers***

The biopharmaceutical composition suitably comprises a tonicifier.

Suitably, the tonicifier is a further tonicifier (i.e. additional to any other already-specified components/ingredients, such as sugar component(s), amino acid component(s), buffer system(s), antioxidant(s), etc., that also happen to contribute to osmolality/tonicity). Suitably, the tonicifier is a tonicifier that is a further tonicifier (i.e. additional to any other already-specified components/ingredients that also happen to contribute to osmolality/tonicity).

Suitably, the tonicifier is a tonicifier that contributes to (or increases) overall osmolality and osmolarity. Suitably, the tonicifier is a tonicifier in a quantity or concentration sufficient for the composition to be (substantially) isotonic with body fluids (especially blood, especially blood plasma). Suitably, the tonicifier is a tonicifier in a quantity or concentration sufficient for the composition to have an osmolarity or osmolality within a range defined herein.

Suitably, the tonicifier is a (further) tonicifier that consists of one or more (further) tonicifiers. Suitably, the tonicifier is a single (further) tonicifier.

Suitably, the tonicifier is a tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

Suitably, the tonicifier is a (or one or more) metal salt tonicifier. Suitably, the tonicifier is a metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the tonicifier is sodium chloride.

Suitably, the tonicifier is a (or one or more) non-metal salt tonicifier. Suitably, the tonicifier is a non-metal salt tonicifier selected from the group consisting of ammonium chloride, ammonium formate, ammonium acetate, histidine hydrochloride, and any combination thereof. Suitably, the tonicifier is ammonium acetate.

Suitably, the tonicifier is a (or one or more) polyol tonicifier. Suitably, the tonicifier is a polyol tonicifier selected from the group consisting of mannitol, sorbitol, maltose, glucose, lactose, and any combination thereof. Suitably, the tonicifier is maltose.

Suitably, the tonicifier is an (or one or more) amino acid tonicifier. Suitably, the tonicifier is an amino acid tonicifier selected from the group consisting of arginine, glycine, histidine, lysine, aspartic acid, glutamic acid, and any combination thereof. Suitably, the tonicifier is arginine. Suitably, the tonicifier is lysine.

Suitably, the tonicifier is a (one or more) non-buffering tonicifier(s) that is either buffering or non-buffering (e.g. at the prevailing pH). Suitably, the tonicifier is a (one or more) non-buffering tonicifier(s) (i.e. which imparts substantially no buffering effect, or does not substantially contribute to buffering, especially at the prevailing pH). Suitably, the tonicifier is a (one or more) buffering tonicifier(s) (i.e. which may impart a buffering effect, or may contribute to buffering, especially at the prevailing pH).

Suitably, the biopharmaceutical composition comprises 5-300 mM tonicifier.

Suitably, the biopharmaceutical composition comprises less than 130 mM tonicifier. Suitably, the biopharmaceutical composition comprises 5-300 mM tonicifier, provided that no single tonicifier compound exceeds 129 mM. Suitably, the biopharmaceutical composition comprises 10-129 mM tonicifier. Suitably, the biopharmaceutical composition comprises 20-120 mM tonicifier. Suitably, the biopharmaceutical composition comprises 20-50 mM tonicifier. Suitably, the biopharmaceutical composition comprises 30 mM tonicifier. Suitably, the biopharmaceutical composition comprises 50-100 mM tonicifier. Suitably, the biopharmaceutical composition comprises 60 mM tonicifier. Suitably, the biopharmaceutical composition comprises 100-129 mM tonicifier. Suitably, the biopharmaceutical composition comprises 105 mM tonicifier.

Suitably, the biopharmaceutical composition comprises less than 200 mM tonicifier. Suitably, the biopharmaceutical composition comprises 5-300 mM tonicifier, provided that no single tonicifier compound exceeds 199 mM. Suitably, the biopharmaceutical composition comprises 10-199 mM tonicifier. Suitably, the biopharmaceutical composition comprises 130-199 mM tonicifier. Suitably, the biopharmaceutical composition comprises 170 mM tonicifier.

Suitably, the biopharmaceutical composition comprises 5-300 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 10-129 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 50-100 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 60 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 105 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 130-199 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof. Suitably, the biopharmaceutical composition comprises 170 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

Suitably, the biopharmaceutical composition comprises 5-300 mM metal salt tonicifier. Suitably, the biopharmaceutical composition comprises 10-129 mM metal salt tonicifier. Suitably, the biopharmaceutical composition comprises 50-100 mM metal salt tonicifier. Suitably, the biopharmaceutical composition comprises 60 mM metal salt tonicifier. Suitably, the biopharmaceutical composition comprises 105 mM metal salt tonicifier. Suitably, the biopharmaceutical composition comprises 130-199 mM metal salt tonicifier. Suitably, the biopharmaceutical composition comprises 170 mM metal salt tonicifier.

Suitably, the biopharmaceutical composition comprises 5-300 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 10-129 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 50-100 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 60 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 105 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 130-199 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 170 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

Suitably, the biopharmaceutical composition comprises 5-300 mM (further) non-buffering tonicifier. Suitably, the biopharmaceutical composition comprises 10-129 mM (further) non-buffering tonicifier. Suitably, the biopharmaceutical composition comprises 50-100 mM (further) non-buffering tonicifier. Suitably, the biopharmaceutical composition comprises 60 mM (further) non-buffering tonicifier. Suitably, the biopharmaceutical composition comprises 105 mM (further) non-buffering tonicifier. Suitably, the biopharmaceutical composition comprises 130-199 mM (further) non-buffering tonicifier. Suitably, the biopharmaceutical composition comprises 170 mM (further) non-buffering tonicifier.

Suitably, the biopharmaceutical composition comprises 5-300 mM sodium chloride. Suitably, the biopharmaceutical composition comprises 10-129 mM sodium chloride. Suitably, the biopharmaceutical composition comprises 50-100 mM sodium chloride. Suitably, the biopharmaceutical composition comprises 60 mM sodium chloride. Suitably, the biopharmaceutical composition comprises 105 mM sodium chloride. Suitably, the biopharmaceutical composition comprises 130-199 mM sodium chloride. Suitably, the biopharmaceutical composition comprises 170 mM sodium chloride.

Suitably, the biopharmaceutical composition is characterised by an absence of any further additional tonicifiers. Suitably, the biopharmaceutical composition is characterised by an absence of metal halides. Suitably, the biopharmaceutical composition is characterised by an absence of sodium chloride. Suitably, the biopharmaceutical composition is characterised by an absence of a (further) tonicifier. Suitably, the biopharmaceutical composition is characterised by an absence of any, some, or all (further) tonicifiers defined herein.

The aforementioned characteristics of a biopharmaceutical composition, in particular in relation to tonicifiers, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

## *Ionic Strength Provider*

The biopharmaceutical composition may suitably comprise an ionic strength provider.

Suitably, the ionic strength provider is a further ionic strength provider (i.e. additional to any other already-specified components/ingredients, such as buffer system(s), that also happen to contribute to ionic strength). Suitably, the ionic strength provider is an ionic strength provider that is a further ionic strength provider (i.e. additional to any other already-specified components/ingredients that also happen to contribute to ionic strength).

Suitably, the ionic strength provider is an ionic strength provider in a quantity or concentration sufficient to provide the composition with an overall molar ionic strength (or molar ionic strength range) as defined herein. Suitably, the ionic strength provider is an ionic strength provider that contributes 2-200 mM of ionic strength. Suitably, the ionic strength provider is an ionic strength provider that contributes 5-120 mM of ionic strength. Suitably, the ionic strength provider is an ionic strength provider that contributes 10-100 mM of ionic strength. Suitably, the ionic strength provider is an ionic strength provider that contributes 20-80 mM of ionic strength. Suitably, the ionic strength provider is an ionic strength provider that contributes 50-70 mM of ionic strength.

Suitably, the ionic strength provider is an ionic strength provider that consists of one or more ionic strength provider compounds. Suitably, the ionic strength provider is a single (further) ionic strength provider.

Suitably, the ionic strength provider is a salt.

Suitably, the ionic strength provider is an ionic strength provider selected from the group consisting of a (or one or more) metal salt ionic strength provider, a (or one or more) non-metal salt ionic strength provider, and any combination thereof.

Suitably, the ionic strength provider is a metal salt. Suitably, the ionic strength provider is a metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the ionic strength provider is sodium chloride.

Suitably, the ionic strength provider is a non-metal salt. Suitably, the ionic strength provider is a non-metal salt ionic strength provider selected from the group consisting of ammonium chloride, ammonium formate, ammonium acetate, histidine hydrochloride, and any combination thereof. Suitably, the ionic strength provider is ammonium acetate.

Suitably, the ionic strength provider is a (one or more) non-buffering ionic strength provider(s) that is either buffering or non-buffering (e.g. at the prevailing pH). Suitably, the ionic strength provider is a (one or more) non-buffering ionic strength provider(s) (i.e. which imparts substantially no buffering effect, or does not substantially contribute to buffering, especially at the prevailing pH). Suitably, the ionic strength provider is a (one or more) buffering ionic strength provider (s) (i.e. which may impart a buffering effect, or may contribute to buffering, especially at the prevailing pH).

Suitably, the biopharmaceutical composition comprises 5-300 mM ionic strength provider.

Suitably, the biopharmaceutical composition comprises less than 130 mM ionic strength provider. Suitably, the biopharmaceutical composition comprises 5-300 mM ionic strength provider, provided that no single ionic strength provider compound exceeds 129 mM. Suitably, the biopharmaceutical composition comprises 10-129 mM ionic strength provider. Suitably, the biopharmaceutical composition comprises 20-120 mM ionic strength provider. Suitably, the biopharmaceutical composition comprises 20-50 mM ionic strength provider. Suitably, the biopharmaceutical composition comprises 30 mM ionic strength provider. Suitably, the biopharmaceutical composition comprises 50-100 mM ionic strength provider. Suitably, the biopharmaceutical composition comprises 60 mM ionic strength provider. Suitably, the biopharmaceutical composition comprises 100-129 mM ionic strength provider. Suitably, the biopharmaceutical composition comprises 105 mM ionic strength provider.

Suitably, the biopharmaceutical composition comprises less than 200 mM ionic strength provider. Suitably, the biopharmaceutical composition comprises 5-300 mM ionic strength provider, provided that no single ionic strength provider compound exceeds 199 mM. Suitably, the biopharmaceutical composition comprises 10-199 mM ionic strength provider. Suitably, the biopharmaceutical composition comprises 130-199 mM ionic strength provider. Suitably, the biopharmaceutical composition comprises 170 mM ionic strength provider.

Suitably, the biopharmaceutical composition comprises 5-300 mM of a salt. Suitably, the biopharmaceutical composition comprises 10-129 mM of a salt. Suitably, the biopharmaceutical composition comprises 50-100 mM of a salt. Suitably, the biopharmaceutical composition comprises 60 mM of a salt. Suitably, the biopharmaceutical composition comprises 105 mM of a salt. Suitably, the biopharmaceutical composition comprises 130-199 mM of a salt. Suitably, the biopharmaceutical composition comprises 170 mM of a salt.

Suitably, the biopharmaceutical composition comprises 5-300 mM metal salt. Suitably, the biopharmaceutical composition comprises 10-129 mM metal salt. Suitably, the biopharmaceutical composition comprises 50-100 mM metal salt. Suitably, the biopharmaceutical composition comprises 60 mM metal salt. Suitably, the biopharmaceutical composition comprises 105 mM metal salt. Suitably, the biopharmaceutical composition comprises 130-199 mM metal salt. Suitably, the biopharmaceutical composition comprises 170 mM metal salt. Suitably, the biopharmaceutical composition comprises 5-300 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 10-129 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 50-100 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 60 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 105 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 130-199 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof. Suitably, the biopharmaceutical composition comprises 170 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

Suitably, the biopharmaceutical composition comprises 5-300 mM sodium chloride. Suitably, the biopharmaceutical composition comprises 10-129 mM sodium chloride. Suitably, the biopharmaceutical composition comprises 50-100 mM sodium chloride. Suitably, the biopharmaceutical composition comprises 60 mM sodium chloride. Suitably, the biopharmaceutical composition comprises 105 mM sodium chloride. Suitably, the biopharmaceutical composition comprises 130-199 mM sodium chloride. Suitably, the biopharmaceutical composition comprises 170 mM sodium chloride.

Suitably, the biopharmaceutical composition may be characterised by an absence of any (further) ionic strength providers. Suitably, the biopharmaceutical composition may be characterised by an absence of metal halides. Suitably, the biopharmaceutical composition may be characterised by an absence of sodium chloride. Suitably, the biopharmaceutical composition may be characterised by an absence of a (further) ionic strength provider. Suitably, the biopharmaceutical composition may be characterised by an absence of any, some, or all (further) ionic strength providers defined herein.

The aforementioned characteristics of a biopharmaceutical composition, in particular in relation to ionic strength providers, amounts thereof, and/or absences thereof, may be applied to any of the embodiments defined in numbered paragraphs A1-A51 1, B1-B255, C1-C255, D1-D1152, E1-E1151, F1-F12, G1-G959, and H1-H1423.

***Chelators***

Suitably, the biopharmaceutical composition may comprise a chelator.

Suitably, the chelator is a chelator that datively binds metal ions via three or more dative bonds.

Suitably, the chelator is one or more chelators. Suitably, the chelator is a single chelator.

Suitably, the chelator is a chelator selected from the group consisting of EDTA (or salts thereof), DTPA (pentetic acid or salts thereof), versetamide (or salts thereof), Calteridol (or salts thereof), and any combination thereof. Suitably, the chelator is a chelator selected from the group consisting of EDTA (or salts thereof), DTPA (pentetic acid or salts thereof), and any combination thereof.

Suitably, the chelator is EDTA (or any salt thereof). Suitably, the chelator is pentetic acid (or any salt thereof).

Suitably, the biopharmaceutical composition comprises 0.0001-5 mM chelator. Suitably, the biopharmaceutical composition comprises 0.001-2 mM chelator. Suitably, the biopharmaceutical composition comprises 2 mM chelator. Suitably, the biopharmaceutical composition comprises 0.005-1.5 mM chelator. Suitably, the biopharmaceutical composition comprises 0.01-1.1 mM chelator. Suitably, the biopharmaceutical composition comprises 0.001-0.1 mM chelator. Suitably, the biopharmaceutical composition comprises 0.005-0.05 mM chelator. Suitably, the biopharmaceutical composition comprises 0.001-0.01 mM chelator. Suitably, the biopharmaceutical composition comprises 0.01 mM chelator. Suitably, the biopharmaceutical composition comprises 0.01-0.1 mM chelator. Suitably, the biopharmaceutical composition comprises 0.05 mM chelator. Suitably, the biopharmaceutical composition comprises 0.1-1 mM chelator. Suitably, the biopharmaceutical composition comprises 0.3-0.7 mM chelator. Suitably, the biopharmaceutical composition comprises 0.5 mM chelator.

Suitably, the biopharmaceutical composition comprises 0.0001-5 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.005-1.5 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.01 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.05 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.1-1 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.3-0.7 mM chelator selected from the group consisting of EDTA (or salts thereof), DTPA (pentetic acid or salts thereof), versetamide (or salts thereof), Calteridol (or salts thereof), and any combination thereof. Suitably, the biopharmaceutical composition comprises 0.5 mM chelator selected from the group consisting of EDTA (or salts thereof), DTPA (pentetic acid or salts thereof), versetamide (or salts thereof), Calteridol (or salts thereof), and any combination thereof.

Suitably, the biopharmaceutical composition comprises 0.0001-5 mM chelator selected from the group consisting of EDTA (or salts thereof), DTPA (pentetic acid or salts thereof), and any combination thereof. Suitably, the biopharmaceutical composition comprises 2 mM chelator selected from the group consisting of EDTA (or salts thereof), DTPA (pentetic acid or salts thereof), and any combination thereof. Suitably, the biopharmaceutical composition comprises 0.005-1.5 mM chelator selected from the group consisting of EDTA (or salts thereof), DTPA (pentetic acid or salts thereof), and any combination thereof. Suitably, the biopharmaceutical composition comprises 0.01 mM chelator selected from the group consisting of EDTA (or salts thereof), DTPA (pentetic acid or salts thereof), and any combination thereof. Suitably, the biopharmaceutical composition comprises 0.05 mM chelator selected from the group consisting of EDTA (or salts thereof), DTPA (pentetic acid or salts thereof), and any combination thereof. Suitably, the biopharmaceutical composition comprises 0.1-1 mM chelator selected from the group consisting of EDTA (or salts thereof), DTPA (pentetic acid or salts thereof), and any combination thereof. Suitably, the biopharmaceutical composition comprises 0.3-0.7 mM chelator selected from the group consisting of EDTA (or salts thereof), DTPA (pentetic acid or salts thereof), and any combination thereof. Suitably, the biopharmaceutical composition comprises 0.5 mM chelator selected from the group consisting of EDTA (or salts thereof), DTPA (pentetic acid or salts thereof), and any combination thereof.

Suitably, the biopharmaceutical composition comprises 0.0001-5 mM EDTA (or any salt thereof). Suitably, the biopharmaceutical composition comprises 2 mM EDTA (or any salt thereof). Suitably, the biopharmaceutical composition comprises 0.005-1.5 mM EDTA (or any salt thereof). Suitably, the biopharmaceutical composition comprises 0.01 mM EDTA (or any salt thereof). Suitably, the biopharmaceutical composition comprises 0.05 mM EDTA (or any salt thereof). Suitably, the biopharmaceutical composition comprises 0.1-1 mM EDTA (or any salt thereof). Suitably, the biopharmaceutical composition comprises 0.3-0.7 mM EDTA (or any salt thereof). Suitably, the biopharmaceutical composition comprises 0.5 mM EDTA (or any salt thereof).

Suitably, the biopharmaceutical composition comprises 0.0001-5 mM pentetic acid (or any salt thereof). Suitably,

the biopharmaceutical composition comprises 0.005-1.5 mM pentetic acid (or any salt thereof). Suitably, the biopharmaceutical composition comprises 0.01 mM pentetic acid (or any salt thereof). Suitably, the biopharmaceutical composition comprises 0.05 mM pentetic acid (or any salt thereof). Suitably, the biopharmaceutical composition comprises 0.1-1 mM pentetic acid (or any salt thereof). Suitably, the biopharmaceutical composition comprises 0.3-0.7 mM pentetic acid (or any salt thereof). Suitably, the biopharmaceutical composition comprises 0.5 mM pentetic acid (or any salt thereof).

Suitably, the biopharmaceutical composition may be characterised by an absence of EDTA. Suitably, the biopharmaceutical composition may be characterised by an absence of a chelator. Suitably, the biopharmaceutical composition may be characterised by an absence of any, some, or all chelators defined herein.

The aforementioned characteristics of a biopharmaceutical composition, in particular in relation to chelators, amounts thereof, and/or absences thereof, may be applied to any of the embodiments defined in numbered paragraphs A1-A511, B1-B255, C1-C255, D1-D1152, E1-E1151, F1-F12, G1-G959, and H1-H1423.

## *Diluents*

The biopharmaceutical composition suitably comprises a diluent. Suitably, the diluent is a diluent that constitutes the balance of ingredients in the composition so that the weight percentages of all ingredients total 100 wt%. Suitably, the diluent is a diluent that is present where the composition is said to consist of certain specified ingredients/components. Suitably, the diluent is a diluent that dissolves all ingredients/components of the composition. Suitably, the diluent is a liquid diluent. Suitably, the diluent is an aqueous diluent. Suitably, the diluent is water. Suitably, the diluent is water for injection (WFI).

The aforementioned characteristics of a biopharmaceutical composition, in particular in relation to diluents, amounts thereof, and/or absences thereof, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

## *Osmolality*

The biopharmaceutical composition may suitably be characterised by an osmolality. Osmolality may be measured (or measurable), for instance, by freezing-point depression methods. Alternatively or additionally osmolality may be calculated (or calculable), such as defined above. Specified osmolalities may suitably relate to a biopharmaceutical composition without the biopharmaceutical active (e.g. to remove any contribution or distortions provided by the biopharmaceutical active). Most preferably, however, specified osmolalities relate to the entire biopharmaceutical composition (i.e. including the biopharmaceutical active).

The biopharmaceutical composition suitably has an osmolality between 100 and 500 mOsm/kg. The biopharmaceutical composition suitably has an osmolality between 150 and 450 mOsm/kg. The biopharmaceutical composition suitably has an osmolality between 200 and 500 mOsm/kg. The biopharmaceutical composition suitably has an osmolality between 200 and 400 mOsm/kg. The biopharmaceutical composition suitably has an osmolality between 220 and 380 mOsm/kg. The biopharmaceutical composition suitably has an osmolality between 220 and 280 mOsm/kg. The biopharmaceutical composition suitably has an osmolality between 240 and 340 mOsm/kg. The biopharmaceutical composition suitably has an osmolality between 240 and 270 mOsm/kg. The biopharmaceutical composition suitably has an osmolality between 260 and 320 mOsm/kg. The biopharmaceutical composition suitably has an osmolality between 270 and 320 mOsm/kg. The biopharmaceutical composition suitably has an osmolality between 280 and 310 mOsm/kg. The biopharmaceutical composition suitably has an osmolality between 100 and 500 mOsm/kg when the biopharmaceutical active is excluded. The biopharmaceutical composition suitably has an osmolality between 150 and 450 mOsm/kg when the biopharmaceutical active is excluded. The biopharmaceutical composition suitably has an osmolality between 200 and 500 mOsm/kg when the biopharmaceutical active is excluded. The biopharmaceutical composition suitably has an osmolality between 200 and 400 mOsm/kg when the biopharmaceutical active is excluded. The biopharmaceutical composition suitably has an osmolality between 220 and 380 mOsm/kg when the biopharmaceutical active is excluded. The biopharmaceutical composition suitably has an osmolality between 220 and 280 mOsm/kg when the biopharmaceutical active is excluded. The biopharmaceutical composition suitably has an osmolality between 240 and 340 mOsm/kg when the biopharmaceutical active is excluded. The biopharmaceutical composition suitably has an osmolality between 240 and 270 mOsm/kg when the biopharmaceutical active is excluded. The biopharmaceutical composition suitably has an osmolality between 260 and 320 mOsm/kg when the biopharmaceutical active is excluded. The biopharmaceutical composition suitably has an osmolality between 270 and 320 mOsm/kg when the biopharmaceutical active is excluded. The biopharmaceutical composition suitably has an osmolality between 280 and 310 mOsm/kg when the biopharmaceutical active is excluded.

The biopharmaceutical composition suitably has an osmolality that affords isotonicity with bodily fluids (especially with blood, especially human blood plasma).

The aforementioned characteristics of a biopharmaceutical composition, in particular in relation to osmolality, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

***Ionic Strength***

The biopharmaceutical composition may be characterised by an ionic strength. Said ionic strength may be measured (or measurable), for instance, *via* electrical conductivity measurements (which can easily by correlated to ionic strength by methods well known in the art). Most preferably, however, the ionic strength is calculated (or calculable), suitably as defined above.

The biopharmaceutical composition suitably has an ionic strength of 1-400 mM. The biopharmaceutical composition suitably has an ionic strength of 2-200 mM. The biopharmaceutical composition suitably has an ionic strength of 5-120 mM. The biopharmaceutical composition suitably has an ionic strength of 20-80 mM. The biopharmaceutical composition suitably has an ionic strength of 5-20 mM. The biopharmaceutical composition suitably has an ionic strength of 10-60 mM. The biopharmaceutical composition suitably has an ionic strength of 20-50 mM. The biopharmaceutical composition suitably has an ionic strength of 30-110 mM. The biopharmaceutical composition suitably has an ionic strength of 40-100 mM. The biopharmaceutical composition suitably has an ionic strength of 50-90 mM. The biopharmaceutical composition suitably has an ionic strength of 60-80 mM. The biopharmaceutical composition suitably has an ionic strength of 70-90 mM.

The biopharmaceutical composition suitably has an ionic strength of 1-400 mM when the biopharmaceutical active is excluded. The biopharmaceutical composition suitably has an ionic strength of 2-200 mM when the biopharmaceutical active is excluded. The biopharmaceutical composition suitably has an ionic strength of 5-120 mM when the biopharmaceutical active is excluded. The biopharmaceutical composition suitably has an ionic strength of 20-80 mM when the biopharmaceutical active is excluded. The biopharmaceutical composition suitably has an ionic strength of 5-20 mM when the biopharmaceutical active is excluded. The biopharmaceutical composition suitably has an ionic strength of 10-60 mM when the biopharmaceutical active is excluded. The biopharmaceutical composition suitably has an ionic strength of 20-50 mM when the biopharmaceutical active is excluded. The biopharmaceutical composition suitably has an ionic strength of 30-110 mM when the biopharmaceutical active is excluded. The biopharmaceutical composition suitably has an ionic strength of 40-100 mM when the biopharmaceutical active is excluded. The biopharmaceutical composition suitably has an ionic strength of 50-90 mM when the biopharmaceutical active is excluded. The biopharmaceutical composition suitably has an ionic strength of 60-80 mM when the biopharmaceutical active is excluded. The biopharmaceutical composition suitably has an ionic strength of 70-90 mM when the biopharmaceutical active is excluded.

The aforementioned characteristics of a biopharmaceutical composition, in particular in relation to ionic strength, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

***Viscosity***

The biopharmaceutical composition of the invention may be characterised by a viscosity, most suitably a dynamic viscosity. The dynamic viscosity is suitably measured in units of mPa.s (millipascal-seconds) at 25°C (suitably at SATP). For comparison, the dynamic viscosity of pure water at 25°C is known to be 0.89 mPa.s. Dynamic viscosity is suitably measured by a rheometer, suitably a rheometer equipped with a cone-plate setup (cone diameter 20 mm, 1° angle). Dynamic viscosity may be measured in accordance with USP-NF 912 (Rotational Rheometer Methods). Dynamic viscosity may be measured in accordance with PhEur (particularly European Pharmacopoeia 7.0) 2.2.10. Viscosity - rotating viscometer method.

The biopharmaceutical composition suitably has a dynamic viscosity of 1-30 mPa.s. The biopharmaceutical composition suitably has a dynamic viscosity of 5-20 mPa.s. The biopharmaceutical composition suitably has a dynamic viscosity of 7-20 mPa.s. The biopharmaceutical composition suitably has a dynamic viscosity of 8-17 mPa.s. The biopharmaceutical composition suitably has a dynamic viscosity of 9-16 mPa.s. The biopharmaceutical composition suitably has a dynamic viscosity of 8-12 mPa.s. The biopharmaceutical composition suitably has a dynamic viscosity of 12-20 mPa.s.

The aforementioned characteristics of a biopharmaceutical composition, in particular in relation to viscosity, may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

***Particular Embodiments***

Described below (and in the below sub-sections) are various aspects, embodiments, and features of the invention which may be combined with any other aspects, embodiments, and features described elsewhere herein.

In a particular embodiment, the composition comprise, consists of (preferably along with water as a diluent), and/or is otherwise characterised by: secukinumab; and a buffer (preferably phosphate-citrate buffer).

In a particular embodiment, the composition comprise, consists of (preferably along with water as a diluent), and/or is otherwise characterised by: 150-195 mg/mL secukinumab; and a buffer (preferably phosphate-citrate buffer).

In a particular embodiment, the composition comprise, consists of (preferably along with water as a diluent), and/or is otherwise characterised by: secukinumab; and 2-60 mM buffer (preferably phosphate-citrate buffer).

In a particular embodiment, the composition comprise, consists of (preferably along with water as a diluent), and/or is otherwise characterised by: 150-195 mg/mL secukinumab; and 2-60 mM buffer (preferably phosphate-citrate buffer).

In a particular embodiment, the composition comprise, consists of (preferably along with water as a diluent), and/or is otherwise characterised by: secukinumab; and a sugar component (preferably sorbitol).

In a particular embodiment, the composition comprise, consists of (preferably along with water as a diluent), and/or is otherwise characterised by: 150-195 mg/mL secukinumab; and a sugar component (preferably sorbitol).

In a particular embodiment, the composition comprise, consists of (preferably along with water as a diluent), and/or is otherwise characterised by: secukinumab; and 100-150 mM sugar component (preferably sorbitol).

In a particular embodiment, the composition comprise, consists of (preferably along with water as a diluent), and/or is otherwise characterised by: 150-195 mg/mL secukinumab; and 100-150 mM sugar component (preferably sorbitol).

In a particular embodiment, the composition comprise, consists of (preferably along with water as a diluent), and/or is otherwise characterised by: secukinumab; and a salt tonicifier (preferably NaCl).

In a particular embodiment, the composition comprise, consists of (preferably along with water as a diluent), and/or is otherwise characterised by: 150-195 mg/mL secukinumab; and a salt tonicifier (preferably NaCl).

In a particular embodiment, the composition comprise, consists of (preferably along with water as a diluent), and/or is otherwise characterised by: secukinumab; and 40-80 mM salt tonicifier (preferably NaCl).

In a particular embodiment, the composition comprise, consists of (preferably along with water as a diluent), and/or is otherwise characterised by: 150-195 mg/mL secukinumab; and 40-80 mM salt tonicifier (preferably NaCl).

In a particular embodiment, the composition comprise, consists of (preferably along with water as a diluent), and/or is otherwise characterised by: secukinumab; a buffer (preferably phosphate-citrate buffer); and a sugar component (preferably sorbitol).

In a particular embodiment, the composition comprise, consists of (preferably along with water as a diluent), and/or is otherwise characterised by: 150-195 mg/mL secukinumab; a buffer (preferably phosphate-citrate buffer); and a sugar component (preferably sorbitol).

In a particular embodiment, the composition comprise, consists of (preferably along with water as a diluent), and/or is otherwise characterised by: secukinumab; 2-60 mM buffer (preferably phosphate-citrate buffer); and a sugar component (preferably sorbitol).

In a particular embodiment, the composition comprise, consists of (preferably along with water as a diluent), and/or is otherwise characterised by: 150-195 mg/mL secukinumab; 2-60 mM buffer (preferably phosphate-citrate buffer); and a sugar component (preferably sorbitol).

In a particular embodiment, the composition comprise, consists of (preferably along with water as a diluent), and/or is otherwise characterised by: secukinumab; a buffer (preferably phosphate-citrate buffer); and 100-150 mM sugar component (preferably sorbitol).

In a particular embodiment, the composition comprise, consists of (preferably along with water as a diluent), and/or is otherwise characterised by: 150-195 mg/mL secukinumab; a buffer (preferably phosphate-citrate buffer); and 100-150 mM sugar component (preferably sorbitol).

In a particular embodiment, the composition comprise, consists of (preferably along with water as a diluent), and/or is otherwise characterised by: secukinumab; 2-60 mM buffer (preferably phosphate-citrate buffer); and 100-150 mM sugar component (preferably sorbitol).

In a particular embodiment, the composition comprise, consists of (preferably along with water as a diluent), and/or is otherwise characterised by: 150-195 mg/mL secukinumab; 2-60 mM buffer (preferably phosphate-citrate buffer); and 100-150 mM sugar component (preferably sorbitol).

In a particular embodiment, the composition comprise, consists of (preferably along with water as a diluent), and/or is otherwise characterised by: secukinumab; a buffer (preferably phosphate-citrate buffer); and a salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); and a salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); and a salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); and a salt tonicifier (preferably NaCl).

In a particular embodiment, the composition comprise, consists of (preferably along with water as a diluent), and/or is otherwise characterised by: secukinumab; a buffer (preferably phosphate-citrate buffer); and 40-80 mM salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); and 40-80 mM salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); and 40-80 mM salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); and 40-80 mM salt tonicifier (preferably NaCl).

In a particular embodiment, the composition comprise, consists of (preferably along with water as a diluent), and/or is otherwise characterised by: secukinumab; a sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; a sugar component (preferably sorbitol); and a salt tonicifier (preferably NaCl).

In a particular embodiment, the composition comprise, consists of (preferably along with water as a diluent), and/or is otherwise characterised by: secukinumab; 100-150 mM sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; 100-150 mM sugar component (preferably sorbitol); and a salt tonicifier (preferably NaCl).

In a particular embodiment, the composition comprise, consists of (preferably along with water as a diluent), and/or is otherwise characterised by: secukinumab; a sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; a sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; a buffer (preferably phosphate-citrate buffer); a sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); a sugar component (preferably sorbitol); and a salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); a sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); a sugar component (preferably sorbitol); and a salt tonicifier (preferably NaCl).

In a particular embodiment, the composition comprise, consists of (preferably along with water as a diluent), and/or is otherwise characterised by: secukinumab; a buffer (preferably phosphate-citrate buffer); 100-150 mM sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); 100-150 mM sugar component (preferably sorbitol); and a salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); 100-150 mM sugar component (preferably sorbitol); and a salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); 100-150 mM sugar component (preferably sorbitol); and a salt tonicifier (preferably NaCl).

In a particular embodiment, the composition comprise, consists of (preferably along with water as a diluent), and/or is otherwise characterised by: secukinumab; a buffer (preferably phosphate-citrate buffer); a sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); a sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); a sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); a sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; a buffer (preferably phosphate-citrate buffer); 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; a buffer (preferably phosphate-citrate buffer); 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; 2-60 mM buffer (preferably phosphate-citrate buffer); 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: 150-195 mg/mL secukinumab; 2-60 mM buffer (preferably phosphate-citrate buffer); 100-150 mM sugar component (preferably sorbitol); and 40-80 mM salt tonicifier (preferably NaCl).

In a particular embodiment, the composition comprise, consists of (preferably along with water as a diluent), and/or is otherwise characterised by: secukinumab; and a phosphate-citrate dual buffer system (preferably about 1:1 by moles mixture of each buffer system).

In a particular embodiment, the composition comprise, consists of (preferably along with water as a diluent), and/or is otherwise characterised by: 150-195 mg/mL secukinumab; and a phosphate-citrate dual buffer system (preferably about 1:1 by moles mixture of each buffer system).

In a particular embodiment, the composition comprise, consists of (preferably along with water as a diluent), and/or is otherwise characterised by: secukinumab; and 1-10 mM phosphate-citrate dual buffer system (preferably about 1:1 by moles mixture of each buffer system).

In a particular embodiment, the composition comprise, consists of (preferably along with water as a diluent), and/or is otherwise characterised by: 150-195 mg/mL secukinumab; and 1-10 mM phosphate-citrate dual buffer system (preferably about 1:1 by moles mixture of each buffer system).

In a particular embodiment, the composition comprise, consists of (preferably along with water as a diluent), and/or is otherwise characterised by: secukinumab; and sorbitol.

In a particular embodiment, the composition comprise, consists of (preferably along with water as a diluent), and/or is otherwise characterised by: 150-195 mg/mL secukinumab; and sorbitol.

In a particular embodiment, the composition comprise, consists of (preferably along with water as a diluent), and/or is otherwise characterised by: secukinumab; and 40-150 mM sorb.

In a particular embodiment, the composition comprise, consists of (preferably along with water as a diluent), and/or is otherwise characterised by: 150-195 mg/mL secukinumab; and 40-150 mM sorb.

In a particular embodiment, the composition comprise, consists of (preferably along with water as a diluent), and/or is otherwise characterised by: secukinumab; and NaCl.

In a particular embodiment, the composition comprise, consists of (preferably along with water as a diluent), and/or is otherwise characterised by: 150-195 mg/mL secukinumab; and NaCl.

In a particular embodiment, the composition comprise, consists of (preferably along with water as a diluent), and/or is otherwise characterised by: secukinumab; and 40-80 mM NaCl.

In a particular embodiment, the composition comprise, consists of (preferably along with water as a diluent), and/or is otherwise characterised by: 150-195 mg/mL secukinumab; and 40-80 mM NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); and sorbitol.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); and sorbitol.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); and sorbitol.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); and sorbitol.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); and 40-150 mM sorb.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); and 40-150 mM sorb.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); and 40-150 mM sorb.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); and 40-150 mM sorb.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); and NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); and NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); and NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); and NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); and 40-80 mM NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); and 40-80 mM NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); and 40-80 mM NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); and 40-80 mM NaCl.

In a particular embodiment, the composition comprise, consists of (preferably along with water as a diluent), and/or is otherwise characterised by: secukinumab; sorbitol; and NaCl.

In a particular embodiment, the composition comprise, consists of (preferably along with water as a diluent), and/or is otherwise characterised by: 150-195 mg/mL secukinumab; sorbitol; and NaCl.

In a particular embodiment, the composition comprise, consists of (preferably along with water as a diluent), and/or is otherwise characterised by: secukinumab; 40-150 mM sorb; and NaCl.

In a particular embodiment, the composition comprise, consists of (preferably along with water as a diluent), and/or is otherwise characterised by: 150-195 mg/mL secukinumab; 40-150 mM sorb; and NaCl.

In a particular embodiment, the composition comprise, consists of (preferably along with water as a diluent), and/or is otherwise characterised by: secukinumab; sorbitol; and 40-80 mM NaCl.

In a particular embodiment, the composition comprise, consists of (preferably along with water as a diluent), and/or is otherwise characterised by: 150-195 mg/mL secukinumab; sorbitol; and 40-80 mM NaCl.

In a particular embodiment, the composition comprise, consists of (preferably along with water as a diluent), and/or is otherwise characterised by: secukinumab; 40-150 mM sorb; and 40-80 mM NaCl.

In a particular embodiment, the composition comprise, consists of (preferably along with water as a diluent), and/or is otherwise characterised by: 150-195 mg/mL secukinumab; 40-150 mM sorb; and 40-80 mM NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); sorbitol; and NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); sorbitol; and NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); sorbitol; and NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); sorbitol; and NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); 40-150 mM sorb; and NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); 40-150 mM sorb; and NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); 40-150 mM sorb; and NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); 40-150 mM sorb; and NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); sorbitol; and 40-80 mM NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); sorbitol; and 40-80 mM NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); sorbitol; and 40-80 mM NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); sorbitol; and 40-80 mM NaCl.

In a particular embodiment, the composition comprise, consists of (preferably along with water as a diluent), and/or is otherwise characterised by: secukinumab; a phosphate-citrate dual buffer system (preferably about 1:1 by moles mixture of each buffer system); 40-150 mM sorb; and 40-80 mM NaCl.

In a particular embodiment, the composition comprise, consists of (preferably along with water as a diluent), and/or is otherwise characterised by: 150-195 mg/mL secukinumab; a phosphate-citrate dual buffer system (preferably about 1:1 by moles mixture of each buffer system); 40-150 mM sorb; and 40-80 mM NaCl.

In a particular embodiment, the composition comprise, consists of (preferably along with water as a diluent), and/or is otherwise characterised by: secukinumab; 1-10 mM phosphate-citrate dual buffer system (preferably about 1:1 by moles mixture of each buffer system); 40-150 mM sorb; and 40-80 mM NaCl.

In a particular embodiment, the composition comprise, consists of (preferably along with water as a diluent), and/or is otherwise characterised by: 150-195 mg/mL secukinumab; 1-10 mM phosphate-citrate dual buffer system (preferably about 1:1 by moles mixture of each buffer system); 40-150 mM sorb; and 40-80 mM NaCl.

In a particular embodiment, the composition comprise, consists of (preferably along with water as a diluent), and/or is otherwise characterised by: secukinumab; and a succinate buffer.

In a particular embodiment, the composition comprise, consists of (preferably along with water as a diluent), and/or is otherwise characterised by: 150-195 mg/mL secukinumab; and a succinate buffer.

In a particular embodiment, the composition comprise, consists of (preferably along with water as a diluent), and/or is otherwise characterised by: secukinumab; and 40-60 mM succinate buffer.

In a particular embodiment, the composition comprise, consists of (preferably along with water as a diluent), and/or is otherwise characterised by: 150-195 mg/mL secukinumab; and 40-60 mM succinate buffer.

In a particular embodiment, the composition comprise, consists of (preferably along with water as a diluent), and/or is otherwise characterised by: secukinumab; a succinate buffer; and a sugar component (preferably sorbitol).

In a particular embodiment, the composition comprise, consists of (preferably along with water as a diluent), and/or is otherwise characterised by: 150-195 mg/mL secukinumab; a succinate buffer; and a sugar component (preferably sorbitol).

In a particular embodiment, the composition comprise, consists of (preferably along with water as a diluent), and/or is otherwise characterised by: secukinumab; 40-60 mM succinate buffer; and a sugar component (preferably sorbitol).

In a particular embodiment, the composition comprise, consists of (preferably along with water as a diluent), and/or is otherwise characterised by: 150-195 mg/mL secukinumab; 40-60 mM succinate buffer; and a sugar component (preferably sorbitol).

In a particular embodiment, the composition comprise, consists of (preferably along with water as a diluent), and/or is otherwise characterised by: secukinumab; a succinate buffer; and sorbitol.

In a particular embodiment, the composition comprise, consists of (preferably along with water as a diluent), and/or is otherwise characterised by: 150-195 mg/mL secukinumab; a succinate buffer; and sorbitol.

In a particular embodiment, the composition comprise, consists of (preferably along with water as a diluent), and/or is otherwise characterised by: secukinumab; 40-60 mM succinate buffer; and sorbitol.

In a particular embodiment, the composition comprise, consists of (preferably along with water as a diluent), and/or is otherwise characterised by: 150-195 mg/mL secukinumab; 40-60 mM succinate buffer; and sorbitol.

In a particular embodiment, the composition comprise, consists of (preferably along with water as a diluent), and/or is otherwise characterised by: secukinumab; a succinate buffer; and 40-150 mM sorb.

In a particular embodiment, the composition comprise, consists of (preferably along with water as a diluent), and/or is otherwise characterised by: 150-195 mg/mL secukinumab; a succinate buffer; and 40-150 mM sorb.

In a particular embodiment, the composition comprise, consists of (preferably along with water as a diluent), and/or is otherwise characterised by: secukinumab; 40-60 mM succinate buffer; and 40-150 mM sorb.

In a particular embodiment, the composition comprise, consists of (preferably along with water as a diluent), and/or is otherwise characterised by: 150-195 mg/mL secukinumab; 40-60 mM succinate buffer; and 40-150 mM sorb.

The aforementioned embodiments may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

## *Composition Embodiments and Features with Dependencies*

### H) Compostion Embodiments and Features with Dependencies

The following numbered paragraphs H1-H1423, with dependencies, describe further embodiments and features of the invention. Numbered paragraphs H1-H1423 may be combined with any of the embodiments, aspects, and features disclosed in any other sections herein, including the embodiments of A1-A511, B1-B255, C1-C255, D1-D1152, E1-E1151, F1-F12, and G1-G959. A person skilled in the art will readily appreciate embodiments elucidated by the dependencies set forth below, including via direct and indirect dependencies. The compositions described in the ensuing paragraphs are suitably biopharmaceutical compositions, most suitably liquid biopharmaceutical compositions. Where compositions are said to "comprise" certain stipulated ingredients/components (especially when interdependencies provide for a multiplicity of ingredients/components in addition to the biopharmaceutical active), said compositions may alternatively consist of (suitably along with a diluent, which is preferably water) said certain stipulated ingredients/components. It will be self-evident to a person skilled in the art that a particular ingredient/component is absent in circumstances where a paragraph reciting the absence of said particular ingredient/component is dependent on other paragraphs reciting the presence (optionally in a specified quantity) of said particular ingredient/component.

H1. A composition comprising a biopharmaceutical active.

H2. The composition of H1, wherein the biopharmaceutical active is secukinumab.

(continued)

H3. The composition of H2, wherein the composition comprises 20-400 mg/mL secukinumab.
H4. The composition of H2, wherein the composition comprises 20-175 mg/mL secukinumab.
H5. The composition of H2, wherein the composition comprises 176-400 mg/mL secukinumab.
H6. The composition of H2, wherein the composition comprises 100-350 mg/mL secukinumab.
H7. The composition of H2, wherein the composition comprises 150-300 mg/mL secukinumab.
H8. The composition of H2, wherein the composition comprises 120-220 mg/mL secukinumab.
H9. The composition of H2, wherein the composition comprises 145-190 mg/mL secukinumab.
H10. The composition of H2, wherein the composition comprises 145-170 mg/mL secukinumab.
H11. The composition of H2, wherein the composition comprises 145-155 mg/mL secukinumab.
H12. The composition of H2, wherein the composition comprises 150 mg/mL secukinumab.
H13. The composition of H2, wherein the composition comprises 155-170 mg/mL secukinumab.
H14. The composition of H2, wherein the composition comprises 165 mg/mL secukinumab.
H15. The composition of H2, wherein the composition comprises 160-185 mg/mL secukinumab.
H16. The composition of H2, wherein the composition comprises 175-185 mg/mL secukinumab.
H17. The composition of H2, wherein the composition comprises 180 mg/mL secukinumab.
H18. The composition of H2, wherein the composition comprises 176-300 mg/mL secukinumab.
H19. The composition of H2, wherein the composition comprises 176-220 mg/mL secukinumab.
H20. The composition of H2, wherein the composition comprises 180-300 mg/mL secukinumab.
H21. The composition of H2, wherein the composition comprises 180-220 mg/mL secukinumab.
H22. The composition of H2, wherein the composition comprises 185-205 mg/mL secukinumab.
H23. The composition of H2, wherein the composition comprises 185-195 mg/mL secukinumab.
H24. The composition of H2, wherein the composition comprises 190 mg/mL secukinumab.
H25. The composition of H2, wherein the composition comprises 195-205 mg/mL secukinumab.
H26. The composition of H2, wherein the composition comprises 200 mg/mL secukinumab.
H27. The composition of H2, wherein the composition comprises 0.135-2.70 mM secukinumab.
H28. The composition of H2, wherein the composition comprises 0.135-1.18 mM secukinumab.
H29. The composition of H2, wherein the composition comprises 1.19-2.70 mM secukinumab.
H30. The composition of H2, wherein the composition comprises 0.68-2.36 mM secukinumab.
H31. The composition of H2, wherein the composition comprises 1.01-2.03 mM secukinumab.
H32. The composition of H2, wherein the composition comprises 0.81-1.49 mM secukinumab.
H33. The composition of H2, wherein the composition comprises 0.98-1.28 mM secukinumab.
H34. The composition of H2, wherein the composition comprises 0.98-1.15 mM secukinumab.
H35. The composition of H2, wherein the composition comprises 0.98-1.05 mM secukinumab.
H36. The composition of H2, wherein the composition comprises 1.01 mM secukinumab.
H37. The composition of H2, wherein the composition comprises 1.05-1.15 mM secukinumab.
H38. The composition of H2, wherein the composition comprises 1.11 mM secukinumab.
H39. The composition of H2, wherein the composition comprises 1.08-1.25 mM secukinumab.
H40. The composition of H2, wherein the composition comprises 1.18-1.25 mM secukinumab.
H41. The composition of H2, wherein the composition comprises 1.22 mM secukinumab.
H42. The composition of H2, wherein the composition comprises 1.19-2.03 mM secukinumab.
H43. The composition of H2, wherein the composition comprises 1.19-1.49 mM secukinumab.
H44. The composition of H2, wherein the composition comprises 1.22-2.03 mM secukinumab.
H45. The composition of H2, wherein the composition comprises 1.22-1.49 mM secukinumab.
H46. The composition of H2, wherein the composition comprises 1.25-1.39 mM secukinumab.
H47. The composition of H2, wherein the composition comprises 1.25-1.32 mM secukinumab.
H48. The composition of H2, wherein the composition comprises 1.28 mM secukinumab.
H49. The composition of H2, wherein the composition comprises 1.32-1.39 mM secukinumab.
H50. The composition of H2, wherein the composition comprises 1.35 mM secukinumab.
H51. The composition of any of H1-H50, wherein the composition comprises a buffer system.
H52. The composition of H51, wherein the composition comprises (and/or the buffer system is) a monoprotic buffer system.

(continued)

H53. The composition of H51 or any preceding paragraph dependent thereon, wherein the composition comprises (and/or the buffer system is) a polyprotic buffer system.

H54. The composition of H51 or any preceding paragraph dependent thereon, wherein the composition comprises (and/or the buffer system is) an zwitterionic buffer system.

H55. The composition of H51 or any preceding paragraph dependent thereon, wherein the composition comprises (and/or the buffer system is) a single-buffer system.

H56. The composition of H51 or any preceding paragraph dependent thereon, wherein the composition comprises (and/or the buffer system is) a multi-buffer system.

H57. The composition of H51 or any preceding paragraph dependent thereon, wherein the composition comprises (and/or the buffer system is) a dual-buffer system.

H58. The composition of H51 or any preceding paragraph dependent thereon, wherein the composition comprises (and/or the buffer system is) a dual-buffer system consisting of a first buffer system and a second buffer system in a respective molar ratio of between 20:1 and 1:20.

H59. The composition of H51 or any preceding paragraph dependent thereon, wherein the composition comprises (and/or the buffer system is) a dual-buffer system consisting of a first buffer system and a second buffer system in a respective molar ratio of between 5:1 and 1:5.

H60. The composition of H51 or any preceding paragraph dependent thereon, wherein the composition comprises (and/or the buffer system is) a dual-buffer system consisting of a first buffer system and a second buffer system in a respective molar ratio of between 3:1 and 1:3.

H61. The composition of H51 or any preceding paragraph dependent thereon, wherein the composition comprises (and/or the buffer system is) a dual-buffer system consisting of a first buffer system and a second buffer system in a respective molar ratio of between 2:1 and 1:2.

H62. The composition of H51 or any preceding paragraph dependent thereon, wherein the composition comprises (and/or the buffer system is) a dual-buffer system consisting of a first buffer system and a second buffer system in a molar ratio of 1:1.

H63. The composition of H51 or any preceding paragraph dependent thereon, wherein the composition comprises (and/or the buffer system is) a buffer system selected from the group consisting of a histidine buffer system, a succinate buffer system, an adipate buffer system, an acetate buffer system, a phosphate buffer system, a citrate buffer system, a phosphate-citrate buffer system, a phosphate-succinate buffer system, a phosphate acetate buffer system, a formate buffer system, a lactate buffer system, a salicylate buffer system, a benzoate buffer system, a maleate buffer system, a malate buffer system, a fumarate buffer system, a tartrate buffer system, a glycine buffer system, a lysine buffer system, a glycylglycine buffer system, a glutamate buffer system, an asparate buffer system, a histidine-acetate buffer system, a gluconate buffer system, a MES buffer system, and any combination thereof (suitably up to a maximum of two specific buffer systems).

H64. The composition of H51 or any preceding paragraph dependent thereon, wherein the composition comprises (and/or the buffer system is) a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a formate buffer system, a lactate buffer system, a salicylate buffer system, a benzoate buffer system, a maleate buffer system, a malate buffer system, a fumarate buffer system, a tartrate buffer system, a glycine buffer system, a lysine buffer system, a glycylglycine buffer system, a glutamate buffer system, an asparate buffer system, a gluconate buffer system, a MES buffer system, and any combination thereof (suitably up to a maximum of two specific buffer systems).

H65. The composition of H51 or any preceding paragraph dependent thereon, wherein the composition comprises (and/or the buffer system is) a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a glycine buffer system, a glutamate buffer system,

a gluconate buffer system, a MES buffer system,and any combination thereof (suitably up to a maximum of two specific buffer systems).

(continued)

H66. The composition of H51 or any preceding paragraph dependent thereon, wherein the composition comprises (and/or the buffer system is) a buffer system selected from the group consisting of a succinate buffer system, an adipate buffer system, an acetate buffer system, a phosphate buffer system, a citrate buffer system, a phosphate-citrate buffer system, a phosphate-succinate buffer system, a phosphate acetate buffer system, a MES buffer system,and any combination thereof (suitably up to a maximum of two specific buffer systems).

H67. The composition of H51 or any preceding paragraph dependent thereon, wherein the composition comprises (and/or the buffer system is) a buffer system selected from the group consisting of a succinate buffer system, an adipate buffer system, a phosphate buffer system, a citrate buffer system, a phosphate-citrate buffer system, and any combination thereof (suitably up to a maximum of two specific buffer systems).

H68. The composition of H51 or any preceding paragraph dependent thereon, wherein the composition comprises (and/or the buffer system is) a histidine buffer system.

H69. The composition of H51 or any preceding paragraph dependent thereon, wherein the composition comprises (and/or the buffer system is) a buffer system excluding histidine.

H70. The composition of H51 or any preceding paragraph dependent thereon, wherein the composition comprises (and/or the buffer system is) a succinate buffer system.

H71. The composition of H51 or any preceding paragraph dependent thereon, wherein the composition comprises (and/or the buffer system is) an adipate buffer system.

H72. The composition of H51 or any preceding paragraph dependent thereon, wherein the composition comprises (and/or the buffer system is) an acetate buffer system.

H73. The composition of H51 or any preceding paragraph dependent thereon, wherein the composition comprises (and/or the buffer system is) a phosphate buffer system.

H74. The composition of H51 or any preceding paragraph dependent thereon, wherein the composition comprises (and/or the buffer system is) a citrate buffer system.

H75. The composition of H51 or any preceding paragraph dependent thereon, wherein the composition comprises (and/or the buffer system is) a phosphate-citrate buffer system.

H76. The composition of H51 or any preceding paragraph dependent thereon, wherein the composition comprises (and/or the buffer system is) a phosphate-citrate buffer system consisting of a phosphate buffer system and a citrate buffer system in a respective molar ratio of between 20:1 and 1:20.

H77. The composition of H51 or any preceding paragraph dependent thereon, wherein the composition comprises (and/or the buffer system is) a phosphate-citrate buffer system consisting of a phosphate buffer system and a citrate buffer system in a respective molar ratio of between 5:1 and 1:5.

H78. The composition of H51 or any preceding paragraph dependent thereon, wherein the composition comprises (and/or the buffer system is) a phosphate-citrate buffer system consisting of a phosphate buffer system and a citrate buffer system in a respective molar ratio of between 3:1 and 1:3.

H79. The composition of H51 or any preceding paragraph dependent thereon, wherein the composition comprises (and/or the buffer system is) a phosphate-citrate buffer system consisting of a phosphate buffer system and a citrate buffer system in a respective molar ratio of between 2:1 and 1:2.

H80. The composition of H51 or any preceding paragraph dependent thereon, wherein the composition comprises (and/or the buffer system is) a phosphate-citrate buffer system consisting of a phosphate buffer system and a citrate buffer system in a molar ratio of 1:1.

H81. The composition of H51 or any preceding paragraph dependent thereon, wherein the composition comprises (and/or the buffer system is) a phosphate-succinate buffer system.

H82. The composition of H51 or any preceding paragraph dependent thereon, wherein the composition comprises (and/or the buffer system is) a phosphate-succinate buffer system consisting of a phosphate buffer system and a succinate buffer system in a respective molar ratio of between 20:1 and 1:20.

H83. The composition of H51 or any preceding paragraph dependent thereon, wherein the composition comprises (and/or the buffer system is) a phosphate-succinate buffer system consisting of a phosphate buffer system and a succinate buffer system in a respective molar ratio of between 5:1 and 1:5.

H84. The composition of H51 or any preceding paragraph dependent thereon, wherein the composition comprises (and/or the buffer system is) a phosphate-succinate buffer system consisting of a phosphate buffer system and a succinate buffer system in a respective molar ratio of between 3:1 and 1:3.

(continued)

H85. The composition of H51 or any preceding paragraph dependent thereon, wherein the composition comprises (and/or the buffer system is) a phosphate-succinate buffer system consisting of a phosphate buffer system and a succinate buffer system in a respective molar ratio of between 2:1 and 1:2.

H86. The composition of H51 or any preceding paragraph dependent thereon, wherein the composition comprises (and/or the buffer system is) a phosphate-succinate buffer system consisting of a phosphate buffer system and a succinate buffer system in a molar ratio of 1:1.

H87. The composition of H51 or any preceding paragraph dependent thereon, wherein the composition comprises (and/or the buffer system is) a phosphate-acetate buffer system.

H88. The composition of H51 or any preceding paragraph dependent thereon, wherein the composition comprises (and/or the buffer system is) a phosphate-acetate buffer system consisting of a phosphate buffer system and a acetate buffer system in a respective molar ratio of between 20:1 and 1:20.

H89. The composition of H51 or any preceding paragraph dependent thereon, wherein the composition comprises (and/or the buffer system is) a phosphate-acetate buffer system consisting of a phosphate buffer system and a acetate buffer system in a respective molar ratio of between 5:1 and 1:5.

H90. The composition of H51 or any preceding paragraph dependent thereon, wherein the composition comprises (and/or the buffer system is) a phosphate-acetate buffer system consisting of a phosphate buffer system and a acetate buffer system in a respective molar ratio of between 3:1 and 1:3.

H91. The composition of H51 or any preceding paragraph dependent thereon, wherein the composition comprises (and/or the buffer system is) a phosphate-acetate buffer system consisting of a phosphate buffer system and a acetate buffer system in a respective molar ratio of between 2:1 and 1:2.

H92. The composition of H51 or any preceding paragraph dependent thereon, wherein the composition comprises (and/or the buffer system is) a phosphate-acetate buffer system consisting of a phosphate buffer system and a acetate buffer system in a molar ratio of 1:1.

H93. The composition of H51 or any preceding paragraph dependent thereon, wherein the composition comprises (and/or the buffer system is) a formate buffer system.

H94. The composition of H51 or any preceding paragraph dependent thereon, wherein the composition comprises (and/or the buffer system is) a lactate buffer system.

H95. The composition of H51 or any preceding paragraph dependent thereon, wherein the composition comprises (and/or the buffer system is) a salicylate buffer system.

H96. The composition of H51 or any preceding paragraph dependent thereon, wherein the composition comprises (and/or the buffer system is) a benzoate buffer system.

H97. The composition of H51 or any preceding paragraph dependent thereon, wherein the composition comprises (and/or the buffer system is) a maleate buffer system.

H98. The composition of H51 or any preceding paragraph dependent thereon, wherein the composition comprises (and/or the buffer system is) a malate buffer system.

H99. The composition of H51 or any preceding paragraph dependent thereon, wherein the composition comprises (and/or the buffer system is) a fumarate buffer system.

H100. The composition of H51 or any preceding paragraph dependent thereon, wherein the composition comprises (and/or the buffer system is) a tartrate buffer system.

H101. The composition of H51 or any preceding paragraph dependent thereon, wherein the composition comprises (and/or the buffer system is) a glycine buffer system.

H102. The composition of H51 or any preceding paragraph dependent thereon, wherein the composition comprises (and/or the buffer system is) a lysine buffer system.

H103. The composition of H51 or any preceding paragraph dependent thereon, wherein the composition comprises (and/or the buffer system is) a glycylglycine buffer system.

H104. The composition of H51 or any preceding paragraph dependent thereon, wherein the composition comprises (and/or the buffer system is) a glutamate buffer system.

H105. The composition of H51 or any preceding paragraph dependent thereon, wherein the composition comprises (and/or the buffer system is) an asparate buffer system.

H106. The composition of H51 or any preceding paragraph dependent thereon, wherein the composition comprises (and/or the buffer system is) a histidine-acetate buffer system.

H107. The composition of H51 or any preceding paragraph dependent thereon, wherein the composition comprises (and/or the buffer system is) a gluconate buffer system.

(continued)

H108. The composition of H51 or any preceding paragraph dependent thereon, wherein the composition comprises (and/or the buffer system is) a MES buffer system.

H109. The composition of H51 or any preceding paragraph dependent thereon, wherein the composition comprises 1-70 mM buffer system.

H110. The composition of H51 or any preceding paragraph dependent thereon, wherein the composition comprises 2-60 mM buffer system.

H111. The composition of H51 or any preceding paragraph dependent thereon, wherein the composition comprises 3-56 mM buffer system.

H112. The composition of H51 or any preceding paragraph dependent thereon, wherein the composition comprises 5-50 mM buffer system.

H113. The composition of H51 or any preceding paragraph dependent thereon, wherein the composition comprises 10-30 mM buffer system.

H114. The composition of H51 or any preceding paragraph dependent thereon, wherein the composition comprises 20 mM buffer system .

H115. The composition of H51 or any preceding paragraph dependent thereon, wherein the composition comprises less than 10 mM buffer system.

H116. The composition of H51 or any preceding paragraph dependent thereon, wherein the composition comprises 1-9 mM buffer system .

H117. The composition of H51 or any preceding paragraph dependent thereon, wherein the composition comprises 6-10 mM buffer system.

H118. The composition of H51 or any preceding paragraph dependent thereon, wherein the composition comprises 8 mM buffer system .

H119. The composition of H51 or any preceding paragraph dependent thereon, wherein the composition comprises less than 5 mM buffer system.

H120. The composition of H51 or any preceding paragraph dependent thereon, wherein the composition comprises 0.5-4.5 mM buffer system.

H121. The composition of H51 or any preceding paragraph dependent thereon, wherein the composition comprises 1-4 mM buffer system .

H122. The composition of H51 or any preceding paragraph dependent thereon, wherein the composition comprises 2-6 mM buffer system .

H123. The composition of H51 or any preceding paragraph dependent thereon, wherein the composition comprises 4 mM buffer system .

H124. The composition of H51 or any preceding paragraph dependent thereon, wherein the composition comprises greater than 50 mM buffer system.

H125. The composition of H51 or any preceding paragraph dependent thereon, wherein the composition comprises 51-70 mM buffer system.

H126. The composition of H51 or any preceding paragraph dependent thereon, wherein the composition comprises 51-60 mM buffer system.

H127. The composition of H51 or any preceding paragraph dependent thereon, wherein the composition comprises 55 mM buffer system .

H128. The composition of H51 or any preceding paragraph dependent thereon, wherein the composition comprises 1-70 mM buffer system excluding histidine.

H129. The composition of H51 or any preceding paragraph dependent thereon, wherein the composition comprises 10-30 mM buffer system excluding histidine.

H130. The composition of H51 or any preceding paragraph dependent thereon, wherein the composition comprises 1-9 mM buffer system excluding histidine.

H131. The composition of H51 or any preceding paragraph dependent thereon, wherein the composition comprises 8 mM buffer system excluding histidine.

H132. The composition of H51 or any preceding paragraph dependent thereon, wherein the composition comprises less than 5 mM buffer system excluding histidine.

H133. The composition of H51 or any preceding paragraph dependent thereon, wherein the composition comprises 0.5-4.5 mM buffer system excluding histidine.

(continued)

H134. The composition of H51 or any preceding paragraph dependent thereon, wherein the composition comprises 4 mM buffer system excluding histidine.

H135. The composition of H51 or any preceding paragraph dependent thereon, wherein the composition comprises 51-70 mM buffer system excluding histidine.

H136. The composition of H51 or any preceding paragraph dependent thereon, wherein the composition comprises 51-60 mM buffer system excluding histidine.

H137. The composition of H51 or any preceding paragraph dependent thereon, wherein the composition comprises 55 mM buffer system excluding histidine.

H138. The composition of H51 or any preceding paragraph dependent thereon, wherein the composition comprises 1-70 mM histidine buffer system.

H139. The composition of H51 or any preceding paragraph dependent thereon, wherein the composition comprises 10-30 mM histidine buffer system.

H140. The composition of H51 or any preceding paragraph dependent thereon, wherein the composition comprises 20 mM histidine buffer system.

H141. The composition of H51 or any preceding paragraph dependent thereon, wherein the composition comprises 1-9 mM histidine buffer system.

H142. The composition of H51 or any preceding paragraph dependent thereon, wherein the composition comprises 4 mM histidine buffer system.

H143. The composition of H51 or any preceding paragraph dependent thereon, wherein the composition comprises 1-70 mM succinate buffer system.

H144. The composition of H51 or any preceding paragraph dependent thereon, wherein the composition comprises 10-30 mM succinate buffer system.

H145. The composition of H51 or any preceding paragraph dependent thereon, wherein the composition comprises 1-9 mM succinate buffer system.

H146. The composition of H51 or any preceding paragraph dependent thereon, wherein the composition comprises 8 mM succinate buffer system.

H147. The composition of H51 or any preceding paragraph dependent thereon, wherein the composition comprises less than 5 mM succinate buffer system.

H148. The composition of H51 or any preceding paragraph dependent thereon, wherein the composition comprises 0.5-4.5 mM succinate buffer system.

H149. The composition of H51 or any preceding paragraph dependent thereon, wherein the composition comprises 4 mM succinate buffer system.

H150. The composition of H51 or any preceding paragraph dependent thereon, wherein the composition comprises 51-70 mM succinate buffer system.

H151. The composition of H51 or any preceding paragraph dependent thereon, wherein the composition comprises 51-60 mM succinate buffer system.

H152. The composition of H51 or any preceding paragraph dependent thereon, wherein the composition comprises 55 mM succinate buffer system.

H153. The composition of H51 or any preceding paragraph dependent thereon, wherein the composition comprises 1-70 mM acetate buffer system.

H154. The composition of H51 or any preceding paragraph dependent thereon, wherein the composition comprises 10-30 mM acetate buffer system.

H155. The composition of H51 or any preceding paragraph dependent thereon, wherein the composition comprises 1-9 mM acetate buffer system.

H156. The composition of H51 or any preceding paragraph dependent thereon, wherein the composition comprises 8 mM acetate buffer system.

H157. The composition of H51 or any preceding paragraph dependent thereon, wherein the composition comprises less than 5 mM acetate buffer system.

H158. The composition of H51 or any preceding paragraph dependent thereon, wherein the composition comprises 0.5-4.5 mM acetate buffer system.

H159. The composition of H51 or any preceding paragraph dependent thereon, wherein the composition comprises 4 mM acetate buffer system.

(continued)

H160. The composition of H51 or any preceding paragraph dependent thereon, wherein the composition comprises 51-70 mM acetate buffer system.

H161. The composition of H51 or any preceding paragraph dependent thereon, wherein the composition comprises 51-60 mM acetate buffer system.

H162. The composition of H51 or any preceding paragraph dependent thereon, wherein the composition comprises 55 mM acetate buffer system.

H163. The composition of H51 or any preceding paragraph dependent thereon, wherein the composition comprises 1-70 mM adipate buffer system.

H164. The composition of H51 or any preceding paragraph dependent thereon, wherein the composition comprises 10-30 mM adipate buffer system.

H165. The composition of H51 or any preceding paragraph dependent thereon, wherein the composition comprises 25 mM adipate buffer system.

H166. The composition of H51 or any preceding paragraph dependent thereon, wherein the composition comprises 1-9 mM adipate buffer system.

H167. The composition of H51 or any preceding paragraph dependent thereon, wherein the composition comprises 8 mM adipate buffer system.

H168. The composition of H51 or any preceding paragraph dependent thereon, wherein the composition comprises less than 5 mM adipate buffer system.

H169. The composition of H51 or any preceding paragraph dependent thereon, wherein the composition comprises 0.5-4.5 mM adipate buffer system.

H170. The composition of H51 or any preceding paragraph dependent thereon, wherein the composition comprises 4 mM adipate buffer system.

H171. The composition of H51 or any preceding paragraph dependent thereon, wherein the composition comprises 51-70 mM adipate buffer system.

H172. The composition of H51 or any preceding paragraph dependent thereon, wherein the composition comprises 51-60 mM adipate buffer system.

H173. The composition of H51 or any preceding paragraph dependent thereon, wherein the composition comprises 55 mM adipate buffer system.

H174. The composition of H51 or any preceding paragraph dependent thereon, wherein the composition comprises 1-70 mM MES buffer system.

H175. The composition of H51 or any preceding paragraph dependent thereon, wherein the composition comprises 10-30 mM MES buffer system.

H176. The composition of H51 or any preceding paragraph dependent thereon, wherein the composition comprises 1-9 mM MES buffer system.

H177. The composition of H51 or any preceding paragraph dependent thereon, wherein the composition comprises 8 mM MES buffer system.

H178. The composition of H51 or any preceding paragraph dependent thereon, wherein the composition comprises less than 5 mM MES buffer system.

H179. The composition of H51 or any preceding paragraph dependent thereon, wherein the composition comprises 0.5-4.5 mM MES buffer system.

H180. The composition of H51 or any preceding paragraph dependent thereon, wherein the composition comprises 4 mM MES buffer system.

H181. The composition of H51 or any preceding paragraph dependent thereon, wherein the composition comprises 51-70 mM MES buffer system.

H182. The composition of H51 or any preceding paragraph dependent thereon, wherein the composition comprises 51-60 mM MES buffer system.

H183. The composition of H51 or any preceding paragraph dependent thereon, wherein the composition comprises 55 mM MES buffer system.

H184. The composition of H51 or any preceding paragraph dependent thereon, wherein the composition comprises 1-70 mM phosphate buffer system.

H185. The composition of H51 or any preceding paragraph dependent thereon, wherein the composition comprises 10-30 mM phosphate buffer system.

(continued)

H186. The composition of H51 or any preceding paragraph dependent thereon, wherein the composition comprises 1-9 mM phosphate buffer system.

H187. The composition of H51 or any preceding paragraph dependent thereon, wherein the composition comprises 8 mM phosphate buffer system.

H188. The composition of H51 or any preceding paragraph dependent thereon, wherein the composition comprises less than 5 mM phosphate buffer system.

H189. The composition of H51 or any preceding paragraph dependent thereon, wherein the composition comprises 0.5-4.5 mM phosphate buffer system.

H190. The composition of H51 or any preceding paragraph dependent thereon, wherein the composition comprises 4 mM phosphate buffer system.

H191. The composition of H51 or any preceding paragraph dependent thereon, wherein the composition comprises 2 mM phosphate buffer system.

H192. The composition of H51 or any preceding paragraph dependent thereon, wherein the composition comprises 51-70 mM phosphate buffer system.

H193. The composition of H51 or any preceding paragraph dependent thereon, wherein the composition comprises 51-60 mM phosphate buffer system.

H194. The composition of H51 or any preceding paragraph dependent thereon, wherein the composition comprises 55 mM phosphate buffer system.

H195. The composition of H51 or any preceding paragraph dependent thereon, wherein the composition comprises 1-70 mM citrate buffer system.

H196. The composition of H51 or any preceding paragraph dependent thereon, wherein the composition comprises 10-30 mM citrate buffer system.

H197. The composition of H51 or any preceding paragraph dependent thereon, wherein the composition comprises 1-9 mM citrate buffer system.

H198. The composition of H51 or any preceding paragraph dependent thereon, wherein the composition comprises 8 mM citrate buffer system.

H199. The composition of H51 or any preceding paragraph dependent thereon, wherein the composition comprises less than 5 mM citrate buffer system.

H200. The composition of H51 or any preceding paragraph dependent thereon, wherein the composition comprises 0.5-4.5 mM citrate buffer system.

H201. The composition of H51 or any preceding paragraph dependent thereon, wherein the composition comprises 4 mM citrate buffer system.

H202. The composition of H51 or any preceding paragraph dependent thereon, wherein the composition comprises 2 mM citrate buffer system.

H203. The composition of H51 or any preceding paragraph dependent thereon, wherein the composition comprises 51-70 mM citrate buffer system.

H204. The composition of H51 or any preceding paragraph dependent thereon, wherein the composition comprises 51-60 mM citrate buffer system.

H205. The composition of H51 or any preceding paragraph dependent thereon, wherein the composition comprises 55 mM citrate buffer system.

H206. The composition of H51 or any preceding paragraph dependent thereon, wherein the composition comprises 1-9 mM dual-buffer system.

H207. The composition of H51 or any preceding paragraph dependent thereon, wherein the composition comprises 0.5-4.5 mM dual-buffer system.

H208. The composition of H51 or any preceding paragraph dependent thereon, wherein the composition comprises 4 mM dual-buffer system.

H209. The composition of H51 or any preceding paragraph dependent thereon, wherein the composition comprises 1-9 mM dual-buffer system (with a first and second buffer system in molar ratio between 3:1 to 1:3).

H210. The composition of H51 or any preceding paragraph dependent thereon, wherein the composition comprises 0.5-4.5 mM dual-buffer system (with a first and second buffer system in molar ratio between 3:1 to 1:3).

(continued)

H211. The composition of H51 or any preceding paragraph dependent thereon, wherein the composition comprises 4 mM dual-buffer system (with a first and second buffer system in molar ratio between 3:1 to 1:3).

H212. The composition of H51 or any preceding paragraph dependent thereon, wherein the composition comprises 1-70 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H213. The composition of H51 or any preceding paragraph dependent thereon, wherein the composition comprises 10-30 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H214. The composition of H51 or any preceding paragraph dependent thereon, wherein the composition comprises 1-9 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H215. The composition of H51 or any preceding paragraph dependent thereon, wherein the composition comprises 8 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H216. The composition of H51 or any preceding paragraph dependent thereon, wherein the composition comprises less than 5 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H217. The composition of H51 or any preceding paragraph dependent thereon, wherein the composition comprises 0.5-4.5 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H218. The composition of H51 or any preceding paragraph dependent thereon, wherein the composition comprises 4 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H219. The composition of H51 or any preceding paragraph dependent thereon, wherein the composition comprises 51-70 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H220. The composition of H51 or any preceding paragraph dependent thereon, wherein the composition comprises 51-60 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H221. The composition of H51 or any preceding paragraph dependent thereon, wherein the composition comprises 55 mM phosphate-succinate buffer system (e.g. with phosphate/succinate in a molar ratio of 20:1-1:20, more preferably 5:1-1:5, most preferably 1:1, i.e. a 1:1 molar ratio of phosphate buffer system and succinate buffer system).

H222. The composition of H51 or any preceding paragraph dependent thereon, wherein the composition comprises 1-70 mM phosphate-succinate buffer system consisting of a phosphate buffer system and a succinate buffer system in a respective molar ratio of between 20:1 and 1:20.

H223. The composition of H51 or any preceding paragraph dependent thereon, wherein the composition comprises 10-30 mM phosphate-succinate buffer system consisting of a phosphate buffer system and a succinate buffer system in a respective molar ratio of between 20:1 and 1:20.

H224. The composition of H51 or any preceding paragraph dependent thereon, wherein the composition comprises 1-9 mM phosphate-succinate buffer system consisting of a phosphate buffer system and a succinate buffer system in a respective molar ratio of between 20:1 and 1:20.

H225. The composition of H51 or any preceding paragraph dependent thereon, wherein the composition comprises 8 mM phosphate-succinate buffer system consisting of a phosphate buffer system and a succinate buffer system in a respective molar ratio of between 20:1 and 1:20.

(continued)

H226. The composition of H51 or any preceding paragraph dependent thereon, wherein the composition comprises less than 5 mM phosphate-succinate buffer system consisting of a phosphate buffer system and a succinate buffer system in a respective molar ratio of between 20:1 and 1:20.

H227. The composition of H51 or any preceding paragraph dependent thereon, wherein the composition comprises 0.5-4.5 mM phosphate-succinate buffer system consisting of a phosphate buffer system and a succinate buffer system in a respective molar ratio of between 20:1 and 1:20.

H228. The composition of H51 or any preceding paragraph dependent thereon, wherein the composition comprises 4 mM phosphate-succinate buffer system consisting of a phosphate buffer system and a succinate buffer system in a respective molar ratio of between 20:1 and 1:20.

H229. The composition of H51 or any preceding paragraph dependent thereon, wherein the composition comprises 51-70 mM phosphate-succinate buffer system consisting of a phosphate buffer system and a succinate buffer system in a respective molar ratio of between 20:1 and 1:20.

H230. The composition of H51 or any preceding paragraph dependent thereon, wherein the composition comprises 51-60 mM phosphate-succinate buffer system consisting of a phosphate buffer system and a succinate buffer system in a respective molar ratio of between 20:1 and 1:20.

H231. The composition of H51 or any preceding paragraph dependent thereon, wherein the composition comprises 55 mM phosphate-succinate buffer system consisting of a phosphate buffer system and a succinate buffer system in a respective molar ratio of between 20:1 and 1:20.

H232. The composition of H51 or any preceding paragraph dependent thereon, wherein the composition comprises 1-70 mM phosphate-succinate buffer system consisting of a phosphate buffer system and a succinate buffer system in a respective molar ratio of between 5:1 and 1:5.

H233. The composition of H51 or any preceding paragraph dependent thereon, wherein the composition comprises 10-30 mM phosphate-succinate buffer system consisting of a phosphate buffer system and a succinate buffer system in a respective molar ratio of between 5:1 and 1:5.

H234. The composition of H51 or any preceding paragraph dependent thereon, wherein the composition comprises 1-9 mM phosphate-succinate buffer system consisting of a phosphate buffer system and a succinate buffer system in a respective molar ratio of between 5:1 and 1:5.

H235. The composition of H51 or any preceding paragraph dependent thereon, wherein the composition comprises 8 mM phosphate-succinate buffer system consisting of a phosphate buffer system and a succinate buffer system in a respective molar ratio of between 5:1 and 1:5.

H236. The composition of H51 or any preceding paragraph dependent thereon, wherein the composition comprises less than 5 mM phosphate-succinate buffer system consisting of a phosphate buffer system and a succinate buffer system in a respective molar ratio of between 5:1 and 1:5.

H237. The composition of H51 or any preceding paragraph dependent thereon, wherein the composition comprises 0.5-4.5 mM phosphate-succinate buffer system consisting of a phosphate buffer system and a succinate buffer system in a respective molar ratio of between 5:1 and 1:5.

H238. The composition of H51 or any preceding paragraph dependent thereon, wherein the composition comprises 4 mM phosphate-succinate buffer system consisting of a phosphate buffer system and a succinate buffer system in a respective molar ratio of between 5:1 and 1:5.

H239. The composition of H51 or any preceding paragraph dependent thereon, wherein the composition comprises 51-70 mM phosphate-succinate buffer system consisting of a phosphate buffer system and a succinate buffer system in a respective molar ratio of between 5:1 and 1:5.

H240. The composition of H51 or any preceding paragraph dependent thereon, wherein the composition comprises 51-60 mM phosphate-succinate buffer system consisting of a phosphate buffer system and a succinate buffer system in a respective molar ratio of between 5:1 and 1:5.

H241. The composition of H51 or any preceding paragraph dependent thereon, wherein the composition comprises 55 mM phosphate-succinate buffer system consisting of a phosphate buffer system and a succinate buffer system in a respective molar ratio of between 5:1 and 1:5.

H242. The composition of H51 or any preceding paragraph dependent thereon, wherein the composition comprises 1-70 mM phosphate-succinate buffer system consisting of a phosphate buffer system and a succinate buffer system in a respective molar ratio of between 3:1 and 1:3.

H243. The composition of H51 or any preceding paragraph dependent thereon, wherein the composition comprises 10-30 mM phosphate-succinate buffer system consisting of a phosphate buffer system and a succinate buffer system in a respective molar ratio of between 3:1 and 1:3.

(continued)

H244. The composition of H51 or any preceding paragraph dependent thereon, wherein the composition comprises 1-9 mM phosphate-succinate buffer system consisting of a phosphate buffer system and a succinate buffer system in a respective molar ratio of between 3:1 and 1:3.

H245. The composition of H51 or any preceding paragraph dependent thereon, wherein the composition comprises 8 mM phosphate-succinate buffer system consisting of a phosphate buffer system and a succinate buffer system in a respective molar ratio of between 3:1 and 1:3.

H246. The composition of H51 or any preceding paragraph dependent thereon, wherein the composition comprises less than 5 mM phosphate-succinate buffer system consisting of a phosphate buffer system and a succinate buffer system in a respective molar ratio of between 3:1 and 1:3.

H247. The composition of H51 or any preceding paragraph dependent thereon, wherein the composition comprises 0.5-4.5 mM phosphate-succinate buffer system consisting of a phosphate buffer system and a succinate buffer system in a respective molar ratio of between 3:1 and 1:3.

H248. The composition of H51 or any preceding paragraph dependent thereon, wherein the composition comprises 4 mM phosphate-succinate buffer system consisting of a phosphate buffer system and a succinate buffer system in a respective molar ratio of between 3:1 and 1:3.

H249. The composition of H51 or any preceding paragraph dependent thereon, wherein the composition comprises 51-70 mM phosphate-succinate buffer system consisting of a phosphate buffer system and a succinate buffer system in a respective molar ratio of between 3:1 and 1:3.

H250. The composition of H51 or any preceding paragraph dependent thereon, wherein the composition comprises 51-60 mM phosphate-succinate buffer system consisting of a phosphate buffer system and a succinate buffer system in a respective molar ratio of between 3:1 and 1:3.

H251. The composition of H51 or any preceding paragraph dependent thereon, wherein the composition comprises 55 mM phosphate-succinate buffer system consisting of a phosphate buffer system and a succinate buffer system in a respective molar ratio of between 3:1 and 1:3.

H252. The composition of H51 or any preceding paragraph dependent thereon, wherein the composition comprises 1-70 mM phosphate-succinate buffer system consisting of a phosphate buffer system and a succinate buffer system in a respective molar ratio of between 2:1 and 1:2.

H253. The composition of H51 or any preceding paragraph dependent thereon, wherein the composition comprises 10-30 mM phosphate-succinate buffer system consisting of a phosphate buffer system and a succinate buffer system in a respective molar ratio of between 2:1 and 1:2.

H254. The composition of H51 or any preceding paragraph dependent thereon, wherein the composition comprises 1-9 mM phosphate-succinate buffer system consisting of a phosphate buffer system and a succinate buffer system in a respective molar ratio of between 2:1 and 1:2.

H255. The composition of H51 or any preceding paragraph dependent thereon, wherein the composition comprises 8 mM phosphate-succinate buffer system consisting of a phosphate buffer system and a succinate buffer system in a respective molar ratio of between 2:1 and 1:2.

H256. The composition of H51 or any preceding paragraph dependent thereon, wherein the composition comprises less than 5 mM phosphate-succinate buffer system consisting of a phosphate buffer system and a succinate buffer system in a respective molar ratio of between 2:1 and 1:2.

H257. The composition of H51 or any preceding paragraph dependent thereon, wherein the composition comprises 0.5-4.5 mM phosphate-succinate buffer system consisting of a phosphate buffer system and a succinate buffer system in a respective molar ratio of between 2:1 and 1:2.

H258. The composition of H51 or any preceding paragraph dependent thereon, wherein the composition comprises 4 mM phosphate-succinate buffer system consisting of a phosphate buffer system and a succinate buffer system in a respective molar ratio of between 2:1 and 1:2.

H259. The composition of H51 or any preceding paragraph dependent thereon, wherein the composition comprises 51-70 mM phosphate-succinate buffer system consisting of a phosphate buffer system and a succinate buffer system in a respective molar ratio of between 2:1 and 1:2.

H260. The composition of H51 or any preceding paragraph dependent thereon, wherein the composition comprises 51-60 mM phosphate-succinate buffer system consisting of a phosphate buffer system and a succinate buffer system in a respective molar ratio of between 2:1 and 1:2.

H261. The composition of H51 or any preceding paragraph dependent thereon, wherein the composition comprises 55 mM phosphate-succinate buffer system consisting of a phosphate buffer system and a succinate buffer system in a respective molar ratio of between 2:1 and 1:2.

(continued)

H262. The composition of H51 or any preceding paragraph dependent thereon, wherein the composition comprises 1-70 mM phosphate-succinate buffer system consisting of a phosphate buffer system and a succinate buffer system in a respective molar ratio of 1:1.

H263. The composition of H51 or any preceding paragraph dependent thereon, wherein the composition comprises 10-30 mM phosphate-succinate buffer system consisting of a phosphate buffer system and a succinate buffer system in a respective molar ratio of 1:1.

H264. The composition of H51 or any preceding paragraph dependent thereon, wherein the composition comprises 1-9 mM phosphate-succinate buffer system consisting of a phosphate buffer system and a succinate buffer system in a respective molar ratio of 1:1.

H265. The composition of H51 or any preceding paragraph dependent thereon, wherein the composition comprises 8 mM phosphate-succinate buffer system consisting of a phosphate buffer system and a succinate buffer system in a respective molar ratio of 1:1.

H266. The composition of H51 or any preceding paragraph dependent thereon, wherein the composition comprises less than 5 mM phosphate-succinate buffer system consisting of a phosphate buffer system and a succinate buffer system in a respective molar ratio of 1:1.

H267. The composition of H51 or any preceding paragraph dependent thereon, wherein the composition comprises 0.5-4.5 mM phosphate-succinate buffer system consisting of a phosphate buffer system and a succinate buffer system in a respective molar ratio of 1:1.

H268. The composition of H51 or any preceding paragraph dependent thereon, wherein the composition comprises 4 mM phosphate-succinate buffer system consisting of a phosphate buffer system and a succinate buffer system in a respective molar ratio of 1:1.

H269. The composition of H51 or any preceding paragraph dependent thereon, wherein the composition comprises 51-70 mM phosphate-succinate buffer system consisting of a phosphate buffer system and a succinate buffer system in a respective molar ratio of 1:1.

H270. The composition of H51 or any preceding paragraph dependent thereon, wherein the composition comprises 51-60 mM phosphate-succinate buffer system consisting of a phosphate buffer system and a succinate buffer system in a respective molar ratio of 1:1.

H271. The composition of H51 or any preceding paragraph dependent thereon, wherein the composition comprises 55 mM phosphate-succinate buffer system consisting of a phosphate buffer system and a succinate buffer system in a respective molar ratio of 1:1.

H272. The composition of H51 or any preceding paragraph dependent thereon, wherein the composition comprises 1-70 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H273. The composition of H51 or any preceding paragraph dependent thereon, wherein the composition comprises 10-30 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H274. The composition of H51 or any preceding paragraph dependent thereon, wherein the composition comprises 1-9 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H275. The composition of H51 or any preceding paragraph dependent thereon, wherein the composition comprises 8 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H276. The composition of H51 or any preceding paragraph dependent thereon, wherein the composition comprises less than 5 mM phosphate-acetate buffer system (e.g. with phosphate/acetate in a molar ratio of 20:1-1 :20, more preferably 5:1-1:5, most preferably 1:1, i.e. a 1:1 molar ratio of phosphate buffer system and acetate buffer system).

H277. The composition of H51 or any preceding paragraph dependent thereon, wherein the composition comprises 0.5-4.5 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

(continued)

H278. The composition of H51 or any preceding paragraph dependent thereon, wherein the composition comprises 4 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H279. The composition of H51 or any preceding paragraph dependent thereon, wherein the composition comprises 51-70 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H280. The composition of H51 or any preceding paragraph dependent thereon, wherein the composition comprises 51-60 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H281. The composition of H51 or any preceding paragraph dependent thereon, wherein the composition comprises 55 mM phosphate-acetate buffer system (e.g. with phosphate/acetate in a molar ratio of 20:1-1:20, more preferably 5:1-1:5, most preferably 1:1, i.e. a 1:1 molar ratio of phosphate buffer system and acetate buffer system).

H282. The composition of H51 or any preceding paragraph dependent thereon, wherein the composition comprises 1-70 mM phosphate-acetate buffer system consisting of a phosphate buffer system and a acetate buffer system in a respective molar ratio of between 20:1 and 1:20.

H283. The composition of H51 or any preceding paragraph dependent thereon, wherein the composition comprises 10-30 mM phosphate-acetate buffer system consisting of a phosphate buffer system and a acetate buffer system in a respective molar ratio of between 20:1 and 1:20.

H284. The composition of H51 or any preceding paragraph dependent thereon, wherein the composition comprises 1-9 mM phosphate-acetate buffer system consisting of a phosphate buffer system and a acetate buffer system in a respective molar ratio of between 20:1 and 1:20.

H285. The composition of H51 or any preceding paragraph dependent thereon, wherein the composition comprises 8 mM phosphate-acetate buffer system consisting of a phosphate buffer system and a acetate buffer system in a respective molar ratio of between 20:1 and 1:20.

H286. The composition of H51 or any preceding paragraph dependent thereon, wherein the composition comprises less than 5 mM phosphate-acetate buffer system consisting of a phosphate buffer system and a acetate buffer system in a respective molar ratio of between 20:1 and 1:20.

H287. The composition of H51 or any preceding paragraph dependent thereon, wherein the composition comprises 0.5-4.5 mM phosphate-acetate buffer system consisting of a phosphate buffer system and a acetate buffer system in a respective molar ratio of between 20:1 and 1:20.

H288. The composition of H51 or any preceding paragraph dependent thereon, wherein the composition comprises 4 mM phosphate-acetate buffer system consisting of a phosphate buffer system and a acetate buffer system in a respective molar ratio of between 20:1 and 1:20.

H289. The composition of H51 or any preceding paragraph dependent thereon, wherein the composition comprises 51-70 mM phosphate-acetate buffer system consisting of a phosphate buffer system and a acetate buffer system in a respective molar ratio of between 20:1 and 1:20.

H290. The composition of H51 or any preceding paragraph dependent thereon, wherein the composition comprises 51-60 mM phosphate-acetate buffer system consisting of a phosphate buffer system and a acetate buffer system in a respective molar ratio of between 20:1 and 1:20.

H291. The composition of H51 or any preceding paragraph dependent thereon, wherein the composition comprises 55 mM phosphate-acetate buffer system consisting of a phosphate buffer system and a acetate buffer system in a respective molar ratio of between 20:1 and 1:20.

H292. The composition of H51 or any preceding paragraph dependent thereon, wherein the composition comprises 1-70 mM phosphate-acetate buffer system consisting of a phosphate buffer system and a acetate buffer system in a respective molar ratio of between 5:1 and 1:5.

H293. The composition of H51 or any preceding paragraph dependent thereon, wherein the composition comprises 10-30 mM phosphate-acetate buffer system consisting of a phosphate buffer system and a acetate buffer system in a respective molar ratio of between 5:1 and 1:5.

(continued)

H294. The composition of H51 or any preceding paragraph dependent thereon, wherein the composition comprises 1-9 mM phosphate-acetate buffer system consisting of a phosphate buffer system and a acetate buffer system in a respective molar ratio of between 5:1 and 1:5.

H295. The composition of H51 or any preceding paragraph dependent thereon, wherein the composition comprises 8 mM phosphate-acetate buffer system consisting of a phosphate buffer system and a acetate buffer system in a respective molar ratio of between 5:1 and 1:5.

H296. The composition of H51 or any preceding paragraph dependent thereon, wherein the composition comprises less than 5 mM phosphate-acetate buffer system consisting of a phosphate buffer system and a acetate buffer system in a respective molar ratio of between 5:1 and 1:5.

H297. The composition of H51 or any preceding paragraph dependent thereon, wherein the composition comprises 0.5-4.5 mM phosphate-acetate buffer system consisting of a phosphate buffer system and a acetate buffer system in a respective molar ratio of between 5:1 and 1:5.

H298. The composition of H51 or any preceding paragraph dependent thereon, wherein the composition comprises 4 mM phosphate-acetate buffer system consisting of a phosphate buffer system and a acetate buffer system in a respective molar ratio of between 5:1 and 1:5.

H299. The composition of H51 or any preceding paragraph dependent thereon, wherein the composition comprises 51-70 mM phosphate-acetate buffer system consisting of a phosphate buffer system and a acetate buffer system in a respective molar ratio of between 5:1 and 1:5.

H300. The composition of H51 or any preceding paragraph dependent thereon, wherein the composition comprises 51-60 mM phosphate-acetate buffer system consisting of a phosphate buffer system and a acetate buffer system in a respective molar ratio of between 5:1 and 1:5.

H301. The composition of H51 or any preceding paragraph dependent thereon, wherein the composition comprises 55 mM phosphate-acetate buffer system consisting of a phosphate buffer system and a acetate buffer system in a respective molar ratio of between 5:1 and 1:5.

H302. The composition of H51 or any preceding paragraph dependent thereon, wherein the composition comprises 1-70 mM phosphate-acetate buffer system consisting of a phosphate buffer system and a acetate buffer system in a respective molar ratio of between 3:1 and 1:3.

H303. The composition of H51 or any preceding paragraph dependent thereon, wherein the composition comprises 10-30 mM phosphate-acetate buffer system consisting of a phosphate buffer system and a acetate buffer system in a respective molar ratio of between 3:1 and 1:3.

H304. The composition of H51 or any preceding paragraph dependent thereon, wherein the composition comprises 1-9 mM phosphate-acetate buffer system consisting of a phosphate buffer system and a acetate buffer system in a respective molar ratio of between 3:1 and 1:3.

H305. The composition of H51 or any preceding paragraph dependent thereon, wherein the composition comprises 8 mM phosphate-acetate buffer system consisting of a phosphate buffer system and a acetate buffer system in a respective molar ratio of between 3:1 and 1:3.

H306. The composition of H51 or any preceding paragraph dependent thereon, wherein the composition comprises less than 5 mM phosphate-acetate buffer system consisting of a phosphate buffer system and a acetate buffer system in a respective molar ratio of between 3:1 and 1:3.

H307. The composition of H51 or any preceding paragraph dependent thereon, wherein the composition comprises 0.5-4.5 mM phosphate-acetate buffer system consisting of a phosphate buffer system and a acetate buffer system in a respective molar ratio of between 3:1 and 1:3.

H308. The composition of H51 or any preceding paragraph dependent thereon, wherein the composition comprises 4 mM phosphate-acetate buffer system consisting of a phosphate buffer system and a acetate buffer system in a respective molar ratio of between 3:1 and 1:3.

H309. The composition of H51 or any preceding paragraph dependent thereon, wherein the composition comprises 51-70 mM phosphate-acetate buffer system consisting of a phosphate buffer system and a acetate buffer system in a respective molar ratio of between 3:1 and 1:3.

H310. The composition of H51 or any preceding paragraph dependent thereon, wherein the composition comprises 51-60 mM phosphate-acetate buffer system consisting of a phosphate buffer system and a acetate buffer system in a respective molar ratio of between 3:1 and 1:3.

H311. The composition of H51 or any preceding paragraph dependent thereon, wherein the composition comprises 55 mM phosphate-acetate buffer system consisting of a phosphate buffer system and a acetate buffer system in a respective molar ratio of between 3:1 and 1:3.

(continued)

H312. The composition of H51 or any preceding paragraph dependent thereon, wherein the composition comprises 1-70 mM phosphate-acetate buffer system consisting of a phosphate buffer system and a acetate buffer system in a respective molar ratio of between 2:1 and 1:2.

H313. The composition of H51 or any preceding paragraph dependent thereon, wherein the composition comprises 10-30 mM phosphate-acetate buffer system consisting of a phosphate buffer system and a acetate buffer system in a respective molar ratio of between 2:1 and 1:2.

H314. The composition of H51 or any preceding paragraph dependent thereon, wherein the composition comprises 1-9 mM phosphate-acetate buffer system consisting of a phosphate buffer system and a acetate buffer system in a respective molar ratio of between 2:1 and 1:2.

H315. The composition of H51 or any preceding paragraph dependent thereon, wherein the composition comprises 8 mM phosphate-acetate buffer system consisting of a phosphate buffer system and a acetate buffer system in a respective molar ratio of between 2:1 and 1:2.

H316. The composition of H51 or any preceding paragraph dependent thereon, wherein the composition comprises less than 5 mM phosphate-acetate buffer system consisting of a phosphate buffer system and a acetate buffer system in a respective molar ratio of between 2:1 and 1:2.

H317. The composition of H51 or any preceding paragraph dependent thereon, wherein the composition comprises 0.5-4.5 mM phosphate-acetate buffer system consisting of a phosphate buffer system and a acetate buffer system in a respective molar ratio of between 2:1 and 1:2.

H318. The composition of H51 or any preceding paragraph dependent thereon, wherein the composition comprises 4 mM phosphate-acetate buffer system consisting of a phosphate buffer system and a acetate buffer system in a respective molar ratio of between 2:1 and 1:2.

H319. The composition of H51 or any preceding paragraph dependent thereon, wherein the composition comprises 51-70 mM phosphate-acetate buffer system consisting of a phosphate buffer system and a acetate buffer system in a respective molar ratio of between 2:1 and 1:2.

H320. The composition of H51 or any preceding paragraph dependent thereon, wherein the composition comprises 51-60 mM phosphate-acetate buffer system consisting of a phosphate buffer system and a acetate buffer system in a respective molar ratio of between 2:1 and 1:2.

H321. The composition of H51 or any preceding paragraph dependent thereon, wherein the composition comprises 55 mM phosphate-acetate buffer system consisting of a phosphate buffer system and a acetate buffer system in a respective molar ratio of between 2:1 and 1:2.

H322. The composition of H51 or any preceding paragraph dependent thereon, wherein the composition comprises 1-70 mM phosphate-acetate buffer system consisting of a phosphate buffer system and a acetate buffer system in a respective molar ratio of 1:1.

H323. The composition of H51 or any preceding paragraph dependent thereon, wherein the composition comprises 10-30 mM phosphate-acetate buffer system consisting of a phosphate buffer system and a acetate buffer system in a respective molar ratio of 1:1.

H324. The composition of H51 or any preceding paragraph dependent thereon, wherein the composition comprises 1-9 mM phosphate-acetate buffer system consisting of a phosphate buffer system and a acetate buffer system in a respective molar ratio of 1:1.

H325. The composition of H51 or any preceding paragraph dependent thereon, wherein the composition comprises 8 mM phosphate-acetate buffer system consisting of a phosphate buffer system and a acetate buffer system in a respective molar ratio of 1:1.

H326. The composition of H51 or any preceding paragraph dependent thereon, wherein the composition comprises less than 5 mM phosphate-acetate buffer system consisting of a phosphate buffer system and a acetate buffer system in a respective molar ratio of 1:1.

H327. The composition of H51 or any preceding paragraph dependent thereon, wherein the composition comprises 0.5-4.5 mM phosphate-acetate buffer system consisting of a phosphate buffer system and a acetate buffer system in a respective molar ratio of 1:1.

H328. The composition of H51 or any preceding paragraph dependent thereon, wherein the composition comprises 4 mM phosphate-acetate buffer system consisting of a phosphate buffer system and a acetate buffer system in a respective molar ratio of 1:1.

H329. The composition of H51 or any preceding paragraph dependent thereon, wherein the composition comprises 51-70 mM phosphate-acetate buffer system consisting of a phosphate buffer system and a acetate buffer system in a respective molar ratio of 1:1.

(continued)

H330. The composition of H51 or any preceding paragraph dependent thereon, wherein the composition comprises 51-60 mM phosphate-acetate buffer system consisting of a phosphate buffer system and a acetate buffer system in a respective molar ratio of 1:1.

H331. The composition of H51 or any preceding paragraph dependent thereon, wherein the composition comprises 55 mM phosphate-acetate buffer system consisting of a phosphate buffer system and a acetate buffer system in a respective molar ratio of 1:1.

H332. The composition of H51 or any preceding paragraph dependent thereon, wherein the composition comprises 1-70 mM phosphate-citrate buffer system (e.g. with phosphate/citrate in a molar ratio of 20: 1-1:20, more preferably 5:1-1:5, most preferably 1:1, i.e. a 1:1 molar ratio of phosphate buffer system and citrate buffer system).

H333. The composition of H51 or any preceding paragraph dependent thereon, wherein the composition comprises 10-30 mM phosphate-citrate buffer system (e.g. with phosphate/citrate in a molar ratio of 20: 1-1:20, more preferably 5:1-1:5, most preferably 1:1, i.e. a 1:1 molar ratio of phosphate buffer system and citrate buffer system).

H334. The composition of H51 or any preceding paragraph dependent thereon, wherein the composition comprises 1-9 mM phosphate-citrate buffer system (e.g. with phosphate/citrate in a molar ratio of 20:1-1: 20, more preferably 5:1-1:5, most preferably 1:1, i.e. a 1:1 molar ratio of phosphate buffer system and citrate buffer system).

H335. The composition of H51 or any preceding paragraph dependent thereon, wherein the composition comprises 8 mM phosphate-citrate buffer system (e.g. with phosphate/citrate in a molar ratio of 20:1-1: 20, more preferably 5:1-1:5, most preferably 1:1, i.e. a 1:1 molar ratio of phosphate buffer system and citrate buffer system).

H336. The composition of H51 or any preceding paragraph dependent thereon, wherein the composition comprises less than 5 mM phosphate-citrate buffer system (e.g. with phosphate/citrate in a molar ratio of 20:1-1:20, more preferably 5:1-1:5, most preferably 1:1, i.e. a 1:1 molar ratio of phosphate buffer system and citrate buffer system).

H337. The composition of H51 or any preceding paragraph dependent thereon, wherein the composition comprises 0.5-4.5 mM phosphate-citrate buffer system (e.g. with phosphate/citrate in a molar ratio of 20: 1-1:20, more preferably 5:1-1:5, most preferably 1:1, i.e. a 1:1 molar ratio of phosphate buffer system and citrate buffer system).

H338. The composition of H51 or any preceding paragraph dependent thereon, wherein the composition comprises 4 mM phosphate-citrate buffer system (e.g. with phosphate/citrate in a molar ratio of 20:1-1: 20, more preferably 5:1-1:5, most preferably 1:1, i.e. a 1:1 molar ratio of phosphate buffer system and citrate buffer system).

H339. The composition of H51 or any preceding paragraph dependent thereon, wherein the composition comprises 51-70 mM phosphate-citrate buffer system (e.g. with phosphate/citrate in a molar ratio of 20: 1-1:20, more preferably 5:1-1:5, most preferably 1:1, i.e. a 1:1 molar ratio of phosphate buffer system and citrate buffer system).

H340. The composition of H51 or any preceding paragraph dependent thereon, wherein the composition comprises 51-60 mM phosphate-citrate buffer system (e.g. with phosphate/citrate in a molar ratio of 20: 1-1:20, more preferably 5:1-1:5, most preferably 1:1, i.e. a 1:1 molar ratio of phosphate buffer system and citrate buffer system).

H341. The composition of H51 or any preceding paragraph dependent thereon, wherein the composition comprises 55 mM phosphate-citrate buffer system (e.g. with phosphate/citrate in a molar ratio of 20:1-1: 20, more preferably 5:1-1:5, most preferably 1:1, i.e. a 1:1 molar ratio of phosphate buffer system and citrate buffer system).

H342. The composition of H51 or any preceding paragraph dependent thereon, wherein the composition comprises 1-70 mM phosphate-citrate buffer system consisting of a phosphate buffer system and a citrate buffer system in a respective molar ratio of between 20:1 and 1:20.

H343. The composition of H51 or any preceding paragraph dependent thereon, wherein the composition comprises 10-30 mM phosphate-citrate buffer system consisting of a phosphate buffer system and a citrate buffer system in a respective molar ratio of between 20:1 and 1:20.

(continued)

H344. The composition of H51 or any preceding paragraph dependent thereon, wherein the composition comprises 1-9 mM phosphate-citrate buffer system consisting of a phosphate buffer system and a citrate buffer system in a respective molar ratio of between 20:1 and 1:20.

H345. The composition of H51 or any preceding paragraph dependent thereon, wherein the composition comprises 8 mM phosphate-citrate buffer system consisting of a phosphate buffer system and a citrate buffer system in a respective molar ratio of between 20:1 and 1:20.

H346. The composition of H51 or any preceding paragraph dependent thereon, wherein the composition comprises less than 5 mM phosphate-citrate buffer system consisting of a phosphate buffer system and a citrate buffer system in a respective molar ratio of between 20:1 and 1:20.

H347. The composition of H51 or any preceding paragraph dependent thereon, wherein the composition comprises 0.5-4.5 mM phosphate-citrate buffer system consisting of a phosphate buffer system and a citrate buffer system in a respective molar ratio of between 20:1 and 1:20.

H348. The composition of H51 or any preceding paragraph dependent thereon, wherein the composition comprises 4 mM phosphate-citrate buffer system consisting of a phosphate buffer system and a citrate buffer system in a respective molar ratio of between 20:1 and 1:20.

H349. The composition of H51 or any preceding paragraph dependent thereon, wherein the composition comprises 51-70 mM phosphate-citrate buffer system consisting of a phosphate buffer system and a citrate buffer system in a respective molar ratio of between 20:1 and 1:20.

H350. The composition of H51 or any preceding paragraph dependent thereon, wherein the composition comprises 51-60 mM phosphate-citrate buffer system consisting of a phosphate buffer system and a citrate buffer system in a respective molar ratio of between 20:1 and 1:20.

H351. The composition of H51 or any preceding paragraph dependent thereon, wherein the composition comprises 55 mM phosphate-citrate buffer system consisting of a phosphate buffer system and a citrate buffer system in a respective molar ratio of between 20:1 and 1:20.

H352. The composition of H51 or any preceding paragraph dependent thereon, wherein the composition comprises 1-70 mM phosphate-citrate buffer system consisting of a phosphate buffer system and a citrate buffer system in a respective molar ratio of between 5:1 and 1:5.

H353. The composition of H51 or any preceding paragraph dependent thereon, wherein the composition comprises 10-30 mM phosphate-citrate buffer system consisting of a phosphate buffer system and a citrate buffer system in a respective molar ratio of between 5:1 and 1:5.

H354. The composition of H51 or any preceding paragraph dependent thereon, wherein the composition comprises 1-9 mM phosphate-citrate buffer system consisting of a phosphate buffer system and a citrate buffer system in a respective molar ratio of between 5:1 and 1:5.

H355. The composition of H51 or any preceding paragraph dependent thereon, wherein the composition comprises 8 mM phosphate-citrate buffer system consisting of a phosphate buffer system and a citrate buffer system in a respective molar ratio of between 5:1 and 1:5.

H356. The composition of H51 or any preceding paragraph dependent thereon, wherein the composition comprises less than 5 mM phosphate-citrate buffer system consisting of a phosphate buffer system and a citrate buffer system in a respective molar ratio of between 5:1 and 1:5.

H357. The composition of H51 or any preceding paragraph dependent thereon, wherein the composition comprises 0.5-4.5 mM phosphate-citrate buffer system consisting of a phosphate buffer system and a citrate buffer system in a respective molar ratio of between 5:1 and 1:5.

H358. The composition of H51 or any preceding paragraph dependent thereon, wherein the composition comprises 4 mM phosphate-citrate buffer system consisting of a phosphate buffer system and a citrate buffer system in a respective molar ratio of between 5:1 and 1:5.

H359. The composition of H51 or any preceding paragraph dependent thereon, wherein the composition comprises 51-70 mM phosphate-citrate buffer system consisting of a phosphate buffer system and a citrate buffer system in a respective molar ratio of between 5:1 and 1:5.

H360. The composition of H51 or any preceding paragraph dependent thereon, wherein the composition comprises 51-60 mM phosphate-citrate buffer system consisting of a phosphate buffer system and a citrate buffer system in a respective molar ratio of between 5:1 and 1:5.

H361. The composition of H51 or any preceding paragraph dependent thereon, wherein the composition comprises 55 mM phosphate-citrate buffer system consisting of a phosphate buffer system and a citrate buffer system in a respective molar ratio of between 5:1 and 1:5.

(continued)

H362. The composition of H51 or any preceding paragraph dependent thereon, wherein the composition comprises 1-70 mM phosphate-citrate buffer system consisting of a phosphate buffer system and a citrate buffer system in a respective molar ratio of between 3:1 and 1:3.

H363. The composition of H51 or any preceding paragraph dependent thereon, wherein the composition comprises 10-30 mM phosphate-citrate buffer system consisting of a phosphate buffer system and a citrate buffer system in a respective molar ratio of between 3:1 and 1:3.

H364. The composition of H51 or any preceding paragraph dependent thereon, wherein the composition comprises 1-9 mM phosphate-citrate buffer system consisting of a phosphate buffer system and a citrate buffer system in a respective molar ratio of between 3:1 and 1:3.

H365. The composition of H51 or any preceding paragraph dependent thereon, wherein the composition comprises 8 mM phosphate-citrate buffer system consisting of a phosphate buffer system and a citrate buffer system in a respective molar ratio of between 3:1 and 1:3.

H366. The composition of H51 or any preceding paragraph dependent thereon, wherein the composition comprises less than 5 mM phosphate-citrate buffer system consisting of a phosphate buffer system and a citrate buffer system in a respective molar ratio of between 3:1 and 1:3.

H367. The composition of H51 or any preceding paragraph dependent thereon, wherein the composition comprises 0.5-4.5 mM phosphate-citrate buffer system consisting of a phosphate buffer system and a citrate buffer system in a respective molar ratio of between 3:1 and 1:3.

H368. The composition of H51 or any preceding paragraph dependent thereon, wherein the composition comprises 4 mM phosphate-citrate buffer system consisting of a phosphate buffer system and a citrate buffer system in a respective molar ratio of between 3:1 and 1:3.

H369. The composition of H51 or any preceding paragraph dependent thereon, wherein the composition comprises 51-70 mM phosphate-citrate buffer system consisting of a phosphate buffer system and a citrate buffer system in a respective molar ratio of between 3:1 and 1:3.

H370. The composition of H51 or any preceding paragraph dependent thereon, wherein the composition comprises 51-60 mM phosphate-citrate buffer system consisting of a phosphate buffer system and a citrate buffer system in a respective molar ratio of between 3:1 and 1:3.

H371. The composition of H51 or any preceding paragraph dependent thereon, wherein the composition comprises 55 mM phosphate-citrate buffer system consisting of a phosphate buffer system and a citrate buffer system in a respective molar ratio of between 3:1 and 1:3.

H372. The composition of H51 or any preceding paragraph dependent thereon, wherein the composition comprises 1-70 mM phosphate-citrate buffer system consisting of a phosphate buffer system and a citrate buffer system in a respective molar ratio of between 2:1 and 1:2.

H373. The composition of H51 or any preceding paragraph dependent thereon, wherein the composition comprises 10-30 mM phosphate-citrate buffer system consisting of a phosphate buffer system and a citrate buffer system in a respective molar ratio of between 2:1 and 1:2.

H374. The composition of H51 or any preceding paragraph dependent thereon, wherein the composition comprises 1-9 mM phosphate-citrate buffer system consisting of a phosphate buffer system and a citrate buffer system in a respective molar ratio of between 2:1 and 1:2.

H375. The composition of H51 or any preceding paragraph dependent thereon, wherein the composition comprises 8 mM phosphate-citrate buffer system consisting of a phosphate buffer system and a citrate buffer system in a respective molar ratio of between 2:1 and 1:2.

H376. The composition of H51 or any preceding paragraph dependent thereon, wherein the composition comprises less than 5 mM phosphate-citrate buffer system consisting of a phosphate buffer system and a citrate buffer system in a respective molar ratio of between 2:1 and 1:2.

H377. The composition of H51 or any preceding paragraph dependent thereon, wherein the composition comprises 0.5-4.5 mM phosphate-citrate buffer system consisting of a phosphate buffer system and a citrate buffer system in a respective molar ratio of between 2:1 and 1:2.

H378. The composition of H51 or any preceding paragraph dependent thereon, wherein the composition comprises 4 mM phosphate-citrate buffer system consisting of a phosphate buffer system and a citrate buffer system in a respective molar ratio of between 2:1 and 1:2.

H379. The composition of H51 or any preceding paragraph dependent thereon, wherein the composition comprises 51-70 mM phosphate-citrate buffer system consisting of a phosphate buffer system and a citrate buffer system in a respective molar ratio of between 2:1 and 1:2.

(continued)

H380. The composition of H51 or any preceding paragraph dependent thereon, wherein the composition comprises 51-60 mM phosphate-citrate buffer system consisting of a phosphate buffer system and a citrate buffer system in a respective molar ratio of between 2:1 and 1:2.

H381. The composition of H51 or any preceding paragraph dependent thereon, wherein the composition comprises 55 mM phosphate-citrate buffer system consisting of a phosphate buffer system and a citrate buffer system in a respective molar ratio of between 2:1 and 1:2.

H382. The composition of H51 or any preceding paragraph dependent thereon, wherein the composition comprises 1-70 mM phosphate-citrate buffer system consisting of a phosphate buffer system and a citrate buffer system in a respective molar ratio of 1:1.

H383. The composition of H51 or any preceding paragraph dependent thereon, wherein the composition comprises 10-30 mM phosphate-citrate buffer system consisting of a phosphate buffer system and a citrate buffer system in a respective molar ratio of 1:1.

H384. The composition of H51 or any preceding paragraph dependent thereon, wherein the composition comprises 1-9 mM phosphate-citrate buffer system consisting of a phosphate buffer system and a citrate buffer system in a respective molar ratio of 1:1.

H385. The composition of H51 or any preceding paragraph dependent thereon, wherein the composition comprises 8 mM phosphate-citrate buffer system consisting of a phosphate buffer system and a citrate buffer system in a respective molar ratio of 1:1.

H386. The composition of H51 or any preceding paragraph dependent thereon, wherein the composition comprises less than 5 mM phosphate-citrate buffer system consisting of a phosphate buffer system and a citrate buffer system in a respective molar ratio of 1:1.

H387. The composition of H51 or any preceding paragraph dependent thereon, wherein the composition comprises 0.5-4.5 mM phosphate-citrate buffer system consisting of a phosphate buffer system and a citrate buffer system in a respective molar ratio of 1:1.

H388. The composition of H51 or any preceding paragraph dependent thereon, wherein the composition comprises 4 mM phosphate-citrate buffer system consisting of a phosphate buffer system and a citrate buffer system in a respective molar ratio of 1:1.

H389. The composition of H51 or any preceding paragraph dependent thereon, wherein the composition comprises 51-70 mM phosphate-citrate buffer system consisting of a phosphate buffer system and a citrate buffer system in a respective molar ratio of 1:1.

H390. The composition of H51 or any preceding paragraph dependent thereon, wherein the composition comprises 51-60 mM phosphate-citrate buffer system consisting of a phosphate buffer system and a citrate buffer system in a respective molar ratio of 1:1.

H391. The composition of H51 or any preceding paragraph dependent thereon, wherein the composition comprises 55 mM phosphate-citrate buffer system consisting of a phosphate buffer system and a citrate buffer system in a respective molar ratio of 1:1.

H392. The composition of H1-H391 or any preceding paragraph dependent thereon, wherein the composition is characterised by an absence of a histidine buffer system.

H393. The composition of H1-H51, wherein the composition is characterised by an absence of a buffer system.

H394. The composition of H1-H391, wherein the composition is characterised by an absence of any, some, or all buffer systems defined in H52-H108 .

H395. The composition of H1-H394, wherein the composition has a pH of pH 4-8.

H396. The composition of H1-H394, wherein the composition has a pH of pH 4.5-7.

H397. The composition of H1-H394, wherein the composition has a pH of pH 4.5-6.5.

H398. The composition of H1-H394, wherein the composition has a pH of pH 5-6.5.

H399. The composition of H1-H394, wherein the composition has a pH of pH 4-5.1.

H400. The composition of H1-H394, wherein the composition has a pH of pH 6.3-7.

H401. The composition of H1-H394, wherein the composition has a pH of pH 5.2-6.2.

H402. The composition of H1-H394, wherein the composition has a pH of pH 4.4-4.8.

H403. The composition of H1-H394, wherein the composition has a pH of pH 4.5-4.7.

H404. The composition of H1-H394, wherein the composition has a pH of pH 4.6.

H405. The composition of H1-H394, wherein the composition has a pH of pH 4.5-5.5.

H406. The composition of H1-H394, wherein the composition has a pH of pH 4.8-5.2.

(continued)

H407. The composition of H1-H394, wherein the composition has a pH of pH 4.9-5.1.
H408. The composition of H1-H394, wherein the composition has a pH of pH 5.0.
H409. The composition of H1-H394, wherein the composition has a pH of pH 5.1.
H410. The composition of H1-H394, wherein the composition has a pH of pH 5.6-6.2.
H411. The composition of H1-H394, wherein the composition has a pH of pH 5.6-6.0.
H412. The composition of H1-H394, wherein the composition has a pH of pH 5.6.
H413. The composition of H1-H394, wherein the composition has a pH of pH 5.7.
H414. The composition of H1-H394, wherein the composition has a pH of pH 5.7-5.9.
H415. The composition of H1-H394, wherein the composition has a pH of pH 5.8.
H416. The composition of H1-H394, wherein the composition has a pH of pH 5.6-6.4.
H417. The composition of H1-H394, wherein the composition has a pH of pH 5.8-6.2.
H418. The composition of H1-H394, wherein the composition has a pH of pH 5.9-6.1.
H419. The composition of H1-H394, wherein the composition has a pH of pH 6.0.
H420. The composition of H1-H394, wherein the composition has a pH of pH 5.9-6.6.
H421. The composition of H1-H394, wherein the composition has a pH of pH 6.0-6.5.
H422. The composition of H1-H394, wherein the composition has a pH of pH 6.1.
H423. The composition of H1-H394, wherein the composition has a pH of pH 6.2-6.4.
H424. The composition of H1-H394, wherein the composition has a pH of pH 6.3.
H425. The composition of H1-H394, wherein the composition has a pH of pH 6.2.
H426. The composition of H1-H394, wherein the composition has a pH of pH 5.0-6.0.
H427. The composition of H1-H394, wherein the composition has a pH of pH 5.6-6.0.
H428. The composition of H1-H394, wherein the composition has a pH of pH 5.7-5.9.
H429. The composition of H1-H394, wherein the composition has a pH of pH 5.8.
H430. The composition of H1-H394, wherein the composition has a pH of pH 6.3-6.7.
H431. The composition of H1-H394, wherein the composition has a pH of pH 6.5.
H432. The composition of H1-H431, wherein the composition comprises a sugar component.
H433. The composition of H432, wherein the composition comprises (and/or the sugar component is) a sugar component that stabilizes the biopharmaceutical active, especially in an aqueous solution.
H434. The composition of H432 or any preceding paragraph dependent thereon, wherein the composition comprises (and/or the sugar component is) a sugar component that facilitates maintainance of the structural integrity of the biopharmaceutical active, particularly during freezing and/or lyophilization and/or storage (especially when exposed to stress).
H435. The composition of H432 or any preceding paragraph dependent thereon, wherein the composition comprises (and/or the sugar component is) a sugar component that acts as a lyoprotectant.
H436. The composition of H432 or any preceding paragraph dependent thereon, wherein the composition comprises (and/or the sugar component is) a single sugar component.
H437. The composition of H432 or any preceding paragraph dependent thereon, wherein the composition comprises (and/or the sugar component is) a single sugar component consisting of a single compound.
H438. The composition of H432 or any preceding paragraph dependent thereon, wherein the composition comprises (and/or the sugar component is) a multi-sugar component.
H439. The composition of H432 or any preceding paragraph dependent thereon, wherein the composition comprises (and/or the sugar component is) a multi-sugar component consisting of a plurality of compounds.
H440. The composition of H432 or any preceding paragraph dependent thereon, wherein the composition comprises (and/or the sugar component is) a non-reducing sugar component.
H441. The composition of H432 or any preceding paragraph dependent thereon, wherein the composition comprises (and/or the sugar component is) a non-reducing sugar component consisting of only non-reducing compound(s).
H442. The composition of H432 or any preceding paragraph dependent thereon, wherein the composition comprises (and/or the sugar component is) a sugar component consisting of one or more sugar(s) and/or one or more sugar alcohol(s).

(continued)

H443. The composition of H432 or any preceding paragraph dependent thereon, wherein the composition comprises (and/or the sugar component is) a sugar component consisting of one or more sugar(s).

H444. The composition of H432 or any preceding paragraph dependent thereon, wherein the composition comprises (and/or the sugar component is) a sugar component consisting of one or more sugar alcohol(s).

H445. The composition of H432 or any preceding paragraph dependent thereon, wherein the composition comprises (and/or the sugar component is) a sugar component that is a single sugar or a single sugar alcohol.

H446. The composition of H432 or any preceding paragraph dependent thereon, wherein the composition comprises (and/or the sugar component is) a sugar component that is a single sugar.

H447. The composition of H432 or any preceding paragraph dependent thereon, wherein the composition comprises (and/or the sugar component is) a sugar component that is a single sugar alcohol.

H448. The composition of H432 or any preceding paragraph dependent thereon, wherein the composition comprises (and/or the sugar component is) a sugar component consisting of one or more short (e.g. 1-3C) polyols or glycols, one or more sugar alcohols, one or more monosaccharides, one or more disaccharidess, one or more polysaccharides, one or more complex carbohydrates, or any combination thereof.

H449. The composition of H432 or any preceding paragraph dependent thereon, wherein the composition comprises (and/or the sugar component is) a sugar component comprising or consisting of one or more short (e.g. 1-3C) polyols or glycols.

H450. The composition of H432 or any preceding paragraph dependent thereon, wherein the composition comprises (and/or the sugar component is) a sugar component comprising or consisting of one or more sugar alcohols.

H451. The composition of H432 or any preceding paragraph dependent thereon, wherein the composition comprises (and/or the sugar component is) a sugar component comprising or consisting of one or more monosaccharides.

H452. The composition of H432 or any preceding paragraph dependent thereon, wherein the composition comprises (and/or the sugar component is) a sugar component comprising or consisting of one or more disaccharides.

H453. The composition of H432 or any preceding paragraph dependent thereon, wherein the composition comprises (and/or the sugar component is) a sugar component comprising or consisting of one or more polysaccharides.

H454. The composition of H432 or any preceding paragraph dependent thereon, wherein the composition comprises (and/or the sugar component is) a sugar component comprising or consisting of one or more complex carbohydrates.

H455. The composition of H432 or any preceding paragraph dependent thereon, wherein the composition comprises (and/or the sugar component is) a sugar component excluding trehalose.

H456. The composition of H432 or any preceding paragraph dependent thereon, wherein the composition comprises (and/or the sugar component is) a sugar component excluding sucrose.

H457. The composition of H432 or any preceding paragraph dependent thereon, wherein the composition comprises (and/or the sugar component is) a sugar component excluding trehalose and sucrose.

H458. The composition of H432 or any preceding paragraph dependent thereon, wherein the composition comprises (and/or the sugar component is) a sugar component excluding disaccharides.

H459. The composition of H432 or any preceding paragraph dependent thereon, wherein the composition comprises (and/or the sugar component is) a sugar component selected from one or more sugar alcohols, one or more monosaccharides, one or more trisaccharides, or any combination thereof.

H460. The composition of H432 or any preceding paragraph dependent thereon, wherein the composition comprises (and/or the sugar component is) one or more sugar alcohols.

H461. The composition of H432 or any preceding paragraph dependent thereon, wherein the composition comprises (and/or the sugar component is) a sugar alcohol.

H462. The composition of H432 or any preceding paragraph dependent thereon, wherein the composition comprises (and/or the sugar component is) a sugar component comprising or consisting of one or more short (e.g. 1-3C) polyols or glycols selected from the group consisting of glycerol, propylene glycol, 2-methyl-2,4-pentanediol, and any combination thereof.

H463. The composition of H432 or any preceding paragraph dependent thereon, wherein the composition comprises (and/or the sugar component is) glycerol and/or propylene glycol.

(continued)

H464. The composition of H432 or any preceding paragraph dependent thereon, wherein the composition comprises (and/or the sugar component is) glycerol.

H465. The composition of H432 or any preceding paragraph dependent thereon, wherein the composition comprises (and/or the sugar component is) propylene glycol.

H466. The composition of H432 or any preceding paragraph dependent thereon, wherein the composition comprises (and/or the sugar component is) a sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof.

H467. The composition of H432 or any preceding paragraph dependent thereon, wherein the composition comprises (and/or the sugar component is) a sugar component comprising or consisting of one or more sugar alcohols selected from mannitol, sorbitol, or a combination thereof.

H468. The composition of H432 or any preceding paragraph dependent thereon, wherein the composition comprises (and/or the sugar component is) a sugar component comprising or consisting of mannitol and/or sorbitol.

H469. The composition of H432 or any preceding paragraph dependent thereon, wherein the composition comprises (and/or the sugar component is) mannitol and/or sorbitol.

H470. The composition of H432 or any preceding paragraph dependent thereon, wherein the composition comprises (and/or the sugar component is) mannitol.

H471. The composition of H432 or any preceding paragraph dependent thereon, wherein the composition comprises (and/or the sugar component is) sorbitol.

H472. The composition of H432 or any preceding paragraph dependent thereon, wherein the composition comprises (and/or the sugar component is) a sugar component comprising or consisting of one or more monosaccharides selected from the group consisting of glucose, mannose, galactose, ribose, xylose, and any combination thereof.

H473. The composition of H432 or any preceding paragraph dependent thereon, wherein the composition comprises (and/or the sugar component is) glucose and/or mannose.

H474. The composition of H432 or any preceding paragraph dependent thereon, wherein the composition comprises (and/or the sugar component is) glucose.

H475. The composition of H432 or any preceding paragraph dependent thereon, wherein the composition comprises (and/or the sugar component is) mannose.

H476. The composition of H432 or any preceding paragraph dependent thereon, wherein the composition comprises (and/or the sugar component is) a sugar component comprising or consisting of one or more disaccharides selected from the group consisting of trehalose, sucrose, maltose, lactose, and any combination thereof.

H477. The composition of H432 or any preceding paragraph dependent thereon, wherein the composition comprises (and/or the sugar component is) trehalose.

H478. The composition of H432 or any preceding paragraph dependent thereon, wherein the composition comprises (and/or the sugar component is) sucrose.

H479. The composition of H432 or any preceding paragraph dependent thereon, wherein the composition comprises (and/or the sugar component is) maltose and/or lactose.

H480. The composition of H432 or any preceding paragraph dependent thereon, wherein the composition comprises (and/or the sugar component is) maltose.

H481. The composition of H432 or any preceding paragraph dependent thereon, wherein the composition comprises (and/or the sugar component is) lactose.

H482. The composition of H432 or any preceding paragraph dependent thereon, wherein the composition comprises (and/or the sugar component is) a sugar component comprising or consisting of one or more polysaccharides and/or one or more complex carbohydrates selected from the group consisting of cyclodextrins, maltodextrins, raffinose, dextran, and any combination thereof.

H483. The composition of H432 or any preceding paragraph dependent thereon, wherein the composition comprises (and/or the sugar component is) a cyclodextrin or a derivative thereof.

H484. The composition of H432 or any preceding paragraph dependent thereon, wherein the composition comprises (and/or the sugar component is) raffinose.

(continued)

H485. The composition of H432 or any preceding paragraph dependent thereon, wherein the composition comprises (and/or the sugar component is) a sugar component comprising one or more reducing sugars selected from the group consisting of fructose, mannose, maltose, lactose, arabinose, xylose, ribose, rhamnose, galactose, glucose, and any combination thereof .

H486. The composition of H432 or any preceding paragraph dependent thereon, wherein the composition comprises 30-400 mM sugar component.

H487. The composition of H432 or any preceding paragraph dependent thereon, wherein the composition comprises 50-350 mM sugar component.

H488. The composition of H432 or any preceding paragraph dependent thereon, wherein the composition comprises 100-300 mM sugar component.

H489. The composition of H432 or any preceding paragraph dependent thereon, wherein the composition comprises 175-300 mM sugar component.

H490. The composition of H432 or any preceding paragraph dependent thereon, wherein the composition comprises 200-300 mM sugar component.

H491. The composition of H432 or any preceding paragraph dependent thereon, wherein the composition comprises 150-250 mM sugar component.

H492. The composition of H432 or any preceding paragraph dependent thereon, wherein the composition comprises 200-250 mM sugar component.

H493. The composition of H432 or any preceding paragraph dependent thereon, wherein the composition comprises 200 mM sugar component.

H494. The composition of H432 or any preceding paragraph dependent thereon, wherein the composition comprises 230 mM sugar component.

H495. The composition of H432 or any preceding paragraph dependent thereon, wherein the composition comprises 250-300 mM sugar component.

H496. The composition of H432 or any preceding paragraph dependent thereon, wherein the composition comprises 270 mM sugar component.

H497. The composition of H432 or any preceding paragraph dependent thereon, wherein the composition comprises 50-200 mM sugar component.

H498. The composition of H432 or any preceding paragraph dependent thereon, wherein the composition comprises less than 130 mM sugar component.

H499. The composition of H432 or any preceding paragraph dependent thereon, wherein the composition comprises 50-129 mM sugar component.

H500. The composition of H432 or any preceding paragraph dependent thereon, wherein the composition comprises 70-129 mM sugar component.

H501. The composition of H432 or any preceding paragraph dependent thereon, wherein the composition comprises 100-150 mM sugar component.

H502. The composition of H432 or any preceding paragraph dependent thereon, wherein the composition comprises 120-130 mM sugar component.

H503. The composition of H432 or any preceding paragraph dependent thereon, wherein the composition comprises 50-350 mM sugar component wherein no single compound thereof exceeds 129 mM.

H504. The composition of H432 or any preceding paragraph dependent thereon, wherein the composition comprises 175-300 mM sugar component wherein no single compound thereof exceeds 129 mM.

H505. The composition of H432 or any preceding paragraph dependent thereon, wherein the composition comprises 200-300 mM sugar component wherein no single compound thereof exceeds 129 mM.

H506. The composition of H432 or any preceding paragraph dependent thereon, wherein the composition comprises 80 mM sugar component.

H507. The composition of H432 or any preceding paragraph dependent thereon, wherein the composition comprises 125 mM sugar component.

H508. The composition of H432 or any preceding paragraph dependent thereon, wherein the composition comprises less than 130 mM single sugar component.

H509. The composition of H432 or any preceding paragraph dependent thereon, wherein the composition comprises 50-129 mM single sugar component.

(continued)

H510. The composition of H432 or any preceding paragraph dependent thereon, wherein the composition comprises 70-129 mM single sugar component.

H511. The composition of H432 or any preceding paragraph dependent thereon, wherein the composition comprises 100-150 mM single sugar component.

H512. The composition of H432 or any preceding paragraph dependent thereon, wherein the composition comprises 120-130 mM single sugar component.

H513. The composition of H432 or any preceding paragraph dependent thereon, wherein the composition comprises 80 mM single sugar component.

H514. The composition of H432 or any preceding paragraph dependent thereon, wherein the composition comprises 125 mM single sugar component.

H515. The composition of H432 or any preceding paragraph dependent thereon, wherein the composition comprises less than 175 mM sugar component.

H516. The composition of H432 or any preceding paragraph dependent thereon, wherein the composition comprises 50-174 mM sugar component.

H517. The composition of H432 or any preceding paragraph dependent thereon, wherein the composition comprises 70-174 mM sugar component.

H518. The composition of H432 or any preceding paragraph dependent thereon, wherein the composition comprises 130-174 mM sugar component.

H519. The composition of H432 or any preceding paragraph dependent thereon, wherein the composition comprises 50-350 mM sugar component wherein no single compound thereof exceeds 174 mM.

H520. The composition of H432 or any preceding paragraph dependent thereon, wherein the composition comprises 175-300 mM sugar component wherein no single compound thereof exceeds 174 mM.

H521. The composition of H432 or any preceding paragraph dependent thereon, wherein the composition comprises 200-300 mM sugar component wherein no single compound thereof exceeds 174 mM.

H522. The composition of H432 or any preceding paragraph dependent thereon, wherein the composition comprises 150 mM sugar component.

H523. The composition of H432 or any preceding paragraph dependent thereon, wherein the composition comprises less than 175 mM single sugar component.

H524. The composition of H432 or any preceding paragraph dependent thereon, wherein the composition comprises 50-174 mM single sugar component.

H525. The composition of H432 or any preceding paragraph dependent thereon, wherein the composition comprises 70-174 mM single sugar component.

H526. The composition of H432 or any preceding paragraph dependent thereon, wherein the composition comprises 130-174 mM single sugar component.

H527. The composition of H432 or any preceding paragraph dependent thereon, wherein the composition comprises 150 mM single sugar component.

H528. The composition of H432 or any preceding paragraph dependent thereon, wherein the composition comprises less than 200 mM sugar component.

H529. The composition of H432 or any preceding paragraph dependent thereon, wherein the composition comprises 50-199 mM sugar component.

H530. The composition of H432 or any preceding paragraph dependent thereon, wherein the composition comprises 70-199 mM sugar component.

H531. The composition of H432 or any preceding paragraph dependent thereon, wherein the composition comprises 175-199 mM sugar component.

H532. The composition of H432 or any preceding paragraph dependent thereon, wherein the composition comprises 50-350 mM sugar component wherein no single compound thereof exceeds 199 mM.

H533. The composition of H432 or any preceding paragraph dependent thereon, wherein the composition comprises 175-300 mM sugar component wherein no single compound thereof exceeds 199 mM.

H534. The composition of H432 or any preceding paragraph dependent thereon, wherein the composition comprises 200-300 mM sugar component wherein no single compound thereof exceeds 199 mM.

H535. The composition of H432 or any preceding paragraph dependent thereon, wherein the composition comprises 180 mM sugar component.

(continued)

H536. The composition of H432 or any preceding paragraph dependent thereon, wherein the composition comprises less than 200 mM single sugar component.

H537. The composition of H432 or any preceding paragraph dependent thereon, wherein the composition comprises 50-199 mM single sugar component.

H538. The composition of H432 or any preceding paragraph dependent thereon, wherein the composition comprises 70-199 mM single sugar component.

H539. The composition of H432 or any preceding paragraph dependent thereon, wherein the composition comprises 175-199 mM single sugar component.

H540. The composition of H432 or any preceding paragraph dependent thereon, wherein the composition comprises 180 mM single sugar component.

H541. The composition of H432 or any preceding paragraph dependent thereon, wherein the composition comprises 50-350 mM sugar component excluding trehalose and sucrose.

H542. The composition of H432 or any preceding paragraph dependent thereon, wherein the composition comprises 50-129 mM sugar component excluding trehalose and sucrose.

H543. The composition of H432 or any preceding paragraph dependent thereon, wherein the composition comprises 80 mM sugar component excluding trehalose and sucrose.

H544. The composition of H432 or any preceding paragraph dependent thereon, wherein the composition comprises 125 mM sugar component excluding trehalose and sucrose.

H545. The composition of H432 or any preceding paragraph dependent thereon, wherein the composition comprises 130-174 mM sugar component excluding trehalose and sucrose.

H546. The composition of H432 or any preceding paragraph dependent thereon, wherein the composition comprises 150 mM sugar component excluding trehalose and sucrose.

H547. The composition of H432 or any preceding paragraph dependent thereon, wherein the composition comprises 175-199 mM sugar component excluding trehalose and sucrose.

H548. The composition of H432 or any preceding paragraph dependent thereon, wherein the composition comprises 180 mM sugar component excluding trehalose and sucrose.

H549. The composition of H432 or any preceding paragraph dependent thereon, wherein the composition comprises 200-250 mM sugar component excluding trehalose and sucrose.

H550. The composition of H432 or any preceding paragraph dependent thereon, wherein the composition comprises 200 mM sugar component excluding trehalose and sucrose.

H551. The composition of H432 or any preceding paragraph dependent thereon, wherein the composition comprises 230 mM sugar component excluding trehalose and sucrose.

H552. The composition of H432 or any preceding paragraph dependent thereon, wherein the composition comprises 50-350 mM one or more sugar alcohols.

H553. The composition of H432 or any preceding paragraph dependent thereon, wherein the composition comprises 50-129 mM one or more sugar alcohols.

H554. The composition of H432 or any preceding paragraph dependent thereon, wherein the composition comprises 100-150 mM one or more sugar alcohols.

H555. The composition of H432 or any preceding paragraph dependent thereon, wherein the composition comprises 120-130 mM one or more sugar alcohols.

H556. The composition of H432 or any preceding paragraph dependent thereon, wherein the composition comprises 80 mM one or more sugar alcohols.

H557. The composition of H432 or any preceding paragraph dependent thereon, wherein the composition comprises 125 mM one or more sugar alcohols.

H558. The composition of H432 or any preceding paragraph dependent thereon, wherein the composition comprises 130-174 mM one or more sugar alcohols.

H559. The composition of H432 or any preceding paragraph dependent thereon, wherein the composition comprises 150 mM one or more sugar alcohols.

H560. The composition of H432 or any preceding paragraph dependent thereon, wherein the composition comprises 175-199 mM one or more sugar alcohols.

H561. The composition of H432 or any preceding paragraph dependent thereon, wherein the composition comprises 180 mM one or more sugar alcohols.

(continued)

H562. The composition of H432 or any preceding paragraph dependent thereon, wherein the composition comprises 200-250 mM one or more sugar alcohols.

H563. The composition of H432 or any preceding paragraph dependent thereon, wherein the composition comprises 200 mM one or more sugar alcohols.

H564. The composition of H432 or any preceding paragraph dependent thereon, wherein the composition comprises 230 mM one or more sugar alcohols.

H565. The composition of H432 or any preceding paragraph dependent thereon, wherein the composition comprises 50-350 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H566. The composition of H432 or any preceding paragraph dependent thereon, wherein the composition comprises 50-129 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H567. The composition of H432 or any preceding paragraph dependent thereon, wherein the composition comprises 80 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof.

H568. The composition of H432 or any preceding paragraph dependent thereon, wherein the composition comprises 125 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H569. The composition of H432 or any preceding paragraph dependent thereon, wherein the composition comprises 130-174 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H570. The composition of H432 or any preceding paragraph dependent thereon, wherein the composition comprises 150 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H571. The composition of H432 or any preceding paragraph dependent thereon, wherein the composition comprises 175-199 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H572. The composition of H432 or any preceding paragraph dependent thereon, wherein the composition comprises 180 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H573. The composition of H432 or any preceding paragraph dependent thereon, wherein the composition comprises 200-250 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H574. The composition of H432 or any preceding paragraph dependent thereon, wherein the composition comprises 200 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H575. The composition of H432 or any preceding paragraph dependent thereon, wherein the composition comprises 230 mM sugar component comprising or consisting of one or more sugar alcohols selected from the group consisting of mannitol, sorbitol, erythritol, meso-erythritol, xylitol, arabitol, erythritol, lactitol, maltitol, inositol, threitol, glycerol, and any combination thereof .

H576. The composition of H432 or any preceding paragraph dependent thereon, wherein the composition comprises 50-350 mM mannitol or sorbitol.

H577. The composition of H432 or any preceding paragraph dependent thereon, wherein the composition comprises 50-129 mM mannitol or sorbitol.

(continued)

H578. The composition of H432 or any preceding paragraph dependent thereon, wherein the composition comprises 80 mM mannitol or sorbitol.

H579. The composition of H432 or any preceding paragraph dependent thereon, wherein the composition comprises 125 mM mannitol or sorbitol.

H580. The composition of H432 or any preceding paragraph dependent thereon, wherein the composition comprises 130-174 mM mannitol or sorbitol.

H581. The composition of H432 or any preceding paragraph dependent thereon, wherein the composition comprises 150 mM mannitol or sorbitol.

H582. The composition of H432 or any preceding paragraph dependent thereon, wherein the composition comprises 175-199 mM mannitol or sorbitol.

H583. The composition of H432 or any preceding paragraph dependent thereon, wherein the composition comprises 180 mM mannitol or sorbitol.

H584. The composition of H432 or any preceding paragraph dependent thereon, wherein the composition comprises 200-250 mM mannitol or sorbitol.

H585. The composition of H432 or any preceding paragraph dependent thereon, wherein the composition comprises 200 mM mannitol or sorbitol.

H586. The composition of H432 or any preceding paragraph dependent thereon, wherein the composition comprises 230 mM mannitol or sorbitol.

H587. The composition of H432 or any preceding paragraph dependent thereon, wherein the composition comprises 50-350 mM mannitol and sorbitol (combined concentration), wherein the molar ratio of mannitol to sorbitol is between 1:20 and 20:1.

H588. The composition of H432 or any preceding paragraph dependent thereon, wherein the composition comprises 50-129 mM mannitol and sorbitol (combined concentration), wherein the molar ratio of mannitol to sorbitol is between 1:20 and 20:1.

H589. The composition of H432 or any preceding paragraph dependent thereon, wherein the composition comprises 80 mM mannitol and sorbitol (combined concentration), wherein the molar ratio of mannitol to sorbitol is between 1:20 and 20:1.

H590. The composition of H432 or any preceding paragraph dependent thereon, wherein the composition comprises 125 mM mannitol and sorbitol (combined concentration), wherein the molar ratio of mannitol to sorbitol is between 1:20 and 20:1.

H591. The composition of H432 or any preceding paragraph dependent thereon, wherein the composition comprises 130-174 mM mannitol and sorbitol (combined concentration), wherein the molar ratio of mannitol to sorbitol is between 1:20 and 20:1.

H592. The composition of H432 or any preceding paragraph dependent thereon, wherein the composition comprises 150 mM mannitol and sorbitol (combined concentration), wherein the molar ratio of mannitol to sorbitol is between 1:20 and 20:1.

H593. The composition of H432 or any preceding paragraph dependent thereon, wherein the composition comprises 175-199 mM mannitol and sorbitol (combined concentration), wherein the molar ratio of mannitol to sorbitol is between 1:20 and 20:1.

H594. The composition of H432 or any preceding paragraph dependent thereon, wherein the composition comprises 180 mM mannitol and sorbitol (combined concentration), wherein the molar ratio of mannitol to sorbitol is between 1:20 and 20:1.

H595. The composition of H432 or any preceding paragraph dependent thereon, wherein the composition comprises 200-250 mM mannitol and sorbitol (combined concentration), wherein the molar ratio of mannitol to sorbitol is between 1:20 and 20:1.

H596. The composition of H432 or any preceding paragraph dependent thereon, wherein the composition comprises 200 mM mannitol and sorbitol (combined concentration), wherein the molar ratio of mannitol to sorbitol is between 1:20 and 20:1.

H597. The composition of H432 or any preceding paragraph dependent thereon, wherein the composition comprises 230 mM mannitol and sorbitol (combined concentration), wherein the molar ratio of mannitol to sorbitol is between 1:20 and 20:1.

H598. The composition of H432 or any preceding paragraph dependent thereon, wherein the composition comprises 50-350 mM mannitol and sorbitol (combined concentration), wherein the molar ratio of mannitol to sorbitol is between 1:5 and 5:1.

(continued)

H599. The composition of H432 or any preceding paragraph dependent thereon, wherein the composition comprises 50-129 mM mannitol and sorbitol (combined concentration), wherein the molar ratio of mannitol to sorbitol is between 1:5 and 5:1.

H600. The composition of H432 or any preceding paragraph dependent thereon, wherein the composition comprises 80 mM mannitol and sorbitol (combined concentration), wherein the molar ratio of mannitol to sorbitol is between 1:5 and 5:1.

H601. The composition of H432 or any preceding paragraph dependent thereon, wherein the composition comprises 125 mM mannitol and sorbitol (combined concentration), wherein the molar ratio of mannitol to sorbitol is between 1:5 and 5:1.

H602. The composition of H432 or any preceding paragraph dependent thereon, wherein the composition comprises 130-174 mM mannitol and sorbitol (combined concentration), wherein the molar ratio of mannitol to sorbitol is between 1:5 and 5:1.

H603. The composition of H432 or any preceding paragraph dependent thereon, wherein the composition comprises 150 mM mannitol and sorbitol (combined concentration), wherein the molar ratio of mannitol to sorbitol is between 1:5 and 5:1.

H604. The composition of H432 or any preceding paragraph dependent thereon, wherein the composition comprises 175-199 mM mannitol and sorbitol (combined concentration), wherein the molar ratio of mannitol to sorbitol is between 1:5 and 5:1.

H605. The composition of H432 or any preceding paragraph dependent thereon, wherein the composition comprises 180 mM mannitol and sorbitol (combined concentration), wherein the molar ratio of mannitol to sorbitol is between 1:5 and 5:1.

H606. The composition of H432 or any preceding paragraph dependent thereon, wherein the composition comprises 200 mM mannitol and sorbitol (combined concentration), wherein the molar ratio of mannitol to sorbitol is between 1:5 and 5:1.

H607. The composition of H432 or any preceding paragraph dependent thereon, wherein the composition comprises 230 mM mannitol and sorbitol (combined concentration), wherein the molar ratio of mannitol to sorbitol is between 1:5 and 5:1.

H608. The composition of H432 or any preceding paragraph dependent thereon, wherein the composition comprises 50-350 mM mannitol.

H609. The composition of H432 or any preceding paragraph dependent thereon, wherein the composition comprises 50-129 mM mannitol.

H610. The composition of H432 or any preceding paragraph dependent thereon, wherein the composition comprises 80 mM mannitol.

H611. The composition of H432 or any preceding paragraph dependent thereon, wherein the composition comprises 125 mM mannitol.

H612. The composition of H432 or any preceding paragraph dependent thereon, wherein the composition comprises 130-174 mM mannitol.

H613. The composition of H432 or any preceding paragraph dependent thereon, wherein the composition comprises 150 mM mannitol.

H614. The composition of H432 or any preceding paragraph dependent thereon, wherein the composition comprises 175-199 mM mannitol.

H615. The composition of H432 or any preceding paragraph dependent thereon, wherein the composition comprises 180 mM mannitol.

H616. The composition of H432 or any preceding paragraph dependent thereon, wherein the composition comprises 200-250 mM mannitol.

H617. The composition of H432 or any preceding paragraph dependent thereon, wherein the composition comprises 200 mM mannitol.

H618. The composition of H432 or any preceding paragraph dependent thereon, wherein the composition comprises 230 mM mannitol.

H619. The composition of H432 or any preceding paragraph dependent thereon, wherein the composition comprises 50-350 mM sorbitol.

H620. The composition of H432 or any preceding paragraph dependent thereon, wherein the composition comprises 40-80 mM sorbitol.

(continued)

H621. The composition of H432 or any preceding paragraph dependent thereon, wherein the composition comprises 50 mM sorbitol.

H622. The composition of H432 or any preceding paragraph dependent thereon, wherein the composition comprises 70 mM sorbitol.

H623. The composition of H432 or any preceding paragraph dependent thereon, wherein the composition comprises 50-129 mM sorbitol.

H624. The composition of H432 or any preceding paragraph dependent thereon, wherein the composition comprises 100-150 mM sorbitol.

H625. The composition of H432 or any preceding paragraph dependent thereon, wherein the composition comprises 120-130 mM sorbitol.

H626. The composition of H432 or any preceding paragraph dependent thereon, wherein the composition comprises 80 mM sorbitol.

H627. The composition of H432 or any preceding paragraph dependent thereon, wherein the composition comprises 125 mM sorbitol (most preferred).

H628. The composition of H432 or any preceding paragraph dependent thereon, wherein the composition comprises 130-174 mM sorbitol.

H629. The composition of H432 or any preceding paragraph dependent thereon, wherein the composition comprises 140 mM sorbitol.

H630. The composition of H432 or any preceding paragraph dependent thereon, wherein the composition comprises 150 mM sorbitol.

H631. The composition of H432 or any preceding paragraph dependent thereon, wherein the composition comprises 175-199 mM sorbitol.

H632. The composition of H432 or any preceding paragraph dependent thereon, wherein the composition comprises 180 mM sorbitol.

H633. The composition of H432 or any preceding paragraph dependent thereon, wherein the composition comprises 200-250 mM sorbitol.

H634. The composition of H432 or any preceding paragraph dependent thereon, wherein the composition comprises 200 mM sorbitol.

H635. The composition of H432 or any preceding paragraph dependent thereon, wherein the composition comprises 230 mM sorbitol.

H636. The composition of H432 or any preceding paragraph dependent thereon, wherein the composition comprises a combination of sorbitol and sodium chloride.

H637. The composition of H432 or any preceding paragraph dependent thereon, wherein the composition comprises a combination of 50-129 mM sorbitol and 10-100 mM sodium chloride.

H638. The composition of H432 or any preceding paragraph dependent thereon, wherein the composition comprises a combination of 120-130 mM sorbitol and 50-70 mM sodium chloride.

H639. The composition of H432 or any preceding paragraph dependent thereon, wherein the composition comprises a combination of 125 mM sorbitol and 60 mM sodium chloride.

H640. The composition of H1-H639, wherein the composition is characterised by an absence of sugars or sugar alcohols.

H641. The composition of H1-H640, wherein the composition is characterised by an absence of trehalose.

H642. The composition of H1-H641, wherein the composition is characterised by an absence of sucrose.

H643. The composition of H1-H642, wherein the composition is characterised by an absence of trehalose and sucrose.

H644. The composition of H1-H643, wherein the composition is characterised by an absence of disaccharides.

H645. The composition of H432 or any preceding paragraph dependent thereon, wherein the composition comprises 50-350 mM trehalose .

H646. The composition of H432 or any preceding paragraph dependent thereon, wherein the composition comprises 150-250 mM trehalose.

H647. The composition of H432 or any preceding paragraph dependent thereon, wherein the composition comprises 200 mM trehalose.

H648. The composition of H432 or any preceding paragraph dependent thereon, wherein the composition comprises 50-350 mM sucrose.

(continued)

H649. The composition of H432 or any preceding paragraph dependent thereon, wherein the composition comprises 150-250 mM sucrose.

H650. The composition of H432 or any preceding paragraph dependent thereon, wherein the composition comprises 200 mM sucrose.

H651. The composition of H1-H431, wherein the composition is characterised by an absence of a sugar component.

H652. The composition of H1-H639, wherein the composition is characterised by an absence of any, some, or all sugar components defined in H433-H485.

H653. The composition of H1-H652, wherein the composition comprises an amino acid component.

H654. The composition of H653, wherein the composition comprises (and/or the amino acid component is) an amino acid component that stabilises the biopharmaceutical active, especially in aqueous solution.

H655. The composition of H653 or any preceding paragraph dependent thereon, wherein the composition comprises (and/or the amino acid component is) a single amino acid component.

H656. The composition of H653 or any preceding paragraph dependent thereon, wherein the composition comprises (and/or the amino acid component is) a single amino acid component consisting of a single amino acid compound.

H657. The composition of H653 or any preceding paragraph dependent thereon, wherein the composition comprises (and/or the amino acid component is) a multi-amino acid component.

H658. The composition of H653 or any preceding paragraph dependent thereon, wherein the composition comprises (and/or the amino acid component is) a multi-amino acid component consisting of a plurality of amino acid compounds.

H659. The composition of H653 or any preceding paragraph dependent thereon, wherein the composition comprises (and/or the amino acid component is) an amino acid component consisting of one or more a natural amino acids, one or more a peptides (short peptide, suitably at most 3 amino acids in length, preferably at most 2 amino acids in length), one or more synthetic amino acids, or any combination thereof.

H660. The composition of H653 or any preceding paragraph dependent thereon, wherein the composition comprises (and/or the amino acid component is) an amino acid component consisting of one or more a natural amino acids.

H661. The composition of H653 or any preceding paragraph dependent thereon, wherein the composition comprises (and/or the amino acid component is) an amino acid component consisting of one or more amino acids selected from alanine, arginine, asparagine, aspartic acid, cysteine, glutamine, glutamic acid, glycine, glycylglycine, histidine, isoleucine, leucine, lysine, lysyllysine, methionine, phenylalanine, proline, serine, threonine, tryptophan, tyrosine, valine, or any combination thereof.

H662. The composition of H653 or any preceding paragraph dependent thereon, wherein the composition comprises (and/or the amino acid component is) an amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H663. The composition of H653 or any preceding paragraph dependent thereon, wherein the composition comprises (and/or the amino acid component is) an amino acid component consisting of one or more amino acids selected from arginine, cysteine, glycine, lysine, phenylalanine, proline, or any combination thereof.

H664. The composition of H653 or any preceding paragraph dependent thereon, wherein the composition comprises (and/or the amino acid component is) an amino acid component consisting of one or more amino acids selected from arginine, glycine, glycylglycine, lysine, lysyllysine, or any combination thereof.

H665. The composition of H653 or any preceding paragraph dependent thereon, wherein the composition comprises (and/or the amino acid component is) arginine and/or glycine.

H666. The composition of H653 or any preceding paragraph dependent thereon, wherein the composition comprises (and/or the amino acid component is) arginine.

H667. The composition of H653 or any preceding paragraph dependent thereon, wherein the composition comprises (and/or the amino acid component is) glycine.

H668. The composition of H653 or any preceding paragraph dependent thereon, wherein the composition comprises (and/or the amino acid component is) lysine.

(continued)

H669. The composition of H653 or any preceding paragraph dependent thereon, wherein the composition comprises (and/or the amino acid component is) phenylalanine.

H670. The composition of H653 or any preceding paragraph dependent thereon, wherein the composition comprises (and/or the amino acid component is) proline.

H671. The composition of H653 or any preceding paragraph dependent thereon, wherein the composition comprises (and/or the amino acid component is) an amino acid component excluding methionine.

H672. The composition of H653 or any preceding paragraph dependent thereon, wherein the composition comprises (and/or the amino acid component is) an amino acid component excluding histidine.

H673. The composition of H653 or any preceding paragraph dependent thereon, wherein the composition comprises (and/or the amino acid component is) an amino acid component excluding histidine and methionine.

H674. The composition of H653 or any preceding paragraph dependent thereon, wherein the composition comprises (and/or the amino acid component is) an amino acid component excluding arginine.

H675. The composition of H653 or any preceding paragraph dependent thereon, wherein the composition comprises (and/or the amino acid component is) an amino acid component excluding glycine.

H676. The composition of H653 or any preceding paragraph dependent thereon, wherein the composition comprises (and/or the amino acid component is) an amino acid component excluding arginine and glycine.

H677. The composition of H653 or any preceding paragraph dependent thereon, wherein the composition comprises (and/or the amino acid component is) an amino acid component excluding arginine, glycine, methionine, and histidine.

H678. The composition of H653 or any preceding paragraph dependent thereon, wherein the composition comprises 2-350 mM amino acid component.

H679. The composition of H653 or any preceding paragraph dependent thereon, wherein the composition comprises 20-300 mM amino acid component.

H680. The composition of H653 or any preceding paragraph dependent thereon, wherein the composition comprises 30-250 mM amino acid component.

H681. The composition of H653 or any preceding paragraph dependent thereon, wherein the composition comprises 100-300 mM amino acid component.

H682. The composition of H653 or any preceding paragraph dependent thereon, wherein the composition comprises less than 100 mM amino acid component.

H683. The composition of H653 or any preceding paragraph dependent thereon, wherein the composition comprises 20-99 mM amino acid component.

H684. The composition of H653 or any preceding paragraph dependent thereon, wherein the composition comprises 50-99 mM amino acid component.

H685. The composition of H653 or any preceding paragraph dependent thereon, wherein the composition comprises 70-99 mM amino acid component.

H686. The composition of H653 or any preceding paragraph dependent thereon, wherein the composition comprises 40 mM amino acid component.

H687. The composition of H653 or any preceding paragraph dependent thereon, wherein the composition comprises 60 mM amino acid component.

H688. The composition of H653 or any preceding paragraph dependent thereon, wherein the composition comprises 80 mM amino acid component.

H689. The composition of H653 or any preceding paragraph dependent thereon, wherein the composition comprises 2-350 mM amino acid component wherein no single amino acid compound exceeds 99 mM.

H690. The composition of H653 or any preceding paragraph dependent thereon, wherein the composition comprises less than 100 mM single amino acid component.

H691. The composition of H653 or any preceding paragraph dependent thereon, wherein the composition comprises 20-99 mM single amino acid component.

H692. The composition of H653 or any preceding paragraph dependent thereon, wherein the composition comprises 50-99 mM single amino acid component.

H693. The composition of H653 or any preceding paragraph dependent thereon, wherein the composition comprises 70-99 mM single amino acid component.

(continued)

H694. The composition of H653 or any preceding paragraph dependent thereon, wherein the composition comprises 40 mM single amino acid component.

H695. The composition of H653 or any preceding paragraph dependent thereon, wherein the composition comprises 80 mM single amino acid component.

H696. The composition of H653 or any preceding paragraph dependent thereon, wherein the composition comprises less than 150 mM amino acid component.

H697. The composition of H653 or any preceding paragraph dependent thereon, wherein the composition comprises 20-149 mM amino acid component.

H698. The composition of H653 or any preceding paragraph dependent thereon, wherein the composition comprises 50-149 mM amino acid component.

H699. The composition of H653 or any preceding paragraph dependent thereon, wherein the composition comprises 100-149 mM amino acid component.

H700. The composition of H653 or any preceding paragraph dependent thereon, wherein the composition comprises 110-140 mM amino acid component.

H701. The composition of H653 or any preceding paragraph dependent thereon, wherein the composition comprises 120-130 mM amino acid component.

H702. The composition of H653 or any preceding paragraph dependent thereon, wherein the composition comprises 120 mM amino acid component.

H703. The composition of H653 or any preceding paragraph dependent thereon, wherein the composition comprises 125 mM amino acid component.

H704. The composition of H653 or any preceding paragraph dependent thereon, wherein the composition comprises 2-350 mM amino acid component wherein no single amino acid compound exceeds 149 mM.

H705. The composition of H653 or any preceding paragraph dependent thereon, wherein the composition comprises less than 150 mM single amino acid component.

H706. The composition of H653 or any preceding paragraph dependent thereon, wherein the composition comprises 20-149 mM single amino acid component.

H707. The composition of H653 or any preceding paragraph dependent thereon, wherein the composition comprises 50-149 mM single amino acid component.

H708. The composition of H653 or any preceding paragraph dependent thereon, wherein the composition comprises 100-149 mM single amino acid component.

H709. The composition of H653 or any preceding paragraph dependent thereon, wherein the composition comprises 110-140 mM single amino acid component.

H710. The composition of H653 or any preceding paragraph dependent thereon, wherein the composition comprises 120-130 mM single amino acid component.

H711. The composition of H653 or any preceding paragraph dependent thereon, wherein the composition comprises 120 mM single amino acid component.

H712. The composition of H653 or any preceding paragraph dependent thereon, wherein the composition comprises 125 mM single amino acid component.

H713. The composition of H653 or any preceding paragraph dependent thereon, wherein the composition comprises less than 200 mM amino acid component.

H714. The composition of H653 or any preceding paragraph dependent thereon, wherein the composition comprises 20-199 mM amino acid component.

H715. The composition of H653 or any preceding paragraph dependent thereon, wherein the composition comprises 50-199 mM amino acid component.

H716. The composition of H653 or any preceding paragraph dependent thereon, wherein the composition comprises 100-199 mM amino acid component.

H717. The composition of H653 or any preceding paragraph dependent thereon, wherein the composition comprises 150-199 mM amino acid component.

H718. The composition of H653 or any preceding paragraph dependent thereon, wherein the composition comprises 170 mM amino acid component.

H719. The composition of H653 or any preceding paragraph dependent thereon, wherein the composition comprises 175 mM amino acid component.

(continued)

H720. The composition of H653 or any preceding paragraph dependent thereon, wherein the composition comprises 2-350 mM amino acid component wherein no single amino acid compound exceeds 199 mM.

H721. The composition of H653 or any preceding paragraph dependent thereon, wherein the composition comprises less than 200 mM single amino acid component.

H722. The composition of H653 or any preceding paragraph dependent thereon, wherein the composition comprises 20-199 mM single amino acid component.

H723. The composition of H653 or any preceding paragraph dependent thereon, wherein the composition comprises 50-199 mM single amino acid component.

H724. The composition of H653 or any preceding paragraph dependent thereon, wherein the composition comprises 100-199 mM single amino acid component.

H725. The composition of H653 or any preceding paragraph dependent thereon, wherein the composition comprises 150-199 mM single amino acid component.

H726. The composition of H653 or any preceding paragraph dependent thereon, wherein the composition comprises 175 mM single amino acid component.

H727. The composition of H653 or any preceding paragraph dependent thereon, wherein the composition comprises 2-350 mM amino acid component excluding histidine and methionine.

H728. The composition of H653 or any preceding paragraph dependent thereon, wherein the composition comprises 20-99 mM amino acid component excluding histidine and methionine.

H729. The composition of H653 or any preceding paragraph dependent thereon, wherein the composition comprises 40 mM amino acid component excluding histidine and methionine.

H730. The composition of H653 or any preceding paragraph dependent thereon, wherein the composition comprises 80 mM amino acid component excluding histidine and methionine.

H731. The composition of H653 or any preceding paragraph dependent thereon, wherein the composition comprises 100-149 mM amino acid component excluding histidine and methionine.

H732. The composition of H653 or any preceding paragraph dependent thereon, wherein the composition comprises 125 mM amino acid component excluding histidine and methionine.

H733. The composition of H653 or any preceding paragraph dependent thereon, wherein the composition comprises 150-199 mM amino acid component excluding histidine and methionine.

H734. The composition of H653 or any preceding paragraph dependent thereon, wherein the composition comprises 175 mM amino acid component excluding histidine and methionine.

H735. The composition of H653 or any preceding paragraph dependent thereon, wherein the composition comprises 2-350 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H736. The composition of H653 or any preceding paragraph dependent thereon, wherein the composition comprises 20-99 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H737. The composition of H653 or any preceding paragraph dependent thereon, wherein the composition comprises 40 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H738. The composition of H653 or any preceding paragraph dependent thereon, wherein the composition comprises 80 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

(continued)

H739. The composition of H653 or any preceding paragraph dependent thereon, wherein the composition comprises 100-149 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H740. The composition of H653 or any preceding paragraph dependent thereon, wherein the composition comprises 125 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H741. The composition of H653 or any preceding paragraph dependent thereon, wherein the composition comprises 150-199 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H742. The composition of H653 or any preceding paragraph dependent thereon, wherein the composition comprises 175 mM amino acid component consisting of one or more amino acids selected from alanine, arginine, asparagine, aspartic acid, cysteine, glutamine, glutamic acid, glycine, glycylglycine, isoleucine, leucine, lysine, lysyllysine, phenylalanine, proline, serine, threonine, tryptophan, tyrosine, valine, or any combination thereof.

H743. The composition of H653 or any preceding paragraph dependent thereon, wherein the composition comprises 2-350 mM arginine or glycine.

H744. The composition of H653 or any preceding paragraph dependent thereon, wherein the composition comprises 20-99 mM arginine or glycine.

H745. The composition of H653 or any preceding paragraph dependent thereon, wherein the composition comprises 40 mM arginine or glycine.

H746. The composition of H653 or any preceding paragraph dependent thereon, wherein the composition comprises 80 mM arginine or glycine.

H747. The composition of H653 or any preceding paragraph dependent thereon, wherein the composition comprises 100-149 mM arginine or glycine.

H748. The composition of H653 or any preceding paragraph dependent thereon, wherein the composition comprises 125 mM arginine or glycine.

H749. The composition of H653 or any preceding paragraph dependent thereon, wherein the composition comprises 150-199 mM arginine or glycine.

H750. The composition of H653 or any preceding paragraph dependent thereon, wherein the composition comprises 175 mM arginine or glycine.

H751. The composition of H653 or any preceding paragraph dependent thereon, wherein the composition comprises 2-350 mM arginine and glycine (combined concentration), wherein the molar ratio of arginine to glycine is between 20:1 and 1:20.

H752. The composition of H653 or any preceding paragraph dependent thereon, wherein the composition comprises 20-99 mM arginine and glycine (combined concentration), wherein the molar ratio of arginine to glycine is between 20:1 and 1:20.

H753. The composition of H653 or any preceding paragraph dependent thereon, wherein the composition comprises 40 mM arginine and glycine (combined concentration), wherein the molar ratio of arginine to glycine is between 20:1 and 1:20.

H754. The composition of H653 or any preceding paragraph dependent thereon, wherein the composition comprises 80 mM arginine and glycine (combined concentration), wherein the molar ratio of arginine to glycine is between 20:1 and 1:20.

H755. The composition of H653 or any preceding paragraph dependent thereon, wherein the composition comprises 100-149 mM arginine and glycine (combined concentration), wherein the molar ratio of arginine to glycine is between 20:1 and 1:20.

H756. The composition of H653 or any preceding paragraph dependent thereon, wherein the composition comprises 125 mM arginine and glycine (combined concentration), wherein the molar ratio of arginine to glycine is between 20:1 and 1:20.

(continued)

H757. The composition of H653 or any preceding paragraph dependent thereon, wherein the composition comprises 150-199 mM arginine and glycine (combined concentration), wherein the molar ratio of arginine to glycine is between 20:1 and 1:20.

H758. The composition of H653 or any preceding paragraph dependent thereon, wherein the composition comprises 175 mM arginine and glycine (combined concentration), wherein the molar ratio of arginine to glycine is between 20:1 and 1:20.

H759. The composition of H653 or any preceding paragraph dependent thereon, wherein the composition comprises 2-350 mM arginine and glycine (combined concentration), wherein the molar ratio of arginine to glycine is between 5:1 and 1:5.

H760. The composition of H653 or any preceding paragraph dependent thereon, wherein the composition comprises 20-99 mM arginine and glycine (combined concentration), wherein the molar ratio of arginine to glycine is between 5:1 and 1:5.

H761. The composition of H653 or any preceding paragraph dependent thereon, wherein the composition comprises 40 mM arginine and glycine (combined concentration), wherein the molar ratio of arginine to glycine is between 5:1 and 1:5.

H762. The composition of H653 or any preceding paragraph dependent thereon, wherein the composition comprises 80 mM arginine and glycine (combined concentration), wherein the molar ratio of arginine to glycine is between 5:1 and 1:5.

H763. The composition of H653 or any preceding paragraph dependent thereon, wherein the composition comprises 100-149 mM arginine and glycine (combined concentration), wherein the molar ratio of arginine to glycine is between 5:1 and 1:5.

H764. The composition of H653 or any preceding paragraph dependent thereon, wherein the composition comprises 125 mM arginine and glycine (combined concentration), wherein the molar ratio of arginine to glycine is between 5:1 and 1:5.

H765. The composition of H653 or any preceding paragraph dependent thereon, wherein the composition comprises 150-199 mM arginine and glycine (combined concentration), wherein the molar ratio of arginine to glycine is between 5:1 and 1:5.

H766. The composition of H653 or any preceding paragraph dependent thereon, wherein the composition comprises 175 mM arginine and glycine (combined concentration), wherein the molar ratio of arginine to glycine is between 5:1 and 1:5.

H767. The composition of H653 or any preceding paragraph dependent thereon, wherein the composition comprises 2-350 mM arginine.

H768. The composition of H653 or any preceding paragraph dependent thereon, wherein the composition comprises 20-99 mM arginine.

H769. The composition of H653 or any preceding paragraph dependent thereon, wherein the composition comprises 40 mM arginine.

H770. The composition of H653 or any preceding paragraph dependent thereon, wherein the composition comprises 60 mM arginine.

H771. The composition of H653 or any preceding paragraph dependent thereon, wherein the composition comprises 80 mM arginine.

H772. The composition of H653 or any preceding paragraph dependent thereon, wherein the composition comprises 100-149 mM arginine.

H773. The composition of H653 or any preceding paragraph dependent thereon, wherein the composition comprises 125 mM arginine.

H774. The composition of H653 or any preceding paragraph dependent thereon, wherein the composition comprises 120 mM arginine.

H775. The composition of H653 or any preceding paragraph dependent thereon, wherein the composition comprises 150-199 mM arginine.

H776. The composition of H653 or any preceding paragraph dependent thereon, wherein the composition comprises 175 mM arginine.

H777. The composition of H653 or any preceding paragraph dependent thereon, wherein the composition comprises 2-350 mM glycine.

H778. The composition of H653 or any preceding paragraph dependent thereon, wherein the composition comprises 20-99 mM glycine.

(continued)

H779. The composition of H653 or any preceding paragraph dependent thereon, wherein the composition comprises 40 mM glycine.

H780. The composition of H653 or any preceding paragraph dependent thereon, wherein the composition comprises 80 mM glycine.

H781. The composition of H653 or any preceding paragraph dependent thereon, wherein the composition comprises 100-149 mM glycine.

H782. The composition of H653 or any preceding paragraph dependent thereon, wherein the composition comprises 125 mM glycine.

H783. The composition of H653 or any preceding paragraph dependent thereon, wherein the composition comprises 150-199 mM glycine.

H784. The composition of H653 or any preceding paragraph dependent thereon, wherein the composition comprises 170 mM glycine.

H785. The composition of H653 or any preceding paragraph dependent thereon, wherein the composition comprises 175 mM glycine.

H786. The composition of H653 or any preceding paragraph dependent thereon, wherein the composition comprises 2-350 mM lysine.

H787. The composition of H653 or any preceding paragraph dependent thereon, wherein the composition comprises 20-99 mM lysine.

H788. The composition of H653 or any preceding paragraph dependent thereon, wherein the composition comprises 40 mM lysine.

H789. The composition of H653 or any preceding paragraph dependent thereon, wherein the composition comprises 80 mM lysine.

H790. The composition of H653 or any preceding paragraph dependent thereon, wherein the composition comprises 100-149 mM lysine.

H791. The composition of H653 or any preceding paragraph dependent thereon, wherein the composition comprises 125 mM lysine.

H792. The composition of H653 or any preceding paragraph dependent thereon, wherein the composition comprises 120 mM lysine.

H793. The composition of H653 or any preceding paragraph dependent thereon, wherein the composition comprises 150-199 mM lysine.

H794. The composition of H653 or any preceding paragraph dependent thereon, wherein the composition comprises 170 mM lysine.

H795. The composition of H653 or any preceding paragraph dependent thereon, wherein the composition comprises 175 mM lysine.

H796. The composition of H653 or any preceding paragraph dependent thereon, wherein the composition comprises 2-350 mM proline.

H797. The composition of H653 or any preceding paragraph dependent thereon, wherein the composition comprises 20-99 mM proline.

H798. The composition of H653 or any preceding paragraph dependent thereon, wherein the composition comprises 40 mM proline.

H799. The composition of H653 or any preceding paragraph dependent thereon, wherein the composition comprises 80 mM proline.

H800. The composition of H653 or any preceding paragraph dependent thereon, wherein the composition comprises 100-149 mM proline.

H801. The composition of H653 or any preceding paragraph dependent thereon, wherein the composition comprises 125 mM proline.

H802. The composition of H653 or any preceding paragraph dependent thereon, wherein the composition comprises 120 mM proline.

H803. The composition of H653 or any preceding paragraph dependent thereon, wherein the composition comprises 150-199 mM proline.

H804. The composition of H653 or any preceding paragraph dependent thereon, wherein the composition comprises 170 mM proline.

(continued)

H805. The composition of H653 or any preceding paragraph dependent thereon, wherein the composition comprises 175 mM proline.

H806. The composition of H653 or any preceding paragraph dependent thereon, wherein the composition comprises 2-350 mM phenylalanine.

H807. The composition of H653 or any preceding paragraph dependent thereon, wherein the composition comprises 5-99 mM phenylalanine.

H808. The composition of H653 or any preceding paragraph dependent thereon, wherein the composition comprises 5-40 mM phenylalanine.

H809. The composition of H653 or any preceding paragraph dependent thereon, wherein the composition comprises 20 mM phenylalanine.

H810. The composition of H1-H809, wherein the composition is characterised by an absence of methionine.

H811. The composition of H1-H810, wherein the composition is characterised by an absence of histidine.

H812. The composition of H1-H811, wherein the composition is characterised by an absence of histidine and methionine.

H813. The composition of H1-H812, wherein the composition is characterised by an absence of arginine.

H814. The composition of H1-H813, wherein the composition is characterised by an absence of glycine.

H815. The composition of H1-H814, wherein the composition is characterised by an absence of arginine and glycine.

H816. The composition of H1-H815, wherein the composition is characterised by an absence of arginine, glycine, methionine, and histidine.

H817. The composition of H1-H652, wherein the composition is characterised by an absence of an amino acid component.

H818. The composition of H1-H809, wherein the composition is characterised by an absence of any, some, or all amino acid components defined in H654-H677.

H819. The composition of H1-H652, wherein the composition is characterised by an absence of any amino acids.

H820. The composition of H1-H819, wherein the composition comprises a surfactant.

H821. The composition of H820, wherein the composition comprises (and/or the surfactant is) a surfactant that mitigates against aggregation of the biopharmaceutical active.

H822. The composition of H820 or any preceding paragraph dependent thereon, wherein the composition comprises (and/or the surfactant is) a single surfactant.

H823. The composition of H820 or any preceding paragraph dependent thereon, wherein the composition comprises (and/or the surfactant is) a non-ionic surfactant.

H824. The composition of H820 or any preceding paragraph dependent thereon, wherein the composition comprises (and/or the surfactant is) a surfactant selected from the group consisting of a fatty alcohol, a fatty alcohol ether, a fatty acid ester, a fatty acid amide, a polyoxyalkylene alkyl ether, a polyoxyethylene alkyl ether, a non-ionic block copolymer, alpha-tocopherol, and any combination thereof.

H825. The composition of H820 or any preceding paragraph dependent thereon, wherein the composition comprises (and/or the surfactant is) a surfactant selected from the group consisting of polysorbates, spans, poloxamers, kolliphors, and any combination thereof.

H826. The composition of H820 or any preceding paragraph dependent thereon, wherein the composition comprises (and/or the surfactant is) a surfactant selected from the group consisting of a sorbitan ester (e.g. Span), an ethoxylated sorbitan ester (e.g. polysorbate), and any combination thereof.

H827. The composition of H820 or any preceding paragraph dependent thereon, wherein the composition comprises (and/or the surfactant is) a surfactant selected from the group consisting of sorbitan monolaurate, sorbitan monostearate, sorbitan tristearate, and any combination thereof.

H828. The composition of H820 or any preceding paragraph dependent thereon, wherein the composition comprises (and/or the surfactant is) a polysorbate surfactant.

H829. The composition of H820 or any preceding paragraph dependent thereon, wherein the composition comprises (and/or the surfactant is) a surfactant selected from the group consisting of polysorbate 20, polysorbate 40, polysorbate 60, polysorbate 80, and any combination thereof.

(continued)

H830. The composition of H820 or any preceding paragraph dependent thereon, wherein the composition comprises (and/or the surfactant is) a surfactant selected from the group consisting of glycerol monostearate, glycerol monolaurate, polyoxylglycerides (e.g. lauroyl polyoxylglycerides), and any combination thereof.

H831. The composition of H820 or any preceding paragraph dependent thereon, wherein the composition comprises (and/or the surfactant is) a surfactant selected from Macrogol 15 hydroxystearate, macrogol cetostearyl ether, macrogol stearyl ether, polyoxyethylene castor oil derivatives, polyoxyethylene stearates, and any combination thereof.

H832. The composition of H820 or any preceding paragraph dependent thereon, wherein the composition comprises (and/or the surfactant is) a surfactant that is or comprises a block alkoxylate.

H833. The composition of H820 or any preceding paragraph dependent thereon, wherein the composition comprises (and/or the surfactant is) a surfactant selected from the group consisting of poloxamers, poloxamer 182, poloxamer 188 (Pluronic F68), poloxamer 407 (Pluronic F127), Synperonics, Kolliphors, and any combination thereof.

H834. The composition of H820 or any preceding paragraph dependent thereon, wherein the composition comprises (and/or the surfactant is) a poloxamer.

H835. The composition of H820 or any preceding paragraph dependent thereon, wherein the composition comprises (and/or the surfactant is) Poloxamer 188 (e.g. Pluronic F68).

H836. The composition of H820 or any preceding paragraph dependent thereon, wherein the composition comprises (and/or the surfactant is) Poloxamer 407 (e.g. Pluronic F127).

H837. The composition of H820 or any preceding paragraph dependent thereon, wherein the composition comprises (and/or the surfactant is) kolliphor hs-15.

H838. The composition of H820 or any preceding paragraph dependent thereon, wherein the composition comprises (and/or the surfactant is) a surfactant selected from the group consisting of polysorbate 20, polysorbate 80, poloxamer 182, poloxamer 188, poloxamer 407, kolliphor hs-15, and any combination thereof.

H839. The composition of H820 or any preceding paragraph dependent thereon, wherein the composition comprises (and/or the surfactant is) polysorbate 20 or polysorbate 80.

H840. The composition of H820 or any preceding paragraph dependent thereon, wherein the composition comprises (and/or the surfactant is) polysorbate 20.

H841. The composition of H820 or any preceding paragraph dependent thereon, wherein the composition comprises (and/or the surfactant is) polysorbate 80.

H842. The composition of H820 or any preceding paragraph dependent thereon, wherein the composition comprises (and/or the surfactant is) a surfactant excluding polysorbate 80.

H843. The composition of H820 or any preceding paragraph dependent thereon, wherein the composition comprises (and/or the surfactant is) a surfactant excluding polysorbates.

H844. The composition of H820 or any preceding paragraph dependent thereon, wherein the composition comprises (and/or the surfactant is) a surfactant excluding poloxamers.

H845. The composition of H820 or any preceding paragraph dependent thereon, wherein the composition comprises (and/or the surfactant is) a surfactant excluding polysorbates and poloxamers.

H846. The composition of H820 or any preceding paragraph dependent thereon, wherein the composition comprises 0.001-5 mg/mL surfactant.

H847. The composition of H820 or any preceding paragraph dependent thereon, wherein the composition comprises 0.005-3 mg/mL surfactant.

H848. The composition of H820 or any preceding paragraph dependent thereon, wherein the composition comprises 0.01-2 mg/mL surfactant.

H849. The composition of H820 or any preceding paragraph dependent thereon, wherein the composition comprises 0.05-1.5 mg/mL surfactant.

H850. The composition of H820 or any preceding paragraph dependent thereon, wherein the composition comprises 0.01-1.2 mg/mL surfactant.

H851. The composition of H820 or any preceding paragraph dependent thereon, wherein the composition comprises 0.1-1.1 mg/mL surfactant.

H852. The composition of H820 or any preceding paragraph dependent thereon, wherein the composition comprises 0.1-0.7 mg/mL surfactant.

(continued)

H853. The composition of H820 or any preceding paragraph dependent thereon, wherein the composition comprises less than 0.1 mg/mL surfactant.

H854. The composition of H820 or any preceding paragraph dependent thereon, wherein the composition comprises 0.001-0.05 mg/mL surfactant.

H855. The composition of H820 or any preceding paragraph dependent thereon, wherein the composition comprises 0.01-0.09 mg/mL surfactant.

H856. The composition of H820 or any preceding paragraph dependent thereon, wherein the composition comprises 0.01 mg/mL surfactant.

H857. The composition of H820 or any preceding paragraph dependent thereon, wherein the composition comprises less than 0.2 mg/mL surfactant.

H858. The composition of H820 or any preceding paragraph dependent thereon, wherein the composition comprises 0.001-0.15 mg/mL surfactant.

H859. The composition of H820 or any preceding paragraph dependent thereon, wherein the composition comprises 0.01-0.15 mg/mL surfactant.

H860. The composition of H820 or any preceding paragraph dependent thereon, wherein the composition comprises 0.1 mg/mL surfactant.

H861. The composition of H820 or any preceding paragraph dependent thereon, wherein the composition comprises 0.05-0.4 mg/mL surfactant.

H862. The composition of H820 or any preceding paragraph dependent thereon, wherein the composition comprises 0.1-0.3 mg/mL surfactant.

H863. The composition of H820 or any preceding paragraph dependent thereon, wherein the composition comprises 0.2 mg/mL surfactant.

H864. The composition of H820 or any preceding paragraph dependent thereon, wherein the composition comprises 0.3 mg/mL surfactant.

H865. The composition of H820 or any preceding paragraph dependent thereon, wherein the composition comprises 0.25-0.75 mg/mL surfactant.

H866. The composition of H820 or any preceding paragraph dependent thereon, wherein the composition comprises 0.4-0.6 mg/mL.

H867. The composition of H820 or any preceding paragraph dependent thereon, wherein the composition comprises 0.5 mg/mL surfactant.

H868. The composition of H820 or any preceding paragraph dependent thereon, wherein the composition comprises 0.8-1.2 mg/mL surfactant.

H869. The composition of H820 or any preceding paragraph dependent thereon, wherein the composition comprises 0.9-1.1 mg/mL surfactant.

H870. The composition of H820 or any preceding paragraph dependent thereon, wherein the composition comprises 1 mg/mL surfactant.

H871. The composition of H820 or any preceding paragraph dependent thereon, wherein the composition comprises greater than 1 mg/mL surfactant.

H872. The composition of H820 or any preceding paragraph dependent thereon, wherein the composition comprises 1.01-2 mg/mL surfactant.

H873. The composition of H820 or any preceding paragraph dependent thereon, wherein the composition comprises 1.05-1.5 mg/mL surfactant.

H874. The composition of H820 or any preceding paragraph dependent thereon, wherein the composition comprises 1.2 mg/mL surfactant.

H875. The composition of H820 or any preceding paragraph dependent thereon, wherein the composition comprises 0.1-0.5 mg/mL surfactant.

H876. The composition of H820 or any preceding paragraph dependent thereon, wherein the composition comprises 0.1-0.3 mg/mL surfactant.

H877. The composition of H820 or any preceding paragraph dependent thereon, wherein the composition comprises 0.2 mg/mL surfactant.

H878. The composition of H820 or any preceding paragraph dependent thereon, wherein the composition comprises 0.001-0.09 mg/mL surfactant.

(continued)

H879. The composition of H820 or any preceding paragraph dependent thereon, wherein the composition comprises 0.001-0.05 mg/mL surfactant.

H880. The composition of H820 or any preceding paragraph dependent thereon, wherein the composition comprises 0.05 mg/mL surfactant.

H881. The composition of H820 or any preceding paragraph dependent thereon, wherein the composition comprises 0.0008-3.82 mM surfactant.

H882. The composition of H820 or any preceding paragraph dependent thereon, wherein the composition comprises 0.004-2.29 mM surfactant.

H883. The composition of H820 or any preceding paragraph dependent thereon, wherein the composition comprises 0.008-1.53 mM surfactant.

H884. The composition of H820 or any preceding paragraph dependent thereon, wherein the composition comprises 0.04-1.15 mM surfactant.

H885. The composition of H820 or any preceding paragraph dependent thereon, wherein the composition comprises 0.008-0.92 mM surfactant.

H886. The composition of H820 or any preceding paragraph dependent thereon, wherein the composition comprises 0.08-0.84 mM surfactant.

H887. The composition of H820 or any preceding paragraph dependent thereon, wherein the composition comprises 0.08-0.53 mM surfactant.

H888. The composition of H820 or any preceding paragraph dependent thereon, wherein the composition comprises 0.08 mM surfactant.

H889. The composition of H820 or any preceding paragraph dependent thereon, wherein the composition comprises 0.0008-0.04 mM surfactant.

H890. The composition of H820 or any preceding paragraph dependent thereon, wherein the composition comprises 0.0008-0.07 mM surfactant.

H891. The composition of H820 or any preceding paragraph dependent thereon, wherein the composition comprises 0.008 mM surfactant.

H892. The composition of H820 or any preceding paragraph dependent thereon, wherein the composition comprises 0.0008-0.11 mM surfactant.

H893. The composition of H820 or any preceding paragraph dependent thereon, wherein the composition comprises 0.008-0.11 mM surfactant.

H894. The composition of H820 or any preceding paragraph dependent thereon, wherein the composition comprises 0.08 mM surfactant.

H895. The composition of H820 or any preceding paragraph dependent thereon, wherein the composition comprises 0.04-0.31 mM surfactant.

H896. The composition of H820 or any preceding paragraph dependent thereon, wherein the composition comprises 0.08-0.23 mM surfactant.

H897. The composition of H820 or any preceding paragraph dependent thereon, wherein the composition comprises 0.15 mM surfactant.

H898. The composition of H820 or any preceding paragraph dependent thereon, wherein the composition comprises 0.19-0.57 mM surfactant.

H899. The composition of H820 or any preceding paragraph dependent thereon, wherein the composition comprises 0.31-0.46 mM surfactant.

H900. The composition of H820 or any preceding paragraph dependent thereon, wherein the composition comprises 0.38 mM surfactant.

H901. The composition of H820 or any preceding paragraph dependent thereon, wherein the composition comprises 0.61-0.92 mM surfactant.

H902. The composition of H820 or any preceding paragraph dependent thereon, wherein the composition comprises 0.68-0.84 mM surfactant.

H903. The composition of H820 or any preceding paragraph dependent thereon, wherein the composition comprises 0.76 mM surfactant.

H904. The composition of H820 or any preceding paragraph dependent thereon, wherein the composition comprises 0.77-1.53 mM surfactant.

(continued)

H905. The composition of H820 or any preceding paragraph dependent thereon, wherein the composition comprises 0.80-1.15 mM surfactant.

H906. The composition of H820 or any preceding paragraph dependent thereon, wherein the composition comprises 0.92 mM surfactant.

H907. The composition of H820 or any preceding paragraph dependent thereon, wherein the composition comprises 0.08-0.38 mM surfactant.

H908. The composition of H820 or any preceding paragraph dependent thereon, wherein the composition comprises 0.08-0.23 mM surfactant.

H909. The composition of H820 or any preceding paragraph dependent thereon, wherein the composition comprises 0.15 mM surfactant.

H910. The composition of H820 or any preceding paragraph dependent thereon, wherein the composition comprises 0.0008-0.07 mM surfactant.

H911. The composition of H820 or any preceding paragraph dependent thereon, wherein the composition comprises 0.0008-0.04 mM surfactant.

H912. The composition of H820 or any preceding paragraph dependent thereon, wherein the composition comprises 0.04 mM surfactant.

H913. The composition of H820 or any preceding paragraph dependent thereon, wherein the composition comprises 0.001-5 mg/mL surfactant selected from the group consisting of polysorbates, spans, poloxamers, and kolliphors.

H914. The composition of H820 or any preceding paragraph dependent thereon, wherein the composition comprises 0.01-2 mg/mL surfactant selected from the group consisting of polysorbates, spans, poloxamers, and kolliphors.

H915. The composition of H820 or any preceding paragraph dependent thereon, wherein the composition comprises 0.1-1.1 mg/mL surfactant selected from the group consisting of polysorbates, spans, poloxamers, and kolliphors.

H916. The composition of H820 or any preceding paragraph dependent thereon, wherein the composition comprises 0.001-0.05 mg/mL surfactant selected from the group consisting of polysorbates, spans, poloxamers, and kolliphors.

H917. The composition of H820 or any preceding paragraph dependent thereon, wherein the composition comprises 0.01 mg/mL surfactant selected from the group consisting of polysorbates, spans, poloxamers, and kolliphors.

H918. The composition of H820 or any preceding paragraph dependent thereon, wherein the composition comprises 0.001-0.15 mg/mL surfactant selected from the group consisting of polysorbates, spans, poloxamers, and kolliphors.

H919. The composition of H820 or any preceding paragraph dependent thereon, wherein the composition comprises 0.1 mg/mL surfactant selected from the group consisting of polysorbates, spans, poloxamers, and kolliphors.

H920. The composition of H820 or any preceding paragraph dependent thereon, wherein the composition comprises 0.1-0.3 mg/mL surfactant selected from the group consisting of polysorbates, spans, poloxamers, and kolliphors.

H921. The composition of H820 or any preceding paragraph dependent thereon, wherein the composition comprises 0.2 mg/mL surfactant selected from the group consisting of polysorbates, spans, poloxamers, and kolliphors.

H922. The composition of H820 or any preceding paragraph dependent thereon, wherein the composition comprises 0.25-0.75 mg/mL surfactant selected from the group consisting of polysorbates, spans, poloxamers, and kolliphors.

H923. The composition of H820 or any preceding paragraph dependent thereon, wherein the composition comprises 0.5 mg/mL surfactant selected from the group consisting of polysorbates, spans, poloxamers, and kolliphors.

H924. The composition of H820 or any preceding paragraph dependent thereon, wherein the composition comprises 0.8-1.2 mg/mL surfactant selected from the group consisting of polysorbates, spans, poloxamers, and kolliphors.

(continued)

H925. The composition of H820 or any preceding paragraph dependent thereon, wherein the composition comprises 1 mg/mL surfactant selected from the group consisting of polysorbates, spans, poloxamers, and kolliphors.

H926. The composition of H820 or any preceding paragraph dependent thereon, wherein the composition comprises 1.2 mg/mL surfactant selected from the group consisting of polysorbates, spans, poloxamers, and kolliphors.

H927. The composition of H820 or any preceding paragraph dependent thereon, wherein the composition comprises 0.001-5 mg/mL surfactant selected from the group consisting of polysorbates and poloxamers.

H928. The composition of H820 or any preceding paragraph dependent thereon, wherein the composition comprises 0.01-2 mg/mL surfactant selected from the group consisting of polysorbates and poloxamers.

H929. The composition of H820 or any preceding paragraph dependent thereon, wherein the composition comprises 0.1-1.1 mg/mL surfactant selected from the group consisting of polysorbates and poloxamers.

H930. The composition of H820 or any preceding paragraph dependent thereon, wherein the composition comprises 0.001-0.05 mg/mL surfactant selected from the group consisting of polysorbates and poloxamers.

H931. The composition of H820 or any preceding paragraph dependent thereon, wherein the composition comprises 0.01 mg/mL surfactant selected from the group consisting of polysorbates and poloxamers.

H932. The composition of H820 or any preceding paragraph dependent thereon, wherein the composition comprises 0.001-0.15 mg/mL surfactant selected from the group consisting of polysorbates and poloxamers.

H933. The composition of H820 or any preceding paragraph dependent thereon, wherein the composition comprises 0.1 mg/mL surfactant selected from the group consisting of polysorbates and poloxamers.

H934. The composition of H820 or any preceding paragraph dependent thereon, wherein the composition comprises 0.1-0.3 mg/mL surfactant selected from the group consisting of polysorbates and poloxamers.

H935. The composition of H820 or any preceding paragraph dependent thereon, wherein the composition comprises 0.2 mg/mL surfactant selected from the group consisting of polysorbates and poloxamers.

H936. The composition of H820 or any preceding paragraph dependent thereon, wherein the composition comprises 0.25-0.75 mg/mL surfactant selected from the group consisting of polysorbates and poloxamers.

H937. The composition of H820 or any preceding paragraph dependent thereon, wherein the composition comprises 0.5 mg/mL surfactant selected from the group consisting of polysorbates and poloxamers.

H938. The composition of H820 or any preceding paragraph dependent thereon, wherein the composition comprises 0.8-1.2 mg/mL surfactant selected from the group consisting of polysorbates and poloxamers.

H939. The composition of H820 or any preceding paragraph dependent thereon, wherein the composition comprises 1 mg/mL surfactant selected from the group consisting of polysorbates and poloxamers.

H940. The composition of H820 or any preceding paragraph dependent thereon, wherein the composition comprises 1.2 mg/mL surfactant selected from the group consisting of polysorbates and poloxamers.

H941. The composition of H820 or any preceding paragraph dependent thereon, wherein the composition comprises 0.001-5 mg/mL surfactant selected from the group consisting of polysorbate 20, polysorbate 40, polysorbate 60, polysorbate 80, poloxamer 182, poloxamer 188, and poloxamer 407.

H942. The composition of H820 or any preceding paragraph dependent thereon, wherein the composition comprises 0.01-2 mg/mL surfactant selected from the group consisting of polysorbate 20, polysorbate 40, polysorbate 60, polysorbate 80, poloxamer 182, poloxamer 188, and poloxamer 407.

H943. The composition of H820 or any preceding paragraph dependent thereon, wherein the composition comprises 0.1-1.1 mg/mL surfactant selected from the group consisting of polysorbate 20, polysorbate 40, polysorbate 60, polysorbate 80, poloxamer 182, poloxamer 188, and poloxamer 407.

H944. The composition of H820 or any preceding paragraph dependent thereon, wherein the composition comprises 0.001-0.05 mg/mL surfactant selected from the group consisting of polysorbate 20, polysorbate 40, polysorbate 60, polysorbate 80, poloxamer 182, poloxamer 188, and poloxamer 407.

H945. The composition of H820 or any preceding paragraph dependent thereon, wherein the composition comprises 0.01 mg/mL surfactant selected from the group consisting of polysorbate 20, polysorbate 40, polysorbate 60, polysorbate 80, poloxamer 182, poloxamer 188, and poloxamer 407.

(continued)

H946. The composition of H820 or any preceding paragraph dependent thereon, wherein the composition comprises 0.001-0.15 mg/mL surfactant selected from the group consisting of polysorbate 20, polysorbate 40, polysorbate 60, polysorbate 80, poloxamer 182, poloxamer 188, and poloxamer 407.

H947. The composition of H820 or any preceding paragraph dependent thereon, wherein the composition comprises 0.1 mg/mL surfactant selected from the group consisting of polysorbate 20, polysorbate 40, polysorbate 60, polysorbate 80, poloxamer 182, poloxamer 188, and poloxamer 407.

H948. The composition of H820 or any preceding paragraph dependent thereon, wherein the composition comprises 0.1-0.3 mg/mL surfactant selected from the group consisting of polysorbate 20, polysorbate 40, polysorbate 60, polysorbate 80, poloxamer 182, poloxamer 188, and poloxamer 407.

H949. The composition of H820 or any preceding paragraph dependent thereon, wherein the composition comprises 0.2 mg/mL surfactant selected from the group consisting of polysorbate 20, polysorbate 40, polysorbate 60, polysorbate 80, poloxamer 182, poloxamer 188, and poloxamer 407.

H950. The composition of H820 or any preceding paragraph dependent thereon, wherein the composition comprises 0.3 mg/mL surfactant selected from the group consisting of polysorbate 20, polysorbate 40, polysorbate 60, polysorbate 80, poloxamer 182, poloxamer 188, and poloxamer 407.

H951. The composition of H820 or any preceding paragraph dependent thereon, wherein the composition comprises 0.25-0.75 mg/mL surfactant selected from the group consisting of polysorbate 20, polysorbate 40, polysorbate 60, polysorbate 80, poloxamer 182, poloxamer 188, and poloxamer 407.

H952. The composition of H820 or any preceding paragraph dependent thereon, wherein the composition comprises 0.5 mg/mL surfactant selected from the group consisting of polysorbate 20, polysorbate 40, polysorbate 60, polysorbate 80, poloxamer 182, poloxamer 188, and poloxamer 407.

H953. The composition of H820 or any preceding paragraph dependent thereon, wherein the composition comprises 0.8-1.2 mg/mL surfactant selected from the group consisting of polysorbate 20, polysorbate 40, polysorbate 60, polysorbate 80, poloxamer 182, poloxamer 188, and poloxamer 407.

H954. The composition of H820 or any preceding paragraph dependent thereon, wherein the composition comprises 1 mg/mL surfactant selected from the group consisting of polysorbate 20, polysorbate 40, polysorbate 60, polysorbate 80, poloxamer 182, poloxamer 188, and poloxamer 407.

H955. The composition of H820 or any preceding paragraph dependent thereon, wherein the composition comprises 1.2 mg/mL surfactant selected from the group consisting of polysorbate 20, polysorbate 40, polysorbate 60, polysorbate 80, poloxamer 182, poloxamer 188, and poloxamer 407.

H956. The composition of H820 or any preceding paragraph dependent thereon, wherein the composition comprises 0.001-5 mg/mL poloxamer 188.

H957. The composition of H820 or any preceding paragraph dependent thereon, wherein the composition comprises 0.01-2 mg/mL poloxamer 188.

H958. The composition of H820 or any preceding paragraph dependent thereon, wherein the composition comprises 0.1-1.1 mg/mL poloxamer 188.

H959. The composition of H820 or any preceding paragraph dependent thereon, wherein the composition comprises 1.0 mg/mL poloxamer 188.

H960. The composition of H820 or any preceding paragraph dependent thereon, wherein the composition comprises 0.001-0.05 mg/mL poloxamer 188.

H961. The composition of H820 or any preceding paragraph dependent thereon, wherein the composition comprises 0.01 mg/mL poloxamer 188.

H962. The composition of H820 or any preceding paragraph dependent thereon, wherein the composition comprises 0.001-0.15 mg/mL poloxamer 188.

H963. The composition of H820 or any preceding paragraph dependent thereon, wherein the composition comprises 0.1 mg/mL poloxamer 188.

H964. The composition of H820 or any preceding paragraph dependent thereon, wherein the composition comprises 0.1-0.3 mg/mL poloxamer 188.

H965. The composition of H820 or any preceding paragraph dependent thereon, wherein the composition comprises 0.2 mg/mL poloxamer 188.

H966. The composition of H820 or any preceding paragraph dependent thereon, wherein the composition comprises 0.3 mg/mL poloxamer 188.

H967. The composition of H820 or any preceding paragraph dependent thereon, wherein the composition comprises 0.25-0.75 mg/mL poloxamer 188.

(continued)

H968. The composition of H820 or any preceding paragraph dependent thereon, wherein the composition comprises 0.5 mg/mL poloxamer 188.

H969. The composition of H820 or any preceding paragraph dependent thereon, wherein the composition comprises 0.8-1.2 mg/mL poloxamer 188.

H970. The composition of H820 or any preceding paragraph dependent thereon, wherein the composition comprises 1 mg/mL poloxamer 188.

H971. The composition of H820 or any preceding paragraph dependent thereon, wherein the composition comprises 1.2 mg/mL poloxamer 188.

H972. The composition of H820 or any preceding paragraph dependent thereon, wherein the composition comprises 0.001-5 mg/mL polysorbate 20 or polysorbate 80.

H973. The composition of H820 or any preceding paragraph dependent thereon, wherein the composition comprises 0.01-2 mg/mL polysorbate 20 or polysorbate 80.

H974. The composition of H820 or any preceding paragraph dependent thereon, wherein the composition comprises 0.1-1.1 mg/mL polysorbate 20 or polysorbate 80.

H975. The composition of H820 or any preceding paragraph dependent thereon, wherein the composition comprises 0.001-0.05 mg/mL polysorbate 20 or polysorbate 80.

H976. The composition of H820 or any preceding paragraph dependent thereon, wherein the composition comprises 0.01 mg/mL polysorbate 20 or polysorbate 80.

H977. The composition of H820 or any preceding paragraph dependent thereon, wherein the composition comprises 0.001-0.15 mg/mL polysorbate 20 or polysorbate 80.

H978. The composition of H820 or any preceding paragraph dependent thereon, wherein the composition comprises 0.1 mg/mL polysorbate 20 or polysorbate 80.

H979. The composition of H820 or any preceding paragraph dependent thereon, wherein the composition comprises 0.1-0.3 mg/mL polysorbate 20 or polysorbate 80.

H980. The composition of H820 or any preceding paragraph dependent thereon, wherein the composition comprises 0.2 mg/mL polysorbate 20 or polysorbate 80.

H981. The composition of H820 or any preceding paragraph dependent thereon, wherein the composition comprises 0.3 mg/mL polysorbate 20 or polysorbate 80.

H982. The composition of H820 or any preceding paragraph dependent thereon, wherein the composition comprises 0.25-0.75 mg/mL polysorbate 20 or polysorbate 80.

H983. The composition of H820 or any preceding paragraph dependent thereon, wherein the composition comprises 0.5 mg/mL polysorbate 20 or polysorbate 80.

H984. The composition of H820 or any preceding paragraph dependent thereon, wherein the composition comprises 0.8-1.2 mg/mL polysorbate 20 or polysorbate 80.

H985. The composition of H820 or any preceding paragraph dependent thereon, wherein the composition comprises 1 mg/mL polysorbate 20 or polysorbate 80.

H986. The composition of H820 or any preceding paragraph dependent thereon, wherein the composition comprises 1.2 mg/mL polysorbate 20 or polysorbate 80.

H987. The composition of H820 or any preceding paragraph dependent thereon, wherein the composition comprises 0.001-5 mg/mL polysorbate 20.

H988. The composition of H820 or any preceding paragraph dependent thereon, wherein the composition comprises 0.01-2 mg/mL polysorbate 20.

H989. The composition of H820 or any preceding paragraph dependent thereon, wherein the composition comprises 0.1-1.1 mg/mL polysorbate 20.

H990. The composition of H820 or any preceding paragraph dependent thereon, wherein the composition comprises 0.001-0.05 mg/mL polysorbate 20.

H991. The composition of H820 or any preceding paragraph dependent thereon, wherein the composition comprises 0.01 mg/mL polysorbate 20.

H992. The composition of H820 or any preceding paragraph dependent thereon, wherein the composition comprises 0.001-0.15 mg/mL polysorbate 20.

H993. The composition of H820 or any preceding paragraph dependent thereon, wherein the composition comprises 0.1 mg/mL polysorbate 20.

(continued)

H994. The composition of H820 or any preceding paragraph dependent thereon, wherein the composition comprises 0.1-0.3 mg/mL polysorbate 20.

H995. The composition of H820 or any preceding paragraph dependent thereon, wherein the composition comprises 0.2 mg/mL polysorbate 20.

H996. The composition of H820 or any preceding paragraph dependent thereon, wherein the composition comprises 0.3 mg/mL polysorbate 20.

H997. The composition of H820 or any preceding paragraph dependent thereon, wherein the composition comprises 0.25-0.75 mg/mL polysorbate 20.

H998. The composition of H820 or any preceding paragraph dependent thereon, wherein the composition comprises 0.5 mg/mL polysorbate 20.

H999. The composition of H820 or any preceding paragraph dependent thereon, wherein the composition comprises 0.8-1.2 mg/mL polysorbate 20.

H1000. The composition of H820 or any preceding paragraph dependent thereon, wherein the composition comprises 1 mg/mL polysorbate 20.

H1001. The composition of H820 or any preceding paragraph dependent thereon, wherein the composition comprises 1.2 mg/mL polysorbate 20.

H1002. The composition of H820 or any preceding paragraph dependent thereon, wherein the composition comprises 0.001-5 mg/mL polysorbate 80.

H1003. The composition of H820 or any preceding paragraph dependent thereon, wherein the composition comprises 0.01-2 mg/mL polysorbate 80.

H1004. The composition of H820 or any preceding paragraph dependent thereon, wherein the composition comprises 0.1-1.1 mg/mL polysorbate 80.

H1005. The composition of H820 or any preceding paragraph dependent thereon, wherein the composition comprises 0.001-0.05 mg/mL polysorbate 80.

H1006. The composition of H820 or any preceding paragraph dependent thereon, wherein the composition comprises 0.01 mg/mL polysorbate 80.

H1007. The composition of H820 or any preceding paragraph dependent thereon, wherein the composition comprises 0.001-0.15 mg/mL polysorbate 80.

H1008. The composition of H820 or any preceding paragraph dependent thereon, wherein the composition comprises 0.1 mg/mL polysorbate 80.

H1009. The composition of H820 or any preceding paragraph dependent thereon, wherein the composition comprises 0.1-0.3 mg/mL polysorbate 80.

H1010. The composition of H820 or any preceding paragraph dependent thereon, wherein the composition comprises 0.2 mg/mL polysorbate 80.

H1011. The composition of H820 or any preceding paragraph dependent thereon, wherein the composition comprises 0.3 mg/mL polysorbate 80.

H1012. The composition of H820 or any preceding paragraph dependent thereon, wherein the composition comprises 0.25-0.75 mg/mL polysorbate 80.

H1013. The composition of H820 or any preceding paragraph dependent thereon, wherein the composition comprises 0.5 mg/mL polysorbate 80.

H1014. The composition of H820 or any preceding paragraph dependent thereon, wherein the composition comprises 0.8-1.2 mg/mL polysorbate 80.

H1015. The composition of H820 or any preceding paragraph dependent thereon, wherein the composition comprises 1 mg/mL polysorbate 80.

H1016. The composition of H820 or any preceding paragraph dependent thereon, wherein the composition comprises 1.2 mg/mL polysorbate 80.

H1017. The composition of H1-H1016, wherein the composition is characterised by an absence of polysorbate 80.

H1018. The composition of H1-H1017, wherein the composition is characterised by an absence of surfactants polysorbates.

H1019. The composition of H1-H1018, wherein the composition is characterised by an absence of surfactants poloxamers.

H1020. The composition of H1-H1019, wherein the composition is characterised by an absence of surfactants polysorbates and poloxamers .

(continued)

H1021. The composition of H1-H819, wherein the composition is characterised by an absence of a surfactant.

H1022. The composition of H1-H1 016, wherein the composition is characterised by an absence of any, some, or all surfactants defined in H821-H845.

H1023. The composition of H1-H1022, wherein the composition comprises an antioxidant.

H1024. The composition of H1023, wherein the composition comprises (and/or the antioxidant is) a single antioxidant.

H1025. The composition of H1023 or any preceding paragraph dependent thereon, wherein the composition comprises (and/or the antioxidant is) an antioxidant selected from the group consisting of an amino acid antioxidant, a peptide antioxidant, a mineral (or inorganic) antioxidant, a vitamin antioxidant, a carotenoid antioxidant, a polyphenol antioxidant, an aromatic antioxidant, a phenolic antioxidant, a chelating agent antioxidant, a thiol antioxidant, and any combination thereof.

H1026. The composition of H1023 or any preceding paragraph dependent thereon, wherein the composition comprises (and/or the antioxidant is) an amino acid antioxidant.

H1027. The composition of H1023 or any preceding paragraph dependent thereon, wherein the composition comprises (and/or the antioxidant is) an amino acid antioxidant selected from the group consisting of methionine, N-acetyl-I-cysteine, cysteine, glutathione, and any combination thereof.

H1028. The composition of H1023 or any preceding paragraph dependent thereon, wherein the composition comprises (and/or the antioxidant is) an amino acid antioxidant selected from the group consisting of N-acetyl-I-cysteine, cysteine, glutathione, and any combination thereof.

H1029. The composition of H1023 or any preceding paragraph dependent thereon, wherein the composition comprises (and/or the antioxidant is) methionine.

H1030. The composition of H1023 or any preceding paragraph dependent thereon, wherein the composition comprises (and/or the antioxidant is) N-acetyl-I-cysteine.

H1031. The composition of H1023 or any preceding paragraph dependent thereon, wherein the composition comprises (and/or the antioxidant is) cysteine.

H1032. The composition of H1023 or any preceding paragraph dependent thereon, wherein the composition comprises (and/or the antioxidant is) glutathione.

H1033. The composition of H1023 or any preceding paragraph dependent thereon, wherein the composition comprises (and/or the antioxidant is) a mineral (or inorganic) antioxidant selected from the group consisting of a reducing metal compound (such as a compound of magnesium, iron, zinc, copper, and/or manganese), a thiosulfate compound (e.g. metal thiosulfate, ammonium thiosulfate), a bisulfite compound (e.g. metal bisulfite, ammonium bisulfite), a metabisulfite compound (e.g. metal metabisulfite, ammonium metabisulfite), a formaldehyde sulfoxylate (e.g. metal formaldehyde sulfoxylate, ammonium formaldehyde sulfoxylate), a glutamate compound (e.g. metal glutamate, ammonium glutamate, glutamic acid), a thioglycolate compound (e.g. metal thioglycolate, ammonium thioglycolate), a compound of sulfur, a compound of selenium, thiourea, and any combination thereof.

H1034. The composition of H1023 or any preceding paragraph dependent thereon, wherein the composition comprises (and/or the antioxidant is) a mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1035. The composition of H1023 or any preceding paragraph dependent thereon, wherein the composition comprises (and/or the antioxidant is) a mineral (or inorganic) antioxidant selected from the group consisting of a reducing metal compound (such as a compound of magnesium, iron, zinc, copper, and/or manganese), sodium thiosulfate, sodium bisulfite, sodium metabisulfite, sodium formaldehyde sulfoxylate, monosodium glutamate, sodium thioglycolate, a compound of sulfur, a compound of selenium, thiourea, and any combination thereof.

H1036. The composition of H1023 or any preceding paragraph dependent thereon, wherein the composition comprises (and/or the antioxidant is) a mineral (or inorganic) antioxidant selected from the group consisting of sodium thiosulfate, sodium bisulfite, sodium metabisulfite, sodium formaldehyde sulfoxylate, and any combination thereof.

H1037. The composition of H1023 or any preceding paragraph dependent thereon, wherein the composition comprises (and/or the antioxidant is) a thiosulfate.

(continued)

H1038. The composition of H1023 or any preceding paragraph dependent thereon, wherein the composition comprises (and/or the antioxidant is) sodium thiosulfate.

H1039. The composition of H1023 or any preceding paragraph dependent thereon, wherein the composition comprises (and/or the antioxidant is) a vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1040. The composition of H1023 or any preceding paragraph dependent thereon, wherein the composition comprises (and/or the antioxidant is) ascorbic acid (and/or any salt or ester thereof).

H1041. The composition of H1023 or any preceding paragraph dependent thereon, wherein the composition comprises (and/or the antioxidant is) a carotenoid antioxidant selected from the group consisting of beta-carotene, lycopene, lutein, and zeaxanthin, and any combination thereof.

H1042. The composition of H1023 or any preceding paragraph dependent thereon, wherein the composition comprises (and/or the antioxidant is) a polyphenol antioxidant selected from the group consisting of phenolic acids, flavonoids, gingerol, curcumin, resveratrol, quercetin, and any combination thereof.

H1043. The composition of H1023 or any preceding paragraph dependent thereon, wherein the composition comprises (and/or the antioxidant is) an aromatic and/or phenolic antioxidant selected from the group consisting of butylated hydroxytoluene, butylated hydroxy anisole, gentisic acid, propyl gallate, and any combination thereof.

H1044. The composition of H1023 or any preceding paragraph dependent thereon, wherein the composition comprises (and/or the antioxidant is) a chelating agent antioxidant selected from EDTA (and/or any salt thereof) and/or pentetic acid (and/or any salt thereof).

H1045. The composition of H1023 or any preceding paragraph dependent thereon, wherein the composition comprises (and/or the antioxidant is) EDTA (and/or any salt thereof).

H1046. The composition of H1023 or any preceding paragraph dependent thereon, wherein the composition comprises (and/or the antioxidant is) pentetic acid (and/or any salt thereof).

H1047. The composition of H1023 or any preceding paragraph dependent thereon, wherein the composition comprises (and/or the antioxidant is) monothioglycerol.

H1048. The composition of H1023 or any preceding paragraph dependent thereon, wherein the composition comprises (and/or the antioxidant is) an antioxidant selected from the group consisting of a thiosulfate, pentetic acid (and/or any salt thereof), cysteine, glutathione, and ascorbic acid.

H1049. The composition of H1023 or any preceding paragraph dependent thereon, wherein the composition comprises (and/or the antioxidant is) an antioxidant selected from the group consisting of a thiosulfate, pentetic acid (and/or any salt thereof), cysteine, and ascorbic acid.

H1050. The composition of H1023 or any preceding paragraph dependent thereon, wherein the composition comprises (and/or the antioxidant is) an antioxidant selected from the group consisting of a thiosulfate, pentetic acid (and/or any salt thereof), and cysteine .

H1051. The composition of H1023 or any preceding paragraph dependent thereon, wherein the composition comprises (and/or the antioxidant is) an antioxidant selected from thiosulfate and/or pentetic acid (and/or any salt thereof).

H1052. The composition of H1023 or any preceding paragraph dependent thereon, wherein the composition comprises 0.001-300 mM antioxidant.

H1053. The composition of H1023 or any preceding paragraph dependent thereon, wherein the composition comprises 0.01-50 mM antioxidant.

H1054. The composition of H1023 or any preceding paragraph dependent thereon, wherein the composition comprises 0.1-20 mM antioxidant.

H1055. The composition of H1023 or any preceding paragraph dependent thereon, wherein the composition comprises less than 2.5 mM antioxidant.

H1056. The composition of H1023 or any preceding paragraph dependent thereon, wherein the composition comprises 0.001-2.4 mM antioxidant.

H1057 . The composition of H1023 or any preceding paragraph dependent thereon, wherein the composition comprises 0.01-2 mM antioxidant.

H1058 . The composition of H1023 or any preceding paragraph dependent thereon, wherein the composition comprises 0.1-1 mM antioxidant.

(continued)

H1059 . The composition of H1023 or any preceding paragraph dependent thereon, wherein the composition comprises 0.5 mM antioxidant.

H1060 . The composition of H1023 or any preceding paragraph dependent thereon, wherein the composition comprises 1-4 mM antioxidant.

H1061 . The composition of H1023 or any preceding paragraph dependent thereon, wherein the composition comprises 2 mM antioxidant.

H1062 . The composition of H1023 or any preceding paragraph dependent thereon, wherein the composition comprises 6-10 mM antioxidant.

H1063 . The composition of H1023 or any preceding paragraph dependent thereon, wherein the composition comprises 7 mM antioxidant.

H1064 . The composition of H1023 or any preceding paragraph dependent thereon, wherein the composition comprises 1-10 mM antioxidant.

H1065 . The composition of H1023 or any preceding paragraph dependent thereon, wherein the composition comprises 5 mM antioxidant.

H1066 . The composition of H1023 or any preceding paragraph dependent thereon, wherein the composition comprises more than 20 mM antioxidant.

H1067 . The composition of H1023 or any preceding paragraph dependent thereon, wherein the composition comprises 21-300 mM antioxidant.

H1068 . The composition of H1023 or any preceding paragraph dependent thereon, wherein the composition comprises 200 mM antioxidant.

H1069 . The composition of H1023 or any preceding paragraph dependent thereon, wherein the composition comprises 0.001-300 mM amino acid antioxidant selected from the group consisting of N-acetyl-l-cysteine, cysteine, glutathione, and any combination thereof.

H1070 . The composition of H1023 or any preceding paragraph dependent thereon, wherein the composition comprises 0.1-20 mM amino acid antioxidant selected from the group consisting of N-acetyl-l-cysteine, cysteine, glutathione, and any combination thereof .

H1071 . The composition of H1023 or any preceding paragraph dependent thereon, wherein the composition comprises 0.001-2.4 mM amino acid antioxidant selected from the group consisting of N-acetyl-l-cysteine, cysteine, glutathione, and any combination thereof.

H1072 . The composition of H1023 or any preceding paragraph dependent thereon, wherein the composition comprises 0.5 mM amino acid antioxidant selected from the group consisting of N-acetyl-l-cysteine, cysteine, glutathione, and any combination thereof .

H1073 . The composition of H1023 or any preceding paragraph dependent thereon, wherein the composition comprises 7 mM amino acid antioxidant selected from the group consisting of N-acetyl-l-cysteine, cysteine, glutathione, and any combination thereof .

H1074. The composition of H1023 or any preceding paragraph dependent thereon, wherein the composition comprises 1-10 mM amino acid antioxidant selected from the group consisting of N-acetyl-l-cysteine, cysteine, glutathione, and any combination thereof .

H1075 . The composition of H1023 or any preceding paragraph dependent thereon, wherein the composition comprises 2 mM amino acid antioxidant selected from the group consisting of N-acetyl-l-cysteine, cysteine, glutathione, and any combination thereof .

H1076 . The composition of H1023 or any preceding paragraph dependent thereon, wherein the composition comprises 5 mM amino acid antioxidant selected from the group consisting of N-acetyl-l-cysteine, cysteine, glutathione, and any combination thereof .

H1077 . The composition of H1023 or any preceding paragraph dependent thereon, wherein the composition comprises 21-300 mM amino acid antioxidant selected from the group consisting of N-acetyl-l-cysteine, cysteine, glutathione, and any combination thereof .

H1078 . The composition of H1023 or any preceding paragraph dependent thereon, wherein the composition comprises 200 mM amino acid antioxidant selected from the group consisting of N-acetyl-l-cysteine, cysteine, glutathione, and any combination thereof .

(continued)

H1079 . The composition of H1023 or any preceding paragraph dependent thereon, wherein the composition comprises 0.001-300 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1080 . The composition of H1023 or any preceding paragraph dependent thereon, wherein the composition comprises 0.1-20 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1081 . The composition of H1023 or any preceding paragraph dependent thereon, wherein the composition comprises 0.001-2.4 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1082 . The composition of H1023 or any preceding paragraph dependent thereon, wherein the composition comprises 0.5 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1083 . The composition of H1023 or any preceding paragraph dependent thereon, wherein the composition comprises 7 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1084 . The composition of H1023 or any preceding paragraph dependent thereon, wherein the composition comprises 1-10 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1085 . The composition of H1023 or any preceding paragraph dependent thereon, wherein the composition comprises 5 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1086 . The composition of H1023 or any preceding paragraph dependent thereon, wherein the composition comprises 21-300 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

H1087 . The composition of H1023 or any preceding paragraph dependent thereon, wherein the composition comprises 200 mM mineral (or inorganic) antioxidant selected from the group consisting of a reducing metal compound (such as a compound of magnesium, iron, zinc, copper, and/or manganese), a metal thiosulfate, a metal bisulfite, a metal metabisulfite, a metal formaldehyde sulfoxylate, a metal glutamate, a metal thioglycolate, a compound of sulfur, a compound of selenium, thiourea, and any combination thereof.

(continued)

H1088. The composition of H1023 or any preceding paragraph dependent thereon, wherein the composition comprises 0.001-300 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1089. The composition of H1023 or any preceding paragraph dependent thereon, wherein the composition comprises 0.1-20 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1090. The composition of H1023 or any preceding paragraph dependent thereon, wherein the composition comprises 0.001-2.4 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1091. The composition of H1023 or any preceding paragraph dependent thereon, wherein the composition comprises 0.5 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1092. The composition of H1023 or any preceding paragraph dependent thereon, wherein the composition comprises 7 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1093. The composition of H1023 or any preceding paragraph dependent thereon, wherein the composition comprises 1-10 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1094. The composition of H1023 or any preceding paragraph dependent thereon, wherein the composition comprises 5 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1095. The composition of H1023 or any preceding paragraph dependent thereon, wherein the composition comprises 21-300 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1096. The composition of H1023 or any preceding paragraph dependent thereon, wherein the composition comprises 200 mM vitamin antioxidant selected from the group consisting of vitamin A (retinol, 3,4-didehydroretinol, and 3-hydroxyretinol), vitamin C (ascorbic acid and salts or esters thereof, ascorbyl palmitate), vitamin E (tocopherols: e.g. alpha-tocopherol, beta-tocopherol, gamma-tocopherol, delta-tocopherol), and any combination thereof.

H1097. The composition of H1023 or any preceding paragraph dependent thereon, wherein the composition comprises 0.001-300 mM thiosulfate (especially sodium thiosulfate).

H1098. The composition of H1023 or any preceding paragraph dependent thereon, wherein the composition comprises 0.1-20 mM thiosulfate (especially sodium thiosulfate).

H1099. The composition of H1023 or any preceding paragraph dependent thereon, wherein the composition comprises 0.001-2.4 mM thiosulfate (especially sodium thiosulfate).

H1100. The composition of H1023 or any preceding paragraph dependent thereon, wherein the composition comprises 0.5 mM thiosulfate (especially sodium thiosulfate).

(continued)

H1101. The composition of H1023 or any preceding paragraph dependent thereon, wherein the composition comprises 7 mM thiosulfate (especially sodium thiosulfate).

H1102. The composition of H1023 or any preceding paragraph dependent thereon, wherein the composition comprises 1-10 mM thiosulfate (especially sodium thiosulfate).

H1103. The composition of H1023 or any preceding paragraph dependent thereon, wherein the composition comprises 5 mM thiosulfate (especially sodium thiosulfate).

H1104 . The composition of H1023 or any preceding paragraph dependent thereon, wherein the composition comprises 21-300 mM thiosulfate (especially sodium thiosulfate).

H1105 . The composition of H1023 or any preceding paragraph dependent thereon, wherein the composition comprises 200 mM thiosulfate (especially sodium thiosulfate).

H1106 . The composition of H1023 or any preceding paragraph dependent thereon, wherein the composition comprises 0.001-300 mM methionine.

H1107 . The composition of H1023 or any preceding paragraph dependent thereon, wherein the composition comprises 0.1-20 mM methionine.

H1108 . The composition of H1023 or any preceding paragraph dependent thereon, wherein the composition comprises 0.001-2.4 mM methionine.

H1109 . The composition of H1023 or any preceding paragraph dependent thereon, wherein the composition comprises 0.5 mM methionine.

H1110 . The composition of H1023 or any preceding paragraph dependent thereon, wherein the composition comprises 7 mM methionine.

H1111 . The composition of H1023 or any preceding paragraph dependent thereon, wherein the composition comprises 1-10 mM methionine.

H1112 . The composition of H1023 or any preceding paragraph dependent thereon, wherein the composition comprises 5 mM methionine.

H1113 . The composition of H1023 or any preceding paragraph dependent thereon, wherein the composition comprises 0.001-300 mM cysteine.

H1114 . The composition of H1023 or any preceding paragraph dependent thereon, wherein the composition comprises 0.1-20 mM cysteine.

H1115 . The composition of H1023 or any preceding paragraph dependent thereon, wherein the composition comprises 0.001-2.4 mM cysteine.

H1116 . The composition of H1023 or any preceding paragraph dependent thereon, wherein the composition comprises 0.5 mM cysteine.

H1117 . The composition of H1023 or any preceding paragraph dependent thereon, wherein the composition comprises 2 mM cysteine.

H1118 . The composition of H1023 or any preceding paragraph dependent thereon, wherein the composition comprises 1-10 mM cysteine.

H1119 . The composition of H1023 or any preceding paragraph dependent thereon, wherein the composition comprises 5 mM cysteine.

H1120 . The composition of H1023 or any preceding paragraph dependent thereon, wherein the composition comprises 0.001-300 mM EDTA (and/or any salt thereof).

H1121. The composition of H1023 or any preceding paragraph dependent thereon, wherein the composition comprises 0.1-20 mM EDTA (and/or any salt thereof).

H1122 . The composition of H1023 or any preceding paragraph dependent thereon, wherein the composition comprises 0.001-2.4 mM EDTA (and/or any salt thereof).

H1123 . The composition of H1023 or any preceding paragraph dependent thereon, wherein the composition comprises 0.5 mM EDTA (and/or any salt thereof).

H1124 . The composition of H1023 or any preceding paragraph dependent thereon, wherein the composition comprises 7 mM EDTA (and/or any salt thereof).

H1125 . The composition of H1023 or any preceding paragraph dependent thereon, wherein the composition comprises 1-10 mM EDTA (and/or any salt thereof).

H1126 . The composition of H1023 or any preceding paragraph dependent thereon, wherein the composition comprises 2 mM EDTA (and/or any salt thereof).

(continued)

H1127 . The composition of H1023 or any preceding paragraph dependent thereon, wherein the composition comprises 5 mM EDTA (and/or any salt thereof).

H1128 The composition of H1023 or any preceding paragraph dependent thereon, wherein the composition comprises 21-300 mM EDTA (and/or any salt thereof).

H1129. The composition of H1023 or any preceding paragraph dependent thereon, wherein the composition comprises 200 mM EDTA (and/or any salt thereof).

H1130. The composition of H1023 or any preceding paragraph dependent thereon, wherein the composition comprises 0.001-300 mM pentetic acid (and/or any salt thereof).

**H1131**. The composition of H1023 or any preceding paragraph dependent thereon, wherein the composition comprises 0.1-20 mM pentetic acid (and/or any salt thereof).

H1132. The composition of H1023 or any preceding paragraph dependent thereon, wherein the composition comprises 0.001-2.4 mM pentetic acid (and/or any salt thereof).

H1133. The composition of H1023 or any preceding paragraph dependent thereon, wherein the composition comprises 0.5 mM pentetic acid (and/or any salt thereof).

H1134. The composition of H1023 or any preceding paragraph dependent thereon, wherein the composition comprises 7 mM pentetic acid (and/or any salt thereof).

H1135. The composition of H1023 or any preceding paragraph dependent thereon, wherein the composition comprises 1-10 mM pentetic acid (and/or any salt thereof).

H1136. The composition of H1023 or any preceding paragraph dependent thereon, wherein the composition comprises 5 mM pentetic acid (and/or any salt thereof).

H1137. The composition of H1023 or any preceding paragraph dependent thereon, wherein the composition comprises 21-300 mM pentetic acid (and/or any salt thereof).

H1138. The composition of H1023 or any preceding paragraph dependent thereon, wherein the composition comprises 200 mM pentetic acid (and/or any salt thereof).

H1139. The composition of H1-H1138, wherein the composition is characterised by an absence of methionine.

H1140. The composition of H1-H1022, wherein the composition is characterised by an absence of an antioxidant.

H1141. The composition of H1-H1138, wherein the composition is characterised by an absence of any, some, or all antioxidants defined in H1024-H1051.

H1142. The composition of H1-H1141, wherein the composition comprises a tonicifier.

H1143. The composition of H1142, wherein the composition comprises (and/or the tonicifier is) a further tonicifier (i.e. additional to any other already-specified components/ingredients, such as sugar component(s), amino acid component(s), buffer system(s), antioxidant(s), etc., that also happen to contribute to osmolality/ tonicity).

H1144. The composition of H1142 of any preceding paragraph dependent thereon, wherein the composition comprises (and/or the tonicifier is) a tonicifier that is a further tonicifier (i.e. additional to any other already-specified components/ingredients that also happen to contribute to osmolality/tonicity).

H1145. The composition of H1142 of any preceding paragraph dependent thereon, wherein the composition comprises (and/or the tonicifier is) a tonicifier that contributes to (or increases) overall osmolality and osmolarity.

H1146. The composition of H1142 of any preceding paragraph dependent thereon, wherein the composition comprises (and/or the tonicifier is) a tonicifier in a quantity or concentration sufficient for the composition to be (substantially) isotonic with body fluids (especially blood, especially blood plasma).

H1147. The composition of H1142 of any preceding paragraph dependent thereon, wherein the composition comprises (and/or the tonicifier is) a tonicifier in a quantity or concentration sufficient for the composition to have an osmolarity or osmolality within a range defined herein.

H1148. The composition of H1142 of any preceding paragraph dependent thereon, wherein the composition comprises (and/or the tonicifier is) a (further) tonicifier that consists of one or more (further) tonicifiers.

H1149. The composition of H1142 of any preceding paragraph dependent thereon, wherein the composition comprises (and/or the tonicifier is) a single (further) tonicifier.

H1150. The composition of H1142 of any preceding paragraph dependent thereon, wherein the composition comprises (and/or the tonicifier is) a tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar

(continued)

alcohols defined herein in relation to a sugar component), an (or one or more) amino acid tonicifier (e.g. including any amino acids defined herein in relation to an amino acid component), and any combination thereof.

H1151. The composition of H1142 of any preceding paragraph dependent thereon, wherein the composition comprises (and/or the tonicifier is) a (or one or more) metal salt tonicifier.

H1152. The composition of H1142 of any preceding paragraph dependent thereon, wherein the composition comprises (and/or the tonicifier is) a (or one or more) non-metal salt tonicifier.

H1153. The composition of H1142 of any preceding paragraph dependent thereon, wherein the composition comprises (and/or the tonicifier is) a (or one or more) polyol tonicifier.

H1154. The composition of H1142 of any preceding paragraph dependent thereon, wherein the composition comprises (and/or the tonicifier is) an (or one or more) amino acid tonicifier.

H1155. The composition of H1142 of any preceding paragraph dependent thereon, wherein the composition comprises (and/or the tonicifier is) a metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1156. The composition of H1142 of any preceding paragraph dependent thereon, wherein the composition comprises (and/or the tonicifier is) sodium chloride.

H1157. The composition of H1142 of any preceding paragraph dependent thereon, wherein the composition comprises (and/or the tonicifier is) a non-metal salt tonicifier selected from the group consisting of ammonium chloride, ammonium formate, ammonium acetate, histidine hydrochloride, and any combination thereof.

H1158. The composition of H1142 of any preceding paragraph dependent thereon, wherein the composition comprises (and/or the tonicifier is) ammonium acetate.

H1159. The composition of H1142 of any preceding paragraph dependent thereon, wherein the composition comprises (and/or the tonicifier is) a polyol tonicifier selected from the group consisting of mannitol, sorbitol, maltose, glucose, lactose, and any combination thereof.

H1160. The composition of H1142 of any preceding paragraph dependent thereon, wherein the composition comprises (and/or the tonicifier is) maltose.

H1161. The composition of H1142 of any preceding paragraph dependent thereon, wherein the composition comprises (and/or the tonicifier is) an amino acid tonicifier selected from the group consisting of arginine, glycine, histidine, lysine, aspartic acid, glutamic acid, and any combination thereof.

H1162. The composition of H1142 of any preceding paragraph dependent thereon, wherein the composition comprises (and/or the tonicifier is) arginine.

H1163. The composition of H1142 of any preceding paragraph dependent thereon, wherein the composition comprises (and/or the tonicifier is) lysine.

H1164. The composition of H1142 of any preceding paragraph dependent thereon, wherein the composition comprises (and/or the tonicifier is) a (one or more) non-buffering tonicifier(s) that is either buffering or non-buffering (e.g. at the prevailing pH).

H1165. The composition of H1142 of any preceding paragraph dependent thereon, wherein the composition comprises (and/or the tonicifier is) a (one or more) non-buffering tonicifier(s) (i.e. which imparts substantially no buffering effect, or does not substantially contribute to buffering, especially at the prevailing pH).

H1166. The composition of H1142 of any preceding paragraph dependent thereon, wherein the composition comprises (and/or the tonicifier is) a (one or more) buffering tonicifier(s) (i.e. which may impart a buffering effect, or may contribute to buffering, especially at the prevailing pH).

H1167. The composition of H1142 of any preceding paragraph dependent thereon, wherein the composition comprises 5-300 mM tonicifier.

H1168. The composition of H1142 of any preceding paragraph dependent thereon, wherein the composition comprises less than 130 mM tonicifier.

H1169. The composition of H1142 of any preceding paragraph dependent thereon, wherein the composition comprises 5-300 mM tonicifier, provided that no single tonicifier compound exceeds 129 mM.

H1170. The composition of H1142 of any preceding paragraph dependent thereon, wherein the composition comprises 10-129 mM tonicifier.

(continued)

H1171. The composition of H1142 of any preceding paragraph dependent thereon, wherein the composition comprises 20-120 mM tonicifier.

H1172 . The composition of H1142 of any preceding paragraph dependent thereon, wherein the composition comprises 20-50 mM tonicifier.

H1173 . The composition of H1142 of any preceding paragraph dependent thereon, wherein the composition comprises 30 mM tonicifier.

H1174 . The composition of H1142 of any preceding paragraph dependent thereon, wherein the composition comprises 50-100 mM tonicifier.

H1175 . The composition of H1142 of any preceding paragraph dependent thereon, wherein the composition comprises 60 mM tonicifier.

H1176 . The composition of H1142 of any preceding paragraph dependent thereon, wherein the composition comprises 100-129 mM tonicifier.

H1177 . The composition of H1142 of any preceding paragraph dependent thereon, wherein the composition comprises 105 mM tonicifier.

H1178 . The composition of H1142 of any preceding paragraph dependent thereon, wherein the composition comprises less than 200 mM tonicifier.

H1179 . The composition of H1142 of any preceding paragraph dependent thereon, wherein the composition comprises 5-300 mM tonicifier, provided that no single tonicifier compound exceeds 199 mM.

H1180 . The composition of H1142 of any preceding paragraph dependent thereon, wherein the composition comprises 10-199 mM tonicifier.

H1181 . The composition of H1142 of any preceding paragraph dependent thereon, wherein the composition comprises 130-199 mM tonicifier.

H1182 . The composition of H1142 of any preceding paragraph dependent thereon, wherein the composition comprises 170 mM tonicifier.

H1183 . The composition of H1142 of any preceding paragraph dependent thereon, wherein the composition comprises 5-300 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1184 . The composition of H1142 of any preceding paragraph dependent thereon, wherein the composition comprises 10-129 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1185 . The composition of H1142 of any preceding paragraph dependent thereon, wherein the composition comprises 50-100 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1186 . The composition of H1142 of any preceding paragraph dependent thereon, wherein the composition comprises 60 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1187 . The composition of H1142 of any preceding paragraph dependent thereon, wherein the composition comprises 105 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

(continued)

H1188. The composition of H1142 of any preceding paragraph dependent thereon, wherein the composition comprises 130-199 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1189. The composition of H1142 of any preceding paragraph dependent thereon, wherein the composition comprises 170 mM (further) tonicifier selected from the group consisting of a (or one or more) metal salt tonicifier, a (or one or more) non-metal salt tonicifier, a (or one or more) polyol tonicifier (e.g. a sugar or sugar alcohol, including any of those sugars or sugar alcohols defined herein in relation to a sugar component), an (or one or more) amino acid tonicifier (e.g. including any amino acids defined herein in relation to an amino acid component), and any combination thereof.

H1190. The composition of H1142 of any preceding paragraph dependent thereon, wherein the composition comprises 5-300 mM metal salt tonicifier.

H1191. The composition of H1142 of any preceding paragraph dependent thereon, wherein the composition comprises 10-129 mM metal salt tonicifier.

H1192. The composition of H1142 of any preceding paragraph dependent thereon, wherein the composition comprises 50-100 mM metal salt tonicifier.

H1193. The composition of H1142 of any preceding paragraph dependent thereon, wherein the composition comprises 60 mM metal salt tonicifier.

H1194. The composition of H1142 of any preceding paragraph dependent thereon, wherein the composition comprises 105 mM metal salt tonicifier.

H1195. The composition of H1142 of any preceding paragraph dependent thereon, wherein the composition comprises 130-199 mM metal salt tonicifier.

H1196. The composition of H1142 of any preceding paragraph dependent thereon, wherein the composition comprises 170 mM metal salt tonicifier.

H1197. The composition of H1142 of any preceding paragraph dependent thereon, wherein the composition comprises 5-300 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1198. The composition of H1142 of any preceding paragraph dependent thereon, wherein the composition comprises 10-129 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1199. The composition of H1142 of any preceding paragraph dependent thereon, wherein the composition comprises 50-100 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1200. The composition of H1142 of any preceding paragraph dependent thereon, wherein the composition comprises 60 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1201. The composition of H1142 of any preceding paragraph dependent thereon, wherein the composition comprises 105 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1202. The composition of H1142 of any preceding paragraph dependent thereon, wherein the composition comprises 130-199 mM metal salt tonicifier selected from the group consisting of sodium chloride, potassium chloride, magnesium chloride, calcium chloride, sodium acetate, sodium citrate, monosodium glutamate, sodium phosphate, potassium phosphate, and any combination thereof.

H1203. The composition of H1142 of any preceding paragraph dependent thereon, wherein the composition comprises 170 mM metal salt tonicifier selected from the group consisting of sodium chloride, potassium chloride, magnesium chloride, calcium

(continued)

chloride, sodium acetate, sodium citrate, monosodium glutamate, sodium phosphate, potassium phosphate, and any combination thereof.

H1204 . The composition of H1142 of any preceding paragraph dependent thereon, wherein the composition comprises 5-300 mM (further) non-buffering tonicifier.

H1205 . The composition of H1142 of any preceding paragraph dependent thereon, wherein the composition comprises 10-129 mM (further) non-buffering tonicifier.

H1206 . The composition of H1142 of any preceding paragraph dependent thereon, wherein the composition comprises 50-100 mM (further) non-buffering tonicifier.

H1207 . The composition of H1142 of any preceding paragraph dependent thereon, wherein the composition comprises 60 mM (further) non-buffering tonicifier.

H1208 . The composition of H1142 of any preceding paragraph dependent thereon, wherein the composition comprises 105 mM (further) non-buffering tonicifier.

H1209 . The composition of H1142 of any preceding paragraph dependent thereon, wherein the composition comprises 130-199 mM (further) non-buffering tonicifier.

H1210 . The composition of H1142 of any preceding paragraph dependent thereon, wherein the composition comprises 170 mM (further) non-buffering tonicifier.

H1211. The composition of H1142 of any preceding paragraph dependent thereon, wherein the composition comprises 5-300 mM sodium chloride.

H1212 . The composition of H1142 of any preceding paragraph dependent thereon, wherein the composition comprises 10-129 mM sodium chloride.

H1213 . The composition of H1142 of any preceding paragraph dependent thereon, wherein the composition comprises 50-100 mM sodium chloride.

H1214 . The composition of H1142 of any preceding paragraph dependent thereon, wherein the composition comprises 60 mM sodium chloride.

H1215 . The composition of H1142 of any preceding paragraph dependent thereon, wherein the composition comprises 105 mM sodium chloride.

H1216 . The composition of H1142 of any preceding paragraph dependent thereon, wherein the composition comprises 130-199 mM sodium chloride.

H1217 . The composition of H1142 of any preceding paragraph dependent thereon, wherein the composition comprises 170 mM sodium chloride.

H1218 . The composition of H1-H1141, wherein the composition is characterised by an absence of any further additional tonicifiers.

H1219 . The composition of H1-H1217, wherein the composition is characterised by an absence of metal halides.

H1220 . The composition of H1-H1217, wherein the composition is characterised by an absence of sodium chloride.

H1221 . The composition of H1-H1141, wherein the composition is characterised by an absence of a (further) tonicifier.

H1222. The composition of H1-H1217, wherein the composition is characterised by an absence of any, some, or all (further) tonicifiers defined H1143-H1166.

H1223. The composition of H1-H1222, wherein the composition comprises an ionic strength provider.

H1224. The composition of H1223, wherein the composition comprises (and/or the ionic strength provider is) a further ionic strength provider (i.e. additional to any other already-specified components/ingredients, such as buffer system(s), that also happen to contribute to ionic strength).

H1225 . The composition of H1223 or any preceding paragraph dependent thereon, wherein the composition comprises (and/or the ionic strength provider is) an ionic strength provider that is a further ionic strength provider (i.e. additional to any other already-specified components/ingredients that also happen to contribute to ionic strength).

H1226 . The composition of H1223 or any preceding paragraph dependent thereon, wherein the composition comprises (and/or the ionic strength provider is) an ionic strength provider in a quantity or concentration sufficient to provide the composition with an overall molar ionic strength (or molar ionic strength range) as defined herein.

H1227 . The composition of H1223 or any preceding paragraph dependent thereon, wherein the composition comprises (and/or the ionic strength provider is) an ionic strength provider that contributes 2-200 mM of ionic strength.

(continued)

H1228. The composition of H1223 or any preceding paragraph dependent thereon, wherein the composition comprises (and/or the ionic strength provider is) an ionic strength provider that contributes 5-120 mM of ionic strength.

H1229. The composition of H1223 or any preceding paragraph dependent thereon, wherein the composition comprises (and/or the ionic strength provider is) an ionic strength provider that contributes 10-100 mM of ionic strength.

H1230. The composition of H1223 or any preceding paragraph dependent thereon, wherein the composition comprises (and/or the ionic strength provider is) an ionic strength provider that contributes 20-80 mM of ionic strength.

H1231. The composition of H1223 or any preceding paragraph dependent thereon, wherein the composition comprises (and/or the ionic strength provider is) an ionic strength provider that contributes 50-70 mM of ionic strength.

H1232. The composition of H1223 or any preceding paragraph dependent thereon, wherein the composition comprises (and/or the ionic strength provider is) an ionic strength provider that consists of one or more ionic strength provider compounds.

H1233. The composition of H1223 or any preceding paragraph dependent thereon, wherein the composition comprises (and/or the ionic strength provider is) a single (further) ionic strength provider.

H1234. The composition of H1223 or any preceding paragraph dependent thereon, wherein the composition comprises (and/or the ionic strength provider is) a salt.

H1235. The composition of H1223 or any preceding paragraph dependent thereon, wherein the composition comprises (and/or the ionic strength provider is) an ionic strength provider selected from the group consisting of a (or one or more) metal salt ionic strength provider, a (or one or more) non-metal salt ionic strength provider, and any combination thereof.

H1236. The composition of H1223 or any preceding paragraph dependent thereon, wherein the composition comprises (and/or the ionic strength provider is) a metal salt.

H1237. The composition of H1223 or any preceding paragraph dependent thereon, wherein the composition comprises (and/or the ionic strength provider is) a non-metal salt.

H1238. The composition of H1223 or any preceding paragraph dependent thereon, wherein the composition comprises (and/or the ionic strength provider is) a metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1239. The composition of H1223 or any preceding paragraph dependent thereon, wherein the composition comprises (and/or the ionic strength provider is) sodium chloride.

H1240. The composition of H1223 or any preceding paragraph dependent thereon, wherein the composition comprises (and/or the ionic strength provider is) a non-metal salt ionic strength provider selected from the group consisting of ammonium chloride, ammonium formate, ammonium acetate, histidine hydrochloride, and any combination thereof.

H1241. The composition of H1223 or any preceding paragraph dependent thereon, wherein the composition comprises (and/or the ionic strength provider is) ammonium acetate.

H1242. The composition of H1223 or any preceding paragraph dependent thereon, wherein the composition comprises (and/or the ionic strength provider is) a (one or more) non-buffering ionic strength provider(s) that is either buffering or non-buffering (e.g. at the prevailing pH).

H1243. The composition of H1223 or any preceding paragraph dependent thereon, wherein the composition comprises (and/or the ionic strength provider is) a (one or more) non-buffering ionic strength provider(s) (i.e. which imparts substantially no buffering effect, or does not substantially contribute to buffering, especially at the prevailing pH).

H1244. The composition of H1223 or any preceding paragraph dependent thereon, wherein the composition comprises (and/or the ionic strength provider is) a (one or more) buffering ionic strength provider (s) (i.e. which may impart a buffering effect, or may contribute to buffering, especially at the prevailing pH).

H1245. The composition of H1223 or any preceding paragraph dependent thereon, wherein the composition comprises 5-300 mM ionic strength provider.

H1246. The composition of H1223 or any preceding paragraph dependent thereon, wherein the composition comprises less than 130 mM ionic strength provider.

(continued)

H1247. The composition of H1223 or any preceding paragraph dependent thereon, wherein the composition comprises 5-300 mM ionic strength provider, provided that no single ionic strength provider compound exceeds 129 mM.

H1248. The composition of H1223 or any preceding paragraph dependent thereon, wherein the composition comprises 10-129 mM ionic strength provider.

H1249. The composition of H1223 or any preceding paragraph dependent thereon, wherein the composition comprises 20-120 mM ionic strength provider.

H1250. The composition of H1223 or any preceding paragraph dependent thereon, wherein the composition comprises 20-50 mM ionic strength provider.

H1251. The composition of H1223 or any preceding paragraph dependent thereon, wherein the composition comprises 30 mM ionic strength provider.

H1252 . The composition of H1223 or any preceding paragraph dependent thereon, wherein the composition comprises 50-100 mM ionic strength provider.

H1253 . The composition of H1223 or any preceding paragraph dependent thereon, wherein the composition comprises 60 mM ionic strength provider.

H1254 . The composition of H1223 or any preceding paragraph dependent thereon, wherein the composition comprises 100-129 mM ionic strength provider.

H1255 . The composition of H1223 or any preceding paragraph dependent thereon, wherein the composition comprises 105 mM ionic strength provider.

H1256 . The composition of H1223 or any preceding paragraph dependent thereon, wherein the composition comprises less than 200 mM ionic strength provider.

H1257 . The composition of H1223 or any preceding paragraph dependent thereon, wherein the composition comprises 5-300 mM ionic strength provider, provided that no single ionic strength provider compound exceeds 199 mM.

H1258 . The composition of H1223 or any preceding paragraph dependent thereon, wherein the composition comprises 10-199 mM ionic strength provider.

H1259 . The composition of H1223 or any preceding paragraph dependent thereon, wherein the composition comprises 130-199 mM ionic strength provider.

H1260 . The composition of H1223 or any preceding paragraph dependent thereon, wherein the composition comprises 170 mM ionic strength provider.

H1261. The composition of H1223 or any preceding paragraph dependent thereon, wherein the composition comprises 5-300 mM of a salt.

H1262 . The composition of H1223 or any preceding paragraph dependent thereon, wherein the composition comprises 10-129 mM of a salt.

H1263 . The composition of H1223 or any preceding paragraph dependent thereon, wherein the composition comprises 50-100 mM of a salt.

H1264 . The composition of H1223 or any preceding paragraph dependent thereon, wherein the composition comprises 60 mM of a salt.

H1265 . The composition of H1223 or any preceding paragraph dependent thereon, wherein the composition comprises 105 mM of a salt.

H1266 . The composition of H1223 or any preceding paragraph dependent thereon, wherein the composition comprises 130-199 mM of a salt.

H1267 . The composition of H1223 or any preceding paragraph dependent thereon, wherein the composition comprises 170 mM of a salt.

H1268 . The composition of H1223 or any preceding paragraph dependent thereon, wherein the composition comprises 5-300 mM metal salt.

H1269 . The composition of H1223 or any preceding paragraph dependent thereon, wherein the composition comprises 10-129 mM metal salt.

H1270 . The composition of H1223 or any preceding paragraph dependent thereon, wherein the composition comprises 50-100 mM metal salt.

H1271. The composition of H1223 or any preceding paragraph dependent thereon, wherein the composition comprises 60 mM metal salt.

(continued)

H1272 . The composition of H1223 or any preceding paragraph dependent thereon, wherein the composition comprises 105 mM metal salt.

H1273. The composition of H1223 or any preceding paragraph dependent thereon, wherein the composition comprises 130-199 mM metal salt.

H1274. The composition of H1223 or any preceding paragraph dependent thereon, wherein the composition comprises 170 mM metal salt.

H1275. The composition of H1223 or any preceding paragraph dependent thereon, wherein the composition comprises 5-300 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1276. The composition of H1223 or any preceding paragraph dependent thereon, wherein the composition comprises 10-129 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1277. The composition of H1223 or any preceding paragraph dependent thereon, wherein the composition comprises 50-100 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1278. The composition of H1223 or any preceding paragraph dependent thereon, wherein the composition comprises 60 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1279. The composition of H1223 or any preceding paragraph dependent thereon, wherein the composition comprises 105 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1280. The composition of H1223 or any preceding paragraph dependent thereon, wherein the composition comprises 130-199 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1281. The composition of H1223 or any preceding paragraph dependent thereon, wherein the composition comprises 170 mM metal salt ionic strength provider selected from the group consisting of sodium chloride, potassium chloride, magnesium chloride, calcium chloride, sodium acetate, sodium citrate, monosodium glutamate, sodium phosphate, potassium phosphate, and any combination thereof.

H1282. The composition of H1223 or any preceding paragraph dependent thereon, wherein the composition comprises 5-300 mM sodium chloride.

H1283. The composition of H1223 or any preceding paragraph dependent thereon, wherein the composition comprises 10-129 mM sodium chloride.

H1284. The composition of H1223 or any preceding paragraph dependent thereon, wherein the composition comprises 50-100 mM sodium chloride.

H1285. The composition of H1223 or any preceding paragraph dependent thereon, wherein the composition comprises 60 mM sodium chloride.

H1286. The composition of H1223 or any preceding paragraph dependent thereon, wherein the composition comprises 105 mM sodium chloride.

H1287. The composition of H1223 or any preceding paragraph dependent thereon, wherein the composition comprises 130-199 mM sodium chloride.

H1288. The composition of H1223 or any preceding paragraph dependent thereon, wherein the composition comprises 170 mM sodium chloride.

H1289. The composition of H1-H1222, wherein the composition is characterised by an absence of any (further) ionic strength providers .

H1290. The composition of H1-H1289, wherein the composition is characterised by an absence of metal halides.

H1291. The composition of H1-H1289, wherein the composition is characterised by an absence of sodium chloride.

H1292. The composition of H1-H1222, wherein the composition is characterised by an absence of a (further) ionic strength provider.

(continued)

H1293. The composition of H1-H1289, wherein the composition is characterised by an absence of any, some, or all (further) ionic strength providers defined H1224-H1244 .

H1294. The composition of H1-H1293, wherein the composition comprises a chelator.

H1295. The composition of H1294, wherein the composition comprises (and/or the chelator is) a chelator that datively binds metal ions via three or more dative bonds.

H1296. The composition of H1294 or any preceding paragraphs dependent thereon, wherein the composition comprises (and/or the chelator is) one or more chelators.

H1297. The composition of H1294 or any preceding paragraphs dependent thereon, wherein the composition comprises (and/or the chelator is) a single chelator.

H1298. The composition of H1294 or any preceding paragraphs dependent thereon, wherein the composition comprises (and/or the chelator is) a chelator selected from the group consisting of EDTA (or salts thereof), DTPA (pentetic acid or salts thereof), versetamide (or salts thereof), Calteridol (or salts thereof), and any combination thereof.

H1299. The composition of H1294 or any preceding paragraphs dependent thereon, wherein the composition comprises (and/or the chelator is) a chelator selected from the group consisting of EDTA (or salts thereof), DTPA (pentetic acid or salts thereof), and any combination thereof.

H1300 . The composition of H1294 or any preceding paragraphs dependent thereon, wherein the composition comprises (and/or the chelator is) EDTA (or any salt thereof).

H1301. The composition of H1294 or any preceding paragraphs dependent thereon, wherein the composition comprises (and/or the chelator is) pentetic acid (or any salt thereof).

H1302 . The composition of H1294 or any preceding paragraphs dependent thereon, wherein the composition comprises 0.0001-5 mM chelator.

H1303 . The composition of H1294 or any preceding paragraphs dependent thereon, wherein the composition comprises 0.001-2 mM chelator.

H1304 . The composition of H1294 or any preceding paragraphs dependent thereon, wherein the composition comprises 2 mM chelator.

H1305 . The composition of H1294 or any preceding paragraphs dependent thereon, wherein the composition comprises 0.005-1.5 mM chelator.

H1306 . The composition of H1294 or any preceding paragraphs dependent thereon, wherein the composition comprises 0.01-1.1 mM chelator.

H1307 . The composition of H1294 or any preceding paragraphs dependent thereon, wherein the composition comprises 0.001-0.1 mM chelator.

H1308 . The composition of H1294 or any preceding paragraphs dependent thereon, wherein the composition comprises 0.005-0.05 mM chelator.

H1309 . The composition of H1294 or any preceding paragraphs dependent thereon, wherein the composition comprises 0.001-0.01 mM chelator.

H1310 . The composition of H1294 or any preceding paragraphs dependent thereon, wherein the composition comprises 0.01 mM chelator.

H1311. The composition of H1294 or any preceding paragraphs dependent thereon, wherein the composition comprises 0.01-0.1 mM chelator.

H1312 . The composition of H1294 or any preceding paragraphs dependent thereon, wherein the composition comprises 0.05 mM chelator.

H1313 . The composition of H1294 or any preceding paragraphs dependent thereon, wherein the composition comprises 0.1-1 mM chelator.

H1314 . The composition of H1294 or any preceding paragraphs dependent thereon, wherein the composition comprises 0.3-0.7 mM chelator.

H1315 . The composition of H1294 or any preceding paragraphs dependent thereon, wherein the composition comprises 0.5 mM chelator.

H1316. The composition of H1294 or any preceding paragraphs dependent thereon, wherein the composition comprises 0.0001-5 mM chelator selected from the group consisting of EDTA (or salts thereof), DTPA (pentetic acid or salts thereof), versetamide (or salts thereof), Calteridol (or salts thereof), and any combination thereof.

(continued)

H1317. The composition of H1294 or any preceding paragraphs dependent thereon, wherein the composition comprises 0.005-1.5 mM chelator selected from the group consisting of EDTA (or salts thereof), DTPA (pentetic acid or salts thereof), versetamide (or salts thereof), Calteridol (or salts thereof), and any combination thereof.

H1318. The composition of H1294 or any preceding paragraphs dependent thereon, wherein the composition comprises 0.01 mM chelator selected from the group consisting of EDTA (or salts thereof), DTPA (pentetic acid or salts thereof), versetamide (or salts thereof), Calteridol (or salts thereof), and any combination thereof.

H1319. The composition of H1294 or any preceding paragraphs dependent thereon, wherein the composition comprises 0.05 mM chelator selected from the group consisting of EDTA (or salts thereof), DTPA (pentetic acid or salts thereof), versetamide (or salts thereof), Calteridol (or salts thereof), and any combination thereof.

H1320. The composition of H1294 or any preceding paragraphs dependent thereon, wherein the composition comprises 0.1-1 mM chelator selected from the group consisting of EDTA (or salts thereof), DTPA (pentetic acid or salts thereof), versetamide (or salts thereof), Calteridol (or salts thereof), and any combination thereof.

H1321. The composition of H1294 or any preceding paragraphs dependent thereon, wherein the composition comprises 0.3-0.7 mM chelator selected from the group consisting of EDTA (or salts thereof), DTPA (pentetic acid or salts thereof), versetamide (or salts thereof), Calteridol (or salts thereof), and any combination thereof.

H1322. The composition of H1294 or any preceding paragraphs dependent thereon, wherein the composition comprises 0.5 mM chelator selected from the group consisting of EDTA (or salts thereof), DTPA (pentetic acid or salts thereof), versetamide (or salts thereof), Calteridol (or salts thereof), and any combination thereof.

H1323. The composition of H1294 or any preceding paragraphs dependent thereon, wherein the composition comprises 0.0001-5 mM chelator selected from the group consisting of EDTA (or salts thereof), DTPA (pentetic acid or salts thereof), and any combination thereof.

H1324. The composition of H1294 or any preceding paragraphs dependent thereon, wherein the composition comprises 2 mM chelator selected from the group consisting of EDTA (or salts thereof), DTPA (pentetic acid or salts thereof), and any combination thereof.

H1325. The composition of H1294 or any preceding paragraphs dependent thereon, wherein the composition comprises 0.005-1.5 mM chelator selected from the group consisting of EDTA (or salts thereof), DTPA (pentetic acid or salts thereof), and any combination thereof.

H1326. The composition of H1294 or any preceding paragraphs dependent thereon, wherein the composition comprises 0.01 mM chelator selected from the group consisting of EDTA (or salts thereof), DTPA (pentetic acid or salts thereof), and any combination thereof.

H1327. The composition of H1294 or any preceding paragraphs dependent thereon, wherein the composition comprises 0.05 mM chelator selected from the group consisting of EDTA (or salts thereof), DTPA (pentetic acid or salts thereof), and any combination thereof.

H1328. The composition of H1294 or any preceding paragraphs dependent thereon, wherein the composition comprises 0.1-1 mM chelator selected from the group consisting of EDTA (or salts thereof), DTPA (pentetic acid or salts thereof), and any combination thereof.

H1329. The composition of H1294 or any preceding paragraphs dependent thereon, wherein the composition comprises 0.3-0.7 mM chelator selected from the group consisting of EDTA (or salts thereof), DTPA (pentetic acid or salts thereof), and any combination thereof.

H1330. The composition of H1294 or any preceding paragraphs dependent thereon, wherein the composition comprises 0.5 mM chelator selected from the group consisting of EDTA (or salts thereof), DTPA (pentetic acid or salts thereof), and any combination thereof.

H1331. The composition of H1294 or any preceding paragraphs dependent thereon, wherein the composition comprises 0.0001-5 mM EDTA (or any salt thereof).

H1332. The composition of H1294 or any preceding paragraphs dependent thereon, wherein the composition comprises 2 mM EDTA (or any salt thereof).

H1333. The composition of H1294 or any preceding paragraphs dependent thereon, wherein the composition comprises 0.005-1.5 mM EDTA (or any salt thereof).

(continued)

H1334. The composition of H1294 or any preceding paragraphs dependent thereon, wherein the composition comprises 0.01 mM EDTA (or any salt thereof).

H1335. The composition of H1294 or any preceding paragraphs dependent thereon, wherein the composition comprises 0.05 mM EDTA (or any salt thereof).

H1336. The composition of H1294 or any preceding paragraphs dependent thereon, wherein the composition comprises 0.1-1 mM EDTA (or any salt thereof).

H1337. The composition of H1294 or any preceding paragraphs dependent thereon, wherein the composition comprises 0.3-0.7 mM EDTA (or any salt thereof).

H1338. The composition of H1294 or any preceding paragraphs dependent thereon, wherein the composition comprises 0.5 mM EDTA (or any salt thereof).

H1339. The composition of H1294 or any preceding paragraphs dependent thereon, wherein the composition comprises 0.0001-5 mM pentetic acid (or any salt thereof).

H1340. The composition of H1294 or any preceding paragraphs dependent thereon, wherein the composition comprises 0.005-1.5 mM pentetic acid (or any salt thereof).

H1341. The composition of H1294 or any preceding paragraphs dependent thereon, wherein the composition comprises 0.01 mM pentetic acid (or any salt thereof).

H1342. The composition of H1294 or any preceding paragraphs dependent thereon, wherein the composition comprises 0.05 mM pentetic acid (or any salt thereof).

H1343. The composition of H1294 or any preceding paragraphs dependent thereon, wherein the composition comprises 0.1-1 mM pentetic acid (or any salt thereof).

H1344. The composition of H1294 or any preceding paragraphs dependent thereon, wherein the composition comprises 0.3-0.7 mM pentetic acid (or any salt thereof).

H1345. The composition of H1294 or any preceding paragraphs dependent thereon, wherein the composition comprises 0.5 mM pentetic acid (or any salt thereof).

H1346. The composition of H1-H1345, wherein the composition is characterised by an absence of EDTA.

H1347. The composition of H1-H1293, wherein the composition is characterised by an absence of a chelator.

H1348. The composition of H1-H1345, wherein the composition is characterised by an absence of any, some, or all chelators defined H1295-H1301.

H1349. The composition of H1-H1348, wherein the composition comprises a diluent.

H1350. The composition of H1349, wherein the composition comprises (and/or the diluent is) a diluent that constitutes the balance of ingredients in the composition so that the weight percentages of all ingredients total 100 wt%.

H1351. The composition of H1349 or any preceding paragraph dependent thereon, wherein the composition comprises (and/or the diluent is) a diluent that is present where the composition is said to consist of certain specified ingredients/components.

H1352. The composition of H1349 or any preceding paragraph dependent thereon, wherein the composition comprises (and/or the diluent is) a diluent that dissolves all ingredients/components of the composition.

H1353. The composition of H1349 or any preceding paragraph dependent thereon, wherein the composition comprises (and/or the diluent is) a liquid diluent.

H1354. The composition of H1349 or any preceding paragraph dependent thereon, wherein the composition comprises (and/or the diluent is) an aqueous diluent.

H1355. The composition of H1349 or any preceding paragraph dependent thereon, wherein the composition comprises (and/or the diluent is) water.

H1356. The composition of H1349 or any preceding paragraph dependent thereon, wherein the composition comprises (and/or the diluent is) water for injection (WFI).

H1357. The composition of H1-H1356, wherein the composition is characterised by an osmolality.

H1358. The composition of H1357, wherein the composition has an osmolality that is measurable.

H1359. The composition of H1357 or any preceding paragraph dependent thereon, wherein the composition has an osmolality that is calculable.

H1360 . The composition of H1357 or any preceding paragraph dependent thereon, wherein the composition has an osmolality between 100 and 500 mOsm/kg.

H1361 . The composition of H1357 or any preceding paragraph dependent thereon, wherein the composition has an osmolality between 150 and 450 mOsm/kg.

(continued)

H1362 . The composition of H1357 or any preceding paragraph dependent thereon, wherein the composition has an osmolality between 200 and 500 mOsm/kg.

H1363 . The composition of H1357 or any preceding paragraph dependent thereon, wherein the composition has an osmolality between 200 and 400 mOsm/kg.

H1364 . The composition of H1357 or any preceding paragraph dependent thereon, wherein the composition has an osmolality between 220 and 380 mOsm/kg.

H1365 . The composition of H1357 or any preceding paragraph dependent thereon, wherein the composition has an osmolality between 220 and 280 mOsm/kg.

H1366 . The composition of H1357 or any preceding paragraph dependent thereon, wherein the composition has an osmolality between 240 and 340 mOsm/kg.

H1367 . The composition of H1357 or any preceding paragraph dependent thereon, wherein the composition has an osmolality between 240 and 270 mOsm/kg.

H1368 . The composition of H1357 or any preceding paragraph dependent thereon, wherein the composition has an osmolality between 260 and 320 mOsm/kg.

H1369 . The composition of H1357 or any preceding paragraph dependent thereon, wherein the composition has an osmolality between 270 and 320 mOsm/kg.

H1370 . The composition of H1357 or any preceding paragraph dependent thereon, wherein the composition has an osmolality between 280 and 310 mOsm/kg.

H1371. The composition of H1357 or any preceding paragraph dependent thereon, wherein the composition has an osmolality between 100 and 500 mOsm/kg when the biopharmaceutical active is excluded.

H1372 . The composition of H1357 or any preceding paragraph dependent thereon, wherein the composition has an osmolality between 150 and 450 mOsm/kg when the biopharmaceutical active is excluded.

H1373 . The composition of H1357 or any preceding paragraph dependent thereon, wherein the composition has an osmolality between 200 and 500 mOsm/kg when the biopharmaceutical active is excluded.

H1374 . The composition of H1357 or any preceding paragraph dependent thereon, wherein the composition has an osmolality between 200 and 400 mOsm/kg when the biopharmaceutical active is excluded.

H1375 . The composition of H1357 or any preceding paragraph dependent thereon, wherein the composition has an osmolality between 220 and 380 mOsm/kg when the biopharmaceutical active is excluded.

H1376 . The composition of H1357 or any preceding paragraph dependent thereon, wherein the composition has an osmolality between 220 and 280 mOsm/kg when the biopharmaceutical active is excluded.

H1377 . The composition of H1357 or any preceding paragraph dependent thereon, wherein the composition has an osmolality between 240 and 340 mOsm/kg when the biopharmaceutical active is excluded.

H1378 . The composition of H1357 or any preceding paragraph dependent thereon, wherein the composition has an osmolality between 240 and 270 mOsm/kg when the biopharmaceutical active is excluded.

H1379 . The composition of H1357 or any preceding paragraph dependent thereon, wherein the composition has an osmolality between 260 and 320 mOsm/kg when the biopharmaceutical active is excluded.

H1380 . The composition of H1357 or any preceding paragraph dependent thereon, wherein the composition has an osmolality between 270 and 320 mOsm/kg when the biopharmaceutical active is excluded.

H1381. The composition of H1357 or any preceding paragraph dependent thereon, wherein the composition has an osmolality between 280 and 310 mOsm/kg when the biopharmaceutical active is excluded.

H1382 . The composition of H1357 or any preceding paragraph dependent thereon, wherein the composition has an osmolality that affords isotonicity with bodily fluids (especially with blood, especially human blood plasma).

H1383 . The composition of H1-H1382, wherein the composition is characterised by an ionic strength.

H1384 . The composition of H1383, wherein the composition has an ionic strength that is measurable.

H1385 . The composition of H1383 or any preceding paragraph dependent thereon, wherein the composition has an ionic strength that is calculable.

H1386 . The composition of H1383 or any preceding paragraph dependent thereon, wherein the composition has an ionic strength of 1-400 mM.

H1387 . The composition of H1383 or any preceding paragraph dependent thereon, wherein the composition has an ionic strength of 2-200 mM.

H1388 . The composition of H1383 or any preceding paragraph dependent thereon, wherein the composition has an ionic strength of 5-120 mM.

(continued)

H1389 . The composition of H1383 or any preceding paragraph dependent thereon, wherein the composition has an ionic strength of 20-80 mM.

H1390 . The composition of H1383 or any preceding paragraph dependent thereon, wherein the composition has an ionic strength of 5-20 mM.

H1391. The composition of H1383 or any preceding paragraph dependent thereon, wherein the composition has an ionic strength of 10-60 mM.

H1392 . The composition of H1383 or any preceding paragraph dependent thereon, wherein the composition has an ionic strength of 20-50 mM.

H1393 . The composition of H1383 or any preceding paragraph dependent thereon, wherein the composition has an ionic strength of 30-11 0 mM.

H1394 . The composition of H1383 or any preceding paragraph dependent thereon, wherein the composition has an ionic strength of 40-100 mM.

H1395 . The composition of H1383 or any preceding paragraph dependent thereon, wherein the composition has an ionic strength of 50-90 mM.

H1396 . The composition of H1383 or any preceding paragraph dependent thereon, wherein the composition has an ionic strength of 60-80 mM.

H1397 . The composition of H1383 or any preceding paragraph dependent thereon, wherein the composition has an ionic strength of 70-90 mM.

H1398 . The composition of H1383 or any preceding paragraph dependent thereon, wherein the composition has an ionic strength of 1-400 mM when the biopharmaceutical active is excluded.

H1399 . The composition of H1383 or any preceding paragraph dependent thereon, wherein the composition has an ionic strength of 2-200 mM when the biopharmaceutical active is excluded.

H1400. The composition of H1383 or any preceding paragraph dependent thereon, wherein the composition has an ionic strength of 5-120 mM when the biopharmaceutical active is excluded.

H1401. The composition of H1383 or any preceding paragraph dependent thereon, wherein the composition has an ionic strength of 20-80 mM when the biopharmaceutical active is excluded.

H1402. The composition of H1383 or any preceding paragraph dependent thereon, wherein the composition has an ionic strength of 5-20 mM when the biopharmaceutical active is excluded.

H1403 . The composition of H1383 or any preceding paragraph dependent thereon, wherein the composition has an ionic strength of 10-60 mM when the biopharmaceutical active is excluded.

H1404 . The composition of H1383 or any preceding paragraph dependent thereon, wherein the composition has an ionic strength of 20-50 mM when the biopharmaceutical active is excluded.

H1405. The composition of H1383 or any preceding paragraph dependent thereon, wherein the composition has an ionic strength of 30-11 0 mM when the biopharmaceutical active is excluded.

H1406. The composition of H1383 or any preceding paragraph dependent thereon, wherein the composition has an ionic strength of 40-100 mM when the biopharmaceutical active is excluded.

H1407. The composition of H1383 or any preceding paragraph dependent thereon, wherein the composition has an ionic strength of 50-90 mM when the biopharmaceutical active is excluded.

H1408. The composition of H1383 or any preceding paragraph dependent thereon, wherein the composition has an ionic strength of 60-80 mM when the biopharmaceutical active is excluded.

H1409. The composition of H1383 or any preceding paragraph dependent thereon, wherein the composition has an ionic strength of 70-90 mM when the biopharmaceutical active is excluded.

H1410 . The composition of H1-H1409, wherein the composition is characterised by a viscosity, suitably a dynamic viscosity, suitably at 25°C.

H1411. The composition of H1410, wherein the composition has a dynamic viscosity of 1-30 mPa.s at 25C.

H1412 . The composition of H1410, wherein the composition has a dynamic viscosity of 5-20 mPa.s at 25C.

H1413 . The composition of H1410, wherein the composition has a dynamic viscosity of 7-20 mPa.s at 25C.

H1414 . The composition of H1410, wherein the composition has a dynamic viscosity of 8-17 mPa.s at 25C.

H1415 . The composition of H1410, wherein the composition has a dynamic viscosity of 9-16 mPa.s at 25C.

H1416 . The composition of H1410, wherein the composition has a dynamic viscosity of 8-12 mPa.s at 25C.

H1417 . The composition of H1410, wherein the composition has a dynamic viscosity of 12-20 mPa.s at 25C.

H1418 . The composition of H1-H1417, wherein the composition is a liquid biopharmaceutical composition.

H1419 . The composition of H1-H1417, wherein the composition is an aqueous biopharmaceutical composition.

(continued)

H1420. The composition of H1-H1417, wherein the composition is a lyophilised biopharmaceutical composition, and any concentrations stipulated in relation to ingredients/components become relative molar ratios (noting that wt% concentrations of secukinumab are easily convertible into molar concentrations, as defined above, for instance based on secukinumab having a molecular weight of 148 kDa).

H1421. The composition of H1-H1420, wherein any concentrations stipulated in relation to ingredients/ components are adapted to be expressed as relative molar ratios (noting that wt% concentrations of secukinumab are easily convertible into molar concentrations, as defined above, for instance based on secukinumab having a molecular weight of 148 kDa). For example: the composition may comprise secukinumab, succinate buffer system, single sugar component, and polysorbate 80 in respective molar ratios of 0.135-2.70 : 51-70 : 70-174 : 0.008-1.53 based on H1421 dependency on H883 through to H841 through to H525 through to H151 through to H27.

H1422 . The composition of H1-H1421, wherein the composition consists of (suitably along with a diluent, which is preferably water) the stipulated ingredients/components.

H1423 . The composition of H1-H1422, wherein the features of said composition are applied to any of the embodiments, or combinations thereof (especially as the embodiments of F1-F12 and G1-G959 defining absent components may be applied to any of embodiments A1-A511, B1-B255, and D1-D1152), defined in numbered paragraphs A1-A511, B1-B255, C1-C255, D1-D1152, E1-E1151, F1-F12, and G1-G959.

By way of example, the following specific embodiments (taken from the Examples below) are also disclosed by way of the above mentioned numbered paragraphs, *via* relevant dependencies as explained:

- Formulation F3 - a composition that consists of: 190 mg/mL secukinumab, 55 mM acetate buffer system, pH 4.6, 125 mM sorbitol, 0.02 wt% (i.e. 0.2 mg/mL) polysorbate 80, water for injection, methionine-free - is specifically disclosed by way of the following numbered paragraphs (connected *via* dependencies): H1422 (composition consists of, with diluent), H1356 (diluent = water for injection), H1349 (composition has diluent), H1139 (methionine-free), H1010 (0.2 mg/mL polysorbate 80) or H863 (0.2 mg/mL surfactant) + H841 (surfactant = polysorbate 80), H627 (125 mM sorbitol) or H507 (125 mM sugar component) + H471 (sugar component = sorbitol), H404 (pH 4.6), H162 (55 mM acetate buffer system) or H127 (55 mM buffer system) + H72 (buffer system = acetate buffer system), and H24 (190 mg/mL secukinumab).

- Formulation F9 - a composition that consists of: 190 mg/mL secukinumab, 55 mM succinate buffer system, pH 6.3, 125 mM sorbitol, 0.02 wt% (i.e. 0.2 mg/mL) polysorbate 80, water for injection - is specifically disclosed by: H1422 (consists of, with diluent), H1356 (water for injection), H1349 (diluent), H1010 (0.2 mg/mL polysorbate 80), H627 (125 mM sorbitol), H424 (pH 6.3), H152 (55 mM succinate buffer system), and H24 (190 mg/mL secukinumab).

- Formulation F10 - a composition that consists of: 190 mg/mL secukinumab, 4 mM phosphate-citrate buffer (1:1), pH 6.0, 125 mM sorbitol, 0.05 wt% (i.e. 0.5 mg/mL) polysorbate 80, 60 mM NaCl, water for injection - is specifically disclosed by: H1422 (consists of, with diluent), H1356 (water for injection), H1349 (diluent), H1214 (60 mM sodium chloride), H1013 (0.5 mg/mL polysorbate 80), H627 (125 mM sorbitol), H419 (pH 6.0), H388 (4 mM 1:1 phosphate-citrate buffer system), and H24 (190 mg/mL secukinumab).

- Formulation F11 - a composition that consists of: 190 mg/mL secukinumab, 4 mM phosphate-citrate buffer (1:1), pH 6.3, 230 mM sorbitol, 0.05 wt% (i.e. 0.5 mg/mL) Kolliphor HS-15, water for injection - is specifically disclosed by: H1422 (consists of, with diluent), H1356 (water for injection), H1349 (diluent), H867 (0.5 mg/mL surfactant) H837 (surfactant = Kolliphor HS-15), H635 (230 mM sorbitol), H424 (pH 6.3), H388 (4 mM 1:1 phosphate-citrate buffer system), and H24 (190 mg/mL secukinumab).

- Formulation F12 - a composition that consists of: 190 mg/mL secukinumab, 4 mM phosphate-citrate buffer (1:1), pH 6.0, 230 mM sorbitol, 0.05 wt% (i.e. 0.5 mg/mL) polysorbate 80, water for injection - is specifically disclosed by: H1422 (consists of, with diluent), H1356 (water for injection), H1349 (diluent), H1013 (0.5 mg/mL polysorbate 80), H635 (230 mM sorbitol), H419 (pH 6.0), H388 (4 mM 1:1 phosphate-citrate buffer system), and H24 (190 mg/mL secukinumab).

- Formulation F13 - a composition that consists of: 190 mg/mL secukinumab, 55 mM succinate buffer system, pH 6.0, 125 mM sorbitol, 0.05 wt% (i.e. 0.5 mg/mL) polysorbate 80, 7 mM thiosulfate, water for injection - is specifically disclosed by: H1422 (consists of, with diluent), H1356 (water for injection), H1349 (diluent), H1101 (7 mM thiosulfate), H1013 (0.5 mg/mL polysorbate 80), H627 (125 mM sorbitol), H419 (pH 6.0), H152 (55 mM succinate buffer system), and H24 (190 mg/mL secukinumab).

- Formulation F14 - a composition that consists of: 190 mg/mL secukinumab, 55 mM succinate buffer system, pH 6.3, 125 mM sorbitol, 0.1 wt% (i.e. 1 mg/mL) polysorbate 80, water for injection - is specifically disclosed by: H1422 (consists of, with diluent), H1356 (water for injection), H1349 (diluent), H1015 (1 mg/mL polysorbate 80), H627 (125 mM sorbitol), H424 (pH 6.3), H152 (55 mM succinate buffer system), and H24 (190 mg/mL secukinumab).

- Formulation F15 - a composition that consists of: 180 mg/mL secukinumab, 4 mM phosphate-citrate buffer (1:1), pH 5.8, 125 mM sorbitol, 0.05 wt% (i.e. 0.5 mg/mL) polysorbate 80, 60 mM NaCl, water for injection - is specifically disclosed by: H1422 (consists of, with diluent), H1356 (water for injection), H1349 (diluent), H1214 (60 mM sodium chloride), H1013 (0.5 mg/mL polysorbate 80), H627 (125 mM sorbitol), H415 (pH 5.8), H388 (4 mM 1:1 phosphate-citrate buffer system), and H17 (180 mg/mL secukinumab).

- Formulation F16 - a composition that consists of: 180 mg/mL secukinumab, 55 mM succinate buffer system, pH 5.8, 125 mM sorbitol, 0.05 wt% (i.e. 0.5 mg/mL) polysorbate 80, water for injection - is specifically disclosed by: H1422 (consists of, with diluent), H1356 (water for injection), H1349 (diluent), H1013 (0.5 mg/mL polysorbate 80), H627 (125 mM sorbitol), H415 (pH 5.8), H152 (55 mM succinate buffer system), and H17 (180 mg/mL secukinumab).

- Formulation F17 - a composition that consists of: 165 mg/mL secukinumab, 4 mM phosphate-citrate buffer (1:1), pH 6.0, 125 mM sorbitol, 0.05 wt% (i.e. 0.5 mg/mL) polysorbate 80, 60 mM NaCl, water for injection - is specifically disclosed by: H1422 (consists of, with diluent), H1356 (water for injection), H1349 (diluent), H1214 (60 mM sodium chloride), H1013 (0.5 mg/mL polysorbate 80), H627 (125 mM sorbitol), H419 (pH 6.0), H388 (4 mM 1:1 phosphate-citrate buffer system), and H14 (165 mg/mL secukinumab).

The features of the compositions of H1-H1422 may be applied to any of the embodiments, or combinations thereof (especially as the embodiments of F1-F12 and G1-G959 defining absent components may be applied to any of embodiments A1-A511, B1-B255, C1-C255, D1-D1152, and E1-E1151), defined in numbered paragraphs A1-A511, B1-B255, C1-C255, D1-D1152, E1-E1151, F1-F12, and G1-G959, I1-I2400, and J1-J1158.

Compositions of the invention may be defined as per anywhere herein, including in respect of numbered paragraphs A1-A511, B1-B255, C1-C255, D1-D1152, E1-E1151, F1-F12, G1-G959, H1-H1422, I1-I2400, J1-J1158, and combinations thereof, and optionally further characterised by a disclaimer to more specifically-defined compositions of any of numbered paragraphs A1-A511, B1-B255, C1-C255, D1-D1152, E1-E1151, F1-F12, G1-G959, H1-H1422, I1-I2400, J1-J1158, and combinations thereof (e.g. on the proviso that the composition excludes more-specifically defined compositions of numbered paragraphs A1-A511, B1-B255, C1-C255, D1-D1152, E1-E1151, F1-F12, G1-G959, H1-H1422, I1-I2400, J1-J1158, and combinations thereof).

*Certain Specific Embodiments*

The biopharmaceutical composition suitably comprises, or consists of (suitably along with a diluent, which is preferably water), and/or is otherwise characterised by: 180-220 mg/mL secukinumab and four or more of any of: either 51-60 mM succinate buffer system or 0.5-4.5 mM phosphate-citrate buffer system consisting of a phosphate buffer system and a citrate buffer system in a respective molar ratio of between 3:1 and 1:3; either 50-350 mannitol or sorbitol or 50-129 mM sorbitol or 200-250 mM sorbitol; either 100-149 mM arginine or 100-149 mM lysine; 0.1-1.1 mg/mL polysorbate 80; 1-10 mM thiosulfate; 10-129 mM sodium chloride; and either pH 4.5-6.5 or pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-129 mM sorbitol; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; and 0.1-1.1 mg/mL polysorbate 80.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; and 0.1-1.1 mg/mL polysorbate 80.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; and 1-10 mM thiosulfate (especially sodium thiosulfate).

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 1-10 mM thiosulfate (especially sodium thiosulfate); and 10-129 mM sodium chloride.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-350 mM mannitol or sorbitol; 100-149 mM lysine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-350 mM mannitol or sorbitol; 100-149 mM lysine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 51-60 mM succinate buffer system; 50-129 mM sorbitol; 100-149 mM lysine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 180-220 mg/mL secukinumab; 0.5-4.5 mM phosphate-citrate buffer system consisting of a phosphate buffer system and a citrate buffer system in a respective molar ratio of between 3:1 and 1:3; 50-129 mM sorbitol; 100-149 mM lysine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 1-10 mM thiosulfate (especially sodium thiosulfate); and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 1-10 mM thiosulfate (especially sodium thiosulfate); and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 1-10 mM thiosulfate (especially sodium thiosulfate); 10-129 mM sodium chloride; and pH 4.5-6.5.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM arginine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 51-60 mM succinate buffer system; 200-250 mM sugar component; 100-149 mM lysine; 0.1-1.1 mg/mL polysorbate 80; 1-10 mM thiosulfate (especially sodium thiosulfate); 10-129 mM sodium chloride; and pH 5.6-6.4.

In a particular embodiment, the biopharmaceutical composition comprises, or consists of (including water), and/or is characterised by 0.81-1.49 mM secukinumab; 0.5-4.5 mM phosphate-citrate buffer system consisting of a phosphate buffer system and a citrate buffer system in a respective molar ratio of between 3:1 and 1:3; 200-250 mM sugar component; 100-149 mM lysine; 0.1-1.1 mg/mL polysorbate 80; 1-10 mM thiosulfate (especially sodium thiosulfate); 10-129 mM sodium chloride; and pH 5.6-6.4.

The aforementioned embodiments may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

*Certain Further Specific Embodiments*

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.6-6.2.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 165 mg/mLsecukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 165 mg/mLsecukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 5.8.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 20-120 mM tonicifier; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 165 mg/mLsecukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.1-1.1 mg/mL surfactant; 60 mM sodium chloride; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 100-150 mM sugar alcohol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 145-190 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 165 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 180 mg/mL secukinumab; 1-9 mM phosphate-citrate buffer system (with a phosphate and citrate buffer system in molar ratio between 3:1 to 1:3); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 145-190 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 165 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 180 mg/mL secukinumab; 4 mM phosphate-citrate buffer system (with a phosphate and citrate buffer system in molar ratio of 1:1); 125 mM sorbitol; 0.5 mg/mL polysorbate 80; 60 mM sodium chloride; water (e.g. WFI); and pH 6.0.

In a particular embodiment, the biopharmaceutical composition comprises, or consists of (including water), and/or is characterised by 145-190 mg/mL secukinumab; 51-70 mM buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-60 mM succinate buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 100-150 mM sugar component; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-60 mM succinate buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 125 mM sorbitol; 0.1-1.1 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-60 mM succinate buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 100-150 mM sugar component; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 51-60 mM succinate buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 125 mM sorbitol; 0.25-0.75 mg/mL surfactant; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 180 mg/mLsecukinumab; 51-60 mM succinate buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 100-150 mM sugar component; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 180 mg/mL secukinumab; 51-70 mM buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 51-60 mM succinate buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 180 mg/mL secukinumab; 51-60 mM succinate buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 55 mM succinate buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 180 mg/mL secukinumab; 55 mM succinate buffer system; 125 mM sorbitol; 0.5 mg/mL polysorbate 80; water (e.g. WFI); and pH 5.6-6.2.

In a particular embodiment, the biopharmaceutical composition comprises, or consists of (including water), and/or is characterised by 145-190 mg/mL secukinumab; 51-70 mM buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 51-70 mM buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 55 mM succinate buffer system; 100-150 mM sugar component; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 51-70 mM buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 51-70 mM buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 180 mg/mL secukinumab; 55 mM succinate buffer system; 125 mM sorbitol; 0.1-1.1 mg/mL surfactant; water (e.g. WFI); and pH 5.8.

In a particular embodiment, the biopharmaceutical composition comprises, or consists of (including water), and/or is characterised by 145-190 mg/mL secukinumab; 51-70 mM buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 51-70 mM buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 55 mM succinate buffer system; 100-150 mM sugar component; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 51-70 mM buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 51-70 mM buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 180 mg/mL secukinumab; 55 mM succinate buffer system; 125 mM sorbitol; 0.25-0.75 mg/mL surfactant; water (e.g. WFI); and pH 5.8.

In a particular embodiment, the biopharmaceutical composition comprises, or consists of (including water), and/or is characterised by 145-190 mg/mL secukinumab; 51-70 mM buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 51-70 mM buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 55 mM succinate buffer system; 100-150 mM sugar component; 0.5 mg/mL polysorbate 80; water (e.g. WFI); and pH 5.8.

In a particular embodiment, the biopharmaceutical composition comprises, or consists of (including water), and/or is characterised by 145-190 mg/mL secukinumab; 51-70 mM buffer system; 125 mM sorbitol; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 51-70 mM buffer system; 125 mM sorbitol; 0.5 mg/mL polysorbate 80; water (e.g. WFI); and pH 5.8.

In a particular embodiment, the biopharmaceutical composition comprises, or consists of (including water), and/or is characterised by 145-190 mg/mL secukinumab; 51-60 mM succinate buffer system; 125 mM sorbitol; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 51-60 mM succinate buffer system; 125 mM sorbitol; 0.5 mg/mL polysorbate 80; water (e.g. WFI); and pH 5.8.

In a particular embodiment, the biopharmaceutical composition comprises, or consists of (including water), and/or is characterised by 145-190 mg/mL secukinumab; 55 mM succinate buffer system; 125 mM sorbitol; 0.5 mg/mL polysorbate 80; water (e.g. WFI); and pH 5.8.

In a particular embodiment, the biopharmaceutical composition comprises, or consists of (including water), and/or is characterised by 180 mg/mL secukinumab; 55 mM succinate buffer system; 125 mM sorbitol; 0.5 mg/mL polysorbate 80; water (e.g. WFI); and pH 5.8.

The aforementioned embodiments may be applied to any of the embodiments defined in numbered paragraphs A1-A511, B1-B255, C1-C255, D1-D1152, E1-E1151, F1-F12, G1-G959, H1-H1423, I1-I2400, and J1-J1158.

*Further Specific Embodiments*

I) Further Specific Embodiments

As the table below illustrates, further specific embodiments may be provided by combining one option (from the "OPTIONS" column) from each of the elements in the "FEATURE / INGREDIENT" column:

| **FEATURE / INGREDIENT** | **OPTIONS** |
|---|---|
| **Secukinumab / concentrations** | 150 mg/mL secukinumab (secu)<br>190 mg/mL secukinumab |
| **Buffer(s) / concentrations** | 0 mM buffer<br>50 mM Histidine buffer (10 mM His)<br>10 mM Succinate buffer (10 mM Succ)<br>20 mM Phosphate buffer (10 mM Phos)<br>15 mM Citrate buffer (10 mM Cit)<br>25 mM Lactate buffer (10 mM Lact)<br>30 mM Phosphate-citrate (1:1 molar ratio) buffer (10 mM Phos-Cit)<br>10 mM Phosphate-lactate (1:1 molar ratio) buffer (10 mM Phos-Lact buffer)<br>15 mM Gluconate buffer (10 mM Gluc) |
| **Sugar/Polyol / concentrations** | 0 mM sugar/polyol<br>150 mM sorbitol (150 mM sorb)<br>75 mM mannitol (75 mM mann)<br>100 mM sucrose (100 mM sucr)<br>200 mM trehalose (200 mM treh) |
| **Amino acid / concentrations** | 50 mM arginine (50 mM arg)<br>100 mM glycine (100 mM gly) |
| **Surfactant / concentrations** | 0.5 mg/mL polysorbate 80 (0.5 mg/mL PS80)<br>0.2 mg/mL polysorbate 20 (0.2 mg/mL PS20) |
| Antioxidant / concentrations | 0 mM antioxidant (or 0 mM methionine)<br>1 mM methionine (1 mM met) |
| Tonicifier (additional) / concentrations | 0 mM (additional) tonicifier<br>50 mM NaCl |

(continued)

| FEATURE / INGREDIENT | OPTIONS |
|---|---|
| Chelator / concentrations | 0 mM chelator (or 0 mM EDTA)<br>0.1 mM EDTA (including salts thereof) |
| Diluent | Water (for injection) |
| pH | pH 5.8 (+/- 0.3) |
| Osmolality | (preferably) 200-400 mOsm/kg |
| Dynamic viscosity | (preferably) 5-20 mPa.s at 25C |

The following numbered paragraphs I1-I2400 are illustrative of such embodiments drawn from the above table, and define further embodiments of a biopharmaceutical composition comprising, or consisting of, and/or otherwise characterised by:

I1. 150 mg/mL secu; 0 mM buffer; 0.5 mg/mL PS80; water; and pH 5.8.
I2. 190 mg/mL secu; 0 mM buffer; 0.5 mg/mL PS80; water; and pH 5.8.
I3. 150 mg/mL secu; 50 mM His; 0.5 mg/mL PS80; water; and pH 5.8.
I4. 190 mg/mL secu; 50 mM His; 0.5 mg/mL PS80; water; and pH 5.8.
I5. 150 mg/mL secu; 25 mM Lact; 0.5 mg/mL PS80; water; and pH 5.8.
I6. 190 mg/mL secu; 25 mM Lact; 0.5 mg/mL PS80; water; and pH 5.8.
I7. 150 mg/mL secu; 10 mM Phos-Lact; 0.5 mg/mL PS80; water; and pH 5.8.
I8. 190 mg/mL secu; 10 mM Phos-Lact; 0.5 mg/mL PS80; water; and pH 5.8.
I9. 150 mg/mL secu; 15 mM Gluc; 0.5 mg/mL PS80; water; and pH 5.8.
I10. 190 mg/mL secu; 15 mM Gluc; 0.5 mg/mL PS80; water; and pH 5.8.
I11. 150 mg/mL secu; 0 mM buffer; 0.2 mg/mL PS20; water; and pH 5.8.
I12. 190 mg/mL secu; 0 mM buffer; 0.2 mg/mL PS20; water; and pH 5.8.
I13. 150 mg/mL secu; 50 mM His; 0.2 mg/mL PS20; water; and pH 5.8.
I14. 190 mg/mL secu; 50 mM His; 0.2 mg/mL PS20; water; and pH 5.8.
I15. 150 mg/mL secu; 25 mM Lact; 0.2 mg/mL PS20; water; and pH 5.8.
I16. 190 mg/mL secu; 25 mM Lact; 0.2 mg/mL PS20; water; and pH 5.8.
I17. 150 mg/mL secu; 10 mM Phos-Lact; 0.2 mg/mL PS20; water; and pH 5.8.
I18. 190 mg/mL secu; 10 mM Phos-Lact; 0.2 mg/mL PS20; water; and pH 5.8.
I19. 150 mg/mL secu; 15 mM Gluc; 0.2 mg/mL PS20; water; and pH 5.8.
I20. 190 mg/mL secu; 15 mM Gluc; 0.2 mg/mL PS20; water; and pH 5.8.
I21. 150 mg/mL secu; 0 mM buffer; 150 mM sorb; 0.5 mg/mL PS80; water; and pH 5.8.
I22. 190 mg/mL secu; 0 mM buffer; 150 mM sorb; 0.5 mg/mL PS80; water; and pH 5.8.
I23. 150 mg/mL secu; 50 mM His; 150 mM sorb; 0.5 mg/mL PS80; water; and pH 5.8.
I24. 190 mg/mL secu; 50 mM His; 150 mM sorb; 0.5 mg/mL PS80; water; and pH 5.8.
I25. 150 mg/mL secu; 25 mM Lact; 150 mM sorb; 0.5 mg/mL PS80; water; and pH 5.8.
I26. 190 mg/mL secu; 25 mM Lact; 150 mM sorb; 0.5 mg/mL PS80; water; and pH 5.8.
I27. 150 mg/mL secu; 10 mM Phos-Lact; 150 mM sorb; 0.5 mg/mL PS80; water; and pH 5.8.
I28. 190 mg/mL secu; 10 mM Phos-Lact; 150 mM sorb; 0.5 mg/mL PS80; water; and pH 5.8.
I29. 150 mg/mL secu; 15 mM Gluc; 150 mM sorb; 0.5 mg/mL PS80; water; and pH 5.8.
I30. 190 mg/mL secu; 15 mM Gluc; 150 mM sorb; 0.5 mg/mL PS80; water; and pH 5.8.
I31. 150 mg/mL secu; 0 mM buffer; 75 mM mann; 0.5 mg/mL PS80; water; and pH 5.8.
I32. 190 mg/mL secu; 0 mM buffer; 75 mM mann; 0.5 mg/mL PS80; water; and pH 5.8.
I33. 150 mg/mL secu; 50 mM His; 75 mM mann; 0.5 mg/mL PS80; water; and pH 5.8.
I34. 190 mg/mL secu; 50 mM His; 75 mM mann; 0.5 mg/mL PS80; water; and pH 5.8.
I35. 150 mg/mL secu; 25 mM Lact; 75 mM mann; 0.5 mg/mL PS80; water; and pH 5.8.
I36. 190 mg/mL secu; 25 mM Lact; 75 mM mann; 0.5 mg/mL PS80; water; and pH 5.8.
I37. 150 mg/mL secu; 10 mM Phos-Lact; 75 mM mann; 0.5 mg/mL PS80; water; and pH 5.8.

(continued)

I38. 190 mg/mL secu; 10 mM Phos-Lact; 75 mM mann; 0.5 mg/mL PS80; water; and pH 5.8.
I39. 150 mg/mL secu; 15 mM Gluc; 75 mM mann; 0.5 mg/mL PS80; water; and pH 5.8.
I40. 190 mg/mL secu; 15 mM Gluc; 75 mM mann; 0.5 mg/mL PS80; water; and pH 5.8.
I41. 150 mg/mL secu; 0 mM buffer; 100 mM sucr; 0.5 mg/mL PS80; water; and pH 5.8.
I42. 190 mg/mL secu; 0 mM buffer; 100 mM sucr; 0.5 mg/mL PS80; water; and pH 5.8.
I43. 150 mg/mL secu; 50 mM His; 100 mM sucr; 0.5 mg/mL PS80; water; and pH 5.8.
I44. 190 mg/mL secu; 50 mM His; 100 mM sucr; 0.5 mg/mL PS80; water; and pH 5.8.
I45. 150 mg/mL secu; 25 mM Lact; 100 mM sucr; 0.5 mg/mL PS80; water; and pH 5.8.
I46. 190 mg/mL secu; 25 mM Lact; 100 mM sucr; 0.5 mg/mL PS80; water; and pH 5.8.
I47. 150 mg/mL secu; 10 mM Phos-Lact; 100 mM sucr; 0.5 mg/mL PS80; water; and pH 5.8.
I48. 190 mg/mL secu; 10 mM Phos-Lact; 100 mM sucr; 0.5 mg/mL PS80; water; and pH 5.8.
I49. 150 mg/mL secu; 15 mM Gluc; 100 mM sucr; 0.5 mg/mL PS80; water; and pH 5.8.
I50. 190 mg/mL secu; 15 mM Gluc; 100 mM sucr; 0.5 mg/mL PS80; water; and pH 5.8.
I51. 150 mg/mL secu; 0 mM buffer; 200 mM treh; 0.5 mg/mL PS80; water; and pH 5.8.
I52. 190 mg/mL secu; 0 mM buffer; 200 mM treh; 0.5 mg/mL PS80; water; and pH 5.8.
I53. 150 mg/mL secu; 50 mM His; 200 mM treh; 0.5 mg/mL PS80; water; and pH 5.8.
I54. 190 mg/mL secu; 50 mM His; 200 mM treh; 0.5 mg/mL PS80; water; and pH 5.8.
I55. 150 mg/mL secu; 25 mM Lact; 200 mM treh; 0.5 mg/mL PS80; water; and pH 5.8.
I56. 190 mg/mL secu; 25 mM Lact; 200 mM treh; 0.5 mg/mL PS80; water; and pH 5.8.
I57. 150 mg/mL secu; 10 mM Phos-Lact; 200 mM treh; 0.5 mg/mL PS80; water; and pH 5.8.
I58. 190 mg/mL secu; 10 mM Phos-Lact; 200 mM treh; 0.5 mg/mL PS80; water; and pH 5.8.
I59. 150 mg/mL secu; 15 mM Gluc; 200 mM treh; 0.5 mg/mL PS80; water; and pH 5.8.
I60. 190 mg/mL secu; 15 mM Gluc; 200 mM treh; 0.5 mg/mL PS80; water; and pH 5.8.
I61. 150 mg/mL secu; 0 mM buffer; 150 mM sorb; 0.2 mg/mL PS20; water; and pH 5.8.
I62. 190 mg/mL secu; 0 mM buffer; 150 mM sorb; 0.2 mg/mL PS20; water; and pH 5.8.
I63. 150 mg/mL secu; 50 mM His; 150 mM sorb; 0.2 mg/mL PS20; water; and pH 5.8.
I64. 190 mg/mL secu; 50 mM His; 150 mM sorb; 0.2 mg/mL PS20; water; and pH 5.8.
I65. 150 mg/mL secu; 25 mM Lact; 150 mM sorb; 0.2 mg/mL PS20; water; and pH 5.8.
I66. 190 mg/mL secu; 25 mM Lact; 150 mM sorb; 0.2 mg/mL PS20; water; and pH 5.8.
I67. 150 mg/mL secu; 10 mM Phos-Lact; 150 mM sorb; 0.2 mg/mL PS20; water; and pH 5.8.
I68. 190 mg/mL secu; 10 mM Phos-Lact; 150 mM sorb; 0.2 mg/mL PS20; water; and pH 5.8.
I69. 150 mg/mL secu; 15 mM Gluc; 150 mM sorb; 0.2 mg/mL PS20; water; and pH 5.8.
I70. 190 mg/mL secu; 15 mM Gluc; 150 mM sorb; 0.2 mg/mL PS20; water; and pH 5.8.
I71. 150 mg/mL secu; 0 mM buffer; 75 mM mann; 0.2 mg/mL PS20; water; and pH 5.8.
I72. 190 mg/mL secu; 0 mM buffer; 75 mM mann; 0.2 mg/mL PS20; water; and pH 5.8.
I73. 150 mg/mL secu; 50 mM His; 75 mM mann; 0.2 mg/mL PS20; water; and pH 5.8.
I74. 190 mg/mL secu; 50 mM His; 75 mM mann; 0.2 mg/mL PS20; water; and pH 5.8.
I75. 150 mg/mL secu; 25 mM Lact; 75 mM mann; 0.2 mg/mL PS20; water; and pH 5.8.
I76. 190 mg/mL secu; 25 mM Lact; 75 mM mann; 0.2 mg/mL PS20; water; and pH 5.8.
I77. 150 mg/mL secu; 10 mM Phos-Lact; 75 mM mann; 0.2 mg/mL PS20; water; and pH 5.8.
I78. 190 mg/mL secu; 10 mM Phos-Lact; 75 mM mann; 0.2 mg/mL PS20; water; and pH 5.8.
I79. 150 mg/mL secu; 15 mM Gluc; 75 mM mann; 0.2 mg/mL PS20; water; and pH 5.8.
I80. 190 mg/mL secu; 15 mM Gluc; 75 mM mann; 0.2 mg/mL PS20; water; and pH 5.8.
I81. 150 mg/mL secu; 0 mM buffer; 100 mM sucr; 0.2 mg/mL PS20; water; and pH 5.8.
I82. 190 mg/mL secu; 0 mM buffer; 100 mM sucr; 0.2 mg/mL PS20; water; and pH 5.8.
I83. 150 mg/mL secu; 50 mM His; 100 mM sucr; 0.2 mg/mL PS20; water; and pH 5.8.
I84. 190 mg/mL secu; 50 mM His; 100 mM sucr; 0.2 mg/mL PS20; water; and pH 5.8.
I85. 150 mg/mL secu; 25 mM Lact; 100 mM sucr; 0.2 mg/mL PS20; water; and pH 5.8.
I86. 190 mg/mL secu; 25 mM Lact; 100 mM sucr; 0.2 mg/mL PS20; water; and pH 5.8.
I87. 150 mg/mL secu; 10 mM Phos-Lact; 100 mM sucr; 0.2 mg/mL PS20; water; and pH 5.8.
I88. 190 mg/mL secu; 10 mM Phos-Lact; 100 mM sucr; 0.2 mg/mL PS20; water; and pH 5.8.

(continued)

I89. 150 mg/mL secu; 15 mM Gluc; 100 mM sucr; 0.2 mg/mL PS20; water; and pH 5.8.
I90. 190 mg/mL secu; 15 mM Gluc; 100 mM sucr; 0.2 mg/mL PS20; water; and pH 5.8.
I91. 150 mg/mL secu; 0 mM buffer; 200 mM treh; 0.2 mg/mL PS20; water; and pH 5.8.
I92. 190 mg/mL secu; 0 mM buffer; 200 mM treh; 0.2 mg/mL PS20; water; and pH 5.8.
I93. 150 mg/mL secu; 50 mM His; 200 mM treh; 0.2 mg/mL PS20; water; and pH 5.8.
I94. 190 mg/mL secu; 50 mM His; 200 mM treh; 0.2 mg/mL PS20; water; and pH 5.8.
I95. 150 mg/mL secu; 25 mM Lact; 200 mM treh; 0.2 mg/mL PS20; water; and pH 5.8.
I96. 190 mg/mL secu; 25 mM Lact; 200 mM treh; 0.2 mg/mL PS20; water; and pH 5.8.
I97. 150 mg/mL secu; 10 mM Phos-Lact; 200 mM treh; 0.2 mg/mL PS20; water; and pH 5.8.
I98. 190 mg/mL secu; 10 mM Phos-Lact; 200 mM treh; 0.2 mg/mL PS20; water; and pH 5.8.
I99. 150 mg/mL secu; 15 mM Gluc; 200 mM treh; 0.2 mg/mL PS20; water; and pH 5.8.
I100. 190 mg/mL secu; 15 mM Gluc; 200 mM treh; 0.2 mg/mL PS20; water; and pH 5.8.
I101. 150 mg/mL secu; 0 mM buffer; 50 mM arg; 0.5 mg/mL PS80; water; and pH 5.8.
I102. 190 mg/mL secu; 0 mM buffer; 50 mM arg; 0.5 mg/mL PS80; water; and pH 5.8.
I103. 150 mg/mL secu; 50 mM His; 50 mM arg; 0.5 mg/mL PS80; water; and pH 5.8.
I104. 190 mg/mL secu; 50 mM His; 50 mM arg; 0.5 mg/mL PS80; water; and pH 5.8.
I105. 150 mg/mL secu; 25 mM Lact; 50 mM arg; 0.5 mg/mL PS80; water; and pH 5.8.
I106. 190 mg/mL secu; 25 mM Lact; 50 mM arg; 0.5 mg/mL PS80; water; and pH 5.8.
I107. 150 mg/mL secu; 10 mM Phos-Lact; 50 mM arg; 0.5 mg/mL PS80; water; and pH 5.8.
I108. 190 mg/mL secu; 10 mM Phos-Lact; 50 mM arg; 0.5 mg/mL PS80; water; and pH 5.8.
I109. 150 mg/mL secu; 15 mM Gluc; 50 mM arg; 0.5 mg/mL PS80; water; and pH 5.8.
I110. 190 mg/mL secu; 15 mM Gluc; 50 mM arg; 0.5 mg/mL PS80; water; and pH 5.8.
I111. 150 mg/mL secu; 0 mM buffer; 100 mM gly; 0.5 mg/mL PS80; water; and pH 5.8.
I112. 190 mg/mL secu; 0 mM buffer; 100 mM gly; 0.5 mg/mL PS80; water; and pH 5.8.
I113. 150 mg/mL secu; 50 mM His; 100 mM gly; 0.5 mg/mL PS80; water; and pH 5.8.
I114. 190 mg/mL secu; 50 mM His; 100 mM gly; 0.5 mg/mL PS80; water; and pH 5.8.
I115. 150 mg/mL secu; 25 mM Lact; 100 mM gly; 0.5 mg/mL PS80; water; and pH 5.8.
I116. 190 mg/mL secu; 25 mM Lact; 100 mM gly; 0.5 mg/mL PS80; water; and pH 5.8.
I117. 150 mg/mL secu; 10 mM Phos-Lact; 100 mM gly; 0.5 mg/mL PS80; water; and pH 5.8.
I118. 190 mg/mL secu; 10 mM Phos-Lact; 100 mM gly; 0.5 mg/mL PS80; water; and pH 5.8.
I119. 150 mg/mL secu; 15 mM Gluc; 100 mM gly; 0.5 mg/mL PS80; water; and pH 5.8.
I120. 190 mg/mL secu; 15 mM Gluc; 100 mM gly; 0.5 mg/mL PS80; water; and pH 5.8.
I121. 150 mg/mL secu; 0 mM buffer; 50 mM arg; 0.2 mg/mL PS20; water; and pH 5.8.
I122. 190 mg/mL secu; 0 mM buffer; 50 mM arg; 0.2 mg/mL PS20; water; and pH 5.8.
I123. 150 mg/mL secu; 50 mM His; 50 mM arg; 0.2 mg/mL PS20; water; and pH 5.8.
I124. 190 mg/mL secu; 50 mM His; 50 mM arg; 0.2 mg/mL PS20; water; and pH 5.8.
I125. 150 mg/mL secu; 25 mM Lact; 50 mM arg; 0.2 mg/mL PS20; water; and pH 5.8.
I126. 190 mg/mL secu; 25 mM Lact; 50 mM arg; 0.2 mg/mL PS20; water; and pH 5.8.
I127. 150 mg/mL secu; 10 mM Phos-Lact; 50 mM arg; 0.2 mg/mL PS20; water; and pH 5.8.
I128. 190 mg/mL secu; 10 mM Phos-Lact; 50 mM arg; 0.2 mg/mL PS20; water; and pH 5.8.
I129. 150 mg/mL secu; 15 mM Gluc; 50 mM arg; 0.2 mg/mL PS20; water; and pH 5.8.
I130. 190 mg/mL secu; 15 mM Gluc; 50 mM arg; 0.2 mg/mL PS20; water; and pH 5.8.
I131. 150 mg/mL secu; 0 mM buffer; 100 mM gly; 0.2 mg/mL PS20; water; and pH 5.8.
I132. 190 mg/mL secu; 0 mM buffer; 100 mM gly; 0.2 mg/mL PS20; water; and pH 5.8.
I133. 150 mg/mL secu; 50 mM His; 100 mM gly; 0.2 mg/mL PS20; water; and pH 5.8.
I134. 190 mg/mL secu; 50 mM His; 100 mM gly; 0.2 mg/mL PS20; water; and pH 5.8.
I135. 150 mg/mL secu; 25 mM Lact; 100 mM gly; 0.2 mg/mL PS20; water; and pH 5.8.
I136. 190 mg/mL secu; 25 mM Lact; 100 mM gly; 0.2 mg/mL PS20; water; and pH 5.8.
I137. 150 mg/mL secu; 10 mM Phos-Lact; 100 mM gly; 0.2 mg/mL PS20; water; and pH 5.8.
I138. 190 mg/mL secu; 10 mM Phos-Lact; 100 mM gly; 0.2 mg/mL PS20; water; and pH 5.8.
I139. 150 mg/mL secu; 15 mM Gluc; 100 mM gly; 0.2 mg/mL PS20; water; and pH 5.8.

(continued)

I140. 190 mg/mL secu; 15 mM Gluc; 100 mM gly; 0.2 mg/mL PS20; water; and pH 5.8.
I141. 150 mg/mL secu; 0 mM buffer; 0.5 mg/mL PS80; 1 mM met; water; and pH 5.8.
I142. 190 mg/mL secu; 0 mM buffer; 0.5 mg/mL PS80; 1 mM met; water; and pH 5.8.
I143. 150 mg/mL secu; 50 mM His; 0.5 mg/mL PS80; 1 mM met; water; and pH 5.8.
I144. 190 mg/mL secu; 50 mM His; 0.5 mg/mL PS80; 1 mM met; water; and pH 5.8.
I145. 150 mg/mL secu; 25 mM Lact; 0.5 mg/mL PS80; 1 mM met; water; and pH 5.8.
I146. 190 mg/mL secu; 25 mM Lact; 0.5 mg/mL PS80; 1 mM met; water; and pH 5.8.
I147. 150 mg/mL secu; 10 mM Phos-Lact; 0.5 mg/mL PS80; 1 mM met; water; and pH 5.8.
I148. 190 mg/mL secu; 10 mM Phos-Lact; 0.5 mg/mL PS80; 1 mM met; water; and pH 5.8.
I149. 150 mg/mL secu; 15 mM Gluc; 0.5 mg/mL PS80; 1 mM met; water; and pH 5.8.
I150. 190 mg/mL secu; 15 mM Gluc; 0.5 mg/mL PS80; 1 mM met; water; and pH 5.8.
I151. 150 mg/mL secu; 0 mM buffer; 0.2 mg/mL PS20; 1 mM met; water; and pH 5.8.
I152. 190 mg/mL secu; 0 mM buffer; 0.2 mg/mL PS20; 1 mM met; water; and pH 5.8.
I153. 150 mg/mL secu; 50 mM His; 0.2 mg/mL PS20; 1 mM met; water; and pH 5.8.
I154. 190 mg/mL secu; 50 mM His; 0.2 mg/mL PS20; 1 mM met; water; and pH 5.8.
I155. 150 mg/mL secu; 25 mM Lact; 0.2 mg/mL PS20; 1 mM met; water; and pH 5.8.
I156. 190 mg/mL secu; 25 mM Lact; 0.2 mg/mL PS20; 1 mM met; water; and pH 5.8.
I157. 150 mg/mL secu; 10 mM Phos-Lact; 0.2 mg/mL PS20; 1 mM met; water; and pH 5.8.
I158. 190 mg/mL secu; 10 mM Phos-Lact; 0.2 mg/mL PS20; 1 mM met; water; and pH 5.8.
I159. 150 mg/mL secu; 15 mM Gluc; 0.2 mg/mL PS20; 1 mM met; water; and pH 5.8.
I160. 190 mg/mL secu; 15 mM Gluc; 0.2 mg/mL PS20; 1 mM met; water; and pH 5.8.
I161. 150 mg/mL secu; 0 mM buffer; 0.5 mg/mL PS80; 50 mM NaCl; water; and pH 5.8.
I162. 190 mg/mL secu; 0 mM buffer; 0.5 mg/mL PS80; 50 mM NaCl; water; and pH 5.8.
I163. 150 mg/mL secu; 50 mM His; 0.5 mg/mL PS80; 50 mM NaCl; water; and pH 5.8.
I164. 190 mg/mL secu; 50 mM His; 0.5 mg/mL PS80; 50 mM NaCl; water; and pH 5.8.
I165. 150 mg/mL secu; 25 mM Lact; 0.5 mg/mL PS80; 50 mM NaCl; water; and pH 5.8.
I166. 190 mg/mL secu; 25 mM Lact; 0.5 mg/mL PS80; 50 mM NaCl; water; and pH 5.8.
I167. 150 mg/mL secu; 10 mM Phos-Lact; 0.5 mg/mL PS80; 50 mM NaCl; water; and pH 5.8.
I168. 190 mg/mL secu; 10 mM Phos-Lact; 0.5 mg/mL PS80; 50 mM NaCl; water; and pH 5.8.
I169. 150 mg/mL secu; 15 mM Gluc; 0.5 mg/mL PS80; 50 mM NaCl; water; and pH 5.8.
I170. 190 mg/mL secu; 15 mM Gluc; 0.5 mg/mL PS80; 50 mM NaCl; water; and pH 5.8.
I171. 150 mg/mL secu; 0 mM buffer; 0.2 mg/mL PS20; 50 mM NaCl; water; and pH 5.8.
I172. 190 mg/mL secu; 0 mM buffer; 0.2 mg/mL PS20; 50 mM NaCl; water; and pH 5.8.
I173. 150 mg/mL secu; 50 mM His; 0.2 mg/mL PS20; 50 mM NaCl; water; and pH 5.8.
I174. 190 mg/mL secu; 50 mM His; 0.2 mg/mL PS20; 50 mM NaCl; water; and pH 5.8.
I175. 150 mg/mL secu; 25 mM Lact; 0.2 mg/mL PS20; 50 mM NaCl; water; and pH 5.8.
I176. 190 mg/mL secu; 25 mM Lact; 0.2 mg/mL PS20; 50 mM NaCl; water; and pH 5.8.
I177. 150 mg/mL secu; 10 mM Phos-Lact; 0.2 mg/mL PS20; 50 mM NaCl; water; and pH 5.8.
I178. 190 mg/mL secu; 10 mM Phos-Lact; 0.2 mg/mL PS20; 50 mM NaCl; water; and pH 5.8.
I179. 150 mg/mL secu; 15 mM Gluc; 0.2 mg/mL PS20; 50 mM NaCl; water; and pH 5.8.
I180. 190 mg/mL secu; 15 mM Gluc; 0.2 mg/mL PS20; 50 mM NaCl; water; and pH 5.8.
I181. 150 mg/mL secu; 0 mM buffer; 0.5 mg/mL PS80; 0.1 mM EDTA; water; and pH 5.8.
I182. 190 mg/mL secu; 0 mM buffer; 0.5 mg/mL PS80; 0.1 mM EDTA; water; and pH 5.8.
I183. 150 mg/mL secu; 50 mM His; 0.5 mg/mL PS80; 0.1 mM EDTA; water; and pH 5.8.
I184. 190 mg/mL secu; 50 mM His; 0.5 mg/mL PS80; 0.1 mM EDTA; water; and pH 5.8.
I185. 150 mg/mL secu; 25 mM Lact; 0.5 mg/mL PS80; 0.1 mM EDTA; water; and pH 5.8.
I186. 190 mg/mL secu; 25 mM Lact; 0.5 mg/mL PS80; 0.1 mM EDTA; water; and pH 5.8.
I187. 150 mg/mL secu; 10 mM Phos-Lact; 0.5 mg/mL PS80; 0.1 mM EDTA; water; and pH 5.8.
I188. 190 mg/mL secu; 10 mM Phos-Lact; 0.5 mg/mL PS80; 0.1 mM EDTA; water; and pH 5.8.
I189. 150 mg/mL secu; 15 mM Gluc; 0.5 mg/mL PS80; 0.1 mM EDTA; water; and pH 5.8.
I190. 190 mg/mL secu; 15 mM Gluc; 0.5 mg/mL PS80; 0.1 mM EDTA; water; and pH 5.8.

(continued)

I191. 150 mg/mL secu; 0 mM buffer; 0.2 mg/mL PS20; 0.1 mM EDTA; water; and pH 5.8.
I192. 190 mg/mL secu; 0 mM buffer; 0.2 mg/mL PS20; 0.1 mM EDTA; water; and pH 5.8.
I193. 150 mg/mL secu; 50 mM His; 0.2 mg/mL PS20; 0.1 mM EDTA; water; and pH 5.8.
I194. 190 mg/mL secu; 50 mM His; 0.2 mg/mL PS20; 0.1 mM EDTA; water; and pH 5.8.
I195. 150 mg/mL secu; 25 mM Lact; 0.2 mg/mL PS20; 0.1 mM EDTA; water; and pH 5.8.
I196. 190 mg/mL secu; 25 mM Lact; 0.2 mg/mL PS20; 0.1 mM EDTA; water; and pH 5.8.
I197. 150 mg/mL secu; 10 mM Phos-Lact; 0.2 mg/mL PS20; 0.1 mM EDTA; water; and pH 5.8.
I198. 190 mg/mL secu; 10 mM Phos-Lact; 0.2 mg/mL PS20; 0.1 mM EDTA; water; and pH 5.8.
I199. 150 mg/mL secu; 15 mM Gluc; 0.2 mg/mL PS20; 0.1 mM EDTA; water; and pH 5.8.
I200. 190 mg/mL secu; 15 mM Gluc; 0.2 mg/mL PS20; 0.1 mM EDTA; water; and pH 5.8.
I201. 150 mg/mL secu; 0 mM buffer; 150 mM sorb; 50 mM arg; 0.5 mg/mL PS80; water; and pH 5.8.
I202. 190 mg/mL secu; 0 mM buffer; 150 mM sorb; 50 mM arg; 0.5 mg/mL PS80; water; and pH 5.8.
I203. 150 mg/mL secu; 50 mM His; 150 mM sorb; 50 mM arg; 0.5 mg/mL PS80; water; and pH 5.8.
I204. 190 mg/mL secu; 50 mM His; 150 mM sorb; 50 mM arg; 0.5 mg/mL PS80; water; and pH 5.8.
I205. 150 mg/mL secu; 25 mM Lact; 150 mM sorb; 50 mM arg; 0.5 mg/mL PS80; water; and pH 5.8.
I206. 190 mg/mL secu; 25 mM Lact; 150 mM sorb; 50 mM arg; 0.5 mg/mL PS80; water; and pH 5.8.
I207. 150 mg/mL secu; 10 mM Phos-Lact; 150 mM sorb; 50 mM arg; 0.5 mg/mL PS80; water; and pH 5.8.
I208. 190 mg/mL secu; 10 mM Phos-Lact; 150 mM sorb; 50 mM arg; 0.5 mg/mL PS80; water; and pH 5.8.
I209. 150 mg/mL secu; 15 mM Gluc; 150 mM sorb; 50 mM arg; 0.5 mg/mL PS80; water; and pH 5.8.
I210. 190 mg/mL secu; 15 mM Gluc; 150 mM sorb; 50 mM arg; 0.5 mg/mL PS80; water; and pH 5.8.
I211. 150 mg/mL secu; 0 mM buffer; 75 mM mann; 50 mM arg; 0.5 mg/mL PS80; water; and pH 5.8.
I212. 190 mg/mL secu; 0 mM buffer; 75 mM mann; 50 mM arg; 0.5 mg/mL PS80; water; and pH 5.8.
I213. 150 mg/mL secu; 50 mM His; 75 mM mann; 50 mM arg; 0.5 mg/mL PS80; water; and pH 5.8.
I214. 190 mg/mL secu; 50 mM His; 75 mM mann; 50 mM arg; 0.5 mg/mL PS80; water; and pH 5.8.
I215. 150 mg/mL secu; 25 mM Lact; 75 mM mann; 50 mM arg; 0.5 mg/mL PS80; water; and pH 5.8.
I216. 190 mg/mL secu; 25 mM Lact; 75 mM mann; 50 mM arg; 0.5 mg/mL PS80; water; and pH 5.8.
I217. 150 mg/mL secu; 10 mM Phos-Lact; 75 mM mann; 50 mM arg; 0.5 mg/mL PS80; water; and pH 5.8.
I218. 190 mg/mL secu; 10 mM Phos-Lact; 75 mM mann; 50 mM arg; 0.5 mg/mL PS80; water; and pH 5.8.
I219. 150 mg/mL secu; 15 mM Gluc; 75 mM mann; 50 mM arg; 0.5 mg/mL PS80; water; and pH 5.8.
I220. 190 mg/mL secu; 15 mM Gluc; 75 mM mann; 50 mM arg; 0.5 mg/mL PS80; water; and pH 5.8.
I221. 150 mg/mL secu; 0 mM buffer; 100 mM sucr; 50 mM arg; 0.5 mg/mL PS80; water; and pH 5.8.
I222. 190 mg/mL secu; 0 mM buffer; 100 mM sucr; 50 mM arg; 0.5 mg/mL PS80; water; and pH 5.8.
I223. 150 mg/mL secu; 50 mM His; 100 mM sucr; 50 mM arg; 0.5 mg/mL PS80; water; and pH 5.8.
I224. 190 mg/mL secu; 50 mM His; 100 mM sucr; 50 mM arg; 0.5 mg/mL PS80; water; and pH 5.8.
I225. 150 mg/mL secu; 25 mM Lact; 100 mM sucr; 50 mM arg; 0.5 mg/mL PS80; water; and pH 5.8.
I226. 190 mg/mL secu; 25 mM Lact; 100 mM sucr; 50 mM arg; 0.5 mg/mL PS80; water; and pH 5.8.
I227. 150 mg/mL secu; 10 mM Phos-Lact; 100 mM sucr; 50 mM arg; 0.5 mg/mL PS80; water; and pH 5.8.
I228. 190 mg/mL secu; 10 mM Phos-Lact; 100 mM sucr; 50 mM arg; 0.5 mg/mL PS80; water; and pH 5.8.
I229. 150 mg/mL secu; 15 mM Gluc; 100 mM sucr; 50 mM arg; 0.5 mg/mL PS80; water; and pH 5.8.
I230. 190 mg/mL secu; 15 mM Gluc; 100 mM sucr; 50 mM arg; 0.5 mg/mL PS80; water; and pH 5.8.
I231. 150 mg/mL secu; 0 mM buffer; 200 mM treh; 50 mM arg; 0.5 mg/mL PS80; water; and pH 5.8.
I232. 190 mg/mL secu; 0 mM buffer; 200 mM treh; 50 mM arg; 0.5 mg/mL PS80; water; and pH 5.8.
I233. 150 mg/mL secu; 50 mM His; 200 mM treh; 50 mM arg; 0.5 mg/mL PS80; water; and pH 5.8.
I234. 190 mg/mL secu; 50 mM His; 200 mM treh; 50 mM arg; 0.5 mg/mL PS80; water; and pH 5.8.
I235. 150 mg/mL secu; 25 mM Lact; 200 mM treh; 50 mM arg; 0.5 mg/mL PS80; water; and pH 5.8.
I236. 190 mg/mL secu; 25 mM Lact; 200 mM treh; 50 mM arg; 0.5 mg/mL PS80; water; and pH 5.8.
I237. 150 mg/mL secu; 10 mM Phos-Lact; 200 mM treh; 50 mM arg; 0.5 mg/mL PS80; water; and pH 5.8.
I238. 190 mg/mL secu; 10 mM Phos-Lact; 200 mM treh; 50 mM arg; 0.5 mg/mL PS80; water; and pH 5.8.
I239. 150 mg/mL secu; 15 mM Gluc; 200 mM treh; 50 mM arg; 0.5 mg/mL PS80; water; and pH 5.8.
I240. 190 mg/mL secu; 15 mM Gluc; 200 mM treh; 50 mM arg; 0.5 mg/mL PS80; water; and pH 5.8.
I241. 150 mg/mL secu; 0 mM buffer; 150 mM sorb; 100 mM gly; 0.5 mg/mL PS80; water; and pH 5.8.

(continued)

I242. 190 mg/mL secu; 0 mM buffer; 150 mM sorb; 100 mM gly; 0.5 mg/mL PS80; water; and pH 5.8.
I243. 150 mg/mL secu; 50 mM His; 150 mM sorb; 100 mM gly; 0.5 mg/mL PS80; water; and pH 5.8.
I244. 190 mg/mL secu; 50 mM His; 150 mM sorb; 100 mM gly; 0.5 mg/mL PS80; water; and pH 5.8.
I245. 150 mg/mL secu; 25 mM Lact; 150 mM sorb; 100 mM gly; 0.5 mg/mL PS80; water; and pH 5.8.
I246. 190 mg/mL secu; 25 mM Lact; 150 mM sorb; 100 mM gly; 0.5 mg/mL PS80; water; and pH 5.8.
I247. 150 mg/mL secu; 10 mM Phos-Lact; 150 mM sorb; 100 mM gly; 0.5 mg/mL PS80; water; and pH 5.8.
I248. 190 mg/mL secu; 10 mM Phos-Lact; 150 mM sorb; 100 mM gly; 0.5 mg/mL PS80; water; and pH 5.8.
I249. 150 mg/mL secu; 15 mM Gluc; 150 mM sorb; 100 mM gly; 0.5 mg/mL PS80; water; and pH 5.8.
I250. 190 mg/mL secu; 15 mM Gluc; 150 mM sorb; 100 mM gly; 0.5 mg/mL PS80; water; and pH 5.8.
I251. 150 mg/mL secu; 0 mM buffer; 75 mM mann; 100 mM gly; 0.5 mg/mL PS80; water; and pH 5.8.
I252. 190 mg/mL secu; 0 mM buffer; 75 mM mann; 100 mM gly; 0.5 mg/mL PS80; water; and pH 5.8.
I253. 150 mg/mL secu; 50 mM His; 75 mM mann; 100 mM gly; 0.5 mg/mL PS80; water; and pH 5.8.
I254. 190 mg/mL secu; 50 mM His; 75 mM mann; 100 mM gly; 0.5 mg/mL PS80; water; and pH 5.8.
I255. 150 mg/mL secu; 25 mM Lact; 75 mM mann; 100 mM gly; 0.5 mg/mL PS80; water; and pH 5.8.
I256. 190 mg/mL secu; 25 mM Lact; 75 mM mann; 100 mM gly; 0.5 mg/mL PS80; water; and pH 5.8.
I257. 150 mg/mL secu; 10 mM Phos-Lact; 75 mM mann; 100 mM gly; 0.5 mg/mL PS80; water; and pH 5.8.
I258. 190 mg/mL secu; 10 mM Phos-Lact; 75 mM mann; 100 mM gly; 0.5 mg/mL PS80; water; and pH 5.8.
I259. 150 mg/mL secu; 15 mM Gluc; 75 mM mann; 100 mM gly; 0.5 mg/mL PS80; water; and pH 5.8.
I260. 190 mg/mL secu; 15 mM Gluc; 75 mM mann; 100 mM gly; 0.5 mg/mL PS80; water; and pH 5.8.
I261. 150 mg/mL secu; 0 mM buffer; 100 mM sucr; 100 mM gly; 0.5 mg/mL PS80; water; and pH 5.8.
I262. 190 mg/mL secu; 0 mM buffer; 100 mM sucr; 100 mM gly; 0.5 mg/mL PS80; water; and pH 5.8.
I263. 150 mg/mL secu; 50 mM His; 100 mM sucr; 100 mM gly; 0.5 mg/mL PS80; water; and pH 5.8.
I264. 190 mg/mL secu; 50 mM His; 100 mM sucr; 100 mM gly; 0.5 mg/mL PS80; water; and pH 5.8.
I265. 150 mg/mL secu; 25 mM Lact; 100 mM sucr; 100 mM gly; 0.5 mg/mL PS80; water; and pH 5.8.
I266. 190 mg/mL secu; 25 mM Lact; 100 mM sucr; 100 mM gly; 0.5 mg/mL PS80; water; and pH 5.8.
I267. 150 mg/mL secu; 10 mM Phos-Lact; 100 mM sucr; 100 mM gly; 0.5 mg/mL PS80; water; and pH 5.8.
I268. 190 mg/mL secu; 10 mM Phos-Lact; 100 mM sucr; 100 mM gly; 0.5 mg/mL PS80; water; and pH 5.8.
I269. 150 mg/mL secu; 15 mM Gluc; 100 mM sucr; 100 mM gly; 0.5 mg/mL PS80; water; and pH 5.8.
I270. 190 mg/mL secu; 15 mM Gluc; 100 mM sucr; 100 mM gly; 0.5 mg/mL PS80; water; and pH 5.8.
I271. 150 mg/mL secu; 0 mM buffer; 200 mM treh; 100 mM gly; 0.5 mg/mL PS80; water; and pH 5.8.
I272. 190 mg/mL secu; 0 mM buffer; 200 mM treh; 100 mM gly; 0.5 mg/mL PS80; water; and pH 5.8.
I273. 150 mg/mL secu; 50 mM His; 200 mM treh; 100 mM gly; 0.5 mg/mL PS80; water; and pH 5.8.
I274. 190 mg/mL secu; 50 mM His; 200 mM treh; 100 mM gly; 0.5 mg/mL PS80; water; and pH 5.8.
I275. 150 mg/mL secu; 25 mM Lact; 200 mM treh; 100 mM gly; 0.5 mg/mL PS80; water; and pH 5.8.
I276. 190 mg/mL secu; 25 mM Lact; 200 mM treh; 100 mM gly; 0.5 mg/mL PS80; water; and pH 5.8.
I277. 150 mg/mL secu; 10 mM Phos-Lact; 200 mM treh; 100 mM gly; 0.5 mg/mL PS80; water; and pH 5.8.
I278. 190 mg/mL secu; 10 mM Phos-Lact; 200 mM treh; 100 mM gly; 0.5 mg/mL PS80; water; and pH 5.8.
I279. 150 mg/mL secu; 15 mM Gluc; 200 mM treh; 100 mM gly; 0.5 mg/mL PS80; water; and pH 5.8.
I280. 190 mg/mL secu; 15 mM Gluc; 200 mM treh; 100 mM gly; 0.5 mg/mL PS80; water; and pH 5.8.
I281. 150 mg/mL secu; 0 mM buffer; 150 mM sorb; 50 mM arg; 0.2 mg/mL PS20; water; and pH 5.8.
I282. 190 mg/mL secu; 0 mM buffer; 150 mM sorb; 50 mM arg; 0.2 mg/mL PS20; water; and pH 5.8.
I283. 150 mg/mL secu; 50 mM His; 150 mM sorb; 50 mM arg; 0.2 mg/mL PS20; water; and pH 5.8.
I284. 190 mg/mL secu; 50 mM His; 150 mM sorb; 50 mM arg; 0.2 mg/mL PS20; water; and pH 5.8.
I285. 150 mg/mL secu; 25 mM Lact; 150 mM sorb; 50 mM arg; 0.2 mg/mL PS20; water; and pH 5.8.
I286. 190 mg/mL secu; 25 mM Lact; 150 mM sorb; 50 mM arg; 0.2 mg/mL PS20; water; and pH 5.8.
I287. 150 mg/mL secu; 10 mM Phos-Lact; 150 mM sorb; 50 mM arg; 0.2 mg/mL PS20; water; and pH 5.8.
I288. 190 mg/mL secu; 10 mM Phos-Lact; 150 mM sorb; 50 mM arg; 0.2 mg/mL PS20; water; and pH 5.8.
I289. 150 mg/mL secu; 15 mM Gluc; 150 mM sorb; 50 mM arg; 0.2 mg/mL PS20; water; and pH 5.8.
I290. 190 mg/mL secu; 15 mM Gluc; 150 mM sorb; 50 mM arg; 0.2 mg/mL PS20; water; and pH 5.8.
I291. 150 mg/mL secu; 0 mM buffer; 75 mM mann; 50 mM arg; 0.2 mg/mL PS20; water; and pH 5.8.
I292. 190 mg/mL secu; 0 mM buffer; 75 mM mann; 50 mM arg; 0.2 mg/mL PS20; water; and pH 5.8.

(continued)

I293. 150 mg/mL secu; 50 mM His; 75 mM mann; 50 mM arg; 0.2 mg/mL PS20; water; and pH 5.8.
I294. 190 mg/mL secu; 50 mM His; 75 mM mann; 50 mM arg; 0.2 mg/mL PS20; water; and pH 5.8.
I295. 150 mg/mL secu; 25 mM Lact; 75 mM mann; 50 mM arg; 0.2 mg/mL PS20; water; and pH 5.8.
I296. 190 mg/mL secu; 25 mM Lact; 75 mM mann; 50 mM arg; 0.2 mg/mL PS20; water; and pH 5.8.
I297. 150 mg/mL secu; 10 mM Phos-Lact; 75 mM mann; 50 mM arg; 0.2 mg/mL PS20; water; and pH 5.8.
I298. 190 mg/mL secu; 10 mM Phos-Lact; 75 mM mann; 50 mM arg; 0.2 mg/mL PS20; water; and pH 5.8.
I299. 150 mg/mL secu; 15 mM Gluc; 75 mM mann; 50 mM arg; 0.2 mg/mL PS20; water; and pH 5.8.
I300. 190 mg/mL secu; 15 mM Gluc; 75 mM mann; 50 mM arg; 0.2 mg/mL PS20; water; and pH 5.8.
I301. 150 mg/mL secu; 0 mM buffer; 100 mM sucr; 50 mM arg; 0.2 mg/mL PS20; water; and pH 5.8.
I302. 190 mg/mL secu; 0 mM buffer; 100 mM sucr; 50 mM arg; 0.2 mg/mL PS20; water; and pH 5.8.
I303. 150 mg/mL secu; 50 mM His; 100 mM sucr; 50 mM arg; 0.2 mg/mL PS20; water; and pH 5.8.
I304. 190 mg/mL secu; 50 mM His; 100 mM sucr; 50 mM arg; 0.2 mg/mL PS20; water; and pH 5.8.
I305. 150 mg/mL secu; 25 mM Lact; 100 mM sucr; 50 mM arg; 0.2 mg/mL PS20; water; and pH 5.8.
I306. 190 mg/mL secu; 25 mM Lact; 100 mM sucr; 50 mM arg; 0.2 mg/mL PS20; water; and pH 5.8.
I307. 150 mg/mL secu; 10 mM Phos-Lact; 100 mM sucr; 50 mM arg; 0.2 mg/mL PS20; water; and pH 5.8.
I308. 190 mg/mL secu; 10 mM Phos-Lact; 100 mM sucr; 50 mM arg; 0.2 mg/mL PS20; water; and pH 5.8.
I309. 150 mg/mL secu; 15 mM Gluc; 100 mM sucr; 50 mM arg; 0.2 mg/mL PS20; water; and pH 5.8.
I310. 190 mg/mL secu; 15 mM Gluc; 100 mM sucr; 50 mM arg; 0.2 mg/mL PS20; water; and pH 5.8.
I311. 150 mg/mL secu; 0 mM buffer; 200 mM treh; 50 mM arg; 0.2 mg/mL PS20; water; and pH 5.8.
I312. 190 mg/mL secu; 0 mM buffer; 200 mM treh; 50 mM arg; 0.2 mg/mL PS20; water; and pH 5.8.
I313. 150 mg/mL secu; 50 mM His; 200 mM treh; 50 mM arg; 0.2 mg/mL PS20; water; and pH 5.8.
I314. 190 mg/mL secu; 50 mM His; 200 mM treh; 50 mM arg; 0.2 mg/mL PS20; water; and pH 5.8.
I315. 150 mg/mL secu; 25 mM Lact; 200 mM treh; 50 mM arg; 0.2 mg/mL PS20; water; and pH 5.8.
I316. 190 mg/mL secu; 25 mM Lact; 200 mM treh; 50 mM arg; 0.2 mg/mL PS20; water; and pH 5.8.
I317. 150 mg/mL secu; 10 mM Phos-Lact; 200 mM treh; 50 mM arg; 0.2 mg/mL PS20; water; and pH 5.8.
I318. 190 mg/mL secu; 10 mM Phos-Lact; 200 mM treh; 50 mM arg; 0.2 mg/mL PS20; water; and pH 5.8.
I319. 150 mg/mL secu; 15 mM Gluc; 200 mM treh; 50 mM arg; 0.2 mg/mL PS20; water; and pH 5.8.
I320. 190 mg/mL secu; 15 mM Gluc; 200 mM treh; 50 mM arg; 0.2 mg/mL PS20; water; and pH 5.8.
I321. 150 mg/mL secu; 0 mM buffer; 150 mM sorb; 100 mM gly; 0.2 mg/mL PS20; water; and pH 5.8.
I322. 190 mg/mL secu; 0 mM buffer; 150 mM sorb; 100 mM gly; 0.2 mg/mL PS20; water; and pH 5.8.
I323. 150 mg/mL secu; 50 mM His; 150 mM sorb; 100 mM gly; 0.2 mg/mL PS20; water; and pH 5.8.
I324. 190 mg/mL secu; 50 mM His; 150 mM sorb; 100 mM gly; 0.2 mg/mL PS20; water; and pH 5.8.
I325. 150 mg/mL secu; 25 mM Lact; 150 mM sorb; 100 mM gly; 0.2 mg/mL PS20; water; and pH 5.8.
I326. 190 mg/mL secu; 25 mM Lact; 150 mM sorb; 100 mM gly; 0.2 mg/mL PS20; water; and pH 5.8.
I327. 150 mg/mL secu; 10 mM Phos-Lact; 150 mM sorb; 100 mM gly; 0.2 mg/mL PS20; water; and pH 5.8.
I328. 190 mg/mL secu; 10 mM Phos-Lact; 150 mM sorb; 100 mM gly; 0.2 mg/mL PS20; water; and pH 5.8.
I329. 150 mg/mL secu; 15 mM Gluc; 150 mM sorb; 100 mM gly; 0.2 mg/mL PS20; water; and pH 5.8.
I330. 190 mg/mL secu; 15 mM Gluc; 150 mM sorb; 100 mM gly; 0.2 mg/mL PS20; water; and pH 5.8.
I331. 150 mg/mL secu; 0 mM buffer; 75 mM mann; 100 mM gly; 0.2 mg/mL PS20; water; and pH 5.8.
I332. 190 mg/mL secu; 0 mM buffer; 75 mM mann; 100 mM gly; 0.2 mg/mL PS20; water; and pH 5.8.
I333. 150 mg/mL secu; 50 mM His; 75 mM mann; 100 mM gly; 0.2 mg/mL PS20; water; and pH 5.8.
I334. 190 mg/mL secu; 50 mM His; 75 mM mann; 100 mM gly; 0.2 mg/mL PS20; water; and pH 5.8.
I335. 150 mg/mL secu; 25 mM Lact; 75 mM mann; 100 mM gly; 0.2 mg/mL PS20; water; and pH 5.8.
I336. 190 mg/mL secu; 25 mM Lact; 75 mM mann; 100 mM gly; 0.2 mg/mL PS20; water; and pH 5.8.
I337. 150 mg/mL secu; 10 mM Phos-Lact; 75 mM mann; 100 mM gly; 0.2 mg/mL PS20; water; and pH 5.8.
I338. 190 mg/mL secu; 10 mM Phos-Lact; 75 mM mann; 100 mM gly; 0.2 mg/mL PS20; water; and pH 5.8.
I339. 150 mg/mL secu; 15 mM Gluc; 75 mM mann; 100 mM gly; 0.2 mg/mL PS20; water; and pH 5.8.
I340. 190 mg/mL secu; 15 mM Gluc; 75 mM mann; 100 mM gly; 0.2 mg/mL PS20; water; and pH 5.8.
I341. 150 mg/mL secu; 0 mM buffer; 100 mM sucr; 100 mM gly; 0.2 mg/mL PS20; water; and pH 5.8.
I342. 190 mg/mL secu; 0 mM buffer; 100 mM sucr; 100 mM gly; 0.2 mg/mL PS20; water; and pH 5.8.
I343. 150 mg/mL secu; 50 mM His; 100 mM sucr; 100 mM gly; 0.2 mg/mL PS20; water; and pH 5.8.

(continued)

I344. 190 mg/mL secu; 50 mM His; 100 mM sucr; 100 mM gly; 0.2 mg/mL PS20; water; and pH 5.8.
I345. 150 mg/mL secu; 25 mM Lact; 100 mM sucr; 100 mM gly; 0.2 mg/mL PS20; water; and pH 5.8.
I346. 190 mg/mL secu; 25 mM Lact; 100 mM sucr; 100 mM gly; 0.2 mg/mL PS20; water; and pH 5.8.
I347. 150 mg/mL secu; 10 mM Phos-Lact; 100 mM sucr; 100 mM gly; 0.2 mg/mL PS20; water; and pH 5.8.
I348. 190 mg/mL secu; 10 mM Phos-Lact; 100 mM sucr; 100 mM gly; 0.2 mg/mL PS20; water; and pH 5.8.
I349. 150 mg/mL secu; 15 mM Gluc; 100 mM sucr; 100 mM gly; 0.2 mg/mL PS20; water; and pH 5.8.
I350. 190 mg/mL secu; 15 mM Gluc; 100 mM sucr; 100 mM gly; 0.2 mg/mL PS20; water; and pH 5.8.
I351. 150 mg/mL secu; 0 mM buffer; 200 mM treh; 100 mM gly; 0.2 mg/mL PS20; water; and pH 5.8.
I352. 190 mg/mL secu; 0 mM buffer; 200 mM treh; 100 mM gly; 0.2 mg/mL PS20; water; and pH 5.8.
I353. 150 mg/mL secu; 50 mM His; 200 mM treh; 100 mM gly; 0.2 mg/mL PS20; water; and pH 5.8.
I354. 190 mg/mL secu; 50 mM His; 200 mM treh; 100 mM gly; 0.2 mg/mL PS20; water; and pH 5.8.
I355. 150 mg/mL secu; 25 mM Lact; 200 mM treh; 100 mM gly; 0.2 mg/mL PS20; water; and pH 5.8.
I356. 190 mg/mL secu; 25 mM Lact; 200 mM treh; 100 mM gly; 0.2 mg/mL PS20; water; and pH 5.8.
I357. 150 mg/mL secu; 10 mM Phos-Lact; 200 mM treh; 100 mM gly; 0.2 mg/mL PS20; water; and pH 5.8.
I358. 190 mg/mL secu; 10 mM Phos-Lact; 200 mM treh; 100 mM gly; 0.2 mg/mL PS20; water; and pH 5.8.
I359. 150 mg/mL secu; 15 mM Gluc; 200 mM treh; 100 mM gly; 0.2 mg/mL PS20; water; and pH 5.8.
I360. 190 mg/mL secu; 15 mM Gluc; 200 mM treh; 100 mM gly; 0.2 mg/mL PS20; water; and pH 5.8.
I361. 150 mg/mL secu; 0 mM buffer; 150 mM sorb; 0.5 mg/mL PS80; 1 mM met; water; and pH 5.8.
I362. 190 mg/mL secu; 0 mM buffer; 150 mM sorb; 0.5 mg/mL PS80; 1 mM met; water; and pH 5.8.
I363. 150 mg/mL secu; 50 mM His; 150 mM sorb; 0.5 mg/mL PS80; 1 mM met; water; and pH 5.8.
I364. 190 mg/mL secu; 50 mM His; 150 mM sorb; 0.5 mg/mL PS80; 1 mM met; water; and pH 5.8.
I365. 150 mg/mL secu; 25 mM Lact; 150 mM sorb; 0.5 mg/mL PS80; 1 mM met; water; and pH 5.8.
I366. 190 mg/mL secu; 25 mM Lact; 150 mM sorb; 0.5 mg/mL PS80; 1 mM met; water; and pH 5.8.
I367. 150 mg/mL secu; 10 mM Phos-Lact; 150 mM sorb; 0.5 mg/mL PS80; 1 mM met; water; and pH 5.8.
I368. 190 mg/mL secu; 10 mM Phos-Lact; 150 mM sorb; 0.5 mg/mL PS80; 1 mM met; water; and pH 5.8.
I369. 150 mg/mL secu; 15 mM Gluc; 150 mM sorb; 0.5 mg/mL PS80; 1 mM met; water; and pH 5.8.
I370. 190 mg/mL secu; 15 mM Gluc; 150 mM sorb; 0.5 mg/mL PS80; 1 mM met; water; and pH 5.8.
I371. 150 mg/mL secu; 0 mM buffer; 75 mM mann; 0.5 mg/mL PS80; 1 mM met; water; and pH 5.8.
I372. 190 mg/mL secu; 0 mM buffer; 75 mM mann; 0.5 mg/mL PS80; 1 mM met; water; and pH 5.8.
I373. 150 mg/mL secu; 50 mM His; 75 mM mann; 0.5 mg/mL PS80; 1 mM met; water; and pH 5.8.
I374. 190 mg/mL secu; 50 mM His; 75 mM mann; 0.5 mg/mL PS80; 1 mM met; water; and pH 5.8.
I375. 150 mg/mL secu; 25 mM Lact; 75 mM mann; 0.5 mg/mL PS80; 1 mM met; water; and pH 5.8.
I376. 190 mg/mL secu; 25 mM Lact; 75 mM mann; 0.5 mg/mL PS80; 1 mM met; water; and pH 5.8.
I377. 150 mg/mL secu; 10 mM Phos-Lact; 75 mM mann; 0.5 mg/mL PS80; 1 mM met; water; and pH 5.8.
I378. 190 mg/mL secu; 10 mM Phos-Lact; 75 mM mann; 0.5 mg/mL PS80; 1 mM met; water; and pH 5.8.
I379. 150 mg/mL secu; 15 mM Gluc; 75 mM mann; 0.5 mg/mL PS80; 1 mM met; water; and pH 5.8.
I380. 190 mg/mL secu; 15 mM Gluc; 75 mM mann; 0.5 mg/mL PS80; 1 mM met; water; and pH 5.8.
I381. 150 mg/mL secu; 0 mM buffer; 100 mM sucr; 0.5 mg/mL PS80; 1 mM met; water; and pH 5.8.
I382. 190 mg/mL secu; 0 mM buffer; 100 mM sucr; 0.5 mg/mL PS80; 1 mM met; water; and pH 5.8.
I383. 150 mg/mL secu; 50 mM His; 100 mM sucr; 0.5 mg/mL PS80; 1 mM met; water; and pH 5.8.
I384. 190 mg/mL secu; 50 mM His; 100 mM sucr; 0.5 mg/mL PS80; 1 mM met; water; and pH 5.8.
I385. 150 mg/mL secu; 25 mM Lact; 100 mM sucr; 0.5 mg/mL PS80; 1 mM met; water; and pH 5.8.
I386. 190 mg/mL secu; 25 mM Lact; 100 mM sucr; 0.5 mg/mL PS80; 1 mM met; water; and pH 5.8.
I387. 150 mg/mL secu; 10 mM Phos-Lact; 100 mM sucr; 0.5 mg/mL PS80; 1 mM met; water; and pH 5.8.
I388. 190 mg/mL secu; 10 mM Phos-Lact; 100 mM sucr; 0.5 mg/mL PS80; 1 mM met; water; and pH 5.8.
I389. 150 mg/mL secu; 15 mM Gluc; 100 mM sucr; 0.5 mg/mL PS80; 1 mM met; water; and pH 5.8.
I390. 190 mg/mL secu; 15 mM Gluc; 100 mM sucr; 0.5 mg/mL PS80; 1 mM met; water; and pH 5.8.
I391. 150 mg/mL secu; 0 mM buffer; 200 mM treh; 0.5 mg/mL PS80; 1 mM met; water; and pH 5.8.
I392. 190 mg/mL secu; 0 mM buffer; 200 mM treh; 0.5 mg/mL PS80; 1 mM met; water; and pH 5.8.
I393. 150 mg/mL secu; 50 mM His; 200 mM treh; 0.5 mg/mL PS80; 1 mM met; water; and pH 5.8.
I394. 190 mg/mL secu; 50 mM His; 200 mM treh; 0.5 mg/mL PS80; 1 mM met; water; and pH 5.8.

(continued)

I395. 150 mg/mL secu; 25 mM Lact; 200 mM treh; 0.5 mg/mL PS80; 1 mM met; water; and pH 5.8.
I396. 190 mg/mL secu; 25 mM Lact; 200 mM treh; 0.5 mg/mL PS80; 1 mM met; water; and pH 5.8.
I397. 150 mg/mL secu; 10 mM Phos-Lact; 200 mM treh; 0.5 mg/mL PS80; 1 mM met; water; and pH 5.8.
I398. 190 mg/mL secu; 10 mM Phos-Lact; 200 mM treh; 0.5 mg/mL PS80; 1 mM met; water; and pH 5.8.
I399. 150 mg/mL secu; 15 mM Gluc; 200 mM treh; 0.5 mg/mL PS80; 1 mM met; water; and pH 5.8.
I400. 190 mg/mL secu; 15 mM Gluc; 200 mM treh; 0.5 mg/mL PS80; 1 mM met; water; and pH 5.8.
I401. 150 mg/mL secu; 0 mM buffer; 150 mM sorb; 0.2 mg/mL PS20; 1 mM met; water; and pH 5.8.
I402. 190 mg/mL secu; 0 mM buffer; 150 mM sorb; 0.2 mg/mL PS20; 1 mM met; water; and pH 5.8.
I403. 150 mg/mL secu; 50 mM His; 150 mM sorb; 0.2 mg/mL PS20; 1 mM met; water; and pH 5.8.
I404. 190 mg/mL secu; 50 mM His; 150 mM sorb; 0.2 mg/mL PS20; 1 mM met; water; and pH 5.8.
I405. 150 mg/mL secu; 25 mM Lact; 150 mM sorb; 0.2 mg/mL PS20; 1 mM met; water; and pH 5.8.
I406. 190 mg/mL secu; 25 mM Lact; 150 mM sorb; 0.2 mg/mL PS20; 1 mM met; water; and pH 5.8.
I407. 150 mg/mL secu; 10 mM Phos-Lact; 150 mM sorb; 0.2 mg/mL PS20; 1 mM met; water; and pH 5.8.
I408. 190 mg/mL secu; 10 mM Phos-Lact; 150 mM sorb; 0.2 mg/mL PS20; 1 mM met; water; and pH 5.8.
I409. 150 mg/mL secu; 15 mM Gluc; 150 mM sorb; 0.2 mg/mL PS20; 1 mM met; water; and pH 5.8.
I410. 190 mg/mL secu; 15 mM Gluc; 150 mM sorb; 0.2 mg/mL PS20; 1 mM met; water; and pH 5.8.
I411. 150 mg/mL secu; 0 mM buffer; 75 mM mann; 0.2 mg/mL PS20; 1 mM met; water; and pH 5.8.
I412. 190 mg/mL secu; 0 mM buffer; 75 mM mann; 0.2 mg/mL PS20; 1 mM met; water; and pH 5.8.
I413. 150 mg/mL secu; 50 mM His; 75 mM mann; 0.2 mg/mL PS20; 1 mM met; water; and pH 5.8.
I414. 190 mg/mL secu; 50 mM His; 75 mM mann; 0.2 mg/mL PS20; 1 mM met; water; and pH 5.8.
I415. 150 mg/mL secu; 25 mM Lact; 75 mM mann; 0.2 mg/mL PS20; 1 mM met; water; and pH 5.8.
I416. 190 mg/mL secu; 25 mM Lact; 75 mM mann; 0.2 mg/mL PS20; 1 mM met; water; and pH 5.8.
I417. 150 mg/mL secu; 10 mM Phos-Lact; 75 mM mann; 0.2 mg/mL PS20; 1 mM met; water; and pH 5.8.
I418. 190 mg/mL secu; 10 mM Phos-Lact; 75 mM mann; 0.2 mg/mL PS20; 1 mM met; water; and pH 5.8.
I419. 150 mg/mL secu; 15 mM Gluc; 75 mM mann; 0.2 mg/mL PS20; 1 mM met; water; and pH 5.8.
I420. 190 mg/mL secu; 15 mM Gluc; 75 mM mann; 0.2 mg/mL PS20; 1 mM met; water; and pH 5.8.
I421. 150 mg/mL secu; 0 mM buffer; 100 mM sucr; 0.2 mg/mL PS20; 1 mM met; water; and pH 5.8.
I422. 190 mg/mL secu; 0 mM buffer; 100 mM sucr; 0.2 mg/mL PS20; 1 mM met; water; and pH 5.8.
I423. 150 mg/mL secu; 50 mM His; 100 mM sucr; 0.2 mg/mL PS20; 1 mM met; water; and pH 5.8.
I424. 190 mg/mL secu; 50 mM His; 100 mM sucr; 0.2 mg/mL PS20; 1 mM met; water; and pH 5.8.
I425. 150 mg/mL secu; 25 mM Lact; 100 mM sucr; 0.2 mg/mL PS20; 1 mM met; water; and pH 5.8.
I426. 190 mg/mL secu; 25 mM Lact; 100 mM sucr; 0.2 mg/mL PS20; 1 mM met; water; and pH 5.8.
I427. 150 mg/mL secu; 10 mM Phos-Lact; 100 mM sucr; 0.2 mg/mL PS20; 1 mM met; water; and pH 5.8.
I428. 190 mg/mL secu; 10 mM Phos-Lact; 100 mM sucr; 0.2 mg/mL PS20; 1 mM met; water; and pH 5.8.
I429. 150 mg/mL secu; 15 mM Gluc; 100 mM sucr; 0.2 mg/mL PS20; 1 mM met; water; and pH 5.8.
I430. 190 mg/mL secu; 15 mM Gluc; 100 mM sucr; 0.2 mg/mL PS20; 1 mM met; water; and pH 5.8.
I431. 150 mg/mL secu; 0 mM buffer; 200 mM treh; 0.2 mg/mL PS20; 1 mM met; water; and pH 5.8.
I432. 190 mg/mL secu; 0 mM buffer; 200 mM treh; 0.2 mg/mL PS20; 1 mM met; water; and pH 5.8.
I433. 150 mg/mL secu; 50 mM His; 200 mM treh; 0.2 mg/mL PS20; 1 mM met; water; and pH 5.8.
I434. 190 mg/mL secu; 50 mM His; 200 mM treh; 0.2 mg/mL PS20; 1 mM met; water; and pH 5.8.
I435. 150 mg/mL secu; 25 mM Lact; 200 mM treh; 0.2 mg/mL PS20; 1 mM met; water; and pH 5.8.
I436. 190 mg/mL secu; 25 mM Lact; 200 mM treh; 0.2 mg/mL PS20; 1 mM met; water; and pH 5.8.
I437. 150 mg/mL secu; 10 mM Phos-Lact; 200 mM treh; 0.2 mg/mL PS20; 1 mM met; water; and pH 5.8.
I438. 190 mg/mL secu; 10 mM Phos-Lact; 200 mM treh; 0.2 mg/mL PS20; 1 mM met; water; and pH 5.8.
I439. 150 mg/mL secu; 15 mM Gluc; 200 mM treh; 0.2 mg/mL PS20; 1 mM met; water; and pH 5.8.
I440. 190 mg/mL secu; 15 mM Gluc; 200 mM treh; 0.2 mg/mL PS20; 1 mM met; water; and pH 5.8.
I441. 150 mg/mL secu; 0 mM buffer; 150 mM sorb; 0.5 mg/mL PS80; 50 mM NaCl; water; and pH 5.8.
I442. 190 mg/mL secu; 0 mM buffer; 150 mM sorb; 0.5 mg/mL PS80; 50 mM NaCl; water; and pH 5.8.
I443. 150 mg/mL secu; 50 mM His; 150 mM sorb; 0.5 mg/mL PS80; 50 mM NaCl; water; and pH 5.8.
I444. 190 mg/mL secu; 50 mM His; 150 mM sorb; 0.5 mg/mL PS80; 50 mM NaCl; water; and pH 5.8.
I445. 150 mg/mL secu; 25 mM Lact; 150 mM sorb; 0.5 mg/mL PS80; 50 mM NaCl; water; and pH 5.8.

(continued)

| | |
|---|---|
| I446. | 190 mg/mL secu; 25 mM Lact; 150 mM sorb; 0.5 mg/mL PS80; 50 mM NaCl; water; and pH 5.8. |
| I447. | 150 mg/mL secu; 10 mM Phos-Lact; 150 mM sorb; 0.5 mg/mL PS80; 50 mM NaCl; water; and pH 5.8. |
| I448. | 190 mg/mL secu; 10 mM Phos-Lact; 150 mM sorb; 0.5 mg/mL PS80; 50 mM NaCl; water; and pH 5.8. |
| I449. | 150 mg/mL secu; 15 mM Gluc; 150 mM sorb; 0.5 mg/mL PS80; 50 mM NaCl; water; and pH 5.8. |
| I450. | 190 mg/mL secu; 15 mM Gluc; 150 mM sorb; 0.5 mg/mL PS80; 50 mM NaCl; water; and pH 5.8. |
| I451. | 150 mg/mL secu; 0 mM buffer; 75 mM mann; 0.5 mg/mL PS80; 50 mM NaCl; water; and pH 5.8. |
| I452. | 190 mg/mL secu; 0 mM buffer; 75 mM mann; 0.5 mg/mL PS80; 50 mM NaCl; water; and pH 5.8. |
| I453. | 150 mg/mL secu; 50 mM His; 75 mM mann; 0.5 mg/mL PS80; 50 mM NaCl; water; and pH 5.8. |
| I454. | 190 mg/mL secu; 50 mM His; 75 mM mann; 0.5 mg/mL PS80; 50 mM NaCl; water; and pH 5.8. |
| I455. | 150 mg/mL secu; 25 mM Lact; 75 mM mann; 0.5 mg/mL PS80; 50 mM NaCl; water; and pH 5.8. |
| I456. | 190 mg/mL secu; 25 mM Lact; 75 mM mann; 0.5 mg/mL PS80; 50 mM NaCl; water; and pH 5.8. |
| I457. | 150 mg/mL secu; 10 mM Phos-Lact; 75 mM mann; 0.5 mg/mL PS80; 50 mM NaCl; water; and pH 5.8. |
| I458. | 190 mg/mL secu; 10 mM Phos-Lact; 75 mM mann; 0.5 mg/mL PS80; 50 mM NaCl; water; and pH 5.8. |
| I459. | 150 mg/mL secu; 15 mM Gluc; 75 mM mann; 0.5 mg/mL PS80; 50 mM NaCl; water; and pH 5.8. |
| I460. | 190 mg/mL secu; 15 mM Gluc; 75 mM mann; 0.5 mg/mL PS80; 50 mM NaCl; water; and pH 5.8. |
| I461. | 150 mg/mL secu; 0 mM buffer; 100 mM sucr; 0.5 mg/mL PS80; 50 mM NaCl; water; and pH 5.8. |
| I462. | 190 mg/mL secu; 0 mM buffer; 100 mM sucr; 0.5 mg/mL PS80; 50 mM NaCl; water; and pH 5.8. |
| I463. | 150 mg/mL secu; 50 mM His; 100 mM sucr; 0.5 mg/mL PS80; 50 mM NaCl; water; and pH 5.8. |
| I464. | 190 mg/mL secu; 50 mM His; 100 mM sucr; 0.5 mg/mL PS80; 50 mM NaCl; water; and pH 5.8. |
| I465. | 150 mg/mL secu; 25 mM Lact; 100 mM sucr; 0.5 mg/mL PS80; 50 mM NaCl; water; and pH 5.8. |
| I466. | 190 mg/mL secu; 25 mM Lact; 100 mM sucr; 0.5 mg/mL PS80; 50 mM NaCl; water; and pH 5.8. |
| I467. | 150 mg/mL secu; 10 mM Phos-Lact; 100 mM sucr; 0.5 mg/mL PS80; 50 mM NaCl; water; and pH 5.8. |
| I468. | 190 mg/mL secu; 10 mM Phos-Lact; 100 mM sucr; 0.5 mg/mL PS80; 50 mM NaCl; water; and pH 5.8. |
| I469. | 150 mg/mL secu; 15 mM Gluc; 100 mM sucr; 0.5 mg/mL PS80; 50 mM NaCl; water; and pH 5.8. |
| I470. | 190 mg/mL secu; 15 mM Gluc; 100 mM sucr; 0.5 mg/mL PS80; 50 mM NaCl; water; and pH 5.8. |
| I471. | 150 mg/mL secu; 0 mM buffer; 200 mM treh; 0.5 mg/mL PS80; 50 mM NaCl; water; and pH 5.8. |
| I472. | 190 mg/mL secu; 0 mM buffer; 200 mM treh; 0.5 mg/mL PS80; 50 mM NaCl; water; and pH 5.8. |
| I473. | 150 mg/mL secu; 50 mM His; 200 mM treh; 0.5 mg/mL PS80; 50 mM NaCl; water; and pH 5.8. |
| I474. | 190 mg/mL secu; 50 mM His; 200 mM treh; 0.5 mg/mL PS80; 50 mM NaCl; water; and pH 5.8. |
| I475. | 150 mg/mL secu; 25 mM Lact; 200 mM treh; 0.5 mg/mL PS80; 50 mM NaCl; water; and pH 5.8. |
| I476. | 190 mg/mL secu; 25 mM Lact; 200 mM treh; 0.5 mg/mL PS80; 50 mM NaCl; water; and pH 5.8. |
| I477. | 150 mg/mL secu; 10 mM Phos-Lact; 200 mM treh; 0.5 mg/mL PS80; 50 mM NaCl; water; and pH 5.8. |
| I478. | 190 mg/mL secu; 10 mM Phos-Lact; 200 mM treh; 0.5 mg/mL PS80; 50 mM NaCl; water; and pH 5.8. |
| I479. | 150 mg/mL secu; 15 mM Gluc; 200 mM treh; 0.5 mg/mL PS80; 50 mM NaCl; water; and pH 5.8. |
| I480. | 190 mg/mL secu; 15 mM Gluc; 200 mM treh; 0.5 mg/mL PS80; 50 mM NaCl; water; and pH 5.8. |
| I481. | 150 mg/mL secu; 0 mM buffer; 150 mM sorb; 0.2 mg/mL PS20; 50 mM NaCl; water; and pH 5.8. |
| I482. | 190 mg/mL secu; 0 mM buffer; 150 mM sorb; 0.2 mg/mL PS20; 50 mM NaCl; water; and pH 5.8. |
| I483. | 150 mg/mL secu; 50 mM His; 150 mM sorb; 0.2 mg/mL PS20; 50 mM NaCl; water; and pH 5.8. |
| I484. | 190 mg/mL secu; 50 mM His; 150 mM sorb; 0.2 mg/mL PS20; 50 mM NaCl; water; and pH 5.8. |
| I485. | 150 mg/mL secu; 25 mM Lact; 150 mM sorb; 0.2 mg/mL PS20; 50 mM NaCl; water; and pH 5.8. |
| I486. | 190 mg/mL secu; 25 mM Lact; 150 mM sorb; 0.2 mg/mL PS20; 50 mM NaCl; water; and pH 5.8. |
| I487. | 150 mg/mL secu; 10 mM Phos-Lact; 150 mM sorb; 0.2 mg/mL PS20; 50 mM NaCl; water; and pH 5.8. |
| I488. | 190 mg/mL secu; 10 mM Phos-Lact; 150 mM sorb; 0.2 mg/mL PS20; 50 mM NaCl; water; and pH 5.8. |
| I489. | 150 mg/mL secu; 15 mM Gluc; 150 mM sorb; 0.2 mg/mL PS20; 50 mM NaCl; water; and pH 5.8. |
| I490. | 190 mg/mL secu; 15 mM Gluc; 150 mM sorb; 0.2 mg/mL PS20; 50 mM NaCl; water; and pH 5.8. |
| I491. | 150 mg/mL secu; 0 mM buffer; 75 mM mann; 0.2 mg/mL PS20; 50 mM NaCl; water; and pH 5.8. |
| I492. | 190 mg/mL secu; 0 mM buffer; 75 mM mann; 0.2 mg/mL PS20; 50 mM NaCl; water; and pH 5.8. |
| I493. | 150 mg/mL secu; 50 mM His; 75 mM mann; 0.2 mg/mL PS20; 50 mM NaCl; water; and pH 5.8. |
| I494. | 190 mg/mL secu; 50 mM His; 75 mM mann; 0.2 mg/mL PS20; 50 mM NaCl; water; and pH 5.8. |
| I495. | 150 mg/mL secu; 25 mM Lact; 75 mM mann; 0.2 mg/mL PS20; 50 mM NaCl; water; and pH 5.8. |
| I496. | 190 mg/mL secu; 25 mM Lact; 75 mM mann; 0.2 mg/mL PS20; 50 mM NaCl; water; and pH 5.8. |

(continued)

I497. 150 mg/mL secu; 10 mM Phos-Lact; 75 mM mann; 0.2 mg/mL PS20; 50 mM NaCl; water; and pH 5.8.
I498. 190 mg/mL secu; 10 mM Phos-Lact; 75 mM mann; 0.2 mg/mL PS20; 50 mM NaCl; water; and pH 5.8.
I499. 150 mg/mL secu; 15 mM Gluc; 75 mM mann; 0.2 mg/mL PS20; 50 mM NaCl; water; and pH 5.8.
I500. 190 mg/mL secu; 15 mM Gluc; 75 mM mann; 0.2 mg/mL PS20; 50 mM NaCl; water; and pH 5.8.
I501. 150 mg/mL secu; 0 mM buffer; 100 mM sucr; 0.2 mg/mL PS20; 50 mM NaCl; water; and pH 5.8.
I502. 190 mg/mL secu; 0 mM buffer; 100 mM sucr; 0.2 mg/mL PS20; 50 mM NaCl; water; and pH 5.8.
I503. 150 mg/mL secu; 50 mM His; 100 mM sucr; 0.2 mg/mL PS20; 50 mM NaCl; water; and pH 5.8.
I504. 190 mg/mL secu; 50 mM His; 100 mM sucr; 0.2 mg/mL PS20; 50 mM NaCl; water; and pH 5.8.
I505. 150 mg/mL secu; 25 mM Lact; 100 mM sucr; 0.2 mg/mL PS20; 50 mM NaCl; water; and pH 5.8.
I506. 190 mg/mL secu; 25 mM Lact; 100 mM sucr; 0.2 mg/mL PS20; 50 mM NaCl; water; and pH 5.8.
I507. 150 mg/mL secu; 10 mM Phos-Lact; 100 mM sucr; 0.2 mg/mL PS20; 50 mM NaCl; water; and pH 5.8.
I508. 190 mg/mL secu; 10 mM Phos-Lact; 100 mM sucr; 0.2 mg/mL PS20; 50 mM NaCl; water; and pH 5.8.
I509. 150 mg/mL secu; 15 mM Gluc; 100 mM sucr; 0.2 mg/mL PS20; 50 mM NaCl; water; and pH 5.8.
I510. 190 mg/mL secu; 15 mM Gluc; 100 mM sucr; 0.2 mg/mL PS20; 50 mM NaCl; water; and pH 5.8.
I511. 150 mg/mL secu; 0 mM buffer; 200 mM treh; 0.2 mg/mL PS20; 50 mM NaCl; water; and pH 5.8.
I512. 190 mg/mL secu; 0 mM buffer; 200 mM treh; 0.2 mg/mL PS20; 50 mM NaCl; water; and pH 5.8.
I513. 150 mg/mL secu; 50 mM His; 200 mM treh; 0.2 mg/mL PS20; 50 mM NaCl; water; and pH 5.8.
I514. 190 mg/mL secu; 50 mM His; 200 mM treh; 0.2 mg/mL PS20; 50 mM NaCl; water; and pH 5.8.
I515. 150 mg/mL secu; 25 mM Lact; 200 mM treh; 0.2 mg/mL PS20; 50 mM NaCl; water; and pH 5.8.
I516. 190 mg/mL secu; 25 mM Lact; 200 mM treh; 0.2 mg/mL PS20; 50 mM NaCl; water; and pH 5.8.
I517. 150 mg/mL secu; 10 mM Phos-Lact; 200 mM treh; 0.2 mg/mL PS20; 50 mM NaCl; water; and pH 5.8.
I518. 190 mg/mL secu; 10 mM Phos-Lact; 200 mM treh; 0.2 mg/mL PS20; 50 mM NaCl; water; and pH 5.8.
I519. 150 mg/mL secu; 15 mM Gluc; 200 mM treh; 0.2 mg/mL PS20; 50 mM NaCl; water; and pH 5.8.
I520. 190 mg/mL secu; 15 mM Gluc; 200 mM treh; 0.2 mg/mL PS20; 50 mM NaCl; water; and pH 5.8.
I521. 150 mg/mL secu; 0 mM buffer; 150 mM sorb; 0.5 mg/mL PS80; 0.1 mM EDTA; water; and pH 5.8.
I522. 190 mg/mL secu; 0 mM buffer; 150 mM sorb; 0.5 mg/mL PS80; 0.1 mM EDTA; water; and pH 5.8.
I523. 150 mg/mL secu; 50 mM His; 150 mM sorb; 0.5 mg/mL PS80; 0.1 mM EDTA; water; and pH 5.8.
I524. 190 mg/mL secu; 50 mM His; 150 mM sorb; 0.5 mg/mL PS80; 0.1 mM EDTA; water; and pH 5.8.
I525. 150 mg/mL secu; 25 mM Lact; 150 mM sorb; 0.5 mg/mL PS80; 0.1 mM EDTA; water; and pH 5.8.
I526. 190 mg/mL secu; 25 mM Lact; 150 mM sorb; 0.5 mg/mL PS80; 0.1 mM EDTA; water; and pH 5.8.
I527. 150 mg/mL secu; 10 mM Phos-Lact; 150 mM sorb; 0.5 mg/mL PS80; 0.1 mM EDTA; water; and pH 5.8.
I528. 190 mg/mL secu; 10 mM Phos-Lact; 150 mM sorb; 0.5 mg/mL PS80; 0.1 mM EDTA; water; and pH 5.8.
I529. 150 mg/mL secu; 15 mM Gluc; 150 mM sorb; 0.5 mg/mL PS80; 0.1 mM EDTA; water; and pH 5.8.
I530. 190 mg/mL secu; 15 mM Gluc; 150 mM sorb; 0.5 mg/mL PS80; 0.1 mM EDTA; water; and pH 5.8.
I531. 150 mg/mL secu; 0 mM buffer; 75 mM mann; 0.5 mg/mL PS80; 0.1 mM EDTA; water; and pH 5.8.
I532. 190 mg/mL secu; 0 mM buffer; 75 mM mann; 0.5 mg/mL PS80; 0.1 mM EDTA; water; and pH 5.8.
I533. 150 mg/mL secu; 50 mM His; 75 mM mann; 0.5 mg/mL PS80; 0.1 mM EDTA; water; and pH 5.8.
I534. 190 mg/mL secu; 50 mM His; 75 mM mann; 0.5 mg/mL PS80; 0.1 mM EDTA; water; and pH 5.8.
I535. 150 mg/mL secu; 25 mM Lact; 75 mM mann; 0.5 mg/mL PS80; 0.1 mM EDTA; water; and pH 5.8.
I536. 190 mg/mL secu; 25 mM Lact; 75 mM mann; 0.5 mg/mL PS80; 0.1 mM EDTA; water; and pH 5.8.
I537. 150 mg/mL secu; 10 mM Phos-Lact; 75 mM mann; 0.5 mg/mL PS80; 0.1 mM EDTA; water; and pH 5.8.
I538. 190 mg/mL secu; 10 mM Phos-Lact; 75 mM mann; 0.5 mg/mL PS80; 0.1 mM EDTA; water; and pH 5.8.
I539. 150 mg/mL secu; 15 mM Gluc; 75 mM mann; 0.5 mg/mL PS80; 0.1 mM EDTA; water; and pH 5.8.
I540. 190 mg/mL secu; 15 mM Gluc; 75 mM mann; 0.5 mg/mL PS80; 0.1 mM EDTA; water; and pH 5.8.
I541. 150 mg/mL secu; 0 mM buffer; 100 mM sucr; 0.5 mg/mL PS80; 0.1 mM EDTA; water; and pH 5.8.
I542. 190 mg/mL secu; 0 mM buffer; 100 mM sucr; 0.5 mg/mL PS80; 0.1 mM EDTA; water; and pH 5.8.
I543. 150 mg/mL secu; 50 mM His; 100 mM sucr; 0.5 mg/mL PS80; 0.1 mM EDTA; water; and pH 5.8.
I544. 190 mg/mL secu; 50 mM His; 100 mM sucr; 0.5 mg/mL PS80; 0.1 mM EDTA; water; and pH 5.8.
I545. 150 mg/mL secu; 25 mM Lact; 100 mM sucr; 0.5 mg/mL PS80; 0.1 mM EDTA; water; and pH 5.8.
I546. 190 mg/mL secu; 25 mM Lact; 100 mM sucr; 0.5 mg/mL PS80; 0.1 mM EDTA; water; and pH 5.8.
I547. 150 mg/mL secu; 10 mM Phos-Lact; 100 mM sucr; 0.5 mg/mL PS80; 0.1 mM EDTA; water; and pH 5.8.

(continued)

I548. 190 mg/mL secu; 10 mM Phos-Lact; 100 mM sucr; 0.5 mg/mL PS80; 0.1 mM EDTA; water; and pH 5.8.
I549. 150 mg/mL secu; 15 mM Gluc; 100 mM sucr; 0.5 mg/mL PS80; 0.1 mM EDTA; water; and pH 5.8.
I550. 190 mg/mL secu; 15 mM Gluc; 100 mM sucr; 0.5 mg/mL PS80; 0.1 mM EDTA; water; and pH 5.8.
I551. 150 mg/mL secu; 0 mM buffer; 200 mM treh; 0.5 mg/mL PS80; 0.1 mM EDTA; water; and pH 5.8.
I552. 190 mg/mL secu; 0 mM buffer; 200 mM treh; 0.5 mg/mL PS80; 0.1 mM EDTA; water; and pH 5.8.
I553. 150 mg/mL secu; 50 mM His; 200 mM treh; 0.5 mg/mL PS80; 0.1 mM EDTA; water; and pH 5.8.
I554. 190 mg/mL secu; 50 mM His; 200 mM treh; 0.5 mg/mL PS80; 0.1 mM EDTA; water; and pH 5.8.
I555. 150 mg/mL secu; 25 mM Lact; 200 mM treh; 0.5 mg/mL PS80; 0.1 mM EDTA; water; and pH 5.8.
I556. 190 mg/mL secu; 25 mM Lact; 200 mM treh; 0.5 mg/mL PS80; 0.1 mM EDTA; water; and pH 5.8.
I557. 150 mg/mL secu; 10 mM Phos-Lact; 200 mM treh; 0.5 mg/mL PS80; 0.1 mM EDTA; water; and pH 5.8.
I558. 190 mg/mL secu; 10 mM Phos-Lact; 200 mM treh; 0.5 mg/mL PS80; 0.1 mM EDTA; water; and pH 5.8.
I559. 150 mg/mL secu; 15 mM Gluc; 200 mM treh; 0.5 mg/mL PS80; 0.1 mM EDTA; water; and pH 5.8.
I560. 190 mg/mL secu; 15 mM Gluc; 200 mM treh; 0.5 mg/mL PS80; 0.1 mM EDTA; water; and pH 5.8.
I561. 150 mg/mL secu; 0 mM buffer; 150 mM sorb; 0.2 mg/mL PS20; 0.1 mM EDTA; water; and pH 5.8.
I562. 190 mg/mL secu; 0 mM buffer; 150 mM sorb; 0.2 mg/mL PS20; 0.1 mM EDTA; water; and pH 5.8.
I563. 150 mg/mL secu; 50 mM His; 150 mM sorb; 0.2 mg/mL PS20; 0.1 mM EDTA; water; and pH 5.8.
I564. 190 mg/mL secu; 50 mM His; 150 mM sorb; 0.2 mg/mL PS20; 0.1 mM EDTA; water; and pH 5.8.
I565. 150 mg/mL secu; 25 mM Lact; 150 mM sorb; 0.2 mg/mL PS20; 0.1 mM EDTA; water; and pH 5.8.
I566. 190 mg/mL secu; 25 mM Lact; 150 mM sorb; 0.2 mg/mL PS20; 0.1 mM EDTA; water; and pH 5.8.
I567. 150 mg/mL secu; 10 mM Phos-Lact; 150 mM sorb; 0.2 mg/mL PS20; 0.1 mM EDTA; water; and pH 5.8.
I568. 190 mg/mL secu; 10 mM Phos-Lact; 150 mM sorb; 0.2 mg/mL PS20; 0.1 mM EDTA; water; and pH 5.8.
I569. 150 mg/mL secu; 15 mM Gluc; 150 mM sorb; 0.2 mg/mL PS20; 0.1 mM EDTA; water; and pH 5.8.
I570. 190 mg/mL secu; 15 mM Gluc; 150 mM sorb; 0.2 mg/mL PS20; 0.1 mM EDTA; water; and pH 5.8.
I571. 150 mg/mL secu; 0 mM buffer; 75 mM mann; 0.2 mg/mL PS20; 0.1 mM EDTA; water; and pH 5.8.
I572. 190 mg/mL secu; 0 mM buffer; 75 mM mann; 0.2 mg/mL PS20; 0.1 mM EDTA; water; and pH 5.8.
I573. 150 mg/mL secu; 50 mM His; 75 mM mann; 0.2 mg/mL PS20; 0.1 mM EDTA; water; and pH 5.8.
I574. 190 mg/mL secu; 50 mM His; 75 mM mann; 0.2 mg/mL PS20; 0.1 mM EDTA; water; and pH 5.8.
I575. 150 mg/mL secu; 25 mM Lact; 75 mM mann; 0.2 mg/mL PS20; 0.1 mM EDTA; water; and pH 5.8.
I576. 190 mg/mL secu; 25 mM Lact; 75 mM mann; 0.2 mg/mL PS20; 0.1 mM EDTA; water; and pH 5.8.
I577. 150 mg/mL secu; 10 mM Phos-Lact; 75 mM mann; 0.2 mg/mL PS20; 0.1 mM EDTA; water; and pH 5.8.
I578. 190 mg/mL secu; 10 mM Phos-Lact; 75 mM mann; 0.2 mg/mL PS20; 0.1 mM EDTA; water; and pH 5.8.
I579. 150 mg/mL secu; 15 mM Gluc; 75 mM mann; 0.2 mg/mL PS20; 0.1 mM EDTA; water; and pH 5.8.
I580. 190 mg/mL secu; 15 mM Gluc; 75 mM mann; 0.2 mg/mL PS20; 0.1 mM EDTA; water; and pH 5.8.
I581. 150 mg/mL secu; 0 mM buffer; 100 mM sucr; 0.2 mg/mL PS20; 0.1 mM EDTA; water; and pH 5.8.
I582. 190 mg/mL secu; 0 mM buffer; 100 mM sucr; 0.2 mg/mL PS20; 0.1 mM EDTA; water; and pH 5.8.
I583. 150 mg/mL secu; 50 mM His; 100 mM sucr; 0.2 mg/mL PS20; 0.1 mM EDTA; water; and pH 5.8.
I584. 190 mg/mL secu; 50 mM His; 100 mM sucr; 0.2 mg/mL PS20; 0.1 mM EDTA; water; and pH 5.8.
I585. 150 mg/mL secu; 25 mM Lact; 100 mM sucr; 0.2 mg/mL PS20; 0.1 mM EDTA; water; and pH 5.8.
I586. 190 mg/mL secu; 25 mM Lact; 100 mM sucr; 0.2 mg/mL PS20; 0.1 mM EDTA; water; and pH 5.8.
I587. 150 mg/mL secu; 10 mM Phos-Lact; 100 mM sucr; 0.2 mg/mL PS20; 0.1 mM EDTA; water; and pH 5.8.
I588. 190 mg/mL secu; 10 mM Phos-Lact; 100 mM sucr; 0.2 mg/mL PS20; 0.1 mM EDTA; water; and pH 5.8.
I589. 150 mg/mL secu; 15 mM Gluc; 100 mM sucr; 0.2 mg/mL PS20; 0.1 mM EDTA; water; and pH 5.8.
I590. 190 mg/mL secu; 15 mM Gluc; 100 mM sucr; 0.2 mg/mL PS20; 0.1 mM EDTA; water; and pH 5.8.
I591. 150 mg/mL secu; 0 mM buffer; 200 mM treh; 0.2 mg/mL PS20; 0.1 mM EDTA; water; and pH 5.8.
I592. 190 mg/mL secu; 0 mM buffer; 200 mM treh; 0.2 mg/mL PS20; 0.1 mM EDTA; water; and pH 5.8.
I593. 150 mg/mL secu; 50 mM His; 200 mM treh; 0.2 mg/mL PS20; 0.1 mM EDTA; water; and pH 5.8.
I594. 190 mg/mL secu; 50 mM His; 200 mM treh; 0.2 mg/mL PS20; 0.1 mM EDTA; water; and pH 5.8.
I595. 150 mg/mL secu; 25 mM Lact; 200 mM treh; 0.2 mg/mL PS20; 0.1 mM EDTA; water; and pH 5.8.
I596. 190 mg/mL secu; 25 mM Lact; 200 mM treh; 0.2 mg/mL PS20; 0.1 mM EDTA; water; and pH 5.8.
I597. 150 mg/mL secu; 10 mM Phos-Lact; 200 mM treh; 0.2 mg/mL PS20; 0.1 mM EDTA; water; and pH 5.8.
I598. 190 mg/mL secu; 10 mM Phos-Lact; 200 mM treh; 0.2 mg/mL PS20; 0.1 mM EDTA; water; and pH 5.8.

(continued)

I599. 150 mg/mL secu; 15 mM Gluc; 200 mM treh; 0.2 mg/mL PS20; 0.1 mM EDTA; water; and pH 5.8.
I600. 190 mg/mL secu; 15 mM Gluc; 200 mM treh; 0.2 mg/mL PS20; 0.1 mM EDTA; water; and pH 5.8.
I601. 150 mg/mL secu; 0 mM buffer; 50 mM arg; 0.5 mg/mL PS80; 1 mM met; water; and pH 5.8.
I602. 190 mg/mL secu; 0 mM buffer; 50 mM arg; 0.5 mg/mL PS80; 1 mM met; water; and pH 5.8.
I603. 150 mg/mL secu; 50 mM His; 50 mM arg; 0.5 mg/mL PS80; 1 mM met; water; and pH 5.8.
I604. 190 mg/mL secu; 50 mM His; 50 mM arg; 0.5 mg/mL PS80; 1 mM met; water; and pH 5.8.
I605. 150 mg/mL secu; 25 mM Lact; 50 mM arg; 0.5 mg/mL PS80; 1 mM met; water; and pH 5.8.
I606. 190 mg/mL secu; 25 mM Lact; 50 mM arg; 0.5 mg/mL PS80; 1 mM met; water; and pH 5.8.
I607. 150 mg/mL secu; 10 mM Phos-Lact; 50 mM arg; 0.5 mg/mL PS80; 1 mM met; water; and pH 5.8.
I608. 190 mg/mL secu; 10 mM Phos-Lact; 50 mM arg; 0.5 mg/mL PS80; 1 mM met; water; and pH 5.8.
I609. 150 mg/mL secu; 15 mM Gluc; 50 mM arg; 0.5 mg/mL PS80; 1 mM met; water; and pH 5.8.
I610. 190 mg/mL secu; 15 mM Gluc; 50 mM arg; 0.5 mg/mL PS80; 1 mM met; water; and pH 5.8.
I611. 150 mg/mL secu; 0 mM buffer; 100 mM gly; 0.5 mg/mL PS80; 1 mM met; water; and pH 5.8.
I612. 190 mg/mL secu; 0 mM buffer; 100 mM gly; 0.5 mg/mL PS80; 1 mM met; water; and pH 5.8.
I613. 150 mg/mL secu; 50 mM His; 100 mM gly; 0.5 mg/mL PS80; 1 mM met; water; and pH 5.8.
I614. 190 mg/mL secu; 50 mM His; 100 mM gly; 0.5 mg/mL PS80; 1 mM met; water; and pH 5.8.
I615. 150 mg/mL secu; 25 mM Lact; 100 mM gly; 0.5 mg/mL PS80; 1 mM met; water; and pH 5.8.
I616. 190 mg/mL secu; 25 mM Lact; 100 mM gly; 0.5 mg/mL PS80; 1 mM met; water; and pH 5.8.
I617. 150 mg/mL secu; 10 mM Phos-Lact; 100 mM gly; 0.5 mg/mL PS80; 1 mM met; water; and pH 5.8.
I618. 190 mg/mL secu; 10 mM Phos-Lact; 100 mM gly; 0.5 mg/mL PS80; 1 mM met; water; and pH 5.8.
I619. 150 mg/mL secu; 15 mM Gluc; 100 mM gly; 0.5 mg/mL PS80; 1 mM met; water; and pH 5.8.
I620. 190 mg/mL secu; 15 mM Gluc; 100 mM gly; 0.5 mg/mL PS80; 1 mM met; water; and pH 5.8.
I621. 150 mg/mL secu; 0 mM buffer; 50 mM arg; 0.2 mg/mL PS20; 1 mM met; water; and pH 5.8.
I622. 190 mg/mL secu; 0 mM buffer; 50 mM arg; 0.2 mg/mL PS20; 1 mM met; water; and pH 5.8.
I623. 150 mg/mL secu; 50 mM His; 50 mM arg; 0.2 mg/mL PS20; 1 mM met; water; and pH 5.8.
I624. 190 mg/mL secu; 50 mM His; 50 mM arg; 0.2 mg/mL PS20; 1 mM met; water; and pH 5.8.
I625. 150 mg/mL secu; 25 mM Lact; 50 mM arg; 0.2 mg/mL PS20; 1 mM met; water; and pH 5.8.
I626. 190 mg/mL secu; 25 mM Lact; 50 mM arg; 0.2 mg/mL PS20; 1 mM met; water; and pH 5.8.
I627. 150 mg/mL secu; 10 mM Phos-Lact; 50 mM arg; 0.2 mg/mL PS20; 1 mM met; water; and pH 5.8.
I628. 190 mg/mL secu; 10 mM Phos-Lact; 50 mM arg; 0.2 mg/mL PS20; 1 mM met; water; and pH 5.8.
I629. 150 mg/mL secu; 15 mM Gluc; 50 mM arg; 0.2 mg/mL PS20; 1 mM met; water; and pH 5.8.
I630. 190 mg/mL secu; 15 mM Gluc; 50 mM arg; 0.2 mg/mL PS20; 1 mM met; water; and pH 5.8.
I631. 150 mg/mL secu; 0 mM buffer; 100 mM gly; 0.2 mg/mL PS20; 1 mM met; water; and pH 5.8.
I632. 190 mg/mL secu; 0 mM buffer; 100 mM gly; 0.2 mg/mL PS20; 1 mM met; water; and pH 5.8.
I633. 150 mg/mL secu; 50 mM His; 100 mM gly; 0.2 mg/mL PS20; 1 mM met; water; and pH 5.8.
I634. 190 mg/mL secu; 50 mM His; 100 mM gly; 0.2 mg/mL PS20; 1 mM met; water; and pH 5.8.
I635. 150 mg/mL secu; 25 mM Lact; 100 mM gly; 0.2 mg/mL PS20; 1 mM met; water; and pH 5.8.
I636. 190 mg/mL secu; 25 mM Lact; 100 mM gly; 0.2 mg/mL PS20; 1 mM met; water; and pH 5.8.
I637. 150 mg/mL secu; 10 mM Phos-Lact; 100 mM gly; 0.2 mg/mL PS20; 1 mM met; water; and pH 5.8.
I638. 190 mg/mL secu; 10 mM Phos-Lact; 100 mM gly; 0.2 mg/mL PS20; 1 mM met; water; and pH 5.8.
I639. 150 mg/mL secu; 15 mM Gluc; 100 mM gly; 0.2 mg/mL PS20; 1 mM met; water; and pH 5.8.
I640. 190 mg/mL secu; 15 mM Gluc; 100 mM gly; 0.2 mg/mL PS20; 1 mM met; water; and pH 5.8.
I641. 150 mg/mL secu; 0 mM buffer; 50 mM arg; 0.5 mg/mL PS80; 50 mM NaCl; water; and pH 5.8.
I642. 190 mg/mL secu; 0 mM buffer; 50 mM arg; 0.5 mg/mL PS80; 50 mM NaCl; water; and pH 5.8.
I643. 150 mg/mL secu; 50 mM His; 50 mM arg; 0.5 mg/mL PS80; 50 mM NaCl; water; and pH 5.8.
I644. 190 mg/mL secu; 50 mM His; 50 mM arg; 0.5 mg/mL PS80; 50 mM NaCl; water; and pH 5.8.
I645. 150 mg/mL secu; 25 mM Lact; 50 mM arg; 0.5 mg/mL PS80; 50 mM NaCl; water; and pH 5.8.
I646. 190 mg/mL secu; 25 mM Lact; 50 mM arg; 0.5 mg/mL PS80; 50 mM NaCl; water; and pH 5.8.
I647. 150 mg/mL secu; 10 mM Phos-Lact; 50 mM arg; 0.5 mg/mL PS80; 50 mM NaCl; water; and pH 5.8.
I648. 190 mg/mL secu; 10 mM Phos-Lact; 50 mM arg; 0.5 mg/mL PS80; 50 mM NaCl; water; and pH 5.8.
I649. 150 mg/mL secu; 15 mM Gluc; 50 mM arg; 0.5 mg/mL PS80; 50 mM NaCl; water; and pH 5.8.

(continued)

I650. 190 mg/mL secu; 15 mM Gluc; 50 mM arg; 0.5 mg/mL PS80; 50 mM NaCl; water; and pH 5.8.
I651. 150 mg/mL secu; 0 mM buffer; 100 mM gly; 0.5 mg/mL PS80; 50 mM NaCl; water; and pH 5.8.
I652. 190 mg/mL secu; 0 mM buffer; 100 mM gly; 0.5 mg/mL PS80; 50 mM NaCl; water; and pH 5.8.
I653. 150 mg/mL secu; 50 mM His; 100 mM gly; 0.5 mg/mL PS80; 50 mM NaCl; water; and pH 5.8.
I654. 190 mg/mL secu; 50 mM His; 100 mM gly; 0.5 mg/mL PS80; 50 mM NaCl; water; and pH 5.8.
I655. 150 mg/mL secu; 25 mM Lact; 100 mM gly; 0.5 mg/mL PS80; 50 mM NaCl; water; and pH 5.8.
I656. 190 mg/mL secu; 25 mM Lact; 100 mM gly; 0.5 mg/mL PS80; 50 mM NaCl; water; and pH 5.8.
I657. 150 mg/mL secu; 10 mM Phos-Lact; 100 mM gly; 0.5 mg/mL PS80; 50 mM NaCl; water; and pH 5.8.
I658. 190 mg/mL secu; 10 mM Phos-Lact; 100 mM gly; 0.5 mg/mL PS80; 50 mM NaCl; water; and pH 5.8.
I659. 150 mg/mL secu; 15 mM Gluc; 100 mM gly; 0.5 mg/mL PS80; 50 mM NaCl; water; and pH 5.8.
I660. 190 mg/mL secu; 15 mM Gluc; 100 mM gly; 0.5 mg/mL PS80; 50 mM NaCl; water; and pH 5.8.
I661. 150 mg/mL secu; 0 mM buffer; 50 mM arg; 0.2 mg/mL PS20; 50 mM NaCl; water; and pH 5.8.
I662. 190 mg/mL secu; 0 mM buffer; 50 mM arg; 0.2 mg/mL PS20; 50 mM NaCl; water; and pH 5.8.
I663. 150 mg/mL secu; 50 mM His; 50 mM arg; 0.2 mg/mL PS20; 50 mM NaCl; water; and pH 5.8.
I664. 190 mg/mL secu; 50 mM His; 50 mM arg; 0.2 mg/mL PS20; 50 mM NaCl; water; and pH 5.8.
I665. 150 mg/mL secu; 25 mM Lact; 50 mM arg; 0.2 mg/mL PS20; 50 mM NaCl; water; and pH 5.8.
I666. 190 mg/mL secu; 25 mM Lact; 50 mM arg; 0.2 mg/mL PS20; 50 mM NaCl; water; and pH 5.8.
I667. 150 mg/mL secu; 10 mM Phos-Lact; 50 mM arg; 0.2 mg/mL PS20; 50 mM NaCl; water; and pH 5.8.
I668. 190 mg/mL secu; 10 mM Phos-Lact; 50 mM arg; 0.2 mg/mL PS20; 50 mM NaCl; water; and pH 5.8.
I669. 150 mg/mL secu; 15 mM Gluc; 50 mM arg; 0.2 mg/mL PS20; 50 mM NaCl; water; and pH 5.8.
I670. 190 mg/mL secu; 15 mM Gluc; 50 mM arg; 0.2 mg/mL PS20; 50 mM NaCl; water; and pH 5.8.
I671. 150 mg/mL secu; 0 mM buffer; 100 mM gly; 0.2 mg/mL PS20; 50 mM NaCl; water; and pH 5.8.
I672. 190 mg/mL secu; 0 mM buffer; 100 mM gly; 0.2 mg/mL PS20; 50 mM NaCl; water; and pH 5.8.
I673. 150 mg/mL secu; 50 mM His; 100 mM gly; 0.2 mg/mL PS20; 50 mM NaCl; water; and pH 5.8.
I674. 190 mg/mL secu; 50 mM His; 100 mM gly; 0.2 mg/mL PS20; 50 mM NaCl; water; and pH 5.8.
I675. 150 mg/mL secu; 25 mM Lact; 100 mM gly; 0.2 mg/mL PS20; 50 mM NaCl; water; and pH 5.8.
I676. 190 mg/mL secu; 25 mM Lact; 100 mM gly; 0.2 mg/mL PS20; 50 mM NaCl; water; and pH 5.8.
I677. 150 mg/mL secu; 10 mM Phos-Lact; 100 mM gly; 0.2 mg/mL PS20; 50 mM NaCl; water; and pH 5.8.
I678. 190 mg/mL secu; 10 mM Phos-Lact; 100 mM gly; 0.2 mg/mL PS20; 50 mM NaCl; water; and pH 5.8.
I679. 150 mg/mL secu; 15 mM Gluc; 100 mM gly; 0.2 mg/mL PS20; 50 mM NaCl; water; and pH 5.8.
I680. 190 mg/mL secu; 15 mM Gluc; 100 mM gly; 0.2 mg/mL PS20; 50 mM NaCl; water; and pH 5.8.
I681. 150 mg/mL secu; 0 mM buffer; 50 mM arg; 0.5 mg/mL PS80; 0.1 mM EDTA; water; and pH 5.8.
I682. 190 mg/mL secu; 0 mM buffer; 50 mM arg; 0.5 mg/mL PS80; 0.1 mM EDTA; water; and pH 5.8.
I683. 150 mg/mL secu; 50 mM His; 50 mM arg; 0.5 mg/mL PS80; 0.1 mM EDTA; water; and pH 5.8.
I684. 190 mg/mL secu; 50 mM His; 50 mM arg; 0.5 mg/mL PS80; 0.1 mM EDTA; water; and pH 5.8.
I685. 150 mg/mL secu; 25 mM Lact; 50 mM arg; 0.5 mg/mL PS80; 0.1 mM EDTA; water; and pH 5.8.
I686. 190 mg/mL secu; 25 mM Lact; 50 mM arg; 0.5 mg/mL PS80; 0.1 mM EDTA; water; and pH 5.8.
I687. 150 mg/mL secu; 10 mM Phos-Lact; 50 mM arg; 0.5 mg/mL PS80; 0.1 mM EDTA; water; and pH 5.8.
I688. 190 mg/mL secu; 10 mM Phos-Lact; 50 mM arg; 0.5 mg/mL PS80; 0.1 mM EDTA; water; and pH 5.8.
I689. 150 mg/mL secu; 15 mM Gluc; 50 mM arg; 0.5 mg/mL PS80; 0.1 mM EDTA; water; and pH 5.8.
I690. 190 mg/mL secu; 15 mM Gluc; 50 mM arg; 0.5 mg/mL PS80; 0.1 mM EDTA; water; and pH 5.8.
I691. 150 mg/mL secu; 0 mM buffer; 100 mM gly; 0.5 mg/mL PS80; 0.1 mM EDTA; water; and pH 5.8.
I692. 190 mg/mL secu; 0 mM buffer; 100 mM gly; 0.5 mg/mL PS80; 0.1 mM EDTA; water; and pH 5.8.
I693. 150 mg/mL secu; 50 mM His; 100 mM gly; 0.5 mg/mL PS80; 0.1 mM EDTA; water; and pH 5.8.
I694. 190 mg/mL secu; 50 mM His; 100 mM gly; 0.5 mg/mL PS80; 0.1 mM EDTA; water; and pH 5.8.
I695. 150 mg/mL secu; 25 mM Lact; 100 mM gly; 0.5 mg/mL PS80; 0.1 mM EDTA; water; and pH 5.8.
I696. 190 mg/mL secu; 25 mM Lact; 100 mM gly; 0.5 mg/mL PS80; 0.1 mM EDTA; water; and pH 5.8.
I697. 150 mg/mL secu; 10 mM Phos-Lact; 100 mM gly; 0.5 mg/mL PS80; 0.1 mM EDTA; water; and pH 5.8.
I698. 190 mg/mL secu; 10 mM Phos-Lact; 100 mM gly; 0.5 mg/mL PS80; 0.1 mM EDTA; water; and pH 5.8.
I699. 150 mg/mL secu; 15 mM Gluc; 100 mM gly; 0.5 mg/mL PS80; 0.1 mM EDTA; water; and pH 5.8.
I700. 190 mg/mL secu; 15 mM Gluc; 100 mM gly; 0.5 mg/mL PS80; 0.1 mM EDTA; water; and pH 5.8.

(continued)

I701. 150 mg/mL secu; 0 mM buffer; 50 mM arg; 0.2 mg/mL PS20; 0.1 mM EDTA; water; and pH 5.8.
I702. 190 mg/mL secu; 0 mM buffer; 50 mM arg; 0.2 mg/mL PS20; 0.1 mM EDTA; water; and pH 5.8.
I703. 150 mg/mL secu; 50 mM His; 50 mM arg; 0.2 mg/mL PS20; 0.1 mM EDTA; water; and pH 5.8.
I704. 190 mg/mL secu; 50 mM His; 50 mM arg; 0.2 mg/mL PS20; 0.1 mM EDTA; water; and pH 5.8.
I705. 150 mg/mL secu; 25 mM Lact; 50 mM arg; 0.2 mg/mL PS20; 0.1 mM EDTA; water; and pH 5.8.
I706. 190 mg/mL secu; 25 mM Lact; 50 mM arg; 0.2 mg/mL PS20; 0.1 mM EDTA; water; and pH 5.8.
I707. 150 mg/mL secu; 10 mM Phos-Lact; 50 mM arg; 0.2 mg/mL PS20; 0.1 mM EDTA; water; and pH 5.8.
I708. 190 mg/mL secu; 10 mM Phos-Lact; 50 mM arg; 0.2 mg/mL PS20; 0.1 mM EDTA; water; and pH 5.8.
I709. 150 mg/mL secu; 15 mM Gluc; 50 mM arg; 0.2 mg/mL PS20; 0.1 mM EDTA; water; and pH 5.8.
I710. 190 mg/mL secu; 15 mM Gluc; 50 mM arg; 0.2 mg/mL PS20; 0.1 mM EDTA; water; and pH 5.8.
I711. 150 mg/mL secu; 0 mM buffer; 100 mM gly; 0.2 mg/mL PS20; 0.1 mM EDTA; water; and pH 5.8.
I712. 190 mg/mL secu; 0 mM buffer; 100 mM gly; 0.2 mg/mL PS20; 0.1 mM EDTA; water; and pH 5.8.
I713. 150 mg/mL secu; 50 mM His; 100 mM gly; 0.2 mg/mL PS20; 0.1 mM EDTA; water; and pH 5.8.
I714. 190 mg/mL secu; 50 mM His; 100 mM gly; 0.2 mg/mL PS20; 0.1 mM EDTA; water; and pH 5.8.
I715. 150 mg/mL secu; 25 mM Lact; 100 mM gly; 0.2 mg/mL PS20; 0.1 mM EDTA; water; and pH 5.8.
I716. 190 mg/mL secu; 25 mM Lact; 100 mM gly; 0.2 mg/mL PS20; 0.1 mM EDTA; water; and pH 5.8.
I717. 150 mg/mL secu; 10 mM Phos-Lact; 100 mM gly; 0.2 mg/mL PS20; 0.1 mM EDTA; water; and pH 5.8.
I718. 190 mg/mL secu; 10 mM Phos-Lact; 100 mM gly; 0.2 mg/mL PS20; 0.1 mM EDTA; water; and pH 5.8.
I719. 150 mg/mL secu; 15 mM Gluc; 100 mM gly; 0.2 mg/mL PS20; 0.1 mM EDTA; water; and pH 5.8.
I720. 190 mg/mL secu; 15 mM Gluc; 100 mM gly; 0.2 mg/mL PS20; 0.1 mM EDTA; water; and pH 5.8.
I721. 150 mg/mL secu; 0 mM buffer; 0.5 mg/mL PS80; 1 mM met; 50 mM NaCl; water; and pH 5.8.
I722. 190 mg/mL secu; 0 mM buffer; 0.5 mg/mL PS80; 1 mM met; 50 mM NaCl; water; and pH 5.8.
I723. 150 mg/mL secu; 50 mM His; 0.5 mg/mL PS80; 1 mM met; 50 mM NaCl; water; and pH 5.8.
I724. 190 mg/mL secu; 50 mM His; 0.5 mg/mL PS80; 1 mM met; 50 mM NaCl; water; and pH 5.8.
I725. 150 mg/mL secu; 25 mM Lact; 0.5 mg/mL PS80; 1 mM met; 50 mM NaCl; water; and pH 5.8.
I726. 190 mg/mL secu; 25 mM Lact; 0.5 mg/mL PS80; 1 mM met; 50 mM NaCl; water; and pH 5.8.
I727. 150 mg/mL secu; 10 mM Phos-Lact; 0.5 mg/mL PS80; 1 mM met; 50 mM NaCl; water; and pH 5.8.
I728. 190 mg/mL secu; 10 mM Phos-Lact; 0.5 mg/mL PS80; 1 mM met; 50 mM NaCl; water; and pH 5.8.
I729. 150 mg/mL secu; 15 mM Gluc; 0.5 mg/mL PS80; 1 mM met; 50 mM NaCl; water; and pH 5.8.
I730. 190 mg/mL secu; 15 mM Gluc; 0.5 mg/mL PS80; 1 mM met; 50 mM NaCl; water; and pH 5.8.
I731. 150 mg/mL secu; 0 mM buffer; 0.2 mg/mL PS20; 1 mM met; 50 mM NaCl; water; and pH 5.8.
I732. 190 mg/mL secu; 0 mM buffer; 0.2 mg/mL PS20; 1 mM met; 50 mM NaCl; water; and pH 5.8.
I733. 150 mg/mL secu; 50 mM His; 0.2 mg/mL PS20; 1 mM met; 50 mM NaCl; water; and pH 5.8.
I734. 190 mg/mL secu; 50 mM His; 0.2 mg/mL PS20; 1 mM met; 50 mM NaCl; water; and pH 5.8.
I735. 150 mg/mL secu; 25 mM Lact; 0.2 mg/mL PS20; 1 mM met; 50 mM NaCl; water; and pH 5.8.
I736. 190 mg/mL secu; 25 mM Lact; 0.2 mg/mL PS20; 1 mM met; 50 mM NaCl; water; and pH 5.8.
I737. 150 mg/mL secu; 10 mM Phos-Lact; 0.2 mg/mL PS20; 1 mM met; 50 mM NaCl; water; and pH 5.8.
I738. 190 mg/mL secu; 10 mM Phos-Lact; 0.2 mg/mL PS20; 1 mM met; 50 mM NaCl; water; and pH 5.8.
I739. 150 mg/mL secu; 15 mM Gluc; 0.2 mg/mL PS20; 1 mM met; 50 mM NaCl; water; and pH 5.8.
I740. 190 mg/mL secu; 15 mM Gluc; 0.2 mg/mL PS20; 1 mM met; 50 mM NaCl; water; and pH 5.8.
I741. 150 mg/mL secu; 0 mM buffer; 0.5 mg/mL PS80; 1 mM met; 0.1 mM EDTA; water; and pH 5.8.
I742. 190 mg/mL secu; 0 mM buffer; 0.5 mg/mL PS80; 1 mM met; 0.1 mM EDTA; water; and pH 5.8.
I743. 150 mg/mL secu; 50 mM His; 0.5 mg/mL PS80; 1 mM met; 0.1 mM EDTA; water; and pH 5.8.
I744. 190 mg/mL secu; 50 mM His; 0.5 mg/mL PS80; 1 mM met; 0.1 mM EDTA; water; and pH 5.8.
I745. 150 mg/mL secu; 25 mM Lact; 0.5 mg/mL PS80; 1 mM met; 0.1 mM EDTA; water; and pH 5.8.
I746. 190 mg/mL secu; 25 mM Lact; 0.5 mg/mL PS80; 1 mM met; 0.1 mM EDTA; water; and pH 5.8.
I747. 150 mg/mL secu; 10 mM Phos-Lact; 0.5 mg/mL PS80; 1 mM met; 0.1 mM EDTA; water; and pH 5.8.
I748. 190 mg/mL secu; 10 mM Phos-Lact; 0.5 mg/mL PS80; 1 mM met; 0.1 mM EDTA; water; and pH 5.8.
I749. 150 mg/mL secu; 15 mM Gluc; 0.5 mg/mL PS80; 1 mM met; 0.1 mM EDTA; water; and pH 5.8.
I750. 190 mg/mL secu; 15 mM Gluc; 0.5 mg/mL PS80; 1 mM met; 0.1 mM EDTA; water; and pH 5.8.
I751. 150 mg/mL secu; 0 mM buffer; 0.2 mg/mL PS20; 1 mM met; 0.1 mM EDTA; water; and pH 5.8.

(continued)

I752. 190 mg/mL secu; 0 mM buffer; 0.2 mg/mL PS20; 1 mM met; 0.1 mM EDTA; water; and pH 5.8.
I753. 150 mg/mL secu; 50 mM His; 0.2 mg/mL PS20; 1 mM met; 0.1 mM EDTA; water; and pH 5.8.
I754. 190 mg/mL secu; 50 mM His; 0.2 mg/mL PS20; 1 mM met; 0.1 mM EDTA; water; and pH 5.8.
I755. 150 mg/mL secu; 25 mM Lact; 0.2 mg/mL PS20; 1 mM met; 0.1 mM EDTA; water; and pH 5.8.
I756. 190 mg/mL secu; 25 mM Lact; 0.2 mg/mL PS20; 1 mM met; 0.1 mM EDTA; water; and pH 5.8.
I757. 150 mg/mL secu; 10 mM Phos-Lact; 0.2 mg/mL PS20; 1 mM met; 0.1 mM EDTA; water; and pH 5.8.
I758. 190 mg/mL secu; 10 mM Phos-Lact; 0.2 mg/mL PS20; 1 mM met; 0.1 mM EDTA; water; and pH 5.8.
I759. 150 mg/mL secu; 15 mM Gluc; 0.2 mg/mL PS20; 1 mM met; 0.1 mM EDTA; water; and pH 5.8.
I760. 190 mg/mL secu; 15 mM Gluc; 0.2 mg/mL PS20; 1 mM met; 0.1 mM EDTA; water; and pH 5.8.
I761. 150 mg/mL secu; 0 mM buffer; 0.5 mg/mL PS80; 50 mM NaCl; 0.1 mM EDTA; water; and pH 5.8.
I762. 190 mg/mL secu; 0 mM buffer; 0.5 mg/mL PS80; 50 mM NaCl; 0.1 mM EDTA; water; and pH 5.8.
I763. 150 mg/mL secu; 50 mM His; 0.5 mg/mL PS80; 50 mM NaCl; 0.1 mM EDTA; water; and pH 5.8.
I764. 190 mg/mL secu; 50 mM His; 0.5 mg/mL PS80; 50 mM NaCl; 0.1 mM EDTA; water; and pH 5.8.
I765. 150 mg/mL secu; 25 mM Lact; 0.5 mg/mL PS80; 50 mM NaCl; 0.1 mM EDTA; water; and pH 5.8.
I766. 190 mg/mL secu; 25 mM Lact; 0.5 mg/mL PS80; 50 mM NaCl; 0.1 mM EDTA; water; and pH 5.8.
I767. 150 mg/mL secu; 10 mM Phos-Lact; 0.5 mg/mL PS80; 50 mM NaCl; 0.1 mM EDTA; water; and pH 5.8.
I768. 190 mg/mL secu; 10 mM Phos-Lact; 0.5 mg/mL PS80; 50 mM NaCl; 0.1 mM EDTA; water; and pH 5.8.
I769. 150 mg/mL secu; 15 mM Gluc; 0.5 mg/mL PS80; 50 mM NaCl; 0.1 mM EDTA; water; and pH 5.8.
I770. 190 mg/mL secu; 15 mM Gluc; 0.5 mg/mL PS80; 50 mM NaCl; 0.1 mM EDTA; water; and pH 5.8.
I771. 150 mg/mL secu; 0 mM buffer; 0.2 mg/mL PS20; 50 mM NaCl; 0.1 mM EDTA; water; and pH 5.8.
I772. 190 mg/mL secu; 0 mM buffer; 0.2 mg/mL PS20; 50 mM NaCl; 0.1 mM EDTA; water; and pH 5.8.
I773. 150 mg/mL secu; 50 mM His; 0.2 mg/mL PS20; 50 mM NaCl; 0.1 mM EDTA; water; and pH 5.8.
I774. 190 mg/mL secu; 50 mM His; 0.2 mg/mL PS20; 50 mM NaCl; 0.1 mM EDTA; water; and pH 5.8.
I775. 150 mg/mL secu; 25 mM Lact; 0.2 mg/mL PS20; 50 mM NaCl; 0.1 mM EDTA; water; and pH 5.8.
I776. 190 mg/mL secu; 25 mM Lact; 0.2 mg/mL PS20; 50 mM NaCl; 0.1 mM EDTA; water; and pH 5.8.
I777. 150 mg/mL secu; 10 mM Phos-Lact; 0.2 mg/mL PS20; 50 mM NaCl; 0.1 mM EDTA; water; and pH 5.8.
I778. 190 mg/mL secu; 10 mM Phos-Lact; 0.2 mg/mL PS20; 50 mM NaCl; 0.1 mM EDTA; water; and pH 5.8.
I779. 150 mg/mL secu; 15 mM Gluc; 0.2 mg/mL PS20; 50 mM NaCl; 0.1 mM EDTA; water; and pH 5.8.
I780. 190 mg/mL secu; 15 mM Gluc; 0.2 mg/mL PS20; 50 mM NaCl; 0.1 mM EDTA; water; and pH 5.8.
I781. 150 mg/mL secu; 0 mM buffer; 150 mM sorb; 50 mM arg; 0.5 mg/mL PS80; 1 mM met; water; and pH 5.8.
I782. 190 mg/mL secu; 0 mM buffer; 150 mM sorb; 50 mM arg; 0.5 mg/mL PS80; 1 mM met; water; and pH 5.8.
I783. 150 mg/mL secu; 50 mM His; 150 mM sorb; 50 mM arg; 0.5 mg/mL PS80; 1 mM met; water; and pH 5.8.
I784. 190 mg/mL secu; 50 mM His; 150 mM sorb; 50 mM arg; 0.5 mg/mL PS80; 1 mM met; water; and pH 5.8.
I785. 150 mg/mL secu; 25 mM Lact; 150 mM sorb; 50 mM arg; 0.5 mg/mL PS80; 1 mM met; water; and pH 5.8.
I786. 190 mg/mL secu; 25 mM Lact; 150 mM sorb; 50 mM arg; 0.5 mg/mL PS80; 1 mM met; water; and pH 5.8.
I787. 150 mg/mL secu; 10 mM Phos-Lact; 150 mM sorb; 50 mM arg; 0.5 mg/mL PS80; 1 mM met; water; and pH 5.8.
I788. 190 mg/mL secu; 10 mM Phos-Lact; 150 mM sorb; 50 mM arg; 0.5 mg/mL PS80; 1 mM met; water; and pH 5.8.
I789. 150 mg/mL secu; 15 mM Gluc; 150 mM sorb; 50 mM arg; 0.5 mg/mL PS80; 1 mM met; water; and pH 5.8.
I790. 190 mg/mL secu; 15 mM Gluc; 150 mM sorb; 50 mM arg; 0.5 mg/mL PS80; 1 mM met; water; and pH 5.8.
I791. 150 mg/mL secu; 0 mM buffer; 75 mM mann; 50 mM arg; 0.5 mg/mL PS80; 1 mM met; water; and pH 5.8.
I792. 190 mg/mL secu; 0 mM buffer; 75 mM mann; 50 mM arg; 0.5 mg/mL PS80; 1 mM met; water; and pH 5.8.
I793. 150 mg/mL secu; 50 mM His; 75 mM mann; 50 mM arg; 0.5 mg/mL PS80; 1 mM met; water; and pH 5.8.
I794. 190 mg/mL secu; 50 mM His; 75 mM mann; 50 mM arg; 0.5 mg/mL PS80; 1 mM met; water; and pH 5.8.
I795. 150 mg/mL secu; 25 mM Lact; 75 mM mann; 50 mM arg; 0.5 mg/mL PS80; 1 mM met; water; and pH 5.8.
I796. 190 mg/mL secu; 25 mM Lact; 75 mM mann; 50 mM arg; 0.5 mg/mL PS80; 1 mM met; water; and pH 5.8.
I797. 150 mg/mL secu; 10 mM Phos-Lact; 75 mM mann; 50 mM arg; 0.5 mg/mL PS80; 1 mM met; water; and pH 5.8.
I798. 190 mg/mL secu; 10 mM Phos-Lact; 75 mM mann; 50 mM arg; 0.5 mg/mL PS80; 1 mM met; water; and pH 5.8.

(continued)

| | |
|---|---|
| I799. | 150 mg/mL secu; 15 mM Gluc; 75 mM mann; 50 mM arg; 0.5 mg/mL PS80; 1 mM met; water; and pH 5.8. |
| I800. | 190 mg/mL secu; 15 mM Gluc; 75 mM mann; 50 mM arg; 0.5 mg/mL PS80; 1 mM met; water; and pH 5.8. |
| I801. | 150 mg/mL secu; 0 mM buffer; 100 mM sucr; 50 mM arg; 0.5 mg/mL PS80; 1 mM met; water; and pH 5.8. |
| I802. | 190 mg/mL secu; 0 mM buffer; 100 mM sucr; 50 mM arg; 0.5 mg/mL PS80; 1 mM met; water; and pH 5.8. |
| I803. | 150 mg/mL secu; 50 mM His; 100 mM sucr; 50 mM arg; 0.5 mg/mL PS80; 1 mM met; water; and pH 5.8. |
| I804. | 190 mg/mL secu; 50 mM His; 100 mM sucr; 50 mM arg; 0.5 mg/mL PS80; 1 mM met; water; and pH 5.8. |
| I805. | 150 mg/mL secu; 25 mM Lact; 100 mM sucr; 50 mM arg; 0.5 mg/mL PS80; 1 mM met; water; and pH 5.8. |
| I806. | 190 mg/mL secu; 25 mM Lact; 100 mM sucr; 50 mM arg; 0.5 mg/mL PS80; 1 mM met; water; and pH 5.8. |
| I807. | 150 mg/mL secu; 10 mM Phos-Lact; 100 mM sucr; 50 mM arg; 0.5 mg/mL PS80; 1 mM met; water; and pH 5.8. |
| I808. | 190 mg/mL secu; 10 mM Phos-Lact; 100 mM sucr; 50 mM arg; 0.5 mg/mL PS80; 1 mM met; water; and pH 5.8. |
| I809. | 150 mg/mL secu; 15 mM Gluc; 100 mM sucr; 50 mM arg; 0.5 mg/mL PS80; 1 mM met; water; and pH 5.8. |
| I810. | 190 mg/mL secu; 15 mM Gluc; 100 mM sucr; 50 mM arg; 0.5 mg/mL PS80; 1 mM met; water; and pH 5.8. |
| I811. | 150 mg/mL secu; 0 mM buffer; 200 mM treh; 50 mM arg; 0.5 mg/mL PS80; 1 mM met; water; and pH 5.8. |
| I812. | 190 mg/mL secu; 0 mM buffer; 200 mM treh; 50 mM arg; 0.5 mg/mL PS80; 1 mM met; water; and pH 5.8. |
| I813. | 150 mg/mL secu; 50 mM His; 200 mM treh; 50 mM arg; 0.5 mg/mL PS80; 1 mM met; water; and pH 5.8. |
| I814. | 190 mg/mL secu; 50 mM His; 200 mM treh; 50 mM arg; 0.5 mg/mL PS80; 1 mM met; water; and pH 5.8. |
| I815. | 150 mg/mL secu; 25 mM Lact; 200 mM treh; 50 mM arg; 0.5 mg/mL PS80; 1 mM met; water; and pH 5.8. |
| I816. | 190 mg/mL secu; 25 mM Lact; 200 mM treh; 50 mM arg; 0.5 mg/mL PS80; 1 mM met; water; and pH 5.8. |
| I817. | 150 mg/mL secu; 10 mM Phos-Lact; 200 mM treh; 50 mM arg; 0.5 mg/mL PS80; 1 mM met; water; and pH 5.8. |
| I818. | 190 mg/mL secu; 10 mM Phos-Lact; 200 mM treh; 50 mM arg; 0.5 mg/mL PS80; 1 mM met; water; and pH 5.8. |
| I819. | 150 mg/mL secu; 15 mM Gluc; 200 mM treh; 50 mM arg; 0.5 mg/mL PS80; 1 mM met; water; and pH 5.8. |
| I820. | 190 mg/mL secu; 15 mM Gluc; 200 mM treh; 50 mM arg; 0.5 mg/mL PS80; 1 mM met; water; and pH 5.8. |
| I821. | 150 mg/mL secu; 0 mM buffer; 150 mM sorb; 100 mM gly; 0.5 mg/mL PS80; 1 mM met; water; and pH 5.8. |
| I822. | 190 mg/mL secu; 0 mM buffer; 150 mM sorb; 100 mM gly; 0.5 mg/mL PS80; 1 mM met; water; and pH 5.8. |
| I823. | 150 mg/mL secu; 50 mM His; 150 mM sorb; 100 mM gly; 0.5 mg/mL PS80; 1 mM met; water; and pH 5.8. |
| I824. | 190 mg/mL secu; 50 mM His; 150 mM sorb; 100 mM gly; 0.5 mg/mL PS80; 1 mM met; water; and pH 5.8. |
| I825. | 150 mg/mL secu; 25 mM Lact; 150 mM sorb; 100 mM gly; 0.5 mg/mL PS80; 1 mM met; water; and pH 5.8. |
| I826. | 190 mg/mL secu; 25 mM Lact; 150 mM sorb; 100 mM gly; 0.5 mg/mL PS80; 1 mM met; water; and pH 5.8. |
| I827. | 150 mg/mL secu; 10 mM Phos-Lact; 150 mM sorb; 100 mM gly; 0.5 mg/mL PS80; 1 mM met; water; and pH 5.8. |
| I828. | 190 mg/mL secu; 10 mM Phos-Lact; 150 mM sorb; 100 mM gly; 0.5 mg/mL PS80; 1 mM met; water; and pH 5.8. |
| I829. | 150 mg/mL secu; 15 mM Gluc; 150 mM sorb; 100 mM gly; 0.5 mg/mL PS80; 1 mM met; water; and pH 5.8. |
| I830. | 190 mg/mL secu; 15 mM Gluc; 150 mM sorb; 100 mM gly; 0.5 mg/mL PS80; 1 mM met; water; and pH 5.8. |
| I831. | 150 mg/mL secu; 0 mM buffer; 75 mM mann; 100 mM gly; 0.5 mg/mL PS80; 1 mM met; water; and pH 5.8. |
| I832. | 190 mg/mL secu; 0 mM buffer; 75 mM mann; 100 mM gly; 0.5 mg/mL PS80; 1 mM met; water; and pH 5.8. |
| I833. | 150 mg/mL secu; 50 mM His; 75 mM mann; 100 mM gly; 0.5 mg/mL PS80; 1 mM met; water; and pH 5.8. |
| I834. | 190 mg/mL secu; 50 mM His; 75 mM mann; 100 mM gly; 0.5 mg/mL PS80; 1 mM met; water; and pH 5.8. |
| I835. | 150 mg/mL secu; 25 mM Lact; 75 mM mann; 100 mM gly; 0.5 mg/mL PS80; 1 mM met; water; and pH 5.8. |
| I836. | 190 mg/mL secu; 25 mM Lact; 75 mM mann; 100 mM gly; 0.5 mg/mL PS80; 1 mM met; water; and pH 5.8. |
| I837. | 150 mg/mL secu; 10 mM Phos-Lact; 75 mM mann; 100 mM gly; 0.5 mg/mL PS80; 1 mM met; water; and pH 5.8. |
| I838. | 190 mg/mL secu; 10 mM Phos-Lact; 75 mM mann; 100 mM gly; 0.5 mg/mL PS80; 1 mM met; water; and pH 5.8. |
| I839. | 150 mg/mL secu; 15 mM Gluc; 75 mM mann; 100 mM gly; 0.5 mg/mL PS80; 1 mM met; water; and pH 5.8. |
| I840. | 190 mg/mL secu; 15 mM Gluc; 75 mM mann; 100 mM gly; 0.5 mg/mL PS80; 1 mM met; water; and pH 5.8. |
| I841. | 150 mg/mL secu; 0 mM buffer; 100 mM sucr; 100 mM gly; 0.5 mg/mL PS80; 1 mM met; water; and pH 5.8. |

(continued)

I842. 190 mg/mL secu; 0 mM buffer; 100 mM sucr; 100 mM gly; 0.5 mg/mL PS80; 1 mM met; water; and pH 5.8.
I843. 150 mg/mL secu; 50 mM His; 100 mM sucr; 100 mM gly; 0.5 mg/mL PS80; 1 mM met; water; and pH 5.8.
I844. 190 mg/mL secu; 50 mM His; 100 mM sucr; 100 mM gly; 0.5 mg/mL PS80; 1 mM met; water; and pH 5.8.
I845. 150 mg/mL secu; 25 mM Lact; 100 mM sucr; 100 mM gly; 0.5 mg/mL PS80; 1 mM met; water; and pH 5.8.
I846. 190 mg/mL secu; 25 mM Lact; 100 mM sucr; 100 mM gly; 0.5 mg/mL PS80; 1 mM met; water; and pH 5.8.
I847. 150 mg/mL secu; 10 mM Phos-Lact; 100 mM sucr; 100 mM gly; 0.5 mg/mL PS80; 1 mM met; water; and pH 5.8.
I848. 190 mg/mL secu; 10 mM Phos-Lact; 100 mM sucr; 100 mM gly; 0.5 mg/mL PS80; 1 mM met; water; and pH 5.8.
I849. 150 mg/mL secu; 15 mM Gluc; 100 mM sucr; 100 mM gly; 0.5 mg/mL PS80; 1 mM met; water; and pH 5.8.
I850. 190 mg/mL secu; 15 mM Gluc; 100 mM sucr; 100 mM gly; 0.5 mg/mL PS80; 1 mM met; water; and pH 5.8.
I851. 150 mg/mL secu; 0 mM buffer; 200 mM treh; 100 mM gly; 0.5 mg/mL PS80; 1 mM met; water; and pH 5.8.
I852. 190 mg/mL secu; 0 mM buffer; 200 mM treh; 100 mM gly; 0.5 mg/mL PS80; 1 mM met; water; and pH 5.8.
I853. 150 mg/mL secu; 50 mM His; 200 mM treh; 100 mM gly; 0.5 mg/mL PS80; 1 mM met; water; and pH 5.8.
I854. 190 mg/mL secu; 50 mM His; 200 mM treh; 100 mM gly; 0.5 mg/mL PS80; 1 mM met; water; and pH 5.8.
I855. 150 mg/mL secu; 25 mM Lact; 200 mM treh; 100 mM gly; 0.5 mg/mL PS80; 1 mM met; water; and pH 5.8.
I856. 190 mg/mL secu; 25 mM Lact; 200 mM treh; 100 mM gly; 0.5 mg/mL PS80; 1 mM met; water; and pH 5.8.
I857. 150 mg/mL secu; 10 mM Phos-Lact; 200 mM treh; 100 mM gly; 0.5 mg/mL PS80; 1 mM met; water; and pH 5.8.
I858. 190 mg/mL secu; 10 mM Phos-Lact; 200 mM treh; 100 mM gly; 0.5 mg/mL PS80; 1 mM met; water; and pH 5.8.
I859. 150 mg/mL secu; 15 mM Gluc; 200 mM treh; 100 mM gly; 0.5 mg/mL PS80; 1 mM met; water; and pH 5.8.
I860. 190 mg/mL secu; 15 mM Gluc; 200 mM treh; 100 mM gly; 0.5 mg/mL PS80; 1 mM met; water; and pH 5.8.
I861. 150 mg/mL secu; 0 mM buffer; 150 mM sorb; 50 mM arg; 0.2 mg/mL PS20; 1 mM met; water; and pH 5.8.
I862. 190 mg/mL secu; 0 mM buffer; 150 mM sorb; 50 mM arg; 0.2 mg/mL PS20; 1 mM met; water; and pH 5.8.
I863. 150 mg/mL secu; 50 mM His; 150 mM sorb; 50 mM arg; 0.2 mg/mL PS20; 1 mM met; water; and pH 5.8.
I864. 190 mg/mL secu; 50 mM His; 150 mM sorb; 50 mM arg; 0.2 mg/mL PS20; 1 mM met; water; and pH 5.8.
I865. 150 mg/mL secu; 25 mM Lact; 150 mM sorb; 50 mM arg; 0.2 mg/mL PS20; 1 mM met; water; and pH 5.8.
I866. 190 mg/mL secu; 25 mM Lact; 150 mM sorb; 50 mM arg; 0.2 mg/mL PS20; 1 mM met; water; and pH 5.8.
I867. 150 mg/mL secu; 10 mM Phos-Lact; 150 mM sorb; 50 mM arg; 0.2 mg/mL PS20; 1 mM met; water; and pH 5.8.
I868. 190 mg/mL secu; 10 mM Phos-Lact; 150 mM sorb; 50 mM arg; 0.2 mg/mL PS20; 1 mM met; water; and pH 5.8.
I869. 150 mg/mL secu; 15 mM Gluc; 150 mM sorb; 50 mM arg; 0.2 mg/mL PS20; 1 mM met; water; and pH 5.8.
I870. 190 mg/mL secu; 15 mM Gluc; 150 mM sorb; 50 mM arg; 0.2 mg/mL PS20; 1 mM met; water; and pH 5.8.
I871. 150 mg/mL secu; 0 mM buffer; 75 mM mann; 50 mM arg; 0.2 mg/mL PS20; 1 mM met; water; and pH 5.8.
I872. 190 mg/mL secu; 0 mM buffer; 75 mM mann; 50 mM arg; 0.2 mg/mL PS20; 1 mM met; water; and pH 5.8.
I873. 150 mg/mL secu; 50 mM His; 75 mM mann; 50 mM arg; 0.2 mg/mL PS20; 1 mM met; water; and pH 5.8.
I874. 190 mg/mL secu; 50 mM His; 75 mM mann; 50 mM arg; 0.2 mg/mL PS20; 1 mM met; water; and pH 5.8.
I875. 150 mg/mL secu; 25 mM Lact; 75 mM mann; 50 mM arg; 0.2 mg/mL PS20; 1 mM met; water; and pH 5.8.
I876. 190 mg/mL secu; 25 mM Lact; 75 mM mann; 50 mM arg; 0.2 mg/mL PS20; 1 mM met; water; and pH 5.8.
I877. 150 mg/mL secu; 10 mM Phos-Lact; 75 mM mann; 50 mM arg; 0.2 mg/mL PS20; 1 mM met; water; and pH 5.8.
I878. 190 mg/mL secu; 10 mM Phos-Lact; 75 mM mann; 50 mM arg; 0.2 mg/mL PS20; 1 mM met; water; and pH 5.8.
I879. 150 mg/mL secu; 15 mM Gluc; 75 mM mann; 50 mM arg; 0.2 mg/mL PS20; 1 mM met; water; and pH 5.8.
I880. 190 mg/mL secu; 15 mM Gluc; 75 mM mann; 50 mM arg; 0.2 mg/mL PS20; 1 mM met; water; and pH 5.8.
I881. 150 mg/mL secu; 0 mM buffer; 100 mM sucr; 50 mM arg; 0.2 mg/mL PS20; 1 mM met; water; and pH 5.8.
I882. 190 mg/mL secu; 0 mM buffer; 100 mM sucr; 50 mM arg; 0.2 mg/mL PS20; 1 mM met; water; and pH 5.8.
I883. 150 mg/mL secu; 50 mM His; 100 mM sucr; 50 mM arg; 0.2 mg/mL PS20; 1 mM met; water; and pH 5.8.
I884. 190 mg/mL secu; 50 mM His; 100 mM sucr; 50 mM arg; 0.2 mg/mL PS20; 1 mM met; water; and pH 5.8.

(continued)

I885. 150 mg/mL secu; 25 mM Lact; 100 mM sucr; 50 mM arg; 0.2 mg/mL PS20; 1 mM met; water; and pH 5.8.

I886. 190 mg/mL secu; 25 mM Lact; 100 mM sucr; 50 mM arg; 0.2 mg/mL PS20; 1 mM met; water; and pH 5.8.

I887. 150 mg/mL secu; 10 mM Phos-Lact; 100 mM sucr; 50 mM arg; 0.2 mg/mL PS20; 1 mM met; water; and pH 5.8.

I888. 190 mg/mL secu; 10 mM Phos-Lact; 100 mM sucr; 50 mM arg; 0.2 mg/mL PS20; 1 mM met; water; and pH 5.8.

I889. 150 mg/mL secu; 15 mM Gluc; 100 mM sucr; 50 mM arg; 0.2 mg/mL PS20; 1 mM met; water; and pH 5.8.

I890. 190 mg/mL secu; 15 mM Gluc; 100 mM sucr; 50 mM arg; 0.2 mg/mL PS20; 1 mM met; water; and pH 5.8.

I891. 150 mg/mL secu; 0 mM buffer; 200 mM treh; 50 mM arg; 0.2 mg/mL PS20; 1 mM met; water; and pH 5.8.

I892. 190 mg/mL secu; 0 mM buffer; 200 mM treh; 50 mM arg; 0.2 mg/mL PS20; 1 mM met; water; and pH 5.8.

I893. 150 mg/mL secu; 50 mM His; 200 mM treh; 50 mM arg; 0.2 mg/mL PS20; 1 mM met; water; and pH 5.8.

I894. 190 mg/mL secu; 50 mM His; 200 mM treh; 50 mM arg; 0.2 mg/mL PS20; 1 mM met; water; and pH 5.8.

I895. 150 mg/mL secu; 25 mM Lact; 200 mM treh; 50 mM arg; 0.2 mg/mL PS20; 1 mM met; water; and pH 5.8.

I896. 190 mg/mL secu; 25 mM Lact; 200 mM treh; 50 mM arg; 0.2 mg/mL PS20; 1 mM met; water; and pH 5.8.

I897. 150 mg/mL secu; 10 mM Phos-Lact; 200 mM treh; 50 mM arg; 0.2 mg/mL PS20; 1 mM met; water; and pH 5.8.

I898. 190 mg/mL secu; 10 mM Phos-Lact; 200 mM treh; 50 mM arg; 0.2 mg/mL PS20; 1 mM met; water; and pH 5.8.

I899. 150 mg/mL secu; 15 mM Gluc; 200 mM treh; 50 mM arg; 0.2 mg/mL PS20; 1 mM met; water; and pH 5.8.

I900. 190 mg/mL secu; 15 mM Gluc; 200 mM treh; 50 mM arg; 0.2 mg/mL PS20; 1 mM met; water; and pH 5.8.

I901. 150 mg/mL secu; 0 mM buffer; 150 mM sorb; 100 mM gly; 0.2 mg/mL PS20; 1 mM met; water; and pH 5.8.

I902. 190 mg/mL secu; 0 mM buffer; 150 mM sorb; 100 mM gly; 0.2 mg/mL PS20; 1 mM met; water; and pH 5.8.

I903. 150 mg/mL secu; 50 mM His; 150 mM sorb; 100 mM gly; 0.2 mg/mL PS20; 1 mM met; water; and pH 5.8.

I904. 190 mg/mL secu; 50 mM His; 150 mM sorb; 100 mM gly; 0.2 mg/mL PS20; 1 mM met; water; and pH 5.8.

I905. 150 mg/mL secu; 25 mM Lact; 150 mM sorb; 100 mM gly; 0.2 mg/mL PS20; 1 mM met; water; and pH 5.8.

I906. 190 mg/mL secu; 25 mM Lact; 150 mM sorb; 100 mM gly; 0.2 mg/mL PS20; 1 mM met; water; and pH 5.8.

I907. 150 mg/mL secu; 10 mM Phos-Lact; 150 mM sorb; 100 mM gly; 0.2 mg/mL PS20; 1 mM met; water; and pH 5.8.

I908. 190 mg/mL secu; 10 mM Phos-Lact; 150 mM sorb; 100 mM gly; 0.2 mg/mL PS20; 1 mM met; water; and pH 5.8.

I909. 150 mg/mL secu; 15 mM Gluc; 150 mM sorb; 100 mM gly; 0.2 mg/mL PS20; 1 mM met; water; and pH 5.8.

I910. 190 mg/mL secu; 15 mM Gluc; 150 mM sorb; 100 mM gly; 0.2 mg/mL PS20; 1 mM met; water; and pH 5.8.

I911. 150 mg/mL secu; 0 mM buffer; 75 mM mann; 100 mM gly; 0.2 mg/mL PS20; 1 mM met; water; and pH 5.8.

I912. 190 mg/mL secu; 0 mM buffer; 75 mM mann; 100 mM gly; 0.2 mg/mL PS20; 1 mM met; water; and pH 5.8.

I913. 150 mg/mL secu; 50 mM His; 75 mM mann; 100 mM gly; 0.2 mg/mL PS20; 1 mM met; water; and pH 5.8.

I914. 190 mg/mL secu; 50 mM His; 75 mM mann; 100 mM gly; 0.2 mg/mL PS20; 1 mM met; water; and pH 5.8.

I915. 150 mg/mL secu; 25 mM Lact; 75 mM mann; 100 mM gly; 0.2 mg/mL PS20; 1 mM met; water; and pH 5.8.

I916. 190 mg/mL secu; 25 mM Lact; 75 mM mann; 100 mM gly; 0.2 mg/mL PS20; 1 mM met; water; and pH 5.8.

I917. 150 mg/mL secu; 10 mM Phos-Lact; 75 mM mann; 100 mM gly; 0.2 mg/mL PS20; 1 mM met; water; and pH 5.8.

I918. 190 mg/mL secu; 10 mM Phos-Lact; 75 mM mann; 100 mM gly; 0.2 mg/mL PS20; 1 mM met; water; and pH 5.8.

I919. 150 mg/mL secu; 15 mM Gluc; 75 mM mann; 100 mM gly; 0.2 mg/mL PS20; 1 mM met; water; and pH 5.8.

I920. 190 mg/mL secu; 15 mM Gluc; 75 mM mann; 100 mM gly; 0.2 mg/mL PS20; 1 mM met; water; and pH 5.8.

I921. 150 mg/mL secu; 0 mM buffer; 100 mM sucr; 100 mM gly; 0.2 mg/mL PS20; 1 mM met; water; and pH 5.8.

I922. 190 mg/mL secu; 0 mM buffer; 100 mM sucr; 100 mM gly; 0.2 mg/mL PS20; 1 mM met; water; and pH 5.8.

I923. 150 mg/mL secu; 50 mM His; 100 mM sucr; 100 mM gly; 0.2 mg/mL PS20; 1 mM met; water; and pH 5.8.

I924. 190 mg/mL secu; 50 mM His; 100 mM sucr; 100 mM gly; 0.2 mg/mL PS20; 1 mM met; water; and pH 5.8.

I925. 150 mg/mL secu; 25 mM Lact; 100 mM sucr; 100 mM gly; 0.2 mg/mL PS20; 1 mM met; water; and pH 5.8.

I926. 190 mg/mL secu; 25 mM Lact; 100 mM sucr; 100 mM gly; 0.2 mg/mL PS20; 1 mM met; water; and pH 5.8.

(continued)

I927. 150 mg/mL secu; 10 mM Phos-Lact; 100 mM sucr; 100 mM gly; 0.2 mg/mL PS20; 1 mM met; water; and pH 5.8.

I928. 190 mg/mL secu; 10 mM Phos-Lact; 100 mM sucr; 100 mM gly; 0.2 mg/mL PS20; 1 mM met; water; and pH 5.8.

I929. 150 mg/mL secu; 15 mM Gluc; 100 mM sucr; 100 mM gly; 0.2 mg/mL PS20; 1 mM met; water; and pH 5.8.

I930. 190 mg/mL secu; 15 mM Gluc; 100 mM sucr; 100 mM gly; 0.2 mg/mL PS20; 1 mM met; water; and pH 5.8.

I931. 150 mg/mL secu; 0 mM buffer; 200 mM treh; 100 mM gly; 0.2 mg/mL PS20; 1 mM met; water; and pH 5.8.

I932. 190 mg/mL secu; 0 mM buffer; 200 mM treh; 100 mM gly; 0.2 mg/mL PS20; 1 mM met; water; and pH 5.8.

I933. 150 mg/mL secu; 50 mM His; 200 mM treh; 100 mM gly; 0.2 mg/mL PS20; 1 mM met; water; and pH 5.8.

I934. 190 mg/mL secu; 50 mM His; 200 mM treh; 100 mM gly; 0.2 mg/mL PS20; 1 mM met; water; and pH 5.8.

I935. 150 mg/mL secu; 25 mM Lact; 200 mM treh; 100 mM gly; 0.2 mg/mL PS20; 1 mM met; water; and pH 5.8.

I936. 190 mg/mL secu; 25 mM Lact; 200 mM treh; 100 mM gly; 0.2 mg/mL PS20; 1 mM met; water; and pH 5.8.

I937. 150 mg/mL secu; 10 mM Phos-Lact; 200 mM treh; 100 mM gly; 0.2 mg/mL PS20; 1 mM met; water; and pH 5.8.

I938. 190 mg/mL secu; 10 mM Phos-Lact; 200 mM treh; 100 mM gly; 0.2 mg/mL PS20; 1 mM met; water; and pH 5.8.

I939. 150 mg/mL secu; 15 mM Gluc; 200 mM treh; 100 mM gly; 0.2 mg/mL PS20; 1 mM met; water; and pH 5.8.

I940. 190 mg/mL secu; 15 mM Gluc; 200 mM treh; 100 mM gly; 0.2 mg/mL PS20; 1 mM met; water; and pH 5.8.

I941. 150 mg/mL secu; 0 mM buffer; 150 mM sorb; 50 mM arg; 0.5 mg/mL PS80; 50 mM NaCl; water; and pH 5.8.

I942. 190 mg/mL secu; 0 mM buffer; 150 mM sorb; 50 mM arg; 0.5 mg/mL PS80; 50 mM NaCl; water; and pH 5.8.

I943. 150 mg/mL secu; 50 mM His; 150 mM sorb; 50 mM arg; 0.5 mg/mL PS80; 50 mM NaCl; water; and pH 5.8.

I944. 190 mg/mL secu; 50 mM His; 150 mM sorb; 50 mM arg; 0.5 mg/mL PS80; 50 mM NaCl; water; and pH 5.8.

I945. 150 mg/mL secu; 25 mM Lact; 150 mM sorb; 50 mM arg; 0.5 mg/mL PS80; 50 mM NaCl; water; and pH 5.8.

I946. 190 mg/mL secu; 25 mM Lact; 150 mM sorb; 50 mM arg; 0.5 mg/mL PS80; 50 mM NaCl; water; and pH 5.8.

I947. 150 mg/mL secu; 10 mM Phos-Lact; 150 mM sorb; 50 mM arg; 0.5 mg/mL PS80; 50 mM NaCl; water; and pH 5.8.

I948. 190 mg/mL secu; 10 mM Phos-Lact; 150 mM sorb; 50 mM arg; 0.5 mg/mL PS80; 50 mM NaCl; water; and pH 5.8.

I949. 150 mg/mL secu; 15 mM Gluc; 150 mM sorb; 50 mM arg; 0.5 mg/mL PS80; 50 mM NaCl; water; and pH 5.8.

I950. 190 mg/mL secu; 15 mM Gluc; 150 mM sorb; 50 mM arg; 0.5 mg/mL PS80; 50 mM NaCl; water; and pH 5.8.

I951. 150 mg/mL secu; 0 mM buffer; 75 mM mann; 50 mM arg; 0.5 mg/mL PS80; 50 mM NaCl; water; and pH 5.8.

I952. 190 mg/mL secu; 0 mM buffer; 75 mM mann; 50 mM arg; 0.5 mg/mL PS80; 50 mM NaCl; water; and pH 5.8.

I953. 150 mg/mL secu; 50 mM His; 75 mM mann; 50 mM arg; 0.5 mg/mL PS80; 50 mM NaCl; water; and pH 5.8.

I954. 190 mg/mL secu; 50 mM His; 75 mM mann; 50 mM arg; 0.5 mg/mL PS80; 50 mM NaCl; water; and pH 5.8.

I955. 150 mg/mL secu; 25 mM Lact; 75 mM mann; 50 mM arg; 0.5 mg/mL PS80; 50 mM NaCl; water; and pH 5.8.

I956. 190 mg/mL secu; 25 mM Lact; 75 mM mann; 50 mM arg; 0.5 mg/mL PS80; 50 mM NaCl; water; and pH 5.8.

I957. 150 mg/mL secu; 10 mM Phos-Lact; 75 mM mann; 50 mM arg; 0.5 mg/mL PS80; 50 mM NaCl; water; and pH 5.8.

I958. 190 mg/mL secu; 10 mM Phos-Lact; 75 mM mann; 50 mM arg; 0.5 mg/mL PS80; 50 mM NaCl; water; and pH 5.8.

I959. 150 mg/mL secu; 15 mM Gluc; 75 mM mann; 50 mM arg; 0.5 mg/mL PS80; 50 mM NaCl; water; and pH 5.8.

I960. 190 mg/mL secu; 15 mM Gluc; 75 mM mann; 50 mM arg; 0.5 mg/mL PS80; 50 mM NaCl; water; and pH 5.8.

I961. 150 mg/mL secu; 0 mM buffer; 100 mM sucr; 50 mM arg; 0.5 mg/mL PS80; 50 mM NaCl; water; and pH 5.8.

(continued)

I962. 190 mg/mL secu; 0 mM buffer; 100 mM sucr; 50 mM arg; 0.5 mg/mL PS80; 50 mM NaCl; water; and pH 5.8.

I963. 150 mg/mL secu; 50 mM His; 100 mM sucr; 50 mM arg; 0.5 mg/mL PS80; 50 mM NaCl; water; and pH 5.8.

I964. 190 mg/mL secu; 50 mM His; 100 mM sucr; 50 mM arg; 0.5 mg/mL PS80; 50 mM NaCl; water; and pH 5.8.

I965. 150 mg/mL secu; 25 mM Lact; 100 mM sucr; 50 mM arg; 0.5 mg/mL PS80; 50 mM NaCl; water; and pH 5.8.

I966. 190 mg/mL secu; 25 mM Lact; 100 mM sucr; 50 mM arg; 0.5 mg/mL PS80; 50 mM NaCl; water; and pH 5.8.

I967. 150 mg/mL secu; 10 mM Phos-Lact; 100 mM sucr; 50 mM arg; 0.5 mg/mL PS80; 50 mM NaCl; water; and pH 5.8.

I968. 190 mg/mL secu; 10 mM Phos-Lact; 100 mM sucr; 50 mM arg; 0.5 mg/mL PS80; 50 mM NaCl; water; and pH 5.8.

I969. 150 mg/mL secu; 15 mM Gluc; 100 mM sucr; 50 mM arg; 0.5 mg/mL PS80; 50 mM NaCl; water; and pH 5.8.

I970. 190 mg/mL secu; 15 mM Gluc; 100 mM sucr; 50 mM arg; 0.5 mg/mL PS80; 50 mM NaCl; water; and pH 5.8.

I971. 150 mg/mL secu; 0 mM buffer; 200 mM treh; 50 mM arg; 0.5 mg/mL PS80; 50 mM NaCl; water; and pH 5.8.

I972. 190 mg/mL secu; 0 mM buffer; 200 mM treh; 50 mM arg; 0.5 mg/mL PS80; 50 mM NaCl; water; and pH 5.8.

I973. 150 mg/mL secu; 50 mM His; 200 mM treh; 50 mM arg; 0.5 mg/mL PS80; 50 mM NaCl; water; and pH 5.8.

I974. 190 mg/mL secu; 50 mM His; 200 mM treh; 50 mM arg; 0.5 mg/mL PS80; 50 mM NaCl; water; and pH 5.8.

I975. 150 mg/mL secu; 25 mM Lact; 200 mM treh; 50 mM arg; 0.5 mg/mL PS80; 50 mM NaCl; water; and pH 5.8.

I976. 190 mg/mL secu; 25 mM Lact; 200 mM treh; 50 mM arg; 0.5 mg/mL PS80; 50 mM NaCl; water; and pH 5.8.

I977. 150 mg/mL secu; 10 mM Phos-Lact; 200 mM treh; 50 mM arg; 0.5 mg/mL PS80; 50 mM NaCl; water; and pH 5.8.

I978. 190 mg/mL secu; 10 mM Phos-Lact; 200 mM treh; 50 mM arg; 0.5 mg/mL PS80; 50 mM NaCl; water; and pH 5.8.

I979. 150 mg/mL secu; 15 mM Gluc; 200 mM treh; 50 mM arg; 0.5 mg/mL PS80; 50 mM NaCl; water; and pH 5.8.

I980. 190 mg/mL secu; 15 mM Gluc; 200 mM treh; 50 mM arg; 0.5 mg/mL PS80; 50 mM NaCl; water; and pH 5.8.

I981. 150 mg/mL secu; 0 mM buffer; 150 mM sorb; 100 mM gly; 0.5 mg/mL PS80; 50 mM NaCl; water; and pH 5.8.

I982. 190 mg/mL secu; 0 mM buffer; 150 mM sorb; 100 mM gly; 0.5 mg/mL PS80; 50 mM NaCl; water; and pH 5.8.

I983. 150 mg/mL secu; 50 mM His; 150 mM sorb; 100 mM gly; 0.5 mg/mL PS80; 50 mM NaCl; water; and pH 5.8.

I984. 190 mg/mL secu; 50 mM His; 150 mM sorb; 100 mM gly; 0.5 mg/mL PS80; 50 mM NaCl; water; and pH 5.8.

I985. 150 mg/mL secu; 25 mM Lact; 150 mM sorb; 100 mM gly; 0.5 mg/mL PS80; 50 mM NaCl; water; and pH 5.8.

I986. 190 mg/mL secu; 25 mM Lact; 150 mM sorb; 100 mM gly; 0.5 mg/mL PS80; 50 mM NaCl; water; and pH 5.8.

I987. 150 mg/mL secu; 10 mM Phos-Lact; 150 mM sorb; 100 mM gly; 0.5 mg/mL PS80; 50 mM NaCl; water; and pH 5.8.

I988. 190 mg/mL secu; 10 mM Phos-Lact; 150 mM sorb; 100 mM gly; 0.5 mg/mL PS80; 50 mM NaCl; water; and pH 5.8.

I989. 150 mg/mL secu; 15 mM Gluc; 150 mM sorb; 100 mM gly; 0.5 mg/mL PS80; 50 mM NaCl; water; and pH 5.8.

I990. 190 mg/mL secu; 15 mM Gluc; 150 mM sorb; 100 mM gly; 0.5 mg/mL PS80; 50 mM NaCl; water; and pH 5.8.

I991. 150 mg/mL secu; 0 mM buffer; 75 mM mann; 100 mM gly; 0.5 mg/mL PS80; 50 mM NaCl; water; and pH 5.8.

I992. 190 mg/mL secu; 0 mM buffer; 75 mM mann; 100 mM gly; 0.5 mg/mL PS80; 50 mM NaCl; water; and pH 5.8.

I993. 150 mg/mL secu; 50 mM His; 75 mM mann; 100 mM gly; 0.5 mg/mL PS80; 50 mM NaCl; water; and pH 5.8.

I994. 190 mg/mL secu; 50 mM His; 75 mM mann; 100 mM gly; 0.5 mg/mL PS80; 50 mM NaCl; water; and pH 5.8.

I995. 150 mg/mL secu; 25 mM Lact; 75 mM mann; 100 mM gly; 0.5 mg/mL PS80; 50 mM NaCl; water; and pH 5.8.

(continued)

I996. 190 mg/mL secu; 25 mM Lact; 75 mM mann; 100 mM gly; 0.5 mg/mL PS80; 50 mM NaCl; water; and pH 5.8.

I997. 150 mg/mL secu; 10 mM Phos-Lact; 75 mM mann; 100 mM gly; 0.5 mg/mL PS80; 50 mM NaCl; water; and pH 5.8.

I998. 190 mg/mL secu; 10 mM Phos-Lact; 75 mM mann; 100 mM gly; 0.5 mg/mL PS80; 50 mM NaCl; water; and pH 5.8.

I999. 150 mg/mL secu; 15 mM Gluc; 75 mM mann; 100 mM gly; 0.5 mg/mL PS80; 50 mM NaCl; water; and pH 5.8.

I1000. 190 mg/mL secu; 15 mM Gluc; 75 mM mann; 100 mM gly; 0.5 mg/mL PS80; 50 mM NaCl; water; and pH 5.8.

I1001. 150 mg/mL secu; 0 mM buffer; 100 mM sucr; 100 mM gly; 0.5 mg/mL PS80; 50 mM NaCl; water; and pH 5.8.

I1002. 190 mg/mL secu; 0 mM buffer; 100 mM sucr; 100 mM gly; 0.5 mg/mL PS80; 50 mM NaCl; water; and pH 5.8.

I1003. 150 mg/mL secu; 50 mM His; 100 mM sucr; 100 mM gly; 0.5 mg/mL PS80; 50 mM NaCl; water; and pH 5.8.

I1004. 190 mg/mL secu; 50 mM His; 100 mM sucr; 100 mM gly; 0.5 mg/mL PS80; 50 mM NaCl; water; and pH 5.8.

I1005. 150 mg/mL secu; 25 mM Lact; 100 mM sucr; 100 mM gly; 0.5 mg/mL PS80; 50 mM NaCl; water; and pH 5.8.

I1006. 190 mg/mL secu; 25 mM Lact; 100 mM sucr; 100 mM gly; 0.5 mg/mL PS80; 50 mM NaCl; water; and pH 5.8.

I1007. 150 mg/mL secu; 10 mM Phos-Lact; 100 mM sucr; 100 mM gly; 0.5 mg/mL PS80; 50 mM NaCl; water; and pH 5.8.

I1008. 190 mg/mL secu; 10 mM Phos-Lact; 100 mM sucr; 100 mM gly; 0.5 mg/mL PS80; 50 mM NaCl; water; and pH 5.8.

I1009. 150 mg/mL secu; 15 mM Gluc; 100 mM sucr; 100 mM gly; 0.5 mg/mL PS80; 50 mM NaCl; water; and pH 5.8.

I1010. 190 mg/mL secu; 15 mM Gluc; 100 mM sucr; 100 mM gly; 0.5 mg/mL PS80; 50 mM NaCl; water; and pH 5.8.

I1011. 150 mg/mL secu; 0 mM buffer; 200 mM treh; 100 mM gly; 0.5 mg/mL PS80; 50 mM NaCl; water; and pH 5.8.

I1012. 190 mg/mL secu; 0 mM buffer; 200 mM treh; 100 mM gly; 0.5 mg/mL PS80; 50 mM NaCl; water; and pH 5.8.

I1013. 150 mg/mL secu; 50 mM His; 200 mM treh; 100 mM gly; 0.5 mg/mL PS80; 50 mM NaCl; water; and pH 5.8.

I1014. 190 mg/mL secu; 50 mM His; 200 mM treh; 100 mM gly; 0.5 mg/mL PS80; 50 mM NaCl; water; and pH 5.8.

I1015. 150 mg/mL secu; 25 mM Lact; 200 mM treh; 100 mM gly; 0.5 mg/mL PS80; 50 mM NaCl; water; and pH 5.8.

I1016. 190 mg/mL secu; 25 mM Lact; 200 mM treh; 100 mM gly; 0.5 mg/mL PS80; 50 mM NaCl; water; and pH 5.8.

I1017. 150 mg/mL secu; 10 mM Phos-Lact; 200 mM treh; 100 mM gly; 0.5 mg/mL PS80; 50 mM NaCl; water; and pH 5.8.

I1018. 190 mg/mL secu; 10 mM Phos-Lact; 200 mM treh; 100 mM gly; 0.5 mg/mL PS80; 50 mM NaCl; water; and pH 5.8.

I1019. 150 mg/mL secu; 15 mM Gluc; 200 mM treh; 100 mM gly; 0.5 mg/mL PS80; 50 mM NaCl; water; and pH 5.8.

I1020. 190 mg/mL secu; 15 mM Gluc; 200 mM treh; 100 mM gly; 0.5 mg/mL PS80; 50 mM NaCl; water; and pH 5.8.

I1021. 150 mg/mL secu; 0 mM buffer; 150 mM sorb; 50 mM arg; 0.2 mg/mL PS20; 50 mM NaCl; water; and pH 5.8.

I1022. 190 mg/mL secu; 0 mM buffer; 150 mM sorb; 50 mM arg; 0.2 mg/mL PS20; 50 mM NaCl; water; and pH 5.8.

I1023. 150 mg/mL secu; 50 mM His; 150 mM sorb; 50 mM arg; 0.2 mg/mL PS20; 50 mM NaCl; water; and pH 5.8.

I1024. 190 mg/mL secu; 50 mM His; 150 mM sorb; 50 mM arg; 0.2 mg/mL PS20; 50 mM NaCl; water; and pH 5.8.

I1025. 150 mg/mL secu; 25 mM Lact; 150 mM sorb; 50 mM arg; 0.2 mg/mL PS20; 50 mM NaCl; water; and pH 5.8.

(continued)

I1026. 190 mg/mL secu; 25 mM Lact; 150 mM sorb; 50 mM arg; 0.2 mg/mL PS20; 50 mM NaCl; water; and pH 5.8.
I1027. 150 mg/mL secu; 10 mM Phos-Lact; 150 mM sorb; 50 mM arg; 0.2 mg/mL PS20; 50 mM NaCl; water; and pH 5.8.
I1028. 190 mg/mL secu; 10 mM Phos-Lact; 150 mM sorb; 50 mM arg; 0.2 mg/mL PS20; 50 mM NaCl; water; and pH 5.8.
I1029. 150 mg/mL secu; 15 mM Gluc; 150 mM sorb; 50 mM arg; 0.2 mg/mL PS20; 50 mM NaCl; water; and pH 5.8.
I1030. 190 mg/mL secu; 15 mM Gluc; 150 mM sorb; 50 mM arg; 0.2 mg/mL PS20; 50 mM NaCl; water; and pH 5.8.
I1031. 150 mg/mL secu; 0 mM buffer; 75 mM mann; 50 mM arg; 0.2 mg/mL PS20; 50 mM NaCl; water; and pH 5.8.
I1032. 190 mg/mL secu; 0 mM buffer; 75 mM mann; 50 mM arg; 0.2 mg/mL PS20; 50 mM NaCl; water; and pH 5.8.
I1033. 150 mg/mL secu; 50 mM His; 75 mM mann; 50 mM arg; 0.2 mg/mL PS20; 50 mM NaCl; water; and pH 5.8.
I1034. 190 mg/mL secu; 50 mM His; 75 mM mann; 50 mM arg; 0.2 mg/mL PS20; 50 mM NaCl; water; and pH 5.8.
I1035. 150 mg/mL secu; 25 mM Lact; 75 mM mann; 50 mM arg; 0.2 mg/mL PS20; 50 mM NaCl; water; and pH 5.8.
I1036. 190 mg/mL secu; 25 mM Lact; 75 mM mann; 50 mM arg; 0.2 mg/mL PS20; 50 mM NaCl; water; and pH 5.8.
I1037. 150 mg/mL secu; 10 mM Phos-Lact; 75 mM mann; 50 mM arg; 0.2 mg/mL PS20; 50 mM NaCl; water; and pH 5.8.
I1038. 190 mg/mL secu; 10 mM Phos-Lact; 75 mM mann; 50 mM arg; 0.2 mg/mL PS20; 50 mM NaCl; water; and pH 5.8.
I1039. 150 mg/mL secu; 15 mM Gluc; 75 mM mann; 50 mM arg; 0.2 mg/mL PS20; 50 mM NaCl; water; and pH 5.8.
I1040. 190 mg/mL secu; 15 mM Gluc; 75 mM mann; 50 mM arg; 0.2 mg/mL PS20; 50 mM NaCl; water; and pH 5.8.
I1041. 150 mg/mL secu; 0 mM buffer; 100 mM sucr; 50 mM arg; 0.2 mg/mL PS20; 50 mM NaCl; water; and pH 5.8.
I1042. 190 mg/mL secu; 0 mM buffer; 100 mM sucr; 50 mM arg; 0.2 mg/mL PS20; 50 mM NaCl; water; and pH 5.8.
I1043. 150 mg/mL secu; 50 mM His; 100 mM sucr; 50 mM arg; 0.2 mg/mL PS20; 50 mM NaCl; water; and pH 5.8.
I1044. 190 mg/mL secu; 50 mM His; 100 mM sucr; 50 mM arg; 0.2 mg/mL PS20; 50 mM NaCl; water; and pH 5.8.
I1045. 150 mg/mL secu; 25 mM Lact; 100 mM sucr; 50 mM arg; 0.2 mg/mL PS20; 50 mM NaCl; water; and pH 5.8.
I1046. 190 mg/mL secu; 25 mM Lact; 100 mM sucr; 50 mM arg; 0.2 mg/mL PS20; 50 mM NaCl; water; and pH 5.8.
I1047. 150 mg/mL secu; 10 mM Phos-Lact; 100 mM sucr; 50 mM arg; 0.2 mg/mL PS20; 50 mM NaCl; water; and pH 5.8.
I1048. 190 mg/mL secu; 10 mM Phos-Lact; 100 mM sucr; 50 mM arg; 0.2 mg/mL PS20; 50 mM NaCl; water; and pH 5.8.
I1049. 150 mg/mL secu; 15 mM Gluc; 100 mM sucr; 50 mM arg; 0.2 mg/mL PS20; 50 mM NaCl; water; and pH 5.8.
I1050. 190 mg/mL secu; 15 mM Gluc; 100 mM sucr; 50 mM arg; 0.2 mg/mL PS20; 50 mM NaCl; water; and pH 5.8.
I1051. 150 mg/mL secu; 0 mM buffer; 200 mM treh; 50 mM arg; 0.2 mg/mL PS20; 50 mM NaCl; water; and pH 5.8.
I1052. 190 mg/mL secu; 0 mM buffer; 200 mM treh; 50 mM arg; 0.2 mg/mL PS20; 50 mM NaCl; water; and pH 5.8.
I1053. 150 mg/mL secu; 50 mM His; 200 mM treh; 50 mM arg; 0.2 mg/mL PS20; 50 mM NaCl; water; and pH 5.8.
I1054. 190 mg/mL secu; 50 mM His; 200 mM treh; 50 mM arg; 0.2 mg/mL PS20; 50 mM NaCl; water; and pH 5.8.
I1055. 150 mg/mL secu; 25 mM Lact; 200 mM treh; 50 mM arg; 0.2 mg/mL PS20; 50 mM NaCl; water; and pH 5.8.
I1056. 190 mg/mL secu; 25 mM Lact; 200 mM treh; 50 mM arg; 0.2 mg/mL PS20; 50 mM NaCl; water; and pH 5.8.
I1057. 150 mg/mL secu; 10 mM Phos-Lact; 200 mM treh; 50 mM arg; 0.2 mg/mL PS20; 50 mM NaCl; water; and pH 5.8.
I1058. 190 mg/mL secu; 10 mM Phos-Lact; 200 mM treh; 50 mM arg; 0.2 mg/mL PS20; 50 mM NaCl; water; and pH 5.8.
I1059. 150 mg/mL secu; 15 mM Gluc; 200 mM treh; 50 mM arg; 0.2 mg/mL PS20; 50 mM NaCl; water; and pH 5.8.

(continued)

I1060. 190 mg/mL secu; 15 mM Gluc; 200 mM treh; 50 mM arg; 0.2 mg/mL PS20; 50 mM NaCl; water; and pH 5.8.

I1061. 150 mg/mL secu; 0 mM buffer; 150 mM sorb; 100 mM gly; 0.2 mg/mL PS20; 50 mM NaCl; water; and pH 5.8.

I1062. 190 mg/mL secu; 0 mM buffer; 150 mM sorb; 100 mM gly; 0.2 mg/mL PS20; 50 mM NaCl; water; and pH 5.8.

I1063. 150 mg/mL secu; 50 mM His; 150 mM sorb; 100 mM gly; 0.2 mg/mL PS20; 50 mM NaCl; water; and pH 5.8.

I1064. 190 mg/mL secu; 50 mM His; 150 mM sorb; 100 mM gly; 0.2 mg/mL PS20; 50 mM NaCl; water; and pH 5.8.

I1065. 150 mg/mL secu; 25 mM Lact; 150 mM sorb; 100 mM gly; 0.2 mg/mL PS20; 50 mM NaCl; water; and pH 5.8.

I1066. 190 mg/mL secu; 25 mM Lact; 150 mM sorb; 100 mM gly; 0.2 mg/mL PS20; 50 mM NaCl; water; and pH 5.8.

I1067. 150 mg/mL secu; 10 mM Phos-Lact; 150 mM sorb; 100 mM gly; 0.2 mg/mL PS20; 50 mM NaCl; water; and pH 5.8.

I1068. 190 mg/mL secu; 10 mM Phos-Lact; 150 mM sorb; 100 mM gly; 0.2 mg/mL PS20; 50 mM NaCl; water; and pH 5.8.

I1069. 150 mg/mL secu; 15 mM Gluc; 150 mM sorb; 100 mM gly; 0.2 mg/mL PS20; 50 mM NaCl; water; and pH 5.8.

I1070. 190 mg/mL secu; 15 mM Gluc; 150 mM sorb; 100 mM gly; 0.2 mg/mL PS20; 50 mM NaCl; water; and pH 5.8.

I1071. 150 mg/mL secu; 0 mM buffer; 75 mM mann; 100 mM gly; 0.2 mg/mL PS20; 50 mM NaCl; water; and pH 5.8.

I1072. 190 mg/mL secu; 0 mM buffer; 75 mM mann; 100 mM gly; 0.2 mg/mL PS20; 50 mM NaCl; water; and pH 5.8.

I1073. 150 mg/mL secu; 50 mM His; 75 mM mann; 100 mM gly; 0.2 mg/mL PS20; 50 mM NaCl; water; and pH 5.8.

I1074. 190 mg/mL secu; 50 mM His; 75 mM mann; 100 mM gly; 0.2 mg/mL PS20; 50 mM NaCl; water; and pH 5.8.

I1075. 150 mg/mL secu; 25 mM Lact; 75 mM mann; 100 mM gly; 0.2 mg/mL PS20; 50 mM NaCl; water; and pH 5.8.

I1076. 190 mg/mL secu; 25 mM Lact; 75 mM mann; 100 mM gly; 0.2 mg/mL PS20; 50 mM NaCl; water; and pH 5.8.

I1077. 150 mg/mL secu; 10 mM Phos-Lact; 75 mM mann; 100 mM gly; 0.2 mg/mL PS20; 50 mM NaCl; water; and pH 5.8.

I1078. 190 mg/mL secu; 10 mM Phos-Lact; 75 mM mann; 100 mM gly; 0.2 mg/mL PS20; 50 mM NaCl; water; and pH 5.8.

I1079. 150 mg/mL secu; 15 mM Gluc; 75 mM mann; 100 mM gly; 0.2 mg/mL PS20; 50 mM NaCl; water; and pH 5.8.

I1080. 190 mg/mL secu; 15 mM Gluc; 75 mM mann; 100 mM gly; 0.2 mg/mL PS20; 50 mM NaCl; water; and pH 5.8.

I1081. 150 mg/mL secu; 0 mM buffer; 100 mM sucr; 100 mM gly; 0.2 mg/mL PS20; 50 mM NaCl; water; and pH 5.8.

I1082. 190 mg/mL secu; 0 mM buffer; 100 mM sucr; 100 mM gly; 0.2 mg/mL PS20; 50 mM NaCl; water; and pH 5.8.

I1083. 150 mg/mL secu; 50 mM His; 100 mM sucr; 100 mM gly; 0.2 mg/mL PS20; 50 mM NaCl; water; and pH 5.8.

I1084. 190 mg/mL secu; 50 mM His; 100 mM sucr; 100 mM gly; 0.2 mg/mL PS20; 50 mM NaCl; water; and pH 5.8.

I1085. 150 mg/mL secu; 25 mM Lact; 100 mM sucr; 100 mM gly; 0.2 mg/mL PS20; 50 mM NaCl; water; and pH 5.8.

I1086. 190 mg/mL secu; 25 mM Lact; 100 mM sucr; 100 mM gly; 0.2 mg/mL PS20; 50 mM NaCl; water; and pH 5.8.

I1087. 150 mg/mL secu; 10 mM Phos-Lact; 100 mM sucr; 100 mM gly; 0.2 mg/mL PS20; 50 mM NaCl; water; and pH 5.8.

I1088. 190 mg/mL secu; 10 mM Phos-Lact; 100 mM sucr; 100 mM gly; 0.2 mg/mL PS20; 50 mM NaCl; water; and pH 5.8.

I1089. 150 mg/mL secu; 15 mM Gluc; 100 mM sucr; 100 mM gly; 0.2 mg/mL PS20; 50 mM NaCl; water; and pH 5.8.

(continued)

I1090. 190 mg/mL secu; 15 mM Gluc; 100 mM sucr; 100 mM gly; 0.2 mg/mL PS20; 50 mM NaCl; water; and pH 5.8.

I1091. 150 mg/mL secu; 0 mM buffer; 200 mM treh; 100 mM gly; 0.2 mg/mL PS20; 50 mM NaCl; water; and pH 5.8.

I1092. 190 mg/mL secu; 0 mM buffer; 200 mM treh; 100 mM gly; 0.2 mg/mL PS20; 50 mM NaCl; water; and pH 5.8.

I1093. 150 mg/mL secu; 50 mM His; 200 mM treh; 100 mM gly; 0.2 mg/mL PS20; 50 mM NaCl; water; and pH 5.8.

I1094. 190 mg/mL secu; 50 mM His; 200 mM treh; 100 mM gly; 0.2 mg/mL PS20; 50 mM NaCl; water; and pH 5.8.

I1095. 150 mg/mL secu; 25 mM Lact; 200 mM treh; 100 mM gly; 0.2 mg/mL PS20; 50 mM NaCl; water; and pH 5.8.

I1096. 190 mg/mL secu; 25 mM Lact; 200 mM treh; 100 mM gly; 0.2 mg/mL PS20; 50 mM NaCl; water; and pH 5.8.

I1097. 150 mg/mL secu; 10 mM Phos-Lact; 200 mM treh; 100 mM gly; 0.2 mg/mL PS20; 50 mM NaCl; water; and pH 5.8.

I1098. 190 mg/mL secu; 10 mM Phos-Lact; 200 mM treh; 100 mM gly; 0.2 mg/mL PS20; 50 mM NaCl; water; and pH 5.8.

I1099. 150 mg/mL secu; 15 mM Gluc; 200 mM treh; 100 mM gly; 0.2 mg/mL PS20; 50 mM NaCl; water; and pH 5.8.

I1100. 190 mg/mL secu; 15 mM Gluc; 200 mM treh; 100 mM gly; 0.2 mg/mL PS20; 50 mM NaCl; water; and pH 5.8.

I1101. 150 mg/mL secu; 0 mM buffer; 150 mM sorb; 50 mM arg; 0.5 mg/mL PS80; 0.1 mM EDTA; water; and pH 5.8.

I1102. 190 mg/mL secu; 0 mM buffer; 150 mM sorb; 50 mM arg; 0.5 mg/mL PS80; 0.1 mM EDTA; water; and pH 5.8.

I1103. 150 mg/mL secu; 50 mM His; 150 mM sorb; 50 mM arg; 0.5 mg/mL PS80; 0.1 mM EDTA; water; and pH 5.8.

I1104. 190 mg/mL secu; 50 mM His; 150 mM sorb; 50 mM arg; 0.5 mg/mL PS80; 0.1 mM EDTA; water; and pH 5.8.

I1105. 150 mg/mL secu; 25 mM Lact; 150 mM sorb; 50 mM arg; 0.5 mg/mL PS80; 0.1 mM EDTA; water; and pH 5.8.

I1106. 190 mg/mL secu; 25 mM Lact; 150 mM sorb; 50 mM arg; 0.5 mg/mL PS80; 0.1 mM EDTA; water; and pH 5.8.

I1107. 150 mg/mL secu; 10 mM Phos-Lact; 150 mM sorb; 50 mM arg; 0.5 mg/mL PS80; 0.1 mM EDTA; water; and pH 5.8.

I1108. 190 mg/mL secu; 10 mM Phos-Lact; 150 mM sorb; 50 mM arg; 0.5 mg/mL PS80; 0.1 mM EDTA; water; and pH 5.8.

I1109. 150 mg/mL secu; 15 mM Gluc; 150 mM sorb; 50 mM arg; 0.5 mg/mL PS80; 0.1 mM EDTA; water; and pH 5.8.

I1110. 190 mg/mL secu; 15 mM Gluc; 150 mM sorb; 50 mM arg; 0.5 mg/mL PS80; 0.1 mM EDTA; water; and pH 5.8.

I1111. 150 mg/mL secu; 0 mM buffer; 75 mM mann; 50 mM arg; 0.5 mg/mL PS80; 0.1 mM EDTA; water; and pH 5.8.

I1112. 190 mg/mL secu; 0 mM buffer; 75 mM mann; 50 mM arg; 0.5 mg/mL PS80; 0.1 mM EDTA; water; and pH 5.8.

I1113. 150 mg/mL secu; 50 mM His; 75 mM mann; 50 mM arg; 0.5 mg/mL PS80; 0.1 mM EDTA; water; and pH 5.8.

I1114. 190 mg/mL secu; 50 mM His; 75 mM mann; 50 mM arg; 0.5 mg/mL PS80; 0.1 mM EDTA; water; and pH 5.8.

I1115. 150 mg/mL secu; 25 mM Lact; 75 mM mann; 50 mM arg; 0.5 mg/mL PS80; 0.1 mM EDTA; water; and pH 5.8.

I1116. 190 mg/mL secu; 25 mM Lact; 75 mM mann; 50 mM arg; 0.5 mg/mL PS80; 0.1 mM EDTA; water; and pH 5.8.

(continued)

I1117. 150 mg/mL secu; 10 mM Phos-Lact; 75 mM mann; 50 mM arg; 0.5 mg/mL PS80; 0.1 mM EDTA; water; and pH 5.8.

I1118. 190 mg/mL secu; 10 mM Phos-Lact; 75 mM mann; 50 mM arg; 0.5 mg/mL PS80; 0.1 mM EDTA; water; and pH 5.8.

I1119. 150 mg/mL secu; 15 mM Gluc; 75 mM mann; 50 mM arg; 0.5 mg/mL PS80; 0.1 mM EDTA; water; and pH 5.8.

I1120. 190 mg/mL secu; 15 mM Gluc; 75 mM mann; 50 mM arg; 0.5 mg/mL PS80; 0.1 mM EDTA; water; and pH 5.8.

I1121. 150 mg/mL secu; 0 mM buffer; 100 mM sucr; 50 mM arg; 0.5 mg/mL PS80; 0.1 mM EDTA; water; and pH 5.8.

I1122. 190 mg/mL secu; 0 mM buffer; 100 mM sucr; 50 mM arg; 0.5 mg/mL PS80; 0.1 mM EDTA; water; and pH 5.8.

I1123. 150 mg/mL secu; 50 mM His; 100 mM sucr; 50 mM arg; 0.5 mg/mL PS80; 0.1 mM EDTA; water; and pH 5.8.

I1124. 190 mg/mL secu; 50 mM His; 100 mM sucr; 50 mM arg; 0.5 mg/mL PS80; 0.1 mM EDTA; water; and pH 5.8.

I1125. 150 mg/mL secu; 25 mM Lact; 100 mM sucr; 50 mM arg; 0.5 mg/mL PS80; 0.1 mM EDTA; water; and pH 5.8.

I1126. 190 mg/mL secu; 25 mM Lact; 100 mM sucr; 50 mM arg; 0.5 mg/mL PS80; 0.1 mM EDTA; water; and pH 5.8.

I1127. 150 mg/mL secu; 10 mM Phos-Lact; 100 mM sucr; 50 mM arg; 0.5 mg/mL PS80; 0.1 mM EDTA; water; and pH 5.8.

I1128. 190 mg/mL secu; 10 mM Phos-Lact; 100 mM sucr; 50 mM arg; 0.5 mg/mL PS80; 0.1 mM EDTA; water; and pH 5.8.

I1129. 150 mg/mL secu; 15 mM Gluc; 100 mM sucr; 50 mM arg; 0.5 mg/mL PS80; 0.1 mM EDTA; water; and pH 5.8.

I1130. 190 mg/mL secu; 15 mM Gluc; 100 mM sucr; 50 mM arg; 0.5 mg/mL PS80; 0.1 mM EDTA; water; and pH 5.8.

I1131. 150 mg/mL secu; 0 mM buffer; 200 mM treh; 50 mM arg; 0.5 mg/mL PS80; 0.1 mM EDTA; water; and pH 5.8.

I1132. 190 mg/mL secu; 0 mM buffer; 200 mM treh; 50 mM arg; 0.5 mg/mL PS80; 0.1 mM EDTA; water; and pH 5.8.

I1133. 150 mg/mL secu; 50 mM His; 200 mM treh; 50 mM arg; 0.5 mg/mL PS80; 0.1 mM EDTA; water; and pH 5.8.

I1134. 190 mg/mL secu; 50 mM His; 200 mM treh; 50 mM arg; 0.5 mg/mL PS80; 0.1 mM EDTA; water; and pH 5.8.

I1135. 150 mg/mL secu; 25 mM Lact; 200 mM treh; 50 mM arg; 0.5 mg/mL PS80; 0.1 mM EDTA; water; and pH 5.8.

I1136. 190 mg/mL secu; 25 mM Lact; 200 mM treh; 50 mM arg; 0.5 mg/mL PS80; 0.1 mM EDTA; water; and pH 5.8.

I1137. 150 mg/mL secu; 10 mM Phos-Lact; 200 mM treh; 50 mM arg; 0.5 mg/mL PS80; 0.1 mM EDTA; water; and pH 5.8.

I1138. 190 mg/mL secu; 10 mM Phos-Lact; 200 mM treh; 50 mM arg; 0.5 mg/mL PS80; 0.1 mM EDTA; water; and pH 5.8.

I1139. 150 mg/mL secu; 15 mM Gluc; 200 mM treh; 50 mM arg; 0.5 mg/mL PS80; 0.1 mM EDTA; water; and pH 5.8.

I1140. 190 mg/mL secu; 15 mM Gluc; 200 mM treh; 50 mM arg; 0.5 mg/mL PS80; 0.1 mM EDTA; water; and pH 5.8.

I1141. 150 mg/mL secu; 0 mM buffer; 150 mM sorb; 100 mM gly; 0.5 mg/mL PS80; 0.1 mM EDTA; water; and pH 5.8.

I1142. 190 mg/mL secu; 0 mM buffer; 150 mM sorb; 100 mM gly; 0.5 mg/mL PS80; 0.1 mM EDTA; water; and pH 5.8.

(continued)

I1143. 150 mg/mL secu; 50 mM His; 150 mM sorb; 100 mM gly; 0.5 mg/mL PS80; 0.1 mM EDTA; water; and pH 5.8.

I1144. 190 mg/mL secu; 50 mM His; 150 mM sorb; 100 mM gly; 0.5 mg/mL PS80; 0.1 mM EDTA; water; and pH 5.8.

I1145. 150 mg/mL secu; 25 mM Lact; 150 mM sorb; 100 mM gly; 0.5 mg/mL PS80; 0.1 mM EDTA; water; and pH 5.8.

I1146. 190 mg/mL secu; 25 mM Lact; 150 mM sorb; 100 mM gly; 0.5 mg/mL PS80; 0.1 mM EDTA; water; and pH 5.8.

I1147. 150 mg/mL secu; 10 mM Phos-Lact; 150 mM sorb; 100 mM gly; 0.5 mg/mL PS80; 0.1 mM EDTA; water; and pH 5.8.

I1148. 190 mg/mL secu; 10 mM Phos-Lact; 150 mM sorb; 100 mM gly; 0.5 mg/mL PS80; 0.1 mM EDTA; water; and pH 5.8.

I1149. 150 mg/mL secu; 15 mM Gluc; 150 mM sorb; 100 mM gly; 0.5 mg/mL PS80; 0.1 mM EDTA; water; and pH 5.8.

I1150. 190 mg/mL secu; 15 mM Gluc; 150 mM sorb; 100 mM gly; 0.5 mg/mL PS80; 0.1 mM EDTA; water; and pH 5.8.

I1151. 150 mg/mL secu; 0 mM buffer; 75 mM mann; 100 mM gly; 0.5 mg/mL PS80; 0.1 mM EDTA; water; and pH 5.8.

I1152. 190 mg/mL secu; 0 mM buffer; 75 mM mann; 100 mM gly; 0.5 mg/mL PS80; 0.1 mM EDTA; water; and pH 5.8.

I1153. 150 mg/mL secu; 50 mM His; 75 mM mann; 100 mM gly; 0.5 mg/mL PS80; 0.1 mM EDTA; water; and pH 5.8.

I1154. 190 mg/mL secu; 50 mM His; 75 mM mann; 100 mM gly; 0.5 mg/mL PS80; 0.1 mM EDTA; water; and pH 5.8.

I1155. 150 mg/mL secu; 25 mM Lact; 75 mM mann; 100 mM gly; 0.5 mg/mL PS80; 0.1 mM EDTA; water; and pH 5.8.

I1156. 190 mg/mL secu; 25 mM Lact; 75 mM mann; 100 mM gly; 0.5 mg/mL PS80; 0.1 mM EDTA; water; and pH 5.8.

I1157. 150 mg/mL secu; 10 mM Phos-Lact; 75 mM mann; 100 mM gly; 0.5 mg/mL PS80; 0.1 mM EDTA; water; and pH 5.8.

I1158. 190 mg/mL secu; 10 mM Phos-Lact; 75 mM mann; 100 mM gly; 0.5 mg/mL PS80; 0.1 mM EDTA; water; and pH 5.8.

I1159. 150 mg/mL secu; 15 mM Gluc; 75 mM mann; 100 mM gly; 0.5 mg/mL PS80; 0.1 mM EDTA; water; and pH 5.8.

I1160. 190 mg/mL secu; 15 mM Gluc; 75 mM mann; 100 mM gly; 0.5 mg/mL PS80; 0.1 mM EDTA; water; and pH 5.8.

I1161. 150 mg/mL secu; 0 mM buffer; 100 mM sucr; 100 mM gly; 0.5 mg/mL PS80; 0.1 mM EDTA; water; and pH 5.8.

I1162. 190 mg/mL secu; 0 mM buffer; 100 mM sucr; 100 mM gly; 0.5 mg/mL PS80; 0.1 mM EDTA; water; and pH 5.8.

I1163. 150 mg/mL secu; 50 mM His; 100 mM sucr; 100 mM gly; 0.5 mg/mL PS80; 0.1 mM EDTA; water; and pH 5.8.

I1164. 190 mg/mL secu; 50 mM His; 100 mM sucr; 100 mM gly; 0.5 mg/mL PS80; 0.1 mM EDTA; water; and pH 5.8.

I1165. 150 mg/mL secu; 25 mM Lact; 100 mM sucr; 100 mM gly; 0.5 mg/mL PS80; 0.1 mM EDTA; water; and pH 5.8.

I1166. 190 mg/mL secu; 25 mM Lact; 100 mM sucr; 100 mM gly; 0.5 mg/mL PS80; 0.1 mM EDTA; water; and pH 5.8.

I1167. 150 mg/mL secu; 10 mM Phos-Lact; 100 mM sucr; 100 mM gly; 0.5 mg/mL PS80; 0.1 mM EDTA; water; and pH 5.8.

I1168. 190 mg/mL secu; 10 mM Phos-Lact; 100 mM sucr; 100 mM gly; 0.5 mg/mL PS80; 0.1 mM EDTA; water; and pH 5.8.

(continued)

I1169. 150 mg/mL secu; 15 mM Gluc; 100 mM sucr; 100 mM gly; 0.5 mg/mL PS80; 0.1 mM EDTA; water; and pH 5.8.

I1170. 190 mg/mL secu; 15 mM Gluc; 100 mM sucr; 100 mM gly; 0.5 mg/mL PS80; 0.1 mM EDTA; water; and pH 5.8.

I11171. 150 mg/mL secu; 0 mM buffer; 200 mM treh; 100 mM gly; 0.5 mg/mL PS80; 0.1 mM EDTA; water; and pH 5.8.

I1172. 190 mg/mL secu; 0 mM buffer; 200 mM treh; 100 mM gly; 0.5 mg/mL PS80; 0.1 mM EDTA; water; and pH 5.8.

I1173. 150 mg/mL secu; 50 mM His; 200 mM treh; 100 mM gly; 0.5 mg/mL PS80; 0.1 mM EDTA; water; and pH 5.8.

I1174. 190 mg/mL secu; 50 mM His; 200 mM treh; 100 mM gly; 0.5 mg/mL PS80; 0.1 mM EDTA; water; and pH 5.8.

I1175. 150 mg/mL secu; 25 mM Lact; 200 mM treh; 100 mM gly; 0.5 mg/mL PS80; 0.1 mM EDTA; water; and pH 5.8.

I1176. 190 mg/mL secu; 25 mM Lact; 200 mM treh; 100 mM gly; 0.5 mg/mL PS80; 0.1 mM EDTA; water; and pH 5.8.

I1177. 150 mg/mL secu; 10 mM Phos-Lact; 200 mM treh; 100 mM gly; 0.5 mg/mL PS80; 0.1 mM EDTA; water; and pH 5.8.

I1178. 190 mg/mL secu; 10 mM Phos-Lact; 200 mM treh; 100 mM gly; 0.5 mg/mL PS80; 0.1 mM EDTA; water; and pH 5.8.

I1179. 150 mg/mL secu; 15 mM Gluc; 200 mM treh; 100 mM gly; 0.5 mg/mL PS80; 0.1 mM EDTA; water; and pH 5.8.

I1180. 190 mg/mL secu; 15 mM Gluc; 200 mM treh; 100 mM gly; 0.5 mg/mL PS80; 0.1 mM EDTA; water; and pH 5.8.

I1181. 150 mg/mL secu; 0 mM buffer; 150 mM sorb; 50 mM arg; 0.2 mg/mL PS20; 0.1 mM EDTA; water; and pH 5.8.

I1182. 190 mg/mL secu; 0 mM buffer; 150 mM sorb; 50 mM arg; 0.2 mg/mL PS20; 0.1 mM EDTA; water; and pH 5.8.

I1183. 150 mg/mL secu; 50 mM His; 150 mM sorb; 50 mM arg; 0.2 mg/mL PS20; 0.1 mM EDTA; water; and pH 5.8.

I1184. 190 mg/mL secu; 50 mM His; 150 mM sorb; 50 mM arg; 0.2 mg/mL PS20; 0.1 mM EDTA; water; and pH 5.8.

I1185. 150 mg/mL secu; 25 mM Lact; 150 mM sorb; 50 mM arg; 0.2 mg/mL PS20; 0.1 mM EDTA; water; and pH 5.8.

I1186. 190 mg/mL secu; 25 mM Lact; 150 mM sorb; 50 mM arg; 0.2 mg/mL PS20; 0.1 mM EDTA; water; and pH 5.8.

I1187. 150 mg/mL secu; 10 mM Phos-Lact; 150 mM sorb; 50 mM arg; 0.2 mg/mL PS20; 0.1 mM EDTA; water; and pH 5.8.

I1188. 190 mg/mL secu; 10 mM Phos-Lact; 150 mM sorb; 50 mM arg; 0.2 mg/mL PS20; 0.1 mM EDTA; water; and pH 5.8.

I1189. 150 mg/mL secu; 15 mM Gluc; 150 mM sorb; 50 mM arg; 0.2 mg/mL PS20; 0.1 mM EDTA; water; and pH 5.8.

I1190. 190 mg/mL secu; 15 mM Gluc; 150 mM sorb; 50 mM arg; 0.2 mg/mL PS20; 0.1 mM EDTA; water; and pH 5.8.

I1191. 150 mg/mL secu; 0 mM buffer; 75 mM mann; 50 mM arg; 0.2 mg/mL PS20; 0.1 mM EDTA; water; and pH 5.8.

I1192. 190 mg/mL secu; 0 mM buffer; 75 mM mann; 50 mM arg; 0.2 mg/mL PS20; 0.1 mM EDTA; water; and pH 5.8.

I1193. 150 mg/mL secu; 50 mM His; 75 mM mann; 50 mM arg; 0.2 mg/mL PS20; 0.1 mM EDTA; water; and pH 5.8.

I1194. 190 mg/mL secu; 50 mM His; 75 mM mann; 50 mM arg; 0.2 mg/mL PS20; 0.1 mM EDTA; water; and pH 5.8.

(continued)

I1195. 150 mg/mL secu; 25 mM Lact; 75 mM mann; 50 mM arg; 0.2 mg/mL PS20; 0.1 mM EDTA; water; and pH 5.8.

I1196. 190 mg/mL secu; 25 mM Lact; 75 mM mann; 50 mM arg; 0.2 mg/mL PS20; 0.1 mM EDTA; water; and pH 5.8.

I1197. 150 mg/mL secu; 10 mM Phos-Lact; 75 mM mann; 50 mM arg; 0.2 mg/mL PS20; 0.1 mM EDTA; water; and pH 5.8.

I1198. 190 mg/mL secu; 10 mM Phos-Lact; 75 mM mann; 50 mM arg; 0.2 mg/mL PS20; 0.1 mM EDTA; water; and pH 5.8.

I1199. 150 mg/mL secu; 15 mM Gluc; 75 mM mann; 50 mM arg; 0.2 mg/mL PS20; 0.1 mM EDTA; water; and pH 5.8.

I1200. 190 mg/mL secu; 15 mM Gluc; 75 mM mann; 50 mM arg; 0.2 mg/mL PS20; 0.1 mM EDTA; water; and pH 5.8.

I1201. 150 mg/mL secu; 0 mM buffer; 100 mM sucr; 50 mM arg; 0.2 mg/mL PS20; 0.1 mM EDTA; water; and pH 5.8.

I1202. 190 mg/mL secu; 0 mM buffer; 100 mM sucr; 50 mM arg; 0.2 mg/mL PS20; 0.1 mM EDTA; water; and pH 5.8.

I1203. 150 mg/mL secu; 50 mM His; 100 mM sucr; 50 mM arg; 0.2 mg/mL PS20; 0.1 mM EDTA; water; and pH 5.8.

I1204. 190 mg/mL secu; 50 mM His; 100 mM sucr; 50 mM arg; 0.2 mg/mL PS20; 0.1 mM EDTA; water; and pH 5.8.

I1205. 150 mg/mL secu; 25 mM Lact; 100 mM sucr; 50 mM arg; 0.2 mg/mL PS20; 0.1 mM EDTA; water; and pH 5.8.

I1206. 190 mg/mL secu; 25 mM Lact; 100 mM sucr; 50 mM arg; 0.2 mg/mL PS20; 0.1 mM EDTA; water; and pH 5.8.

I1207. 150 mg/mL secu; 10 mM Phos-Lact; 100 mM sucr; 50 mM arg; 0.2 mg/mL PS20; 0.1 mM EDTA; water; and pH 5.8.

I1208. 190 mg/mL secu; 10 mM Phos-Lact; 100 mM sucr; 50 mM arg; 0.2 mg/mL PS20; 0.1 mM EDTA; water; and pH 5.8.

I1209. 150 mg/mL secu; 15 mM Gluc; 100 mM sucr; 50 mM arg; 0.2 mg/mL PS20; 0.1 mM EDTA; water; and pH 5.8.

I1210. 190 mg/mL secu; 15 mM Gluc; 100 mM sucr; 50 mM arg; 0.2 mg/mL PS20; 0.1 mM EDTA; water; and pH 5.8.

I1211. 150 mg/mL secu; 0 mM buffer; 200 mM treh; 50 mM arg; 0.2 mg/mL PS20; 0.1 mM EDTA; water; and pH 5.8.

I1212. 190 mg/mL secu; 0 mM buffer; 200 mM treh; 50 mM arg; 0.2 mg/mL PS20; 0.1 mM EDTA; water; and pH 5.8.

I1213. 150 mg/mL secu; 50 mM His; 200 mM treh; 50 mM arg; 0.2 mg/mL PS20; 0.1 mM EDTA; water; and pH 5.8.

I1214. 190 mg/mL secu; 50 mM His; 200 mM treh; 50 mM arg; 0.2 mg/mL PS20; 0.1 mM EDTA; water; and pH 5.8.

I1215. 150 mg/mL secu; 25 mM Lact; 200 mM treh; 50 mM arg; 0.2 mg/mL PS20; 0.1 mM EDTA; water; and pH 5.8.

I1216. 190 mg/mL secu; 25 mM Lact; 200 mM treh; 50 mM arg; 0.2 mg/mL PS20; 0.1 mM EDTA; water; and pH 5.8.

I1217. 150 mg/mL secu; 10 mM Phos-Lact; 200 mM treh; 50 mM arg; 0.2 mg/mL PS20; 0.1 mM EDTA; water; and pH 5.8.

I1218. 190 mg/mL secu; 10 mM Phos-Lact; 200 mM treh; 50 mM arg; 0.2 mg/mL PS20; 0.1 mM EDTA; water; and pH 5.8.

I1219. 150 mg/mL secu; 15 mM Gluc; 200 mM treh; 50 mM arg; 0.2 mg/mL PS20; 0.1 mM EDTA; water; and pH 5.8.

I1220. 190 mg/mL secu; 15 mM Gluc; 200 mM treh; 50 mM arg; 0.2 mg/mL PS20; 0.1 mM EDTA; water; and pH 5.8.

(continued)

I1221. 150 mg/mL secu; 0 mM buffer; 150 mM sorb; 100 mM gly; 0.2 mg/mL PS20; 0.1 mM EDTA; water; and pH 5.8.

I1222. 190 mg/mL secu; 0 mM buffer; 150 mM sorb; 100 mM gly; 0.2 mg/mL PS20; 0.1 mM EDTA; water; and pH 5.8.

I1223. 150 mg/mL secu; 50 mM His; 150 mM sorb; 100 mM gly; 0.2 mg/mL PS20; 0.1 mM EDTA; water; and pH 5.8.

I1224. 190 mg/mL secu; 50 mM His; 150 mM sorb; 100 mM gly; 0.2 mg/mL PS20; 0.1 mM EDTA; water; and pH 5.8.

I1225. 150 mg/mL secu; 25 mM Lact; 150 mM sorb; 100 mM gly; 0.2 mg/mL PS20; 0.1 mM EDTA; water; and pH 5.8.

I1226. 190 mg/mL secu; 25 mM Lact; 150 mM sorb; 100 mM gly; 0.2 mg/mL PS20; 0.1 mM EDTA; water; and pH 5.8.

I1227. 150 mg/mL secu; 10 mM Phos-Lact; 150 mM sorb; 100 mM gly; 0.2 mg/mL PS20; 0.1 mM EDTA; water; and pH 5.8.

I1228. 190 mg/mL secu; 10 mM Phos-Lact; 150 mM sorb; 100 mM gly; 0.2 mg/mL PS20; 0.1 mM EDTA; water; and pH 5.8.

I1229. 150 mg/mL secu; 15 mM Gluc; 150 mM sorb; 100 mM gly; 0.2 mg/mL PS20; 0.1 mM EDTA; water; and pH 5.8.

I1230. 190 mg/mL secu; 15 mM Gluc; 150 mM sorb; 100 mM gly; 0.2 mg/mL PS20; 0.1 mM EDTA; water; and pH 5.8.

I1231. 150 mg/mL secu; 0 mM buffer; 75 mM mann; 100 mM gly; 0.2 mg/mL PS20; 0.1 mM EDTA; water; and pH 5.8.

I1232. 190 mg/mL secu; 0 mM buffer; 75 mM mann; 100 mM gly; 0.2 mg/mL PS20; 0.1 mM EDTA; water; and pH 5.8.

I1233. 150 mg/mL secu; 50 mM His; 75 mM mann; 100 mM gly; 0.2 mg/mL PS20; 0.1 mM EDTA; water; and pH 5.8.

I1234. 190 mg/mL secu; 50 mM His; 75 mM mann; 100 mM gly; 0.2 mg/mL PS20; 0.1 mM EDTA; water; and pH 5.8.

I1235. 150 mg/mL secu; 25 mM Lact; 75 mM mann; 100 mM gly; 0.2 mg/mL PS20; 0.1 mM EDTA; water; and pH 5.8.

I1236. 190 mg/mL secu; 25 mM Lact; 75 mM mann; 100 mM gly; 0.2 mg/mL PS20; 0.1 mM EDTA; water; and pH 5.8.

I1237. 150 mg/mL secu; 10 mM Phos-Lact; 75 mM mann; 100 mM gly; 0.2 mg/mL PS20; 0.1 mM EDTA; water; and pH 5.8.

I1238. 190 mg/mL secu; 10 mM Phos-Lact; 75 mM mann; 100 mM gly; 0.2 mg/mL PS20; 0.1 mM EDTA; water; and pH 5.8.

I1239. 150 mg/mL secu; 15 mM Gluc; 75 mM mann; 100 mM gly; 0.2 mg/mL PS20; 0.1 mM EDTA; water; and pH 5.8.

I1240. 190 mg/mL secu; 15 mM Gluc; 75 mM mann; 100 mM gly; 0.2 mg/mL PS20; 0.1 mM EDTA; water; and pH 5.8.

I1241. 150 mg/mL secu; 0 mM buffer; 100 mM sucr; 100 mM gly; 0.2 mg/mL PS20; 0.1 mM EDTA; water; and pH 5.8.

I1242. 190 mg/mL secu; 0 mM buffer; 100 mM sucr; 100 mM gly; 0.2 mg/mL PS20; 0.1 mM EDTA; water; and pH 5.8.

I1243. 150 mg/mL secu; 50 mM His; 100 mM sucr; 100 mM gly; 0.2 mg/mL PS20; 0.1 mM EDTA; water; and pH 5.8.

I1244. 190 mg/mL secu; 50 mM His; 100 mM sucr; 100 mM gly; 0.2 mg/mL PS20; 0.1 mM EDTA; water; and pH 5.8.

I1245. 150 mg/mL secu; 25 mM Lact; 100 mM sucr; 100 mM gly; 0.2 mg/mL PS20; 0.1 mM EDTA; water; and pH 5.8.

I1246. 190 mg/mL secu; 25 mM Lact; 100 mM sucr; 100 mM gly; 0.2 mg/mL PS20; 0.1 mM EDTA; water; and pH 5.8.

(continued)

I1247. 150 mg/mL secu; 10 mM Phos-Lact; 100 mM sucr; 100 mM gly; 0.2 mg/mL PS20; 0.1 mM EDTA; water; and pH 5.8.

I1248. 190 mg/mL secu; 10 mM Phos-Lact; 100 mM sucr; 100 mM gly; 0.2 mg/mL PS20; 0.1 mM EDTA; water; and pH 5.8.

I1249. 150 mg/mL secu; 15 mM Gluc; 100 mM sucr; 100 mM gly; 0.2 mg/mL PS20; 0.1 mM EDTA; water; and pH 5.8.

I1250. 190 mg/mL secu; 15 mM Gluc; 100 mM sucr; 100 mM gly; 0.2 mg/mL PS20; 0.1 mM EDTA; water; and pH 5.8.

I1251. 150 mg/mL secu; 0 mM buffer; 200 mM treh; 100 mM gly; 0.2 mg/mL PS20; 0.1 mM EDTA; water; and pH 5.8.

I1252. 190 mg/mL secu; 0 mM buffer; 200 mM treh; 100 mM gly; 0.2 mg/mL PS20; 0.1 mM EDTA; water; and pH 5.8.

I1253. 150 mg/mL secu; 50 mM His; 200 mM treh; 100 mM gly; 0.2 mg/mL PS20; 0.1 mM EDTA; water; and pH 5.8.

I1254. 190 mg/mL secu; 50 mM His; 200 mM treh; 100 mM gly; 0.2 mg/mL PS20; 0.1 mM EDTA; water; and pH 5.8.

I1255. 150 mg/mL secu; 25 mM Lact; 200 mM treh; 100 mM gly; 0.2 mg/mL PS20; 0.1 mM EDTA; water; and pH 5.8.

I1256. 190 mg/mL secu; 25 mM Lact; 200 mM treh; 100 mM gly; 0.2 mg/mL PS20; 0.1 mM EDTA; water; and pH 5.8.

I1257. 150 mg/mL secu; 10 mM Phos-Lact; 200 mM treh; 100 mM gly; 0.2 mg/mL PS20; 0.1 mM EDTA; water; and pH 5.8.

I1258. 190 mg/mL secu; 10 mM Phos-Lact; 200 mM treh; 100 mM gly; 0.2 mg/mL PS20; 0.1 mM EDTA; water; and pH 5.8.

I1259. 150 mg/mL secu; 15 mM Gluc; 200 mM treh; 100 mM gly; 0.2 mg/mL PS20; 0.1 mM EDTA; water; and pH 5.8.

I1260. 190 mg/mL secu; 15 mM Gluc; 200 mM treh; 100 mM gly; 0.2 mg/mL PS20; 0.1 mM EDTA; water; and pH 5.8.

I1261. 150 mg/mL secu; 0 mM buffer; 150 mM sorb; 0.5 mg/mL PS80; 1 mM met; 50 mM NaCl; water; and pH 5.8.

I1262. 190 mg/mL secu; 0 mM buffer; 150 mM sorb; 0.5 mg/mL PS80; 1 mM met; 50 mM NaCl; water; and pH 5.8.

I1263. 150 mg/mL secu; 50 mM His; 150 mM sorb; 0.5 mg/mL PS80; 1 mM met; 50 mM NaCl; water; and pH 5.8.

I1264. 190 mg/mL secu; 50 mM His; 150 mM sorb; 0.5 mg/mL PS80; 1 mM met; 50 mM NaCl; water; and pH 5.8.

I1265. 150 mg/mL secu; 25 mM Lact; 150 mM sorb; 0.5 mg/mL PS80; 1 mM met; 50 mM NaCl; water; and pH 5.8.

I1266. 190 mg/mL secu; 25 mM Lact; 150 mM sorb; 0.5 mg/mL PS80; 1 mM met; 50 mM NaCl; water; and pH 5.8.

I1267. 150 mg/mL secu; 10 mM Phos-Lact; 150 mM sorb; 0.5 mg/mL PS80; 1 mM met; 50 mM NaCl; water; and pH 5.8.

I1268. 190 mg/mL secu; 10 mM Phos-Lact; 150 mM sorb; 0.5 mg/mL PS80; 1 mM met; 50 mM NaCl; water; and pH 5.8.

I1269. 150 mg/mL secu; 15 mM Gluc; 150 mM sorb; 0.5 mg/mL PS80; 1 mM met; 50 mM NaCl; water; and pH 5.8.

I1270. 190 mg/mL secu; 15 mM Gluc; 150 mM sorb; 0.5 mg/mL PS80; 1 mM met; 50 mM NaCl; water; and pH 5.8.

I1271. 150 mg/mL secu; 0 mM buffer; 75 mM mann; 0.5 mg/mL PS80; 1 mM met; 50 mM NaCl; water; and pH 5.8.

I1272. 190 mg/mL secu; 0 mM buffer; 75 mM mann; 0.5 mg/mL PS80; 1 mM met; 50 mM NaCl; water; and pH 5.8.

I1273. 150 mg/mL secu; 50 mM His; 75 mM mann; 0.5 mg/mL PS80; 1 mM met; 50 mM NaCl; water; and pH 5.8.

I1274. 190 mg/mL secu; 50 mM His; 75 mM mann; 0.5 mg/mL PS80; 1 mM met; 50 mM NaCl; water; and pH 5.8.

I1275. 150 mg/mL secu; 25 mM Lact; 75 mM mann; 0.5 mg/mL PS80; 1 mM met; 50 mM NaCl; water; and pH 5.8.

I1276. 190 mg/mL secu; 25 mM Lact; 75 mM mann; 0.5 mg/mL PS80; 1 mM met; 50 mM NaCl; water; and pH 5.8.

I1277. 150 mg/mL secu; 10 mM Phos-Lact; 75 mM mann; 0.5 mg/mL PS80; 1 mM met; 50 mM NaCl; water; and pH 5.8.

I1278. 190 mg/mL secu; 10 mM Phos-Lact; 75 mM mann; 0.5 mg/mL PS80; 1 mM met; 50 mM NaCl; water; and pH 5.8.

I1279. 150 mg/mL secu; 15 mM Gluc; 75 mM mann; 0.5 mg/mL PS80; 1 mM met; 50 mM NaCl; water; and pH 5.8.

I1280. 190 mg/mL secu; 15 mM Gluc; 75 mM mann; 0.5 mg/mL PS80; 1 mM met; 50 mM NaCl; water; and pH 5.8.

(continued)

I1281. 150 mg/mL secu; 0 mM buffer; 100 mM sucr; 0.5 mg/mL PS80; 1 mM met; 50 mM NaCl; water; and pH 5.8.
I1282. 190 mg/mL secu; 0 mM buffer; 100 mM sucr; 0.5 mg/mL PS80; 1 mM met; 50 mM NaCl; water; and pH 5.8.
I1283. 150 mg/mL secu; 50 mM His; 100 mM sucr; 0.5 mg/mL PS80; 1 mM met; 50 mM NaCl; water; and pH 5.8.
I1284. 190 mg/mL secu; 50 mM His; 100 mM sucr; 0.5 mg/mL PS80; 1 mM met; 50 mM NaCl; water; and pH 5.8.
I1285. 150 mg/mL secu; 25 mM Lact; 100 mM sucr; 0.5 mg/mL PS80; 1 mM met; 50 mM NaCl; water; and pH 5.8.
I1286. 190 mg/mL secu; 25 mM Lact; 100 mM sucr; 0.5 mg/mL PS80; 1 mM met; 50 mM NaCl; water; and pH 5.8.
I1287. 150 mg/mL secu; 10 mM Phos-Lact; 100 mM sucr; 0.5 mg/mL PS80; 1 mM met; 50 mM NaCl; water; and pH 5.8.
I1288. 190 mg/mL secu; 10 mM Phos-Lact; 100 mM sucr; 0.5 mg/mL PS80; 1 mM met; 50 mM NaCl; water; and pH 5.8.
I1289. 150 mg/mL secu; 15 mM Gluc; 100 mM sucr; 0.5 mg/mL PS80; 1 mM met; 50 mM NaCl; water; and pH 5.8.
I1290. 190 mg/mL secu; 15 mM Gluc; 100 mM sucr; 0.5 mg/mL PS80; 1 mM met; 50 mM NaCl; water; and pH 5.8.
I1291. 150 mg/mL secu; 0 mM buffer; 200 mM treh; 0.5 mg/mL PS80; 1 mM met; 50 mM NaCl; water; and pH 5.8.
I1292. 190 mg/mL secu; 0 mM buffer; 200 mM treh; 0.5 mg/mL PS80; 1 mM met; 50 mM NaCl; water; and pH 5.8.
I1293. 150 mg/mL secu; 50 mM His; 200 mM treh; 0.5 mg/mL PS80; 1 mM met; 50 mM NaCl; water; and pH 5.8.
I1294. 190 mg/mL secu; 50 mM His; 200 mM treh; 0.5 mg/mL PS80; 1 mM met; 50 mM NaCl; water; and pH 5.8.
I1295. 150 mg/mL secu; 25 mM Lact; 200 mM treh; 0.5 mg/mL PS80; 1 mM met; 50 mM NaCl; water; and pH 5.8.
I1296. 190 mg/mL secu; 25 mM Lact; 200 mM treh; 0.5 mg/mL PS80; 1 mM met; 50 mM NaCl; water; and pH 5.8.
I1297. 150 mg/mL secu; 10 mM Phos-Lact; 200 mM treh; 0.5 mg/mL PS80; 1 mM met; 50 mM NaCl; water; and pH 5.8.
I1298. 190 mg/mL secu; 10 mM Phos-Lact; 200 mM treh; 0.5 mg/mL PS80; 1 mM met; 50 mM NaCl; water; and pH 5.8.
I1299. 150 mg/mL secu; 15 mM Gluc; 200 mM treh; 0.5 mg/mL PS80; 1 mM met; 50 mM NaCl; water; and pH 5.8.
I1300. 190 mg/mL secu; 15 mM Gluc; 200 mM treh; 0.5 mg/mL PS80; 1 mM met; 50 mM NaCl; water; and pH 5.8.
I1301. 150 mg/mL secu; 0 mM buffer; 150 mM sorb; 0.2 mg/mL PS20; 1 mM met; 50 mM NaCl; water; and pH 5.8.
I1302. 190 mg/mL secu; 0 mM buffer; 150 mM sorb; 0.2 mg/mL PS20; 1 mM met; 50 mM NaCl; water; and pH 5.8.
I1303. 150 mg/mL secu; 50 mM His; 150 mM sorb; 0.2 mg/mL PS20; 1 mM met; 50 mM NaCl; water; and pH 5.8.
I1304. 190 mg/mL secu; 50 mM His; 150 mM sorb; 0.2 mg/mL PS20; 1 mM met; 50 mM NaCl; water; and pH 5.8.
I1305. 150 mg/mL secu; 25 mM Lact; 150 mM sorb; 0.2 mg/mL PS20; 1 mM met; 50 mM NaCl; water; and pH 5.8.
I1306. 190 mg/mL secu; 25 mM Lact; 150 mM sorb; 0.2 mg/mL PS20; 1 mM met; 50 mM NaCl; water; and pH 5.8.
I1307. 150 mg/mL secu; 10 mM Phos-Lact; 150 mM sorb; 0.2 mg/mL PS20; 1 mM met; 50 mM NaCl; water; and pH 5.8.
I1308. 190 mg/mL secu; 10 mM Phos-Lact; 150 mM sorb; 0.2 mg/mL PS20; 1 mM met; 50 mM NaCl; water; and pH 5.8.
I1309. 150 mg/mL secu; 15 mM Gluc; 150 mM sorb; 0.2 mg/mL PS20; 1 mM met; 50 mM NaCl; water; and pH 5.8.
I1310. 190 mg/mL secu; 15 mM Gluc; 150 mM sorb; 0.2 mg/mL PS20; 1 mM met; 50 mM NaCl; water; and pH 5.8.
I1311. 150 mg/mL secu; 0 mM buffer; 75 mM mann; 0.2 mg/mL PS20; 1 mM met; 50 mM NaCl; water; and pH 5.8.
I1312. 190 mg/mL secu; 0 mM buffer; 75 mM mann; 0.2 mg/mL PS20; 1 mM met; 50 mM NaCl; water; and pH 5.8.
I1313. 150 mg/mL secu; 50 mM His; 75 mM mann; 0.2 mg/mL PS20; 1 mM met; 50 mM NaCl; water; and pH 5.8.
I1314. 190 mg/mL secu; 50 mM His; 75 mM mann; 0.2 mg/mL PS20; 1 mM met; 50 mM NaCl; water; and pH 5.8.
I1315. 150 mg/mL secu; 25 mM Lact; 75 mM mann; 0.2 mg/mL PS20; 1 mM met; 50 mM NaCl; water; and pH 5.8.
I1316. 190 mg/mL secu; 25 mM Lact; 75 mM mann; 0.2 mg/mL PS20; 1 mM met; 50 mM NaCl; water; and pH 5.8.
I1317. 150 mg/mL secu; 10 mM Phos-Lact; 75 mM mann; 0.2 mg/mL PS20; 1 mM met; 50 mM NaCl; water; and pH 5.8.
I1318. 190 mg/mL secu; 10 mM Phos-Lact; 75 mM mann; 0.2 mg/mL PS20; 1 mM met; 50 mM NaCl; water; and pH 5.8.
I1319. 150 mg/mL secu; 15 mM Gluc; 75 mM mann; 0.2 mg/mL PS20; 1 mM met; 50 mM NaCl; water; and pH 5.8.
I1320. 190 mg/mL secu; 15 mM Gluc; 75 mM mann; 0.2 mg/mL PS20; 1 mM met; 50 mM NaCl; water; and pH 5.8.
I1321. 150 mg/mL secu; 0 mM buffer; 100 mM sucr; 0.2 mg/mL PS20; 1 mM met; 50 mM NaCl; water; and pH 5.8.
I1322. 190 mg/mL secu; 0 mM buffer; 100 mM sucr; 0.2 mg/mL PS20; 1 mM met; 50 mM NaCl; water; and pH 5.8.
I1323. 150 mg/mL secu; 50 mM His; 100 mM sucr; 0.2 mg/mL PS20; 1 mM met; 50 mM NaCl; water; and pH 5.8.

(continued)

I1324. 190 mg/mL secu; 50 mM His; 100 mM sucr; 0.2 mg/mL PS20; 1 mM met; 50 mM NaCl; water; and pH 5.8.
I1325. 150 mg/mL secu; 25 mM Lact; 100 mM sucr; 0.2 mg/mL PS20; 1 mM met; 50 mM NaCl; water; and pH 5.8.
I1326. 190 mg/mL secu; 25 mM Lact; 100 mM sucr; 0.2 mg/mL PS20; 1 mM met; 50 mM NaCl; water; and pH 5.8.
I1327. 150 mg/mL secu; 10 mM Phos-Lact; 100 mM sucr; 0.2 mg/mL PS20; 1 mM met; 50 mM NaCl; water; and pH 5.8.
I1328. 190 mg/mL secu; 10 mM Phos-Lact; 100 mM sucr; 0.2 mg/mL PS20; 1 mM met; 50 mM NaCl; water; and pH 5.8.
I1329. 150 mg/mL secu; 15 mM Gluc; 100 mM sucr; 0.2 mg/mL PS20; 1 mM met; 50 mM NaCl; water; and pH 5.8.
I1330. 190 mg/mL secu; 15 mM Gluc; 100 mM sucr; 0.2 mg/mL PS20; 1 mM met; 50 mM NaCl; water; and pH 5.8.
I1331. 150 mg/mL secu; 0 mM buffer; 200 mM treh; 0.2 mg/mL PS20; 1 mM met; 50 mM NaCl; water; and pH 5.8.
I1332. 190 mg/mL secu; 0 mM buffer; 200 mM treh; 0.2 mg/mL PS20; 1 mM met; 50 mM NaCl; water; and pH 5.8.
I1333. 150 mg/mL secu; 50 mM His; 200 mM treh; 0.2 mg/mL PS20; 1 mM met; 50 mM NaCl; water; and pH 5.8.
I1334. 190 mg/mL secu; 50 mM His; 200 mM treh; 0.2 mg/mL PS20; 1 mM met; 50 mM NaCl; water; and pH 5.8.
I1335. 150 mg/mL secu; 25 mM Lact; 200 mM treh; 0.2 mg/mL PS20; 1 mM met; 50 mM NaCl; water; and pH 5.8.
I1336. 190 mg/mL secu; 25 mM Lact; 200 mM treh; 0.2 mg/mL PS20; 1 mM met; 50 mM NaCl; water; and pH 5.8.
I1337. 150 mg/mL secu; 10 mM Phos-Lact; 200 mM treh; 0.2 mg/mL PS20; 1 mM met; 50 mM NaCl; water; and pH 5.8.
I1338. 190 mg/mL secu; 10 mM Phos-Lact; 200 mM treh; 0.2 mg/mL PS20; 1 mM met; 50 mM NaCl; water; and pH 5.8.
I1339. 150 mg/mL secu; 15 mM Gluc; 200 mM treh; 0.2 mg/mL PS20; 1 mM met; 50 mM NaCl; water; and pH 5.8.
I1340. 190 mg/mL secu; 15 mM Gluc; 200 mM treh; 0.2 mg/mL PS20; 1 mM met; 50 mM NaCl; water; and pH 5.8.
I1341. 150 mg/mL secu; 0 mM buffer; 150 mM sorb; 0.5 mg/mL PS80; 1 mM met; 0.1 mM EDTA; water; and pH 5.8.
I1342. 190 mg/mL secu; 0 mM buffer; 150 mM sorb; 0.5 mg/mL PS80; 1 mM met; 0.1 mM EDTA; water; and pH 5.8.
I1343. 150 mg/mL secu; 50 mM His; 150 mM sorb; 0.5 mg/mL PS80; 1 mM met; 0.1 mM EDTA; water; and pH 5.8.
I1344. 190 mg/mL secu; 50 mM His; 150 mM sorb; 0.5 mg/mL PS80; 1 mM met; 0.1 mM EDTA; water; and pH 5.8.
I1345. 150 mg/mL secu; 25 mM Lact; 150 mM sorb; 0.5 mg/mL PS80; 1 mM met; 0.1 mM EDTA; water; and pH 5.8.
I1346. 190 mg/mL secu; 25 mM Lact; 150 mM sorb; 0.5 mg/mL PS80; 1 mM met; 0.1 mM EDTA; water; and pH 5.8.
I1347. 150 mg/mL secu; 10 mM Phos-Lact; 150 mM sorb; 0.5 mg/mL PS80; 1 mM met; 0.1 mM EDTA; water; and pH 5.8.
I1348. 190 mg/mL secu; 10 mM Phos-Lact; 150 mM sorb; 0.5 mg/mL PS80; 1 mM met; 0.1 mM EDTA; water; and pH 5.8.
I1349. 150 mg/mL secu; 15 mM Gluc; 150 mM sorb; 0.5 mg/mL PS80; 1 mM met; 0.1 mM EDTA; water; and pH 5.8.
I1350. 190 mg/mL secu; 15 mM Gluc; 150 mM sorb; 0.5 mg/mL PS80; 1 mM met; 0.1 mM EDTA; water; and pH 5.8.
I1351. 150 mg/mL secu; 0 mM buffer; 75 mM mann; 0.5 mg/mL PS80; 1 mM met; 0.1 mM EDTA; water; and pH 5.8.
I1352. 190 mg/mL secu; 0 mM buffer; 75 mM mann; 0.5 mg/mL PS80; 1 mM met; 0.1 mM EDTA; water; and pH 5.8.
I1353. 150 mg/mL secu; 50 mM His; 75 mM mann; 0.5 mg/mL PS80; 1 mM met; 0.1 mM EDTA; water; and pH 5.8.
I1354. 190 mg/mL secu; 50 mM His; 75 mM mann; 0.5 mg/mL PS80; 1 mM met; 0.1 mM EDTA; water; and pH 5.8.
I1355. 150 mg/mL secu; 25 mM Lact; 75 mM mann; 0.5 mg/mL PS80; 1 mM met; 0.1 mM EDTA; water; and pH 5.8.
I1356. 190 mg/mL secu; 25 mM Lact; 75 mM mann; 0.5 mg/mL PS80; 1 mM met; 0.1 mM EDTA; water; and pH 5.8.
I1357. 150 mg/mL secu; 10 mM Phos-Lact; 75 mM mann; 0.5 mg/mL PS80; 1 mM met; 0.1 mM EDTA; water; and pH 5.8.

(continued)

I1358. 190 mg/mL secu; 10 mM Phos-Lact; 75 mM mann; 0.5 mg/mL PS80; 1 mM met; 0.1 mM EDTA; water; and pH 5.8.

I1359. 150 mg/mL secu; 15 mM Gluc; 75 mM mann; 0.5 mg/mL PS80; 1 mM met; 0.1 mM EDTA; water; and pH 5.8.

I1360. 190 mg/mL secu; 15 mM Gluc; 75 mM mann; 0.5 mg/mL PS80; 1 mM met; 0.1 mM EDTA; water; and pH 5.8.

I1361. 150 mg/mL secu; 0 mM buffer; 100 mM sucr; 0.5 mg/mL PS80; 1 mM met; 0.1 mM EDTA; water; and pH 5.8.

I1362. 190 mg/mL secu; 0 mM buffer; 100 mM sucr; 0.5 mg/mL PS80; 1 mM met; 0.1 mM EDTA; water; and pH 5.8.

I1363. 150 mg/mL secu; 50 mM His; 100 mM sucr; 0.5 mg/mL PS80; 1 mM met; 0.1 mM EDTA; water; and pH 5.8.

I1364. 190 mg/mL secu; 50 mM His; 100 mM sucr; 0.5 mg/mL PS80; 1 mM met; 0.1 mM EDTA; water; and pH 5.8.

I1365. 150 mg/mL secu; 25 mM Lact; 100 mM sucr; 0.5 mg/mL PS80; 1 mM met; 0.1 mM EDTA; water; and pH 5.8.

I1366. 190 mg/mL secu; 25 mM Lact; 100 mM sucr; 0.5 mg/mL PS80; 1 mM met; 0.1 mM EDTA; water; and pH 5.8.

I1367. 150 mg/mL secu; 10 mM Phos-Lact; 100 mM sucr; 0.5 mg/mL PS80; 1 mM met; 0.1 mM EDTA; water; and pH 5.8.

I1368. 190 mg/mL secu; 10 mM Phos-Lact; 100 mM sucr; 0.5 mg/mL PS80; 1 mM met; 0.1 mM EDTA; water; and pH 5.8.

I1369. 150 mg/mL secu; 15 mM Gluc; 100 mM sucr; 0.5 mg/mL PS80; 1 mM met; 0.1 mM EDTA; water; and pH 5.8.

I1370. 190 mg/mL secu; 15 mM Gluc; 100 mM sucr; 0.5 mg/mL PS80; 1 mM met; 0.1 mM EDTA; water; and pH 5.8.

I1371. 150 mg/mL secu; 0 mM buffer; 200 mM treh; 0.5 mg/mL PS80; 1 mM met; 0.1 mM EDTA; water; and pH 5.8.

I1372. 190 mg/mL secu; 0 mM buffer; 200 mM treh; 0.5 mg/mL PS80; 1 mM met; 0.1 mM EDTA; water; and pH 5.8.

I1373. 150 mg/mL secu; 50 mM His; 200 mM treh; 0.5 mg/mL PS80; 1 mM met; 0.1 mM EDTA; water; and pH 5.8.

I1374. 190 mg/mL secu; 50 mM His; 200 mM treh; 0.5 mg/mL PS80; 1 mM met; 0.1 mM EDTA; water; and pH 5.8.

I1375. 150 mg/mL secu; 25 mM Lact; 200 mM treh; 0.5 mg/mL PS80; 1 mM met; 0.1 mM EDTA; water; and pH 5.8.

I11376. 190 mg/mL secu; 25 mM Lact; 200 mM treh; 0.5 mg/mL PS80; 1 mM met; 0.1 mM EDTA; water; and pH 5.8.

I1377. 150 mg/mL secu; 10 mM Phos-Lact; 200 mM treh; 0.5 mg/mL PS80; 1 mM met; 0.1 mM EDTA; water; and pH 5.8.

I1378. 190 mg/mL secu; 10 mM Phos-Lact; 200 mM treh; 0.5 mg/mL PS80; 1 mM met; 0.1 mM EDTA; water; and pH 5.8.

I1379. 150 mg/mL secu; 15 mM Gluc; 200 mM treh; 0.5 mg/mL PS80; 1 mM met; 0.1 mM EDTA; water; and pH 5.8.

I1380. 190 mg/mL secu; 15 mM Gluc; 200 mM treh; 0.5 mg/mL PS80; 1 mM met; 0.1 mM EDTA; water; and pH 5.8.

I1381. 150 mg/mL secu; 0 mM buffer; 150 mM sorb; 0.2 mg/mL PS20; 1 mM met; 0.1 mM EDTA; water; and pH 5.8.

I1382. 190 mg/mL secu; 0 mM buffer; 150 mM sorb; 0.2 mg/mL PS20; 1 mM met; 0.1 mM EDTA; water; and pH 5.8.

I1383. 150 mg/mL secu; 50 mM His; 150 mM sorb; 0.2 mg/mL PS20; 1 mM met; 0.1 mM EDTA; water; and pH 5.8.

I1384. 190 mg/mL secu; 50 mM His; 150 mM sorb; 0.2 mg/mL PS20; 1 mM met; 0.1 mM EDTA; water; and pH 5.8.

I1385. 150 mg/mL secu; 25 mM Lact; 150 mM sorb; 0.2 mg/mL PS20; 1 mM met; 0.1 mM EDTA; water; and pH 5.8.

I1386. 190 mg/mL secu; 25 mM Lact; 150 mM sorb; 0.2 mg/mL PS20; 1 mM met; 0.1 mM EDTA; water; and pH 5.8.

(continued)

I1387. 150 mg/mL secu; 10 mM Phos-Lact; 150 mM sorb; 0.2 mg/mL PS20; 1 mM met; 0.1 mM EDTA; water; and pH 5.8.

I1388. 190 mg/mL secu; 10 mM Phos-Lact; 150 mM sorb; 0.2 mg/mL PS20; 1 mM met; 0.1 mM EDTA; water; and pH 5.8.

I1389. 150 mg/mL secu; 15 mM Gluc; 150 mM sorb; 0.2 mg/mL PS20; 1 mM met; 0.1 mM EDTA; water; and pH 5.8.

I1390. 190 mg/mL secu; 15 mM Gluc; 150 mM sorb; 0.2 mg/mL PS20; 1 mM met; 0.1 mM EDTA; water; and pH 5.8.

I1391. 150 mg/mL secu; 0 mM buffer; 75 mM mann; 0.2 mg/mL PS20; 1 mM met; 0.1 mM EDTA; water; and pH 5.8.

I1392. 190 mg/mL secu; 0 mM buffer; 75 mM mann; 0.2 mg/mL PS20; 1 mM met; 0.1 mM EDTA; water; and pH 5.8.

I1393. 150 mg/mL secu; 50 mM His; 75 mM mann; 0.2 mg/mL PS20; 1 mM met; 0.1 mM EDTA; water; and pH 5.8.

I1394. 190 mg/mL secu; 50 mM His; 75 mM mann; 0.2 mg/mL PS20; 1 mM met; 0.1 mM EDTA; water; and pH 5.8.

I1395. 150 mg/mL secu; 25 mM Lact; 75 mM mann; 0.2 mg/mL PS20; 1 mM met; 0.1 mM EDTA; water; and pH 5.8.

I1396. 190 mg/mL secu; 25 mM Lact; 75 mM mann; 0.2 mg/mL PS20; 1 mM met; 0.1 mM EDTA; water; and pH 5.8.

I1397. 150 mg/mL secu; 10 mM Phos-Lact; 75 mM mann; 0.2 mg/mL PS20; 1 mM met; 0.1 mM EDTA; water; and pH 5.8.

I1398. 190 mg/mL secu; 10 mM Phos-Lact; 75 mM mann; 0.2 mg/mL PS20; 1 mM met; 0.1 mM EDTA; water; and pH 5.8.

I1399. 150 mg/mL secu; 15 mM Gluc; 75 mM mann; 0.2 mg/mL PS20; 1 mM met; 0.1 mM EDTA; water; and pH 5.8.

I1400. 190 mg/mL secu; 15 mM Gluc; 75 mM mann; 0.2 mg/mL PS20; 1 mM met; 0.1 mM EDTA; water; and pH 5.8.

I1401. 150 mg/mL secu; 0 mM buffer; 100 mM sucr; 0.2 mg/mL PS20; 1 mM met; 0.1 mM EDTA; water; and pH 5.8.

I1402. 190 mg/mL secu; 0 mM buffer; 100 mM sucr; 0.2 mg/mL PS20; 1 mM met; 0.1 mM EDTA; water; and pH 5.8.

I1403. 150 mg/mL secu; 50 mM His; 100 mM sucr; 0.2 mg/mL PS20; 1 mM met; 0.1 mM EDTA; water; and pH 5.8.

I1404. 190 mg/mL secu; 50 mM His; 100 mM sucr; 0.2 mg/mL PS20; 1 mM met; 0.1 mM EDTA; water; and pH 5.8.

I1405. 150 mg/mL secu; 25 mM Lact; 100 mM sucr; 0.2 mg/mL PS20; 1 mM met; 0.1 mM EDTA; water; and pH 5.8.

I1406. 190 mg/mL secu; 25 mM Lact; 100 mM sucr; 0.2 mg/mL PS20; 1 mM met; 0.1 mM EDTA; water; and pH 5.8.

I1407. 150 mg/mL secu; 10 mM Phos-Lact; 100 mM sucr; 0.2 mg/mL PS20; 1 mM met; 0.1 mM EDTA; water; and pH 5.8.

I1408. 190 mg/mL secu; 10 mM Phos-Lact; 100 mM sucr; 0.2 mg/mL PS20; 1 mM met; 0.1 mM EDTA; water; and pH 5.8.

I1409. 150 mg/mL secu; 15 mM Gluc; 100 mM sucr; 0.2 mg/mL PS20; 1 mM met; 0.1 mM EDTA; water; and pH 5.8.

I1410. 190 mg/mL secu; 15 mM Gluc; 100 mM sucr; 0.2 mg/mL PS20; 1 mM met; 0.1 mM EDTA; water; and pH 5.8.

I1411. 150 mg/mL secu; 0 mM buffer; 200 mM treh; 0.2 mg/mL PS20; 1 mM met; 0.1 mM EDTA; water; and pH 5.8.

I1412. 190 mg/mL secu; 0 mM buffer; 200 mM treh; 0.2 mg/mL PS20; 1 mM met; 0.1 mM EDTA; water; and pH 5.8.

I1413. 150 mg/mL secu; 50 mM His; 200 mM treh; 0.2 mg/mL PS20; 1 mM met; 0.1 mM EDTA; water; and pH 5.8.

I1414. 190 mg/mL secu; 50 mM His; 200 mM treh; 0.2 mg/mL PS20; 1 mM met; 0.1 mM EDTA; water; and pH 5.8.

I1415. 150 mg/mL secu; 25 mM Lact; 200 mM treh; 0.2 mg/mL PS20; 1 mM met; 0.1 mM EDTA; water; and pH 5.8.

(continued)

I1416. 190 mg/mL secu; 25 mM Lact; 200 mM treh; 0.2 mg/mL PS20; 1 mM met; 0.1 mM EDTA; water; and pH 5.8.

I1417. 150 mg/mL secu; 10 mM Phos-Lact; 200 mM treh; 0.2 mg/mL PS20; 1 mM met; 0.1 mM EDTA; water; and pH 5.8.

I1418. 190 mg/mL secu; 10 mM Phos-Lact; 200 mM treh; 0.2 mg/mL PS20; 1 mM met; 0.1 mM EDTA; water; and pH 5.8.

I1419. 150 mg/mL secu; 15 mM Gluc; 200 mM treh; 0.2 mg/mL PS20; 1 mM met; 0.1 mM EDTA; water; and pH 5.8.

I1420. 190 mg/mL secu; 15 mM Gluc; 200 mM treh; 0.2 mg/mL PS20; 1 mM met; 0.1 mM EDTA; water; and pH 5.8.

I1421. 150 mg/mL secu; 0 mM buffer; 150 mM sorb; 0.5 mg/mL PS80; 50 mM NaCl; 0.1 mM EDTA; water; and pH 5.8.

I1422. 190 mg/mL secu; 0 mM buffer; 150 mM sorb; 0.5 mg/mL PS80; 50 mM NaCl; 0.1 mM EDTA; water; and pH 5.8.

I1423. 150 mg/mL secu; 50 mM His; 150 mM sorb; 0.5 mg/mL PS80; 50 mM NaCl; 0.1 mM EDTA; water; and pH 5.8.

I1424. 190 mg/mL secu; 50 mM His; 150 mM sorb; 0.5 mg/mL PS80; 50 mM NaCl; 0.1 mM EDTA; water; and pH 5.8.

I1425. 150 mg/mL secu; 25 mM Lact; 150 mM sorb; 0.5 mg/mL PS80; 50 mM NaCl; 0.1 mM EDTA; water; and pH 5.8.

I1426. 190 mg/mL secu; 25 mM Lact; 150 mM sorb; 0.5 mg/mL PS80; 50 mM NaCl; 0.1 mM EDTA; water; and pH 5.8.

I1427. 150 mg/mL secu; 10 mM Phos-Lact; 150 mM sorb; 0.5 mg/mL PS80; 50 mM NaCl; 0.1 mM EDTA; water; and pH 5.8.

I1428. 190 mg/mL secu; 10 mM Phos-Lact; 150 mM sorb; 0.5 mg/mL PS80; 50 mM NaCl; 0.1 mM EDTA; water; and pH 5.8.

I1429. 150 mg/mL secu; 15 mM Gluc; 150 mM sorb; 0.5 mg/mL PS80; 50 mM NaCl; 0.1 mM EDTA; water; and pH 5.8.

I1430. 190 mg/mL secu; 15 mM Gluc; 150 mM sorb; 0.5 mg/mL PS80; 50 mM NaCl; 0.1 mM EDTA; water; and pH 5.8.

I1431. 150 mg/mL secu; 0 mM buffer; 75 mM mann; 0.5 mg/mL PS80; 50 mM NaCl; 0.1 mM EDTA; water; and pH 5.8.

I1432. 190 mg/mL secu; 0 mM buffer; 75 mM mann; 0.5 mg/mL PS80; 50 mM NaCl; 0.1 mM EDTA; water; and pH 5.8.

I1433. 150 mg/mL secu; 50 mM His; 75 mM mann; 0.5 mg/mL PS80; 50 mM NaCl; 0.1 mM EDTA; water; and pH 5.8.

I1434. 190 mg/mL secu; 50 mM His; 75 mM mann; 0.5 mg/mL PS80; 50 mM NaCl; 0.1 mM EDTA; water; and pH 5.8.

I1435. 150 mg/mL secu; 25 mM Lact; 75 mM mann; 0.5 mg/mL PS80; 50 mM NaCl; 0.1 mM EDTA; water; and pH 5.8.

I1436. 190 mg/mL secu; 25 mM Lact; 75 mM mann; 0.5 mg/mL PS80; 50 mM NaCl; 0.1 mM EDTA; water; and pH 5.8.

I1437. 150 mg/mL secu; 10 mM Phos-Lact; 75 mM mann; 0.5 mg/mL PS80; 50 mM NaCl; 0.1 mM EDTA; water; and pH 5.8.

I1438. 190 mg/mL secu; 10 mM Phos-Lact; 75 mM mann; 0.5 mg/mL PS80; 50 mM NaCl; 0.1 mM EDTA; water; and pH 5.8.

I1439. 150 mg/mL secu; 15 mM Gluc; 75 mM mann; 0.5 mg/mL PS80; 50 mM NaCl; 0.1 mM EDTA; water; and pH 5.8.

I1440. 190 mg/mL secu; 15 mM Gluc; 75 mM mann; 0.5 mg/mL PS80; 50 mM NaCl; 0.1 mM EDTA; water; and pH 5.8.

I1441. 150 mg/mL secu; 0 mM buffer; 100 mM sucr; 0.5 mg/mL PS80; 50 mM NaCl; 0.1 mM EDTA; water; and pH 5.8.

(continued)

I1442. 190 mg/mL secu; 0 mM buffer; 100 mM sucr; 0.5 mg/mL PS80; 50 mM NaCl; 0.1 mM EDTA; water; and pH 5.8.

I1443. 150 mg/mL secu; 50 mM His; 100 mM sucr; 0.5 mg/mL PS80; 50 mM NaCl; 0.1 mM EDTA; water; and pH 5.8.

I1444. 190 mg/mL secu; 50 mM His; 100 mM sucr; 0.5 mg/mL PS80; 50 mM NaCl; 0.1 mM EDTA; water; and pH 5.8.

I1445. 150 mg/mL secu; 25 mM Lact; 100 mM sucr; 0.5 mg/mL PS80; 50 mM NaCl; 0.1 mM EDTA; water; and pH 5.8.

I1446. 190 mg/mL secu; 25 mM Lact; 100 mM sucr; 0.5 mg/mL PS80; 50 mM NaCl; 0.1 mM EDTA; water; and pH 5.8.

I1447. 150 mg/mL secu; 10 mM Phos-Lact; 100 mM sucr; 0.5 mg/mL PS80; 50 mM NaCl; 0.1 mM EDTA; water; and pH 5.8.

I1448. 190 mg/mL secu; 10 mM Phos-Lact; 100 mM sucr; 0.5 mg/mL PS80; 50 mM NaCl; 0.1 mM EDTA; water; and pH 5.8.

I1449. 150 mg/mL secu; 15 mM Gluc; 100 mM sucr; 0.5 mg/mL PS80; 50 mM NaCl; 0.1 mM EDTA; water; and pH 5.8.

I1450. 190 mg/mL secu; 15 mM Gluc; 100 mM sucr; 0.5 mg/mL PS80; 50 mM NaCl; 0.1 mM EDTA; water; and pH 5.8.

I1451. 150 mg/mL secu; 0 mM buffer; 200 mM treh; 0.5 mg/mL PS80; 50 mM NaCl; 0.1 mM EDTA; water; and pH 5.8.

I1452. 190 mg/mL secu; 0 mM buffer; 200 mM treh; 0.5 mg/mL PS80; 50 mM NaCl; 0.1 mM EDTA; water; and pH 5.8.

I1453. 150 mg/mL secu; 50 mM His; 200 mM treh; 0.5 mg/mL PS80; 50 mM NaCl; 0.1 mM EDTA; water; and pH 5.8.

I1454. 190 mg/mL secu; 50 mM His; 200 mM treh; 0.5 mg/mL PS80; 50 mM NaCl; 0.1 mM EDTA; water; and pH 5.8.

I1455. 150 mg/mL secu; 25 mM Lact; 200 mM treh; 0.5 mg/mL PS80; 50 mM NaCl; 0.1 mM EDTA; water; and pH 5.8.

I1456. 190 mg/mL secu; 25 mM Lact; 200 mM treh; 0.5 mg/mL PS80; 50 mM NaCl; 0.1 mM EDTA; water; and pH 5.8.

I1457. 150 mg/mL secu; 10 mM Phos-Lact; 200 mM treh; 0.5 mg/mL PS80; 50 mM NaCl; 0.1 mM EDTA; water; and pH 5.8.

I1458. 190 mg/mL secu; 10 mM Phos-Lact; 200 mM treh; 0.5 mg/mL PS80; 50 mM NaCl; 0.1 mM EDTA; water; and pH 5.8.

I1459. 150 mg/mL secu; 15 mM Gluc; 200 mM treh; 0.5 mg/mL PS80; 50 mM NaCl; 0.1 mM EDTA; water; and pH 5.8.

I1460. 190 mg/mL secu; 15 mM Gluc; 200 mM treh; 0.5 mg/mL PS80; 50 mM NaCl; 0.1 mM EDTA; water; and pH 5.8.

I1461. 150 mg/mL secu; 0 mM buffer; 150 mM sorb; 0.2 mg/mL PS20; 50 mM NaCl; 0.1 mM EDTA; water; and pH 5.8.

I1462. 190 mg/mL secu; 0 mM buffer; 150 mM sorb; 0.2 mg/mL PS20; 50 mM NaCl; 0.1 mM EDTA; water; and pH 5.8.

I1463. 150 mg/mL secu; 50 mM His; 150 mM sorb; 0.2 mg/mL PS20; 50 mM NaCl; 0.1 mM EDTA; water; and pH 5.8.

I1464. 190 mg/mL secu; 50 mM His; 150 mM sorb; 0.2 mg/mL PS20; 50 mM NaCl; 0.1 mM EDTA; water; and pH 5.8.

I1465. 150 mg/mL secu; 25 mM Lact; 150 mM sorb; 0.2 mg/mL PS20; 50 mM NaCl; 0.1 mM EDTA; water; and pH 5.8.

I1466. 190 mg/mL secu; 25 mM Lact; 150 mM sorb; 0.2 mg/mL PS20; 50 mM NaCl; 0.1 mM EDTA; water; and pH 5.8.

I1467. 150 mg/mL secu; 10 mM Phos-Lact; 150 mM sorb; 0.2 mg/mL PS20; 50 mM NaCl; 0.1 mM EDTA; water; and pH 5.8.

(continued)

I1468. 190 mg/mL secu; 10 mM Phos-Lact; 150 mM sorb; 0.2 mg/mL PS20; 50 mM NaCl; 0.1 mM EDTA; water; and pH 5.8.

I1469. 150 mg/mL secu; 15 mM Gluc; 150 mM sorb; 0.2 mg/mL PS20; 50 mM NaCl; 0.1 mM EDTA; water; and pH 5.8.

I1470. 190 mg/mL secu; 15 mM Gluc; 150 mM sorb; 0.2 mg/mL PS20; 50 mM NaCl; 0.1 mM EDTA; water; and pH 5.8.

I1471. 150 mg/mL secu; 0 mM buffer; 75 mM mann; 0.2 mg/mL PS20; 50 mM NaCl; 0.1 mM EDTA; water; and pH 5.8.

I1472. 190 mg/mL secu; 0 mM buffer; 75 mM mann; 0.2 mg/mL PS20; 50 mM NaCl; 0.1 mM EDTA; water; and pH 5.8.

I1473. 150 mg/mL secu; 50 mM His; 75 mM mann; 0.2 mg/mL PS20; 50 mM NaCl; 0.1 mM EDTA; water; and pH 5.8.

I1474. 190 mg/mL secu; 50 mM His; 75 mM mann; 0.2 mg/mL PS20; 50 mM NaCl; 0.1 mM EDTA; water; and pH 5.8.

I1475. 150 mg/mL secu; 25 mM Lact; 75 mM mann; 0.2 mg/mL PS20; 50 mM NaCl; 0.1 mM EDTA; water; and pH 5.8.

I1476. 190 mg/mL secu; 25 mM Lact; 75 mM mann; 0.2 mg/mL PS20; 50 mM NaCl; 0.1 mM EDTA; water; and pH 5.8.

I1477. 150 mg/mL secu; 10 mM Phos-Lact; 75 mM mann; 0.2 mg/mL PS20; 50 mM NaCl; 0.1 mM EDTA; water; and pH 5.8.

I1478. 190 mg/mL secu; 10 mM Phos-Lact; 75 mM mann; 0.2 mg/mL PS20; 50 mM NaCl; 0.1 mM EDTA; water; and pH 5.8.

I1479. 150 mg/mL secu; 15 mM Gluc; 75 mM mann; 0.2 mg/mL PS20; 50 mM NaCl; 0.1 mM EDTA; water; and pH 5.8.

I1480. 190 mg/mL secu; 15 mM Gluc; 75 mM mann; 0.2 mg/mL PS20; 50 mM NaCl; 0.1 mM EDTA; water; and pH 5.8.

I1481. 150 mg/mL secu; 0 mM buffer; 100 mM sucr; 0.2 mg/mL PS20; 50 mM NaCl; 0.1 mM EDTA; water; and pH 5.8.

I1482. 190 mg/mL secu; 0 mM buffer; 100 mM sucr; 0.2 mg/mL PS20; 50 mM NaCl; 0.1 mM EDTA; water; and pH 5.8.

I1483. 150 mg/mL secu; 50 mM His; 100 mM sucr; 0.2 mg/mL PS20; 50 mM NaCl; 0.1 mM EDTA; water; and pH 5.8.

I1484. 190 mg/mL secu; 50 mM His; 100 mM sucr; 0.2 mg/mL PS20; 50 mM NaCl; 0.1 mM EDTA; water; and pH 5.8.

I1485. 150 mg/mL secu; 25 mM Lact; 100 mM sucr; 0.2 mg/mL PS20; 50 mM NaCl; 0.1 mM EDTA; water; and pH 5.8.

I1486. 190 mg/mL secu; 25 mM Lact; 100 mM sucr; 0.2 mg/mL PS20; 50 mM NaCl; 0.1 mM EDTA; water; and pH 5.8.

I1487. 150 mg/mL secu; 10 mM Phos-Lact; 100 mM sucr; 0.2 mg/mL PS20; 50 mM NaCl; 0.1 mM EDTA; water; and pH 5.8.

I1488. 190 mg/mL secu; 10 mM Phos-Lact; 100 mM sucr; 0.2 mg/mL PS20; 50 mM NaCl; 0.1 mM EDTA; water; and pH 5.8.

I1489. 150 mg/mL secu; 15 mM Gluc; 100 mM sucr; 0.2 mg/mL PS20; 50 mM NaCl; 0.1 mM EDTA; water; and pH 5.8.

I1490. 190 mg/mL secu; 15 mM Gluc; 100 mM sucr; 0.2 mg/mL PS20; 50 mM NaCl; 0.1 mM EDTA; water; and pH 5.8.

I1491. 150 mg/mL secu; 0 mM buffer; 200 mM treh; 0.2 mg/mL PS20; 50 mM NaCl; 0.1 mM EDTA; water; and pH 5.8.

I1492. 190 mg/mL secu; 0 mM buffer; 200 mM treh; 0.2 mg/mL PS20; 50 mM NaCl; 0.1 mM EDTA; water; and pH 5.8.

I1493. 150 mg/mL secu; 50 mM His; 200 mM treh; 0.2 mg/mL PS20; 50 mM NaCl; 0.1 mM EDTA; water; and pH 5.8.

(continued)

I1494. 190 mg/mL secu; 50 mM His; 200 mM treh; 0.2 mg/mL PS20; 50 mM NaCl; 0.1 mM EDTA; water; and pH 5.8.

I1495. 150 mg/mL secu; 25 mM Lact; 200 mM treh; 0.2 mg/mL PS20; 50 mM NaCl; 0.1 mM EDTA; water; and pH 5.8.

I1496. 190 mg/mL secu; 25 mM Lact; 200 mM treh; 0.2 mg/mL PS20; 50 mM NaCl; 0.1 mM EDTA; water; and pH 5.8.

I1497. 150 mg/mL secu; 10 mM Phos-Lact; 200 mM treh; 0.2 mg/mL PS20; 50 mM NaCl; 0.1 mM EDTA; water; and pH 5.8.

I1498. 190 mg/mL secu; 10 mM Phos-Lact; 200 mM treh; 0.2 mg/mL PS20; 50 mM NaCl; 0.1 mM EDTA; water; and pH 5.8.

I1499. 150 mg/mL secu; 15 mM Gluc; 200 mM treh; 0.2 mg/mL PS20; 50 mM NaCl; 0.1 mM EDTA; water; and pH 5.8.

I1500. 190 mg/mL secu; 15 mM Gluc; 200 mM treh; 0.2 mg/mL PS20; 50 mM NaCl; 0.1 mM EDTA; water; and pH 5.8.

I1501. 150 mg/mL secu; 0 mM buffer; 50 mM arg; 0.5 mg/mL PS80; 1 mM met; 50 mM NaCl; water; and pH 5.8.

I1502. 190 mg/mL secu; 0 mM buffer; 50 mM arg; 0.5 mg/mL PS80; 1 mM met; 50 mM NaCl; water; and pH 5.8.

I1503. 150 mg/mL secu; 50 mM His; 50 mM arg; 0.5 mg/mL PS80; 1 mM met; 50 mM NaCl; water; and pH 5.8.

I1504. 190 mg/mL secu; 50 mM His; 50 mM arg; 0.5 mg/mL PS80; 1 mM met; 50 mM NaCl; water; and pH 5.8.

I1505. 150 mg/mL secu; 25 mM Lact; 50 mM arg; 0.5 mg/mL PS80; 1 mM met; 50 mM NaCl; water; and pH 5.8.

I1506. 190 mg/mL secu; 25 mM Lact; 50 mM arg; 0.5 mg/mL PS80; 1 mM met; 50 mM NaCl; water; and pH 5.8.

I1507. 150 mg/mL secu; 10 mM Phos-Lact; 50 mM arg; 0.5 mg/mL PS80; 1 mM met; 50 mM NaCl; water; and pH 5.8.

I1508. 190 mg/mL secu; 10 mM Phos-Lact; 50 mM arg; 0.5 mg/mL PS80; 1 mM met; 50 mM NaCl; water; and pH 5.8.

I1509. 150 mg/mL secu; 15 mM Gluc; 50 mM arg; 0.5 mg/mL PS80; 1 mM met; 50 mM NaCl; water; and pH 5.8.

I1510. 190 mg/mL secu; 15 mM Gluc; 50 mM arg; 0.5 mg/mL PS80; 1 mM met; 50 mM NaCl; water; and pH 5.8.

I1511. 150 mg/mL secu; 0 mM buffer; 100 mM gly; 0.5 mg/mL PS80; 1 mM met; 50 mM NaCl; water; and pH 5.8.

I1512. 190 mg/mL secu; 0 mM buffer; 100 mM gly; 0.5 mg/mL PS80; 1 mM met; 50 mM NaCl; water; and pH 5.8.

I1513. 150 mg/mL secu; 50 mM His; 100 mM gly; 0.5 mg/mL PS80; 1 mM met; 50 mM NaCl; water; and pH 5.8.

I1514. 190 mg/mL secu; 50 mM His; 100 mM gly; 0.5 mg/mL PS80; 1 mM met; 50 mM NaCl; water; and pH 5.8.

I1515. 150 mg/mL secu; 25 mM Lact; 100 mM gly; 0.5 mg/mL PS80; 1 mM met; 50 mM NaCl; water; and pH 5.8.

I1516. 190 mg/mL secu; 25 mM Lact; 100 mM gly; 0.5 mg/mL PS80; 1 mM met; 50 mM NaCl; water; and pH 5.8.

I1517. 150 mg/mL secu; 10 mM Phos-Lact; 100 mM gly; 0.5 mg/mL PS80; 1 mM met; 50 mM NaCl; water; and pH 5.8.

I1518. 190 mg/mL secu; 10 mM Phos-Lact; 100 mM gly; 0.5 mg/mL PS80; 1 mM met; 50 mM NaCl; water; and pH 5.8.

I1519. 150 mg/mL secu; 15 mM Gluc; 100 mM gly; 0.5 mg/mL PS80; 1 mM met; 50 mM NaCl; water; and pH 5.8.

I1520. 190 mg/mL secu; 15 mM Gluc; 100 mM gly; 0.5 mg/mL PS80; 1 mM met; 50 mM NaCl; water; and pH 5.8.

I1521. 150 mg/mL secu; 0 mM buffer; 50 mM arg; 0.2 mg/mL PS20; 1 mM met; 50 mM NaCl; water; and pH 5.8.

I1522. 190 mg/mL secu; 0 mM buffer; 50 mM arg; 0.2 mg/mL PS20; 1 mM met; 50 mM NaCl; water; and pH 5.8.

I1523. 150 mg/mL secu; 50 mM His; 50 mM arg; 0.2 mg/mL PS20; 1 mM met; 50 mM NaCl; water; and pH 5.8.

I1524. 190 mg/mL secu; 50 mM His; 50 mM arg; 0.2 mg/mL PS20; 1 mM met; 50 mM NaCl; water; and pH 5.8.

I1525. 150 mg/mL secu; 25 mM Lact; 50 mM arg; 0.2 mg/mL PS20; 1 mM met; 50 mM NaCl; water; and pH 5.8.

I1526. 190 mg/mL secu; 25 mM Lact; 50 mM arg; 0.2 mg/mL PS20; 1 mM met; 50 mM NaCl; water; and pH 5.8.

I1527. 150 mg/mL secu; 10 mM Phos-Lact; 50 mM arg; 0.2 mg/mL PS20; 1 mM met; 50 mM NaCl; water; and pH 5.8.

I1528. 190 mg/mL secu; 10 mM Phos-Lact; 50 mM arg; 0.2 mg/mL PS20; 1 mM met; 50 mM NaCl; water; and pH 5.8.

I1529. 150 mg/mL secu; 15 mM Gluc; 50 mM arg; 0.2 mg/mL PS20; 1 mM met; 50 mM NaCl; water; and pH 5.8.

I1530. 190 mg/mL secu; 15 mM Gluc; 50 mM arg; 0.2 mg/mL PS20; 1 mM met; 50 mM NaCl; water; and pH 5.8.

I1531. 150 mg/mL secu; 0 mM buffer; 100 mM gly; 0.2 mg/mL PS20; 1 mM met; 50 mM NaCl; water; and pH 5.8.

I1532. 190 mg/mL secu; 0 mM buffer; 100 mM gly; 0.2 mg/mL PS20; 1 mM met; 50 mM NaCl; water; and pH 5.8.

(continued)

| | |
|---|---|
| I1533. | 150 mg/mL secu; 50 mM His; 100 mM gly; 0.2 mg/mL PS20; 1 mM met; 50 mM NaCl; water; and pH 5.8. |
| I1534. | 190 mg/mL secu; 50 mM His; 100 mM gly; 0.2 mg/mL PS20; 1 mM met; 50 mM NaCl; water; and pH 5.8. |
| I1535. | 150 mg/mL secu; 25 mM Lact; 100 mM gly; 0.2 mg/mL PS20; 1 mM met; 50 mM NaCl; water; and pH 5.8. |
| I1536. | 190 mg/mL secu; 25 mM Lact; 100 mM gly; 0.2 mg/mL PS20; 1 mM met; 50 mM NaCl; water; and pH 5.8. |
| I1537. | 150 mg/mL secu; 10 mM Phos-Lact; 100 mM gly; 0.2 mg/mL PS20; 1 mM met; 50 mM NaCl; water; and pH 5.8. |
| I1538. | 190 mg/mL secu; 10 mM Phos-Lact; 100 mM gly; 0.2 mg/mL PS20; 1 mM met; 50 mM NaCl; water; and pH 5.8. |
| I1539. | 150 mg/mL secu; 15 mM Gluc; 100 mM gly; 0.2 mg/mL PS20; 1 mM met; 50 mM NaCl; water; and pH 5.8. |
| I1540. | 190 mg/mL secu; 15 mM Gluc; 100 mM gly; 0.2 mg/mL PS20; 1 mM met; 50 mM NaCl; water; and pH 5.8. |
| I1541. | 150 mg/mL secu; 0 mM buffer; 50 mM arg; 0.5 mg/mL PS80; 1 mM met; 0.1 mM EDTA; water; and pH 5.8. |
| I1542. | 190 mg/mL secu; 0 mM buffer; 50 mM arg; 0.5 mg/mL PS80; 1 mM met; 0.1 mM EDTA; water; and pH 5.8. |
| I1543. | 150 mg/mL secu; 50 mM His; 50 mM arg; 0.5 mg/mL PS80; 1 mM met; 0.1 mM EDTA; water; and pH 5.8. |
| I1544. | 190 mg/mL secu; 50 mM His; 50 mM arg; 0.5 mg/mL PS80; 1 mM met; 0.1 mM EDTA; water; and pH 5.8. |
| I1545. | 150 mg/mL secu; 25 mM Lact; 50 mM arg; 0.5 mg/mL PS80; 1 mM met; 0.1 mM EDTA; water; and pH 5.8. |
| I1546. | 190 mg/mL secu; 25 mM Lact; 50 mM arg; 0.5 mg/mL PS80; 1 mM met; 0.1 mM EDTA; water; and pH 5.8. |
| I1547. | 150 mg/mL secu; 10 mM Phos-Lact; 50 mM arg; 0.5 mg/mL PS80; 1 mM met; 0.1 mM EDTA; water; and pH 5.8. |
| I1548. | 190 mg/mL secu; 10 mM Phos-Lact; 50 mM arg; 0.5 mg/mL PS80; 1 mM met; 0.1 mM EDTA; water; and pH 5.8. |
| I1549. | 150 mg/mL secu; 15 mM Gluc; 50 mM arg; 0.5 mg/mL PS80; 1 mM met; 0.1 mM EDTA; water; and pH 5.8. |
| I1550. | 190 mg/mL secu; 15 mM Gluc; 50 mM arg; 0.5 mg/mL PS80; 1 mM met; 0.1 mM EDTA; water; and pH 5.8. |
| I1551. | 150 mg/mL secu; 0 mM buffer; 100 mM gly; 0.5 mg/mL PS80; 1 mM met; 0.1 mM EDTA; water; and pH 5.8. |
| I1552. | 190 mg/mL secu; 0 mM buffer; 100 mM gly; 0.5 mg/mL PS80; 1 mM met; 0.1 mM EDTA; water; and pH 5.8. |
| I1553. | 150 mg/mL secu; 50 mM His; 100 mM gly; 0.5 mg/mL PS80; 1 mM met; 0.1 mM EDTA; water; and pH 5.8. |
| I1554. | 190 mg/mL secu; 50 mM His; 100 mM gly; 0.5 mg/mL PS80; 1 mM met; 0.1 mM EDTA; water; and pH 5.8. |
| I1555. | 150 mg/mL secu; 25 mM Lact; 100 mM gly; 0.5 mg/mL PS80; 1 mM met; 0.1 mM EDTA; water; and pH 5.8. |
| I1556. | 190 mg/mL secu; 25 mM Lact; 100 mM gly; 0.5 mg/mL PS80; 1 mM met; 0.1 mM EDTA; water; and pH 5.8. |
| I1557. | 150 mg/mL secu; 10 mM Phos-Lact; 100 mM gly; 0.5 mg/mL PS80; 1 mM met; 0.1 mM EDTA; water; and pH 5.8. |
| I1558. | 190 mg/mL secu; 10 mM Phos-Lact; 100 mM gly; 0.5 mg/mL PS80; 1 mM met; 0.1 mM EDTA; water; and pH 5.8. |
| I1559. | 150 mg/mL secu; 15 mM Gluc; 100 mM gly; 0.5 mg/mL PS80; 1 mM met; 0.1 mM EDTA; water; and pH 5.8. |
| I1560. | 190 mg/mL secu; 15 mM Gluc; 100 mM gly; 0.5 mg/mL PS80; 1 mM met; 0.1 mM EDTA; water; and pH 5.8. |
| I1561. | 150 mg/mL secu; 0 mM buffer; 50 mM arg; 0.2 mg/mL PS20; 1 mM met; 0.1 mM EDTA; water; and pH 5.8. |
| I1562. | 190 mg/mL secu; 0 mM buffer; 50 mM arg; 0.2 mg/mL PS20; 1 mM met; 0.1 mM EDTA; water; and pH 5.8. |
| I1563. | 150 mg/mL secu; 50 mM His; 50 mM arg; 0.2 mg/mL PS20; 1 mM met; 0.1 mM EDTA; water; and pH 5.8. |
| I1564. | 190 mg/mL secu; 50 mM His; 50 mM arg; 0.2 mg/mL PS20; 1 mM met; 0.1 mM EDTA; water; and pH 5.8. |
| I1565. | 150 mg/mL secu; 25 mM Lact; 50 mM arg; 0.2 mg/mL PS20; 1 mM met; 0.1 mM EDTA; water; and pH 5.8. |
| I1566. | 190 mg/mL secu; 25 mM Lact; 50 mM arg; 0.2 mg/mL PS20; 1 mM met; 0.1 mM EDTA; water; and pH 5.8. |
| I1567. | 150 mg/mL secu; 10 mM Phos-Lact; 50 mM arg; 0.2 mg/mL PS20; 1 mM met; 0.1 mM EDTA; water; and pH 5.8. |
| I1568. | 190 mg/mL secu; 10 mM Phos-Lact; 50 mM arg; 0.2 mg/mL PS20; 1 mM met; 0.1 mM EDTA; water; and pH 5.8. |
| I1569. | 150 mg/mL secu; 15 mM Gluc; 50 mM arg; 0.2 mg/mL PS20; 1 mM met; 0.1 mM EDTA; water; and pH 5.8. |
| I1570. | 190 mg/mL secu; 15 mM Gluc; 50 mM arg; 0.2 mg/mL PS20; 1 mM met; 0.1 mM EDTA; water; and pH 5.8. |
| I1571. | 150 mg/mL secu; 0 mM buffer; 100 mM gly; 0.2 mg/mL PS20; 1 mM met; 0.1 mM EDTA; water; and pH 5.8. |
| I1572. | 190 mg/mL secu; 0 mM buffer; 100 mM gly; 0.2 mg/mL PS20; 1 mM met; 0.1 mM EDTA; water; and pH 5.8. |
| I1573. | 150 mg/mL secu; 50 mM His; 100 mM gly; 0.2 mg/mL PS20; 1 mM met; 0.1 mM EDTA; water; and pH 5.8. |
| I1574. | 190 mg/mL secu; 50 mM His; 100 mM gly; 0.2 mg/mL PS20; 1 mM met; 0.1 mM EDTA; water; and pH 5.8. |
| I1575. | 150 mg/mL secu; 25 mM Lact; 100 mM gly; 0.2 mg/mL PS20; 1 mM met; 0.1 mM EDTA; water; and pH 5.8. |

(continued)

I1576. 190 mg/mL secu; 25 mM Lact; 100 mM gly; 0.2 mg/mL PS20; 1 mM met; 0.1 mM EDTA; water; and pH 5.8.

I1577. 150 mg/mL secu; 10 mM Phos-Lact; 100 mM gly; 0.2 mg/mL PS20; 1 mM met; 0.1 mM EDTA; water; and pH 5.8.

I1578. 190 mg/mL secu; 10 mM Phos-Lact; 100 mM gly; 0.2 mg/mL PS20; 1 mM met; 0.1 mM EDTA; water; and pH 5.8.

I1579. 150 mg/mL secu; 15 mM Gluc; 100 mM gly; 0.2 mg/mL PS20; 1 mM met; 0.1 mM EDTA; water; and pH 5.8.

I1580. 190 mg/mL secu; 15 mM Gluc; 100 mM gly; 0.2 mg/mL PS20; 1 mM met; 0.1 mM EDTA; water; and pH 5.8.

I1581. 150 mg/mL secu; 0 mM buffer; 50 mM arg; 0.5 mg/mL PS80; 50 mM NaCl; 0.1 mM EDTA; water; and pH 5.8.

I1582. 190 mg/mL secu; 0 mM buffer; 50 mM arg; 0.5 mg/mL PS80; 50 mM NaCl; 0.1 mM EDTA; water; and pH 5.8.

I1583. 150 mg/mL secu; 50 mM His; 50 mM arg; 0.5 mg/mL PS80; 50 mM NaCl; 0.1 mM EDTA; water; and pH 5.8.

I1584. 190 mg/mL secu; 50 mM His; 50 mM arg; 0.5 mg/mL PS80; 50 mM NaCl; 0.1 mM EDTA; water; and pH 5.8.

I1585. 150 mg/mL secu; 25 mM Lact; 50 mM arg; 0.5 mg/mL PS80; 50 mM NaCl; 0.1 mM EDTA; water; and pH 5.8.

I1586. 190 mg/mL secu; 25 mM Lact; 50 mM arg; 0.5 mg/mL PS80; 50 mM NaCl; 0.1 mM EDTA; water; and pH 5.8.

I1587. 150 mg/mL secu; 10 mM Phos-Lact; 50 mM arg; 0.5 mg/mL PS80; 50 mM NaCl; 0.1 mM EDTA; water; and pH 5.8.

I1588. 190 mg/mL secu; 10 mM Phos-Lact; 50 mM arg; 0.5 mg/mL PS80; 50 mM NaCl; 0.1 mM EDTA; water; and pH 5.8.

I1589. 150 mg/mL secu; 15 mM Gluc; 50 mM arg; 0.5 mg/mL PS80; 50 mM NaCl; 0.1 mM EDTA; water; and pH 5.8.

I1590. 190 mg/mL secu; 15 mM Gluc; 50 mM arg; 0.5 mg/mL PS80; 50 mM NaCl; 0.1 mM EDTA; water; and pH 5.8.

I1591. 150 mg/mL secu; 0 mM buffer; 100 mM gly; 0.5 mg/mL PS80; 50 mM NaCl; 0.1 mM EDTA; water; and pH 5.8.

I1592. 190 mg/mL secu; 0 mM buffer; 100 mM gly; 0.5 mg/mL PS80; 50 mM NaCl; 0.1 mM EDTA; water; and pH 5.8.

I1593. 150 mg/mL secu; 50 mM His; 100 mM gly; 0.5 mg/mL PS80; 50 mM NaCl; 0.1 mM EDTA; water; and pH 5.8.

I1594. 190 mg/mL secu; 50 mM His; 100 mM gly; 0.5 mg/mL PS80; 50 mM NaCl; 0.1 mM EDTA; water; and pH 5.8.

I1595. 150 mg/mL secu; 25 mM Lact; 100 mM gly; 0.5 mg/mL PS80; 50 mM NaCl; 0.1 mM EDTA; water; and pH 5.8.

I1596. 190 mg/mL secu; 25 mM Lact; 100 mM gly; 0.5 mg/mL PS80; 50 mM NaCl; 0.1 mM EDTA; water; and pH 5.8.

I1597. 150 mg/mL secu; 10 mM Phos-Lact; 100 mM gly; 0.5 mg/mL PS80; 50 mM NaCl; 0.1 mM EDTA; water; and pH 5.8.

I1598. 190 mg/mL secu; 10 mM Phos-Lact; 100 mM gly; 0.5 mg/mL PS80; 50 mM NaCl; 0.1 mM EDTA; water; and pH 5.8.

I1599. 150 mg/mL secu; 15 mM Gluc; 100 mM gly; 0.5 mg/mL PS80; 50 mM NaCl; 0.1 mM EDTA; water; and pH 5.8.

I1600. 190 mg/mL secu; 15 mM Gluc; 100 mM gly; 0.5 mg/mL PS80; 50 mM NaCl; 0.1 mM EDTA; water; and pH 5.8.

I1601. 150 mg/mL secu; 0 mM buffer; 50 mM arg; 0.2 mg/mL PS20; 50 mM NaCl; 0.1 mM EDTA; water; and pH 5.8.

I1602. 190 mg/mL secu; 0 mM buffer; 50 mM arg; 0.2 mg/mL PS20; 50 mM NaCl; 0.1 mM EDTA; water; and pH 5.8.

I1603. 150 mg/mL secu; 50 mM His; 50 mM arg; 0.2 mg/mL PS20; 50 mM NaCl; 0.1 mM EDTA; water; and pH 5.8.

I1604. 190 mg/mL secu; 50 mM His; 50 mM arg; 0.2 mg/mL PS20; 50 mM NaCl; 0.1 mM EDTA; water; and pH 5.8.

I1605. 150 mg/mL secu; 25 mM Lact; 50 mM arg; 0.2 mg/mL PS20; 50 mM NaCl; 0.1 mM EDTA; water; and pH 5.8.

(continued)

I1606. 190 mg/mL secu; 25 mM Lact; 50 mM arg; 0.2 mg/mL PS20; 50 mM NaCl; 0.1 mM EDTA; water; and pH 5.8.

I1607. 150 mg/mL secu; 10 mM Phos-Lact; 50 mM arg; 0.2 mg/mL PS20; 50 mM NaCl; 0.1 mM EDTA; water; and pH 5.8.

I1608. 190 mg/mL secu; 10 mM Phos-Lact; 50 mM arg; 0.2 mg/mL PS20; 50 mM NaCl; 0.1 mM EDTA; water; and pH 5.8.

I1609. 150 mg/mL secu; 15 mM Gluc; 50 mM arg; 0.2 mg/mL PS20; 50 mM NaCl; 0.1 mM EDTA; water; and pH 5.8.

I1610. 190 mg/mL secu; 15 mM Gluc; 50 mM arg; 0.2 mg/mL PS20; 50 mM NaCl; 0.1 mM EDTA; water; and pH 5.8.

I1611. 150 mg/mL secu; 0 mM buffer; 100 mM gly; 0.2 mg/mL PS20; 50 mM NaCl; 0.1 mM EDTA; water; and pH 5.8.

I1612. 190 mg/mL secu; 0 mM buffer; 100 mM gly; 0.2 mg/mL PS20; 50 mM NaCl; 0.1 mM EDTA; water; and pH 5.8.

I1613. 150 mg/mL secu; 50 mM His; 100 mM gly; 0.2 mg/mL PS20; 50 mM NaCl; 0.1 mM EDTA; water; and pH 5.8.

I1614. 190 mg/mL secu; 50 mM His; 100 mM gly; 0.2 mg/mL PS20; 50 mM NaCl; 0.1 mM EDTA; water; and pH 5.8.

I1615. 150 mg/mL secu; 25 mM Lact; 100 mM gly; 0.2 mg/mL PS20; 50 mM NaCl; 0.1 mM EDTA; water; and pH 5.8.

I1616. 190 mg/mL secu; 25 mM Lact; 100 mM gly; 0.2 mg/mL PS20; 50 mM NaCl; 0.1 mM EDTA; water; and pH 5.8.

I1617. 150 mg/mL secu; 10 mM Phos-Lact; 100 mM gly; 0.2 mg/mL PS20; 50 mM NaCl; 0.1 mM EDTA; water; and pH 5.8.

I1618. 190 mg/mL secu; 10 mM Phos-Lact; 100 mM gly; 0.2 mg/mL PS20; 50 mM NaCl; 0.1 mM EDTA; water; and pH 5.8.

I1619. 150 mg/mL secu; 15 mM Gluc; 100 mM gly; 0.2 mg/mL PS20; 50 mM NaCl; 0.1 mM EDTA; water; and pH 5.8.

I1620. 190 mg/mL secu; 15 mM Gluc; 100 mM gly; 0.2 mg/mL PS20; 50 mM NaCl; 0.1 mM EDTA; water; and pH 5.8.

I1621. 150 mg/mL secu; 0 mM buffer; 0.5 mg/mL PS80; 1 mM met; 50 mM NaCl; 0.1 mM EDTA; water; and pH 5.8.

I1622. 190 mg/mL secu; 0 mM buffer; 0.5 mg/mL PS80; 1 mM met; 50 mM NaCl; 0.1 mM EDTA; water; and pH 5.8.

I1623. 150 mg/mL secu; 50 mM His; 0.5 mg/mL PS80; 1 mM met; 50 mM NaCl; 0.1 mM EDTA; water; and pH 5.8.

I1624. 190 mg/mL secu; 50 mM His; 0.5 mg/mL PS80; 1 mM met; 50 mM NaCl; 0.1 mM EDTA; water; and pH 5.8.

I1625. 150 mg/mL secu; 25 mM Lact; 0.5 mg/mL PS80; 1 mM met; 50 mM NaCl; 0.1 mM EDTA; water; and pH 5.8.

I1626. 190 mg/mL secu; 25 mM Lact; 0.5 mg/mL PS80; 1 mM met; 50 mM NaCl; 0.1 mM EDTA; water; and pH 5.8.

I1627. 150 mg/mL secu; 10 mM Phos-Lact; 0.5 mg/mL PS80; 1 mM met; 50 mM NaCl; 0.1 mM EDTA; water; and pH 5.8.

I1628. 190 mg/mL secu; 10 mM Phos-Lact; 0.5 mg/mL PS80; 1 mM met; 50 mM NaCl; 0.1 mM EDTA; water; and pH 5.8.

I1629. 150 mg/mL secu; 15 mM Gluc; 0.5 mg/mL PS80; 1 mM met; 50 mM NaCl; 0.1 mM EDTA; water; and pH 5.8.

I1630. 190 mg/mL secu; 15 mM Gluc; 0.5 mg/mL PS80; 1 mM met; 50 mM NaCl; 0.1 mM EDTA; water; and pH 5.8.

I1631. 150 mg/mL secu; 0 mM buffer; 0.2 mg/mL PS20; 1 mM met; 50 mM NaCl; 0.1 mM EDTA; water; and pH 5.8.

I1632. 190 mg/mL secu; 0 mM buffer; 0.2 mg/mL PS20; 1 mM met; 50 mM NaCl; 0.1 mM EDTA; water; and pH 5.8.

I1633. 150 mg/mL secu; 50 mM His; 0.2 mg/mL PS20; 1 mM met; 50 mM NaCl; 0.1 mM EDTA; water; and pH 5.8.

(continued)

I1634. 190 mg/mL secu; 50 mM His; 0.2 mg/mL PS20; 1 mM met; 50 mM NaCl; 0.1 mM EDTA; water; and pH 5.8.

I1635. 150 mg/mL secu; 25 mM Lact; 0.2 mg/mL PS20; 1 mM met; 50 mM NaCl; 0.1 mM EDTA; water; and pH 5.8.

I1636. 190 mg/mL secu; 25 mM Lact; 0.2 mg/mL PS20; 1 mM met; 50 mM NaCl; 0.1 mM EDTA; water; and pH 5.8.

I1637. 150 mg/mL secu; 10 mM Phos-Lact; 0.2 mg/mL PS20; 1 mM met; 50 mM NaCl; 0.1 mM EDTA; water; and pH 5.8.

I1638. 190 mg/mL secu; 10 mM Phos-Lact; 0.2 mg/mL PS20; 1 mM met; 50 mM NaCl; 0.1 mM EDTA; water; and pH 5.8.

I1639. 150 mg/mL secu; 15 mM Gluc; 0.2 mg/mL PS20; 1 mM met; 50 mM NaCl; 0.1 mM EDTA; water; and pH 5.8.

I1640. 190 mg/mL secu; 15 mM Gluc; 0.2 mg/mL PS20; 1 mM met; 50 mM NaCl; 0.1 mM EDTA; water; and pH 5.8.

I1641. 150 mg/mL secu; 0 mM buffer; 150 mM sorb; 50 mM arg; 0.5 mg/mL PS80; 1 mM met; 50 mM NaCl; water; and pH 5.8.

I1642. 190 mg/mL secu; 0 mM buffer; 150 mM sorb; 50 mM arg; 0.5 mg/mL PS80; 1 mM met; 50 mM NaCl; water; and pH 5.8.

I1643. 150 mg/mL secu; 50 mM His; 150 mM sorb; 50 mM arg; 0.5 mg/mL PS80; 1 mM met; 50 mM NaCl; water; and pH 5.8.

I1644. 190 mg/mL secu; 50 mM His; 150 mM sorb; 50 mM arg; 0.5 mg/mL PS80; 1 mM met; 50 mM NaCl; water; and pH 5.8.

I1645. 150 mg/mL secu; 25 mM Lact; 150 mM sorb; 50 mM arg; 0.5 mg/mL PS80; 1 mM met; 50 mM NaCl; water; and pH 5.8.

I1646. 190 mg/mL secu; 25 mM Lact; 150 mM sorb; 50 mM arg; 0.5 mg/mL PS80; 1 mM met; 50 mM NaCl; water; and pH 5.8.

I1647. 150 mg/mL secu; 10 mM Phos-Lact; 150 mM sorb; 50 mM arg; 0.5 mg/mL PS80; 1 mM met; 50 mM NaCl; water; and pH 5.8.

I1648. 190 mg/mL secu; 10 mM Phos-Lact; 150 mM sorb; 50 mM arg; 0.5 mg/mL PS80; 1 mM met; 50 mM NaCl; water; and pH 5.8.

I1649. 150 mg/mL secu; 15 mM Gluc; 150 mM sorb; 50 mM arg; 0.5 mg/mL PS80; 1 mM met; 50 mM NaCl; water; and pH 5.8.

I1650. 190 mg/mL secu; 15 mM Gluc; 150 mM sorb; 50 mM arg; 0.5 mg/mL PS80; 1 mM met; 50 mM NaCl; water; and pH 5.8.

I1651. 150 mg/mL secu; 0 mM buffer; 75 mM mann; 50 mM arg; 0.5 mg/mL PS80; 1 mM met; 50 mM NaCl; water; and pH 5.8.

I1652. 190 mg/mL secu; 0 mM buffer; 75 mM mann; 50 mM arg; 0.5 mg/mL PS80; 1 mM met; 50 mM NaCl; water; and pH 5.8.

I1653. 150 mg/mL secu; 50 mM His; 75 mM mann; 50 mM arg; 0.5 mg/mL PS80; 1 mM met; 50 mM NaCl; water; and pH 5.8.

I1654. 190 mg/mL secu; 50 mM His; 75 mM mann; 50 mM arg; 0.5 mg/mL PS80; 1 mM met; 50 mM NaCl; water; and pH 5.8.

I1655. 150 mg/mL secu; 25 mM Lact; 75 mM mann; 50 mM arg; 0.5 mg/mL PS80; 1 mM met; 50 mM NaCl; water; and pH 5.8.

I1656. 190 mg/mL secu; 25 mM Lact; 75 mM mann; 50 mM arg; 0.5 mg/mL PS80; 1 mM met; 50 mM NaCl; water; and pH 5.8.

I1657. 150 mg/mL secu; 10 mM Phos-Lact; 75 mM mann; 50 mM arg; 0.5 mg/mL PS80; 1 mM met; 50 mM NaCl; water; and pH 5.8.

I1658. 190 mg/mL secu; 10 mM Phos-Lact; 75 mM mann; 50 mM arg; 0.5 mg/mL PS80; 1 mM met; 50 mM NaCl; water; and pH 5.8.

I1659. 150 mg/mL secu; 15 mM Gluc; 75 mM mann; 50 mM arg; 0.5 mg/mL PS80; 1 mM met; 50 mM NaCl; water; and pH 5.8.

I1660. 190 mg/mL secu; 15 mM Gluc; 75 mM mann; 50 mM arg; 0.5 mg/mL PS80; 1 mM met; 50 mM NaCl; water; and pH 5.8.

(continued)

I1661. 150 mg/mL secu; 0 mM buffer; 100 mM sucr; 50 mM arg; 0.5 mg/mL PS80; 1 mM met; 50 mM NaCl; water; and pH 5.8.

I1662. 190 mg/mL secu; 0 mM buffer; 100 mM sucr; 50 mM arg; 0.5 mg/mL PS80; 1 mM met; 50 mM NaCl; water; and pH 5.8.

I1663. 150 mg/mL secu; 50 mM His; 100 mM sucr; 50 mM arg; 0.5 mg/mL PS80; 1 mM met; 50 mM NaCl; water; and pH 5.8.

I1664. 190 mg/mL secu; 50 mM His; 100 mM sucr; 50 mM arg; 0.5 mg/mL PS80; 1 mM met; 50 mM NaCl; water; and pH 5.8.

I1665. 150 mg/mL secu; 25 mM Lact; 100 mM sucr; 50 mM arg; 0.5 mg/mL PS80; 1 mM met; 50 mM NaCl; water; and pH 5.8.

I1666. 190 mg/mL secu; 25 mM Lact; 100 mM sucr; 50 mM arg; 0.5 mg/mL PS80; 1 mM met; 50 mM NaCl; water; and pH 5.8.

I1667. 150 mg/mL secu; 10 mM Phos-Lact; 100 mM sucr; 50 mM arg; 0.5 mg/mL PS80; 1 mM met; 50 mM NaCl; water; and pH 5.8.

I1668. 190 mg/mL secu; 10 mM Phos-Lact; 100 mM sucr; 50 mM arg; 0.5 mg/mL PS80; 1 mM met; 50 mM NaCl; water; and pH 5.8.

I1669. 150 mg/mL secu; 15 mM Gluc; 100 mM sucr; 50 mM arg; 0.5 mg/mL PS80; 1 mM met; 50 mM NaCl; water; and pH 5.8.

I1670. 190 mg/mL secu; 15 mM Gluc; 100 mM sucr; 50 mM arg; 0.5 mg/mL PS80; 1 mM met; 50 mM NaCl; water; and pH 5.8.

I1671. 150 mg/mL secu; 0 mM buffer; 200 mM treh; 50 mM arg; 0.5 mg/mL PS80; 1 mM met; 50 mM NaCl; water; and pH 5.8.

I1672. 190 mg/mL secu; 0 mM buffer; 200 mM treh; 50 mM arg; 0.5 mg/mL PS80; 1 mM met; 50 mM NaCl; water; and pH 5.8.

I1673. 150 mg/mL secu; 50 mM His; 200 mM treh; 50 mM arg; 0.5 mg/mL PS80; 1 mM met; 50 mM NaCl; water; and pH 5.8.

I1674. 190 mg/mL secu; 50 mM His; 200 mM treh; 50 mM arg; 0.5 mg/mL PS80; 1 mM met; 50 mM NaCl; water; and pH 5.8.

I1675. 150 mg/mL secu; 25 mM Lact; 200 mM treh; 50 mM arg; 0.5 mg/mL PS80; 1 mM met; 50 mM NaCl; water; and pH 5.8.

I1676. 190 mg/mL secu; 25 mM Lact; 200 mM treh; 50 mM arg; 0.5 mg/mL PS80; 1 mM met; 50 mM NaCl; water; and pH 5.8.

I1677. 150 mg/mL secu; 10 mM Phos-Lact; 200 mM treh; 50 mM arg; 0.5 mg/mL PS80; 1 mM met; 50 mM NaCl; water; and pH 5.8.

I1678. 190 mg/mL secu; 10 mM Phos-Lact; 200 mM treh; 50 mM arg; 0.5 mg/mL PS80; 1 mM met; 50 mM NaCl; water; and pH 5.8.

I1679. 150 mg/mL secu; 15 mM Gluc; 200 mM treh; 50 mM arg; 0.5 mg/mL PS80; 1 mM met; 50 mM NaCl; water; and pH 5.8.

I1680. 190 mg/mL secu; 15 mM Gluc; 200 mM treh; 50 mM arg; 0.5 mg/mL PS80; 1 mM met; 50 mM NaCl; water; and pH 5.8.

I1681. 150 mg/mL secu; 0 mM buffer; 150 mM sorb; 100 mM gly; 0.5 mg/mL PS80; 1 mM met; 50 mM NaCl; water; and pH 5.8.

I1682. 190 mg/mL secu; 0 mM buffer; 150 mM sorb; 100 mM gly; 0.5 mg/mL PS80; 1 mM met; 50 mM NaCl; water; and pH 5.8.

I1683. 150 mg/mL secu; 50 mM His; 150 mM sorb; 100 mM gly; 0.5 mg/mL PS80; 1 mM met; 50 mM NaCl; water; and pH 5.8.

I1684. 190 mg/mL secu; 50 mM His; 150 mM sorb; 100 mM gly; 0.5 mg/mL PS80; 1 mM met; 50 mM NaCl; water; and pH 5.8.

I1685. 150 mg/mL secu; 25 mM Lact; 150 mM sorb; 100 mM gly; 0.5 mg/mL PS80; 1 mM met; 50 mM NaCl; water; and pH 5.8.

I1686. 190 mg/mL secu; 25 mM Lact; 150 mM sorb; 100 mM gly; 0.5 mg/mL PS80; 1 mM met; 50 mM NaCl; water; and pH 5.8.

(continued)

I1687. 150 mg/mL secu; 10 mM Phos-Lact; 150 mM sorb; 100 mM gly; 0.5 mg/mL PS80; 1 mM met; 50 mM NaCl; water; and pH 5.8.

I1688. 190 mg/mL secu; 10 mM Phos-Lact; 150 mM sorb; 100 mM gly; 0.5 mg/mL PS80; 1 mM met; 50 mM NaCl; water; and pH 5.8.

I1689. 150 mg/mL secu; 15 mM Gluc; 150 mM sorb; 100 mM gly; 0.5 mg/mL PS80; 1 mM met; 50 mM NaCl; water; and pH 5.8.

I1690. 190 mg/mL secu; 15 mM Gluc; 150 mM sorb; 100 mM gly; 0.5 mg/mL PS80; 1 mM met; 50 mM NaCl; water; and pH 5.8.

I1691. 150 mg/mL secu; 0 mM buffer; 75 mM mann; 100 mM gly; 0.5 mg/mL PS80; 1 mM met; 50 mM NaCl; water; and pH 5.8.

I1692. 190 mg/mL secu; 0 mM buffer; 75 mM mann; 100 mM gly; 0.5 mg/mL PS80; 1 mM met; 50 mM NaCl; water; and pH 5.8.

I1693. 150 mg/mL secu; 50 mM His; 75 mM mann; 100 mM gly; 0.5 mg/mL PS80; 1 mM met; 50 mM NaCl; water; and pH 5.8.

I1694. 190 mg/mL secu; 50 mM His; 75 mM mann; 100 mM gly; 0.5 mg/mL PS80; 1 mM met; 50 mM NaCl; water; and pH 5.8.

I1695. 150 mg/mL secu; 25 mM Lact; 75 mM mann; 100 mM gly; 0.5 mg/mL PS80; 1 mM met; 50 mM NaCl; water; and pH 5.8.

I1696. 190 mg/mL secu; 25 mM Lact; 75 mM mann; 100 mM gly; 0.5 mg/mL PS80; 1 mM met; 50 mM NaCl; water; and pH 5.8.

I1697. 150 mg/mL secu; 10 mM Phos-Lact; 75 mM mann; 100 mM gly; 0.5 mg/mL PS80; 1 mM met; 50 mM NaCl; water; and pH 5.8.

I1698. 190 mg/mL secu; 10 mM Phos-Lact; 75 mM mann; 100 mM gly; 0.5 mg/mL PS80; 1 mM met; 50 mM NaCl; water; and pH 5.8.

I1699. 150 mg/mL secu; 15 mM Gluc; 75 mM mann; 100 mM gly; 0.5 mg/mL PS80; 1 mM met; 50 mM NaCl; water; and pH 5.8.

I1700. 190 mg/mL secu; 15 mM Gluc; 75 mM mann; 100 mM gly; 0.5 mg/mL PS80; 1 mM met; 50 mM NaCl; water; and pH 5.8.

I1701. 150 mg/mL secu; 0 mM buffer; 100 mM sucr; 100 mM gly; 0.5 mg/mL PS80; 1 mM met; 50 mM NaCl; water; and pH 5.8.

I1702. 190 mg/mL secu; 0 mM buffer; 100 mM sucr; 100 mM gly; 0.5 mg/mL PS80; 1 mM met; 50 mM NaCl; water; and pH 5.8.

I1703. 150 mg/mL secu; 50 mM His; 100 mM sucr; 100 mM gly; 0.5 mg/mL PS80; 1 mM met; 50 mM NaCl; water; and pH 5.8.

I1704. 190 mg/mL secu; 50 mM His; 100 mM sucr; 100 mM gly; 0.5 mg/mL PS80; 1 mM met; 50 mM NaCl; water; and pH 5.8.

I1705. 150 mg/mL secu; 25 mM Lact; 100 mM sucr; 100 mM gly; 0.5 mg/mL PS80; 1 mM met; 50 mM NaCl; water; and pH 5.8.

I1706. 190 mg/mL secu; 25 mM Lact; 100 mM sucr; 100 mM gly; 0.5 mg/mL PS80; 1 mM met; 50 mM NaCl; water; and pH 5.8.

I1707. 150 mg/mL secu; 10 mM Phos-Lact; 100 mM sucr; 100 mM gly; 0.5 mg/mL PS80; 1 mM met; 50 mM NaCl; water; and pH 5.8.

I1708. 190 mg/mL secu; 10 mM Phos-Lact; 100 mM sucr; 100 mM gly; 0.5 mg/mL PS80; 1 mM met; 50 mM NaCl; water; and pH 5.8.

I1709. 150 mg/mL secu; 15 mM Gluc; 100 mM sucr; 100 mM gly; 0.5 mg/mL PS80; 1 mM met; 50 mM NaCl; water; and pH 5.8.

I1710. 190 mg/mL secu; 15 mM Gluc; 100 mM sucr; 100 mM gly; 0.5 mg/mL PS80; 1 mM met; 50 mM NaCl; water; and pH 5.8.

I1711. 150 mg/mL secu; 0 mM buffer; 200 mM treh; 100 mM gly; 0.5 mg/mL PS80; 1 mM met; 50 mM NaCl; water; and pH 5.8.

I1712. 190 mg/mL secu; 0 mM buffer; 200 mM treh; 100 mM gly; 0.5 mg/mL PS80; 1 mM met; 50 mM NaCl; water; and pH 5.8.

(continued)

I1713. 150 mg/mL secu; 50 mM His; 200 mM treh; 100 mM gly; 0.5 mg/mL PS80; 1 mM met; 50 mM NaCl; water; and pH 5.8.

I1714. 190 mg/mL secu; 50 mM His; 200 mM treh; 100 mM gly; 0.5 mg/mL PS80; 1 mM met; 50 mM NaCl; water; and pH 5.8.

I1715. 150 mg/mL secu; 25 mM Lact; 200 mM treh; 100 mM gly; 0.5 mg/mL PS80; 1 mM met; 50 mM NaCl; water; and pH 5.8.

I1716. 190 mg/mL secu; 25 mM Lact; 200 mM treh; 100 mM gly; 0.5 mg/mL PS80; 1 mM met; 50 mM NaCl; water; and pH 5.8.

I1717. 150 mg/mL secu; 10 mM Phos-Lact; 200 mM treh; 100 mM gly; 0.5 mg/mL PS80; 1 mM met; 50 mM NaCl; water; and pH 5.8.

I1718. 190 mg/mL secu; 10 mM Phos-Lact; 200 mM treh; 100 mM gly; 0.5 mg/mL PS80; 1 mM met; 50 mM NaCl; water; and pH 5.8.

I1719. 150 mg/mL secu; 15 mM Gluc; 200 mM treh; 100 mM gly; 0.5 mg/mL PS80; 1 mM met; 50 mM NaCl; water; and pH 5.8.

I1720. 190 mg/mL secu; 15 mM Gluc; 200 mM treh; 100 mM gly; 0.5 mg/mL PS80; 1 mM met; 50 mM NaCl; water; and pH 5.8.

I1721. 150 mg/mL secu; 0 mM buffer; 150 mM sorb; 50 mM arg; 0.2 mg/mL PS20; 1 mM met; 50 mM NaCl; water; and pH 5.8.

I1722. 190 mg/mL secu; 0 mM buffer; 150 mM sorb; 50 mM arg; 0.2 mg/mL PS20; 1 mM met; 50 mM NaCl; water; and pH 5.8.

I1723. 150 mg/mL secu; 50 mM His; 150 mM sorb; 50 mM arg; 0.2 mg/mL PS20; 1 mM met; 50 mM NaCl; water; and pH 5.8.

I1724. 190 mg/mL secu; 50 mM His; 150 mM sorb; 50 mM arg; 0.2 mg/mL PS20; 1 mM met; 50 mM NaCl; water; and pH 5.8.

I1725. 150 mg/mL secu; 25 mM Lact; 150 mM sorb; 50 mM arg; 0.2 mg/mL PS20; 1 mM met; 50 mM NaCl; water; and pH 5.8.

I1726. 190 mg/mL secu; 25 mM Lact; 150 mM sorb; 50 mM arg; 0.2 mg/mL PS20; 1 mM met; 50 mM NaCl; water; and pH 5.8.

I1727. 150 mg/mL secu; 10 mM Phos-Lact; 150 mM sorb; 50 mM arg; 0.2 mg/mL PS20; 1 mM met; 50 mM NaCl; water; and pH 5.8.

I1728. 190 mg/mL secu; 10 mM Phos-Lact; 150 mM sorb; 50 mM arg; 0.2 mg/mL PS20; 1 mM met; 50 mM NaCl; water; and pH 5.8.

I1729. 150 mg/mL secu; 15 mM Gluc; 150 mM sorb; 50 mM arg; 0.2 mg/mL PS20; 1 mM met; 50 mM NaCl; water; and pH 5.8.

I1730. 190 mg/mL secu; 15 mM Gluc; 150 mM sorb; 50 mM arg; 0.2 mg/mL PS20; 1 mM met; 50 mM NaCl; water; and pH 5.8.

I1731. 150 mg/mL secu; 0 mM buffer; 75 mM mann; 50 mM arg; 0.2 mg/mL PS20; 1 mM met; 50 mM NaCl; water; and pH 5.8.

I1732. 190 mg/mL secu; 0 mM buffer; 75 mM mann; 50 mM arg; 0.2 mg/mL PS20; 1 mM met; 50 mM NaCl; water; and pH 5.8.

I1733. 150 mg/mL secu; 50 mM His; 75 mM mann; 50 mM arg; 0.2 mg/mL PS20; 1 mM met; 50 mM NaCl; water; and pH 5.8.

I1734. 190 mg/mL secu; 50 mM His; 75 mM mann; 50 mM arg; 0.2 mg/mL PS20; 1 mM met; 50 mM NaCl; water; and pH 5.8.

I1735. 150 mg/mL secu; 25 mM Lact; 75 mM mann; 50 mM arg; 0.2 mg/mL PS20; 1 mM met; 50 mM NaCl; water; and pH 5.8.

I1736. 190 mg/mL secu; 25 mM Lact; 75 mM mann; 50 mM arg; 0.2 mg/mL PS20; 1 mM met; 50 mM NaCl; water; and pH 5.8.

I1737. 150 mg/mL secu; 10 mM Phos-Lact; 75 mM mann; 50 mM arg; 0.2 mg/mL PS20; 1 mM met; 50 mM NaCl; water; and pH 5.8.

I1738. 190 mg/mL secu; 10 mM Phos-Lact; 75 mM mann; 50 mM arg; 0.2 mg/mL PS20; 1 mM met; 50 mM NaCl; water; and pH 5.8.

(continued)

I1739. 150 mg/mL secu; 15 mM Gluc; 75 mM mann; 50 mM arg; 0.2 mg/mL PS20; 1 mM met; 50 mM NaCl; water; and pH 5.8.

I1740. 190 mg/mL secu; 15 mM Gluc; 75 mM mann; 50 mM arg; 0.2 mg/mL PS20; 1 mM met; 50 mM NaCl; water; and pH 5.8.

I1741. 150 mg/mL secu; 0 mM buffer; 100 mM sucr; 50 mM arg; 0.2 mg/mL PS20; 1 mM met; 50 mM NaCl; water; and pH 5.8.

I1742. 190 mg/mL secu; 0 mM buffer; 100 mM sucr; 50 mM arg; 0.2 mg/mL PS20; 1 mM met; 50 mM NaCl; water; and pH 5.8.

I1743. 150 mg/mL secu; 50 mM His; 100 mM sucr; 50 mM arg; 0.2 mg/mL PS20; 1 mM met; 50 mM NaCl; water; and pH 5.8.

I1744. 190 mg/mL secu; 50 mM His; 100 mM sucr; 50 mM arg; 0.2 mg/mL PS20; 1 mM met; 50 mM NaCl; water; and pH 5.8.

I1745. 150 mg/mL secu; 25 mM Lact; 100 mM sucr; 50 mM arg; 0.2 mg/mL PS20; 1 mM met; 50 mM NaCl; water; and pH 5.8.

I1746. 190 mg/mL secu; 25 mM Lact; 100 mM sucr; 50 mM arg; 0.2 mg/mL PS20; 1 mM met; 50 mM NaCl; water; and pH 5.8.

I1747. 150 mg/mL secu; 10 mM Phos-Lact; 100 mM sucr; 50 mM arg; 0.2 mg/mL PS20; 1 mM met; 50 mM NaCl; water; and pH 5.8.

I1748. 190 mg/mL secu; 10 mM Phos-Lact; 100 mM sucr; 50 mM arg; 0.2 mg/mL PS20; 1 mM met; 50 mM NaCl; water; and pH 5.8.

I1749. 150 mg/mL secu; 15 mM Gluc; 100 mM sucr; 50 mM arg; 0.2 mg/mL PS20; 1 mM met; 50 mM NaCl; water; and pH 5.8.

I1750. 190 mg/mL secu; 15 mM Gluc; 100 mM sucr; 50 mM arg; 0.2 mg/mL PS20; 1 mM met; 50 mM NaCl; water; and pH 5.8.

I1751. 150 mg/mL secu; 0 mM buffer; 200 mM treh; 50 mM arg; 0.2 mg/mL PS20; 1 mM met; 50 mM NaCl; water; and pH 5.8.

I1752. 190 mg/mL secu; 0 mM buffer; 200 mM treh; 50 mM arg; 0.2 mg/mL PS20; 1 mM met; 50 mM NaCl; water; and pH 5.8.

I1753. 150 mg/mL secu; 50 mM His; 200 mM treh; 50 mM arg; 0.2 mg/mL PS20; 1 mM met; 50 mM NaCl; water; and pH 5.8.

I1754. 190 mg/mL secu; 50 mM His; 200 mM treh; 50 mM arg; 0.2 mg/mL PS20; 1 mM met; 50 mM NaCl; water; and pH 5.8.

I1755. 150 mg/mL secu; 25 mM Lact; 200 mM treh; 50 mM arg; 0.2 mg/mL PS20; 1 mM met; 50 mM NaCl; water; and pH 5.8.

I1756. 190 mg/mL secu; 25 mM Lact; 200 mM treh; 50 mM arg; 0.2 mg/mL PS20; 1 mM met; 50 mM NaCl; water; and pH 5.8.

I1757. 150 mg/mL secu; 10 mM Phos-Lact; 200 mM treh; 50 mM arg; 0.2 mg/mL PS20; 1 mM met; 50 mM NaCl; water; and pH 5.8.

I1758. 190 mg/mL secu; 10 mM Phos-Lact; 200 mM treh; 50 mM arg; 0.2 mg/mL PS20; 1 mM met; 50 mM NaCl; water; and pH 5.8.

I1759. 150 mg/mL secu; 15 mM Gluc; 200 mM treh; 50 mM arg; 0.2 mg/mL PS20; 1 mM met; 50 mM NaCl; water; and pH 5.8.

I1760. 190 mg/mL secu; 15 mM Gluc; 200 mM treh; 50 mM arg; 0.2 mg/mL PS20; 1 mM met; 50 mM NaCl; water; and pH 5.8.

I1761. 150 mg/mL secu; 0 mM buffer; 150 mM sorb; 100 mM gly; 0.2 mg/mL PS20; 1 mM met; 50 mM NaCl; water; and pH 5.8.

I1762. 190 mg/mL secu; 0 mM buffer; 150 mM sorb; 100 mM gly; 0.2 mg/mL PS20; 1 mM met; 50 mM NaCl; water; and pH 5.8.

I1763. 150 mg/mL secu; 50 mM His; 150 mM sorb; 100 mM gly; 0.2 mg/mL PS20; 1 mM met; 50 mM NaCl; water; and pH 5.8.

I1764. 190 mg/mL secu; 50 mM His; 150 mM sorb; 100 mM gly; 0.2 mg/mL PS20; 1 mM met; 50 mM NaCl; water; and pH 5.8.

(continued)

I1765. 150 mg/mL secu; 25 mM Lact; 150 mM sorb; 100 mM gly; 0.2 mg/mL PS20; 1 mM met; 50 mM NaCl; water; and pH 5.8.

I1766. 190 mg/mL secu; 25 mM Lact; 150 mM sorb; 100 mM gly; 0.2 mg/mL PS20; 1 mM met; 50 mM NaCl; water; and pH 5.8.

I1767. 150 mg/mL secu; 10 mM Phos-Lact; 150 mM sorb; 100 mM gly; 0.2 mg/mL PS20; 1 mM met; 50 mM NaCl; water; and pH 5.8.

I1768. 190 mg/mL secu; 10 mM Phos-Lact; 150 mM sorb; 100 mM gly; 0.2 mg/mL PS20; 1 mM met; 50 mM NaCl; water; and pH 5.8.

I1769. 150 mg/mL secu; 15 mM Gluc; 150 mM sorb; 100 mM gly; 0.2 mg/mL PS20; 1 mM met; 50 mM NaCl; water; and pH 5.8.

I1770. 190 mg/mL secu; 15 mM Gluc; 150 mM sorb; 100 mM gly; 0.2 mg/mL PS20; 1 mM met; 50 mM NaCl; water; and pH 5.8.

I1771. 150 mg/mL secu; 0 mM buffer; 75 mM mann; 100 mM gly; 0.2 mg/mL PS20; 1 mM met; 50 mM NaCl; water; and pH 5.8.

I1772. 190 mg/mL secu; 0 mM buffer; 75 mM mann; 100 mM gly; 0.2 mg/mL PS20; 1 mM met; 50 mM NaCl; water; and pH 5.8.

I1773. 150 mg/mL secu; 50 mM His; 75 mM mann; 100 mM gly; 0.2 mg/mL PS20; 1 mM met; 50 mM NaCl; water; and pH 5.8.

I1774. 190 mg/mL secu; 50 mM His; 75 mM mann; 100 mM gly; 0.2 mg/mL PS20; 1 mM met; 50 mM NaCl; water; and pH 5.8.

I1775. 150 mg/mL secu; 25 mM Lact; 75 mM mann; 100 mM gly; 0.2 mg/mL PS20; 1 mM met; 50 mM NaCl; water; and pH 5.8.

I1776. 190 mg/mL secu; 25 mM Lact; 75 mM mann; 100 mM gly; 0.2 mg/mL PS20; 1 mM met; 50 mM NaCl; water; and pH 5.8.

I1777. 150 mg/mL secu; 10 mM Phos-Lact; 75 mM mann; 100 mM gly; 0.2 mg/mL PS20; 1 mM met; 50 mM NaCl; water; and pH 5.8.

I1778. 190 mg/mL secu; 10 mM Phos-Lact; 75 mM mann; 100 mM gly; 0.2 mg/mL PS20; 1 mM met; 50 mM NaCl; water; and pH 5.8.

I1779. 150 mg/mL secu; 15 mM Gluc; 75 mM mann; 100 mM gly; 0.2 mg/mL PS20; 1 mM met; 50 mM NaCl; water; and pH 5.8.

I1780. 190 mg/mL secu; 15 mM Gluc; 75 mM mann; 100 mM gly; 0.2 mg/mL PS20; 1 mM met; 50 mM NaCl; water; and pH 5.8.

I1781. 150 mg/mL secu; 0 mM buffer; 100 mM sucr; 100 mM gly; 0.2 mg/mL PS20; 1 mM met; 50 mM NaCl; water; and pH 5.8.

I1782. 190 mg/mL secu; 0 mM buffer; 100 mM sucr; 100 mM gly; 0.2 mg/mL PS20; 1 mM met; 50 mM NaCl; water; and pH 5.8.

I1783. 150 mg/mL secu; 50 mM His; 100 mM sucr; 100 mM gly; 0.2 mg/mL PS20; 1 mM met; 50 mM NaCl; water; and pH 5.8.

I1784. 190 mg/mL secu; 50 mM His; 100 mM sucr; 100 mM gly; 0.2 mg/mL PS20; 1 mM met; 50 mM NaCl; water; and pH 5.8.

I1785. 150 mg/mL secu; 25 mM Lact; 100 mM sucr; 100 mM gly; 0.2 mg/mL PS20; 1 mM met; 50 mM NaCl; water; and pH 5.8.

I1786. 190 mg/mL secu; 25 mM Lact; 100 mM sucr; 100 mM gly; 0.2 mg/mL PS20; 1 mM met; 50 mM NaCl; water; and pH 5.8.

I1787. 150 mg/mL secu; 10 mM Phos-Lact; 100 mM sucr; 100 mM gly; 0.2 mg/mL PS20; 1 mM met; 50 mM NaCl; water; and pH 5.8.

I1788. 190 mg/mL secu; 10 mM Phos-Lact; 100 mM sucr; 100 mM gly; 0.2 mg/mL PS20; 1 mM met; 50 mM NaCl; water; and pH 5.8.

I1789. 150 mg/mL secu; 15 mM Gluc; 100 mM sucr; 100 mM gly; 0.2 mg/mL PS20; 1 mM met; 50 mM NaCl; water; and pH 5.8.

I1790. 190 mg/mL secu; 15 mM Gluc; 100 mM sucr; 100 mM gly; 0.2 mg/mL PS20; 1 mM met; 50 mM NaCl; water; and pH 5.8.

(continued)

I1791. 150 mg/mL secu; 0 mM buffer; 200 mM treh; 100 mM gly; 0.2 mg/mL PS20; 1 mM met; 50 mM NaCl; water; and pH 5.8.

I1792. 190 mg/mL secu; 0 mM buffer; 200 mM treh; 100 mM gly; 0.2 mg/mL PS20; 1 mM met; 50 mM NaCl; water; and pH 5.8.

I1793. 150 mg/mL secu; 50 mM His; 200 mM treh; 100 mM gly; 0.2 mg/mL PS20; 1 mM met; 50 mM NaCl; water; and pH 5.8.

I1794. 190 mg/mL secu; 50 mM His; 200 mM treh; 100 mM gly; 0.2 mg/mL PS20; 1 mM met; 50 mM NaCl; water; and pH 5.8.

I1795. 150 mg/mL secu; 25 mM Lact; 200 mM treh; 100 mM gly; 0.2 mg/mL PS20; 1 mM met; 50 mM NaCl; water; and pH 5.8.

I1796. 190 mg/mL secu; 25 mM Lact; 200 mM treh; 100 mM gly; 0.2 mg/mL PS20; 1 mM met; 50 mM NaCl; water; and pH 5.8.

I1797. 150 mg/mL secu; 10 mM Phos-Lact; 200 mM treh; 100 mM gly; 0.2 mg/mL PS20; 1 mM met; 50 mM NaCl; water; and pH 5.8.

I1798. 190 mg/mL secu; 10 mM Phos-Lact; 200 mM treh; 100 mM gly; 0.2 mg/mL PS20; 1 mM met; 50 mM NaCl; water; and pH 5.8.

I1799. 150 mg/mL secu; 15 mM Gluc; 200 mM treh; 100 mM gly; 0.2 mg/mL PS20; 1 mM met; 50 mM NaCl; water; and pH 5.8.

I1800. 190 mg/mL secu; 15 mM Gluc; 200 mM treh; 100 mM gly; 0.2 mg/mL PS20; 1 mM met; 50 mM NaCl; water; and pH 5.8.

I1801. 150 mg/mL secu; 0 mM buffer; 150 mM sorb; 50 mM arg; 0.5 mg/mL PS80; 1 mM met; 0.1 mM EDTA; water; and pH 5.8.

I1802. 190 mg/mL secu; 0 mM buffer; 150 mM sorb; 50 mM arg; 0.5 mg/mL PS80; 1 mM met; 0.1 mM EDTA; water; and pH 5.8.

I1803. 150 mg/mL secu; 50 mM His; 150 mM sorb; 50 mM arg; 0.5 mg/mL PS80; 1 mM met; 0.1 mM EDTA; water; and pH 5.8.

I1804. 190 mg/mL secu; 50 mM His; 150 mM sorb; 50 mM arg; 0.5 mg/mL PS80; 1 mM met; 0.1 mM EDTA; water; and pH 5.8.

I1805. 150 mg/mL secu; 25 mM Lact; 150 mM sorb; 50 mM arg; 0.5 mg/mL PS80; 1 mM met; 0.1 mM EDTA; water; and pH 5.8.

I1806. 190 mg/mL secu; 25 mM Lact; 150 mM sorb; 50 mM arg; 0.5 mg/mL PS80; 1 mM met; 0.1 mM EDTA; water; and pH 5.8.

I1807. 150 mg/mL secu; 10 mM Phos-Lact; 150 mM sorb; 50 mM arg; 0.5 mg/mL PS80; 1 mM met; 0.1 mM EDTA; water; and pH 5.8.

I1808. 190 mg/mL secu; 10 mM Phos-Lact; 150 mM sorb; 50 mM arg; 0.5 mg/mL PS80; 1 mM met; 0.1 mM EDTA; water; and pH 5.8.

I1809. 150 mg/mL secu; 15 mM Gluc; 150 mM sorb; 50 mM arg; 0.5 mg/mL PS80; 1 mM met; 0.1 mM EDTA; water; and pH 5.8.

I1810. 190 mg/mL secu; 15 mM Gluc; 150 mM sorb; 50 mM arg; 0.5 mg/mL PS80; 1 mM met; 0.1 mM EDTA; water; and pH 5.8.

I1811. 150 mg/mL secu; 0 mM buffer; 75 mM mann; 50 mM arg; 0.5 mg/mL PS80; 1 mM met; 0.1 mM EDTA; water; and pH 5.8.

I1812. 190 mg/mL secu; 0 mM buffer; 75 mM mann; 50 mM arg; 0.5 mg/mL PS80; 1 mM met; 0.1 mM EDTA; water; and pH 5.8.

I1813. 150 mg/mL secu; 50 mM His; 75 mM mann; 50 mM arg; 0.5 mg/mL PS80; 1 mM met; 0.1 mM EDTA; water; and pH 5.8.

I1814. 190 mg/mL secu; 50 mM His; 75 mM mann; 50 mM arg; 0.5 mg/mL PS80; 1 mM met; 0.1 mM EDTA; water; and pH 5.8.

I1815. 150 mg/mL secu; 25 mM Lact; 75 mM mann; 50 mM arg; 0.5 mg/mL PS80; 1 mM met; 0.1 mM EDTA; water; and pH 5.8.

I1816. 190 mg/mL secu; 25 mM Lact; 75 mM mann; 50 mM arg; 0.5 mg/mL PS80; 1 mM met; 0.1 mM EDTA; water; and pH 5.8.

(continued)

I1817. 150 mg/mL secu; 10 mM Phos-Lact; 75 mM mann; 50 mM arg; 0.5 mg/mL PS80; 1 mM met; 0.1 mM EDTA; water; and pH 5.8.

I1818. 190 mg/mL secu; 10 mM Phos-Lact; 75 mM mann; 50 mM arg; 0.5 mg/mL PS80; 1 mM met; 0.1 mM EDTA; water; and pH 5.8.

I1819. 150 mg/mL secu; 15 mM Gluc; 75 mM mann; 50 mM arg; 0.5 mg/mL PS80; 1 mM met; 0.1 mM EDTA; water; and pH 5.8.

I1820. 190 mg/mL secu; 15 mM Gluc; 75 mM mann; 50 mM arg; 0.5 mg/mL PS80; 1 mM met; 0.1 mM EDTA; water; and pH 5.8.

I1821. 150 mg/mL secu; 0 mM buffer; 100 mM sucr; 50 mM arg; 0.5 mg/mL PS80; 1 mM met; 0.1 mM EDTA; water; and pH 5.8.

I1822. 190 mg/mL secu; 0 mM buffer; 100 mM sucr; 50 mM arg; 0.5 mg/mL PS80; 1 mM met; 0.1 mM EDTA; water; and pH 5.8.

I1823. 150 mg/mL secu; 50 mM His; 100 mM sucr; 50 mM arg; 0.5 mg/mL PS80; 1 mM met; 0.1 mM EDTA; water; and pH 5.8.

I1824. 190 mg/mL secu; 50 mM His; 100 mM sucr; 50 mM arg; 0.5 mg/mL PS80; 1 mM met; 0.1 mM EDTA; water; and pH 5.8.

I1825. 150 mg/mL secu; 25 mM Lact; 100 mM sucr; 50 mM arg; 0.5 mg/mL PS80; 1 mM met; 0.1 mM EDTA; water; and pH 5.8.

I1826. 190 mg/mL secu; 25 mM Lact; 100 mM sucr; 50 mM arg; 0.5 mg/mL PS80; 1 mM met; 0.1 mM EDTA; water; and pH 5.8.

I1827. 150 mg/mL secu; 10 mM Phos-Lact; 100 mM sucr; 50 mM arg; 0.5 mg/mL PS80; 1 mM met; 0.1 mM EDTA; water; and pH 5.8.

I1828. 190 mg/mL secu; 10 mM Phos-Lact; 100 mM sucr; 50 mM arg; 0.5 mg/mL PS80; 1 mM met; 0.1 mM EDTA; water; and pH 5.8.

I1829. 150 mg/mL secu; 15 mM Gluc; 100 mM sucr; 50 mM arg; 0.5 mg/mL PS80; 1 mM met; 0.1 mM EDTA; water; and pH 5.8.

I1830. 190 mg/mL secu; 15 mM Gluc; 100 mM sucr; 50 mM arg; 0.5 mg/mL PS80; 1 mM met; 0.1 mM EDTA; water; and pH 5.8.

I1831. 150 mg/mL secu; 0 mM buffer; 200 mM treh; 50 mM arg; 0.5 mg/mL PS80; 1 mM met; 0.1 mM EDTA; water; and pH 5.8.

I1832. 190 mg/mL secu; 0 mM buffer; 200 mM treh; 50 mM arg; 0.5 mg/mL PS80; 1 mM met; 0.1 mM EDTA; water; and pH 5.8.

I1833. 150 mg/mL secu; 50 mM His; 200 mM treh; 50 mM arg; 0.5 mg/mL PS80; 1 mM met; 0.1 mM EDTA; water; and pH 5.8.

I1834. 190 mg/mL secu; 50 mM His; 200 mM treh; 50 mM arg; 0.5 mg/mL PS80; 1 mM met; 0.1 mM EDTA; water; and pH 5.8.

I1835. 150 mg/mL secu; 25 mM Lact; 200 mM treh; 50 mM arg; 0.5 mg/mL PS80; 1 mM met; 0.1 mM EDTA; water; and pH 5.8.

I1836. 190 mg/mL secu; 25 mM Lact; 200 mM treh; 50 mM arg; 0.5 mg/mL PS80; 1 mM met; 0.1 mM EDTA; water; and pH 5.8.

I1837. 150 mg/mL secu; 10 mM Phos-Lact; 200 mM treh; 50 mM arg; 0.5 mg/mL PS80; 1 mM met; 0.1 mM EDTA; water; and pH 5.8.

I1838. 190 mg/mL secu; 10 mM Phos-Lact; 200 mM treh; 50 mM arg; 0.5 mg/mL PS80; 1 mM met; 0.1 mM EDTA; water; and pH 5.8.

I1839. 150 mg/mL secu; 15 mM Gluc; 200 mM treh; 50 mM arg; 0.5 mg/mL PS80; 1 mM met; 0.1 mM EDTA; water; and pH 5.8.

I1840. 190 mg/mL secu; 15 mM Gluc; 200 mM treh; 50 mM arg; 0.5 mg/mL PS80; 1 mM met; 0.1 mM EDTA; water; and pH 5.8.

I1841. 150 mg/mL secu; 0 mM buffer; 150 mM sorb; 100 mM gly; 0.5 mg/mL PS80; 1 mM met; 0.1 mM EDTA; water; and pH 5.8.

I1842. 190 mg/mL secu; 0 mM buffer; 150 mM sorb; 100 mM gly; 0.5 mg/mL PS80; 1 mM met; 0.1 mM EDTA; water; and pH 5.8.

(continued)

I1843. 150 mg/mL secu; 50 mM His; 150 mM sorb; 100 mM gly; 0.5 mg/mL PS80; 1 mM met; 0.1 mM EDTA; water; and pH 5.8.

I1844. 190 mg/mL secu; 50 mM His; 150 mM sorb; 100 mM gly; 0.5 mg/mL PS80; 1 mM met; 0.1 mM EDTA; water; and pH 5.8.

I1845. 150 mg/mL secu; 25 mM Lact; 150 mM sorb; 100 mM gly; 0.5 mg/mL PS80; 1 mM met; 0.1 mM EDTA; water; and pH 5.8.

I1846. 190 mg/mL secu; 25 mM Lact; 150 mM sorb; 100 mM gly; 0.5 mg/mL PS80; 1 mM met; 0.1 mM EDTA; water; and pH 5.8.

I1847. 150 mg/mL secu; 10 mM Phos-Lact; 150 mM sorb; 100 mM gly; 0.5 mg/mL PS80; 1 mM met; 0.1 mM EDTA; water; and pH 5.8.

I1848. 190 mg/mL secu; 10 mM Phos-Lact; 150 mM sorb; 100 mM gly; 0.5 mg/mL PS80; 1 mM met; 0.1 mM EDTA; water; and pH 5.8.

I1849. 150 mg/mL secu; 15 mM Gluc; 150 mM sorb; 100 mM gly; 0.5 mg/mL PS80; 1 mM met; 0.1 mM EDTA; water; and pH 5.8.

I1850. 190 mg/mL secu; 15 mM Gluc; 150 mM sorb; 100 mM gly; 0.5 mg/mL PS80; 1 mM met; 0.1 mM EDTA; water; and pH 5.8.

I1851. 150 mg/mL secu; 0 mM buffer; 75 mM mann; 100 mM gly; 0.5 mg/mL PS80; 1 mM met; 0.1 mM EDTA; water; and pH 5.8.

I1852. 190 mg/mL secu; 0 mM buffer; 75 mM mann; 100 mM gly; 0.5 mg/mL PS80; 1 mM met; 0.1 mM EDTA; water; and pH 5.8.

I1853. 150 mg/mL secu; 50 mM His; 75 mM mann; 100 mM gly; 0.5 mg/mL PS80; 1 mM met; 0.1 mM EDTA; water; and pH 5.8.

I1854. 190 mg/mL secu; 50 mM His; 75 mM mann; 100 mM gly; 0.5 mg/mL PS80; 1 mM met; 0.1 mM EDTA; water; and pH 5.8.

I1855. 150 mg/mL secu; 25 mM Lact; 75 mM mann; 100 mM gly; 0.5 mg/mL PS80; 1 mM met; 0.1 mM EDTA; water; and pH 5.8.

I1856. 190 mg/mL secu; 25 mM Lact; 75 mM mann; 100 mM gly; 0.5 mg/mL PS80; 1 mM met; 0.1 mM EDTA; water; and pH 5.8.

I1857. 150 mg/mL secu; 10 mM Phos-Lact; 75 mM mann; 100 mM gly; 0.5 mg/mL PS80; 1 mM met; 0.1 mM EDTA; water; and pH 5.8.

I1858. 190 mg/mL secu; 10 mM Phos-Lact; 75 mM mann; 100 mM gly; 0.5 mg/mL PS80; 1 mM met; 0.1 mM EDTA; water; and pH 5.8.

I1859. 150 mg/mL secu; 15 mM Gluc; 75 mM mann; 100 mM gly; 0.5 mg/mL PS80; 1 mM met; 0.1 mM EDTA; water; and pH 5.8.

I1860. 190 mg/mL secu; 15 mM Gluc; 75 mM mann; 100 mM gly; 0.5 mg/mL PS80; 1 mM met; 0.1 mM EDTA; water; and pH 5.8.

I1861. 150 mg/mL secu; 0 mM buffer; 100 mM sucr; 100 mM gly; 0.5 mg/mL PS80; 1 mM met; 0.1 mM EDTA; water; and pH 5.8.

I1862. 190 mg/mL secu; 0 mM buffer; 100 mM sucr; 100 mM gly; 0.5 mg/mL PS80; 1 mM met; 0.1 mM EDTA; water; and pH 5.8.

I1863. 150 mg/mL secu; 50 mM His; 100 mM sucr; 100 mM gly; 0.5 mg/mL PS80; 1 mM met; 0.1 mM EDTA; water; and pH 5.8.

I1864. 190 mg/mL secu; 50 mM His; 100 mM sucr; 100 mM gly; 0.5 mg/mL PS80; 1 mM met; 0.1 mM EDTA; water; and pH 5.8.

I1865. 150 mg/mL secu; 25 mM Lact; 100 mM sucr; 100 mM gly; 0.5 mg/mL PS80; 1 mM met; 0.1 mM EDTA; water; and pH 5.8.

I1866. 190 mg/mL secu; 25 mM Lact; 100 mM sucr; 100 mM gly; 0.5 mg/mL PS80; 1 mM met; 0.1 mM EDTA; water; and pH 5.8.

I1867. 150 mg/mL secu; 10 mM Phos-Lact; 100 mM sucr; 100 mM gly; 0.5 mg/mL PS80; 1 mM met; 0.1 mM EDTA; water; and pH 5.8.

I1868. 190 mg/mL secu; 10 mM Phos-Lact; 100 mM sucr; 100 mM gly; 0.5 mg/mL PS80; 1 mM met; 0.1 mM EDTA; water; and pH 5.8.

(continued)

I1869. 150 mg/mL secu; 15 mM Gluc; 100 mM sucr; 100 mM gly; 0.5 mg/mL PS80; 1 mM met; 0.1 mM EDTA; water; and pH 5.8.

I1870. 190 mg/mL secu; 15 mM Gluc; 100 mM sucr; 100 mM gly; 0.5 mg/mL PS80; 1 mM met; 0.1 mM EDTA; water; and pH 5.8.

I1871. 150 mg/mL secu; 0 mM buffer; 200 mM treh; 100 mM gly; 0.5 mg/mL PS80; 1 mM met; 0.1 mM EDTA; water; and pH 5.8.

I1872. 190 mg/mL secu; 0 mM buffer; 200 mM treh; 100 mM gly; 0.5 mg/mL PS80; 1 mM met; 0.1 mM EDTA; water; and pH 5.8.

I1873. 150 mg/mL secu; 50 mM His; 200 mM treh; 100 mM gly; 0.5 mg/mL PS80; 1 mM met; 0.1 mM EDTA; water; and pH 5.8.

I1874. 190 mg/mL secu; 50 mM His; 200 mM treh; 100 mM gly; 0.5 mg/mL PS80; 1 mM met; 0.1 mM EDTA; water; and pH 5.8.

I1875. 150 mg/mL secu; 25 mM Lact; 200 mM treh; 100 mM gly; 0.5 mg/mL PS80; 1 mM met; 0.1 mM EDTA; water; and pH 5.8.

I1876. 190 mg/mL secu; 25 mM Lact; 200 mM treh; 100 mM gly; 0.5 mg/mL PS80; 1 mM met; 0.1 mM EDTA; water; and pH 5.8.

I1877. 150 mg/mL secu; 10 mM Phos-Lact; 200 mM treh; 100 mM gly; 0.5 mg/mL PS80; 1 mM met; 0.1 mM EDTA; water; and pH 5.8.

I1878. 190 mg/mL secu; 10 mM Phos-Lact; 200 mM treh; 100 mM gly; 0.5 mg/mL PS80; 1 mM met; 0.1 mM EDTA; water; and pH 5.8.

I1879. 150 mg/mL secu; 15 mM Gluc; 200 mM treh; 100 mM gly; 0.5 mg/mL PS80; 1 mM met; 0.1 mM EDTA; water; and pH 5.8.

I1880. 190 mg/mL secu; 15 mM Gluc; 200 mM treh; 100 mM gly; 0.5 mg/mL PS80; 1 mM met; 0.1 mM EDTA; water; and pH 5.8.

I1881. 150 mg/mL secu; 0 mM buffer; 150 mM sorb; 50 mM arg; 0.2 mg/mL PS20; 1 mM met; 0.1 mM EDTA; water; and pH 5.8.

I1882. 190 mg/mL secu; 0 mM buffer; 150 mM sorb; 50 mM arg; 0.2 mg/mL PS20; 1 mM met; 0.1 mM EDTA; water; and pH 5.8.

I1883. 150 mg/mL secu; 50 mM His; 150 mM sorb; 50 mM arg; 0.2 mg/mL PS20; 1 mM met; 0.1 mM EDTA; water; and pH 5.8.

I1884. 190 mg/mL secu; 50 mM His; 150 mM sorb; 50 mM arg; 0.2 mg/mL PS20; 1 mM met; 0.1 mM EDTA; water; and pH 5.8.

I1885. 150 mg/mL secu; 25 mM Lact; 150 mM sorb; 50 mM arg; 0.2 mg/mL PS20; 1 mM met; 0.1 mM EDTA; water; and pH 5.8.

I1886. 190 mg/mL secu; 25 mM Lact; 150 mM sorb; 50 mM arg; 0.2 mg/mL PS20; 1 mM met; 0.1 mM EDTA; water; and pH 5.8.

I1887. 150 mg/mL secu; 10 mM Phos-Lact; 150 mM sorb; 50 mM arg; 0.2 mg/mL PS20; 1 mM met; 0.1 mM EDTA; water; and pH 5.8.

I1888. 190 mg/mL secu; 10 mM Phos-Lact; 150 mM sorb; 50 mM arg; 0.2 mg/mL PS20; 1 mM met; 0.1 mM EDTA; water; and pH 5.8.

I1889. 150 mg/mL secu; 15 mM Gluc; 150 mM sorb; 50 mM arg; 0.2 mg/mL PS20; 1 mM met; 0.1 mM EDTA; water; and pH 5.8.

I1890. 190 mg/mL secu; 15 mM Gluc; 150 mM sorb; 50 mM arg; 0.2 mg/mL PS20; 1 mM met; 0.1 mM EDTA; water; and pH 5.8.

I1891. 150 mg/mL secu; 0 mM buffer; 75 mM mann; 50 mM arg; 0.2 mg/mL PS20; 1 mM met; 0.1 mM EDTA; water; and pH 5.8.

I1892. 190 mg/mL secu; 0 mM buffer; 75 mM mann; 50 mM arg; 0.2 mg/mL PS20; 1 mM met; 0.1 mM EDTA; water; and pH 5.8.

I1893. 150 mg/mL secu; 50 mM His; 75 mM mann; 50 mM arg; 0.2 mg/mL PS20; 1 mM met; 0.1 mM EDTA; water; and pH 5.8.

I1894. 190 mg/mL secu; 50 mM His; 75 mM mann; 50 mM arg; 0.2 mg/mL PS20; 1 mM met; 0.1 mM EDTA; water; and pH 5.8.

(continued)

I1895. 150 mg/mL secu; 25 mM Lact; 75 mM mann; 50 mM arg; 0.2 mg/mL PS20; 1 mM met; 0.1 mM EDTA; water; and pH 5.8.

I1896. 190 mg/mL secu; 25 mM Lact; 75 mM mann; 50 mM arg; 0.2 mg/mL PS20; 1 mM met; 0.1 mM EDTA; water; and pH 5.8.

I1897. 150 mg/mL secu; 10 mM Phos-Lact; 75 mM mann; 50 mM arg; 0.2 mg/mL PS20; 1 mM met; 0.1 mM EDTA; water; and pH 5.8.

I1898. 190 mg/mL secu; 10 mM Phos-Lact; 75 mM mann; 50 mM arg; 0.2 mg/mL PS20; 1 mM met; 0.1 mM EDTA; water; and pH 5.8.

I1899. 150 mg/mL secu; 15 mM Gluc; 75 mM mann; 50 mM arg; 0.2 mg/mL PS20; 1 mM met; 0.1 mM EDTA; water; and pH 5.8.

I1900. 190 mg/mL secu; 15 mM Gluc; 75 mM mann; 50 mM arg; 0.2 mg/mL PS20; 1 mM met; 0.1 mM EDTA; water; and pH 5.8.

I1901. 150 mg/mL secu; 0 mM buffer; 100 mM sucr; 50 mM arg; 0.2 mg/mL PS20; 1 mM met; 0.1 mM EDTA; water; and pH 5.8.

I1902. 190 mg/mL secu; 0 mM buffer; 100 mM sucr; 50 mM arg; 0.2 mg/mL PS20; 1 mM met; 0.1 mM EDTA; water; and pH 5.8.

I1903. 150 mg/mL secu; 50 mM His; 100 mM sucr; 50 mM arg; 0.2 mg/mL PS20; 1 mM met; 0.1 mM EDTA; water; and pH 5.8.

I1904. 190 mg/mL secu; 50 mM His; 100 mM sucr; 50 mM arg; 0.2 mg/mL PS20; 1 mM met; 0.1 mM EDTA; water; and pH 5.8.

I1905. 150 mg/mL secu; 25 mM Lact; 100 mM sucr; 50 mM arg; 0.2 mg/mL PS20; 1 mM met; 0.1 mM EDTA; water; and pH 5.8.

I1906. 190 mg/mL secu; 25 mM Lact; 100 mM sucr; 50 mM arg; 0.2 mg/mL PS20; 1 mM met; 0.1 mM EDTA; water; and pH 5.8.

I1907. 150 mg/mL secu; 10 mM Phos-Lact; 100 mM sucr; 50 mM arg; 0.2 mg/mL PS20; 1 mM met; 0.1 mM EDTA; water; and pH 5.8.

I1908 . 190 mg/mL secu; 10 mM Phos-Lact; 100 mM sucr; 50 mM arg; 0.2 mg/mL PS20; 1 mM met; 0.1 mM EDTA; water; and pH 5.8.

I1909. 150 mg/mL secu; 15 mM Gluc; 100 mM sucr; 50 mM arg; 0.2 mg/mL PS20; 1 mM met; 0.1 mM EDTA; water; and pH 5.8.

I1910. 190 mg/mL secu; 15 mM Gluc; 100 mM sucr; 50 mM arg; 0.2 mg/mL PS20; 1 mM met; 0.1 mM EDTA; water; and pH 5.8.

I1911. 150 mg/mL secu; 0 mM buffer; 200 mM treh; 50 mM arg; 0.2 mg/mL PS20; 1 mM met; 0.1 mM EDTA; water; and pH 5.8.

I1912. 190 mg/mL secu; 0 mM buffer; 200 mM treh; 50 mM arg; 0.2 mg/mL PS20; 1 mM met; 0.1 mM EDTA; water; and pH 5.8.

I1913. 150 mg/mL secu; 50 mM His; 200 mM treh; 50 mM arg; 0.2 mg/mL PS20; 1 mM met; 0.1 mM EDTA; water; and pH 5.8.

I1914. 190 mg/mL secu; 50 mM His; 200 mM treh; 50 mM arg; 0.2 mg/mL PS20; 1 mM met; 0.1 mM EDTA; water; and pH 5.8.

I1915. 150 mg/mL secu; 25 mM Lact; 200 mM treh; 50 mM arg; 0.2 mg/mL PS20; 1 mM met; 0.1 mM EDTA; water; and pH 5.8.

I1916. 190 mg/mL secu; 25 mM Lact; 200 mM treh; 50 mM arg; 0.2 mg/mL PS20; 1 mM met; 0.1 mM EDTA; water; and pH 5.8.

I1917. 150 mg/mL secu; 10 mM Phos-Lact; 200 mM treh; 50 mM arg; 0.2 mg/mL PS20; 1 mM met; 0.1 mM EDTA; water; and pH 5.8.

I1918. 190 mg/mL secu; 10 mM Phos-Lact; 200 mM treh; 50 mM arg; 0.2 mg/mL PS20; 1 mM met; 0.1 mM EDTA; water; and pH 5.8.

I1919. 150 mg/mL secu; 15 mM Gluc; 200 mM treh; 50 mM arg; 0.2 mg/mL PS20; 1 mM met; 0.1 mM EDTA; water; and pH 5.8.

I1920. 190 mg/mL secu; 15 mM Gluc; 200 mM treh; 50 mM arg; 0.2 mg/mL PS20; 1 mM met; 0.1 mM EDTA; water; and pH 5.8.

(continued)

I1921. 150 mg/mL secu; 0 mM buffer; 150 mM sorb; 100 mM gly; 0.2 mg/mL PS20; 1 mM met; 0.1 mM EDTA; water; and pH 5.8.

I1922. 190 mg/mL secu; 0 mM buffer; 150 mM sorb; 100 mM gly; 0.2 mg/mL PS20; 1 mM met; 0.1 mM EDTA; water; and pH 5.8.

I1923. 150 mg/mL secu; 50 mM His; 150 mM sorb; 100 mM gly; 0.2 mg/mL PS20; 1 mM met; 0.1 mM EDTA; water; and pH 5.8.

I1924. 190 mg/mL secu; 50 mM His; 150 mM sorb; 100 mM gly; 0.2 mg/mL PS20; 1 mM met; 0.1 mM EDTA; water; and pH 5.8.

I1925. 150 mg/mL secu; 25 mM Lact; 150 mM sorb; 100 mM gly; 0.2 mg/mL PS20; 1 mM met; 0.1 mM EDTA; water; and pH 5.8.

I1926. 190 mg/mL secu; 25 mM Lact; 150 mM sorb; 100 mM gly; 0.2 mg/mL PS20; 1 mM met; 0.1 mM EDTA; water; and pH 5.8.

I1927. 150 mg/mL secu; 10 mM Phos-Lact; 150 mM sorb; 100 mM gly; 0.2 mg/mL PS20; 1 mM met; 0.1 mM EDTA; water; and pH 5.8.

I1928. 190 mg/mL secu; 10 mM Phos-Lact; 150 mM sorb; 100 mM gly; 0.2 mg/mL PS20; 1 mM met; 0.1 mM EDTA; water; and pH 5.8.

I1929. 150 mg/mL secu; 15 mM Gluc; 150 mM sorb; 100 mM gly; 0.2 mg/mL PS20; 1 mM met; 0.1 mM EDTA; water; and pH 5.8.

I1930. 190 mg/mL secu; 15 mM Gluc; 150 mM sorb; 100 mM gly; 0.2 mg/mL PS20; 1 mM met; 0.1 mM EDTA; water; and pH 5.8.

I1931. 150 mg/mL secu; 0 mM buffer; 75 mM mann; 100 mM gly; 0.2 mg/mL PS20; 1 mM met; 0.1 mM EDTA; water; and pH 5.8.

I1932. 190 mg/mL secu; 0 mM buffer; 75 mM mann; 100 mM gly; 0.2 mg/mL PS20; 1 mM met; 0.1 mM EDTA; water; and pH 5.8.

I1933. 150 mg/mL secu; 50 mM His; 75 mM mann; 100 mM gly; 0.2 mg/mL PS20; 1 mM met; 0.1 mM EDTA; water; and pH 5.8.

I1934. 190 mg/mL secu; 50 mM His; 75 mM mann; 100 mM gly; 0.2 mg/mL PS20; 1 mM met; 0.1 mM EDTA; water; and pH 5.8.

I1935. 150 mg/mL secu; 25 mM Lact; 75 mM mann; 100 mM gly; 0.2 mg/mL PS20; 1 mM met; 0.1 mM EDTA; water; and pH 5.8.

I1936. 190 mg/mL secu; 25 mM Lact; 75 mM mann; 100 mM gly; 0.2 mg/mL PS20; 1 mM met; 0.1 mM EDTA; water; and pH 5.8.

I1937. 150 mg/mL secu; 10 mM Phos-Lact; 75 mM mann; 100 mM gly; 0.2 mg/mL PS20; 1 mM met; 0.1 mM EDTA; water; and pH 5.8.

I1938. 190 mg/mL secu; 10 mM Phos-Lact; 75 mM mann; 100 mM gly; 0.2 mg/mL PS20; 1 mM met; 0.1 mM EDTA; water; and pH 5.8.

I1939. 150 mg/mL secu; 15 mM Gluc; 75 mM mann; 100 mM gly; 0.2 mg/mL PS20; 1 mM met; 0.1 mM EDTA; water; and pH 5.8.

I1940. 190 mg/mL secu; 15 mM Gluc; 75 mM mann; 100 mM gly; 0.2 mg/mL PS20; 1 mM met; 0.1 mM EDTA; water; and pH 5.8.

I1941. 150 mg/mL secu; 0 mM buffer; 100 mM sucr; 100 mM gly; 0.2 mg/mL PS20; 1 mM met; 0.1 mM EDTA; water; and pH 5.8.

I1942. 190 mg/mL secu; 0 mM buffer; 100 mM sucr; 100 mM gly; 0.2 mg/mL PS20; 1 mM met; 0.1 mM EDTA; water; and pH 5.8.

I1943. 150 mg/mL secu; 50 mM His; 100 mM sucr; 100 mM gly; 0.2 mg/mL PS20; 1 mM met; 0.1 mM EDTA; water; and pH 5.8.

I1944. 190 mg/mL secu; 50 mM His; 100 mM sucr; 100 mM gly; 0.2 mg/mL PS20; 1 mM met; 0.1 mM EDTA; water; and pH 5.8.

I1945. 150 mg/mL secu; 25 mM Lact; 100 mM sucr; 100 mM gly; 0.2 mg/mL PS20; 1 mM met; 0.1 mM EDTA; water; and pH 5.8.

I1946. 190 mg/mL secu; 25 mM Lact; 100 mM sucr; 100 mM gly; 0.2 mg/mL PS20; 1 mM met; 0.1 mM EDTA; water; and pH 5.8.

(continued)

I1947. 150 mg/mL secu; 10 mM Phos-Lact; 100 mM sucr; 100 mM gly; 0.2 mg/mL PS20; 1 mM met; 0.1 mM EDTA; water; and pH 5.8.

I1948. 190 mg/mL secu; 10 mM Phos-Lact; 100 mM sucr; 100 mM gly; 0.2 mg/mL PS20; 1 mM met; 0.1 mM EDTA; water; and pH 5.8.

I1949. 150 mg/mL secu; 15 mM Gluc; 100 mM sucr; 100 mM gly; 0.2 mg/mL PS20; 1 mM met; 0.1 mM EDTA; water; and pH 5.8.

I1950. 190 mg/mL secu; 15 mM Gluc; 100 mM sucr; 100 mM gly; 0.2 mg/mL PS20; 1 mM met; 0.1 mM EDTA; water; and pH 5.8.

I1951. 150 mg/mL secu; 0 mM buffer; 200 mM treh; 100 mM gly; 0.2 mg/mL PS20; 1 mM met; 0.1 mM EDTA; water; and pH 5.8.

I1952. 190 mg/mL secu; 0 mM buffer; 200 mM treh; 100 mM gly; 0.2 mg/mL PS20; 1 mM met; 0.1 mM EDTA; water; and pH 5.8.

I1953. 150 mg/mL secu; 50 mM His; 200 mM treh; 100 mM gly; 0.2 mg/mL PS20; 1 mM met; 0.1 mM EDTA; water; and pH 5.8.

I1954. 190 mg/mL secu; 50 mM His; 200 mM treh; 100 mM gly; 0.2 mg/mL PS20; 1 mM met; 0.1 mM EDTA; water; and pH 5.8.

I1955. 150 mg/mL secu; 25 mM Lact; 200 mM treh; 100 mM gly; 0.2 mg/mL PS20; 1 mM met; 0.1 mM EDTA; water; and pH 5.8.

I1956. 190 mg/mL secu; 25 mM Lact; 200 mM treh; 100 mM gly; 0.2 mg/mL PS20; 1 mM met; 0.1 mM EDTA; water; and pH 5.8.

I1957. 150 mg/mL secu; 10 mM Phos-Lact; 200 mM treh; 100 mM gly; 0.2 mg/mL PS20; 1 mM met; 0.1 mM EDTA; water; and pH 5.8.

I1958. 190 mg/mL secu; 10 mM Phos-Lact; 200 mM treh; 100 mM gly; 0.2 mg/mL PS20; 1 mM met; 0.1 mM EDTA; water; and pH 5.8.

I1959. 150 mg/mL secu; 15 mM Gluc; 200 mM treh; 100 mM gly; 0.2 mg/mL PS20; 1 mM met; 0.1 mM EDTA; water; and pH 5.8.

I1960. 190 mg/mL secu; 15 mM Gluc; 200 mM treh; 100 mM gly; 0.2 mg/mL PS20; 1 mM met; 0.1 mM EDTA; water; and pH 5.8.

I1961. 150 mg/mL secu; 0 mM buffer; 150 mM sorb; 50 mM arg; 0.5 mg/mL PS80; 50 mM NaCl; 0.1 mM EDTA; water; and pH 5.8.

I1962. 190 mg/mL secu; 0 mM buffer; 150 mM sorb; 50 mM arg; 0.5 mg/mL PS80; 50 mM NaCl; 0.1 mM EDTA; water; and pH 5.8.

I1963. 150 mg/mL secu; 50 mM His; 150 mM sorb; 50 mM arg; 0.5 mg/mL PS80; 50 mM NaCl; 0.1 mM EDTA; water; and pH 5.8.

I1964. 190 mg/mL secu; 50 mM His; 150 mM sorb; 50 mM arg; 0.5 mg/mL PS80; 50 mM NaCl; 0.1 mM EDTA; water; and pH 5.8.

I1965. 150 mg/mL secu; 25 mM Lact; 150 mM sorb; 50 mM arg; 0.5 mg/mL PS80; 50 mM NaCl; 0.1 mM EDTA; water; and pH 5.8.

I1966. 190 mg/mL secu; 25 mM Lact; 150 mM sorb; 50 mM arg; 0.5 mg/mL PS80; 50 mM NaCl; 0.1 mM EDTA; water; and pH 5.8.

I1967. 150 mg/mL secu; 10 mM Phos-Lact; 150 mM sorb; 50 mM arg; 0.5 mg/mL PS80; 50 mM NaCl; 0.1 mM EDTA; water; and pH 5.8.

I1968. 190 mg/mL secu; 10 mM Phos-Lact; 150 mM sorb; 50 mM arg; 0.5 mg/mL PS80; 50 mM NaCl; 0.1 mM EDTA; water; and pH 5.8.

I1969. 150 mg/mL secu; 15 mM Gluc; 150 mM sorb; 50 mM arg; 0.5 mg/mL PS80; 50 mM NaCl; 0.1 mM EDTA; water; and pH 5.8.

I1970. 190 mg/mL secu; 15 mM Gluc; 150 mM sorb; 50 mM arg; 0.5 mg/mL PS80; 50 mM NaCl; 0.1 mM EDTA; water; and pH 5.8.

I1971. 150 mg/mL secu; 0 mM buffer; 75 mM mann; 50 mM arg; 0.5 mg/mL PS80; 50 mM NaCl; 0.1 mM EDTA; water; and pH 5.8.

I1972. 190 mg/mL secu; 0 mM buffer; 75 mM mann; 50 mM arg; 0.5 mg/mL PS80; 50 mM NaCl; 0.1 mM EDTA; water; and pH 5.8.

(continued)

I1973. 150 mg/mL secu; 50 mM His; 75 mM mann; 50 mM arg; 0.5 mg/mL PS80; 50 mM NaCl; 0.1 mM EDTA; water; and pH 5.8.

I1974. 190 mg/mL secu; 50 mM His; 75 mM mann; 50 mM arg; 0.5 mg/mL PS80; 50 mM NaCl; 0.1 mM EDTA; water; and pH 5.8.

I1975. 150 mg/mL secu; 25 mM Lact; 75 mM mann; 50 mM arg; 0.5 mg/mL PS80; 50 mM NaCl; 0.1 mM EDTA; water; and pH 5.8.

I1976. 190 mg/mL secu; 25 mM Lact; 75 mM mann; 50 mM arg; 0.5 mg/mL PS80; 50 mM NaCl; 0.1 mM EDTA; water; and pH 5.8.

I1977. 150 mg/mL secu; 10 mM Phos-Lact; 75 mM mann; 50 mM arg; 0.5 mg/mL PS80; 50 mM NaCl; 0.1 mM EDTA; water; and pH 5.8.

I1978. 190 mg/mL secu; 10 mM Phos-Lact; 75 mM mann; 50 mM arg; 0.5 mg/mL PS80; 50 mM NaCl; 0.1 mM EDTA; water; and pH 5.8.

I1979. 150 mg/mL secu; 15 mM Gluc; 75 mM mann; 50 mM arg; 0.5 mg/mL PS80; 50 mM NaCl; 0.1 mM EDTA; water; and pH 5.8.

I1980. 190 mg/mL secu; 15 mM Gluc; 75 mM mann; 50 mM arg; 0.5 mg/mL PS80; 50 mM NaCl; 0.1 mM EDTA; water; and pH 5.8.

I1981. 150 mg/mL secu; 0 mM buffer; 100 mM sucr; 50 mM arg; 0.5 mg/mL PS80; 50 mM NaCl; 0.1 mM EDTA; water; and pH 5.8.

I1982. 190 mg/mL secu; 0 mM buffer; 100 mM sucr; 50 mM arg; 0.5 mg/mL PS80; 50 mM NaCl; 0.1 mM EDTA; water; and pH 5.8.

I1983. 150 mg/mL secu; 50 mM His; 100 mM sucr; 50 mM arg; 0.5 mg/mL PS80; 50 mM NaCl; 0.1 mM EDTA; water; and pH 5.8.

I1984. 190 mg/mL secu; 50 mM His; 100 mM sucr; 50 mM arg; 0.5 mg/mL PS80; 50 mM NaCl; 0.1 mM EDTA; water; and pH 5.8.

I1985. 150 mg/mL secu; 25 mM Lact; 100 mM sucr; 50 mM arg; 0.5 mg/mL PS80; 50 mM NaCl; 0.1 mM EDTA; water; and pH 5.8.

I1986. 190 mg/mL secu; 25 mM Lact; 100 mM sucr; 50 mM arg; 0.5 mg/mL PS80; 50 mM NaCl; 0.1 mM EDTA; water; and pH 5.8.

I1987. 150 mg/mL secu; 10 mM Phos-Lact; 100 mM sucr; 50 mM arg; 0.5 mg/mL PS80; 50 mM NaCl; 0.1 mM EDTA; water; and pH 5.8.

I1988. 190 mg/mL secu; 10 mM Phos-Lact; 100 mM sucr; 50 mM arg; 0.5 mg/mL PS80; 50 mM NaCl; 0.1 mM EDTA; water; and pH 5.8.

I1989. 150 mg/mL secu; 15 mM Gluc; 100 mM sucr; 50 mM arg; 0.5 mg/mL PS80; 50 mM NaCl; 0.1 mM EDTA; water; and pH 5.8.

I1990. 190 mg/mL secu; 15 mM Gluc; 100 mM sucr; 50 mM arg; 0.5 mg/mL PS80; 50 mM NaCl; 0.1 mM EDTA; water; and pH 5.8.

I1991. 150 mg/mL secu; 0 mM buffer; 200 mM treh; 50 mM arg; 0.5 mg/mL PS80; 50 mM NaCl; 0.1 mM EDTA; water; and pH 5.8.

I1992. 190 mg/mL secu; 0 mM buffer; 200 mM treh; 50 mM arg; 0.5 mg/mL PS80; 50 mM NaCl; 0.1 mM EDTA; water; and pH 5.8.

I1993. 150 mg/mL secu; 50 mM His; 200 mM treh; 50 mM arg; 0.5 mg/mL PS80; 50 mM NaCl; 0.1 mM EDTA; water; and pH 5.8.

I1994. 190 mg/mL secu; 50 mM His; 200 mM treh; 50 mM arg; 0.5 mg/mL PS80; 50 mM NaCl; 0.1 mM EDTA; water; and pH 5.8.

I1995. 150 mg/mL secu; 25 mM Lact; 200 mM treh; 50 mM arg; 0.5 mg/mL PS80; 50 mM NaCl; 0.1 mM EDTA; water; and pH 5.8.

I1996. 190 mg/mL secu; 25 mM Lact; 200 mM treh; 50 mM arg; 0.5 mg/mL PS80; 50 mM NaCl; 0.1 mM EDTA; water; and pH 5.8.

I1997. 150 mg/mL secu; 10 mM Phos-Lact; 200 mM treh; 50 mM arg; 0.5 mg/mL PS80; 50 mM NaCl; 0.1 mM EDTA; water; and pH 5.8.

I1998. 190 mg/mL secu; 10 mM Phos-Lact; 200 mM treh; 50 mM arg; 0.5 mg/mL PS80; 50 mM NaCl; 0.1 mM EDTA; water; and pH 5.8.

(continued)

I1999. 150 mg/mL secu; 15 mM Gluc; 200 mM treh; 50 mM arg; 0.5 mg/mL PS80; 50 mM NaCl; 0.1 mM EDTA; water; and pH 5.8.

I2000. 190 mg/mL secu; 15 mM Gluc; 200 mM treh; 50 mM arg; 0.5 mg/mL PS80; 50 mM NaCl; 0.1 mM EDTA; water; and pH 5.8.

I2001. 150 mg/mL secu; 0 mM buffer; 150 mM sorb; 100 mM gly; 0.5 mg/mL PS80; 50 mM NaCl; 0.1 mM EDTA; water; and pH 5.8.

I2002. 190 mg/mL secu; 0 mM buffer; 150 mM sorb; 100 mM gly; 0.5 mg/mL PS80; 50 mM NaCl; 0.1 mM EDTA; water; and pH 5.8.

I2003. 150 mg/mL secu; 50 mM His; 150 mM sorb; 100 mM gly; 0.5 mg/mL PS80; 50 mM NaCl; 0.1 mM EDTA; water; and pH 5.8.

I2004. 190 mg/mL secu; 50 mM His; 150 mM sorb; 100 mM gly; 0.5 mg/mL PS80; 50 mM NaCl; 0.1 mM EDTA; water; and pH 5.8.

I2005. 150 mg/mL secu; 25 mM Lact; 150 mM sorb; 100 mM gly; 0.5 mg/mL PS80; 50 mM NaCl; 0.1 mM EDTA; water; and pH 5.8.

I2006. 190 mg/mL secu; 25 mM Lact; 150 mM sorb; 100 mM gly; 0.5 mg/mL PS80; 50 mM NaCl; 0.1 mM EDTA; water; and pH 5.8.

I2007. 150 mg/mL secu; 10 mM Phos-Lact; 150 mM sorb; 100 mM gly; 0.5 mg/mL PS80; 50 mM NaCl; 0.1 mM EDTA; water; and pH 5.8.

I2008. 190 mg/mL secu; 10 mM Phos-Lact; 150 mM sorb; 100 mM gly; 0.5 mg/mL PS80; 50 mM NaCl; 0.1 mM EDTA; water; and pH 5.8.

I2009. 150 mg/mL secu; 15 mM Gluc; 150 mM sorb; 100 mM gly; 0.5 mg/mL PS80; 50 mM NaCl; 0.1 mM EDTA; water; and pH 5.8.

I2010. 190 mg/mL secu; 15 mM Gluc; 150 mM sorb; 100 mM gly; 0.5 mg/mL PS80; 50 mM NaCl; 0.1 mM EDTA; water; and pH 5.8.

I2011. 150 mg/mL secu; 0 mM buffer; 75 mM mann; 100 mM gly; 0.5 mg/mL PS80; 50 mM NaCl; 0.1 mM EDTA; water; and pH 5.8.

I2012. 190 mg/mL secu; 0 mM buffer; 75 mM mann; 100 mM gly; 0.5 mg/mL PS80; 50 mM NaCl; 0.1 mM EDTA; water; and pH 5.8.

I2013. 150 mg/mL secu; 50 mM His; 75 mM mann; 100 mM gly; 0.5 mg/mL PS80; 50 mM NaCl; 0.1 mM EDTA; water; and pH 5.8.

I2014. 190 mg/mL secu; 50 mM His; 75 mM mann; 100 mM gly; 0.5 mg/mL PS80; 50 mM NaCl; 0.1 mM EDTA; water; and pH 5.8.

I2015. 150 mg/mL secu; 25 mM Lact; 75 mM mann; 100 mM gly; 0.5 mg/mL PS80; 50 mM NaCl; 0.1 mM EDTA; water; and pH 5.8.

I2016. 190 mg/mL secu; 25 mM Lact; 75 mM mann; 100 mM gly; 0.5 mg/mL PS80; 50 mM NaCl; 0.1 mM EDTA; water; and pH 5.8.

I2017. 150 mg/mL secu; 10 mM Phos-Lact; 75 mM mann; 100 mM gly; 0.5 mg/mL PS80; 50 mM NaCl; 0.1 mM EDTA; water; and pH 5.8.

I2018. 190 mg/mL secu; 10 mM Phos-Lact; 75 mM mann; 100 mM gly; 0.5 mg/mL PS80; 50 mM NaCl; 0.1 mM EDTA; water; and pH 5.8.

I2019. 150 mg/mL secu; 15 mM Gluc; 75 mM mann; 100 mM gly; 0.5 mg/mL PS80; 50 mM NaCl; 0.1 mM EDTA; water; and pH 5.8.

I2020. 190 mg/mL secu; 15 mM Gluc; 75 mM mann; 100 mM gly; 0.5 mg/mL PS80; 50 mM NaCl; 0.1 mM EDTA; water; and pH 5.8.

I2021. 150 mg/mL secu; 0 mM buffer; 100 mM sucr; 100 mM gly; 0.5 mg/mL PS80; 50 mM NaCl; 0.1 mM EDTA; water; and pH 5.8.

I2022. 190 mg/mL secu; 0 mM buffer; 100 mM sucr; 100 mM gly; 0.5 mg/mL PS80; 50 mM NaCl; 0.1 mM EDTA; water; and pH 5.8.

I2023. 150 mg/mL secu; 50 mM His; 100 mM sucr; 100 mM gly; 0.5 mg/mL PS80; 50 mM NaCl; 0.1 mM EDTA; water; and pH 5.8.

I2024. 190 mg/mL secu; 50 mM His; 100 mM sucr; 100 mM gly; 0.5 mg/mL PS80; 50 mM NaCl; 0.1 mM EDTA; water; and pH 5.8.

(continued)

I2025. 150 mg/mL secu; 25 mM Lact; 100 mM sucr; 100 mM gly; 0.5 mg/mL PS80; 50 mM NaCl; 0.1 mM EDTA; water; and pH 5.8.

I2026. 190 mg/mL secu; 25 mM Lact; 100 mM sucr; 100 mM gly; 0.5 mg/mL PS80; 50 mM NaCl; 0.1 mM EDTA; water; and pH 5.8.

I2027. 150 mg/mL secu; 10 mM Phos-Lact; 100 mM sucr; 100 mM gly; 0.5 mg/mL PS80; 50 mM NaCl; 0.1 mM EDTA; water; and pH 5.8.

I2028. 190 mg/mL secu; 10 mM Phos-Lact; 100 mM sucr; 100 mM gly; 0.5 mg/mL PS80; 50 mM NaCl; 0.1 mM EDTA; water; and pH 5.8.

I2029. 150 mg/mL secu; 15 mM Gluc; 100 mM sucr; 100 mM gly; 0.5 mg/mL PS80; 50 mM NaCl; 0.1 mM EDTA; water; and pH 5.8.

I2030. 190 mg/mL secu; 15 mM Gluc; 100 mM sucr; 100 mM gly; 0.5 mg/mL PS80; 50 mM NaCl; 0.1 mM EDTA; water; and pH 5.8.

I2031. 150 mg/mL secu; 0 mM buffer; 200 mM treh; 100 mM gly; 0.5 mg/mL PS80; 50 mM NaCl; 0.1 mM EDTA; water; and pH 5.8.

I2032. 190 mg/mL secu; 0 mM buffer; 200 mM treh; 100 mM gly; 0.5 mg/mL PS80; 50 mM NaCl; 0.1 mM EDTA; water; and pH 5.8.

I2033. 150 mg/mL secu; 50 mM His; 200 mM treh; 100 mM gly; 0.5 mg/mL PS80; 50 mM NaCl; 0.1 mM EDTA; water; and pH 5.8.

I2034. 190 mg/mL secu; 50 mM His; 200 mM treh; 100 mM gly; 0.5 mg/mL PS80; 50 mM NaCl; 0.1 mM EDTA; water; and pH 5.8.

I2035. 150 mg/mL secu; 25 mM Lact; 200 mM treh; 100 mM gly; 0.5 mg/mL PS80; 50 mM NaCl; 0.1 mM EDTA; water; and pH 5.8.

I2036. 190 mg/mL secu; 25 mM Lact; 200 mM treh; 100 mM gly; 0.5 mg/mL PS80; 50 mM NaCl; 0.1 mM EDTA; water; and pH 5.8.

I2037. 150 mg/mL secu; 10 mM Phos-Lact; 200 mM treh; 100 mM gly; 0.5 mg/mL PS80; 50 mM NaCl; 0.1 mM EDTA; water; and pH 5.8.

I2038. 190 mg/mL secu; 10 mM Phos-Lact; 200 mM treh; 100 mM gly; 0.5 mg/mL PS80; 50 mM NaCl; 0.1 mM EDTA; water; and pH 5.8.

I2039. 150 mg/mL secu; 15 mM Gluc; 200 mM treh; 100 mM gly; 0.5 mg/mL PS80; 50 mM NaCl; 0.1 mM EDTA; water; and pH 5.8.

I2040. 190 mg/mL secu; 15 mM Gluc; 200 mM treh; 100 mM gly; 0.5 mg/mL PS80; 50 mM NaCl; 0.1 mM EDTA; water; and pH 5.8.

I2041. 150 mg/mL secu; 0 mM buffer; 150 mM sorb; 50 mM arg; 0.2 mg/mL PS20; 50 mM NaCl; 0.1 mM EDTA; water; and pH 5.8.

I2042. 190 mg/mL secu; 0 mM buffer; 150 mM sorb; 50 mM arg; 0.2 mg/mL PS20; 50 mM NaCl; 0.1 mM EDTA; water; and pH 5.8.

I2043. 150 mg/mL secu; 50 mM His; 150 mM sorb; 50 mM arg; 0.2 mg/mL PS20; 50 mM NaCl; 0.1 mM EDTA; water; and pH 5.8.

I2044. 190 mg/mL secu; 50 mM His; 150 mM sorb; 50 mM arg; 0.2 mg/mL PS20; 50 mM NaCl; 0.1 mM EDTA; water; and pH 5.8.

I2045. 150 mg/mL secu; 25 mM Lact; 150 mM sorb; 50 mM arg; 0.2 mg/mL PS20; 50 mM NaCl; 0.1 mM EDTA; water; and pH 5.8.

I2046. 190 mg/mL secu; 25 mM Lact; 150 mM sorb; 50 mM arg; 0.2 mg/mL PS20; 50 mM NaCl; 0.1 mM EDTA; water; and pH 5.8.

I2047. 150 mg/mL secu; 10 mM Phos-Lact; 150 mM sorb; 50 mM arg; 0.2 mg/mL PS20; 50 mM NaCl; 0.1 mM EDTA; water; and pH 5.8.

I2048. 190 mg/mL secu; 10 mM Phos-Lact; 150 mM sorb; 50 mM arg; 0.2 mg/mL PS20; 50 mM NaCl; 0.1 mM EDTA; water; and pH 5.8.

I2049. 150 mg/mL secu; 15 mM Gluc; 150 mM sorb; 50 mM arg; 0.2 mg/mL PS20; 50 mM NaCl; 0.1 mM EDTA; water; and pH 5.8.

I2050. 190 mg/mL secu; 15 mM Gluc; 150 mM sorb; 50 mM arg; 0.2 mg/mL PS20; 50 mM NaCl; 0.1 mM EDTA; water; and pH 5.8.

(continued)

I2051. 150 mg/mL secu; 0 mM buffer; 75 mM mann; 50 mM arg; 0.2 mg/mL PS20; 50 mM NaCl; 0.1 mM EDTA; water; and pH 5.8.

I2052. 190 mg/mL secu; 0 mM buffer; 75 mM mann; 50 mM arg; 0.2 mg/mL PS20; 50 mM NaCl; 0.1 mM EDTA; water; and pH 5.8.

I2053. 150 mg/mL secu; 50 mM His; 75 mM mann; 50 mM arg; 0.2 mg/mL PS20; 50 mM NaCl; 0.1 mM EDTA; water; and pH 5.8.

I2054. 190 mg/mL secu; 50 mM His; 75 mM mann; 50 mM arg; 0.2 mg/mL PS20; 50 mM NaCl; 0.1 mM EDTA; water; and pH 5.8.

I2055. 150 mg/mL secu; 25 mM Lact; 75 mM mann; 50 mM arg; 0.2 mg/mL PS20; 50 mM NaCl; 0.1 mM EDTA; water; and pH 5.8.

I2056. 190 mg/mL secu; 25 mM Lact; 75 mM mann; 50 mM arg; 0.2 mg/mL PS20; 50 mM NaCl; 0.1 mM EDTA; water; and pH 5.8.

I2057. 150 mg/mL secu; 10 mM Phos-Lact; 75 mM mann; 50 mM arg; 0.2 mg/mL PS20; 50 mM NaCl; 0.1 mM EDTA; water; and pH 5.8.

I2058. 190 mg/mL secu; 10 mM Phos-Lact; 75 mM mann; 50 mM arg; 0.2 mg/mL PS20; 50 mM NaCl; 0.1 mM EDTA; water; and pH 5.8.

I2059. 150 mg/mL secu; 15 mM Gluc; 75 mM mann; 50 mM arg; 0.2 mg/mL PS20; 50 mM NaCl; 0.1 mM EDTA; water; and pH 5.8.

I2060. 190 mg/mL secu; 15 mM Gluc; 75 mM mann; 50 mM arg; 0.2 mg/mL PS20; 50 mM NaCl; 0.1 mM EDTA; water; and pH 5.8.

I2061. 150 mg/mL secu; 0 mM buffer; 100 mM sucr; 50 mM arg; 0.2 mg/mL PS20; 50 mM NaCl; 0.1 mM EDTA; water; and pH 5.8.

I2062. 190 mg/mL secu; 0 mM buffer; 100 mM sucr; 50 mM arg; 0.2 mg/mL PS20; 50 mM NaCl; 0.1 mM EDTA; water; and pH 5.8.

I2063. 150 mg/mL secu; 50 mM His; 100 mM sucr; 50 mM arg; 0.2 mg/mL PS20; 50 mM NaCl; 0.1 mM EDTA; water; and pH 5.8.

I2064. 190 mg/mL secu; 50 mM His; 100 mM sucr; 50 mM arg; 0.2 mg/mL PS20; 50 mM NaCl; 0.1 mM EDTA; water; and pH 5.8.

I2065. 150 mg/mL secu; 25 mM Lact; 100 mM sucr; 50 mM arg; 0.2 mg/mL PS20; 50 mM NaCl; 0.1 mM EDTA; water; and pH 5.8.

I2066. 190 mg/mL secu; 25 mM Lact; 100 mM sucr; 50 mM arg; 0.2 mg/mL PS20; 50 mM NaCl; 0.1 mM EDTA; water; and pH 5.8.

I2067. 150 mg/mL secu; 10 mM Phos-Lact; 100 mM sucr; 50 mM arg; 0.2 mg/mL PS20; 50 mM NaCl; 0.1 mM EDTA; water; and pH 5.8.

I2068. 190 mg/mL secu; 10 mM Phos-Lact; 100 mM sucr; 50 mM arg; 0.2 mg/mL PS20; 50 mM NaCl; 0.1 mM EDTA; water; and pH 5.8.

I2069. 150 mg/mL secu; 15 mM Gluc; 100 mM sucr; 50 mM arg; 0.2 mg/mL PS20; 50 mM NaCl; 0.1 mM EDTA; water; and pH 5.8.

I2070. 190 mg/mL secu; 15 mM Gluc; 100 mM sucr; 50 mM arg; 0.2 mg/mL PS20; 50 mM NaCl; 0.1 mM EDTA; water; and pH 5.8.

I2071. 150 mg/mL secu; 0 mM buffer; 200 mM treh; 50 mM arg; 0.2 mg/mL PS20; 50 mM NaCl; 0.1 mM EDTA; water; and pH 5.8.

I2072. 190 mg/mL secu; 0 mM buffer; 200 mM treh; 50 mM arg; 0.2 mg/mL PS20; 50 mM NaCl; 0.1 mM EDTA; water; and pH 5.8.

I2073. 150 mg/mL secu; 50 mM His; 200 mM treh; 50 mM arg; 0.2 mg/mL PS20; 50 mM NaCl; 0.1 mM EDTA; water; and pH 5.8.

I2074. 190 mg/mL secu; 50 mM His; 200 mM treh; 50 mM arg; 0.2 mg/mL PS20; 50 mM NaCl; 0.1 mM EDTA; water; and pH 5.8.

I2075. 150 mg/mL secu; 25 mM Lact; 200 mM treh; 50 mM arg; 0.2 mg/mL PS20; 50 mM NaCl; 0.1 mM EDTA; water; and pH 5.8.

I2076. 190 mg/mL secu; 25 mM Lact; 200 mM treh; 50 mM arg; 0.2 mg/mL PS20; 50 mM NaCl; 0.1 mM EDTA; water; and pH 5.8.

(continued)

I2077. 150 mg/mL secu; 10 mM Phos-Lact; 200 mM treh; 50 mM arg; 0.2 mg/mL PS20; 50 mM NaCl; 0.1 mM EDTA; water; and pH 5.8.

I2078. 190 mg/mL secu; 10 mM Phos-Lact; 200 mM treh; 50 mM arg; 0.2 mg/mL PS20; 50 mM NaCl; 0.1 mM EDTA; water; and pH 5.8.

I2079. 150 mg/mL secu; 15 mM Gluc; 200 mM treh; 50 mM arg; 0.2 mg/mL PS20; 50 mM NaCl; 0.1 mM EDTA; water; and pH 5.8.

I2080. 190 mg/mL secu; 15 mM Gluc; 200 mM treh; 50 mM arg; 0.2 mg/mL PS20; 50 mM NaCl; 0.1 mM EDTA; water; and pH 5.8.

I2081. 150 mg/mL secu; 0 mM buffer; 150 mM sorb; 100 mM gly; 0.2 mg/mL PS20; 50 mM NaCl; 0.1 mM EDTA; water; and pH 5.8.

I2082. 190 mg/mL secu; 0 mM buffer; 150 mM sorb; 100 mM gly; 0.2 mg/mL PS20; 50 mM NaCl; 0.1 mM EDTA; water; and pH 5.8.

I2083. 150 mg/mL secu; 50 mM His; 150 mM sorb; 100 mM gly; 0.2 mg/mL PS20; 50 mM NaCl; 0.1 mM EDTA; water; and pH 5.8.

I2084. 190 mg/mL secu; 50 mM His; 150 mM sorb; 100 mM gly; 0.2 mg/mL PS20; 50 mM NaCl; 0.1 mM EDTA; water; and pH 5.8.

I2085. 150 mg/mL secu; 25 mM Lact; 150 mM sorb; 100 mM gly; 0.2 mg/mL PS20; 50 mM NaCl; 0.1 mM EDTA; water; and pH 5.8.

I2086. 190 mg/mL secu; 25 mM Lact; 150 mM sorb; 100 mM gly; 0.2 mg/mL PS20; 50 mM NaCl; 0.1 mM EDTA; water; and pH 5.8.

I2087. 150 mg/mL secu; 10 mM Phos-Lact; 150 mM sorb; 100 mM gly; 0.2 mg/mL PS20; 50 mM NaCl; 0.1 mM EDTA; water; and pH 5.8.

I2088. 190 mg/mL secu; 10 mM Phos-Lact; 150 mM sorb; 100 mM gly; 0.2 mg/mL PS20; 50 mM NaCl; 0.1 mM EDTA; water; and pH 5.8.

I2089. 150 mg/mL secu; 15 mM Gluc; 150 mM sorb; 100 mM gly; 0.2 mg/mL PS20; 50 mM NaCl; 0.1 mM EDTA; water; and pH 5.8.

I2090. 190 mg/mL secu; 15 mM Gluc; 150 mM sorb; 100 mM gly; 0.2 mg/mL PS20; 50 mM NaCl; 0.1 mM EDTA; water; and pH 5.8.

I2091. 150 mg/mL secu; 0 mM buffer; 75 mM mann; 100 mM gly; 0.2 mg/mL PS20; 50 mM NaCl; 0.1 mM EDTA; water; and pH 5.8.

I2092. 190 mg/mL secu; 0 mM buffer; 75 mM mann; 100 mM gly; 0.2 mg/mL PS20; 50 mM NaCl; 0.1 mM EDTA; water; and pH 5.8.

I2093. 150 mg/mL secu; 50 mM His; 75 mM mann; 100 mM gly; 0.2 mg/mL PS20; 50 mM NaCl; 0.1 mM EDTA; water; and pH 5.8.

I2094. 190 mg/mL secu; 50 mM His; 75 mM mann; 100 mM gly; 0.2 mg/mL PS20; 50 mM NaCl; 0.1 mM EDTA; water; and pH 5.8.

I2095. 150 mg/mL secu; 25 mM Lact; 75 mM mann; 100 mM gly; 0.2 mg/mL PS20; 50 mM NaCl; 0.1 mM EDTA; water; and pH 5.8.

I2096. 190 mg/mL secu; 25 mM Lact; 75 mM mann; 100 mM gly; 0.2 mg/mL PS20; 50 mM NaCl; 0.1 mM EDTA; water; and pH 5.8.

I2097. 150 mg/mL secu; 10 mM Phos-Lact; 75 mM mann; 100 mM gly; 0.2 mg/mL PS20; 50 mM NaCl; 0.1 mM EDTA; water; and pH 5.8.

I2098. 190 mg/mL secu; 10 mM Phos-Lact; 75 mM mann; 100 mM gly; 0.2 mg/mL PS20; 50 mM NaCl; 0.1 mM EDTA; water; and pH 5.8.

I2099. 150 mg/mL secu; 15 mM Gluc; 75 mM mann; 100 mM gly; 0.2 mg/mL PS20; 50 mM NaCl; 0.1 mM EDTA; water; and pH 5.8.

I2100. 190 mg/mL secu; 15 mM Gluc; 75 mM mann; 100 mM gly; 0.2 mg/mL PS20; 50 mM NaCl; 0.1 mM EDTA; water; and pH 5.8.

I2101. 150 mg/mL secu; 0 mM buffer; 100 mM sucr; 100 mM gly; 0.2 mg/mL PS20; 50 mM NaCl; 0.1 mM EDTA; water; and pH 5.8.

I2102. 190 mg/mL secu; 0 mM buffer; 100 mM sucr; 100 mM gly; 0.2 mg/mL PS20; 50 mM NaCl; 0.1 mM EDTA; water; and pH 5.8.

(continued)

I2103. 150 mg/mL secu; 50 mM His; 100 mM sucr; 100 mM gly; 0.2 mg/mL PS20; 50 mM NaCl; 0.1 mM EDTA; water; and pH 5.8.

I2104. 190 mg/mL secu; 50 mM His; 100 mM sucr; 100 mM gly; 0.2 mg/mL PS20; 50 mM NaCl; 0.1 mM EDTA; water; and pH 5.8.

I2105. 150 mg/mL secu; 25 mM Lact; 100 mM sucr; 100 mM gly; 0.2 mg/mL PS20; 50 mM NaCl; 0.1 mM EDTA; water; and pH 5.8.

I2106. 190 mg/mL secu; 25 mM Lact; 100 mM sucr; 100 mM gly; 0.2 mg/mL PS20; 50 mM NaCl; 0.1 mM EDTA; water; and pH 5.8.

I2107. 150 mg/mL secu; 10 mM Phos-Lact; 100 mM sucr; 100 mM gly; 0.2 mg/mL PS20; 50 mM NaCl; 0.1 mM EDTA; water; and pH 5.8.

I2108. 190 mg/mL secu; 10 mM Phos-Lact; 100 mM sucr; 100 mM gly; 0.2 mg/mL PS20; 50 mM NaCl; 0.1 mM EDTA; water; and pH 5.8.

I2109. 150 mg/mL secu; 15 mM Gluc; 100 mM sucr; 100 mM gly; 0.2 mg/mL PS20; 50 mM NaCl; 0.1 mM EDTA; water; and pH 5.8.

I2110. 190 mg/mL secu; 15 mM Gluc; 100 mM sucr; 100 mM gly; 0.2 mg/mL PS20; 50 mM NaCl; 0.1 mM EDTA; water; and pH 5.8.

I2111. 150 mg/mL secu; 0 mM buffer; 200 mM treh; 100 mM gly; 0.2 mg/mL PS20; 50 mM NaCl; 0.1 mM EDTA; water; and pH 5.8.

I2112. 190 mg/mL secu; 0 mM buffer; 200 mM treh; 100 mM gly; 0.2 mg/mL PS20; 50 mM NaCl; 0.1 mM EDTA; water; and pH 5.8.

I2113. 150 mg/mL secu; 50 mM His; 200 mM treh; 100 mM gly; 0.2 mg/mL PS20; 50 mM NaCl; 0.1 mM EDTA; water; and pH 5.8.

I2114. 190 mg/mL secu; 50 mM His; 200 mM treh; 100 mM gly; 0.2 mg/mL PS20; 50 mM NaCl; 0.1 mM EDTA; water; and pH 5.8.

I2115. 150 mg/mL secu; 25 mM Lact; 200 mM treh; 100 mM gly; 0.2 mg/mL PS20; 50 mM NaCl; 0.1 mM EDTA; water; and pH 5.8.

I2116. 190 mg/mL secu; 25 mM Lact; 200 mM treh; 100 mM gly; 0.2 mg/mL PS20; 50 mM NaCl; 0.1 mM EDTA; water; and pH 5.8.

I2117. 150 mg/mL secu; 10 mM Phos-Lact; 200 mM treh; 100 mM gly; 0.2 mg/mL PS20; 50 mM NaCl; 0.1 mM EDTA; water; and pH 5.8.

I2118. 190 mg/mL secu; 10 mM Phos-Lact; 200 mM treh; 100 mM gly; 0.2 mg/mL PS20; 50 mM NaCl; 0.1 mM EDTA; water; and pH 5.8.

I2119. 150 mg/mL secu; 15 mM Gluc; 200 mM treh; 100 mM gly; 0.2 mg/mL PS20; 50 mM NaCl; 0.1 mM EDTA; water; and pH 5.8.

I2120. 190 mg/mL secu; 15 mM Gluc; 200 mM treh; 100 mM gly; 0.2 mg/mL PS20; 50 mM NaCl; 0.1 mM EDTA; water; and pH 5.8.

I2121. 150 mg/mL secu; 0 mM buffer; 150 mM sorb; 0.5 mg/mL PS80; 1 mM met; 50 mM NaCl; 0.1 mM EDTA; water; and pH 5.8.

I2122. 190 mg/mL secu; 0 mM buffer; 150 mM sorb; 0.5 mg/mL PS80; 1 mM met; 50 mM NaCl; 0.1 mM EDTA; water; and pH 5.8.

I2123. 150 mg/mL secu; 50 mM His; 150 mM sorb; 0.5 mg/mL PS80; 1 mM met; 50 mM NaCl; 0.1 mM EDTA; water; and pH 5.8.

I2124. 190 mg/mL secu; 50 mM His; 150 mM sorb; 0.5 mg/mL PS80; 1 mM met; 50 mM NaCl; 0.1 mM EDTA; water; and pH 5.8.

I2125. 150 mg/mL secu; 25 mM Lact; 150 mM sorb; 0.5 mg/mL PS80; 1 mM met; 50 mM NaCl; 0.1 mM EDTA; water; and pH 5.8.

I2126. 190 mg/mL secu; 25 mM Lact; 150 mM sorb; 0.5 mg/mL PS80; 1 mM met; 50 mM NaCl; 0.1 mM EDTA; water; and pH 5.8.

I2127. 150 mg/mL secu; 10 mM Phos-Lact; 150 mM sorb; 0.5 mg/mL PS80; 1 mM met; 50 mM NaCl; 0.1 mM EDTA; water; and pH 5.8.

I2128. 190 mg/mL secu; 10 mM Phos-Lact; 150 mM sorb; 0.5 mg/mL PS80; 1 mM met; 50 mM NaCl; 0.1 mM EDTA; water; and pH 5.8.

(continued)

I2129. 150 mg/mL secu; 15 mM Gluc; 150 mM sorb; 0.5 mg/mL PS80; 1 mM met; 50 mM NaCl; 0.1 mM EDTA; water; and pH 5.8.

I2130. 190 mg/mL secu; 15 mM Gluc; 150 mM sorb; 0.5 mg/mL PS80; 1 mM met; 50 mM NaCl; 0.1 mM EDTA; water; and pH 5.8.

I2131. 150 mg/mL secu; 0 mM buffer; 75 mM mann; 0.5 mg/mL PS80; 1 mM met; 50 mM NaCl; 0.1 mM EDTA; water; and pH 5.8.

I2132. 190 mg/mL secu; 0 mM buffer; 75 mM mann; 0.5 mg/mL PS80; 1 mM met; 50 mM NaCl; 0.1 mM EDTA; water; and pH 5.8.

I2133. 150 mg/mL secu; 50 mM His; 75 mM mann; 0.5 mg/mL PS80; 1 mM met; 50 mM NaCl; 0.1 mM EDTA; water; and pH 5.8.

I2134. 190 mg/mL secu; 50 mM His; 75 mM mann; 0.5 mg/mL PS80; 1 mM met; 50 mM NaCl; 0.1 mM EDTA; water; and pH 5.8.

I2135. 150 mg/mL secu; 25 mM Lact; 75 mM mann; 0.5 mg/mL PS80; 1 mM met; 50 mM NaCl; 0.1 mM EDTA; water; and pH 5.8.

I2136. 190 mg/mL secu; 25 mM Lact; 75 mM mann; 0.5 mg/mL PS80; 1 mM met; 50 mM NaCl; 0.1 mM EDTA; water; and pH 5.8.

I2137. 150 mg/mL secu; 10 mM Phos-Lact; 75 mM mann; 0.5 mg/mL PS80; 1 mM met; 50 mM NaCl; 0.1 mM EDTA; water; and pH 5.8.

I2138. 190 mg/mL secu; 10 mM Phos-Lact; 75 mM mann; 0.5 mg/mL PS80; 1 mM met; 50 mM NaCl; 0.1 mM EDTA; water; and pH 5.8.

I2139. 150 mg/mL secu; 15 mM Glue; 75 mM mann; 0.5 mg/mL PS80; 1 mM met; 50 mM NaCl; 0.1 mM EDTA; water; and pH 5.8.

I2140. 190 mg/mL secu; 15 mM Glue; 75 mM mann; 0.5 mg/mL PS80; 1 mM met; 50 mM NaCl; 0.1 mM EDTA; water; and pH 5.8.

I2141. 150 mg/mL secu; 0 mM buffer; 100 mM sucr; 0.5 mg/mL PS80; 1 mM met; 50 mM NaCl; 0.1 mM EDTA; water; and pH 5.8.

I2142. 190 mg/mL secu; 0 mM buffer; 100 mM sucr; 0.5 mg/mL PS80; 1 mM met; 50 mM NaCl; 0.1 mM EDTA; water; and pH 5.8.

I2143. 150 mg/mL secu; 50 mM His; 100 mM sucr; 0.5 mg/mL PS80; 1 mM met; 50 mM NaCl; 0.1 mM EDTA; water; and pH 5.8.

I2144. 190 mg/mL secu; 50 mM His; 100 mM sucr; 0.5 mg/mL PS80; 1 mM met; 50 mM NaCl; 0.1 mM EDTA; water; and pH 5.8.

I2145. 150 mg/mL secu; 25 mM Lact; 100 mM sucr; 0.5 mg/mL PS80; 1 mM met; 50 mM NaCl; 0.1 mM EDTA; water; and pH 5.8.

I2146. 190 mg/mL secu; 25 mM Lact; 100 mM sucr; 0.5 mg/mL PS80; 1 mM met; 50 mM NaCl; 0.1 mM EDTA; water; and pH 5.8.

I2147. 150 mg/mL secu; 10 mM Phos-Lact; 100 mM sucr; 0.5 mg/mL PS80; 1 mM met; 50 mM NaCl; 0.1 mM EDTA; water; and pH 5.8.

I2148. 190 mg/mL secu; 10 mM Phos-Lact; 100 mM sucr; 0.5 mg/mL PS80; 1 mM met; 50 mM NaCl; 0.1 mM EDTA; water; and pH 5.8.

I2149. 150 mg/mL secu; 15 mM Glue; 100 mM sucr; 0.5 mg/mL PS80; 1 mM met; 50 mM NaCl; 0.1 mM EDTA; water; and pH 5.8.

I2150. 190 mg/mL secu; 15 mM Glue; 100 mM sucr; 0.5 mg/mL PS80; 1 mM met; 50 mM NaCl; 0.1 mM EDTA; water; and pH 5.8.

I2151. 150 mg/mL secu; 0 mM buffer; 200 mM treh; 0.5 mg/mL PS80; 1 mM met; 50 mM NaCl; 0.1 mM EDTA; water; and pH 5.8.

I2152. 190 mg/mL secu; 0 mM buffer; 200 mM treh; 0.5 mg/mL PS80; 1 mM met; 50 mM NaCl; 0.1 mM EDTA; water; and pH 5.8.

I2153. 150 mg/mL secu; 50 mM His; 200 mM treh; 0.5 mg/mL PS80; 1 mM met; 50 mM NaCl; 0.1 mM EDTA; water; and pH 5.8.

I2154. 190 mg/mL secu; 50 mM His; 200 mM treh; 0.5 mg/mL PS80; 1 mM met; 50 mM NaCl; 0.1 mM EDTA; water; and pH 5.8.

(continued)

I2155. 150 mg/mL secu; 25 mM Lact; 200 mM treh; 0.5 mg/mL PS80; 1 mM met; 50 mM NaCl; 0.1 mM EDTA; water; and pH 5.8.

I2156. 190 mg/mL secu; 25 mM Lact; 200 mM treh; 0.5 mg/mL PS80; 1 mM met; 50 mM NaCl; 0.1 mM EDTA; water; and pH 5.8.

I2157. 150 mg/mL secu; 10 mM Phos-Lact; 200 mM treh; 0.5 mg/mL PS80; 1 mM met; 50 mM NaCl; 0.1 mM EDTA; water; and pH 5.8.

I2158. 190 mg/mL secu; 10 mM Phos-Lact; 200 mM treh; 0.5 mg/mL PS80; 1 mM met; 50 mM NaCl; 0.1 mM EDTA; water; and pH 5.8.

I2159. 150 mg/mL secu; 15 mM Glue; 200 mM treh; 0.5 mg/mL PS80; 1 mM met; 50 mM NaCl; 0.1 mM EDTA; water; and pH 5.8.

I2160. 190 mg/mL secu; 15 mM Glue; 200 mM treh; 0.5 mg/mL PS80; 1 mM met; 50 mM NaCl; 0.1 mM EDTA; water; and pH 5.8.

I2161. 150 mg/mL secu; 0 mM buffer; 150 mM sorb; 0.2 mg/mL PS20; 1 mM met; 50 mM NaCl; 0.1 mM EDTA; water; and pH 5.8.

I2162. 190 mg/mL secu; 0 mM buffer; 150 mM sorb; 0.2 mg/mL PS20; 1 mM met; 50 mM NaCl; 0.1 mM EDTA; water; and pH 5.8.

I2163. 150 mg/mL secu; 50 mM His; 150 mM sorb; 0.2 mg/mL PS20; 1 mM met; 50 mM NaCl; 0.1 mM EDTA; water; and pH 5.8.

I2164. 190 mg/mL secu; 50 mM His; 150 mM sorb; 0.2 mg/mL PS20; 1 mM met; 50 mM NaCl; 0.1 mM EDTA; water; and pH 5.8.

I2165. 150 mg/mL secu; 25 mM Lact; 150 mM sorb; 0.2 mg/mL PS20; 1 mM met; 50 mM NaCl; 0.1 mM EDTA; water; and pH 5.8.

I2166. 190 mg/mL secu; 25 mM Lact; 150 mM sorb; 0.2 mg/mL PS20; 1 mM met; 50 mM NaCl; 0.1 mM EDTA; water; and pH 5.8.

I2167. 150 mg/mL secu; 10 mM Phos-Lact; 150 mM sorb; 0.2 mg/mL PS20; 1 mM met; 50 mM NaCl; 0.1 mM EDTA; water; and pH 5.8.

I2168. 190 mg/mL secu; 10 mM Phos-Lact; 150 mM sorb; 0.2 mg/mL PS20; 1 mM met; 50 mM NaCl; 0.1 mM EDTA; water; and pH 5.8.

I2169. 150 mg/mL secu; 15 mM Glue; 150 mM sorb; 0.2 mg/mL PS20; 1 mM met; 50 mM NaCl; 0.1 mM EDTA; water; and pH 5.8.

I2170. 190 mg/mL secu; 15 mM Glue; 150 mM sorb; 0.2 mg/mL PS20; 1 mM met; 50 mM NaCl; 0.1 mM EDTA; water; and pH 5.8.

I2171. 150 mg/mL secu; 0 mM buffer; 75 mM mann; 0.2 mg/mL PS20; 1 mM met; 50 mM NaCl; 0.1 mM EDTA; water; and pH 5.8.

I2172. 190 mg/mL secu; 0 mM buffer; 75 mM mann; 0.2 mg/mL PS20; 1 mM met; 50 mM NaCl; 0.1 mM EDTA; water; and pH 5.8.

I2173. 150 mg/mL secu; 50 mM His; 75 mM mann; 0.2 mg/mL PS20; 1 mM met; 50 mM NaCl; 0.1 mM EDTA; water; and pH 5.8.

I2174. 190 mg/mL secu; 50 mM His; 75 mM mann; 0.2 mg/mL PS20; 1 mM met; 50 mM NaCl; 0.1 mM EDTA; water; and pH 5.8.

I2175. 150 mg/mL secu; 25 mM Lact; 75 mM mann; 0.2 mg/mL PS20; 1 mM met; 50 mM NaCl; 0.1 mM EDTA; water; and pH 5.8.

I2176. 190 mg/mL secu; 25 mM Lact; 75 mM mann; 0.2 mg/mL PS20; 1 mM met; 50 mM NaCl; 0.1 mM EDTA; water; and pH 5.8.

I2177. 150 mg/mL secu; 10 mM Phos-Lact; 75 mM mann; 0.2 mg/mL PS20; 1 mM met; 50 mM NaCl; 0.1 mM EDTA; water; and pH 5.8.

I2178. 190 mg/mL secu; 10 mM Phos-Lact; 75 mM mann; 0.2 mg/mL PS20; 1 mM met; 50 mM NaCl; 0.1 mM EDTA; water; and pH 5.8.

I2179. 150 mg/mL secu; 15 mM Glue; 75 mM mann; 0.2 mg/mL PS20; 1 mM met; 50 mM NaCl; 0.1 mM EDTA; water; and pH 5.8.

I2180. 190 mg/mL secu; 15 mM Glue; 75 mM mann; 0.2 mg/mL PS20; 1 mM met; 50 mM NaCl; 0.1 mM EDTA; water; and pH 5.8.

(continued)

I2181. 150 mg/mL secu; 0 mM buffer; 100 mM sucr; 0.2 mg/mL PS20; 1 mM met; 50 mM NaCl; 0.1 mM EDTA; water; and pH 5.8.

I2182. 190 mg/mL secu; 0 mM buffer; 100 mM sucr; 0.2 mg/mL PS20; 1 mM met; 50 mM NaCl; 0.1 mM EDTA; water; and pH 5.8.

I2183. 150 mg/mL secu; 50 mM His; 100 mM sucr; 0.2 mg/mL PS20; 1 mM met; 50 mM NaCl; 0.1 mM EDTA; water; and pH 5.8.

I2184. 190 mg/mL secu; 50 mM His; 100 mM sucr; 0.2 mg/mL PS20; 1 mM met; 50 mM NaCl; 0.1 mM EDTA; water; and pH 5.8.

I2185. 150 mg/mL secu; 25 mM Lact; 100 mM sucr; 0.2 mg/mL PS20; 1 mM met; 50 mM NaCl; 0.1 mM EDTA; water; and pH 5.8.

I2186. 190 mg/mL secu; 25 mM Lact; 100 mM sucr; 0.2 mg/mL PS20; 1 mM met; 50 mM NaCl; 0.1 mM EDTA; water; and pH 5.8.

I2187. 150 mg/mL secu; 10 mM Phos-Lact; 100 mM sucr; 0.2 mg/mL PS20; 1 mM met; 50 mM NaCl; 0.1 mM EDTA; water; and pH 5.8.

I2188. 190 mg/mL secu; 10 mM Phos-Lact; 100 mM sucr; 0.2 mg/mL PS20; 1 mM met; 50 mM NaCl; 0.1 mM EDTA; water; and pH 5.8.

I2189. 150 mg/mL secu; 15 mM Glue; 100 mM sucr; 0.2 mg/mL PS20; 1 mM met; 50 mM NaCl; 0.1 mM EDTA; water; and pH 5.8.

I2190. 190 mg/mL secu; 15 mM Glue; 100 mM sucr; 0.2 mg/mL PS20; 1 mM met; 50 mM NaCl; 0.1 mM EDTA; water; and pH 5.8.

I2191. 150 mg/mL secu; 0 mM buffer; 200 mM treh; 0.2 mg/mL PS20; 1 mM met; 50 mM NaCl; 0.1 mM EDTA; water; and pH 5.8.

I2192. 190 mg/mL secu; 0 mM buffer; 200 mM treh; 0.2 mg/mL PS20; 1 mM met; 50 mM NaCl; 0.1 mM EDTA; water; and pH 5.8.

I2193. 150 mg/mL secu; 50 mM His; 200 mM treh; 0.2 mg/mL PS20; 1 mM met; 50 mM NaCl; 0.1 mM EDTA; water; and pH 5.8.

I2194. 190 mg/mL secu; 50 mM His; 200 mM treh; 0.2 mg/mL PS20; 1 mM met; 50 mM NaCl; 0.1 mM EDTA; water; and pH 5.8.

I2195. 150 mg/mL secu; 25 mM Lact; 200 mM treh; 0.2 mg/mL PS20; 1 mM met; 50 mM NaCl; 0.1 mM EDTA; water; and pH 5.8.

I2196. 190 mg/mL secu; 25 mM Lact; 200 mM treh; 0.2 mg/mL PS20; 1 mM met; 50 mM NaCl; 0.1 mM EDTA; water; and pH 5.8.

I2197. 150 mg/mL secu; 10 mM Phos-Lact; 200 mM treh; 0.2 mg/mL PS20; 1 mM met; 50 mM NaCl; 0.1 mM EDTA; water; and pH 5.8.

I2198. 190 mg/mL secu; 10 mM Phos-Lact; 200 mM treh; 0.2 mg/mL PS20; 1 mM met; 50 mM NaCl; 0.1 mM EDTA; water; and pH 5.8.

I2199. 150 mg/mL secu; 15 mM Glue; 200 mM treh; 0.2 mg/mL PS20; 1 mM met; 50 mM NaCl; 0.1 mM EDTA; water; and pH 5.8.

I2200. 190 mg/mL secu; 15 mM Glue; 200 mM treh; 0.2 mg/mL PS20; 1 mM met; 50 mM NaCl; 0.1 mM EDTA; water; and pH 5.8.

I2201. 150 mg/mL secu; 0 mM buffer; 50 mM arg; 0.5 mg/mL PS80; 1 mM met; 50 mM NaCl; 0.1 mM EDTA; water; and pH 5.8.

I2202. 190 mg/mL secu; 0 mM buffer; 50 mM arg; 0.5 mg/mL PS80; 1 mM met; 50 mM NaCl; 0.1 mM EDTA; water; and pH 5.8.

I2203. 150 mg/mL secu; 50 mM His; 50 mM arg; 0.5 mg/mL PS80; 1 mM met; 50 mM NaCl; 0.1 mM EDTA; water; and pH 5.8.

I2204. 190 mg/mL secu; 50 mM His; 50 mM arg; 0.5 mg/mL PS80; 1 mM met; 50 mM NaCl; 0.1 mM EDTA; water; and pH 5.8.

I2205. 150 mg/mL secu; 25 mM Lact; 50 mM arg; 0.5 mg/mL PS80; 1 mM met; 50 mM NaCl; 0.1 mM EDTA; water; and pH 5.8.

I2206. 190 mg/mL secu; 25 mM Lact; 50 mM arg; 0.5 mg/mL PS80; 1 mM met; 50 mM NaCl; 0.1 mM EDTA; water; and pH 5.8.

(continued)

I2207. 150 mg/mL secu; 10 mM Phos-Lact; 50 mM arg; 0.5 mg/mL PS80; 1 mM met; 50 mM NaCl; 0.1 mM EDTA; water; and pH 5.8.

I2208. 190 mg/mL secu; 10 mM Phos-Lact; 50 mM arg; 0.5 mg/mL PS80; 1 mM met; 50 mM NaCl; 0.1 mM EDTA; water; and pH 5.8.

I2209. 150 mg/mL secu; 15 mM Glue; 50 mM arg; 0.5 mg/mL PS80; 1 mM met; 50 mM NaCl; 0.1 mM EDTA; water; and pH 5.8.

I2210. 190 mg/mL secu; 15 mM Glue; 50 mM arg; 0.5 mg/mL PS80; 1 mM met; 50 mM NaCl; 0.1 mM EDTA; water; and pH 5.8.

I2211. 150 mg/mL secu; 0 mM buffer; 100 mM gly; 0.5 mg/mL PS80; 1 mM met; 50 mM NaCl; 0.1 mM EDTA; water; and pH 5.8.

I2212. 190 mg/mL secu; 0 mM buffer; 100 mM gly; 0.5 mg/mL PS80; 1 mM met; 50 mM NaCl; 0.1 mM EDTA; water; and pH 5.8.

I2213. 150 mg/mL secu; 50 mM His; 100 mM gly; 0.5 mg/mL PS80; 1 mM met; 50 mM NaCl; 0.1 mM EDTA; water; and pH 5.8.

I2214. 190 mg/mL secu; 50 mM His; 100 mM gly; 0.5 mg/mL PS80; 1 mM met; 50 mM NaCl; 0.1 mM EDTA; water; and pH 5.8.

I2215. 150 mg/mL secu; 25 mM Lact; 100 mM gly; 0.5 mg/mL PS80; 1 mM met; 50 mM NaCl; 0.1 mM EDTA; water; and pH 5.8.

I2216. 190 mg/mL secu; 25 mM Lact; 100 mM gly; 0.5 mg/mL PS80; 1 mM met; 50 mM NaCl; 0.1 mM EDTA; water; and pH 5.8.

I2217. 150 mg/mL secu; 10 mM Phos-Lact; 100 mM gly; 0.5 mg/mL PS80; 1 mM met; 50 mM NaCl; 0.1 mM EDTA; water; and pH 5.8.

I2218. 190 mg/mL secu; 10 mM Phos-Lact; 100 mM gly; 0.5 mg/mL PS80; 1 mM met; 50 mM NaCl; 0.1 mM EDTA; water; and pH 5.8.

I2219. 150 mg/mL secu; 15 mM Glue; 100 mM gly; 0.5 mg/mL PS80; 1 mM met; 50 mM NaCl; 0.1 mM EDTA; water; and pH 5.8.

I2220. 190 mg/mL secu; 15 mM Glue; 100 mM gly; 0.5 mg/mL PS80; 1 mM met; 50 mM NaCl; 0.1 mM EDTA; water; and pH 5.8.

I2221. 150 mg/mL secu; 0 mM buffer; 50 mM arg; 0.2 mg/mL PS20; 1 mM met; 50 mM NaCl; 0.1 mM EDTA; water; and pH 5.8.

I2222. 190 mg/mL secu; 0 mM buffer; 50 mM arg; 0.2 mg/mL PS20; 1 mM met; 50 mM NaCl; 0.1 mM EDTA; water; and pH 5.8.

I2223. 150 mg/mL secu; 50 mM His; 50 mM arg; 0.2 mg/mL PS20; 1 mM met; 50 mM NaCl; 0.1 mM EDTA; water; and pH 5.8.

I2224. 190 mg/mL secu; 50 mM His; 50 mM arg; 0.2 mg/mL PS20; 1 mM met; 50 mM NaCl; 0.1 mM EDTA; water; and pH 5.8.

I2225. 150 mg/mL secu; 25 mM Lact; 50 mM arg; 0.2 mg/mL PS20; 1 mM met; 50 mM NaCl; 0.1 mM EDTA; water; and pH 5.8.

I2226. 190 mg/mL secu; 25 mM Lact; 50 mM arg; 0.2 mg/mL PS20; 1 mM met; 50 mM NaCl; 0.1 mM EDTA; water; and pH 5.8.

I2227. 150 mg/mL secu; 10 mM Phos-Lact; 50 mM arg; 0.2 mg/mL PS20; 1 mM met; 50 mM NaCl; 0.1 mM EDTA; water; and pH 5.8.

I2228. 190 mg/mL secu; 10 mM Phos-Lact; 50 mM arg; 0.2 mg/mL PS20; 1 mM met; 50 mM NaCl; 0.1 mM EDTA; water; and pH 5.8.

I2229. 150 mg/mL secu; 15 mM Glue; 50 mM arg; 0.2 mg/mL PS20; 1 mM met; 50 mM NaCl; 0.1 mM EDTA; water; and pH 5.8.

I2230. 190 mg/mL secu; 15 mM Glue; 50 mM arg; 0.2 mg/mL PS20; 1 mM met; 50 mM NaCl; 0.1 mM EDTA; water; and pH 5.8.

I2231 . 150 mg/mL secu; 0 mM buffer; 100 mM gly; 0.2 mg/mL PS20; 1 mM met; 50 mM NaCl; 0.1 mM EDTA; water; and pH 5.8.

I2232. 190 mg/mL secu; 0 mM buffer; 100 mM gly; 0.2 mg/mL PS20; 1 mM met; 50 mM NaCl; 0.1 mM EDTA; water; and pH 5.8.

(continued)

I2233. 150 mg/mL secu; 50 mM His; 100 mM gly; 0.2 mg/mL PS20; 1 mM met; 50 mM NaCl; 0.1 mM EDTA; water; and pH 5.8.

I2234. 190 mg/mL secu; 50 mM His; 100 mM gly; 0.2 mg/mL PS20; 1 mM met; 50 mM NaCl; 0.1 mM EDTA; water; and pH 5.8.

I2235. 150 mg/mL secu; 25 mM Lact; 100 mM gly; 0.2 mg/mL PS20; 1 mM met; 50 mM NaCl; 0.1 mM EDTA; water; and pH 5.8.

I2236. 190 mg/mL secu; 25 mM Lact; 100 mM gly; 0.2 mg/mL PS20; 1 mM met; 50 mM NaCl; 0.1 mM EDTA; water; and pH 5.8.

I2237. 150 mg/mL secu; 10 mM Phos-Lact; 100 mM gly; 0.2 mg/mL PS20; 1 mM met; 50 mM NaCl; 0.1 mM EDTA; water; and pH 5.8.

I2238. 190 mg/mL secu; 10 mM Phos-Lact; 100 mM gly; 0.2 mg/mL PS20; 1 mM met; 50 mM NaCl; 0.1 mM EDTA; water; and pH 5.8.

I2239. 150 mg/mL secu; 15 mM Glue; 100 mM gly; 0.2 mg/mL PS20; 1 mM met; 50 mM NaCl; 0.1 mM EDTA; water; and pH 5.8.

I2240. 190 mg/mL secu; 15 mM Glue; 100 mM gly; 0.2 mg/mL PS20; 1 mM met; 50 mM NaCl; 0.1 mM EDTA; water; and pH 5.8.

I2241. 150 mg/mL secu; 0 mM buffer; 150 mM sorb; 50 mM arg; 0.5 mg/mL PS80; 1 mM met; 50 mM NaCl; 0.1 mM EDTA; water; and pH 5.8.

I2242. 190 mg/mL secu; 0 mM buffer; 150 mM sorb; 50 mM arg; 0.5 mg/mL PS80; 1 mM met; 50 mM NaCl; 0.1 mM EDTA; water; and pH 5.8.

I2243. 150 mg/mL secu; 50 mM His; 150 mM sorb; 50 mM arg; 0.5 mg/mL PS80; 1 mM met; 50 mM NaCl; 0.1 mM EDTA; water; and pH 5.8.

I2244. 190 mg/mL secu; 50 mM His; 150 mM sorb; 50 mM arg; 0.5 mg/mL PS80; 1 mM met; 50 mM NaCl; 0.1 mM EDTA; water; and pH 5.8.

I2245. 150 mg/mL secu; 25 mM Lact; 150 mM sorb; 50 mM arg; 0.5 mg/mL PS80; 1 mM met; 50 mM NaCl; 0.1 mM EDTA; water; and pH 5.8.

I2246. 190 mg/mL secu; 25 mM Lact; 150 mM sorb; 50 mM arg; 0.5 mg/mL PS80; 1 mM met; 50 mM NaCl; 0.1 mM EDTA; water; and pH 5.8.

I2247. 150 mg/mL secu; 10 mM Phos-Lact; 150 mM sorb; 50 mM arg; 0.5 mg/mL PS80; 1 mM met; 50 mM NaCl; 0.1 mM EDTA; water; and pH 5.8.

I2248. 190 mg/mL secu; 10 mM Phos-Lact; 150 mM sorb; 50 mM arg; 0.5 mg/mL PS80; 1 mM met; 50 mM NaCl; 0.1 mM EDTA; water; and pH 5.8.

I2249. 150 mg/mL secu; 15 mM Glue; 150 mM sorb; 50 mM arg; 0.5 mg/mL PS80; 1 mM met; 50 mM NaCl; 0.1 mM EDTA; water; and pH 5.8.

I2250. 190 mg/mL secu; 15 mM Glue; 150 mM sorb; 50 mM arg; 0.5 mg/mL PS80; 1 mM met; 50 mM NaCl; 0.1 mM EDTA; water; and pH 5.8.

I2251. 150 mg/mL secu; 0 mM buffer; 75 mM mann; 50 mM arg; 0.5 mg/mL PS80; 1 mM met; 50 mM NaCl; 0.1 mM EDTA; water; and pH 5.8.

I2252. 190 mg/mL secu; 0 mM buffer; 75 mM mann; 50 mM arg; 0.5 mg/mL PS80; 1 mM met; 50 mM NaCl; 0.1 mM EDTA; water; and pH 5.8.

I2253. 150 mg/mL secu; 50 mM His; 75 mM mann; 50 mM arg; 0.5 mg/mL PS80; 1 mM met; 50 mM NaCl; 0.1 mM EDTA; water; and pH 5.8.

I2254. 190 mg/mL secu; 50 mM His; 75 mM mann; 50 mM arg; 0.5 mg/mL PS80; 1 mM met; 50 mM NaCl; 0.1 mM EDTA; water; and pH 5.8.

I2255. 150 mg/mL secu; 25 mM Lact; 75 mM mann; 50 mM arg; 0.5 mg/mL PS80; 1 mM met; 50 mM NaCl; 0.1 mM EDTA; water; and pH 5.8.

I2256. 190 mg/mL secu; 25 mM Lact; 75 mM mann; 50 mM arg; 0.5 mg/mL PS80; 1 mM met; 50 mM NaCl; 0.1 mM EDTA; water; and pH 5.8.

I2257. 150 mg/mL secu; 10 mM Phos-Lact; 75 mM mann; 50 mM arg; 0.5 mg/mL PS80; 1 mM met; 50 mM NaCl; 0.1 mM EDTA; water; and pH 5.8.

I2258. 190 mg/mL secu; 10 mM Phos-Lact; 75 mM mann; 50 mM arg; 0.5 mg/mL PS80; 1 mM met; 50 mM NaCl; 0.1 mM EDTA; water; and pH 5.8.

(continued)

I2259. 150 mg/mL secu; 15 mM Glue; 75 mM mann; 50 mM arg; 0.5 mg/mL PS80; 1 mM met; 50 mM NaCl; 0.1 mM EDTA; water; and pH 5.8.

I2260. 190 mg/mL secu; 15 mM Glue; 75 mM mann; 50 mM arg; 0.5 mg/mL PS80; 1 mM met; 50 mM NaCl; 0.1 mM EDTA; water; and pH 5.8.

I2261. 150 mg/mL secu; 0 mM buffer; 100 mM sucr; 50 mM arg; 0.5 mg/mL PS80; 1 mM met; 50 mM NaCl; 0.1 mM EDTA; water; and pH 5.8.

I2262. 190 mg/mL secu; 0 mM buffer; 100 mM sucr; 50 mM arg; 0.5 mg/mL PS80; 1 mM met; 50 mM NaCl; 0.1 mM EDTA; water; and pH 5.8.

I2263. 150 mg/mL secu; 50 mM His; 100 mM sucr; 50 mM arg; 0.5 mg/mL PS80; 1 mM met; 50 mM NaCl; 0.1 mM EDTA; water; and pH 5.8.

I2264. 190 mg/mL secu; 50 mM His; 100 mM sucr; 50 mM arg; 0.5 mg/mL PS80; 1 mM met; 50 mM NaCl; 0.1 mM EDTA; water; and pH 5.8.

I2265. 150 mg/mL secu; 25 mM Lact; 100 mM sucr; 50 mM arg; 0.5 mg/mL PS80; 1 mM met; 50 mM NaCl; 0.1 mM EDTA; water; and pH 5.8.

I2266. 190 mg/mL secu; 25 mM Lact; 100 mM sucr; 50 mM arg; 0.5 mg/mL PS80; 1 mM met; 50 mM NaCl; 0.1 mM EDTA; water; and pH 5.8.

I2267. 150 mg/mL secu; 10 mM Phos-Lact; 100 mM sucr; 50 mM arg; 0.5 mg/mL PS80; 1 mM met; 50 mM NaCl; 0.1 mM EDTA; water; and pH 5.8.

I2268. 190 mg/mL secu; 10 mM Phos-Lact; 100 mM sucr; 50 mM arg; 0.5 mg/mL PS80; 1 mM met; 50 mM NaCl; 0.1 mM EDTA; water; and pH 5.8.

I2269. 150 mg/mL secu; 15 mM Glue; 100 mM sucr; 50 mM arg; 0.5 mg/mL PS80; 1 mM met; 50 mM NaCl; 0.1 mM EDTA; water; and pH 5.8.

I2270. 190 mg/mL secu; 15 mM Glue; 100 mM sucr; 50 mM arg; 0.5 mg/mL PS80; 1 mM met; 50 mM NaCl; 0.1 mM EDTA; water; and pH 5.8.

I2271. 150 mg/mL secu; 0 mM buffer; 200 mM treh; 50 mM arg; 0.5 mg/mL PS80; 1 mM met; 50 mM NaCl; 0.1 mM EDTA; water; and pH 5.8.

I2272. 190 mg/mL secu; 0 mM buffer; 200 mM treh; 50 mM arg; 0.5 mg/mL PS80; 1 mM met; 50 mM NaCl; 0.1 mM EDTA; water; and pH 5.8.

I2273. 150 mg/mL secu; 50 mM His; 200 mM treh; 50 mM arg; 0.5 mg/mL PS80; 1 mM met; 50 mM NaCl; 0.1 mM EDTA; water; and pH 5.8.

I2274. 190 mg/mL secu; 50 mM His; 200 mM treh; 50 mM arg; 0.5 mg/mL PS80; 1 mM met; 50 mM NaCl; 0.1 mM EDTA; water; and pH 5.8.

I2275. 150 mg/mL secu; 25 mM Lact; 200 mM treh; 50 mM arg; 0.5 mg/mL PS80; 1 mM met; 50 mM NaCl; 0.1 mM EDTA; water; and pH 5.8.

I2276. 190 mg/mL secu; 25 mM Lact; 200 mM treh; 50 mM arg; 0.5 mg/mL PS80; 1 mM met; 50 mM NaCl; 0.1 mM EDTA; water; and pH 5.8.

I2277. 150 mg/mL secu; 10 mM Phos-Lact; 200 mM treh; 50 mM arg; 0.5 mg/mL PS80; 1 mM met; 50 mM NaCl; 0.1 mM EDTA; water; and pH 5.8.

I2278. 190 mg/mL secu; 10 mM Phos-Lact; 200 mM treh; 50 mM arg; 0.5 mg/mL PS80; 1 mM met; 50 mM NaCl; 0.1 mM EDTA; water; and pH 5.8.

I2279. 150 mg/mL secu; 15 mM Glue; 200 mM treh; 50 mM arg; 0.5 mg/mL PS80; 1 mM met; 50 mM NaCl; 0.1 mM EDTA; water; and pH 5.8.

I2280. 190 mg/mL secu; 15 mM Glue; 200 mM treh; 50 mM arg; 0.5 mg/mL PS80; 1 mM met; 50 mM NaCl; 0.1 mM EDTA; water; and pH 5.8.

I2281. 150 mg/mL secu; 0 mM buffer; 150 mM sorb; 100 mM gly; 0.5 mg/mL PS80; 1 mM met; 50 mM NaCl; 0.1 mM EDTA; water; and pH 5.8.

I2282. 190 mg/mL secu; 0 mM buffer; 150 mM sorb; 100 mM gly; 0.5 mg/mL PS80; 1 mM met; 50 mM NaCl; 0.1 mM EDTA; water; and pH 5.8.

I2283. 150 mg/mL secu; 50 mM His; 150 mM sorb; 100 mM gly; 0.5 mg/mL PS80; 1 mM met; 50 mM NaCl; 0.1 mM EDTA; water; and pH 5.8.

I2284. 190 mg/mL secu; 50 mM His; 150 mM sorb; 100 mM gly; 0.5 mg/mL PS80; 1 mM met; 50 mM NaCl; 0.1 mM EDTA; water; and pH 5.8.

(continued)

I2285. 150 mg/mL secu; 25 mM Lact; 150 mM sorb; 100 mM gly; 0.5 mg/mL PS80; 1 mM met; 50 mM NaCl; 0.1 mM EDTA; water; and pH 5.8.

I2286. 190 mg/mL secu; 25 mM Lact; 150 mM sorb; 100 mM gly; 0.5 mg/mL PS80; 1 mM met; 50 mM NaCl; 0.1 mM EDTA; water; and pH 5.8.

I2287. 150 mg/mL secu; 10 mM Phos-Lact; 150 mM sorb; 100 mM gly; 0.5 mg/mL PS80; 1 mM met; 50 mM NaCl; 0.1 mM EDTA; water; and pH 5.8.

I2288. 190 mg/mL secu; 10 mM Phos-Lact; 150 mM sorb; 100 mM gly; 0.5 mg/mL PS80; 1 mM met; 50 mM NaCl; 0.1 mM EDTA; water; and pH 5.8.

I2289. 150 mg/mL secu; 15 mM Glue; 150 mM sorb; 100 mM gly; 0.5 mg/mL PS80; 1 mM met; 50 mM NaCl; 0.1 mM EDTA; water; and pH 5.8.

I2290. 190 mg/mL secu; 15 mM Glue; 150 mM sorb; 100 mM gly; 0.5 mg/mL PS80; 1 mM met; 50 mM NaCl; 0.1 mM EDTA; water; and pH 5.8.

I2291 . 150 mg/mL secu; 0 mM buffer; 75 mM mann; 100 mM gly; 0.5 mg/mL PS80; 1 mM met; 50 mM NaCl; 0.1 mM EDTA; water; and pH 5.8.

I2292. 190 mg/mL secu; 0 mM buffer; 75 mM mann; 100 mM gly; 0.5 mg/mL PS80; 1 mM met; 50 mM NaCl; 0.1 mM EDTA; water; and pH 5.8.

I2293. 150 mg/mL secu; 50 mM His; 75 mM mann; 100 mM gly; 0.5 mg/mL PS80; 1 mM met; 50 mM NaCl; 0.1 mM EDTA; water; and pH 5.8.

I2294. 190 mg/mL secu; 50 mM His; 75 mM mann; 100 mM gly; 0.5 mg/mL PS80; 1 mM met; 50 mM NaCl; 0.1 mM EDTA; water; and pH 5.8.

I2295. 150 mg/mL secu; 25 mM Lact; 75 mM mann; 100 mM gly; 0.5 mg/mL PS80; 1 mM met; 50 mM NaCl; 0.1 mM EDTA; water; and pH 5.8.

I2296. 190 mg/mL secu; 25 mM Lact; 75 mM mann; 100 mM gly; 0.5 mg/mL PS80; 1 mM met; 50 mM NaCl; 0.1 mM EDTA; water; and pH 5.8.

I2297. 150 mg/mL secu; 10 mM Phos-Lact; 75 mM mann; 100 mM gly; 0.5 mg/mL PS80; 1 mM met; 50 mM NaCl; 0.1 mM EDTA; water; and pH 5.8.

I2298. 190 mg/mL secu; 10 mM Phos-Lact; 75 mM mann; 100 mM gly; 0.5 mg/mL PS80; 1 mM met; 50 mM NaCl; 0.1 mM EDTA; water; and pH 5.8.

I2299. 150 mg/mL secu; 15 mM Glue; 75 mM mann; 100 mM gly; 0.5 mg/mL PS80; 1 mM met; 50 mM NaCl; 0.1 mM EDTA; water; and pH 5.8.

I2300. 190 mg/mL secu; 15 mM Glue; 75 mM mann; 100 mM gly; 0.5 mg/mL PS80; 1 mM met; 50 mM NaCl; 0.1 mM EDTA; water; and pH 5.8.

I2301. 150 mg/mL secu; 0 mM buffer; 100 mM sucr; 100 mM gly; 0.5 mg/mL PS80; 1 mM met; 50 mM NaCl; 0.1 mM EDTA; water; and pH 5.8.

I2302. 190 mg/mL secu; 0 mM buffer; 100 mM sucr; 100 mM gly; 0.5 mg/mL PS80; 1 mM met; 50 mM NaCl; 0.1 mM EDTA; water; and pH 5.8.

I2303. 150 mg/mL secu; 50 mM His; 100 mM sucr; 100 mM gly; 0.5 mg/mL PS80; 1 mM met; 50 mM NaCl; 0.1 mM EDTA; water; and pH 5.8.

I2304. 190 mg/mL secu; 50 mM His; 100 mM sucr; 100 mM gly; 0.5 mg/mL PS80; 1 mM met; 50 mM NaCl; 0.1 mM EDTA; water; and pH 5.8.

I2305. 150 mg/mL secu; 25 mM Lact; 100 mM sucr; 100 mM gly; 0.5 mg/mL PS80; 1 mM met; 50 mM NaCl; 0.1 mM EDTA; water; and pH 5.8.

I2306. 190 mg/mL secu; 25 mM Lact; 100 mM sucr; 100 mM gly; 0.5 mg/mL PS80; 1 mM met; 50 mM NaCl; 0.1 mM EDTA; water; and pH 5.8.

I2307. 150 mg/mL secu; 10 mM Phos-Lact; 100 mM sucr; 100 mM gly; 0.5 mg/mL PS80; 1 mM met; 50 mM NaCl; 0.1 mM EDTA; water; and pH 5.8.

I2308. 190 mg/mL secu; 10 mM Phos-Lact; 100 mM sucr; 100 mM gly; 0.5 mg/mL PS80; 1 mM met; 50 mM NaCl; 0.1 mM EDTA; water; and pH 5.8.

I2309. 150 mg/mL secu; 15 mM Glue; 100 mM sucr; 100 mM gly; 0.5 mg/mL PS80; 1 mM met; 50 mM NaCl; 0.1 mM EDTA; water; and pH 5.8.

I2310. 190 mg/mL secu; 15 mM Glue; 100 mM sucr; 100 mM gly; 0.5 mg/mL PS80; 1 mM met; 50 mM NaCl; 0.1 mM EDTA; water; and pH 5.8.

(continued)

I2311. 150 mg/mL secu; 0 mM buffer; 200 mM treh; 100 mM gly; 0.5 mg/mL PS80; 1 mM met; 50 mM NaCl; 0.1 mM EDTA; water; and pH 5.8.

I2312. 190 mg/mL secu; 0 mM buffer; 200 mM treh; 100 mM gly; 0.5 mg/mL PS80; 1 mM met; 50 mM NaCl; 0.1 mM EDTA; water; and pH 5.8.

I2313. 150 mg/mL secu; 50 mM His; 200 mM treh; 100 mM gly; 0.5 mg/mL PS80; 1 mM met; 50 mM NaCl; 0.1 mM EDTA; water; and pH 5.8.

I2314. 190 mg/mL secu; 50 mM His; 200 mM treh; 100 mM gly; 0.5 mg/mL PS80; 1 mM met; 50 mM NaCl; 0.1 mM EDTA; water; and pH 5.8.

I2315. 150 mg/mL secu; 25 mM Lact; 200 mM treh; 100 mM gly; 0.5 mg/mL PS80; 1 mM met; 50 mM NaCl; 0.1 mM EDTA; water; and pH 5.8.

I2316. 190 mg/mL secu; 25 mM Lact; 200 mM treh; 100 mM gly; 0.5 mg/mL PS80; 1 mM met; 50 mM NaCl; 0.1 mM EDTA; water; and pH 5.8.

I2317. 150 mg/mL secu; 10 mM Phos-Lact; 200 mM treh; 100 mM gly; 0.5 mg/mL PS80; 1 mM met; 50 mM NaCl; 0.1 mM EDTA; water; and pH 5.8.

I2318. 190 mg/mL secu; 10 mM Phos-Lact; 200 mM treh; 100 mM gly; 0.5 mg/mL PS80; 1 mM met; 50 mM NaCl; 0.1 mM EDTA; water; and pH 5.8.

I2319. 150 mg/mL secu; 15 mM Glue; 200 mM treh; 100 mM gly; 0.5 mg/mL PS80; 1 mM met; 50 mM NaCl; 0.1 mM EDTA; water; and pH 5.8.

I2320. 190 mg/mL secu; 15 mM Glue; 200 mM treh; 100 mM gly; 0.5 mg/mL PS80; 1 mM met; 50 mM NaCl; 0.1 mM EDTA; water; and pH 5.8.

I2321. 150 mg/mL secu; 0 mM buffer; 150 mM sorb; 50 mM arg; 0.2 mg/mL PS20; 1 mM met; 50 mM NaCl; 0.1 mM EDTA; water; and pH 5.8.

I2322. 190 mg/mL secu; 0 mM buffer; 150 mM sorb; 50 mM arg; 0.2 mg/mL PS20; 1 mM met; 50 mM NaCl; 0.1 mM EDTA; water; and pH 5.8.

I2323. 150 mg/mL secu; 50 mM His; 150 mM sorb; 50 mM arg; 0.2 mg/mL PS20; 1 mM met; 50 mM NaCl; 0.1 mM EDTA; water; and pH 5.8.

I2324. 190 mg/mL secu; 50 mM His; 150 mM sorb; 50 mM arg; 0.2 mg/mL PS20; 1 mM met; 50 mM NaCl; 0.1 mM EDTA; water; and pH 5.8.

I2325. 150 mg/mL secu; 25 mM Lact; 150 mM sorb; 50 mM arg; 0.2 mg/mL PS20; 1 mM met; 50 mM NaCl; 0.1 mM EDTA; water; and pH 5.8.

I2326. 190 mg/mL secu; 25 mM Lact; 150 mM sorb; 50 mM arg; 0.2 mg/mL PS20; 1 mM met; 50 mM NaCl; 0.1 mM EDTA; water; and pH 5.8.

I2327. 150 mg/mL secu; 10 mM Phos-Lact; 150 mM sorb; 50 mM arg; 0.2 mg/mL PS20; 1 mM met; 50 mM NaCl; 0.1 mM EDTA; water; and pH 5.8.

I2328. 190 mg/mL secu; 10 mM Phos-Lact; 150 mM sorb; 50 mM arg; 0.2 mg/mL PS20; 1 mM met; 50 mM NaCl; 0.1 mM EDTA; water; and pH 5.8.

I2329. 150 mg/mL secu; 15 mM Glue; 150 mM sorb; 50 mM arg; 0.2 mg/mL PS20; 1 mM met; 50 mM NaCl; 0.1 mM EDTA; water; and pH 5.8.

I2330. 190 mg/mL secu; 15 mM Glue; 150 mM sorb; 50 mM arg; 0.2 mg/mL PS20; 1 mM met; 50 mM NaCl; 0.1 mM EDTA; water; and pH 5.8.

I2331. 150 mg/mL secu; 0 mM buffer; 75 mM mann; 50 mM arg; 0.2 mg/mL PS20; 1 mM met; 50 mM NaCl; 0.1 mM EDTA; water; and pH 5.8.

I2332. 190 mg/mL secu; 0 mM buffer; 75 mM mann; 50 mM arg; 0.2 mg/mL PS20; 1 mM met; 50 mM NaCl; 0.1 mM EDTA; water; and pH 5.8.

I2333. 150 mg/mL secu; 50 mM His; 75 mM mann; 50 mM arg; 0.2 mg/mL PS20; 1 mM met; 50 mM NaCl; 0.1 mM EDTA; water; and pH 5.8.

I2334. 190 mg/mL secu; 50 mM His; 75 mM mann; 50 mM arg; 0.2 mg/mL PS20; 1 mM met; 50 mM NaCl; 0.1 mM EDTA; water; and pH 5.8.

I2335. 150 mg/mL secu; 25 mM Lact; 75 mM mann; 50 mM arg; 0.2 mg/mL PS20; 1 mM met; 50 mM NaCl; 0.1 mM EDTA; water; and pH 5.8.

I2336. 190 mg/mL secu; 25 mM Lact; 75 mM mann; 50 mM arg; 0.2 mg/mL PS20; 1 mM met; 50 mM NaCl; 0.1 mM EDTA; water; and pH 5.8.

(continued)

I2337. 150 mg/mL secu; 10 mM Phos-Lact; 75 mM mann; 50 mM arg; 0.2 mg/mL PS20; 1 mM met; 50 mM NaCl; 0.1 mM EDTA; water; and pH 5.8.

I2338. 190 mg/mL secu; 10 mM Phos-Lact; 75 mM mann; 50 mM arg; 0.2 mg/mL PS20; 1 mM met; 50 mM NaCl; 0.1 mM EDTA; water; and pH 5.8.

I2339. 150 mg/mL secu; 15 mM Glue; 75 mM mann; 50 mM arg; 0.2 mg/mL PS20; 1 mM met; 50 mM NaCl; 0.1 mM EDTA; water; and pH 5.8.

I2340. 190 mg/mL secu; 15 mM Glue; 75 mM mann; 50 mM arg; 0.2 mg/mL PS20; 1 mM met; 50 mM NaCl; 0.1 mM EDTA; water; and pH 5.8.

I2341. 150 mg/mL secu; 0 mM buffer; 100 mM sucr; 50 mM arg; 0.2 mg/mL PS20; 1 mM met; 50 mM NaCl; 0.1 mM EDTA; water; and pH 5.8.

I2342. 190 mg/mL secu; 0 mM buffer; 100 mM sucr; 50 mM arg; 0.2 mg/mL PS20; 1 mM met; 50 mM NaCl; 0.1 mM EDTA; water; and pH 5.8.

I2343. 150 mg/mL secu; 50 mM His; 100 mM sucr; 50 mM arg; 0.2 mg/mL PS20; 1 mM met; 50 mM NaCl; 0.1 mM EDTA; water; and pH 5.8.

I2344. 190 mg/mL secu; 50 mM His; 100 mM sucr; 50 mM arg; 0.2 mg/mL PS20; 1 mM met; 50 mM NaCl; 0.1 mM EDTA; water; and pH 5.8.

I2345. 150 mg/mL secu; 25 mM Lact; 100 mM sucr; 50 mM arg; 0.2 mg/mL PS20; 1 mM met; 50 mM NaCl; 0.1 mM EDTA; water; and pH 5.8.

I2346. 190 mg/mL secu; 25 mM Lact; 100 mM sucr; 50 mM arg; 0.2 mg/mL PS20; 1 mM met; 50 mM NaCl; 0.1 mM EDTA; water; and pH 5.8.

I2347. 150 mg/mL secu; 10 mM Phos-Lact; 100 mM sucr; 50 mM arg; 0.2 mg/mL PS20; 1 mM met; 50 mM NaCl; 0.1 mM EDTA; water; and pH 5.8.

I2348. 190 mg/mL secu; 10 mM Phos-Lact; 100 mM sucr; 50 mM arg; 0.2 mg/mL PS20; 1 mM met; 50 mM NaCl; 0.1 mM EDTA; water; and pH 5.8.

I2349. 150 mg/mL secu; 15 mM Glue; 100 mM sucr; 50 mM arg; 0.2 mg/mL PS20; 1 mM met; 50 mM NaCl; 0.1 mM EDTA; water; and pH 5.8.

I2350. 190 mg/mL secu; 15 mM Glue; 100 mM sucr; 50 mM arg; 0.2 mg/mL PS20; 1 mM met; 50 mM NaCl; 0.1 mM EDTA; water; and pH 5.8.

I2351. 150 mg/mL secu; 0 mM buffer; 200 mM treh; 50 mM arg; 0.2 mg/mL PS20; 1 mM met; 50 mM NaCl; 0.1 mM EDTA; water; and pH 5.8.

I2352. 190 mg/mL secu; 0 mM buffer; 200 mM treh; 50 mM arg; 0.2 mg/mL PS20; 1 mM met; 50 mM NaCl; 0.1 mM EDTA; water; and pH 5.8.

I2353. 150 mg/mL secu; 50 mM His; 200 mM treh; 50 mM arg; 0.2 mg/mL PS20; 1 mM met; 50 mM NaCl; 0.1 mM EDTA; water; and pH 5.8.

I2354. 190 mg/mL secu; 50 mM His; 200 mM treh; 50 mM arg; 0.2 mg/mL PS20; 1 mM met; 50 mM NaCl; 0.1 mM EDTA; water; and pH 5.8.

I2355. 150 mg/mL secu; 25 mM Lact; 200 mM treh; 50 mM arg; 0.2 mg/mL PS20; 1 mM met; 50 mM NaCl; 0.1 mM EDTA; water; and pH 5.8.

I2356. 190 mg/mL secu; 25 mM Lact; 200 mM treh; 50 mM arg; 0.2 mg/mL PS20; 1 mM met; 50 mM NaCl; 0.1 mM EDTA; water; and pH 5.8.

I2357. 150 mg/mL secu; 10 mM Phos-Lact; 200 mM treh; 50 mM arg; 0.2 mg/mL PS20; 1 mM met; 50 mM NaCl; 0.1 mM EDTA; water; and pH 5.8.

I2358. 190 mg/mL secu; 10 mM Phos-Lact; 200 mM treh; 50 mM arg; 0.2 mg/mL PS20; 1 mM met; 50 mM NaCl; 0.1 mM EDTA; water; and pH 5.8.

I2359. 150 mg/mL secu; 15 mM Glue; 200 mM treh; 50 mM arg; 0.2 mg/mL PS20; 1 mM met; 50 mM NaCl; 0.1 mM EDTA; water; and pH 5.8.

I2360. 190 mg/mL secu; 15 mM Glue; 200 mM treh; 50 mM arg; 0.2 mg/mL PS20; 1 mM met; 50 mM NaCl; 0.1 mM EDTA; water; and pH 5.8.

I2361. 150 mg/mL secu; 0 mM buffer; 150 mM sorb; 100 mM gly; 0.2 mg/mL PS20; 1 mM met; 50 mM NaCl; 0.1 mM EDTA; water; and pH 5.8.

I2362. 190 mg/mL secu; 0 mM buffer; 150 mM sorb; 100 mM gly; 0.2 mg/mL PS20; 1 mM met; 50 mM NaCl; 0.1 mM EDTA; water; and pH 5.8.

(continued)

I2363. 150 mg/mL secu; 50 mM His; 150 mM sorb; 100 mM gly; 0.2 mg/mL PS20; 1 mM met; 50 mM NaCl; 0.1 mM EDTA; water; and pH 5.8.

I2364. 190 mg/mL secu; 50 mM His; 150 mM sorb; 100 mM gly; 0.2 mg/mL PS20; 1 mM met; 50 mM NaCl; 0.1 mM EDTA; water; and pH 5.8.

I2365. 150 mg/mL secu; 25 mM Lact; 150 mM sorb; 100 mM gly; 0.2 mg/mL PS20; 1 mM met; 50 mM NaCl; 0.1 mM EDTA; water; and pH 5.8.

I2366. 190 mg/mL secu; 25 mM Lact; 150 mM sorb; 100 mM gly; 0.2 mg/mL PS20; 1 mM met; 50 mM NaCl; 0.1 mM EDTA; water; and pH 5.8.

I2367. 150 mg/mL secu; 10 mM Phos-Lact; 150 mM sorb; 100 mM gly; 0.2 mg/mL PS20; 1 mM met; 50 mM NaCl; 0.1 mM EDTA; water; and pH 5.8.

I2368. 190 mg/mL secu; 10 mM Phos-Lact; 150 mM sorb; 100 mM gly; 0.2 mg/mL PS20; 1 mM met; 50 mM NaCl; 0.1 mM EDTA; water; and pH 5.8.

I2369. 150 mg/mL secu; 15 mM Glue; 150 mM sorb; 100 mM gly; 0.2 mg/mL PS20; 1 mM met; 50 mM NaCl; 0.1 mM EDTA; water; and pH 5.8.

I2370. 190 mg/mL secu; 15 mM Glue; 150 mM sorb; 100 mM gly; 0.2 mg/mL PS20; 1 mM met; 50 mM NaCl; 0.1 mM EDTA; water; and pH 5.8.

I2371. 150 mg/mL secu; 0 mM buffer; 75 mM mann; 100 mM gly; 0.2 mg/mL PS20; 1 mM met; 50 mM NaCl; 0.1 mM EDTA; water; and pH 5.8.

I2372. 190 mg/mL secu; 0 mM buffer; 75 mM mann; 100 mM gly; 0.2 mg/mL PS20; 1 mM met; 50 mM NaCl; 0.1 mM EDTA; water; and pH 5.8.

I2373. 150 mg/mL secu; 50 mM His; 75 mM mann; 100 mM gly; 0.2 mg/mL PS20; 1 mM met; 50 mM NaCl; 0.1 mM EDTA; water; and pH 5.8.

I2374. 190 mg/mL secu; 50 mM His; 75 mM mann; 100 mM gly; 0.2 mg/mL PS20; 1 mM met; 50 mM NaCl; 0.1 mM EDTA; water; and pH 5.8.

I2375. 150 mg/mL secu; 25 mM Lact; 75 mM mann; 100 mM gly; 0.2 mg/mL PS20; 1 mM met; 50 mM NaCl; 0.1 mM EDTA; water; and pH 5.8.

I2376. 190 mg/mL secu; 25 mM Lact; 75 mM mann; 100 mM gly; 0.2 mg/mL PS20; 1 mM met; 50 mM NaCl; 0.1 mM EDTA; water; and pH 5.8.

I2377. 150 mg/mL secu; 10 mM Phos-Lact; 75 mM mann; 100 mM gly; 0.2 mg/mL PS20; 1 mM met; 50 mM NaCl; 0.1 mM EDTA; water; and pH 5.8.

I2378. 190 mg/mL secu; 10 mM Phos-Lact; 75 mM mann; 100 mM gly; 0.2 mg/mL PS20; 1 mM met; 50 mM NaCl; 0.1 mM EDTA; water; and pH 5.8.

I2379. 150 mg/mL secu; 15 mM Glue; 75 mM mann; 100 mM gly; 0.2 mg/mL PS20; 1 mM met; 50 mM NaCl; 0.1 mM EDTA; water; and pH 5.8.

I2380. 190 mg/mL secu; 15 mM Glue; 75 mM mann; 100 mM gly; 0.2 mg/mL PS20; 1 mM met; 50 mM NaCl; 0.1 mM EDTA; water; and pH 5.8.

I2381. 150 mg/mL secu; 0 mM buffer; 100 mM sucr; 100 mM gly; 0.2 mg/mL PS20; 1 mM met; 50 mM NaCl; 0.1 mM EDTA; water; and pH 5.8.

I2382. 190 mg/mL secu; 0 mM buffer; 100 mM sucr; 100 mM gly; 0.2 mg/mL PS20; 1 mM met; 50 mM NaCl; 0.1 mM EDTA; water; and pH 5.8.

I2383. 150 mg/mL secu; 50 mM His; 100 mM sucr; 100 mM gly; 0.2 mg/mL PS20; 1 mM met; 50 mM NaCl; 0.1 mM EDTA; water; and pH 5.8.

I2384. 190 mg/mL secu; 50 mM His; 100 mM sucr; 100 mM gly; 0.2 mg/mL PS20; 1 mM met; 50 mM NaCl; 0.1 mM EDTA; water; and pH 5.8.

I2385. 150 mg/mL secu; 25 mM Lact; 100 mM sucr; 100 mM gly; 0.2 mg/mL PS20; 1 mM met; 50 mM NaCl; 0.1 mM EDTA; water; and pH 5.8.

I2386. 190 mg/mL secu; 25 mM Lact; 100 mM sucr; 100 mM gly; 0.2 mg/mL PS20; 1 mM met; 50 mM NaCl; 0.1 mM EDTA; water; and pH 5.8.

I2387. 150 mg/mL secu; 10 mM Phos-Lact; 100 mM sucr; 100 mM gly; 0.2 mg/mL PS20; 1 mM met; 50 mM NaCl; 0.1 mM EDTA; water; and pH 5.8.

I2388. 190 mg/mL secu; 10 mM Phos-Lact; 100 mM sucr; 100 mM gly; 0.2 mg/mL PS20; 1 mM met; 50 mM NaCl; 0.1 mM EDTA; water; and pH 5.8.

(continued)

I2389. 150 mg/mL secu; 15 mM Glue; 100 mM sucr; 100 mM gly; 0.2 mg/mL PS20; 1 mM met; 50 mM NaCl; 0.1 mM EDTA; water; and pH 5.8.

I2390. 190 mg/mL secu; 15 mM Glue; 100 mM sucr; 100 mM gly; 0.2 mg/mL PS20; 1 mM met; 50 mM NaCl; 0.1 mM EDTA; water; and pH 5.8.

I2391. 150 mg/mL secu; 0 mM buffer; 200 mM treh; 100 mM gly; 0.2 mg/mL PS20; 1 mM met; 50 mM NaCl; 0.1 mM EDTA; water; and pH 5.8.

I2392. 190 mg/mL secu; 0 mM buffer; 200 mM treh; 100 mM gly; 0.2 mg/mL PS20; 1 mM met; 50 mM NaCl; 0.1 mM EDTA; water; and pH 5.8.

I2393. 150 mg/mL secu; 50 mM His; 200 mM treh; 100 mM gly; 0.2 mg/mL PS20; 1 mM met; 50 mM NaCl; 0.1 mM EDTA; water; and pH 5.8.

I2394. 190 mg/mL secu; 50 mM His; 200 mM treh; 100 mM gly; 0.2 mg/mL PS20; 1 mM met; 50 mM NaCl; 0.1 mM EDTA; water; and pH 5.8.

I2395. 150 mg/mL secu; 25 mM Lact; 200 mM treh; 100 mM gly; 0.2 mg/mL PS20; 1 mM met; 50 mM NaCl; 0.1 mM EDTA; water; and pH 5.8.

I2396. 190 mg/mL secu; 25 mM Lact; 200 mM treh; 100 mM gly; 0.2 mg/mL PS20; 1 mM met; 50 mM NaCl; 0.1 mM EDTA; water; and pH 5.8.

I2397. 150 mg/mL secu; 10 mM Phos-Lact; 200 mM treh; 100 mM gly; 0.2 mg/mL PS20; 1 mM met; 50 mM NaCl; 0.1 mM EDTA; water; and pH 5.8.

I2398. 190 mg/mL secu; 10 mM Phos-Lact; 200 mM treh; 100 mM gly; 0.2 mg/mL PS20; 1 mM met; 50 mM NaCl; 0.1 mM EDTA; water; and pH 5.8.

I2399. 150 mg/mL secu; 15 mM Glue; 200 mM treh; 100 mM gly; 0.2 mg/mL PS20; 1 mM met; 50 mM NaCl; 0.1 mM EDTA; water; and pH 5.8.

I2400. 190 mg/mL secu; 15 mM Glue; 200 mM treh; 100 mM gly; 0.2 mg/mL PS20; 1 mM met; 50 mM NaCl; 0.1 mM EDTA; water; and pH 5.8.

*Further Specific Embodiments*

J) Further Specific Embodiments

As the table below illustrates, specific embodiments may be provided by combining one option (from the "OPTIONS" column) from each of the elements in the "FEATURE / INGREDIENT" column:

| FEATURE / INGREDIENT | OPTIONS |
|---|---|
| **Secukinumab** / **concentrations** | 150-195 mg/mL secukinumab (secu) |
| **Buffer(s)** / **concentrations** | 2-60 mM buffer<br>2-25 mM Histidine buffer (2-25 mM His)<br>1-10 mM or 40-60 mM Succinate buffer (1-10 mM or 40-60 mM Succ)<br>1-10 mM Phosphate buffer (1-10 mM Phos)<br>1-10 mM or 40-60 mM Phosphate-citrate (1:1 molar ratio) buffer (1-10 mM or 40-60 mM Phos-Cit)<br>1-10 mM or 40-60 mM Phosphate-succinate (1:1 molar ratio) buffer (1-10 mM or 40-60 mM Phos-Succ)<br>1-10 mM or 40-60 mM Phosphate-acetate (1:1 molar ratio) buffer (1-10 mM or 40-60 mM Phos-Acet)<br>40-60 mM MES |
| **Sugar/Polyol** / **concentrations** | 30-250 mM sugar component (30-250 mM sug)<br>40-150 mM sorbitol (50-150 mM sorb)<br>150-250 mM trehalose (150-250 mM treh) |

(continued)

| FEATURE / INGREDIENT | OPTIONS |
|---|---|
| **Amino acid / concentrations** | None<br>10-200 mM amino acid component (10-200 mM AA)<br>10-200 mM amino acid component selected from arginine, glycine, lysine, proline, phenylalanine, or any combination thereof (10-200 mM Arg, Gly, Lys, Pro, and/or Phe)<br>30-150 mM arginine (30-150 mM Arg)<br>100-200 mM glycine (100-200 mM Gly)<br>100-200 mM lysine (100-200 mM Lys)<br>100-200 mM proline (100-200 mM Pro)<br>5-100 mM phenylalanine (5-100 mM Phe) |
| **Surfactant / concentrations** | 0.01-1.5 mg/mL surfactant (0.01-1.5 mg/mL surf)<br>0.01-1.5 mg/mL surfactant selected from polysorbate 80 , polysorbate 20, or poloxamer 188 (0.01-1.5 mg/mL PS80, PS20, or Px188)<br>0.2-0.8 mg/mL polysorbate 80 (0.2-0.8 mg/mL PS80)<br>0.2-0.8 mg/mL polysorbate 20 (0.2-0.8 mg/mL PS20)<br>0.2-1.5 mg/mL poloxamer 188 (0.2-1.5 mg/mL Px188) |
| Antioxidant (or chelator) / concentrations | None<br>0.01-10 mM antioxidant or chelator (0.01-10 mM antiox/chel)<br>0.01-10 mM antioxidant or chelator selected from methionine, cysteine, EDTA, DTPA, or any combination thereof (0.01-10 mM Met, Cys, EDTA, and/or DTPA)<br>1-10 mM methionine (1-10 mM Met)<br>1-10 mM cysteine (1-10 mM Cys)<br>0.01-5 mM EDTA (including salts thereof)<br>0.01-5 mM Pentetic Acid (0.01-5 mM DTPA) |
| Ionic strength provider (additional) / concentrations | None<br>10-110 mM salt tonicifier<br>40-80 mM NaCl |
| Diluent | Water (for injection) |
| pH | pH 6.0 (+/- 0.4) |
| Osmolality | (preferably) 200-400 mOsm/kg |
| Dynamic viscosity | (preferably) 5-20 mPa.s at 25C |

The table below illustrates further more-specific embodiments which may be provided by combining one option (from the "OPTIONS" column) from each of the elements in the "FEATURE 1 INGREDIENT" column:

| **FEATURE** 1 **INGREDIENT** | **OPTIONS** |
|---|---|
| **Secukinumab / concentrations** | 165 mg/mL secukinumab (secu)<br>180 mg/mL secukinumab |

(continued)

| **FEATURE** 1 **INGREDIENT** | **OPTIONS** |
|---|---|
| **Buffer(s)** 1 **concentrations** | 20 mM Histidine buffer (20 mM His)<br>4 mM Histidine buffer (4 mM His)<br>4 mM Succinate buffer (4 mM Succ)<br>55 mM Succinate buffer (55 mM Succ)<br>4 mM Phosphate buffer (4 mM Phos)<br>4 mM Phosphate-citrate (1:1 molar ratio) buffer (4 mM Phos-Cit)<br>55 mM Phosphate-citrate (1:1 molar ratio) buffer (55 mM Phos-Cit)<br>4 mM Phosphate-succinate (1:1 molar ratio) buffer (4 mM Phos-Succ)<br>4 mM Phosphate-acetate (1:1 molar ratio) buffer (4 mM Phos-Acet)<br>55 mM MES |
| **Sugar/Polyol** / **concentrations** | 125 mM sorbitol (150 mM sorb)<br>50 mM sorbitol (50 mM sorb)<br>70 mM sorbitol (70 mM sorb)<br>140 mM sorbitol (140 mM sorb)<br>200 mM trehalose (200 mM treh) |
| **Amino acid** 1 **concentrations** | None<br>60 mM arginine (60 mM arg)<br>120 mM arginine (120 mM arg)<br>170 mM glycine (170 mM gly)<br>120 mM lysine (170 mM lys)<br>170 mM proline (170 mM pro)<br>20 mM phenylalanine (20 mM phe) |
| **Surfactant** / **concentrations** | 0.5 mg/mL polysorbate 80 (0.5 mg/mL PS80)<br>0.3 mg/mL polysorbate 80 (0.3 mg/mL PS80)<br>0.5 mg/mL polysorbate 20 (0.5 mg/mL PS20)<br>0.3 mg/mL polysorbate 20 (0.3 mg/mL PS20)<br>0.5 mg/mL poloxamer 188 (0.5 mg/mL Px188)<br>1.0 mg/mL poloxamer 188 (1.0 mg/mL Px188) |
| Antioxidant (or chelator) / concentrations | None<br>5 mM methionine (5 mM met)<br>2 mM cysteine (2 mM cys)<br>2 mM EDTA (including salts thereof)<br>0.5 mM Pentetic Acid (0.5 mM DTPA) |
| Ionic strength provider (additional) / concentrations | None<br>60 mM NaCl |
| Diluent | Water (for injection) |
| pH | pH 6.0 (+/- 0.4) |
| Osmolality | (preferably) 200-400 mOsm/kg |
| Dynamic viscosity | (preferably) 5-20 mPa.s at 25C |

The following numbered paragraphs J1-J1158 are illustrative of such embodiments drawn from the above tables, and define further embodiments of a biopharmaceutical composition comprising, or consisting of, and/or otherwise characterised by:

J1. 150-195 mg/mL secu; 2-60 mM buffer; 30-250 mM sug; 0.01-1.5 mg/mL surf; water; and pH 6.0 (+/- 0.4).

J2. 150-195 mg/mL secu; 2-25 mM His; 30-250 mM sug; 0.01-1.5 mg/mL surf; water; and pH 6.0 (+/- 0.4).

(continued)

J3. 150-195 mg/mL secu; 1-10 mM or 40-60 mM Succ; 30-250 mM sug; 0.01-1.5 mg/mL surf; water; and pH 6.0 (+/- 0.4).

J4. 150-195 mg/mL secu; 1-10 mM Phos; 30-250 mM sug; 0.01-1.5 mg/mL surf; water; and pH 6.0 (+/- 0.4).

J5. 150-195 mg/mL secu; 1-10 mM or 40-60 mM Phos-Cit; 30-250 mM sug; 0.01-1.5 mg/mL surf; water; and pH 6.0 (+/- 0.4).

J6. 150-195 mg/mL secu; 2-60 mM buffer; 40-150 mM sorb; 0.01-1.5 mg/mL surf; water; and pH 6.0 (+/- 0.4).

J7. 150-195 mg/mL secu; 2-25 mM His; 40-150 mM sorb; 0.01-1.5 mg/mL surf; water; and pH 6.0 (+/- 0.4).

J8. 150-195 mg/mL secu; 1-10 mM or 40-60 mM Succ; 40-150 mM sorb; 0.01-1.5 mg/mL surf; water; and pH 6.0 (+/- 0.4).

J9. 150-195 mg/mL secu; 1-10 mM Phos; 40-150 mM sorb; 0.01-1.5 mg/mL surf; water; and pH 6.0 (+/- 0.4).

J10. 150-195 mg/mL secu; 1-10 mM or 40-60 mM Phos-Cit; 40-150 mM sorb; 0.01-1.5 mg/mL surf; water; and pH 6.0 (+/- 0.4).

J11. 150-195 mg/mL secu; 2-60 mM buffer; 150-250 mM treh; 0.01-1.5 mg/mL surf; water; and pH 6.0 (+/- 0.4).

J12. 150-195 mg/mL secu; 2-25 mM His; 150-250 mM treh; 0.01-1.5 mg/mL surf; water; and pH 6.0 (+/- 0.4).

J13. 150-195 mg/mL secu; 1-10 mM or 40-60 mM Succ; 150-250 mM treh; 0.01-1.5 mg/mL surf; water; and pH 6.0 (+/- 0.4).

J14. 150-195 mg/mL secu; 1-10 mM Phos; 150-250 mM treh; 0.01-1.5 mg/mL surf; water; and pH 6.0 (+/- 0.4).

J15. 150-195 mg/mL secu; 1-10 mM or 40-60 mM Phos-Cit; 150-250 mM treh; 0.01-1.5 mg/mL surf; water; and pH 6.0 (+/-0.4).

J16. 150-195 mg/mL secu; 2-60 mM buffer; 30-250 mM sug; 0.01-1.5 mg/mL PS80, PS20, or Px188; water; and pH 6.0 (+/-0.4).

J17. 150-195 mg/mL secu; 2-25 mM His; 30-250 mM sug; 0.01-1.5 mg/mL PS80, PS20, or Px188; water; and pH 6.0 (+/- 0.4).

J18. 150-195 mg/mL secu; 1-10 mM or 40-60 mM Succ; 30-250 mM sug; 0.01-1.5 mg/mL PS80, PS20, or Px188; water; and pH 6.0 (+/- 0.4).

J19. 150-195 mg/mL secu; 1-10 mM Phos; 30-250 mM sug; 0.01-1.5 mg/mL PS80, PS20, orPx188; water; and pH 6.0 (+/-0.4).

J20. 150-195 mg/mL secu; 1-10 mM or 40-60 mM Phos-Cit; 30-250 mM sug; 0.01-1.5 mg/mL PS80, PS20, or Px188; water; and pH 6.0 (+/- 0.4).

J21. 150-195 mg/mL secu; 2-60 mM buffer; 40-150 mM sorb; 0.01-1.5 mg/mL PS80, PS20, or Px188; water; and pH 6.0 (+/-0.4).

J22. 150-195 mg/mL secu; 2-25 mM His; 40-150 mM sorb; 0.01-1.5 mg/mL PS80, PS20, or Px188; water; and pH 6.0 (+/- 0.4).

J23. 150-195 mg/mL secu; 1-10 mM or 40-60 mM Succ; 40-150 mM sorb; 0.01-1.5 mg/mL PS80, PS20, or Px188; water; and pH 6.0 (+/- 0.4).

J24. 150-195 mg/mL secu; 1-10 mM Phos; 40-150 mM sorb; 0.01-1.5 mg/mL PS80, PS20, or Px188; water; and pH 6.0 (+/-0.4).

J25. 150-195 mg/mL secu; 1-10 mM or 40-60 mM Phos-Cit; 40-150 mM sorb; 0.01-1.5 mg/mL PS80, PS20, or Px188; water; and pH 6.0 (+/- 0.4).

J26. 150-195 mg/mL secu; 2-60 mM buffer; 150-250 mM treh; 0.01-1.5 mg/mL PS80, PS20, or Px188; water; and pH 6.0 (+/-0.4).

J27. 150-195 mg/mL secu; 2-25 mM His; 150-250 mM treh; 0.01-1.5 mg/mL PS80, PS20, or Px188; water; and pH 6.0 (+/-0.4).

J28. 150-195 mg/mL secu; 1-10 mM or 40-60 mM Succ; 150-250 mM treh; 0.01-1.5 mg/mL PS80, PS20, or Px188; water; and pH 6.0 (+/- 0.4).

J29. 150-195 mg/mL secu; 1-10 mM Phos; 150-250 mM treh; 0.01-1.5 mg/mL PS80, PS20, or Px188; water; and pH 6.0 (+/-0.4).

J30. 150-195 mg/mL secu; 1-10 mM or 40-60 mM Phos-Cit; 150-250 mM treh; 0.01-1.5 mg/mL PS80, PS20, or Px188; water; and pH 6.0 (+/- 0.4).

J31. 150-195 mg/mL secu; 2-60 mM buffer; 30-250 mM sug; 10-200 mM AA; 0.01-1.5 mg/mL surf; water; and pH 6.0 (+/- 0.4).

J32. 150-195 mg/mL secu; 2-25 mM His; 30-250 mM sug; 10-200 mM AA; 0.01-1.5 mg/mL surf; water; and pH 6.0 (+/- 0.4).

(continued)

J33. 150-195 mg/mL secu; 1-10 mM or 40-60 mM Succ; 30-250 mM sug; 10-200 mM AA; 0.01-1.5 mg/mL surf; water; and pH 6.0 (+/- 0.4).

J34. 150-195 mg/mL secu; 1-10 mM Phos; 30-250 mM sug; 10-200 mM AA; 0.01-1.5 mg/mL surf; water; and pH 6.0 (+/- 0.4).

J35. 150-195 mg/mL secu; 1-10 mM or 40-60 mM Phos-Cit; 30-250 mM sug; 10-200 mM AA; 0.01-1.5 mg/mL surf; water; and pH 6.0 (+/- 0.4).

J36. 150-195 mg/mL secu; 2-60 mM buffer; 40-150 mM sorb; 10-200 mM AA; 0.01-1.5 mg/mL surf; water; and pH 6.0 (+/- 0.4).

J37. 150-195 mg/mL secu; 2-25 mM His; 40-150 mM sorb; 10-200 mM AA; 0.01-1.5 mg/mL surf; water; and pH 6.0 (+/- 0.4).

J38. 150-195 mg/mL secu; 1-10 mM or 40-60 mM Succ; 40-150 mM sorb; 10-200 mM AA; 0.01-1.5 mg/mL surf; water; and pH 6.0 (+/- 0.4).

J39. 150-195 mg/mL secu; 1-10 mM Phos; 40-150 mM sorb; 10-200 mM AA; 0.01-1.5 mg/mL surf; water; and pH 6.0 (+/- 0.4).

J40. 150-195 mg/mL secu; 1-10 mM or 40-60 mM Phos-Cit; 40-150 mM sorb; 10-200 mM AA; 0.01-1.5 mg/mL surf; water; and pH 6.0 (+/- 0.4).

J41. 150-195 mg/mL secu; 2-60 mM buffer; 150-250 mM treh; 10-200 mM AA; 0.01-1.5 mg/mL surf; water; and pH 6.0 (+/-0.4).

J42. 150-195 mg/mL secu; 2-25 mM His; 150-250 mM treh; 10-200 mM AA; 0.01-1.5 mg/mL surf; water; and pH 6.0 (+/- 0.4).

J43. 150-195 mg/mL secu; 1-10 mM or 40-60 mM Succ; 150-250 mM treh; 10-200 mM AA; 0.01-1.5 mg/mL surf; water; and pH 6.0 (+/- 0.4).

J44. 150-195 mg/mL secu; 1-10 mM Phos; 150-250 mM treh; 10-200 mM AA; 0.01-1.5 mg/mL surf; water; and pH 6.0 (+/- 0.4).

J45. 150-195 mg/mL secu; 1-10 mM or 40-60 mM Phos-Cit; 150-250 mM treh; 10-200 mM AA; 0.01-1.5 mg/mL surf; water; and pH 6.0 (+/- 0.4).

J46. 150-195 mg/mL secu; 2-60 mM buffer; 30-250 mM sug; 10-200 mM Arg, Gly, Lys, Pro, and/or Phe; 0.01-1.5 mg/mL surf; water; and pH 6.0 (+/- 0.4).

J47. 150-195 mg/mL secu; 2-25 mM His; 30-250 mM sug; 10-200 mM Arg, Gly, Lys, Pro, and/or Phe; 0.01-1.5 mg/mL surf; water; and pH 6.0 (+/- 0.4).

J48. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; water; and pH 6.0 (+/- 0.4).

J49. 150-195 mg/mL secu; 1-10 mM Phos; 30-250 mM sug; 10-200 mM Arg, Gly, Lys, Pro, and/or Phe; 0.01-1.5 mg/mL surf; water; and pH 6.0 (+/- 0.4).

J50. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; water; and pH 6.0 (+/- 0.4).

J51. 150-195 mg/mL secu; 2-60 mM buffer; 40-150 mM sorb; 10-200 mM Arg, Gly, Lys, Pro, and/or Phe; 0.01-1.5 mg/mL surf; water; and pH 6.0 (+/- 0.4).

J52. 150-195 mg/mL secu; 2-25 mM His; 40-150 mM sorb; 10-200 mM Arg, Gly, Lys, Pro, and/or Phe; 0.01-1.5 mg/mL surf; water; and pH 6.0 (+/- 0.4).

J53. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; water; and pH 6.0 (+/- 0.4).

J54. 150-195 mg/mL secu; 1-10 mM Phos; 40-150 mM sorb; 10-200 mM Arg, Gly, Lys, Pro, and/or Phe; 0.01-1.5 mg/mL surf; water; and pH 6.0 (+/- 0.4).

J55. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; water; and pH 6.0 (+/- 0.4).

J56. 150-195 mg/mL secu; 2-60 mM buffer; 150-250 mM treh; 10-200 mM Arg, Gly, Lys, Pro, and/or Phe; 0.01-1.5 mg/mL surf; water; and pH 6.0 (+/- 0.4).

J57. 150-195 mg/mL secu; 2-25 mM His; 150-250 mM treh; 10-200 mM Arg, Gly, Lys, Pro, and/or Phe; 0.01-1.5 mg/mL surf; water; and pH 6.0 (+/- 0.4).

J58. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; water; and pH 6.0 (+/- 0.4).

(continued)

J59. 150-195 mg/mL secu; 1-10 mM Phos; 150-250 mM treh; 10-200 mM Arg, Gly, Lys, Pro, and/or Phe; 0.01-1.5 mg/mL surf; water; and pH 6.0 (+/- 0.4).

J60. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; water; and pH 6.0 (+/- 0.4).

J61. 150-195 mg/mL secu; 2-60 mM buffer; 30-250 mM sug; 10-200 mM AA; 0.01-1.5 mg/mL PS80, PS20, or Px188; water; and pH 6.0 (+/- 0.4).

J62. 150-195 mg/mL secu; 2-25 mM His; 30-250 mM sug; 10-200 mM AA; 0.01-1.5 mg/mL PS80, PS20, or Px188; water; and pH 6.0 (+/- 0.4).

J63. 150-195 mg/mL secu; 1-10 mM or 40-60 mM Succ; 30-250 mM sug; 10-200 mM AA; 0.01-1.5 mg/mL PS80, PS20, or Px188; water; and pH 6.0 (+/- 0.4).

J64. 150-195 mg/mL secu; 1-10 mM Phos; 30-250 mM sug; 10-200 mM AA; 0.01-1.5 mg/mL PS80, PS20, or Px188; water; and pH 6.0 (+/- 0.4).

J65. 150-195 mg/mL secu; 1-10 mM or 40-60 mM Phos-Cit; 30-250 mM sug; 10-200 mM AA; 0.01-1.5 mg/mL PS80, PS20, or Px188; water; and pH 6.0 (+/- 0.4).

J66. 150-195 mg/mL secu; 2-60 mM buffer; 40-150 mM sorb; 10-200 mM AA; 0.01-1.5 mg/mL PS80, PS20, or Px188; water; and pH 6.0 (+/- 0.4).

J67. 150-195 mg/mL secu; 2-25 mM His; 40-150 mM sorb; 10-200 mM AA; 0.01-1.5 mg/mL PS80, PS20, or Px188; water; and pH 6.0 (+/- 0.4).

J68. 150-195 mg/mL secu; 1-1 0 mM or 40-60 mM Succ; 40-150 mM sorb; 10-200 mM AA; 0.01-1.5 mg/mL PS80, PS20, or Px188; water; and pH 6.0 (+/- 0.4).

J69. 150-195 mg/mL secu; 1-10 mM Phos; 40-150 mM sorb; 10-200 mM AA; 0.01-1.5 mg/mL PS80, PS20, or Px188; water; and pH 6.0 (+/- 0.4).

J70. 150-195 mg/mL secu; 1-10 mM or 40-60 mM Phos-Cit; 40-150 mM sorb; 10-200 mM AA; 0.01-1.5 mg/mL PS80, PS20, or Px188; water; and pH 6.0 (+/- 0.4).

J71. 150-195 mg/mL secu; 2-60 mM buffer; 150-250 mM treh; 10-200 mM AA; 0.01-1.5 mg/mL PS80, PS20, or Px188; water; and pH 6.0 (+/- 0.4).

J72. 150-195 mg/mL secu; 2-25 mM His; 150-250 mM treh; 10-200 mM AA; 0.01-1.5 mg/mL PS80, PS20, or Px188; water; and pH 6.0 (+/- 0.4).

J73. 150-195 mg/mL secu; 1-10 mM or 40-60 mM Succ; 150-250 mM treh; 10-200 mM AA; 0.01-1.5 mg/mL PS80, PS20, or Px188; water; and pH 6.0 (+/- 0.4).

J74. 150-195 mg/mL secu; 1-10 mM Phos; 150-250 mM treh; 10-200 mM AA; 0.01-1.5 mg/mL PS80, PS20, or Px188; water; and pH 6.0 (+/- 0.4).

J75. 150-195 mg/mL secu; 1-10 mM or 40-60 mM Phos-Cit; 150-250 mM treh; 10-200 mM AA; 0.01-1.5 mg/mL PS80, PS20, or Px188; water; and pH 6.0 (+/- 0.4).

J76. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; water; and pH 6.0 (+/- 0.4).

J77. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; water; and pH 6.0 (+/- 0.4).

J78. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; water; and pH 6.0 (+/- 0.4).

J79. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; water; and pH 6.0 (+/- 0.4).

J80. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; water; and pH 6.0 (+/- 0.4).

J81. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J82. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J83. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J84. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; water; and pH 6.0 (+/- 0.4).

(continued)

J85. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J86. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; water; and pH 6.0 (+/- 0.4).

J87. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; water; and pH 6.0 (+/- 0.4).

J88. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; water; and pH 6.0 (+/- 0.4).

J89. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; water; and pH 6.0 (+/- 0.4).

J90. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; water; and pH 6.0 (+/- 0.4).

J91. 150-195 mg/mL secu; 2-60 mM buffer; 30-250 mM sug; 0.01-1.5 mg/mL surf; 0.01-10 mM antiox/chel; water; and pH 6.0 (+/- 0.4).

J92. 150-195 mg/mL secu; 2-25 mM His; 30-250 mM sug; 0.01-1.5 mg/mL surf; 0.01-10 mM antiox/chel; water; and pH 6.0 (+/-0.4).

J93. 150-195 mg/mL secu; 1-10 mM or 40-60 mM Succ; 30-250 mM sug; 0.01-1.5 mg/mL surf; 0.01-10 mM antiox/chel; water; and pH 6.0 (+/- 0.4).

J94. 150-195 mg/mL secu; 1-10 mM Phos; 30-250 mM sug; 0.01-1.5 mg/mL surf; 0.01-10 mM antiox/chel; water; and pH 6.0 (+/- 0.4).

J95. 150-195 mg/mL secu; 1-10 mM or 40-60 mM Phos-Cit; 30-250 mM sug; 0.01-1.5 mg/mL surf; 0.01-10 mM antiox/chel; water; and pH 6.0 (+/- 0.4).

J96. 150-195 mg/mL secu; 2-60 mM buffer; 40-150 mM sorb; 0.01-1.5 mg/mL surf; 0.01-10 mM antiox/chel; water; and pH 6.0 (+/- 0.4).

J97. 150-195 mg/mL secu; 2-25 mM His; 40-150 mM sorb; 0.01-1.5 mg/mL surf; 0.01-10 mM antiox/chel; water; and pH 6.0 (+/- 0.4).

J98. 150-195 mg/mL secu; 1-10 mM or 40-60 mM Succ; 40-150 mM sorb; 0.01-1.5 mg/mL surf; 0.01-10 mM antiox/chel; water; and pH 6.0 (+/- 0.4).

J99. 150-195 mg/mL secu; 1-10 mM Phos; 40-150 mM sorb; 0.01-1.5 mg/mL surf; 0.01-10 mM antiox/chel; water; and pH 6.0 (+/- 0.4).

J100. 150-195 mg/mL secu; 1-10 mM or 40-60 mM Phos-Cit; 40-150 mM sorb; 0.01-1.5 mg/mL surf; 0.01-10 mM antiox/chel; water; and pH 6.0 (+/- 0.4).

J101. 150-195 mg/mL secu; 2-60 mM buffer; 150-250 mM treh; 0.01-1.5 mg/mL surf; 0.01-10 mM antiox/chel; water; and pH 6.0 (+/- 0.4).

J102. 150-195 mg/mL secu; 2-25 mM His; 150-250 mM treh; 0.01-1.5 mg/mL surf; 0.01-10 mM antiox/chel; water; and pH 6.0 (+/- 0.4).

J103. 150-195 mg/mL secu; 1-10 mM or 40-60 mM Succ; 150-250 mM treh; 0.01-1.5 mg/mL surf; 0.01-10 mM antiox/chel; water; and pH 6.0 (+/- 0.4).

J104. 150-195 mg/mL secu; 1-10 mM Phos; 150-250 mM treh; 0.01-1.5 mg/mL surf; 0.01-10 mM antiox/chel; water; and pH 6.0 (+/- 0.4).

J105. 150-195 mg/mL secu; 1-10 mM or 40-60 mM Phos-Cit; 150-250 mM treh; 0.01-1.5 mg/mL surf; 0.01-10 mM antiox/chel; water; and pH 6.0 (+/- 0.4).

J106. 150-195 mg/mL secu; 2-60 mM buffer; 30-250 mM sug; 0.01-1.5 mg/mL PS80, PS20, or Px188; 0.01-10 mM antiox/chel; water; and pH 6.0 (+/- 0.4).

J107. 150-195 mg/mL secu; 2-25 mM His; 30-250 mM sug; 0.01-1.5 mg/mL PS80, PS20, or Px188; 0.01-10 mM antiox/chel; water; and pH 6.0 (+/- 0.4).

J108. 150-195 mg/mL secu; 1-10 mM or 40-60 mM Succ; 30-250 mM sug; 0.01-1.5 mg/mL PS80, PS20, or Px188; 0.01-10 mM antiox/chel; water; and pH 6.0 (+/- 0.4).

J109. 150-195 mg/mL secu; 1-10 mM Phos; 30-250 mM sug; 0.01-1.5 mg/mL PS80, PS20, or Px188; 0.01-10 mM antiox/chel; water; and pH 6.0 (+/- 0.4).

J110. 150-195 mg/mL secu; 1-10 mM or 40-60 mM Phos-Cit; 30-250 mM sug; 0.01-1.5 mg/mL PS80, PS20, or Px188; 0.01-10 mM antiox/chel; water; and pH 6.0 (+/- 0.4).

(continued)

J111. 150-195 mg/mL secu; 2-60 mM buffer; 40-150 mM sorb; 0.01-1.5 mg/mL PS80, PS20, or Px188; 0.01-10 mM antiox/chel; water; and pH 6.0 (+/- 0.4).

J112. 150-195 mg/mL secu; 2-25 mM His; 40-150 mM sorb; 0.01-1.5 mg/mL PS80, PS20, or Px188; 0.01-10 mM antiox/chel; water; and pH 6.0 (+/- 0.4).

J113. 150-195 mg/mL secu; 1-10 mM or 40-60 mM Succ; 40-150 mM sorb; 0.01-1.5 mg/mL PS80, PS20, or Px188; 0.01-10 mM antiox/chel; water; and pH 6.0 (+/- 0.4).

J114. 150-195 mg/mL secu; 1-10 mM Phos; 40-150 mM sorb; 0.01-1.5 mg/mL PS80, PS20, or Px188; 0.01-10 mM antiox/chel; water; and pH 6.0 (+/- 0.4).

J115. 150-195 mg/mL secu; 1-10 mM or 40-60 mM Phos-Cit; 40-150 mM sorb; 0.01-1.5 mg/mL PS80, PS20, or Px188; 0.01-10 mM antiox/chel; water; and pH 6.0 (+/- 0.4).

J116. 150-195 mg/mL secu; 2-60 mM buffer; 150-250 mM treh; 0.01-1.5 mg/mL PS80, PS20, or Px188; 0.01-10 mM antiox/chel; water; and pH 6.0 (+/- 0.4).

J117. 150-195 mg/mL secu; 2-25 mM His; 150-250 mM treh; 0.01-1.5 mg/mL PS80, PS20, or Px188; 0.01-10 mM antiox/chel; water; and pH 6.0 (+/- 0.4).

J118. 150-195 mg/mL secu; 1-1 0 mM or 40-60 mM Succ; 150-250 mM treh; 0.01-1.5 mg/mL PS80, PS20, or Px188; 0.01-10 mM antiox/chel; water; and pH 6.0 (+/- 0.4).

J119. 150-195 mg/mL secu; 1-10 mM Phos; 150-250 mM treh; 0.01-1.5 mg/mL PS80, PS20, or Px188; 0.01-10 mM antiox/chel; water; and pH 6.0 (+/- 0.4).

J120. 150-195 mg/mL secu; 1-10 mM or 40-60 mM Phos-Cit; 150-250 mM treh; 0.01-1.5 mg/mL PS80, PS20, or Px188; 0.01-10 mM antiox/chel; water; and pH 6.0 (+/- 0.4).

J121. 150-195 mg/mL secu; 2-60 mM buffer; 30-250 mM sug; 0.01-1.5 mg/mL surf; 0.01-10 mM Met, Cys, EDTA, and/or DTPA; water; and pH 6.0 (+/- 0.4).

J122. 150-195 mg/mL secu; 2-25 mM His; 30-250 mM sug; 0.01-1.5 mg/mL surf; 0.01-10 mM Met, Cys, EDTA, and/or DTPA; water; and pH 6.0 (+/- 0.4).

J123. 150-195 mg/mL secu; 1-10 mM or 40-60 mM Succ; 30-250 mM sug; 0.01-1.5 mg/mL surf; 0.01-10 mM Met, Cys, EDTA, and/or DTPA; water; and pH 6.0 (+/- 0.4).

J124. 150-195 mg/mL secu; 1-10 mM Phos; 30-250 mM sug; 0.01-1.5 mg/mL surf; 0.01-10 mM Met, Cys, EDTA, and/or DTPA; water; and pH 6.0 (+/- 0.4).

J125. 150-195 mg/mL secu; 1-10 mM or 40-60 mM Phos-Cit; 30-250 mM sug; 0.01-1.5 mg/mL surf; 0.01-10 mM Met, Cys, EDTA, and/or DTPA; water; and pH 6.0 (+/- 0.4).

J126. 150-195 mg/mL secu; 2-60 mM buffer; 40-150 mM sorb; 0.01-1.5 mg/mL surf; 0.01-10 mM Met, Cys, EDTA, and/or DTPA; water; and pH 6.0 (+/- 0.4).

J127. 150-195 mg/mL secu; 2-25 mM His; 40-150 mM sorb; 0.01-1.5 mg/mL surf; 0.01-10 mM Met, Cys, EDTA, and/or DTPA; water; and pH 6.0 (+/- 0.4).

J128. 150-195 mg/mL secu; 1-10 mM or 40-60 mM Succ; 40-150 mM sorb; 0.01-1.5 mg/mL surf; 0.01-10 mM Met, Cys, EDTA, and/or DTPA; water; and pH 6.0 (+/- 0.4).

J129. 150-195 mg/mL secu; 1-10 mM Phos; 40-150 mM sorb; 0.01-1.5 mg/mL surf; 0.01-10 mM Met, Cys, EDTA, and/or DTPA; water; and pH 6.0 (+/- 0.4).

J130. 150-195 mg/mL secu; 1-10 mM or 40-60 mM Phos-Cit; 40-150 mM sorb; 0.01-1.5 mg/mL surf; 0.01-10 mM Met, Cys, EDTA, and/or DTPA; water; and pH 6.0 (+/- 0.4).

J131. 150-195 mg/mL secu; 2-60 mM buffer; 150-250 mM treh; 0.01-1.5 mg/mL surf; 0.01-10 mM Met, Cys, EDTA, and/or DTPA; water; and pH 6.0 (+/- 0.4).

J132. 150-195 mg/mL secu; 2-25 mM His; 150-250 mM treh; 0.01-1.5 mg/mL surf; 0.01-10 mM Met, Cys, EDTA, and/or DTPA; water; and pH 6.0 (+/- 0.4).

J133. 150-195 mg/mL secu; 1-10 mM or 40-60 mM Succ; 150-250 mM treh; 0.01-1.5 mg/mL surf; 0.01-10 mM Met, Cys, EDTA, and/or DTPA; water; and pH 6.0 (+/- 0.4).

J134. 150-195 mg/mL secu; 1-10 mM Phos; 150-250 mM treh; 0.01-1.5 mg/mL surf; 0.01-10 mM Met, Cys, EDTA, and/or DTPA; water; and pH 6.0 (+/- 0.4).

J135. 150-195 mg/mL secu; 1-10 mM or 40-60 mM Phos-Cit; 150-250 mM treh; 0.01-1.5 mg/mL surf; 0.01-10 mM Met, Cys, EDTA, and/or DTPA; water; and pH 6.0 (+/- 0.4).

J136. 150-195 mg/mL secu; 2-60 mM buffer; 30-250 mM sug; 0.01-1.5 mg/mL PS80, PS20, or Px188; 0.01-10 mM Met, Cys, EDTA, and/or DTPA; water; and pH 6.0 (+/- 0.4).

(continued)

J137. 150-195 mg/mL secu; 2-25 mM His; 30-250 mM sug; 0.01-1.5 mg/mL PS80, PS20, or Px188; 0.01-10 mM Met, Cys, EDTA, and/or DTPA; water; and pH 6.0 (+/- 0.4).

J138. 150-195 mg/mL secu; 1-10 mM or 40-60 mM Succ; 30-250 mM sug; 0.01-1.5 mg/mL PS80, PS20, or Px188; 0.01-10 mM Met, Cys, EDTA, and/or DTPA; water; and pH 6.0 (+/- 0.4).

J139. 150-195 mg/mL secu; 1-10 mM Phos; 30-250 mM sug; 0.01-1.5 mg/mL PS80, PS20, or Px188; 0.01-10 mM Met, Cys, EDTA, and/or DTPA; water; and pH 6.0 (+/- 0.4).

J140. 150-195 mg/mL secu; 1-10 mM or 40-60 mM Phos-Cit; 30-250 mM sug; 0.01-1.5 mg/mL PS80, PS20, or Px188; 0.01-10 mM Met, Cys, EDTA, and/or DTPA; water; and pH 6.0 (+/- 0.4).

J141. 150-195 mg/mL secu; 2-60 mM buffer; 40-150 mM sorb; 0.01-1.5 mg/mL PS80, PS20, or Px188; 0.01-10 mM Met, Cys, EDTA, and/or DTPA; water; and pH 6.0 (+/- 0.4).

J142. 150-195 mg/mL secu; 2-25 mM His; 40-150 mM sorb; 0.01-1.5 mg/mL PS80, PS20, or Px188; 0.01-10 mM Met, Cys, EDTA, and/or DTPA; water; and pH 6.0 (+/- 0.4).

J143. 150-195 mg/mL secu; 1-10 mM or 40-60 mM Succ; 40-150 mM sorb; 0.01-1.5 mg/mL PS80, PS20, or Px188; 0.01-10 mM Met, Cys, EDTA, and/or DTPA; water; and pH 6.0 (+/- 0.4).

J144. 150-195 mg/mL secu; 1-10 mM Phos; 40-150 mM sorb; 0.01-1.5 mg/mL PS80, PS20, or Px188; 0.01-10 mM Met, Cys, EDTA, and/or DTPA; water; and pH 6.0 (+/- 0.4).

J145. 150-195 mg/mL secu; 1-10 mM or 40-60 mM Phos-Cit; 40-150 mM sorb; 0.01-1.5 mg/mL PS80, PS20, or Px188; 0.01-10 mM Met, Cys, EDTA, and/or DTPA; water; and pH 6.0 (+/- 0.4).

J146. 150-195 mg/mL secu; 2-60 mM buffer; 150-250 mM treh; 0.01-1.5 mg/mL PS80, PS20, or Px188; 0.01-10 mM Met, Cys, EDTA, and/or DTPA; water; and pH 6.0 (+/- 0.4).

J147. 150-195 mg/mL secu; 2-25 mM His; 150-250 mM treh; 0.01-1.5 mg/mL PS80, PS20, or Px188; 0.01-10 mM Met, Cys, EDTA, and/or DTPA; water; and pH 6.0 (+/- 0.4).

J148. 150-195 mg/mL secu; 1-10 mM or 40-60 mM Succ; 150-250 mM treh; 0.01-1.5 mg/mL PS80, PS20, or Px188; 0.01-10 mM Met, Cys, EDTA, and/or DTPA; water; and pH 6.0 (+/- 0.4).

J149. 150-195 mg/mL secu; 1-10 mM Phos; 150-250 mM treh; 0.01-1.5 mg/mL PS80, PS20, or Px188; 0.01-10 mM Met, Cys, EDTA, and/or DTPA; water; and pH 6.0 (+/- 0.4).

J150. 150-195 mg/mL secu; 1-10 mM or 40-60 mM Phos-Cit; 150-250 mM treh; 0.01-1.5 mg/mL PS80, PS20, or Px188; 0.01-10 mM Met, Cys, EDTA, and/or DTPA; water; and pH 6.0 (+/- 0.4).

J151. 150-195 mg/mL secu; 2-60 mM buffer; 30-250 mM sug; 0.01-1.5 mg/mL surf; 10-110 mM salt tonicifier; water; and pH 6.0 (+/- 0.4).

J152. 150-195 mg/mL secu; 2-25 mM His; 30-250 mM sug; 0.01-1.5 mg/mL surf; 10-110 mM salt tonicifier; water; and pH 6.0 (+/- 0.4).

J153. 150-195 mg/mL secu; 1-10 mM or 40-60 mM Succ; 30-250 mM sug; 0.01-1.5 mg/mL surf; 10-110 mM salt tonicifier; water; and pH 6.0 (+/- 0.4).

J154. 150-195 mg/mL secu; 1-10 mM Phos; 30-250 mM sug; 0.01-1.5 mg/mL surf; 10-110 mM salt tonicifier; water; and pH 6.0 (+/- 0.4).

J155. 150-195 mg/mL secu; 1-10 mM or 40-60 mM Phos-Cit; 30-250 mM sug; 0.01-1.5 mg/mL surf; 10-110 mM salt tonicifier; water; and pH 6.0 (+/- 0.4).

J156. 150-195 mg/mL secu; 2-60 mM buffer; 40-150 mM sorb; 0.01-1.5 mg/mL surf; 10-110 mM salt tonicifier; water; and pH 6.0 (+/- 0.4).

J157. 150-195 mg/mL secu; 2-25 mM His; 40-150 mM sorb; 0.01-1.5 mg/mL surf; 10-110 mM salt tonicifier; water; and pH 6.0 (+/- 0.4).

J158. 150-195 mg/mL secu; 1-10 mM or 40-60 mM Succ; 40-150 mM sorb; 0.01-1.5 mg/mL surf; 10-110 mM salt tonicifier; water; and pH 6.0 (+/- 0.4).

J159. 150-195 mg/mL secu; 1-10 mM Phos; 40-150 mM sorb; 0.01-1.5 mg/mL surf; 10-110 mM salt tonicifier; water; and pH 6.0 (+/- 0.4).

J160. 150-195 mg/mL secu; 1-10 mM or 40-60 mM Phos-Cit; 40-150 mM sorb; 0.01-1.5 mg/mL surf; 10-110 mM salt tonicifier; water; and pH 6.0 (+/- 0.4).

J161. 150-195 mg/mL secu; 2-60 mM buffer; 150-250 mM treh; 0.01-1.5 mg/mL surf; 10-110 mM salt tonicifier; water; and pH 6.0 (+/- 0.4).

J162. 150-195 mg/mL secu; 2-25 mM His; 150-250 mM treh; 0.01-1.5 mg/mL surf; 10-110 mM salt tonicifier; water; and pH 6.0 (+/- 0.4).

(continued)

J163. 150-195 mg/mL secu; 1-10 mM or 40-60 mM Succ; 150-250 mM treh; 0.01-1.5 mg/mL surf; 10-110 mM salt tonicifier; water; and pH 6.0 (+/- 0.4).

J164. 150-195 mg/mL secu; 1-10 mM Phos; 150-250 mM treh; 0.01-1.5 mg/mL surf; 10-110 mM salt tonicifier; water; and pH 6.0 (+/- 0.4).

J165. 150-195 mg/mL secu; 1-10 mM or 40-60 mM Phos-Cit; 150-250 mM treh; 0.01-1.5 mg/mL surf; 10-110 mM salt tonicifier; water; and pH 6.0 (+/- 0.4).

J166. 150-195 mg/mL secu; 2-60 mM buffer; 30-250 mM sug; 0.01-1.5 mg/mL PS80, PS20, or Px188; 10-110 mM salt tonicifier; water; and pH 6.0 (+/- 0.4).

J167. 150-195 mg/mL secu; 2-25 mM His; 30-250 mM sug; 0.01-1.5 mg/mL PS80, PS20, or Px188; 10-110 mM salt tonicifier; water; and pH 6.0 (+/- 0.4).

J168. 150-195 mg/mL secu; 1-10 mM or 40-60 mM Succ; 30-250 mM sug; 0.01-1.5 mg/mL PS80, PS20, or Px188; 10-110 mM salt tonicifier; water; and pH 6.0 (+/- 0.4).

J169. 150-195 mg/mL secu; 1-10 mM Phos; 30-250 mM sug; 0.01-1.5 mg/mL PS80, PS20, orPx188; 10-110 mM salt tonicifier; water; and pH 6.0 (+/- 0.4).

J170. 150-195 mg/mL secu; 1-10 mM or 40-60 mM Phos-Cit; 30-250 mM sug; 0.01-1.5 mg/mL PS80, PS20, or Px188; 10-110 mM salt tonicifier; water; and pH 6.0 (+/- 0.4).

J171. 150-195 mg/mL secu; 2-60 mM buffer; 40-150 mM sorb; 0.01-1.5 mg/mL PS80, PS20, or Px188; 10-110 mM salt tonicifier; water; and pH 6.0 (+/- 0.4).

J172. 150-195 mg/mL secu; 2-25 mM His; 40-150 mM sorb; 0.01-1.5 mg/mL PS80, PS20, or Px188; 10-110 mM salt tonicifier; water; and pH 6.0 (+/- 0.4).

J173. 150-195 mg/mL secu; 1-10 mM or 40-60 mM Succ; 40-150 mM sorb; 0.01-1.5 mg/mL PS80, PS20, or Px188; 10-110 mM salt tonicifier; water; and pH 6.0 (+/- 0.4).

J174. 150-195 mg/mL secu; 1-10 mM Phos; 40-150 mM sorb; 0.01-1.5 mg/mL PS80, PS20, or Px188; 10-110 mM salt tonicifier; water; and pH 6.0 (+/- 0.4).

J175. 150-195 mg/mL secu; 1-10 mM or 40-60 mM Phos-Cit; 40-150 mM sorb; 0.01-1.5 mg/mL PS80, PS20, or Px188; 10-110 mM salt tonicifier; water; and pH 6.0 (+/- 0.4).

J176. 150-195 mg/mL secu; 2-60 mM buffer; 150-250 mM treh; 0.01-1.5 mg/mL PS80, PS20, or Px188; 10-110 mM salt tonicifier; water; and pH 6.0 (+/- 0.4).

J177. 150-195 mg/mL secu; 2-25 mM His; 150-250 mM treh; 0.01-1.5 mg/mL PS80, PS20, or Px188; 10-110 mM salt tonicifier; water; and pH 6.0 (+/- 0.4).

J178. 150-195 mg/mL secu; 1-1 0 mM or 40-60 mM Succ; 150-250 mM treh; 0.01-1.5 mg/mL PS80, PS20, or Px188; 10-110 mM salt tonicifier; water; and pH 6.0 (+/- 0.4).

J179. 150-195 mg/mL secu; 1-10 mM Phos; 150-250 mM treh; 0.01-1.5 mg/mL PS80, PS20, or Px188; 10-110 mM salt tonicifier; water; and pH 6.0 (+/- 0.4).

J180. 150-195 mg/mL secu; 1-10 mM or 40-60 mM Phos-Cit; 150-250 mM treh; 0.01-1.5 mg/mL PS80, PS20, or Px188; 10-110 mM salt tonicifier; water; and pH 6.0 (+/- 0.4).

J181. 150-195 mg/mL secu; 2-60 mM buffer; 30-250 mM sug; 0.01-1.5 mg/mL surf; 40-80 mM NaCl; water; and pH 6.0 (+/- 0.4).

J182. 150-195 mg/mL secu; 2-25 mM His; 30-250 mM sug; 0.01-1.5 mg/mL surf; 40-80 mM NaCl; water; and pH 6.0 (+/- 0.4).

J183. 150-195 mg/mL secu; 1-10 mM or 40-60 mM Succ; 30-250 mM sug; 0.01-1.5 mg/mL surf; 40-80 mM NaCl; water; and pH 6.0 (+/- 0.4).

J184. 150-195 mg/mL secu; 1-10 mM Phos; 30-250 mM sug; 0.01-1.5 mg/mL surf; 40-80 mM NaCl; water; and pH 6.0 (+/- 0.4).

J185. 150-195 mg/mL secu; 1-10 mM or 40-60 mM Phos-Cit; 30-250 mM sug; 0.01-1.5 mg/mL surf; 40-80 mM NaCl; water; and pH 6.0 (+/- 0.4).

J186. 150-195 mg/mL secu; 2-60 mM buffer; 40-150 mM sorb; 0.01-1.5 mg/mL surf; 40-80 mM NaCl; water; and pH 6.0 (+/-0.4).

J187. 150-195 mg/mL secu; 2-25 mM His; 40-150 mM sorb; 0.01-1.5 mg/mL surf; 40-80 mM NaCl; water; and pH 6.0 (+/- 0.4).

J188. 150-195 mg/mL secu; 1-10 mM or 40-60 mM Succ; 40-150 mM sorb; 0.01-1.5 mg/mL surf; 40-80 mM NaCl; water; and pH 6.0 (+/- 0.4).

(continued)

J189. 150-195 mg/mL secu; 1-10 mM Phos; 40-150 mM sorb; 0.01-1.5 mg/mL surf; 40-80 mM NaCl; water; and pH 6.0 (+/- 0.4).

J190. 150-195 mg/mL secu; 1-10 mM or 40-60 mM Phos-Cit; 40-150 mM sorb; 0.01-1.5 mg/mL surf; 40-80 mM NaCl; water; and pH 6.0 (+/- 0.4).

J191. 150-195 mg/mL secu; 2-60 mM buffer; 150-250 mM treh; 0.01-1.5 mg/mL surf; 40-80 mM NaCl; water; and pH 6.0 (+/-0.4).

J192. 150-195 mg/mL secu; 2-25 mM His; 150-250 mM treh; 0.01-1.5 mg/mL surf; 40-80 mM NaCl; water; and pH 6.0 (+/- 0.4).

J193. 150-195 mg/mL secu; 1-10 mM or 40-60 mM Succ; 150-250 mM treh; 0.01-1.5 mg/mL surf; 40-80 mM NaCl; water; and pH 6.0 (+/- 0.4).

J194. 150-195 mg/mL secu; 1-10 mM Phos; 150-250 mM treh; 0.01-1.5 mg/mL surf; 40-80 mM NaCl; water; and pH 6.0 (+/-0.4).

J195. 150-195 mg/mL secu; 1-10 mM or 40-60 mM Phos-Cit; 150-250 mM treh; 0.01-1.5 mg/mL surf; 40-80 mM NaCl; water; and pH 6.0 (+/- 0.4).

J196. 150-195 mg/mL secu; 2-60 mM buffer; 30-250 mM sug; 0.01-1.5 mg/mL PS80, PS20, or Px188; 40-80 mM NaCl; water; and pH 6.0 (+/- 0.4).

J197. 150-195 mg/mL secu; 2-25 mM His; 30-250 mM sug; 0.01-1.5 mg/mL PS80, PS20, or Px188; 40-80 mM NaCl; water; and pH 6.0 (+/- 0.4).

J198. 150-195 mg/mL secu; 1-1 0 mM or 40-60 mM Succ; 30-250 mM sug; 0.01-1.5 mg/mL PS80, PS20, or Px188; 40-80 mM NaCl; water; and pH 6.0 (+/- 0.4).

J199. 150-195 mg/mL secu; 1-10 mM Phos; 30-250 mM sug; 0.01-1.5 mg/mL PS80, PS20, or Px188; 40-80 mM NaCl; water; and pH 6.0 (+/- 0.4).

J200. 150-195 mg/mL secu; 1-10 mM or 40-60 mM Phos-Cit; 30-250 mM sug; 0.01-1.5 mg/mL PS80, PS20, or Px188; 40-80 mM NaCl; water; and pH 6.0 (+/- 0.4).

J201. 150-195 mg/mL secu; 2-60 mM buffer; 40-150 mM sorb; 0.01-1.5 mg/mL PS80, PS20, or Px188; 40-80 mM NaCl; water; and pH 6.0 (+/- 0.4).

J202. 150-195 mg/mL secu; 2-25 mM His; 40-150 mM sorb; 0.01-1.5 mg/mL PS80, PS20, or Px188; 40-80 mM NaCl; water; and pH 6.0 (+/- 0.4).

J203. 150-195 mg/mL secu; 1-10 mM or 40-60 mM Succ; 40-150 mM sorb; 0.01-1.5 mg/mL PS80, PS20, or Px188; 40-80 mM NaCl; water; and pH 6.0 (+/- 0.4).

J204. 150-195 mg/mL secu; 1-10 mM Phos; 40-150 mM sorb; 0.01-1.5 mg/mL PS80, PS20, or Px188; 40-80 mM NaCl; water; and pH 6.0 (+/- 0.4).

J205. 150-195 mg/mL secu; 1-10 mM or 40-60 mM Phos-Cit; 40-150 mM sorb; 0.01-1.5 mg/mL PS80, PS20, or Px188; 40-80 mM NaCl; water; and pH 6.0 (+/- 0.4).

J206. 150-195 mg/mL secu; 2-60 mM buffer; 150-250 mM treh; 0.01-1.5 mg/mL PS80, PS20, or Px188; 40-80 mM NaCl; water; and pH 6.0 (+/- 0.4).

J207. 150-195 mg/mL secu; 2-25 mM His; 150-250 mM treh; 0.01-1.5 mg/mL PS80, PS20, or Px188; 40-80 mM NaCl; water; and pH 6.0 (+/- 0.4).

J208. 150-195 mg/mL secu; 1-10 mM or 40-60 mM Succ; 150-250 mM treh; 0.01-1.5 mg/mL PS80, PS20, or Px188; 40-80 mM NaCl; water; and pH 6.0 (+/- 0.4).

J209. 150-195 mg/mL secu; 1-10 mM Phos; 150-250 mM treh; 0.01-1.5 mg/mL PS80, PS20, or Px188; 40-80 mM NaCl; water; and pH 6.0 (+/- 0.4).

J210. 150-195 mg/mL secu; 1-10 mM or 40-60 mM Phos-Cit; 150-250 mM treh; 0.01-1.5 mg/mL PS80, PS20, or Px188; 40-80 mM NaCl; water; and pH 6.0 (+/- 0.4).

J211. 150-195 mg/mL secu; 2-60 mM buffer; 30-250 mM sug; 10-200 mM AA; 0.01-1.5 mg/mL surf; 0.01-10 mM antiox/chel; water; and pH 6.0 (+/- 0.4).

J212. 150-195 mg/mL secu; 2-25 mM His; 30-250 mM sug; 10-200 mM AA; 0.01-1.5 mg/mL surf; 0.01-10 mM antiox/chel; water; and pH 6.0 (+/- 0.4).

J213. 150-195 mg/mL secu; 1-10 mM or 40-60 mM Succ; 30-250 mM sug; 10-200 mM AA; 0.01-1.5 mg/mL surf; 0.01-10 mM antiox/chel; water; and pH 6.0 (+/- 0.4).

J214. 150-195 mg/mL secu; 1-10 mM Phos; 30-250 mM sug; 10-200 mM AA; 0.01-1.5 mg/mL surf; 0.01-10 mM antiox/chel; water; and pH 6.0 (+/- 0.4).

(continued)

J215. 150-195 mg/mL secu; 1-10 mM or 40-60 mM Phos-Cit; 30-250 mM sug; 10-200 mM AA; 0.01-1.5 mg/mL surf; 0.01-10 mM antiox/chel; water; and pH 6.0 (+/- 0.4).

J216. 150-195 mg/mL secu; 2-60 mM buffer; 40-150 mM sorb; 10-200 mM AA; 0.01-1.5 mg/mL surf; 0.01-10 mM antiox/chel; water; and pH 6.0 (+/- 0.4).

J217. 150-195 mg/mL secu; 2-25 mM His; 40-150 mM sorb; 10-200 mM AA; 0.01-1.5 mg/mL surf; 0.01-10 mM antiox/chel; water; and pH 6.0 (+/- 0.4).

J218. 150-195 mg/mL secu; 1-10 mM or 40-60 mM Succ; 40-150 mM sorb; 10-200 mM AA; 0.01-1.5 mg/mL surf; 0.01-10 mM antiox/chel; water; and pH 6.0 (+/- 0.4).

J219. 150-195 mg/mL secu; 1-10 mM Phos; 40-150 mM sorb; 10-200 mM AA; 0.01-1.5 mg/mL surf; 0.01-10 mM antiox/chel; water; and pH 6.0 (+/- 0.4).

J220. 150-195 mg/mL secu; 1-10 mM or 40-60 mM Phos-Cit; 40-150 mM sorb; 10-200 mM AA; 0.01-1.5 mg/mL surf; 0.01-10 mM antiox/chel; water; and pH 6.0 (+/- 0.4).

J221. 150-195 mg/mL secu; 2-60 mM buffer; 150-250 mM treh; 10-200 mM AA; 0.01-1.5 mg/mL surf; 0.01-10 mM antiox/chel; water; and pH 6.0 (+/- 0.4).

J222. 150-195 mg/mL secu; 2-25 mM His; 150-250 mM treh; 10-200 mM AA; 0.01-1.5 mg/mL surf; 0.01-10 mM antiox/chel; water; and pH 6.0 (+/- 0.4).

J223. 150-195 mg/mL secu; 1-10 mM or 40-60 mM Succ; 150-250 mM treh; 10-200 mM AA; 0.01-1.5 mg/mL surf; 0.01-10 mM antiox/chel; water; and pH 6.0 (+/- 0.4).

J224. 150-195 mg/mL secu; 1-10 mM Phos; 150-250 mM treh; 10-200 mM AA; 0.01-1.5 mg/mL surf; 0.01-10 mM antiox/chel; water; and pH 6.0 (+/- 0.4).

J225. 150-195 mg/mL secu; 1-10 mM or 40-60 mM Phos-Cit; 150-250 mM treh; 10-200 mM AA; 0.01-1.5 mg/mL surf; 0.01-10 mM antiox/chel; water; and pH 6.0 (+/- 0.4).

J226. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J227. 150-195 mg/mL secu; 2-25 mM His; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J228. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J229. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J230. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J231. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J232. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J233. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J234. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J235. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J236. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM antiox/chel; water; and pH 6.0 (+/- 0.4).

J237. 150-195 mg/mL secu; 2-25 mM His; 150-250 mM treh; 10-200 mM Arg, Gly, Lys, Pro, and/or Phe; 0.01-1.5 mg/mL surf; 0.01-10 mM antiox/chel; water; and pH 6.0 (+/- 0.4).

J238. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM antiox/chel; water; and pH 6.0 (+/- 0.4).

J239. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM antiox/chel; water; and pH 6.0 (+/- 0.4).

J240. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM antioxlehel; water; and pH 6.0 (+/- 0.4).

(continued)

J241. 150-195 mg/mL secu; 2-60 mM buffer; 30-250 mM sug; 10-200 mM AA; 0.01-1.5 mg/mL PS80, PS20, or Px188; 0.01-10 mM antioxlehel; water; and pH 6.0 (+/- 0.4).

J242. 150-195 mg/mL secu; 2-25 mM His; 30-250 mM sug; 10-200 mM AA; 0.01-1.5 mg/mL PS80, PS20, or Px188; 0.01-10 mM antioxlehel; water; and pH 6.0 (+/- 0.4).

J243. 150-195 mg/mL secu; 1-10 mM or 40-60 mM Succ; 30-250 mM sug; 10-200 mM AA; 0.01-1.5 mg/mL PS80, PS20, or Px188; 0.01-10 mM antiox/chel; water; and pH 6.0 (+/- 0.4).

J244. 150-195 mg/mL secu; 1-10 mM Phos; 30-250 mM sug; 10-200 mM AA; 0.01-1.5 mg/mL PS80, PS20, or Px188; 0.01-10 mM antioxlehel; water; and pH 6.0 (+/- 0.4).

J245. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM antiox/chel; water; and pH 6.0 (+/- 0.4).

J246. 150-195 mg/mL secu; 2-60 mM buffer; 40-150 mM sorb; 10-200 mM AA; 0.01-1.5 mg/mL PS80, PS20, or Px188; 0.01-10 mM antioxlehel; water; and pH 6.0 (+/- 0.4).

J247. 150-195 mg/mL secu; 2-25 mM His; 40-150 mM sorb; 10-200 mM AA; 0.01-1.5 mg/mL PS80, PS20, or Px188; 0.01-10 mM antioxlehel; water; and pH 6.0 (+/- 0.4).

J248. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM antiox/chel; water; and pH 6.0 (+/- 0.4).

J249. 150-195 mg/mL secu; 1-10 mM Phos; 40-150 mM sorb; 10-200 mM AA; 0.01-1.5 mg/mL PS80, PS20, or Px188; 0.01-10 mM antiox/chel; water; and pH 6.0 (+/- 0.4).

J250. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM antiox/chel; water; and pH 6.0 (+/- 0.4).

J251. 150-195 mg/mL secu; 2-60 mM buffer; 150-250 mM treh; 10-200 mM AA; 0.01-1.5 mg/mL PS80, PS20, or Px188; 0.01-10 mM antiox/chel; water; and pH 6.0 (+/- 0.4).

J252. 150-195 mg/mL secu; 2-25 mM His; 150-250 mM treh; 10-200 mM AA; 0.01-1.5 mg/mL PS80, PS20, or Px188; 0.01-10 mM antiox/chel; water; and pH 6.0 (+/- 0.4).

J253. 150-195 mg/mL secu; 1-10 mM or 40-60 mM Succ; 150-250 mM treh; 10-200 mM AA; 0.01-1.5 mg/mL PS80, PS20, or Px188; 0.01-10 mM antiox/chel; water; and pH 6.0 (+/- 0.4).

J254. 150-195 mg/mL secu; 1-10 mM Phos; 150-250 mM treh; 10-200 mM AA; 0.01-1.5 mg/mL PS80, PS20, or Px188; 0.01-10 mM antiox/chel; water; and pH 6.0 (+/- 0.4).

J255. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM antiox/chel; water; and pH 6.0 (+/- 0.4).

J256. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J257. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J258. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J259. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J260. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J261. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J262. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J263. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J264. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J265. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J266. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

(continued)

J267. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J268. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J269. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J270. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J271. 150-195 mg/mL secu; 2-60 mM buffer; 30-250 mM sug; 10-200 mM AA; 0.01-1.5 mg/mL surf; 0.01-10 mM Met, Cys, EDTA, and/or DTPA; water; and pH 6.0 (+/- 0.4).

J272. 150-195 mg/mL secu; 2-25 mM His; 30-250 mM sug; 10-200 mM AA; 0.01-1.5 mg/mL surf; 0.01-10 mM Met, Cys, EDTA, and/or DTPA; water; and pH 6.0 (+/- 0.4).

J273. 150-195 mg/mL secu; 1-10 mM or 40-60 mM Succ; 30-250 mM sug; 10-200 mM AA; 0.01-1.5 mg/mL surf; 0.01-10 mM Met, Cys, EDTA, and/or DTPA; water; and pH 6.0 (+/- 0.4).

J274. 150-195 mg/mL secu; 1-10 mM Phos; 30-250 mM sug; 10-200 mM AA; 0.01-1.5 mg/mL surf; 0.01-10 mM Met, Cys, EDTA, and/or DTPA; water; and pH 6.0 (+/- 0.4).

J275. 150-195 mg/mL secu; 1-10 mM or 40-60 mM Phos-Cit; 30-250 mM sug; 10-200 mM AA; 0.01-1.5 mg/mL surf; 0.01-10 mM Met, Cys, EDTA, and/or DTPA; water; and pH 6.0 (+/- 0.4).

J276. 150-195 mg/mL secu; 2-60 mM buffer; 40-150 mM sorb; 10-200 mM AA; 0.01-1.5 mg/mL surf; 0.01-10 mM Met, Cys, EDTA, and/or DTPA; water; and pH 6.0 (+/- 0.4).

J277. 150-195 mg/mL secu; 2-25 mM His; 40-150 mM sorb; 10-200 mM AA; 0.01-1.5 mg/mL surf; 0.01-10 mM Met, Cys, EDTA, and/or DTPA; water; and pH 6.0 (+/- 0.4).

J278. 150-195 mg/mL secu; 1-10 mM or 40-60 mM Succ; 40-150 mM sorb; 10-200 mM AA; 0.01-1.5 mg/mL surf; 0.01-10 mM Met, Cys, EDTA, and/or DTPA; water; and pH 6.0 (+/- 0.4).

J279. 150-195 mg/mL secu; 1-10 mM Phos; 40-150 mM sorb; 10-200 mM AA; 0.01-1.5 mg/mL surf; 0.01-10 mM Met, Cys, EDTA, and/or DTPA; water; and pH 6.0 (+/- 0.4).

J280. 150-195 mg/mL secu; 1-10 mM or 40-60 mM Phos-Cit; 40-150 mM sorb; 10-200 mM AA; 0.01-1.5 mg/mL surf; 0.01-10 mM Met, Cys, EDTA, and/or DTPA; water; and pH 6.0 (+/- 0.4).

J281. 150-195 mg/mL secu; 2-60 mM buffer; 150-250 mM treh; 10-200 mM AA; 0.01-1.5 mg/mL surf; 0.01-10 mM Met, Cys, EDTA, and/or DTPA; water; and pH 6.0 (+/- 0.4).

J282. 150-195 mg/mL secu; 2-25 mM His; 150-250 mM treh; 10-200 mM AA; 0.01-1.5 mg/mL surf; 0.01-10 mM Met, Cys, EDTA, and/or DTPA; water; and pH 6.0 (+/- 0.4).

J283. 150-195 mg/mL secu; 1-10 mM or 40-60 mM Succ; 150-250 mM treh; 10-200 mM AA; 0.01-1.5 mg/mL surf; 0.01-10 mM Met, Cys, EDTA, and/or DTPA; water; and pH 6.0 (+/- 0.4).

J284. 150-195 mg/mL secu; 1-10 mM Phos; 150-250 mM treh; 10-200 mM AA; 0.01-1.5 mg/mL surf; 0.01-10 mM Met, Cys, EDTA, and/or DTPA; water; and pH 6.0 (+/- 0.4).

J285. 150-195 mg/mL secu; 1-10 mM or 40-60 mM Phos-Cit; 150-250 mM treh; 10-200 mM AA; 0.01-1.5 mg/mL surf; 0.01-10 mM Met, Cys, EDTA, and/or DTPA; water; and pH 6.0 (+/- 0.4).

J286. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J287. 150-195 mg/mL secu; 2-25 mM His; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J288. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J289. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J290. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J291. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J292. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

(continued)

J293. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J294. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J295. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J296. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J297. 150-195 mg/mL secu; 2-25 mM His; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J298. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J299. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J300. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J301. 150-195 mg/mL secu; 2-60 mM buffer; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J302. 150-195 mg/mL secu; 2-25 mM His; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J303. 150-195 mg/mL secu; 1-10 mM or 40-60 mM Succ; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J304. 150-195 mg/mL secu; 1-10 mM Phos; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J305. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J306. 150-195 mg/mL secu; 2-60 mM buffer; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J307. 150-195 mg/mL secu; 2-25 mM His; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J308. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J309. 150-195 mg/mL secu; 1-10 mM Phos; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J310. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J311. 150-195 mg/mL secu; 2-60 mM buffer; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J312. 150-195 mg/mL secu; 2-25 mM His; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J313. 150-195 mg/mL secu; 1-10 mM or 40-60 mM Succ; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J314. 150-195 mg/mL secu; 1-10 mM Phos; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J315. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J316. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J317. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J318. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

(continued)

J319. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J320. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J321. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J322. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J323. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J324. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J325. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J326. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J327. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J328. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J329. 150-195 mg/mL secu; 1-1 0 mM Phos; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J330. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J331. 150-195 mg/mL secu; 2-60 mM buffer; 30-250 mM sug; 10-200 mM AA; 0.01-1.5 mg/mL surf; 10-110 mM salt tonicifier; water; and pH 6.0 (+/- 0.4).

J332. 150-195 mg/mL secu; 2-25 mM His; 30-250 mM sug; 10-200 mM AA; 0.01-1.5 mg/mL surf; 10-110 mM salt tonicifier; water; and pH 6.0 (+/- 0.4).

J333. 150-195 mg/mL secu; 1-10 mM or 40-60 mM Succ; 30-250 mM sug; 10-200 mM AA; 0.01-1.5 mg/mL surf; 10-110 mM salt tonicifier; water; and pH 6.0 (+/- 0.4).

J334. 150-195 mg/mL secu; 1-10 mM Phos; 30-250 mM sug; 10-200 mM AA; 0.01-1.5 mg/mL surf; 10-110 mM salt tonicifier; water; and pH 6.0 (+/- 0.4).

J335. 150-195 mg/mL secu; 1-1 0 mM or 40-60 mM Phos-Cit; 30-250 mM sug; 10-200 mM AA; 0.01-1.5 mg/mL surf; 10-110 mM salt tonicifier; water; and pH 6.0 (+/- 0.4).

J336. 150-195 mg/mL secu; 2-60 mM buffer; 40-150 mM sorb; 10-200 mM AA; 0.01-1.5 mg/mL surf; 10-110 mM salt tonicifier; water; and pH 6.0 (+/- 0.4).

J337. 150-195 mg/mL secu; 2-25 mM His; 40-150 mM sorb; 10-200 mM AA; 0.01-1.5 mg/mL surf; 10-110 mM salt tonicifier; water; and pH 6.0 (+/- 0.4).

J338. 150-195 mg/mL secu; 1-10 mM or 40-60 mM Succ; 40-150 mM sorb; 10-200 mM AA; 0.01-1.5 mg/mL surf; 10-110 mM salt tonicifier; water; and pH 6.0 (+/- 0.4).

J339. 150-195 mg/mL secu; 1-10 mM Phos; 40-150 mM sorb; 10-200 mM AA; 0.01-1.5 mg/mL surf; 10-110 mM salt tonicifier; water; and pH 6.0 (+/- 0.4).

J340. 150-195 mg/mL secu; 1-1 0 mM or 40-60 mM Phos-Cit; 40-150 mM sorb; 10-200 mM AA; 0.01-1.5 mg/mL surf; 10-110 mM salt tonicifier; water; and pH 6.0 (+/- 0.4).

J341. 150-195 mg/mL secu; 2-60 mM buffer; 150-250 mM treh; 10-200 mM AA; 0.01-1.5 mg/mL surf; 10-110 mM salt tonicifier; water; and pH 6.0 (+/- 0.4).

(continued)

J342. 150-195 mg/mL secu; 2-25 mM His; 150-250 mM treh; 10-200 mM AA; 0.01-1.5 mg/mL surf; 10-110 mM salt tonicifier; water; and pH 6.0 (+/- 0.4).

J343. 150-195 mg/mL secu; 1-10 mM or 40-60 mM Succ; 150-250 mM treh; 10-200 mM AA; 0.01-1.5 mg/mL surf; 10-110 mM salt tonicifier; water; and pH 6.0 (+/- 0.4).

J344. 150-195 mg/mL secu; 1-1 0 mM Phos; 150-250 mM treh; 10-200 mM AA; 0.01-1.5 mg/mL surf; 10-110 mM salt tonicifier; water; and pH 6.0 (+/- 0.4).

J345. 150-195 mg/mL secu; 1-10 mM or 40-60 mM Phos-Cit; 150-250 mM treh; 10-200 mM AA; 0.01-1.5 mg/mL surf; 10-110 mM salt tonicifier; water; and pH 6.0 (+/- 0.4).

J346. 150-195 mg/mL secu; 2-60 mM buffer; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J347. 150-195 mg/mL secu; 2-25 mM His; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J348. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J349. 150-195 mg/mL secu; 1-10 mM Phos; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J350. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J351. 150-195 mg/mL secu; 2-60 mM buffer; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J352. 150-195 mg/mL secu; 2-25 mM His; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J353. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J354. 150-195 mg/mL secu; 1-10 mM Phos; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J355. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J356. 150-195 mg/mL secu; 2-60 mM buffer; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J357. 150-195 mg/mL secu; 2-25 mM His; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J358. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J359. 150-195 mg/mL secu; 1-10 mM Phos; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J360. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J361. 150-195 mg/mL secu; 2-60 mM buffer; 30-250 mM sug; 10-200 mM AA; 0.01-1.5 mg/mL PS80, PS20, or Px188; 10-110 mM salt tonicifier; water; and pH 6.0 (+/- 0.4).

J362. 150-195 mg/mL secu; 2-25 mM His; 30-250 mM sug; 10-200 mM AA; 0.01-1.5 mg/mL PS80, PS20, or Px188; 10-110 mM salt tonicifier; water; and pH 6.0 (+/- 0.4).

J363. 150-195 mg/mL secu; 1-10 mM or 40-60 mM Succ; 30-250 mM sug; 10-200 mM AA; 0.01-1.5 mg/mL PS80, PS20, or Px188; 10-110 mM salt tonicifier; water; and pH 6.0 (+/- 0.4).

J364. 150-195 mg/mL secu; 1-10 mM Phos; 30-250 mM sug; 10-200 mM AA; 0.01-1.5 mg/mL PS80, PS20, or Px188; 10-110 mM salt tonicifier; water; and pH 6.0 (+/- 0.4).

J365. 150-195 mg/mL secu; 1-10 mM or 40-60 mM Phos-Cit; 30-250 mM sug; 10-200 mM AA; 0.01-1.5 mg/mL PS80, PS20, or Px188; 10-110 mM salt tonicifier; water; and pH 6.0 (+/- 0.4).

J366. 150-195 mg/mL secu; 2-60 mM buffer; 40-150 mM sorb; 10-200 mM AA; 0.01-1.5 mg/mL PS80, PS20, or Px188; 10-110 mM salt tonicifier; water; and pH 6.0 (+/- 0.4).

J367. 150-195 mg/mL secu; 2-25 mM His; 40-150 mM sorb; 10-200 mM AA; 0.01-1.5 mg/mL PS80, PS20, or Px188; 10-110 mM salt tonicifier; water; and pH 6.0 (+/- 0.4).

(continued)

J368. 150-195 mg/mL secu; 1-10 mM or 40-60 mM Succ; 40-150 mM sorb; 10-200 mM AA; 0.01-1.5 mg/mL PS80, PS20, or Px188; 10-110 mM salt tonicifier; water; and pH 6.0 (+/- 0.4).

J369. 150-195 mg/mL secu; 1-10 mM Phos; 40-150 mM sorb; 10-200 mM AA; 0.01-1.5 mg/mL PS80, PS20, or Px188; 10-110 mM salt tonicifier; water; and pH 6.0 (+/- 0.4).

J370. 150-195 mg/mL secu; 1-10 mM or 40-60 mM Phos-Cit; 40-150 mM sorb; 10-200 mM AA; 0.01-1.5 mg/mL PS80, PS20, or Px188; 10-110 mM salt tonicifier; water; and pH 6.0 (+/- 0.4).

J371. 150-195 mg/mL secu; 2-60 mM buffer; 150-250 mM treh; 10-200 mM AA; 0.01-1.5 mg/mL PS80, PS20, or Px188; 10-110 mM salt tonicifier; water; and pH 6.0 (+/- 0.4).

J372. 150-195 mg/mL secu; 2-25 mM His; 150-250 mM treh; 10-200 mM AA; 0.01-1.5 mg/mL PS80, PS20, or Px188; 10-110 mM salt tonicifier; water; and pH 6.0 (+/- 0.4).

J373. 150-195 mg/mL secu; 1-10 mM or 40-60 mM Succ; 150-250 mM treh; 10-200 mM AA; 0.01-1.5 mg/mL PS80, PS20, or Px188; 10-110 mM salt tonicifier; water; and pH 6.0 (+/- 0.4).

J374. 150-195 mg/mL secu; 1-10 mM Phos; 150-250 mM treh; 10-200 mM AA; 0.01-1.5 mg/mL PS80, PS20, or Px188; 10-110 mM salt tonicifier; water; and pH 6.0 (+/- 0.4).

J375. 150-195 mg/mL secu; 1-10 mM or 40-60 mM Phos-Cit; 150-250 mM treh; 10-200 mM AA; 0.01-1.5 mg/mL PS80, PS20, or Px188; 10-110 mM salt tonicifier; water; and pH 6.0 (+/- 0.4).

J376. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J377. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J378. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J379. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J380. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J381. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J382. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J383. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J384. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J385. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J386. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J387. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J388. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J389. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J390. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J391. 150-195 mg/mL secu; 2-60 mM buffer; 30-250 mM sug; 10-200 mM AA; 0.01-1.5 mg/mL surf; 40-80 mM NaCl; water; and pH 6.0 (+/- 0.4).

J392. 150-195 mg/mL secu; 2-25 mM His; 30-250 mM sug; 10-200 mM AA; 0.01-1.5 mg/mL surf; 40-80 mM NaCl; water; and pH 6.0 (+/- 0.4).

J393. 150-195 mg/mL secu; 1-10 mM or 40-60 mM Succ; 30-250 mM sug; 10-200 mM AA; 0.01-1.5 mg/mL surf; 40-80 mM NaCl; water; and pH 6.0 (+/- 0.4).

(continued)

J394. 150-195 mg/mL secu; 1-10 mM Phos; 30-250 mM sug; 10-200 mM AA; 0.01-1.5 mg/mL surf; 40-80 mM NaCl; water; and pH 6.0 (+/- 0.4).

J395. 150-195 mg/mL secu; 1-10 mM or 40-60 mM Phos-Cit; 30-250 mM sug; 10-200 mM AA; 0.01-1.5 mg/mL surf; 40-80 mM NaCl; water; and pH 6.0 (+/- 0.4).

J396. 150-195 mg/mL secu; 2-60 mM buffer; 40-150 mM sorb; 10-200 mM AA; 0.01-1.5 mg/mL surf; 40-80 mM NaCl; water; and pH 6.0 (+/- 0.4).

J397. 150-195 mg/mL secu; 2-25 mM His; 40-150 mM sorb; 10-200 mM AA; 0.01-1.5 mg/mL surf; 40-80 mM NaCl; water; and pH 6.0 (+/- 0.4).

J398. 150-195 mg/mL secu; 1-10 mM or 40-60 mM Succ; 40-150 mM sorb; 10-200 mM AA; 0.01-1.5 mg/mL surf; 40-80 mM NaCl; water; and pH 6.0 (+/- 0.4).

J399. 150-195 mg/mL secu; 1-10 mM Phos; 40-150 mM sorb; 10-200 mM AA; 0.01-1.5 mg/mL surf; 40-80 mM NaCl; water; and pH 6.0 (+/- 0.4).

J400. 150-195 mg/mL secu; 1-10 mM or 40-60 mM Phos-Cit; 40-150 mM sorb; 10-200 mM AA; 0.01-1.5 mg/mL surf; 40-80 mM NaCl; water; and pH 6.0 (+/- 0.4).

J401. 150-195 mg/mL secu; 2-60 mM buffer; 150-250 mM treh; 10-200 mM AA; 0.01-1.5 mg/mL surf; 40-80 mM NaCl; water; and pH 6.0 (+/- 0.4).

J402. 150-195 mg/mL secu; 2-25 mM His; 150-250 mM treh; 10-200 mM AA; 0.01-1.5 mg/mL surf; 40-80 mM NaCl; water; and pH 6.0 (+/- 0.4).

J403. 150-195 mg/mL secu; 1-10 mM or 40-60 mM Succ; 150-250 mM treh; 10-200 mM AA; 0.01-1.5 mg/mL surf; 40-80 mM NaCl; water; and pH 6.0 (+/- 0.4).

J404. 150-195 mg/mL secu; 1-10 mM Phos; 150-250 mM treh; 10-200 mM AA; 0.01-1.5 mg/mL surf; 40-80 mM NaCl; water; and pH 6.0 (+/- 0.4).

J405. 150-195 mg/mL secu; 1-10 mM or 40-60 mM Phos-Cit; 150-250 mM treh; 10-200 mM AA; 0.01-1.5 mg/mL surf; 40-80 mM NaCl; water; and pH 6.0 (+/- 0.4).

J406. 150-195 mg/mL secu; 2-60 mM buffer; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J407. 150-195 mg/mL secu; 2-25 mM His; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J408. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J409. 150-195 mg/mL secu; 1-10 mM Phos; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J410. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J411. 150-195 mg/mL secu; 2-60 mM buffer; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J412. 150-195 mg/mL secu; 2-25 mM His; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J413. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J414. 150-195 mg/mL secu; 1-10 mM Phos; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J415. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J416. 150-195 mg/mL secu; 2-60 mM buffer; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J417. 150-195 mg/mL secu; 2-25 mM His; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J418. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J419. 150-195 mg/mL secu; 1-10 mM Phos; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

(continued)

J420. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J421. 150-195 mg/mL secu; 2-60 mM buffer; 30-250 mM sug; 10-200 mM AA; 0.01-1.5 mg/mL PS80, PS20, or Px188; 40-80 mM NaCl; water; and pH 6.0 (+/- 0.4).

J422. 150-195 mg/mL secu; 2-25 mM His; 30-250 mM sug; 10-200 mM AA; 0.01-1.5 mg/mL PS80, PS20, or Px188; 40-80 mM NaCl; water; and pH 6.0 (+/- 0.4).

J423. 150-195 mg/mL secu; 1-10 mM or 40-60 mM Succ; 30-250 mM sug; 10-200 mM AA; 0.01-1.5 mg/mL PS80, PS20, or Px188; 40-80 mM NaCl; water; and pH 6.0 (+/- 0.4).

J424. 150-195 mg/mL secu; 1-10 mM Phos; 30-250 mM sug; 10-200 mM AA; 0.01-1.5 mg/mL PS80, PS20, or Px188; 40-80 mM NaCl; water; and pH 6.0 (+/- 0.4).

J425. 150-195 mg/mL secu; 1-10 mM or 40-60 mM Phos-Cit; 30-250 mM sug; 10-200 mM AA; 0.01-1.5 mg/mL PS80, PS20, or Px188; 40-80 mM NaCl; water; and pH 6.0 (+/- 0.4).

J426. 150-195 mg/mL secu; 2-60 mM buffer; 40-150 mM sorb; 10-200 mM AA; 0.01-1.5 mg/mL PS80, PS20, or Px188; 40-80 mM NaCl; water; and pH 6.0 (+/- 0.4).

J427. 150-195 mg/mL secu; 2-25 mM His; 40-150 mM sorb; 10-200 mM AA; 0.01-1.5 mg/mL PS80, PS20, or Px188; 40-80 mM NaCl; water; and pH 6.0 (+/- 0.4).

J428. 150-195 mg/mL secu; 1-10 mM or 40-60 mM Succ; 40-150 mM sorb; 10-200 mM AA; 0.01-1.5 mg/mL PS80, PS20, or Px188; 40-80 mM NaCl; water; and pH 6.0 (+/- 0.4).

J429. 150-195 mg/mL secu; 1-10 mM Phos; 40-150 mM sorb; 10-200 mM AA; 0.01-1.5 mg/mL PS80, PS20, or Px188; 40-80 mM NaCl; water; and pH 6.0 (+/- 0.4).

J430. 150-195 mg/mL secu; 1-10 mM or 40-60 mM Phos-Cit; 40-150 mM sorb; 10-200 mM AA; 0.01-1.5 mg/mL PS80, PS20, or Px188; 40-80 mM NaCl; water; and pH 6.0 (+/- 0.4).

J431. 150-195 mg/mL secu; 2-60 mM buffer; 150-250 mM treh; 10-200 mM AA; 0.01-1.5 mg/mL PS80, PS20, or Px188; 40-80 mM NaCl; water; and pH 6.0 (+/- 0.4).

J432. 150-195 mg/mL secu; 2-25 mM His; 150-250 mM treh; 10-200 mM AA; 0.01-1.5 mg/mL PS80, PS20, or Px188; 40-80 mM NaCl; water; and pH 6.0 (+/- 0.4).

J433. 150-195 mg/mL secu; 1-10 mM or 40-60 mM Succ; 150-250 mM treh; 10-200 mM AA; 0.01-1.5 mg/mL PS80, PS20, or Px188; 40-80 mM NaCl; water; and pH 6.0 (+/- 0.4).

J434. 150-195 mg/mL secu; 1-10 mM Phos; 150-250 mM treh; 10-200 mM AA; 0.01-1.5 mg/mL PS80, PS20, or Px188; 40-80 mM NaCl; water; and pH 6.0 (+/- 0.4).

J435. 150-195 mg/mL secu; 1-10 mM or 40-60 mM Phos-Cit; 150-250 mM treh; 10-200 mM AA; 0.01-1.5 mg/mL PS80, PS20, or Px188; 40-80 mM NaCl; water; and pH 6.0 (+/- 0.4).

J436. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J437. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J438. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J439. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J440. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J441. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J442. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J443. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J444. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J445. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

(continued)

J446. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J447. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J448. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J449. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J450. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J451. 150-195 mg/mL secu; 2-60 mM buffer; 30-250 mM sug; 0.01-1.5 mg/mL surf; 0.01-10 mM antiox/chel; 10-110 mM salt tonicifier; water; and pH 6.0 (+/- 0.4).

J452. 150-195 mg/mL secu; 2-25 mM His; 30-250 mM sug; 0.01-1.5 mg/mL surf; 0.01-10 mM antiox/chel; 10-110 mM salt tonicifier; water; and pH 6.0 (+/- 0.4).

J453. 150-195 mg/mL secu; 1-10 mM or 40-60 mM Succ; 30-250 mM sug; 0.01-1.5 mg/mL surf; 0.01-10 mM antiox/chel; 10-110 mM salt tonicifier; water; and pH 6.0 (+/- 0.4).

J454. 150-195 mg/mL secu; 1-10 mM Phos; 30-250 mM sug; 0.01-1.5 mg/mL surf; 0.01-10 mM antiox/chel; 10-110 mM salt tonicifier; water; and pH 6.0 (+/- 0.4).

J455. 150-195 mg/mL secu; 1-10 mM or 40-60 mM Phos-Cit; 30-250 mM sug; 0.01-1.5 mg/mL surf; 0.01-10 mM antiox/chel; 10-110 mM salt tonicifier; water; and pH 6.0 (+/- 0.4).

J456. 150-195 mg/mL secu; 2-60 mM buffer; 40-150 mM sorb; 0.01-1.5 mg/mL surf; 0.01-10 mM antiox/chel; 10-110 mM salt tonicifier; water; and pH 6.0 (+/- 0.4).

J457. 150-195 mg/mL secu; 2-25 mM His; 40-150 mM sorb; 0.01-1.5 mg/mL surf; 0.01-10 mM antiox/chel; 10-110 mM salt tonicifier; water; and pH 6.0 (+/- 0.4).

J458. 150-195 mg/mL secu; 1-10 mM or 40-60 mM Succ; 40-150 mM sorb; 0.01-1.5 mg/mL surf; 0.01-10 mM antiox/chel; 10-110 mM salt tonicifier; water; and pH 6.0 (+/- 0.4).

J459. 150-195 mg/mL secu; 1-10 mM Phos; 40-150 mM sorb; 0.01-1.5 mg/mL surf; 0.01-10 mM antiox/chel; 10-110 mM salt tonicifier; water; and pH 6.0 (+/- 0.4).

J460. 150-195 mg/mL secu; 1-10 mM or 40-60 mM Phos-Cit; 40-150 mM sorb; 0.01-1.5 mg/mL surf; 0.01-10 mM antiox/chel; 10-110 mM salt tonicifier; water; and pH 6.0 (+/- 0.4).

J461. 150-195 mg/mL secu; 2-60 mM buffer; 150-250 mM treh; 0.01-1.5 mg/mL surf; 0.01-10 mM antiox/chel; 10-110 mM salt tonicifier; water; and pH 6.0 (+/- 0.4).

J462. 150-195 mg/mL secu; 2-25 mM His; 150-250 mM treh; 0.01-1.5 mg/mL surf; 0.01-10 mM antiox/chel; 10-110 mM salt tonicifier; water; and pH 6.0 (+/- 0.4).

J463. 150-195 mg/mL secu; 1-10 mM or 40-60 mM Succ; 150-250 mM treh; 0.01-1.5 mg/mL surf; 0.01-10 mM antiox/chel; 10-110 mM salt tonicifier; water; and pH 6.0 (+/- 0.4).

J464. 150-195 mg/mL secu; 1-10 mM Phos; 150-250 mM treh; 0.01-1.5 mg/mL surf; 0.01-10 mM antiox/chel; 10-110 mM salt tonicifier; water; and pH 6.0 (+/- 0.4).

J465. 150-195 mg/mL secu; 1-10 mM or 40-60 mM Phos-Cit; 150-250 mM treh; 0.01-1.5 mg/mL surf; 0.01-10 mM antiox/chel; 10-110 mM salt tonicifier; water; and pH 6.0 (+/- 0.4).

J466. 150-195 mg/mL secu; 2-60 mM buffer; 30-250 mM sug; 0.01-1.5 mg/mL PS80, PS20, or Px188; 0.01-10 mM antiox/chel; 10-110 mM salt tonicifier; water; and pH 6.0 (+/- 0.4).

J467. 150-195 mg/mL secu; 2-25 mM His; 30-250 mM sug; 0.01-1.5 mg/mL PS80, PS20, or Px188; 0.01-10 mM antiox/chel; 10-110 mM salt tonicifier; water; and pH 6.0 (+/- 0.4).

J468. 150-195 mg/mL secu; 1-10 mM or 40-60 mM Succ; 30-250 mM sug; 0.01-1.5 mg/mL PS80, PS20, or Px188; 0.01-10 mM antioxjchel; 10-110 mM salt tonicifier; water; and pH 6.0 (+/- 0.4).

J469. 150-195 mg/mL secu; 1-10 mM Phos; 30-250 mM sug; 0.01-1.5 mg/mL PS80, PS20, or Px188; 0.01-10 mM antiox/chel; 10-110 mM salt tonicifier; water; and pH 6.0 (+/- 0.4).

J470. 150-195 mg/mL secu; 1-10 mM or 40-60 mM Phos-Cit; 30-250 mM sug; 0.01-1.5 mg/mL PS80, PS20, or Px188; 0.01-10 mM antioxjchel; 10-110 mM salt tonicifier; water; and pH 6.0 (+/- 0.4).

J471. 150-195 mg/mL secu; 2-60 mM buffer; 40-150 mM sorb; 0.01-1.5 mg/mL PS80, PS20, or Px188; 0.01-10 mM antiox/chel; 10-110 mM salt tonicifier; water; and pH 6.0 (+/- 0.4).

(continued)

J472. 150-195 mg/mL secu; 2-25 mM His; 40-150 mM sorb; 0.01-1.5 mg/mL PS80, PS20, or Px188; 0.01-10 mM antiox/chel; 10-110 mM salt tonicifier; water; and pH 6.0 (+/- 0.4).

J473. 150-195 mg/mL secu; 1-10 mM or 40-60 mM Succ; 40-150 mM sorb; 0.01-1.5 mg/mL PS80, PS20, or Px188; 0.01-10 mM antioxjchel; 10-110 mM salt tonicifier; water; and pH 6.0 (+/- 0.4).

J474. 150-195 mg/mL secu; 1-10 mM Phos; 40-150 mM sorb; 0.01-1.5 mg/mL PS80, PS20, or Px188; 0.01-10 mM antiox/chel; 10-110 mM salt tonicifier; water; and pH 6.0 (+/- 0.4).

J475. 150-195 mg/mL secu; 1-10 mM or 40-60 mM Phos-Cit; 40-150 mM sorb; 0.01-1.5 mg/mL PS80, PS20, or Px188; 0.01-10 mM antioxjchel; 10-110 mM salt tonicifier; water; and pH 6.0 (+/- 0.4).

J476. 150-195 mg/mL secu; 2-60 mM buffer; 150-250 mM treh; 0.01-1.5 mg/mL PS80, PS20, or Px188; 0.01-10 mM antioxjchel; 10-110 mM salt tonicifier; water; and pH 6.0 (+/- 0.4).

J477. 150-195 mg/mL secu; 2-25 mM His; 150-250 mM treh; 0.01-1.5 mg/mL PS80, PS20, or Px188; 0.01-10 mM antiox/chel; 10-110 mM salt tonicifier; water; and pH 6.0 (+/- 0.4).

J478. 150-195 mg/mL secu; 1-10 mM or 40-60 mM Succ; 150-250 mM treh; 0.01-1.5 mg/mL PS80, PS20, or Px188; 0.01-10 mM antioxjchel; 10-110 mM salt tonicifier; water; and pH 6.0 (+/- 0.4).

J479. 150-195 mg/mL secu; 1-10 mM Phos; 150-250 mM treh; 0.01-1.5 mg/mL PS80, PS20, or Px188; 0.01-10 mM antiox/chel; 10-110 mM salt tonicifier; water; and pH 6.0 (+/- 0.4).

J480. 150-195 mg/mL secu; 1-10 mM or 40-60 mM Phos-Cit; 150-250 mM treh; 0.01-1.5 mg/mL PS80, PS20, or Px188; 0.01-10 mM antioxjchel; 10-110 mM salt tonicifier; water; and pH 6.0 (+/- 0.4).

J481. 150-195 mg/mL secu; 2-60 mM buffer; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J482. 150-195 mg/mL secu; 2-25 mM His; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J483. 150-195 mg/mL secu; 1-10 mM or 40-60 mM Succ; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J484. 150-195 mg/mL secu; 1-10 mM Phos; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J485. 150-195 mg/mL secu; 1-10 mM or 40-60 mM Phos-Cit; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J486. 150-195 mg/mL secu; 2-60 mM buffer; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J487. 150-195 mg/mL secu; 2-25 mM His; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J488. 150-195 mg/mL secu; 1-10 mM or 40-60 mM Succ; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J489. 150-195 mg/mL secu; 1-10 mM Phos; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J490. 150-195 mg/mL secu; 1-10 mM or 40-60 mM Phos-Cit; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J491. 150-195 mg/mL secu; 2-60 mM buffer; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J492. 150-195 mg/mL secu; 2-25 mM His; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J493. 150-195 mg/mL secu; 1-10 mM or 40-60 mM Succ; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J494. 150-195 mg/mL secu; 1-10 mM Phos; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J495. 150-195 mg/mL secu; 1-10 mM or 40-60 mM Phos-Cit; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J496. 150-195 mg/mL secu; 2-60 mM buffer; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J497. 150-195 mg/mL secu; 2-25 mM His; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

(continued)

J498. 150-195 mg/mL secu; 1-10 mM or 40-60 mM Succ; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J499. 150-195 mg/mL secu; 1-10 mM Phos; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J500. 150-195 mg/mL secu; 1-10 mM or 40-60 mM Phos-Cit; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J501. 150-195 mg/mL secu; 2-60 mM buffer; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J502. 150-195 mg/mL secu; 2-25 mM His; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J503. 150-195 mg/mL secu; 1-10 mM or 40-60 mM Succ; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J504. 150-195 mg/mL secu; 1-10 mM Phos; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J505. 150-195 mg/mL secu; 1-10 mM or 40-60 mM Phos-Cit; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J506. 150-195 mg/mL secu; 2-60 mM buffer; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J507. 150-195 mg/mL secu; 2-25 mM His; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J508. 150-195 mg/mL secu; 1-10 mM or 40-60 mM Succ; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J509. 150-195 mg/mL secu; 1-10 mM Phos; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J510. 150-195 mg/mL secu; 1-10 mM or 40-60 mM Phos-Cit; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J511. 150-195 mg/mL secu; 2-60 mM buffer; 30-250 mM sug; 0.01-1.5 mg/mL surf; 0.01-10 mM antiox/chel; 40-80 mM NaCl; water; and pH 6.0 (+/- 0.4).

J512. 150-195 mg/mL secu; 2-25 mM His; 30-250 mM sug; 0.01-1.5 mg/mL surf; 0.01-10 mM antiox/chel; 40-80 mM NaCl; water; and pH 6.0 (+/- 0.4).

J513. 150-195 mg/mL secu; 1-10 mM or 40-60 mM Succ; 30-250 mM sug; 0.01-1.5 mg/mL surf; 0.01-10 mM antiox/chel; 40-80 mM NaCl; water; and pH 6.0 (+/- 0.4).

J514. 150-195 mg/mL secu; 1-10 mM Phos; 30-250 mM sug; 0.01-1.5 mg/mL surf; 0.01-10 mM antiox/chel; 40-80 mM NaCl; water; and pH 6.0 (+/- 0.4).

J515. 150-195 mg/mL secu; 1-10 mM or 40-60 mM Phos-Cit; 30-250 mM sug; 0.01-1.5 mg/mL surf; 0.01-10 mM antiox/chel; 40-80 mM NaCl; water; and pH 6.0 (+/- 0.4).

J516. 150-195 mg/mL secu; 2-60 mM buffer; 40-150 mM sorb; 0.01-1.5 mg/mL surf; 0.01-10 mM antiox/chel; 40-80 mM NaCl; water; and pH 6.0 (+/- 0.4).

J517. 150-195 mg/mL secu; 2-25 mM His; 40-150 mM sorb; 0.01-1.5 mg/mL surf; 0.01-10 mM antiox/chel; 40-80 mM NaCl; water; and pH 6.0 (+/- 0.4).

J518. 150-195 mg/mL secu; 1-10 mM or 40-60 mM Succ; 40-150 mM sorb; 0.01-1.5 mg/mL surf; 0.01-10 mM antiox/chel; 40-80 mM NaCl; water; and pH 6.0 (+/- 0.4).

J519. 150-195 mg/mL secu; 1-10 mM Phos; 40-150 mM sorb; 0.01-1.5 mg/mL surf; 0.01-10 mM antiox/chel; 40-80 mM NaCl; water; and pH 6.0 (+/- 0.4).

J520. 150-195 mg/mL secu; 1-10 mM or 40-60 mM Phos-Cit; 40-150 mM sorb; 0.01-1.5 mg/mL surf; 0.01-10 mM antiox/chel; 40-80 mM NaCl; water; and pH 6.0 (+/- 0.4).

J521. 150-195 mg/mL secu; 2-60 mM buffer; 150-250 mM treh; 0.01-1.5 mg/mL surf; 0.01-10 mM antiox/chel; 40-80 mM NaCl; water; and pH 6.0 (+/- 0.4).

J522. 150-195 mg/mL secu; 2-25 mM His; 150-250 mM treh; 0.01-1.5 mg/mL surf; 0.01-10 mM antiox/chel; 40-80 mM NaCl; water; and pH 6.0 (+/- 0.4).

J523. 150-195 mg/mL secu; 1-10 mM or 40-60 mM Succ; 150-250 mM treh; 0.01-1.5 mg/mL surf; 0.01-10 mM antiox/chel; 40-80 mM NaCl; water; and pH 6.0 (+/- 0.4).

(continued)

J524. 150-195 mg/mL secu; 1-10 mM Phos; 150-250 mM treh; 0.01-1.5 mg/mL surf; 0.01-10 mM antiox/chel; 40-80 mM NaCl; water; and pH 6.0 (+/- 0.4).

J525. 150-195 mg/mL secu; 1-10 mM or 40-60 mM Phos-Cit; 150-250 mM treh; 0.01-1.5 mg/mL surf; 0.01-10 mM antiox/chel; 40-80 mM NaCl; water; and pH 6.0 (+/- 0.4).

J526. 150-195 mg/mL secu; 2-60 mM buffer; 30-250 mM sug; 0.01-1.5 mg/mL PS80, PS20, or Px188; 0.01-10 mM antiox/chel; 40-80 mM NaCl; water; and pH 6.0 (+/- 0.4).

J527. 150-195 mg/mL secu; 2-25 mM His; 30-250 mM sug; 0.01-1.5 mg/mL PS80, PS20, or Px188; 0.01-10 mM antiox/chel; 40-80 mM NaCl; water; and pH 6.0 (+/- 0.4).

J528. 150-195 mg/mL secu; 1-10 mM or 40-60 mM Succ; 30-250 mM sug; 0.01-1.5 mg/mL PS80, PS20, or Px188; 0.01-10 mM antiox/chel; 40-80 mM NaCl; water; and pH 6.0 (+/- 0.4).

J529. 150-195 mg/mL secu; 1-10 mM Phos; 30-250 mM sug; 0.01-1.5 mg/mL PS80, PS20, or Px188; 0.01-10 mM antiox/chel; 40-80 mM NaCl; water; and pH 6.0 (+/- 0.4).

J530. 150-195 mg/mL secu; 1-10 mM or 40-60 mM Phos-Cit; 30-250 mM sug; 0.01-1.5 mg/mL PS80, PS20, or Px188; 0.01-10 mM antiox/chel; 40-80 mM NaCl; water; and pH 6.0 (+/- 0.4).

J531. 150-195 mg/mL secu; 2-60 mM buffer; 40-150 mM sorb; 0.01-1.5 mg/mL PS80, PS20, or Px188; 0.01-10 mM antiox/chel; 40-80 mM NaCl; water; and pH 6.0 (+/- 0.4).

J532. 150-195 mg/mL secu; 2-25 mM His; 40-150 mM sorb; 0.01-1.5 mg/mL PS80, PS20, or Px188; 0.01-10 mM antiox/chel; 40-80 mM NaCl; water; and pH 6.0 (+/- 0.4).

J533. 150-195 mg/mL secu; 1-10 mM or 40-60 mM Succ; 40-150 mM sorb; 0.01-1.5 mg/mL PS80, PS20, or Px188; 0.01-10 mM antiox/chel; 40-80 mM NaCl; water; and pH 6.0 (+/- 0.4).

J534. 150-195 mg/mL secu; 1-10 mM Phos; 40-150 mM sorb; 0.01-1.5 mg/mL PS80, PS20, or Px188; 0.01-10 mM antiox/chel; 40-80 mM NaCl; water; and pH 6.0 (+/- 0.4).

J535. 150-195 mg/mL secu; 1-10 mM or 40-60 mM Phos-Cit; 40-150 mM sorb; 0.01-1.5 mg/mL PS80, PS20, or Px188; 0.01-10 mM antiox/chel; 40-80 mM NaCl; water; and pH 6.0 (+/- 0.4).

J536. 150-195 mg/mL secu; 2-60 mM buffer; 150-250 mM treh; 0.01-1.5 mg/mL PS80, PS20, or Px188; 0.01-10 mM antiox/chel; 40-80 mM NaCl; water; and pH 6.0 (+/- 0.4).

J537. 150-195 mg/mL secu; 2-25 mM His; 150-250 mM treh; 0.01-1.5 mg/mL PS80, PS20, or Px188; 0.01-10 mM antiox/chel; 40-80 mM NaCl; water; and pH 6.0 (+/- 0.4).

J538. 150-195 mg/mL secu; 1-10 mM or 40-60 mM Succ; 150-250 mM treh; 0.01-1.5 mg/mL PS80, PS20, or Px188; 0.01-10 mM antiox/chel; 40-80 mM NaCl; water; and pH 6.0 (+/- 0.4).

J539. 150-195 mg/mL secu; 1-10 mM Phos; 150-250 mM treh; 0.01-1.5 mg/mL PS80, PS20, or Px188; 0.01-10 mM antiox/chel; 40-80 mM NaCl; water; and pH 6.0 (+/- 0.4).

J540. 150-195 mg/mL secu; 1-10 mM or 40-60 mM Phos-Cit; 150-250 mM treh; 0.01-1.5 mg/mL PS80, PS20, or Px188; 0.01-10 mM antiox/chel; 40-80 mM NaCl; water; and pH 6.0 (+/- 0.4).

J541. 150-195 mg/mL secu; 2-60 mM buffer; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J542. 150-195 mg/mL secu; 2-25 mM His; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J543. 150-195 mg/mL secu; 1-10 mM or 40-60 mM Succ; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J544. 150-195 mg/mL secu; 1-10 mM Phos; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J545. 150-195 mg/mL secu; 1-10 mM or 40-60 mM Phos-Cit; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J546. 150-195 mg/mL secu; 2-60 mM buffer; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J547. 150-195 mg/mL secu; 2-25 mM His; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J548. 150-195 mg/mL secu; 1-10 mM or 40-60 mM Succ; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J549. 150-195 mg/mL secu; 1-10 mM Phos; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

(continued)

J550. 150-195 mg/mL secu; 1-10 mM or 40-60 mM Phos-Cit; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J551. 150-195 mg/mL secu; 2-60 mM buffer; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J552. 150-195 mg/mL secu; 2-25 mM His; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J553. 150-195 mg/mL secu; 1-10 mM or 40-60 mM Succ; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J554. 150-195 mg/mL secu; 1-10 mM Phos; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J555. 150-195 mg/mL secu; 1-10 mM or 40-60 mM Phos-Cit; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J556. 150-195 mg/mL secu; 2-60 mM buffer; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J557. 150-195 mg/mL secu; 2-25 mM His; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J558. 150-195 mg/mL secu; 1-10 mM or 40-60 mM Succ; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J559. 150-195 mg/mL secu; 1-10 mM Phos; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J560. 150-195 mg/mL secu; 1-10 mM or 40-60 mM Phos-Cit; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J561. 150-195 mg/mL secu; 2-60 mM buffer; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J562. 150-195 mg/mL secu; 2-25 mM His; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J563. 150-195 mg/mL secu; 1-10 mM or 40-60 mM Succ; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J564. 150-195 mg/mL secu; 1-10 mM Phos; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J565. 150-195 mg/mL secu; 1-10 mM or 40-60 mM Phos-Cit; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J566. 150-195 mg/mL secu; 2-60 mM buffer; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J567. 150-195 mg/mL secu; 2-25 mM His; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J568. 150-195 mg/mL secu; 1-10 mM or 40-60 mM Succ; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J569. 150-195 mg/mL secu; 1-10 mM Phos; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J570. 150-195 mg/mL secu; 1-10 mM or 40-60 mM Phos-Cit; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J571. 150-195 mg/mL secu; 2-60 mM buffer; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J572. 150-195 mg/mL secu; 2-25 mM His; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J573. 150-195 mg/mL secu; 1-10 mM or 40-60 mM Succ; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J574. 150-195 mg/mL secu; 1-10 mM Phos; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J575. 150-195 mg/mL secu; 1-10 mM or 40-60 mM Phos-Cit; 30-250 mM sug; 10-200 mM AA; 0.01-1.5 mg/mL surf; 0.01-10 mM antiox/chel; 10-110 mM salt tonicifier; water; and pH 6.0 (+/- 0.4).

(continued)

J576. 150-195 mg/mL secu; 2-60 mM buffer; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J577. 150-195 mg/mL secu; 2-25 mM His; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J578. 150-195 mg/mL secu; 1-1 0 mM or 40-60 mM Succ; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J579. 150-195 mg/mL secu; 1-10 mM Phos; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J580. 150-195 mg/mL secu; 1-10 mM or 40-60 mM Phos-Cit; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J581. 150-195 mg/mL secu; 2-60 mM buffer; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J582. 150-195 mg/mL secu; 2-25 mM His; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J583. 150-195 mg/mL secu; 1-10 mM or 40-60 mM Succ; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J584. 150-195 mg/mL secu; 1-10 mM Phos; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J585. 150-195 mg/mL secu; 1-10 mM or 40-60 mM Phos-Cit; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J586. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J587. 150-195 mg/mL secu; 2-25 mM His; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J588. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J589. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J590. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J591. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J592. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J593. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J594. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J595. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J596. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J597. 150-195 mg/mL secu; 2-25 mM His; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J598. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J599. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

(continued)

J600. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM antioxjchel; 10-110 mM salt tonicifier; water; and pH 6.0 (+/- 0.4).

J601. 150-195 mg/mL secu; 2-60 mM buffer; 30-250 mM sug; 10-200 mM AA; 0.01-1.5 mg/mL PS80, PS20, or Px188; 0.01-10 mM antioxjchel; 10-110 mM salt tonicifier; water; and pH 6.0 (+/- 0.4).

J602. 150-195 mg/mL secu; 2-25 mM His; 30-250 mM sug; 10-200 mM AA; 0.01-1.5 mg/mL PS80, PS20, or Px188; 0.01-10 mM antioxjchel; 10-110 mM salt tonicifier; water; and pH 6.0 (+/- 0.4).

J603. 150-195 mg/mL secu; 1-10 mM or 40-60 mM Succ; 30-250 mM sug; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J604. 150-195 mg/mL secu; 1-10 mM Phos; 30-250 mM sug; 10-200 mM AA; 0.01-1.5 mg/mL PS80, PS20, or Px188; 0.01-10 mM antioxjchel; 10-110 mM salt tonicifier; water; and pH 6.0 (+/- 0.4).

J605. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J606. 150-195 mg/mL secu; 2-60 mM buffer; 40-150 mM sorb; 10-200 mM AA; 0.01-1.5 mg/mL PS80, PS20, or Px188; 0.01-10 mM antioxjchel; 10-110 mM salt tonicifier; water; and pH 6.0 (+/- 0.4).

J607. 150-195 mg/mL secu; 2-25 mM His; 40-150 mM sorb; 10-200 mM AA; 0.01-1.5 mg/mL PS80, PS20, or Px188; 0.01-10 mM antioxjchel; 10-110 mM salt tonicifier; water; and pH 6.0 (+/- 0.4).

J608. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J609. 150-195 mg/mL secu; 1-10 mM Phos; 40-150 mM sorb; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J610. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J611. 150-195 mg/mL secu; 2-60 mM buffer; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J612. 150-195 mg/mL secu; 2-25 mM His; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J613. 150-195 mg/mL secu; 1-10 mM or 40-60 mM Succ; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J614. 150-195 mg/mL secu; 1-10 mM Phos; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J615. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J616. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J617. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J618. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J619. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J620. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J621. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J622. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J623. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

(continued)

J624. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J625. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J626. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J627. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J628. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J629. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J630. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J631. 150-195 mg/mL secu; 2-60 mM buffer; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J632. 150-195 mg/mL secu; 2-25 mM His; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J633. 150-195 mg/mL secu; 1-10 mM or 40-60 mM Succ; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J634. 150-195 mg/mL secu; 1-10 mM Phos; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J635. 150-195 mg/mL secu; 1-10 mM or 40-60 mM Phos-Cit; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J636. 150-195 mg/mL secu; 2-60 mM buffer; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J637. 150-195 mg/mL secu; 2-25 mM His; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J638. 150-195 mg/mL secu; 1-10 mM or 40-60 mM Succ; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J639. 150-195 mg/mL secu; 1-10 mM Phos; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J640. 150-195 mg/mL secu; 1-10 mM or 40-60 mM Phos-Cit; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J641. 150-195 mg/mL secu; 2-60 mM buffer; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J642. 150-195 mg/mL secu; 2-25 mM His; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J643. 150-195 mg/mL secu; 1-10 mM or 40-60 mM Succ; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J644. 150-195 mg/mL secu; 1-10 mM Phos; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J645. 150-195 mg/mL secu; 1-10 mM or 40-60 mM Phos-Cit; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J646. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

(continued)

J647. 150-195 mg/mL secu; 2-25 mM His; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J648. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J649. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J650. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J651. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J652. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J653. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J654. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J655. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J656. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J657. 150-195 mg/mL secu; 2-25 mM His; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J658. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J659. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J660. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J661. 150-195 mg/mL secu; 2-60 mM buffer; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J662. 150-195 mg/mL secu; 2-25 mM His; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J663. 150-195 mg/mL secu; 1-10 mM or 40-60 mM Succ; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J664. 150-195 mg/mL secu; 1-10 mM Phos; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J665. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

(continued)

J666. 150-195 mg/mL secu; 2-60 mM buffer; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J667. 150-195 mg/mL secu; 2-25 mM His; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J668. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J669. 150-195 mg/mL secu; 1-10 mM Phos; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J670. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J671. 150-195 mg/mL secu; 2-60 mM buffer; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J672. 150-195 mg/mL secu; 2-25 mM His; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J673. 150-195 mg/mL secu; 1-10 mM or 40-60 mM Succ; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J674. 150-195 mg/mL secu; 1-10 mM Phos; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J675. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J676. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J677. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J678. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J679. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J680. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J681. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J682. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J683. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J684. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J685. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

(continued)

J686. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J687. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J688. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J689. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J690. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J691. 150-195 mg/mL secu; 2-60 mM buffer; 30-250 mM sug; 10-200 mM AA; 0.01-1.5 mg/mL surf; 0.01-10 mM antiox/chel; 40-80 mM NaCl; water; and pH 6.0 (+/- 0.4).

J692. 150-195 mg/mL secu; 2-25 mM His; 30-250 mM sug; 10-200 mM AA; 0.01-1.5 mg/mL surf; 0.01-10 mM antiox/chel; 40-80 mM NaCl; water; and pH 6.0 (+/- 0.4).

J693. 150-195 mg/mL secu; 1-10 mM or 40-60 mM Succ; 30-250 mM sug; 10-200 mM AA; 0.01-1.5 mg/mL surf; 0.01-10 mM antiox/chel; 40-80 mM NaCl; water; and pH 6.0 (+/- 0.4).

J694. 150-195 mg/mL secu; 1-10 mM Phos; 30-250 mM sug; 10-200 mM AA; 0.01-1.5 mg/mL surf; 0.01-10 mM antiox/chel; 40-80 mM NaCl; water; and pH 6.0 (+/- 0.4).

J695. 150-195 mg/mL secu; 1-10 mM or 40-60 mM Phos-Cit; 30-250 mM sug; 10-200 mM AA; 0.01-1.5 mg/mL surf; 0.01-10 mM antiox/chel; 40-80 mM NaCl; water; and pH 6.0 (+/- 0.4).

J696. 150-195 mg/mL secu; 2-60 mM buffer; 40-150 mM sorb; 10-200 mM AA; 0.01-1.5 mg/mL surf; 0.01-10 mM antiox/chel; 40-80 mM NaCl; water; and pH 6.0 (+/- 0.4).

J697. 150-195 mg/mL secu; 2-25 mM His; 40-150 mM sorb; 10-200 mM AA; 0.01-1.5 mg/mL surf; 0.01-10 mM antiox/chel; 40-80 mM NaCl; water; and pH 6.0 (+/- 0.4).

J698. 150-195 mg/mL secu; 1-10 mM or 40-60 mM Succ; 40-150 mM sorb; 10-200 mM AA; 0.01-1.5 mg/mL surf; 0.01-10 mM antiox/chel; 40-80 mM NaCl; water; and pH 6.0 (+/- 0.4).

J699. 150-195 mg/mL secu; 1-10 mM Phos; 40-150 mM sorb; 10-200 mM AA; 0.01-1.5 mg/mL surf; 0.01-10 mM antiox/chel; 40-80 mM NaCl; water; and pH 6.0 (+/- 0.4).

J700. 150-195 mg/mL secu; 1-10 mM or 40-60 mM Phos-Cit; 40-150 mM sorb; 10-200 mM AA; 0.01-1.5 mg/mL surf; 0.01-10 mM antiox/chel; 40-80 mM NaCl; water; and pH 6.0 (+/- 0.4).

J701. 150-195 mg/mL secu; 2-60 mM buffer; 150-250 mM treh; 10-200 mM AA; 0.01-1.5 mg/mL surf; 0.01-10 mM antiox/chel; 40-80 mM NaCl; water; and pH 6.0 (+/- 0.4).

J702. 150-195 mg/mL secu; 2-25 mM His; 150-250 mM treh; 10-200 mM AA; 0.01-1.5 mg/mL surf; 0.01-10 mM antiox/chel; 40-80 mM NaCl; water; and pH 6.0 (+/- 0.4).

J703. 150-195 mg/mL secu; 1-10 mM or 40-60 mM Succ; 150-250 mM treh; 10-200 mM AA; 0.01-1.5 mg/mL surf; 0.01-10 mM antiox/chel; 40-80 mM NaCl; water; and pH 6.0 (+/- 0.4).

J704. 150-195 mg/mL secu; 1-10 mM Phos; 150-250 mM treh; 10-200 mM AA; 0.01-1.5 mg/mL surf; 0.01-10 mM antiox/chel; 40-80 mM NaCl; water; and pH 6.0 (+/- 0.4).

J705. 150-195 mg/mL secu; 1-10 mM or 40-60 mM Phos-Cit; 150-250 mM treh; 10-200 mM AA; 0.01-1.5 mg/mL surf; 0.01-10 mM antiox/chel; 40-80 mM NaCl; water; and pH 6.0 (+/- 0.4).

J706. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J707. 150-195 mg/mL secu; 2-25 mM His; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J708. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J709. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

(continued)

J710. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J711. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J712. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J713. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J714. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J715. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J716. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J717. 150-195 mg/mL secu; 2-25 mM His; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J718. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J719. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J720. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J721. 150-195 mg/mL secu; 2-60 mM buffer; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J722. 150-195 mg/mL secu; 2-25 mM His; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J723. 150-195 mg/mL secu; 1-10 mM or 40-60 mM Succ; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J724. 150-195 mg/mL secu; 1-10 mM Phos; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J725. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J726. 150-195 mg/mL secu; 2-60 mM buffer; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J727. 150-195 mg/mL secu; 2-25 mM His; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J728. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J729. 150-195 mg/mL secu; 1-10 mM Phos; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J730. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J731. 150-195 mg/mL secu; 2-60 mM buffer; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J732. 150-195 mg/mL secu; 2-25 mM His; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J733. 150-195 mg/mL secu; 1-10 mM or 40-60 mM Succ; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J734. 150-195 mg/mL secu; 1-10 mM Phos; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J735. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

(continued)

J736. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J737. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J738. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J739. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J740. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J741. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J742. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J743. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J744. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J745. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J746. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J747. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J748. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J749. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J750. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J751. 150-195 mg/mL secu; 2-60 mM buffer; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J752. 150-195 mg/mL secu; 2-25 mM His; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J753. 150-195 mg/mL secu; 1-10 mM or 40-60 mM Succ; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J754. 150-195 mg/mL secu; 1-10 mM Phos; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J755. 150-195 mg/mL secu; 1-10 mM or 40-60 mM Phos-Cit; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J756. 150-195 mg/mL secu; 2-60 mM buffer; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J757. 150-195 mg/mL secu; 2-25 mM His; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J758. 150-195 mg/mL secu; 1-1 0 mM or 40-60 mM Succ; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

(continued)

J759. 150-195 mg/mL secu; 1-10 mM Phos; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J760. 150-195 mg/mL secu; 1-1 0 mM or 40-60 mM Phos-Cit; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J761. 150-195 mg/mL secu; 2-60 mM buffer; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J762. 150-195 mg/mL secu; 2-25 mM His; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J763. 150-195 mg/mL secu; 1-10 mM or 40-60 mM Succ; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J764. 150-195 mg/mL secu; 1-1 0 mM Phos; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J765. 150-195 mg/mL secu; 1-10 mM or 40-60 mM Phos-Cit; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J766. 150-195 mg/mL secu; 2-60 mM buffer; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J767. 150-195 mg/mL secu; 2-25 mM His; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J768. 150-195 mg/mL secu; 1-10 mM or 40-60 mM Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J769. 150-195 mg/mL secu; 1-10 mM Phos; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J770. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J771. 150-195 mg/mL secu; 2-60 mM buffer; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J772. 150-195 mg/mL secu; 2-25 mM His; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J773. 150-195 mg/mL secu; 1-10 mM or 40-60 mM Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J774. 150-195 mg/mL secu; 1-10 mM Phos; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J775. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J776. 150-195 mg/mL secu; 2-60 mM buffer; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J777. 150-195 mg/mL secu; 2-25 mM His; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J778. 150-195 mg/mL secu; 1-10 mM or 40-60 mM Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J779. 150-195 mg/mL secu; 1-10 mM Phos; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J780. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J781. 150-195 mg/mL secu; 2-60 mM buffer; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

(continued)

J782. 150-195 mg/mL secu; 2-25 mM His; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J783. 150-195 mg/mL secu; 1-10 mM or 40-60 mM Succ; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J784. 150-195 mg/mL secu; 1-10 mM Phos; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J785. 150-195 mg/mL secu; 1-10 mM or 40-60 mM Phos-Cit; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J786. 150-195 mg/mL secu; 2-60 mM buffer; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J787. 150-195 mg/mL secu; 2-25 mM His; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J788. 150-195 mg/mL secu; 1-10 mM or 40-60 mM Succ; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J789. 150-195 mg/mL secu; 1-10 mM Phos; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J790. 150-195 mg/mL secu; 1-10 mM or 40-60 mM Phos-Cit; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J791. 150-195 mg/mL secu; 2-60 mM buffer; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J792. 150-195 mg/mL secu; 2-25 mM His; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J793. 150-195 mg/mL secu; 1-10 mM or 40-60 mM Succ; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J794. 150-195 mg/mL secu; 1-10 mM Phos; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J795. 150-195 mg/mL secu; 1-10 mM or 40-60 mM Phos-Cit; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J796. 150-195 mg/mL secu; 2-60 mM buffer; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J797. 150-195 mg/mL secu; 2-25 mM His; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J798. 150-195 mg/mL secu; 1-10 mM or 40-60 mM Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J799. 150-195 mg/mL secu; 1-10 mM Phos; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J800. 150-195 mg/mL secu; 1-10 mM or 40-60 mM Phos-Cit; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J801. 150-195 mg/mL secu; 2-60 mM buffer; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

(continued)

J802. 150-195 mg/mL secu; 2-25 mM His; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J803. 150-195 mg/mL secu; 1-10 mM or 40-60 mM Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J804. 150-195 mg/mL secu; 1-10 mM Phos; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J805. 150-195 mg/mL secu; 1-10 mM or 40-60 mM Phos-Cit; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J806. 150-195 mg/mL secu; 2-60 mM buffer; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J807. 150-195 mg/mL secu; 2-25 mM His; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J808. 150-195 mg/mL secu; 1-10 mM or 40-60 mM Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J809. 150-195 mg/mL secu; 1-10 mM Phos; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J810. 150-195 mg/mL secu; 1-10 mM or 40-60 mM Phos-Cit; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J811. 150-195 mg/mL secu; 40-60 mM MES; 40-150 mM sorb; 0.01-1.5 mg/mL surf; water; and pH 6.0 (+/- 0.4).

J812. 150-195 mg/mL secu; 40-60 mM MES; 40-150 mM sorb; 0.01-1.5 mg/mL PS80, PS20, or Px188; water; and pH 6.0 (+/-0.4).

J813. 150-195 mg/mL secu; 40-60 mM MES; 40-150 mM sorb; 10-200 mM Arg, Gly, Lys, Pro, and/or Phe; 0.01-1.5 mg/mL surf; water; and pH 6.0 (+/- 0.4).

J814. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J815. 150-195 mg/mL secu; 40-60 mM MES; 40-150 mM sorb; 0.01-1.5 mg/mL surf; 0.01-10 mM Met, Cys, EDTA, and/or DTPA; water; and pH 6.0 (+/- 0.4).

J816. 150-195 mg/mL secu; 40-60 mM MES; 40-150 mM sorb; 0.01-1.5 mg/mL PS80, PS20, or Px188; 0.01-10 mM Met, Cys, EDTA, and/or DTPA; water; and pH 6.0 (+/- 0.4).

J817. 150-195 mg/mL secu; 40-60 mM MES; 40-150 mM sorb; 0.01-1.5 mg/mL surf; a salt tonicifier (preferably NaCl); water; and pH 6.0 (+/- 0.4).

J818. 150-195 mg/mL secu; 40-60 mM MES; 40-150 mM sorb; 0.01-1.5 mg/mL PS80, PS20, or Px188; a salt tonicifier (preferably NaCl); water; and pH 6.0 (+/- 0.4).

J819. 150-195 mg/mL secu; 40-60 mM MES; 40-150 mM sorb; 0.01-1.5 mg/mL surf; 40-80 mM salt tonicifier (preferably NaCl); water; and pH 6.0 (+/- 0.4).

J820. 150-195 mg/mL secu; 40-60 mM MES; 40-150 mM sorb; 0.01-1.5 mg/mL PS80, PS20, or Px188; 40-80 mM salt tonicifier (preferably NaCl); water; and pH 6.0 (+/- 0.4).

J821. 150-195 mg/mL secu; 40-60 mM MES; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J822. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J823. 150-195 mg/mL secu; 40-60 mM MES; 40-150 mM sorb; 10-200 mM Arg, Gly, Lys, Pro, and/or Phe; 0.01-1.5 mg/mL surf; a salt tonicifier (preferably NaCl); water; and pH 6.0 (+/- 0.4).

(continued)

J824. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; a salt tonicifier (preferably NaCl); water; and pH 6.0 (+/- 0.4).

J825. 150-195 mg/mL secu; 40-60 mM MES; 40-150 mM sorb; 10-200 mM Arg, Gly, Lys, Pro, and/or Phe; 0.01-1.5 mg/mL surf; 40-80 mM salt tonicifier (preferably NaCl); water; and pH 6.0 (+/- 0.4).

J826. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; 40-80 mM salt tonicifier (preferably NaCl); water; and pH 6.0 (+/- 0.4).

J827. 150-195 mg/mL secu; 40-60 mM MES; 40-150 mM sorb; 0.01-1.5 mg/mL surf; 0.01-10 mM Met, Cys, EDTA, and/or DTPA; a salt tonicifier (preferably NaCl); water; and pH 6.0 (+/- 0.4).

J828. 150-195 mg/mL secu; 40-60 mM MES; 40-150 mM sorb; 0.01-1.5 mg/mL PS80, PS20, or Px188; 0.01-10 mM Met, Cys, EDTA, and/or DTPA; a salt tonicifier (preferably NaCl); water; and pH 6.0 (+/- 0.4).

J829. 150-195 mg/mL secu; 40-60 mM MES; 40-150 mM sorb; 0.01-1.5 mg/mL surf; 0.01-10 mM Met, Cys, EDTA, and/or DTPA; 40-80 mM salt tonicifier (preferably NaCl); water; and pH 6.0 (+/- 0.4).

J830. 150-195 mg/mL secu; 40-60 mM MES; 40-150 mM sorb; 0.01-1.5 mg/mL PS80, PS20, or Px188; 0.01-10 mM Met, Cys, EDTA, and/or DTPA; 40-80 mM salt tonicifier (preferably NaCl); water; and pH 6.0 (+/- 0.4).

J831. 150-195 mg/mL secu; 40-60 mM MES; 40-150 mM sorb; 10-200 mM Arg, Gly, Lys, Pro, and/or Phe; 0.01-1.5 mg/mL surf; 0.01-10 mM Met, Cys, EDTA, and/or DTPA; a salt tonicifier (preferably NaCl); water; and pH 6.0 (+/- 0.4).

J832. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; a salt tonicifier (preferably NaCl); water; and pH 6.0 (+/- 0.4).

J833. 150-195 mg/mL secu; 40-60 mM MES; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM salt tonicifier (preferably NaCl); water; and pH 6.0 (+/- 0.4).

J834. 150-195 mg/mL secu; 40-60 mM MES; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM salt tonicifier (preferably NaCl); water; and pH 6.0 (+/- 0.4).

J835. 150-195 mg/mL secu; 1-10 mM or 40-60 mM Phos-Succ; 30-250 mM sug; 0.01-1.5 mg/mL surf; water; and pH 6.0 (+/- 0.4).

J836. 150-195 mg/mL secu; 1-10 mM or 40-60 mM Phos-Acet; 30-250 mM sug; 0.01-1.5 mg/mL surf; water; and pH 6.0 (+/- 0.4).

J837. 150-195 mg/mL secu; 1-10 mM or 40-60 mM Phos-Succ; 40-150 mM sorb; 0.01-1.5 mg/mL surf; water; and pH 6.0 (+/-0.4).

J838. 150-195 mg/mL secu; 1-10 mM or 40-60 mM Phos-Acet; 40-150 mM sorb; 0.01-1.5 mg/mL surf; water; and pH 6.0 (+/- 0.4).

J839. 150-195 mg/mL secu; 1-10 mM or 40-60 mM Phos-Succ; 150-250 mM treh; 0.01-1.5 mg/mL surf; water; and pH 6.0 (+/-0.4).

J840. 150-195 mg/mL secu; 1-10 mM or 40-60 mM Phos-Acet; 150-250 mM treh; 0.01-1.5 mg/mL surf; water; and pH 6.0 (+/-0.4).

J841. 150-195 mg/mL secu; 1-10 mM or 40-60 mM Phos-Succ; 30-250 mM sug; 0.01-1.5 mg/mL PS80, PS20, or Px188; water; and pH 6.0 (+/- 0.4).

J842. 150-195 mg/mL secu; 1-10 mM or 40-60 mM Phos-Acet; 30-250 mM sug; 0.01-1.5 mg/mL PS80, PS20, or Px188; water; and pH 6.0 (+/- 0.4).

J843. 150-195 mg/mL secu; 1-10 mM or 40-60 mM Phos-Succ; 40-150 mM sorb; 0.01-1.5 mg/mL PS80, PS20, or Px188; water; and pH 6.0 (+/- 0.4).

J844. 150-195 mg/mL secu; 1-10 mM or 40-60 mM Phos-Acet; 40-150 mM sorb; 0.01-1.5 mg/mL PS80, PS20, or Px188; water; and pH 6.0 (+/- 0.4).

J845. 150-195 mg/mL secu; 1-10 mM or 40-60 mM Phos-Succ; 150-250 mM treh; 0.01-1.5 mg/mL PS80, PS20, or Px188; water; and pH 6.0 (+/- 0.4).

J846. 150-195 mg/mL secu; 1-10 mM or 40-60 mM Phos-Acet; 150-250 mM treh; 0.01-1.5 mg/mL PS80, PS20, or Px188; water; and pH 6.0 (+/- 0.4).

J847. 150-195 mg/mL secu; 1-10 mM or 40-60 mM Phos-Succ; 30-250 mM sug; 10-200 mM AA; 0.01-1.5 mg/mL surf; water; and pH 6.0 (+/- 0.4).

(continued)

J848. 150-195 mg/mL secu; 1-10 mM or 40-60 mM Phos-Acet; 30-250 mM sug; 10-200 mM AA; 0.01-1.5 mg/mL surf; water; and pH 6.0 (+/- 0.4).

J849. 150-195 mg/mL secu; 1-10 mM or 40-60 mM Phos-Succ; 40-150 mM sorb; 10-200 mM AA; 0.01-1.5 mg/mL surf; water; and pH 6.0 (+/- 0.4).

J850. 150-195 mg/mL secu; 1-10 mM or 40-60 mM Phos-Acet; 40-150 mM sorb; 10-200 mM AA; 0.01-1.5 mg/mL surf; water; and pH 6.0 (+/- 0.4).

J851. 150-195 mg/mL secu; 1-10 mM or 40-60 mM Phos-Succ; 150-250 mM treh; 10-200 mM AA; 0.01-1.5 mg/mL surf; water; and pH 6.0 (+/- 0.4).

J852. 150-195 mg/mL secu; 1-10 mM or 40-60 mM Phos-Acet; 150-250 mM treh; 10-200 mM AA; 0.01-1.5 mg/mL surf; water; and pH 6.0 (+/- 0.4).

J853. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; water; and pH 6.0 (+/- 0.4).

J854. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; water; and pH 6.0 (+/- 0.4).

J855. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; water; and pH 6.0 (+/- 0.4).

J856. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; water; and pH 6.0 (+/- 0.4).

J857. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; water; and pH 6.0 (+/- 0.4).

J858. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; water; and pH 6.0 (+/- 0.4).

J859. 150-195 mg/mL secu; 1-10 mM or 40-60 mM Phos-Succ; 30-250 mM sug; 10-200 mM AA; 0.01-1.5 mg/mL PS80, PS20, or Px188; water; and pH 6.0 (+/- 0.4).

J860. 150-195 mg/mL secu; 1-10 mM or 40-60 mM Phos-Acet; 30-250 mM sug; 10-200 mM AA; 0.01-1.5 mg/mL PS80, PS20, or Px188; water; and pH 6.0 (+/- 0.4).

J861. 150-195 mg/mL secu; 1-10 mM or 40-60 mM Phos-Succ; 40-150 mM sorb; 10-200 mM AA; 0.01-1.5 mg/mL PS80, PS20, or Px188; water; and pH 6.0 (+/- 0.4).

J862. 150-195 mg/mL secu; 1-10 mM or 40-60 mM Phos-Acet; 40-150 mM sorb; 10-200 mM AA; 0.01-1.5 mg/mL PS80, PS20, or Px188; water; and pH 6.0 (+/- 0.4).

J863. 150-195 mg/mL secu; 1-10 mM or 40-60 mM Phos-Succ; 150-250 mM treh; 10-200 mM AA; 0.01-1.5 mg/mL PS80, PS20, or Px188; water; and pH 6.0 (+/- 0.4).

J864. 150-195 mg/mL secu; 1-10 mM or 40-60 mM Phos-Acet; 150-250 mM treh; 10-200 mM AA; 0.01-1.5 mg/mL PS80, PS20, or Px188; water; and pH 6.0 (+/- 0.4).

J865. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; water; and pH 6.0 (+/- 0.4).

J866. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; water; and pH 6.0 (+/- 0.4).

J867. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J868. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; water; and pH 6.0 (+/- 0.4).

J869. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; water; and pH 6.0 (+/- 0.4).

J870. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; water; and pH 6.0 (+/- 0.4).

J871. 150-195 mg/mL secu; 1-10 mM or 40-60 mM Phos-Succ; 30-250 mM sug; 0.01-1.5 mg/mL surf; 0.01-10 mM antiox/chel; water; and pH 6.0 (+/- 0.4).

J872. 150-195 mg/mL secu; 1-10 mM or 40-60 mM Phos-Acet; 30-250 mM sug; 0.01-1.5 mg/mL surf; 0.01-10 mM antiox/chel; water; and pH 6.0 (+/- 0.4).

J873. 150-195 mg/mL secu; 1-10 mM or 40-60 mM Phos-Succ; 40-150 mM sorb; 0.01-1.5 mg/mL surf; 0.01-10 mM antiox/chel; water; and pH 6.0 (+/- 0.4).

(continued)

J874. 150-195 mg/mL secu; 1-10 mM or 40-60 mM Phos-Acet; 40-150 mM sorb; 0.01-1.5 mg/mL surf; 0.01-10 mM antiox/chel; water; and pH 6.0 (+/- 0.4).

J875. 150-195 mg/mL secu; 1-10 mM or 40-60 mM Phos-Succ; 150-250 mM treh; 0.01-1.5 mg/mL surf; 0.01-10 mM antiox/chel; water; and pH 6.0 (+/- 0.4).

J876. 150-195 mg/mL secu; 1-10 mM or 40-60 mM Phos-Acet; 150-250 mM treh; 0.01-1.5 mg/mL surf; 0.01-10 mM antiox/chel; water; and pH 6.0 (+/- 0.4).

J877. 150-195 mg/mL secu; 1-10 mM or 40-60 mM Phos-Succ; 30-250 mM sug; 0.01-1.5 mg/mL PS80, PS20, or Px188; 0.01-10 mM antiox/chel; water; and pH 6.0 (+/- 0.4).

J878. 150-195 mg/mL secu; 1-10 mM or 40-60 mM Phos-Acet; 30-250 mM sug; 0.01-1.5 mg/mL PS80, PS20, or Px188; 0.01-10 mM antiox/chel; water; and pH 6.0 (+/- 0.4).

J879. 150-195 mg/mL secu; 1-10 mM or 40-60 mM Phos-Succ; 40-150 mM sorb; 0.01-1.5 mg/mL PS80, PS20, or Px188; 0.01-10 mM antiox/chel; water; and pH 6.0 (+/- 0.4).

J880. 150-195 mg/mL secu; 1-10 mM or 40-60 mM Phos-Acet; 40-150 mM sorb; 0.01-1.5 mg/mL PS80, PS20, or Px188; 0.01-10 mM antiox/chel; water; and pH 6.0 (+/- 0.4).

J881. 150-195 mg/mL secu; 1-10 mM or 40-60 mM Phos-Succ; 150-250 mM treh; 0.01-1.5 mg/mL PS80, PS20, or Px188; 0.01-10 mM antiox/chel; water; and pH 6.0 (+/- 0.4).

J882. 150-195 mg/mL secu; 1-10 mM or 40-60 mM Phos-Acet; 150-250 mM treh; 0.01-1.5 mg/mL PS80, PS20, or Px188; 0.01-10 mM antiox/chel; water; and pH 6.0 (+/- 0.4).

J883. 150-195 mg/mL secu; 1-10 mM or 40-60 mM Phos-Succ; 30-250 mM sug; 0.01-1.5 mg/mL surf; 0.01-10 mM Met, Cys, EDTA, and/or DTPA; water; and pH 6.0 (+/- 0.4).

J884. 150-195 mg/mL secu; 1-10 mM or 40-60 mM Phos-Acet; 30-250 mM sug; 0.01-1.5 mg/mL surf; 0.01-10 mM Met, Cys, EDTA, and/or DTPA; water; and pH 6.0 (+/- 0.4).

J885. 150-195 mg/mL secu; 1-10 mM or 40-60 mM Phos-Succ; 40-150 mM sorb; 0.01-1.5 mg/mL surf; 0.01-10 mM Met, Cys, EDTA, and/or DTPA; water; and pH 6.0 (+/- 0.4).

J886. 150-195 mg/mL secu; 1-10 mM or 40-60 mM Phos-Acet; 40-150 mM sorb; 0.01-1.5 mg/mL surf; 0.01-10 mM Met, Cys, EDTA, and/or DTPA; water; and pH 6.0 (+/- 0.4).

J887. 150-195 mg/mL secu; 1-10 mM or 40-60 mM Phos-Succ; 150-250 mM treh; 0.01-1.5 mg/mL surf; 0.01-10 mM Met, Cys, EDTA, and/or DTPA; water; and pH 6.0 (+/- 0.4).

J888. 150-195 mg/mL secu; 1-10 mM or 40-60 mM Phos-Acet; 150-250 mM treh; 0.01-1.5 mg/mL surf; 0.01-10 mM Met, Cys, EDTA, and/or DTPA; water; and pH 6.0 (+/- 0.4).

J889. 150-195 mg/mL secu; 1-10 mM or 40-60 mM Phos-Succ; 30-250 mM sug; 0.01-1.5 mg/mL PS80, PS20, or Px188; 0.01-10 mM Met, Cys, EDTA, and/or DTPA; water; and pH 6.0 (+/- 0.4).

J890. 150-195 mg/mL secu; 1-10 mM or 40-60 mM Phos-Acet; 30-250 mM sug; 0.01-1.5 mg/mL PS80, PS20, or Px188; 0.01-10 mM Met, Cys, EDTA, and/or DTPA; water; and pH 6.0 (+/- 0.4).

J891. 150-195 mg/mL secu; 1-10 mM or 40-60 mM Phos-Succ; 40-150 mM sorb; 0.01-1.5 mg/mL PS80, PS20, or Px188; 0.01-10 mM Met, Cys, EDTA, and/or DTPA; water; and pH 6.0 (+/- 0.4).

J892. 150-195 mg/mL secu; 1-10 mM or 40-60 mM Phos-Acet; 40-150 mM sorb; 0.01-1.5 mg/mL PS80, PS20, or Px188; 0.01-10 mM Met, Cys, EDTA, and/or DTPA; water; and pH 6.0 (+/- 0.4).

J893. 150-195 mg/mL secu; 1-10 mM or 40-60 mM Phos-Succ; 150-250 mM treh; 0.01-1.5 mg/mL PS80, PS20, or Px188; 0.01-10 mM Met, Cys, EDTA, and/or DTPA; water; and pH 6.0 (+/- 0.4).

J894. 150-195 mg/mL secu; 1-10 mM or 40-60 mM Phos-Acet; 150-250 mM treh; 0.01-1.5 mg/mL PS80, PS20, or Px188; 0.01-10 mM Met, Cys, EDTA, and/or DTPA; water; and pH 6.0 (+/- 0.4).

J895. 150-195 mg/mL secu; 1-10 mM or 40-60 mM Phos-Succ; 30-250 mM sug; 0.01-1.5 mg/mL surf; 10-110 mM salt tonicifier; water; and pH 6.0 (+/- 0.4).

J896. 150-195 mg/mL secu; 1-10 mM or 40-60 mM Phos-Acet; 30-250 mM sug; 0.01-1.5 mg/mL surf; 10-110 mM salt tonicifier; water; and pH 6.0 (+/- 0.4).

J897. 150-195 mg/mL secu; 1-10 mM or 40-60 mM Phos-Succ; 40-150 mM sorb; 0.01-1.5 mg/mL surf; 10-110 mM salt tonicifier; water; and pH 6.0 (+/- 0.4).

J898. 150-195 mg/mL secu; 1-10 mM or 40-60 mM Phos-Acet; 40-150 mM sorb; 0.01-1.5 mg/mL surf; 10-110 mM salt tonicifier; water; and pH 6.0 (+/- 0.4).

J899. 150-195 mg/mL secu; 1-10 mM or 40-60 mM Phos-Succ; 150-250 mM treh; 0.01-1.5 mg/mL surf; 10-110 mM salt tonicifier; water; and pH 6.0 (+/- 0.4).

(continued)

J900. 150-195 mg/mL secu; 1-10 mM or 40-60 mM Phos-Acet; 150-250 mM treh; 0.01-1.5 mg/mL surf; 10-110 mM salt tonicifier; water; and pH 6.0 (+/- 0.4).

J901. 150-195 mg/mL secu; 1-10 mM or 40-60 mM Phos-Succ; 30-250 mM sug; 0.01-1.5 mg/mL PS80, PS20, or Px188; 10-110 mM salt tonicifier; water; and pH 6.0 (+/- 0.4).

J902. 150-195 mg/mL secu; 1-10 mM or 40-60 mM Phos-Acet; 30-250 mM sug; 0.01-1.5 mg/mL PS80, PS20, or Px188; 10-110 mM salt tonicifier; water; and pH 6.0 (+/- 0.4).

J903. 150-195 mg/mL secu; 1-10 mM or 40-60 mM Phos-Succ; 40-150 mM sorb; 0.01-1.5 mg/mL PS80, PS20, or Px188; 10-110 mM salt tonicifier; water; and pH 6.0 (+/- 0.4).

J904. 150-195 mg/mL secu; 1-10 mM or 40-60 mM Phos-Acet; 40-150 mM sorb; 0.01-1.5 mg/mL PS80, PS20, or Px188; 10-110 mM salt tonicifier; water; and pH 6.0 (+/- 0.4).

J905. 150-195 mg/mL secu; 1-10 mM or 40-60 mM Phos-Succ; 150-250 mM treh; 0.01-1.5 mg/mL PS80, PS20, or Px188; 10-110 mM salt tonicifier; water; and pH 6.0 (+/- 0.4).

J906. 150-195 mg/mL secu; 1-10 mM or 40-60 mM Phos-Acet; 150-250 mM treh; 0.01-1.5 mg/mL PS80, PS20, or Px188; 10-110 mM salt tonicifier; water; and pH 6.0 (+/- 0.4).

J907. 150-195 mg/mL secu; 1-10 mM or 40-60 mM Phos-Succ; 30-250 mM sug; 0.01-1.5 mg/mL surf; 40-80 mM NaCl; water; and pH 6.0 (+/- 0.4).

J908. 150-195 mg/mL secu; 1-10 mM or 40-60 mM Phos-Acet; 30-250 mM sug; 0.01-1.5 mg/mL surf; 40-80 mM NaCl; water; and pH 6.0 (+/- 0.4).

J909. 150-195 mg/mL secu; 1-10 mM or 40-60 mM Phos-Succ; 40-150 mM sorb; 0.01-1.5 mg/mL surf; 40-80 mM NaCl; water; and pH 6.0 (+/- 0.4).

J910. 150-195 mg/mL secu; 1-10 mM or 40-60 mM Phos-Acet; 40-150 mM sorb; 0.01-1.5 mg/mL surf; 40-80 mM NaCl; water; and pH 6.0 (+/- 0.4).

J911. 150-195 mg/mL secu; 1-10 mM or 40-60 mM Phos-Succ; 150-250 mM treh; 0.01-1.5 mg/mL surf; 40-80 mM NaCl; water; and pH 6.0 (+/- 0.4).

J912. 150-195 mg/mL secu; 1-10 mM or 40-60 mM Phos-Acet; 150-250 mM treh; 0.01-1.5 mg/mL surf; 40-80 mM NaCl; water; and pH 6.0 (+/- 0.4).

J913. 150-195 mg/mL secu; 1-10 mM or 40-60 mM Phos-Succ; 30-250 mM sug; 0.01-1.5 mg/mL PS80, PS20, or Px188; 40-80 mM NaCl; water; and pH 6.0 (+/- 0.4).

J914. 150-195 mg/mL secu; 1-10 mM or 40-60 mM Phos-Acet; 30-250 mM sug; 0.01-1.5 mg/mL PS80, PS20, or Px188; 40-80 mM NaCl; water; and pH 6.0 (+/- 0.4).

J915. 150-195 mg/mL secu; 1-10 mM or 40-60 mM Phos-Succ; 40-150 mM sorb; 0.01-1.5 mg/mL PS80, PS20, or Px188; 40-80 mM NaCl; water; and pH 6.0 (+/- 0.4).

J916. 150-195 mg/mL secu; 1-10 mM or 40-60 mM Phos-Acet; 40-150 mM sorb; 0.01-1.5 mg/mL PS80, PS20, or Px188; 40-80 mM NaCl; water; and pH 6.0 (+/- 0.4).

J917. 150-195 mg/mL secu; 1-10 mM or 40-60 mM Phos-Succ; 150-250 mM treh; 0.01-1.5 mg/mL PS80, PS20, or Px188; 40-80 mM NaCl; water; and pH 6.0 (+/- 0.4).

J918. 150-195 mg/mL secu; 1-10 mM or 40-60 mM Phos-Acet; 150-250 mM treh; 0.01-1.5 mg/mL PS80, PS20, or Px188; 40-80 mM NaCl; water; and pH 6.0 (+/- 0.4).

J919. 150-195 mg/mL secu; 1-10 mM or 40-60 mM Phos-Succ; 30-250 mM sug; 10-200 mM AA; 0.01-1.5 mg/mL surf; 0.01-10 mM antiox/chel; water; and pH 6.0 (+/- 0.4).

J920. 150-195 mg/mL secu; 1-10 mM or 40-60 mM Phos-Acet; 30-250 mM sug; 10-200 mM AA; 0.01-1.5 mg/mL surf; 0.01-10 mM antiox/chel; water; and pH 6.0 (+/- 0.4).

J921. 150-195 mg/mL secu; 1-10 mM or 40-60 mM Phos-Succ; 40-150 mM sorb; 10-200 mM AA; 0.01-1.5 mg/mL surf; 0.01-10 mM antiox/chel; water; and pH 6.0 (+/- 0.4).

J922. 150-195 mg/mL secu; 1-10 mM or 40-60 mM Phos-Acet; 40-150 mM sorb; 10-200 mM AA; 0.01-1.5 mg/mL surf; 0.01-10 mM antiox/chel; water; and pH 6.0 (+/- 0.4).

J923. 150-195 mg/mL secu; 1-10 mM or 40-60 mM Phos-Succ; 150-250 mM treh; 10-200 mM AA; 0.01-1.5 mg/mL surf; 0.01-10 mM antiox/chel; water; and pH 6.0 (+/- 0.4).

J924. 150-195 mg/mL secu; 1-10 mM or 40-60 mM Phos-Acet; 150-250 mM treh; 10-200 mM AA; 0.01-1.5 mg/mL surf; 0.01-10 mM antiox/chel; water; and pH 6.0 (+/- 0.4).

J925. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

(continued)

J926. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM antiox/chel; water; and pH 6.0 (+/- 0.4).

J927. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J928. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM antiox/chel; water; and pH 6.0 (+/- 0.4).

J929. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM antioxlchel; water; and pH 6.0 (+/- 0.4).

J930. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM antioxlchel; water; and pH 6.0 (+/- 0.4).

J931. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM antiox/chel; water; and pH 6.0 (+/- 0.4).

J932. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM antiox/chel; water; and pH 6.0 (+/- 0.4).

J933. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM antiox/chel; water; and pH 6.0 (+/- 0.4).

J934. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM antiox/chel; water; and pH 6.0 (+/- 0.4).

J935. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM antiox/chel; water; and pH 6.0 (+/- 0.4).

J936. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM antiox/chel; water; and pH 6.0 (+/- 0.4).

J937. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J938. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J939. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J940. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J941. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM antiox/chel; water; and pH 6.0 (+/- 0.4).

J942. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM antioxlchel; water; and pH 6.0 (+/- 0.4).

J943. 150-195 mg/mL secu; 1-10 mM or 40-60 mM Phos-Succ; 30-250 mM sug; 10-200 mM AA; 0.01-1.5 mg/mL surf; 0.01-10 mM Met, Cys, EDTA, and/or DTPA; water; and pH 6.0 (+/- 0.4).

J944. 150-195 mg/mL secu; 1-10 mM or 40-60 mM Phos-Acet; 30-250 mM sug; 10-200 mM AA; 0.01-1.5 mg/mL surf; 0.01-10 mM Met, Cys, EDTA, and/or DTPA; water; and pH 6.0 (+/- 0.4).

J945. 150-195 mg/mL secu; 1-10 mM or 40-60 mM Phos-Succ; 40-150 mM sorb; 10-200 mM AA; 0.01-1.5 mg/mL surf; 0.01-10 mM Met, Cys, EDTA, and/or DTPA; water; and pH 6.0 (+/- 0.4).

J946. 150-195 mg/mL secu; 1-10 mM or 40-60 mM Phos-Acet; 40-150 mM sorb; 10-200 mM AA; 0.01-1.5 mg/mL surf; 0.01-10 mM Met, Cys, EDTA, and/or DTPA; water; and pH 6.0 (+/- 0.4).

J947. 150-195 mg/mL secu; 1-10 mM or 40-60 mM Phos-Succ; 150-250 mM treh; 10-200 mM AA; 0.01-1.5 mg/mL surf; 0.01-10 mM Met, Cys, EDTA, and/or DTPA; water; and pH 6.0 (+/- 0.4).

J948. 150-195 mg/mL secu; 1-10 mM or 40-60 mM Phos-Acet; 150-250 mM treh; 10-200 mM AA; 0.01-1.5 mg/mL surf; 0.01-10 mM Met, Cys, EDTA, and/or DTPA; water; and pH 6.0 (+/- 0.4).

J949. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J950. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J951. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

(continued)

J952. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J953. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J954. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM Met, Cys, EDTA, and/or DTPA; water; and pH 6.0 (+/- 0.4).

J955. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J956. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM Met, Cys, EDTA, and/or DTPA; water; and pH 6.0 (+/- 0.4).

J957. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J958. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM Met, Cys, EDTA, and/or DTPA; water; and pH 6.0 (+/- 0.4).

J959. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J960. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM Met, Cys, EDTA, and/or DTPA; water; and pH 6.0 (+/- 0.4).

J961. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J962. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J963. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J964. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J965. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J966. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; water; and pH 6.0 (+/- 0.4).

J967. 150-195 mg/mL secu; 1-10 mM or 40-60 mM Phos-Succ; 30-250 mM sug; 10-200 mM AA; 0.01-1.5 mg/mL surf; 10-110 mM salt tonicifier; water; and pH 6.0 (+/- 0.4).

J968. 150-195 mg/mL secu; 1-10 mM or 40-60 mM Phos-Acet; 30-250 mM sug; 10-200 mM AA; 0.01-1.5 mg/mL surf; 10-110 mM salt tonicifier; water; and pH 6.0 (+/- 0.4).

J969. 150-195 mg/mL secu; 1-10 mM or 40-60 mM Phos-Succ; 40-150 mM sorb; 10-200 mM AA; 0.01-1.5 mg/mL surf; 10-110 mM salt tonicifier; water; and pH 6.0 (+/- 0.4).

J970. 150-195 mg/mL secu; 1-10 mM or 40-60 mM Phos-Acet; 40-150 mM sorb; 10-200 mM AA; 0.01-1.5 mg/mL surf; 10-110 mM salt tonicifier; water; and pH 6.0 (+/- 0.4).

J971. 150-195 mg/mL secu; 1-10 mM or 40-60 mM Phos-Succ; 150-250 mM treh; 10-200 mM AA; 0.01-1.5 mg/mL surf; 10-110 mM salt tonicifier; water; and pH 6.0 (+/- 0.4).

J972. 150-195 mg/mL secu; 1-10 mM or 40-60 mM Phos-Acet; 150-250 mM treh; 10-200 mM AA; 0.01-1.5 mg/mL surf; 10-110 mM salt tonicifier; water; and pH 6.0 (+/- 0.4).

J973. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

J974. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 10-110 mM salt tonicifier; water; and pH 6.0 (+/- 0.4).

(continued)

J975. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J976. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 10-110 mM salt tonicifier; water; and pH 6.0 (+/- 0.4).

J977. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J978. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 10-110 mM salt tonicifier; water; and pH 6.0 (+/- 0.4).

J979. 150-195 mg/mL secu; 1-10 mM or 40-60 mM Phos-Succ; 30-250 mM sug; 10-200 mM AA; 0.01-1.5 mg/mL PS80, PS20, or Px188; 10-110 mM salt tonicifier; water; and pH 6.0 (+/- 0.4).

J980. 150-195 mg/mL secu; 1-10 mM or 40-60 mM Phos-Acet; 30-250 mM sug; 10-200 mM AA; 0.01-1.5 mg/mL PS80, PS20, or Px188; 10-110 mM salt tonicifier; water; and pH 6.0 (+/- 0.4).

J981. 150-195 mg/mL secu; 1-10 mM or 40-60 mM Phos-Succ; 40-150 mM sorb; 10-200 mM AA; 0.01-1.5 mg/mL PS80, PS20, or Px188; 10-110 mM salt tonicifier; water; and pH 6.0 (+/- 0.4).

J982. 150-195 mg/mL secu; 1-10 mM or 40-60 mM Phos-Acet; 40-150 mM sorb; 10-200 mM AA; 0.01-1.5 mg/mL PS80, PS20, or Px188; 10-110 mM salt tonicifier; water; and pH 6.0 (+/- 0.4).

J983. 150-195 mg/mL secu; 1-10 mM or 40-60 mM Phos-Succ; 150-250 mM treh; 10-200 mM AA; 0.01-1.5 mg/mL PS80, PS20, or Px188; 10-110 mM salt tonicifier; water; and pH 6.0 (+/- 0.4).

J984. 150-195 mg/mL secu; 1-10 mM or 40-60 mM Phos-Acet; 150-250 mM treh; 10-200 mM AA; 0.01-1.5 mg/mL PS80, PS20, or Px188; 10-110 mM salt tonicifier; water; and pH 6.0 (+/- 0.4).

J985. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J986. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J987. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J988. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J989. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J990. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 10-110 mM salt tonicifier; water; and pH 6.0 (+/- 0.4).

J991. 150-195 mg/mL secu; 1-10 mM or 40-60 mM Phos-Succ; 30-250 mM sug; 10-200 mM AA; 0.01-1.5 mg/mL surf; 40-80 mM NaCl; water; and pH 6.0 (+/- 0.4).

J992. 150-195 mg/mL secu; 1-10 mM or 40-60 mM Phos-Acet; 30-250 mM sug; 10-200 mM AA; 0.01-1.5 mg/mL surf; 40-80 mM NaCl; water; and pH 6.0 (+/- 0.4).

J993. 150-195 mg/mL secu; 1-10 mM or 40-60 mM Phos-Succ; 40-150 mM sorb; 10-200 mM AA; 0.01-1.5 mg/mL surf; 40-80 mM NaCl; water; and pH 6.0 (+/- 0.4).

J994. 150-195 mg/mL secu; 1-10 mM or 40-60 mM Phos-Acet; 40-150 mM sorb; 10-200 mM AA; 0.01-1.5 mg/mL surf; 40-80 mM NaCl; water; and pH 6.0 (+/- 0.4).

J995. 150-195 mg/mL secu; 1-10 mM or 40-60 mM Phos-Succ; 150-250 mM treh; 10-200 mM AA; 0.01-1.5 mg/mL surf; 40-80 mM NaCl; water; and pH 6.0 (+/- 0.4).

J996. 150-195 mg/mL secu; 1-10 mM or 40-60 mM Phos-Acet; 150-250 mM treh; 10-200 mM AA; 0.01-1.5 mg/mL surf; 40-80 mM NaCl; water; and pH 6.0 (+/- 0.4).

J997. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J998. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 40-80 mM NaCl; water; and pH 6.0 (+/- 0.4).

J999. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

J1000. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 40-80 mM NaCl; water; and pH 6.0 (+/- 0.4).

(continued)

J1001. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J1002. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 40-80 mM NaCl; water; and pH 6.0 (+/- 0.4).

J1003. 150-195 mg/mL secu; 1-10 mM or 40-60 mM Phos-Succ; 30-250 mM sug; 10-200 mM AA; 0.01-1.5 mg/mL PS80, PS20, or Px188; 40-80 mM NaCl; water; and pH 6.0 (+/- 0.4).

J1004. 150-195 mg/mL secu; 1-10 mM or 40-60 mM Phos-Acet; 30-250 mM sug; 10-200 mM AA; 0.01-1.5 mg/mL PS80, PS20, or Px188; 40-80 mM NaCl; water; and pH 6.0 (+/- 0.4).

J1005. 150-195 mg/mL secu; 1-10 mM or 40-60 mM Phos-Succ; 40-150 mM sorb; 10-200 mM AA; 0.01-1.5 mg/mL PS80, PS20, or Px188; 40-80 mM NaCl; water; and pH 6.0 (+/- 0.4).

J1006. 150-195 mg/mL secu; 1-10 mM or 40-60 mM Phos-Acet; 40-150 mM sorb; 10-200 mM AA; 0.01-1.5 mg/mL PS80, PS20, or Px188; 40-80 mM NaCl; water; and pH 6.0 (+/- 0.4).

J1007. 150-195 mg/mL secu; 1-10 mM or 40-60 mM Phos-Succ; 150-250 mM treh; 10-200 mM AA; 0.01-1.5 mg/mL PS80, PS20, or Px188; 40-80 mM NaCl; water; and pH 6.0 (+/- 0.4).

J1008. 150-195 mg/mL secu; 1-10 mM or 40-60 mM Phos-Acet; 150-250 mM treh; 10-200 mM AA; 0.01-1.5 mg/mL PS80, PS20, or Px188; 40-80 mM NaCl; water; and pH 6.0 (+/- 0.4).

J1009. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1010. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1011. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1012. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1013. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1014. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 40-80 mM NaCl; water; and pH 6.0 (+/- 0.4).

J1015. 150-195 mg/mL secu; 1-10 mM or 40-60 mM Phos-Succ; 30-250 mM sug; 0.01-1.5 mg/mL surf; 0.01-10 mM antiox/chel; 10-110 mM salt tonicifier; water; and pH 6.0 (+/- 0.4).

J1016. 150-195 mg/mL secu; 1-10 mM or 40-60 mM Phos-Acet; 30-250 mM sug; 0.01-1.5 mg/mL surf; 0.01-10 mM antiox/chel; 10-110 mM salt tonicifier; water; and pH 6.0 (+/- 0.4).

J1017. 150-195 mg/mL secu; 1-10 mM or 40-60 mM Phos-Succ; 40-150 mM sorb; 0.01-1.5 mg/mL surf; 0.01-10 mM antiox/chel; 10-110 mM salt tonicifier; water; and pH 6.0 (+/- 0.4).

J1018. 150-195 mg/mL secu; 1-10 mM or 40-60 mM Phos-Acet; 40-150 mM sorb; 0.01-1.5 mg/mL surf; 0.01-10 mM antiox/chel; 10-110 mM salt tonicifier; water; and pH 6.0 (+/- 0.4).

J1019. 150-195 mg/mL secu; 1-10 mM or 40-60 mM Phos-Succ; 150-250 mM treh; 0.01-1.5 mg/mL surf; 0.01-10 mM antiox/chel; 10-110 mM salt tonicifier; water; and pH 6.0 (+/- 0.4).

J1020. 150-195 mg/mL secu; 1-10 mM or 40-60 mM Phos-Acet; 150-250 mM treh; 0.01-1.5 mg/mL surf; 0.01-10 mM antiox/chel; 10-110 mM salt tonicifier; water; and pH 6.0 (+/- 0.4).

J102. 150-195 mg/mL secu; 1-10 mM or 40-60 mM Phos-Succ; 30-250 mM sug; 0.01-1.5 mg/mL PS80, PS20, or Px188; 0.01-10 mM antioxlchel; 10-110 mM salt tonicifier; water; and pH 6.0 (+/- 0.4).

J1022. 150-195 mg/mL secu; 1-10 mM or 40-60 mM Phos-Acet; 30-250 mM sug; 0.01-1.5 mg/mL PS80, PS20, or Px188; 0.01-10 mM antioxlchel; 10-110 mM salt tonicifier; water; and pH 6.0 (+/- 0.4).

J1023. 150-195 mg/mL secu; 1-10 mM or 40-60 mM Phos-Succ; 40-150 mM sorb; 0.01-1.5 mg/mL PS80, PS20, or Px188; 0.01-10 mM antiox/chel; 10-110 mM salt tonicifier; water; and pH 6.0 (+/- 0.4).

J1024. 150-195 mg/mL secu; 1-10 mM or 40-60 mM Phos-Acet; 40-150 mM sorb; 0.01-1.5 mg/mL PS80, PS20, or Px188; 0.01-10 mM antiox/chel; 10-110 mM salt tonicifier; water; and pH 6.0 (+/- 0.4).

J1025. 150-195 mg/mL secu; 1-10 mM or 40-60 mM Phos-Succ; 150-250 mM treh; 0.01-1.5 mg/mL PS80, PS20, or Px188; 0.01-10 mM antiox/chel; 10-110 mM salt tonicifier; water; and pH 6.0 (+/- 0.4).

J1026. 150-195 mg/mL secu; 1-10 mM or 40-60 mM Phos-Acet; 150-250 mM treh; 0.01-1.5 mg/mL PS80, PS20, or Px188; 0.01-10 mM antiox/chel; 10-110 mM salt tonicifier; water; and pH 6.0 (+/- 0.4).

(continued)

J1027. 150-195 mg/mL secu; 1-10 mM or 40-60 mM Phos-Succ; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J1028. 150-195 mg/mL secu; 1-10 mM or 40-60 mM Phos-Acet; 30-250 mM sug; 0.01-1.5 mg/mL surf; 0.01-10 mM Met, Cys, EDTA, and/or DTPA; 10-110 mM salt tonicifier; water; and pH 6.0 (+/- 0.4).

J1029. 150-195 mg/mL secu; 1-10 mM or 40-60 mM Phos-Succ; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J1030. 150-195 mg/mL secu; 1-10 mM or 40-60 mM Phos-Acet; 40-150 mM sorb; 0.01-1.5 mg/mL surf; 0.01-10 mM Met, Cys, EDTA, and/or DTPA; 10-110 mM salt tonicifier; water; and pH 6.0 (+/- 0.4).

J1031. 150-195 mg/mL secu; 1-10 mM or 40-60 mM Phos-Succ; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J1032. 150-195 mg/mL secu; 1-10 mM or 40-60 mM Phos-Acet; 150-250 mM treh; 0.01-1.5 mg/mL surf; 0.01-10 mM Met, Cys, EDTA, and/or DTPA; 10-110 mM salt tonicifier; water; and pH 6.0 (+/- 0.4).

J1033. 150-195 mg/mL secu; 1-10 mM or 40-60 mM Phos-Succ; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1034. 150-195 mg/mL secu; 1-10 mM or 40-60 mM Phos-Acet; 30-250 mM sug; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1035. 150-195 mg/mL secu; 1-10 mM or 40-60 mM Phos-Succ; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1036. 150-195 mg/mL secu; 1-10 mM or 40-60 mM Phos-Acet; 40-150 mM sorb; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1037. 150-195 mg/mL secu; 1-10 mM or 40-60 mM Phos-Succ; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1038. 150-195 mg/mL secu; 1-10 mM or 40-60 mM Phos-Acet; 150-250 mM treh; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1039. 150-195 mg/mL secu; 1-10 mM or 40-60 mM Phos-Succ; 30-250 mM sug; 0.01-1.5 mg/mL surf; 0.01-10 mM antiox/chel; 40-80 mM NaCl; water; and pH 6.0 (+/- 0.4).

J1040. 150-195 mg/mL secu; 1-10 mM or 40-60 mM Phos-Acet; 30-250 mM sug; 0.01-1.5 mg/mL surf; 0.01-10 mM antiox/chel; 40-80 mM NaCl; water; and pH 6.0 (+/- 0.4).

J1041. 150-195 mg/mL secu; 1-10 mM or 40-60 mM Phos-Succ; 40-150 mM sorb; 0.01-1.5 mg/mL surf; 0.01-10 mM antiox/chel; 40-80 mM NaCl; water; and pH 6.0 (+/- 0.4).

J1042. 150-195 mg/mL secu; 1-10 mM or 40-60 mM Phos-Acet; 40-150 mM sorb; 0.01-1.5 mg/mL surf; 0.01-10 mM antiox/chel; 40-80 mM NaCl; water; and pH 6.0 (+/- 0.4).

J1043. 150-195 mg/mL secu; 1-10 mM or 40-60 mM Phos-Succ; 150-250 mM treh; 0.01-1.5 mg/mL surf; 0.01-10 mM antiox/chel; 40-80 mM NaCl; water; and pH 6.0 (+/- 0.4).

J1044. 150-195 mg/mL secu; 1-10 mM or 40-60 mM Phos-Acet; 150-250 mM treh; 0.01-1.5 mg/mL surf; 0.01-10 mM antiox/chel; 40-80 mM NaCl; water; and pH 6.0 (+/- 0.4).

J1045. 150-195 mg/mL secu; 1-10 mM or 40-60 mM Phos-Succ; 30-250 mM sug; 0.01-1.5 mg/mL PS80, PS20, or Px188; 0.01-10 mM antioxlchel; 40-80 mM NaCl; water; and pH 6.0 (+/- 0.4).

J1046. 150-195 mg/mL secu; 1-10 mM or 40-60 mM Phos-Acet; 30-250 mM sug; 0.01-1.5 mg/mL PS80, PS20, or Px188; 0.01-10 mM antioxlchel; 40-80 mM NaCl; water; and pH 6.0 (+/- 0.4).

J1047. 150-195 mg/mL secu; 1-10 mM or 40-60 mM Phos-Succ; 40-150 mM sorb; 0.01-1.5 mg/mL PS80, PS20, or Px188; 0.01-10 mM antiox/chel; 40-80 mM NaCl; water; and pH 6.0 (+/- 0.4).

J1048. 150-195 mg/mL secu; 1-10 mM or 40-60 mM Phos-Acet; 40-150 mM sorb; 0.01-1.5 mg/mL PS80, PS20, or Px188; 0.01-10 mM antiox/chel; 40-80 mM NaCl; water; and pH 6.0 (+/- 0.4).

J1049. 150-195 mg/mL secu; 1-10 mM or 40-60 mM Phos-Succ; 150-250 mM treh; 0.01-1.5 mg/mL PS80, PS20, or Px188; 0.01-10 mM antiox/chel; 40-80 mM NaCl; water; and pH 6.0 (+/- 0.4).

J1050. 150-195 mg/mL secu; 1-10 mM or 40-60 mM Phos-Acet; 150-250 mM treh; 0.01-1.5 mg/mL PS80, PS20, or Px188; 0.01-10 mM antiox/chel; 40-80 mM NaCl; water; and pH 6.0 (+/- 0.4).

J1051. 150-195 mg/mL secu; 1-10 mM or 40-60 mM Phos-Succ; 30-250 mM sug; 0.01-1.5 mg/mL surf; 0.01-10 mM Met, Cys, EDTA, and/or DTPA; 40-80 mM NaCl; water; and pH 6.0 (+/- 0.4).

J1052. 150-195 mg/mL secu; 1-10 mM or 40-60 mM Phos-Acet; 30-250 mM sug; 0.01-1.5 mg/mL surf; 0.01-10 mM Met, Cys, EDTA, and/or DTPA; 40-80 mM NaCl; water; and pH 6.0 (+/- 0.4).

(continued)

J1053. 150-195 mg/mL secu; 1-10 mM or 40-60 mM Phos-Succ; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J1054. 150-195 mg/mL secu; 1-10 mM or 40-60 mM Phos-Acet; 40-150 mM sorb; 0.01-1.5 mg/mL surf; 0.01-10 mM Met, Cys, EDTA, and/or DTPA; 40-80 mM NaCl; water; and pH 6.0 (+/- 0.4).

J1055. 150-195 mg/mL secu; 1-10 mM or 40-60 mM Phos-Succ; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J1056. 150-195 mg/mL secu; 1-10 mM or 40-60 mM Phos-Acet; 150-250 mM treh; 0.01-1.5 mg/mL surf; 0.01-10 mM Met, Cys, EDTA, and/or DTPA; 40-80 mM NaCl; water; and pH 6.0 (+/- 0.4).

J1057. 150-195 mg/mL secu; 1-10 mM or 40-60 mM Phos-Succ; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1058. 150-195 mg/mL secu; 1-10 mM or 40-60 mM Phos-Acet; 30-250 mM sug; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1059. 150-195 mg/mL secu; 1-10 mM or 40-60 mM Phos-Succ; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1060. 150-195 mg/mL secu; 1-10 mM or 40-60 mM Phos-Acet; 40-150 mM sorb; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1061. 150-195 mg/mL secu; 1-10 mM or 40-60 mM Phos-Succ; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1062. 150-195 mg/mL secu; 1-10 mM or 40-60 mM Phos-Acet; 150-250 mM treh; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1063. 150-195 mg/mL secu; 1-10 mM or 40-60 mM Phos-Succ; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J1064. 150-195 mg/mL secu; 1-10 mM or 40-60 mM Phos-Acet; 30-250 mM sug; 10-200 mM AA; 0.01-1.5 mg/mL surf; 0.01-10 mM antiox/chel; 10-110 mM salt tonicifier; water; and pH 6.0 (+/- 0.4).

J1065. 150-195 mg/mL secu; 1-10 mM or 40-60 mM Phos-Succ; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J1066. 150-195 mg/mL secu; 1-10 mM or 40-60 mM Phos-Acet; 40-150 mM sorb; 10-200 mM AA; 0.01-1.5 mg/mL surf; 0.01-10 mM antiox/chel; 10-110 mM salt tonicifier; water; and pH 6.0 (+/- 0.4).

J1067. 150-195 mg/mL secu; 1-10 mM or 40-60 mM Phos-Succ; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J1068. 150-195 mg/mL secu; 1-10 mM or 40-60 mM Phos-Acet; 150-250 mM treh; 10-200 mM AA; 0.01-1.5 mg/mL surf; 0.01-10 mM antiox/chel; 10-110 mM salt tonicifier; water; and pH 6.0 (+/- 0.4).

J1069. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1070. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1071. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1072. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1073. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1074. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM antiox/chel; 10-110 mM salt tonicifier; water; and pH 6.0 (+/- 0.4).

J1075. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM antiox/chel; 10-110 mM salt tonicifier; water; and pH 6.0 (+/- 0.4).

(continued)

J1076. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J1077. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J1078. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J1079. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J1080. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM antiox/chel; 10-110 mM salt tonicifier; water; and pH 6.0 (+/- 0.4).

J1081. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J1082. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J1083. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J1084. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J1085. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM antiox/chel; 10-110 mM salt tonicifier; water; and pH 6.0 (+/- 0.4).

J1086. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM antioxlchel; 10-110 mM salt tonicifier; water; and pH 6.0 (+/- 0.4).

J1087. 150-195 mg/mL secu; 1-10 mM or 40-60 mM Phos-Succ; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1088. 150-195 mg/mL secu; 1-10 mM or 40-60 mM Phos-Acet; 30-250 mM sug; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1089. 150-195 mg/mL secu; 1-10 mM or 40-60 mM Phos-Succ; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1090. 150-195 mg/mL secu; 1-10 mM or 40-60 mM Phos-Acet; 40-150 mM sorb; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1091. 150-195 mg/mL secu; 1-10 mM or 40-60 mM Phos-Succ; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1092. 150-195 mg/mL secu; 1-10 mM or 40-60 mM Phos-Acet; 150-250 mM treh; 10-200 mM AA; 0.01-1.5 mg/mL surf; 0.01-10 mM Met, Cys, EDTA, and/or DTPA; 10-110 mM salt tonicifier; water; and pH 6.0 (+/- 0.4).

J1093. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J1094. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J1095. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J1096. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

(continued)

J1097. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J1098. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM Met, Cys, EDTA, and/or DTPA; 10-110 mM salt tonicifier; water; and pH 6.0 (+/- 0.4).

J1099. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1100. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1101. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1102. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1103. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1104. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1105. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1106. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1107. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1108. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1109. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1110. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 10-110 mM salt tonicifier; water; and pH 6.0 (+/- 0.4).

J1111. 150-195 mg/mL secu; 1-10 mM or 40-60 mM Phos-Succ; 30-250 mM sug; 10-200 mM AA; 0.01-1.5 mg/mL surf; 0.01-10 mM antiox/chel; 40-80 mM NaCl; water; and pH 6.0 (+/- 0.4).

J1112. 150-195 mg/mL secu; 1-10 mM or 40-60 mM Phos-Acet; 30-250 mM sug; 10-200 mM AA; 0.01-1.5 mg/mL surf; 0.01-10 mM antiox/chel; 40-80 mM NaCl; water; and pH 6.0 (+/- 0.4).

J1113. 150-195 mg/mL secu; 1-10 mM or 40-60 mM Phos-Succ; 40-150 mM sorb; 10-200 mM AA; 0.01-1.5 mg/mL surf; 0.01-10 mM antiox/chel; 40-80 mM NaCl; water; and pH 6.0 (+/- 0.4).

J1114. 150-195 mg/mL secu; 1-10 mM or 40-60 mM Phos-Acet; 40-150 mM sorb; 10-200 mM AA; 0.01-1.5 mg/mL surf; 0.01-10 mM antiox/chel; 40-80 mM NaCl; water; and pH 6.0 (+/- 0.4).

J1115. 150-195 mg/mL secu; 1-10 mM or 40-60 mM Phos-Succ; 150-250 mM treh; 10-200 mM AA; 0.01-1.5 mg/mL surf; 0.01-10 mM antiox/chel; 40-80 mM NaCl; water; and pH 6.0 (+/- 0.4).

J1116. 150-195 mg/mL secu; 1-10 mM or 40-60 mM Phos-Acet; 150-250 mM treh; 10-200 mM AA; 0.01-1.5 mg/mL surf; 0.01-10 mM antiox/chel; 40-80 mM NaCl; water; and pH 6.0 (+/- 0.4).

(continued)

J1117. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1118. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1119. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1120. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1121. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1122. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM antiox/chel; 40-80 mM NaCl; water; and pH 6.0 (+/- 0.4).

J1123. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J1124. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM antiox/chel; 40-80 mM NaCl; water; and pH 6.0 (+/- 0.4).

J1125. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J1126. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM antiox/chel; 40-80 mM NaCl; water; and pH 6.0 (+/- 0.4).

J1127. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J1128. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM antiox/chel; 40-80 mM NaCl; water; and pH 6.0 (+/- 0.4).

J1129. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J1130. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J1131. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM antioxlchel; 40-80 mM NaCl; water; and pH 6.0 (+/- 0.4).

J1132. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM antioxlchel; 40-80 mM NaCl; water; and pH 6.0 (+/- 0.4).

J1133. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM antioxlchel; 40-80 mM NaCl; water; and pH 6.0 (+/- 0.4).

J1134. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM antiox/chel; 40-80 mM NaCl; water; and pH 6.0 (+/- 0.4).

J1135. 150-195 mg/mL secu; 1-10 mM or 40-60 mM Phos-Succ; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J1136. 150-195 mg/mL secu; 1-10 mM or 40-60 mM Phos-Acet; 30-250 mM sug; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J1137. 150-195 mg/mL secu; 1-10 mM or 40-60 mM Phos-Succ; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J1138. 150-195 mg/mL secu; 1-10 mM or 40-60 mM Phos-Acet; 40-150 mM sorb; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J1139. 150-195 mg/mL secu; 1-10 mM or 40-60 mM Phos-Succ; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

(continued)

J1140. 150-195 mg/mL secu; 1-10 mM or 40-60 mM Phos-Acet; 150-250 mM treh; 10-200 mM AA; 0.01-1.5 mg/mL surf; 0.01-10 mM Met, Cys, EDTA, and/or DTPA; 40-80 mM NaCl; water; and pH 6.0 (+/- 0.4).

J1141. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1142. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1143. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1144. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1145. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1146. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL surf; 0.01-10 mM Met, Cys, EDTA, and/or DTPA; 40-80 mM NaCl; water; and pH 6.0 (+/- 0.4).

J1147. 150-195 mg/mL secu; 1-10 mM or 40-60 mM Phos-Succ; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1148. 150-195 mg/mL secu; 1-10 mM or 40-60 mM Phos-Acet; 30-250 mM sug; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1149. 150-195 mg/mL secu; 1-10 mM or 40-60 mM Phos-Succ; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1150. 150-195 mg/mL secu; 1-10 mM or 40-60 mM Phos-Acet; 40-150 mM sorb; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1151. 150-195 mg/mL secu; 1-10 mM or 40-60 mM Phos-Succ; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1152. 150-195 mg/mL secu; 1-10 mM or 40-60 mM Phos-Acet; 150-250 mM treh; 10-200 mM AA; 0.01-1.5 mg/mL PS80, PS20, or Px188; 0.01-10 mM Met, Cys, EDTA, and/or DTPA; 40-80 mM NaCl; water; and pH 6.0 (+/- 0.4).

J1153. 150-195 mg/mL secu; 1-10 mM or 40-60 mM Phos-Succ; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J1154. 150-195 mg/mL secu; 1-10 mM or 40-60 mM Phos-Acet; 30-250 mM sug; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J1155. 150-195 mg/mL secu; 1-10 mM or40-60 mM Phos-Succ; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J1156. 150-195 mg/mL secu; 1-10 mM or 40-60 mM Phos-Acet; 40-150 mM sorb; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/-0.4).

J1157. 150-195 mg/mL secu; 1-10 mM or 40-60 mM Phos-Succ; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

(continued)

J1158. 150-195 mg/mL secu; 1-10 mM or 40-60 mM Phos-Acet; 150-250 mM treh; 10-200 mM Arg, Gly, Lys, Pro, and/or Phe; 0.01-1.5 mg/mL PS80, PS20, or Px188; 0.01-10 mM Met, Cys, EDTA, and/or DTPA; 40-80 mM NaCl; water; and pH 6.0 (+/- 0.4).

## METHODS OF STABILISING BIOPHARMACEUTICAL ACTIVE

In view of the aforementioned points in this sub-section, and the data presented in the examples, the present invention also provides a method of stabilising biopharmaceutical compositions (chemically and/or physically optionally in relation to any one or more of the aforementioned parameters/properties), comprising mixing the relevant biopharmaceutical active with any relevant components required to form a biopharmaceutical composition as defined herein. Different embodiments will suitably require different combinations of components to be mixed, potentially in different amounts, and the skilled person can readily deduce such combinations and amounts by reference to the foregoing disclosure relating to the biopharmaceutical composition. Such different combinations of components may stabilise biopharmaceutical compositions in different respects. For instance, mixing the relevant biopharmaceutical active with the aforementioned components to form a biopharmaceutical composition as defined herein may stabilise the relevant biopharmaceutical active by:

i) Increasing the protein unfolding temperature of a biopharmaceutical;

ii) Inhibiting the formation of aggregates;

iii) Inhibiting the formation of fragments;

iv) Inhibiting the formation of sub-visible particles (either ≤25 microns or ≤10 microns);

v) Inhibiting turbidification;

vi) Inhibiting pH changes;

vii) Inhibiting photo-oxidation; and/or

viii) Reducing instability upon freeze/thaw cycles.

As such, the present invention provides a method of achieving one, some, or all of the following benefits:

i) Increased protein unfolding temperatures for a biopharmaceutical;

ii) Inhibition of formation of aggregates;

iii) Inhibition of formation of fragments;

iv) Inhibition of formation of sub-visible particles (either ≤25 microns or ≤10 microns);

v) Inhibition of turbidification;

vi) Inhibition of pH changes;

vii) Inhibition of photo-oxidation;

viii) Reduced instability upon freeze/thaw cycles; and/or

ix) Stabilisation of the isoform profile (especially with respect to the "main peak" as defined herein);

the method comprising manufacturing a biopharmaceutical composition as defined herein.

Suitably, the biopharmaceutical compositions of the invention have a shelf life of at least 6 months, suitably at least 12 months, suitably at least 18 months, more suitably at least 24 months. Suitably, the biopharmaceutical compositions of the invention have a shelf life of at least 6 months, suitably at least 12 months, suitably at least 18 months, more suitably at least 24 months, at a temperature of 2-8°C.

The method of stabilising described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said method.

*Enabling the skilled person to optimise key stability properties*

The novel combination of components disclosed for use in compositions of the invention enables the skilled person to produce (and judiciously fine-tube) compositions which exhibit comparable or enhanced properties relative to compositions of the prior art. In particular, the present disclosure now provides the skilled person with all the necessary tools to optimise formulation stability, and in particular optimise one or more of: inhibition of aggregation, fragmentation, protein unfolding, precipitation, pH slippage, and oxidation (especially photo-oxidation). Furthermore, the skilled person is given guidance on how to achieve such optimisations (through judiciously varying the compositions) and how, in the process, to minimise any detrimental side-effects. The present disclosure enables the skilled person to work across the scope of the invention to produce a variety of specific compositions which exhibit comparable or improved properties relative to compositions of the prior art.

## METHOD OF MANUFACTURING A LIQUID PHARMACEUTICAL COMPOSITION

The present invention provides a method of manufacturing a biopharmaceutical composition, suitably as defined herein.

In an aspect of the invention, the method of manufacturing a biopharmaceutical composition (suitably as defined herein) comprises: mixing together (or otherwise combining) secukinumab with one or more pharmaceutically-acceptable excipients, carriers, and/or diluents (wherein suitably the pharmaceutically-acceptable excipients, carriers, and/or diluents are those defined anywhere herein in relation to the biopharmaceutical composition), optionally in any amount, concentration, or form stipulated herein; and optionally adjusting any one or more parameters stipulated herein in relation to a biopharmaceutical composition (e.g. adjusting pH or osmolality).

The method suitably comprises mixing together, in any particular order deemed appropriate, any relevant ingredients/components required to form a composition as defined herein - suitably such ingredients/components being defined by reference to the biopharmaceutical composition (since other than ion-exchanges, proton exchanges, ionic associations, and ionic dissociations, suitably no actual chemical reactions take place during or after mixing). The skilled person may refer to the Examples or techniques well known in the art for forming biopharmaceutical compositions (especially those for injection *via* syringe). Different embodiments will suitably require different combinations of components to be mixed, potentially in different amounts. The skilled person can readily deduce such combinations and amounts by reference to the foregoing disclosure relating to the liquid pharmaceutical composition.

Suitably the method involves mixing together the relevant ingredients / components suitably, in a diluent (e.g. water), suitably so that all of the ingredients / components are (substantially or entirely) dissolved in the diluent.

In the context of the method of manufacture, the ingredients / components of the biopharmaceutical composition may be listed in the method, for instance, in the context of mixing. Such a list of ingredients / components may be an exhaustive list (especially where a plurality of ingredients are mixed together, in which case a diluent is preferably present as well) - such embodiments correspond to embodiments of the biopharmaceutical composition which are said to consist of (or consist essentially of) said ingredients / components.

The method may involve first preparing a pre-mixture (or pre-solution) of some or all ingredients / components (optionally with some or all of the diluent) excluding the biopharmaceutical active, and the biopharmaceutical active may then itself (optionally with or pre-dissolved in some of the diluent) be mixed with the pre-mixture (or pre-solution) to afford the biopharmaceutical composition, or a composition to which final components are then added to furnish the final biopharmaceutical composition. Most suitably, the pre-mixture contains all components except for the biopharmaceutical active and optionally also some diluent (which may be used to pre-dissolve the biopharmaceutical active, suitably so that the biopharmaceutical active is added to a mixture which offers optimal stabilisation of the biopharmaceutical active. Suitably the aforementioned pre-mixture is prepared with the desired pH for the final formulation.

Suitably, the method involves forming a buffer system, suitably a buffer system. The buffer system is suitably formed in a pre-mixture prior to the addition of the biopharmaceutical active, though the buffer system may optionally be formed with the biopharmaceutical active present. The buffer system may be formed through simply mixing a buffering agent (supplied ready-made) with its acid/base conjugate (suitably in appropriate relative quantities to provide the desired pH - this can be determined by the skilled person either theoretically or experimentally). In the case of an acetate buffer system, this means mixing sodium acetate with acetic acid. Alternatively, the buffer system may be formed through adding a strong acid (e.g. HCl) to the buffering agent (e.g. sodium acetate) in order to form *in situ* the acid/base conjugate (e.g. acetic acid) (again suitably in appropriate relative quantities to provide the desired pH). Alternatively, the buffer system may be formed through adding a strong base (e.g. sodium hydroxide) to the acid/base conjugate (e.g. acetic acid) of the buffering agent (e.g. sodium acetate) in order to form *in situ* the buffering agent (again suitably in appropriate relative quantities to provide the desired pH). The pH of either the pre-mixture of final composition may be judiciously adjusted by adding the required quantity of strong base or strong acid, or even a quantity of buffering agent or acid/base conjugate.

In certain embodiments, the buffering agent and/or buffer system is pre-formed as a separate mixture, and the buffer system is transferred to a precursor of the composition (comprising some or all components save for the buffering agent and/or buffer system, suitably comprising the biopharmaceutical active and potentially only the biopharmaceutical active) *via* buffer exchange (e.g. using diafiltration until the relevant concentrations or osmolality is reached). Additional excipients may be added thereafter if necessary in order to produce the final composition. The pH may be adjusted once or before all the components are present.

Any, some, or all components may be pre-dissolved or pre-mixed with a diluent prior to mixing with other components.

The final composition may be filtered, suitably to remove particulate matter. Suitably filtration is through filters sized at or below 1 μm, suitably sized at 0.1-1 μm, most suitably sized at 0.22μm. Suitably, filtration is through either PES filters or PVDF filters, suitably with 0.22 μm PES filters.

The present invention also provides a biopharmaceutical composition obtainable by, obtained by, or directly obtained by the method of manufacture herein described.

The method of manufacturing described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said method.

## CONTAINER

The present invention provides a container, suitably as defined herein. Said container suitably comprises the biopharmaceutical composition (suitably liquid biopharmaceutical composition) as defined herein.

The container suitably comprises a headspace, wherein the oxygen content in the headspace is greater than 13%, most suitably greater than about 15%. Suitably the container is or is part of a drug-delivery device as defined herein.

The present invention provides a method of manufacturing a container (suitably the aforesaid container, or as otherwise defined herein), the method comprising incorporating a (suitably liquid) biopharmaceutical composition (as defined herein) within a container, suitably so as to leave the aforesaid headspace.

The present invention also provides a container comprising a liquid biopharmaceutical composition, as defined herein, and (substantially) no headspace. Said container may be manufactured by incorporating a (suitably liquid) biopharmaceutical composition (as defined herein) within a container so as to leave (substantially) no headspace.

The present invention provides a container obtainable, obtained by, or directly obtained by a method of manufacturing a container as defined herein.

Notwithstanding the above, the headspace may be characterised by an oxygen content less than 13%, suitably less than about 15%. The headspace may substantially comprise (or consist essentially of or consist of) an inert gas, such as nitrogen, suitably with an oxygen content less than 10%, suitably less than 5%, suitably less than 2%.

The container described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said container.

## PHARMACEUTICAL PRODUCT

The present invention provides a pharmaceutical product, suitably as defined herein.

Said pharmaceutical product may suitably comprise a container (suitably as defined herein), wherein said container comprises a headspace and a (liquid) biopharmaceutical composition as defined herein, wherein the oxygen content in the headspace is greater than 13%, most suitably greater than about 15%. Suitably the pharmaceutical product is or is part of a drug-delivery device as defined herein. Said pharmaceutical product may be manufactured by incorporating a (suitably liquid) biopharmaceutical composition (as defined herein) within a container so as to leave a headspace. Suitably said headspace is left with or otherwise conditioned to have an oxygen content greater than 13%, most suitably greater than about 15%.

Said pharmaceutical product may suitably comprise a container (suitably as defined herein), wherein said container comprises no headspace and a (liquid) biopharmaceutical composition as defined herein. Again, suitably said pharmaceutical product is or is part of a drug-delivery device as defined herein. This pharmaceutical product may suitably be manufactured by incorporating a (suitably liquid) biopharmaceutical composition (as defined herein) within a container so as to leave (substantially) no headspace.

The pharmaceutical product may comprise a container, wherein said container comprises a headspace and a liquid biopharmaceutical composition as defined herein; wherein the oxygen content in the headspace is greater than 13%, most suitably greater than about 15%; and wherein the liquid biopharmaceutical composition is free of (or characterised by an absence of) methionine. Again, suitably said pharmaceutical product is or is part of a drug-delivery device as defined herein. This pharmaceutical product may be manufactured by incorporating a (suitably liquid) biopharmaceutical composition (as defined herein) within a container so as to leave a headspace (suitably having an oxygen content greater than 13%, most suitably greater than about 15%), wherein the liquid biopharmaceutical composition is free of (or characterised by an absence of) methionine.

A pharmaceutical product may also by manufactured by incorporating a (suitably liquid) biopharmaceutical composition (as defined herein) within a container so as to leave (substantially) no headspace, wherein the liquid biopharmaceutical composition is free of (or characterised by an absence of) methionine.

According to a further aspect of the present invention there is provided a pharmaceutical product obtainable, obtained by, or directly obtained by a method of manufacturing a pharmaceutical product as defined herein (suitably as defined hereinbefore with respect to how a pharmaceutical product is manufactured).

The pharmaceutical product described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said pharmaceutical product.

## DRUG-DELIVERY DEVICE

The present invention provides a drug delivery device comprising a biopharmaceutical composition as defined herein. Suitably the drug delivery device comprises a chamber within which the biopharmaceutical composition resides. Suitably the drug delivery device is sterile.

The drug delivery device may be a vial, ampoule, syringe, injection pen (e.g. essentially incorporating a syringe), autoinjector, or intravenous bag. Most suitably the drug delivery device is a syringe, suitably an injection pen. Suitably the syringe is a glass syringe. Suitably the syringe comprises a needle, suitably a 27G ½" needle. The drug delivery device is most suitably a device for subcutaneously-administering the biopharmaceutical composition of the invention.

The present invention provides a method of manufacturing a drug delivery device, suitably as defined herein, the method comprising incorporating a biopharmaceutical composition as defined herein within a drug delivery device. Such manufacture typically involves charging the biopharmaceutical composition as defined herein to a syringe, suitably *via* a needle affixed thereto. The needle may thereafter be removed, replaced, or remain.

The present invention provides a drug delivery device obtainable by, obtained by, or directly obtained by a method of manufacture defined herein.

According to an aspect of the invention there is provided a drug delivery device (e.g. vial, ampoule, syringe, pre-filled syringe, injection pen (e.g. essentially incorporating a syringe), autoinjector, or intravenous bag) comprising a biopharmaceutical composition as defined herein. Most preferably, the drug delivery device is an injection pen (sometimes termed "injector pen") or a pre-filled syringe, most suitably fitted with a needle, suitably wherein the needle has a gauge between 21 and 35G (preferably 27G) and a length between 0.5 mm and 10 mM (preferably a ½-inch needle). Preferably, the drug delivery device comprises a headspace, wherein the oxygen content in the headspace is greater than 13%, most suitably greater than about 15%. Preferably the drug-delivery device comprises a container as defined herein. Preferably the drug-delivery device is or comprises a pharmaceutical product as defined herein. This drug-delivery device may be manufactured by incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave a headspace (suitable having an oxygen content greater than 13%, most suitably greater than about 15%). Alternatively, the drug-deliver device may be manufactured by incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave (substantially) no headspace.

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave a headspace (suitable having an oxygen content greater than 13%, most suitably greater than about 15%), wherein the liquid biopharmaceutical composition is free of (or characterised by an absence of) methionine.

According to a further aspect of the present invention there is provided a method of manufacturing a drug delivery device (suitably as defined herein), the method comprising incorporating a (suitably liquid) biopharmaceutical composition (as defined herein) within a container that is associated with the drug delivery device (e.g. vial, ampoule, syringe, pre-filled syringe, injection pen (e.g. essentially incorporating a syringe), autoinjector, or intravenous bag) so as to leave (substantially) no headspace, wherein the liquid biopharmaceutical composition is free of (or characterised by an absence of) methionine.

The drug delivery device described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said drug delivery device.

## PACKAGE

The present invention provides a package comprising a biopharmaceutical composition as defined herein. Suitably the package comprises one or more containers as defined herein. Suitably the package comprises one or more pharmaceutical products as defined herein. Suitably the package comprises one or more drug-delivery devices as defined herein. The package may be manufactured by incorporating a biopharmaceutical composition as defined herein within a package. Suitably this is achieved by incorporating said biopharmaceutical composition within one or more drug delivery devices, and thereafter incorporating the one or more drug delivery devices within the package. Suitably this is achieved by incorporating said biopharmaceutical composition within one or more containers, and thereafter incorporating the one or more containers within the package. Suitably this is achieved by incorporating said biopharmaceutical composition within one or more pharmaceutical products, and thereafter incorporating the one or more pharmaceutical products within the package.

The present invention provides a package obtainable by, obtained by, or directly obtained by a method of manufacture defined herein.

The package described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said package.

## KIT OF PARTS

The present invention provides a kit of parts comprising a drug delivery device (without the biopharmaceutical composition incorporated therein), a biopharmaceutical composition as defined herein (optionally contained in a container, suitably as defined herein, which container is suitably is, is associated with, or is a part of the drug delivery device), and optionally a set of instructions with directions regarding the administration (e.g. sub-cutaneous) of the biopharmaceutical composition. The user may then fill the drug delivery device with the biopharmaceutical composition (which may be provided in a vial or ampoule or such like) prior to administration.

The kit described in this section, and elsewhere herein, suitably pertains to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said kit.

## USES OF PHARMACEUTICAL LIQUID COMPOSITION AND METHODS OF TREATMENT

According to a further aspect of the present invention there is provided a method of treating a disease or medical disorder in a patient in need of such treatment, said method comprising administering to said patient a therapeutically effective amount of a biopharmaceutical composition as defined herein. Administering most preferably comprises subcutaneously-administering a therapeutically effective amount of the biopharmaceutical composition, suitably *via* subcutaneous injection.

According to a further aspect of the present invention there is provided a biopharmaceutical composition, as defined herein, for use in treating a disease or medical disorder in a patient in need of such treatment.

According to a further aspect of the present invention there is provided a use of a biopharmaceutical composition, as defined herein, in the manufacture of a medicament for the treatment of a disease or disorder.

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is an IL-17A-related autoimmune disease.

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is selected from the group consisting of psoriasis, ankylosing spondylitis, psoriatic arthritis, and rheumatoid arthritis).

According to a further aspect of the present invention, there is provided (suitably as defined herein) a method of treating a disease or medical disorder, a biopharmaceutical composition for use in treating a disease or medical disorder, or a use of a biopharmaceutical composition in the manufacture of a medicament for a treatment of a disease or disorder, wherein the disease or medical disorder is selected from the group consisting of psoriasis, ankylosing spondylitis, and psoriatic arthritis.

All of the aforesaid methods of treatment, composition(s) for use, and use of composition(s) in the manufacture of a medicament, are equally applicable to the relevant packages, containers, pharmaceutical products, drug-delivery devices, and kits incorporating said composition(s).

The uses and methods of treatment described in this section, and elsewhere herein, suitably pertain to a composition as defined anywhere herein, included in any of numbered paragraphs A1-A511, B1-B255, C1-C255, D1-D1152, E1-E1151, F1-F12, G1-G959, H1-H1423, I1-I2400, and J1-J1158. As such, components/ingredients and characteristics of said compositions are likewise applicable to said uses and methods of treatment.

## *EXAMPLES*

The present invention is now further described by reference to the following further non-limiting examples.

### Materials and Equipment

Secukinumab was obtained (as the injectable liquid formulation, Cosentyx®) from commercial sources. Secukinumab, and biosimilars thereof, are sometimes referred to herein as FKS730.

The various excipients used in formulation preparation are pharmaceutical grade.

### Analytical Techniques and Protocols

#### *Micro-Flow Imaging (MFI)*

Sub-visible particles were analyzed by Microflow imaging using a MFI-5200 particle analyzer system (ProteinSimple, Santa Clara, CA, USA).

MFI View System Software (MVSS) version 2-R2-6.1.20.1915 is used to perform the measurements and Lumetics LINK software version 2.0.21.181221 is used to analyze the samples. The concentrations of particles $\geq 2$ μm, $\geq 5$ μm, $\geq 10$ μm and $\geq 25$ μm as well as exemplary particle images of particles $\geq 5$ μm are reported (particle concentrations without correction for dilution factor).

#### *UV spectroscopy (soloVPE) for determination of concentration and turbidity*

UV spectroscopy is used, in a manner well known in the art, to determine concentration of turbidity of the various secukinumab formulations described herein. UV spectroscopy is carried out with a soloVPE system from C Technologies (Bridgewater) coupled to an Agilent Cary 60 UV/VIS spectrometer (Agilent).

#### *Osmolality measurements*

Osmolality of the samples is measured by method of freezing-point depression using a Gonotec Osmomat 3000 (Gonotec). Samples are measured undiluted.

#### *nanoDSF*

NanoDSF measurements utilizing the intrinsic fluorescence of protein are performed with a Prometheus Panta system, in a fashion well known in the art.

### *Viscosity*

The dynamic viscosity is measured by using a Kinexus ultra plus rheometer (Malvern Instruments).

Viscosity measurements are performed for 3 minutes at a fixed temperature of 25 °C by applying a shear rate of 400 $s^{-1,}$ where the fluid exhibits newtonian behaviour.

### *Hydrophobic interaction chromatography (HIC)*

HIC is used to assess the cysteinylation status of FKS730. Prior to analysis, the sample are diluted with water to a final concentration of 1 mg/ml secukinumab. Samples are stored at 5 °C in the autosampler prior to analysis.

#### *Performance check*

Prior to sample analysis, the column is conditioned with 6 injections of FKS730 CYS CTL and performance of the ProPac™ HIC-10 column is tested with a reference sample (Cosentyx Lot SNH96). The standard is stored at -80 °C.

Figure 1 is an exemplary chromatogram of a water blank injection taken from Cysteinylation analysis of Secukinumab by HIC HPLC

Figure 2 is an exemplary chromatogram of a mobile phase A blank injection taken from Cysteinylation analysis of Secukinumab by HIC HPLC.

Figure 3 is an exemplary chromatogram of a FKS730 CYS CTL injection taken from Cysteinylation analysis of Secukinumab by HIC HPLC .

Figure 4 shows: a) an exemplary chromatographic profile of reference material (Cosentyx); and b) a zoomed in view of a) Cysteinylation analysis of Secukinumab by HIC HPLC.

#### *Data evaluation*

The following parameters (shown in Table 1) are used for HIC data evaluation:

Table 1: Data evaluation parameters that will be used for the HIC method.

| | |
|---|---|
| Integration | Valley to valley integration with manual verification |
| Integration area | According to the integration depicted in Figure 3, Figure 4a, and Figure 4b |
| Componnents | - FKS730_Uncysteinylated<br>- FKS730 + Oxidation<br>- FKS730 + Pyroglutamate |

### *Size exclusion ultra high performance chromatography (SE-UPLC)*

#### *Method description*

SE-UPLC is used *inter alia* to assess aggregation. Samples are diluted with water to a secukinumab concentration of 5 mg/ml. The samples are stored at 5 - 8 °C in the autosampler prior to analysis.

#### *SST/performance check*

Prior to sample analysis, the performance of the SE-UPLC column is tested with the Biorad gel filtration standard (dissolved in water).

The system/sample stability during the sequence is further monitored by injecting reference samples (Cosentyx Lot SNH96). Reference samples are stored at -80 °C.

Figure 5 shows a reference profile for Biorad gel filtration standard (Biorad GFS) taken from aggregate (HMW) analysis by SEC HPLC.

Figure 6 shows a reference profile for FKS730 (and also Cosentyx®) reference standard taken from aggregate analysis by SEC HPLC.

Figure 7 shows an enlarged view of reference profile for Cosentyx SNH96 reference standard taken from aggregate analysis by SEC HPLC.

*Data evaluation:*

The following parameters (as shown in Table 2) are used for SE-UPLC data evaluation:

Table 2: Data evaluation parameters that will be used for the SE-UPLC method.

| | |
|---|---|
| Integration | Valley to valley integration with manual verification as shown in Figure 7 |
| Integration area | According to the integration depicted in Figure 7 |
| Components | - Monomer (main peak)<br>- High-molecular-weight species 1 (HMW1) %<br>- HMW2 %<br>- HMW3 %<br>- HMW total %<br>- Fragments (peak eluting after monomer) |

***Imaged capillary isoelectric focusing (IcIEF)***

*Method description*

Imaged capillary isoelectric focusing (IcIEF) is performed on a Maurice C. instrument (ProteinSimple).

*Sample preparation*

**Carboxypeptidase B digestion:**

Samples requiring carboxypeptidase B (CpB) digestion are diluted to a protein concentration of 1 mg/ml with 1x PBS.

For CpB digestion, 100 μl of each sample at a protein concentration of 1 mg/ml is transferred to 1 5-ml microcentrifuge tubes and 1.7 μl of resuspended CpB is added to each tube. The samples are incubated for 15 min at 25 °C in a heating block.

Directly after the CpB digestion, the samples are desalted (within 30 min after end of the incubation time).

**Desalting OR sample dilution:**

All samples (CpB treated or not) are desalted by using 50-kDa Amicon filters (buffer exchange into water). Alternatively (where CpB digestion is not performed): All samples are diluted to 1 mg/ml with water. After desalting, sample protein concentrations are measured by UV spectroscopy using NanoDrop and, where needed, samples are diluted to exactly 1 mg/ml with water.

**Preparation of master mix:**

The master mix is prepared according to the table below (Table 3) for n+5 samples (n= number of samples). The master mix is mixed by vortexing, then allowed to sit on the bench for at least 5 min until all air bubbles have risen to the surface.

Table 3: Description of the master mix for the IcIEF analysis of secukinumab.

| Component | Percentage | Volume for 1 sample [µl] |
|---|---|---|
| Pharmalyte 8-10.5 | 2% | 4 |
| Servalyte 4-9 | 2% | 4 |
| Methyl cellulose 1% | 35% | 70 |
| pI marker 7.05 | 0.5% | 1 |
| pI marker 9.50 | 0.5% | 1 |
| Water | 50% | 100 |
| Total volume (without protein sample) | | 180 |

**Final sample preparation:**

20 µl of the diluted samples at a protein concentration of 1 mg/ml is mixed with 180 µl of the master mix, resulting in a total sample volume of 200 µl with a protein concentration of 0.1 mg/ml. The samples are vortexed, then spun in a benchtop centrifuge for 3 minutes at 10,000 rpm to sediment particles. Then, the top 160 µl of each sample is transferred into Maurice and placed sample vials, carefully avoiding the introduction of air bubbles. Vials are closed with clear screw caps into the Maurice autosampler.

*SST/performance check*

Prior to sample analysis, the IcIEF performance is tested with the SST mix by ProteinSimple.
A typical profile of the SST is shown in Figure 8.
Figure 8 shows a typical peak profile of the SST analyzed in fluorescence mode taken from charged variant analysis by icIEF.
The system/sample stability during the sequence will be further monitored by injecting reference samples (Cosentyx). A typical peak profile for the reference standard with/without CpB digestion is shown in Figure 9.
Figure 9 shows a typical peak profile of the reference standard a) with CpB digestion; and b) without CpB digestion taken from charged variant analysis by icIEF.

*Sample analysis*

The autosampler temperature is set to 10 °C. Samples are analyzed as n=1 with the method specified in Table 4.

Table 4 - Method parameters for the IcIEF analysis of secukinumab

| Parameter | Setting FKS730 method |
|---|---|
| Pre-focusing | 1 min at 1,500 V |
| Focusing | 9 min at 3,000 V |
| Detection | Absorbance (0.005 s)<br>Fluorescence (3 s, 5 s, 10 s, 20 s) |
| pI markers | 7.05 and 650 pixels<br>9.50 and 1,800 pixels |
| Ampholytes | 2% servalyte 4-9, 2% pharmalyte 8-10.5 |
| Additives | none |
| Sample load | 55 s |

*Data evaluation*

Data evaluation is performed using the data acquired in the fluorescence 20 seconds exposure time mode.

Correct detection of the pI markers is verified (and adjusted as necessary), then the data is exported and further processed in Chromeleon to extract the % area of the peaks. The following parameters (as shown in Table 5) are used for data integration:

Table 5: Data evaluation parameters that will be used for the IcIEF method

| | |
|---|---|
| Integration | Valley to valley integration with manual verification |
| Integration area | According to the integration depicted in Figure 9. |
| Components | - Main peak<br>- Main peak variant / Acidic 5<br>- Acidic 1<br>- Acidic 2<br>- Acidic 3<br>- Acidic 4 (no separate integration, shoulder of Acidic 3)<br>- Basic 1<br>- Basic 2 (for samples without CpB treatment) |

***Polysorbate characterization and quantification by using LC-CAD***

PS80 content is quantified, *via* RP-HPLC-CAD analysis, for the pooled starting materials, the material after the TFF-process, and in the final formulations at T0. Using the quantification method described in Kranz, W., Wuchner, K., Corradini, E., Berger, M., & Hawe, A. (2019). *Factors influencing polysorbate's sensitivity against enzymatic hydrolysis and oxidative degradation. Journal of Pharmaceutical Sciences. doi:10.1016/j.xphs.2019.01.006.*

**Preparations 1 - PS80-depleted pre-formulated secukinumab**

Secukinumab was obtained (as the injectable liquid formulation, Cosentyx®) from commercial sources, with the formulation as follows:

150 mg/mL secukinumab

20 mM histidine buffer system

5 mM L-methionine

0.2 mg/mL polysorbate 80

200 mM trehalose dihydrate

Water for injection

pH adjusted to pH 5.8

This formulation was processed to provide PS80-depleted stock solutions having the following formulation:

16 mg/ml secukinumab

acetate/Tris buffer

150 mM NaCl

pH ~7.15

The PS80-depleted stock solution (referred to herein as "Preparation 1" and sometimes as DS) was verified *via* standard analytical protocols, and had the following characteristics:

- pH 7.15
- Conductivity of 17.03 mS/cm
- Osmolality of 352 mOsmol/kg
- Secukinumab concentration of 15.88 mg/mL

**Preparations 2 - Formulation Preparation**

The following formulations of Table 6 are examples of formulations prepared for screening.

Table 6 - Formulations for formulation screen

| Formulation ID | API concentration | pH | Buffer | Tonicity adjustment | Antioxidant | Surfactant | Headspace $O_2$ content |
|---|---|---|---|---|---|---|---|
| **F1** | 150 mg/ml | 5.8 | 20 mM histidine | 200 mM trehalose | 5 mM methionine | 0.02% PS80 | target $\leq$12% |
| **F2** | 150 mg/ml | 5.8 | 20 mM histidine | 200 mM trehalose | 5 mM methionine | 0.02% PS80 | ambient air |
| **F3** | 190 mg/mL | 4.6 | 55 mM acetate | 125 mM sorbitol | | 0.02% PS80 | ambient air |
| **F4** | 190 mg/mL | 5.0 | 25 mM adipic acid | 125 mM sorbitol | | 0.02% PS80 | ambient air |
| **F5** | 190 mg/mL | 5.0 | 25 mM adipic acid | 120 mM arginine, 80 mM sorbitol | | 0.02% PS80 | ambient air |
| **F6** | 190 mg/mL | 5.0 | 55 mM succinate | 120 mM lysine, 80 mM sorbitol | | 0.02% PS80 | ambient air |
| **F7** | 190 mg/mL | 5.0 | 55 mM succinate | 125 mM sorbitol | 7 mM sodium thiosulfate | 0.02% PS80 | ambient air |
| **F8** | 190 mg/mL | 5.0 | 55 mM succinate | 125 mM sorbitol | 0.5 mM pentetic acid | 0.02% PS80 | ambient air |
| **F9** | 190 mg/mL | 6.3 | 55 mM succinate | 125 mM sorbitol | | 0.02% PS80 | ambient air |

Formulations, such as those of Table 8, are prepared in accordance with standard methodologies well known to those skilled in the art. This generally involves processes such as diafiltration, buffer-exchange, and concentration to provide the target formulations at the target pH (± 0.15).

**Example 1 - Assessing Upconcentration of Secukinumab formulations**

As explained in the sections above relating to preparation of formulations, it is apparent that secukinumab formulations can be up-concentrated. As such, further formulation studies were performed with up-concentrated formulations.

**Example 2 - Formulation Screen**

Nine formulations are prepared according to Table 7, filled into primary packaging material (2R vials) with a fill volume to achieve a target dose per vial of 150 mg secukinumab per vial (i.e. 1 ml at 150 mg/ml secukinumab or 0.79 ml at 190 mg/ml). These formulations were prepared as set forth above under "Preparations 2". Formulations are subjected to accelerated storage (i.e. stressing conditions) and thereafter analysed.

Table 7 - Secukinumab Formulations (F1-F9) of a formulation screen

| Formulation ID | API concentration | pH | Buffer | Tonicity adjustment | Antioxidant | Surfactant | Headspace $O_2$ content |
|---|---|---|---|---|---|---|---|
| **F1** | 150 mg/ml | 5.8 | 20 mM histidine | 200 mM trehalose | 5 mM methionine | 0.02% PS80 | target $\leq$12% |
| **F2** | 150 mg/ml | 5.8 | 20 mM histidine | 200 mM trehalose | 5 mM methionine | 0.02% PS80 | ambient air |
| **F3** | 190 mg/mL | 4.6 | 55 mM acetate | 125 mM sorbitol | | 0.02% PS80 | ambient air |
| **F4** | 190 mg/mL | 5.0 | 25 mM adipic acid | 125 mM sorbitol | | 0.02% PS80 | ambient air |
| **F5** | 190 mg/mL | 5.0 | 25 mM adipic acid | 120 mM arginine, 80 mM sorbitol | | 0.02% PS80 | ambient air |
| **F6** | 190 mg/mL | 5.0 | 55 mM succinate | 120 mM lysine, 80 mM sorbitol | | 0.02% PS80 | ambient air |
| **F7** | 190 mg/mL | 5.0 | 55 mM succinate | 125 mM sorbitol | 7 mM sodium thiosulfate | 0.02% PS80 | ambient air |
| **F8** | 190 mg/mL | 5.0 | 55 mM succinate | 125 mM sorbitol | 0.5 mM pentetic acid | 0.02% PS80 | ambient air |
| **F9** | 190 mg/mL | 6.3 | 55 mM succinate | 125 mM sorbitol | | 0.02% PS80 | ambient air |

***Initial Characteristics of Formulations F1-F9***

The formulation characteristics described in Table 7 were met.

Measured osmolalities of both the final formulations F1-F9 (at Time 0) and their corresponding formulation buffers (i.e. containing all excipients, except surfactant, but no secukinumab) are given in Table 8 below. Osmolalities of the final formulations range between 210 mOsmol/kg (for F4) to 486 mOsmol/kg (for F6), whereas osmolalities of corresponding formulation buffers (i.e. essentially the osmolality of the final formulation minus secukinumab's contribution) range from 178 mOsmol/kg (for F4) to 406 mOsmol/kg (for F6).

Table 8 – Osmolalities of Formulations F1-F9 and their corresponding Formulation Buffers FB1-FB9

| Formulation | Osmolality [mOsmol/kg] | |
|---|---|---|
| | T0 | Formulation buffer |
| F1 | 329 | 256 |
| F2 | 330 | 256 |
| F3 | 293 | 198 |
| F4 | 210 | 178 |
| F5 | 359 | 299 |
| F6 | 486 | 406 |
| F7 | 301 | 268 |
| F8 | 275 | 237 |
| F9 | 313 | 258 |

Measured viscosities of final formulations F1-F9 (at Time 0) are shown in Table 9 below.

Table 9 – Viscosities of Formulation F1-F9

| | Viscosity [mPa*s] |
|---|---|
| Formulation | T0 |
| F1 | 10.4 |
| F2 | 9.2 |
| F3 | 18.1 |
| F4 | 17.8 |
| F5 | 17.1 |
| F6 | 17.6 |
| F7 | 18.3 |
| F8 | 18.4 |
| F9 | 15.2 |

The viscosities of F1 and F2 were similar at T0. The viscosities of F3-8 were higher than reference formulation F1, but F9 has a viscosity somewhat lower than F3-F8.

Figure 10 shows nanoDSF results for F1-F9, namely: a) Mean $Tm_1$ (left bar) and Mean $Tm_2$ (right bar) for each formulation; and b) $Tm_{onset}$ (left bar) and $Tm_{agg}$ (right bar) for each formulation. The apparent $Tm_1$ and $Tm_1$ and $Tm_{onset}$ of F3-F8 are lower than in reference formulation (F1). $T_{agg}$ of F3-F8 is lowerthan in reference formulation (F1). Lower pH (4.6 and 5.0) appears to reduce thermal stability, at least with respect to these particular formulations.

Formulations were examined by visual inspection at appropriate time points - all remained substantially free of visible particles according to Ph. Eur. Visual inspection.

Table 10 shows the results of protein concentration analysis at various time points. There were no substantial changes in protein concentration after 1 month of storage.

Table 10 – Protein concentration results for F1-F9 at various time points

| | Protein concentration [mg/ml] | | |
|---|---|---|---|
| Formulation | T0 | 25 °C T1m | 40 °C T1m |
| F1 | 154.2 | 155.4 | 153.8 |
| F2 | 153.0 | 153.8 | 152.6 |
| F3 | 192.9 | 190.6 | 189.6 |
| F4 | 187.0 | 192.1 | 189.6 |
| F5 | 191.6 | 192.7 | 192.2 |
| F6 | 189.0 | 191.2 | 189.9 |
| F7 | 192.6 | 193.2 | 193.6 |
| F8 | 192.9 | 193.3 | 194.3 |
| F9 | 189.7 | 191.3 | 191.5 |

Table 11 shows the results of turbidity analysis for formulations F1-F9. $OD_{350}$ increased in all formulations upon storage. $OD_{350}$ was lowest in F1 at each storage temperature, with highest $OD_{350}$ values being observed for F5 and F6.

Table 11 – Sample turbidity results for F1-F9 at various time points (by $OD_{350}$ – soloVPE)

| | Sample turbidity ($OD_{350}$ at 10 mm) | | |
|---|---|---|---|
| Formulation | T0 | 25 °C T1m | 40 °C T1m |
| F1 | 0.50 | 0.52 | 0.56 |
| F2 | 0.51 | 0.54 | 0.71 |
| F3 | 0.52 | 0.60 | 0.96 |
| F4 | 0.54 | 0.59 | 0.90 |
| F5 | 0.56 | 0.66 | 1.14 |
| F6 | 0.58 | 0.71 | 1.16 |
| F7 | 0.56 | 0.57 | 0.60 |
| F8 | 0.55 | 0.57 | 0.64 |
| F9 | 0.59 | 0.61 | 0.72 |

Figure 11 shows the results of micro-flow imaging (MFI), by reference to particles per container: a) for particles ≥ 2 mm; b) for particles ≥ 5 mm; c) for particles ≥ 10 mm; and d) for particles ≥ 25 mm. Sub-visible particle levels (≥2 μm and ≥5 μm) were similar for F1-F8 and increased in F9 at 25 °C compared to T0. Sub-visible particle levels (≥2 μm and ≥5 μm) were similar for F1-F3 and increased in F4-F9 at 40 °C compared to T0. Sub-visible particle levels (≥10 μm and ≥25 μm) were similar for F1-F9 at 25° C, comparable to T0. Sub-visible particle levels (≥10 μm and ≥25 μm) were increased in F5 at 40° C compared to T0.

Size exclusion chromatography was also performed on samples F1-F9..

Figure 12 shows SEC results as bar charts for each formulation F1-F9 (bars left to right are T0, T1m_25°C, and T1m_40°C respectively) for: a) high molecular weights (HMW); b) low molecular weights (LMW); and c) monomer.

Figure 13 shows hydrophobic interaction chromatography (HIC) traces for: a) F1 at T1m_40°C, F2 at T1m_40°C, and Cosentyx; and b) F1 at T1m_40°C, F6 at T1m_40°C, and Cosentyx.

Figure 14 shows hydrophobic interaction chromatography (HIC) results in the form of bar charts (left-to-right bars relate to T0, T1m_25C, and T1m_40C respectively with T0 results missing for "unknown" components) in relation to: a) uncysteinylated form; b) "unknown" components; c) oxidized form; d) pyroglutamate form.

Table 12 shows a summary of the data for formulations F1-F9 as measured at T0 (i.e. before any stress testing or storage).

Table 12 – Summary of data for formulations F1-F9 for measurements taken at T0

| T0 | | Formulation ID | | | | | | | | |
|---|---|---|---|---|---|---|---|---|---|---|
| | | 1 | 2 | 3 | 4 | 5 | 6 | 7 | 8 | 9 |
| Attributes | pH target | 5.80 | 5.80 | 4.60 | 5.00 | 5.00 | 5.00 | 5.00 | 5.00 | 6.30 |
| | pH measured | 5.84 | 5.86 | 4.68 | 5.06 | 4.98 | 5.00 | 5.06 | 5.06 | 6.35 |
| | fill volume [ml] | 1.00 | 1.00 | 0.80 | 0.80 | 0.80 | 0.80 | 0.80 | 0.80 | 0.80 |
| | protein conc. [mg/ml] | 154.2 | 153.0 | 192.9 | 187.0 | 191.6 | 189.0 | 192.6 | 192.9 | 189.7 |
| Stability indicating methods | Turbidity T0 | 0.50 | 0.51 | 0.52 | 0.54 | 0.56 | 0.58 | 0.56 | 0.55 | 0.59 |
| | MFI ≤ 2 µm T0 | 2731 | 1446 | 6447 | 3191 | 901 | 608 | 3082 | 1172 | 2279 |
| | MFI ≤ 5 µm T0 | 857 | 257 | 2431 | 912 | 293 | 163 | 478 | 239 | 260 |
| | MFI ≤ 10 µm T0 | 343 | 64 | 836 | 402 | 119 | 54 | 130 | 76 | 65 |
| | MFI ≤ 25 µm T0 | 75 | 11 | 174 | 54 | 11 | 0 | 11 | 33 | 0 |
| | acidic-iclEF [%] T0 | 19.61 | 19.37 | 20.08 | 19.29 | 19.27 | 19.88 | 21.02 | 20.06 | 19.47 |
| | main-iclEF [%] T0 | 57.53 | 58.24 | 56.91 | 57.98 | 58.02 | 57.15 | 56.38 | 57.38 | 57.85 |
| | basic-iclEF [%] T0 | 22.87 | 22.40 | 23.01 | 22.71 | 22.72 | 22.95 | 22.60 | 22.57 | 22.67 |
| | HMW-SEC [%] T0 | 1.24 | 1.25 | 1.20 | 1.22 | 1.20 | 1.20 | 0.93 | 1.20 | 1.47 |
| | Main-SEC [%] T0 | 98.58 | 98.57 | 98.61 | 98.58 | 98.59 | 98.62 | 98.87 | 98.46 | 98.37 |
| | LMW-SEC [%] T0 | 0.18 | 0.18 | 0.19 | 0.20 | 0.21 | 0.18 | 0.20 | 0.34 | 0.17 |
| | components-HIC [%] T0 | 0.00 | 0.00 | 0.00 | 0.00 | 0.00 | 0.00 | 0.00 | 0.00 | 0.00 |
| | Uncys-HIC [%] T0 | 89.39 | 89.20 | 88.49 | 88.14 | 89.55 | 89.04 | 89.26 | 88.96 | 88.18 |
| | Oxidation-HIC [%] T0 | 7.40 | 7.45 | 7.75 | 7.75 | 7.09 | 7.49 | 7.04 | 7.44 | 7.69 |
| | Pyro-E-HIC [%] T0 | 3.21 | 3.35 | 3.76 | 4.11 | 3.36 | 3.47 | 3.70 | 3.60 | 4.13 |
| | $Tm_1$ [°C] | 68.2 | 68.3 | 60.4 | 63.5 | 62.0 | 62.1 | 62.5 | 62.9 | 69.1 |
| | $Tm_2$ [°C] | 80.0 | 80.0 | 77.1 | 77.6 | 77.6 | 77.9 | 78.5 | 78.4 | 80.2 |
| | $Tm_{onset}$ [°C] | 62.9 | 62.9 | 55.0 | 58.2 | 56.7 | 56.3 | 56.9 | 58.3 | 65.1 |
| | $T_{agg}$ [°C] | 74.6 | 73.9 | 70.6 | 64.3 | 62.1 | 62.0 | 63.0 | 63.5 | 68.5 |

Table 13 shows a summary of the data for formulations F1-F9 as measured at T1m (i.e. after 1-month storage at 25°C).

Table 13 – Summary of data for formulations F1-F9 for measurements taken at T1m

| T1m 25 °C | | Formulation ID | | | | | | | | |
|---|---|---|---|---|---|---|---|---|---|---|
| | | 1 | 2 | 3 | 4 | 5 | 6 | 7 | 8 | 9 |
| Formulation attributes | pH target | 5.80 | 5.80 | 4.60 | 5.00 | 5.00 | 5.00 | 5.00 | 5.00 | 6.30 |
| | pH measured | 5.84 | 5.86 | 4.68 | 5.06 | 4.98 | 5.00 | 5.06 | 5.06 | 6.35 |
| | fill volume [ml] | 1.00 | 1.00 | 0.80 | 0.80 | 0.80 | 0.80 | 0.80 | 0.80 | 0.80 |
| | protein conc. [mg/ml] | 155.42 | 153.79 | 190.63 | 192.11 | 192.67 | 191.17 | 193.22 | 193.27 | 191.33 |

| | | | | | | | | | | |
|---|---|---|---|---|---|---|---|---|---|---|
| Stability indicating methods | Turbidity T1m | 0.52 | 0.54 | 0.60 | 0.59 | 0.66 | 0.71 | 0.57 | 0.57 | 0.61 |
| | MFI ≤ 2 µm T1m | 2463 | 2335 | 3755 | 3896 | 4026 | 1867 | 2735 | 2181 | 471234 |
| | MFI ≤ 5 µm T1m | 353 | 311 | 488 | 412 | 640 | 434 | 651 | 271 | 13642 |
| | MFI ≤ 10 µm T1m | 107 | 54 | 22 | 98 | 76 | 109 | 185 | 65 | 98 |
| | MFI ≤ 25 µm T1m | 21 | 11 | 0 | 11 | 22 | 22 | 11 | 11 | 11 |
| | acidic-icIEF [%] T1m | 20.71 | 20.70 | 23.55 | 23.38 | 23.53 | 24.55 | 25.54 | 22.96 | 21.88 |
| | main-icIEF [%] T1m | 55.73 | 56.26 | 50.89 | 52.18 | 51.93 | 50.52 | 49.74 | 51.83 | 55.39 |
| | basic-icIEF [%] T1m | 23.56 | 23.03 | 25.57 | 24.45 | 24.54 | 24.91 | 24.71 | 25.21 | 22.72 |
| | HMW-SEC [%] T1m | 1.37 | 1.50 | 2.05 | 1.98 | 1.79 | 1.70 | 1.27 | 1.66 | 2.08 |
| | Main-SEC [%] T1m | 98.32 | 98.22 | 97.50 | 97.64 | 97.82 | 97.86 | 98.30 | 97.71 | 97.63 |
| | LMW-SEC [%] T1m | 0.30 | 0.28 | 0.45 | 0.38 | 0.39 | 0.43 | 0.43 | 0.62 | 0.29 |
| | components-HIC [%] T1m | 0.39 | 0.39 | 0.80 | 0.52 | 0.57 | 0.60 | 0.62 | 0.64 | 0.42 |
| | Uncys-HIC [%] T1m | 88.82 | 88.24 | 82.90 | 86.16 | 83.75 | 81.65 | 87.02 | 85.77 | 88.40 |
| | Oxidation-HIC [%] T1m | 6.82 | 6.95 | 8.47 | 7.36 | 7.61 | 8.40 | 6.52 | 7.50 | 7.59 |
| | Pyro-E-HIC [%] T1m | 3.97 | 4.43 | 7.82 | 5.96 | 8.07 | 9.36 | 5.83 | 6.10 | 3.59 |
| | | | | | | | | | | |

Table 14 shows a summary of the data for formulations F1-F9 illustrating the difference between T0 and T1m - this is thus an indication of changes over time

Table 14 – Summary of data for formulations F1-F9 showing differences between T0 and T1m

| T1m 25 °C - T0 | | Formulation ID | | | | | | | | |
|---|---|---|---|---|---|---|---|---|---|---|
| | | 1 | 2 | 3 | 4 | 5 | 6 | 7 | 8 | 9 |
| Formulation attributes | pH target | 5.80 | 5.80 | 4.60 | 5.00 | 5.00 | 5.00 | 5.00 | 5.00 | 6.30 |
| | pH measured | 5.84 | 5.86 | 4.68 | 5.06 | 4.98 | 5.00 | 5.06 | 5.06 | 6.35 |
| | fill volume [ml] | 1.00 | 1.00 | 0.80 | 0.80 | 0.80 | 0.80 | 0.80 | 0.80 | 0.80 |
| | protein conc. [mg/ml] | 155.42 | 153.79 | 190.63 | 192.11 | 192.67 | 191.17 | 193.22 | 193.27 | 191.33 |
| Stability indicating methods | Turbidity T1m | 0.02 | 0.03 | 0.07 | 0.05 | 0.10 | 0.13 | 0.01 | 0.02 | 0.02 |
| | MFI ≤ 2 µm T1m | -268 | 889 | -2692 | 705 | 3126 | 1259 | -347 | 1009 | 468955 |
| | MFI ≤ 5 µm T1m | -503 | 54 | -1943 | -499 | 347 | 271 | 174 | 33 | 13382 |
| | MFI ≤ 10 µm T1m | -236 | -11 | -814 | -304 | -43 | 54 | 54 | -11 | 33 |
| | MFI ≤ 25 µm T1m | -54 | 0 | -174 | -43 | 11 | 22 | 0 | -22 | 11 |
| | acidic-icIEF T1m | 1.10 | 1.33 | 3.47 | 4.09 | 4.26 | 4.67 | 4.52 | 2.90 | 2.41 |
| | main-icIEF T1m | -1.80 | -1.98 | -6.02 | -5.80 | -6.09 | -6.63 | -6.64 | -5.55 | -2.46 |
| | basic-icIEF T1m | 0.69 | 0.63 | 2.56 | 1.74 | 1.82 | 1.96 | 2.11 | 2.64 | 0.05 |
| | HMW-SEC T1m | 0.13 | 0.25 | 0.85 | 0.76 | 0.59 | 0.51 | 0.33 | 0.46 | 0.62 |
| | Main-SEC T1m | -0.26 | -0.35 | -1.11 | -0.94 | -0.77 | -0.75 | -0.57 | -0.74 | -0.74 |
| | LMW-SEC T1m | 0.13 | 0.10 | 0.26 | 0.19 | 0.18 | 0.25 | 0.23 | 0.28 | 0.12 |
| | components-HIC T1m | 0.39 | 0.39 | 0.80 | 0.52 | 0.57 | 0.60 | 0.62 | 0.64 | 0.42 |
| | Uncys-HIC T1m | -0.57 | -0.96 | -5.59 | -1.98 | -5.80 | -7.39 | -2.24 | -3.19 | 0.22 |
| | Oxidation-HIC T1m | -0.58 | -0.50 | 0.72 | -0.39 | 0.52 | 0.91 | -0.52 | 0.06 | -0.10 |
| | Pyro-E-HIC T1m | 0.76 | 1.08 | 4.06 | 1.85 | 4.71 | 5.89 | 2.13 | 2.50 | -0.54 |
| | | | | | | | | | | |

In response to the results obtained in respect of formulations F1-F9, further formulations were tested as detailed in Example 3.

## Example 3 - Further Formulation Screen

Based on insights gleaned from Example 2, inventive judgements lead to the examination of six further formulations.

These six further formulations are prepared according to Table 15, filled into primary packaging material (2R vials) with a fill volume to achieve a target dose per vial of 150 mg secukinumab per vial (i.e. 1 ml at 150 mg/ml secukinumab or 0.75 ml at 200 mg/ml).

Table 15 - Secukinumab Formulations F2. F10-F14

| Formulation ID | API concentration | pH | Buffer concentration | Buffer type | Tonicity agent | Antioxidant | Surfactant | Headspace oxygen content |
|---|---|---|---|---|---|---|---|---|
| **F2** | 150 mg/ml | 5.8 | 20 mM | histidine | 200 mM trehalose | 5 mM methionine | 0.02 % PS80 | ambient air |
| **F10** | 200 mg/ml | 6.0 | 4 mM (2mM + 2mM) | phosphate/ citrate (1:1) | 125 mM Sorbitol 60 mM NaCl | | 0.05 % PS80 | ambient air |
| **F11** | 200 mg/ml | 6.3 | 4 mM (2mM + 2mM) | phosphate/ citrate (1:1) | 230 mM Sorbitol | | 0.05 % Kolliphor HS-15 | ambient air |
| **F12** | 200 mg/ml | 6.0 | 4 mM (2mM + 2mM) | phosphate/ citrate (1:1) | 230 mM Sorbitol | | 0.05 % PS80 | ambient air |
| **F13** | 200 mg/ml | 6.0 | 55 mM | Succinate | 125 mM Sorbitol | 7 mM Thiosulfate | 0.05 % PS80 | ambient air |
| **F14** | 200 mg/ml | 6.3 | 55 mM | Succinate | 125 mM Sorbitol | | 0.1 % PS80 | ambient air |

The examples that include 4 mM phosphate/citrate buffer include 2 mM phosphate buffer and 2 mM citrate buffer (i.e. the two buffers in a 1:1 molar ratio).

These formulations were prepared in a similar manner to those of Example 2.

For this example, in addition to subjecting the formulations to accelerated storage conditions (at 25°C and 60% r.h., for 1 month, T1m) the formulations were subjected to the following stress conditions:

1. Oxidative stress for 24 h at 25 °C and 60% r.h. ($T_{ox}$.) 0.05 % (v/v) $H_2O_2$, followed by quenching with methionine.

2. Five freeze thaw cycles between -40 °C and 20 °C (TFT) using an Epsilon1-6CC freeze-thaw unit (Martin Christ GmbH, Osterode, Germany).

***Initial Characteristics of Formulations F2 and F10-F14***

The formulation characteristics described in Table 15 were met.

Measured osmolalities of both the final formulations (F2, F10-F14) (at Time 0) and their corresponding Formulation Buffers (i.e. FB2, FB10-FB14, containing all excipients, except surfactant, but no secukinumab) are given in Table 16 below. Osmolalities of the final formulations range between 308 Osmol/kg to 376 mOsmol/kg, whereas osmolalities of corresponding formulation buffers (i.e. essentially the osmolality of the final formulation minus secukinumab's contribution) range from 240-287 mOsmol/kg.

Table 16 - Osmolalities of Formulations F2, F10-F14 and their corresponding Formulation Buffers FB2, FB10-FB14

| | Osmolality [mOsmol/kg] | |
|---|---|---|
| | T0 | Formulation buffer |
| F2 | 348 | 265 |
| F10 | 308 | 255 |
| F11 | 328 | 240 |
| F12 | 328 | 241 |
| F13 | 363 | 287 |
| F14 | 376 | 276 |

Measured viscosities of final formulations (F2, F10-F14) (at Time 0) are shown in Table 17 below

Table 17 - Viscosities of Formulation F2, F10-F14

| | Viscosity [mPa*s] |
|---|---|
| Formulation | T0 |
| F2 | 9.8 |
| F10 | 23.4 |
| F11 | 26.2 |
| F12 | 26.6 |
| F13 | 22.9 |
| F14 | 23.8 |

The viscosities of F10-F14 are evidently higher than F2 owing to their higher protein concentrations. There is, however, little difference between the viscosities of F10-F14.

Figure 16 shows nanoDSF results for F2, F10-F14, namely: a) Mean $Tm_1$ (left bar) and Mean $Tm_2$ (right bar) for each formulation; and b) $Tm_{onset}$ (left bar) and $Tm_{agg}$ (right bar) for each formulation. The apparent $Tm_1$ and $Tm_1$ and $Tm_{onset}$ of all formulations are comparable. $T_{agg}$ of F10-F14 is lower than in reference formulation (F2).

Formulations were examined by visual inspection at appropriate time points - - all remained substantially free of visible particles according to Ph. Eur. Visual inspection.

Table 18 shows the results of protein concentration analysis at various time points. There were no substantial changes in protein concentration between T), TFT, and T1m_25°C. There were slightly lower protein concentrations in Tox samples due to the dilution by $H_2O_2$ spiking and methionine quenching.

Table 18 - Protein concentration results for F2, F10-F14 at various time points

| | Protein concentration [mg/ml] | | | |
|---|---|---|---|---|
| Formulation | T0 | Tox | TFT | T1m_25 °C |
| F2 | 156.0 | 147.7 | 155.0 | 156.9 |
| F10 | 208.4 | 199.5 | 209.2 | 213.2 |
| F11 | 204.3 | 193.4 | 200.2 | 209.0 |
| F12 | 203.6 | 194.7 | 201.5 | 206.1 |
| F13 | 204.9 | 199.0 | 202.5 | 207.9 |
| F14 | 204.2 | 192.3 | 205.3 | 206.3 |

Table 19 shows the results of turbidity analysis for formulations F2, and F10-F14. $OD_{350}$ appeared to be lower after 1 month at 25°C compared to at T0. $OD_{350}$ slightly increased in F14 after 1 month at 25°C. $OD_{350}$ slightly increased in F13 upon oxidative stress. No substantial changes of $OD_{350}$ were detected upon freeze-thaw stress.

Table 19 - Sample turbidity results for F2, F10-F14 at various time points (by $OD_{350}$ – soloVPE)

| | Sample turbidity ($OD_{350}$ at 10 mm) | | | |
|---|---|---|---|---|
| Formulation | T0 | Tox | TFT | T1m_25 °C |
| F2 | 0.55 | 0.57 | 0.56 | 0.52 |
| F10 | 0.60 | 0.64 | 0.62 | 0.54 |
| F11 | 0.62 | 0.66 | 0.63 | 0.54 |
| F12 | 0.61 | 0.64 | 0.62 | 0.56 |
| F13 | 0.63 | 0.72 | 0.65 | 0.56 |
| F14 | 0.63 | 0.67 | 0.62 | 0.59 |

Figure 17 shows the results of micro-flow imaging (MFI) for each of F2, F10-F14, by reference to particles per container: a) for particles ≥ 2 µm; b) for particles ≥ 5 µm; c) for particles ≥ 10 µm; and d) for particles ≥ 25 µm. Sub-visible particle levels (≥2 µm, ≥5 µm, ≥10 µm and ≥25 µm) of T0, Tox, TFT, and T1m_25°C were similar for all formulations.

Size exclusion chromatography was also performed on samples F2, F10-F14. Figure 18 shows SEC results as bar charts for each formulation F2, F10-F14 (bars left to right are T0, Tox, TFT, T1m 25°C respectively) for: a) high molecular weights (HMW); b) low molecular weights (LMW); and c) monomer.

Figure 19 shows hydrophobic interaction chromatography (HIC) results in the form of bar charts charts for each formulation F2, F10-F14 (left-to-right bars relate to T0, Tox, TFT, and T1m_25C respectively) in relation to: a) uncysteinylated form; b) "unknown" components; c) oxidized form; d) pyroglutamate form.

Table 20 shows a summary of the data for formulations F2, F10-F14 as measured at T0 (i.e. before any stress testing or storage). Red-to-green heat map colouring is also applied (with red indicating relative instability and green indicating relative stability).

Table 20 - Summary of data for formulations F2, F10-F14 for measurements taken at T0

| T0 | | Formulation ID | | | | | |
|---|---|---|---|---|---|---|---|
| | | 2 | 10 | 11 | 12 | 13 | 14 |
| Formulation attributes | pH target | 5.80 | 6.00 | 6.30 | 6.00 | 6.00 | 6.30 |
| | pH measured | 5.81 | 5.95 | 6.31 | 6.08 | 6.07 | 6.33 |
| | fill volume [ml] | 1.00 | 0.75 | 0.75 | 0.75 | 0.75 | 0.75 |
| | protein conc. [mg/ml] | 155.98 | 208.44 | 204.27 | 203.61 | 204.87 | 204.18 |
| Stability indicating methods | Turbidity T0 | 0.55 | 0.60 | 0.62 | 0.61 | 0.63 | 0.63 |
| | MFI ≤ 2 µm T0 | 2560 | 6083 | 9286 | 9682 | 7979 | 9296 |
| | MFI ≤ 5 µm T0 | 557 | 1114 | 1939 | 1864 | 1639 | 1971 |
| | MFI ≤ 10 µm T0 | 150 | 268 | 600 | 396 | 386 | 578 |
| | MFI ≤ 25 µm T0 | 43 | 64 | 118 | 32 | 54 | 96 |
| | acidic-icIEF [%] T0 | 20.75 | 20.37 | 19.92 | 20.03 | 21.00 | 20.39 |
| | main-icIEF [%] T0 | 57.61 | 57.84 | 57.96 | 58.07 | 57.19 | 57.63 |
| | basic-icIEF [%] T0 | 21.63 | 21.78 | 22.12 | 21.89 | 21.81 | 21.98 |
| | HMW-SEC [%] T0 | 1.46 | 1.59 | 1.71 | 1.49 | 1.24 | 1.61 |
| | Main-SEC [%] T0 | 98.22 | 98.05 | 97.88 | 98.12 | 98.31 | 98.01 |
| | LMW-SEC [%] T0 | 0.31 | 0.36 | 0.42 | 0.39 | 0.45 | 0.38 |
| | components-HIC [%] T0 | 0.14 | 0.21 | 0.48 | 0.28 | 0.42 | 0.31 |
| | Uncys-HIC [%] T0 | 89.06 | 89.29 | 87.15 | 89.59 | 89.08 | 89.44 |
| | Oxidation-HIC [%] T0 | 7.39 | 7.39 | 7.91 | 7.24 | 7.15 | 7.32 |
| | Pyro-E-HIC [%] T0 | 3.41 | 3.12 | 4.46 | 2.89 | 3.35 | 2.93 |
| | $Tm_1$ [°C] T0 | 67.9 | 67.2 | 68.8 | 68.1 | 67.9 | 68.8 |
| | $Tm_2$ [°C] T0 | 79.7 | 79.5 | 79.8 | 79.9 | 79.7 | 79.8 |
| | $Tm_{onset}$ [°C] T0 | 63.0 | 63.2 | 64.7 | 63.7 | 63.7 | 64.7 |
| | $T_{agg}$ [°C] T0 | 75.9 | 68.6 | 70.0 | 68.4 | 67.0 | 67.4 |

Table 21 shows a summary of the data for formulations F2, F10-F14 as measured after Tox (i.e. after oxidative stress). Red-to-green heat map colouring is also applied (with red indicating relative instability and green indicating relative stability).

Table 21 - Summary of data for formulations F2, F10-F14 for measurements taken after Tox

| Tox | | Formulation ID | | | | | |
|---|---|---|---|---|---|---|---|
| | | 2 | 10 | 11 | 12 | 13 | 14 |
| Formulation attributes | pH target | 5.80 | 6.00 | 6.30 | 6.00 | 6.00 | 6.30 |
| | pH measured (T0) | 5.81 | 5.95 | 6.31 | 6.08 | 6.07 | 6.33 |
| | fill volume [ml] | 1.00 | 0.75 | 0.75 | 0.75 | 0.75 | 0.75 |
| | protein conc. [mg/ml] Tox | 147.73 | 199.48 | 193.41 | 194.71 | 199.02 | 192.28 |
| Stability indicating methods | Turbidity Tox | 0.57 | 0.64 | 0.66 | 0.64 | 0.72 | 0.67 |
| | MFI ≤ 2 µm Tox | 9821 | 7369 | 6640 | 5783 | 3299 | 13730 |
| | MFI ≤ 5 µm Tox | 1917 | 1232 | 1178 | 1221 | 364 | 2785 |
| | MFI ≤ 10 µm Tox | 568 | 364 | 182 | 257 | 21 | 610 |
| | MFI ≤ 25 µm Tox | 118 | 43 | 11 | 21 | 11 | 64 |
| | acidic-icIEF [%] Tox | 28.58 | 32.31 | 37.88 | 33.36 | 47.01 | 39.15 |
| | main-icIEF [%] Tox | 51.59 | 48.88 | 45.13 | 48.37 | 38.50 | 43.53 |
| | basic-icIEF [%] Tox | 19.83 | 18.81 | 16.99 | 18.27 | 14.49 | 17.31 |
| | HMW-SEC [%] Tox | 1.50 | 1.67 | 1.83 | 1.55 | 1.84 | 1.79 |
| | Main-SEC [%] Tox | 98.00 | 97.82 | 97.60 | 97.94 | 97.66 | 97.56 |
| | LMW-SEC [%] Tox | 0.50 | 0.51 | 0.57 | 0.51 | 0.49 | 0.65 |
| | components-HIC [%] Tox | 0.36 | 0.35 | 0.38 | 0.27 | 0.43 | 0.30 |
| | Uncys-HIC [%] Tox | 73.63 | 67.05 | 54.38 | 63.77 | 59.67 | 54.88 |
| | Oxidation-HIC [%] Tox | 16.62 | 21.02 | 27.76 | 22.53 | 13.67 | 27.69 |
| | Pyro-E-HIC [%] Tox | 9.39 | 11.58 | 17.47 | 13.43 | 26.23 | 17.13 |

Table 22 shows a summary of the data for formulations F2, F10-F14 as measured after TFT (i.e. after freeze-thaw stress). Red-to-green heat map colouring is also applied (with red indicating relative instability and green indicating relative stability).

Table 22 - Summary of data for formulations F2, F10-F14 for measurements taken after TFT

| TFT | | Formulation ID | | | | | |
|---|---|---|---|---|---|---|---|
| | | 2 | 10 | 11 | 12 | 13 | 14 |
| Formulation attributes | pH target | 5.80 | 6.00 | 6.30 | 6.00 | 6.00 | 6.30 |
| | pH measured (T0) | 5.81 | 5.95 | 6.31 | 6.08 | 6.07 | 6.33 |
| | fill volume [ml] | 1.00 | 0.75 | 0.75 | 0.75 | 0.75 | 0.75 |
| | protein conc. [mg/ml] TFT | 155.01 | 209.20 | 200.24 | 201.49 | 202.48 | 205.27 |
| Stability indicating methods | Turbidity TFT | 0.56 | 0.62 | 0.63 | 0.62 | 0.65 | 0.62 |
| | MFI ≤ 2 µm TFT | 4905 | 3363 | 4188 | 2185 | 3513 | 4884 |
| | MFI ≤ 5 µm TFT | 493 | 578 | 578 | 439 | 557 | 975 |
| | MFI ≤ 10 µm TFT | 96 | 150 | 107 | 54 | 150 | 289 |
| | MFI ≤ 25 µm TFT | 0 | 32 | 21 | 0 | 54 | 75 |
| | acidic-icIEF [%] TFT | 20.33 | 20.76 | 20.54 | 20.34 | 19.89 | 19.95 |
| | main-icIEF [%] TFT | 58.13 | 56.84 | 57.72 | 57.96 | 58.20 | 58.07 |
| | basic-icIEF [%] TFT | 21.53 | 22.38 | 21.73 | 21.69 | 21.91 | 21.98 |
| | HMW-SEC [%] TFT | 1.41 | 1.59 | 1.66 | 1.59 | 1.36 | 1.78 |
| | Main-SEC [%] TFT | 98.22 | 98.01 | 97.95 | 97.99 | 98.24 | 97.98 |
| | LMW-SEC [%] TFT | 0.37 | 0.40 | 0.40 | 0.42 | 0.40 | 0.24 |
| | components-HIC [%] TFT | 0.67 | 0.47 | 0.52 | 0.43 | 0.44 | 0.49 |
| | Uncys-HIC [%] TFT | 86.90 | 87.91 | 88.55 | 88.86 | 88.83 | 88.63 |
| | Oxidation-HIC [%] TFT | 7.61 | 6.91 | 6.67 | 6.51 | 6.91 | 7.21 |
| | Pyro-E-HIC [%] TFT | 4.82 | 4.71 | 4.26 | 4.19 | 3.82 | 3.67 |

Table 23 shows a summary of the data for formulations F2, F10-F14 as measured at T1m (i.e. after 1-month storage at 25°C). Red-to-green heat map colouring is also applied (with red indicating relative instability and green indicating relative stability)

Table 23 - Summary of data for formulations F2, F10-F14 for measurements taken at T1m (at 25°C)

| T1m_25°C | | Formulation ID | | | | | |
|---|---|---|---|---|---|---|---|
| | | 2 | 10 | 11 | 12 | 13 | 14 |
| Formulation attributes | pH target | 5.80 | 6.00 | 6.30 | 6.00 | 6.00 | 6.30 |
| | pH measured (T0) | 5.81 | 5.95 | 6.31 | 6.08 | 6.07 | 6.33 |
| | fill volume [ml] | 1.00 | 0.75 | 0.75 | 0.75 | 0.75 | 0.75 |
| | protein conc. [mg/ml] T1m_25°C | 156.9 | 213.2 | 209.0 | 206.1 | 207.9 | 206.3 |
| Stability indicating methods | Turbidity T1m_25°C | 0.52 | 0.54 | 0.54 | 0.56 | 0.56 | 0.59 |
| | MFI ≤ 2 µm T1m_25°C | 1660 | 3556 | 1446 | 2495 | 4091 | 2924 |
| | MFI ≤ 5 µm T1m_25°C | 289 | 525 | 396 | 471 | 471 | 439 |
| | MFI ≤ 10 µm T1m_25°C | 96 | 96 | 86 | 139 | 32 | 150 |
| | MFI ≤ 25 µm T1m_25°C | 11 | 0 | 0 | 11 | 21 | 54 |
| | acidic-icIEF [%] T1m_25°C | 22.88 | 21.82 | 22.38 | 22.78 | 26.22 | 23.38 |
| | main-icIEF [%] T1m_25°C | 55.29 | 55.99 | 55.13 | 55.27 | 52.64 | 54.60 |
| | basic-icIEF [%] T1m_25°C | 21.84 | 22.19 | 22.47 | 21.94 | 21.13 | 22.02 |
| | HMW-SEC [%] T1m_25°C | 1.79 | 2.13 | 2.41 | 2.21 | 1.69 | 2.62 |
| | Main-SEC [%] T1m_25°C | 97.79 | 97.49 | 97.20 | 97.44 | 97.95 | 96.95 |
| | LMW-SEC [%] T1m_25°C | 0.42 | 0.38 | 0.39 | 0.36 | 0.36 | 0.43 |
| | components-HIC [%] T1m_25°C | 0.25 | 0.20 | 0.16 | 0.27 | 0.27 | 0.20 |
| | Uncys-HIC [%] T1m_25°C | 90.08 | 90.41 | 90.76 | 90.38 | 90.73 | 90.70 |
| | Oxidation-HIC [%] T1m_25°C | 6.25 | 6.53 | 6.74 | 6.66 | 6.34 | 6.75 |
| | Pyro-E-HIC [%] T1m_25°C | 3.42 | 2.86 | 2.34 | 2.69 | 2.66 | 2.35 |

In response to the results obtained in respect of formulations F2, F10-F14, further formulations were tested as detailed in Example 4.

Example 4 - Further Formulation Screen

Based on insights gleaned from Example 3, inventive judgements lead to the examination of further secukinumab formulations, as shown in Table 24.

Table 24 - Secukinumab Formulations F2, F15-F17

| Formulation ID | API concentration | pH | Buffer concentration | Buffer type | Tonicity adjuster | Surfactant | Headspace oxygen content |
|---|---|---|---|---|---|---|---|
| **F2** | 150 mg/ml | 5.8 | 20 mM | histidine | 200 mM trehalose | 0.02 % PS80 | ambient air |
| **F15** | 180 mg/ml | 5.8 | 4 mM (2+2) | Citrate/phosphate (1:1) | 125 mM Sorbitol / 60 mM NaCl | 0.05 % PS80 | ambient air |
| **F16** | 180 mg/ml | 5.8 | 55 mM | succinate | 125 mM Sorbitol | 0.05 % PS80 | ambient air |
| **F17** | 165 mg/ml | 6.0 | 4 mM (2+2) | Citrate/phosphate (1:1) | 125 mM Sorbitol / 60 mM NaCl | 0.05 % PS80 | ambient air |

**Example 5 - Further Formulation Screen**

Based on insights gleaned from Example 3, inventive judgements lead to the examination of further secukinumab formulations, as shown in Table 25. In all formulations their headspace oxygen content corresponds with ambient air. Dual buffer systems, such as phosphate/citrate, are 1:1 in terms of molar ratio of each buffer. MES is 2-(N-morpholino)ethanesulfonic acid.

Table 25 - Secukinumab Formulations F2 and F18-F56

| ID | API conc. (mg/ mL) | pH | Buffer conc. | Buffer Type | Tonicity agent | Antioxidant / Chelator | Surfactant |
|---|---|---|---|---|---|---|---|
| F2 | 165 | 5.8 | 20 mM | Histidine | 200 mM Trehalose | 5 mM Methionine | 0.05% PS80 |
| F18 | 165 | 5.8 | 20 mM | Histidine | 200 mM Trehalose | - | 0.05% PS80 |
| F19 | 165 | 5.8 | 4 mM | Histidine | 200 mM Trehalose | - | 0.05% PS80 |
| F20 | 165 | 5.8 | 4 mM | phosphate | 200 mM Trehalose | - | 0.05% PS80 |
| F21 | 165 | 5.8 | 4 mM | succinate | 200 mM Trehalose | - | 0.05% PS80 |
| F22 | 165 | 5.8 | 20mM | Histidine | 125 mM Sorbitol 60 mM NaCl | - | 0.05% PS80 |
| F23 | 165 | 5.6 | 4 mM | phosphate/ citrate | 125 mM Sorbitol 60 mM NaCl | - | 0.05% PS80 |
| F24 | 165 | 5.8 | 4 mM | phosphate/ citrate | 125 mM Sorbitol 60 mM NaCl | - | 0.05% PS80 |
| F25 | 165 | 6.0 | 4 mM | phosphate/ citrate | 125 mM Sorbitol 60 mM NaCl | - | 0.05% PS80 |
| F26 | 165 | 6.3 | 4 mM | phosphate/ citrate | 125 mM Sorbitol 60 mM NaCl | - | 0.05% PS80 |
| F27 | 165 | 5.6 | 4 mM | phosphate/ succinate | 125 mM Sorbitol 60 mM NaCl | - | 0.05% PS80 |
| F28 | 165 | 5.8 | 4 mM | phosphate/ succinate | 125 mM Sorbitol 60 mM NaCl | - | 0.05% PS80 |
| F29 | 165 | 6.0 | 4 mM | phosphate/ succinate | 125 mM Sorbitol 60 mM NaCl | - | 0.05% PS80 |
| F30 | 165 | 6.3 | 4 mM | phosphate/ succinate | 125 mM Sorbitol 60 mM NaCl | - | 0.05% PS80 |

(continued)

| ID | API conc. (mg/ mL) | pH | Buffer conc. | Buffer Type | Tonicity agent | Antioxidant / Chelator | Surfactant |
|---|---|---|---|---|---|---|---|
| F31 | 165 | 5.6 | 4 mM | phosphate/ acetate | 125 mM Sorbitol 60 mM NaCl | - | 0.05% PS80 |
| F32 | 165 | 5.8 | 4 mM | phosphate/ acetate | 125 mM Sorbitol 60 mM NaCl | - | 0.05% PS80 |
| F33 | 165 | 6.0 | 4 mM | phosphate/ acetate | 125 mM Sorbitol 60 mM NaCl | - | 0.05% PS80 |
| F34 | 180 | 5.6 | 4 mM | phosphate/ citrate | 125 mM Sorbitol 60 mM NaCl | - | 0.05% PS80 |
| F35 | 180 | 5.8 | 4 mM | phosphate/ citrate | 125 mM Sorbitol 60 mM NaCl | - | 0.05% PS80 |
| F36 | 180 | 6.0 | 4 mM | phosphate/ citrate | 125 mM Sorbitol 60 mM NaCl | - | 0.05% PS80 |
| F37 | 165 | 6.0 | 4 mM | phosphate/ citrate | 125 mM Sorbitol 60 mM NaCl | 5 mM Methionine | 0.05% PS80 |
| F38 | 165 | 6.0 | 4 mM | phosphate/ citrate | 125 mM Sorbitol 60 mM NaCl | 2 mM EDTA | 0.05% PS80 |
| F39 | 165 | 5.6 | 4 mM | phosphate/ citrate | 125 mM Sorbitol 60 mM NaCl | 0.5 mM Pentetic Acid | 0.05% PS80 |
| F40 | 165 | 5.8 | 4 mM | phosphate/ citrate | 125 mM Sorbitol 60 mM NaCl | 0.5 mM Pentetic Acid | 0.05% PS80 |
| F41 | 165 | 6.0 | 4 mM | phosphate/ citrate | 125 mM Sorbitol 60 mM NaCl | 0.5 mM Pentetic Acid | 0.05% PS80 |
| F42 | 165 | 5.8 | 55mM | MES | 125 mM Sorbitol | - | 0.05% PS80 |
| F43 | 165 | 6.0 | 55mM | MES | 125 mM Sorbitol | - | 0.05% PS80 |
| F44 | 165 | 6.0 | 4 mM | succinate | 125 mM Sorbitol 60 mM NaCl | - | 0.05% PS80 |
| F45 | 165 | 6.0 | 55 mM | succinate | 125 mM Sorbitol | - | 0.05% PS80 |
| F46 | 165 | 6.0 | 55 mM | succinate | 125 mM Sorbitol | 5 mM Methionine | 0.05% PS80 |

(continued)

| ID | API conc. (mg/ mL) | pH | Buffer conc. | Buffer Type | Tonicity agent | Antioxidant / Chelator | Surfactant |
|---|---|---|---|---|---|---|---|
| F47 | 165 | 6.0 | 55 mM | phosphate/ citrate | 125 mM Sorbitol | - | 0.05% PS80 |
| F48 | 165 | 6.0 | 4 mM | phosphate/ citrate | 20 mM Phenylalanine 125 mM Sorbitol 60 mM NaCl | - | 0.05% PS80 |
| F49 | 165 | 6.0 | 4 mM | phosphate/ citrate | 170 mM Proline 70 mM Sorbitol | - | 0.05% PS80 |
| F50 | 165 | 6.0 | 4 mM | phosphate/ citrate | 170 mM Glycine 70 mM Sorbitol | - | 0.05% PS80 |
| F51 | 165 | 6.0 | 4 mM | phosphate/ citrate | 120 mM Lysine 70 mM Sorbitol | - | 0.05% PS80 |
| F52 | 165 | 6.0 | 4 mM | phosphate/ citrate | 120 mM Arginine 50 mM Sorbitol | - | 0.05% PS80 |
| F53 | 165 | 6.0 | 4 mM | phosphate/ citrate | 60 mM Arginine 140 mM Sorbitol | - | 0.05% PS80 |
| F54 | 165 | 6.0 | 4 mM | phosphate/ citrate | 125 mM Sorbitol 60 mM NaCl | - | 0.05 % PS20 |
| F55 | 165 | 6.0 | 4 mM | phosphate/ citrate | 125 mM Sorbitol 60 mM NaCl | - | 0.05 % Px188 |
| F56 | 165 | 6.0 | 4 mM | phosphate/ citrate | 125 mM Sorbitol 60 mM NaCl | - | 0.03 % PS80 |

ABBREVIATIONS

**ACN** acetonitrile
**BSA** bovine serum albumine
**CAD** charged aerosol detector
**CDR** complementarity-determining region
**cGE** capillary gel electrophoresis
**CpB** carboxypeptidase B
**DAD** diode array detector
**DoE** Design of experiment
**DP** Drug product
**DS** drug substance
**DSF** Differential scanning fluorimetry
**FKS730** Code name for Secukinumab biosimilar

| | |
|---|---|
| **FTO** | freedom to operate |
| **GRP** | good research practice |
| **HIC** | hydrophobic interaction chromatography |
| **HMW** | high molecular weight species |
| **HPLC** | high performance liquid chromatography |
| **IcIEF** | Imaged capillary isoelectric focusing |
| **IPA** | isopropyl alcohol |
| **LAF** | Laminar air flow |
| **LC-CAD** | liquid chromatography charged aerosol detection |
| **MeOH** | Methanol |
| **MES** | 2-(N-morpholino)ethanesulfonic acid |
| **MFI** | Micro-Flow Imaging |
| **MW** | molecular weight |
| **MWCO** | molecular weight cut-off |
| **n.a.** | not applicable |
| **nanoDSF** | nano differential scanning fluorimetry |
| **NWP** | normalized water pressure |
| **OD** | Optical density |
| **PES** | polyether sulfone |
| **PFS** | pre-filled syringe |
| **Ph.Eur.** | European Pharmacopoeia |
| **pI** | isoelectric point |
| **PP** | polypropylene |
| **PS** | polysorbate |
| **RCF** | relative centrifugal force |
| **Ref STD** | reference standard |
| **RFU** | relative fluorescence unit |
| **R&D** | research and development |
| **RP-HPLC** | reversed-phase high performance liquid chromatography |
| **rpm** | revolutions per minute |
| **RSD** | Relative standard deviation |
| **RT** | Room temperature |
| **s.c.** | subcutaneous |
| **SE-UPLC** | Size exclusion UPLC |
| **SE-HPLC** | Size exclusion high performance liquid chromatography |
| **SOP** | standard operating procedure |
| **SST** | system suitability test |
| **TFF** | Tangential flow filtration |
| **$T_m$** | melting temperature |
| **TMP** | Transmembrane pressure |
| **TPP** | target product profile |
| **UPLC** | ultra-performance liquid chromatography |
| **USP** | United States Pharmacopoeia |
| **UV** | ultraviolet |
| **Vis** | visible |
| **WP** | work package |

## Claims

1. A liquid biopharmaceutical composition comprising secukinumab and one or more pharmaceutically-acceptable excipients, carriers, and/or diluents, wherein the composition is **characterised by** an absence of methionine.

2. The liquid biopharmaceutical composition as claimed in claim 1, wherein the composition is **characterised by** an absence of histidine.

3. The liquid biopharmaceutical composition as claimed in any preceding claim, wherein the composition is **characterised by** an absence of trehalose and/or sucrose.

4. The liquid biopharmaceutical composition as claimed in any preceding claim, wherein the composition comprises 145-190 mg/mL secukinumab.

5. The liquid biopharmaceutical composition as claimed in any preceding claim, wherein the composition comprises 1-9 mM buffer system or 51-60 mM buffer system.

6. The liquid biopharmaceutical composition as claimed in claim 5, wherein the buffer system is selected from the group consisting of a succinate buffer system, an adipate buffer system, a phosphate buffer system, a citrate buffer system, a phosphate-citrate buffer system, and any combination thereof (suitably up to a maximum of two specific buffer systems).

7. The liquid biopharmaceutical composition as claimed in any preceding claim, wherein the composition comprises 50-129 mM mannitol, 50-129 mM sorbitol, or 200-250 mM sorbitol.

8. The liquid biopharmaceutical compsoition as claimed in any preceding claim, wherein the composition comprises 100-149 mM amino acid component selected from the group consisting of arginine, glycine, lysine, and any combination thereof.

9. The liquid biopharmaceutical composition as claimed in any preceding claim, wherein the composition comprise 0.1-1.1 mg/mL surfactant selected from polysorbate 20, polysorbate 80, and Kolliphor HS-15.

10. The liquid biopharmaceutical composition as claimed in any preceding claim, wherein the composition comprises 1-10 mM thiosulfate.

11. The liquid biopharmaceutical composition as claimed in any preceding claim, wherein the composition comprises 50-100 mM sodium chloride.

12. The liquid biopharmaceutical composition as claimed in any preceding claim, wherein the composition has a pH of pH 5.6-6.2.

13. The liquid biopharmaceutical composition as claimed in any preceding claim:

a) wherein the composition comprises or consists of:

- 145-190 mg/mL secukinumab;
- 1-9 mM phosphate-citrate buffer system (with a phosphate and citrate buffer system in molar ratio between 3:1 to 1:3);
- 100-150 mM sugar alcohol;
- 0.1-1.1 mg/mL surfactant;
- 20-120 mM tonicifier; and
- water (e.g. WFI);

wherein the composition has a pH of pH 5.6-6.0;
b) wherein the composition comprises or consists of:

- 145-190 mg/mL secukinumab;
- 51-60 mM succinate buffer system;
- 100-150 mM sugar alcohol;
- 0.1-1.1 mg/mL surfactant; and
- water (e.g. WFI);

wherein the composition has a pH of pH 5.6-6.0;
OR
c) wherein the composition comprises or consists of:

- 145-170 mg/mL secukinumab;
- 1-9 mM phosphate-citrate buffer system (with a phosphate and citrate buffer system in molar ratio between 3:1 to 1:3);

- 100-150 mM sugar alcohol;
- 0.1-1.1 mg/mL surfactant;
- 20-120 mM tonicifier; and
- water (e.g. WFI);

wherein the composition has a pH of pH 5.8-6.2.

**14.** The liquid biopharmaceutical composition as claimed in any preceding claim:

a) wherein the composition consists of:

- 180 mg/mL secukinumab;
- 4 mM phosphate-citrate buffer system (with a phosphate and citrate buffer system in molar ratio of 1:1);
- 125 mM sorbitol;
- 0.5 mg/mL polysorbate 80;
- 60 mM NaCl; and
- water (e.g. WFI);

wherein the composition has a pH of pH 5.8;
b) wherein the composition consists of:

- 180 mg/mL secukinumab;
- 55 mM succinate buffer system;
- 125 mM sorbitol;
- 0.5 mg/mL polysorbate 80; and
- water (e.g. WFI);

wherein the composition has a pH of pH 5.8;
OR
c) wherein the composition consists of:

- 165 mg/mL secukinumab;
- 4 mM phosphate-citrate buffer system (with a phosphate and citrate buffer system in molar ratio of 1:1);
- 125 mM sorbitol;
- 0.5 mg/mL polysorbate 80;
- 60 mM NaCl; and
- water (e.g. WFI);

wherein the composition has a pH of pH 6.0.

**15.** A container comprising the liquid biopharmaceutical composition as claimed in any preceding claim, wherein the container comprises a headspace, wherein the oxygen content in the headspace is greater 15%.

**16.** A liquid biopharmaceutical composition for use in treating a disease or medical disorder, or a use of a biopharmaceutical composition in the manufacture of a medicament for a treatment of a disease or disorder, wherein the disease or medical disorder is selected from the group consisting of psoriasis, ankylosing spondylitis, and psoriatic arthritis; wherein the composition is as claimed in any of claims 1-14.

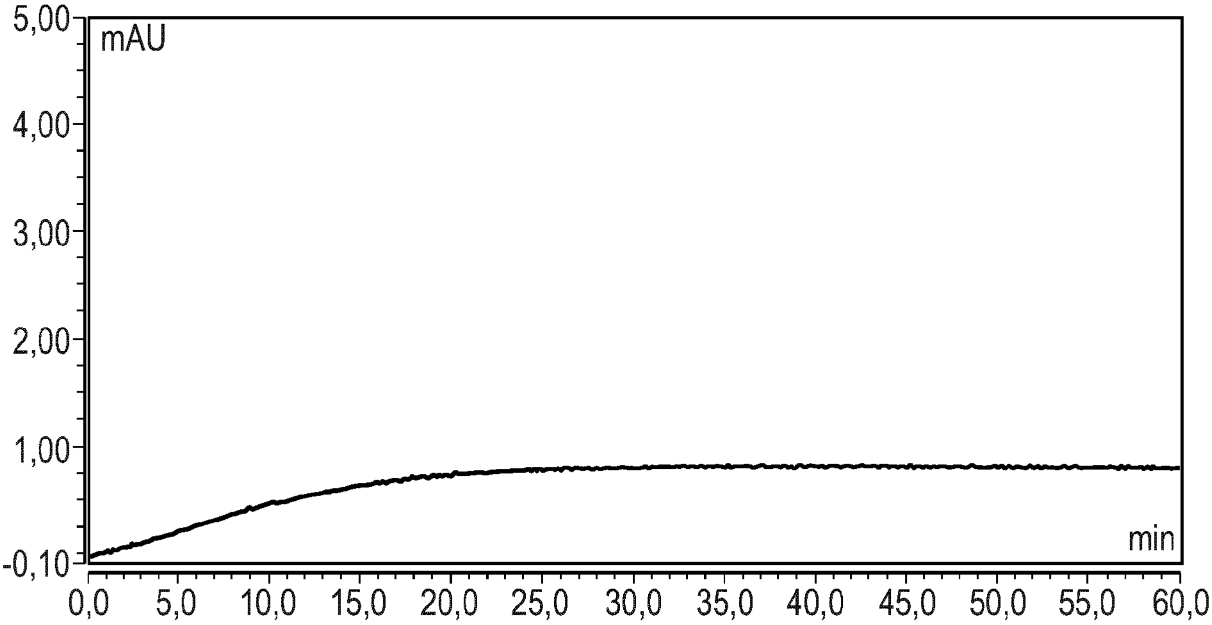
mAU
5,00
4,00
3,00
2,00
1,00
-0,10
min
0,0
5,0
10,0
15,0
20,0
25,0
30,0
35,0
40,0
45,0
50,0
55,0
60,0

Fig. 1

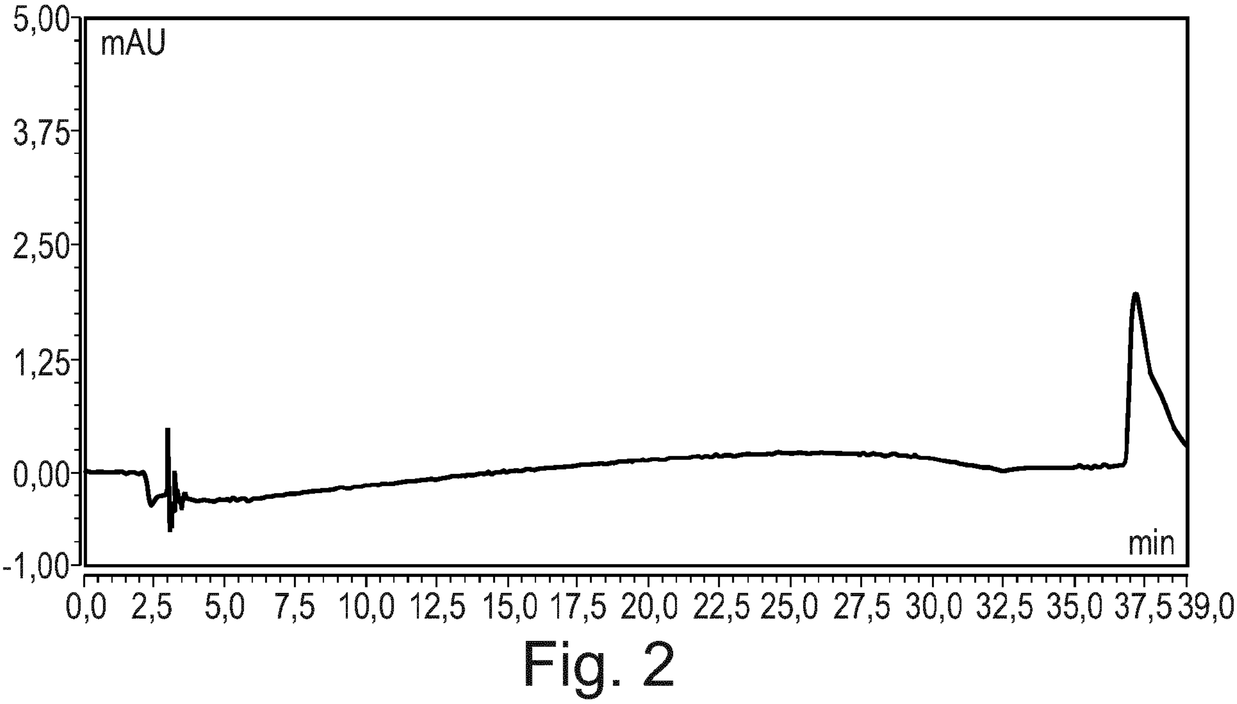
mAU
5,00
3,75
2,50
1,25
0,00
-1,00
min
0,0
2,5
5,0
7,5
10,0
12,5
15,0
17,5
20,0
22,5
25,0
27,5
30,0
32,5
35,0
37,5
39,0

Fig. 2

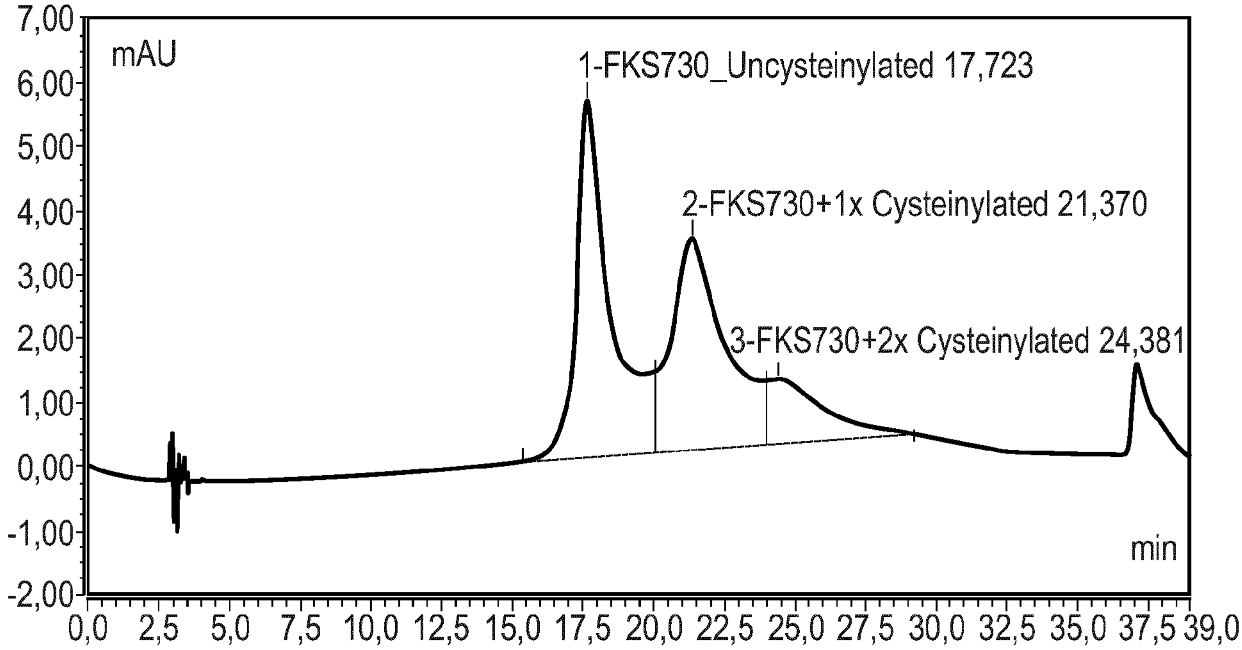

mAU
1-FKS730_Uncysteinylated 17,723
2-FKS730+1x Cysteinylated 21,370
3-FKS730+2x Cysteinylated 24,381
min
7,00
6,00
5,00
4,00
3,00
2,00
1,00
0,00
-1,00
-2,00
0,0 2,5 5,0 7,5 10,0 12,5 15,0 17,5 20,0 22,5 25,0 27,5 30,0 32,5 35,0 37,5 39,0


Fig. 3

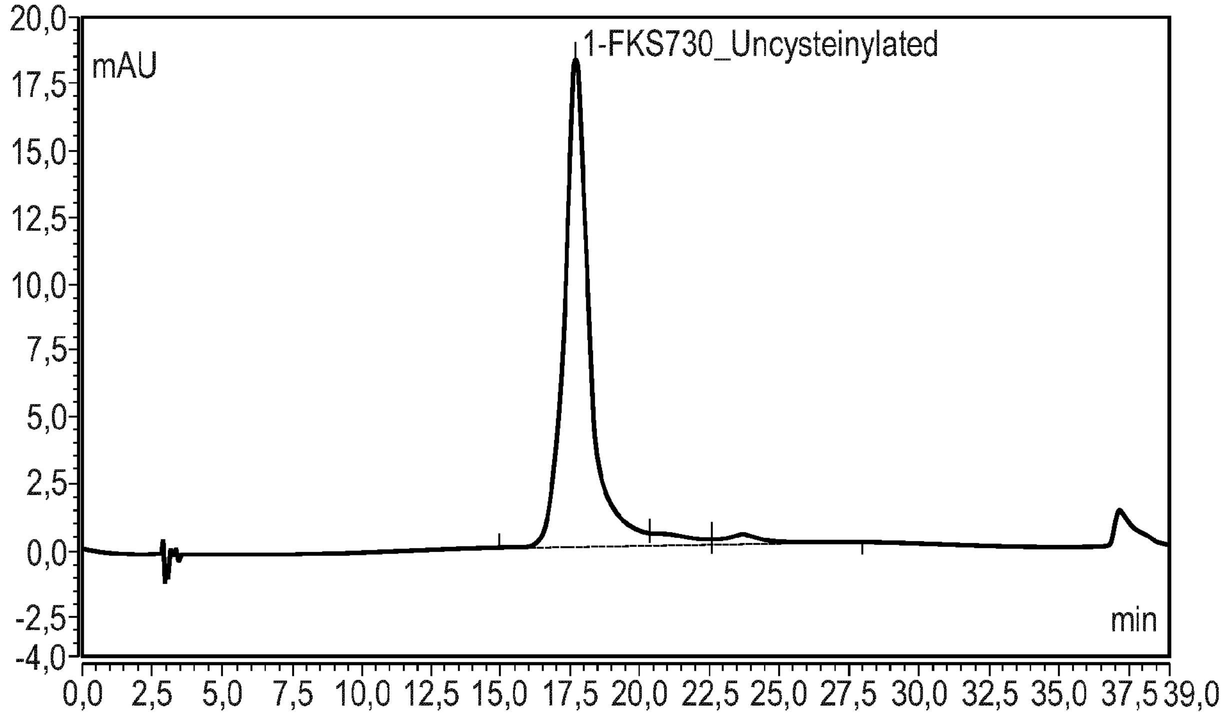

mAU
1-FKS730_Uncysteinylated
min
20,0
17,5
15,0
12,5
10,0
7,5
5,0
2,5
0,0
-2,5
-4,0
0,0 2,5 5,0 7,5 10,0 12,5 15,0 17,5 20,0 22,5 25,0 27,5 30,0 32,5 35,0 37,5 39,0


Fig. 4a

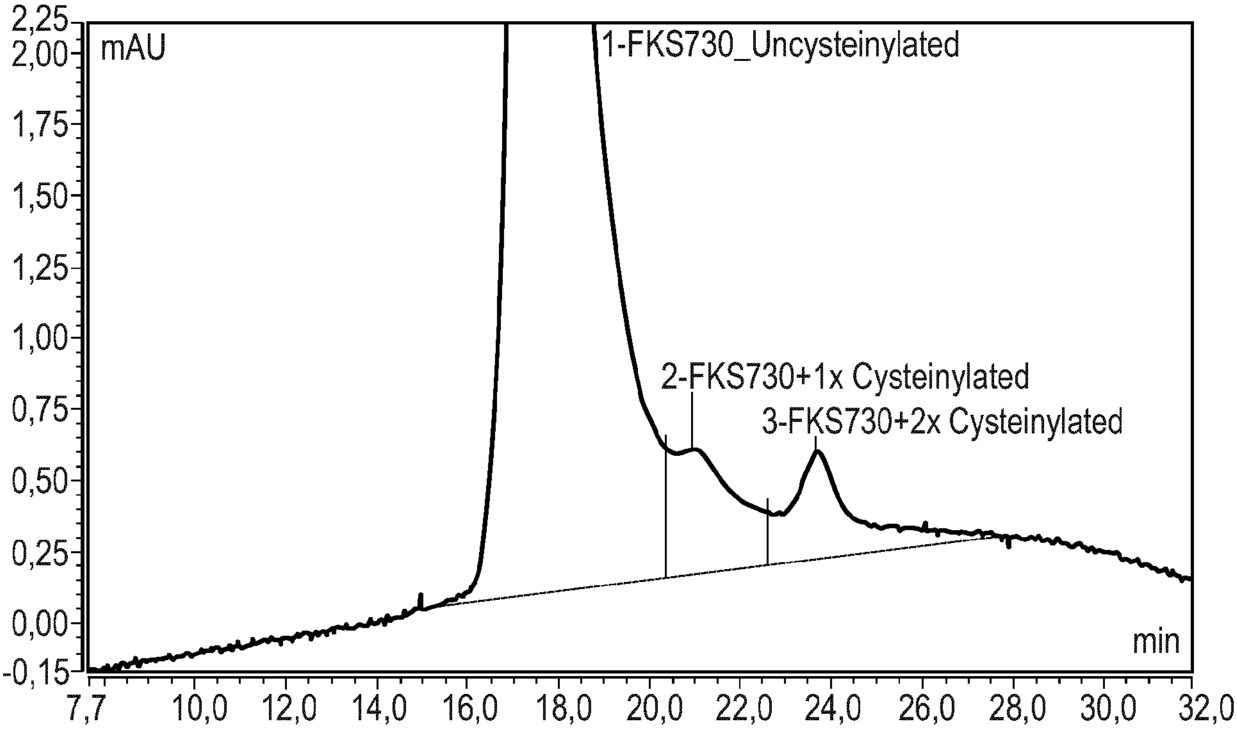
mAU
1-FKS730_Uncysteinylated
2-FKS730+1x Cysteinylated
3-FKS730+2x Cysteinylated
min
2,25
2,00
1,75
1,50
1,25
1,00
0,75
0,50
0,25
0,00
-0,15
7,7
10,0
12,0
14,0
16,0
18,0
20,0
22,0
24,0
26,0
28,0
30,0
32,0

Fig. 4b

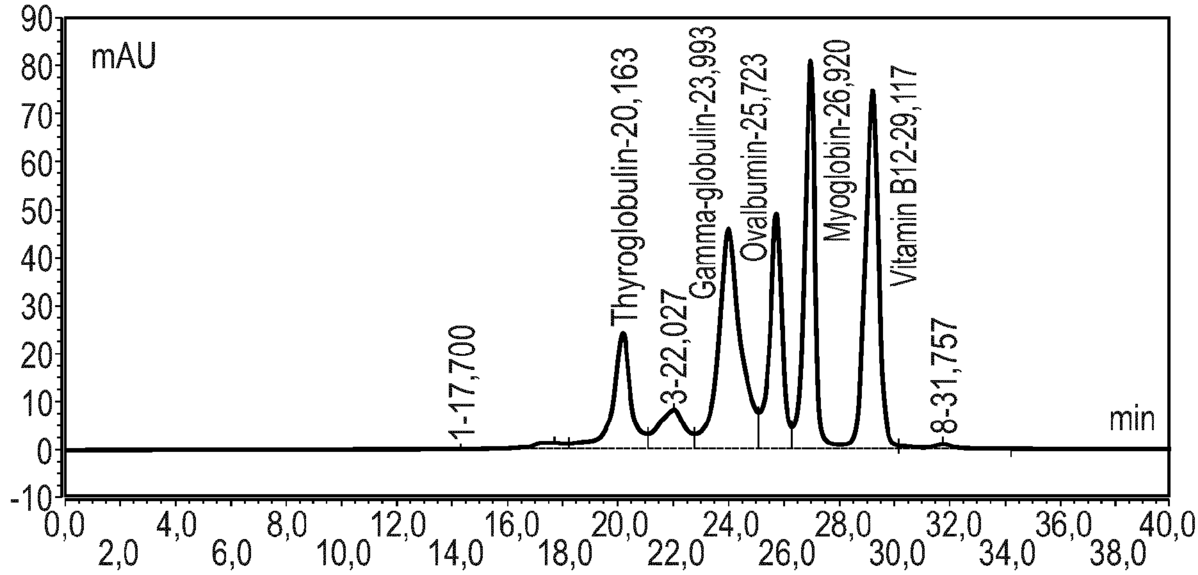
mAU
1-17,700
Thyroglobulin-20,163
3-22,027
Gamma-globulin-23,993
Ovalbumin-25,723
Myoglobin-26,920
Vitamin B12-29,117
8-31,757
min
90
80
70
60
50
40
30
20
10
0
-10
0,0
2,0
4,0
6,0
8,0
10,0
12,0
14,0
16,0
18,0
20,0
22,0
24,0
26,0
28,0
30,0
32,0
34,0
36,0
38,0
40,0

Fig. 5

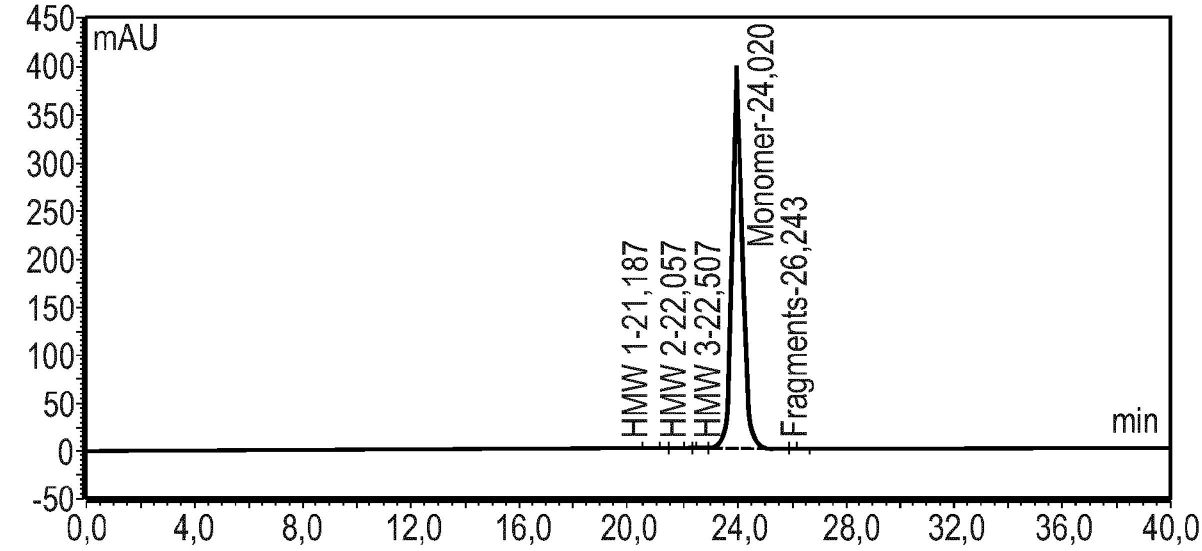


Fig. 6

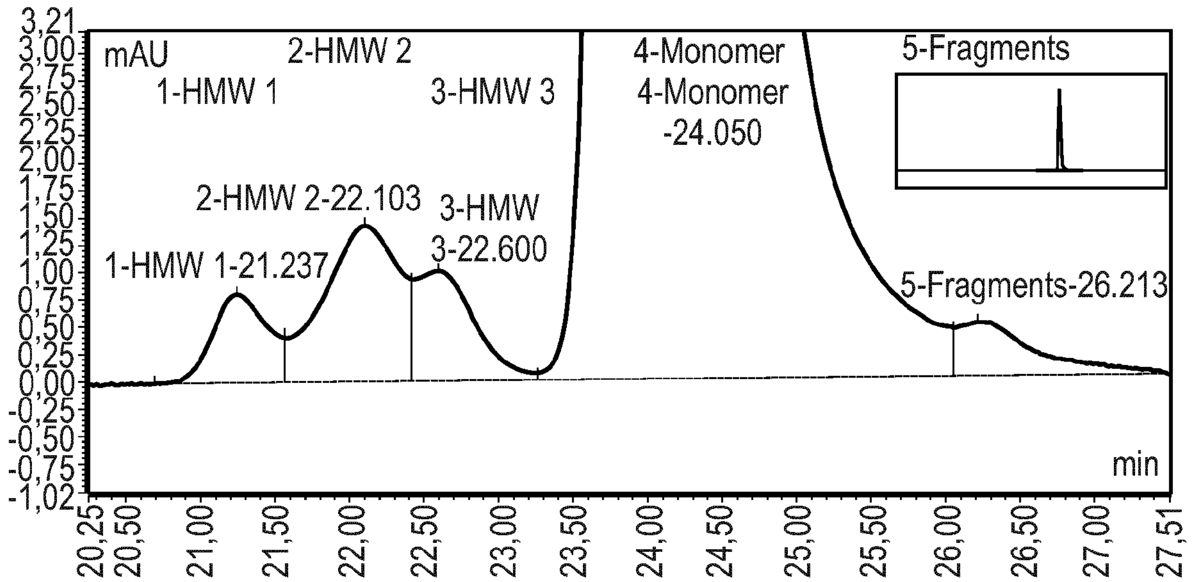


Fig. 7

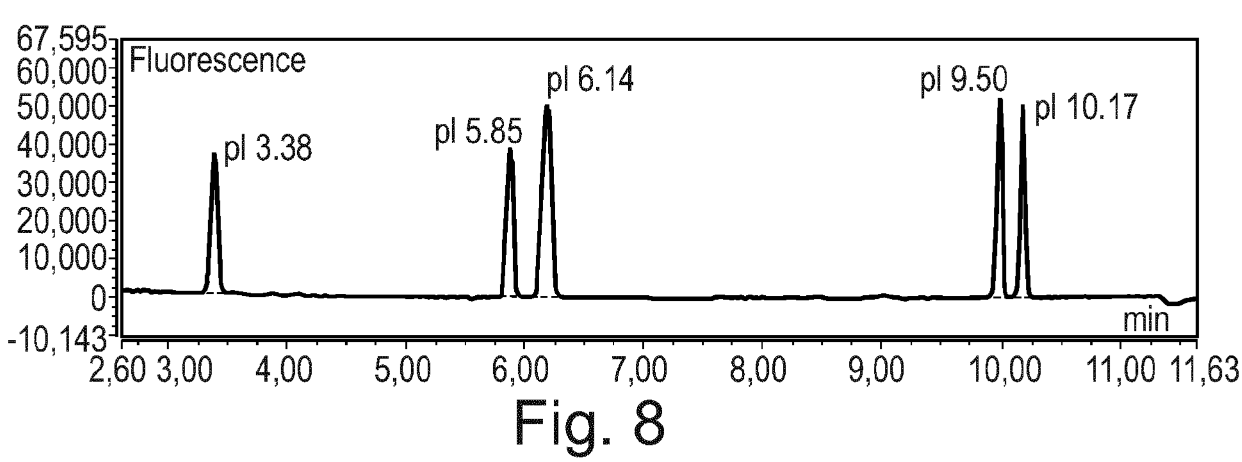


Fig. 8

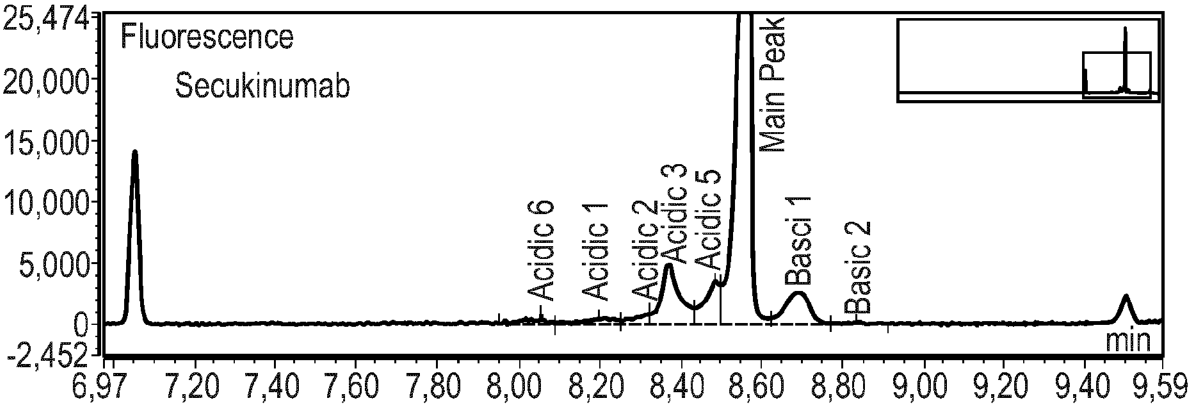


Fig. 9a

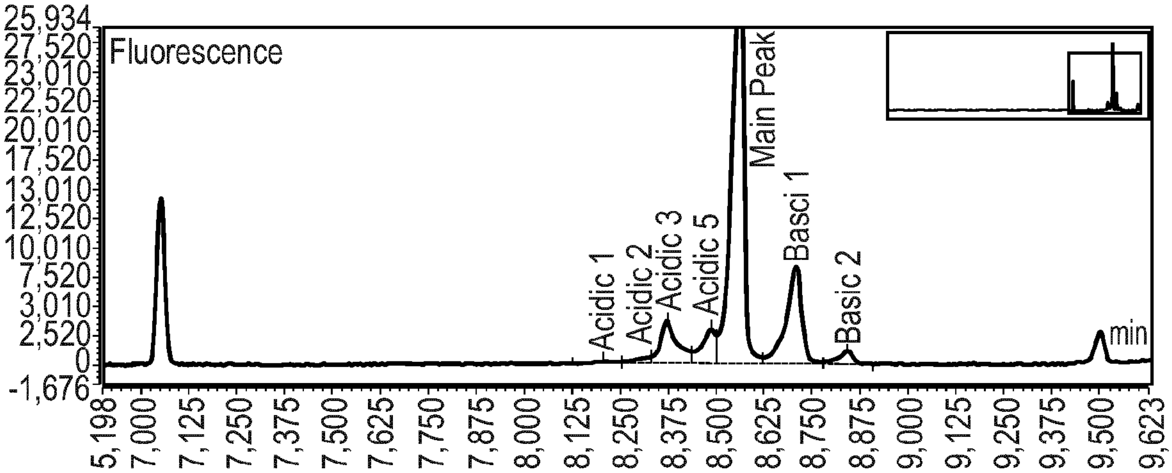


Fig. 9b

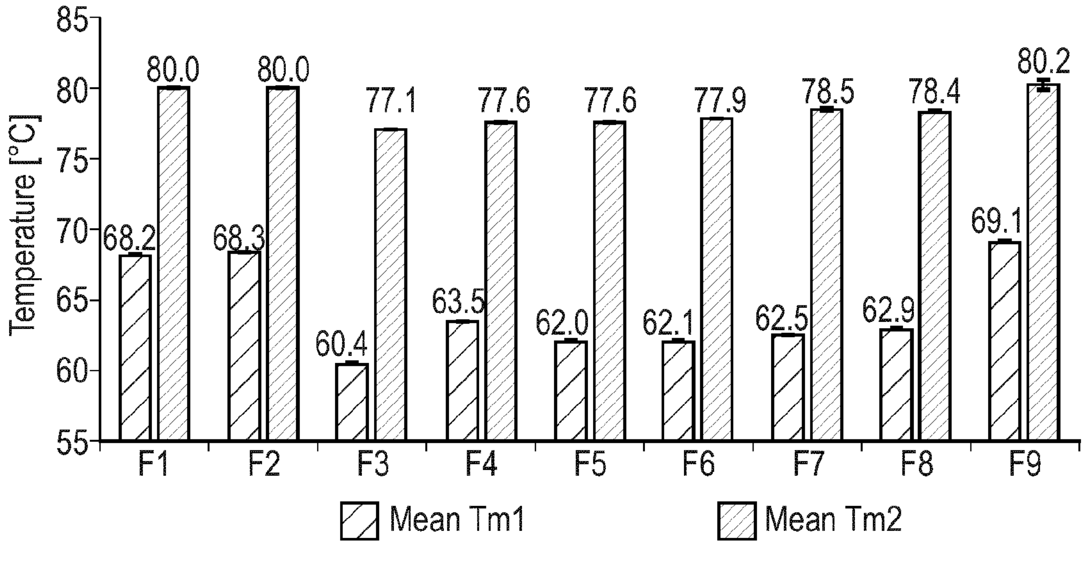


Fig. 10a

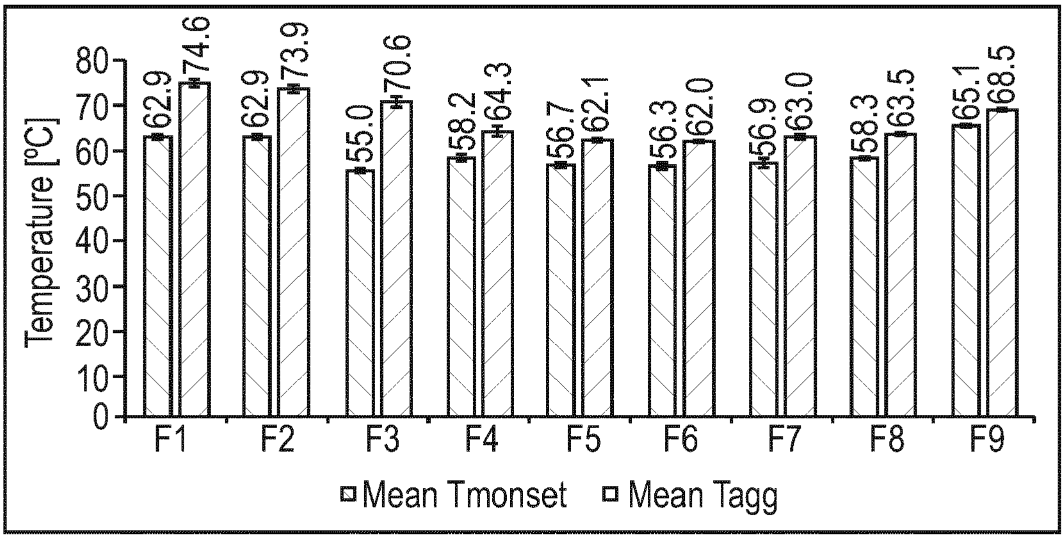


Fig. 10b

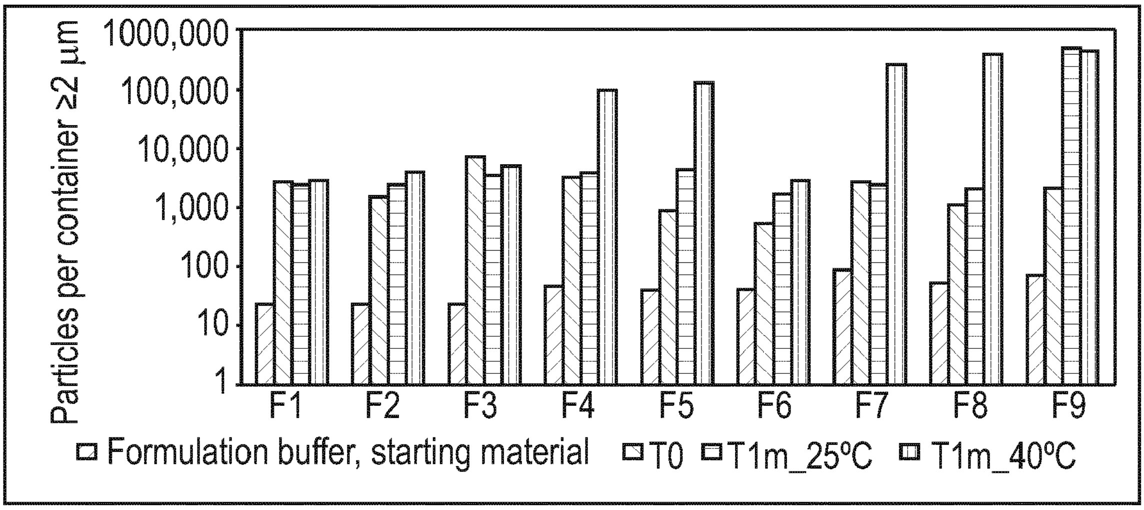


Fig. 11a

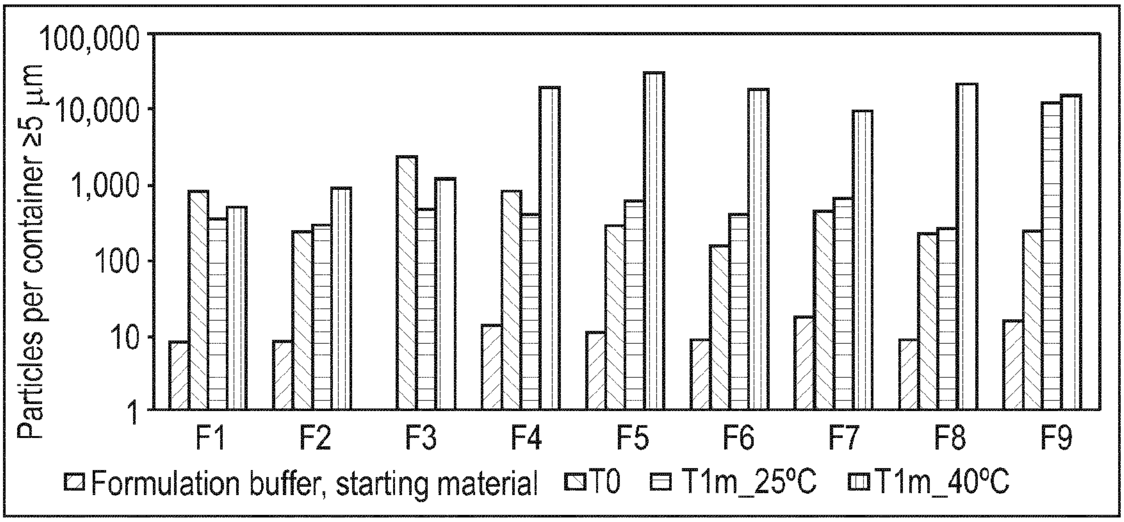


Fig. 11b

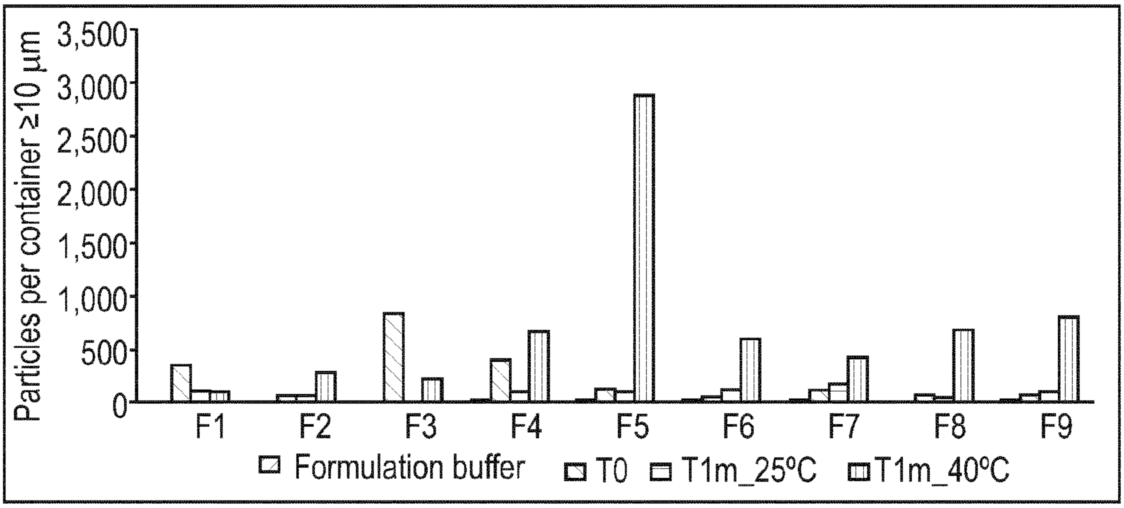


Fig. 11c

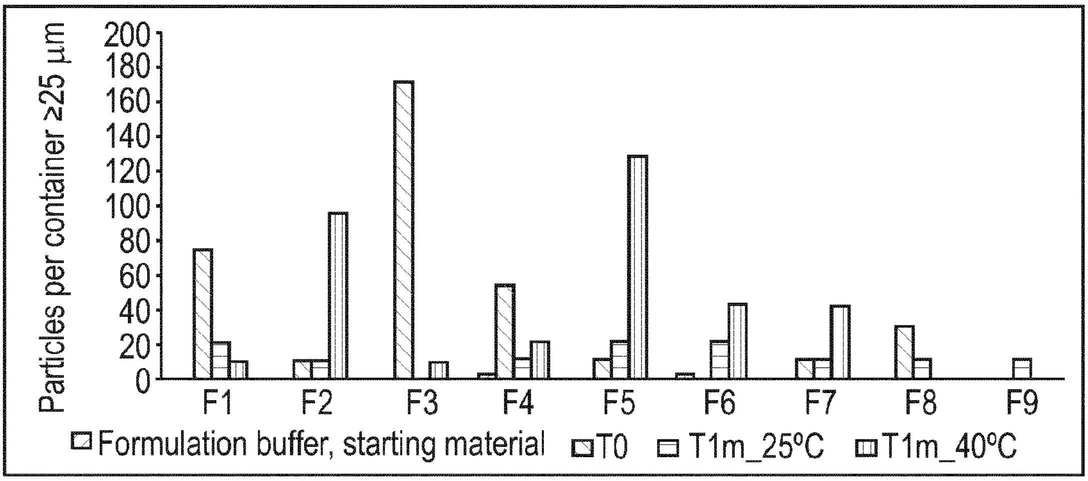


Fig. 11d

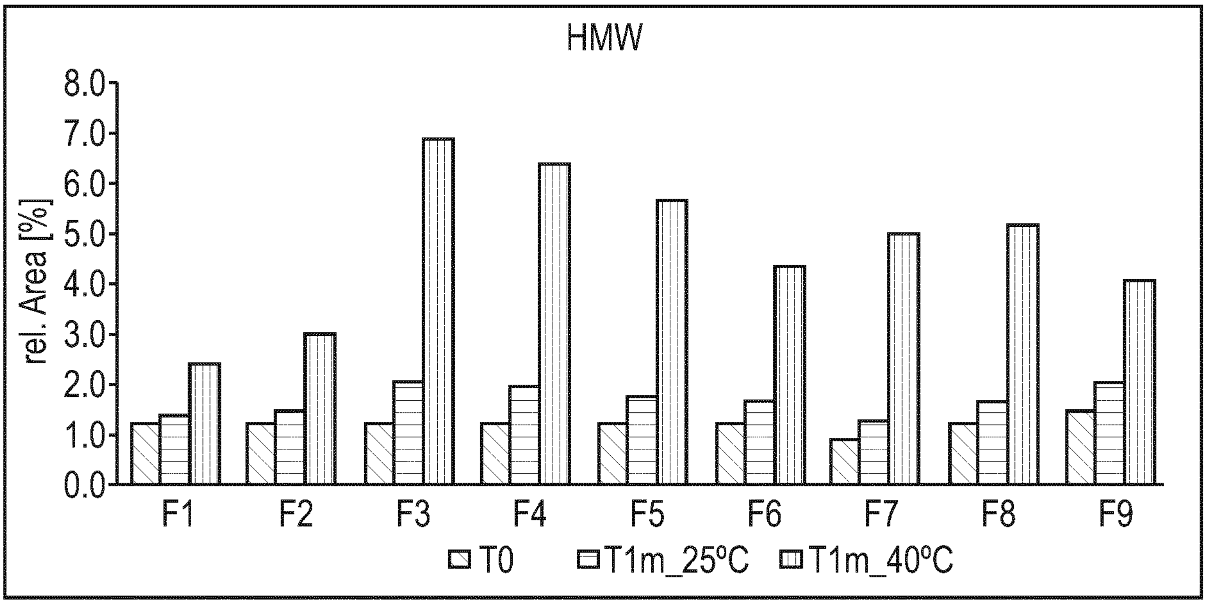


Fig. 12a

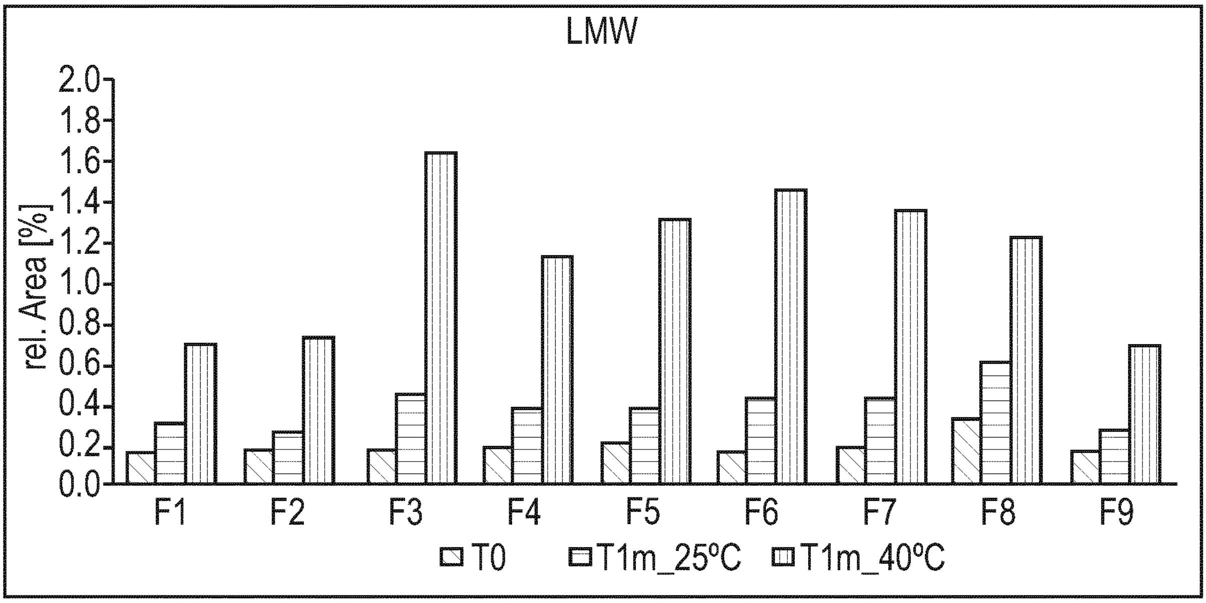


Fig. 12b

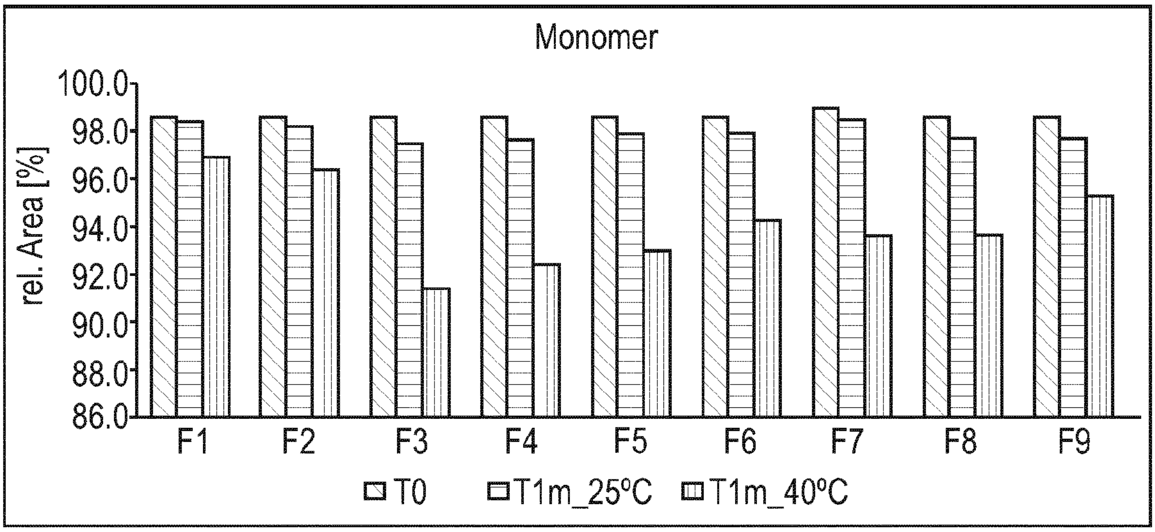


Fig. 12c

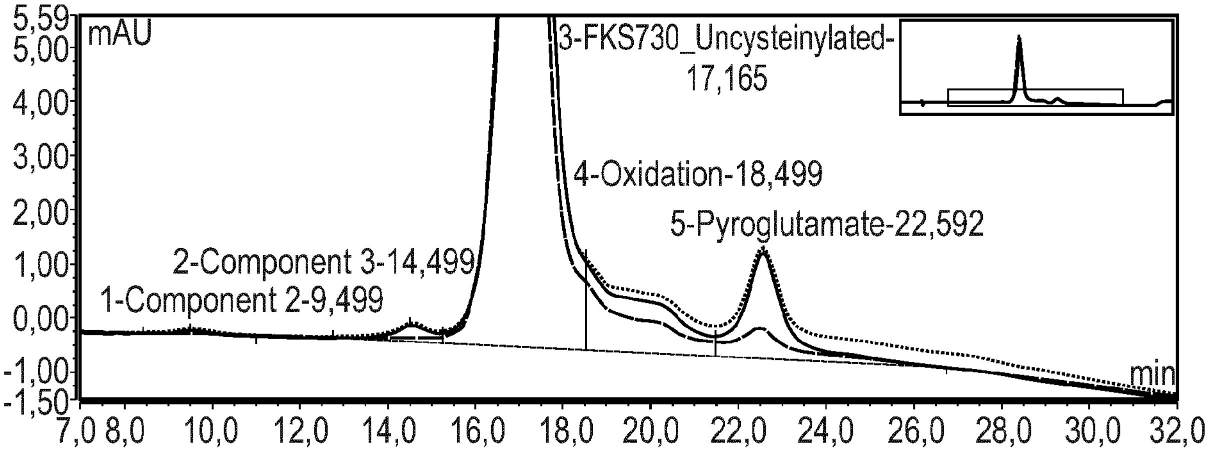


Fig. 13a

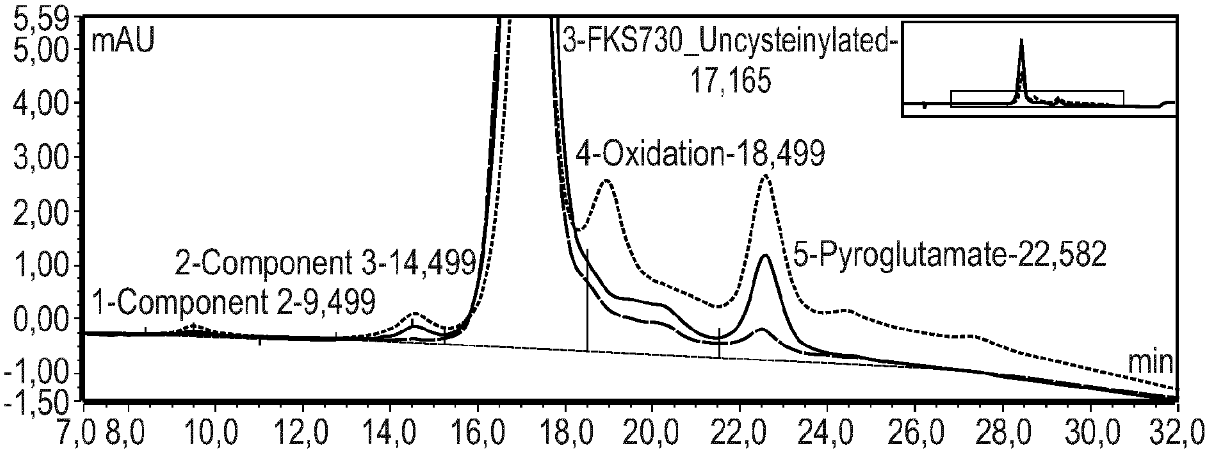


Fig. 13b

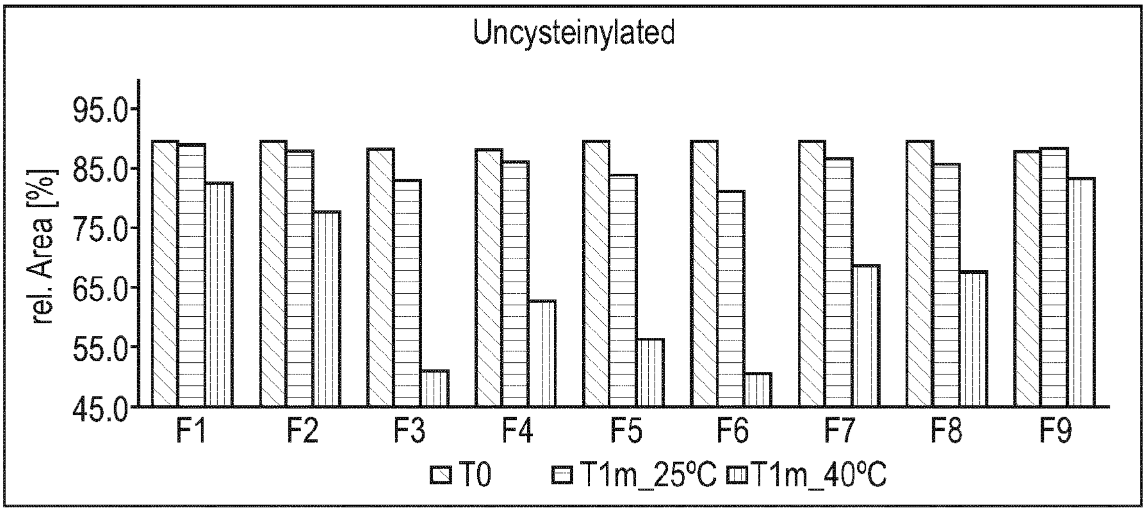


Fig. 14a

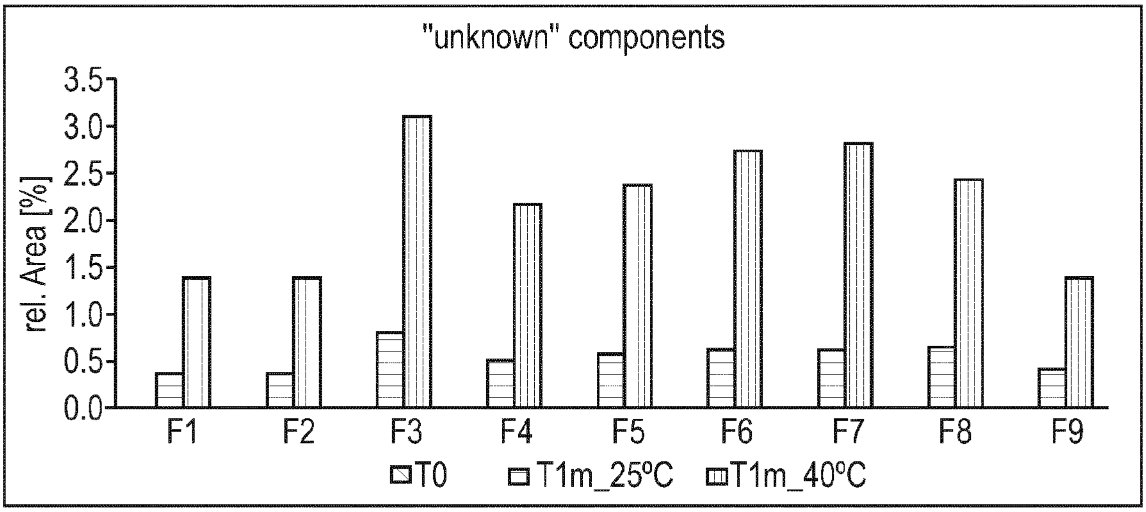


Fig. 14b

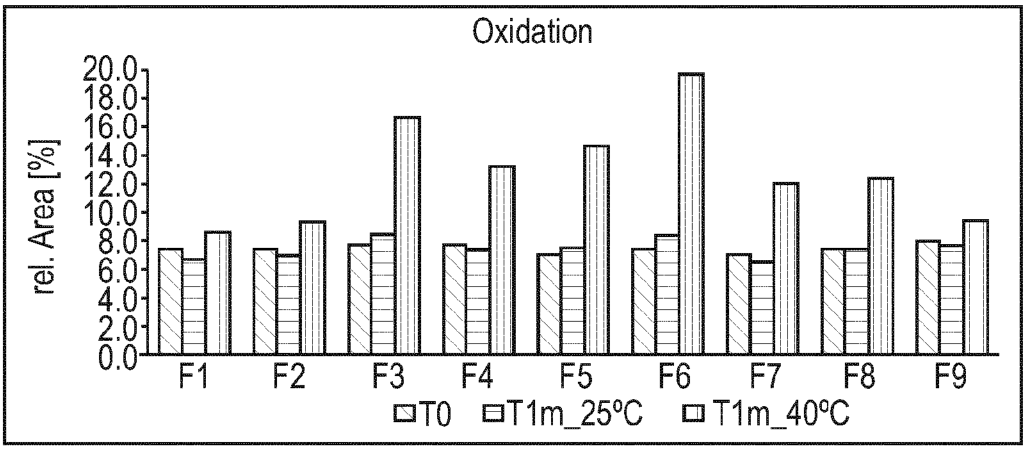


Fig. 14c

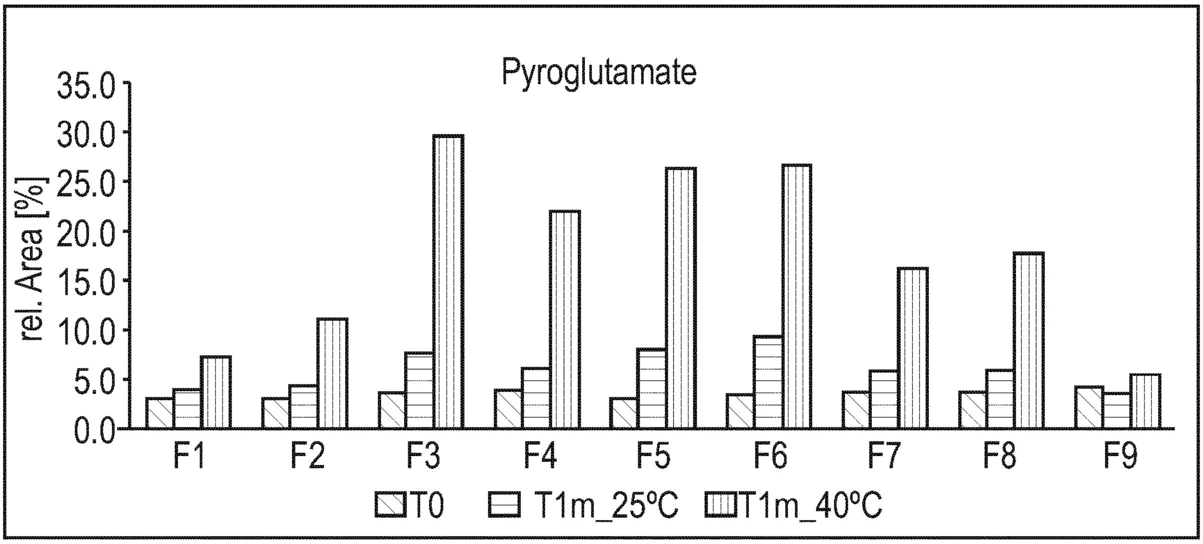


Fig. 14d

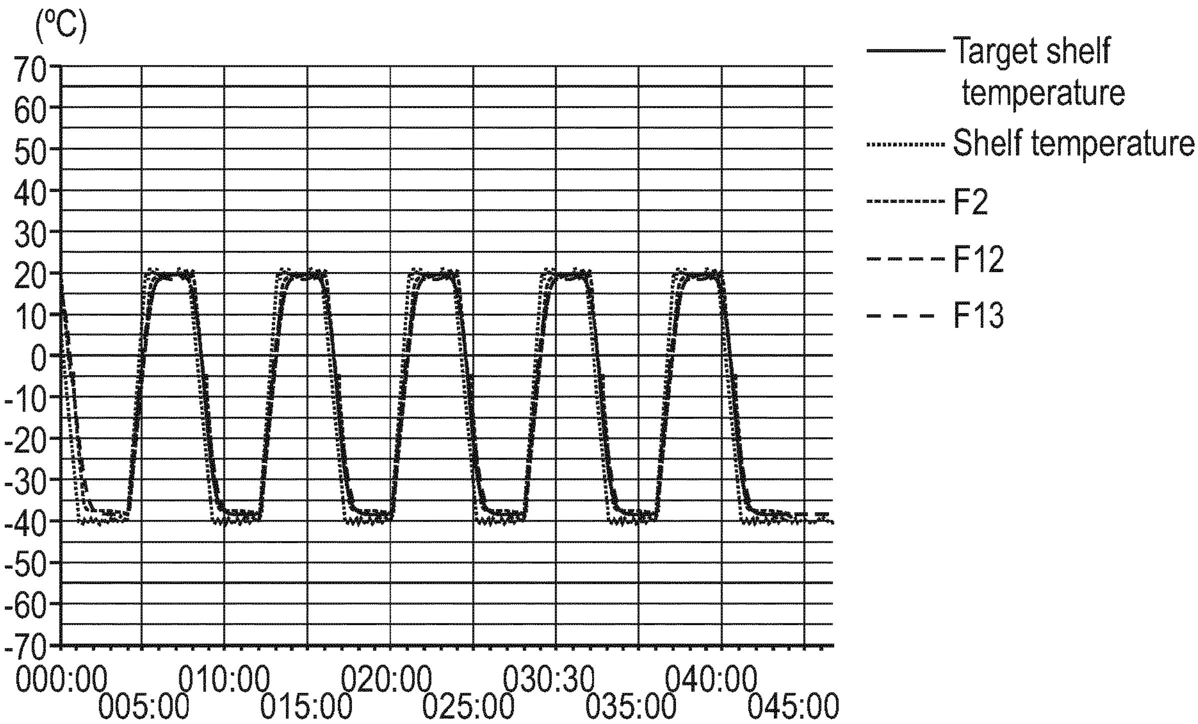


Fig. 15

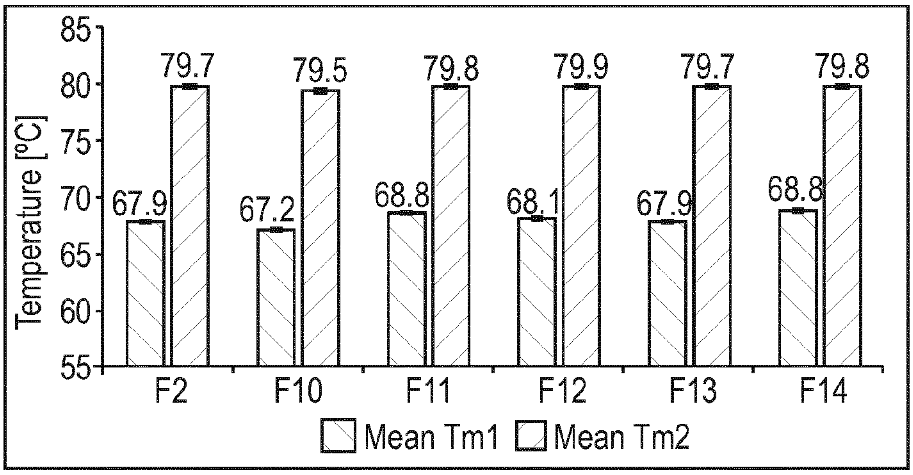


Fig. 16a

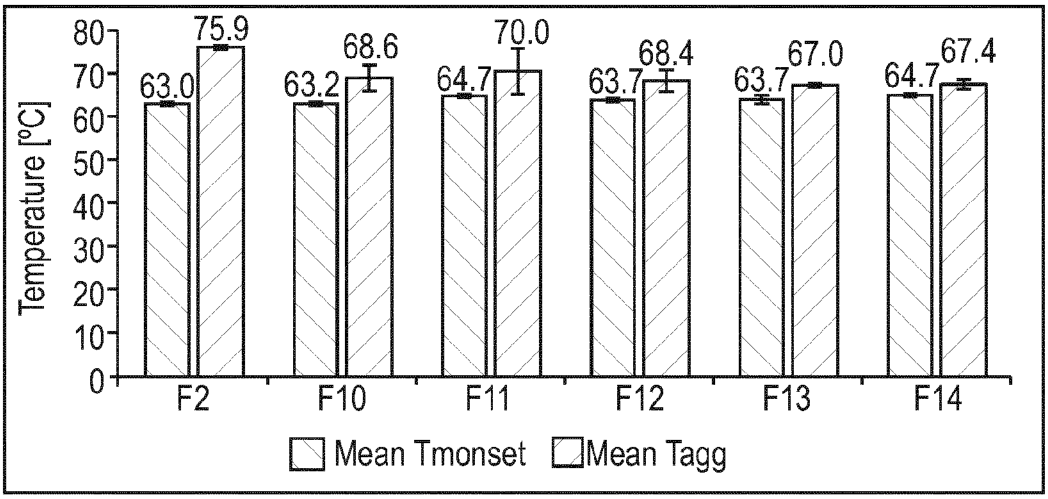


Fig. 16b

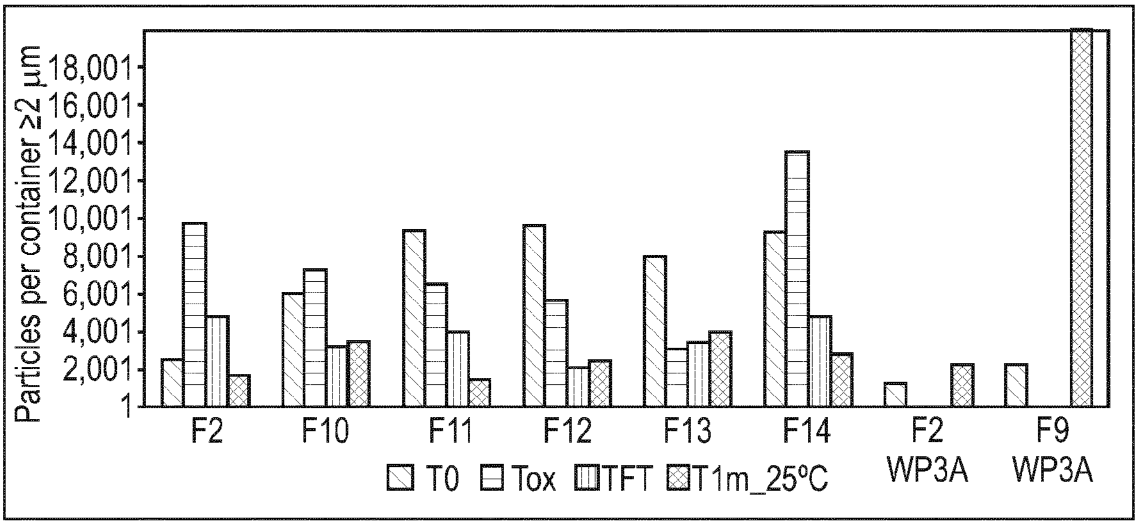


Fig. 17a

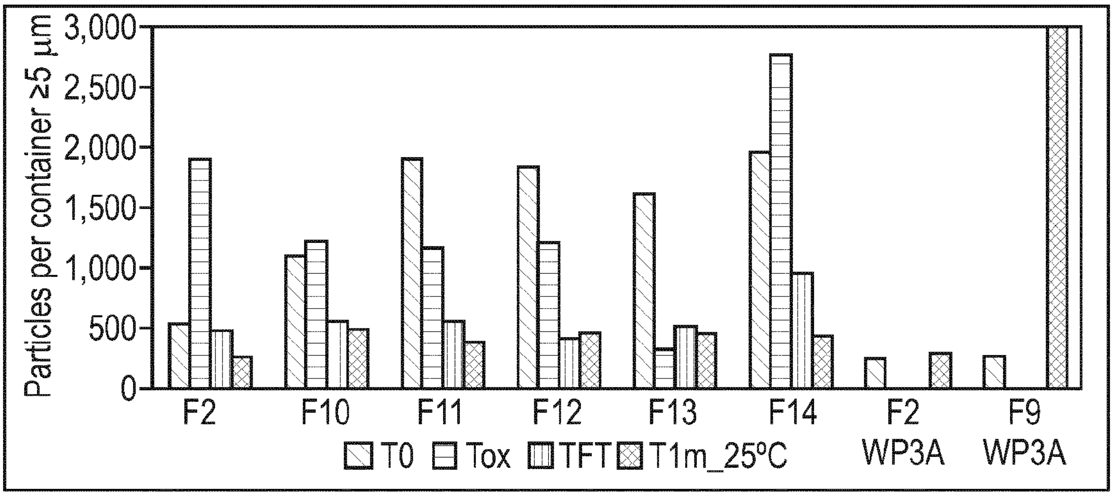


Fig. 17b

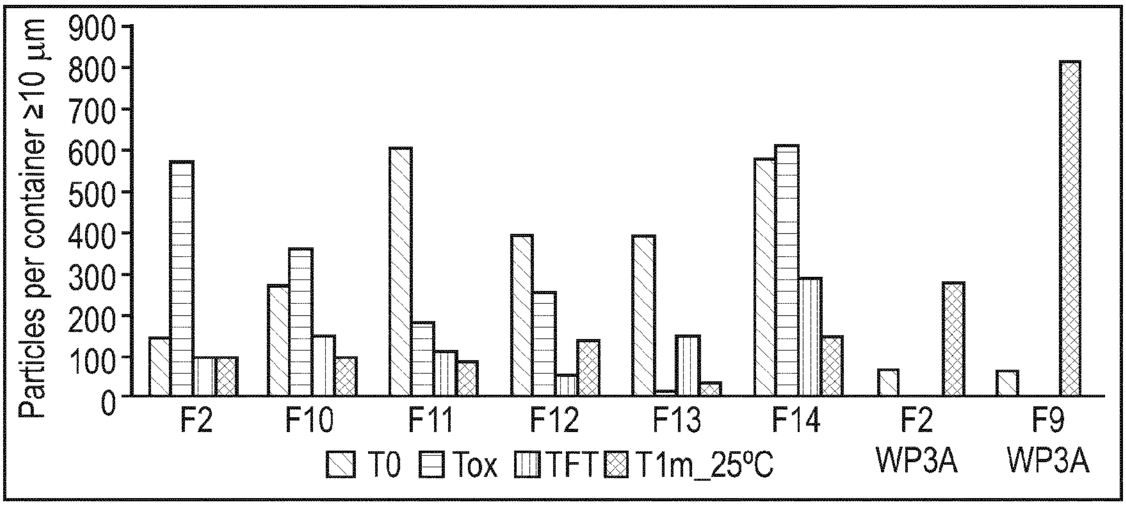


Fig. 17c

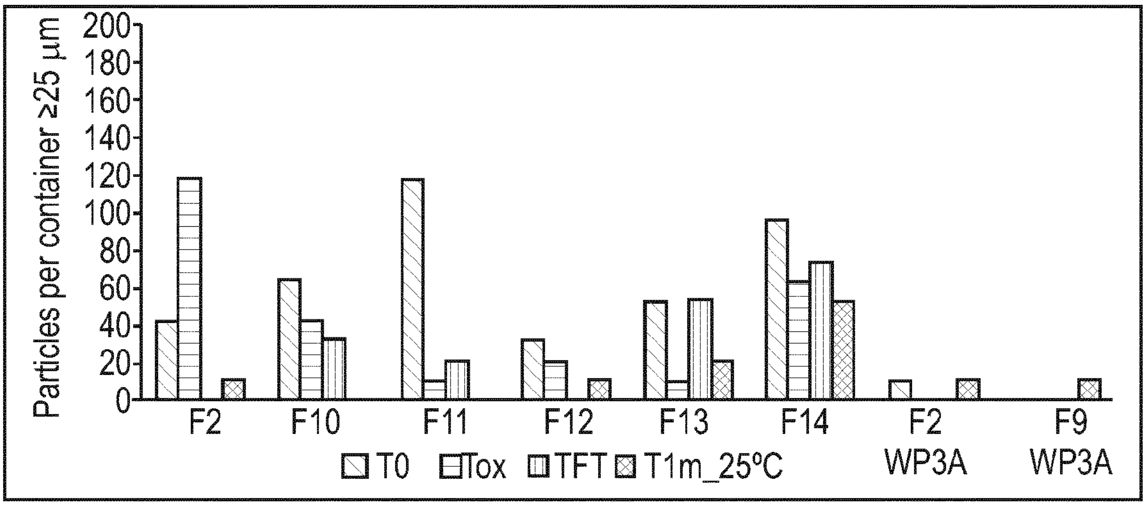


Fig. 17d

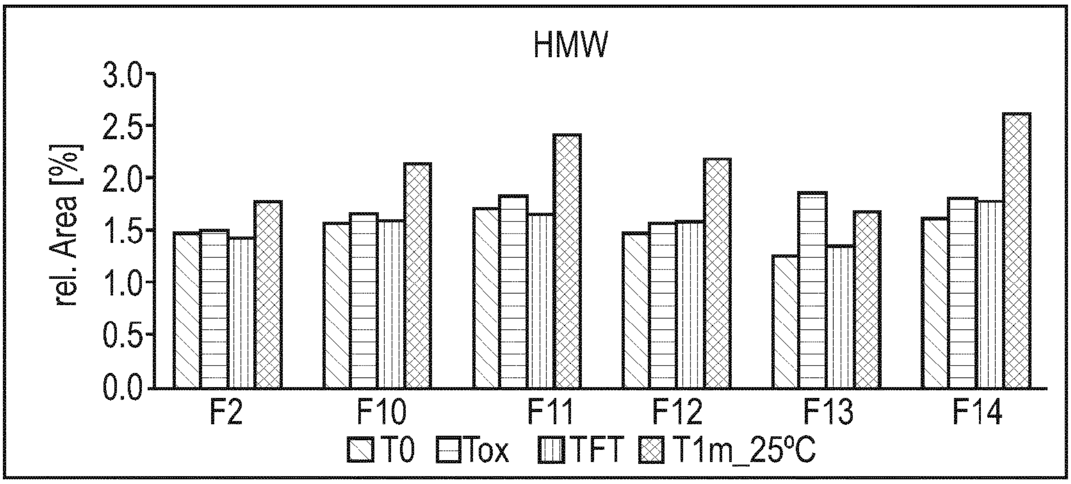


Fig. 18a

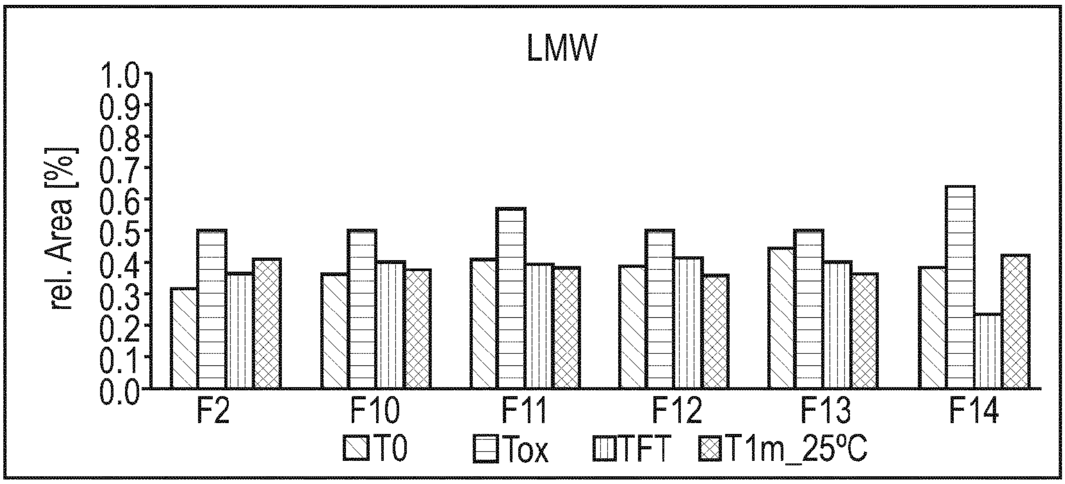


Fig. 18b

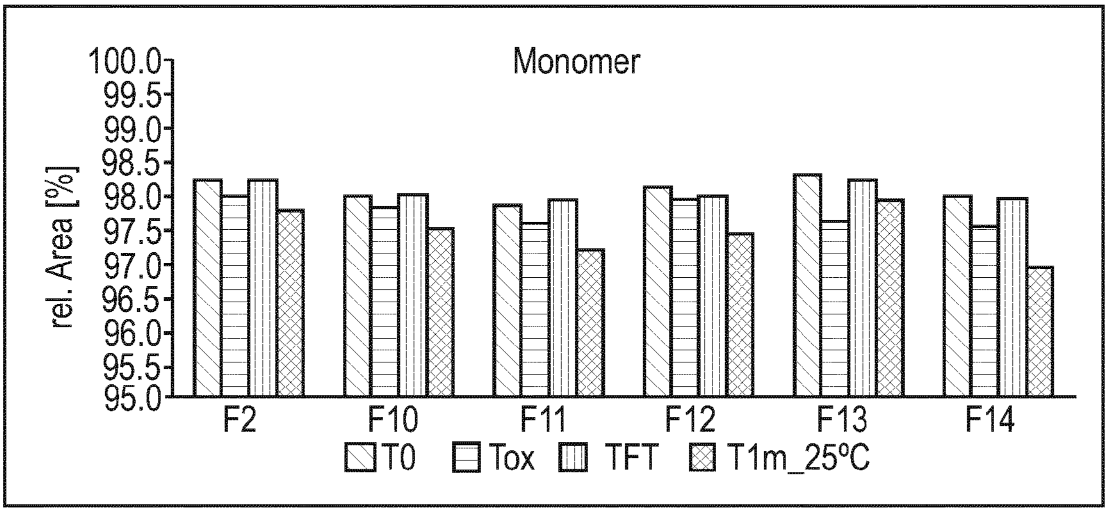


Fig. 18c

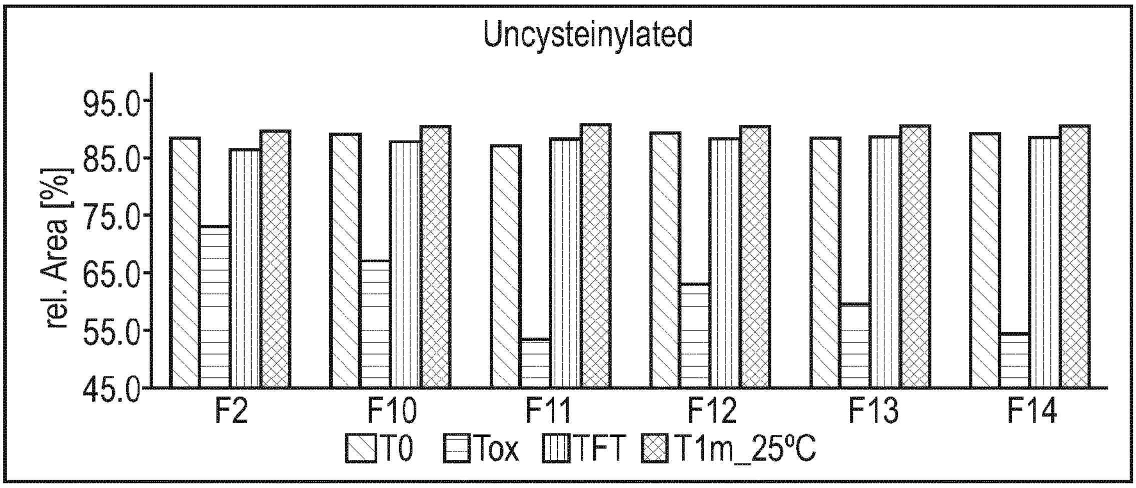


Fig. 19a

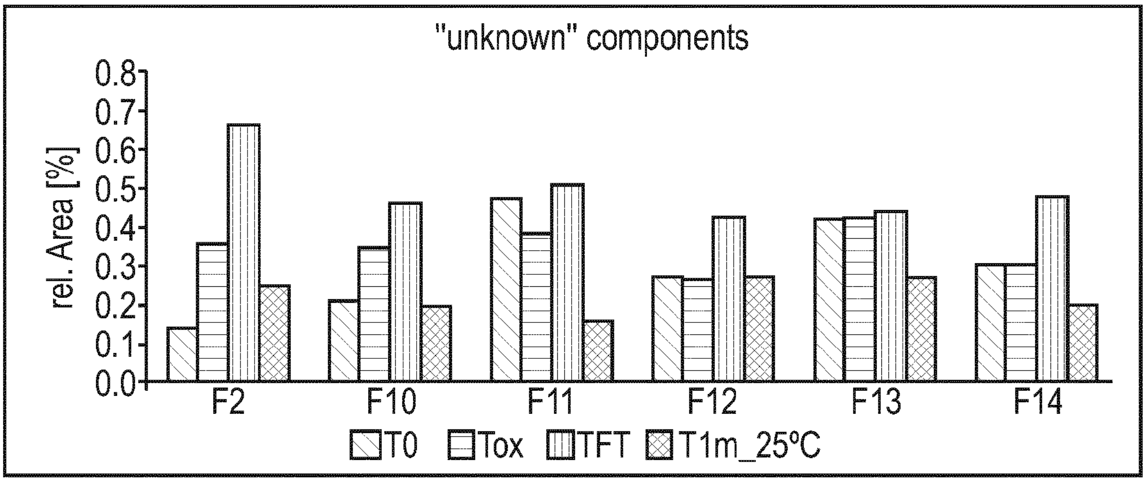


Fig. 19b

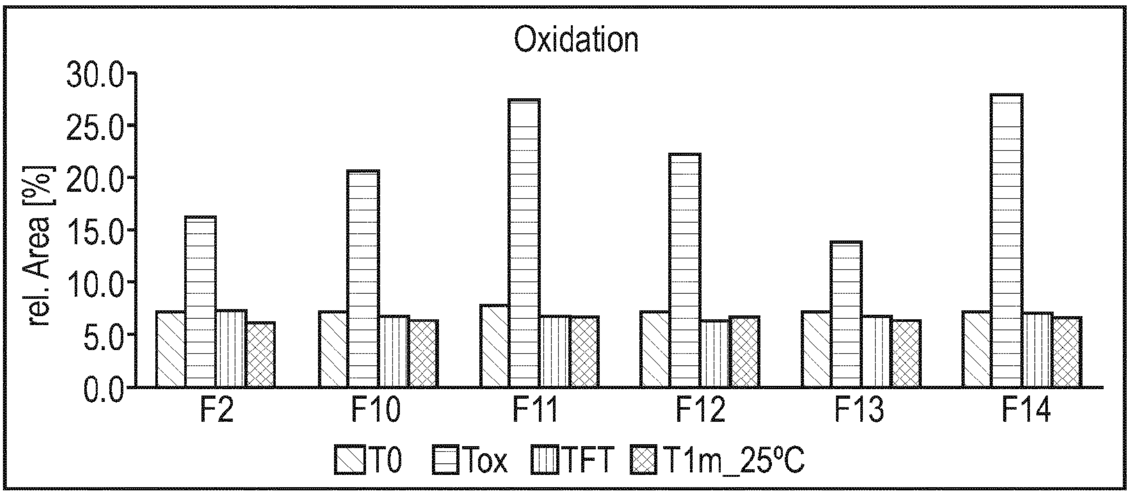


Fig. 19c

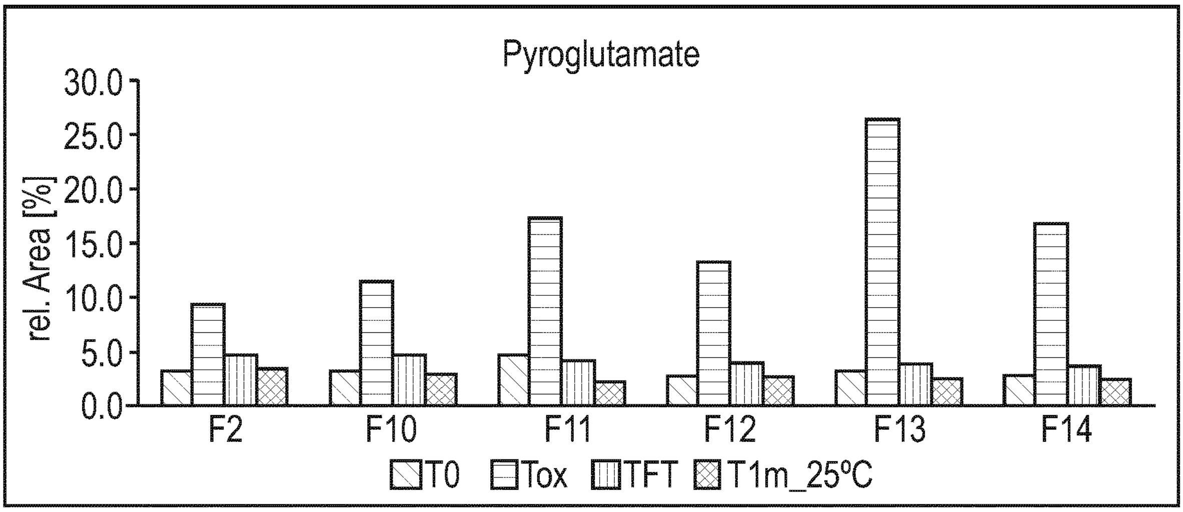
Pyroglutamate
rel. Area [%]
30.0
25.0
20.0
15.0
10.0
5.0
0.0
F2
F10
F11
F12
F13
F14
T0
Tox
TFT
T1m_25ºC

Fig. 19d

Europäisches Patentamt
European Patent Office
Office européen des brevets

# EUROPEAN SEARCH REPORT

Application Number
EP 23 15 3806

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/135395 A1 (BIO THERA SOLUTIONS LTD [CN]) 30 June 2022 (2022-06-30)<br>* examples 1, 3, 5 *<br>* page 8, lines 18-19 *<br>* page 2, lines 24-26 *<br>----- | 1-9,12, 16 | INV.<br>A61K39/395<br>A61K31/047<br>C07K16/24 |
| X | US 2009/280131 A1 (DI PADOVA FRANCO E [CH] ET AL) 12 November 2009 (2009-11-12)<br>* paragraph [0149] *<br>----- | 1-3,5,6, 12 | |
| X | US 2017/368174 A1 (JOERG SUSANNE [CH] ET AL) 28 December 2017 (2017-12-28)<br>* paragraphs [0188], [0189]; examples 1, 2 *<br>----- | 1-16 | |
| A | WO 2016/103146 A1 (NOVARTIS AG [CH]; HEITZMANN MARKUS [CH]; WINKLER JOHANN [AT]) 30 June 2016 (2016-06-30)<br>* page 2, lines 1-10 *<br>----- | 1-16 | |
| | | | TECHNICAL FIELDS SEARCHED (IPC) |
| | | | A61K<br>C07K |

The present search report has been drawn up for all claims

2

| Place of search | Date of completion of the search | Examiner |
|---|---|---|
| The Hague | 6 July 2023 | Patti, Gabriele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 15 3806

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2023

| Patent document cited in search report | Publication date | Patent family member(s) | Publication date |
|---|---|---|---|
| WO 2022135395 A1 | 30-06-2022 | CN 114652825 A | 24-06-2022 |
| | | WO 2022135395 A1 | 30-06-2022 |
| US 2009280131 A1 | 12-11-2009 | AR 050200 A1 | 04-10-2006 |
| | | AT 517924 T | 15-08-2011 |
| | | AU 2005268857 A1 | 09-02-2006 |
| | | AU 2010201689 A1 | 20-05-2010 |
| | | BR PI0513078 A | 22-04-2008 |
| | | BR 122017009404 B1 | 22-02-2022 |
| | | BR 122018075556 B1 | 18-10-2022 |
| | | CA 2573586 A1 | 09-02-2006 |
| | | CN 101001645 A | 18-07-2007 |
| | | CY 1111963 T1 | 04-11-2015 |
| | | CY 1115444 T1 | 04-01-2017 |
| | | CY 1116256 T1 | 08-02-2017 |
| | | CY 1120723 T1 | 11-12-2019 |
| | | DK 1776142 T3 | 31-10-2011 |
| | | DK 2364729 T3 | 21-07-2014 |
| | | DK 2366405 T3 | 11-05-2015 |
| | | DK 2902039 T3 | 16-07-2018 |
| | | EC SP077198 A | 28-02-2007 |
| | | EP 1776142 A1 | 25-04-2007 |
| | | EP 2364729 A2 | 14-09-2011 |
| | | EP 2366405 A2 | 21-09-2011 |
| | | EP 2902039 A1 | 05-08-2015 |
| | | EP 3409288 A1 | 05-12-2018 |
| | | ES 2367440 T3 | 03-11-2011 |
| | | ES 2487533 T3 | 21-08-2014 |
| | | ES 2536228 T3 | 21-05-2015 |
| | | ES 2677245 T3 | 31-07-2018 |
| | | FR 15C0048 I1 | 28-08-2015 |
| | | HK 1101277 A1 | 12-10-2007 |
| | | HK 1207559 A1 | 05-02-2016 |
| | | HK 1256639 A1 | 27-09-2019 |
| | | HR P20110758 T1 | 30-11-2011 |
| | | HR P20150480 T1 | 17-07-2015 |
| | | HR P20181069 T1 | 07-09-2018 |
| | | HU E025815 T2 | 28-04-2016 |
| | | HU E038187 T2 | 29-10-2018 |
| | | HU S1500037 I1 | 30-10-2017 |
| | | HU S1800040 I1 | 28-11-2018 |
| | | IL 180717 A | 31-07-2012 |
| | | JP 4682200 B2 | 11-05-2011 |
| | | JP 2008507988 A | 21-03-2008 |
| | | KR 20070036166 A | 02-04-2007 |
| | | KR 20080029018 A | 02-04-2008 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 23 15 3806

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2023

| Patent document cited in search report | Publication date | Patent family member(s) | Publication date |
|---|---|---|---|
| | | LT 2902039 T | 25-06-2018 |
| | | LT PA2015029 I1 | 11-07-2022 |
| | | LT PA2018515 I1 | 25-10-2018 |
| | | LU 92768 I2 | 03-11-2015 |
| | | LU C00088 I2 | 17-12-2018 |
| | | MA 28982 B1 | 01-11-2007 |
| | | MY 144925 A | 30-11-2011 |
| | | NO 336279 B1 | 06-07-2015 |
| | | NO 337129 B1 | 25-01-2016 |
| | | NO 337286 B1 | 29-02-2016 |
| | | NO 341384 B1 | 30-10-2017 |
| | | NO 2015023 I1 | 02-11-2015 |
| | | NO 2016017 I1 | 23-08-2016 |
| | | NO 2018007 I1 | 14-02-2018 |
| | | NZ 552658 A | 27-11-2009 |
| | | PE 20060418 A1 | 15-06-2006 |
| | | PL 1776142 T3 | 30-12-2011 |
| | | PL 2364729 T3 | 31-10-2014 |
| | | PL 2366405 T3 | 31-08-2015 |
| | | PL 2902039 T3 | 28-09-2018 |
| | | PT 1776142 E | 06-10-2011 |
| | | PT 2364729 E | 01-09-2014 |
| | | PT 2366405 E | 30-06-2015 |
| | | PT 2902039 T | 17-07-2018 |
| | | RU 2011113153 A | 20-10-2012 |
| | | SG 155186 A1 | 30-09-2009 |
| | | SI 1776142 T1 | 30-11-2011 |
| | | SI 2364729 T1 | 29-08-2014 |
| | | SI 2366405 T1 | 31-07-2015 |
| | | SI 2902039 T1 | 31-07-2018 |
| | | TN SN07034 A1 | 02-06-2008 |
| | | TR 201808057 T4 | 21-06-2018 |
| | | TW I359153 B | 01-03-2012 |
| | | US 2009280131 A1 | 12-11-2009 |
| | | US 2010215666 A1 | 26-08-2010 |
| | | US 2012107325 A1 | 03-05-2012 |
| | | US 2014079719 A1 | 20-03-2014 |
| | | US 2015152178 A1 | 04-06-2015 |
| | | US 2017355762 A1 | 14-12-2017 |
| | | US 2019270804 A1 | 05-09-2019 |
| | | WO 2006013107 A1 | 09-02-2006 |
| | | ZA 200700242 B | 27-08-2008 |
| US 2017368174 A1 | 28-12-2017 | AR 103173 A1 | 19-04-2017 |
| | | AU 2015370522 A1 | 01-06-2017 |
| | | BR 112017013240 A2 | 06-02-2018 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 23 15 3806

This annex lists the patent family members relating to the patent documents cited in the above-mentioned European search report. The members are as contained in the European Patent Office EDP file on
The European Patent Office is in no way liable for these particulars which are merely given for the purpose of information.

06-07-2023

| Patent document cited in search report | Publication date | Patent family member(s) | Publication date |
|---|---|---|---|
| | | CA 2971800 A1 | 30-06-2016 |
| | | CL 2017001638 A1 | 13-04-2018 |
| | | CN 107257692 A | 17-10-2017 |
| | | CN 113855800 A | 31-12-2021 |
| | | CN 113876947 A | 04-01-2022 |
| | | CN 114452380 A | 10-05-2022 |
| | | CN 114452381 A | 10-05-2022 |
| | | CO 2017005950 A2 | 31-08-2017 |
| | | EA 201791422 A1 | 31-10-2017 |
| | | EC SP17047155 A | 28-02-2019 |
| | | EP 3237001 A1 | 01-11-2017 |
| | | IL 287328 A | 01-12-2021 |
| | | JP 6770974 B2 | 21-10-2020 |
| | | JP 6878524 B2 | 26-05-2021 |
| | | JP 2018502910 A | 01-02-2018 |
| | | JP 2020011963 A | 23-01-2020 |
| | | JP 2021119165 A | 12-08-2021 |
| | | KR 20170095878 A | 23-08-2017 |
| | | KR 20230037677 A | 16-03-2023 |
| | | PE 20170952 A1 | 13-07-2017 |
| | | PH 12017500966 A1 | 18-10-2017 |
| | | SG 11201703828V A | 28-07-2017 |
| | | TN 2017000181 A1 | 19-10-2018 |
| | | TW 201641104 A | 01-12-2016 |
| | | US 2017368174 A1 | 28-12-2017 |
| | | WO 2016103153 A1 | 30-06-2016 |
| WO 2016103146 A1 | 30-06-2016 | AR 103172 A1 | 19-04-2017 |
| | | AU 2015370515 A1 | 18-05-2017 |
| | | BR 112017010069 A2 | 06-02-2018 |
| | | CA 2971541 A1 | 30-06-2016 |
| | | CN 107001460 A | 01-08-2017 |
| | | CN 114010780 A | 08-02-2022 |
| | | CN 114249826 A | 29-03-2022 |
| | | CN 114316044 A | 12-04-2022 |
| | | EP 3237445 A1 | 01-11-2017 |
| | | IL 298761 A | 01-02-2023 |
| | | JP 7021950 B2 | 17-02-2022 |
| | | JP 2018507250 A | 15-03-2018 |
| | | JP 2022062194 A | 19-04-2022 |
| | | KR 20170088906 A | 02-08-2017 |
| | | PH 12017500967 A1 | 18-10-2017 |
| | | RU 2017126025 A | 24-01-2019 |
| | | RU 2020113344 A | 02-09-2020 |
| | | SG 11201703639Q A | 28-07-2017 |
| | | US 2017369567 A1 | 28-12-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 23 15 3806

This annex lists the patent family members relating to the patent documents cited in the above-mentioned European search report. The members are as contained in the European Patent Office EDP file on
The European Patent Office is in no way liable for these particulars which are merely given for the purpose of information.

06-07-2023

| Patent document cited in search report | Publication date | Patent family member(s) | Publication date |
|---|---|---|---|
| | | US 2022315650 A1 | 06-10-2022 |
| | | WO 2016103146 A1 | 30-06-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006013107 A, Novartis Ag
- WO 2012059598 A
- WO 2016103153 A


### Non-patent literature cited in the description


- *CHEMICAL ABSTRACTS,* 1229022-83-6
- DESCRIPTION. FDA secukinumab Label. 28 May 2021
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat. Acad Sci. USA,* 1988, vol. 85, 2444
- **HIGGINS ; SHARP.** *Gene,* 1988, vol. 73, 23744
- **HIGGINS ; SHARP.** *CABIOS,* 1989, vol. 5, 151-3
- **CORPET et al.** *Nuc. Acids Res.,* 1988, vol. 16, 10881-90
- **HUANG et al.** Computer Appls. *Biosciences,* 1992, vol. 8, 155-65
- **PEARSON et al.** *Meth Mol. Bio.,* 1994, vol. 24, 307-31
- **ALTSCHUL et al.** *J. Mol. Biol.,* 1990, vol. 215, 403-10
- **ALTSCHUL et al.** J. Mol. Biol. National Center for Biological Information, 1990, vol. 215, 403-10
- **HANCOCK ; ARMSTRONG.** *Comput. Appl. Biosci.,* 1994, vol. 10, 67-70